# EUROPEAN PATENT APPLICATION

(11) **EP 2 080 812 A1**
(43) Date of publication of application: **22.07.2009**
(21) Application number: 08300038.0
(22) Date of filing: 18.01.2008
(51) Int. Cl.: C12Q 1/68, G01N 33/574, C07K 14/46

(54) **Compositions and methods of detecting post-stop peptides**

(71) Applicant: Transmedi SA, 54500 Vandoeuvre les Nancy (FR)
(72) Inventor: Brulliard, Marie, 54000 Nancy (FR); Bihain, Bernard, 54000 Nancy (FR)
(74) Representative: Becker, Philippe

(57) **Abstract**

The present invention relates to novel methods and products for assessing the physiological status of a subject. More particularly, the invention relates to methods of assessing the presence, risk or stage of a cancer in a subject by identifying or measuring the levels of proteins that exhibits post-stop peptides in a sample from the subject. The invention is also suitable to assess the responsiveness of a subject to a treatment, as well as to screen candidate drugs and design novel therapies. The invention may be used in any mammalian subject, particularly in human subjects.

## Description

The present invention relates to novel methods and products for assessing the physiological status of a subject. More particularly, the invention relates to methods of assessing the presence, risk or stage of a cancer in a subject by identifying or measuring the levels of proteins that exhibits post-stop peptides in a sample from the subject. The invention is also suitable to assess the responsiveness of a subject to a treatment, as well as to screen candidate drugs and design novel therapies. The invention may be used in any mammalian subject, particularly in human subjects.

### INTRODUCTION

We have previously shown that expressed sequence tag (EST) libraries that correspond to human mRNA derived from cancer cells contain significantly more base substitutions than those from normal cells ¹. This causes significant differences in mRNA heterogeneity isolated from normal and cancer cells from the same patient. The occurrence of base substitution in cancer mRNA is not random, but determined first by the nature of the substituted base and second by the composition of DNA context. Substitutions in cancer mRNA occur at sites that are 10⁴ more commonly encountered than those bearing somatic mutations ^{1,2} and do not correspond to single nucleotide polymorphisms (SNP) ^{1,3}. Further, > 80% of base substitutions cannot be explained by known enzymatic base modification processes ⁴. Considering the strong influence of DNA context that matches with the RNA Polymerase II (Pol II) active site ⁵ and *in vitro* evidence demonstrating forward slipping of Pol II in specific DNA contexts ^{6,7}, we proposed that nonrandom transcription infidelity (TI) events are responsible for the fact that a small fraction (2 to 10%) of cancer mRNA encoding a given transcript are not completely faithful copies of genomic DNA.

We have now expanded this first analysis to the whole genome and all available human transcripts. By conducting this extended analysis, we have shown that base substitutions occurring in natural stop codons as a result of transcription infidelity create novel coding regions that encode specific amino acid sequences (AA). These novel AA sequences, located at the carboxy-terminal end of proteins, which we call post-stop peptides, represent highly valuable products for the design of therapeutic or diagnostic methods and compositions.

### SUMMARY OF THE INVENTION

An object of this invention therefore relates to polypeptides comprising the sequence of a post-stop peptide created by transcription infidelity in a stop codon. In a preferred embodiment, the polypeptides of this invention comprise the sequence of a post-stop peptide of a human protein, preferably a secreted, plasmatic or membrane protein. In a particular embodiment, the polypeptide of this invention comprises a sequence selected from SEQ ID NOs: 1 to 1596 or an epitope-containing fragment thereof. In a particular embodiment, the polypeptide of this invention comprises the sequence located on the C-terminal side of the X residue in any one of SEQ ID NOs: 1 to1596, or of an epitope-containing fragment thereof.

Another object of this invention relates to a polynucleotide encoding a polypeptide as defined above, or a complementary strand thereof.

The invention also relates to a vector comprising a polynucleotide as defined above, as well as to recombinant host cells comprising such a vector or polynucleotide.

A further object of this invention is an isolated immune cell comprising a TCR specific for a post-stop peptide as defined above. Such a cell is preferably a mammalian cell, typically a human cell, and may include B cells, dendritic cells or T cells.

The invention also relates to a device or product comprising, immobilized on a support, at least one polypeptide or polynucleotide as defined above.

The invention also relates to an antibody that specifically binds a polypeptide as defined above. The antibody may be monoclonal or polyclonal. The term antibody also designates antibody fragments or derivatives, such as Fab, CDR, Single chain antibodies, humanized antibodies, etc.

A further object of this invention is a composition comprising a polypeptide, polynucleotide, antibody or immune cell as defined above, and a suitable excipient or vehicle.

Another object of this invention relates to a method for detecting the presence, risk or stage of development of a cancer in a subject, the method comprising a step of measuring the presence or level of a protein that exhibits a post-stop peptide in the subject or in a sample from the subject, wherein the presence or level of such protein that exhibits a post-stop peptide is an indication of the presence, risk or stage of development of a cancer.

In a preferred embodiment, the method comprises detecting simultaneously within the sample several proteins that exhibits post-stop peptides created by transcription infidelity, preferably from 2 to 100, 2 to 50 or from 2 to 10. In a further preferred embodiment, the protein comprises at least a post-stop peptide sequence as contained in the sequences selected from SEQ ID NOs: 1 to 1596, or an epitope-containing fragment thereof.

A further object of this invention relates to a method for detecting post-stop peptides by tandem mass spectrometry, the method comprising creating spectral libraries or fragmentation pattern databases specific of post-stop peptides and running software programs or algorithms to search or compare such databases or libraries with the output of MS/MS experiments.

A further object of this invention relates to a method for detecting the presence, risk or stage of development of a cancer in a subject, the method comprising contacting in vitro a sample from the subject with a polypeptide comprising the sequence of a post-stop peptide domain created by transcription infidelity and determining whether the sample contains any antibody or TCR-bearing cell that binds to said peptide, wherein the presence of such antibody or cell is an indication of the presence, risk or stage of development of a cancer.

In a preferred embodiment, the method comprises contacting simultaneously with the sample several polypeptides comprising the sequence of a post-stop peptide created by transcription infidelity, preferably from 2 to 100, 2 to 50 or from 2 to 10. In a further preferred embodiment, the polypeptide comprises a post-stop peptide sequence as contained in a selected from SEQ ID NOs: 1 to 1596, or an epitope-containing fragment thereof.

Also, in a specific embodiment, the polypeptide(s) is (are) immobilized on a support.

A further object of this invention relates to a method of assessing the physiological status of a subject, the method comprising a step of measuring the presence or level of a protein that exhibits a post-stop peptide in a sample from the subject and comparing said level to a reference level, wherein a deviation as compared to said reference level is an indication of a physiological disorder. The reference level may be e.g., a pre-determined mean or median value, a control value determined from a control sample, or a value determined at an earlier stage in a sample from the subject.

A further object of this invention relates to a method of assessing the physiological status of a subject, the method comprising a step of measuring the presence or level of antibodies specific for a post-stop peptide or of TCR-bearing immune cells that bind to such post-stop peptide in a sample from the subject, wherein a modified level of said antibodies or immune cells in said sample as compared to a reference level is an indication of a physiological disorder.

A further object of this invention relates to a method of direct detection of a protein that exhibits a post-stop peptide in a sample from the subject, the method comprising treating the sample to improve availability of the protein and detecting the protein by mass spectrometry. The sample is typically blood or a sub-fraction thereof. The sample is typically treated by lysis and/or dilution and/or fractioning and/or concentration and/or dialysis.

An other object of this invention resides in a method of producing a post-stop peptide specific for transcription infidelity, the method comprising :
- identifying a post-stop peptide sequence resulting from base substitution because of transcription infidelity in a natural stop codon;
- synthesizing said post-stop peptide.

A further object of this invention is a method a producing a polypeptide of this invention, the method comprising expressing a polynucleotide of this invention and recovering the polypeptide. Expression may be obtained e.g. in an acellular system, or in a cell cultured in vitro.

A further object of this invention is a method of producing an antibody, the method comprising immunizing a non-human animal with a polypeptide of this invention and recovering antibodies or antibody-producing cells from said animal. The antibody may be polyclonal or monoclonal, and may be subsequently modified to produce fragments thereof (e.g., Fab, CDR, etc) or derivatives thereof (e.g., Single chain antibodies, humanized antibodies, bi-functional antibodies, etc.) retaining at least substantially the same antigen specificity.

A further object of this invention is a method of selecting, optimizing or producing a drug candidate, the method comprising a step of determining whether a candidate compound modifies expression of a protein that exhibits a post-stop peptide. According to the target purpose, the candidate compound that increases or decreases said expression is selected.

Alternatively, candidate compounds which do not affect said expression may also be selected.

### LEGEND TO THE FIGURES

Figure 1. Principle of cDNA library construction and sequencing.
Figure 2. Results of bioinformatics and statistical analysis.
Figure 3. Results of C>N substitutions occurring within stop codon. Figure 3 presents C>N positions occurring within stop codon. RefSeq identifier and position on RefSeq are shown in the first column. The number of cancer and normal ESTs having A, T, C or G are also given.

### DETAILED DESCRIPTION OF THE INVENTION

The present invention relates to novel products and their uses in the medical area, e.g., for assessing the physiological status of a subject. More particularly, the invention relates to methods of assessing the presence, risk or stage of a cancer in a subject by measuring the presence or level of proteins that exhibits post-stop peptides in a sample from the subject. The invention is also suitable to assess the responsiveness of a subject to a treatment, as well as to screen or design candidate drugs.

Transcription infidelity designates a novel mechanism by which several distinct RNA molecules are produced in a cell from a single gene sequence. This newly identified mechanism potentially affects any gene, is non-random, and follows particular rules, as disclosed in co-pending application n° PCT/EP07/057541, the disclosure of which is incorporated herein in its entirety.

The present application shows that transcription infidelity introduces base substitutions in natural stop codons of RNA molecules, thereby creating novel coding regions that encode novel AA sequences at the carboxy-terminal end of proteins called post-stop peptides. These post-stop peptide sequences are long enough to contain epitopes against which antibodies may be generated by mammalians. As a result, the expression of proteins that exhibits post-stop peptides in a subject can be assessed by measuring the presence of corresponding antibodies or TCR-bearing cells in a sample from the subject.

The present invention now provides a method for predicting and/or identifying the sequence of post-stop peptides generated by transcription infidelity events from any gene, as well as methods of producing post-stop peptides. The invention also discloses more than 1,500 post-stop peptides.

In a first embodiment, the present invention is drawn to an isolated polypeptide comprising a post-stop peptide, i.e., a novel sequence of an aberrant protein domain created by a base substitution in a natural stop codon because of transcription infidelity. Specific examples of polypeptides of this invention comprise a sequence selected from SEQ ID NOs: 1 to 1596, or an epitope containing fragment thereof.

The term "epitope-containing fragment" denotes any fragment containing at least 3 consecutive amino acid residues, preferably at least 5, 6, 7 or 8 consecutive amino acid residues, which form an immunologic epitope for antibodies or TCR-expressing cells. Such an epitope may be linear or conformational, and specific for B- or T-cells.

Within the context of this invention, the term "isolated", when referring to a polypeptide, means the polypeptide is not in a naturally occurring medium (e.g., it is at least partially purified, or present e.g., in a synthetic medium).

The sequences as depicted in SEQ ID Nos. 1-1596 have the following general structure: native protein-X-PSP peptide. Accordingly, the amino acid sequences located on the C-terminal side of the X residue represent PSP peptides of this invention. In a specific embodiment, a polypeptide of this invention comprises the sequence of a PSP peptide as contained in any one of SEQ ID NOs: 1 to 1596, or of an epitope-containing fragment thereof, i.e., the sequence located on the C-terminal side of the X residue in any one of SEQ ID NOs: 1 to1596, or an epitope-containing fragment thereof.

A post-stop peptide sequence of this invention typically comprises between 3 and 100 amino acids, preferably between 3 and 50, more preferably between 3 and 30 amino acids. The post-stop peptides of this invention may be produced by any conventional technique, such as artificial polypeptide synthesis or recombinant technology.

Post-stop peptides of this invention may optionally comprise additional residues or functions, such as, without limitation, additional amino acid residues, chemical or biological groups, including labels, tags, stabilizer, targeting moieties, purification tags, secretory peptides, functionalizing reactive groups, etc. Such additional residues or functions may be chemically derivatized, added as an amino acid sequence region of a fusion protein, complexed with or otherwise either covalently or non-covalently attached. They may also contain natural or non-natural amino acids. The post-stop peptide may be in soluble form, or attached to (or complexed with or embedded in) a support, such as a matrix, a column, a bead, a plate, a membrane, a slide, a cell, a lipid, a well, etc.

The post-stop peptides of this invention may be present as monomers, or as multimers. Also, they may be in linear conformation, or in particular spatial conformation. In this respect, the post-stop peptides may be included in particular scaffold to display specific configuration.

Post stop peptides of the present invention may be used as immunogens in vaccine compositions or to produce specific antibodies. They may also by used to target drugs or other molecules (e.g., labels) to specific sites within an organism. They may also be used as specific reagents to detect or dose specific antibodies or TCR-bearing immune cells from any sample.

In this respect, a particular object of this invention resides in a device or product comprising a post-stop peptide as defined above attached to a solid support. The attachment is preferably a terminal attachment, thereby maintaining the post-stop peptide in a suitable conformation to allow binding of a specific antibody when contacted with a sample containing the same. The attachment may be covalent or non-covalent, directly to the support or through a spacer group. Various techniques have been reported in the art to immobilize a peptide on a support (polymers, ceramic, plastic, glass, silica, etc.), as disclosed for instance in Hall et al., Mechanisms of ageing and development 128 (2007) 161. The support may be magnetic, such as magnetic beads, to facilitate e.g., separation.

The device preferably comprises a plurality of post-stop peptides of this invention, e.g., arrayed in a pre-defined order, so that several antibodies may be detected or measured with the same device.

The device is typically made of any solid or semi-solid support, such as a titration plate, dish, slide, wells, membrane, bead, column, etc. The support preferably comprises at least two polypeptides comprising a PSP sequence as contained in any one of SEQ ID NO: 1 to 1596, more preferably from 2 to 10.

The support may comprise additional objects or biological elements, such as control polypeptides and/or polypeptides having a different immune reactivity.

Formation of an immune complex between the post-stop peptide and an antibody may be assessed by known techniques, such as by using a second labelled antibody specific for human antibodies, or by competition reactions, etc.

A further aspect of this invention resides in a kit comprising a device as disclosed above, as well as a reagent to perform an immune reaction.

A further aspect of this invention relates to a polynucleotide comprising a nucleotide sequence encoding a polypeptide as defined above, or a complementary strand thereof. Particularly, this polynucleotide comprises a first nucleotide sequence encoding a polypeptide comprising a PSP sequence as contained in any one of SEQ ID NOs: 1-1596 or a sequence complementary thereto, and a second nucleotide sequence of 100 or less nucleotides in length, wherein said second nucleotide sequence is adjacent to said first nucleotide sequence in a naturally occurring nucleic acid. The length of the second nucleotide sequence which is adjacent to the first nucleotide sequence may be, for example, 75, 50, 25, 10 or 0.

In a specific embodiment, the invention relates to a polynucleotide consisting of a nucleic acid sequence encoding a polypeptide as defined above.

The polynucleotides of the present invention may be DNA or RNA, such as complementary DNA, synthetic DNA, mRNA, or analogs of these containing, for example, modified nucleotides such as 3'alkoxyribonucleotides, methylphosphanates, and the like, and peptide nucleic acids (PNAs), etc. The polynucleotide may be labelled. The polynucleotide may be produced according to techniques well-known per se in the art, such as by chemical synthetic methods, in vitro transcription, or through recombinant DNA methodologies, using sequence information contained in the present application. In particular, the polynucleotide may be produced by chemical oligonucleotide synthesis, library screening, amplification, ligation, recombinant techniques, and combination(s) thereof.

Polynucleotides of this invention may comprise additional regulatory nucleotide sequences, such as e.g., promoters, enhancers, silencers, terminators, and the like that can be used to cause or regulate expression of a polypeptide.

Polynucleotides of this invention may be used to produce a recombinant polypeptide of this invention. They may also be used to design specific reagents such as primers, probes or antisense molecules (including antisense RNA, iRNA, aptamers, ribozymes, etc.), that specifically detect, bind or affect expression of a polynucleotide encoding a polypeptide as defined above. They may also be used as therapeutic molecules (e.g., as part of an engineered virus, such as, without limitation, an engineered adenovirus or adeno-associated virus vector in gene therapy programs) or to generate recombinant cells or genetically modified non-human animals, which are useful, for instance, in screening compound libraries for agents that modulate the activity of a polypeptide as defined above.

Within the context of this invention, a nucleic acid "probe" refers to a nucleic acid or oligonucleotide having a nucleotide sequence which is capable of selective hybridization with a polynucleotide of this invention or a complement thereof, and which is suitable for detecting the presence (or amount) thereof in a sample. Probes are preferably perfectly complementary to a transcription infidelity domain. However, certain mismatch may be tolerated. Probes typically comprise single-stranded nucleic acids of between 8 to 1500 nucleotides in length, for instance between 10 and 1000, more preferably between 10 and 800, typically between 20 and 400, even more preferably below 200. A preferred probe of this invention is a single stranded nucleic acid molecule of between 8 to 200 nucleotides in length, which can specifically hybridize to a transcription infidelity domain.

The term "primer" designates a nucleic acid or oligonucleotide having a nucleotide sequence which is capable of selective hybridization with a polynucleotide of this invention or a complement thereof, or with a region of a nucleic acid that flanks a transcription infidelity domain in a broader, naturally-occurring molecule, and which is suitable for amplifying all or a portion of said transcription infidelity domain in a sample containing the same. Typical primers of this invention are single-stranded nucleic acid molecules of about 5 to 60 nucleotides in length, more preferably of about 8 to about 50 nucleotides in length, further preferably of about 10 to 40, 35, 30 or 25 nucleotides in length. Perfect complementarity is preferred, to ensure high specificity. However, certain mismatch may be tolerated, as discussed above for probes.

Another aspect of this invention resides in a vector, such as an expression or cloning vector comprising a polynucleotide as defined above. Such vectors may be selected from plasmids, recombinant viruses, phages, episomes, artificial chromosomes, and the like. Many such vectors are commercially available and may be produced according to recombinant techniques well known in the art, such as the methods set forth in manuals such as Sambrook et al., Molecular Cloning (2d ed. Cold Spring Harbor Press 1989), which is hereby incorporated by reference herein in its entirety.

A further aspect of this invention resides in a host cell transformed or transfected with a polynucleotide or a vector as defined above. The host cell may be any cell that can be genetically modified and, preferably, cultivated. The cell can be eukaryotic or prokaryotic, such as a mammalian cell, an insect cell, a plant cell, a yeast, a fungus, a bacterial cell, etc. Typical examples include mammalian primary or established cells (3T3, CHO, Vero, Hela, etc.), as well as yeast cells (e.g., Saccharomyces species, Kluyveromyces, etc.) and bacteria (e.g., E. Coli). It should be understood that the invention is not limited with respect to any particular cell type, and can be applied to all kinds of cells, following common general knowledge.

The present invention allows the performance of detection or diagnostic assays that can be used, e.g., to detect the presence, absence, predisposition, risk or severity of a disease from a sample derived from a subject. In a particular embodiment, the disease is a cancer. The term "diagnostics" shall be construed as including methods of pharmacogenomics, prognostic, and so forth.

In a particular aspect, the invention relates to a method of detecting *in vitro* or *ex vivo* the presence, absence, predisposition, risk or severity of a disease in a subject, preferably a human subject, comprising placing a sample from the subject in contact with a polypeptide as defined above and determining the formation of an immune complex. Most preferably, the polypeptide is immobilized on a support. In a preferred embodiment, the method comprises contacting the sample with a device as disclosed above and determining the formation of immune complexes. Preferably, the polypeptide comprises a PSP sequence as contained in any one of SEQ ID NOs: 1-1596, or an epitope-containing fragment thereof.

In another aspect, the invention relates to a method of detecting *in vitro* or *ex vivo* the presence, absence, predisposition, risk or severity of a disease in a subject, preferably a human subject, comprising placing a sample from the subject in contact with an antibody that binds a polypeptide as defined above, and determining the formation of an immune complex. The antibody may be immobilized on a support. In a preferred embodiment, the method comprises contacting the sample with a device as disclosed above and determining the formation of immune complexes. In another preferred embodiment, the antibody is specific for a polypeptide comprising a PSP sequence as contained in any one of SEQ ID NOs: 1-1596.

In another aspect, the invention relates to a method of detecting *in vitro* or *ex vivo* the presence, absence, predisposition, risk or severity of a disease in a subject, preferably a human subject, comprising detecting a polypeptide as defined above by mass spectrometry, most preferably tandem mass spectrometry. In a preferred embodiment the method comprises creating spectra or fragmentation patterns specific of a polypeptide as defined above and running software programs or algorithms to search or compare such spectra or fragmentation patterns with the output of MS/MS experiments. In another preferred embodiment, the spectra or fragmentation patterns are specifically created for a polypeptide comprising a PSP sequence as contained in any one of SEQ ID NOs: 1-1596.

A particular object of this invention resides in a method of detecting the presence, absence, predisposition, risk or severity of cancers in a subject, the method comprising placing *in vitro* or *ex vivo* a sample from the subject in contact with a polypeptide as defined above and determining the formation of an immune complex. More preferably, the polypeptide is immobilized on a support and comprises a PSP sequence as contained in any one of SEQ ID NOs: 1-1596.

Another object of this invention resides in a method of detecting the presence, absence, predisposition, risk or severity of cancers in a subject, the method comprising placing a sample from the subject in contact with an antibody that binds a polypeptide as defined above, and determining the formation of an immune complex. The antibody may be immobilized on a support. In a preferred embodiment, the antibody is specific for a polypeptide comprising a PSP sequence as contained in any one of SEQ ID NOs: 1-1596.

Another object of this invention resides in a method of detecting the presence, absence, predisposition, risk or severity of cancers in a subject, the method comprising detecting a polypeptide as defined above in a sample from the subject by mass spectrometry, most preferably tandem mass spectrometry. In a preferred embodiment the method comprises creating spectra or fragmentation patterns specific of a polypeptide as defined above and running software programs or algorithms to search or compare such spectra or fragmentation patterns with the output of MS/MS experiments. In another preferred embodiment, the spectra or fragmentation patterns are specifically created for a polypeptide comprising a PSP sequence as contained in any one of SEQ ID NOs: 1-1596.

Another object of the invention relates to a method of detecting *in vitro* or *ex vivo* the presence, absence, predisposition, risk or severity of a disease in a biological sample, preferably, a human biological sample, comprising placing said sample in contact with a polypeptide as defined above and determining the presence of immune cells expressing a TCR specific for such a polypeptide. Preferably, the polypeptide comprises a PSP sequence as contained in any one of SEQ ID NOs: 1-1596.

A further aspect of this invention resides in a method of assessing *in vitro* or *ex vivo* the level of transcription infidelity in a subject, preferably, a human subject, comprising placing a sample from the subject in contact with a polypeptide as defined above and determining the formation of an immune complex. Most preferably, the polypeptide is immobilized on a support. In a preferred embodiment, the method comprises contacting the sample with a device as disclosed above and determining the formation of immune complexes.

A further aspect of this invention resides in a method of assessing *in vitro* or *ex vivo* the level of transcription infidelity in a subject, preferably, a human subject, comprising placing a sample from the subject in contact with an antibody that binds a polypeptide as defined above, and determining the formation of an immune complex. The antibody may be immobilized on a support. In a preferred embodiment, the antibody is specific for a polypeptide comprising a PSP sequence as contained in any one of SEQ ID NOs: 1-1596.

A further aspect of this invention resides in a method of assessing *in vitro* or *ex vivo* the level of transcription infidelity in a subject, preferably, a human subject, comprising placing a sample from the subject in contact with a polypeptide as defined above and determining the presence of immune cells expressing a TCR specific for such a polypeptide.

Another embodiment of this invention is directed to a method of determining the efficacy of a treatment of a cancer, the method comprising (i) determining the level of at least one polypeptide as defined above, in a sample from the subject and (ii) comparing said level to the level in a sample from said subject taken prior to or at an earlier stage of the treatment. Preferably, the polypeptide(s) comprise(s) a PSP sequence as contained in any one of SEQ ID NOs: 1-1596.

Another embodiment of this invention is directed to a method of determining the efficacy of a treatment of a cancer, the method comprising detecting a polypeptide as defined above in a sample from the subject by mass spectrometry, most preferably tandem mass spectrometry. In a preferred embodiment the method comprises creating spectra or fragmentation patterns specific of a polypeptide as defined above and running software programs or algorithms to search or compare such spectra or fragmentation patterns with the output of MS/MS experiments. In another preferred embodiment, the spectra or fragmentation patterns are specifically created for a polypeptide comprising a PSP sequence as contained in any one of SEQ ID NOs: 1-1596.

A further aspect of this invention is directed to a method of determining whether an individual is making a polypeptide comprising a PSP sequence as contained in any one of SEQ ID NOs: 1-1596, said method comprising contacting a sample obtained from said individual with an agent indicative of the presence of said polypeptide and determining whether said agent binds to said sample. In a first embodiment of said method, the sample obtained from the subject is placed in contact with a polypeptide which binds to an antibody specific for said polypeptide. In another embodiment, the sample obtained is placed in contact with a polypeptide which binds an immune cell comprising a TCR specific for said polypeptide. According to another embodiment, the sample is placed in contact with an antibody or portion thereof which is specific for said polypeptide.

The detection or diagnostic methods of the present invention can be performed *in vitro, ex vivo* or *in vivo,* preferably *in vitro* or *ex vivo.* The sample may be any biological sample derived from a subject, which contains polypeptides, antibodies or immune cells, as appropriate. Examples of such samples include body fluids, tissues, cell samples, organs, biopsies, etc. Most preferred samples are blood, plasma, serum, saliva, seminal fluid, and the like. The sample may be treated prior to performing the method, in order to render or improve availability of antibodies for testing. Treatments may include, for instance one or more of the following: cell lysis (e.g., mechanical, physical, chemical, etc.), centrifugation, extraction, column chromatography, and the like.

Determination of the presence, absence, or relative abundance of a protein, antibody or specific immune cell in a sample can be performed by a variety of techniques known per se in the art. Such techniques include, without limitation, methods for detecting an immune complex such as, without limitation, ELISA, radio-immunoassays (RIA), fluoro-immunoassays, microarray, microchip, dot-blot, western blot, EIA, IEMA, IRMA or IFMA (see also Immunoassays, a practical approach, Edited by JP Gosling, Oxford University Press). In a particular embodiment, the method comprises contacting the sample and polypeptide(s) under conditions allowing formation of an immune complex and revealing said formation using a second labelled reagent.

In a typical embodiment, the method comprises comparing the measured level to a reference level, wherein a difference is indicative of a dysfunction in the subject. More particularly, an increase in the level as compared to the reference value is indicative of the presence of a cancer. An increase is typically a 10%, 20%, 30%, 40%, 50% or more increase as compared to the reference value. The reference value may be a mean or median value determined from individuals not having a cancer or disease, a reference level obtained from a control patient, a reference level obtained from the subject before cancer onset or with a control polypeptide. In a preferred embodiment, an increase in the level of polypeptides, antibodies or immune cells in said sample as compared to the reference level is indicative of the presence, risk or stage of development of a cancer.

Contacting may be performed in any suitable device, such as a plate, microtitration dish, test tube, wells, glass, column, and so forth. In specific embodiments, the contacting is performed on a substrate coated with the polypeptide. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable antibody-antigen complex to be formed between the polypeptide and antibodies of the sample.

In a specific embodiment, the method comprises contacting a sample from the subject with (a support coated with) a plurality of polypeptides as described above, and determining the presence of immune complexes. In a particular embodiment, the method comprises contacting the sample with a plurality of sets of beads, each set of beads being coated with a distinct polypeptide as defined above. In an other particular embodiment, the method comprises contacting the sample with a slide or membrane on which several polypeptides as defined above are arrayed. In an other particular embodiment, the method comprises contacting the sample with a multi-wells titration plate, wherein at least part of the wells are coated with distinct polypeptides as defined above.

The invention may be used for determining the presence, risk or stage of any cancer in a subject. This includes solid tumors, such as, without limitation, colon, lung, breast, ovarian, uterus, liver, or head and neck cancers, melanoma, and brain tumors, as well as liquid tumors, such as e.g., leukemia. The invention may also be used to detect other physiological disorders such as ageing, immune disorders, proliferative disorders.

The invention also allows the design (or screening) of novel drugs by assessing the ability of a candidate molecule to modulate expression of a polypeptide of this invention.

A particular object of this invention resides in a method of selecting, characterizing, screening or optimizing a biologically active compound, said method comprising determining whether a test compound modulates expression of a polypeptide of this invention.

Expression may be assessed at the gene, RNA or protein levels. For instance, expression may be assessed using a nucleic acid primer or probe as defined above, to detect any alteration in the transcription level. Expression may also be assessed using e.g., and antibody or any other specific ligand, to measure alteration in the translation level. The above screening assays may be performed in any suitable device, such as plates, tubes, dishes, flasks, etc. Typically, the assay is performed in multi-well microtiter dishes. Using the present invention, several test compounds can be assayed in parallel. Furthermore, the test compound may be of various origin, nature and composition. It may be any organic or inorganic substance, such as a lipid, peptide, polypeptide, nucleic acid, small molecule, in isolated or in mixture with other substances. The compounds may be all or part of a combinatorial library of compounds, for instance.

Further aspects and advantages of this invention will be disclosed in the following examples, which shall be considered as illustrative and not limiting the scope of protection.

### EXAMPLES

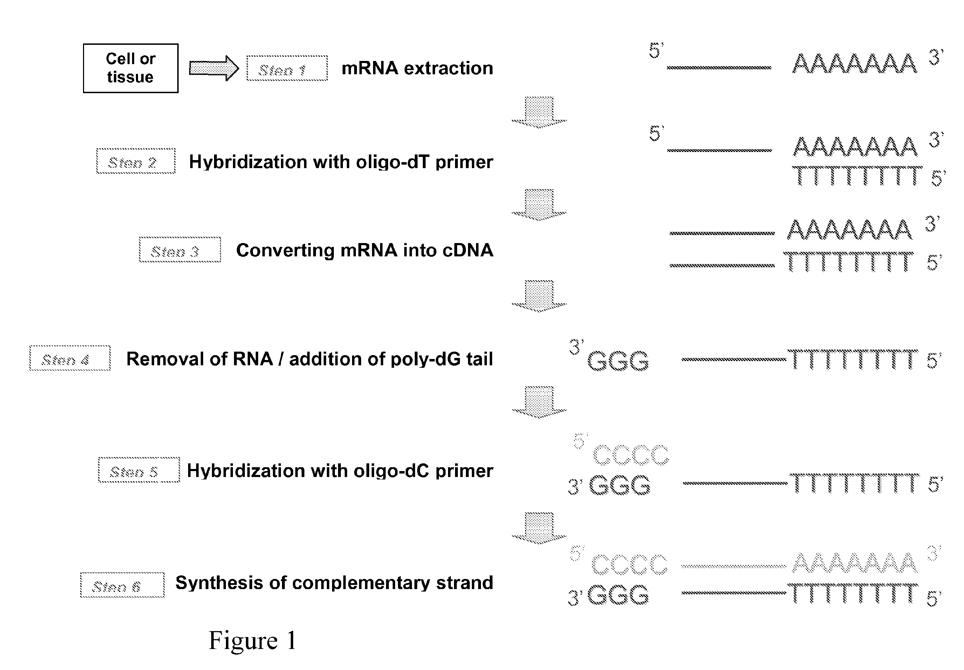

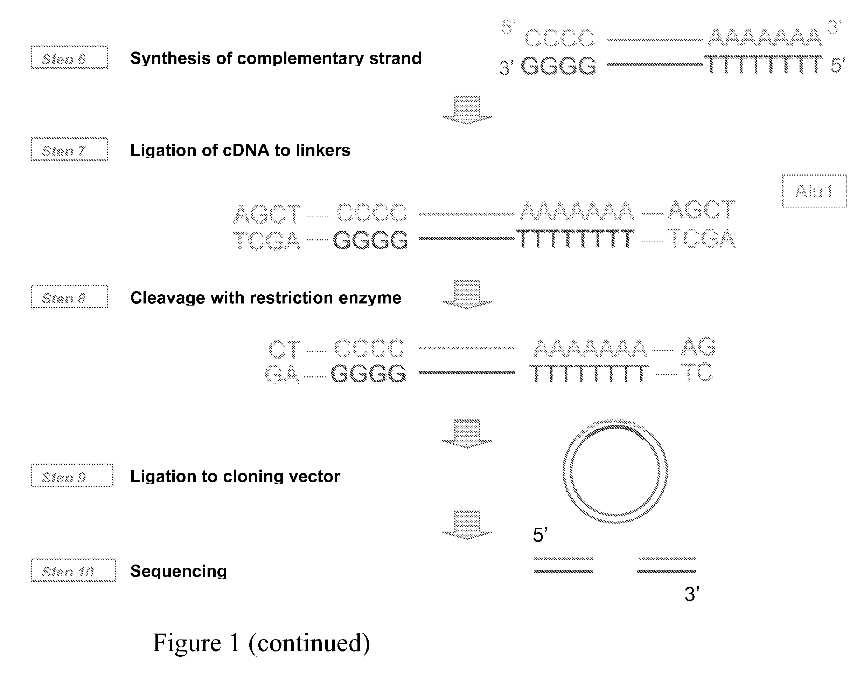

### Example 1: Principle of typical cDNA library construction and sequencing (see Fig 1)

The first step in preparing a complementary DNA (cDNA) library is to isolate the mature mRNA from the cell or tissue type of interest. Because of their poly(A) tail, it is straightforward to obtain a mixture of all cell mRNA by hybridization with complementary oligo dT linked covalently to a matrix. The bound mRNA is then eluted with a low salt buffer. The poly(A) tail of mRNA is then allowed to hybridize with oligo dT in the presence of a reverse transcriptase, an enzyme that synthesizes a complementary DNA strand from the mRNA template. This yields double strand nucleotides containing the original mRNA template and its complementary DNA sequence. Single strand DNA is next obtained by removing the RNA strand by alkali treatment or by the action of RNase H. A series of dG is then added to the 3' end of single strand DNA by the action of an enzyme called terminal transferase, a DNA polymerase that does not require a template but adds deoxyoligonucleotide to the free 3' end of each cDNA strand. The oligo dG is allowed to hybridize with oligo dC, which acts as a primer to synthesize, by the DNA polymerase, a DNA strand complementary to the original cDNA strand. These reactions produce a complete double strand DNA molecule corresponding to the mRNA molecules found in the original preparation. Each of these double strand DNA molecules are commonly referred to as cDNA, each containing an oligo dC - oligo dG double strand on one end and an oligo dT - oligo dA double strand region on the other end. This DNA is then protected by methylation at restriction sites. Short restriction linkers are then ligated to both ends. These are double strand synthetic DNA segments that contain the recognition site for a particular restriction enzyme. The ligation is carried out by DNA ligase from bacteriophage T4 which can join "blunt ended" double strand DNA molecules. The resulting double strand blunt ended DNA with a restriction site at each extremity is then treated with restriction enzyme that creates a sticky end. The final step in construction of cDNA libraries is ligation of the restriction cleaved double strand with a specific plasmid that is transfected into a bacterium. Recombinant bacteria are then grown to produce a library of plasmids - in the presence of antibiotics corresponding to the specific antibiotic resistance of the plasmid. Each clone carries a cDNA derived from a single part of mRNA. Each of these clones is then isolated and sequenced using classical sequencing methods. A typical run of sequencing starts at the insertion site and yields 400 to 800 base pair sequences for each clone. This sequence serves as a template to start the second run of sequencing. This forward progression leads to progressive sequencing of the entire plasmid insert. The results of sequencing of numerous cDNA designated ESTs have been deposited in several public databases.

### Example 2: Database annotation

EST databases contain sequence information that correspond to the cDNA sequence obtained from cDNA libraries and therefore correspond essentially to the sequence of individual mRNA present at any given time in the tissue that was used to produce these libraries. The quality of these sequences has been called into question for several reasons. First, as discussed above, the process of producing cDNA libraries initially relied heavily on the presence of a poly(A) tail at the 3' end of eukaryotic mRNA. Second, mRNA are quite fragile molecules that are easily digested by high abundance nucleases called RNases.

Third, while building and sequencing these libraries, little attention was paid to the quality of the original material used and its storage. Because of this, EST sequences have been used to annotate genomic information i.e., to determine whether an identified and fully sequenced segment of genomic DNA encodes any specific mRNA. In this context, EST sequences were useful in order to identify coding genomic sequence. However, little attention has been paid to the information borne by the EST sequence itself. Indeed, DNA genomic sequence is considered as much more reliable with strong technical arguments in support of this position. We speculate that diversity included in EST sequences might contain biologically, analytically or clinically relevant information. Indeed, EST databases were produced by a number of investigators that all used various methods: this led us to speculate that each methodological bias must contribute to a background noise level with a certain number of errors. However, if differences in errors were to exist due to the source of material used to generate the library, then the difference in error rate would be directly related to the underlying source.

### Example 3: Genome-wide identification of sequence variations between ESTs from normal and cancer origins occurring within the stop codon

In order to test our hypothesis, we retrieved human EST databases available at the NCBI ftp site. We selected these databases because these sequences were not annotated or cured by human or bioinformatic tools.
We used a library identification system in order to determine whether an EST was obtained from a cancerous tissue or a normal one since each library has been labeled "normal" or "cancer". By matching the accession number of each EST with the identifier of the corresponding library, we classified 3 millions ESTs as those obtained from cancerous tissues and 3.9 millions ESTs as those obtained from normal tissues. We built two sets of sequences that we named cancer and normal sets respectively (i.e. set of ESTs extracting from cancerous tissue and normal tissue respectively).

We then retrieved all human RNA RefSeq sequences from NCBI, i.e. 38746 RNA sequences:
- transcripts products; mature messenger RNA (mRNA) transcripts. N = 24704,
- non-coding transcripts including structural RNAs, transcribed pseudogenes and others. N = 898,
- transcript products; model mRNA provided by a genome annotation process; sequence corresponds to the genomic contig. N = 8721,
- transcript products; model non-coding transcripts provided by a genome annotation process; sequence corresponds to the genomic contig. N = 4423.
We retrieved all RefSeq sequences in order to be representative of the human transcriptome.

We then aligned each normal and each cancer EST to all RNA RefSeq. We used publicly available megaBLAST 2.2.16 software (Basic Local Alignment Search Tool, ⁸) and selected default parameters except :
- b 1 : maximal number of sequences for which the alignment is reported,
- p 90 : minimal percent identity between EST and the reference sequence,
- W 16 : length of best perfect match to start with alignment extension.

For each EST, we retained only the best alignment. Therefore, each EST could match with no more than one RefSeq. We built two sets of alignment outputs, the first one corresponding to normal ESTs and the second one to cancer ESTs.
We then split alignments according to the corresponding RefSeq and obtained, for each RefSeq, a set of alignments with cancer ESTs and a set of alignments with normal ESTs. Out of 38746 transcripts, we found at least one alignment with cancer EST and one alignment with normal EST for 34184 transcripts.
We retained alignments for which EST aligned once and on more than 70% of its length. We also cut the 10 first and last elements of each alignment.

This created a matrix associated with each RefSeq in which any given base is defined by the number of cancer and normal ESTs matching to this position. We then measured the proportion of ESTs deviating from RefSeq at any given position, i.e., the number of base substitutions at any position. We focused on the three positions corresponding to stop codon.

The next step of this analysis was to test the statistical significance of the differences in sequence substitutions occurring between cancer and normal ESTs. For each position, we compared the proportion of the RefSeq base to that of the three other bases between normal and cancer groups using proportion test. This test was systematically applied provided that the following conditions were met: n >70 and (n_{i.} * n_{.j})/n >5 i=1,2 ; j=1,2 (where n = the number of cancer and normal ESTs for a position, n_{1.} = the number of cancer ESTs, n_{2.} = the number of normal ESTs, n_{.1} = the number of ESTs having the RefSeq, n_{.2} = the number of ESTs having a variation). A statistical test is said to be positive at the threshold level of 5% whenever corresponding P-value is lower than 0.05; in this case, the null hypothesis is rejected.
The two following one-sided proportion tests were considered in order to precise in which set the variability was bigger. The first one allowed to conclude that variabilities were different in both groups when statistical test is positive, then it measured in this case whether variability was statistically greater in the cancer set. On the contrary the second test verified the hypothesis that variability was significantly higher in the normal set.
An estimated error resulting from multiple testing, defined by the Location Based Estimator ⁹ was also calculated.

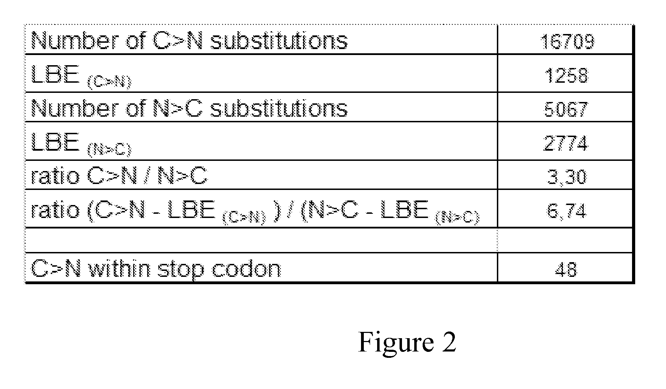

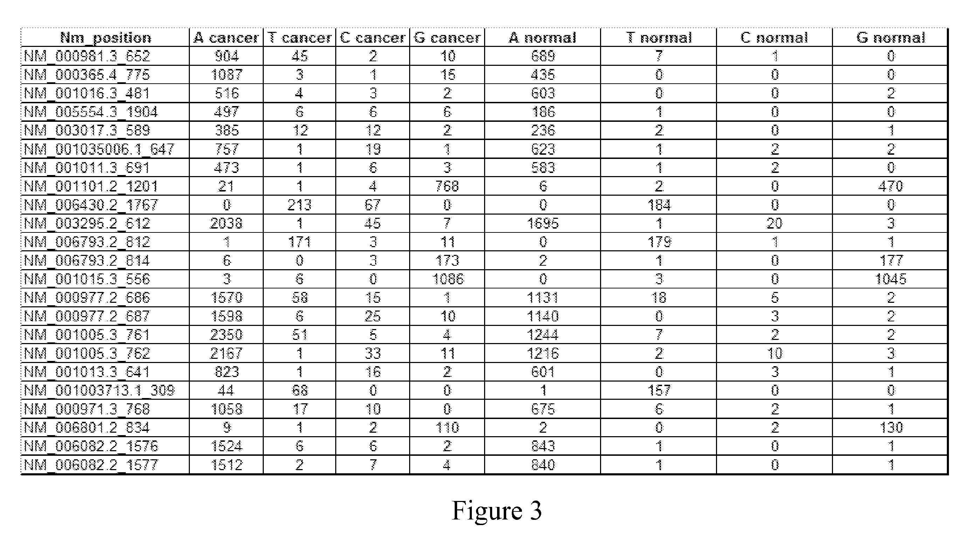

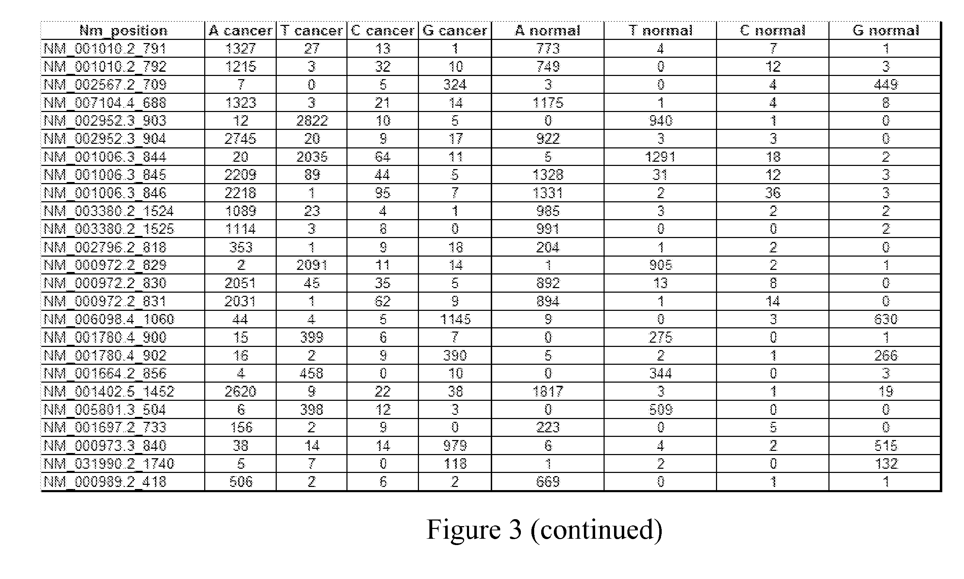

Results of statistical analysis are shown in figure 2. Positions with statistically significant sequence substitutions are referred to as C>N if the variation is in excess in cancer and conversely N>C when in excess in normal. We obtained 48 C>N positions occurring within a stop codon (Figure 3). We also identified 36 transcripts where the stop codon is significantly more substituted in cancer ESTs than in normal ones.
We therefore predict that the natural stop codon will be modified into an amino acid. This opens a new reading frame between the natural stop and the first alternative stop codon in frame. Out of the 36 identified transcripts, 4 have no alternate stop codon. For the 32 other transcripts, we determined the amino acid sequence which is translated when the canonical stop codon is substituted with an amino acid. This newly defined sequence is named "post-stop peptide" and corresponds to the peptide read after the canonical stop until the first alternate stop. 25 post-stop peptides, longer than 3 amino acids, are depicted in SEQ ID: 1 to 25.

### Example 4: Identification of additional post-stop peptides

Significant base substitutions affecting the natural stop codon were observed in 36 transcripts. We can note that, in the genetic code, the UGA codon has a dual function as it can encode selenocysteine (Sec) and serve as a stop signal for proteins called selenoproteins. Nevertheless, only 25 human selenoprotein are described ¹⁰, none of them belong to the set of 36 transcripts and UGA is the fewest represented stop codon within our 36 transcripts. Therefore, before the concept of transcription infidelity, it had not been proposed that usual human proteins would contain additional coding sequences encoded by RNA sequences considered thus far as "untranslated regions". We now show that base substitution occurring in natural stop codons because of transcription infidelity reveals novel coding regions that encode specific AA. This novel coding region is in phase with the native open reading frame. The natural stop codon is transformed into a coding codon. The next triplet of base is then read as an AA and the translation proceeds with a novel coding region until a new stop codon is reached. The addition of these AA has the potential to create motifs that will be greatly enhanced in cancer; these motifs will or will not result in novel function of the proteins. Predicting this occurrence leads to development of useful tools that could be use in diagnostic, therapeutic or other goals. Predicting this occurrence leads also to development of specific antibodies that will recognize cancer specific sequences in the carboxy-terminal end of the protein. No analytical method is currently capable of direct protein sequencing at the carboxy-terminal end. It is, however, possible to cleave proteins enzymatically and sequence cleavage products from their NH₂ terminal end. It is also possible to analyze the AA content of peptides generated by proteolysis using mass spectrometry. The same phenomenon described above can further expand the reading to a novel set of sequences. Annotation of all protein sequences using our method will reveal several unsuspected coding mRNA sequences resulting from base substitution in the natural stop codon. On the basis of the occurrence of stop codon alterations, we estimate in affected genes that 2 to 10% of mRNA in cancer tissues contain these additional coding regions.

### Example 5: Identification of putative post-stop peptides for proteins of interest

A specific program based on several filters can be used to annotate all protein sequences for the presence of a putative Post STOP Peptide (PSP). After retrieving nucleic sequence corresponding to the studied proteins, the program searches the presence or not of an in phase nucleic sequence after the canonical STOP, with another STOP in phase (the possibility to bypass one or more STOP in case of transcription infidelity affecting these alternative STOP codons can be taken into account). A minimal length can be fixed (e.g. only sequences coding more than 3 amino acids). PSPs are then stored. This program is applied to two sets of proteins sequences. The first set is constituted of 1784 tumor markers ¹¹ (updated database, may 2007). The second set is constituted of 1175 sequences of plasma proteins ¹². From the first set the program identifies 1109 putative PSPs corresponding to 1109 different RefSeq protein identifiers (>=3AA). From the second set the program identifies 913 putative PSPs (>=3AA). For these 1109 and 913 post stop sequences, we built predicted sequences as being the sequence of native protein - X - post stop peptide, which is called predicted aberrant protein PAP. X represents any amino acid. In fact, a stop codon can be substituted on the three positions of the codon (example : RPS3A, i.e. NM_001006.3 figure 3). Thus, we have to consider that a mRNA can be substituted on 2 or 3 positions of the stop codon ; stop codon can therefore be any of the 20 AA. We found 831 putative PSPs identified solely in the first set (the identification is based on the accession number of the protein NP). There are identical PAPs corresponding to different transcripts. Removal of these doublets leads to 725 different PAPs that are specific from the first set. The 725 PAPs are listed as SEQ ID Nos : 26 to 750. We then found 635 putative PSPs identified solely in the second set (the identification is also based on the accession number of the protein NP). Removal of the doublets leads to 594 different PAPs that are specific from the second set. The 594 PAPs are listed as SEQ ID Nos : 751 to 1344. We also found 278 putative PSPs identified in the first and the second set. Removal of the doublets leads to 252 different PAPs. The 252 PAPs are listed as SEQ ID Nos : 1345 to 1596.

### Example 6: Refining the selection of putative post-stop peptides

We focus on novel proteins induced when the natural stop codon is affected. That leads to distinct specific populations of proteins with a novel sequence in the carboxy-terminal end. We estimate that cancerous tissues for affected genes contain 2 to 10% proteins that are longer than normal ones.
In view of this hypothesis, we select possible PSPs. The intial selection is based on a list of plasma proteins or tumor markers. We then apply additional criteria to refine this selection. For example certain post-stop peptide sequences are predicted to be immunogenic, and antibodies directed against these novel sequences represent specific ligands to measure transcription infidelity. Similarly, Kyte-Doolittle analysis can indicate that certain post-stop sequences are not hydrophobic, and therefore the corresponding novel proteins are expected to be secreted into the circulation.

### Example 7: Identification of post-stop peptide (PSP) of a selected Protein X in a biological sample

Putative PSPs that result from base substitutions in the canonical stop codon are identified and characterized in a biological sample in the following manner. Rabbit polyclonal antibodies are prepared that recognize an immunogenic portion of the PSP in question. These anti-peptide antibodies are checked by dot blot using the purified peptide to verify that they indeed recognize the PSP. Western blots are then performed on samples obtained from cancer patients using the antibodies directed against the PSP. The anti-PSP Protein X antibody recognizes a band in Western blots performed on samples obtained from cancer patients, that is not observed when using rabbit pre-immune serum as a negative control. The PSP Protein X band has a slightly higher molecular mass as compared to that of the native monomer form of Protein X. This molecular mass corresponds to that predicted based on the additional peptide sequence. Two-dimensional gels can also be performed in order to further characterize this band.

Affinity chromatography experiments is carried out to isolate the PSP form of Protein X using the anti-PSP antibody. The anti-PSP antibody is immobilized on matrix beads and the following column is incubated in presence of sample then sequentially washed to remove aspecifically bound proteins and finally eluted with detergent or chaotropic reagents. The eluted fraction is analysed by Western blotting using both the anti-PSP and anti-Protein X antibodies. Two bands are recognized by the anti-Protein X antibody whereas only one band is recognized by the anti-PSP antibody. Therefore, the smaller molecular mass band corresponds to the native Protein X form and the larger molecular mass band corresponds to the PSP form of Protein X.

Protein X can be isolated by various methods, including sequential ultracentrifugation, gel filtration and preparative electrophoresis. The PSP form of Protein X is tracked by Western blotting. The purified PSP form of Protein X is then cleaved enzymatically (trypsin) and the resulting peptides are analyzed on MS-MS for full AA sequencing. Results show that canonical STOP is replaced preferentially by a specific amino acid sequence. This is the exact sequence of amino acid predicted to occur following bypass of Protein X canonical STOP.

### Example 8 : Large scale identification ofpost-stop peptides by tandem mass spectrometry

The large-scale identification of post-stop peptides can be conducted in any biological sample, including sera and various tissues.

The currency of information for tandem mass spectrometry (MS/MS) is the fragment ion spectrum of a specific peptide ion that is fragmented, typically in the collision cell of a tandem mass spectrometer. The correct assignment of such a spectrum to a peptide sequence can be done with a large number of computational approaches and software tools that have been developed to automatically assign peptide sequences to fragment ion spectra. However post-stop peptides are typically absent from existing spectra and sequences databases. Three approaches are therefore possible for the assignment of fragment ion spectra to post-stop peptides sequences:
i) *De novo* sequencing, where peptide sequences are explicitly read out directly from fragment ion spectra;
ii) database searching, where post-stop peptide sequences are identified by correlating acquired fragment ion spectra with theoretical spectra predicted for each post-stop peptide, or with libraries of experimental MS/MS spectra identified in previous experiments;
iii) hybrid approaches, such as those based on the extraction of short sequence tags of 3-5 residues in length, followed by error-tolerant database searching.

For large scale proteomics studies database searching remains the most frequently used peptide identification method. Several MS/MS database search programs are available (Table 1). The programs take the fragment ion spectrum of a peptide as input and score it against theoretical fragmentation patterns constructed for peptides from the searched database. The pool of candidate post-stop peptides is restricted based on criteria such as mass tolerance and proteolytic enzyme constraint. The output from the program is a list of fragment ion spectra matched to post-stop peptide sequences, ranked according to the search score. The search score measures the degree of similarity between the experimental and the theoretical spectrum.
In another approach a spectral library is compiled meticulously from a large collection of observed mass spectra of correctly identified post-stop peptides. An unknown spectrum can then be identified by comparing it to all the candidates in the spectral library to determine the match with the highest spectral similarity. The spectral matching method substantially outperforms database searching in speed, error-rate and sensitivity. However post-stop peptide identification requires prior entry of the post-stop peptide spectrum into the spectral library. Synthetic post-stop peptides can be used to create *de novo* spectral libraries.
A combination of the above methods is applied with the programs and databases listed in Table 1 to build fragmentation pattern databases and spectral libraries of specific post-stop peptides of known amino-acid sequence. These databases and libraries are then used for the large scale identification of post-stop peptides in sera or other tissue samples. The above methods are used for the systematic analysis of post-stop peptides of SEQ ID Nos : 1 to 1596 in biologic samples.

**Table 1. Publicly available programs and databases for MS/MS-based post-stop peptide analysis**

| **Database search** | **Spectral matching** | ***De novo* sequencing** | **Sequence tag/hybrid approaches** | **Statistical validation of peptide identifications** | **Databases for storing and mining** |
|---|---|---|---|---|---|
| SEQUEST MASCOT ProteinProspector ProbID | SpectraST X! P3 Biblispec | Lutefisk Pepnovo PEAKS Sequit | GutenTag Inspect Popitam | PeptideProphet Scaffold | PeptideAtlas Proteios SBEAMS CPAS |
| TANDEM | | | | | PRIDE |
| SpectrumMill | | | | | |
| Phenyx | | | | | |
| OMSSA | | | | | |
| VEMS | | | | | |
| MyriMatch | | | | | |

### REFERENCES

1. Brulliard, M. et al. Nonrandom variations in human cancer ESTs indicate that mRNA heterogeneity increases during carcinogenesis. Proc Natl Acad Sci U S A 104, 7522-7 (2007).
2. Sjoblom, T. et al. The consensus coding sequences of human breast and colorectal cancers. Science 314, 268-74 (2006).
3. Sherry, S. T. et al. dbSNP: the NCBI database of genetic variation. Nucleic Acids Res 29, 308-11 (2001).
4. Gott, J. M. & Emeson, R. B. Functions and mechanisms of RNA editing. Annu Rev Genet 34, 499-531 (2000).
5. Armache, K. J., Kettenberger, H. & Cramer, P. The dynamic machinery of mRNA elongation. Curr Opin Struct Biol 15, 197-203 (2005).
6. Pomerantz, R. T., Temiakov, D., Anikin, M., Vassylyev, D. G. & McAllister, W. T. A mechanism of nucleotide misincorporation during transcription due to template-strand misalignment. Mol Ce// 24, 245-55 (2006).
7. Kashkina, E. et al. Template misalignment in multisubunit RNA polymerases and transcription fidelity. Mol Ce// 24, 257-66 (2006).
8. Zhang, Z., Schwartz, S., Wagner, L. & Miller, W. A greedy algorithm for aligning DNA sequences. J Comput Biol 7, 203-14 (2000).
9. Dalmasso, C., Broet, P. Procedures d'estimation du false discovery rate basées sur la distribution des degrés de signification. Journal de la Société Française de Statistiques 146 (2005).
10. Kryukov, G. V. et al. Characterization of mammalian selenoproteomes. Science 300, 1439-43 (2003).
11. Polanski, M., Anderson, N. L. A list of candidate cancer biomarkers for targeted proteomics. Biomarker Insights, 1-48 (2006).
12. Anderson, N. L. et al. The human plasma proteome: a nonredundant list developed by combination of four separate sources. Mol Cell Proteomics 3, 311-26 (2004).

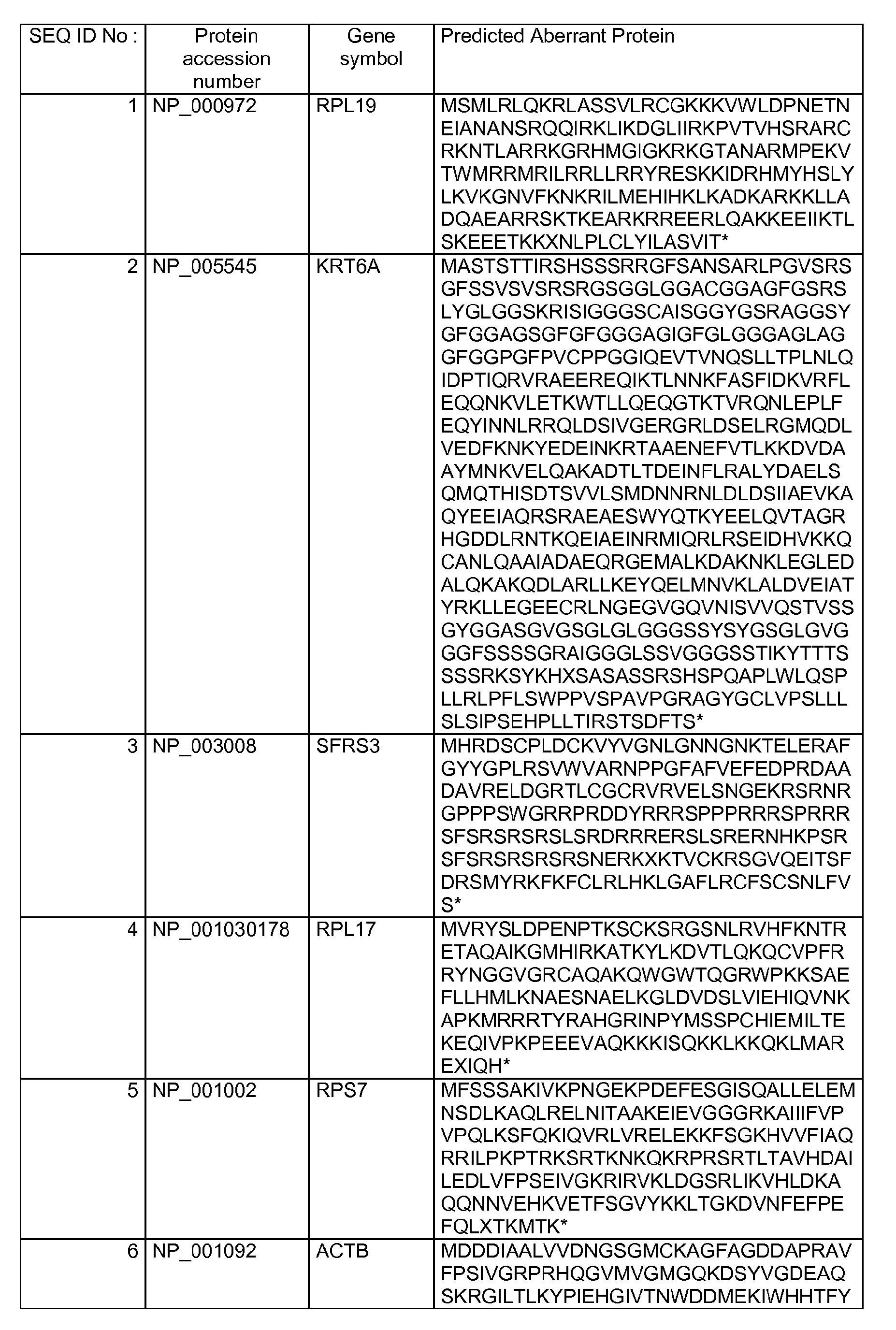

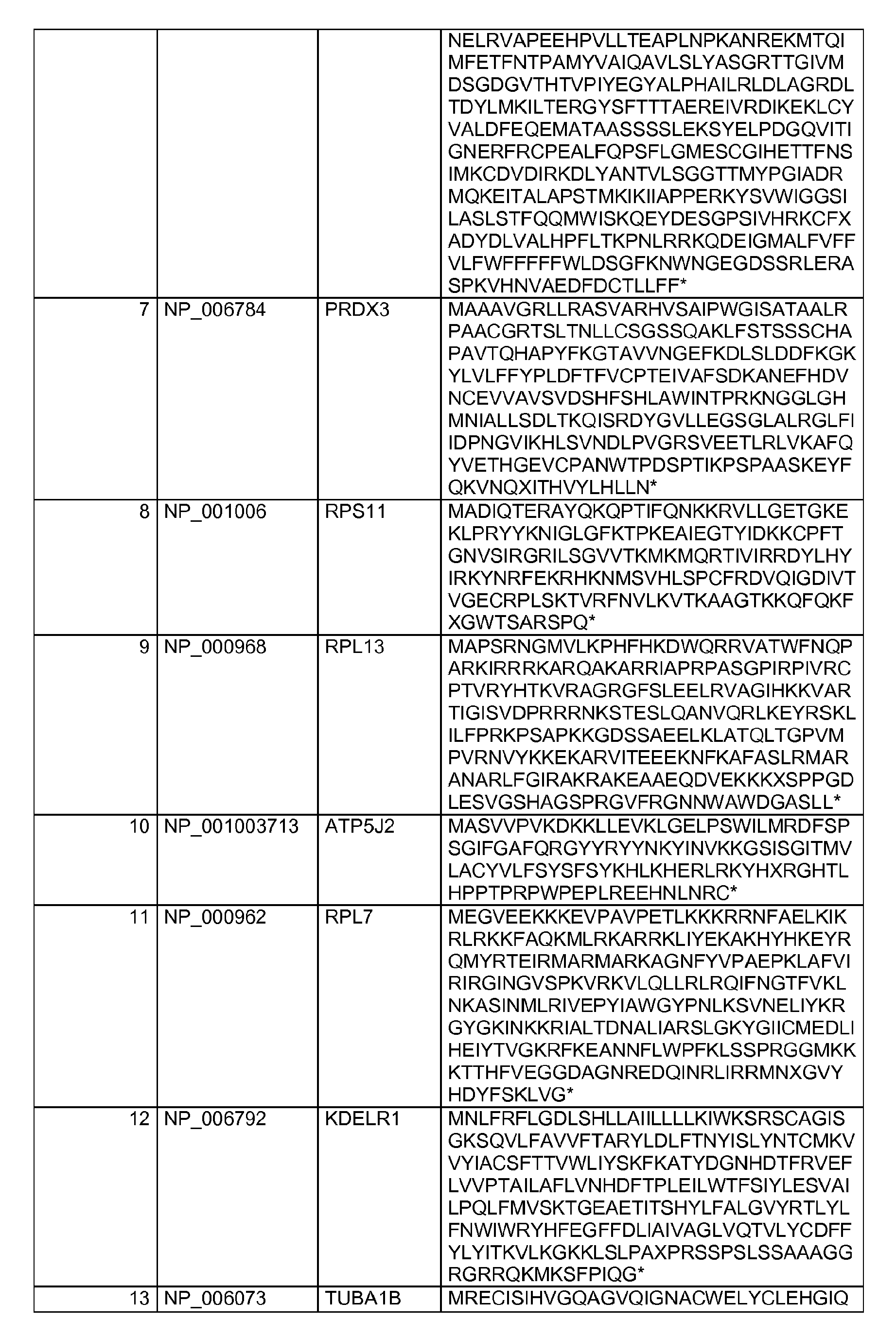

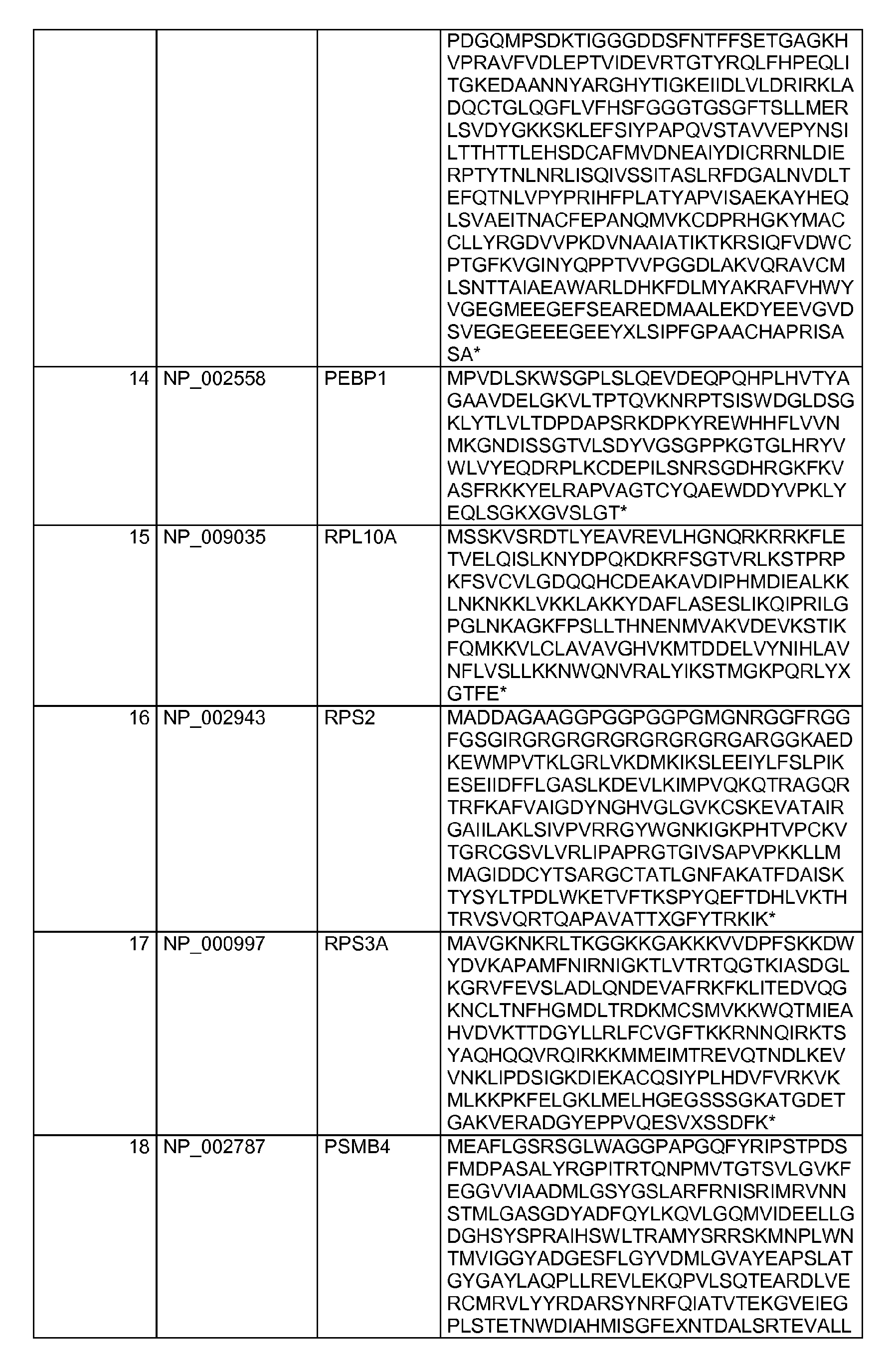

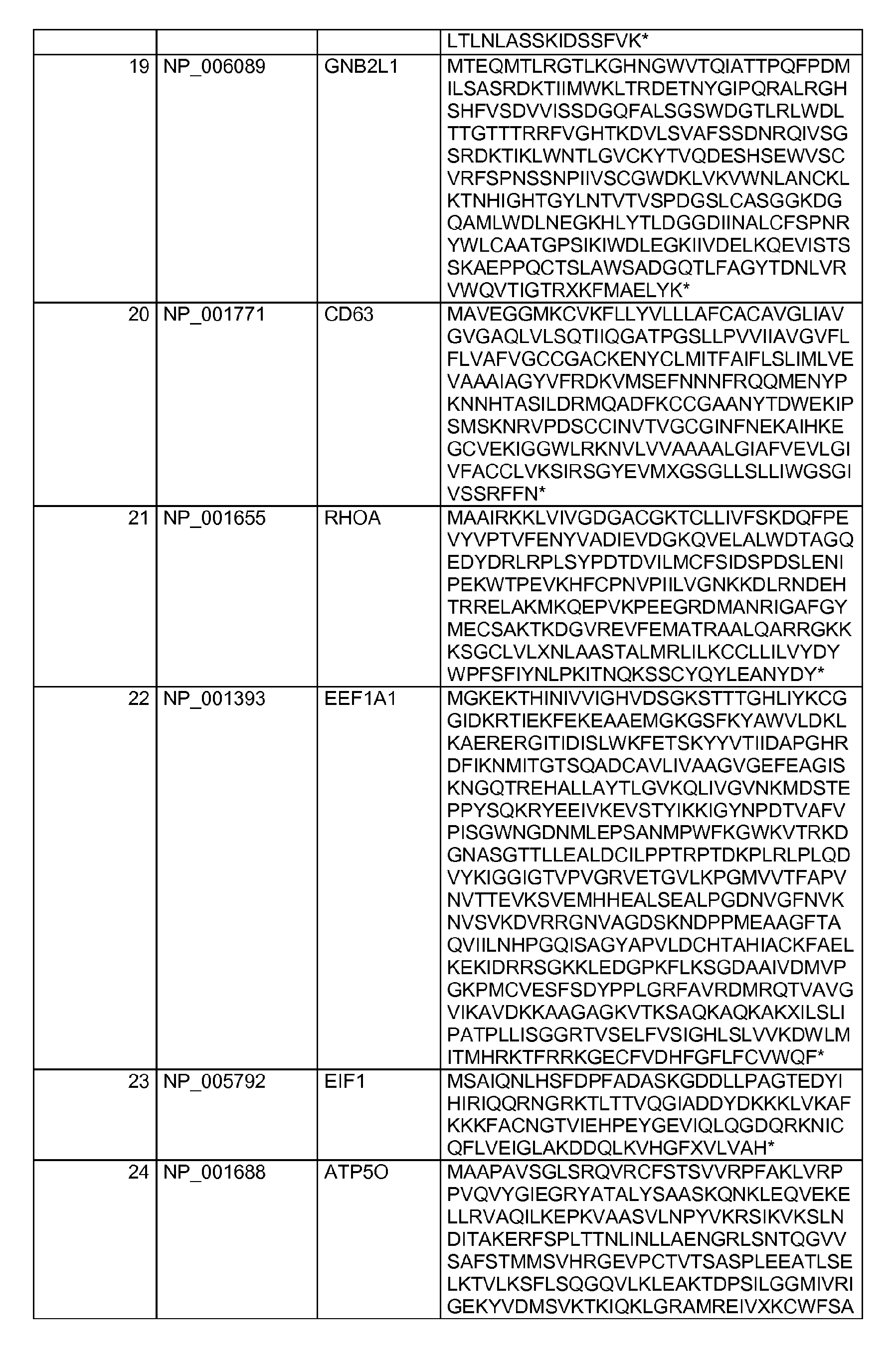

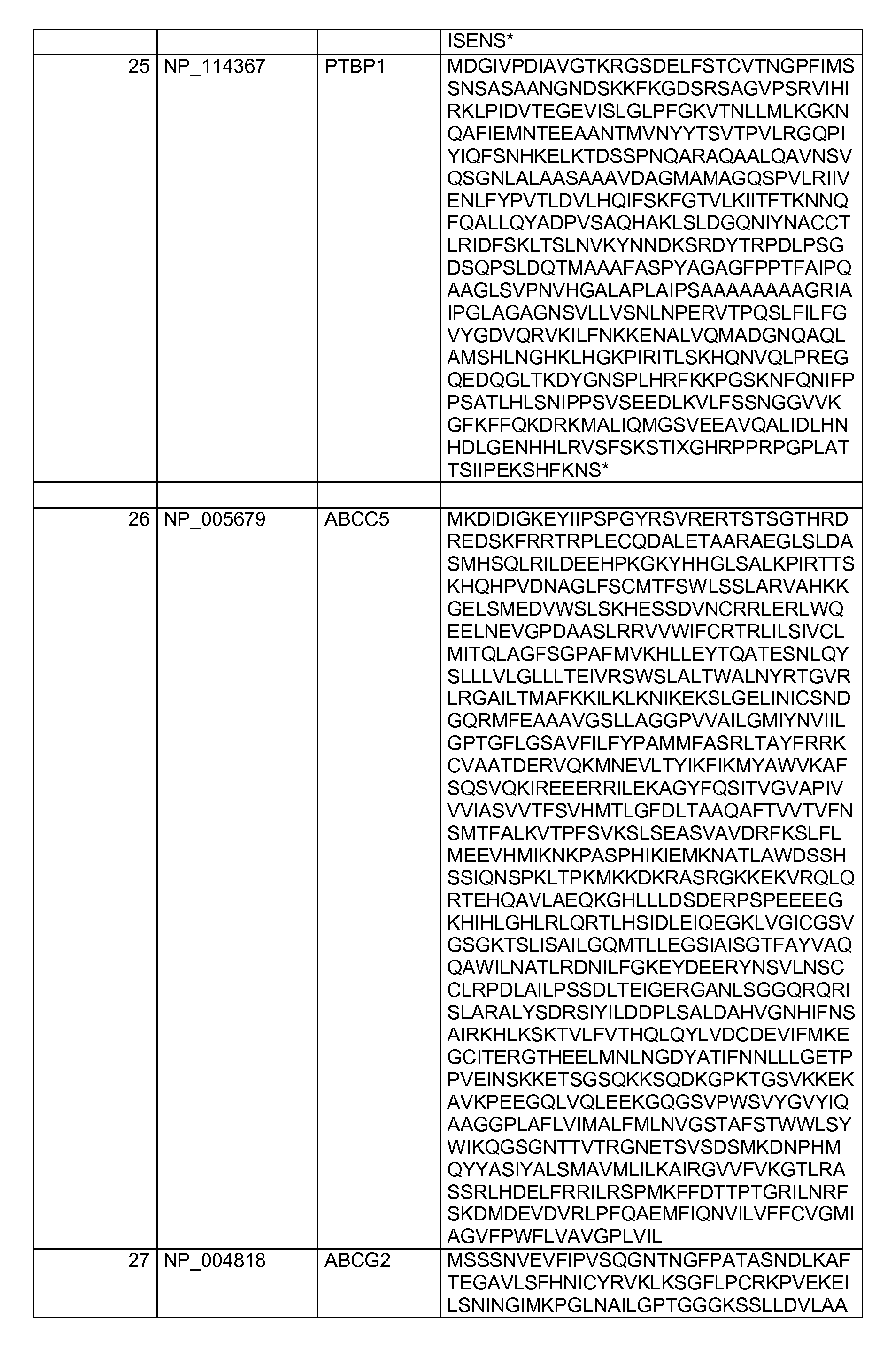

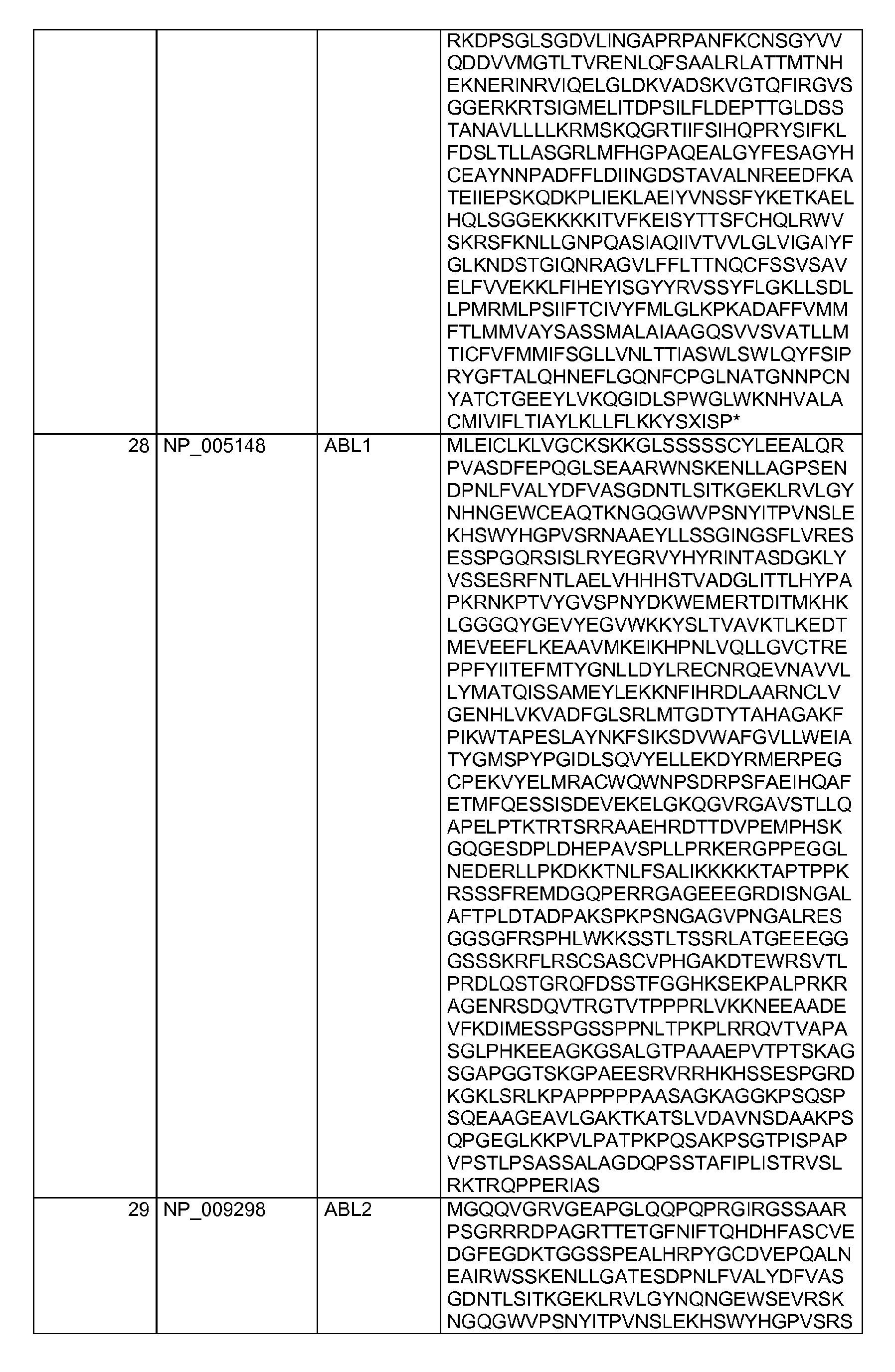

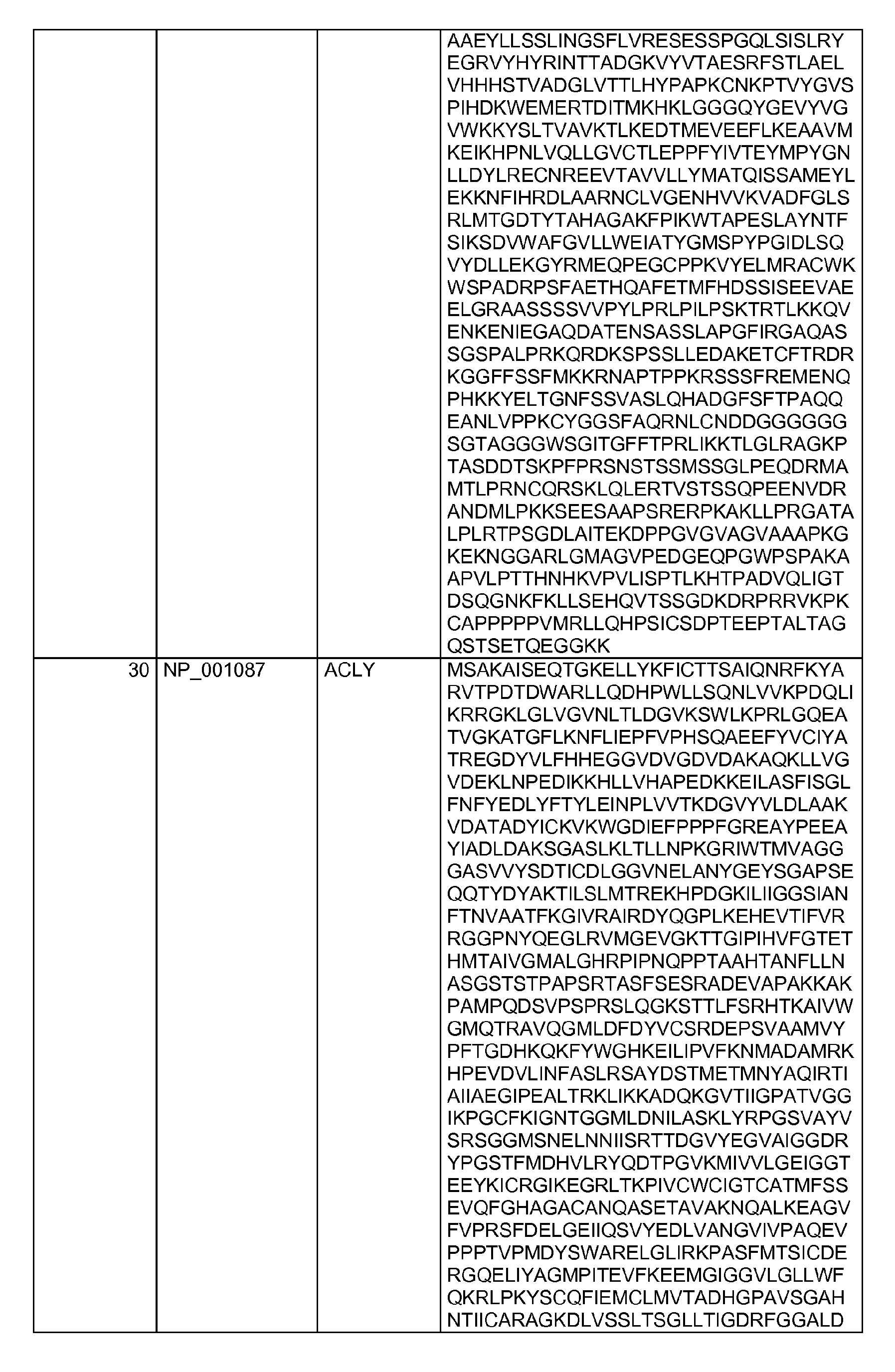

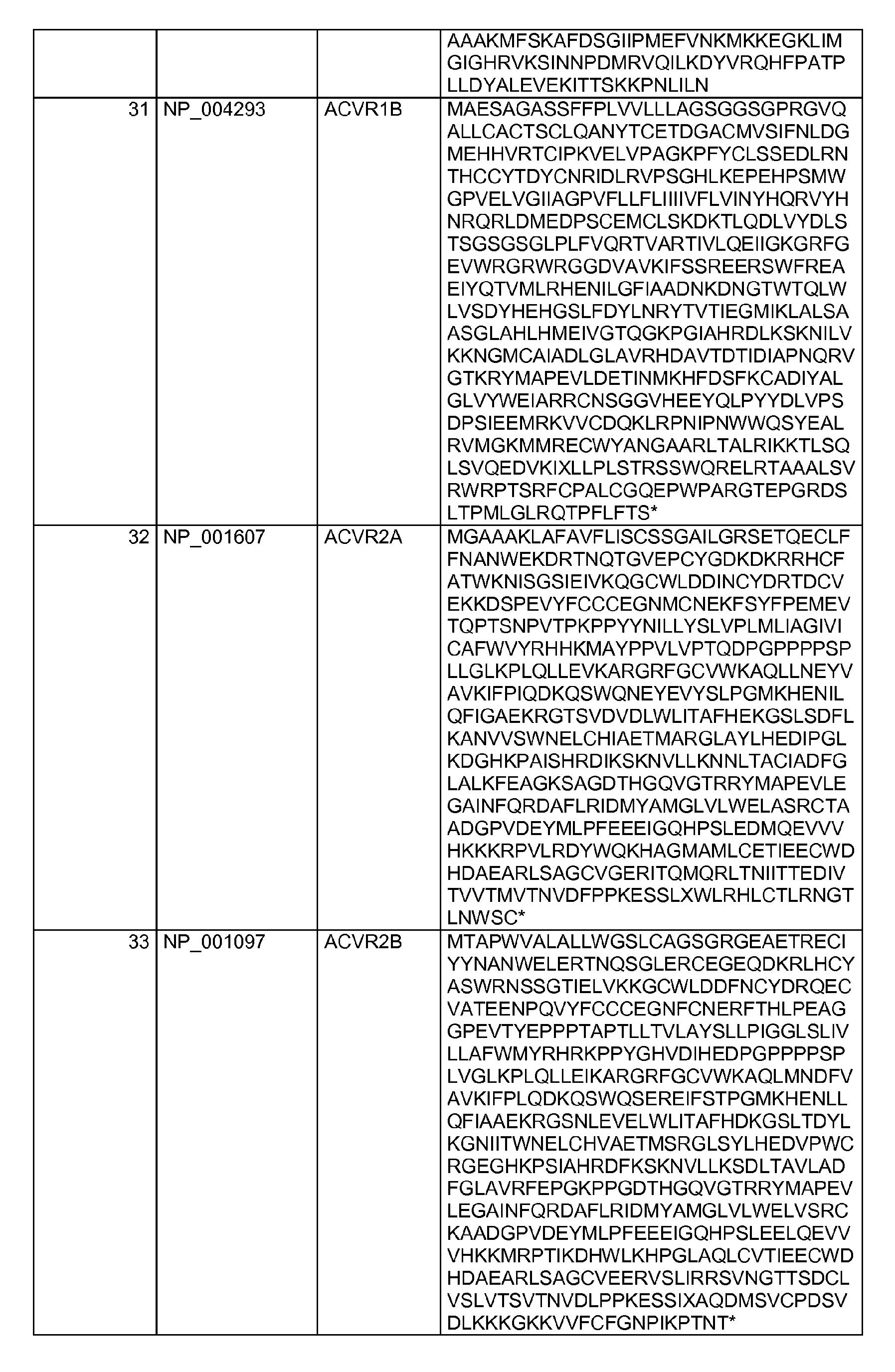

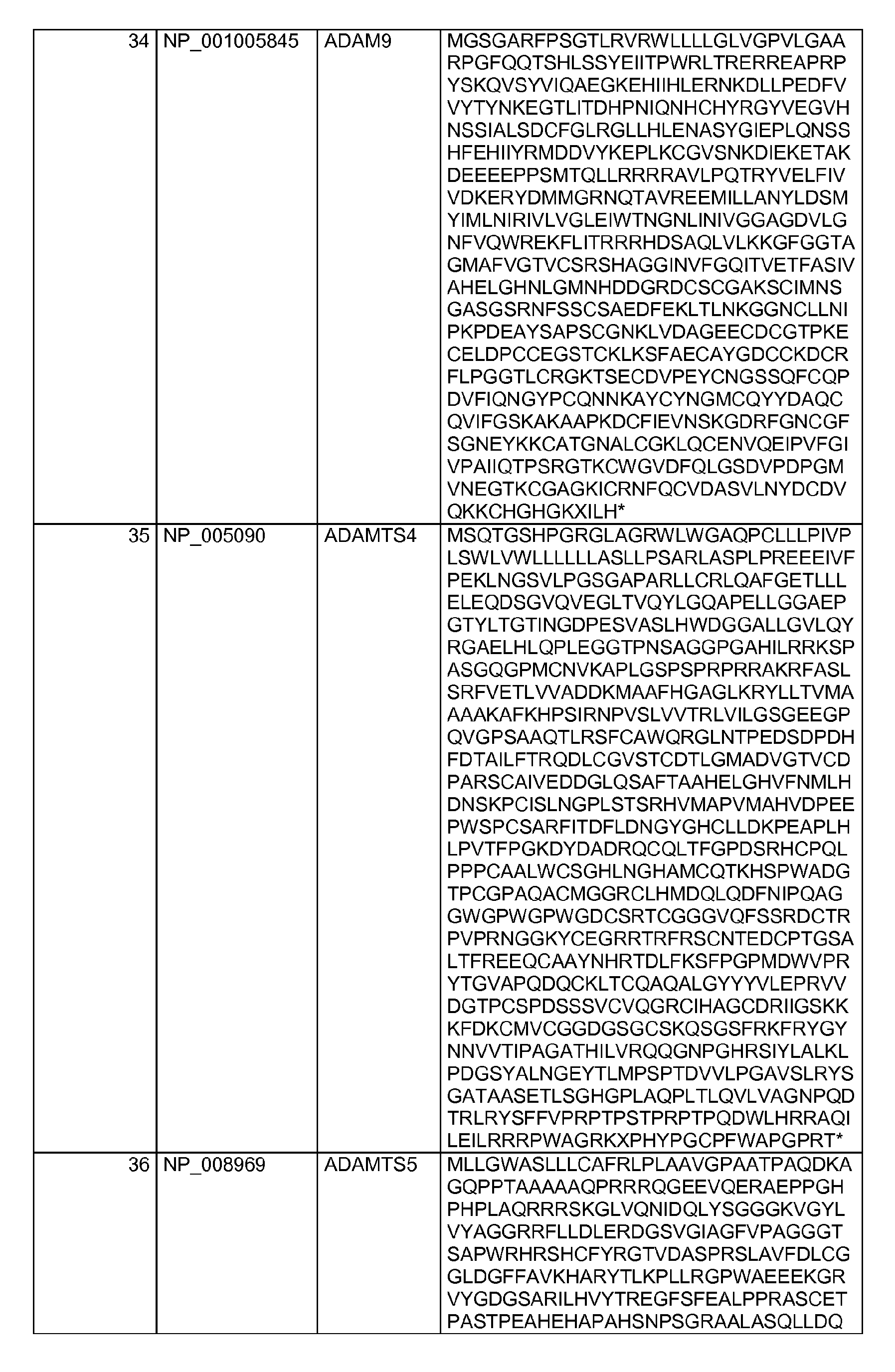

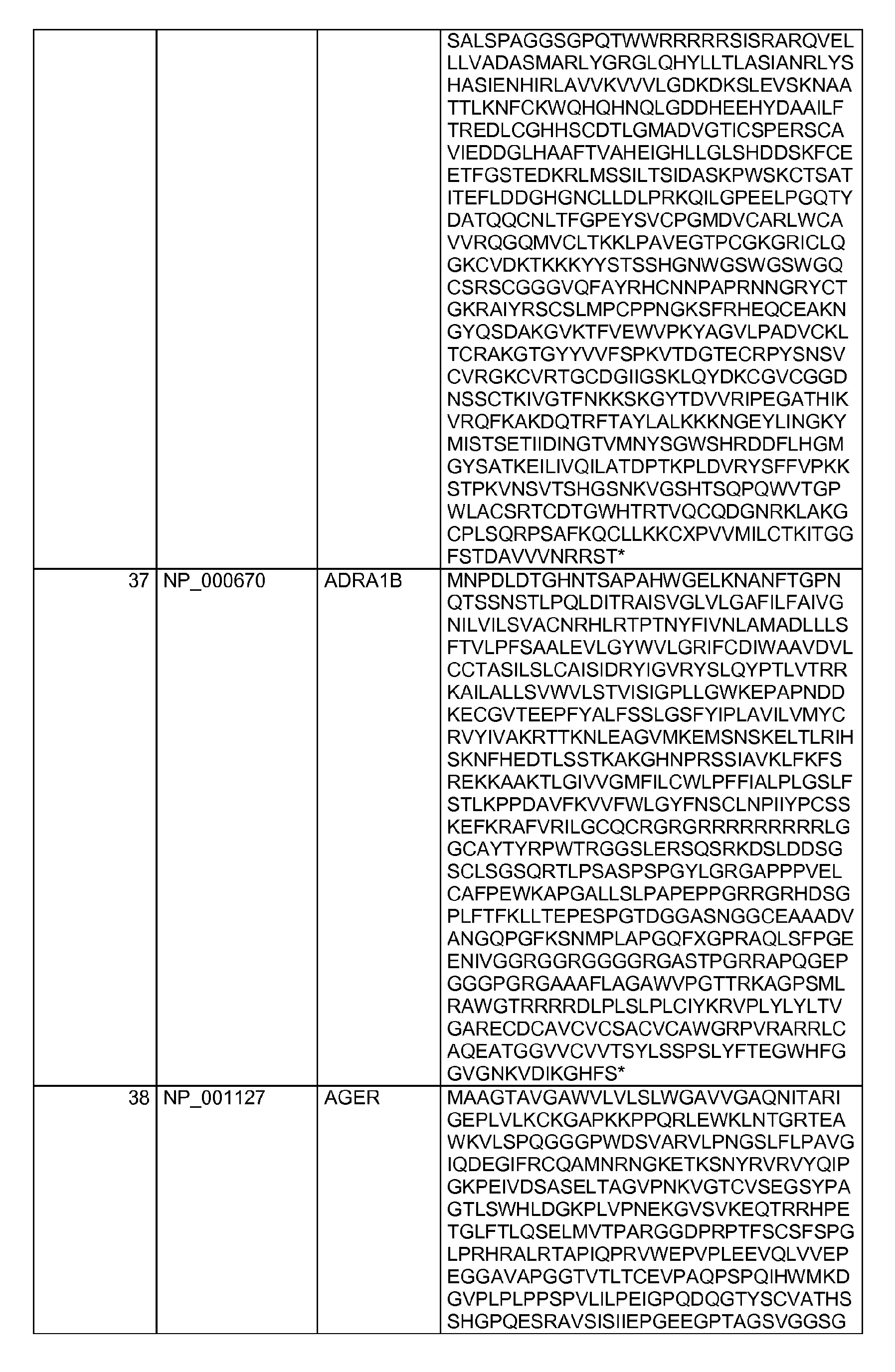

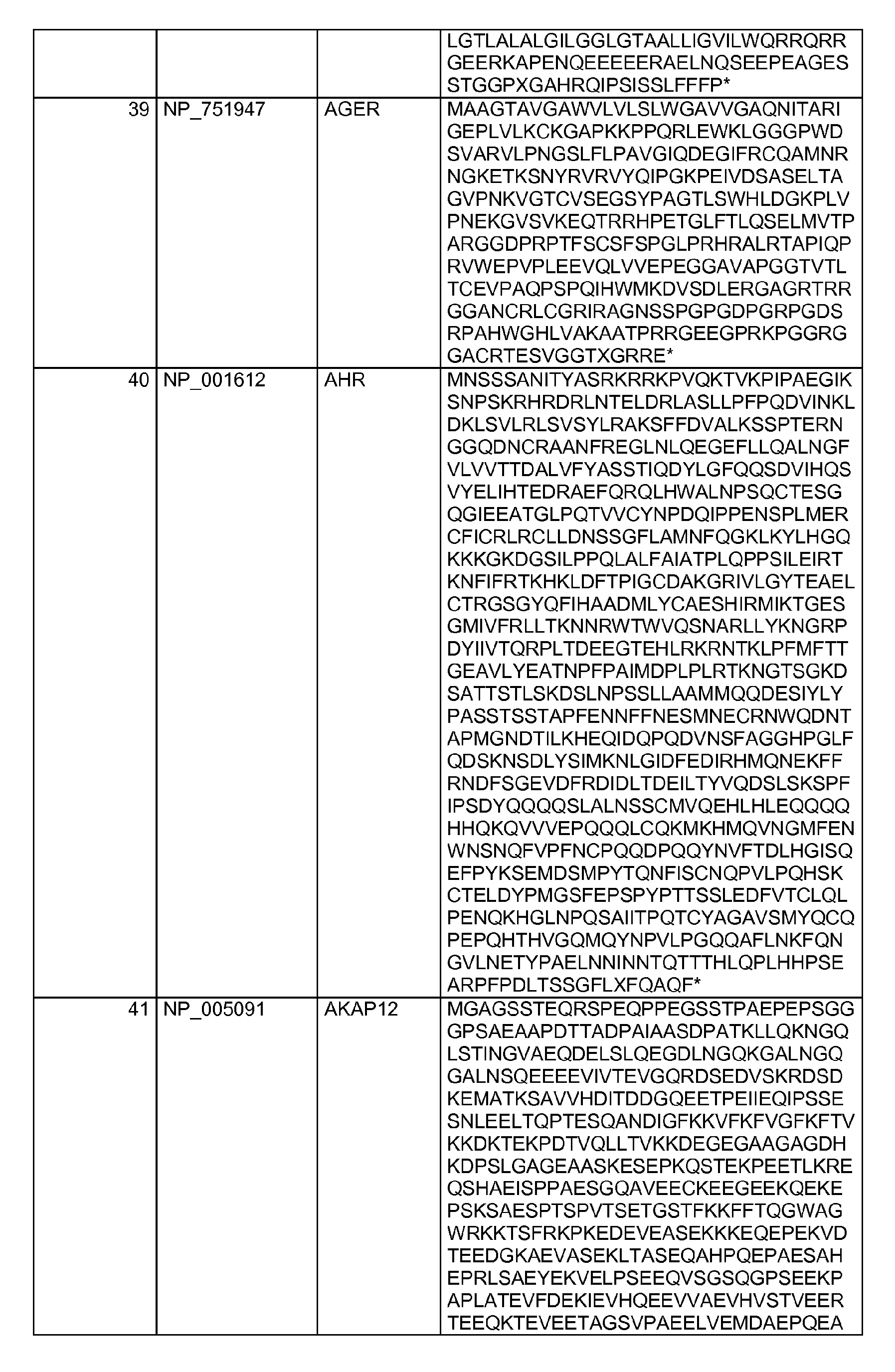

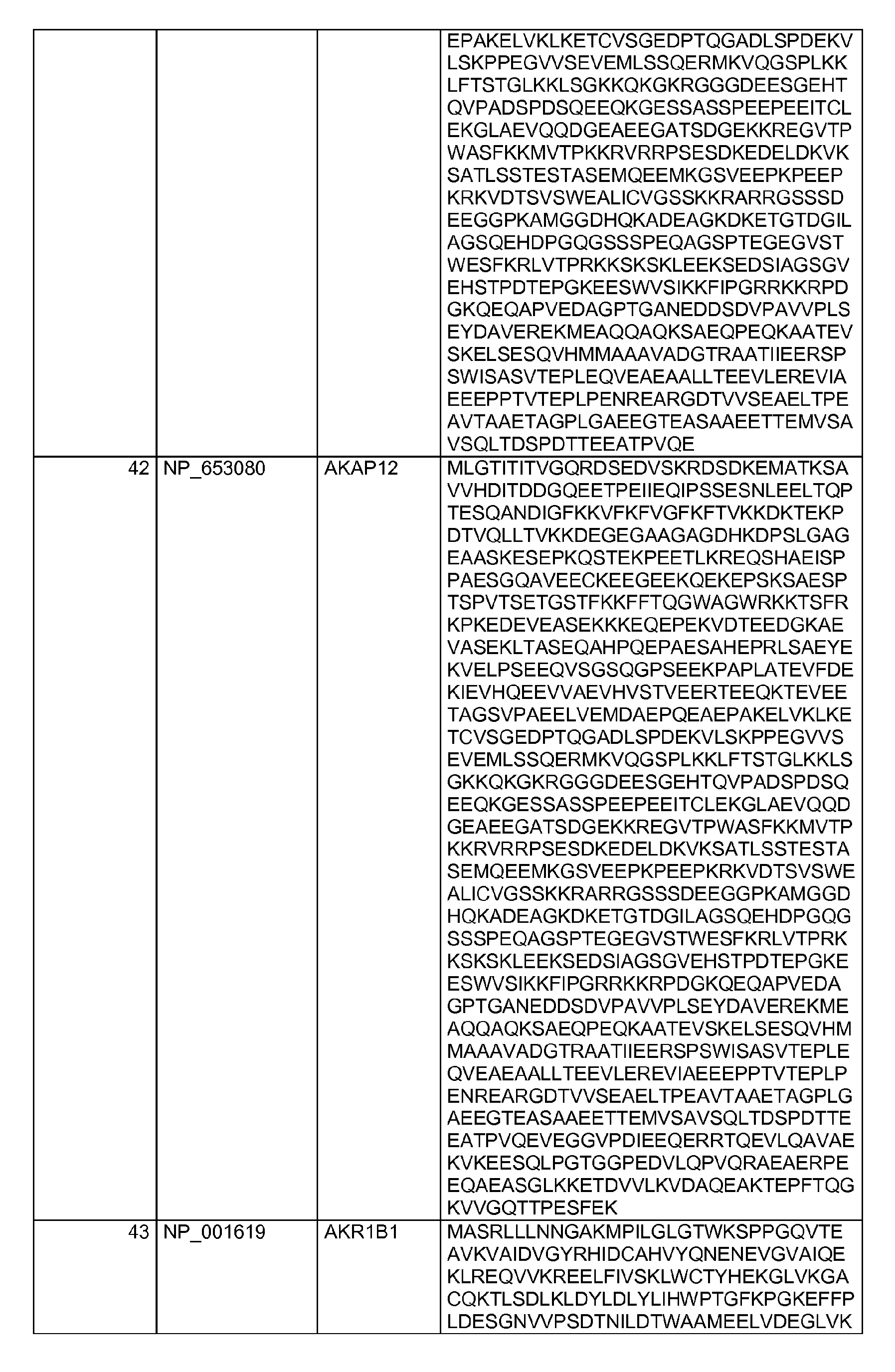

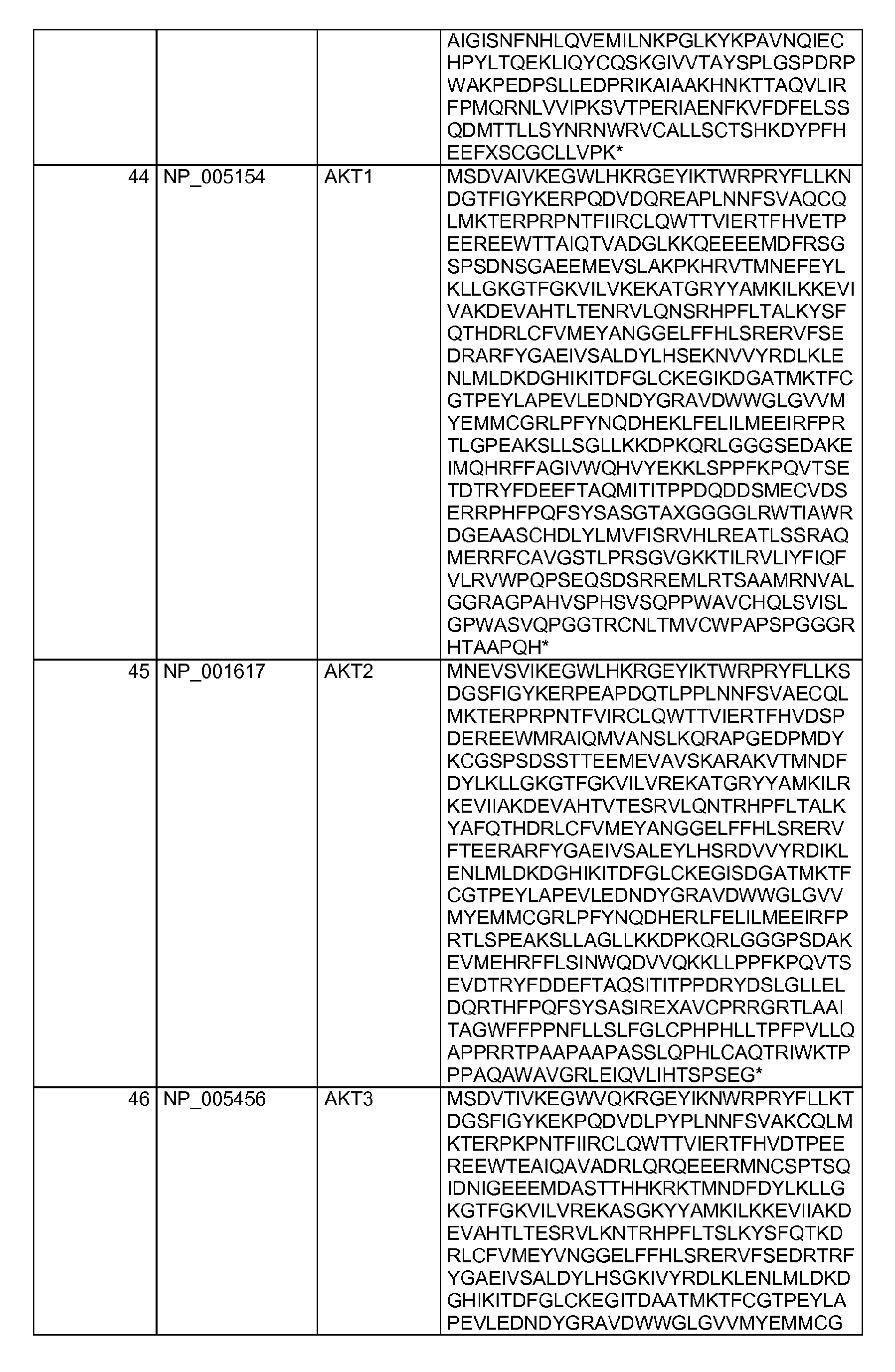

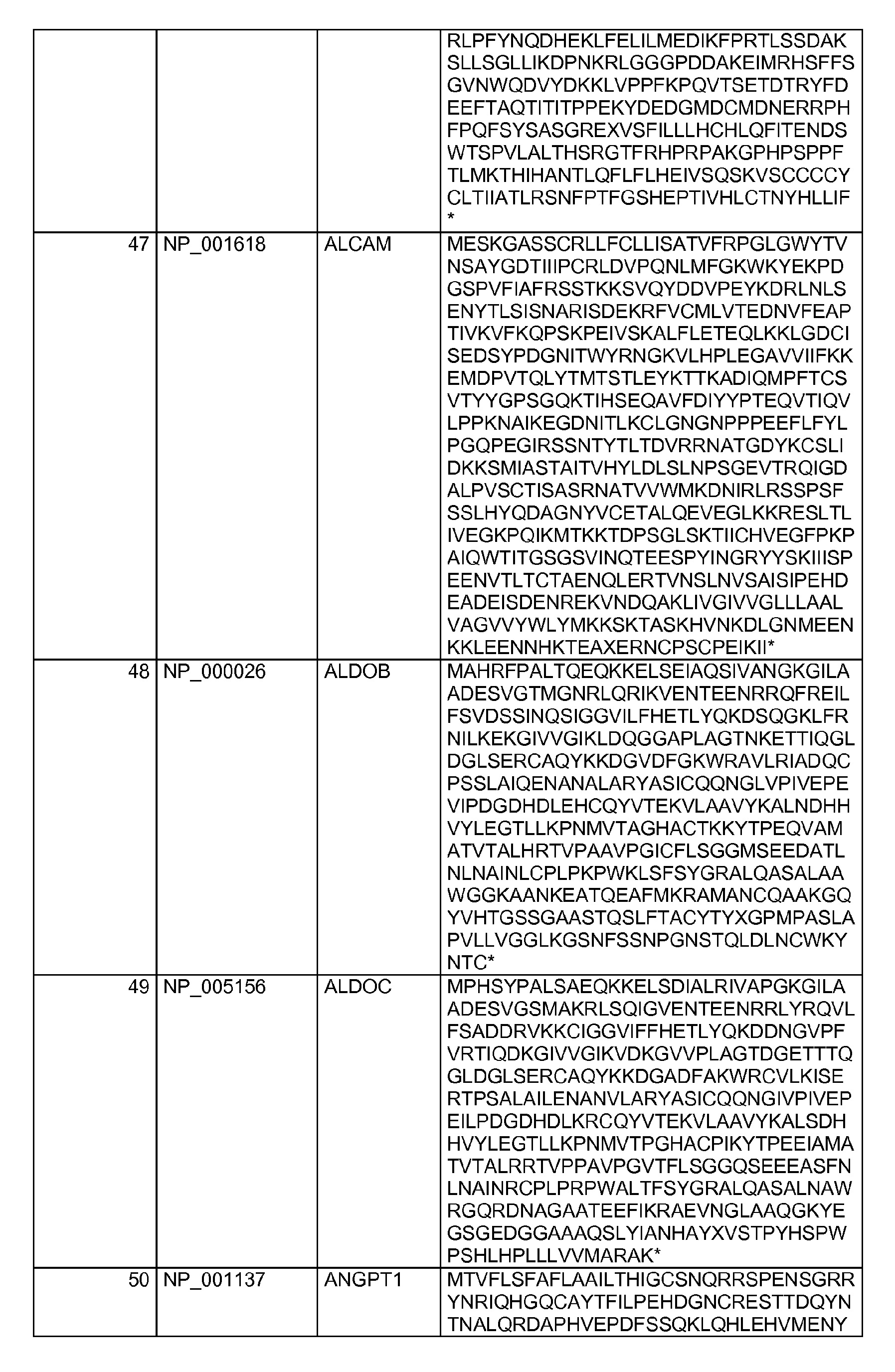

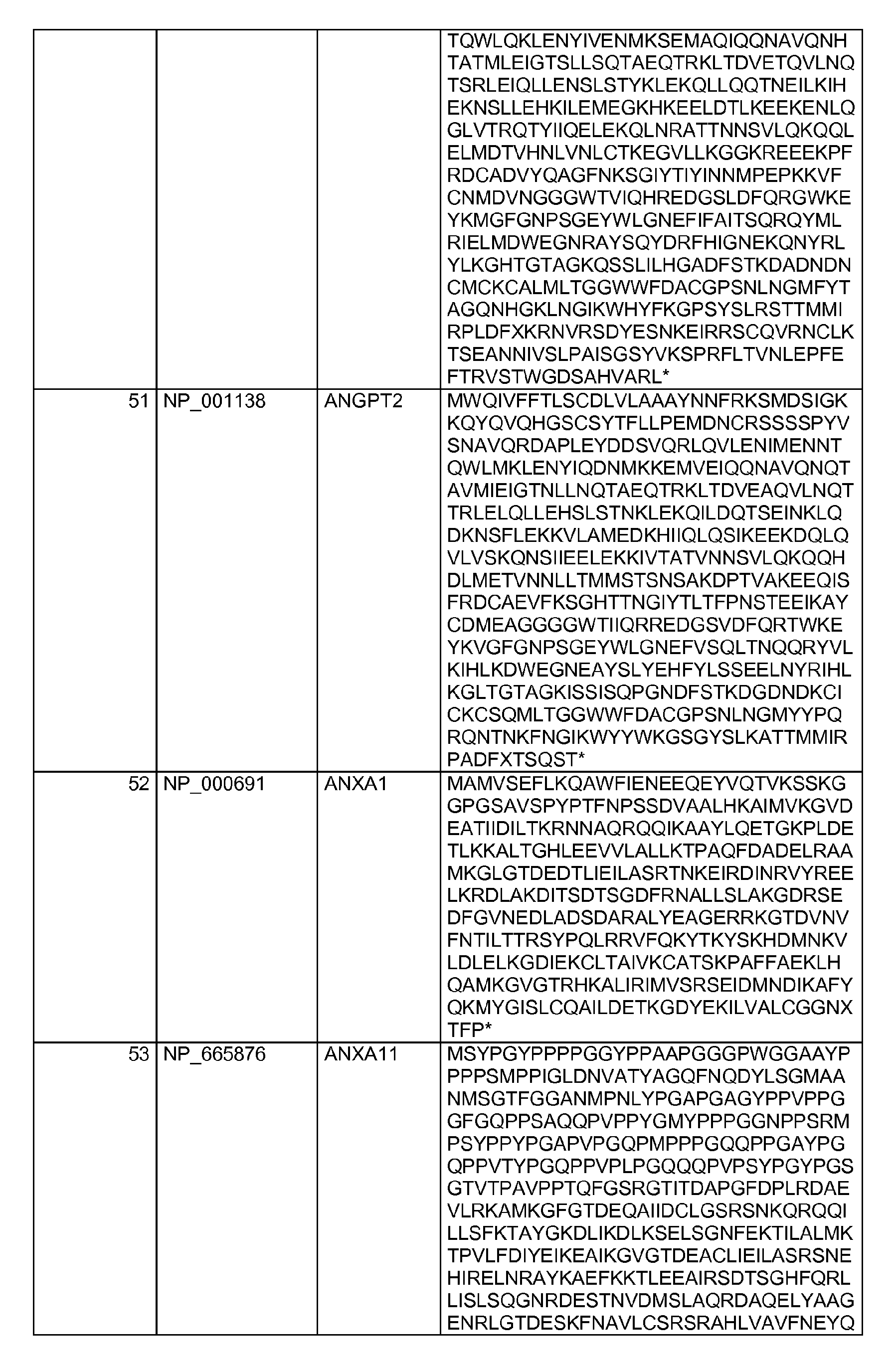

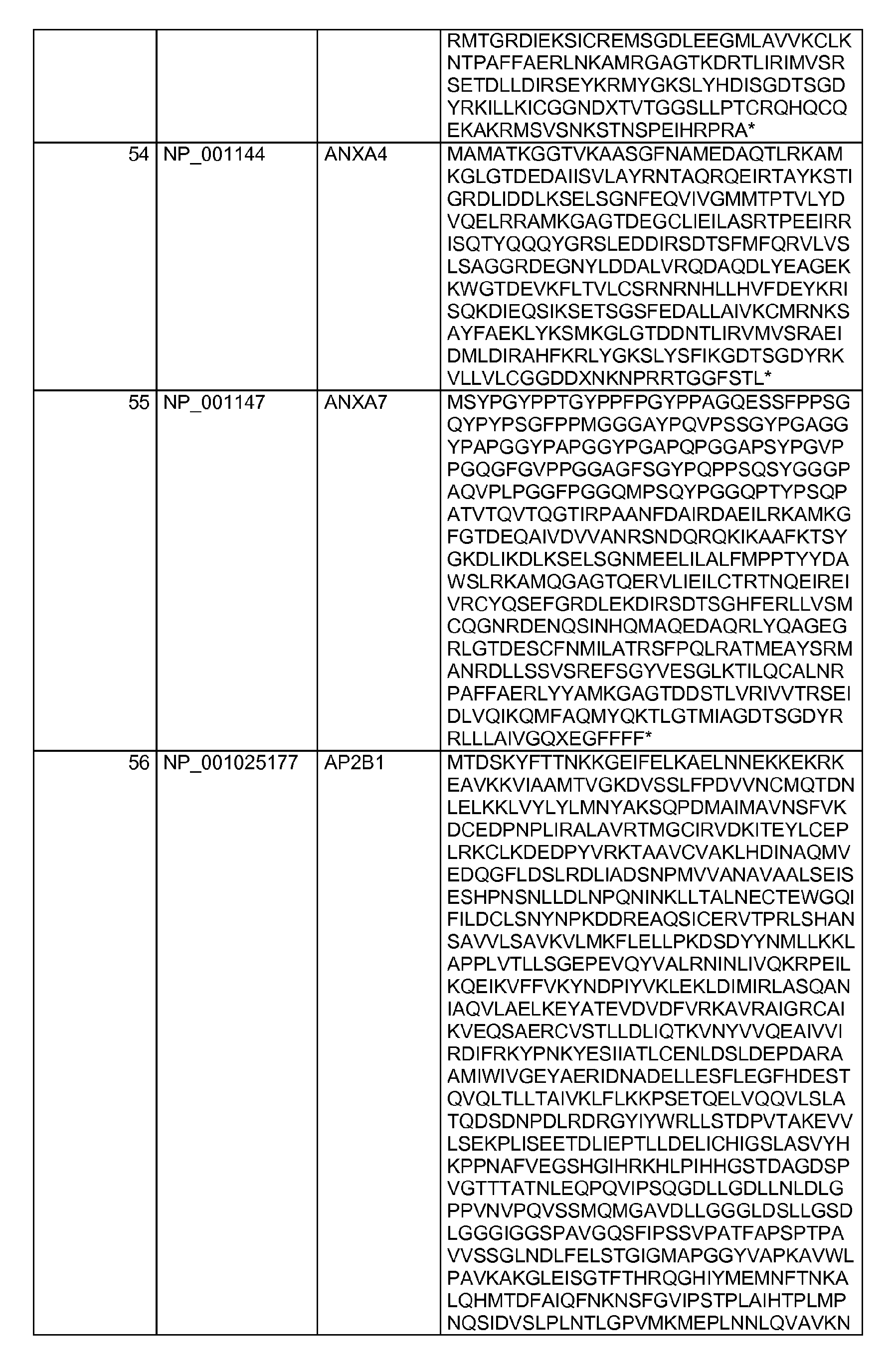

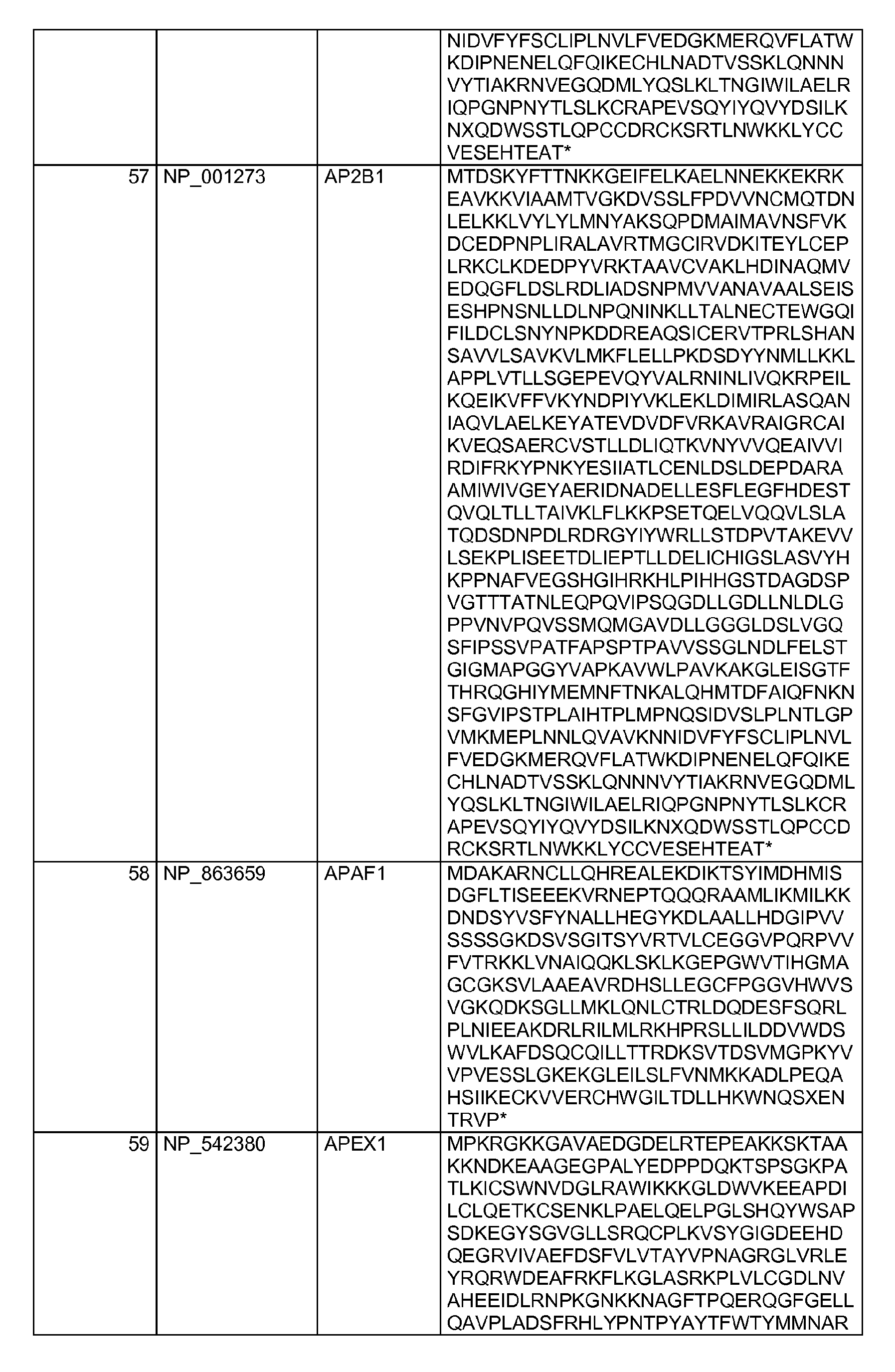

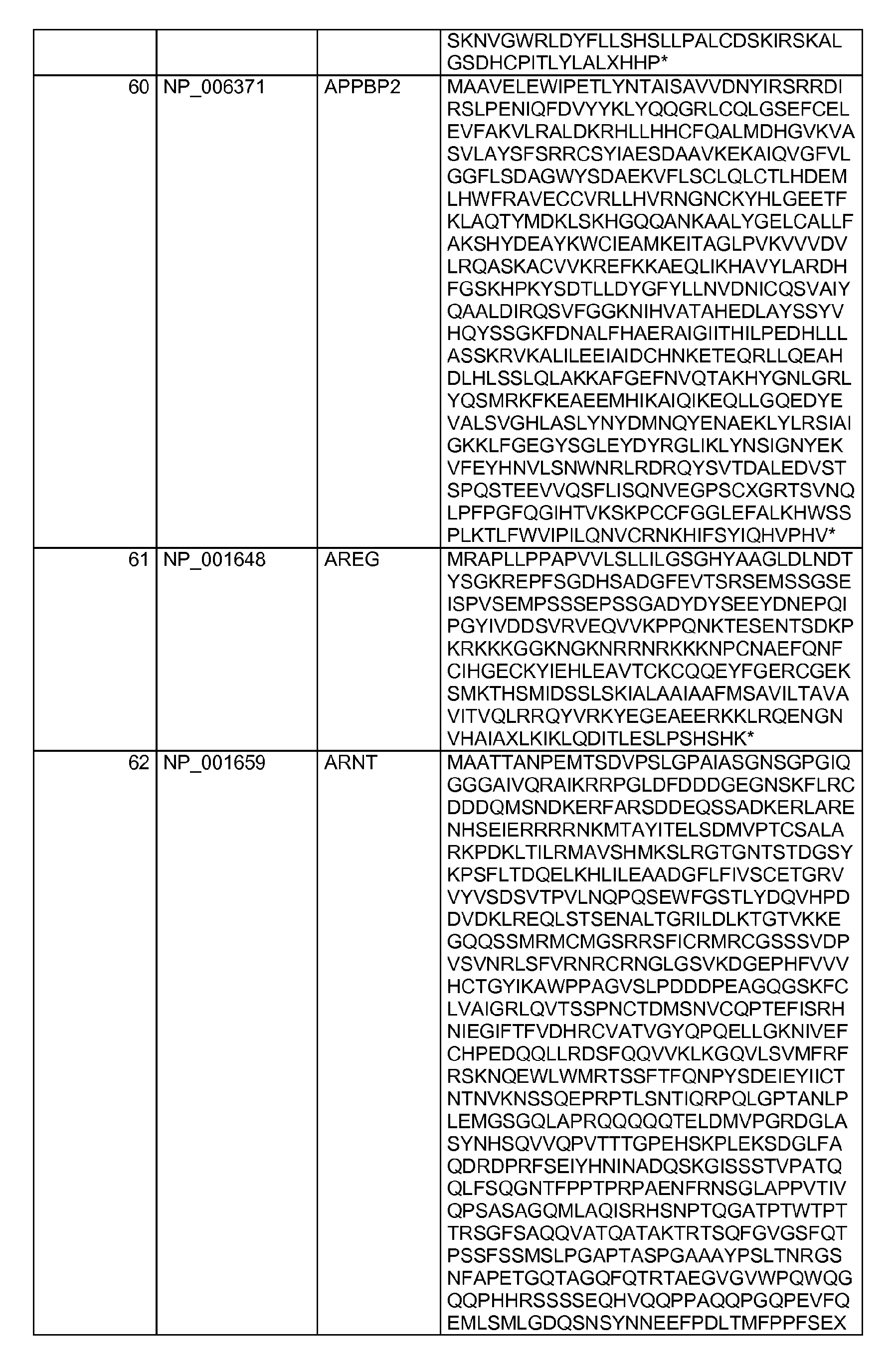

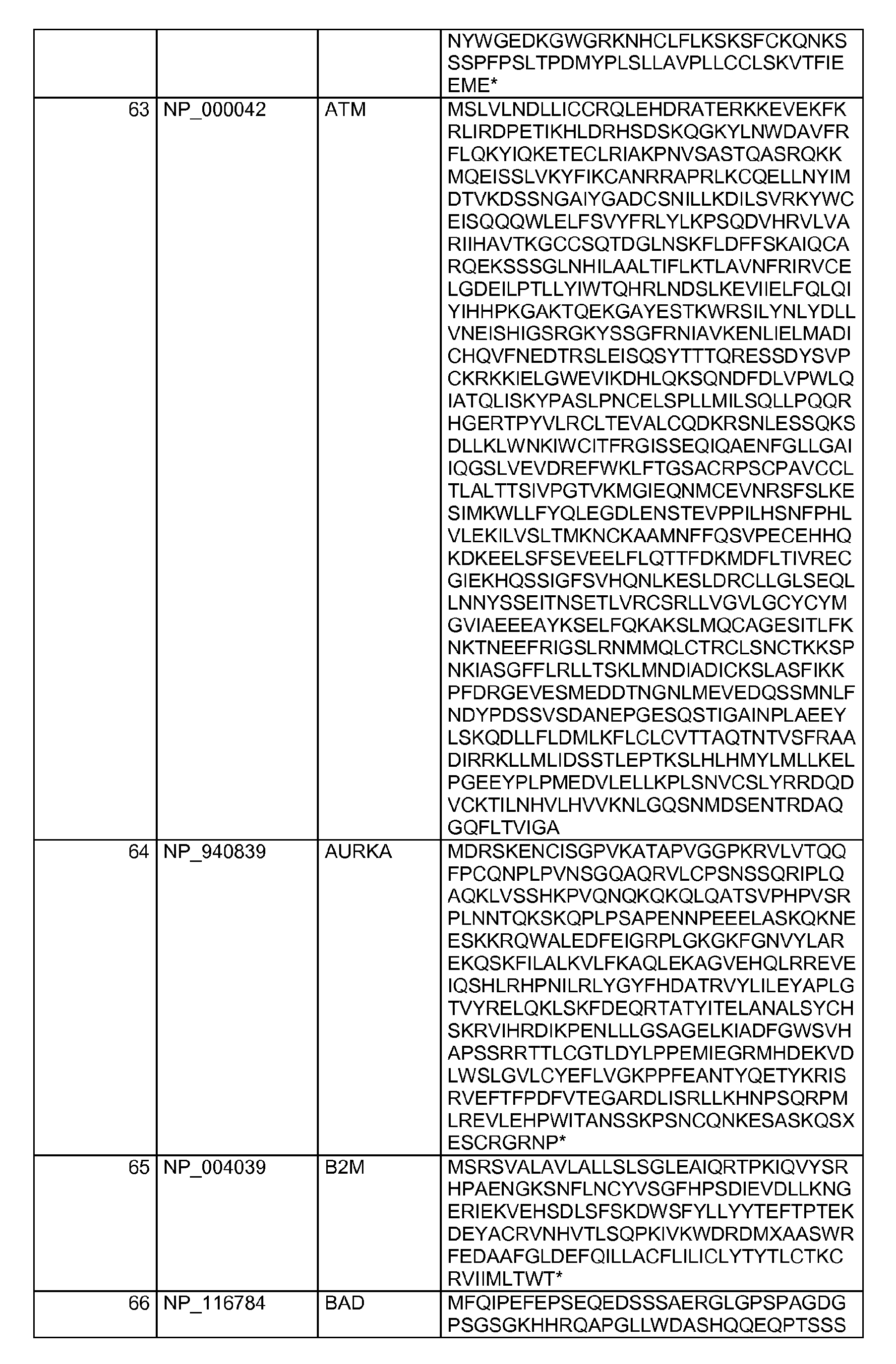

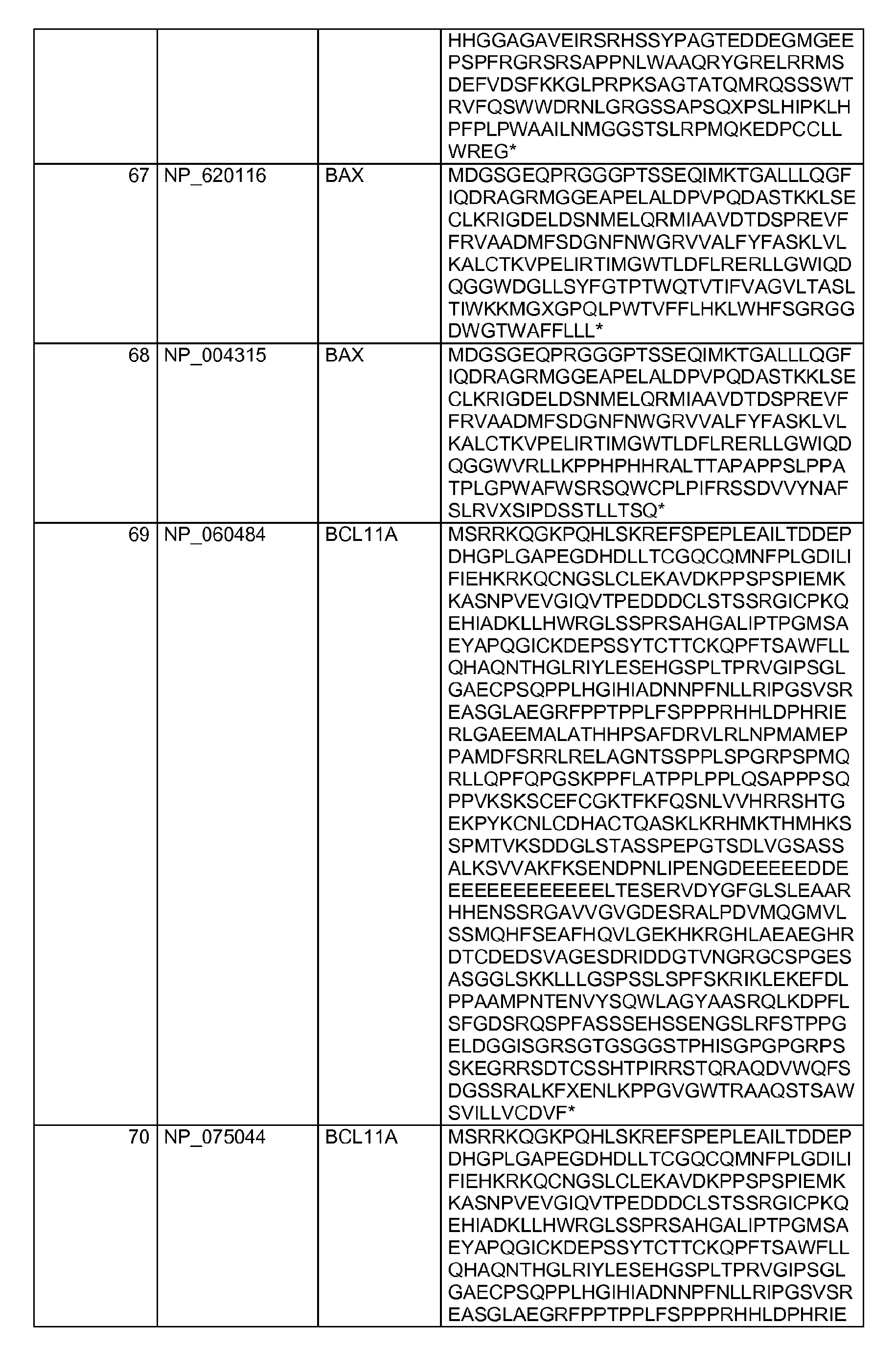

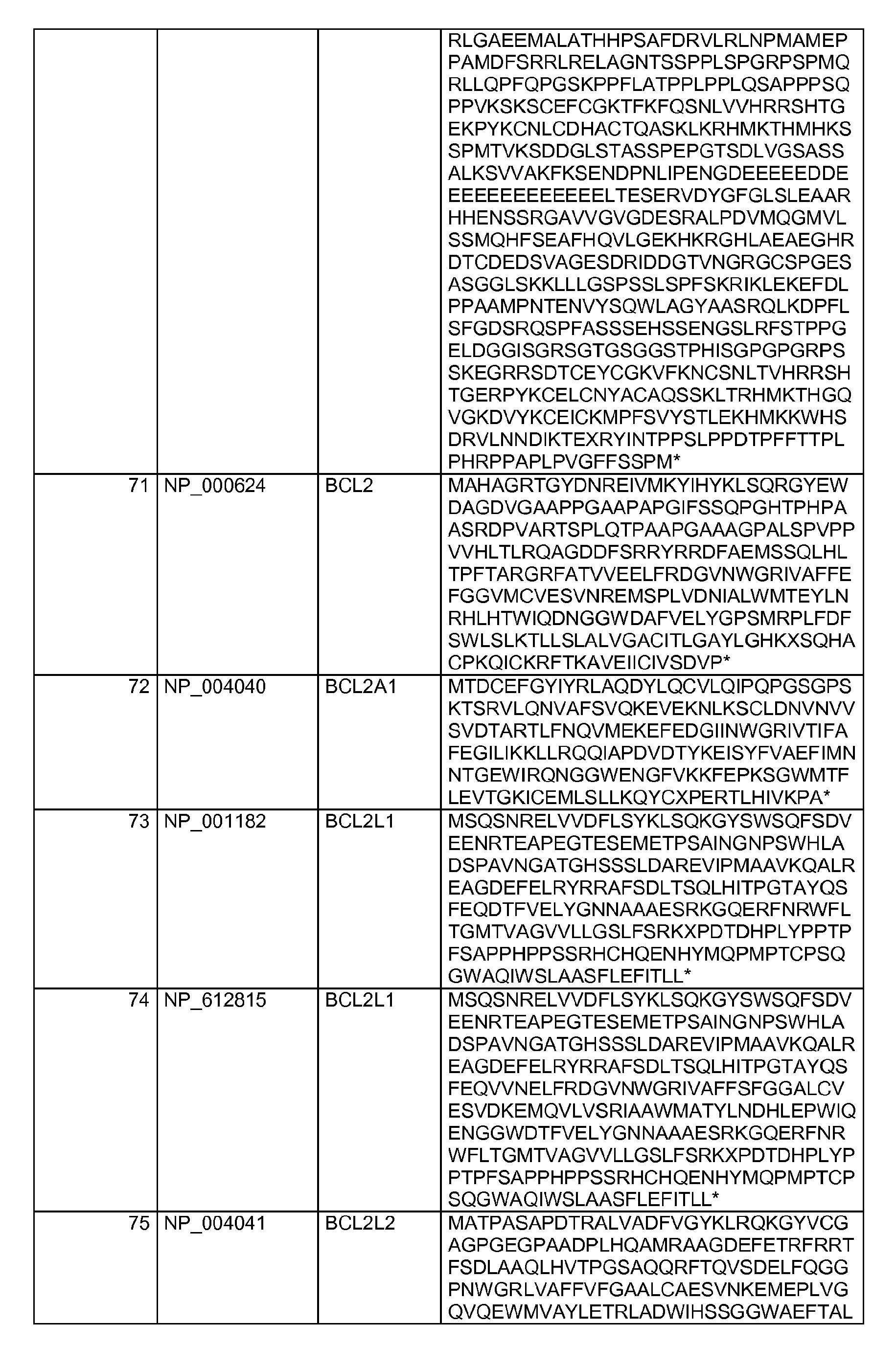

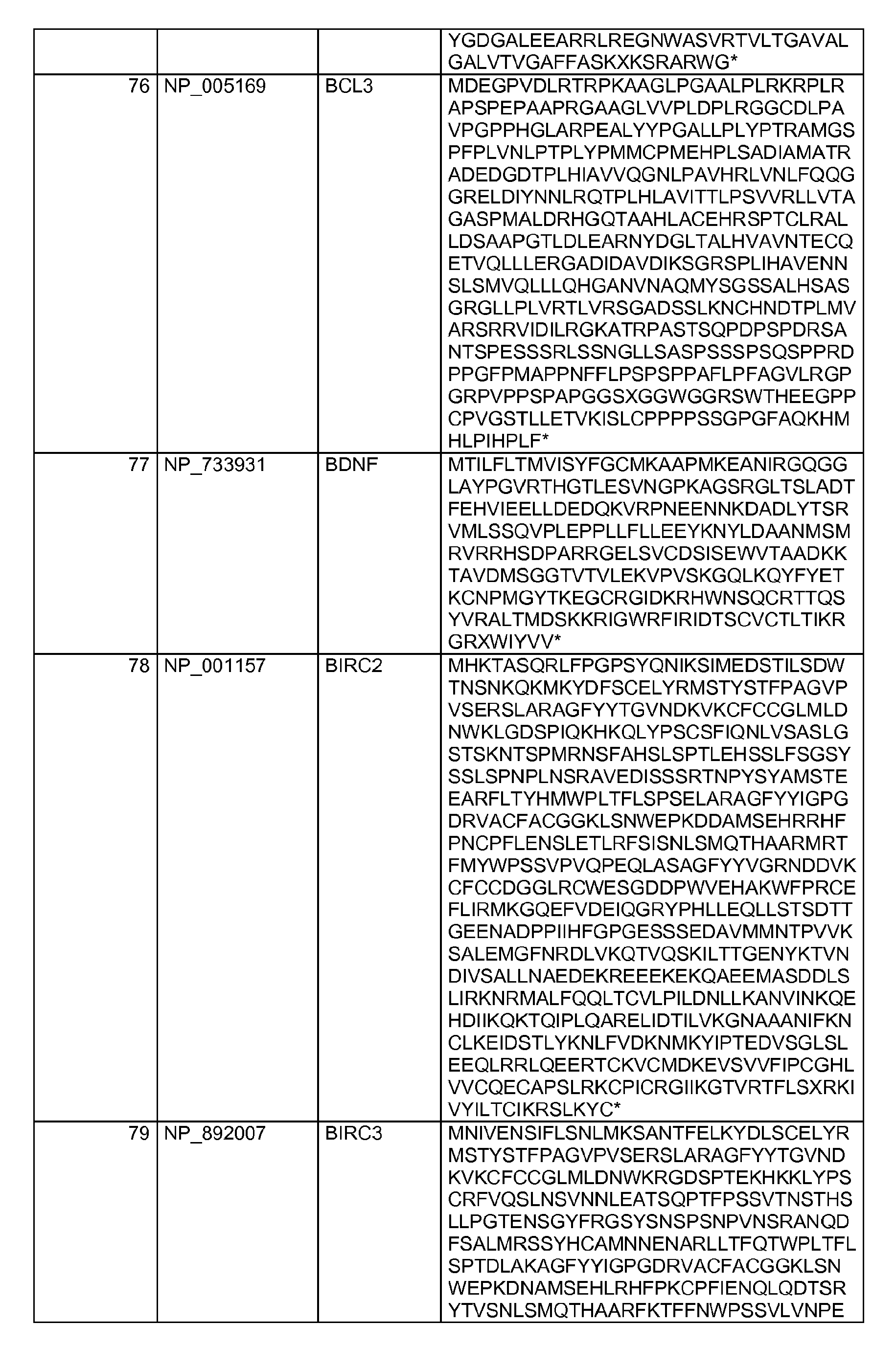

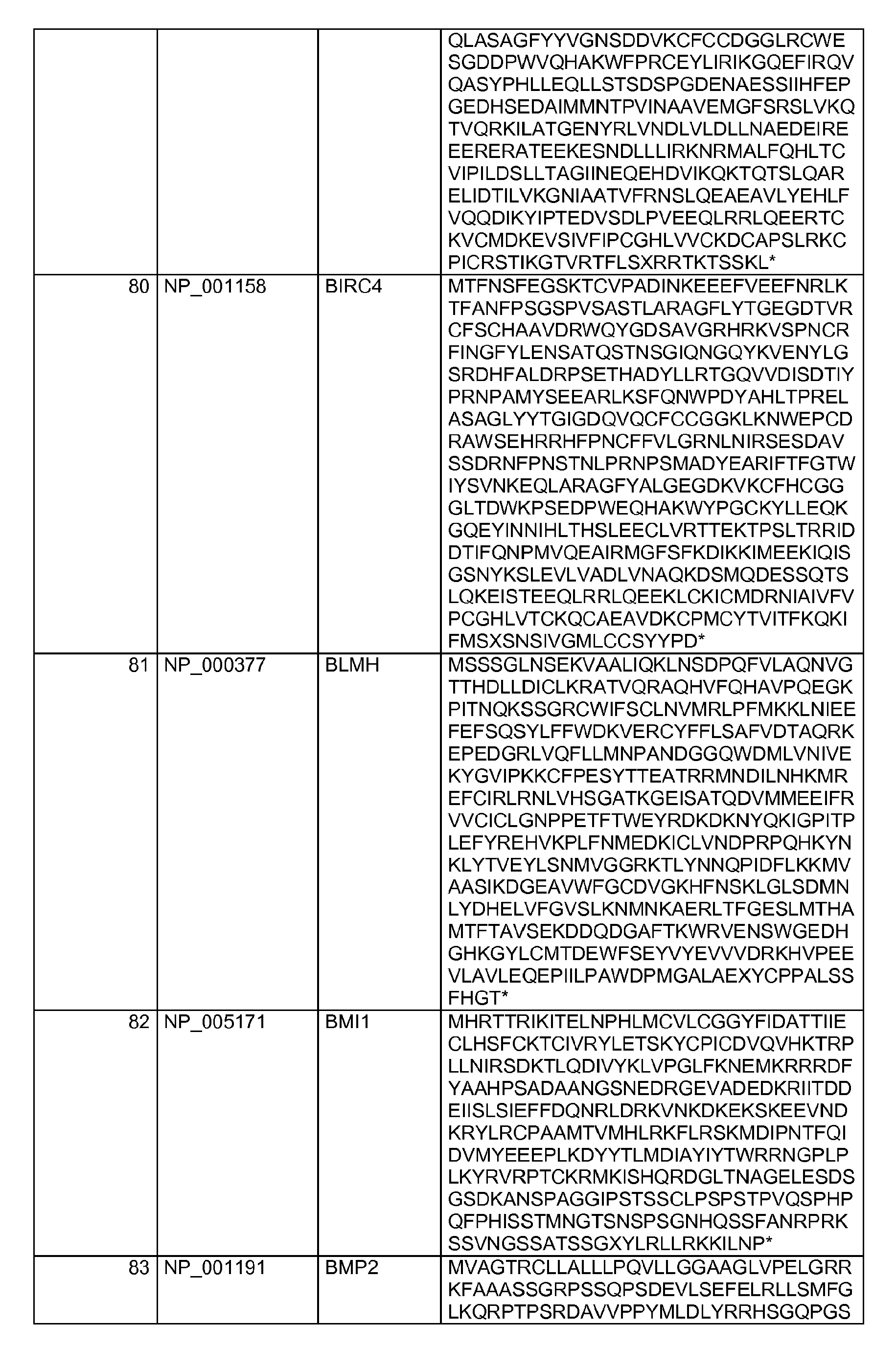

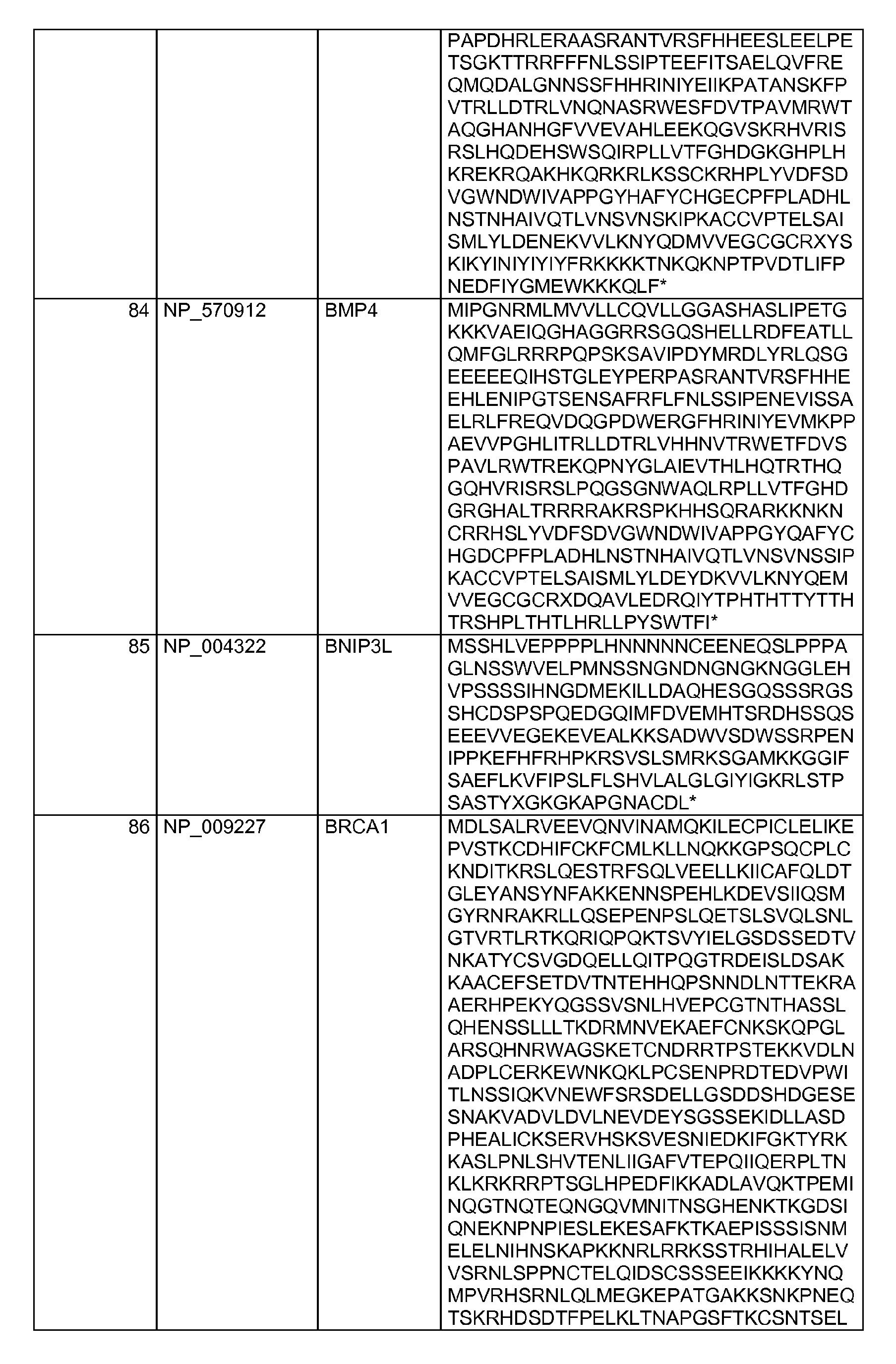

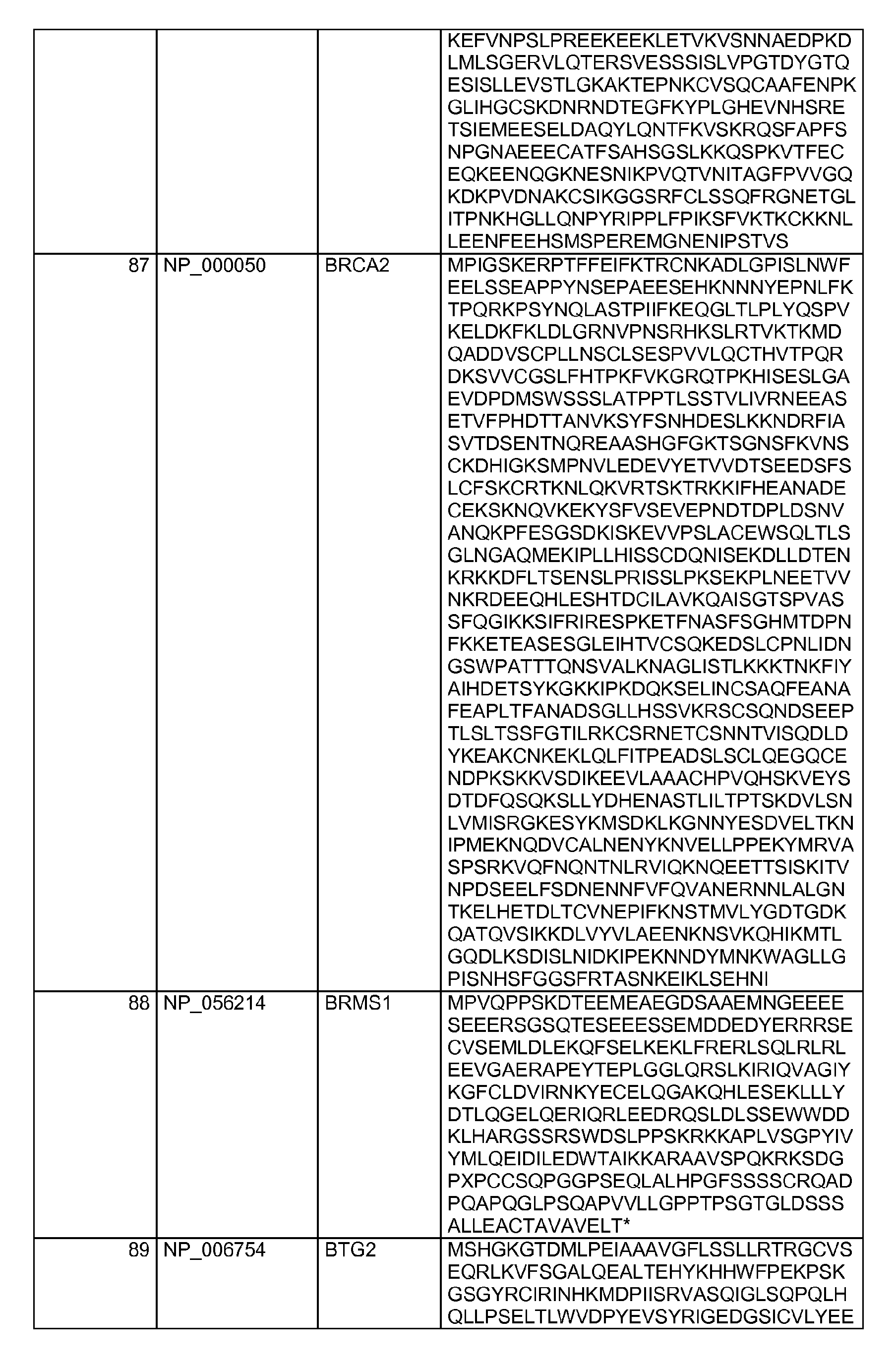

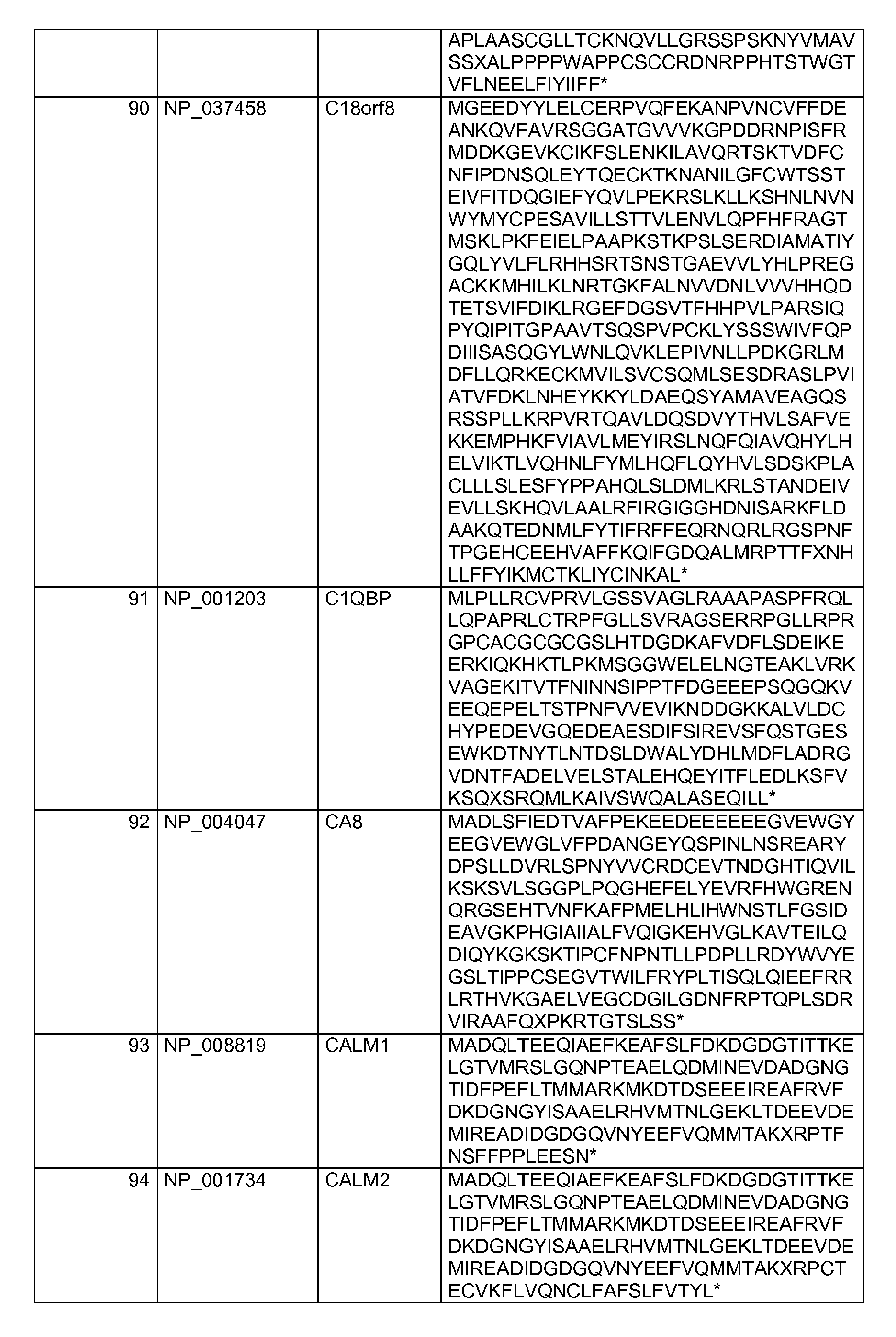

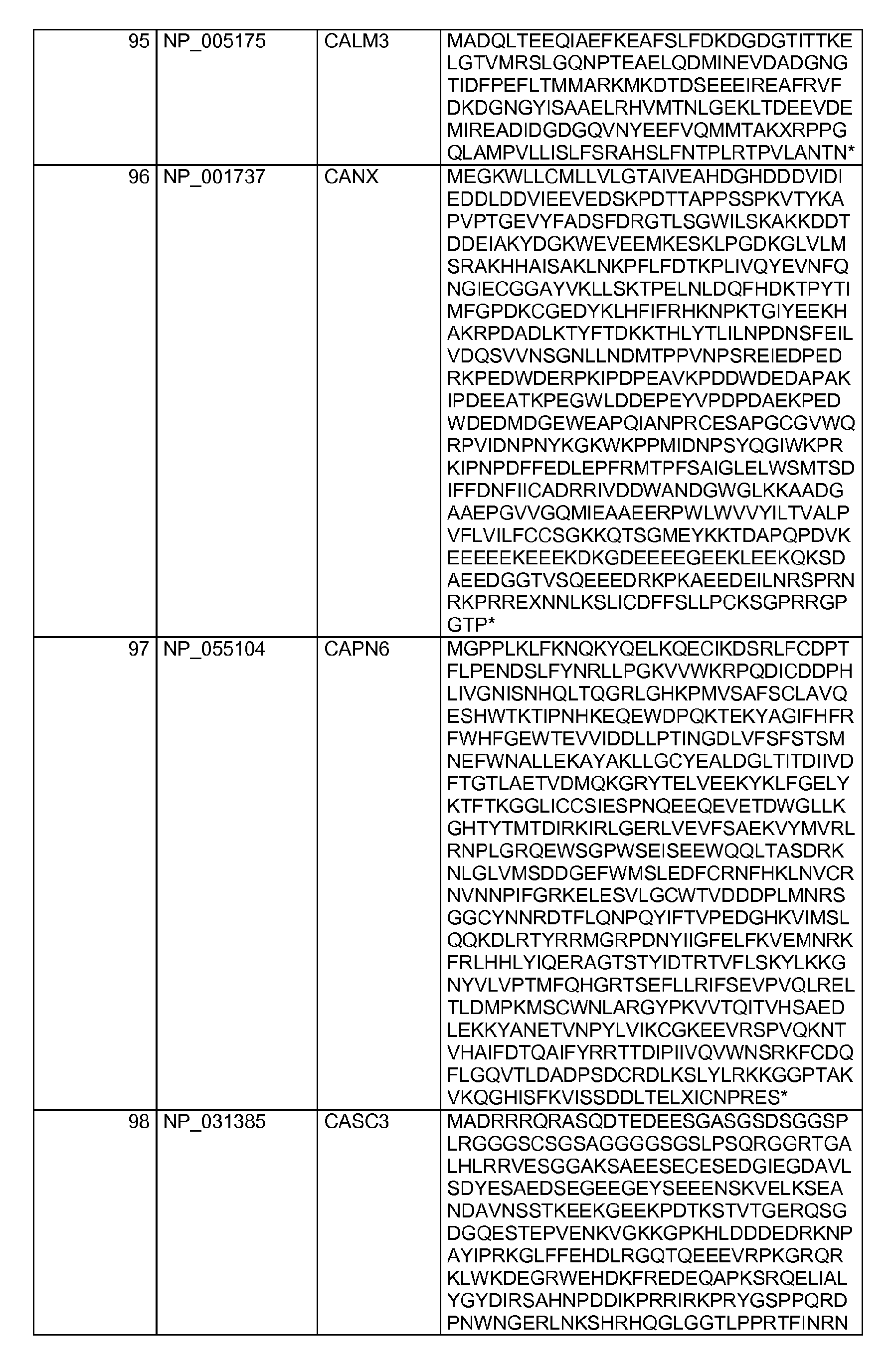

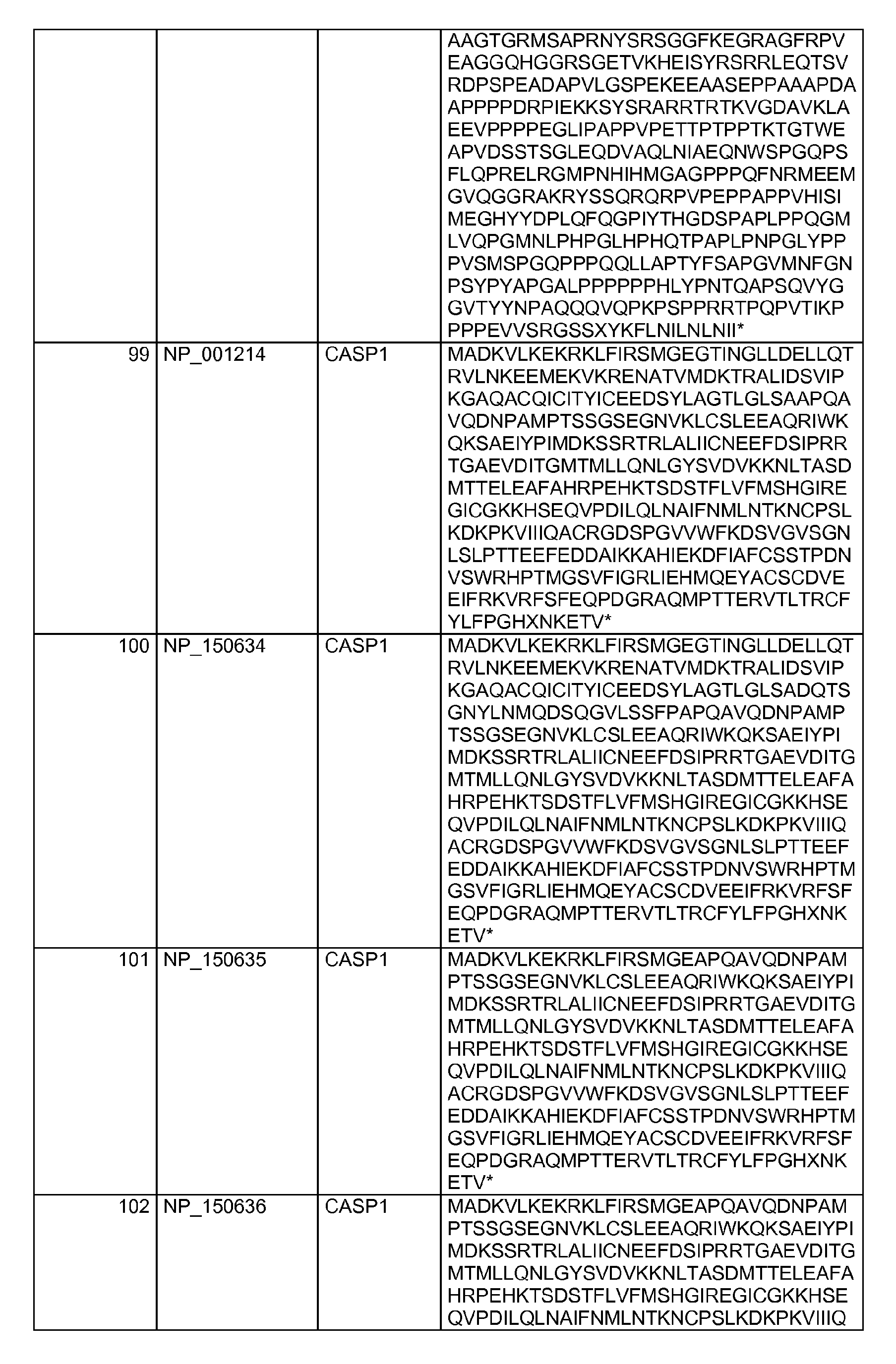

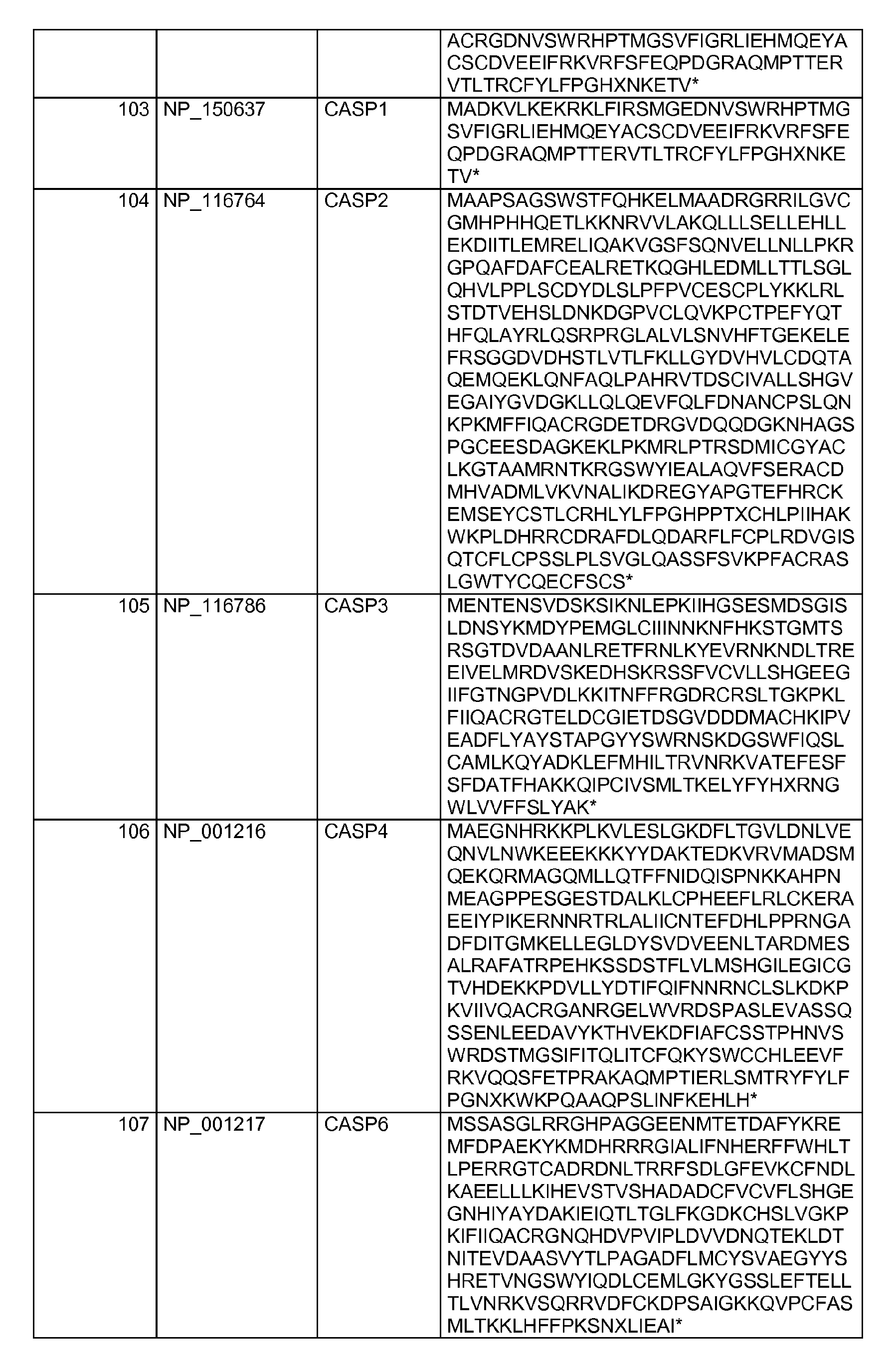

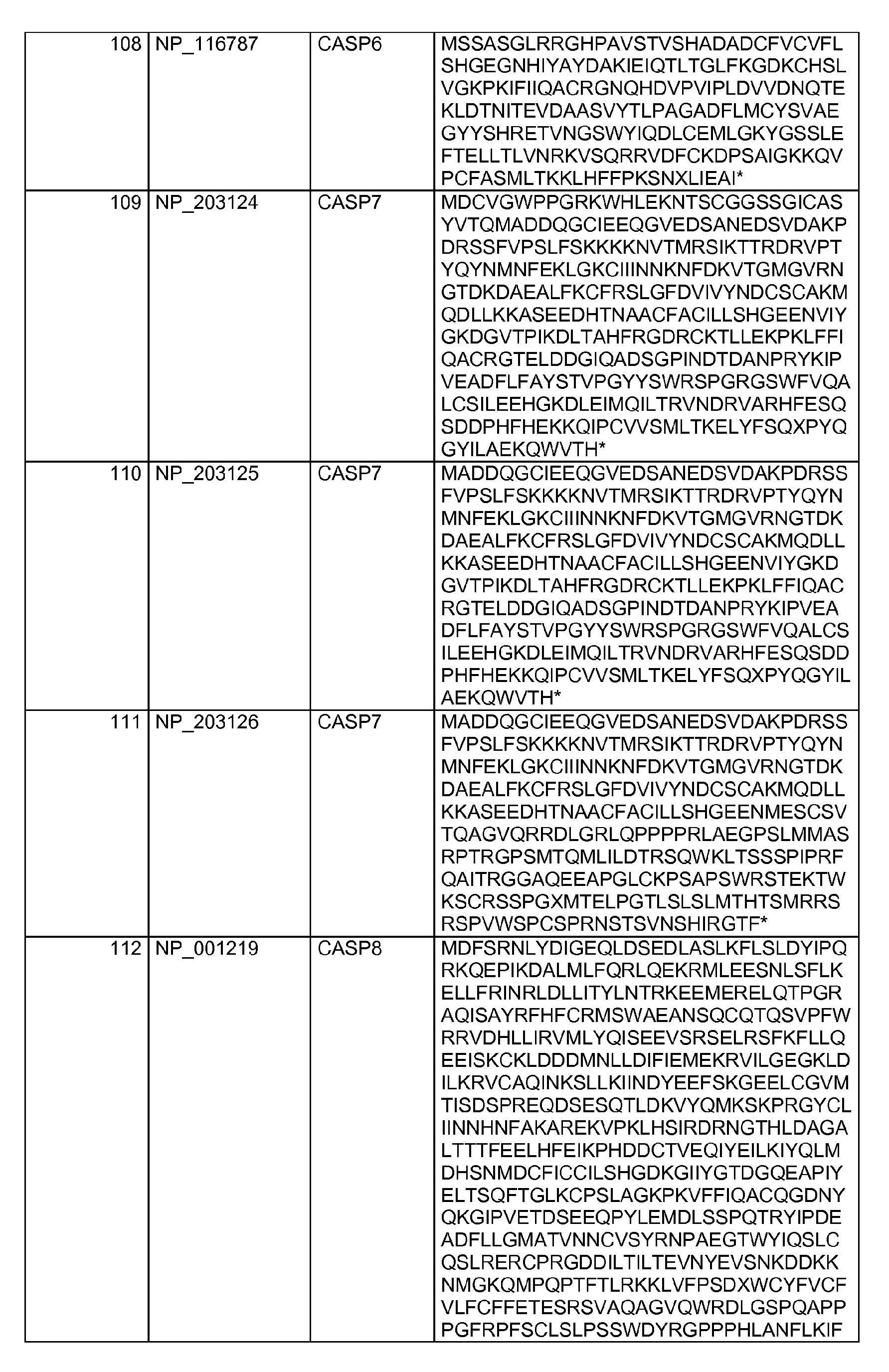

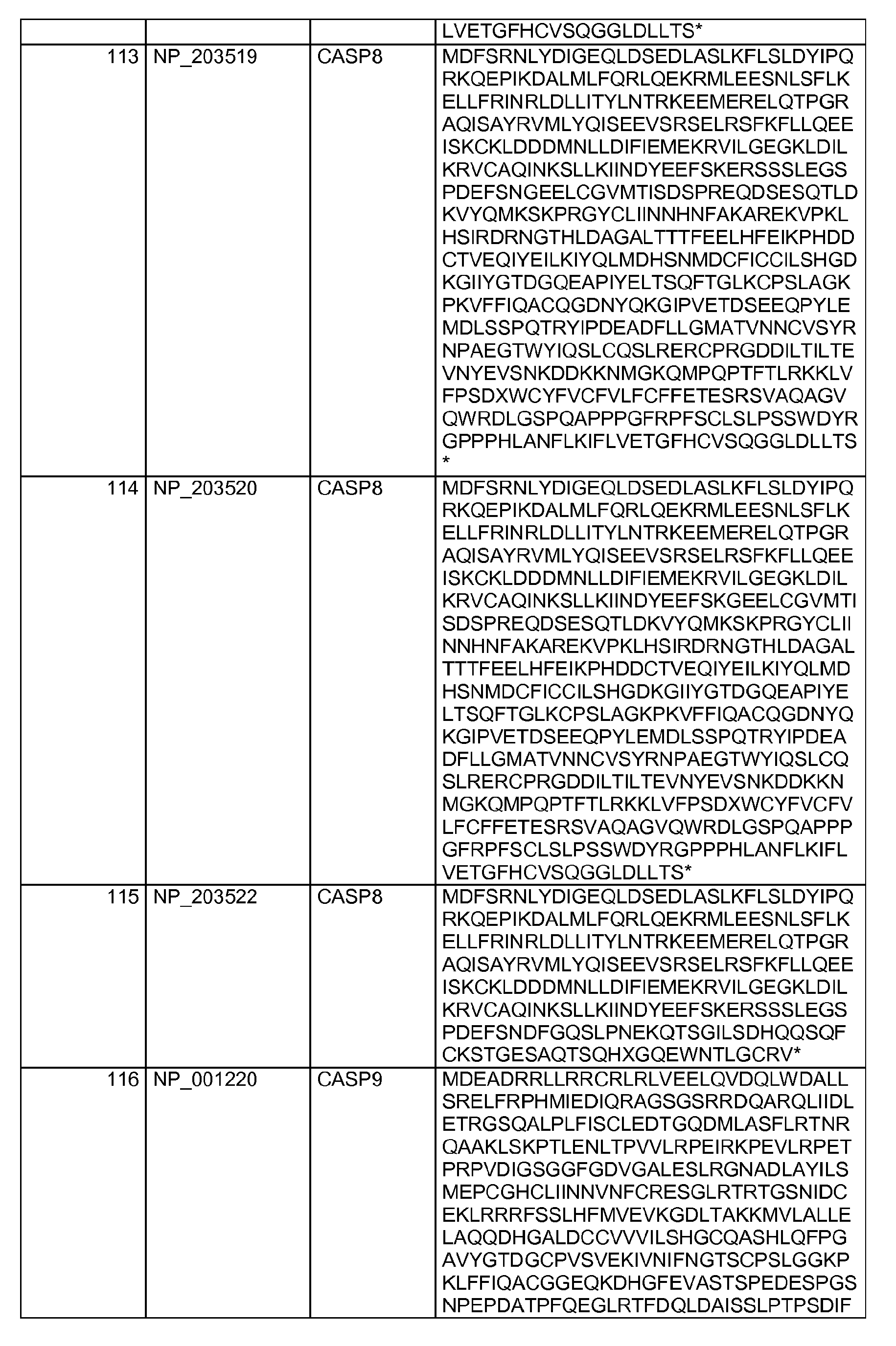

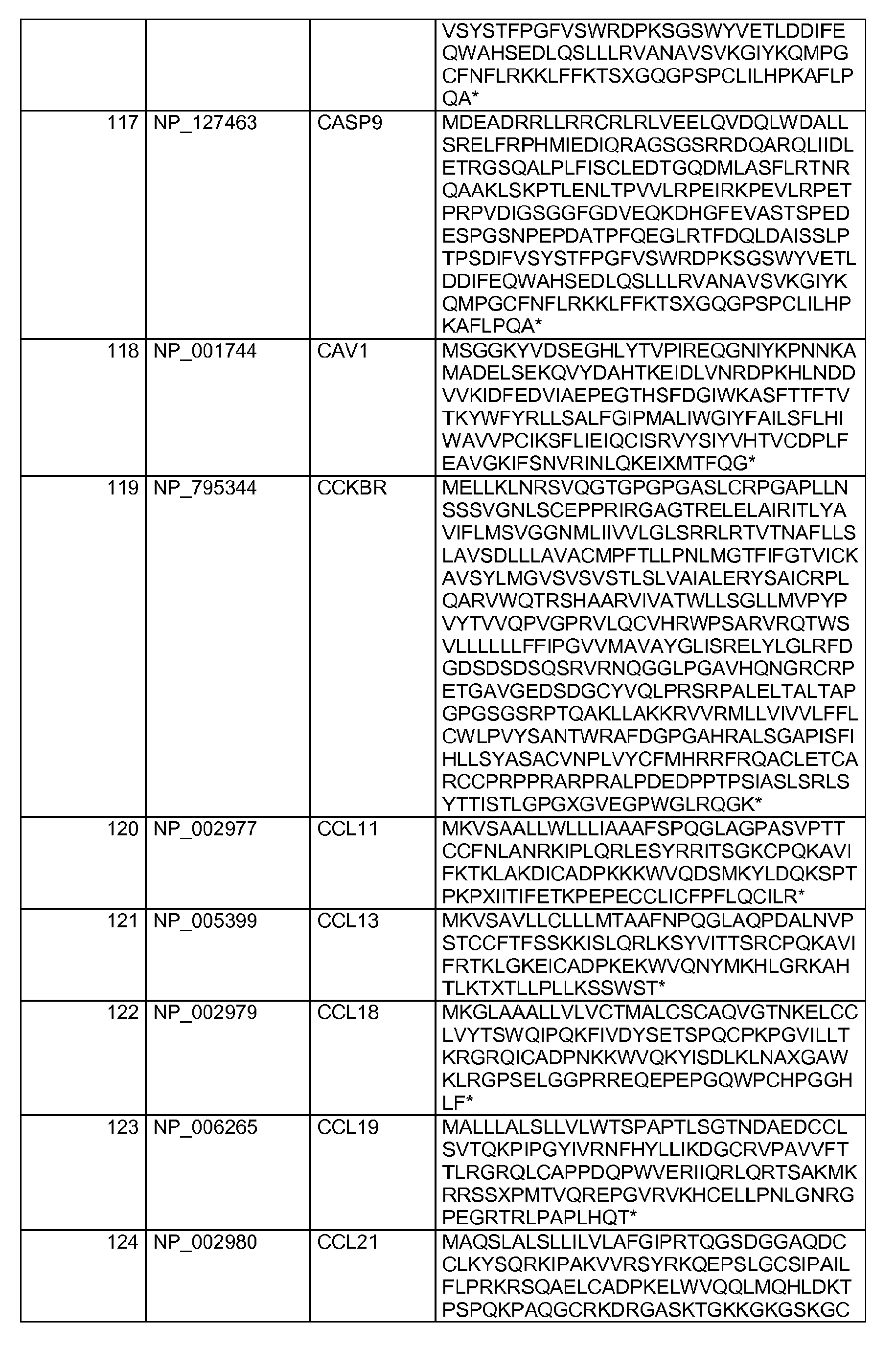

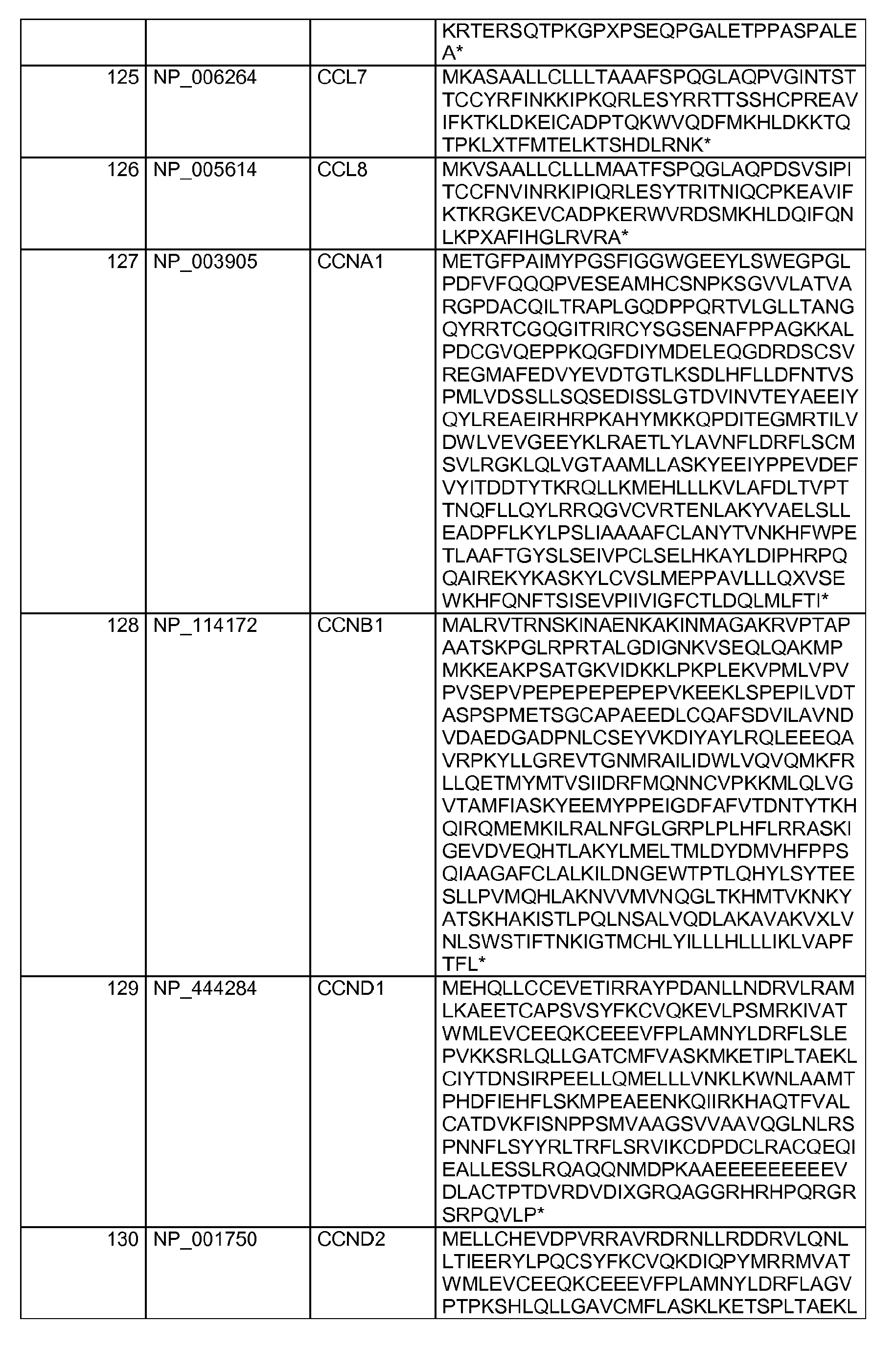

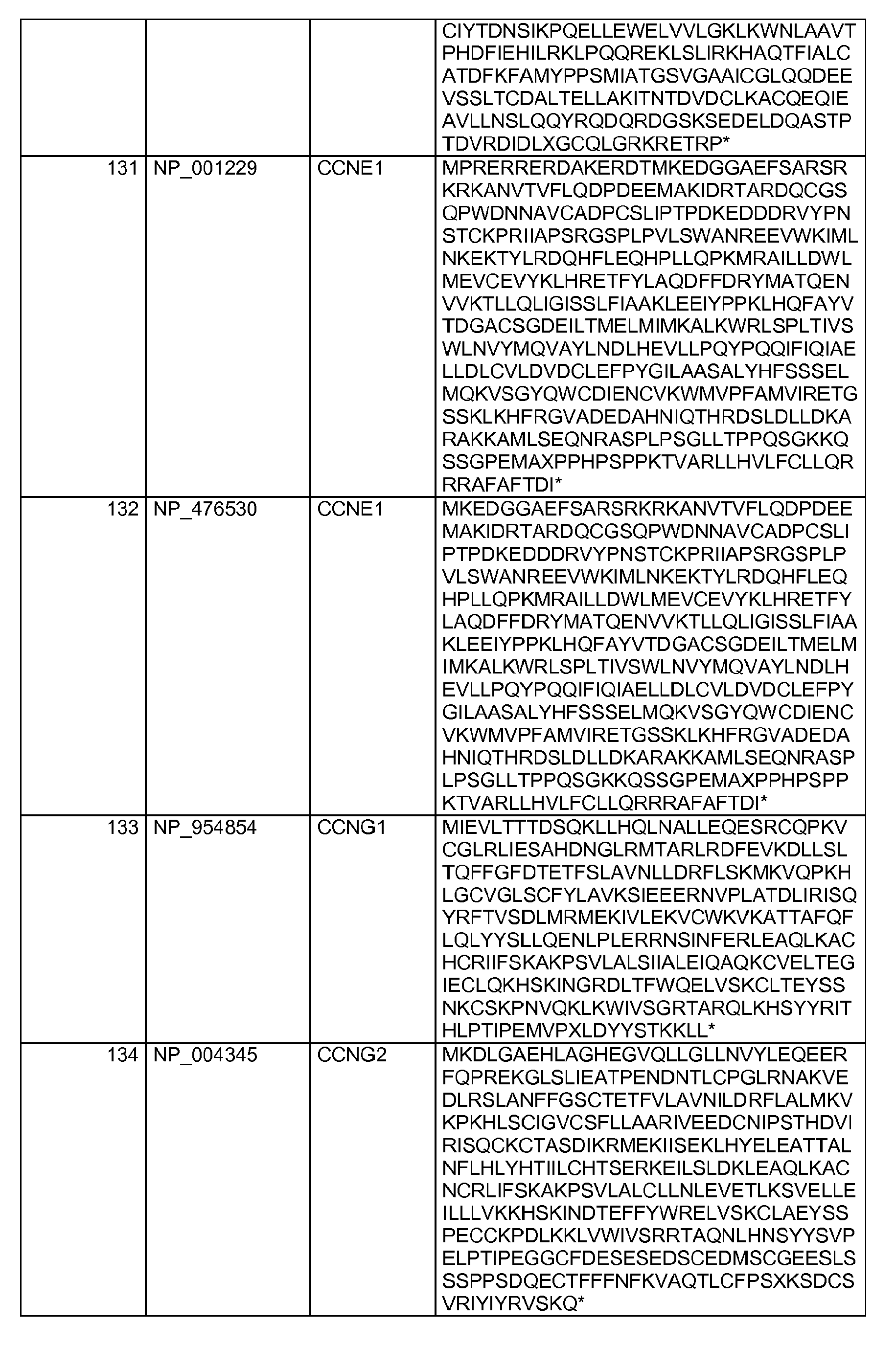

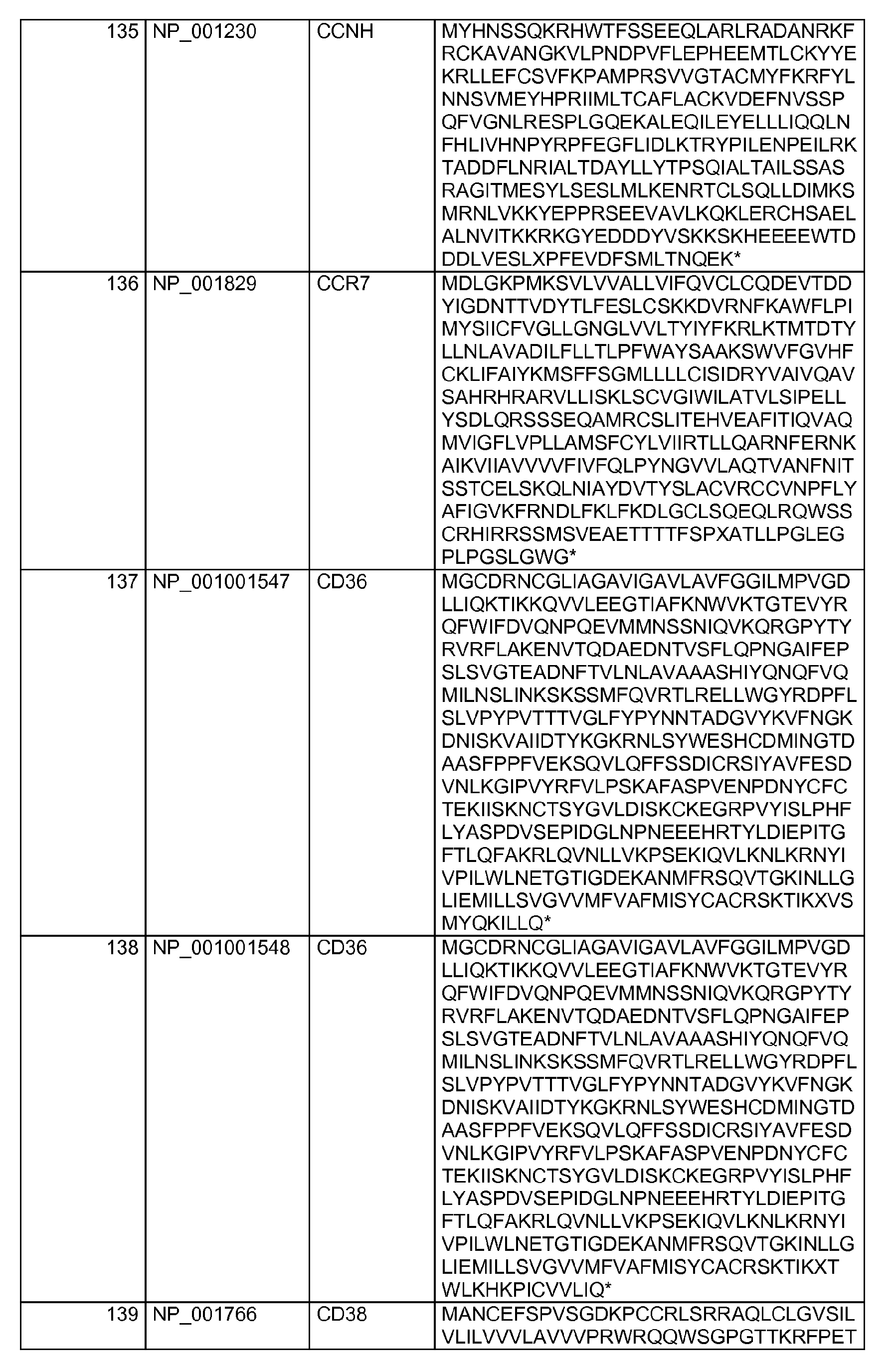

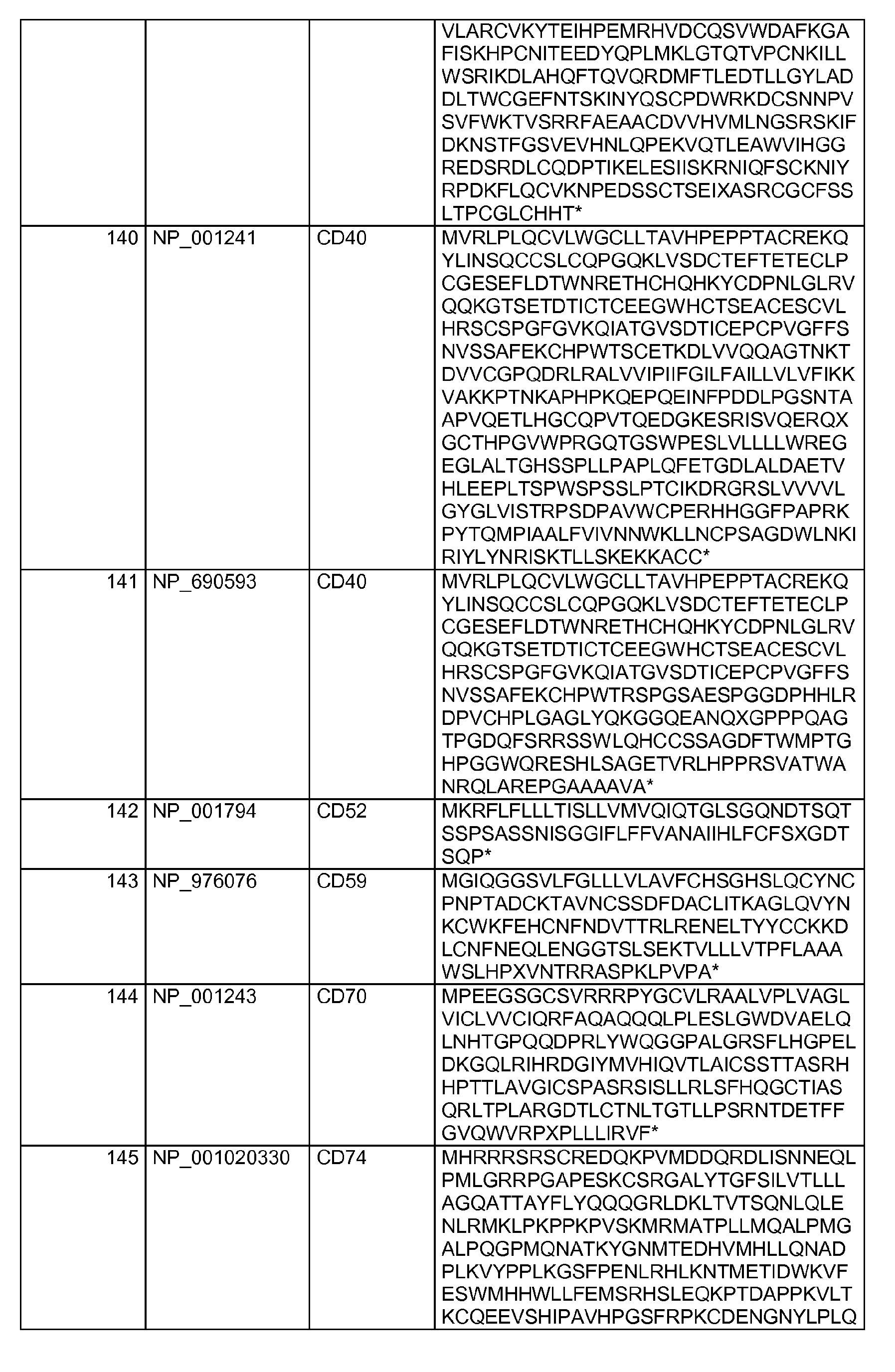

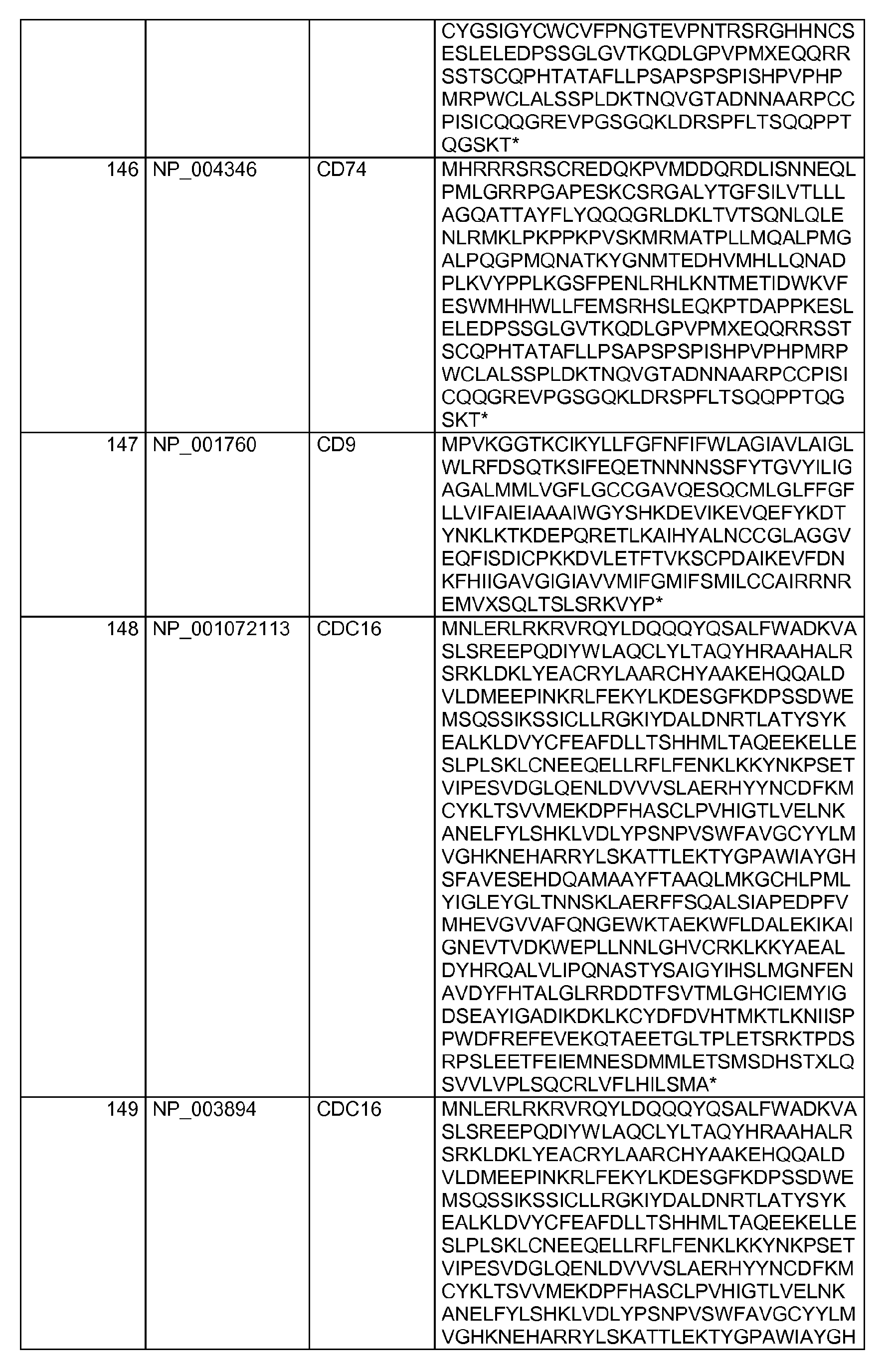

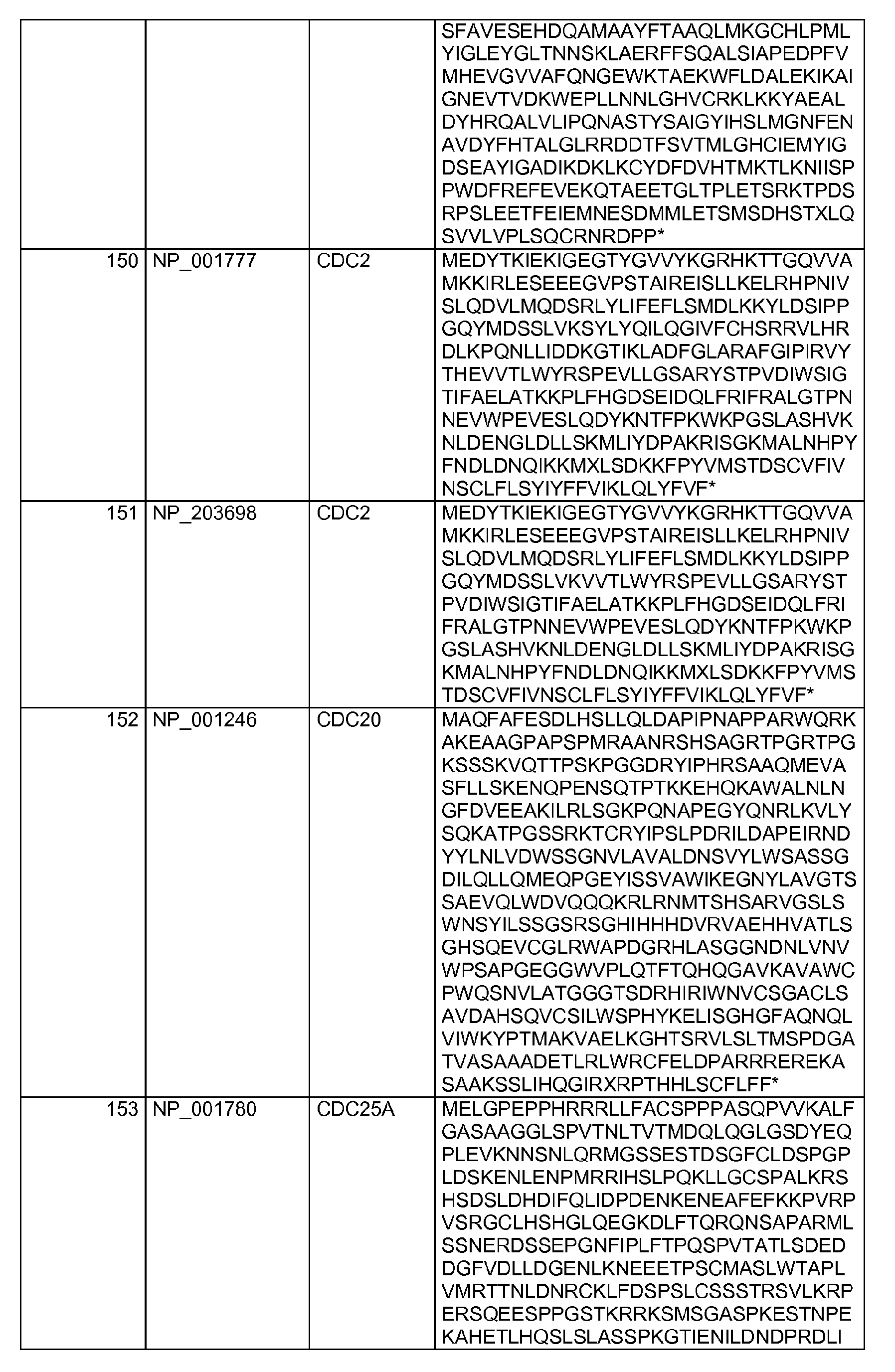

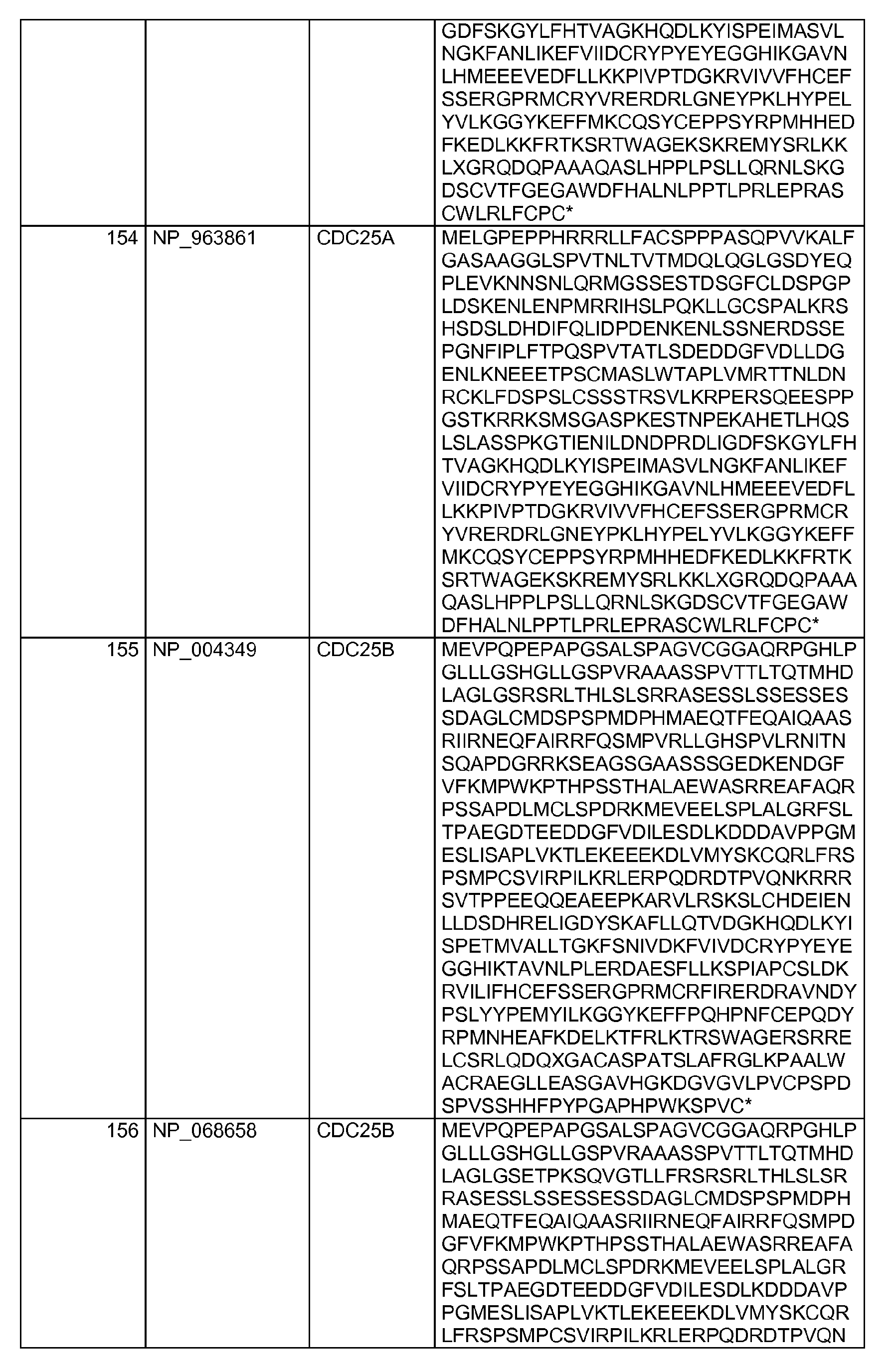

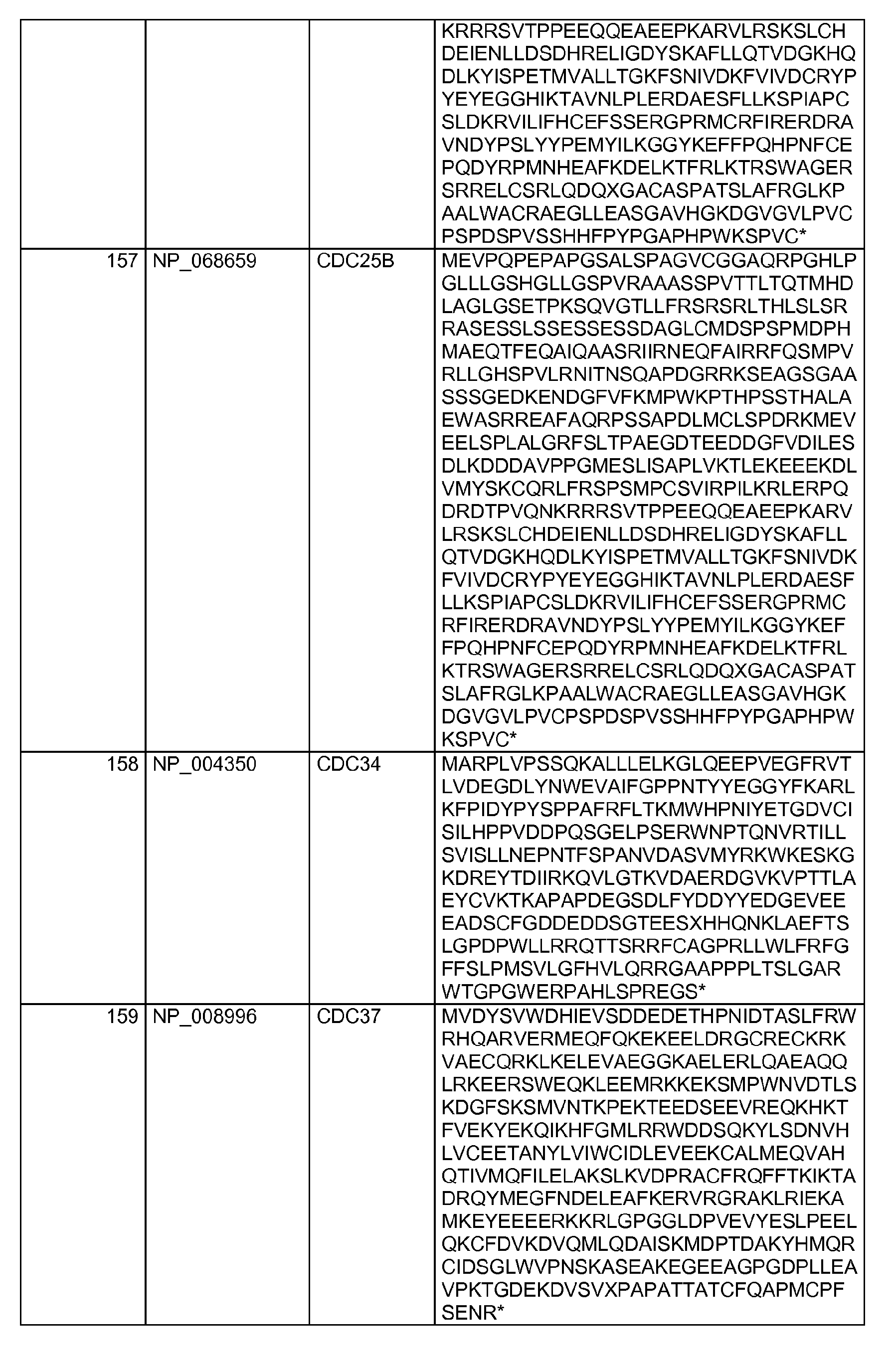

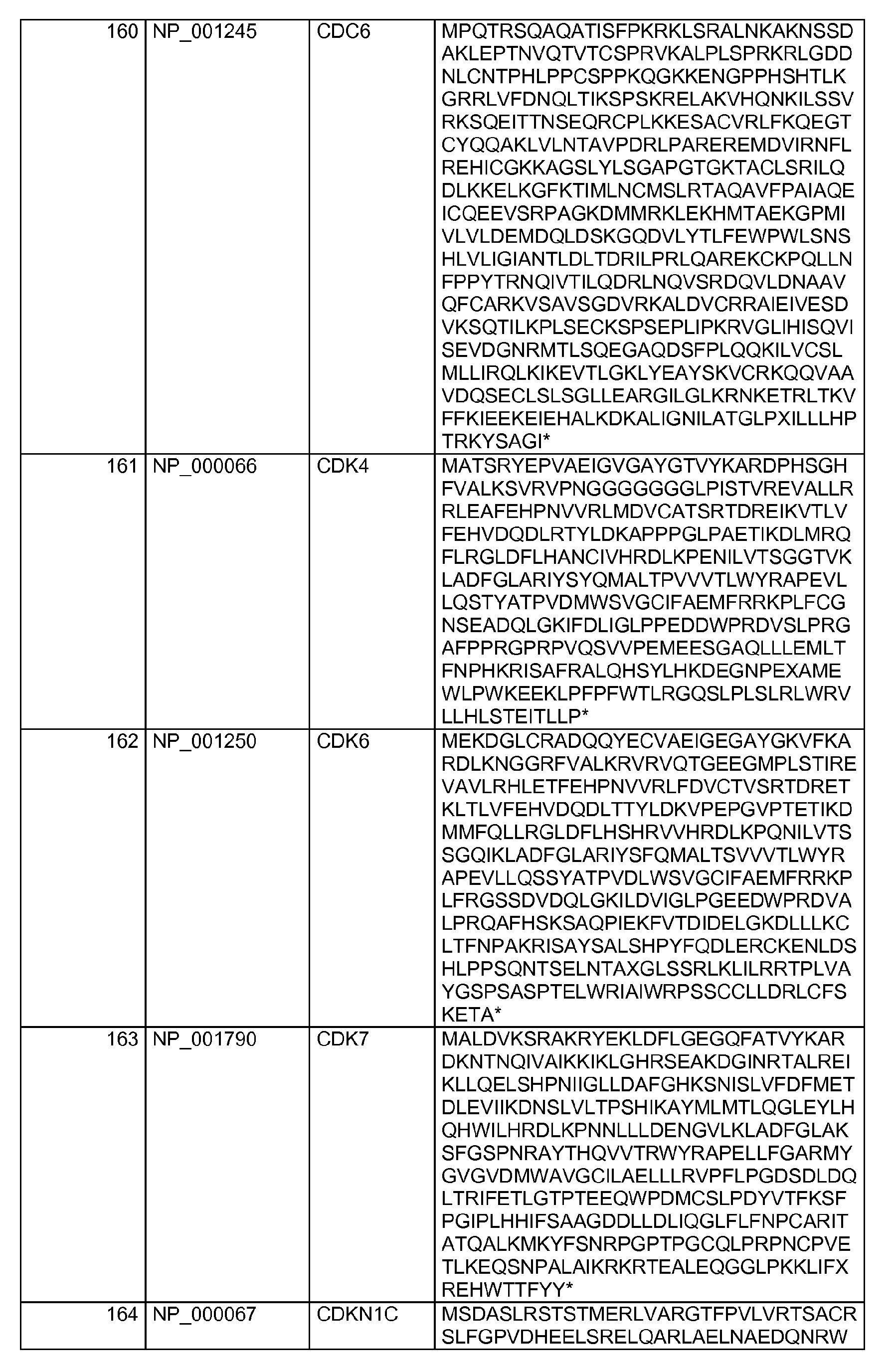

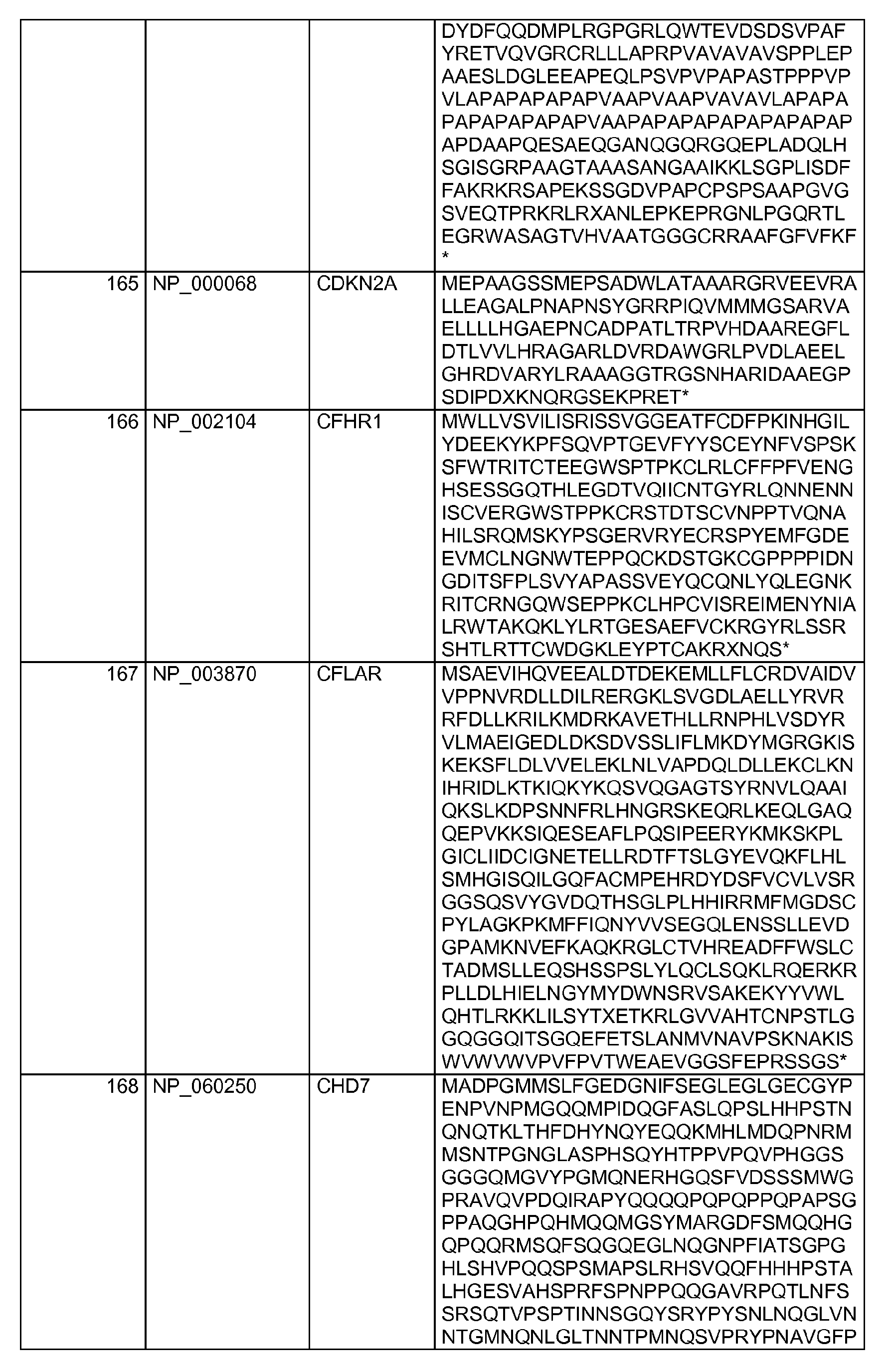

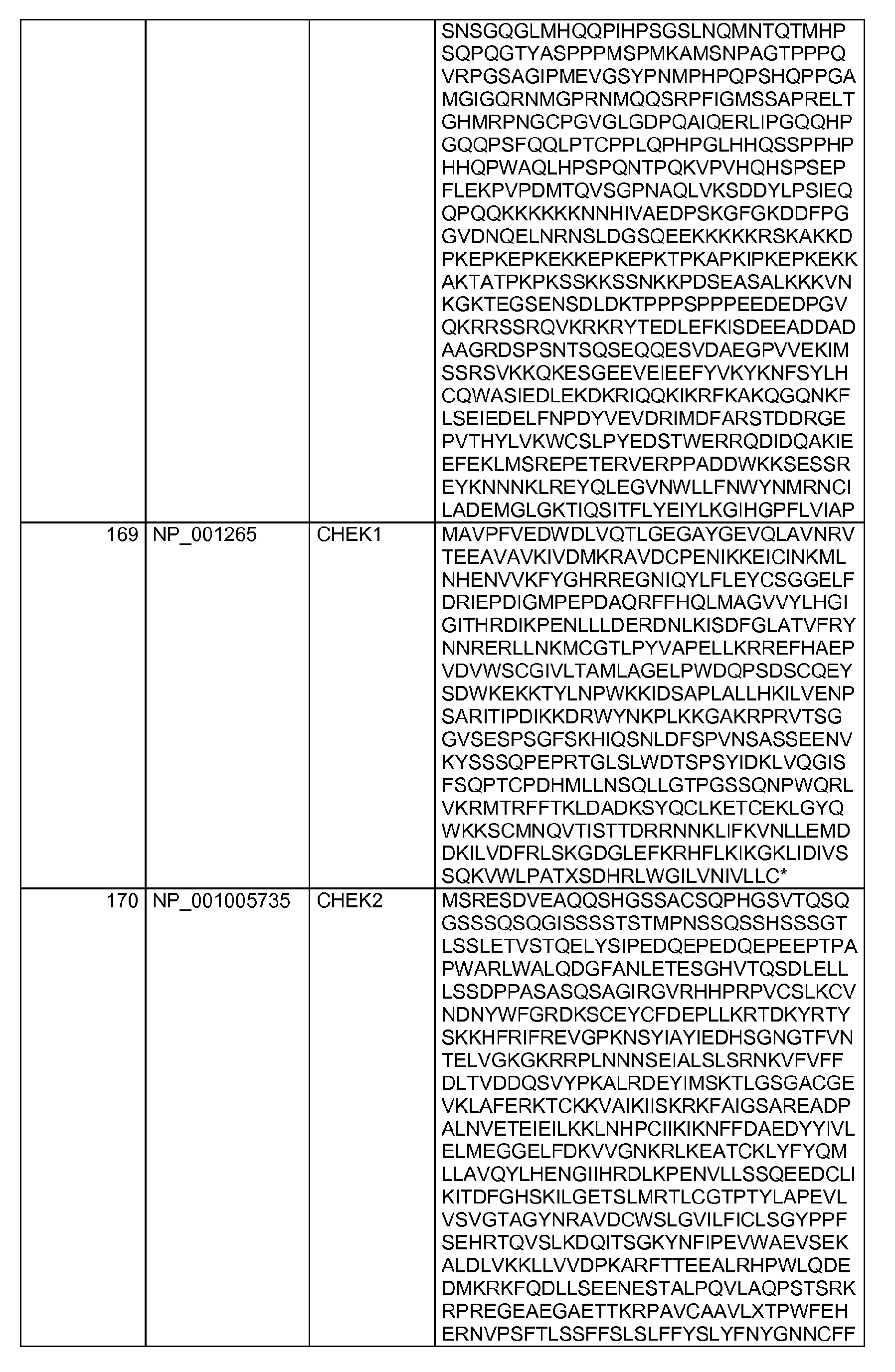

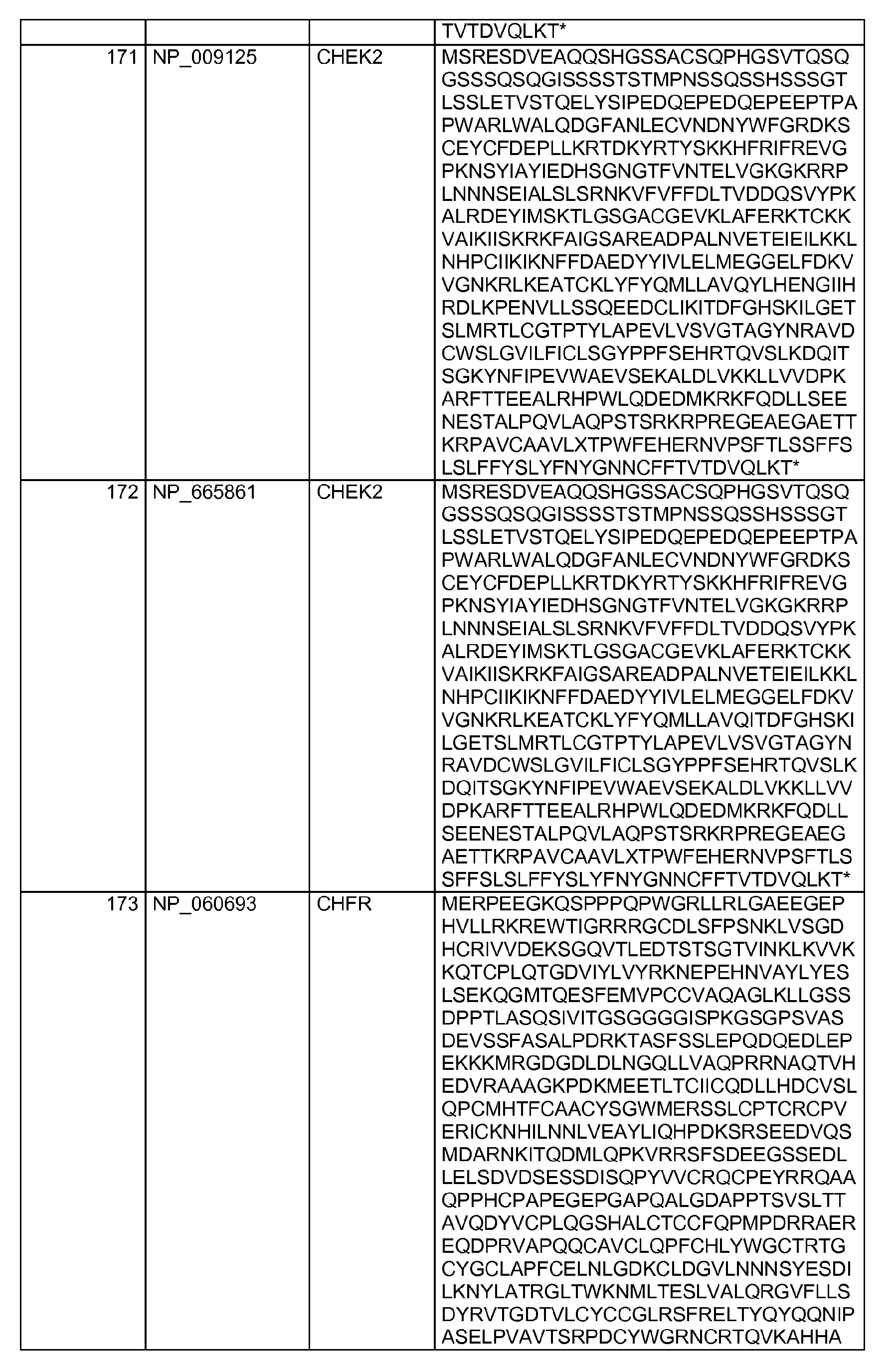

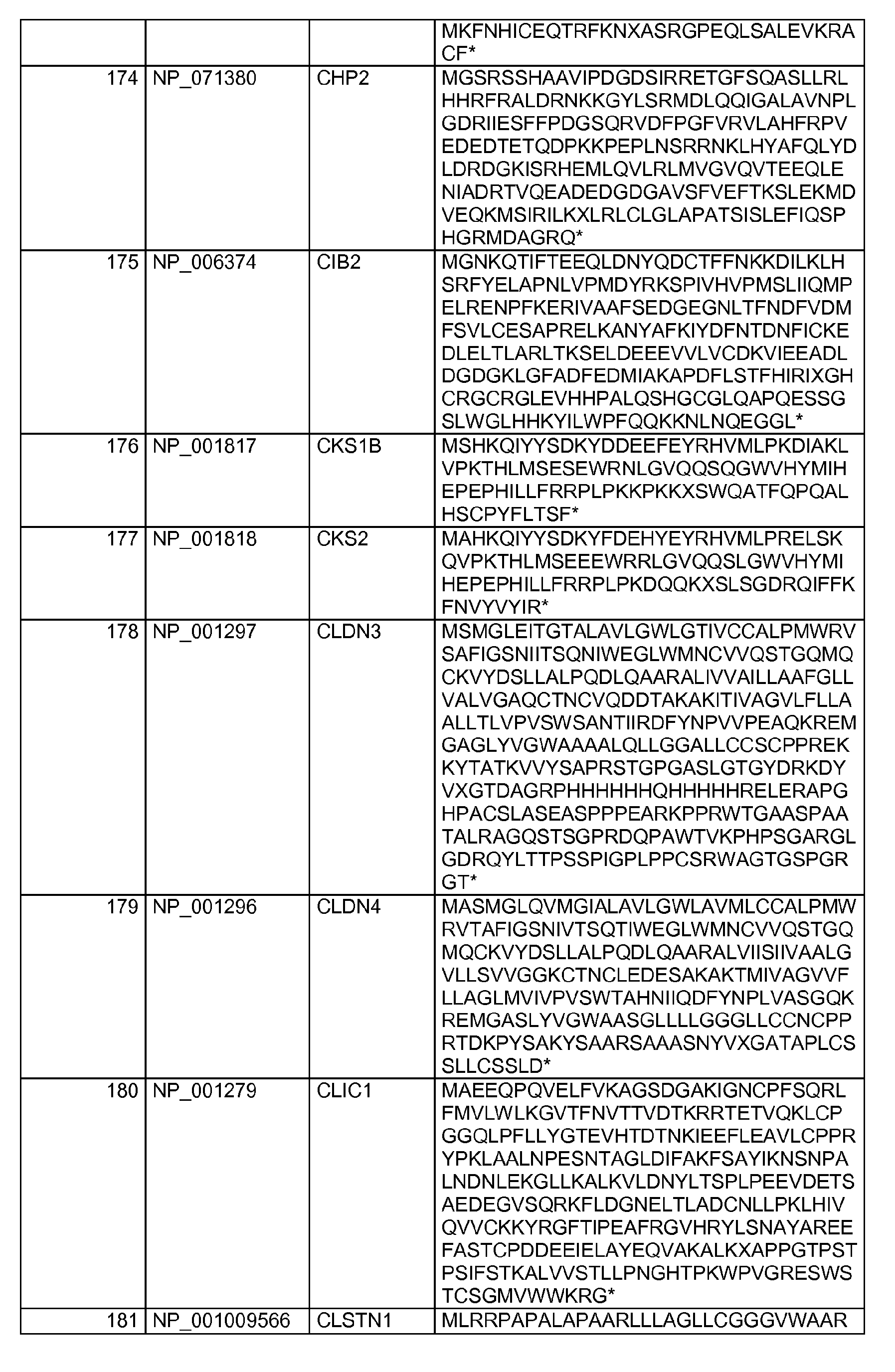

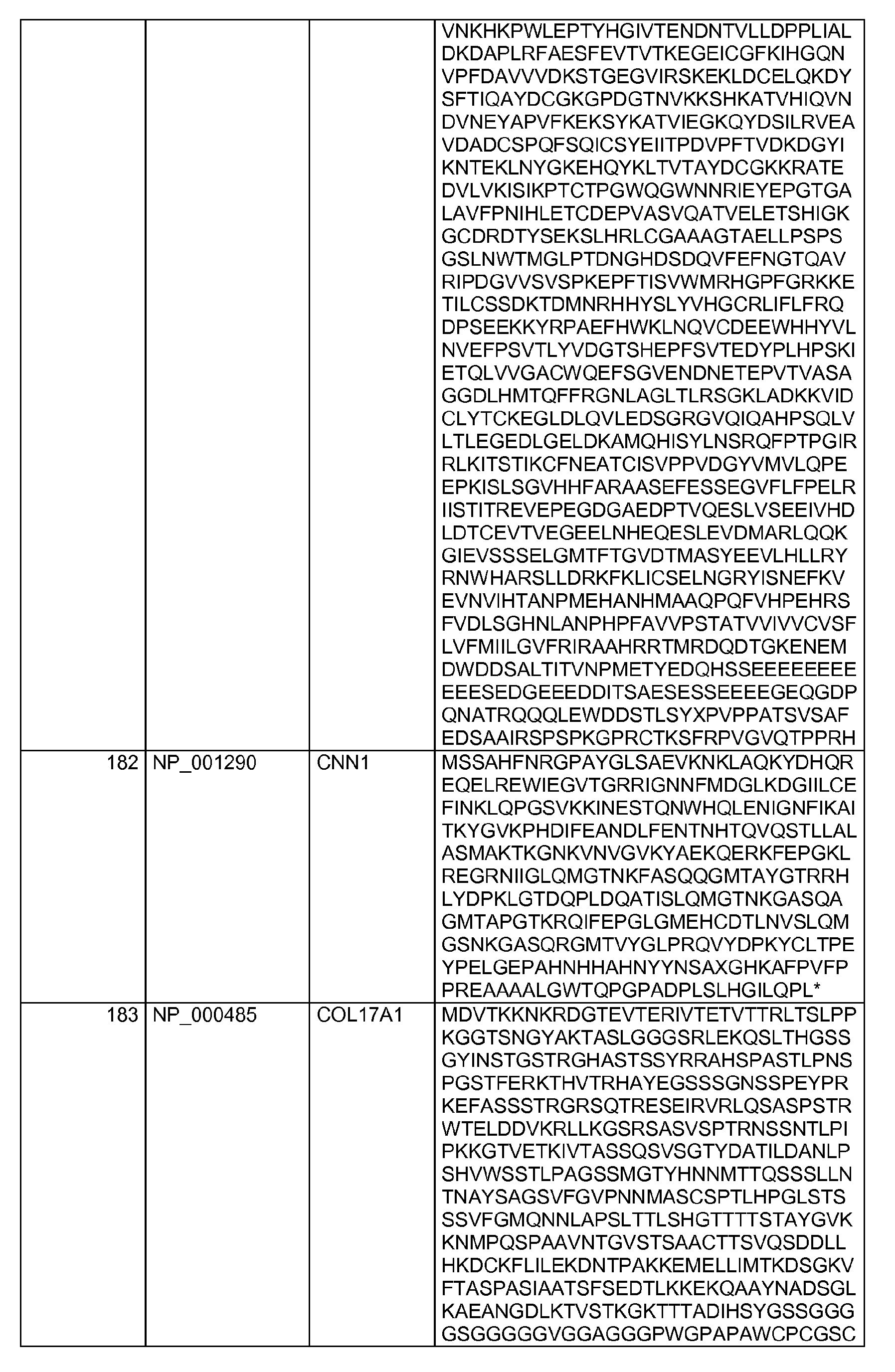

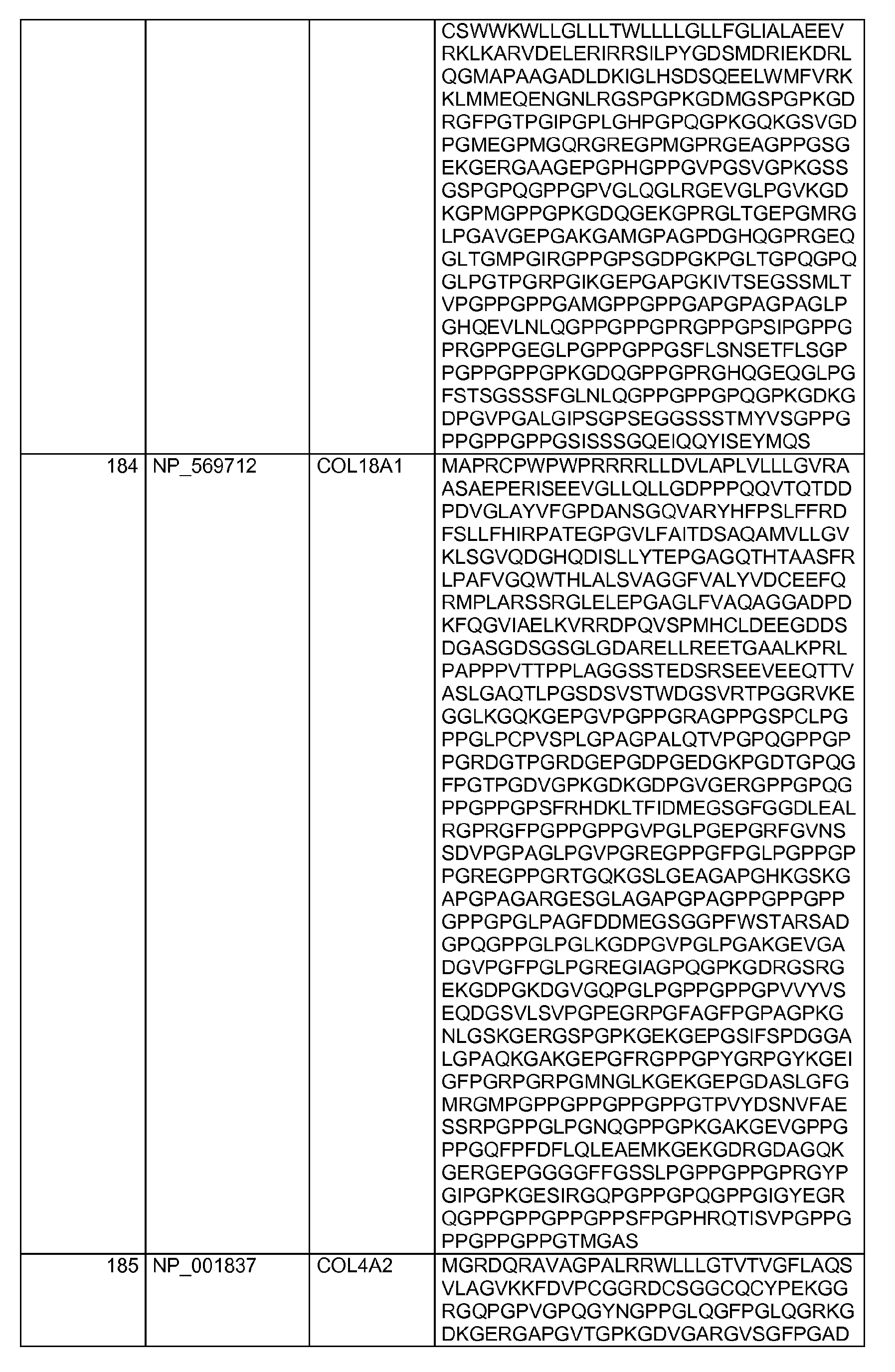

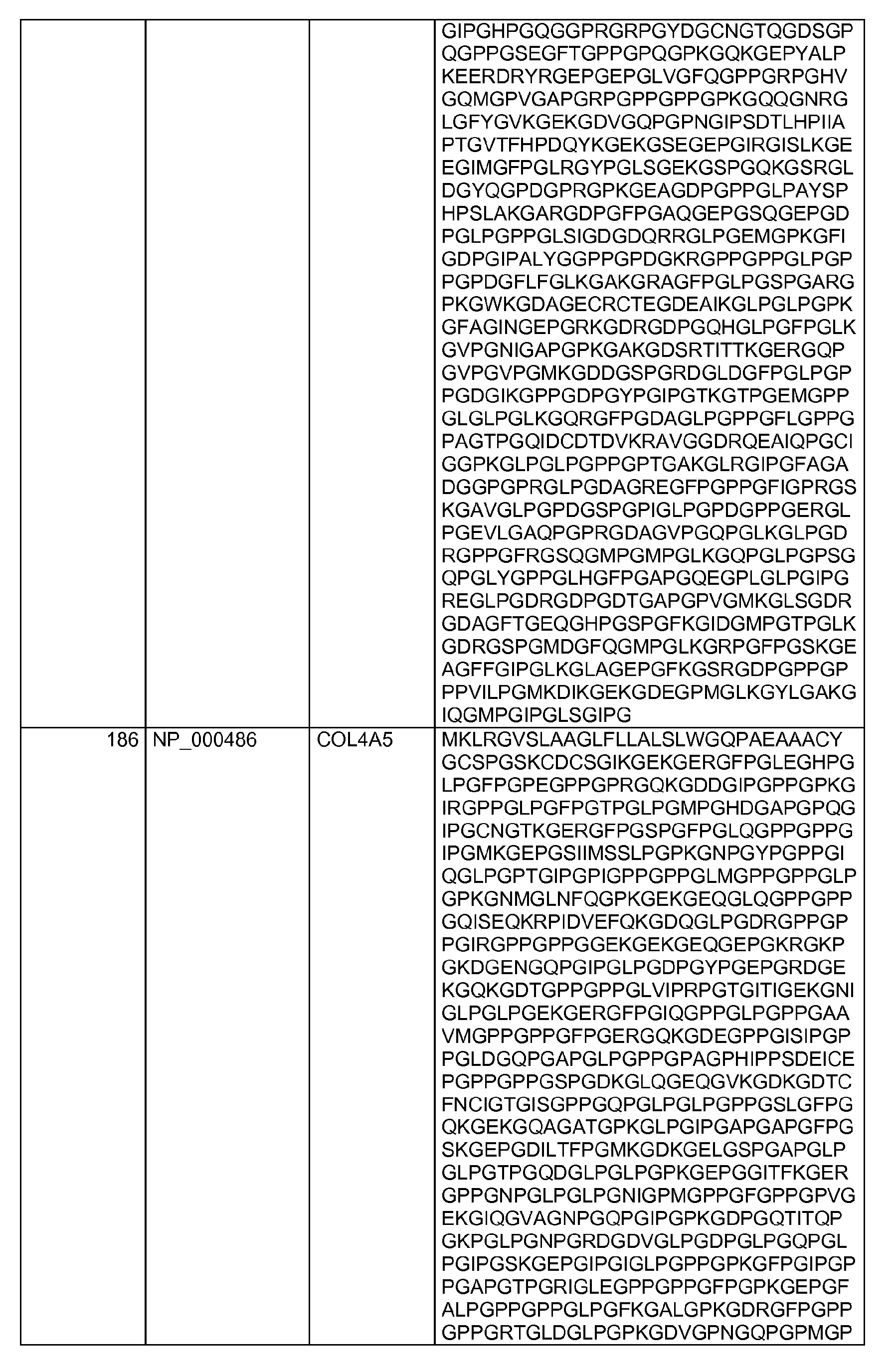

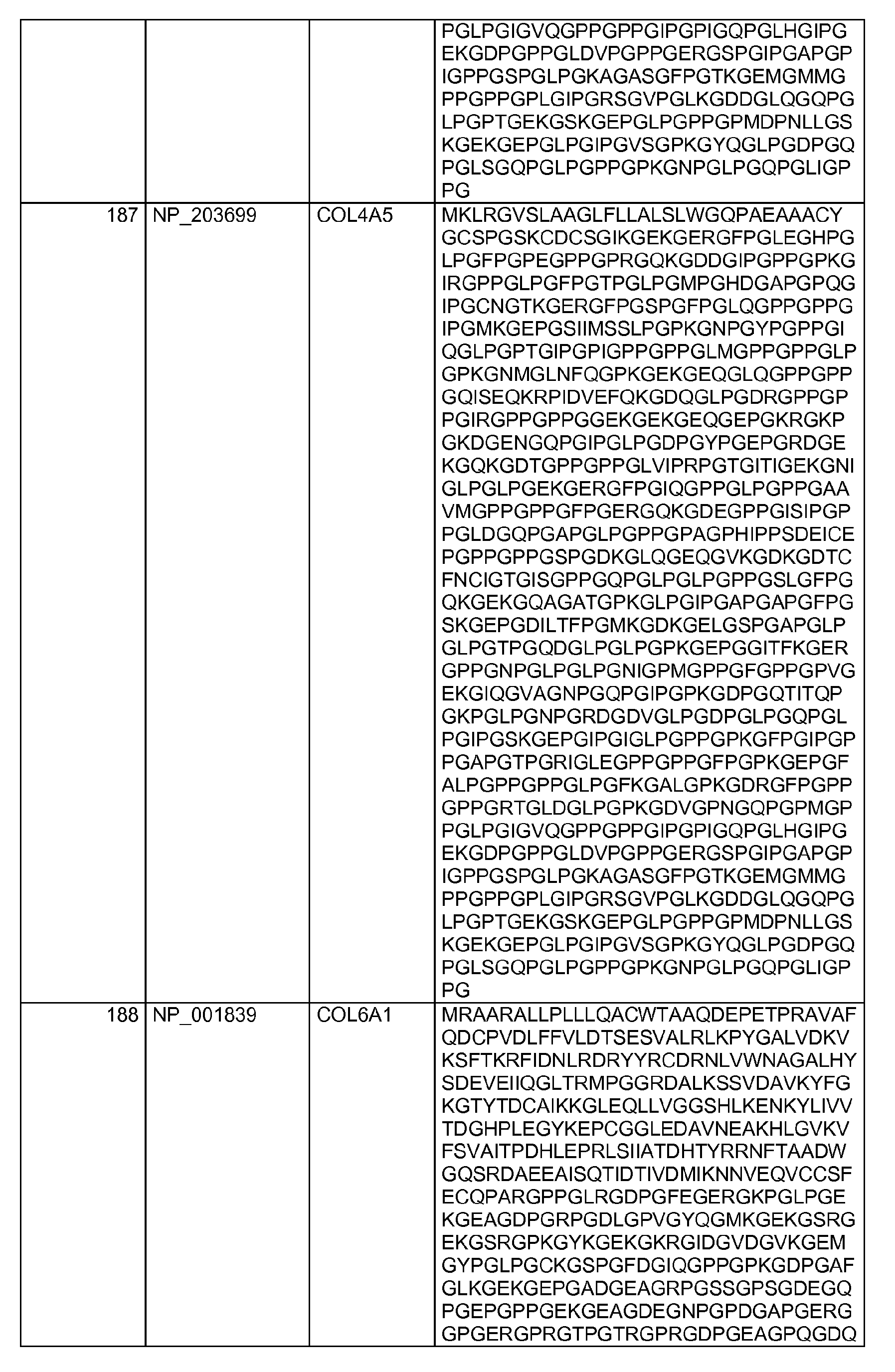

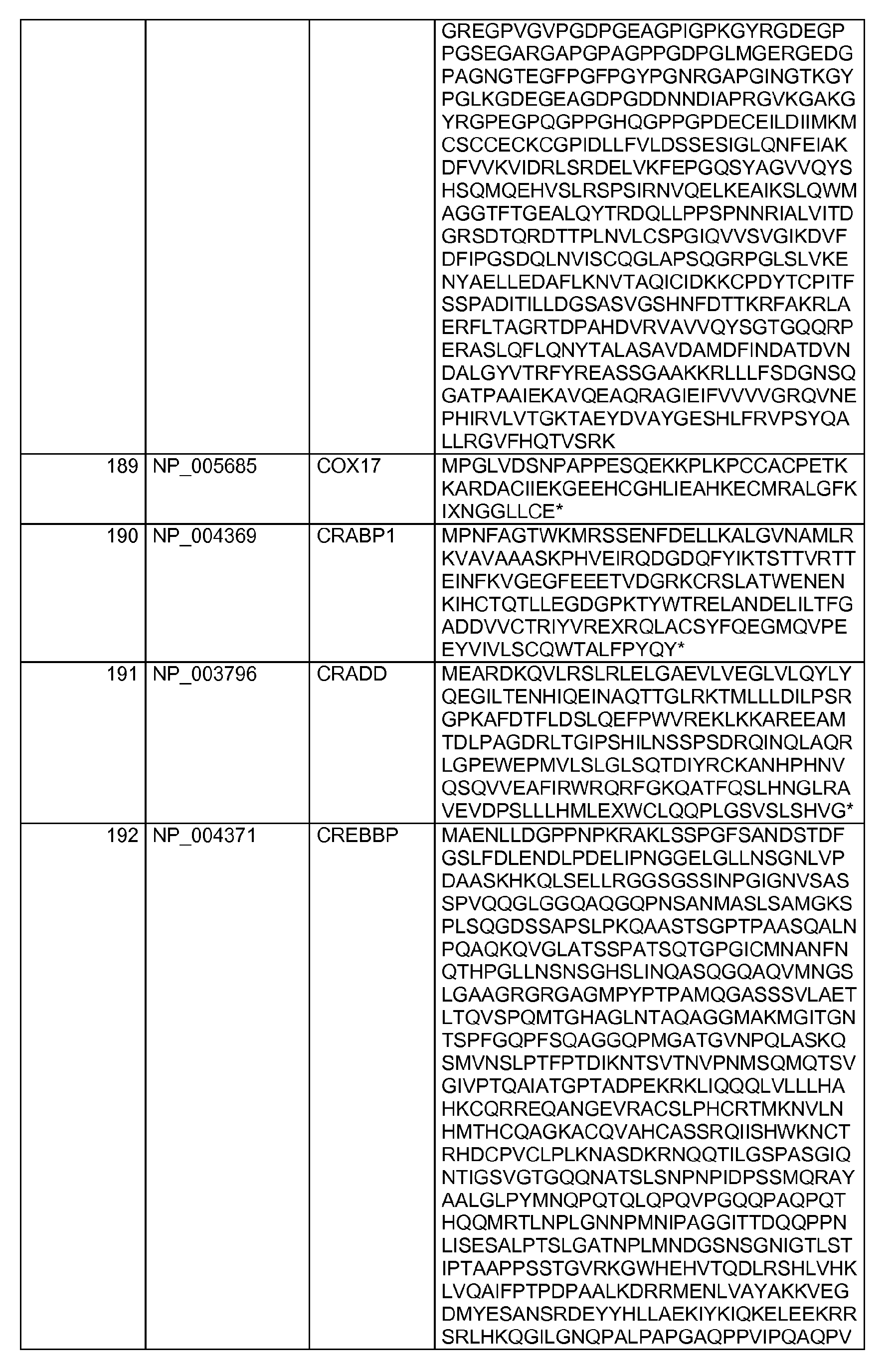

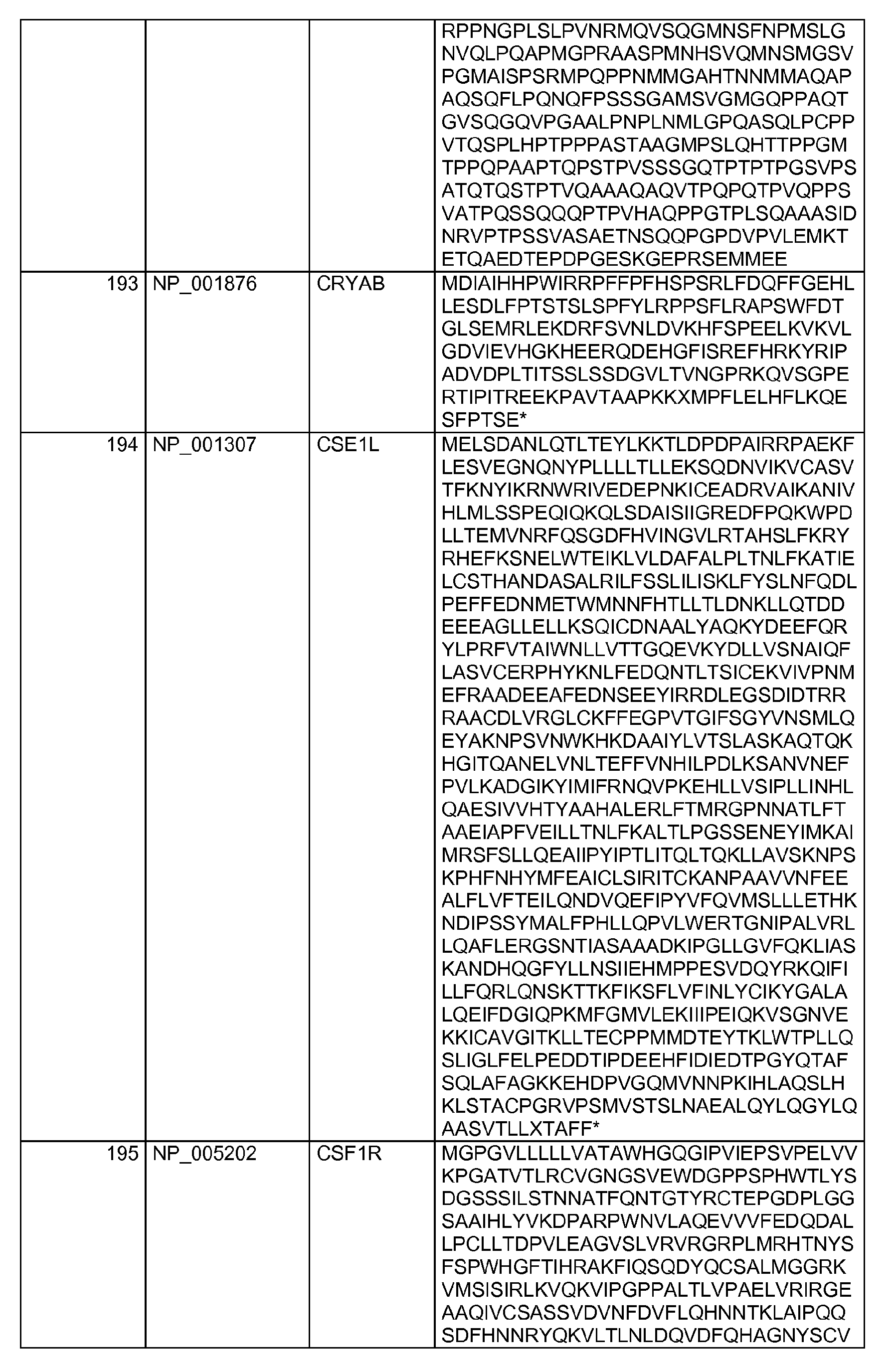

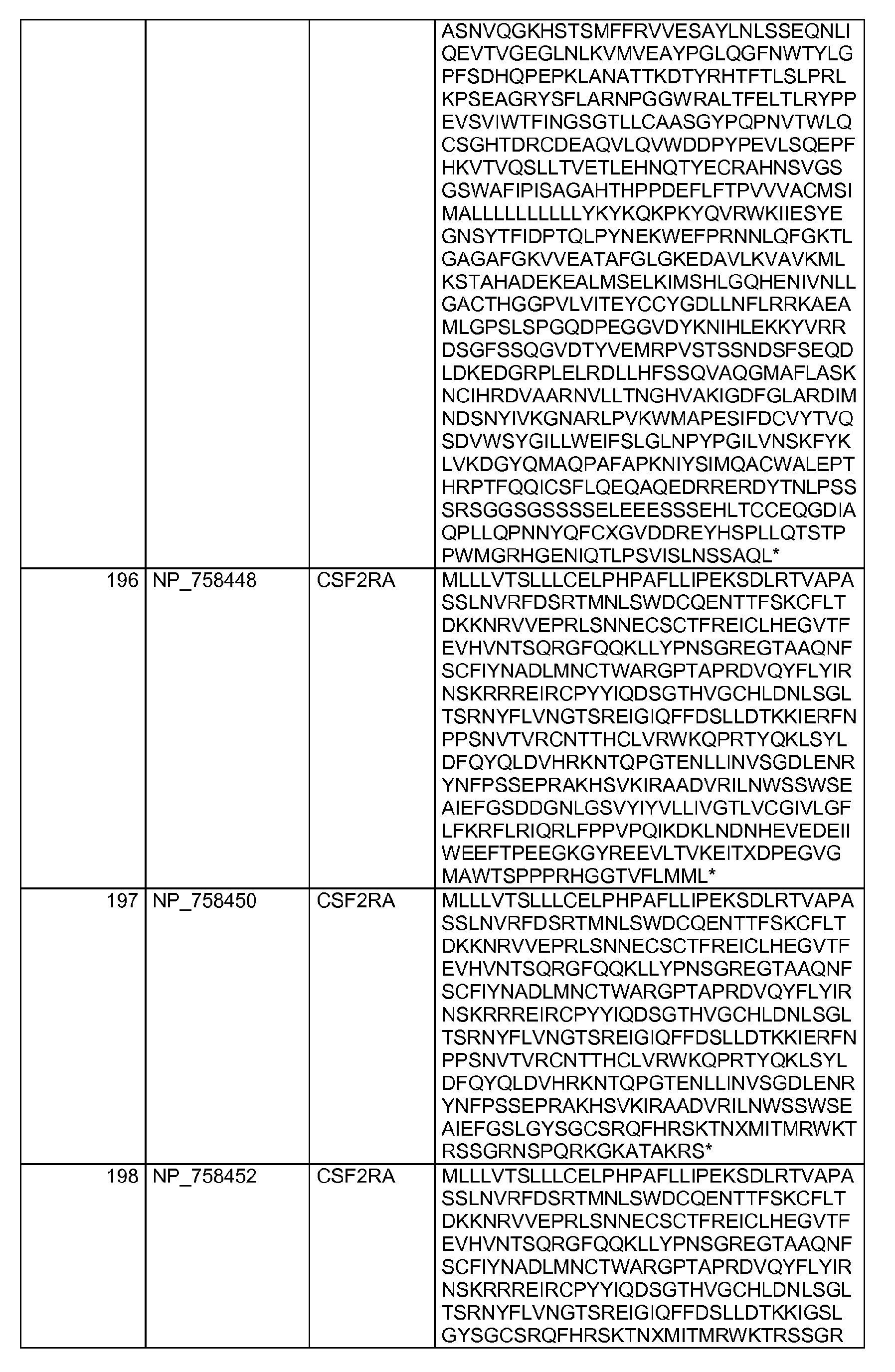

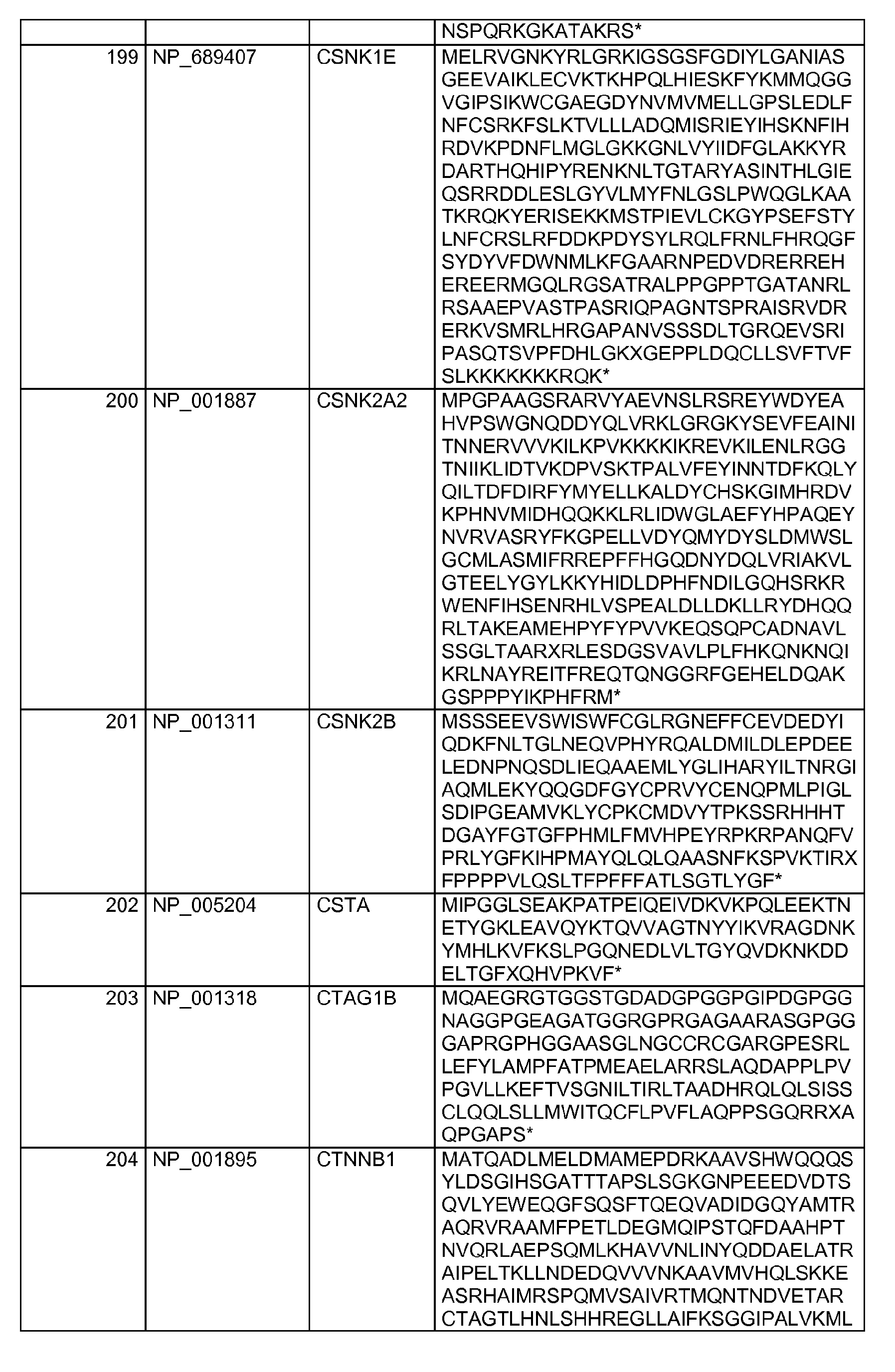

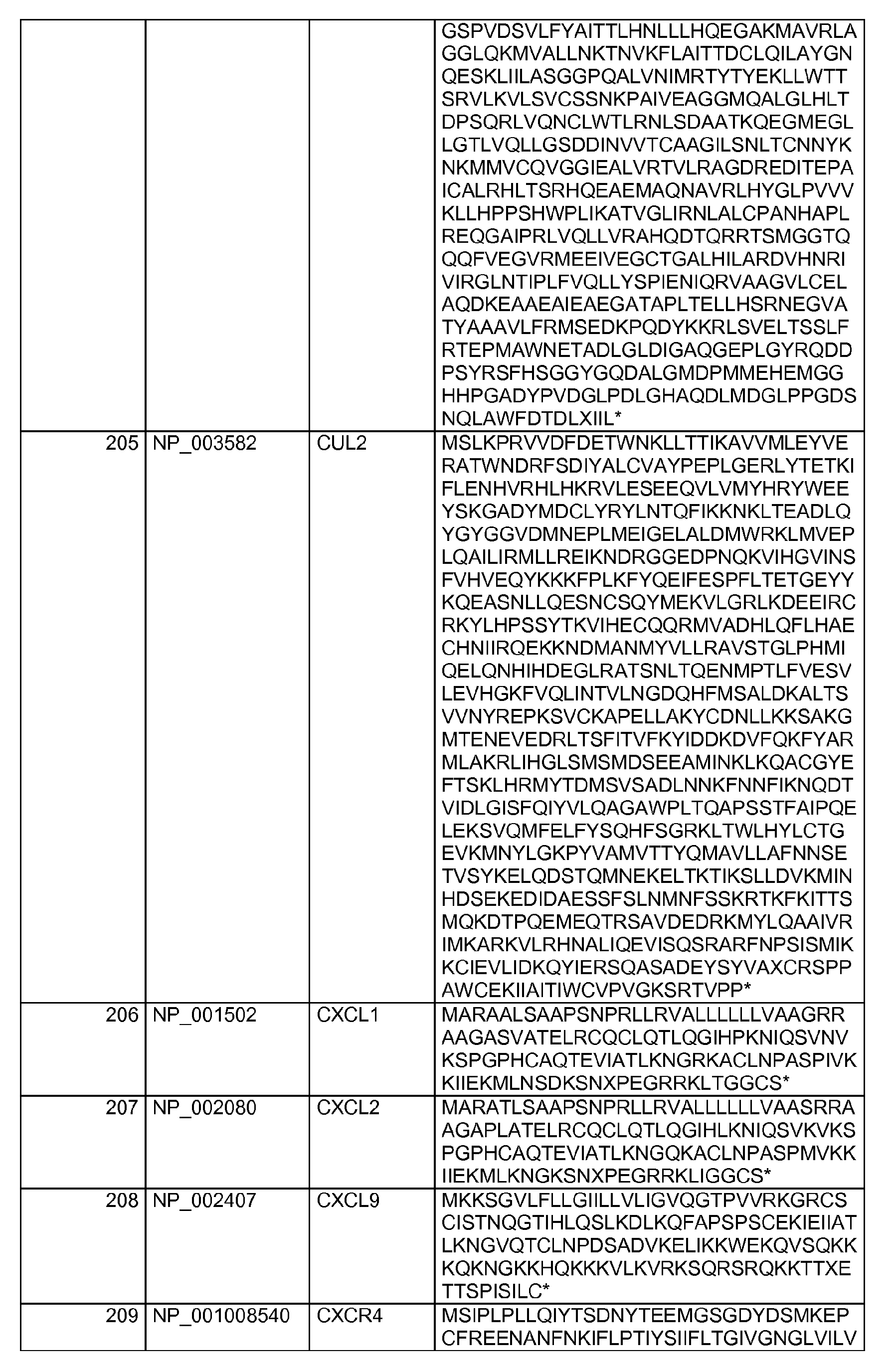

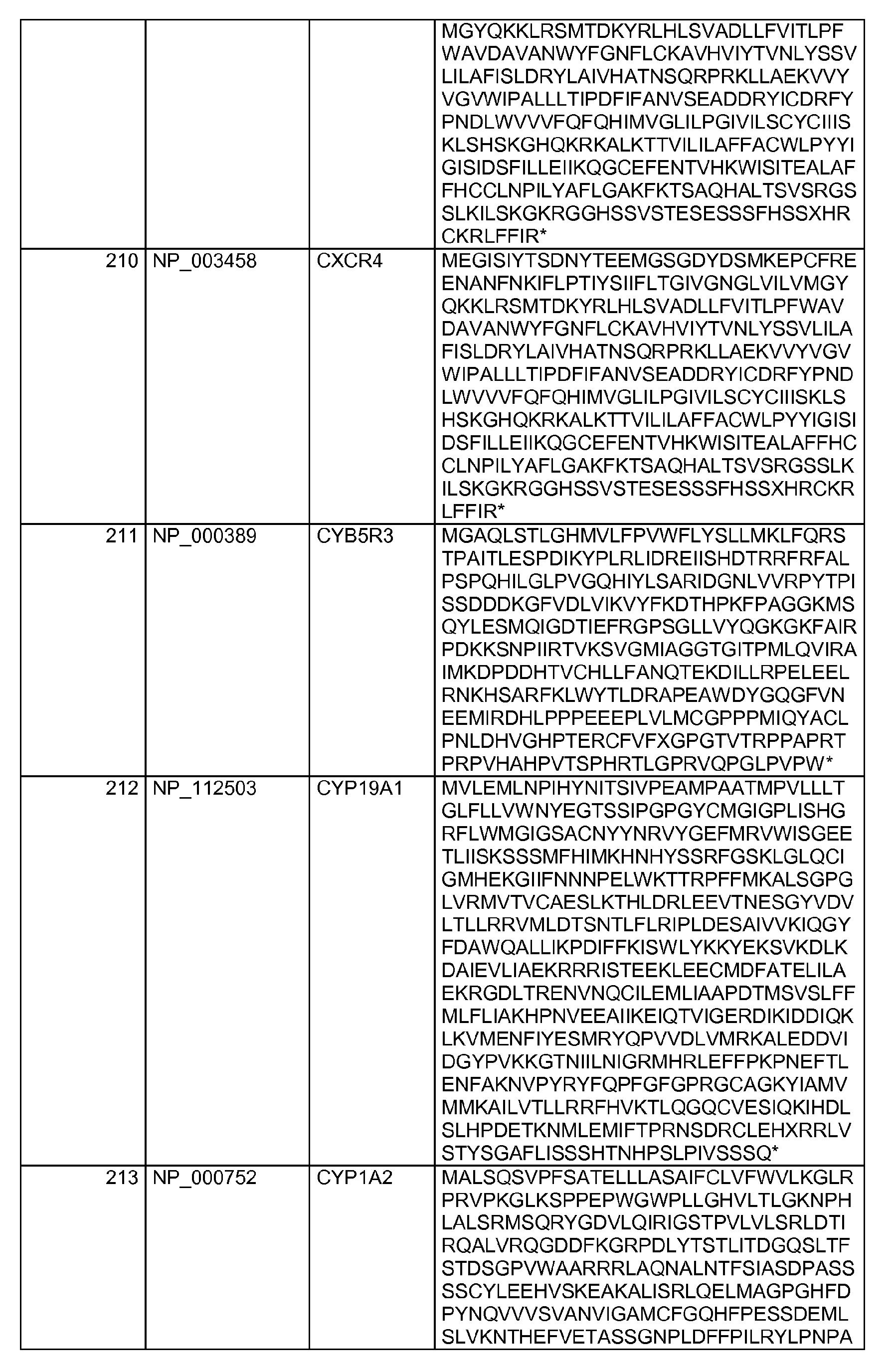

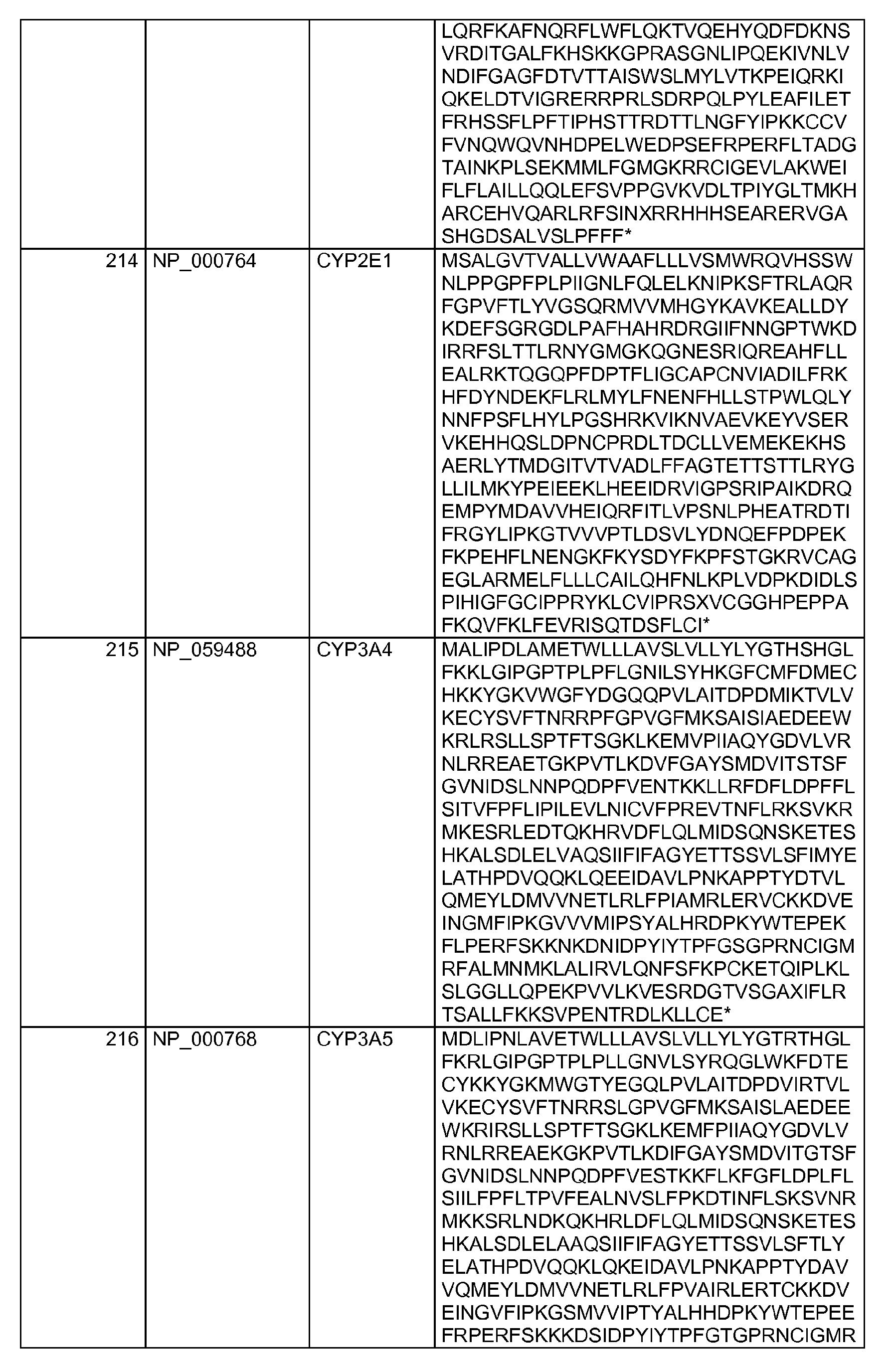

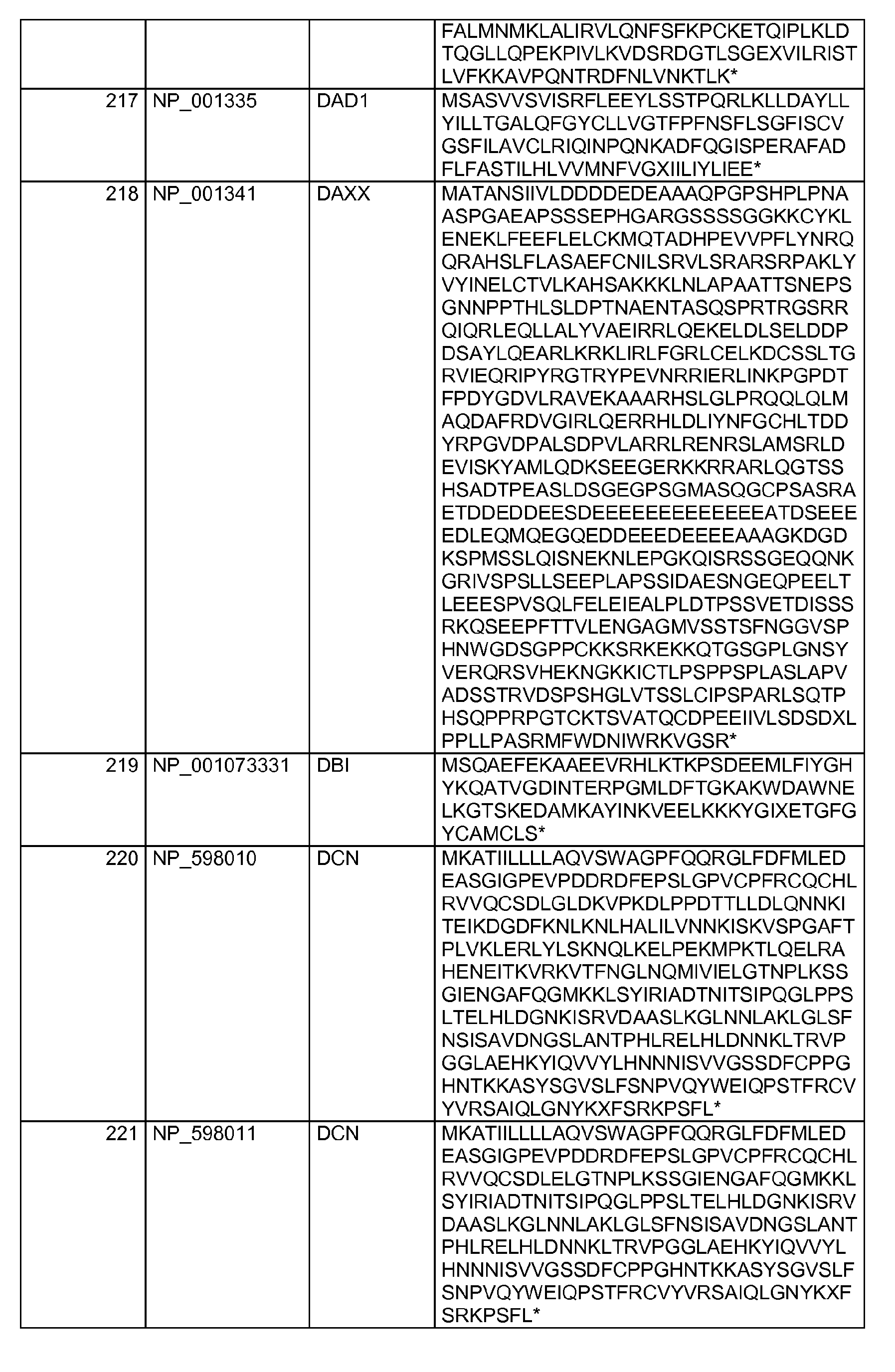

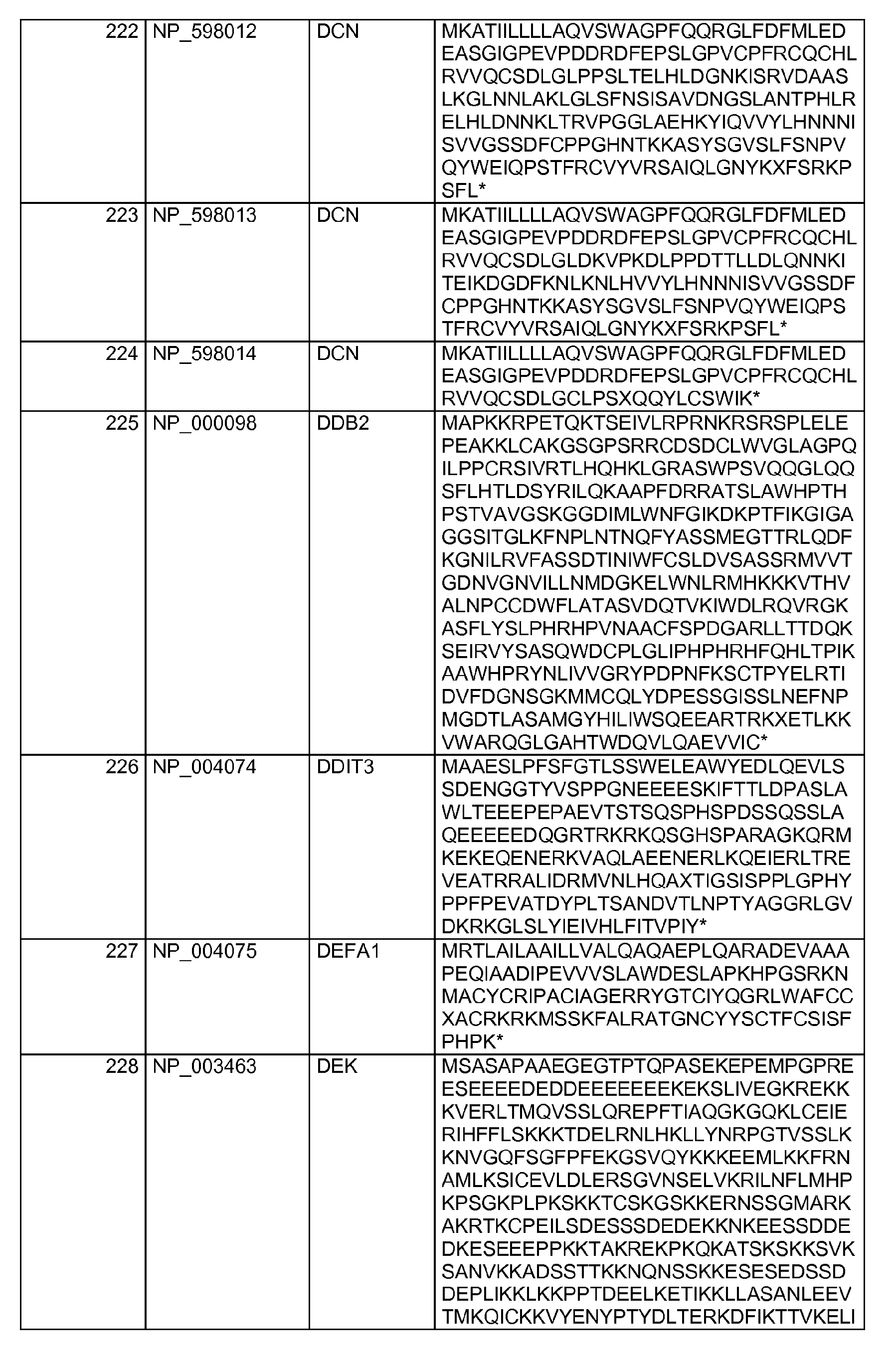

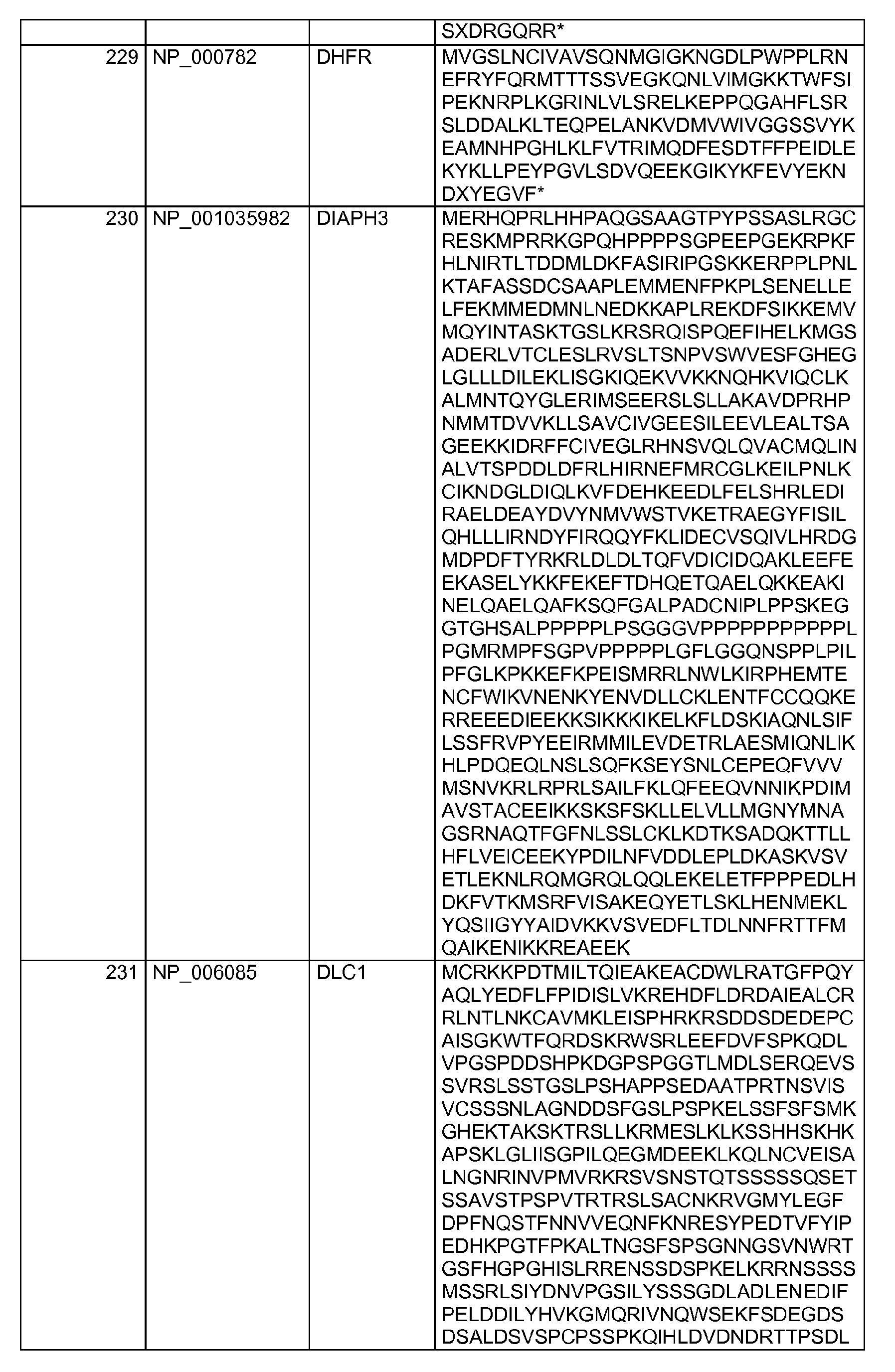

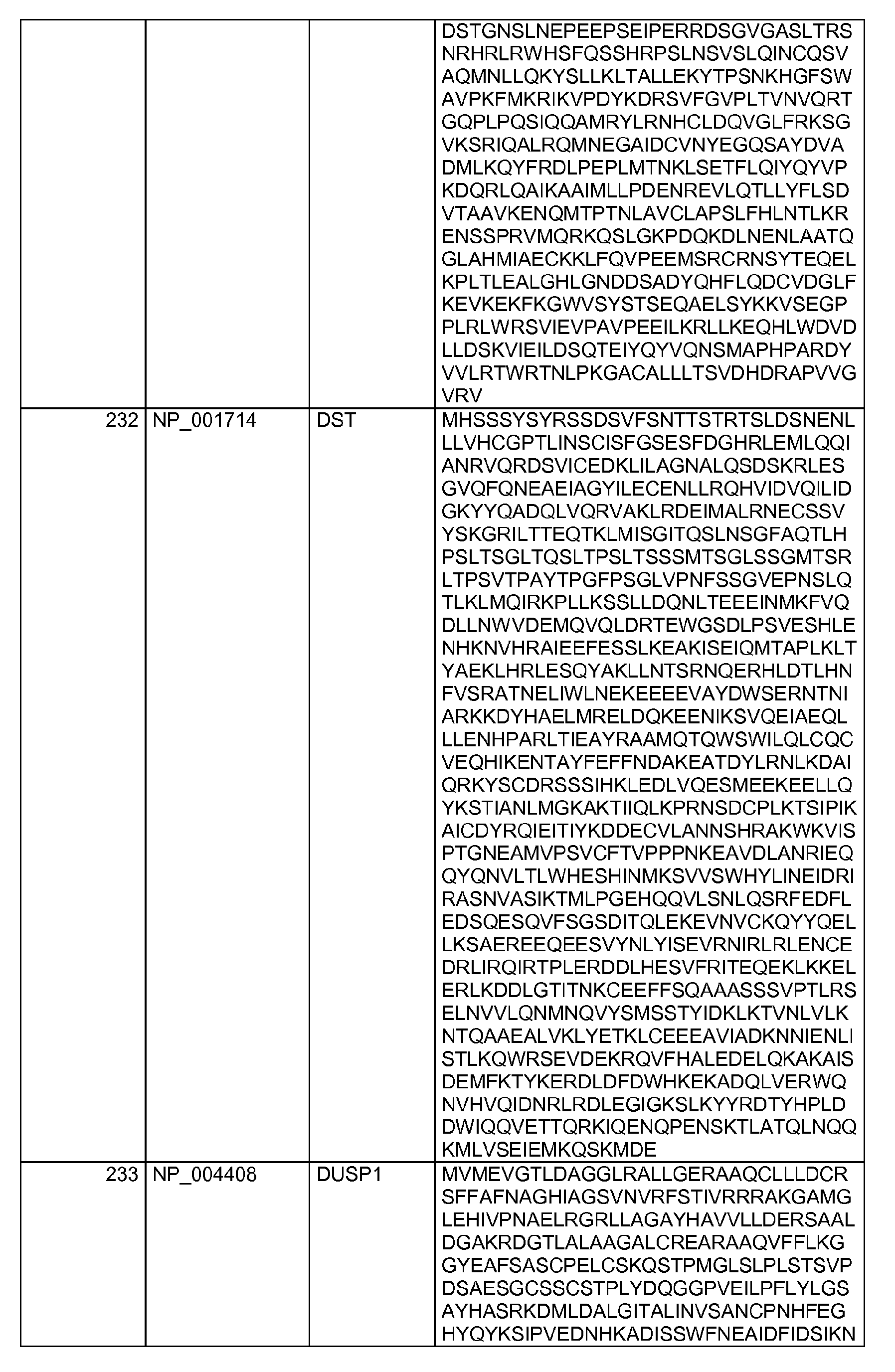

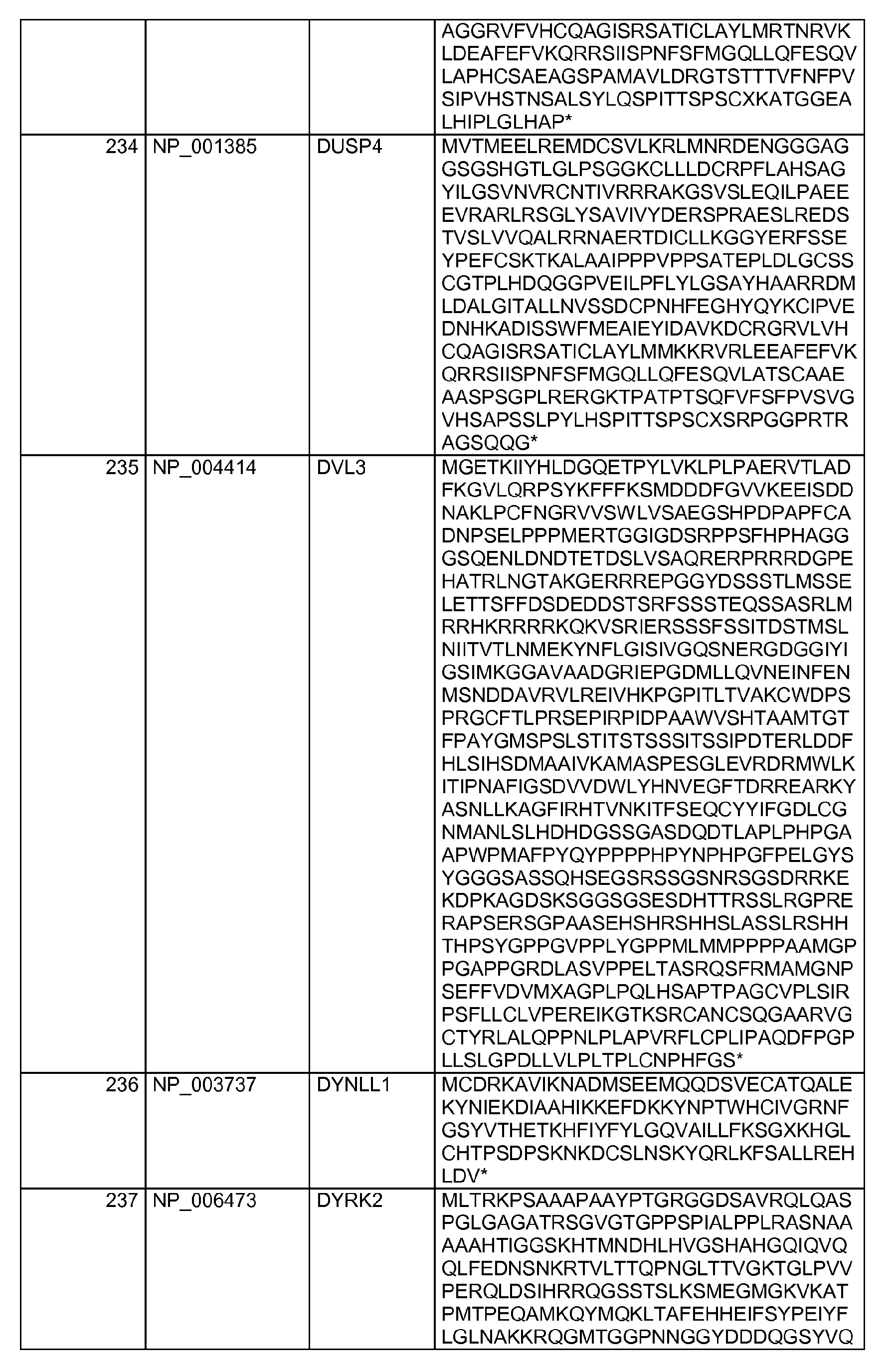

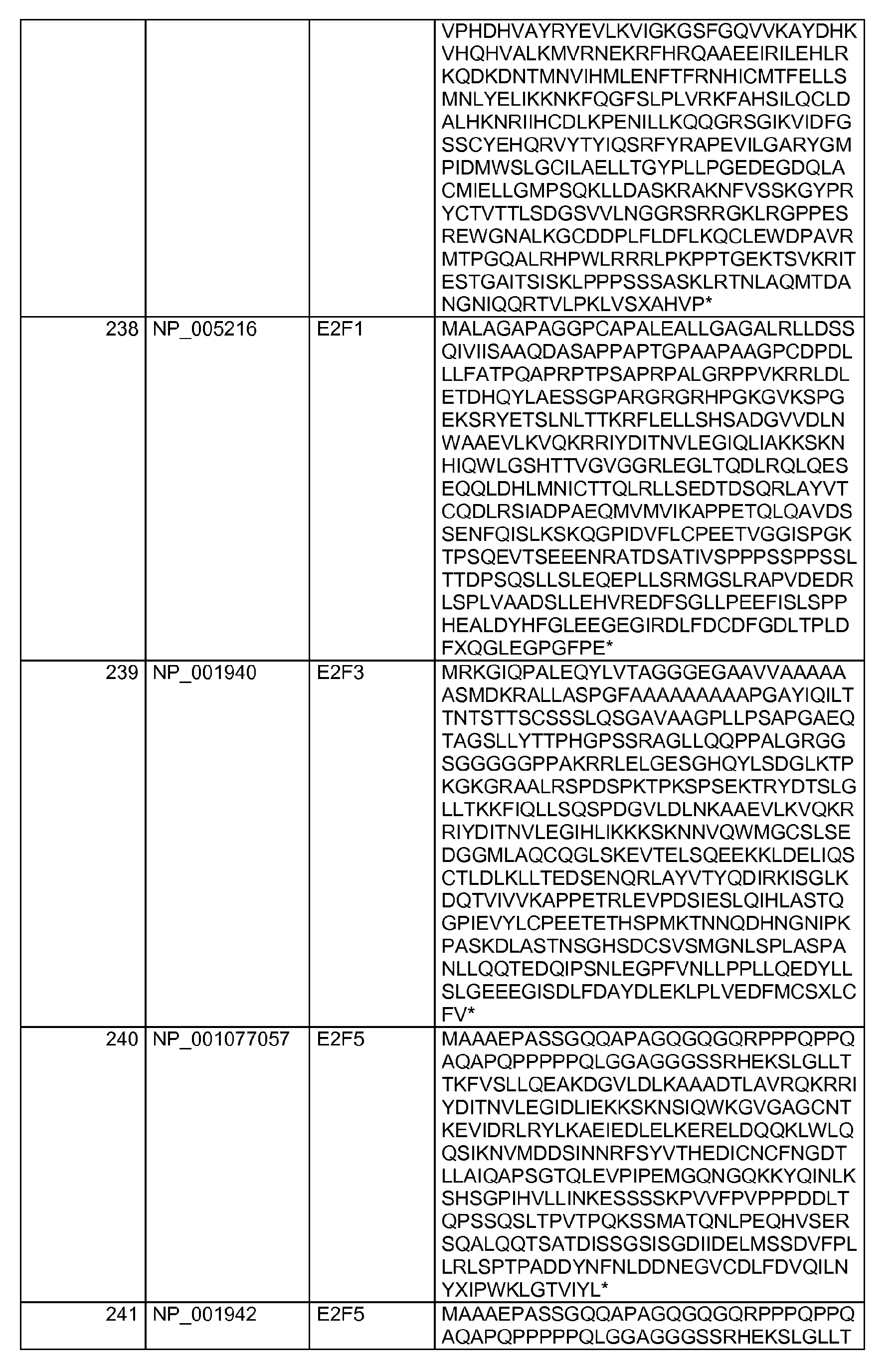

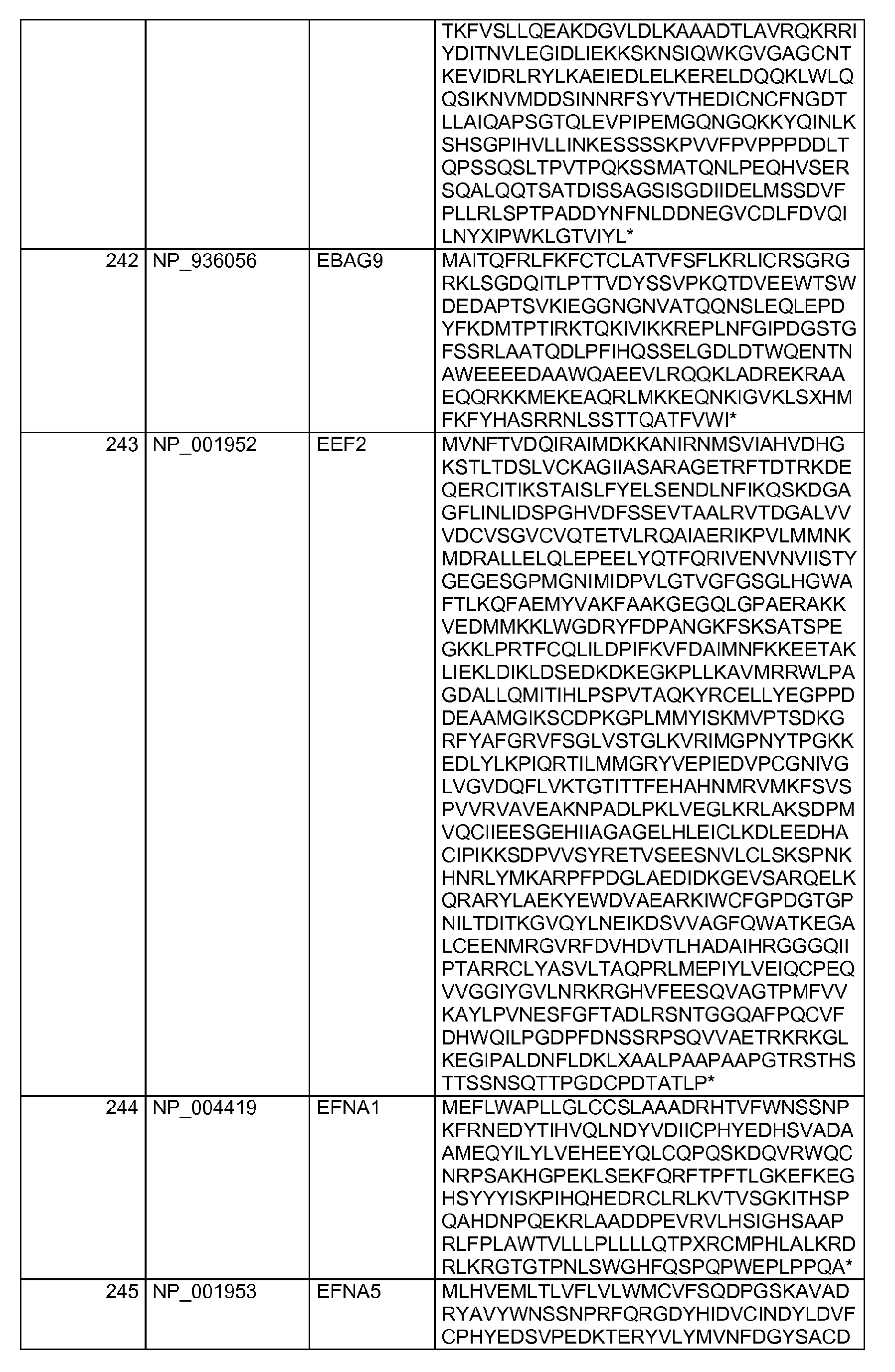

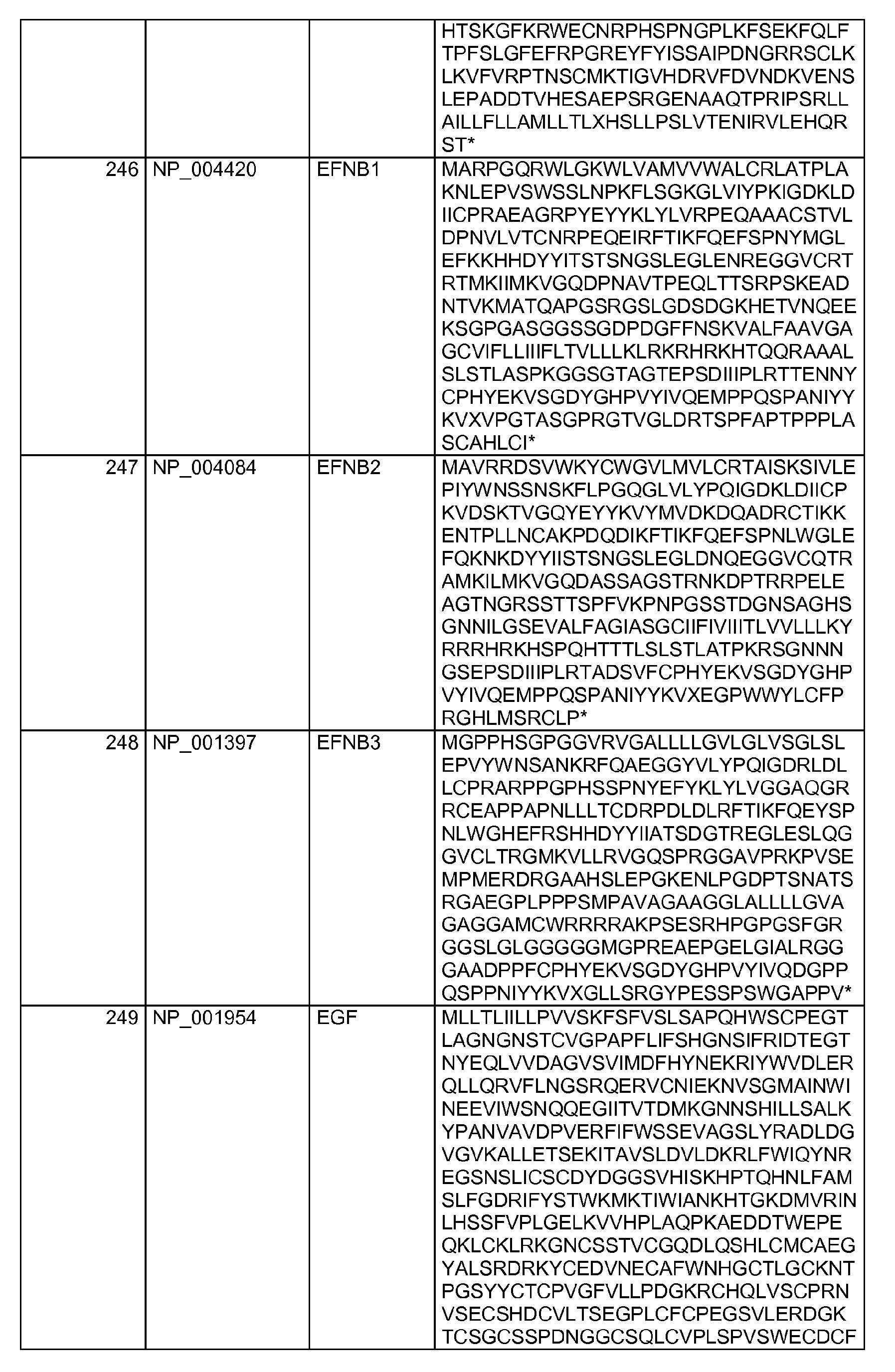

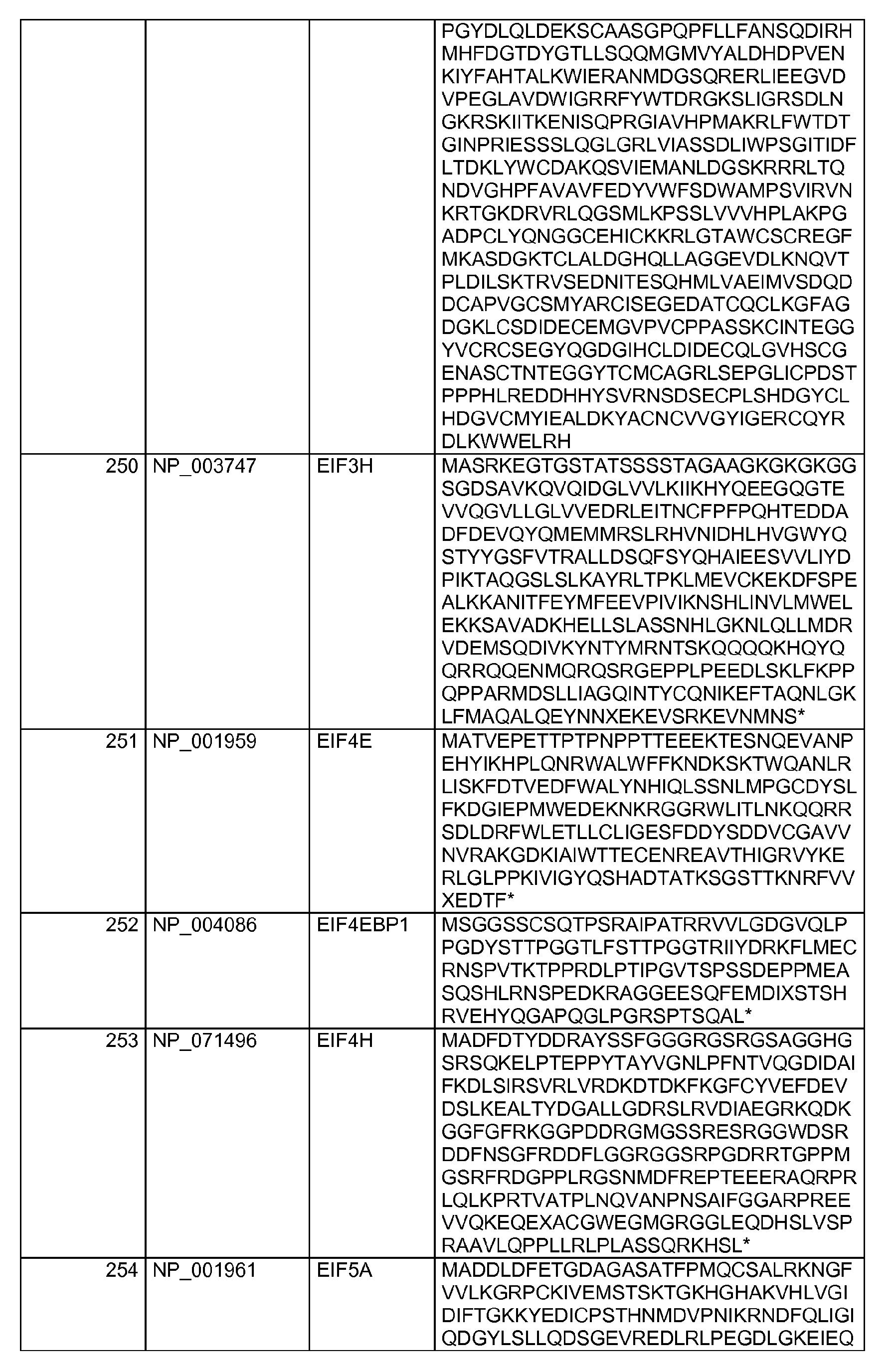

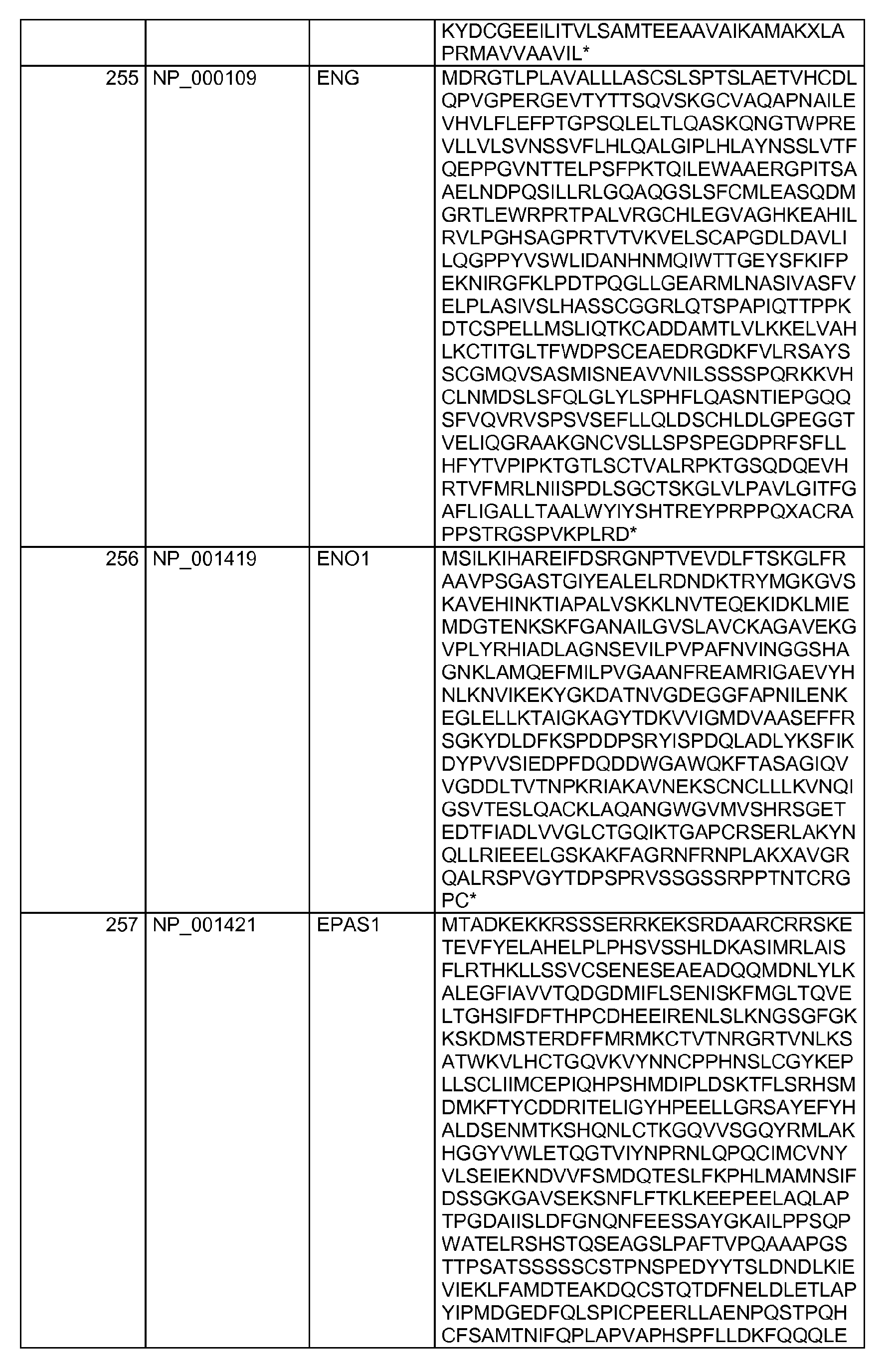

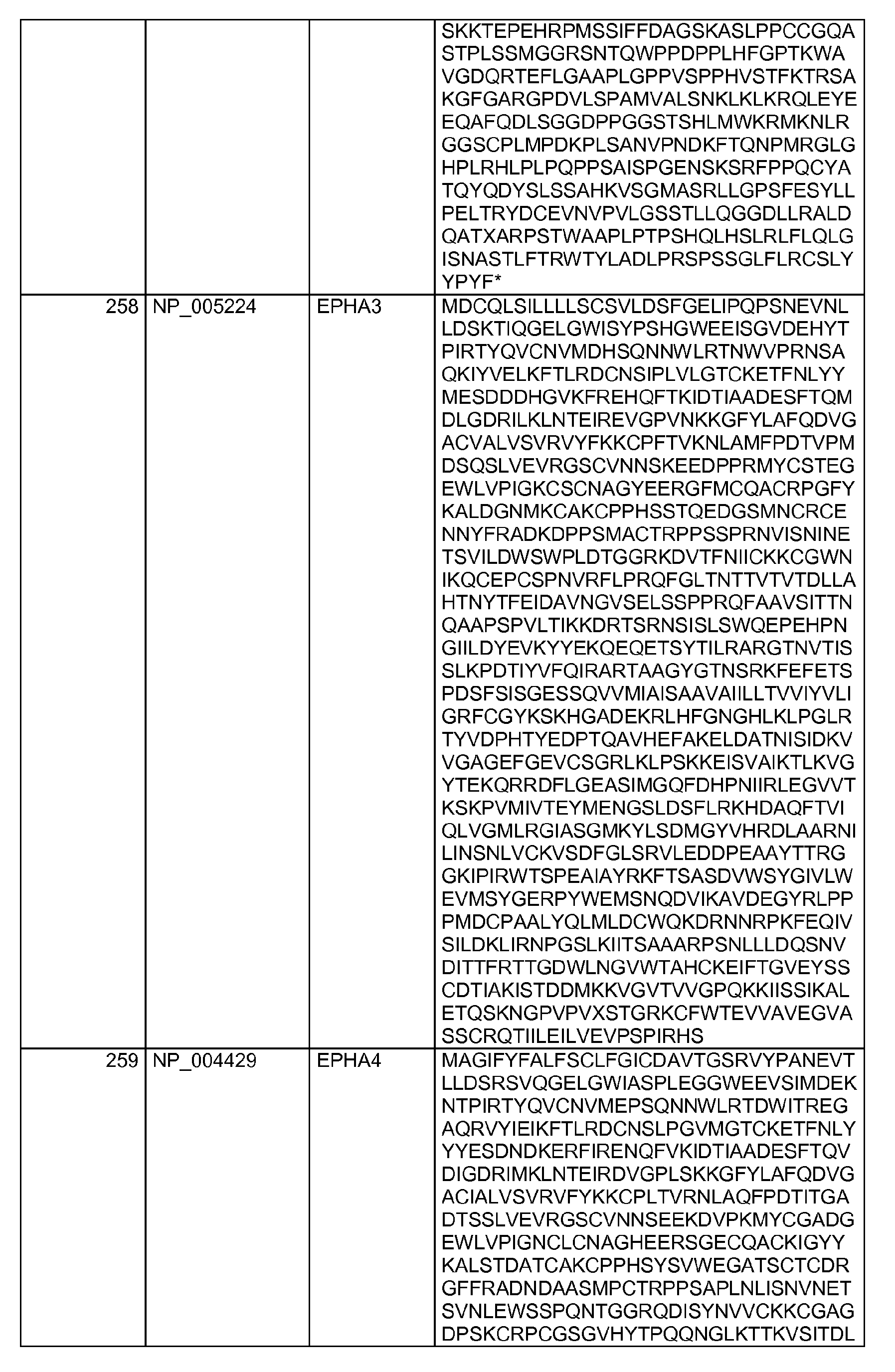

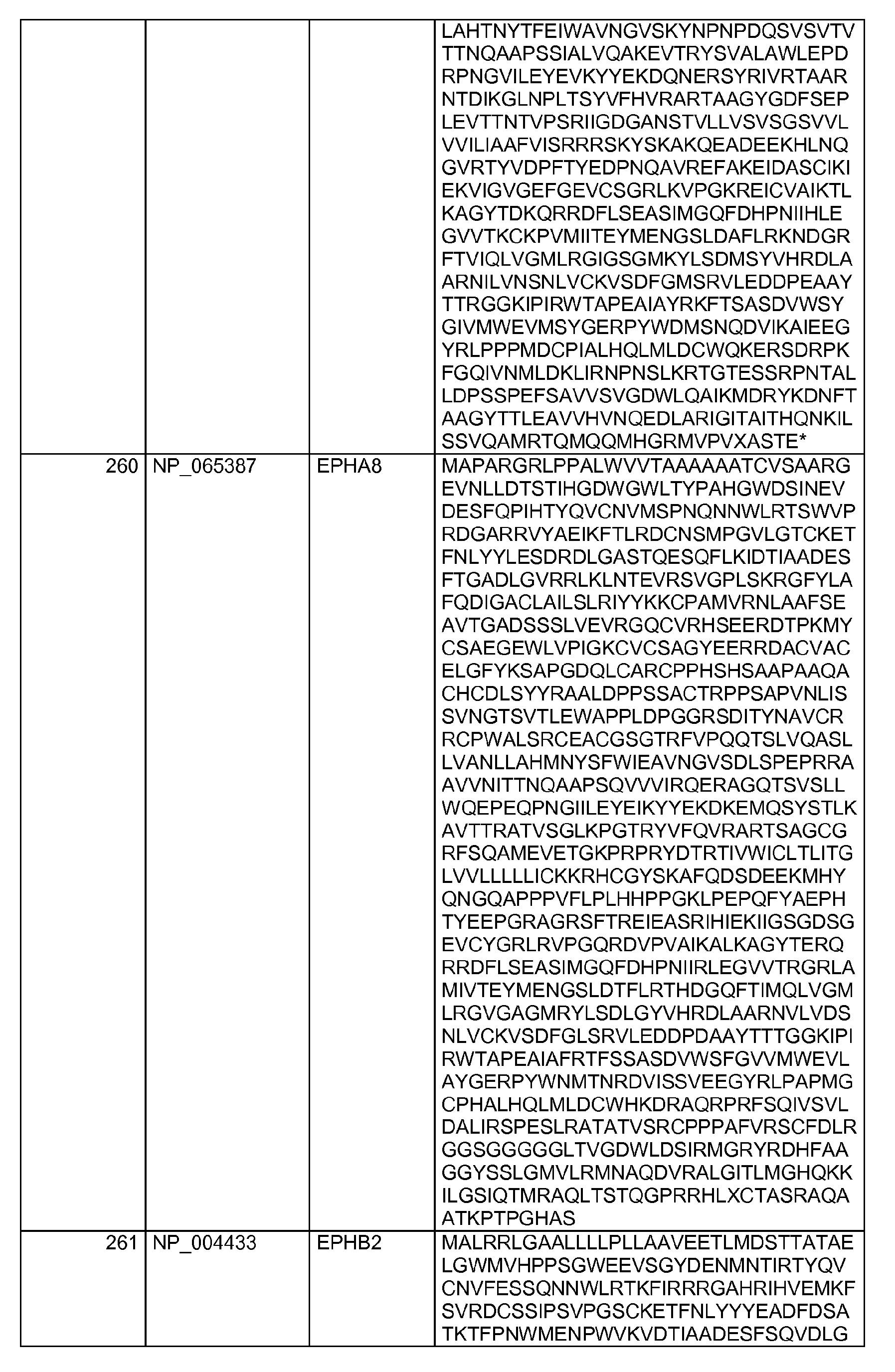

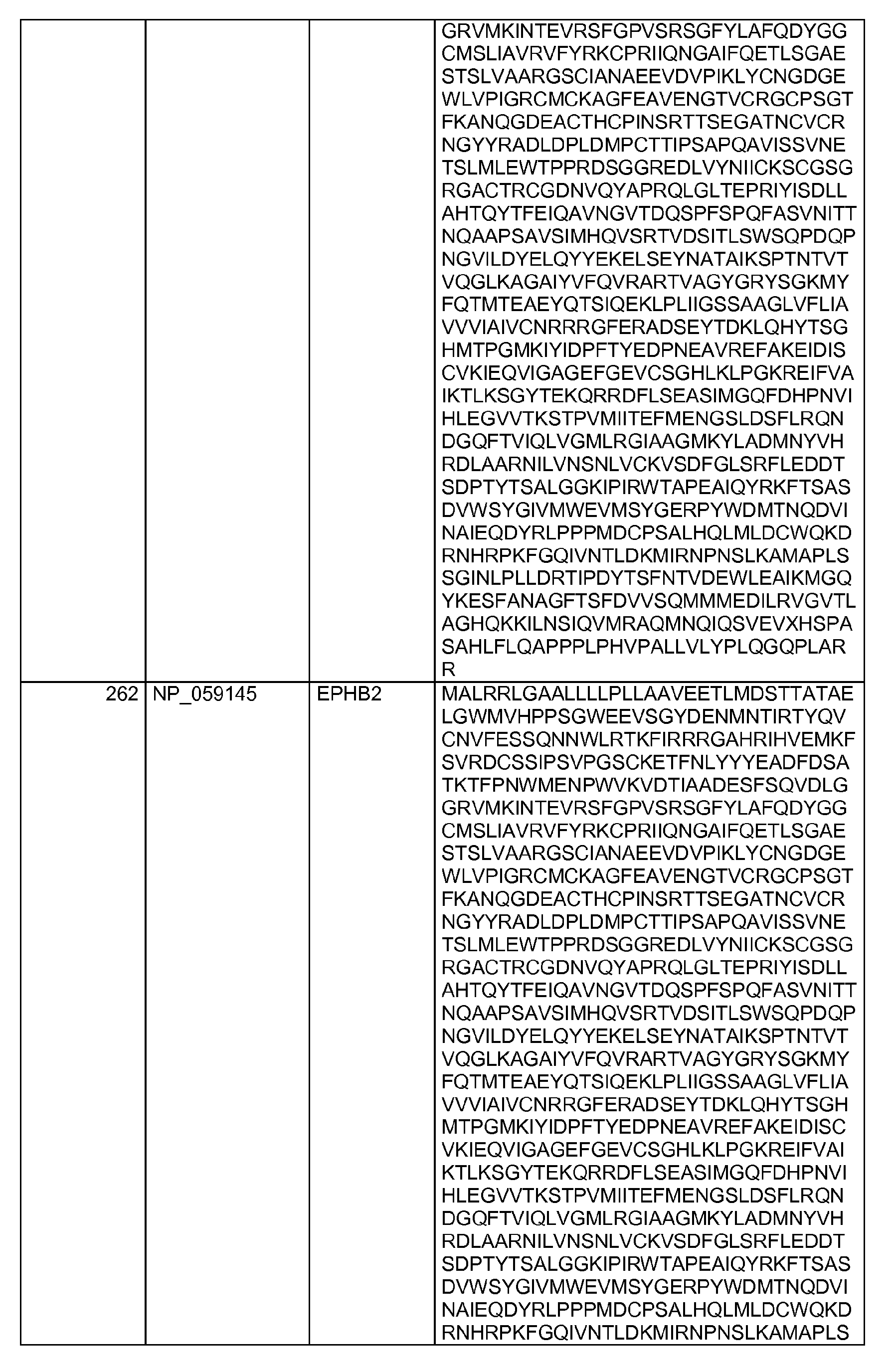

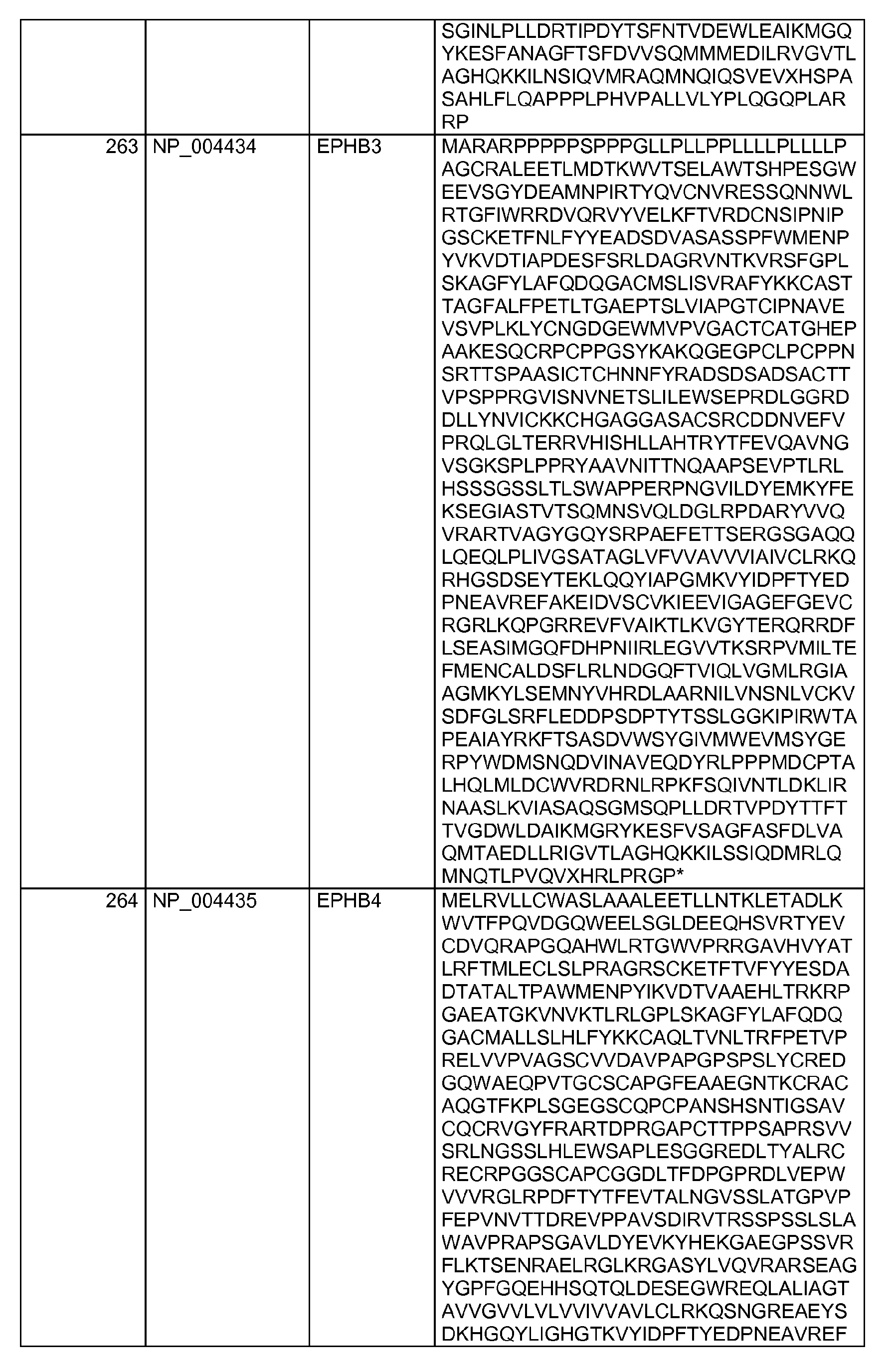

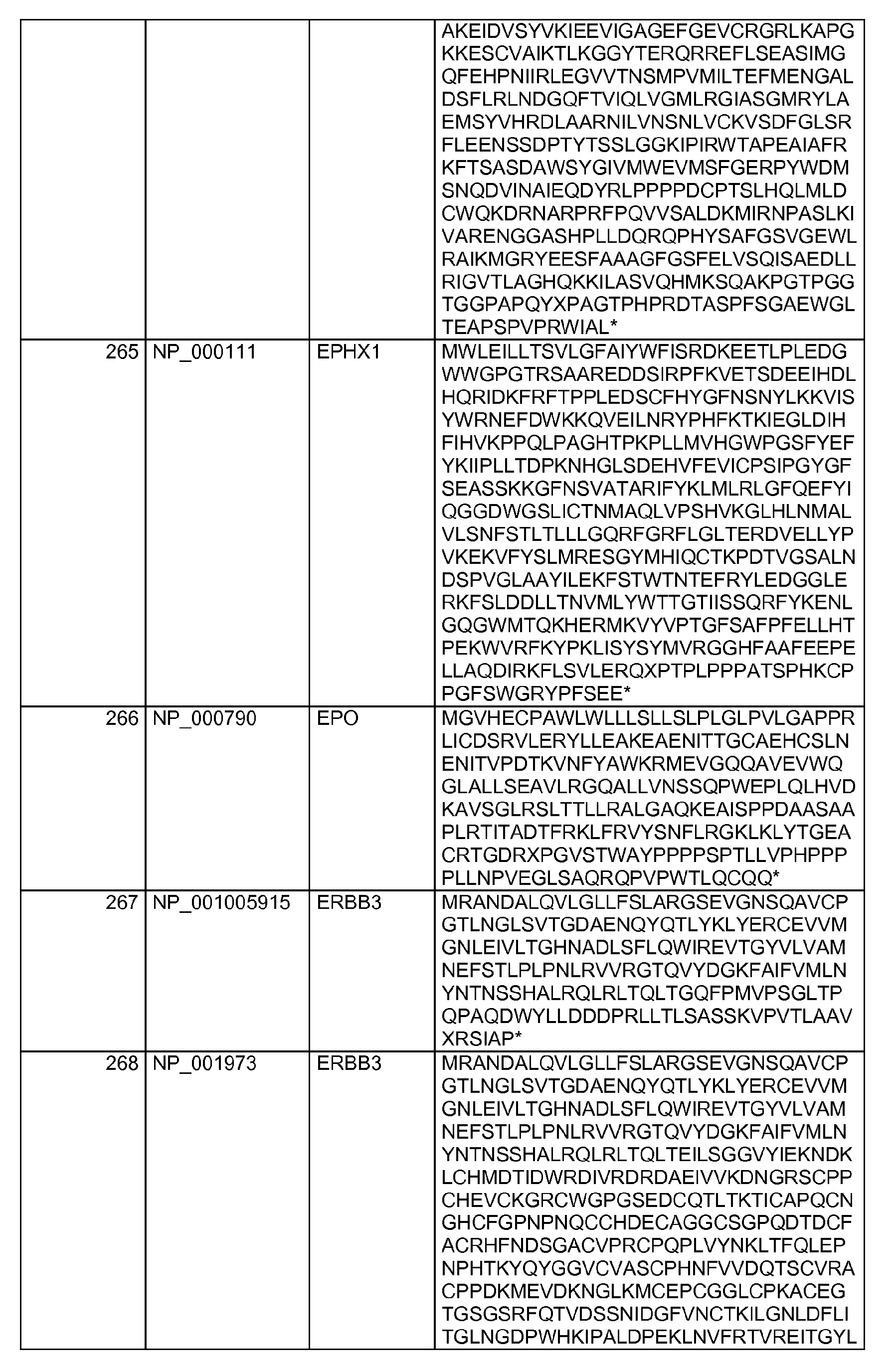

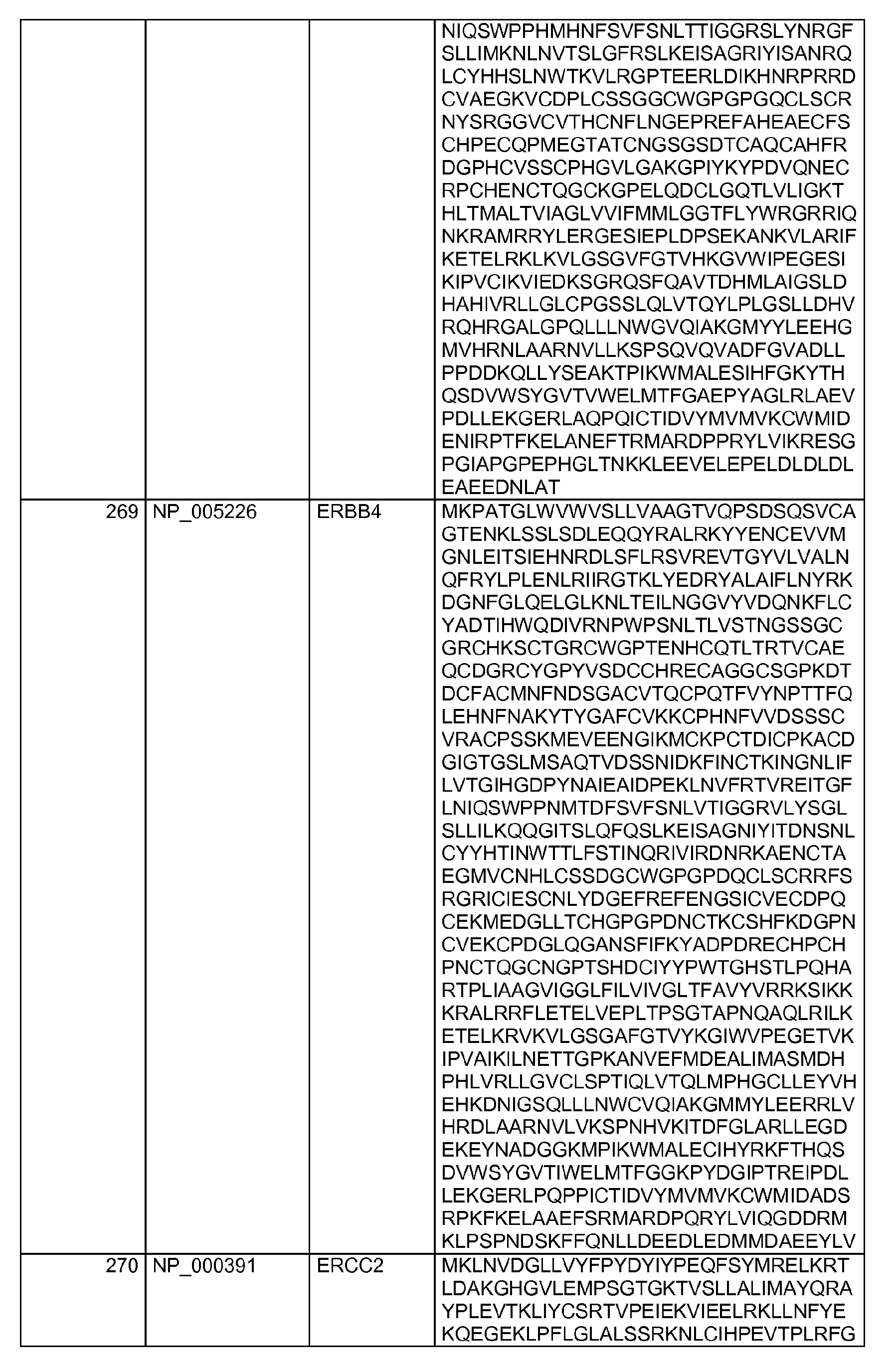

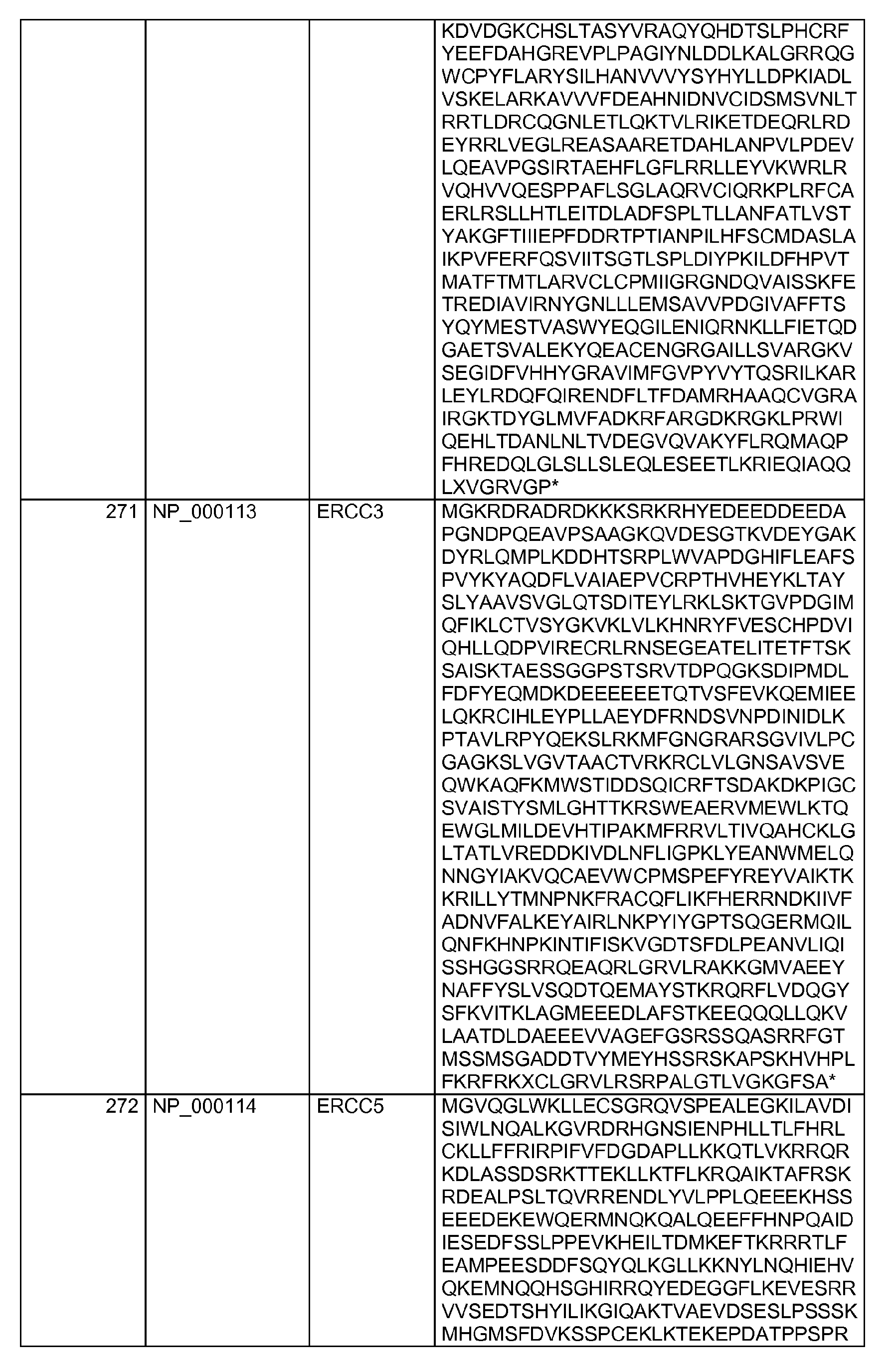

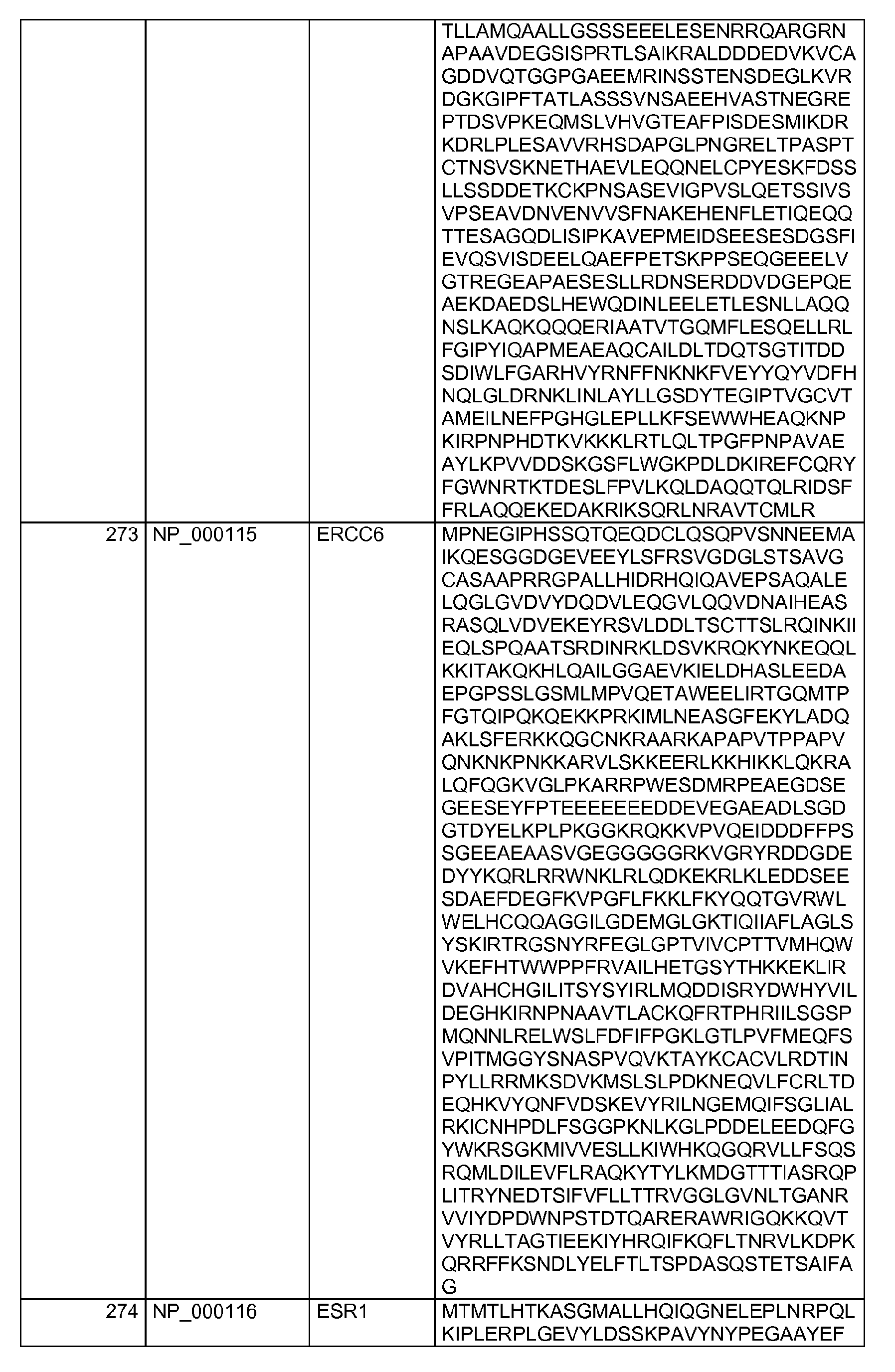

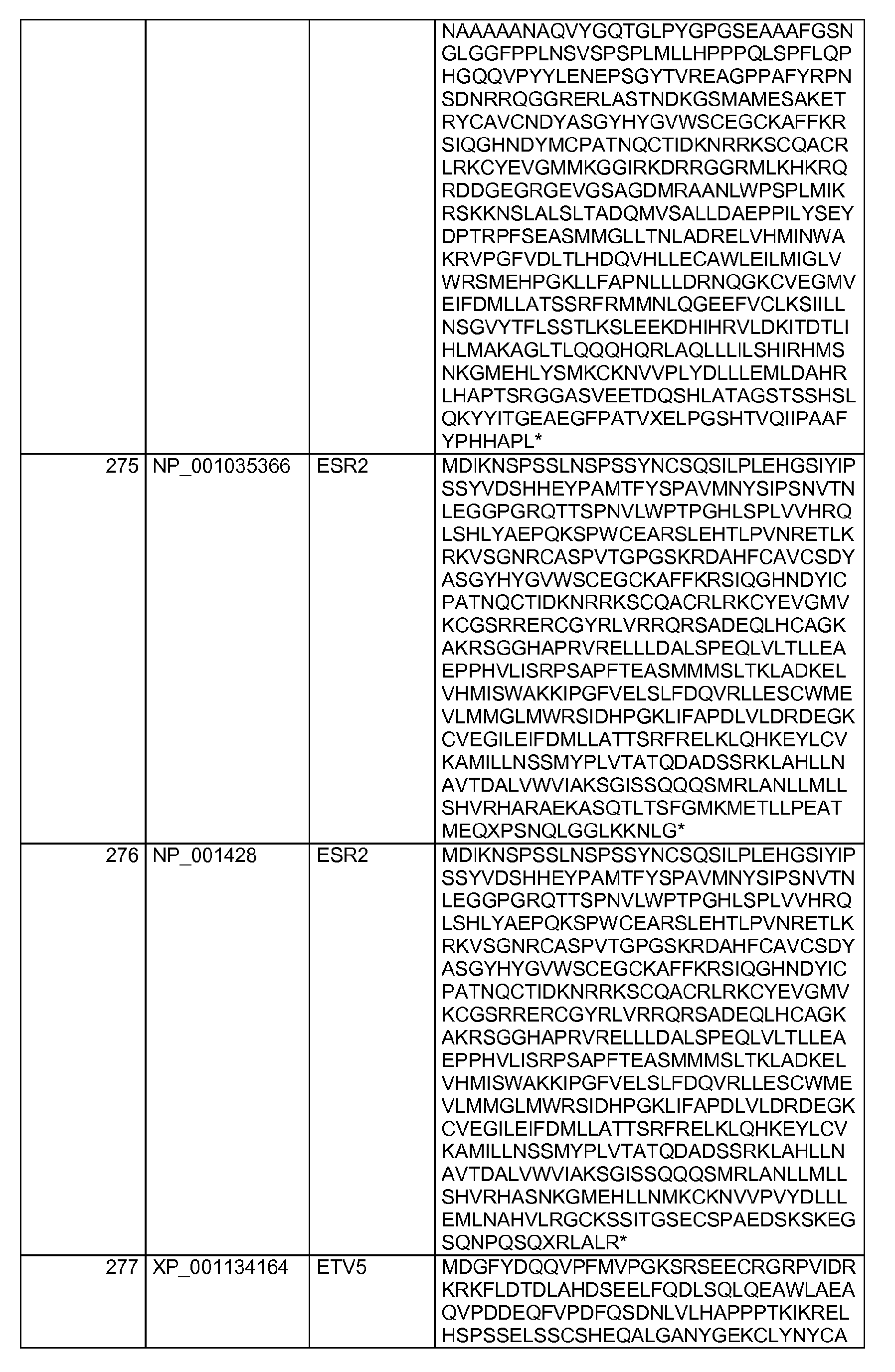

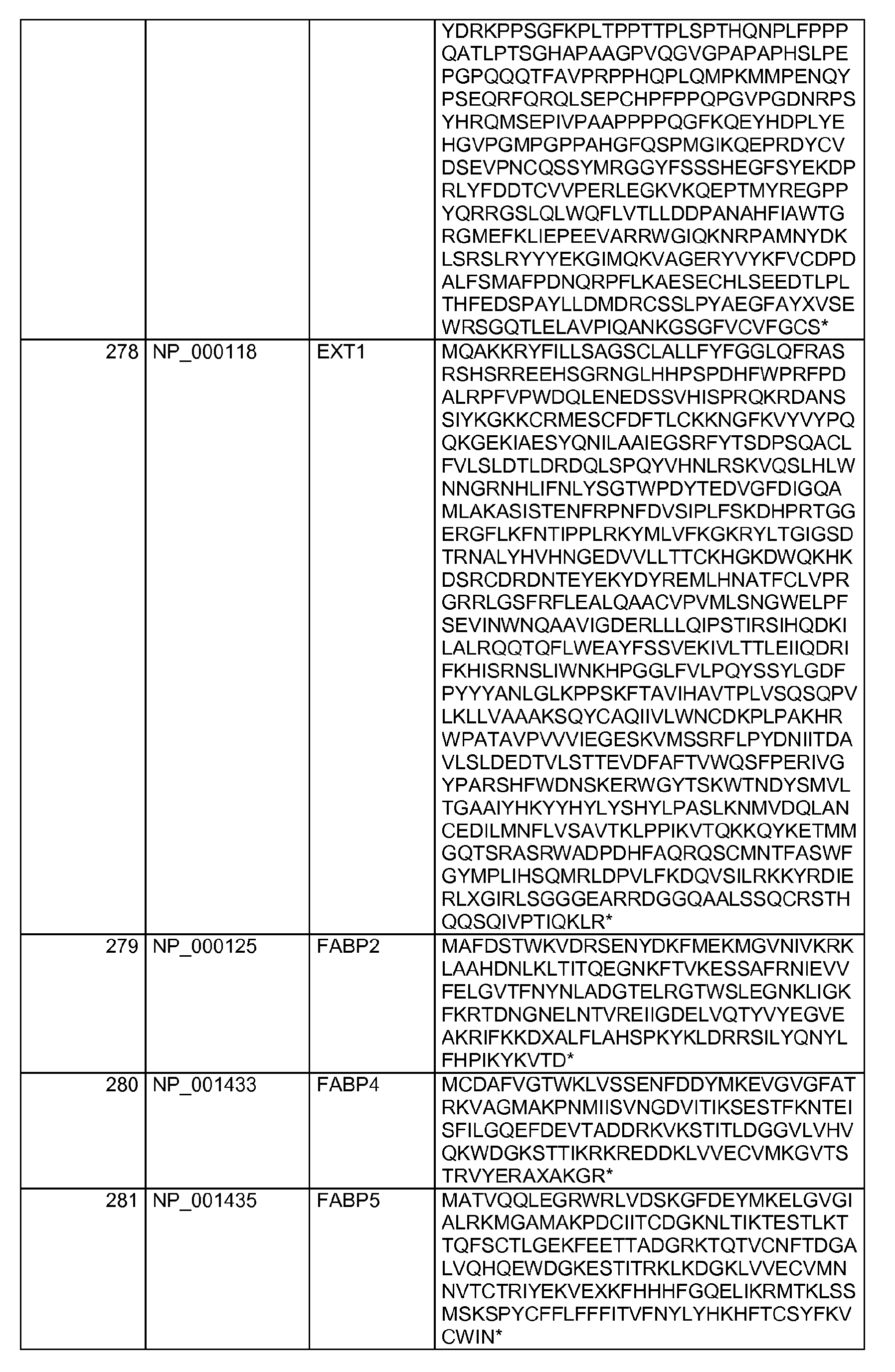

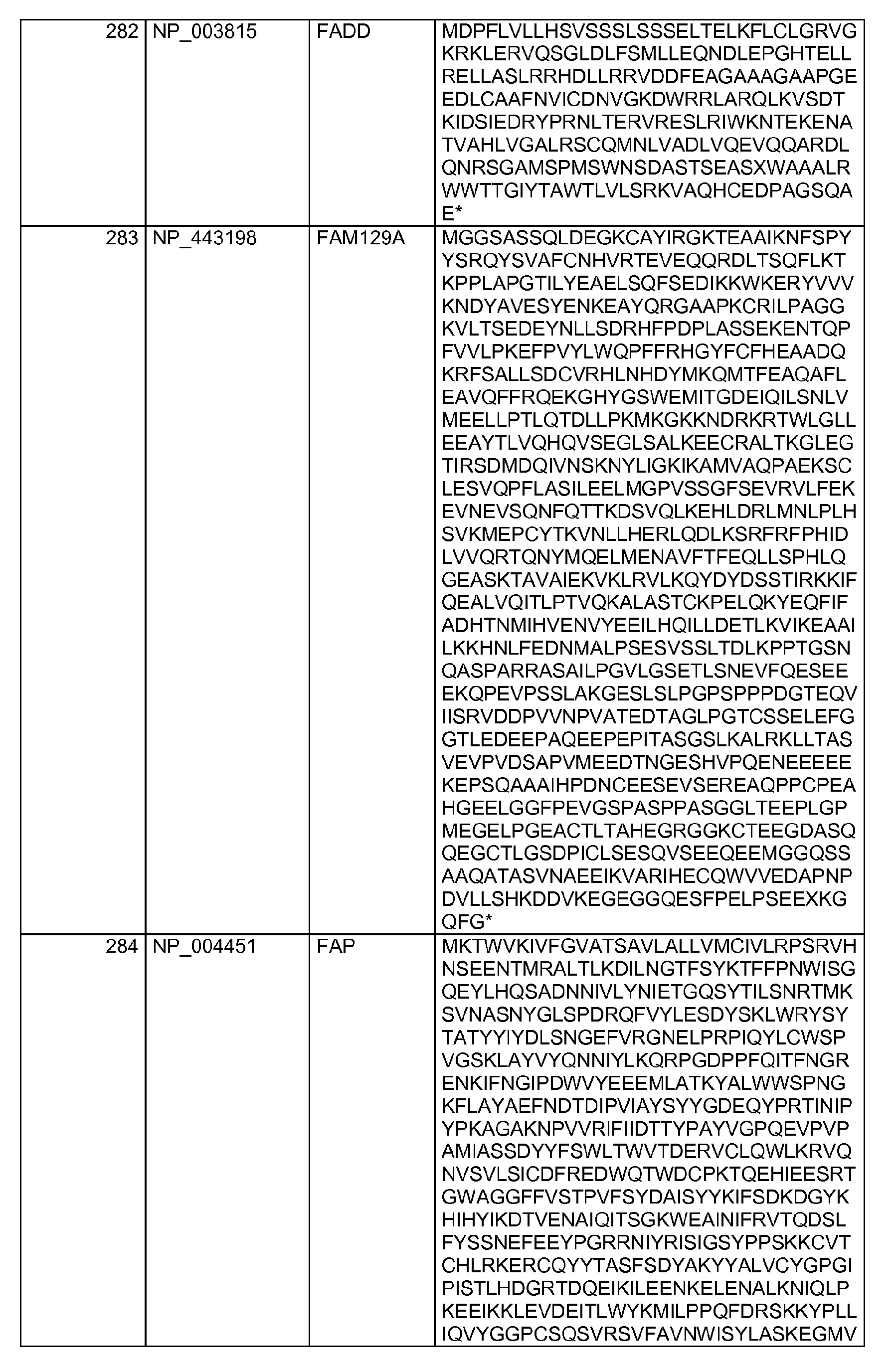

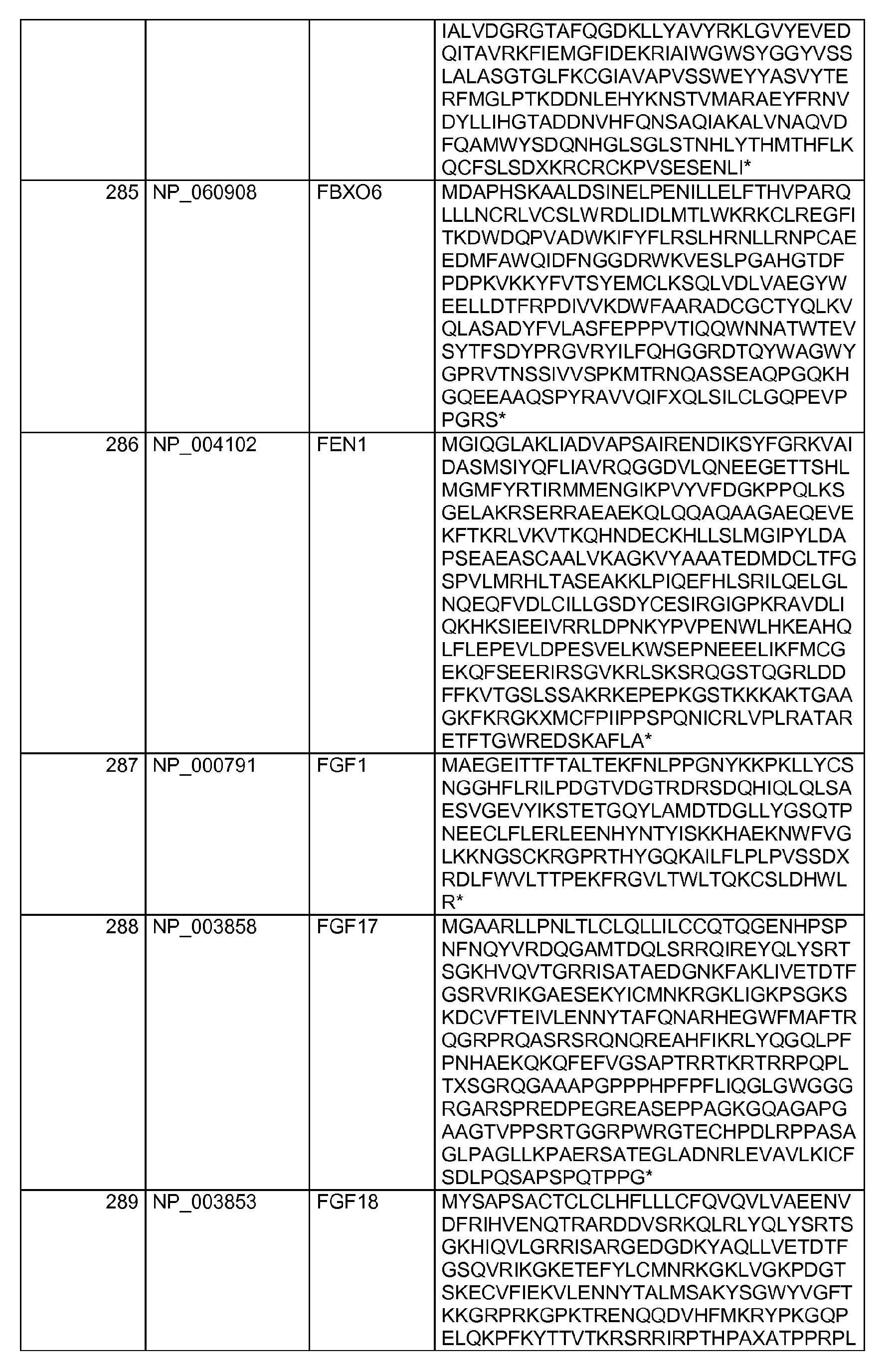

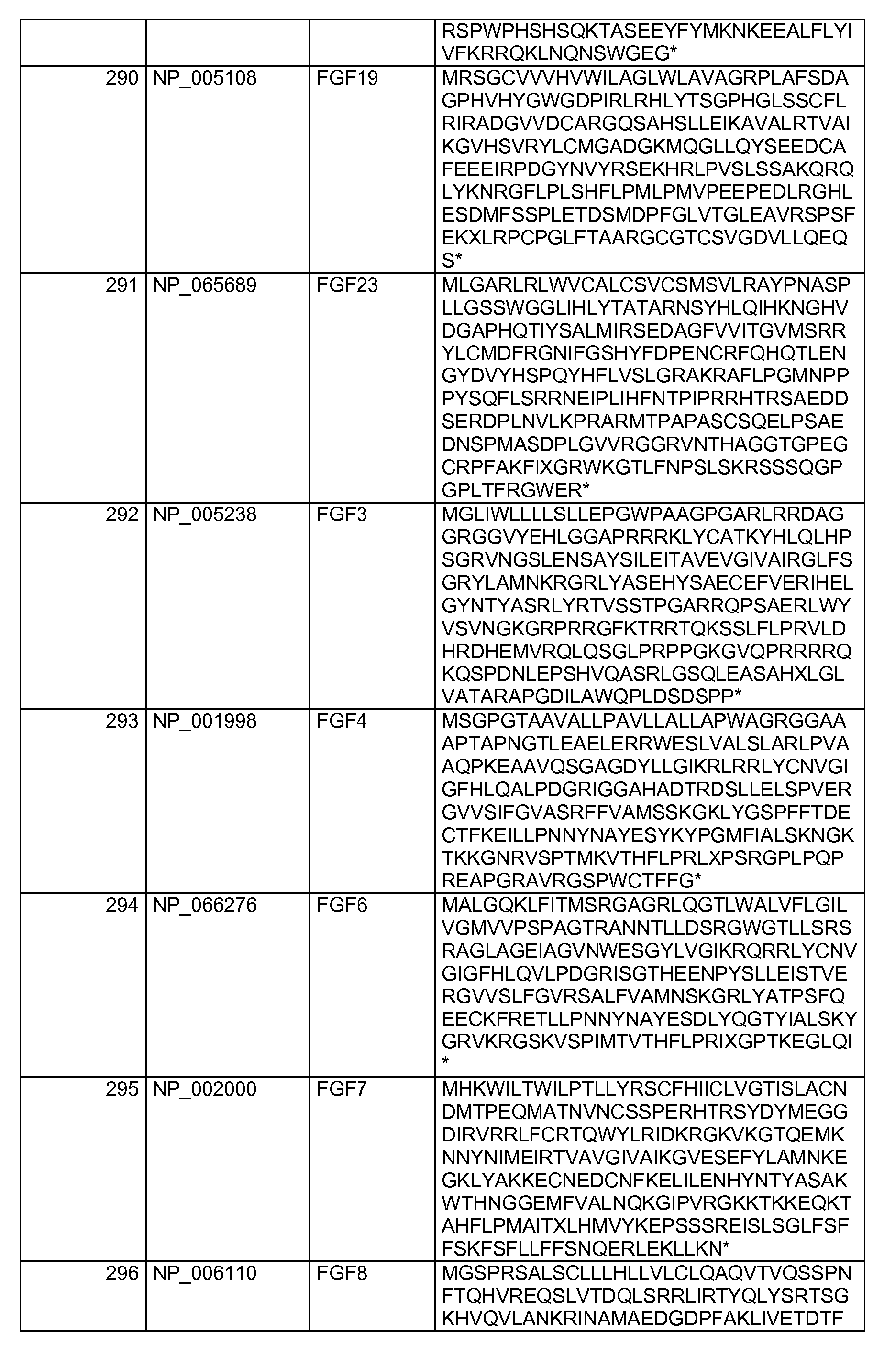

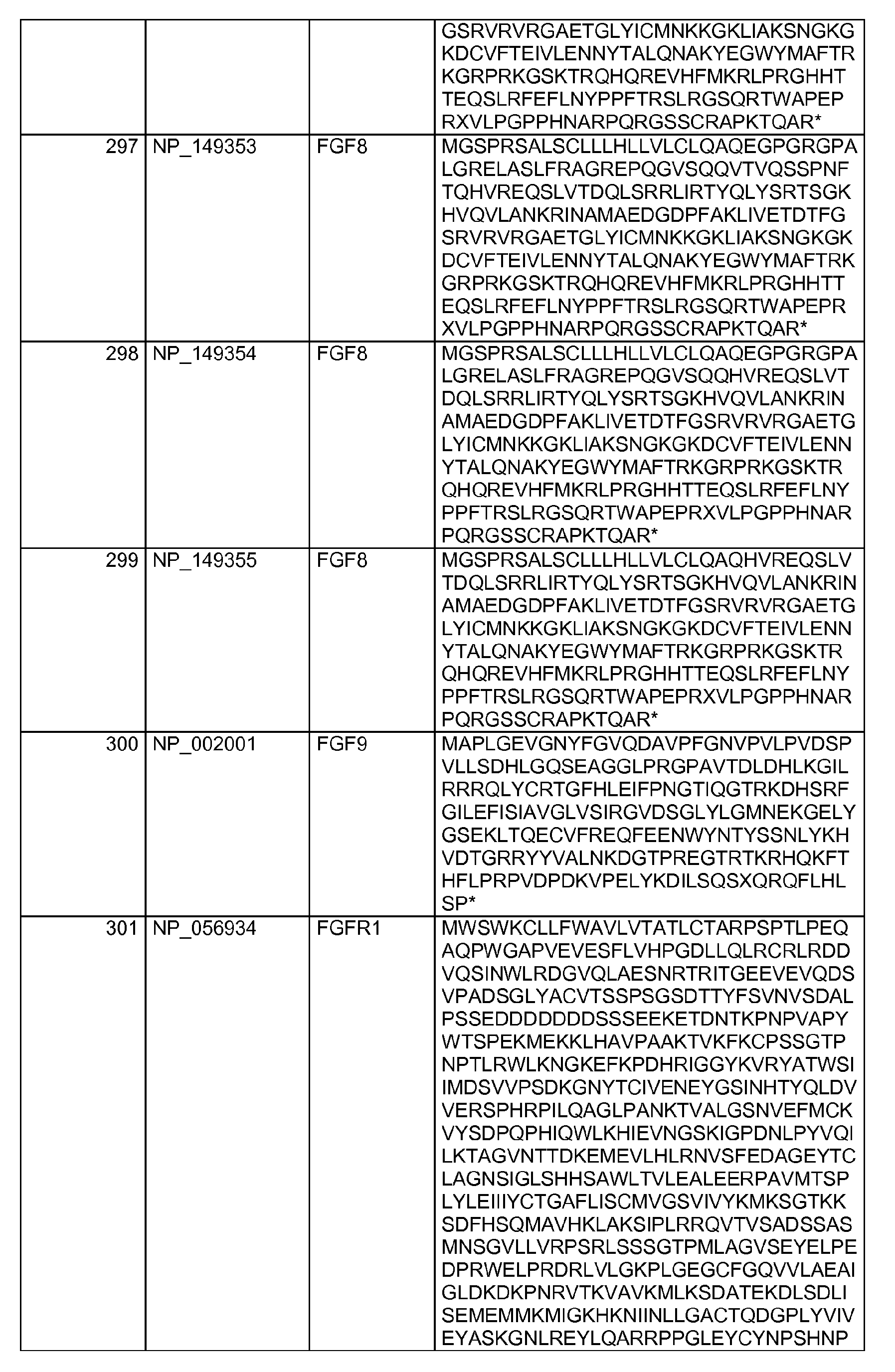

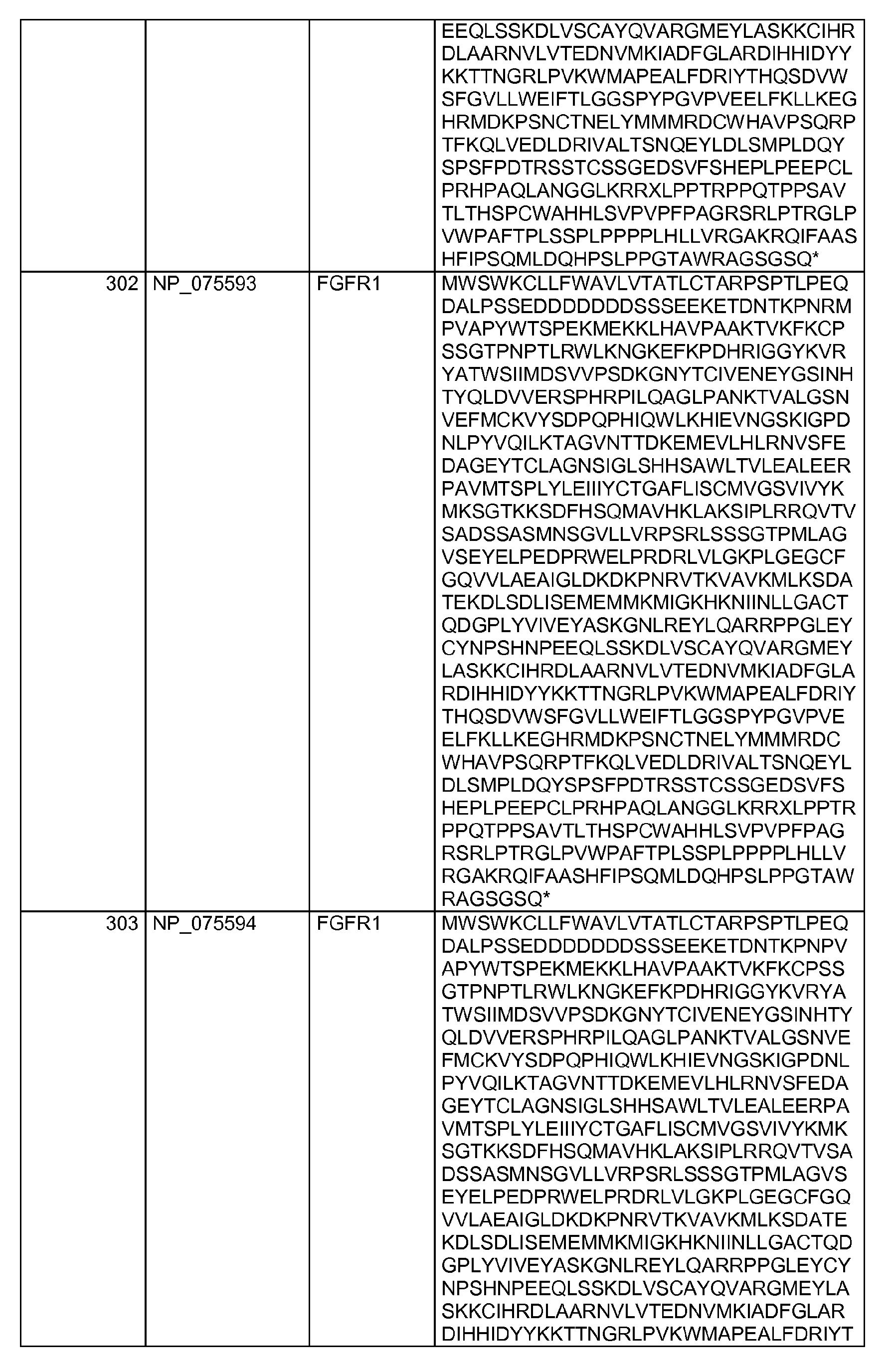

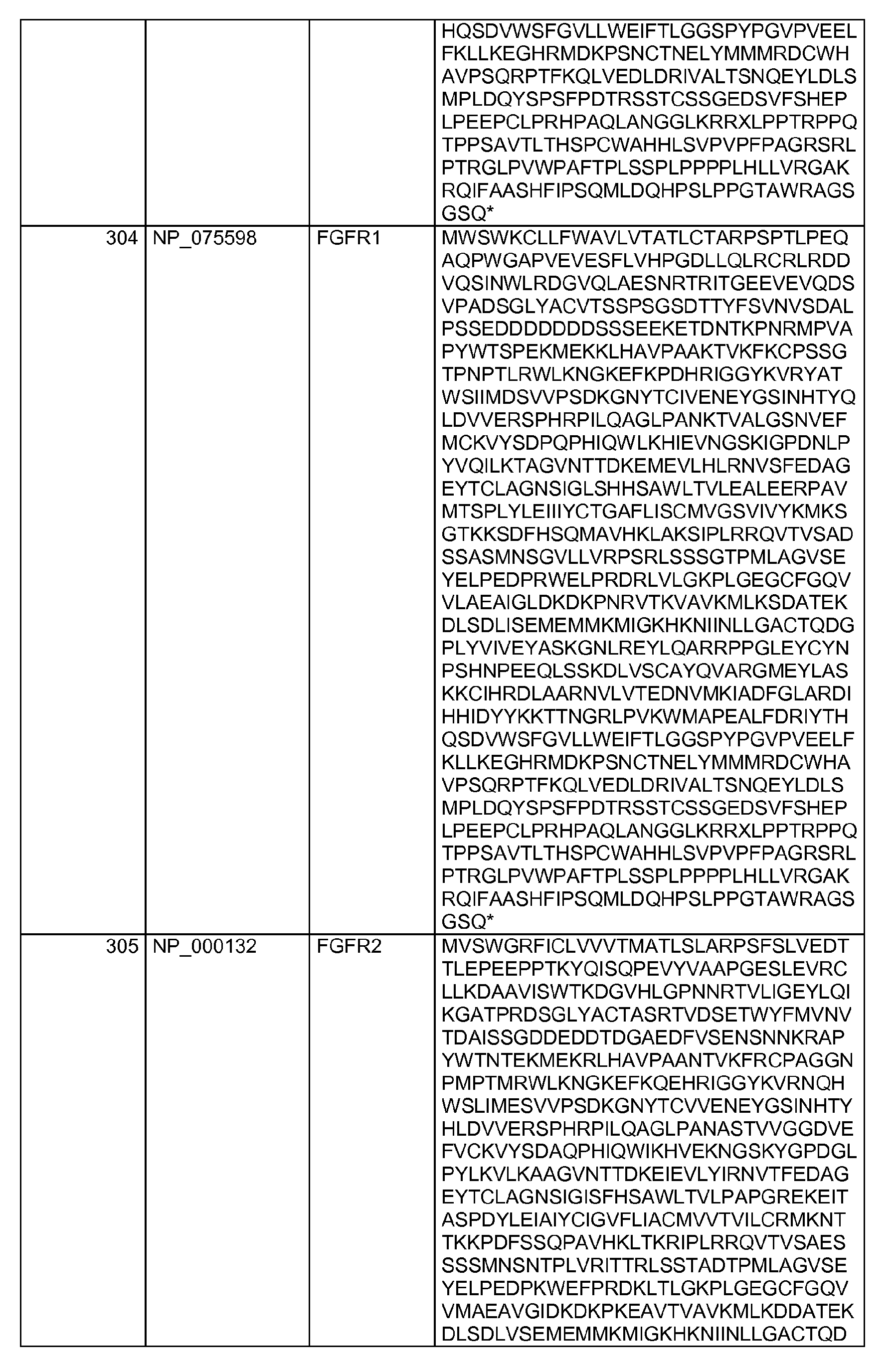

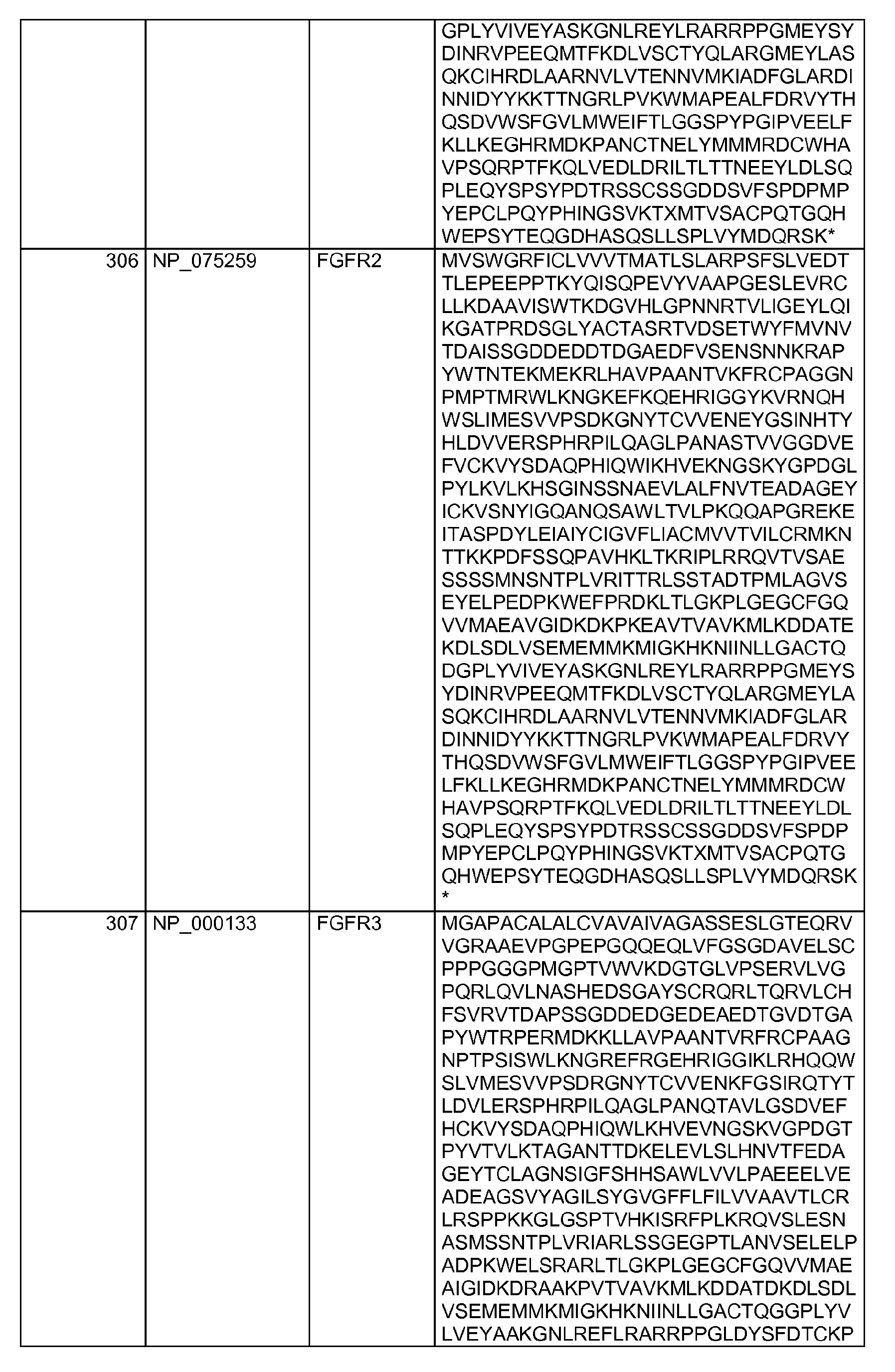

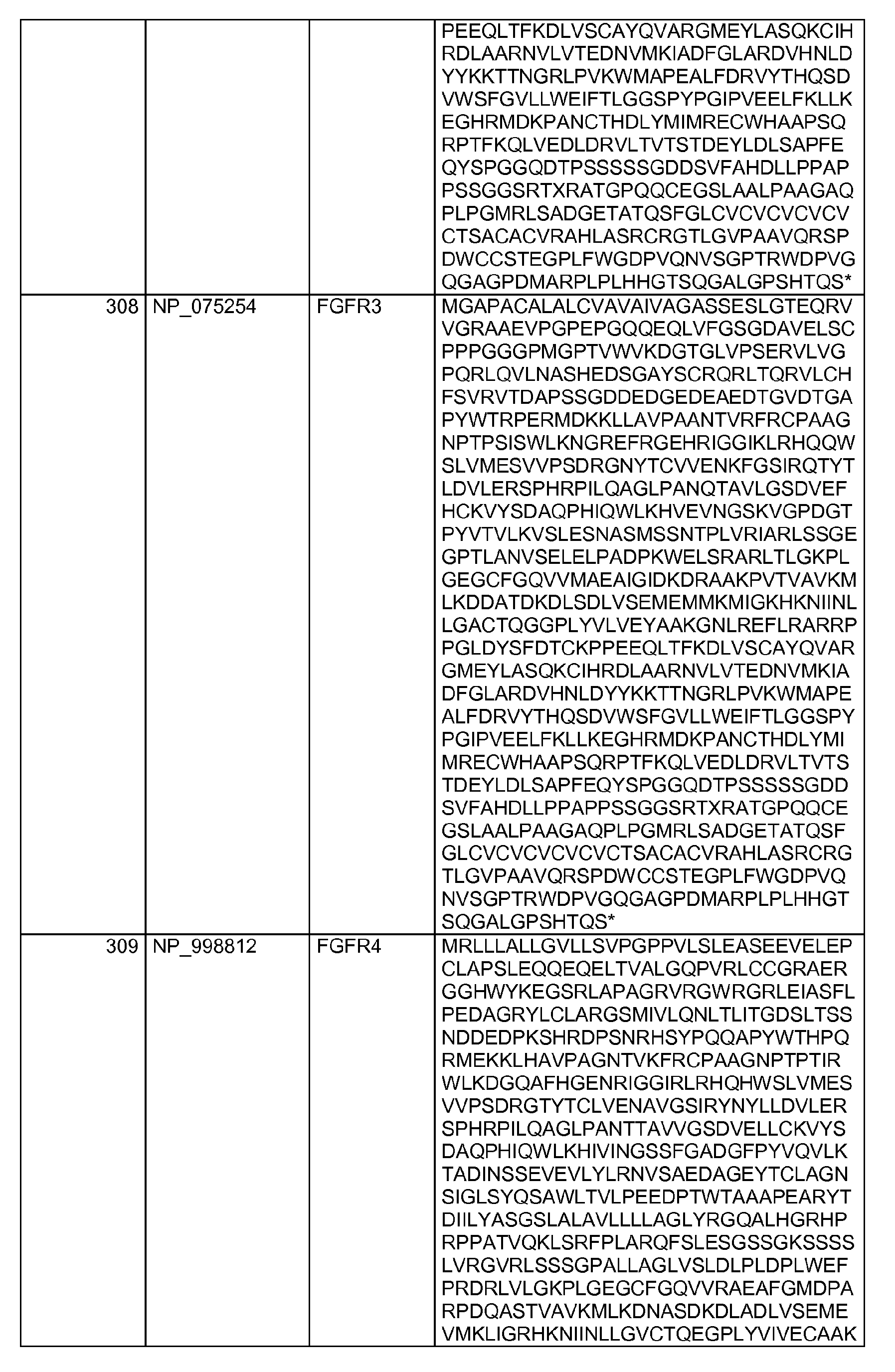

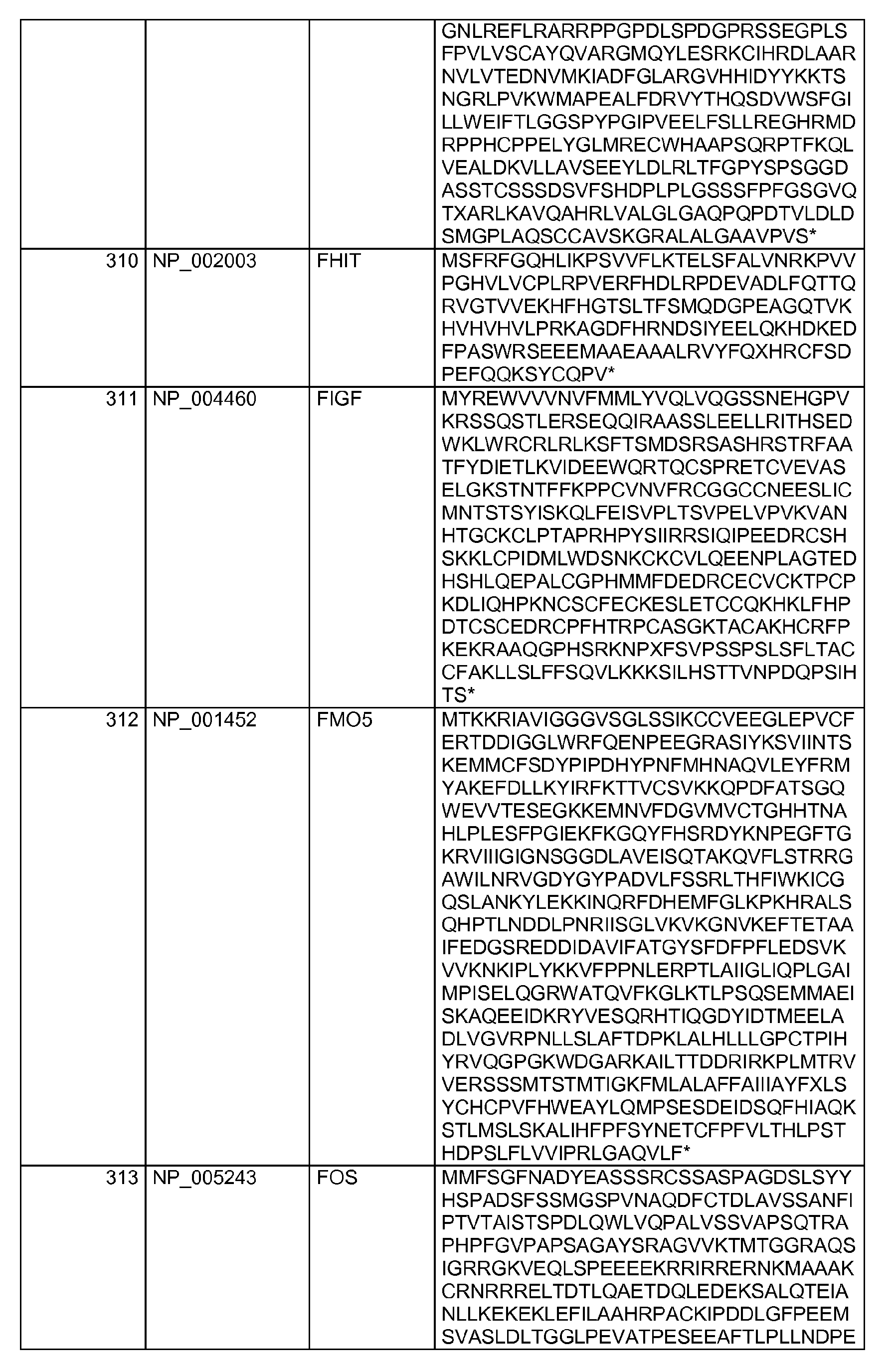

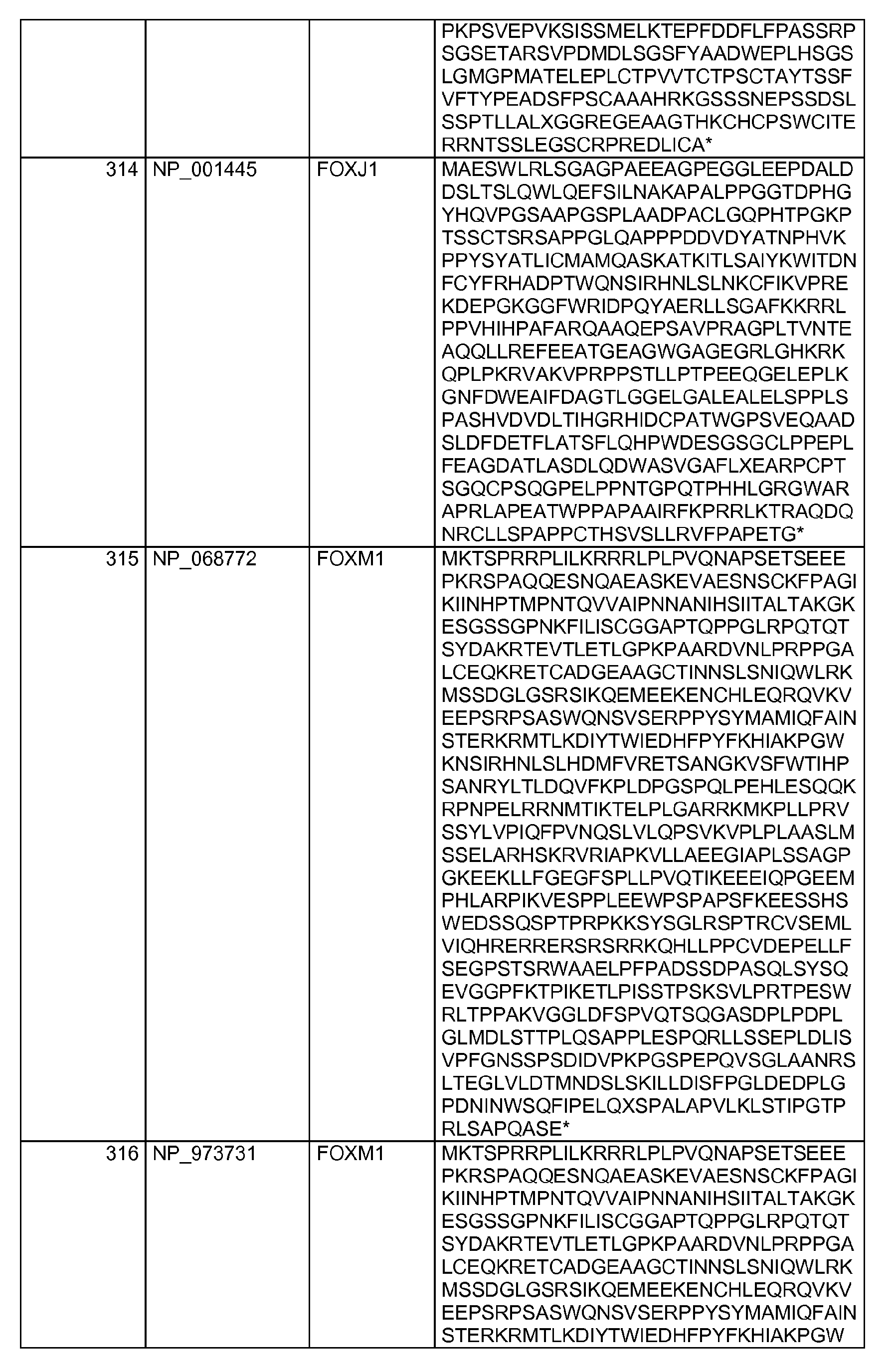

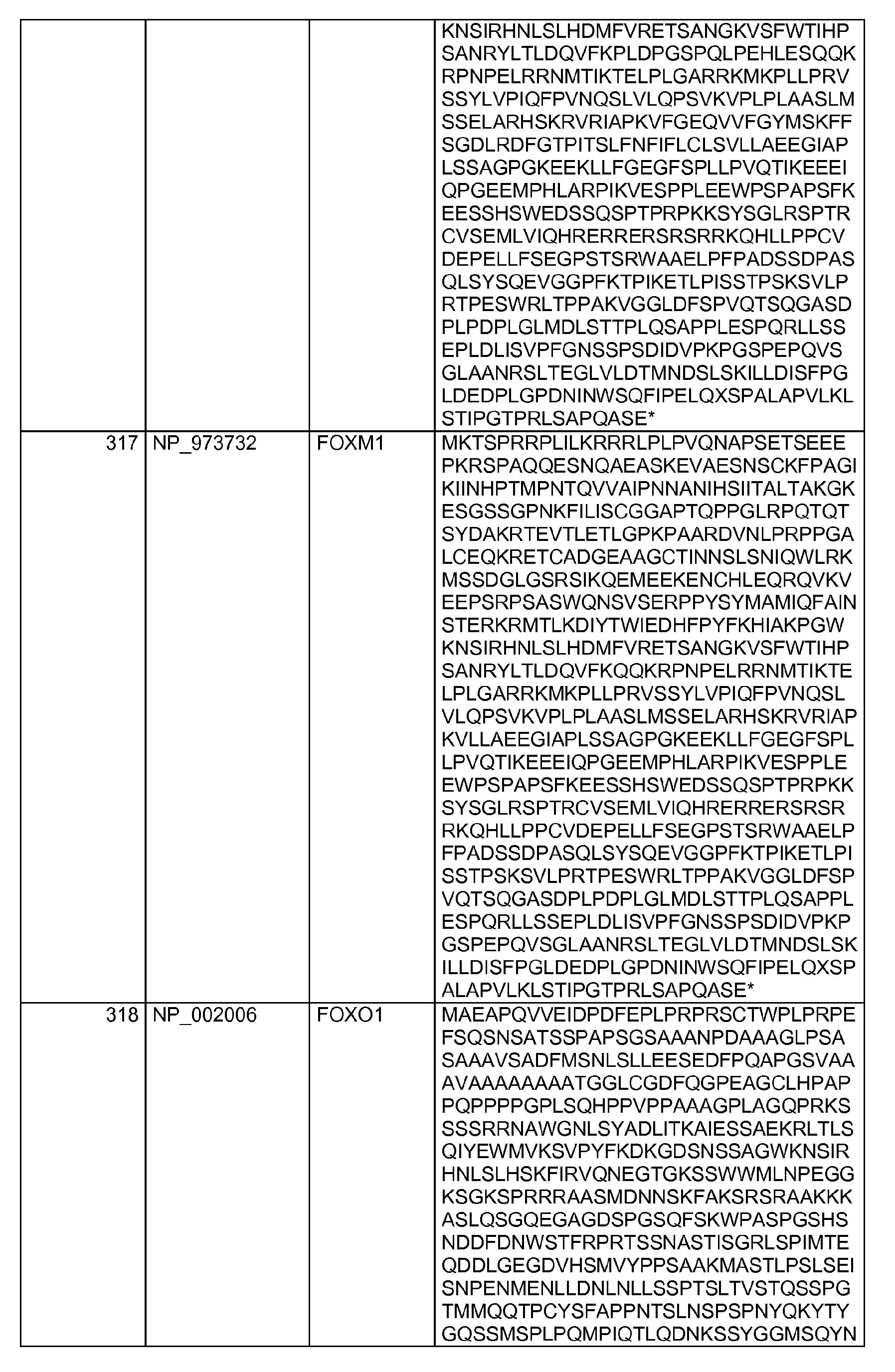

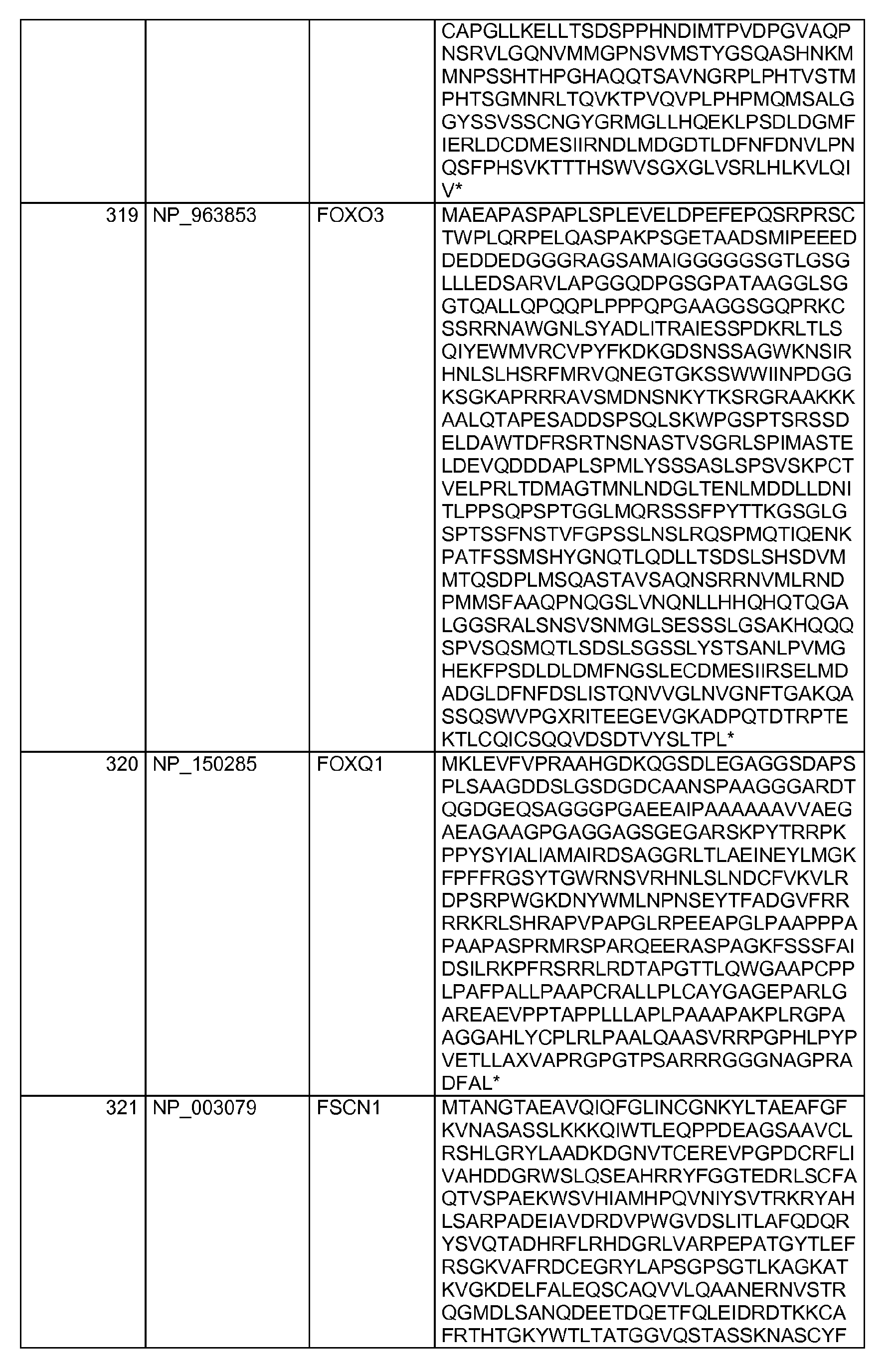

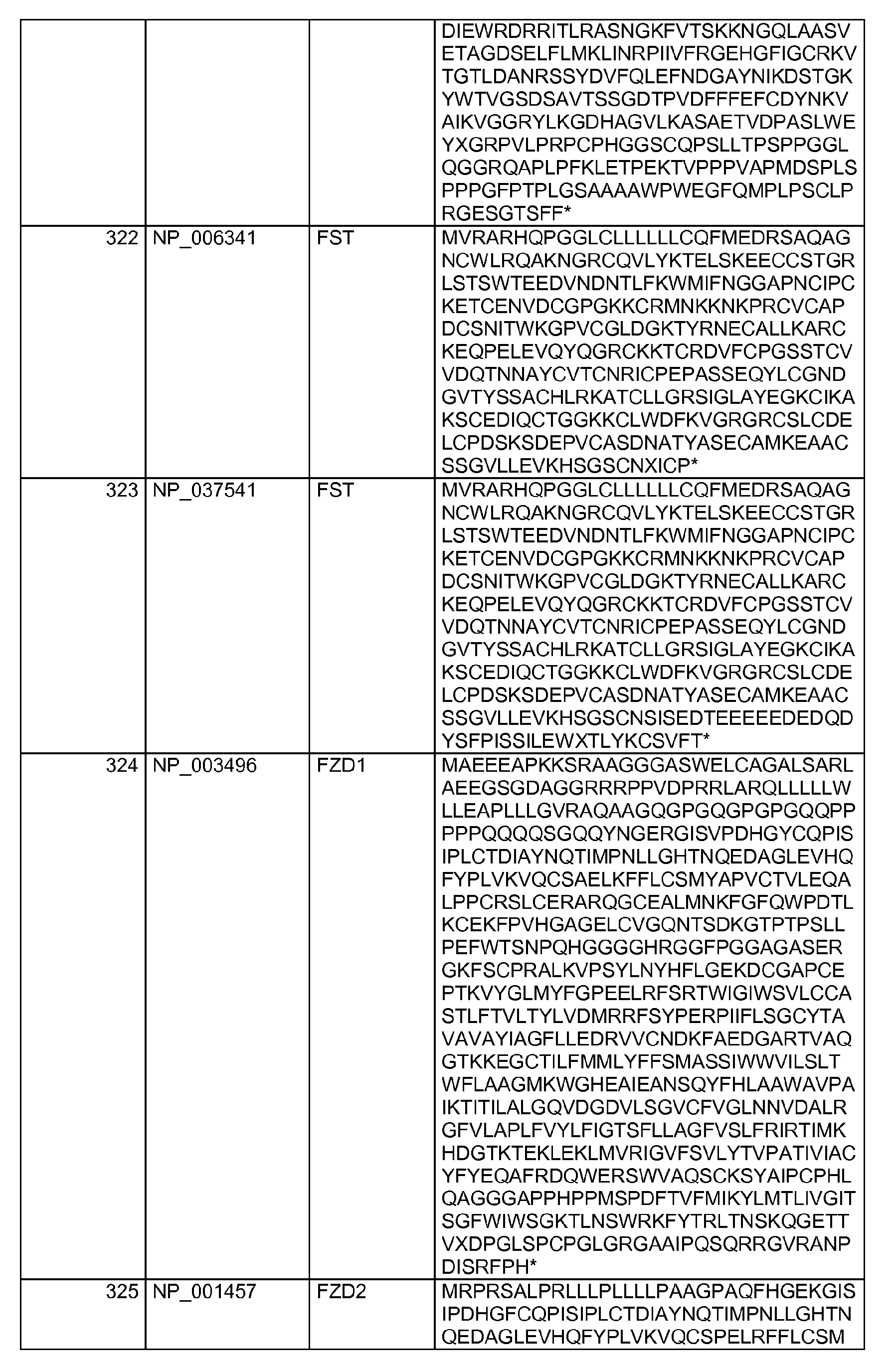

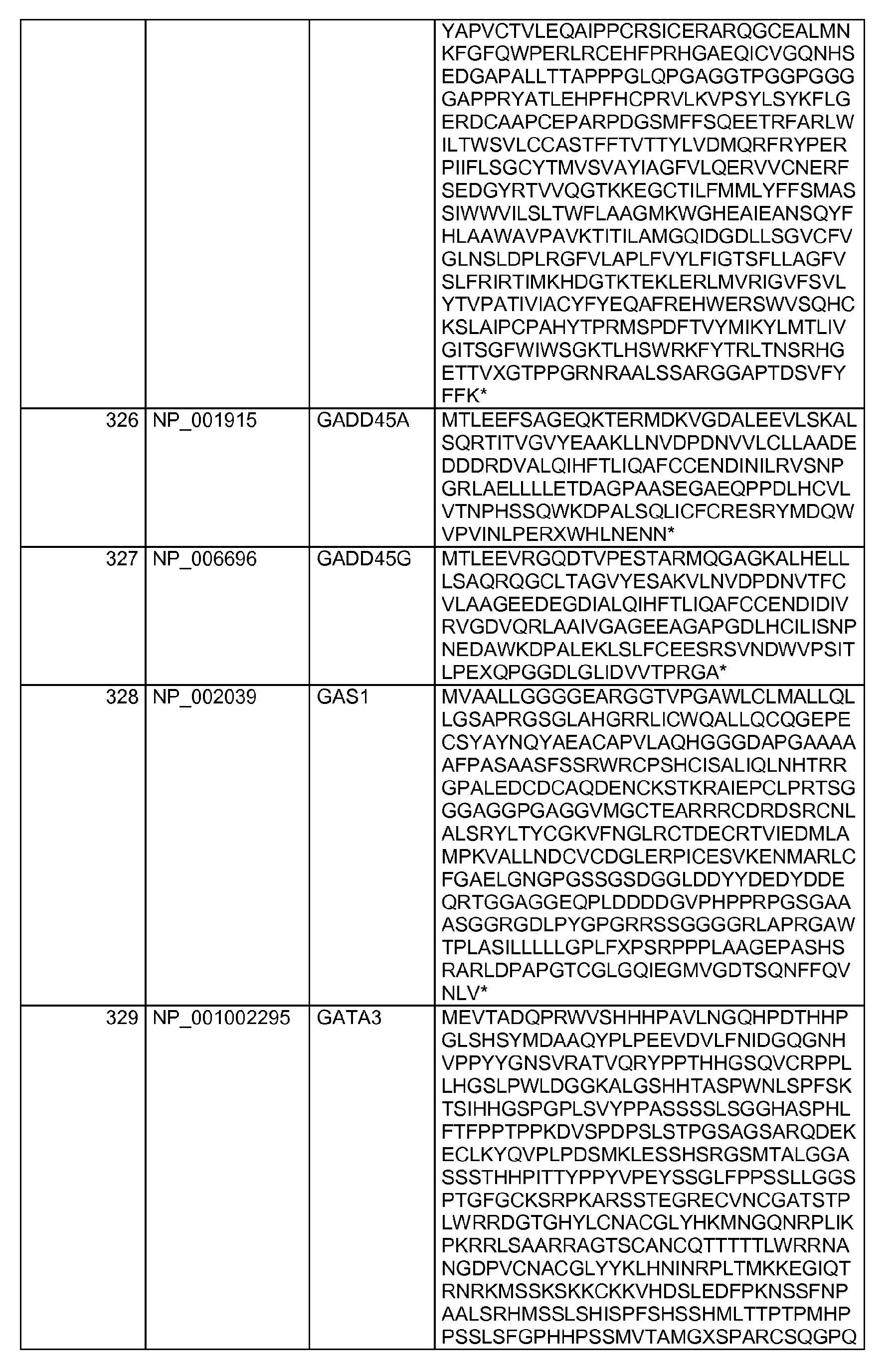

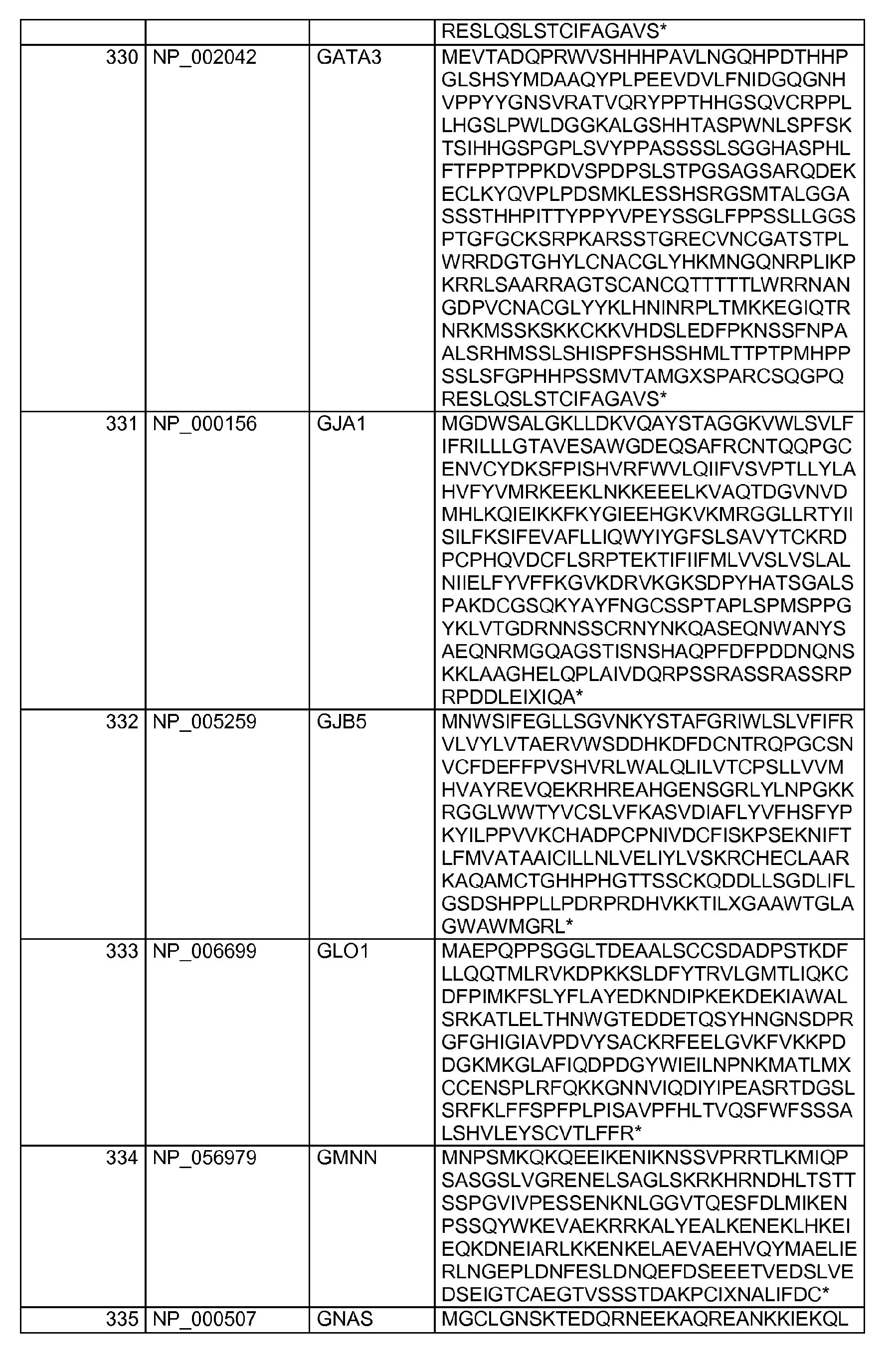

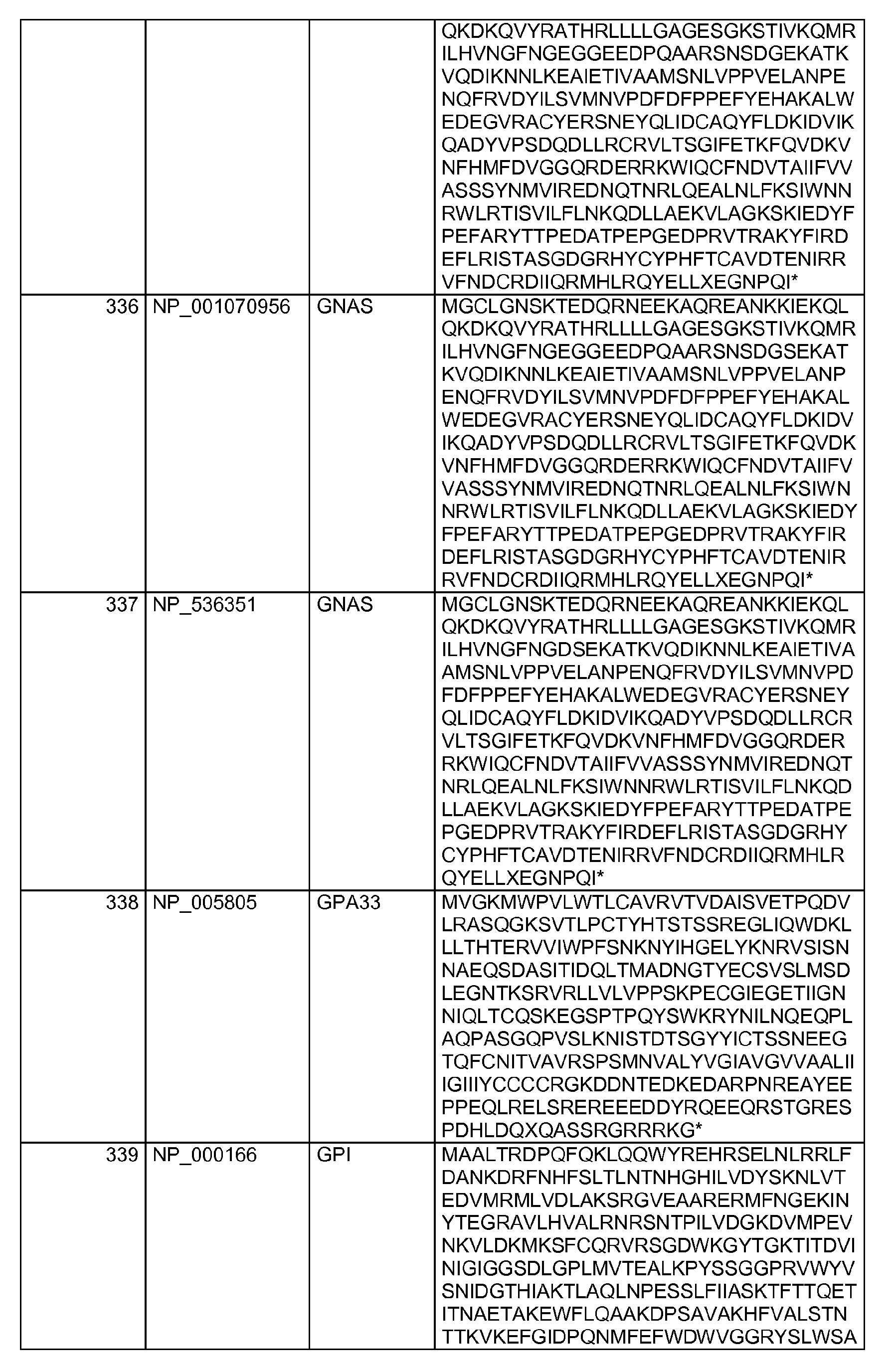

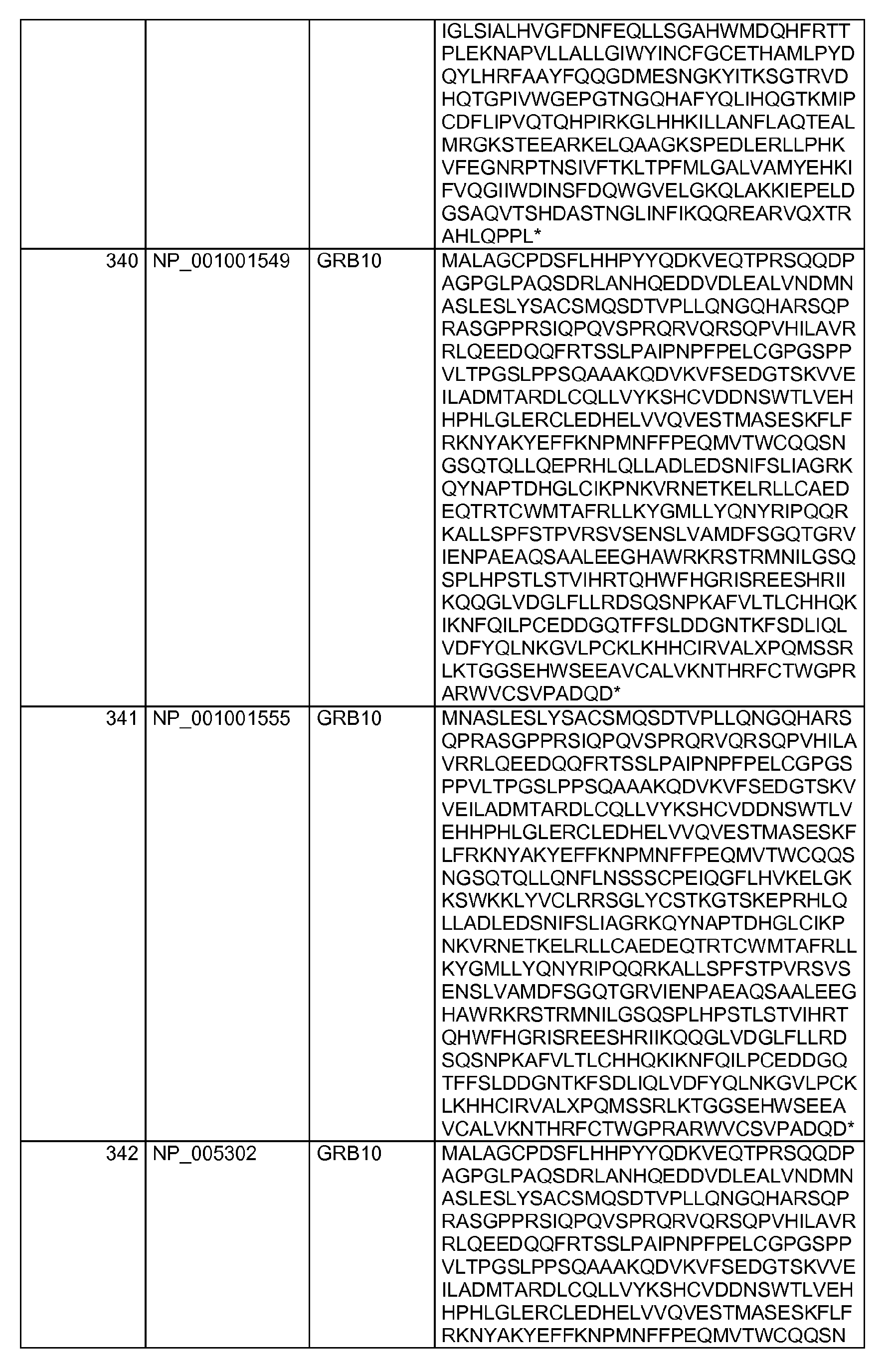

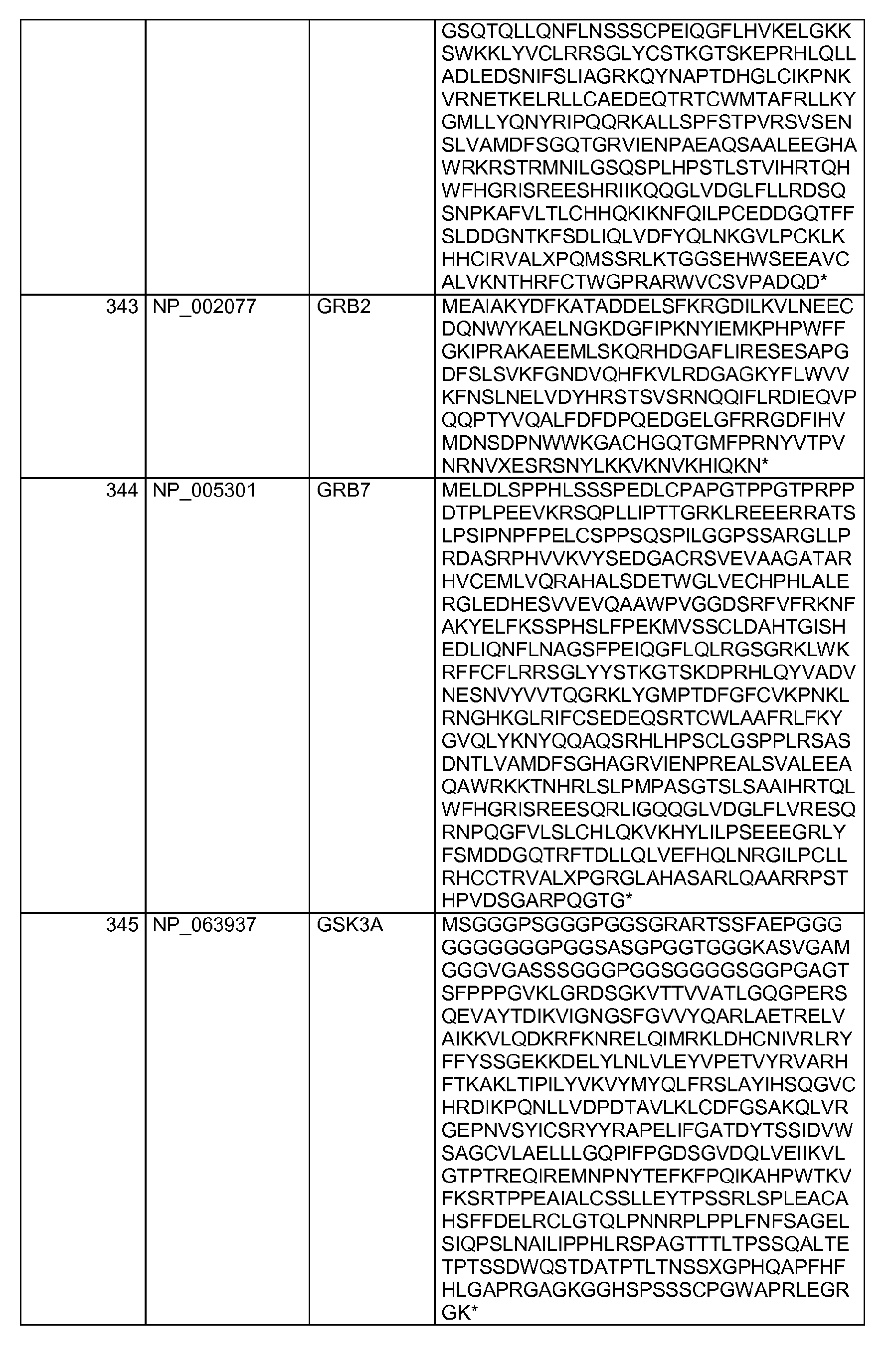

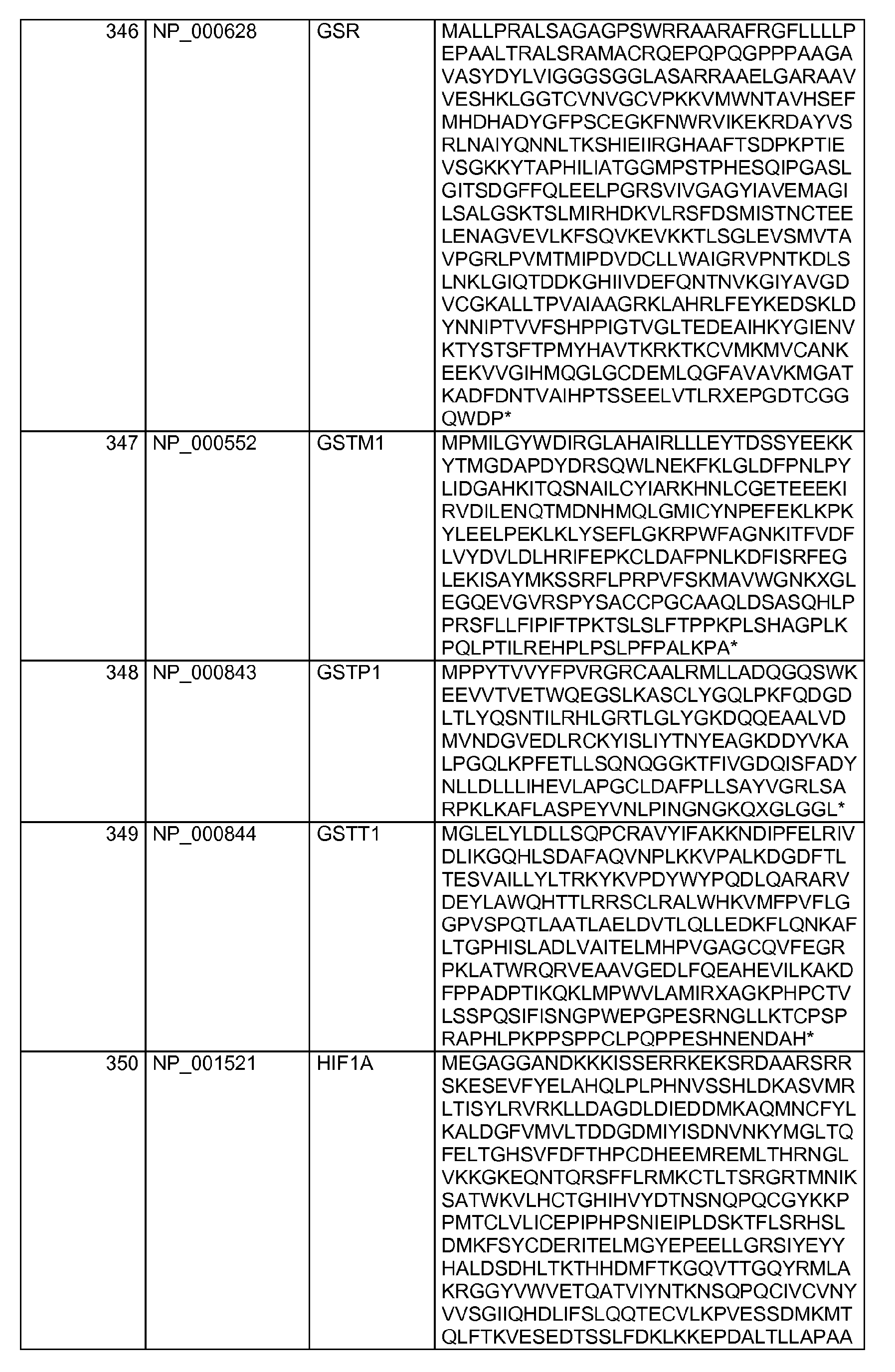

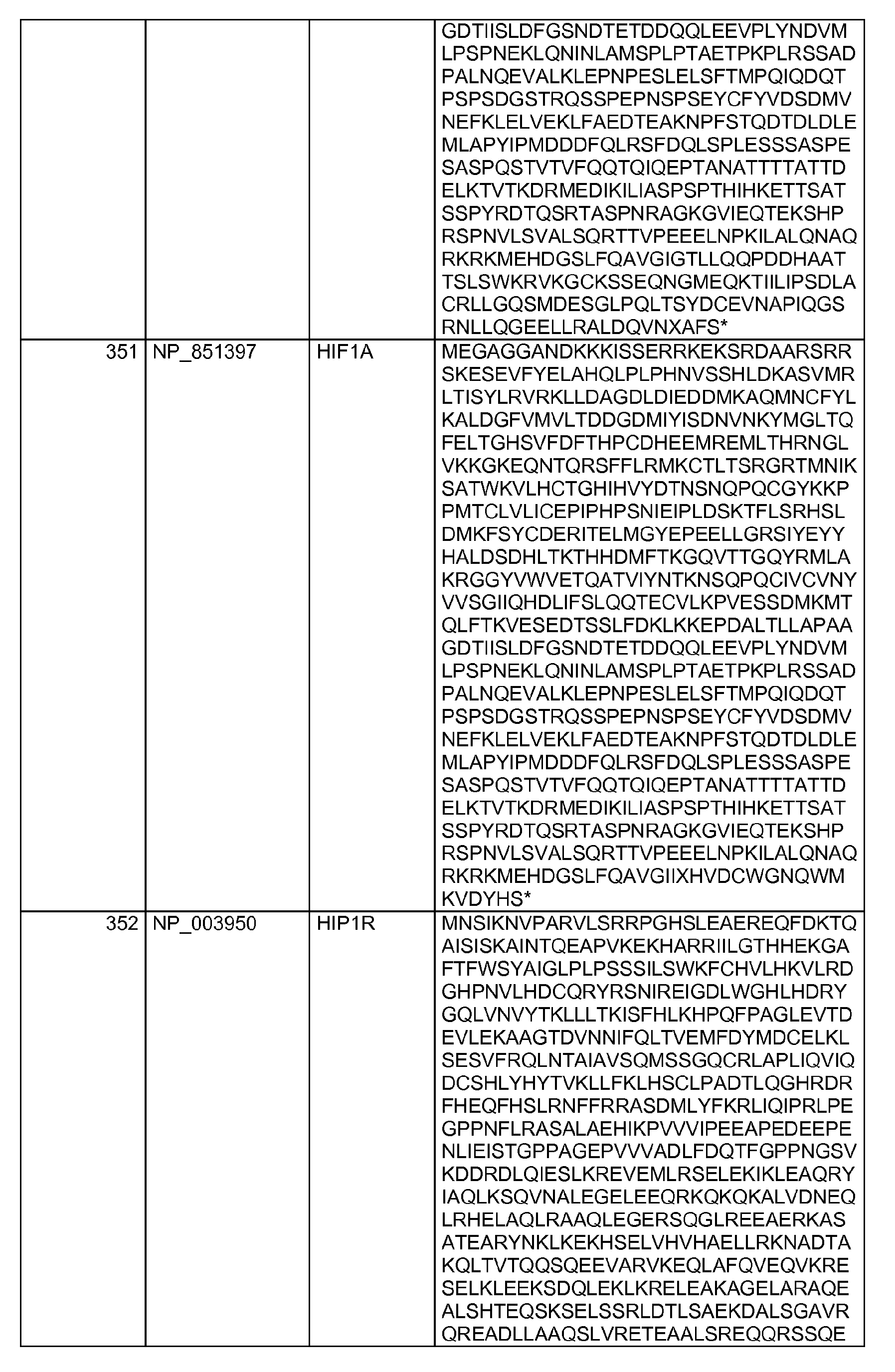

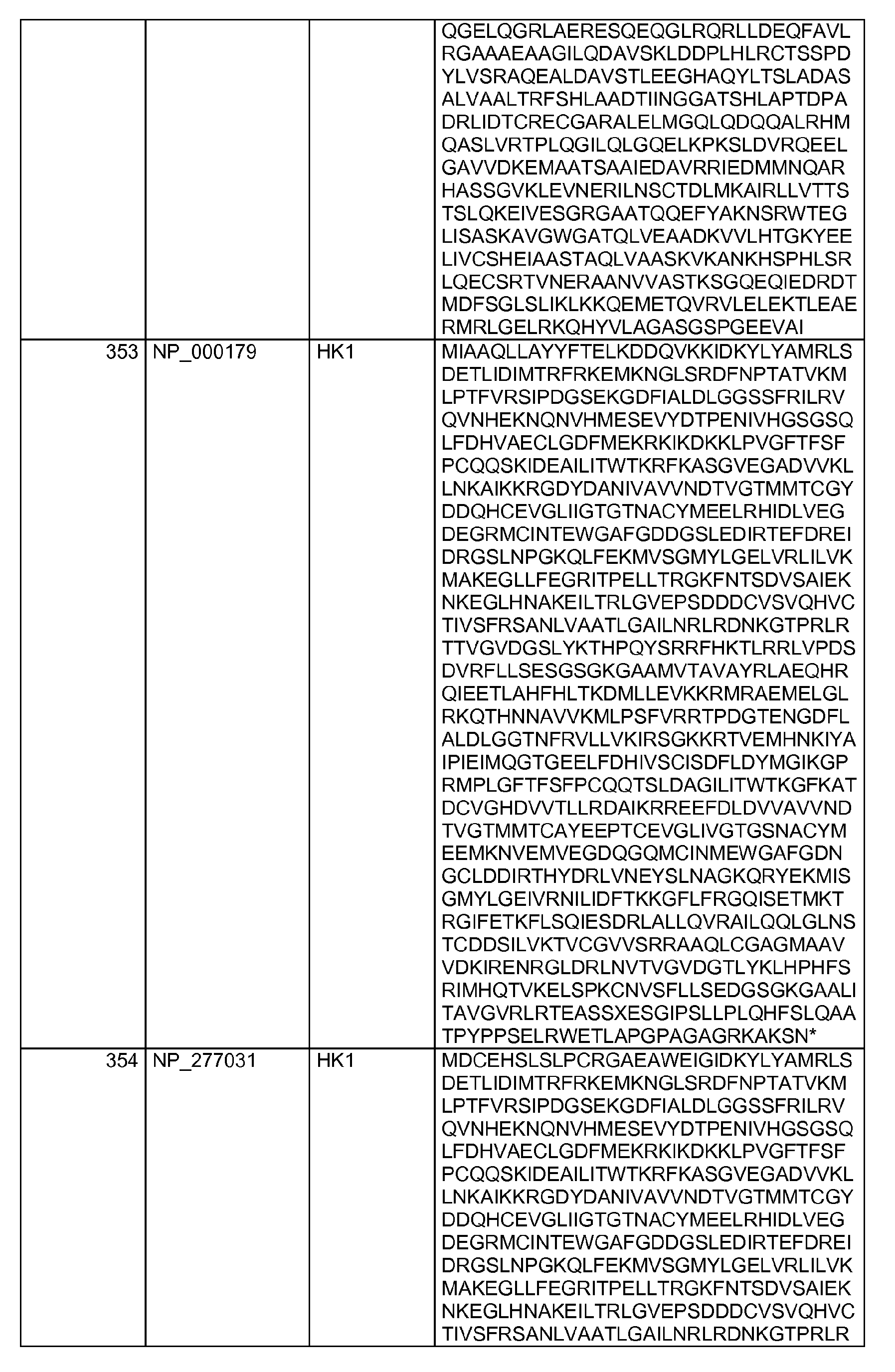

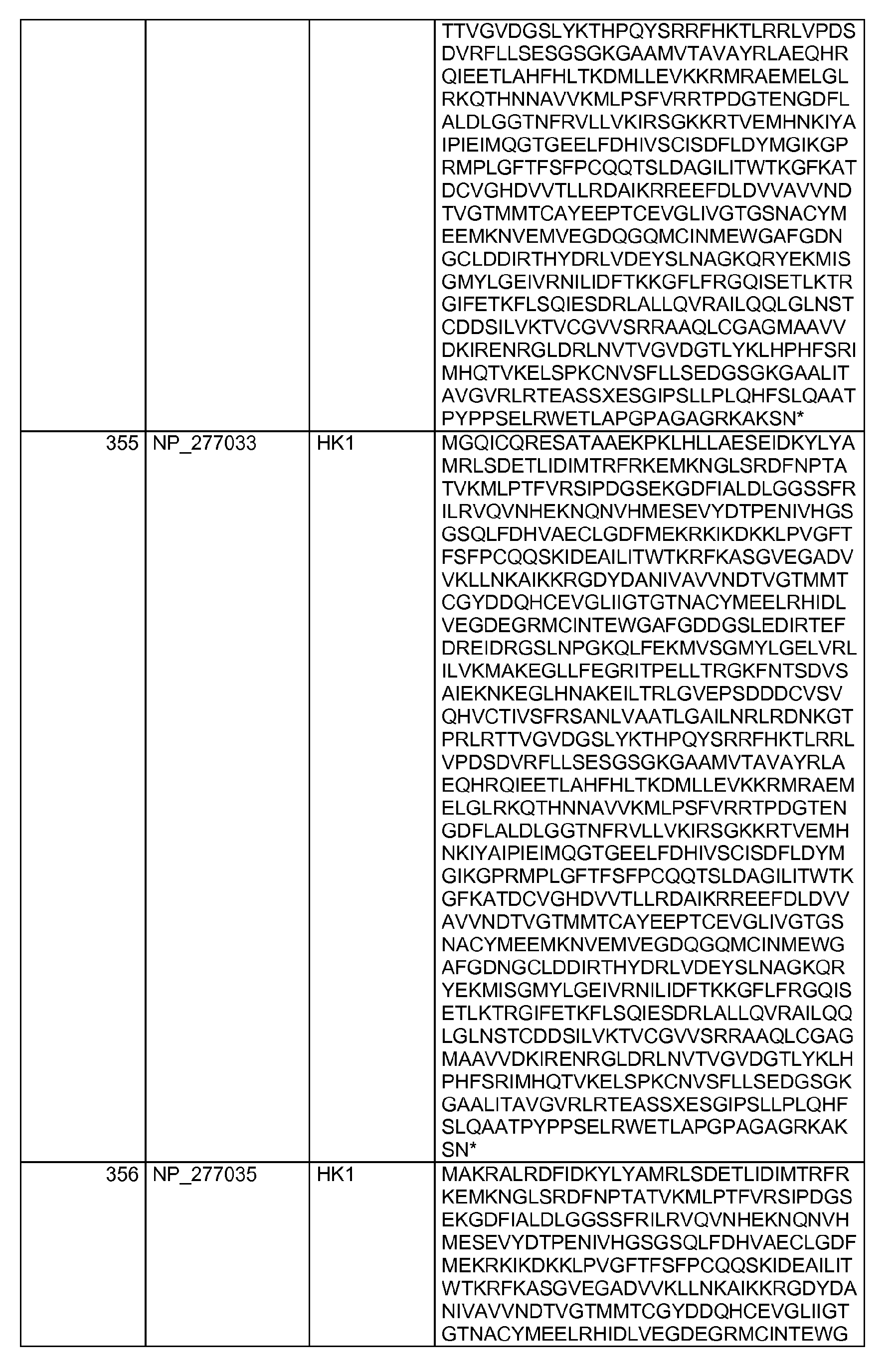

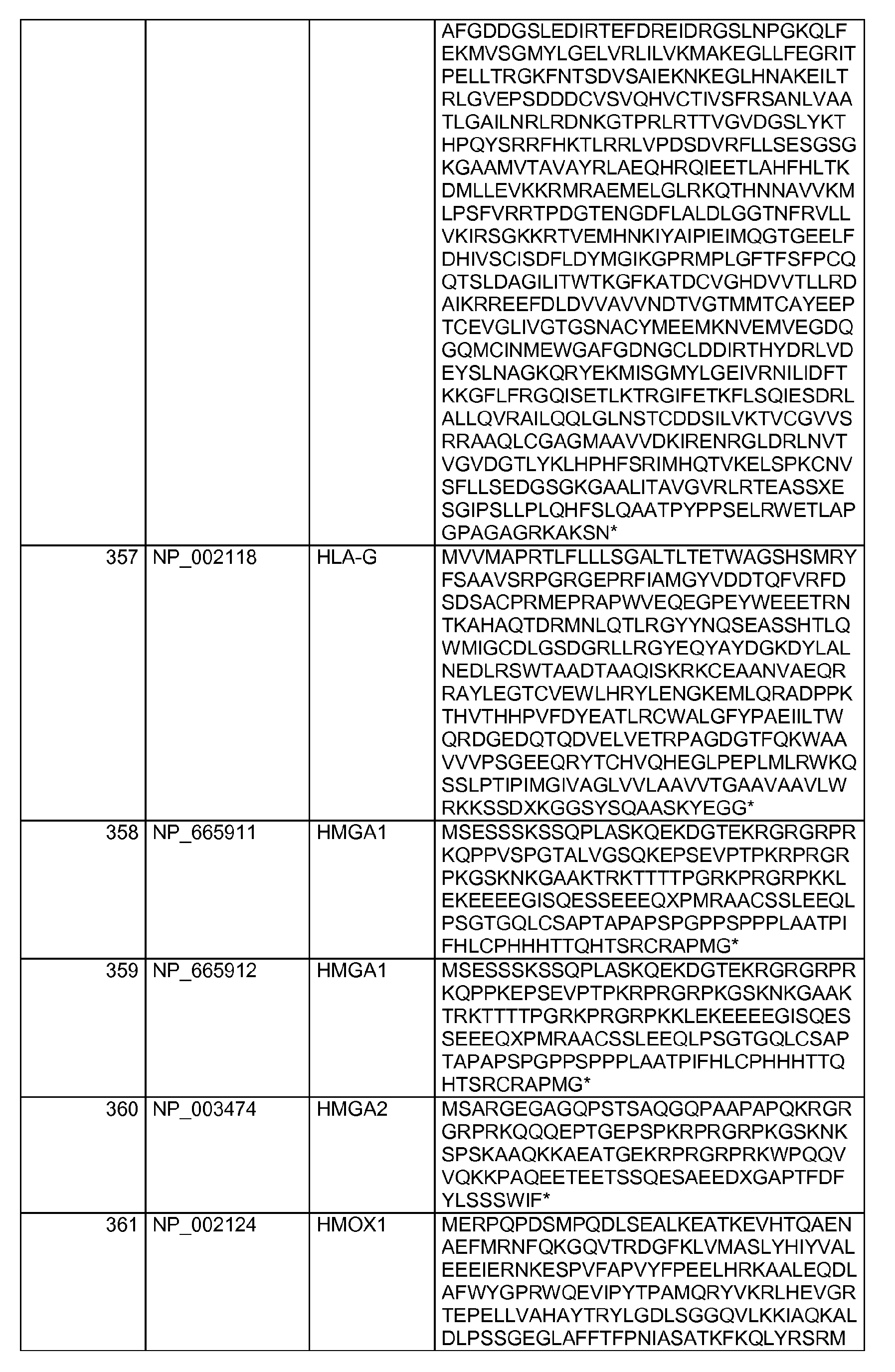

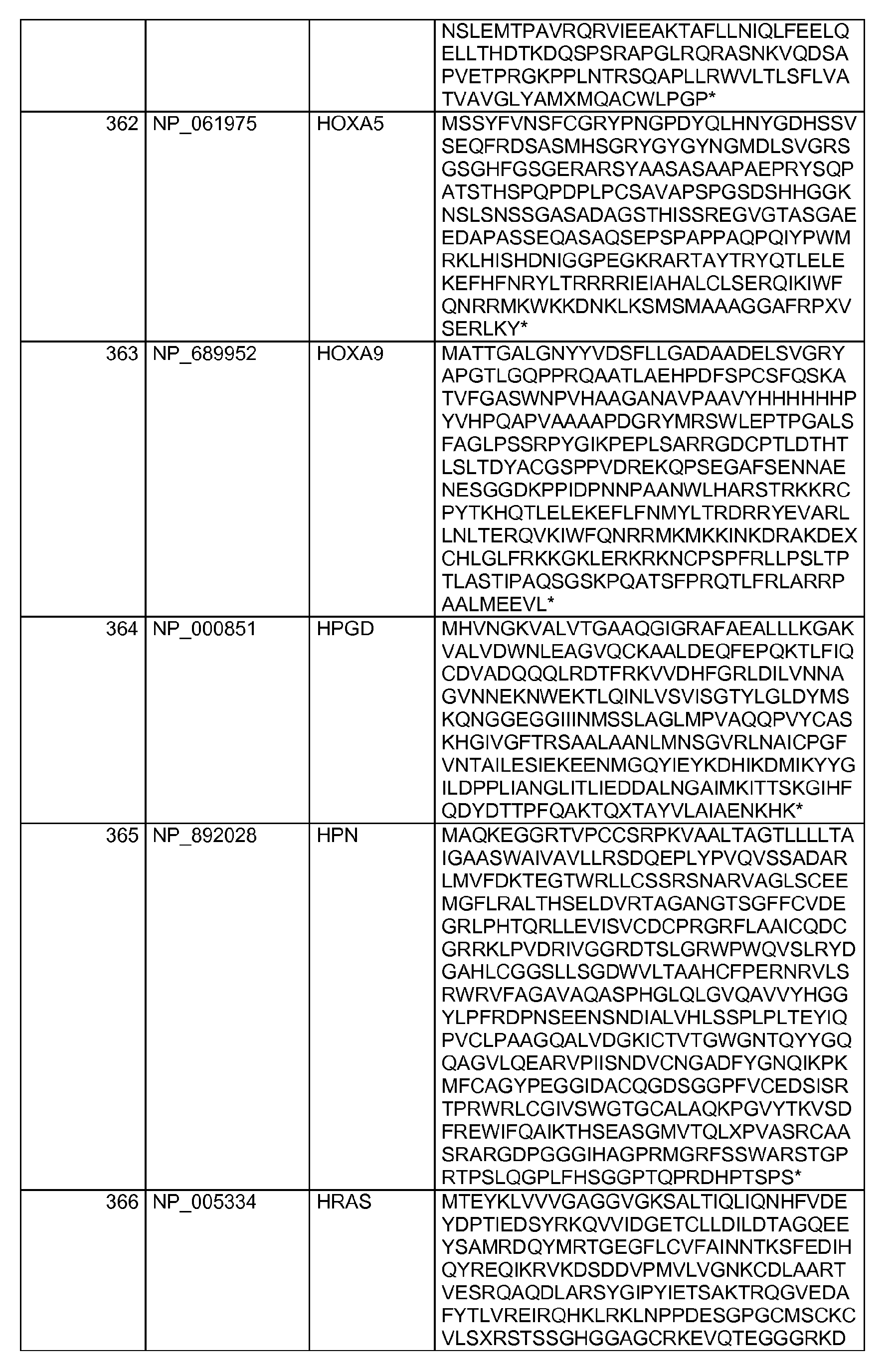

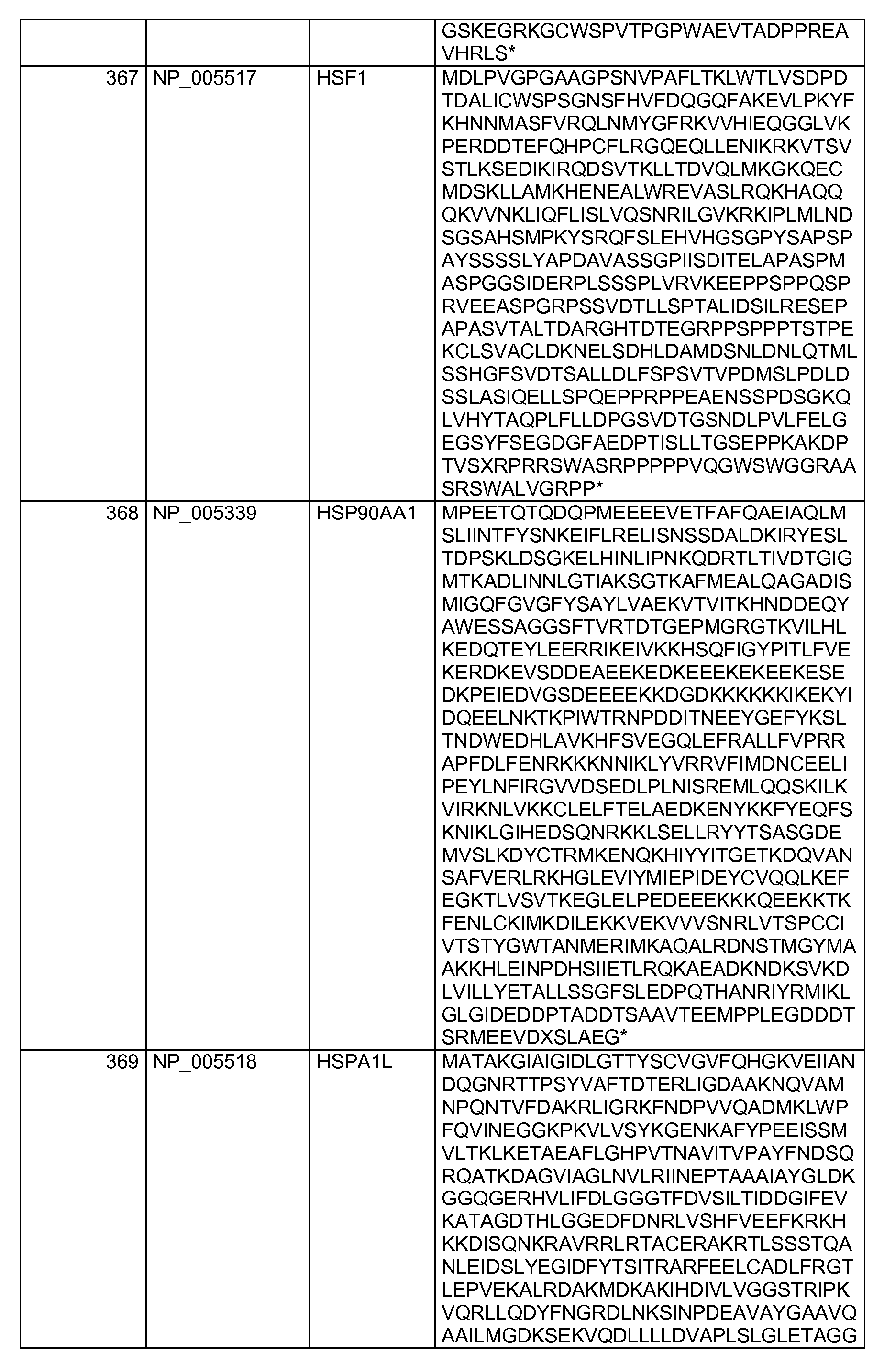

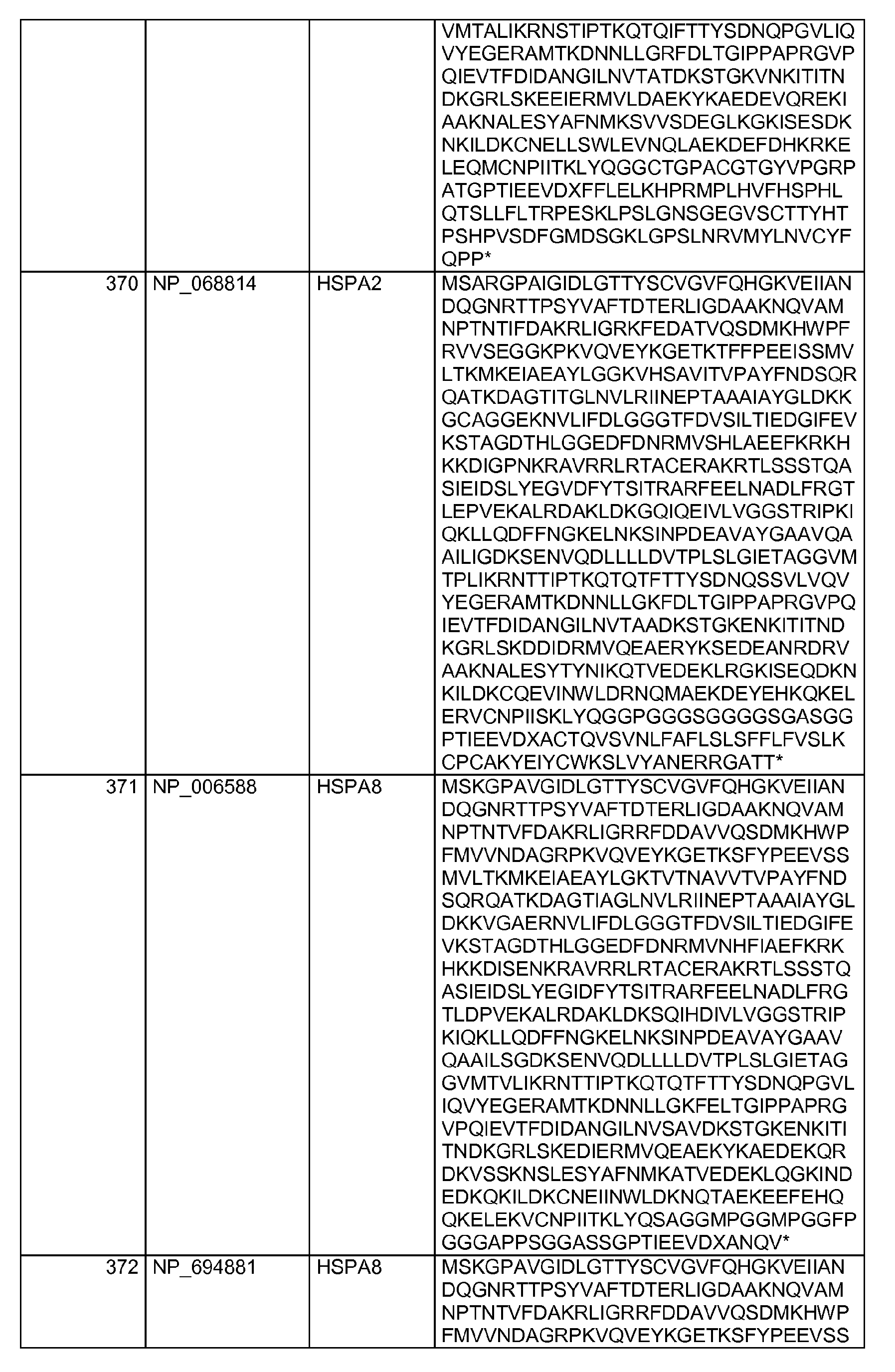

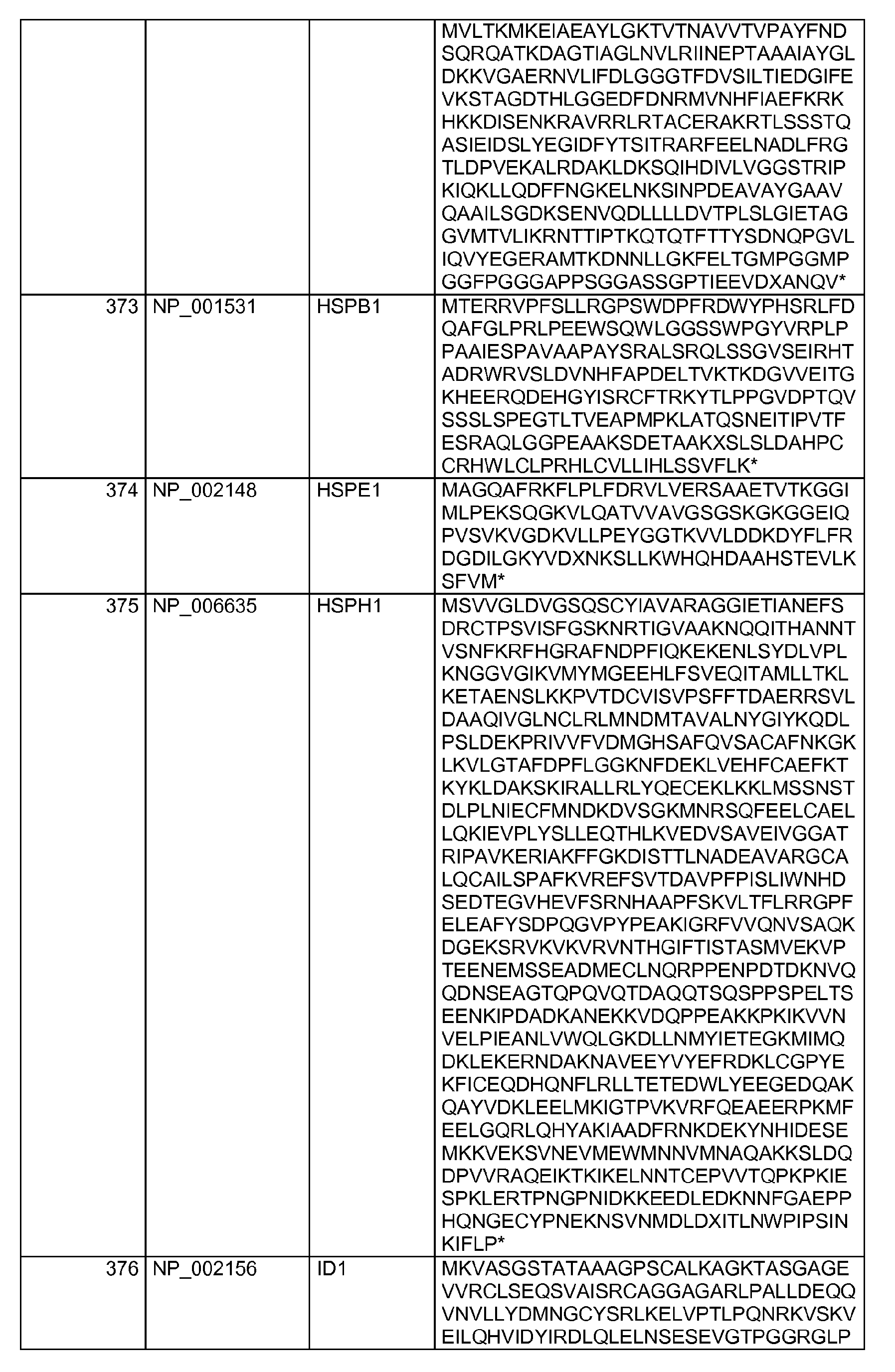

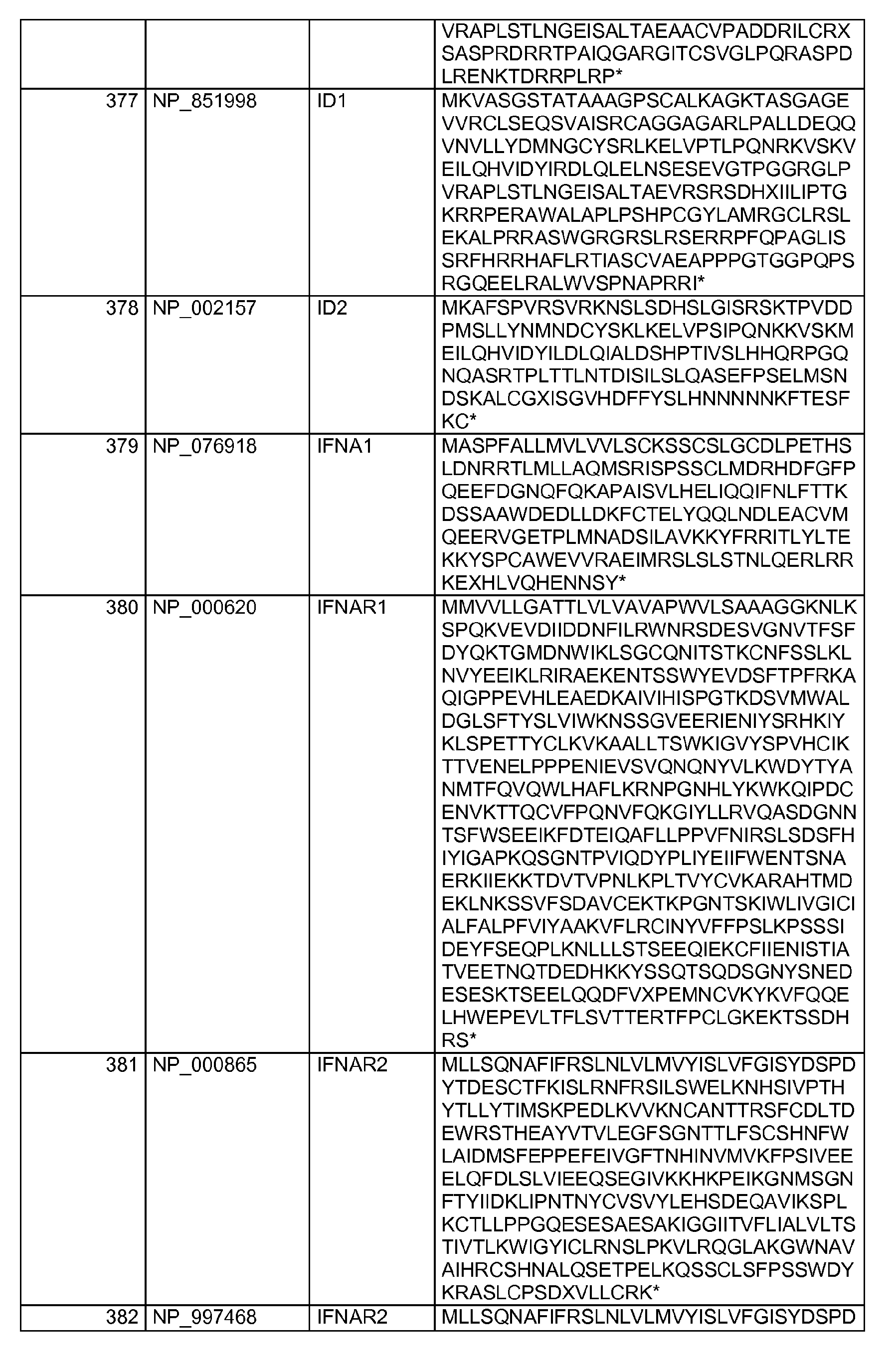

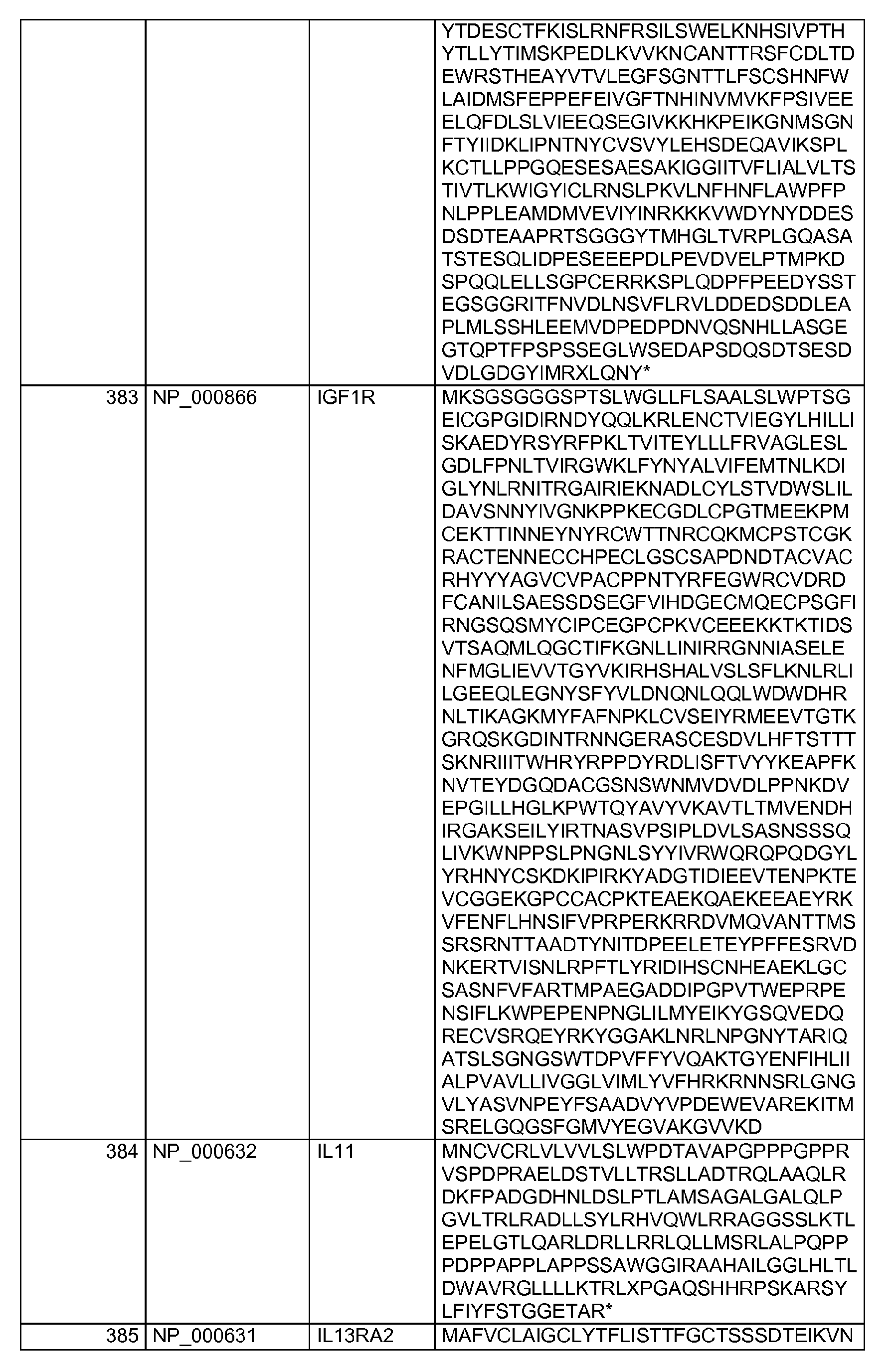

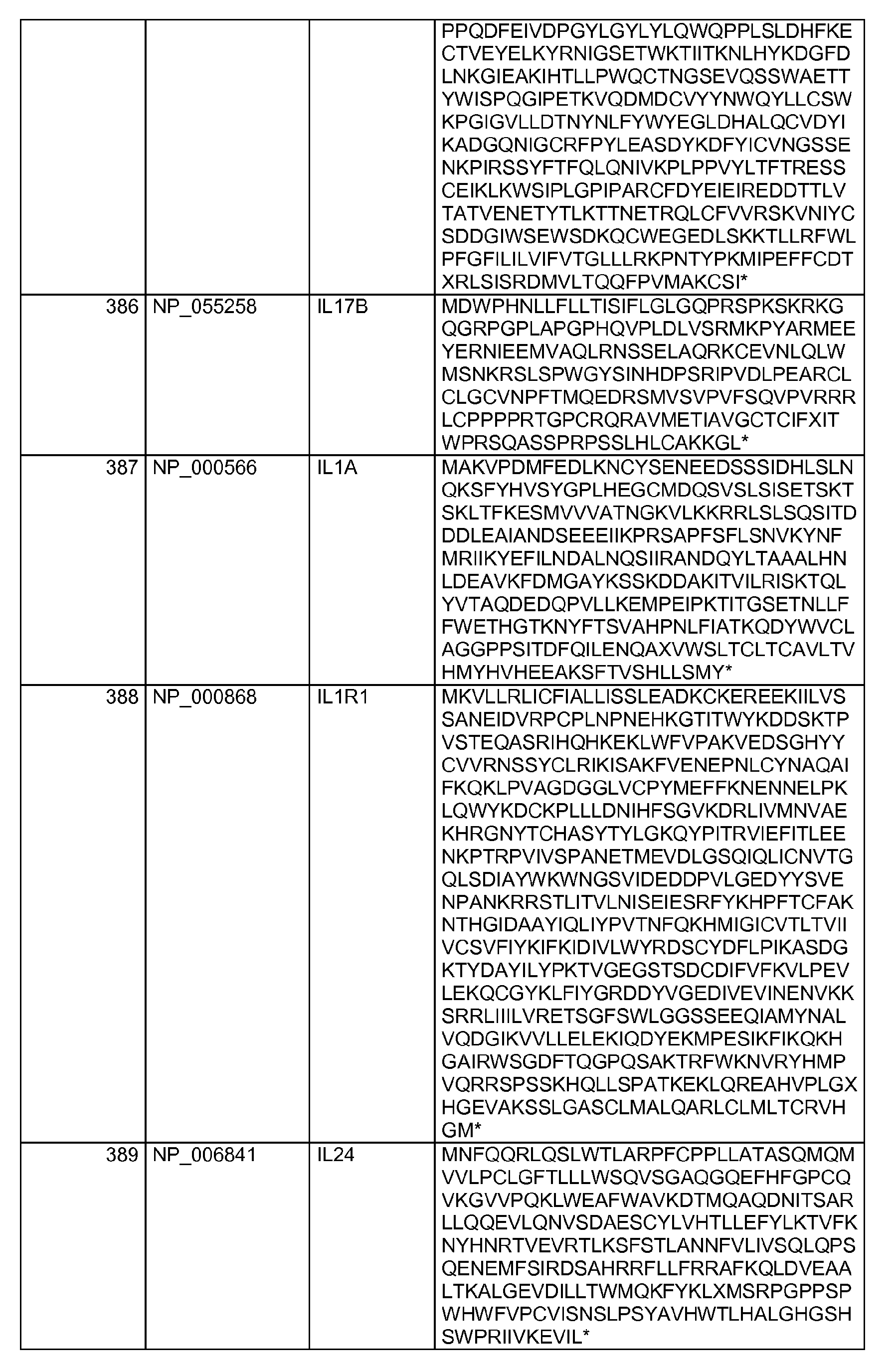

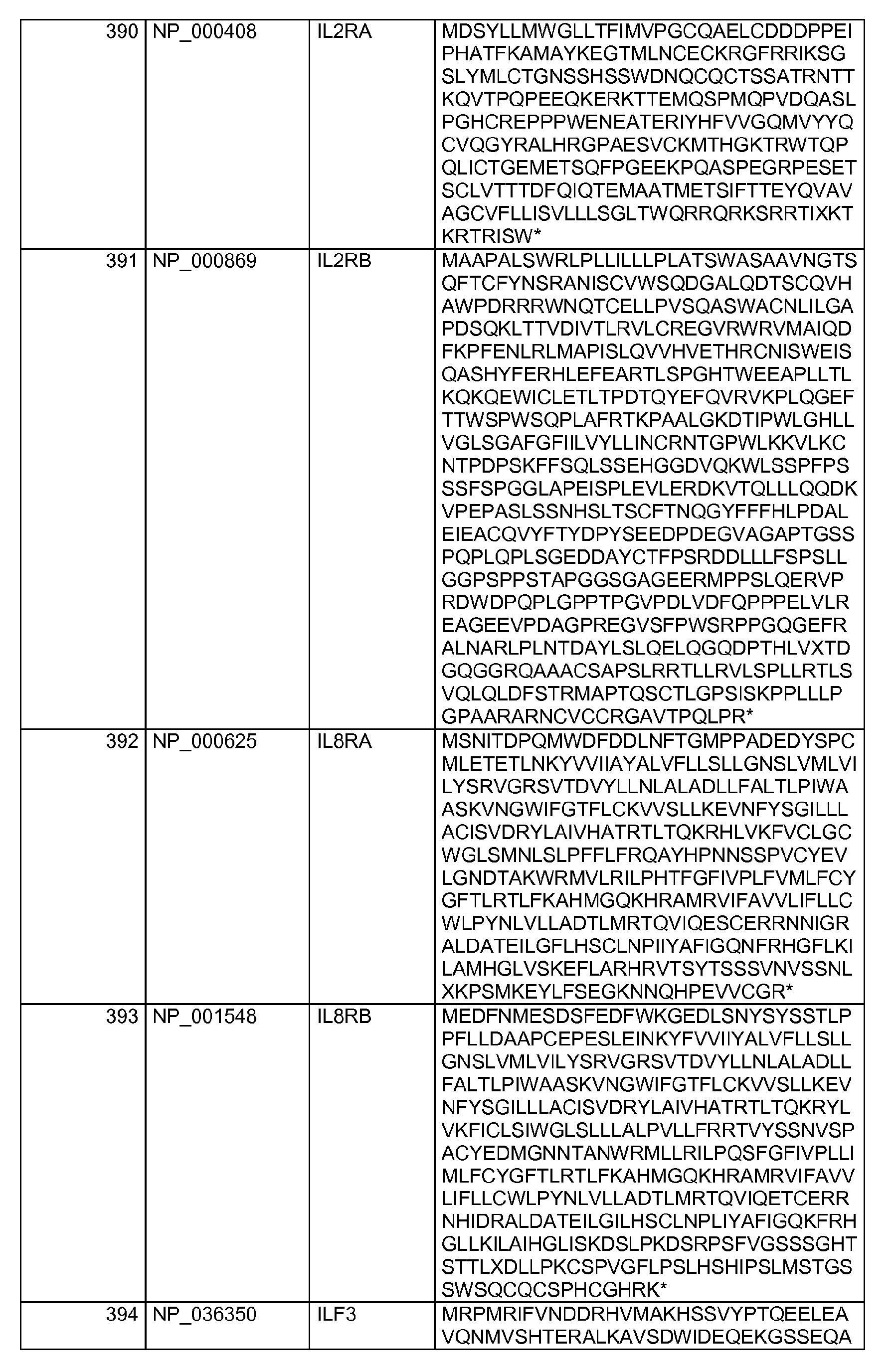

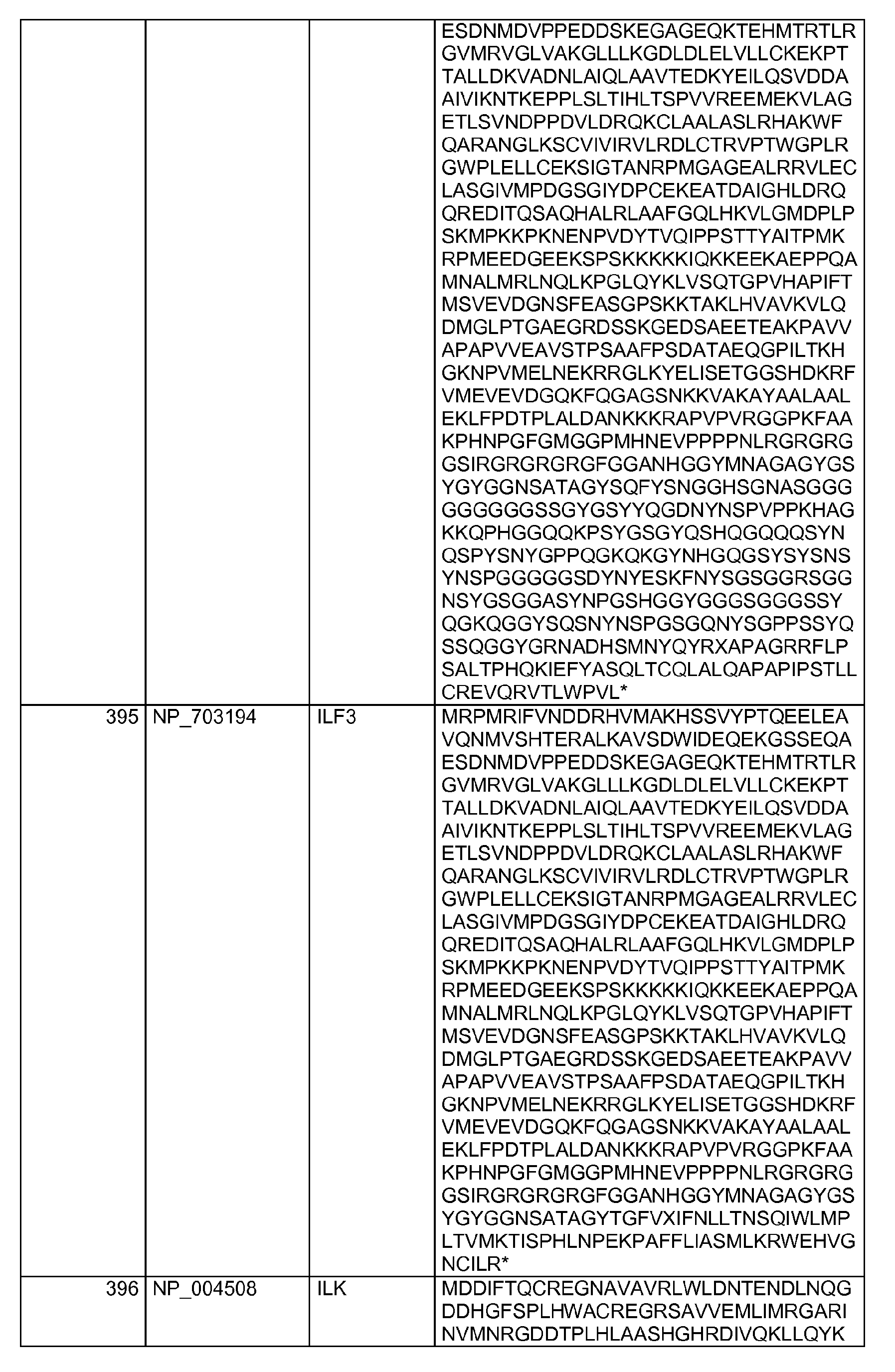

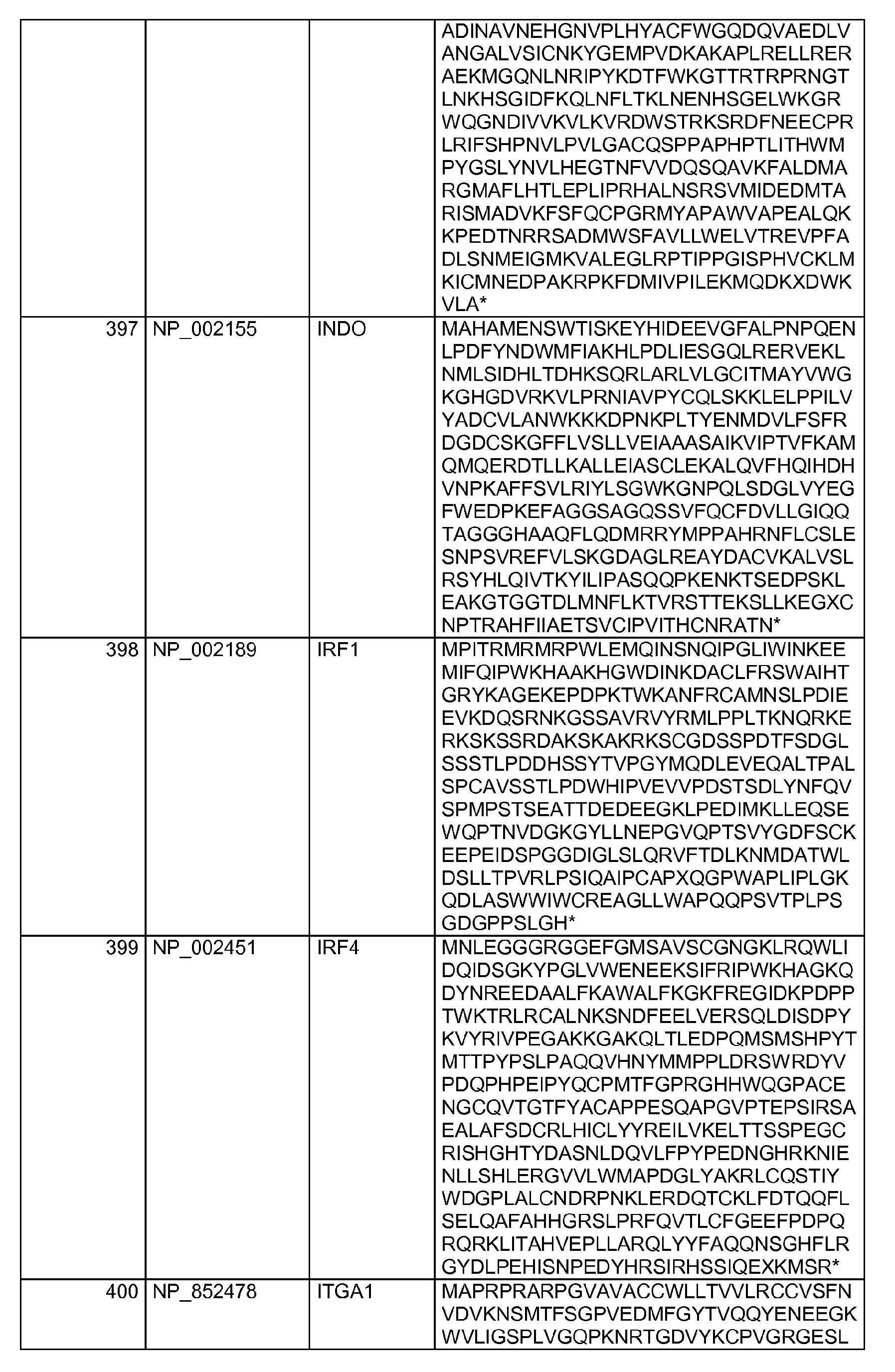

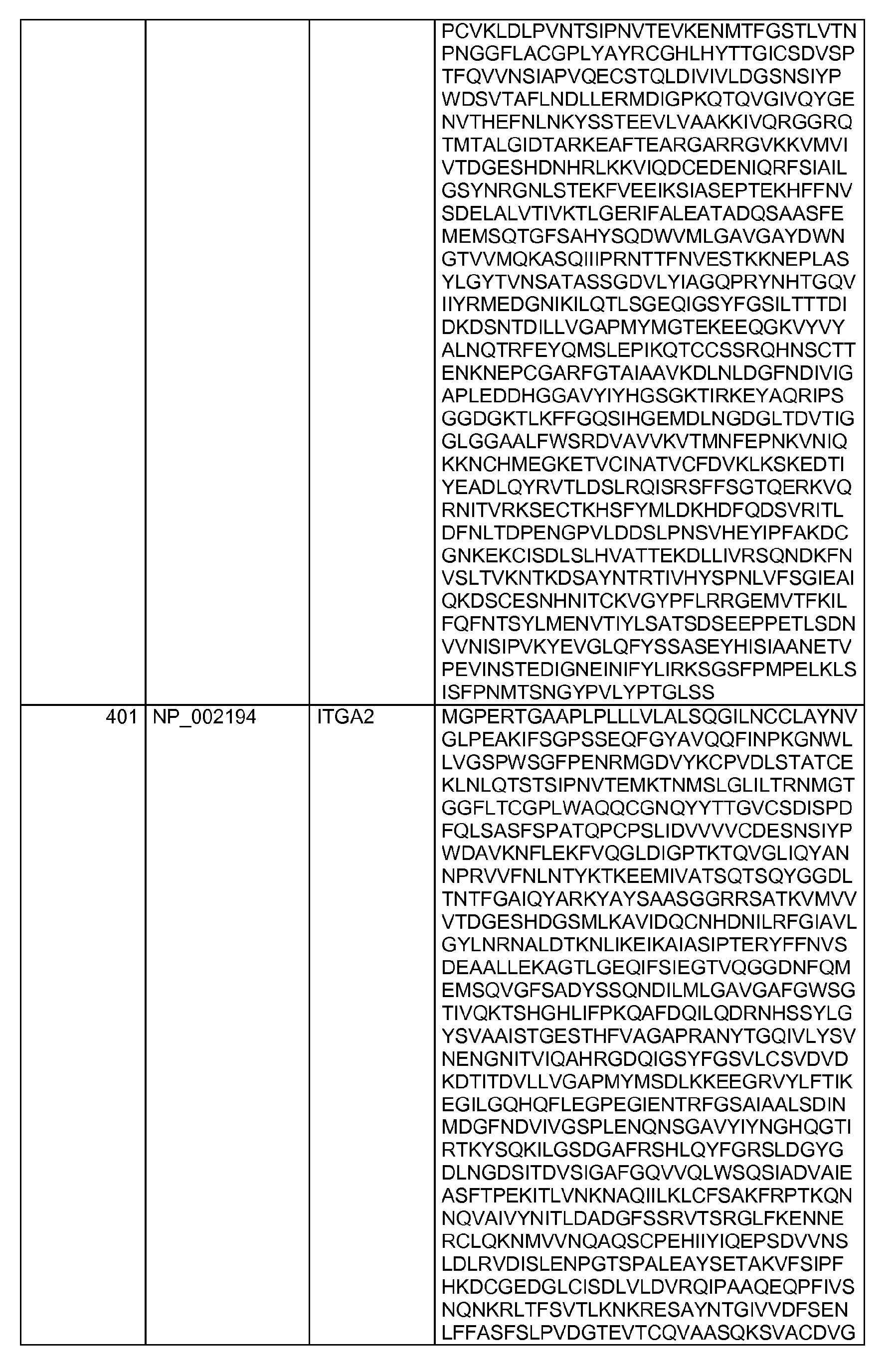

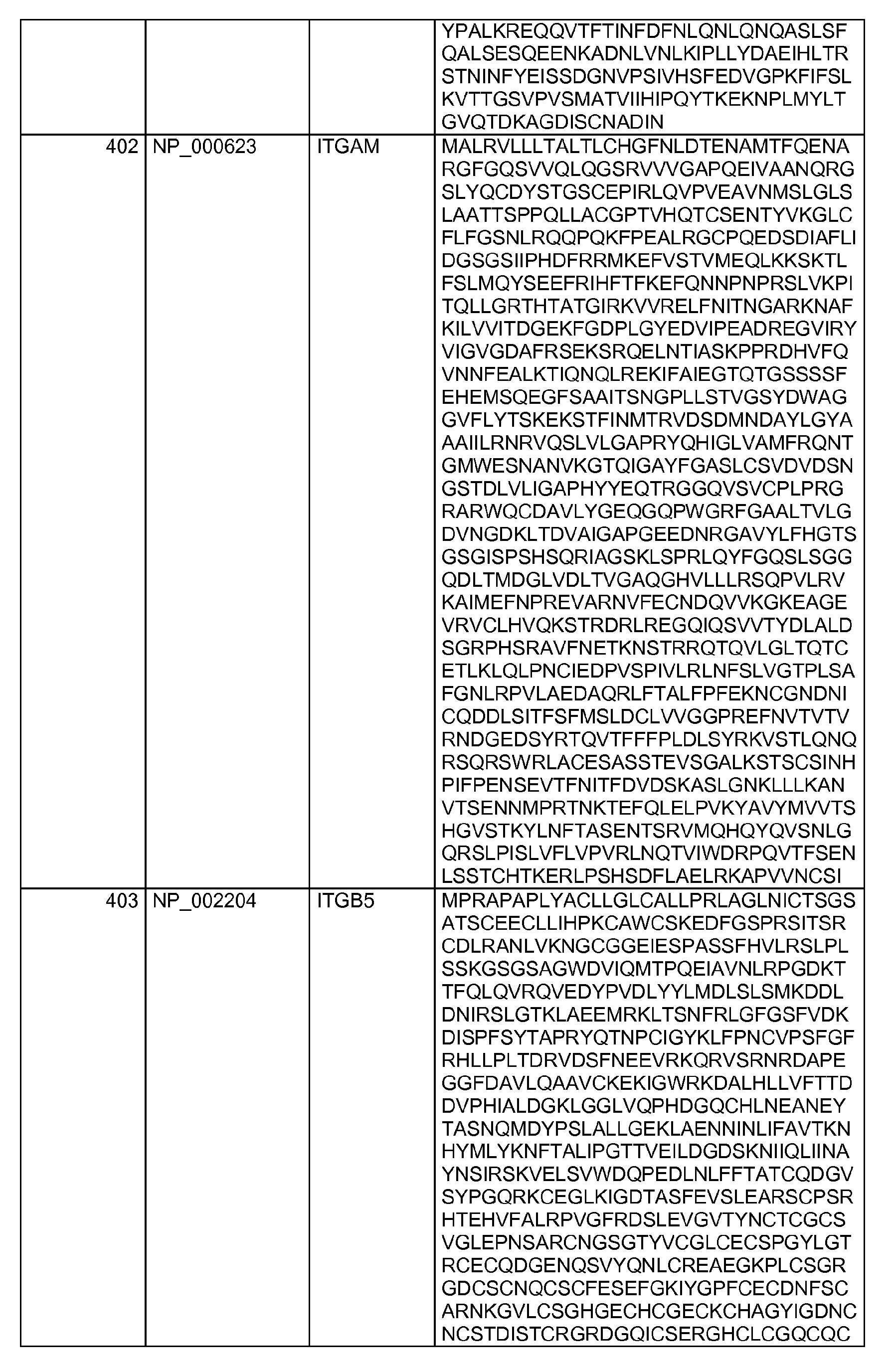

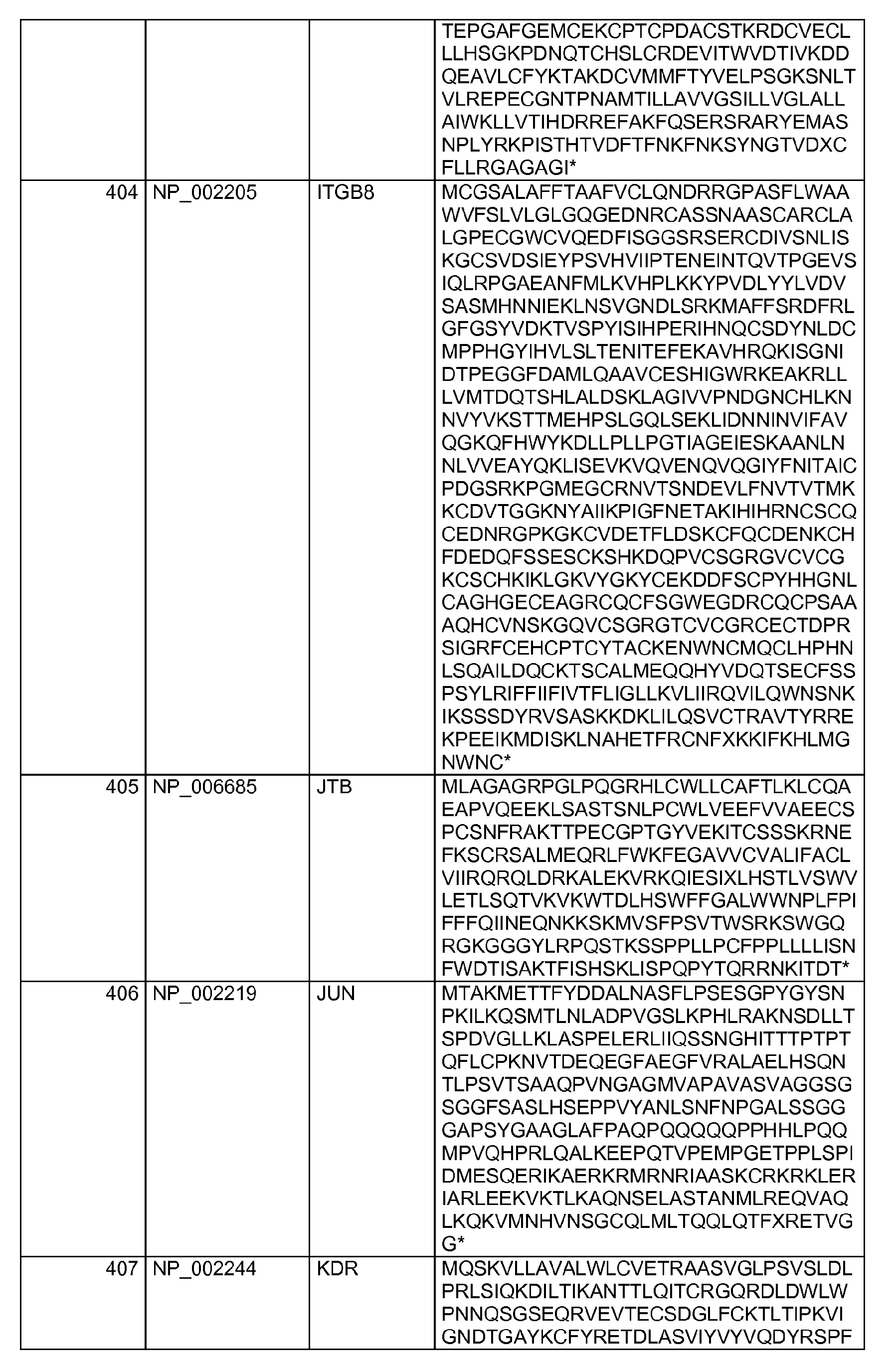

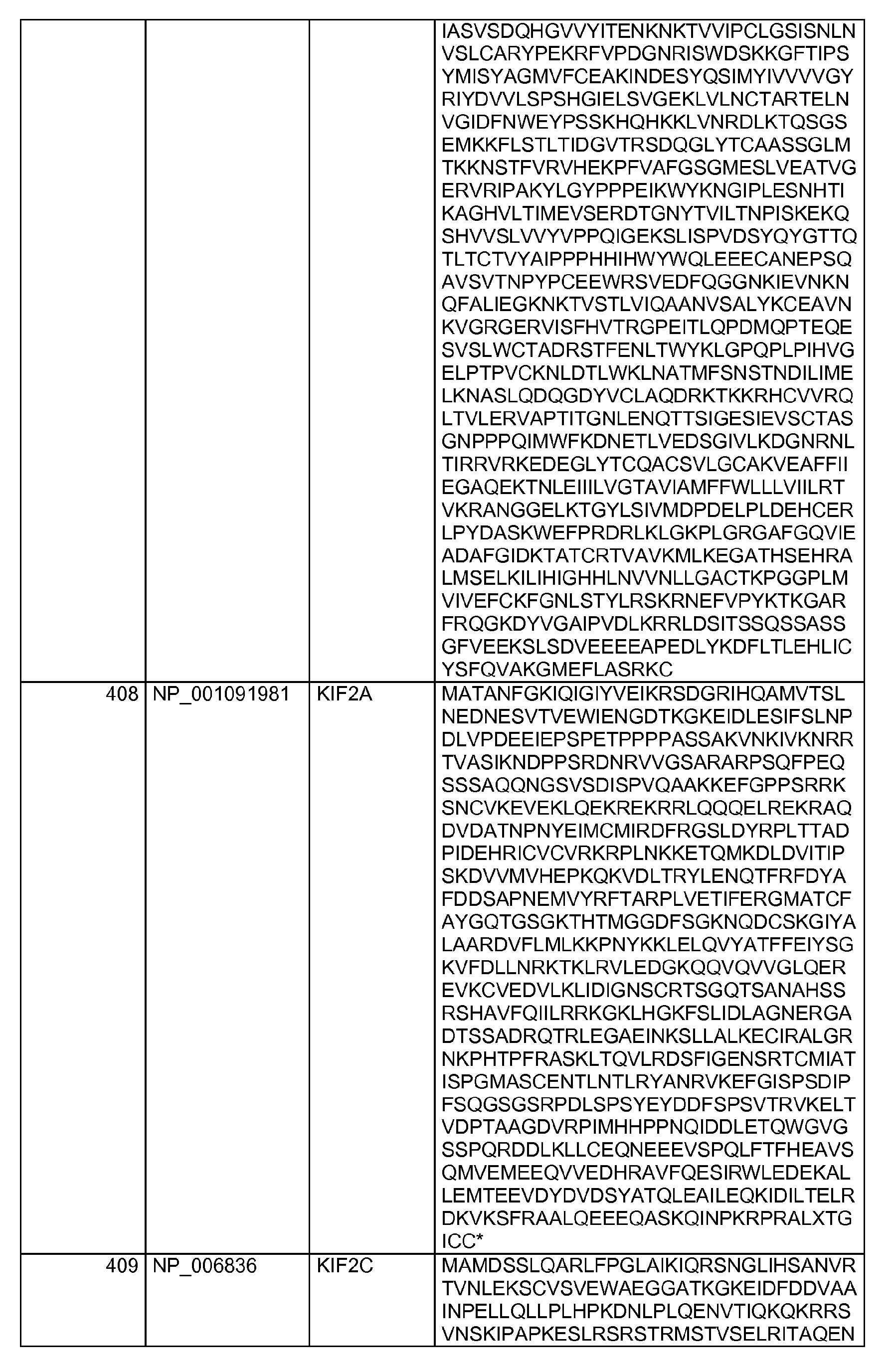

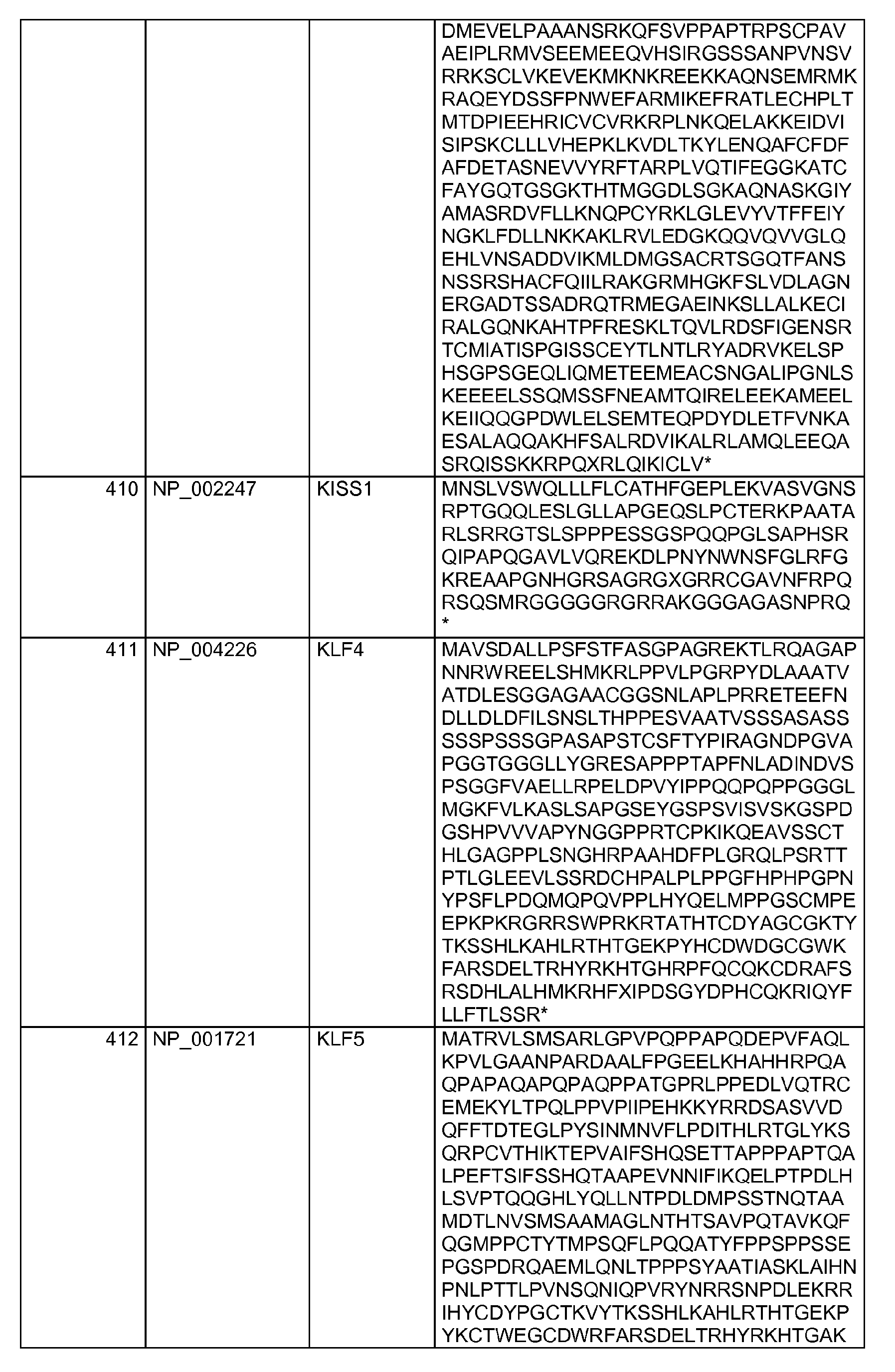

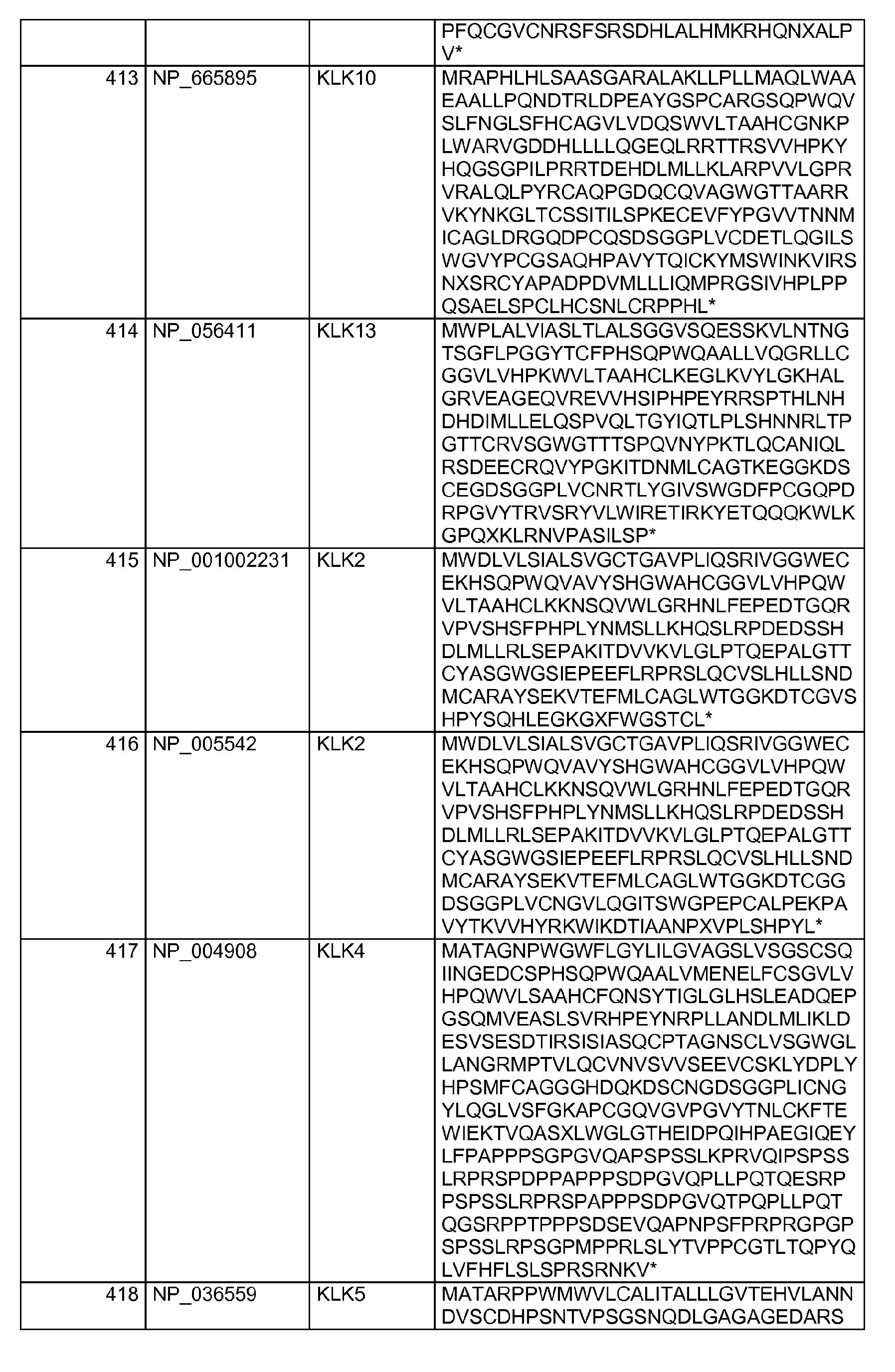

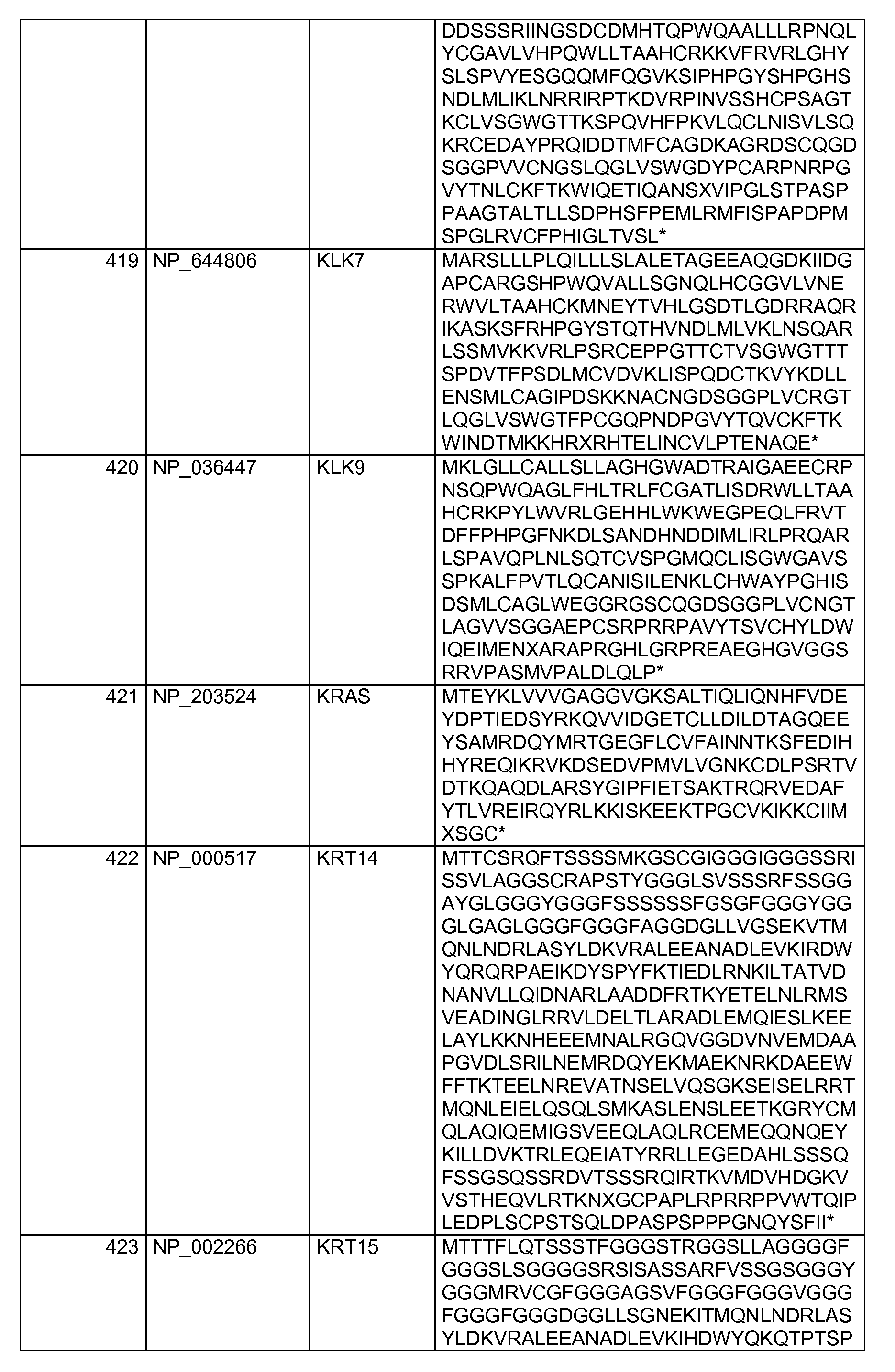

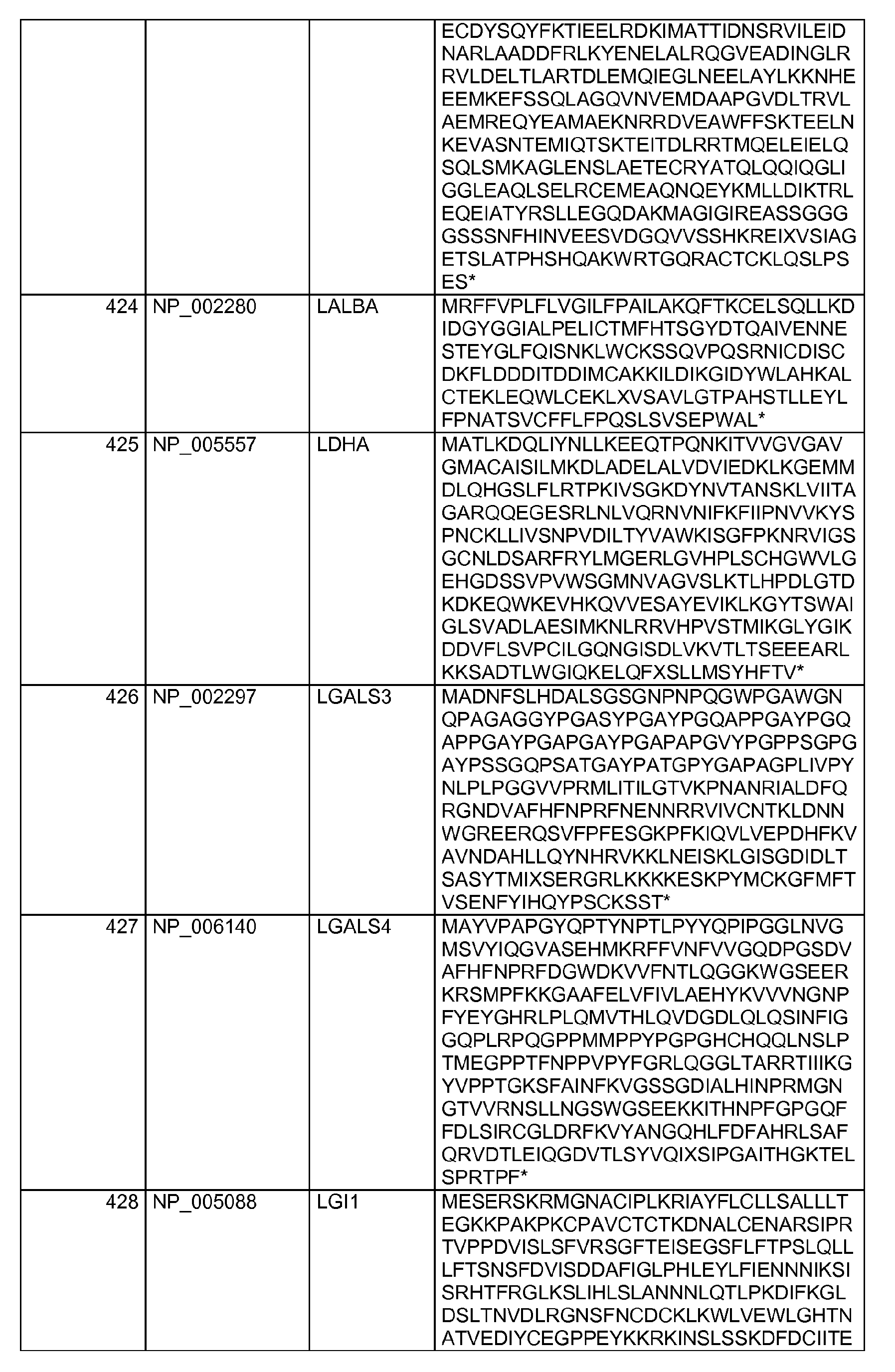

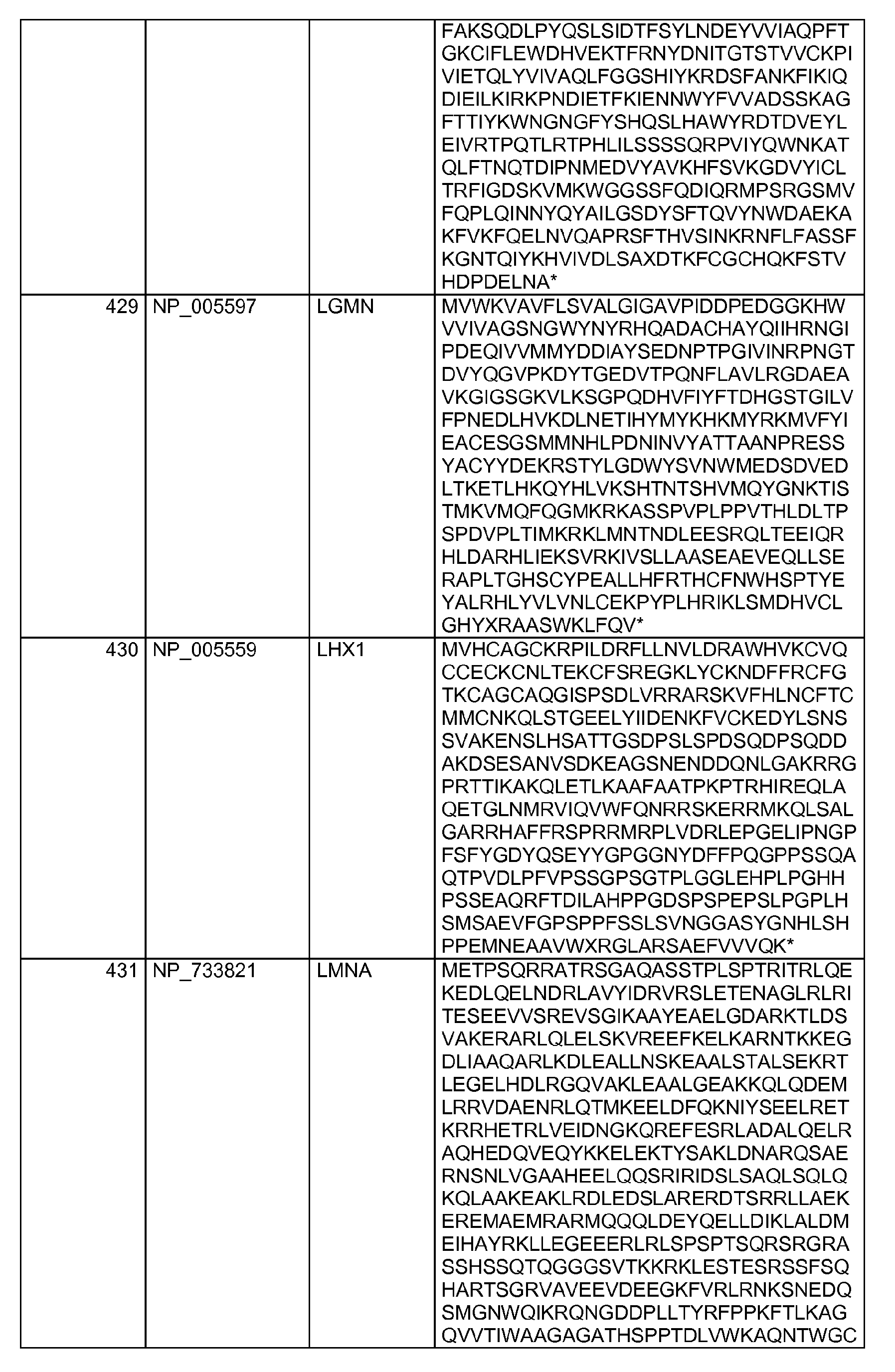

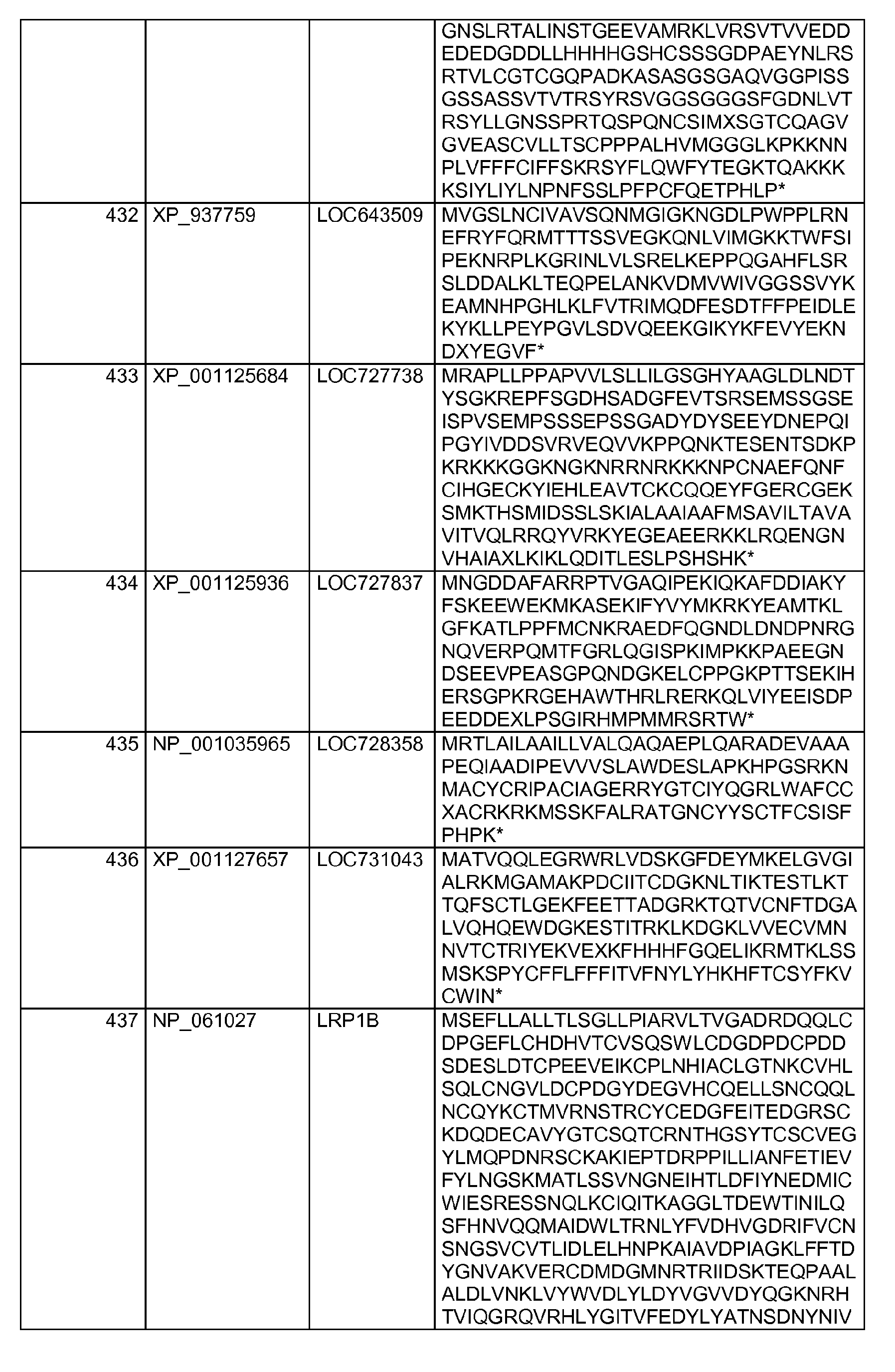

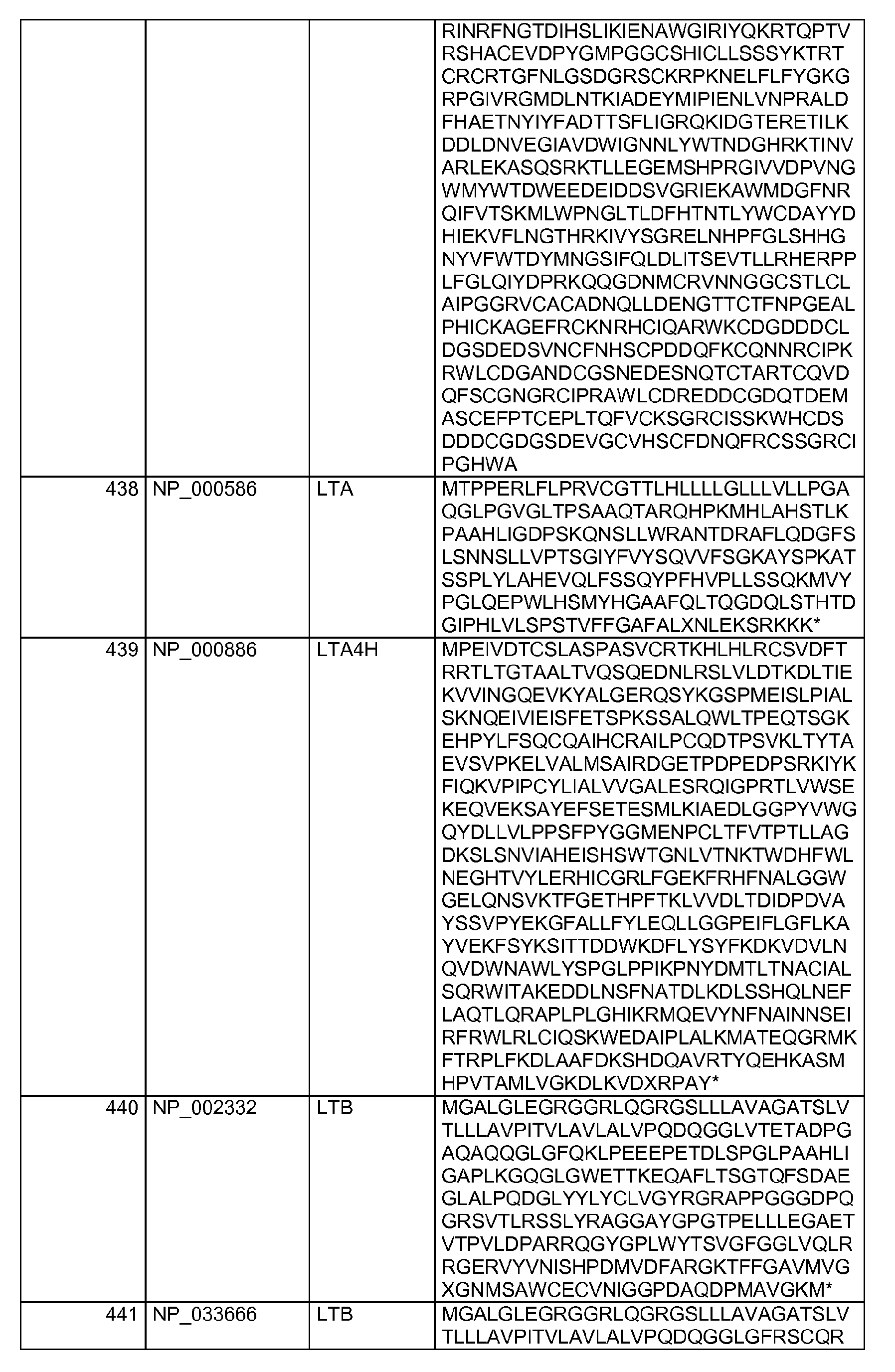

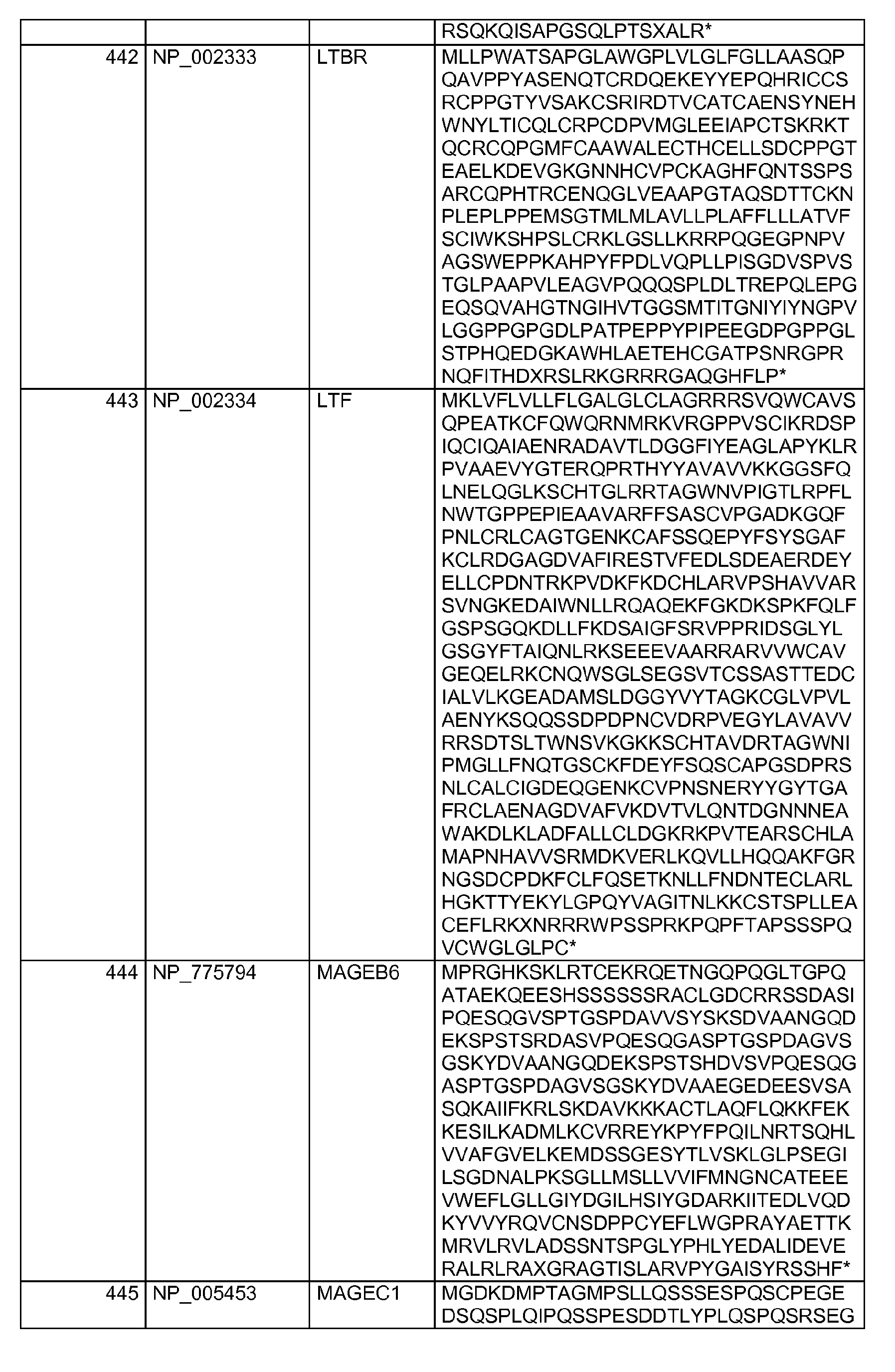

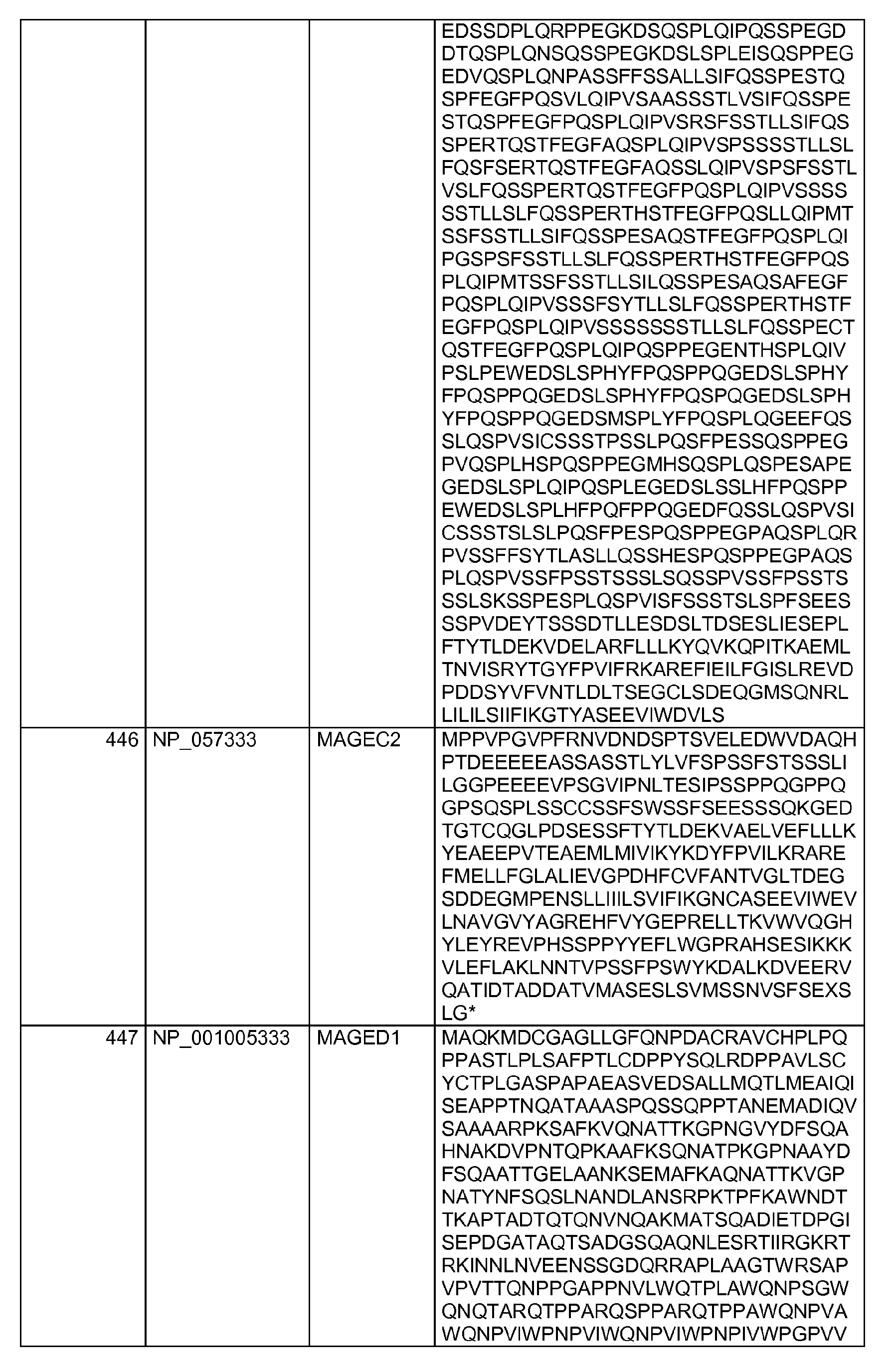

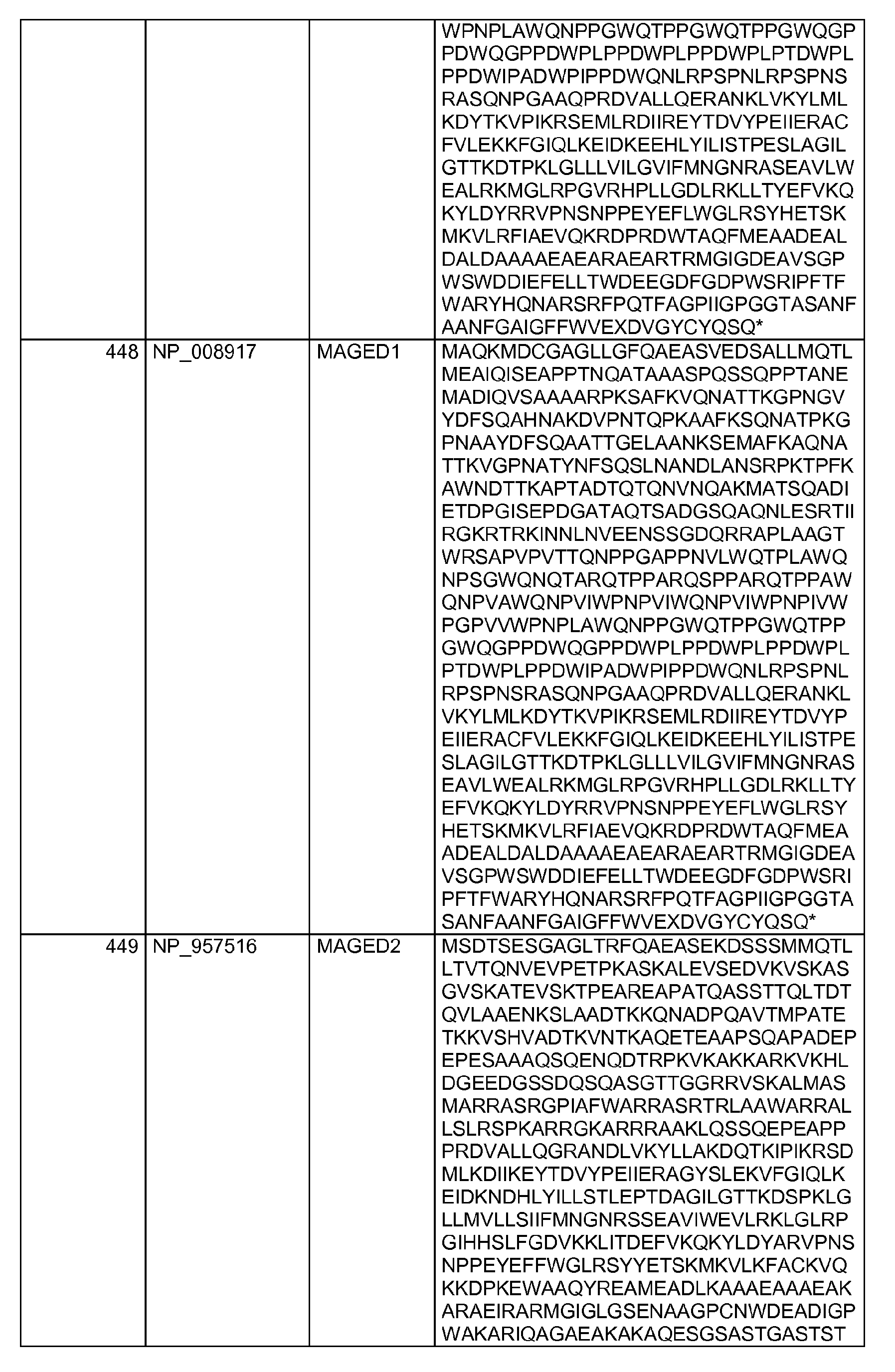

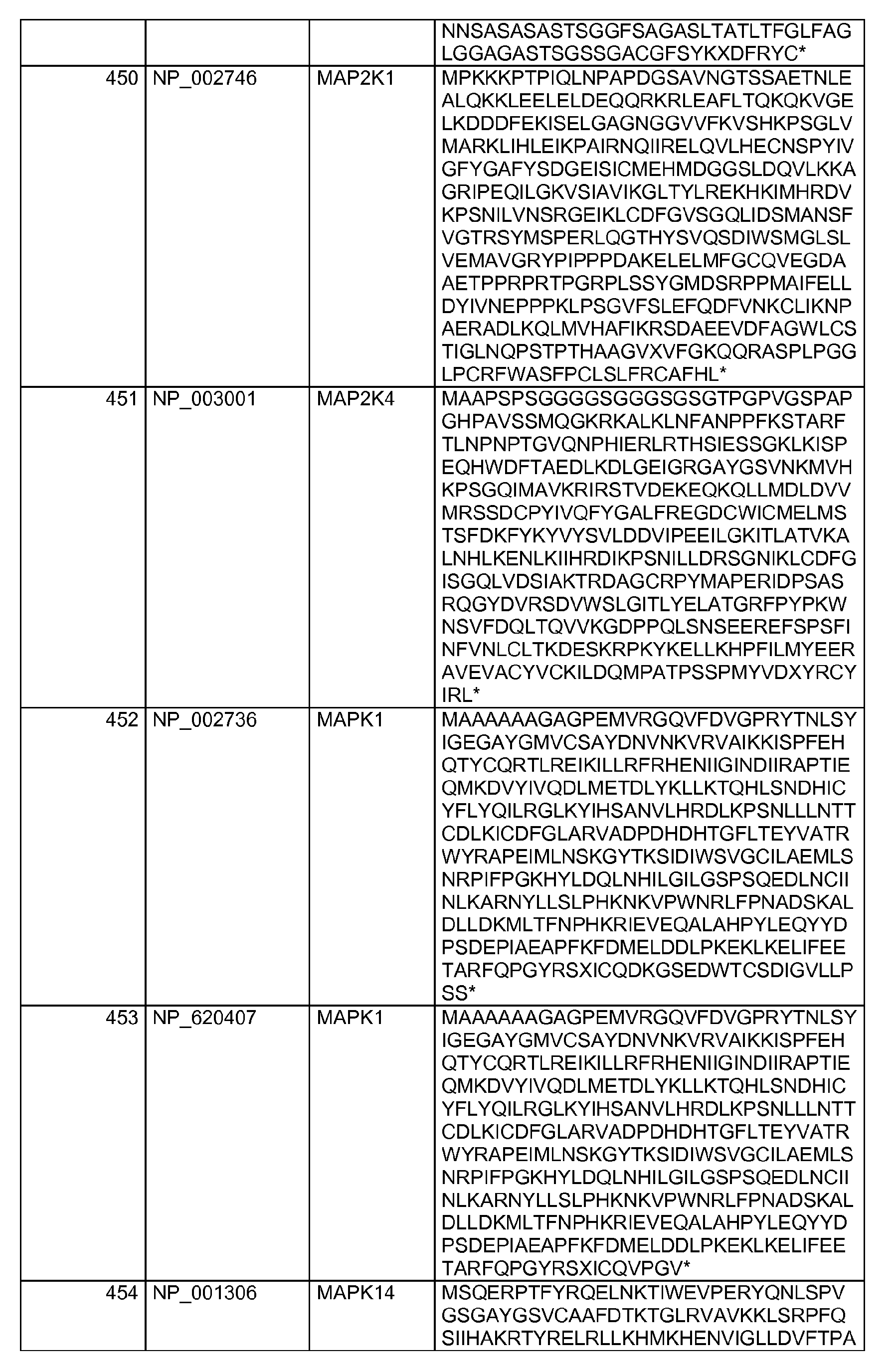

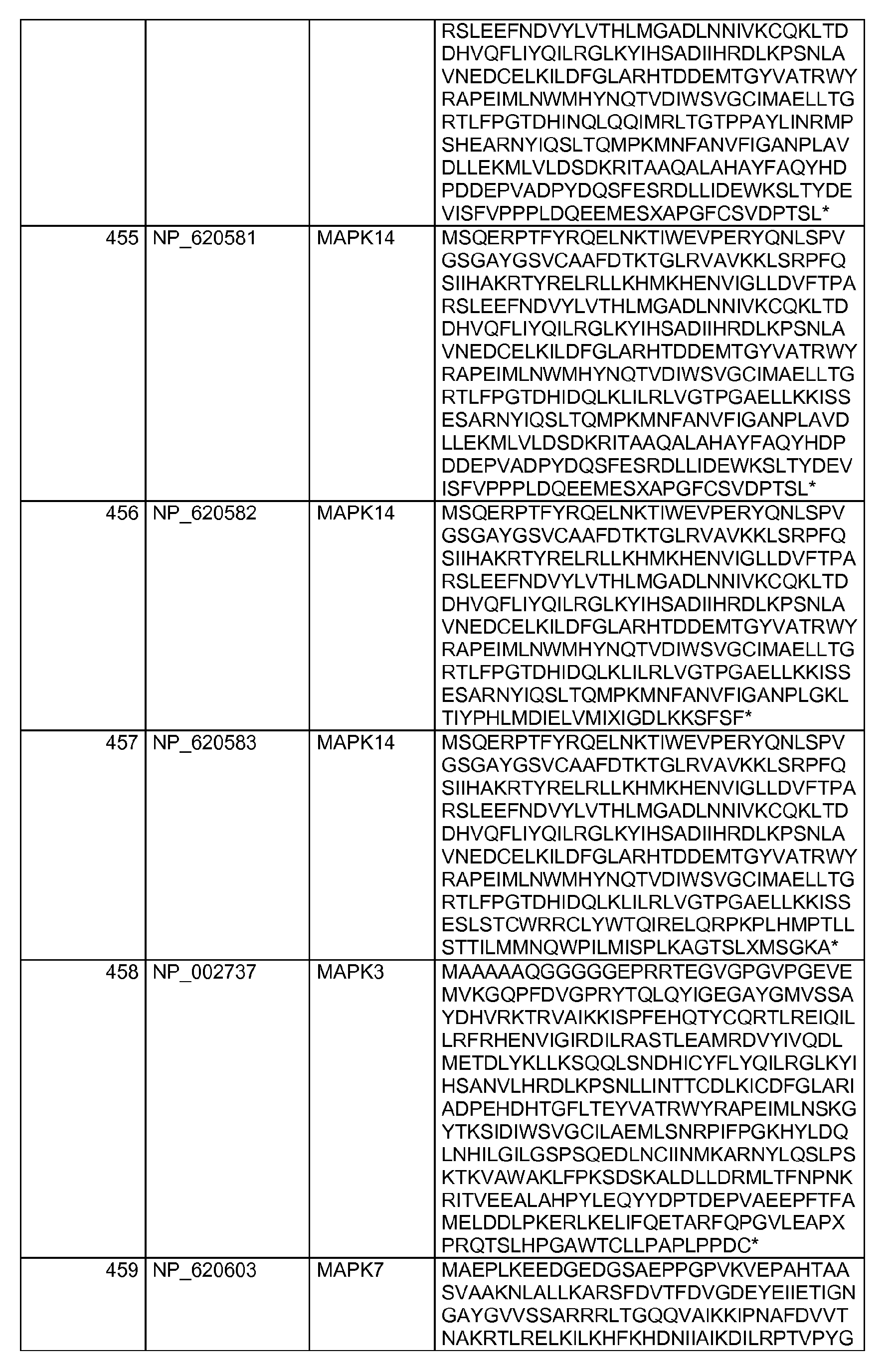

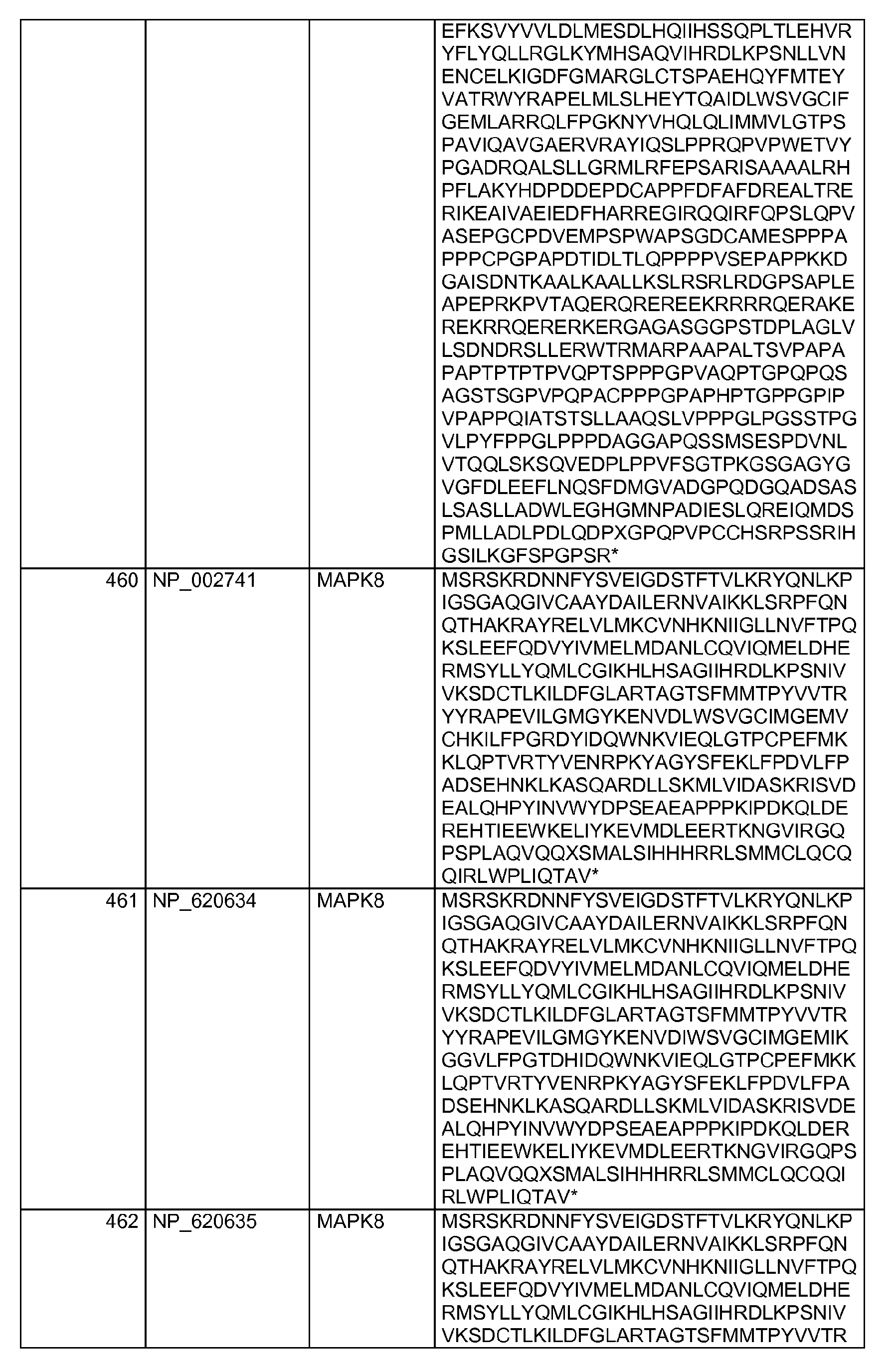

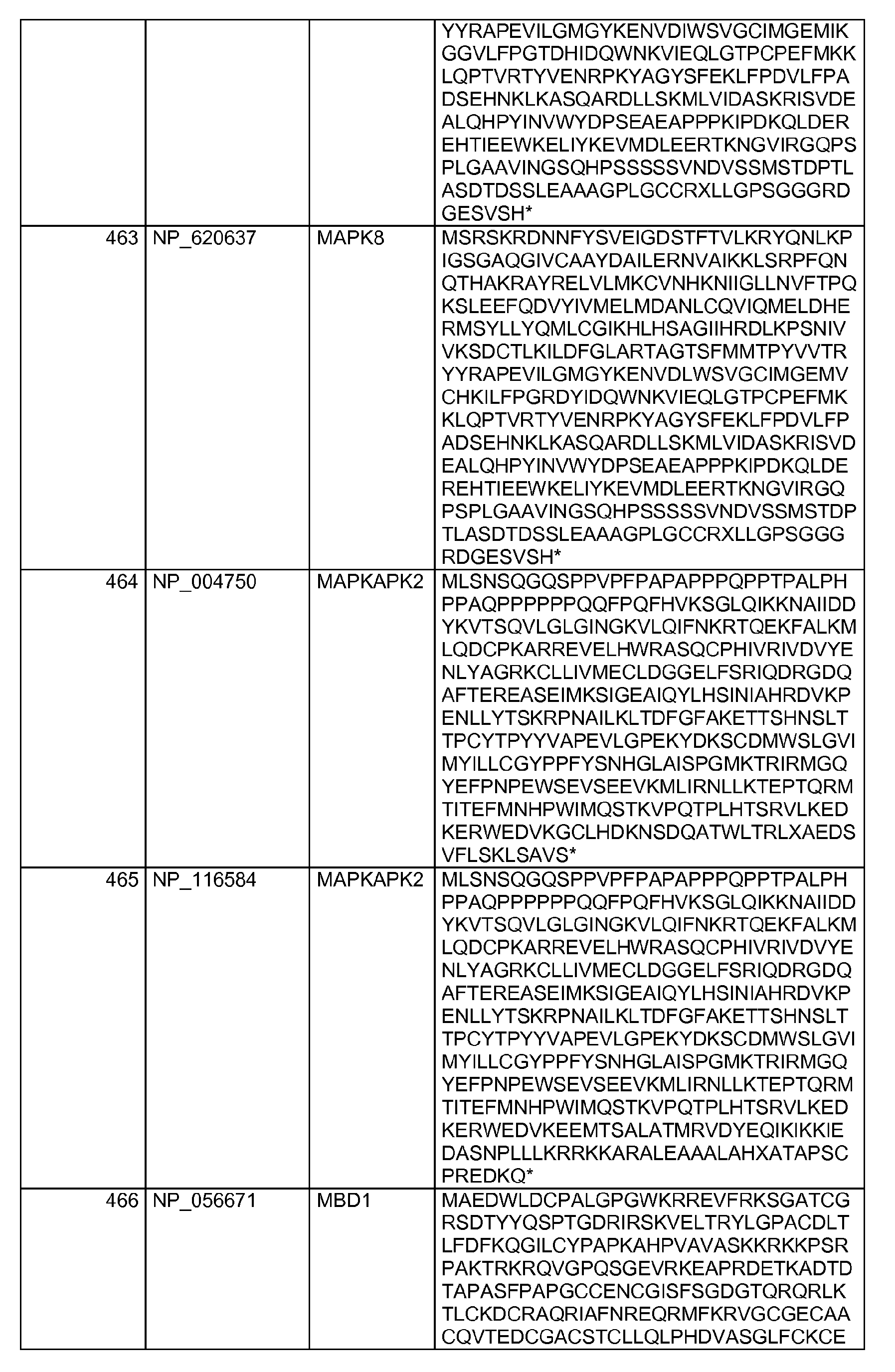

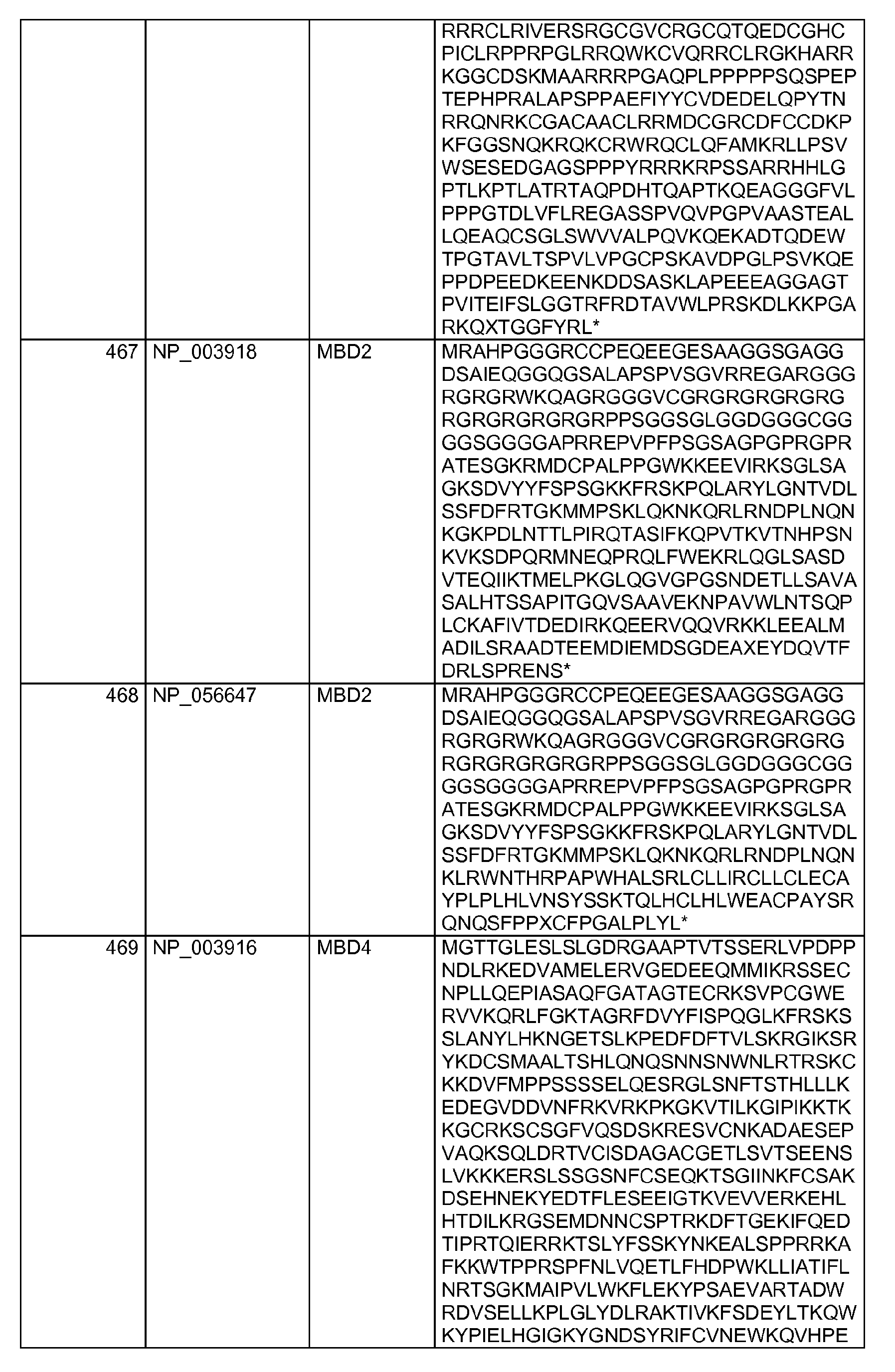

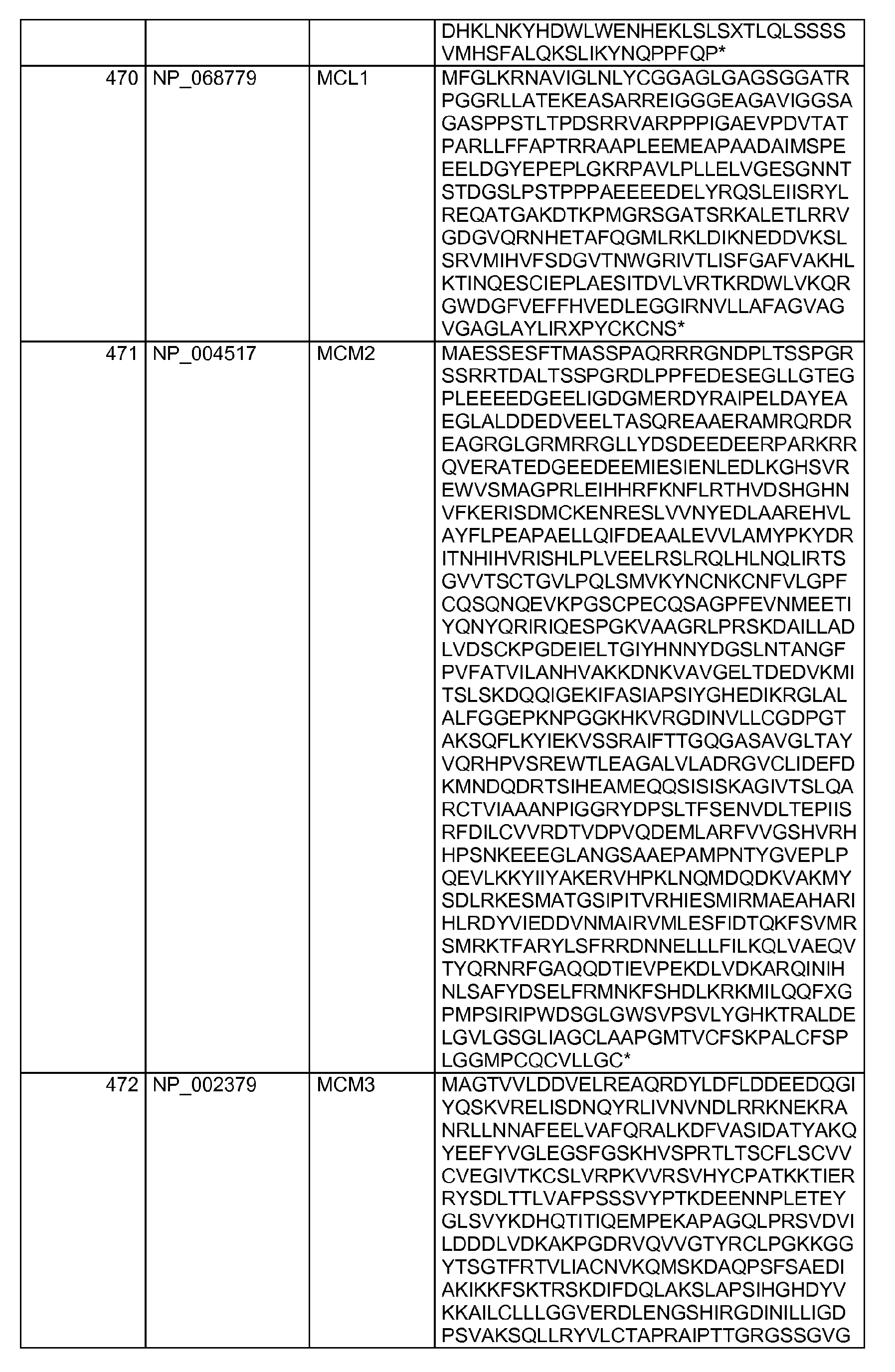

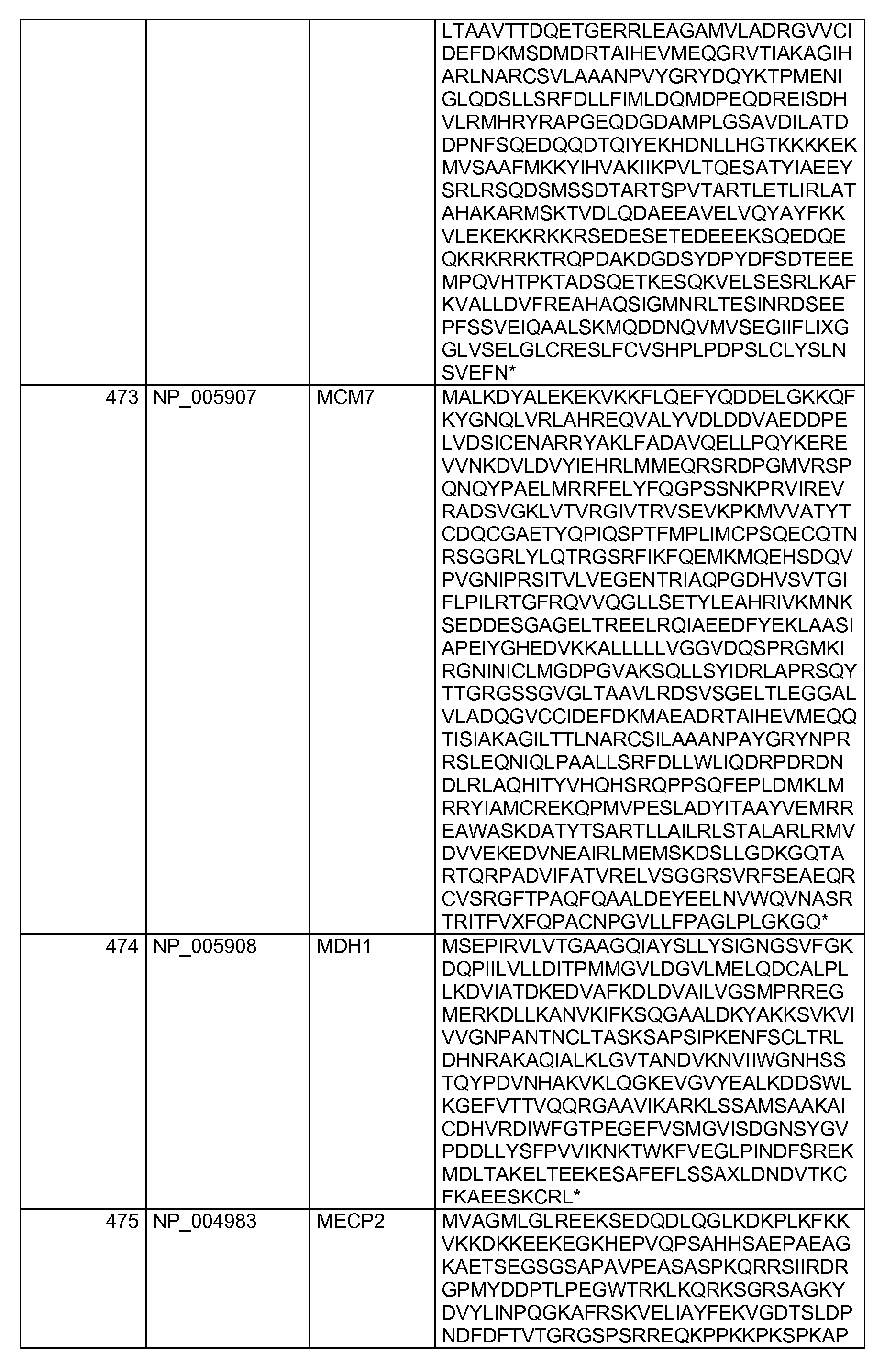

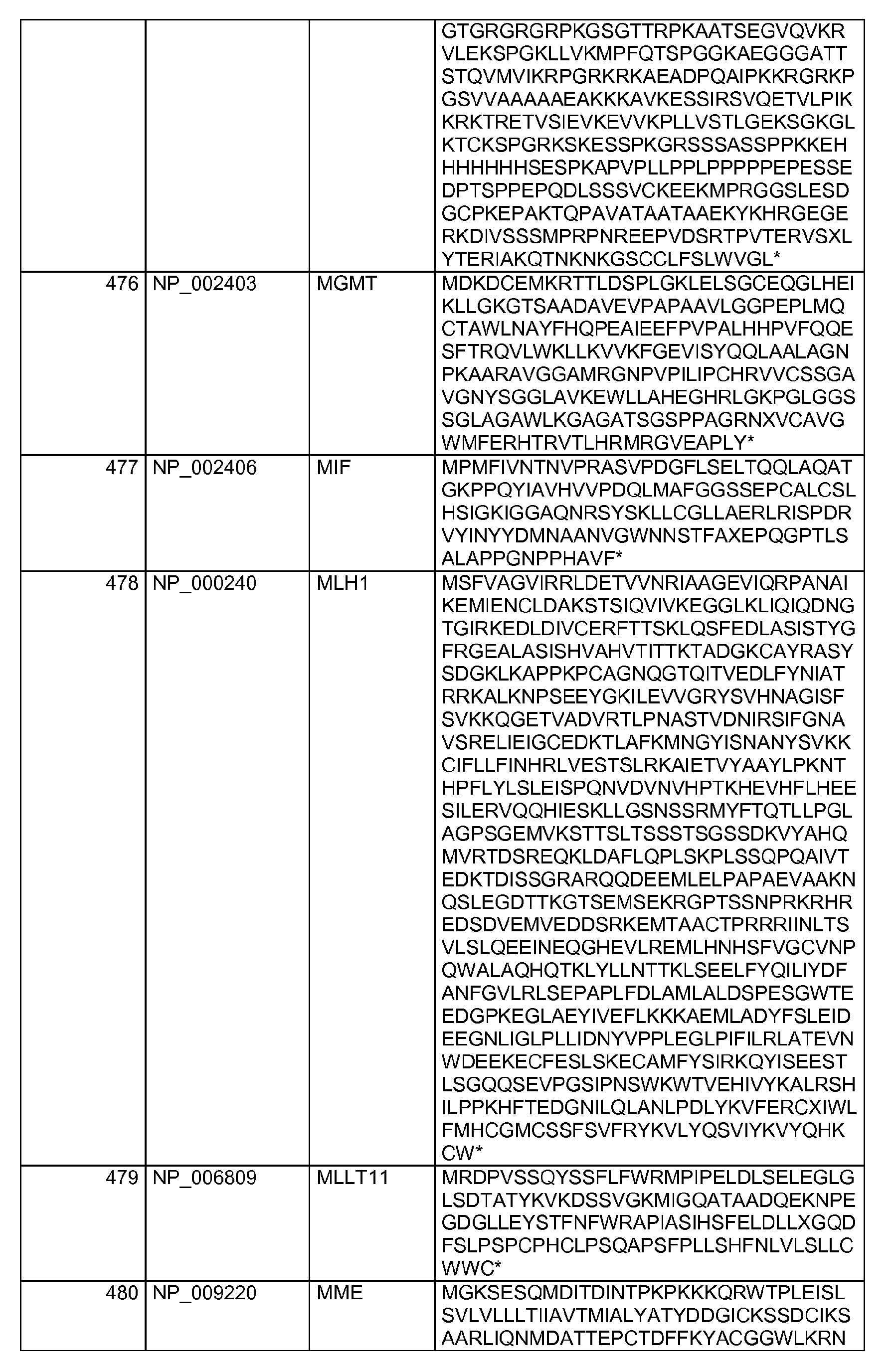

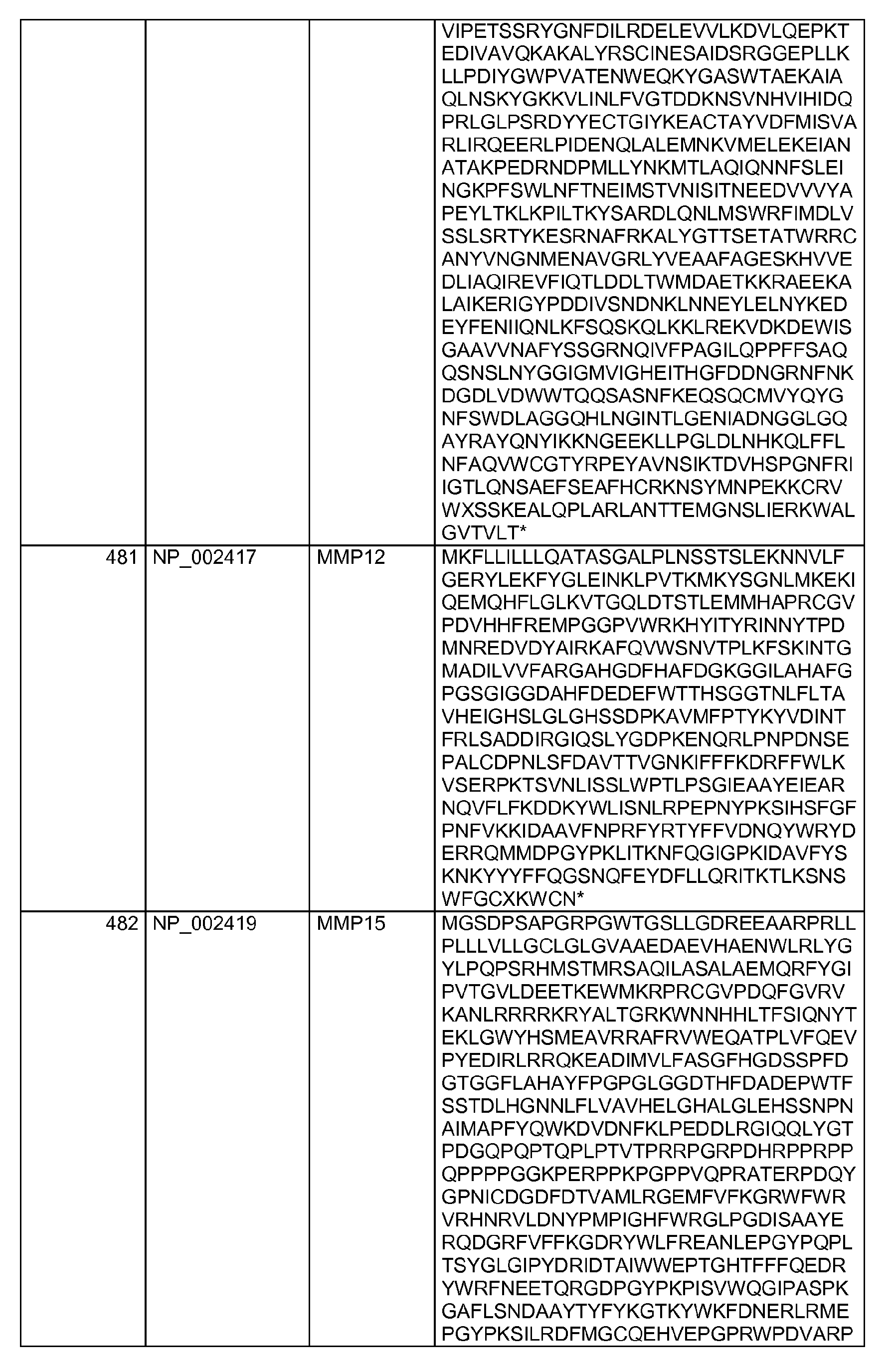

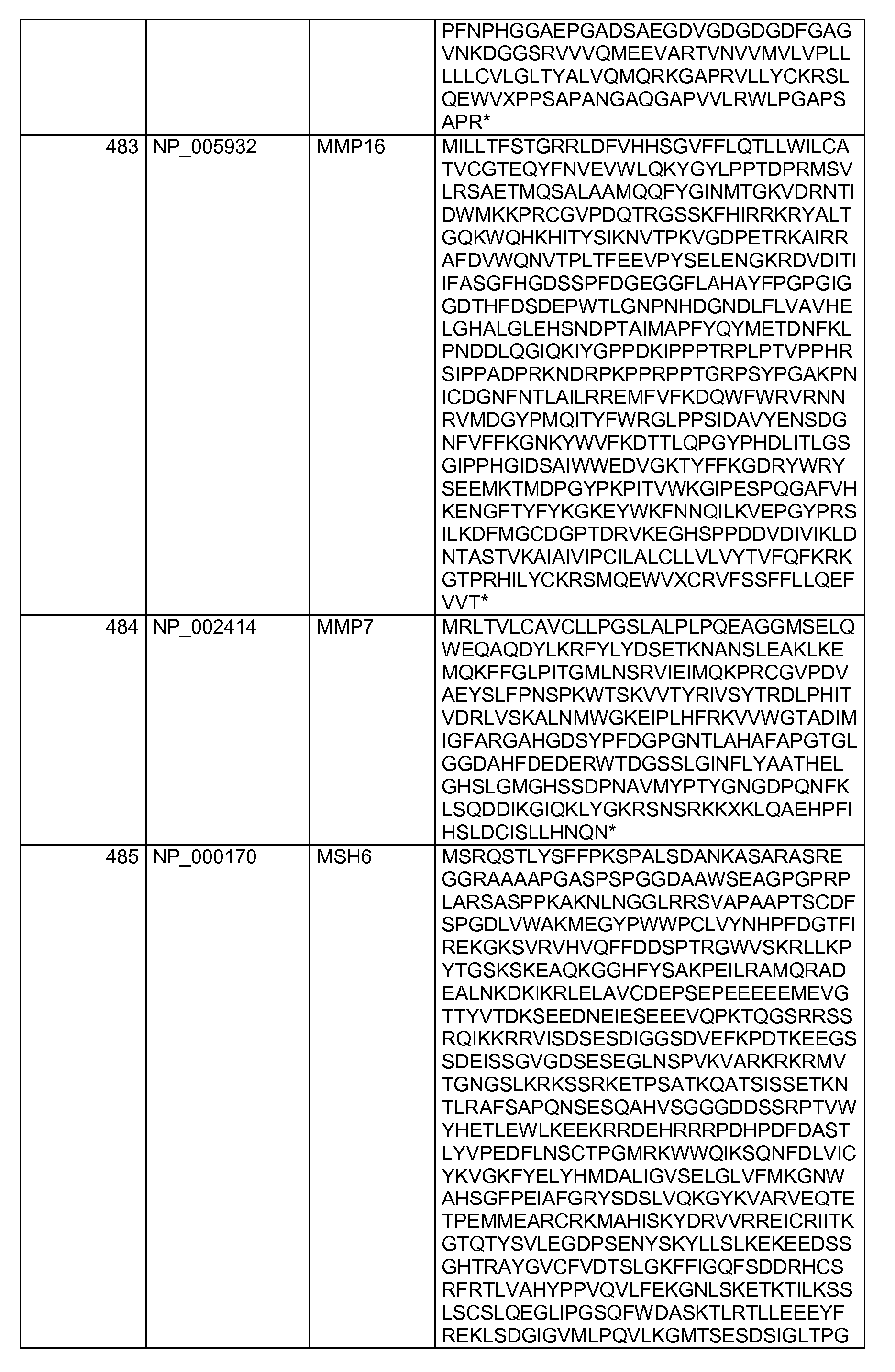

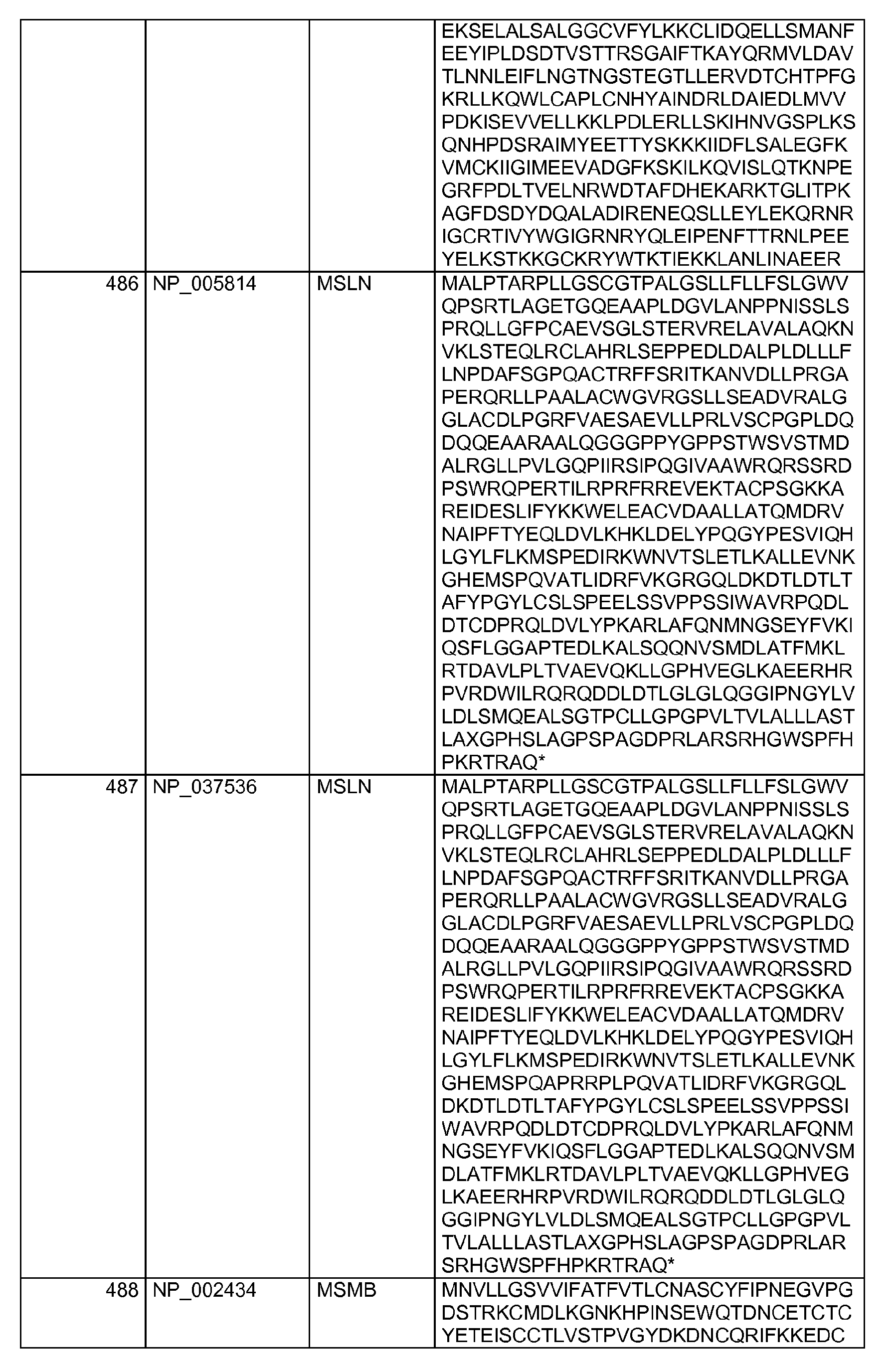

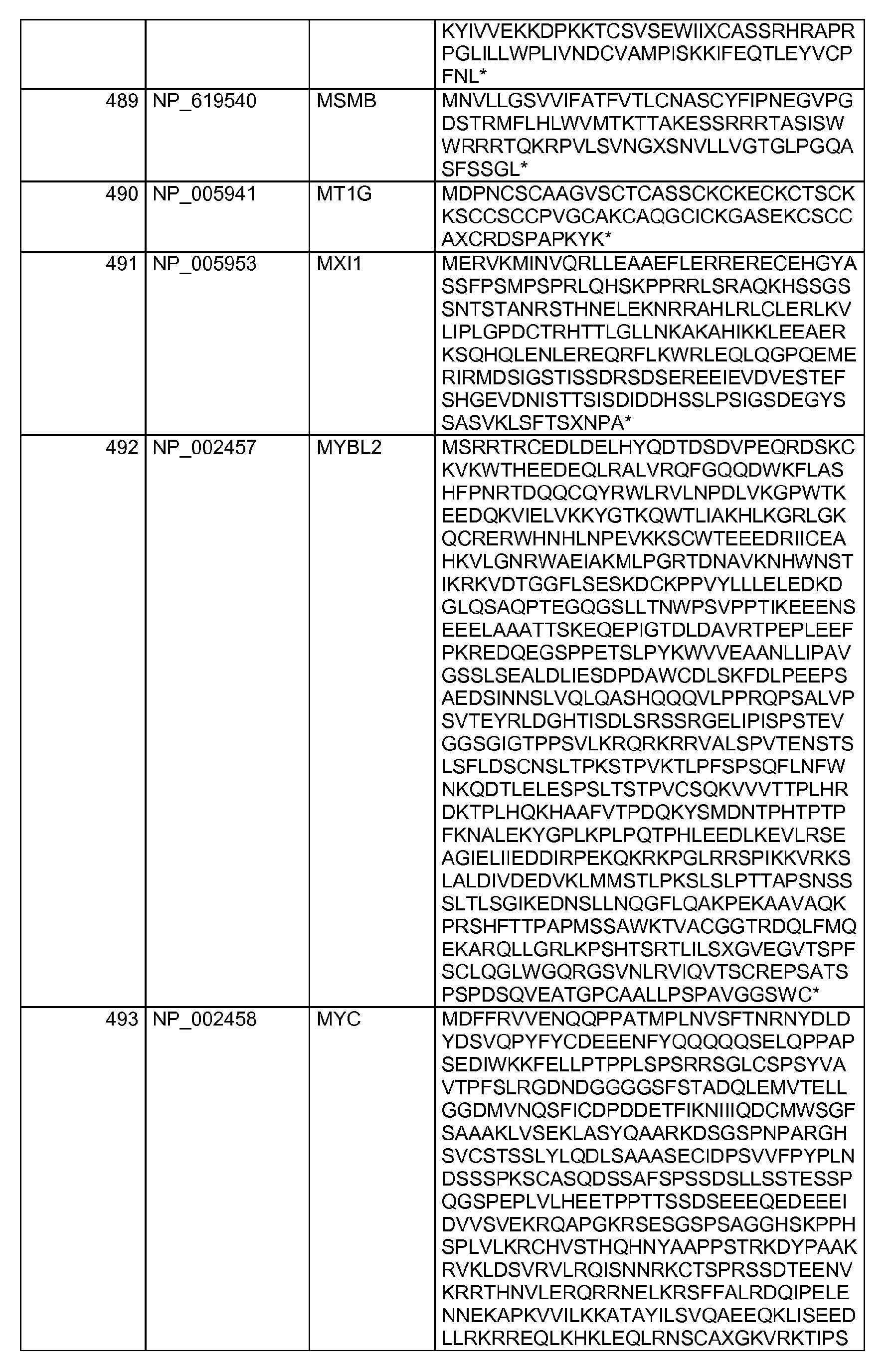

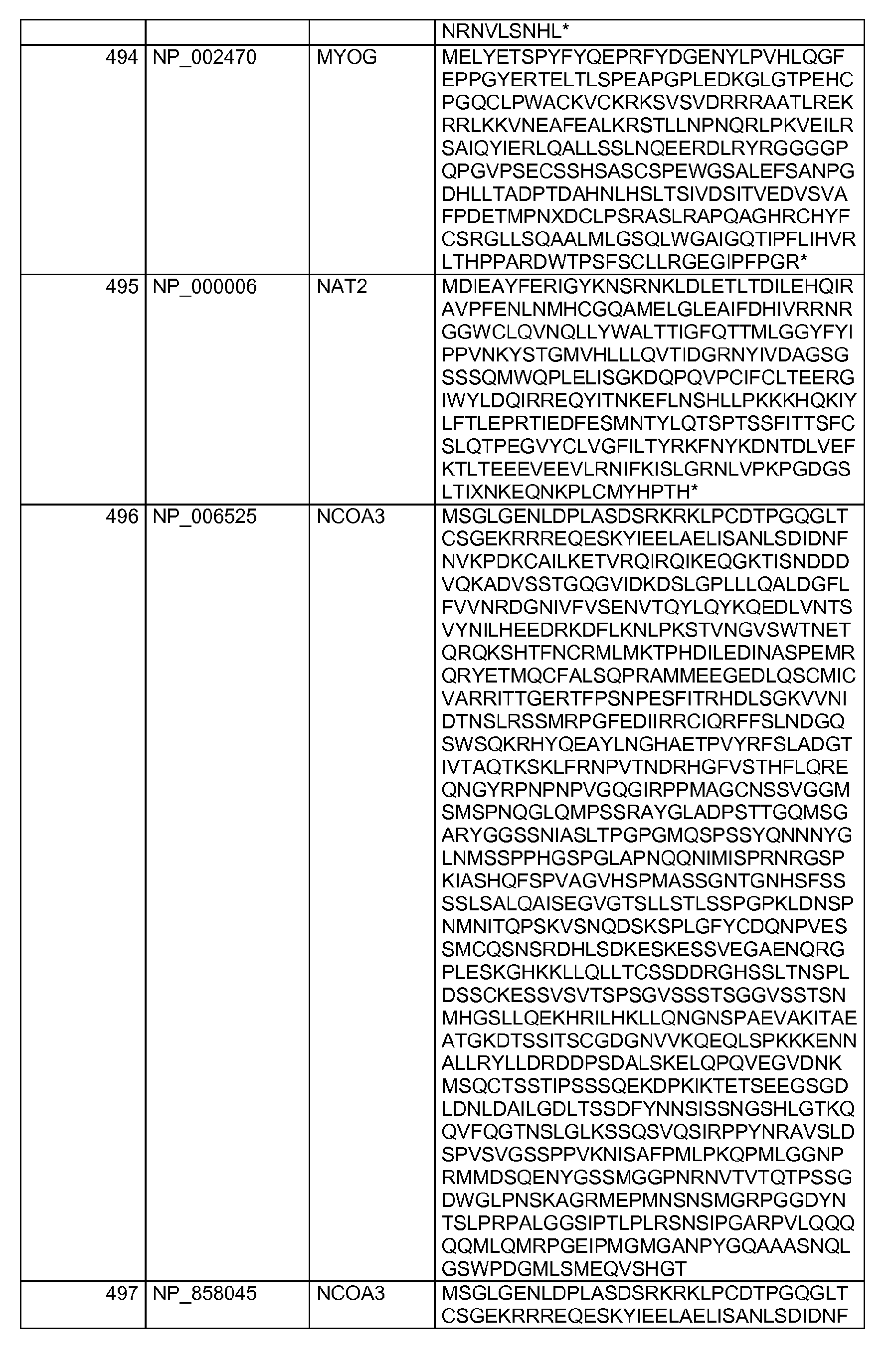

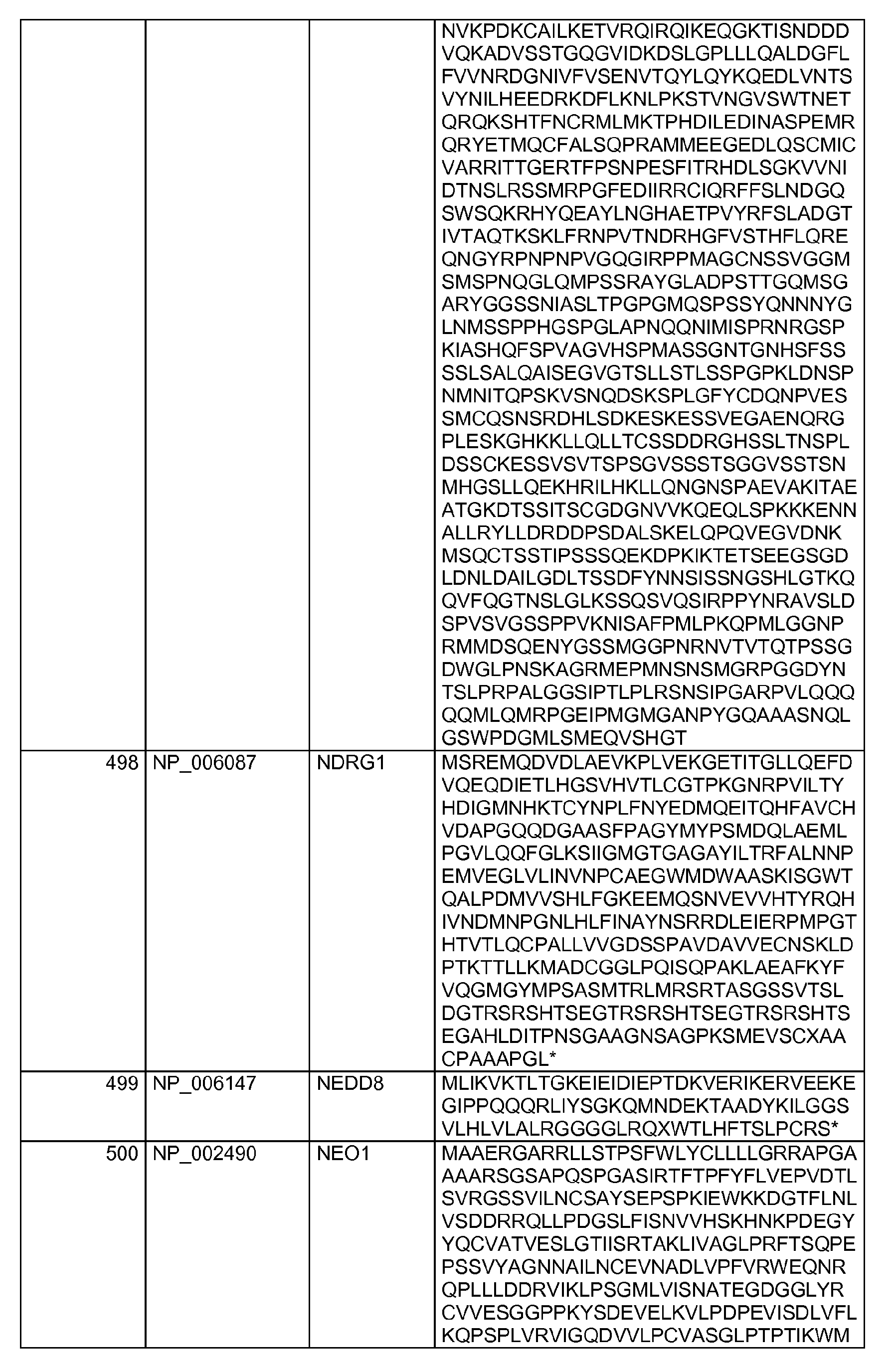

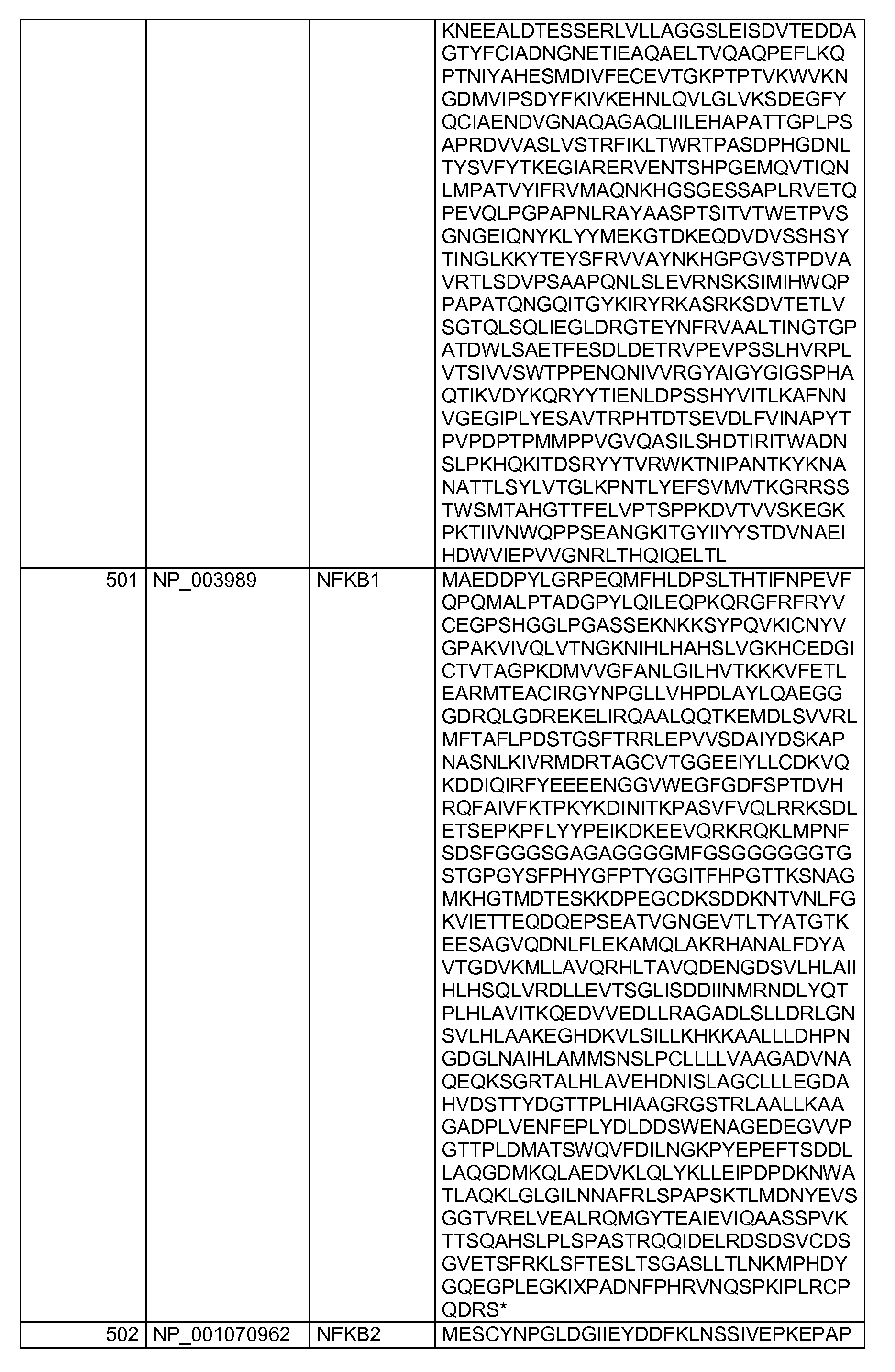

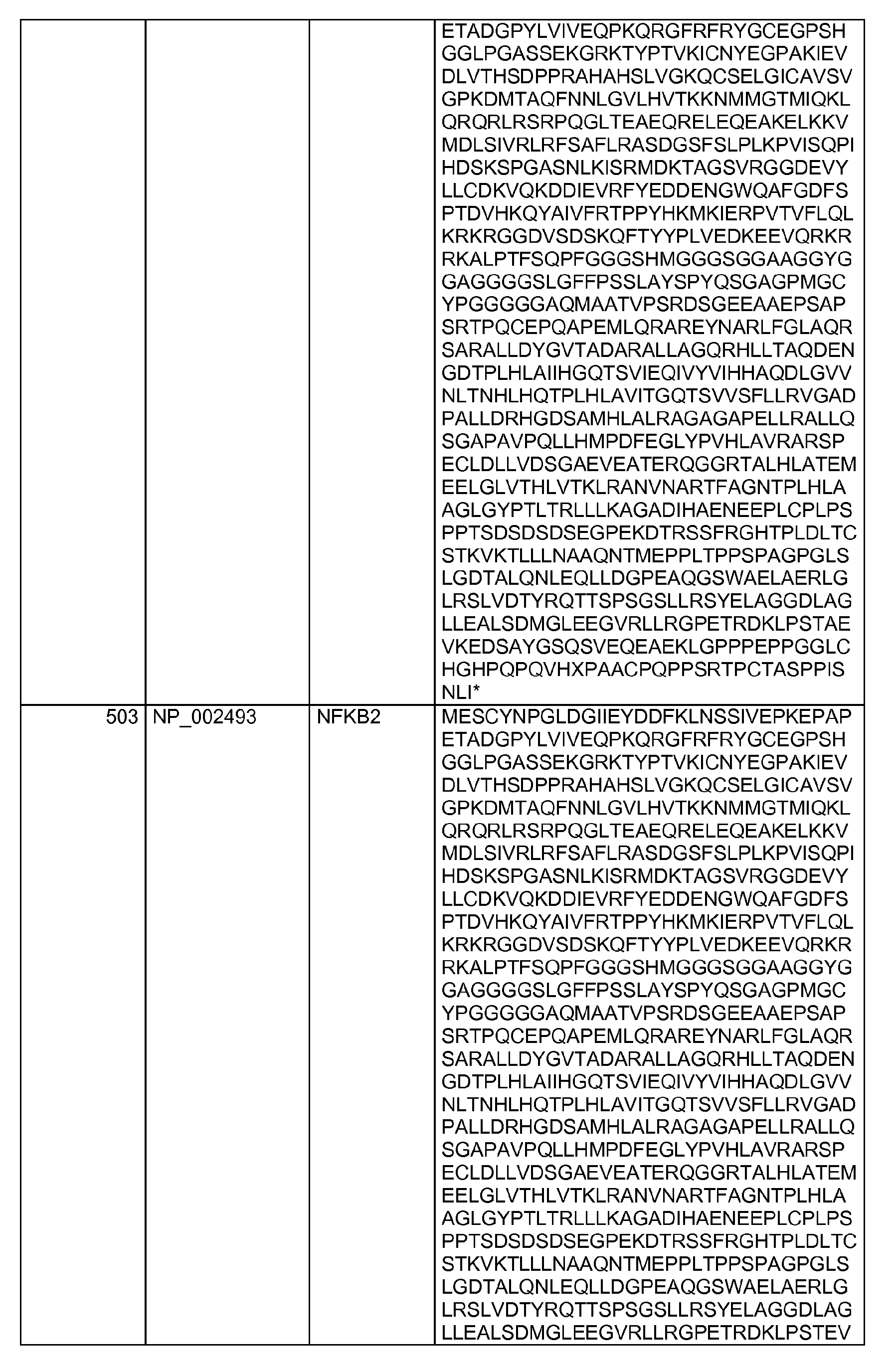

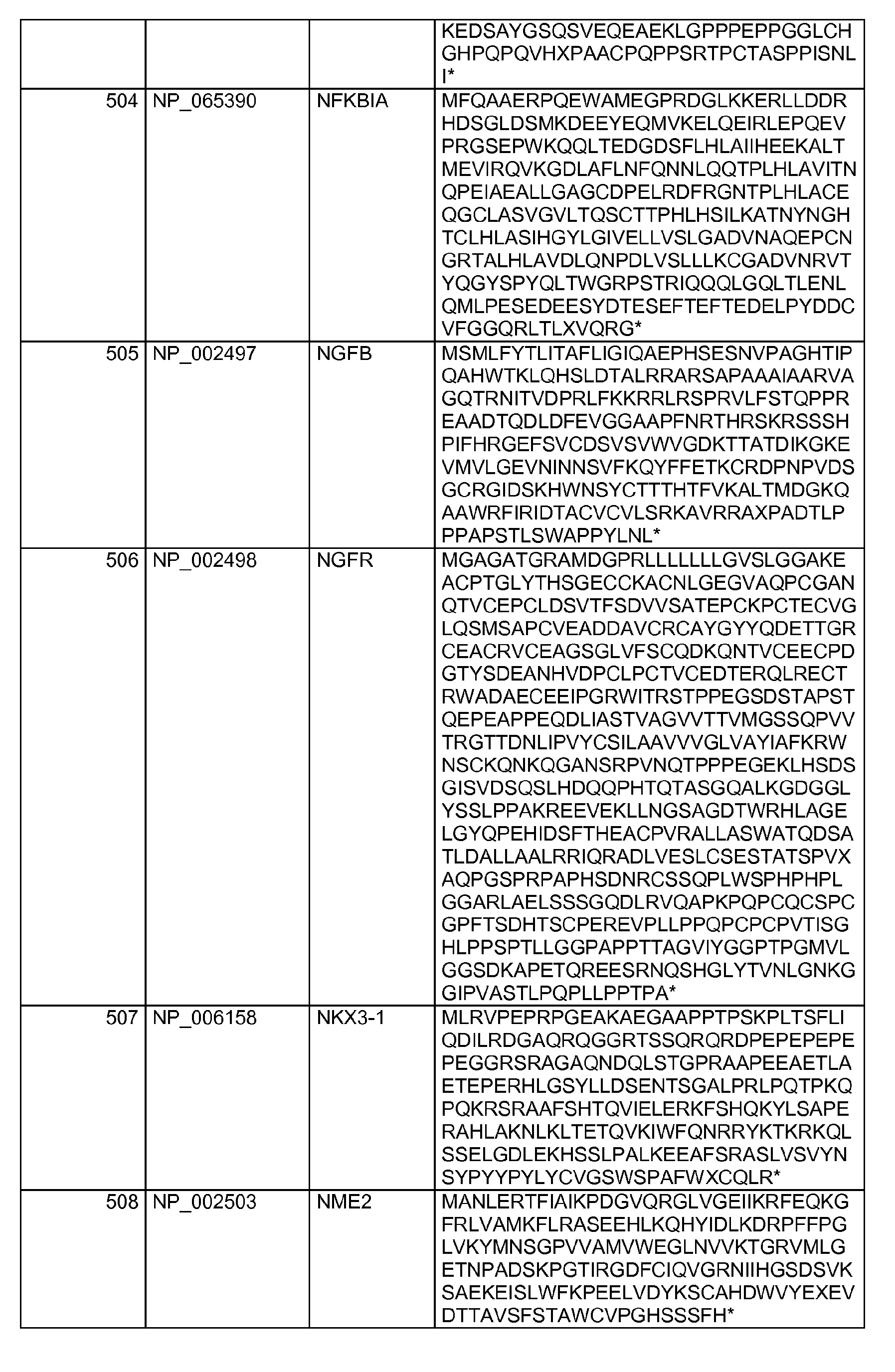

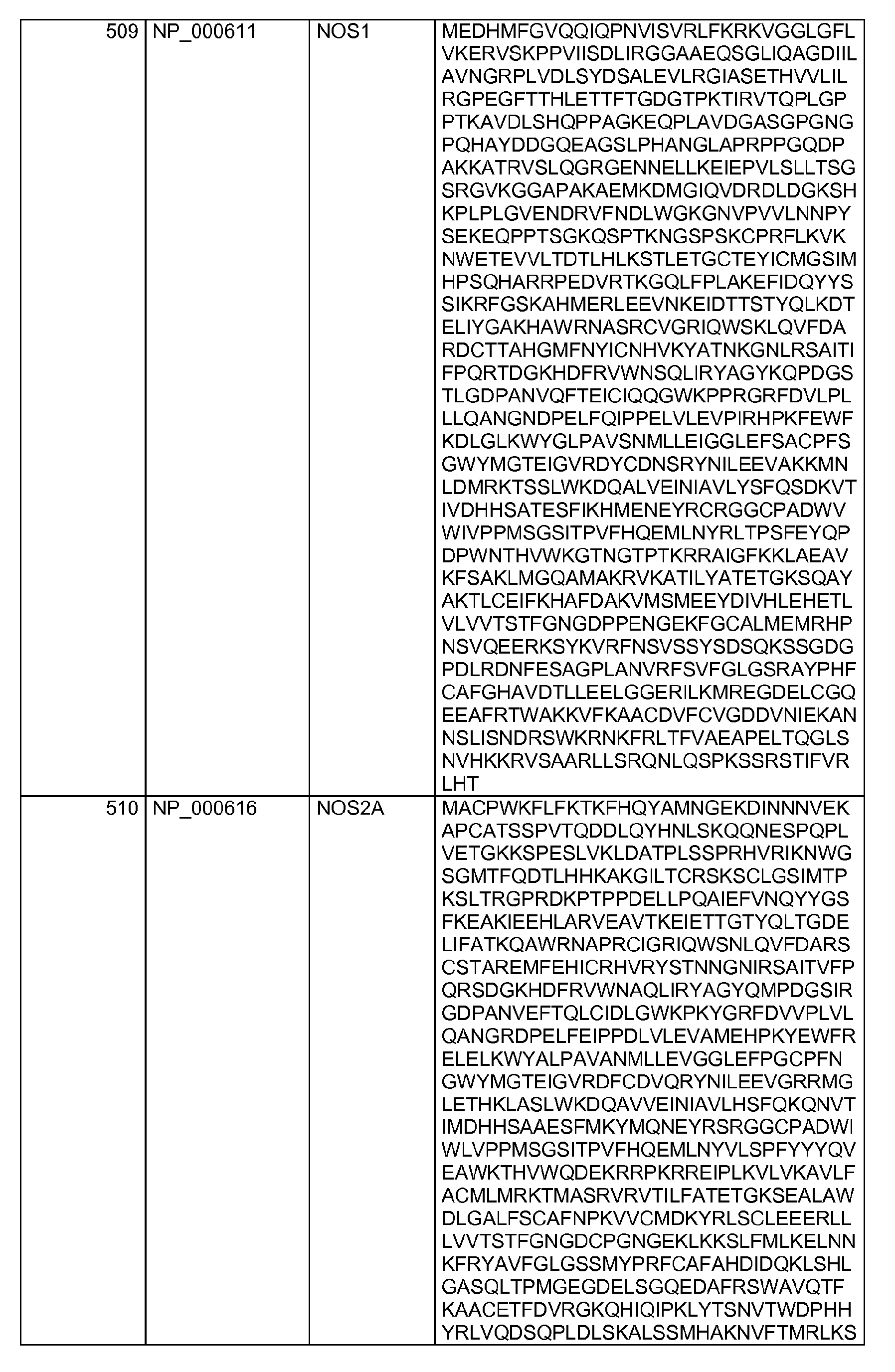

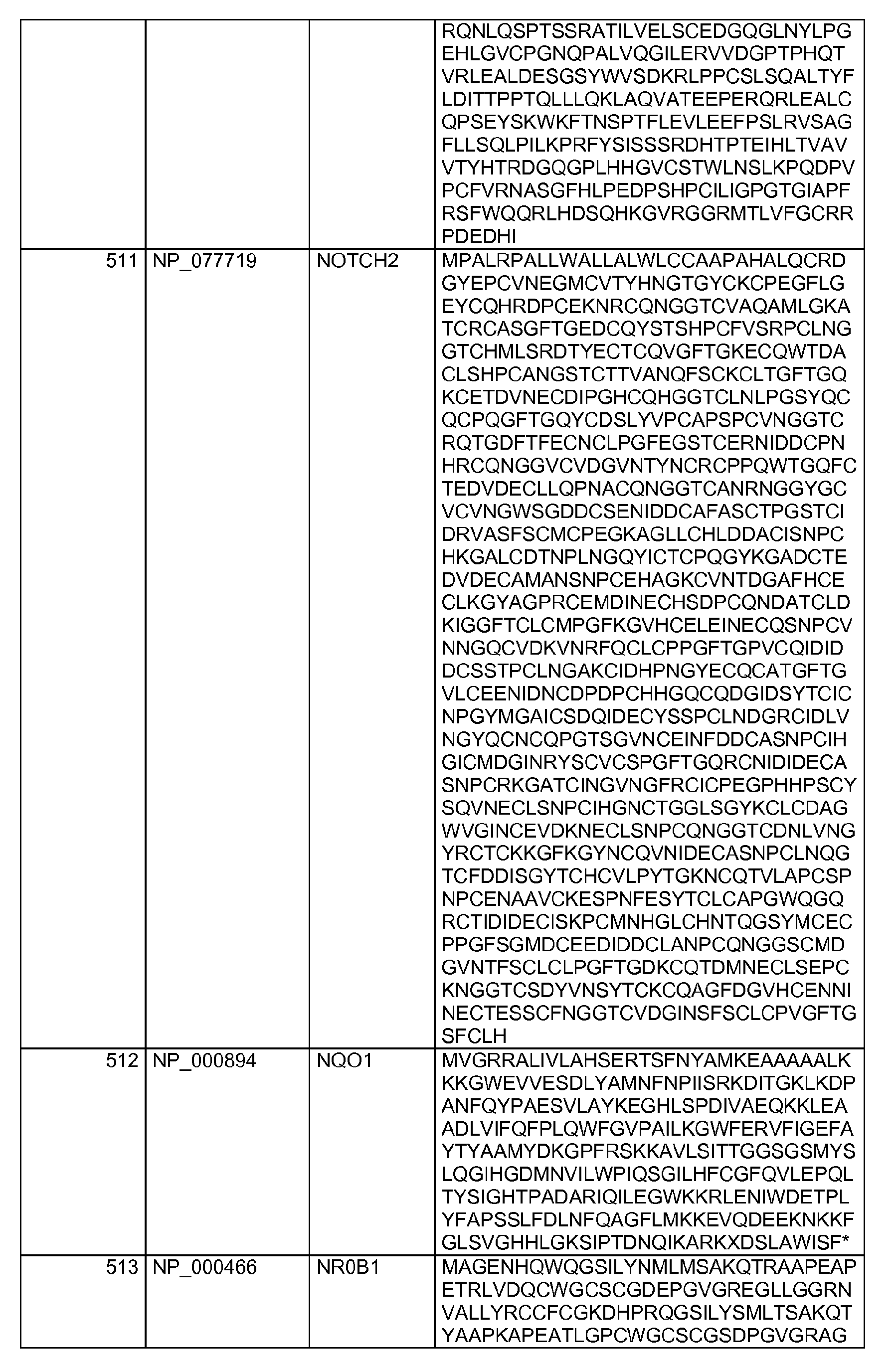

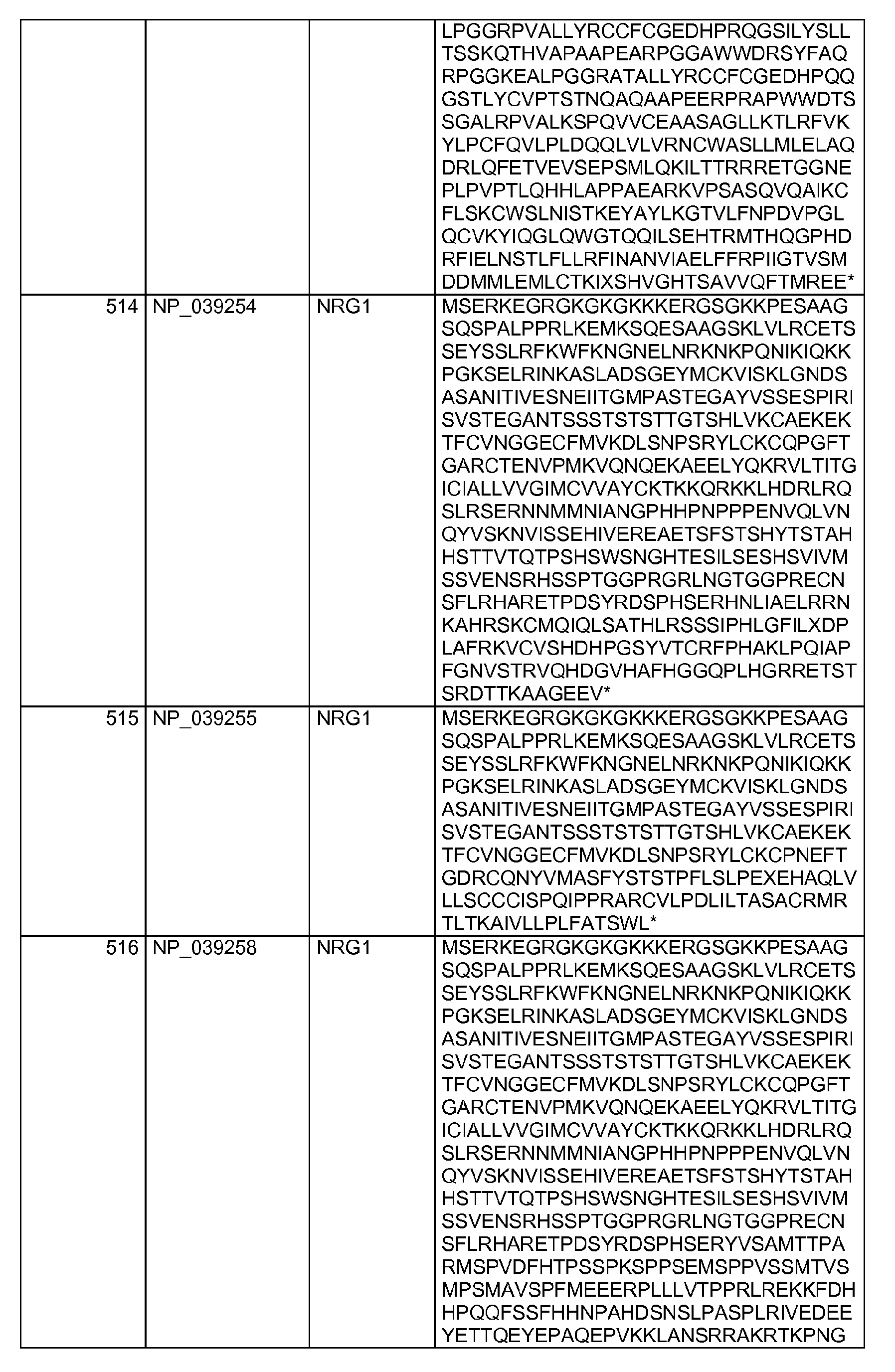

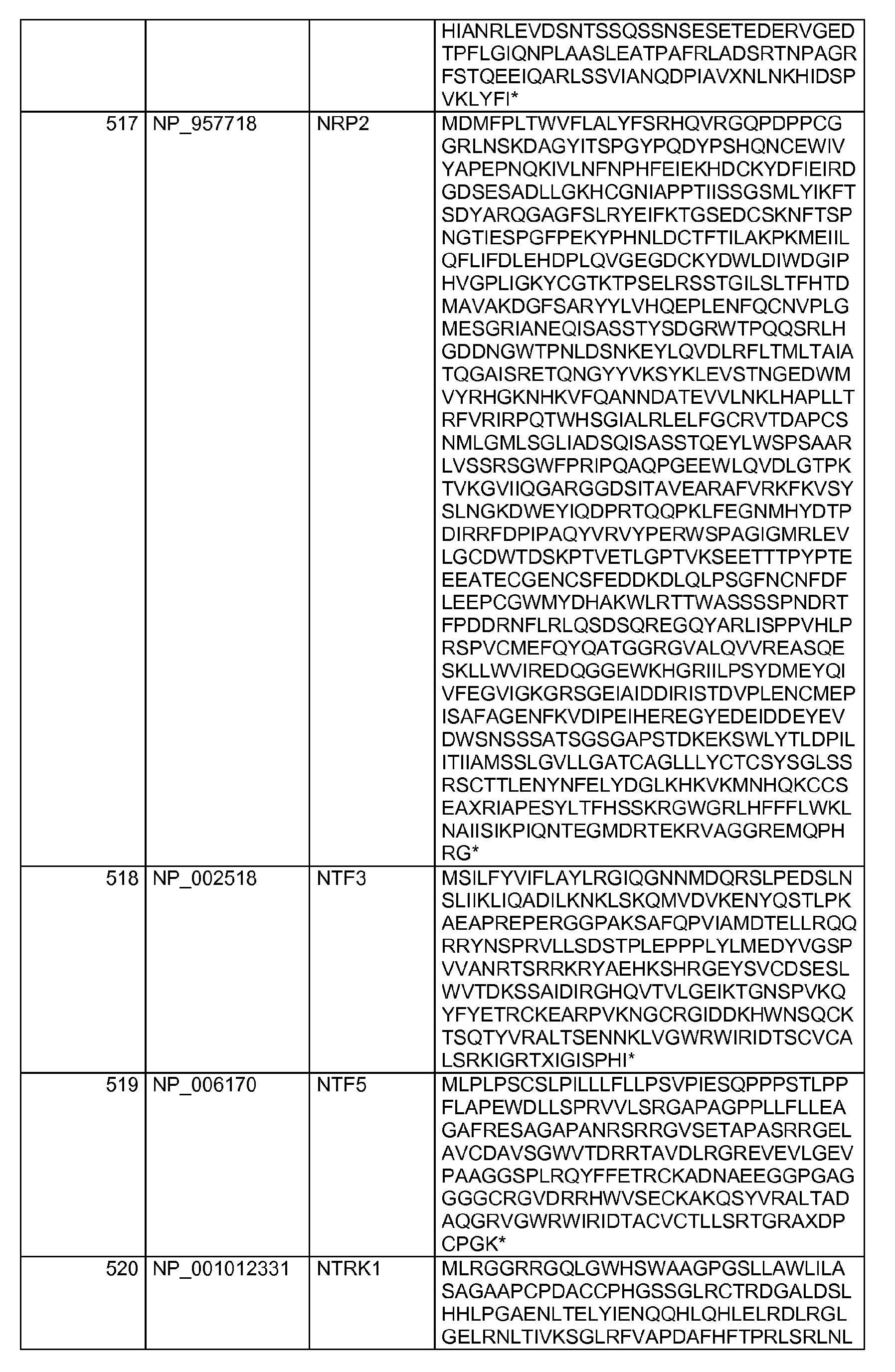

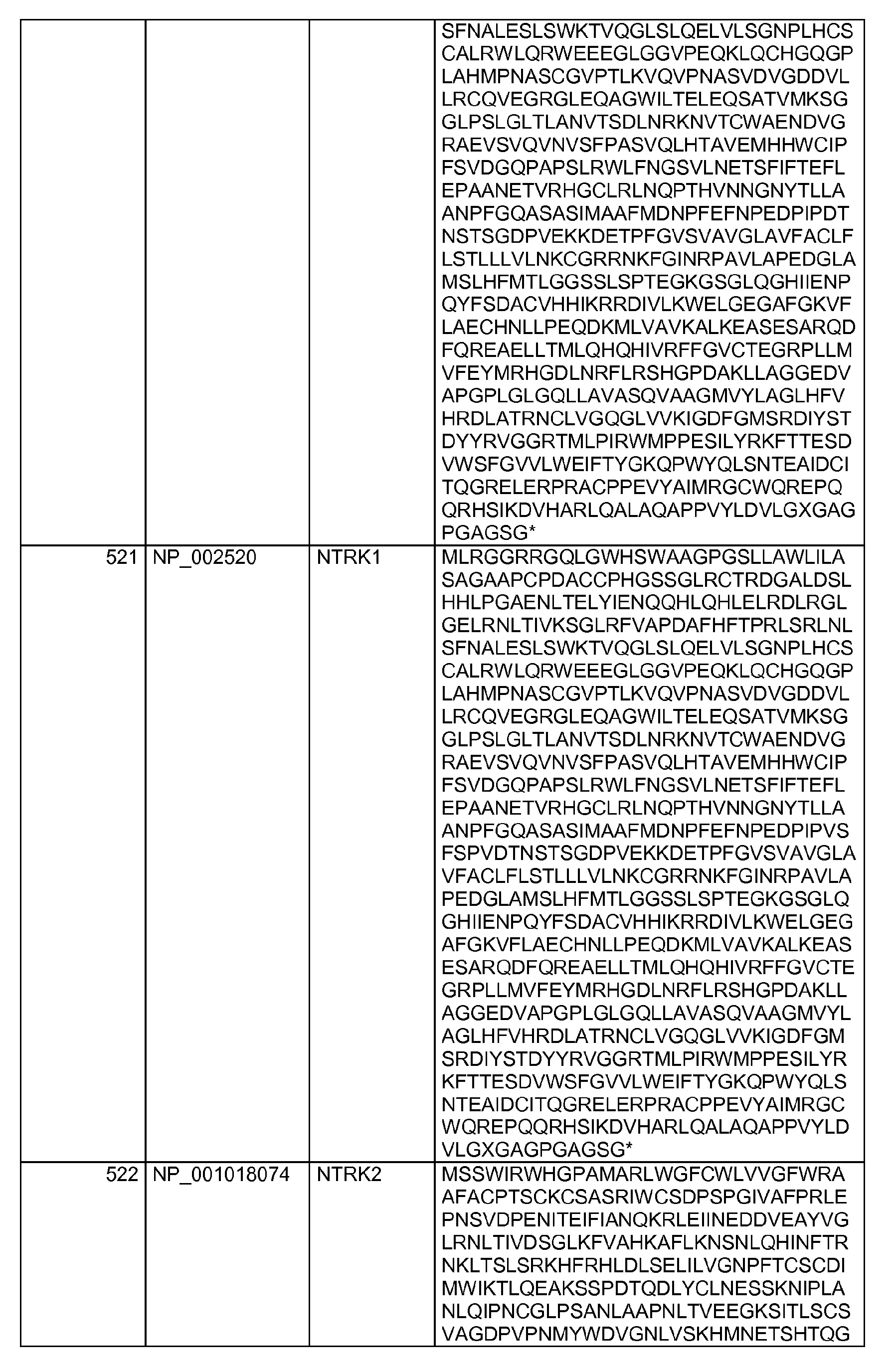

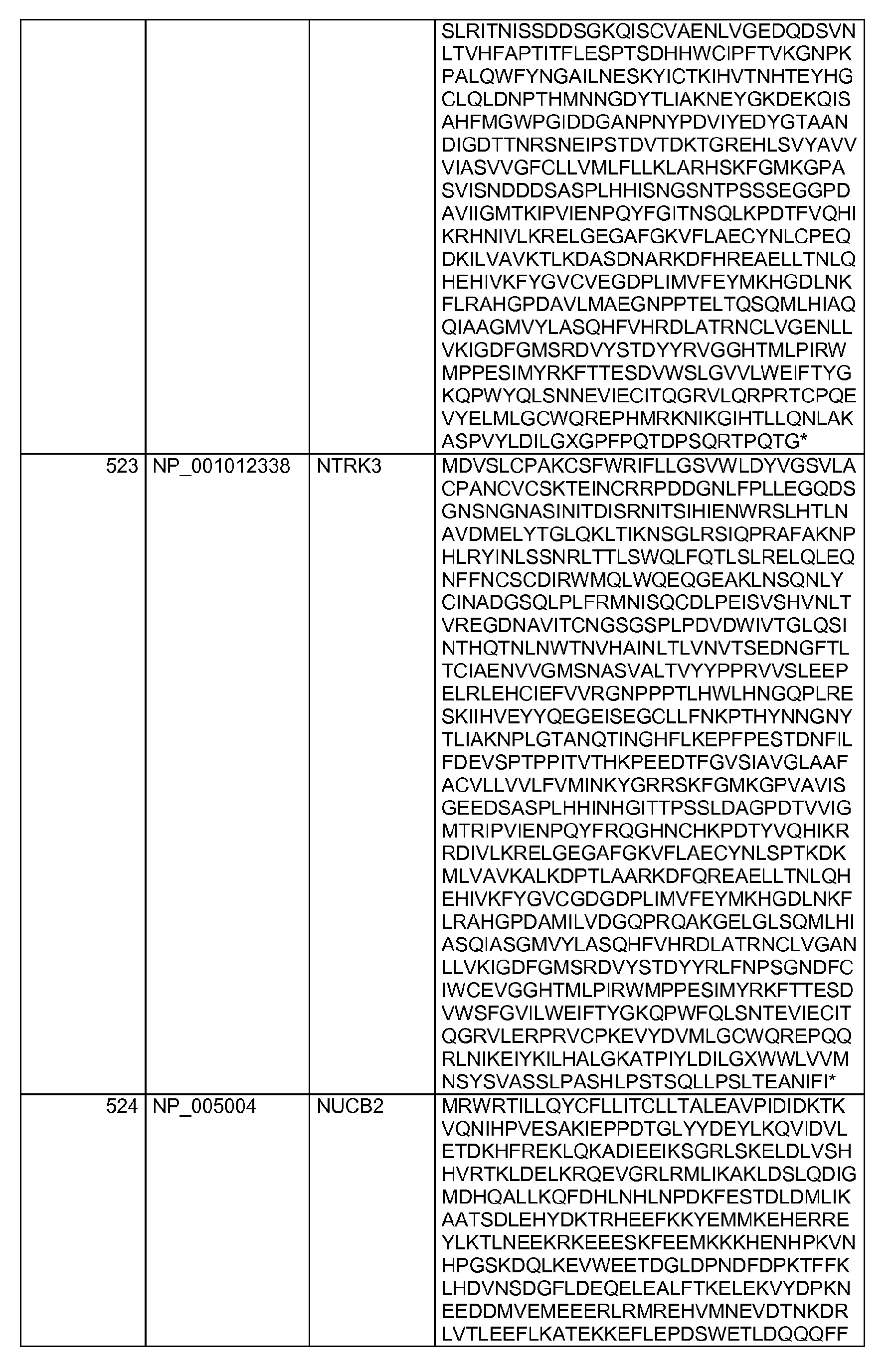

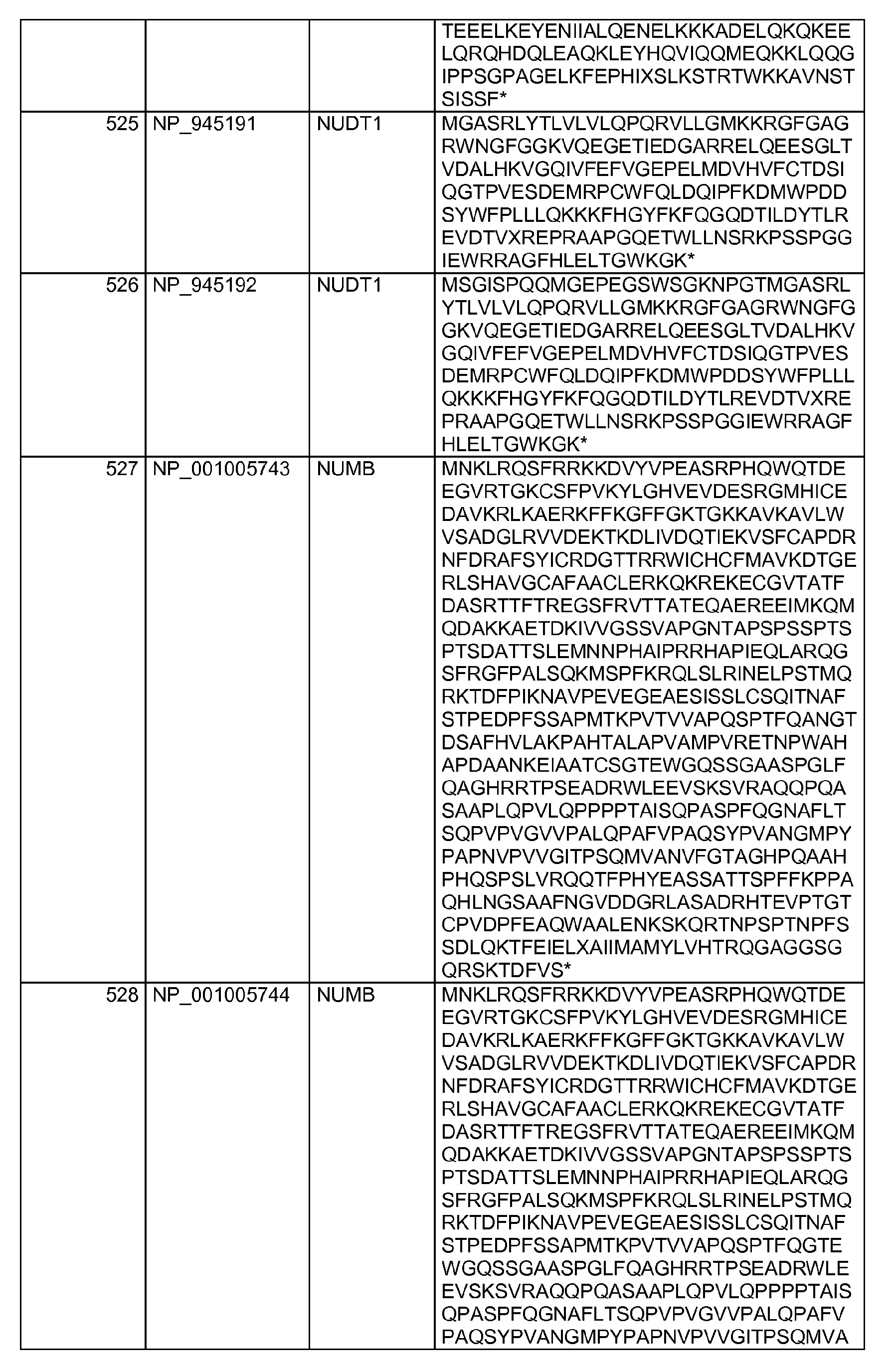

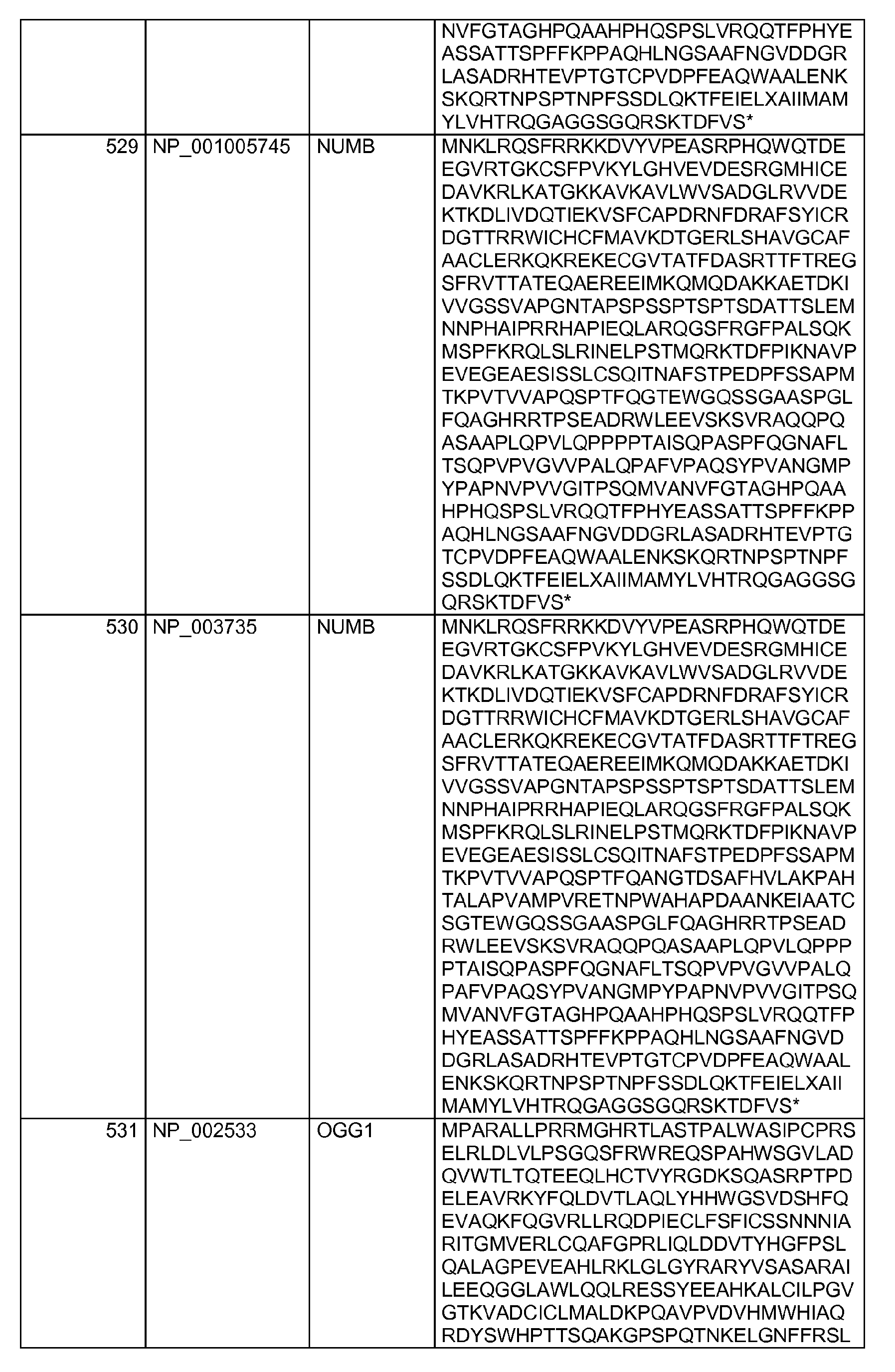

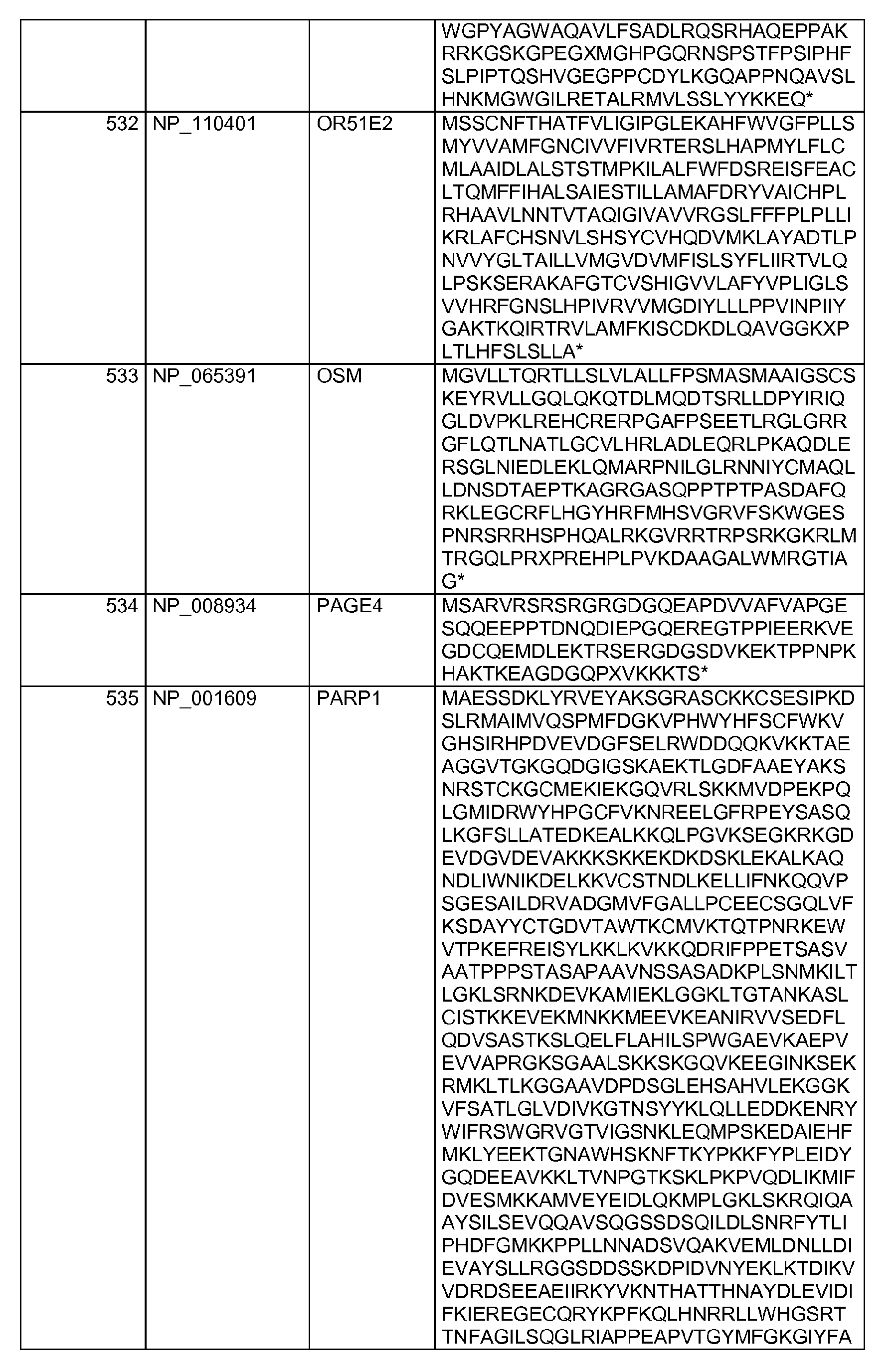

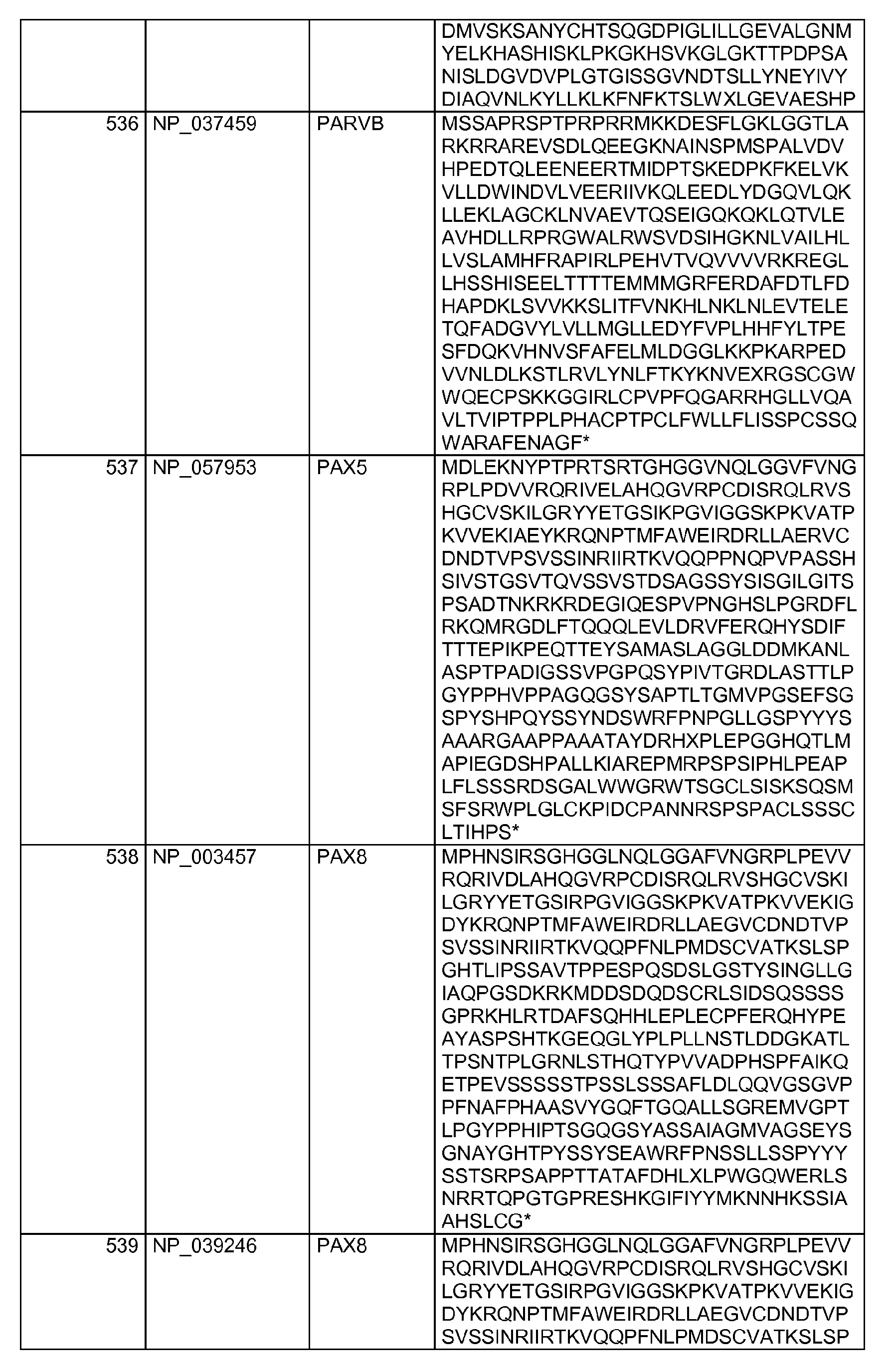

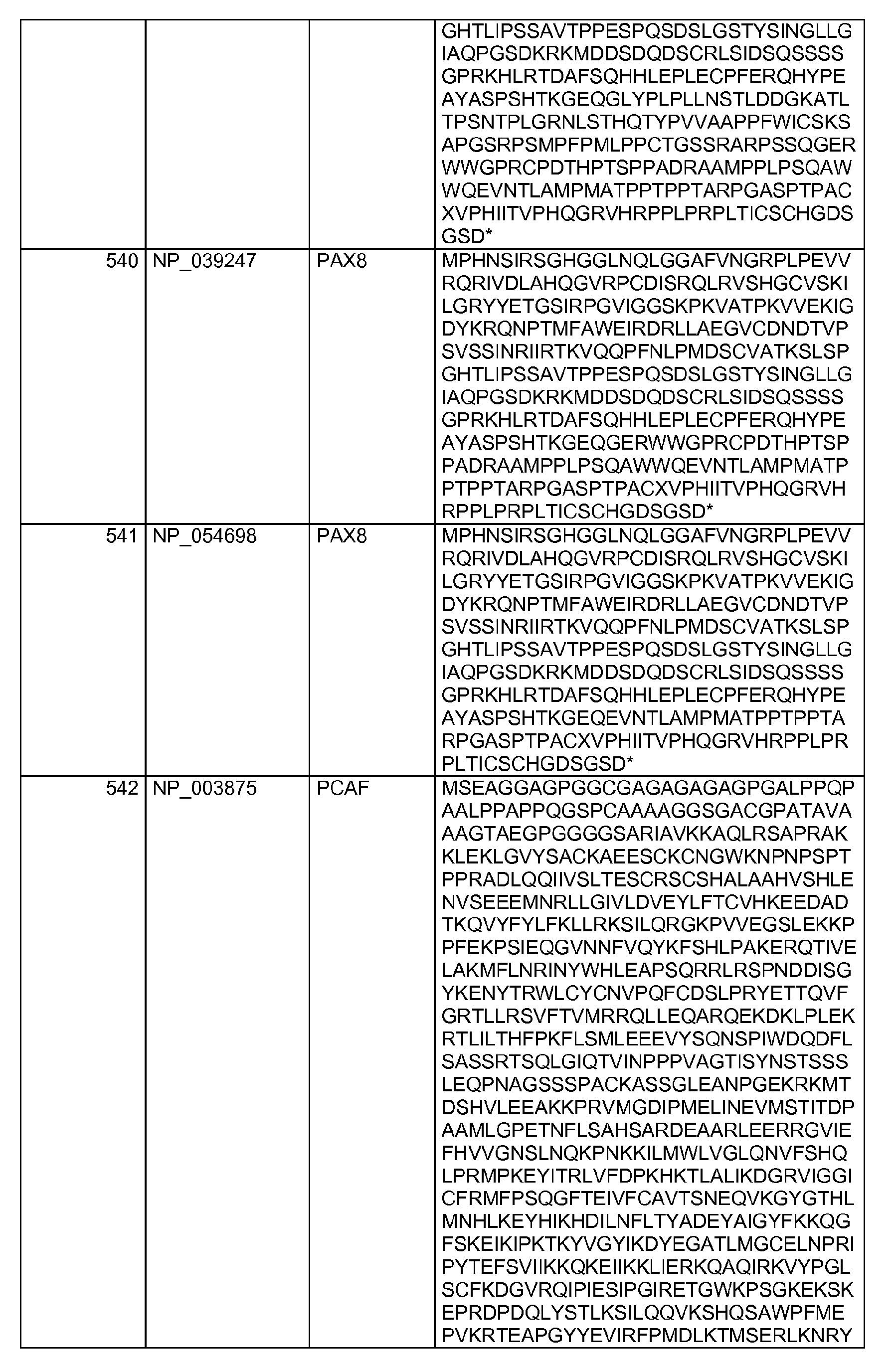

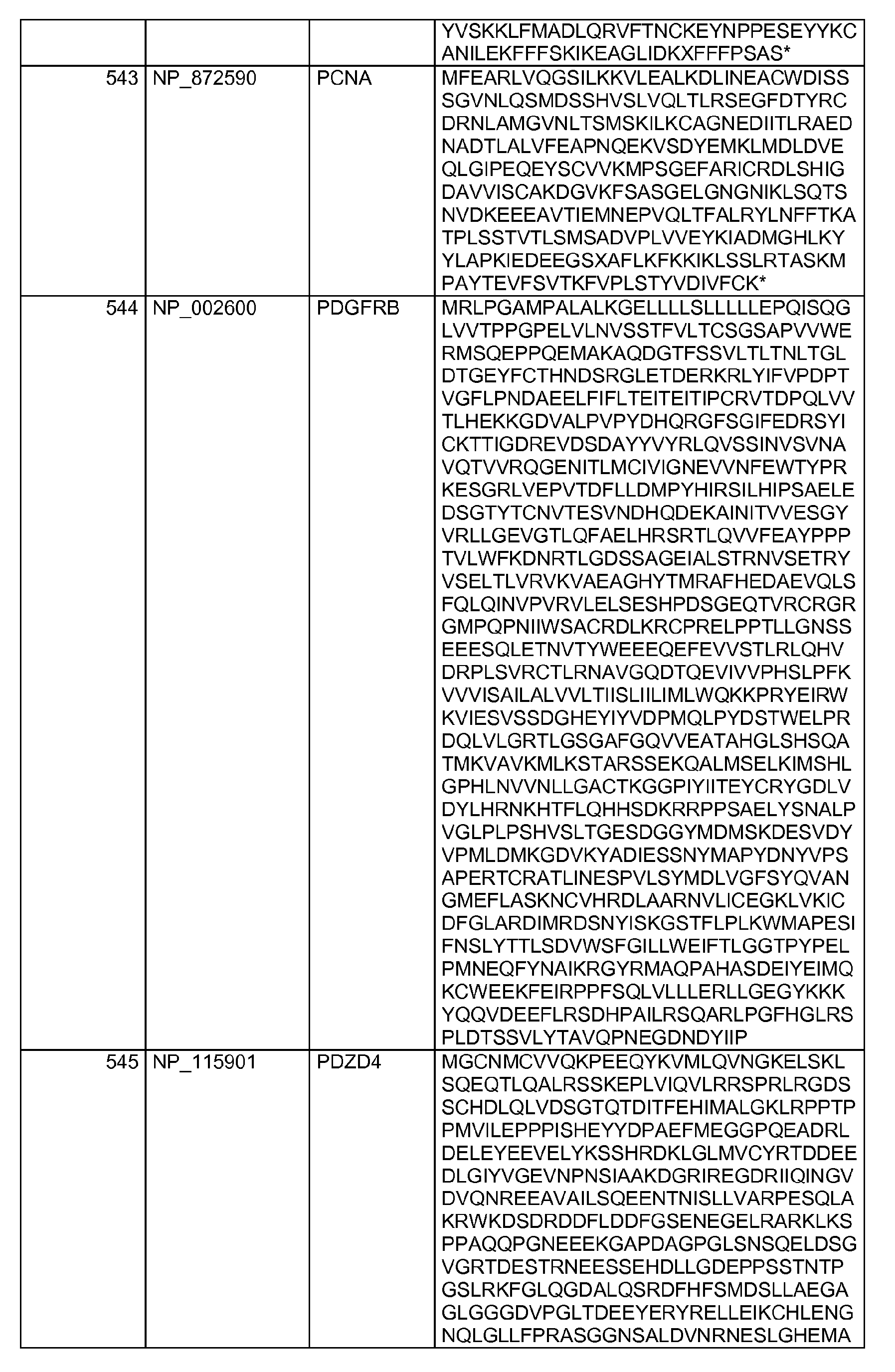

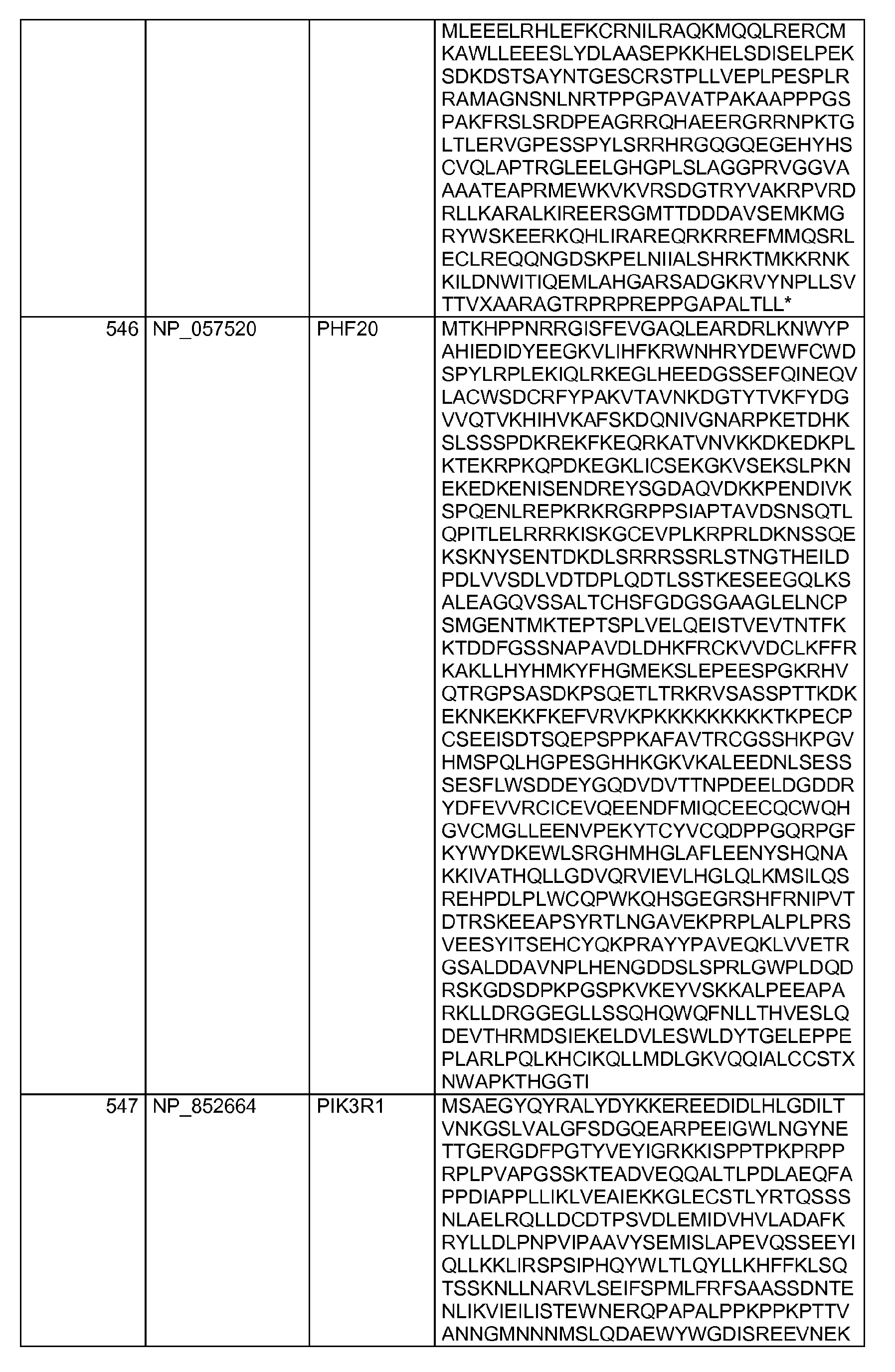

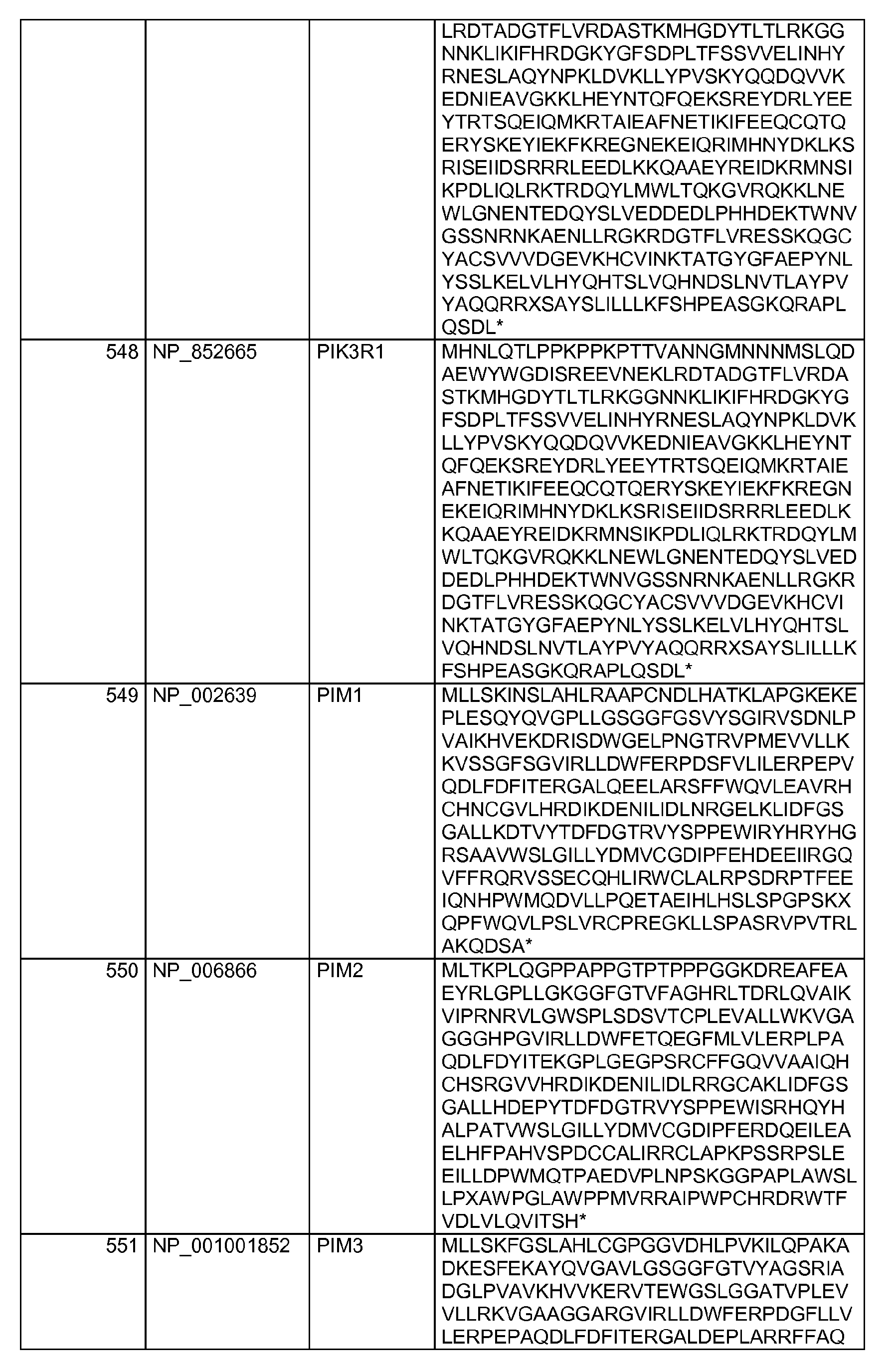

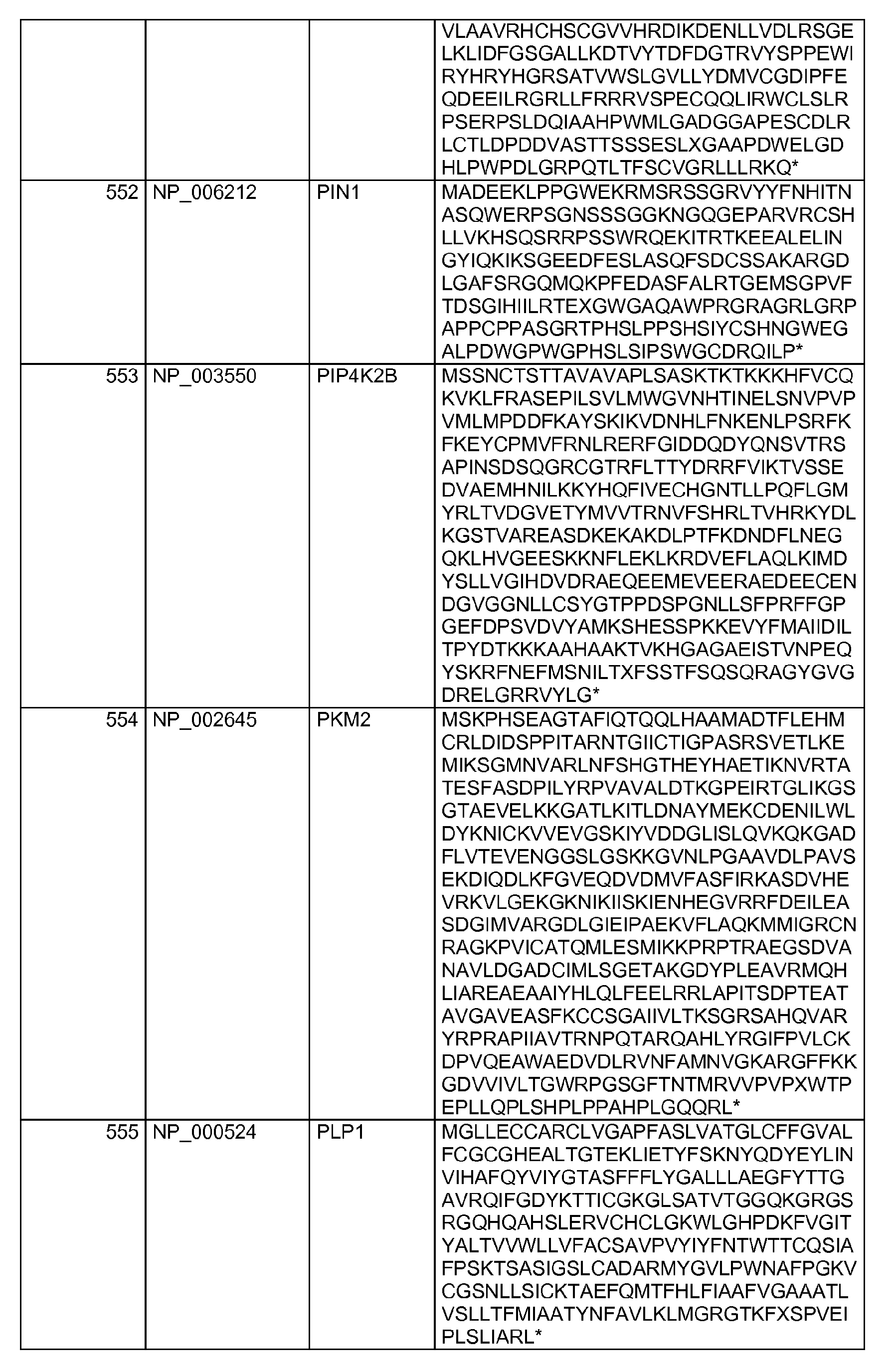

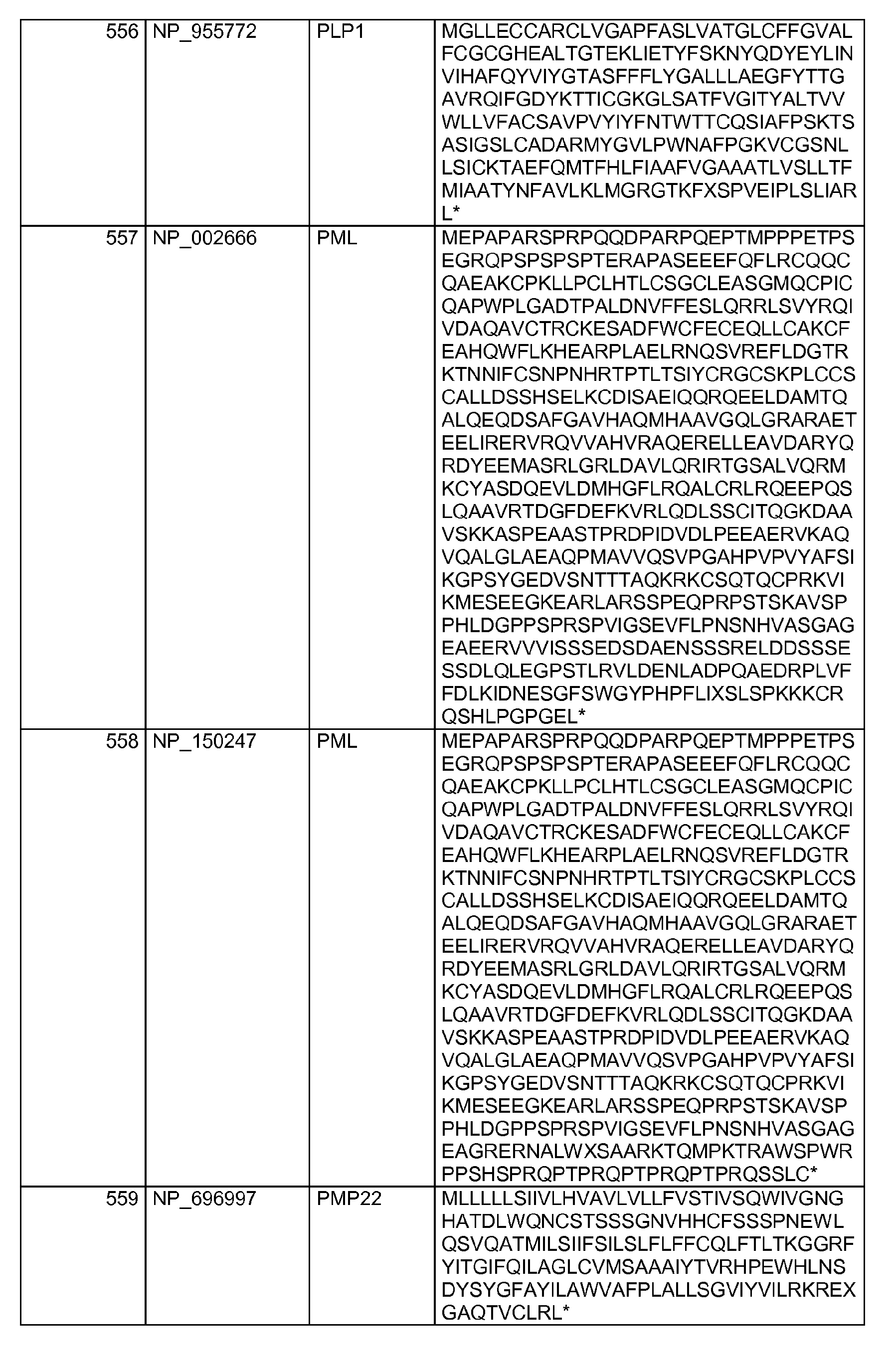

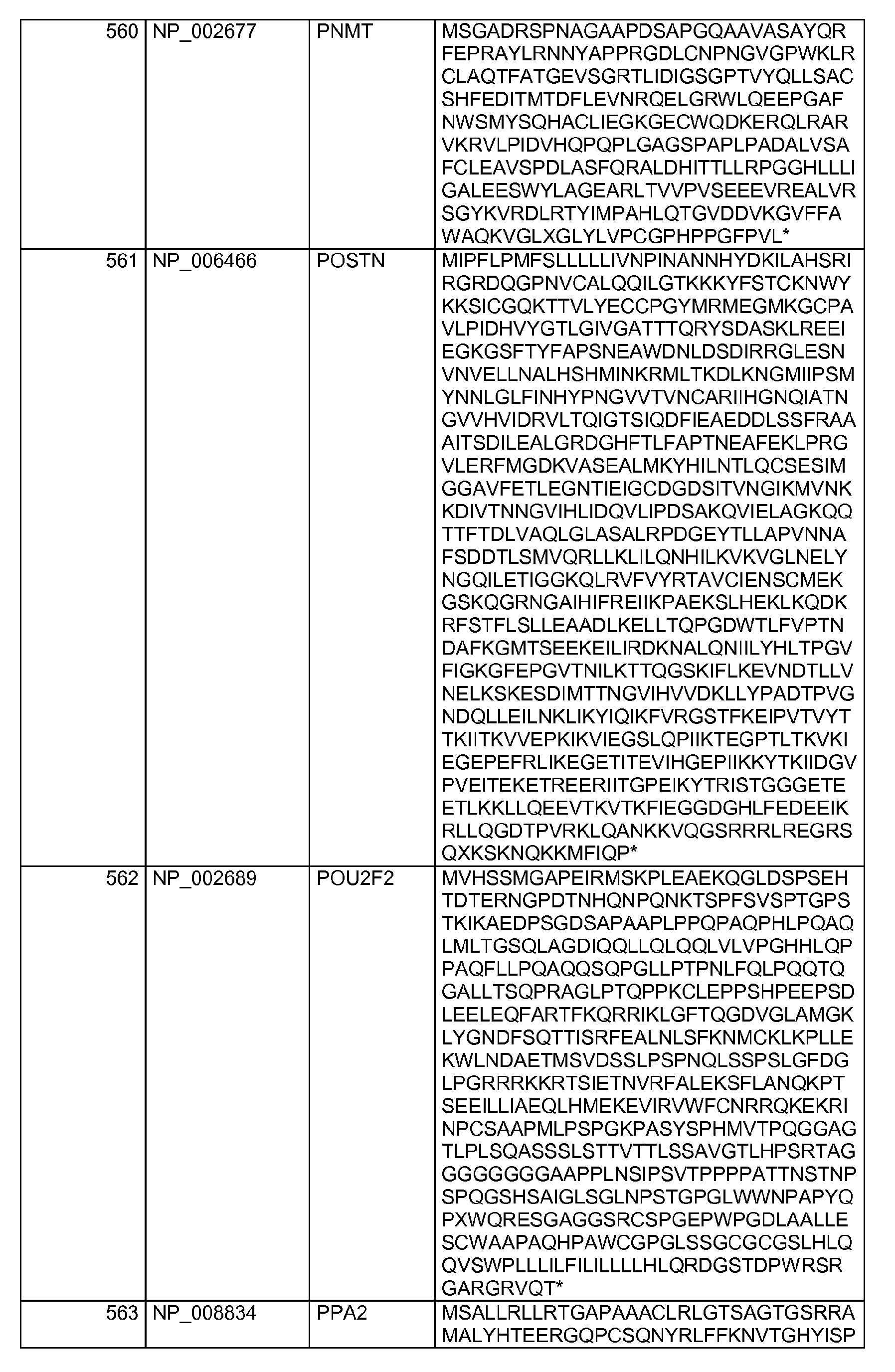

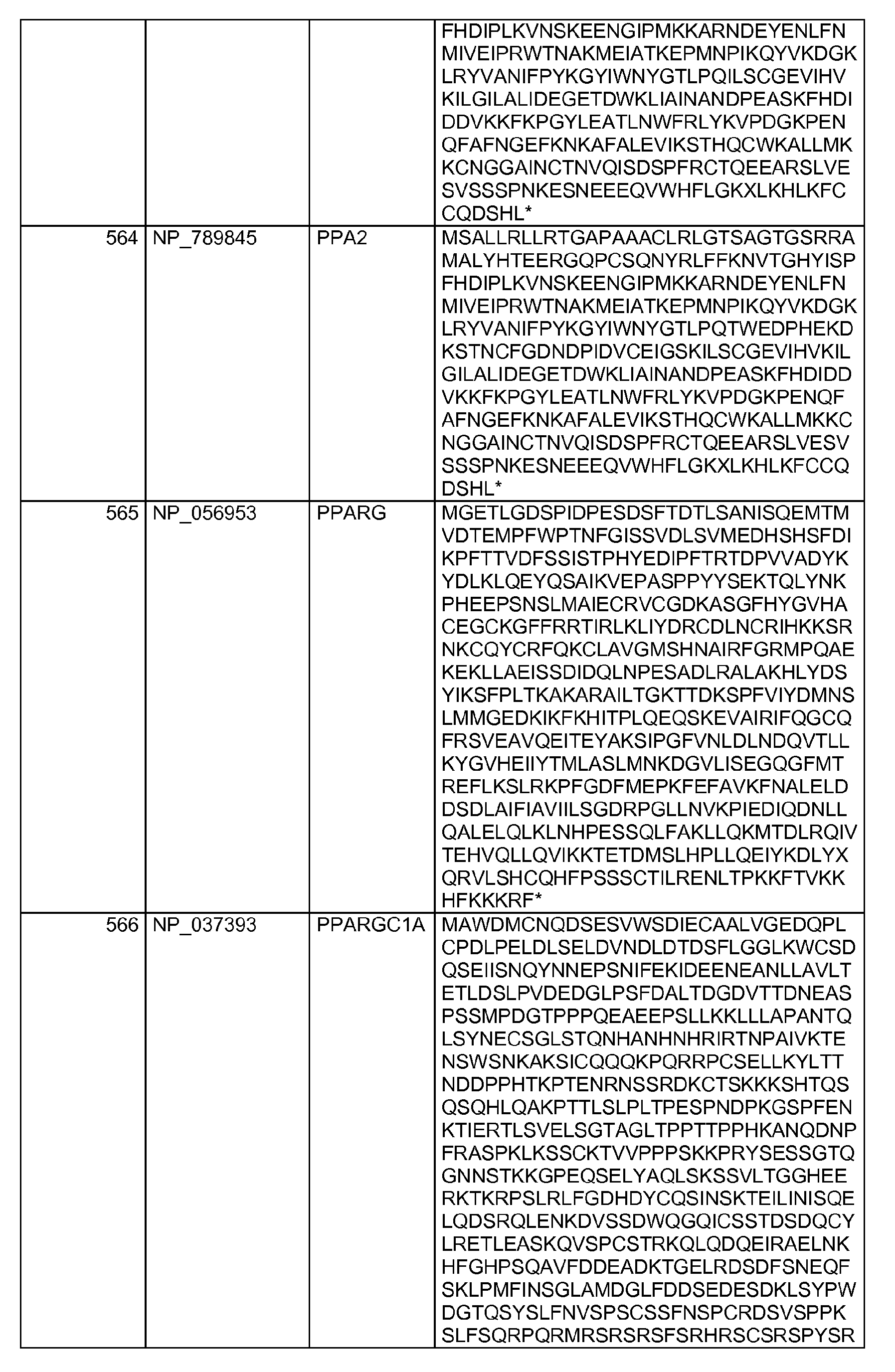

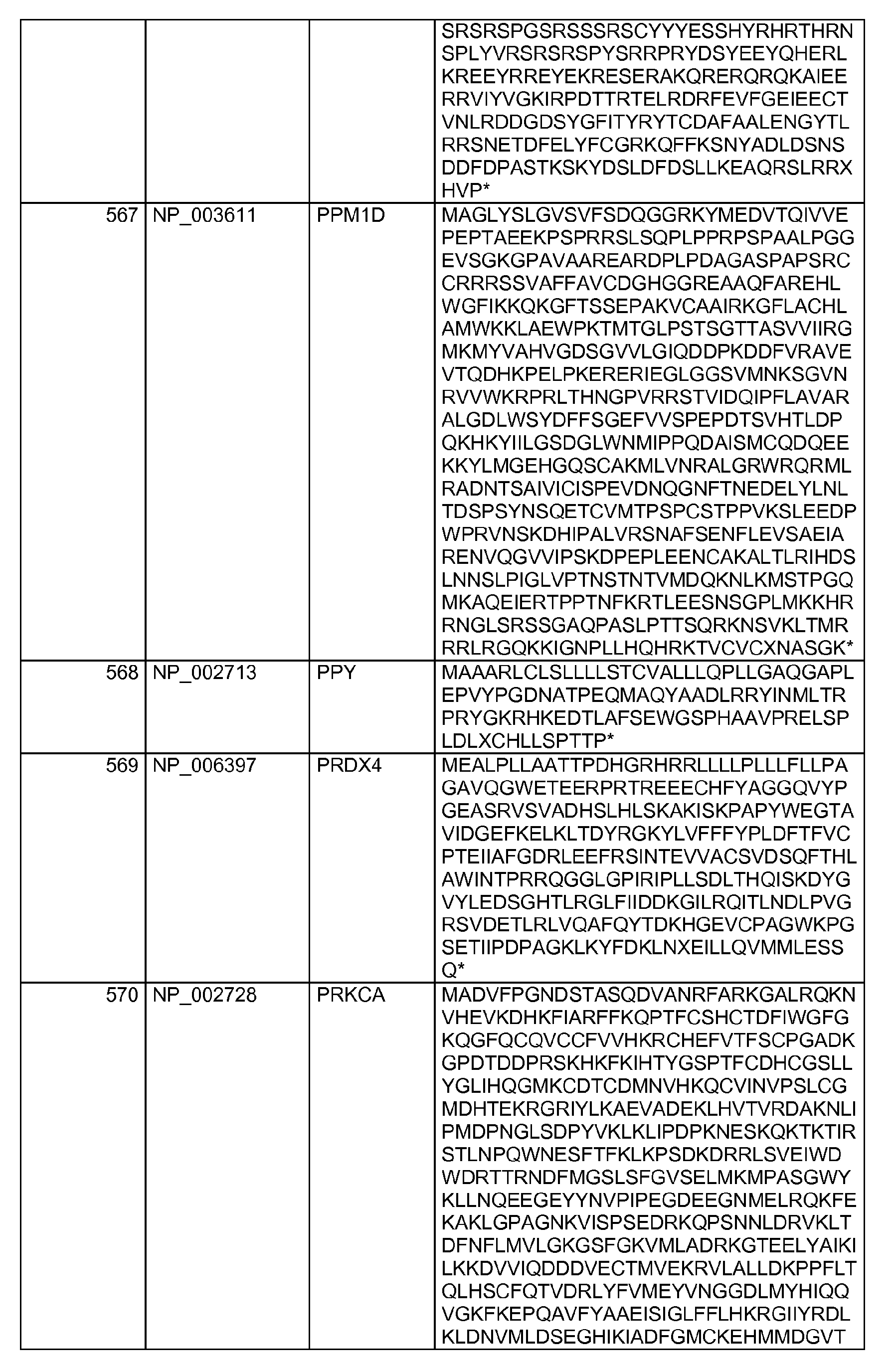

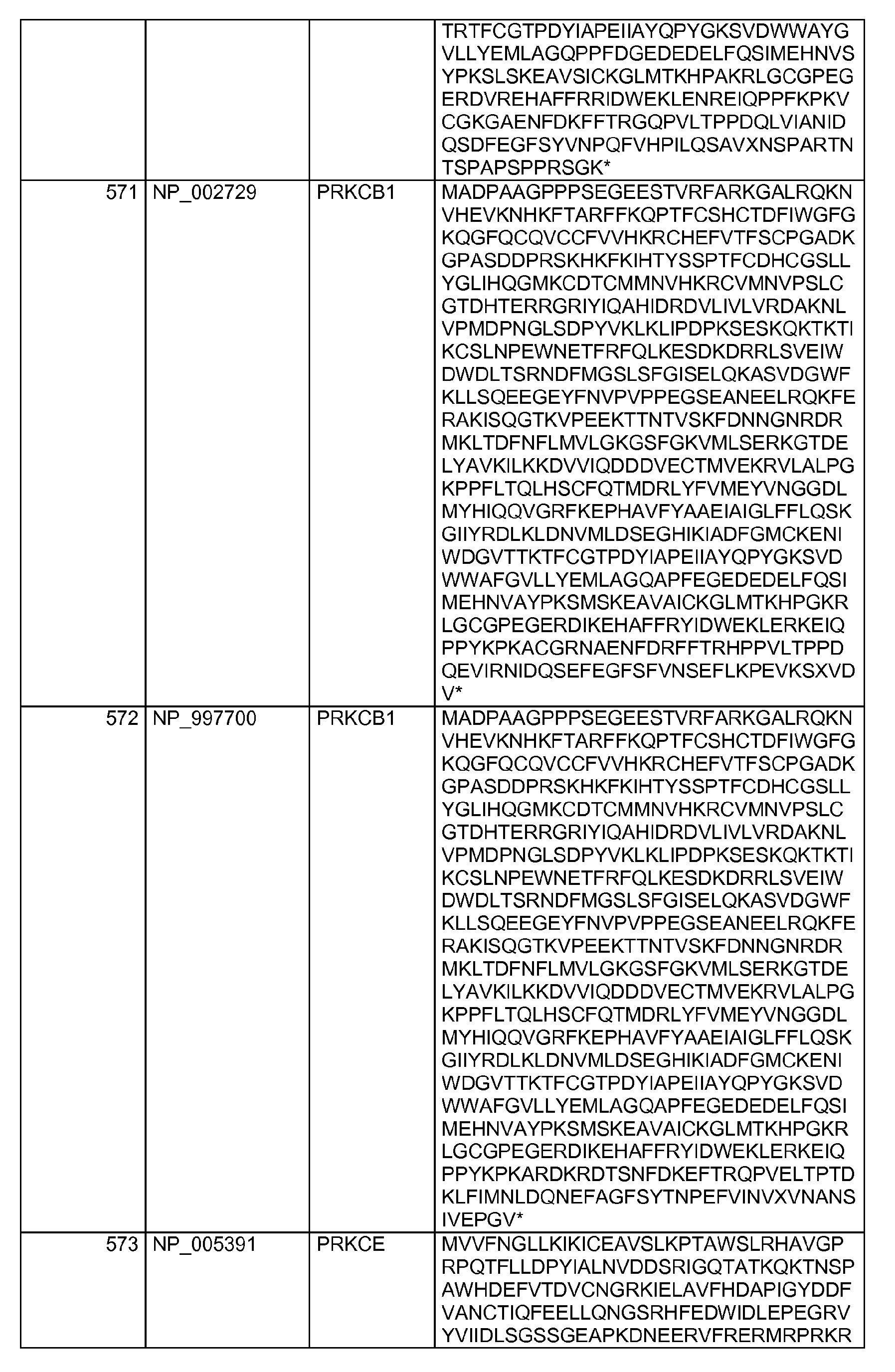

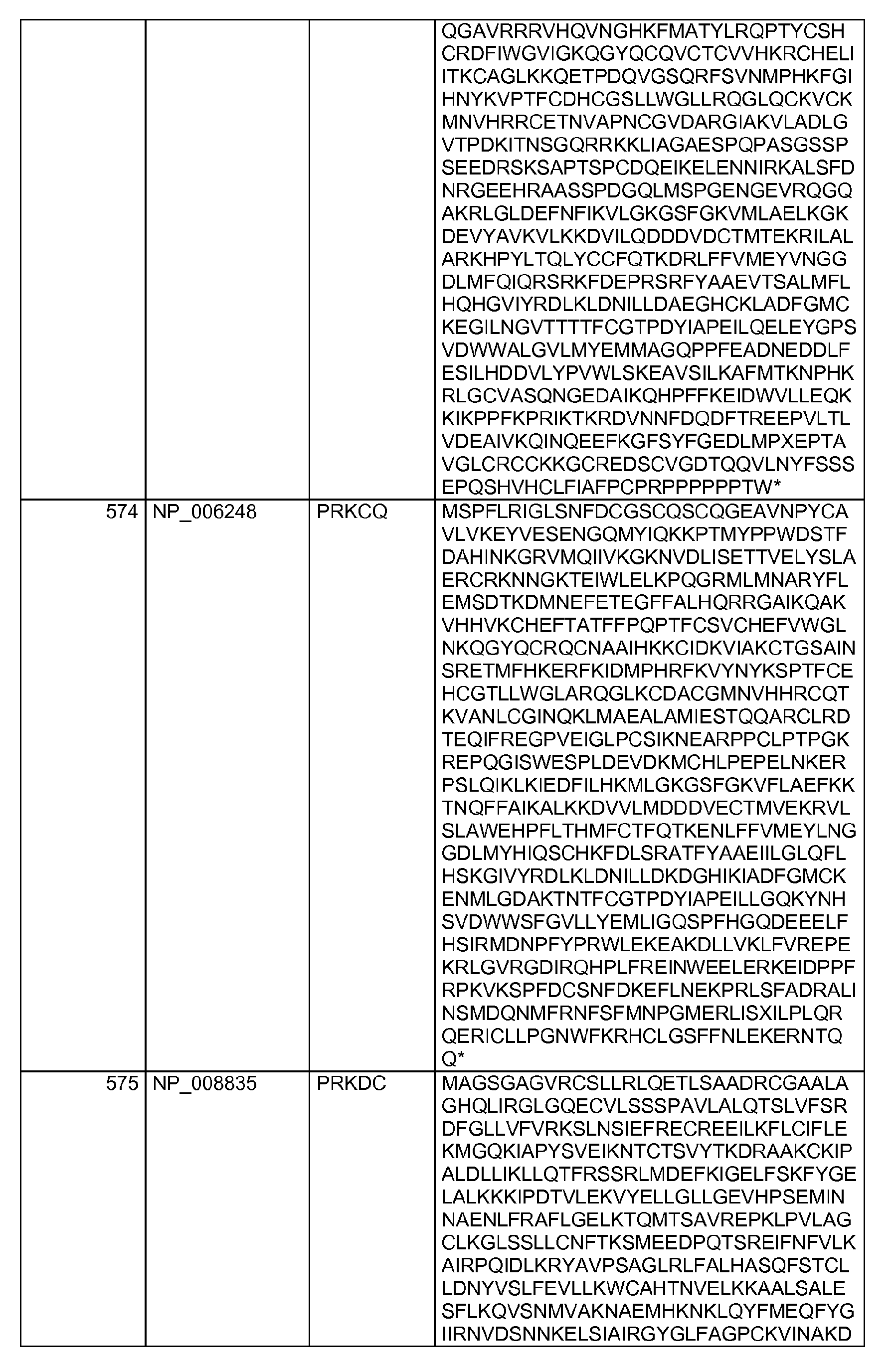

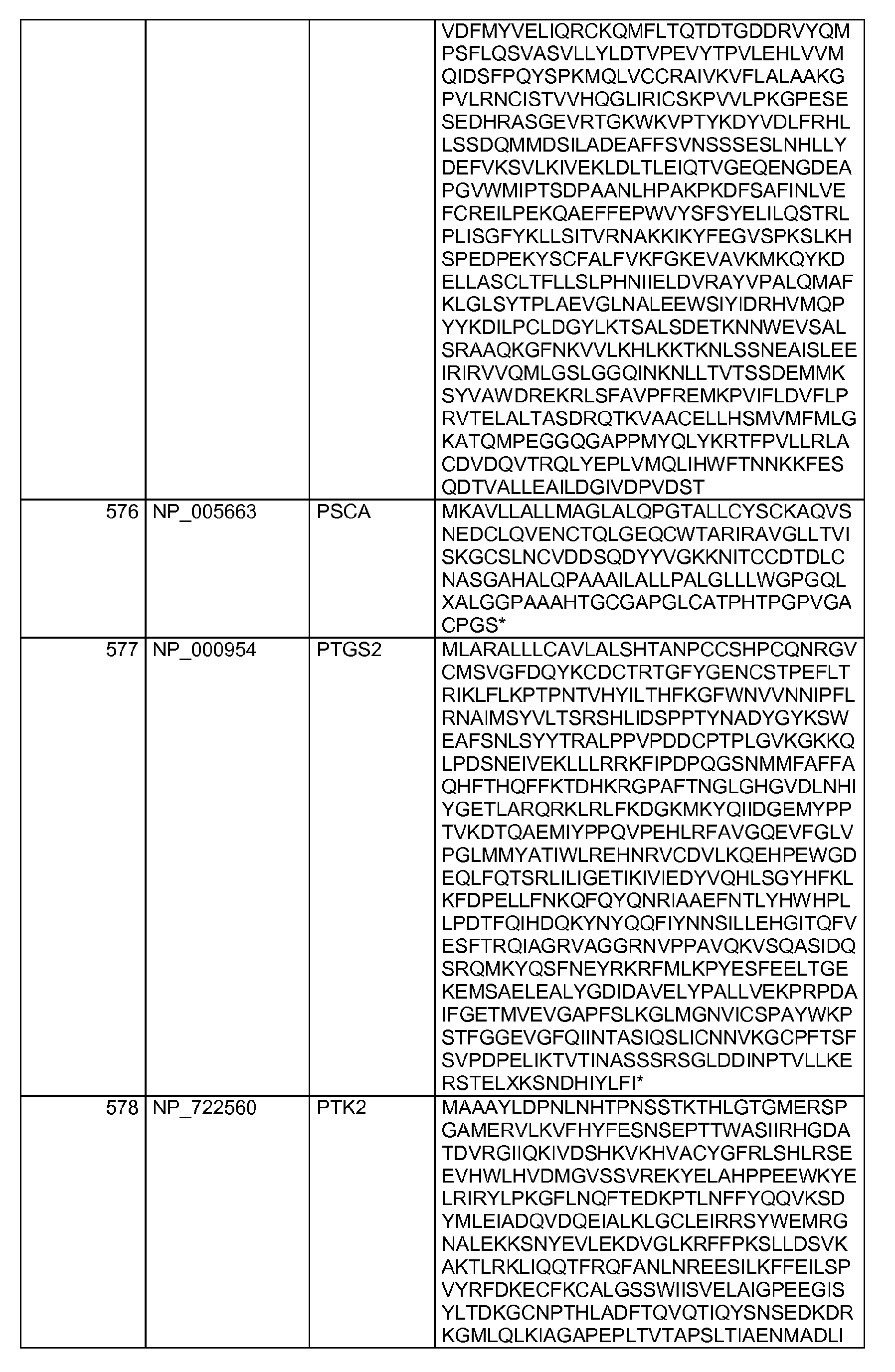

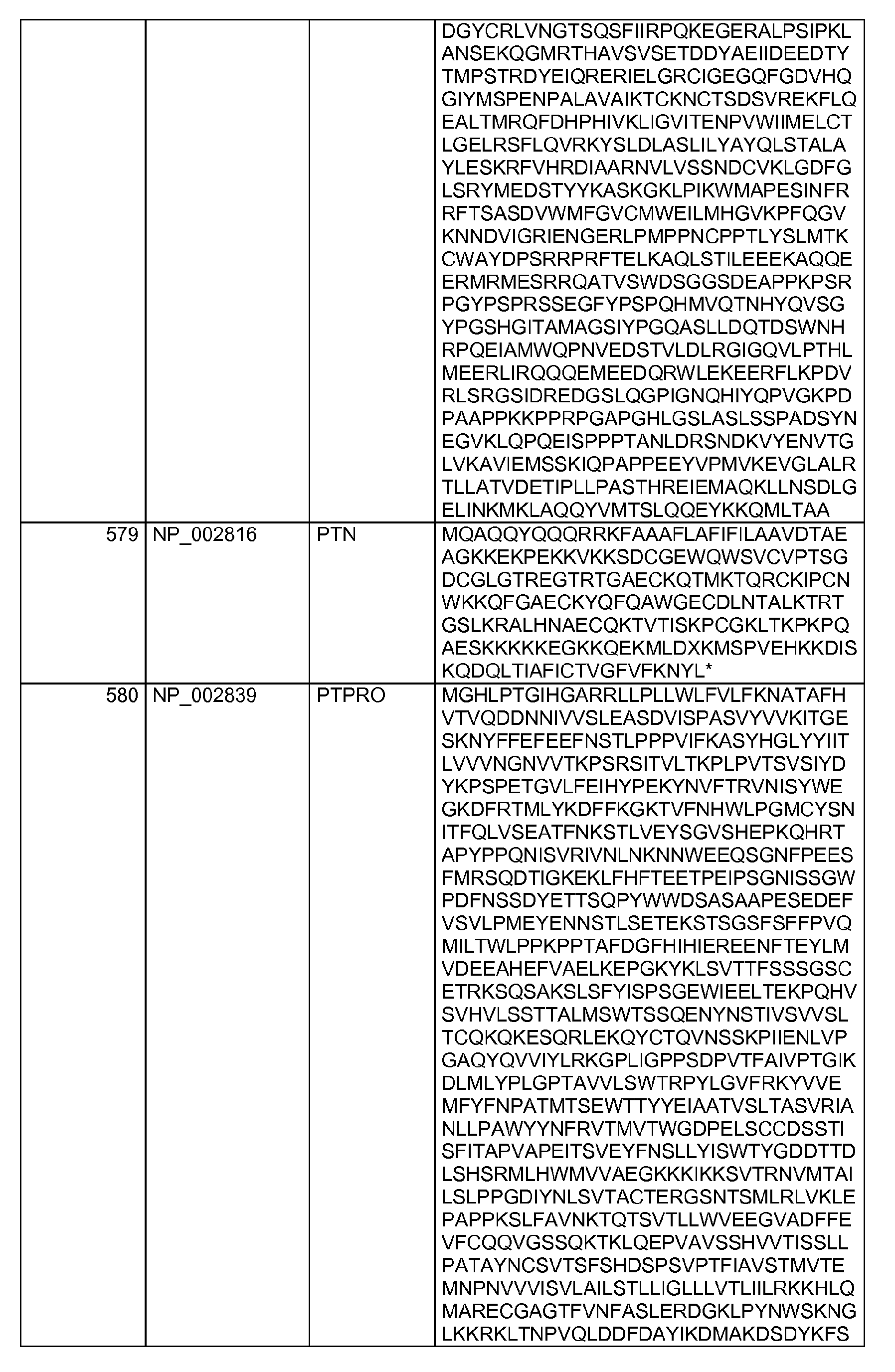

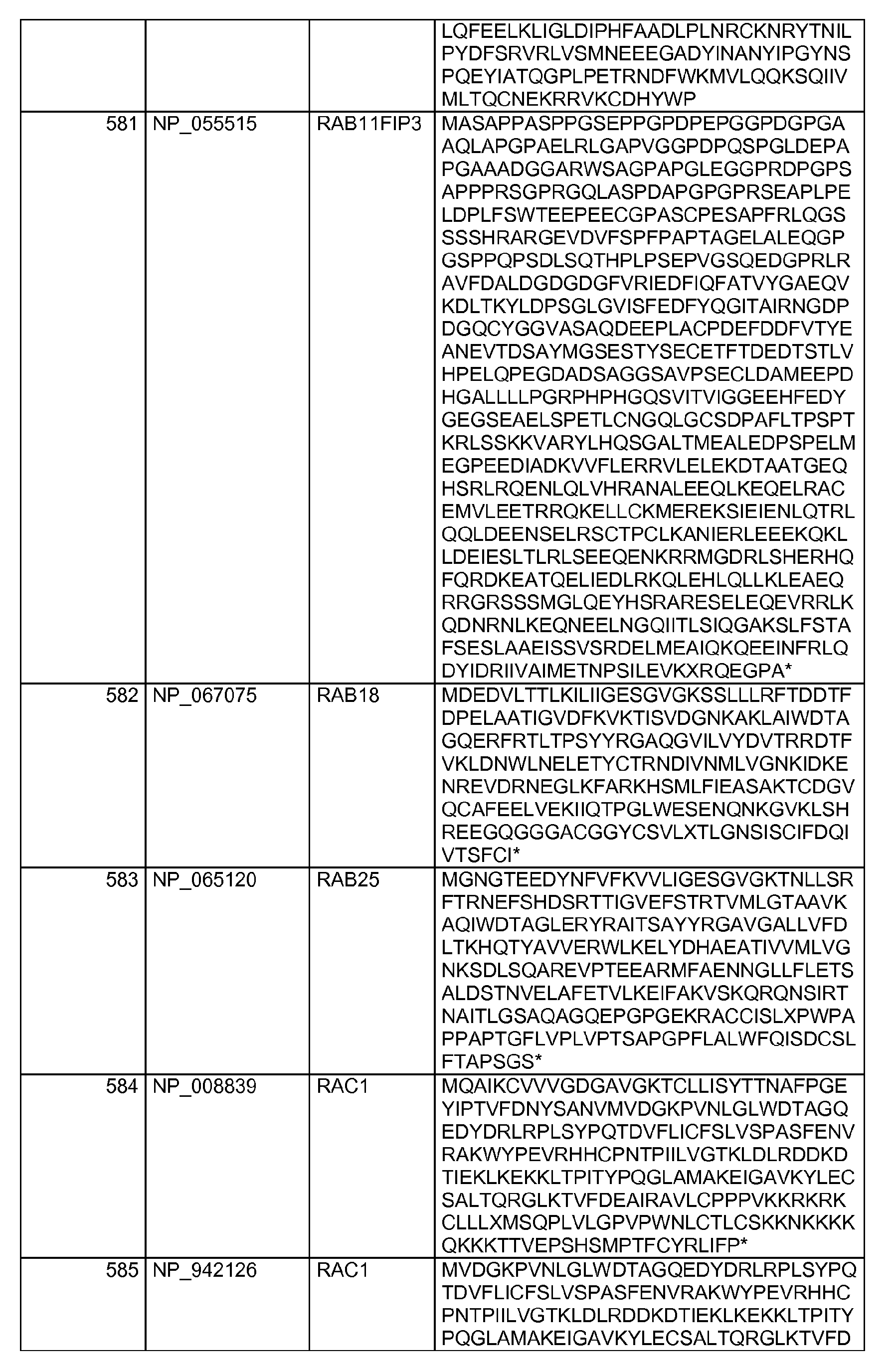

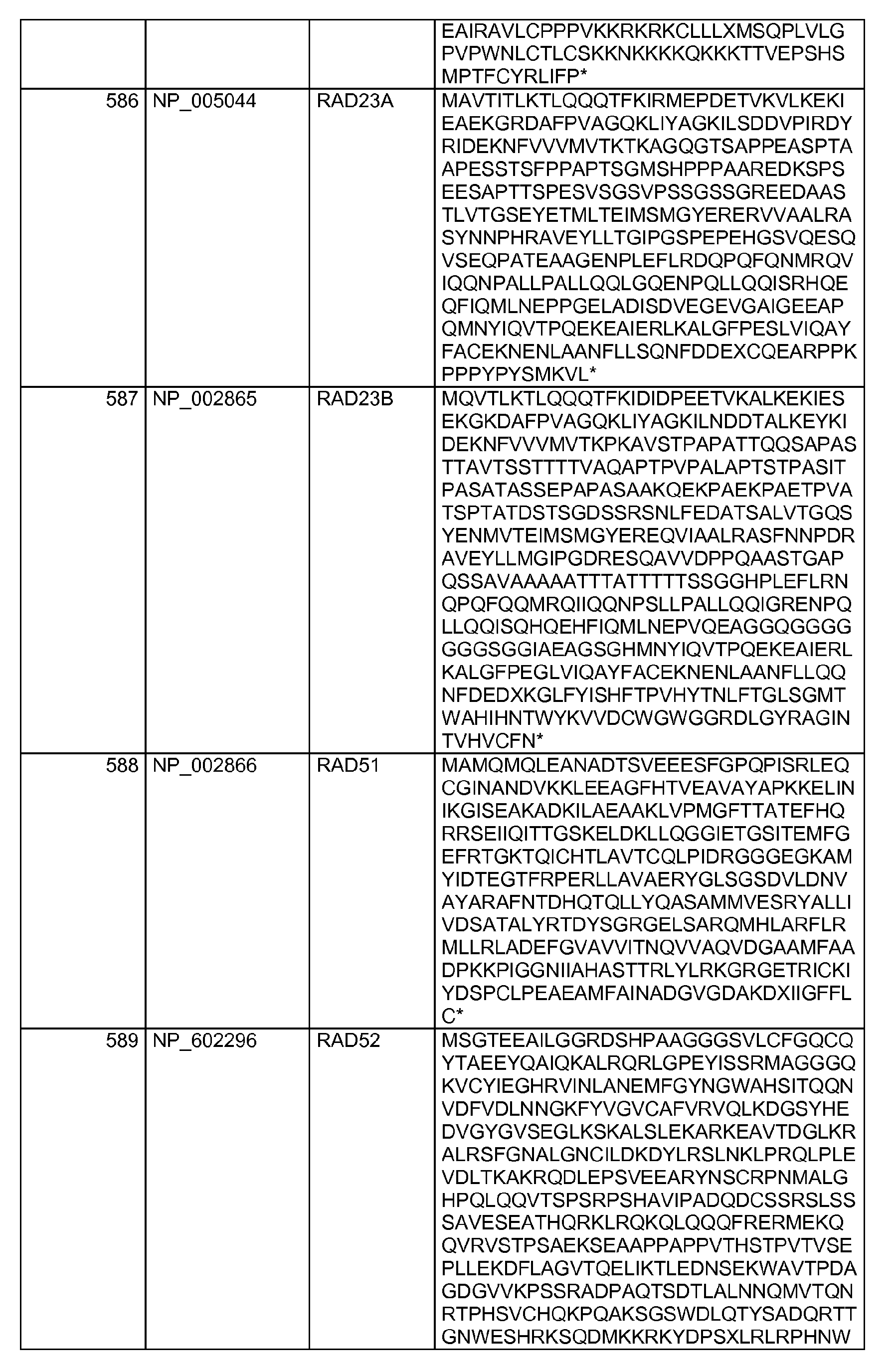

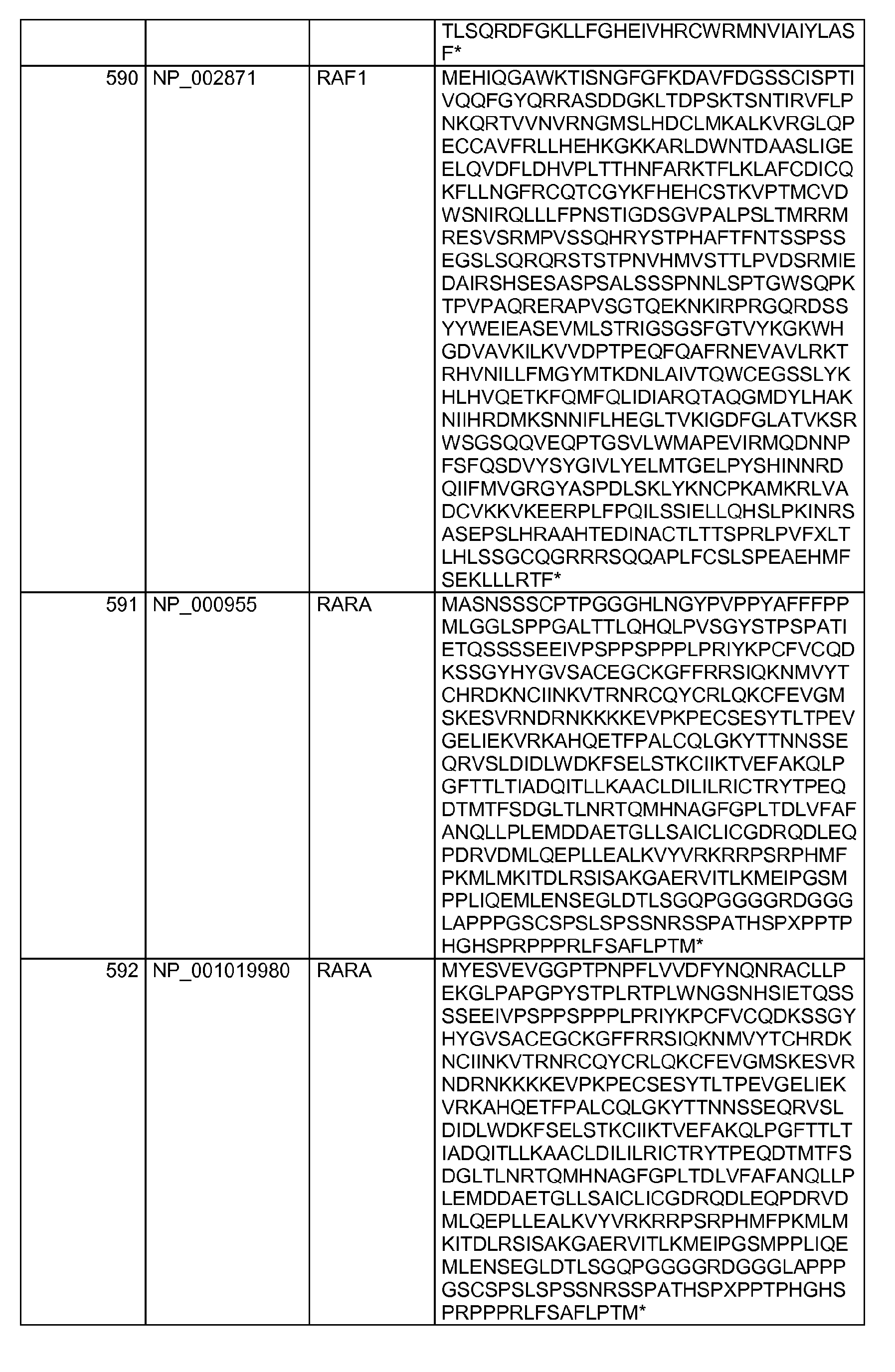

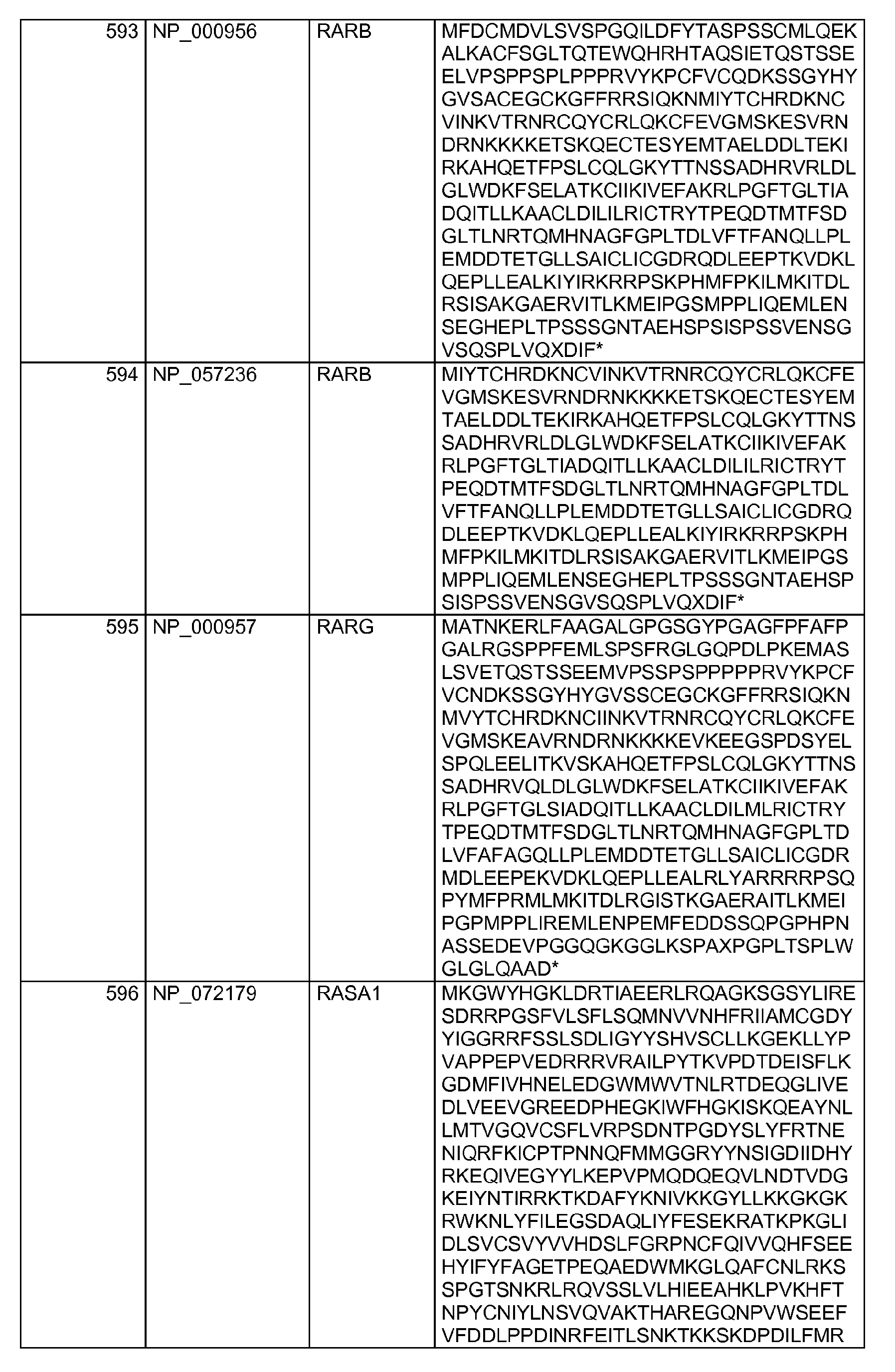

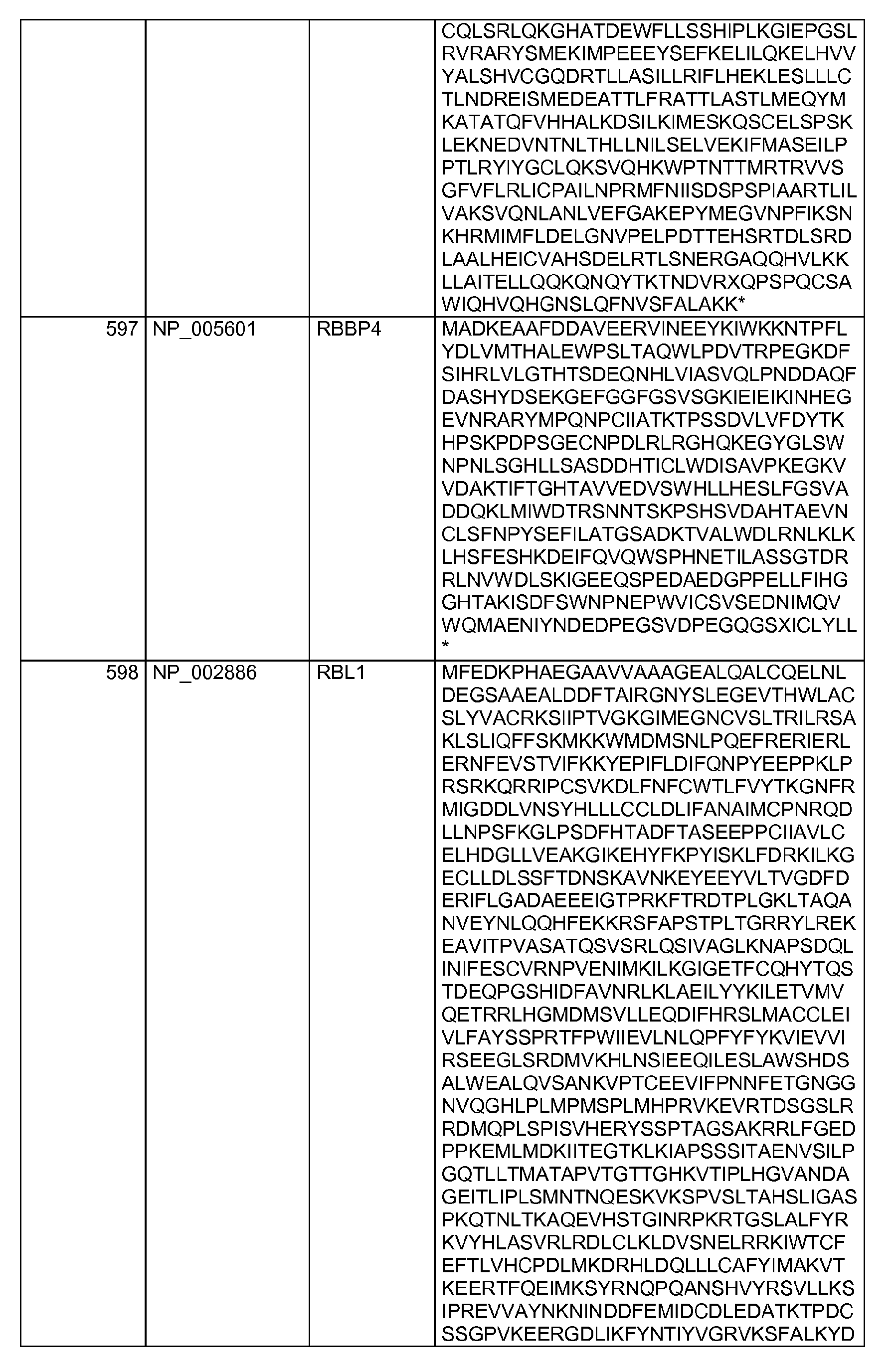

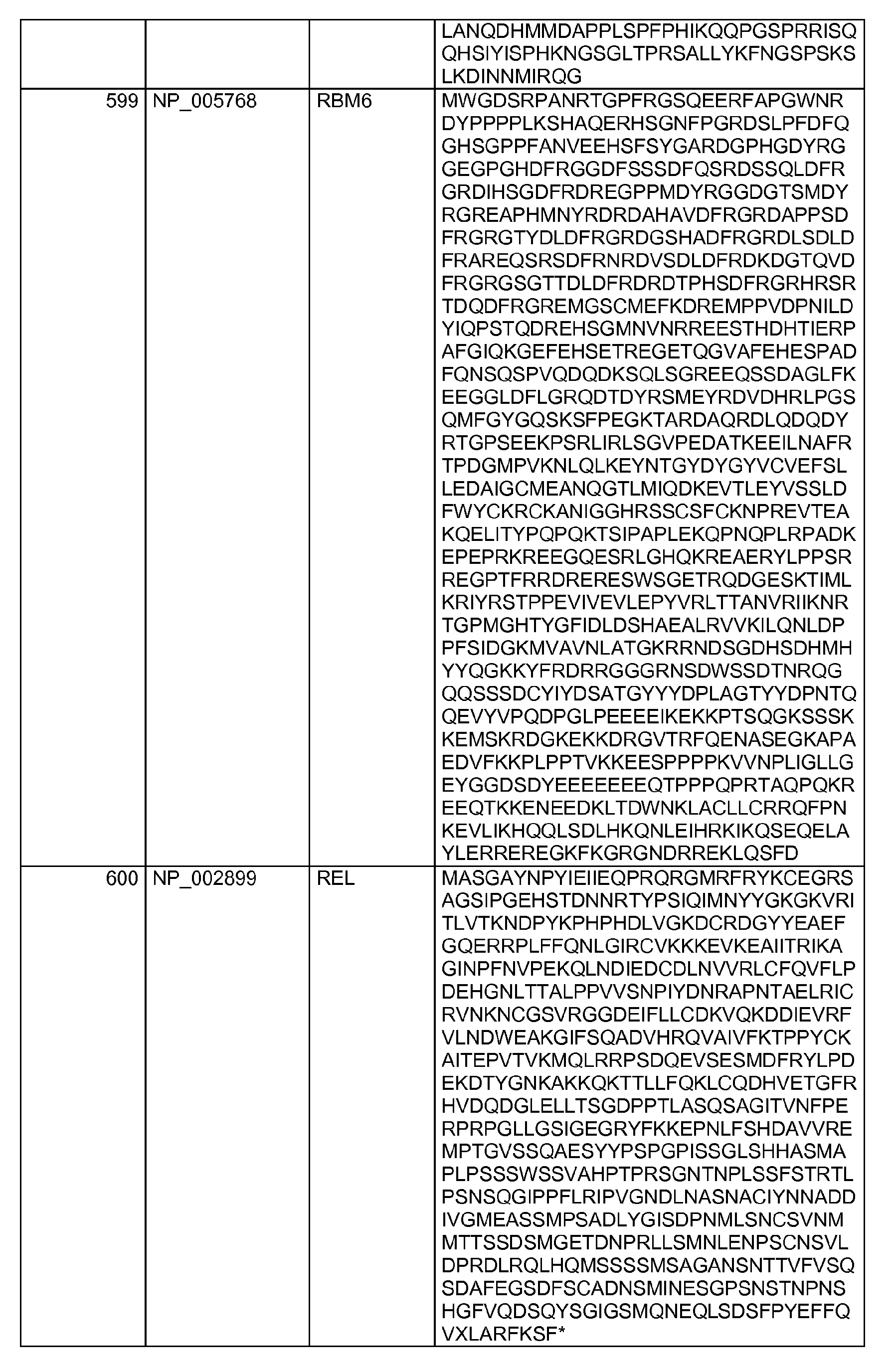

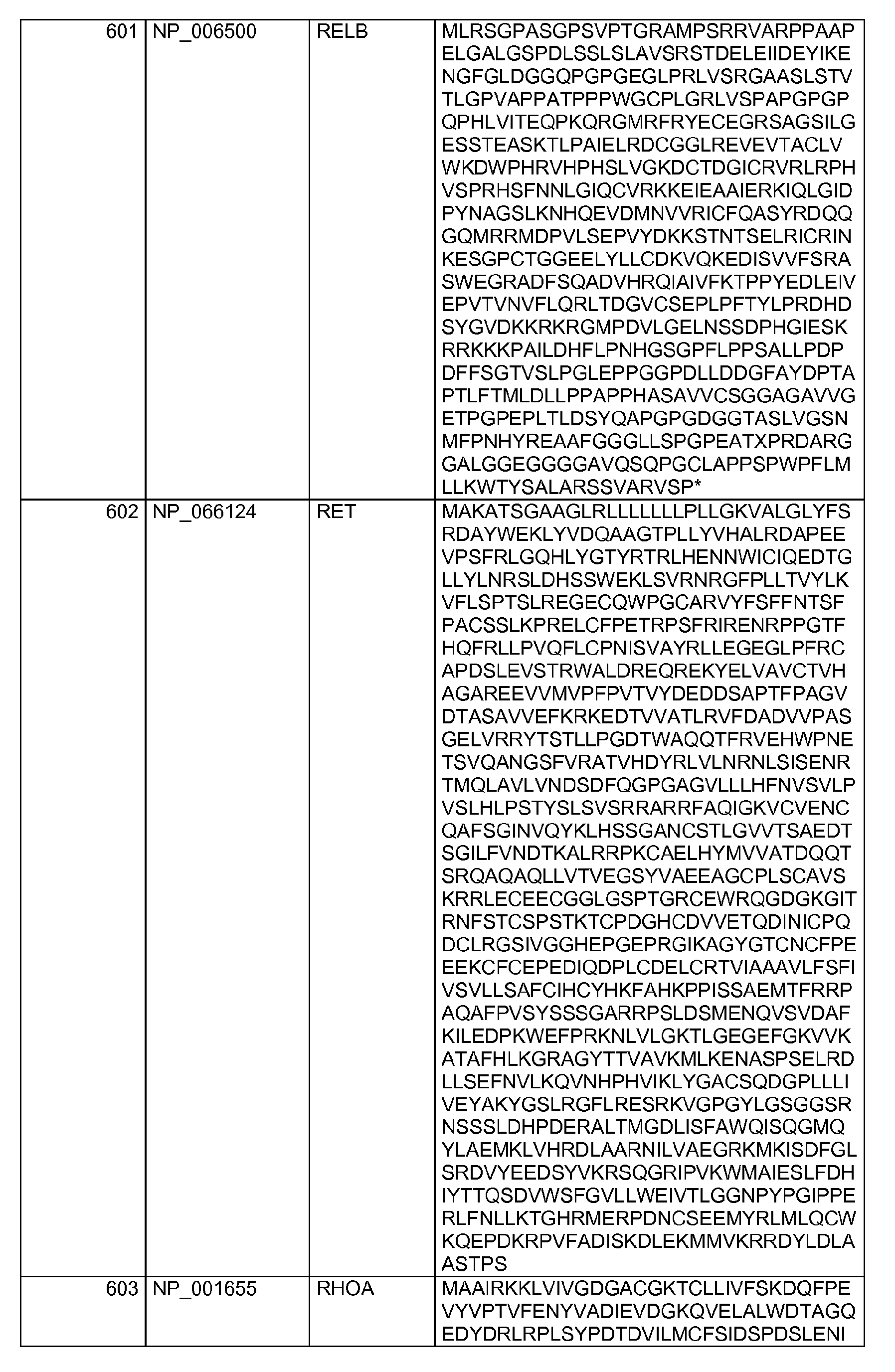

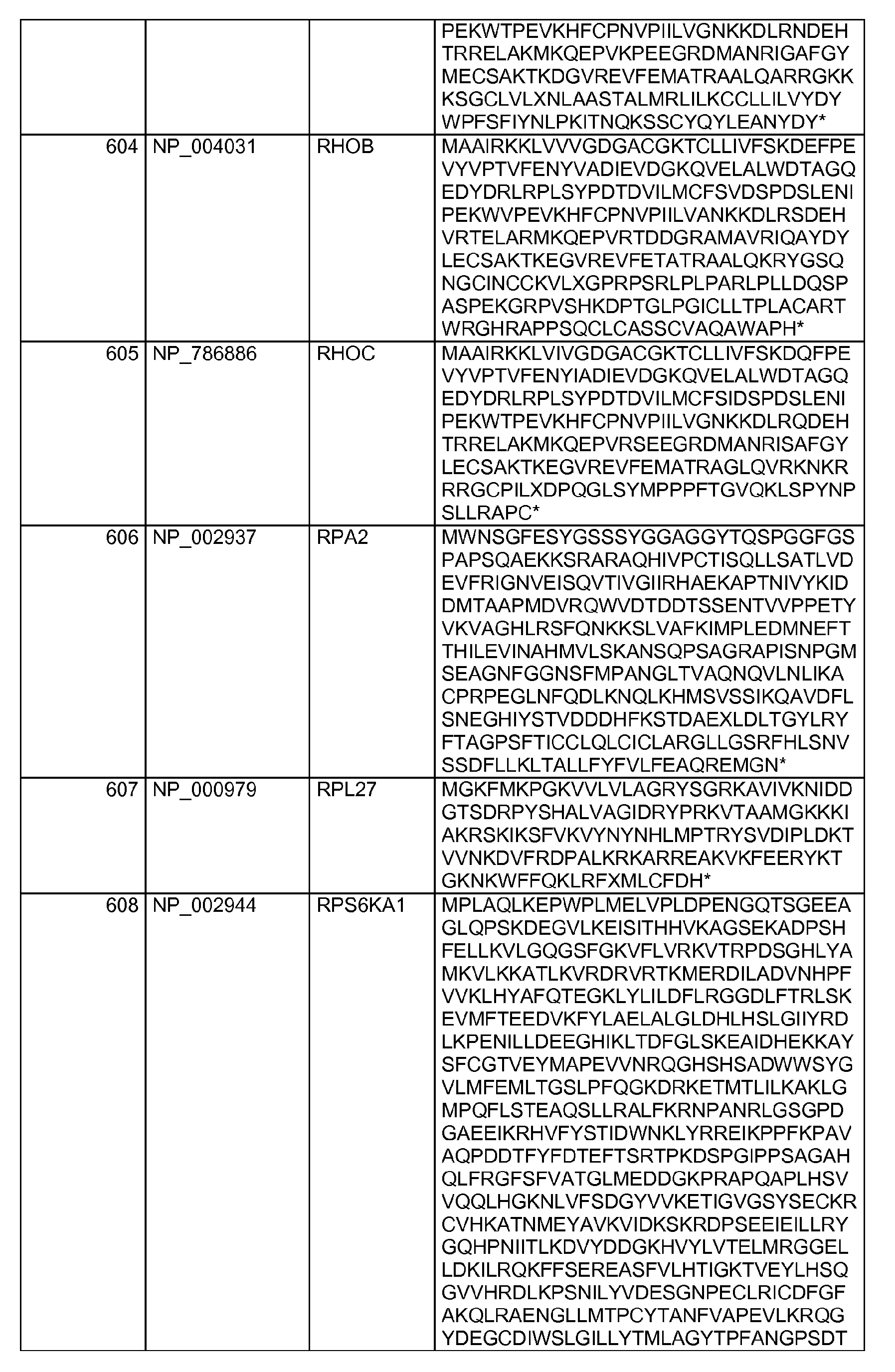

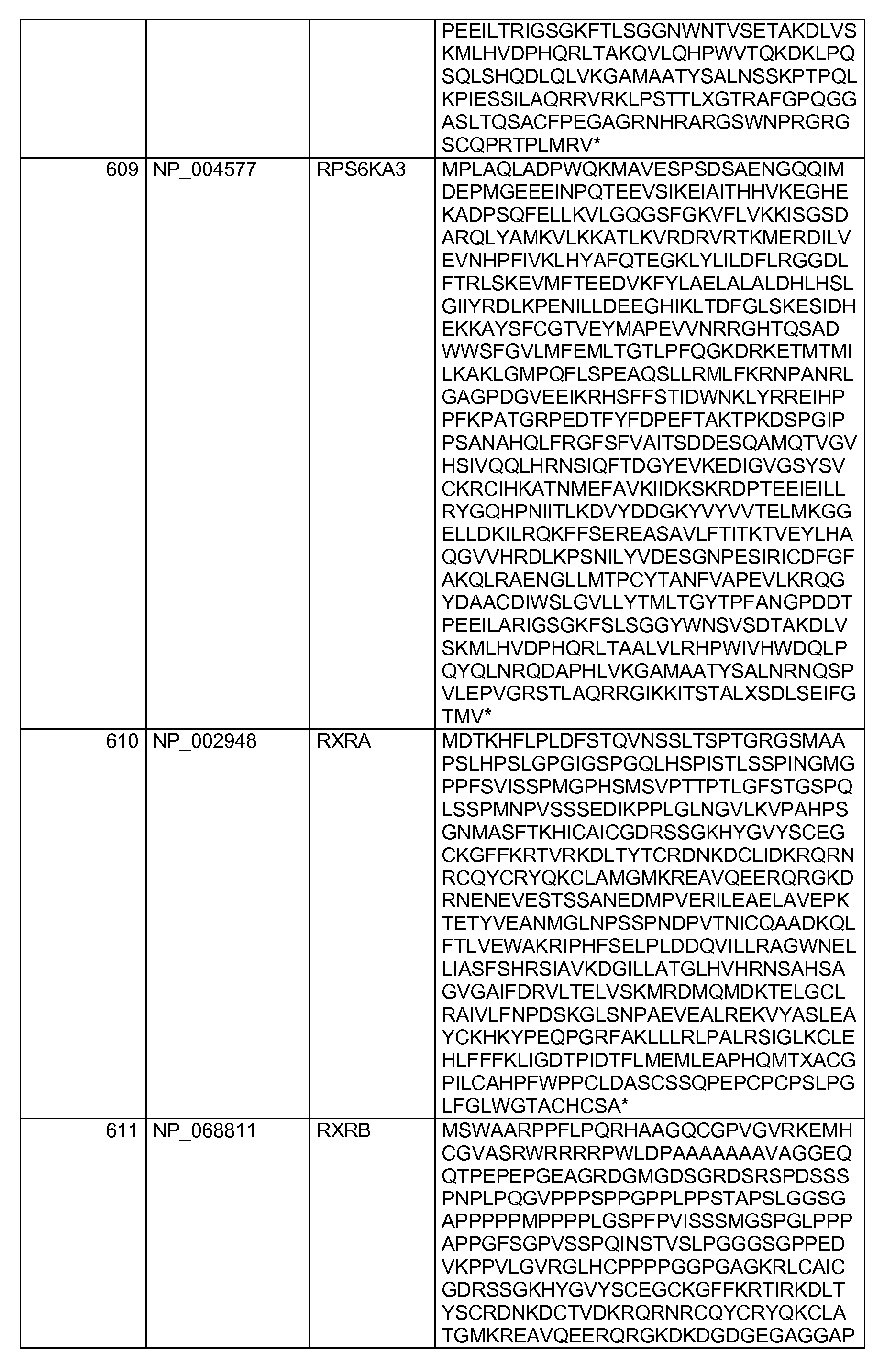

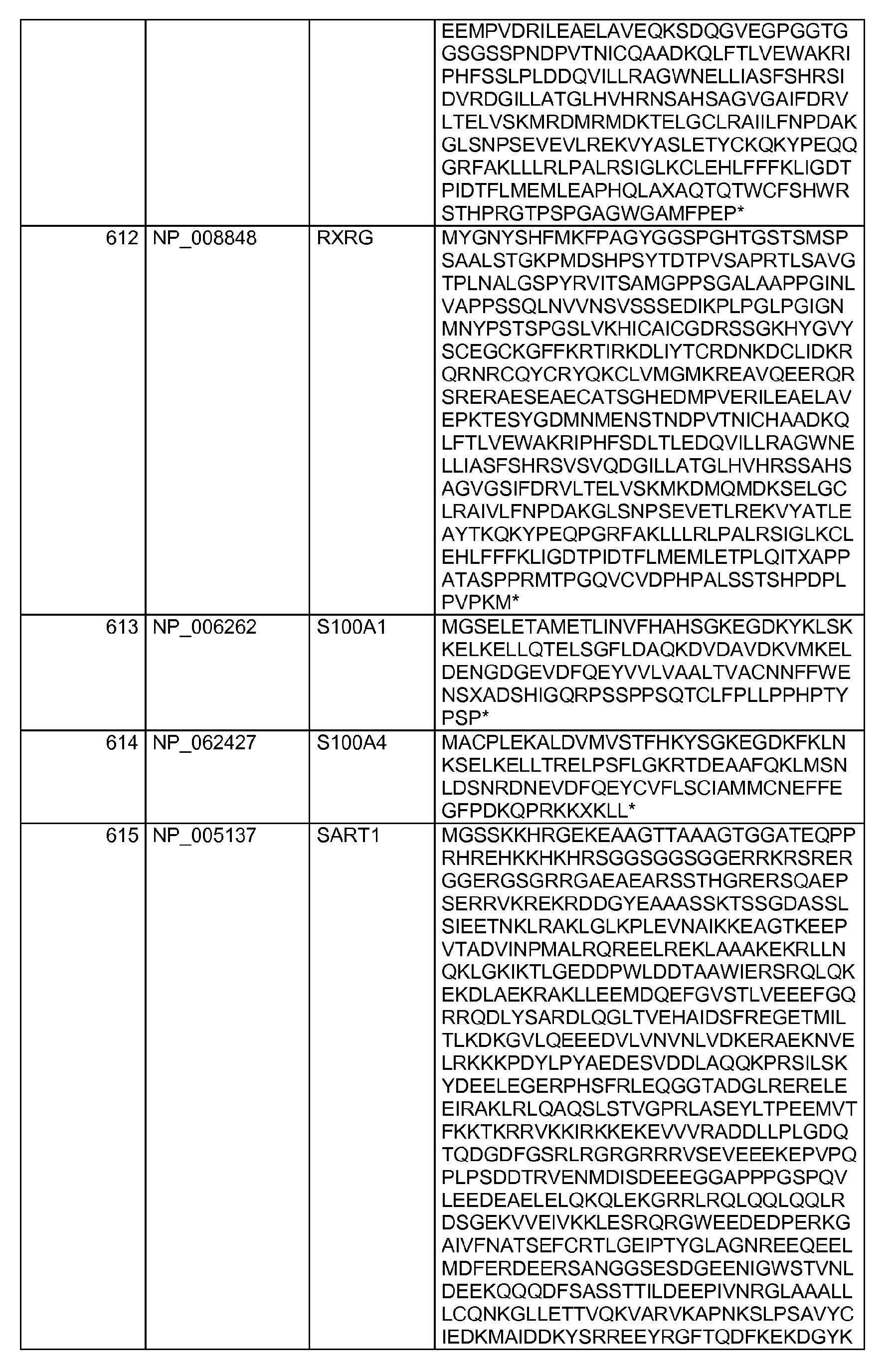

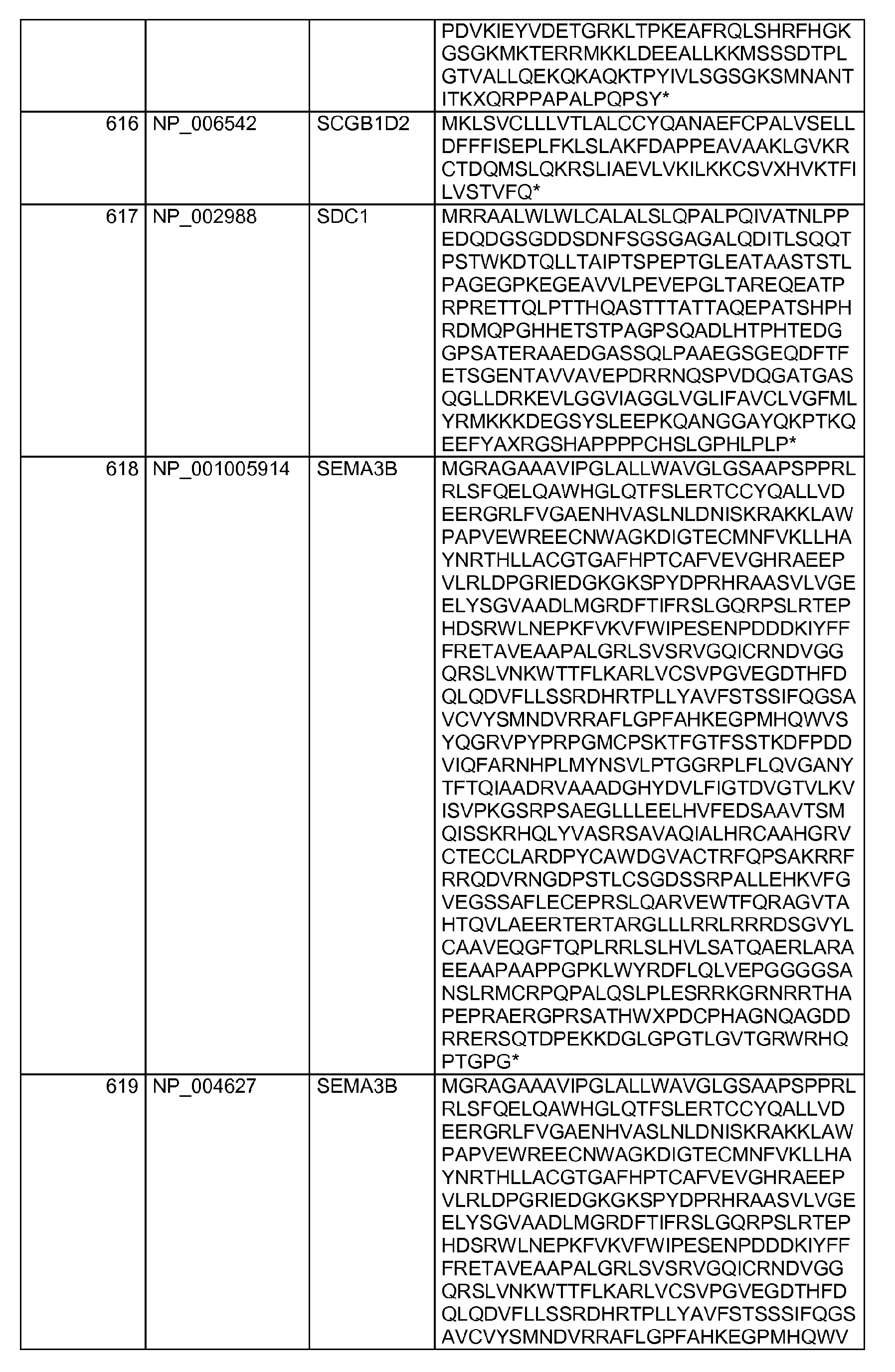

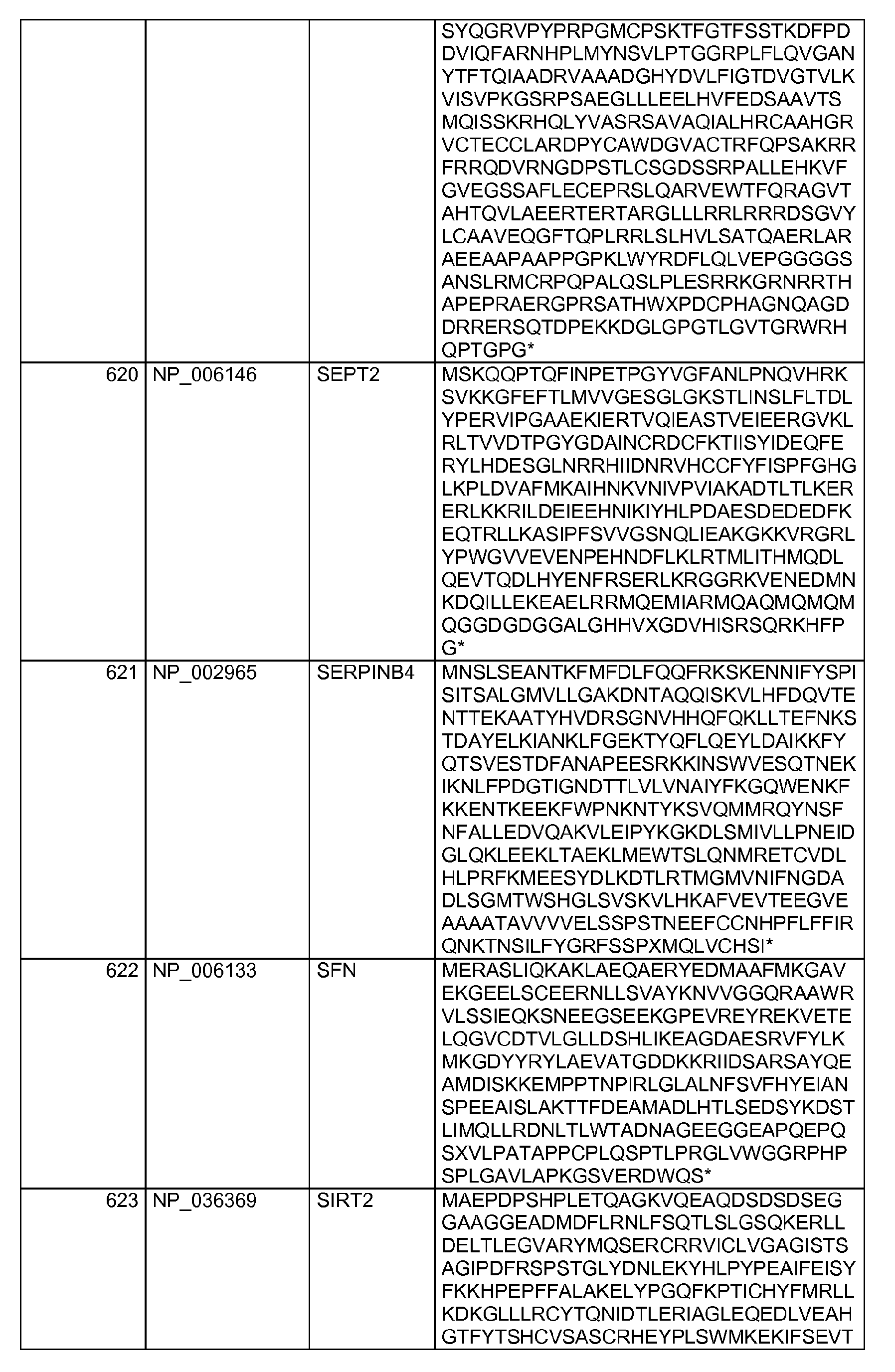

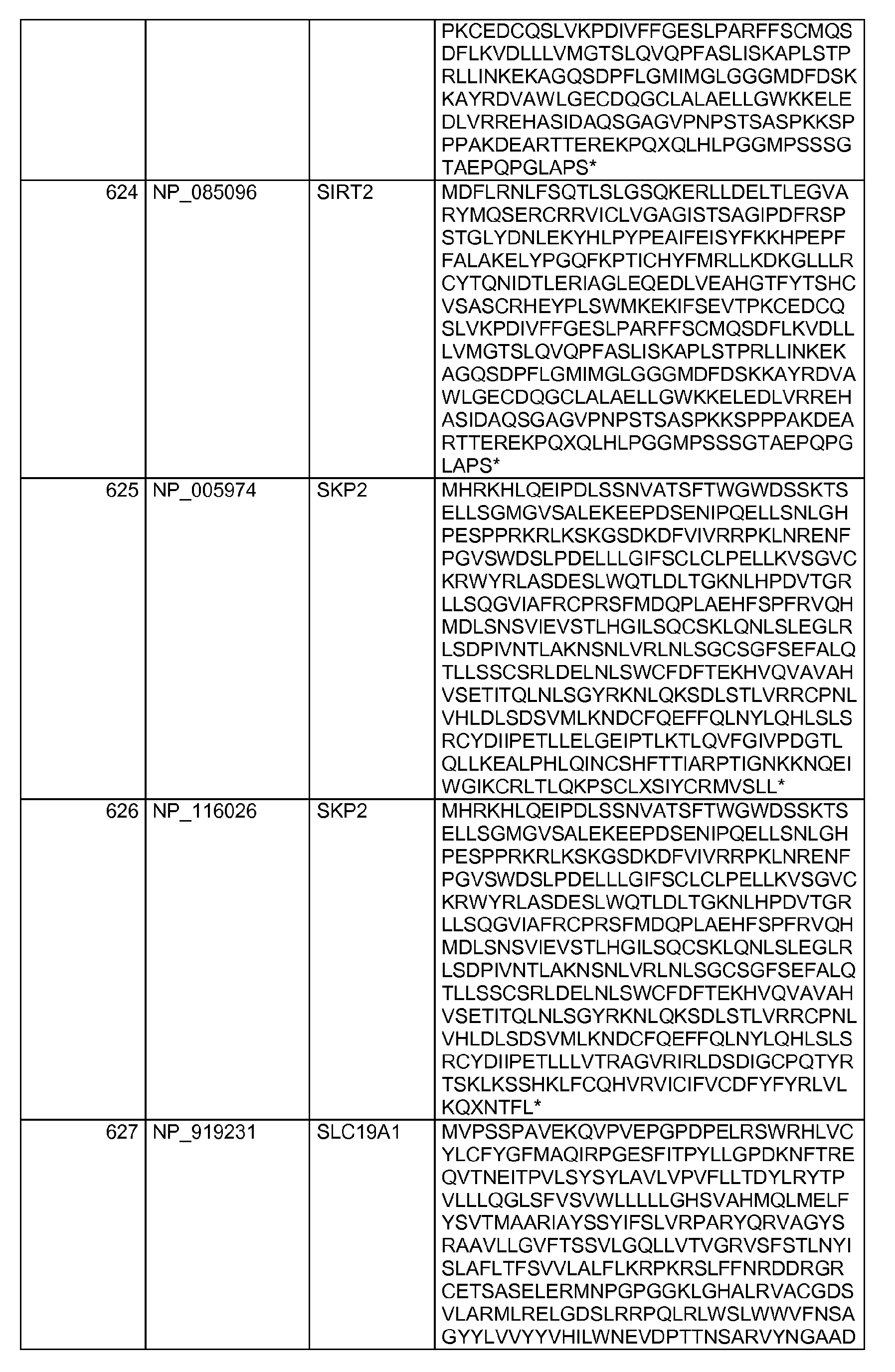

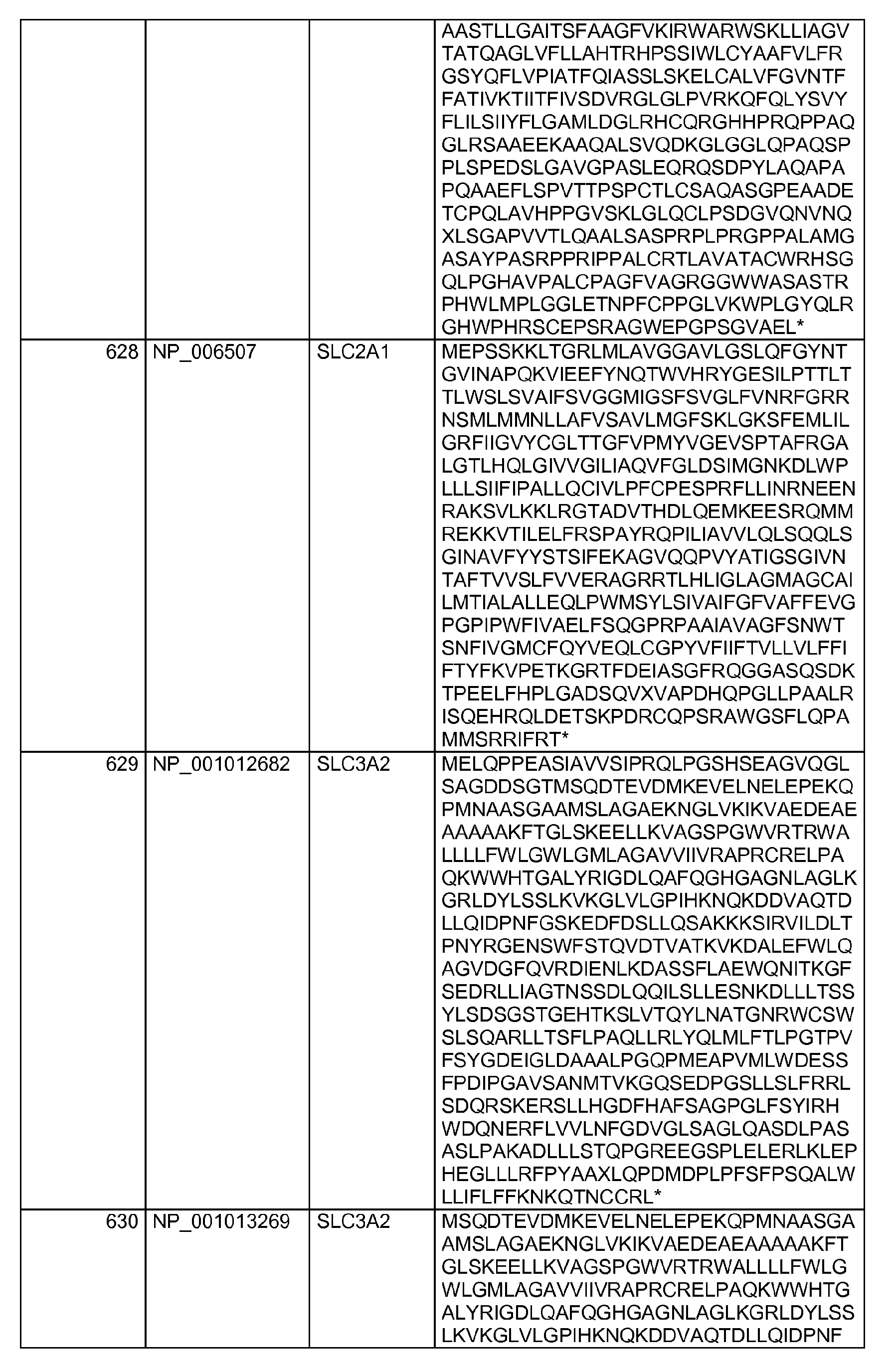

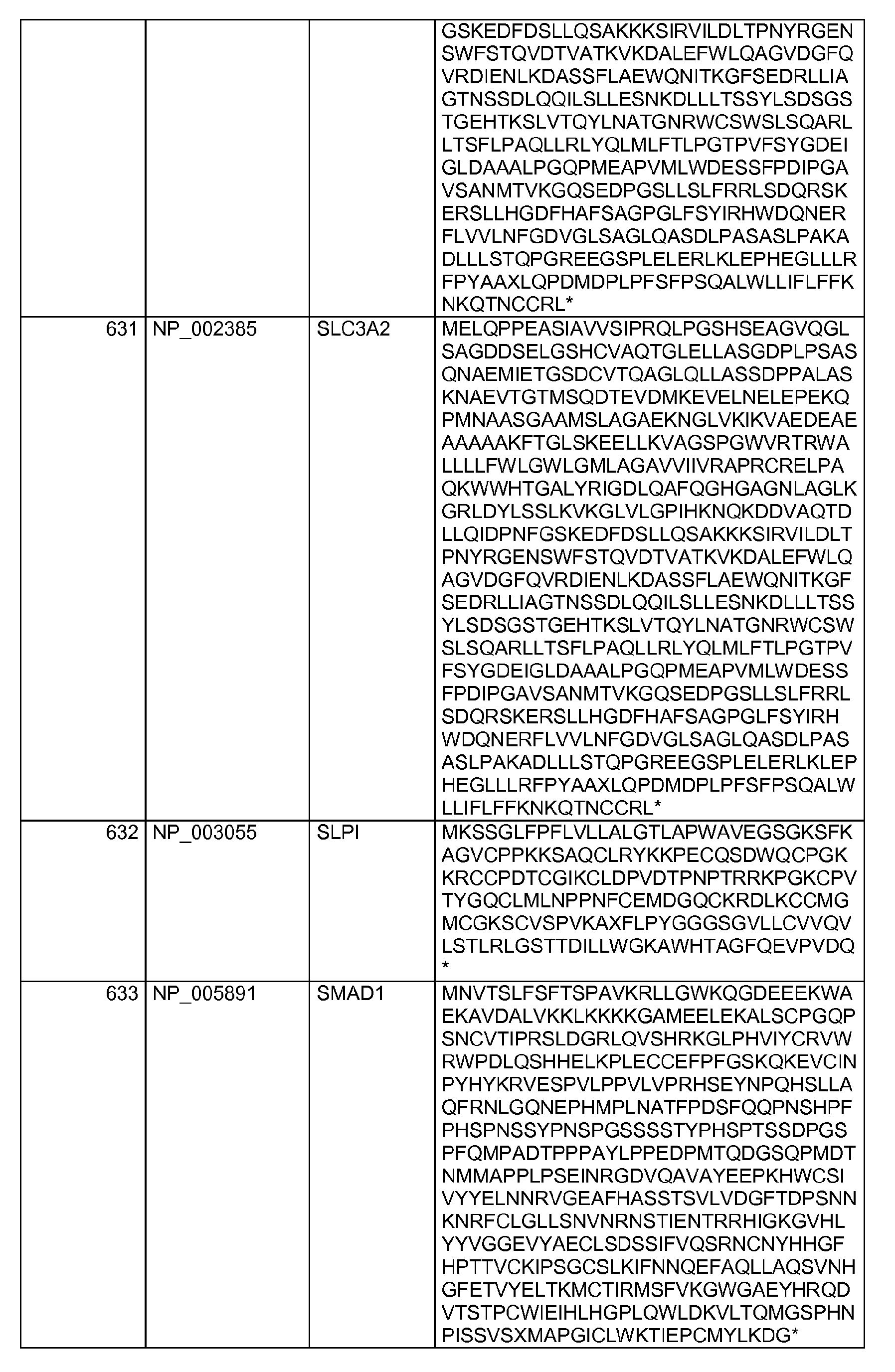

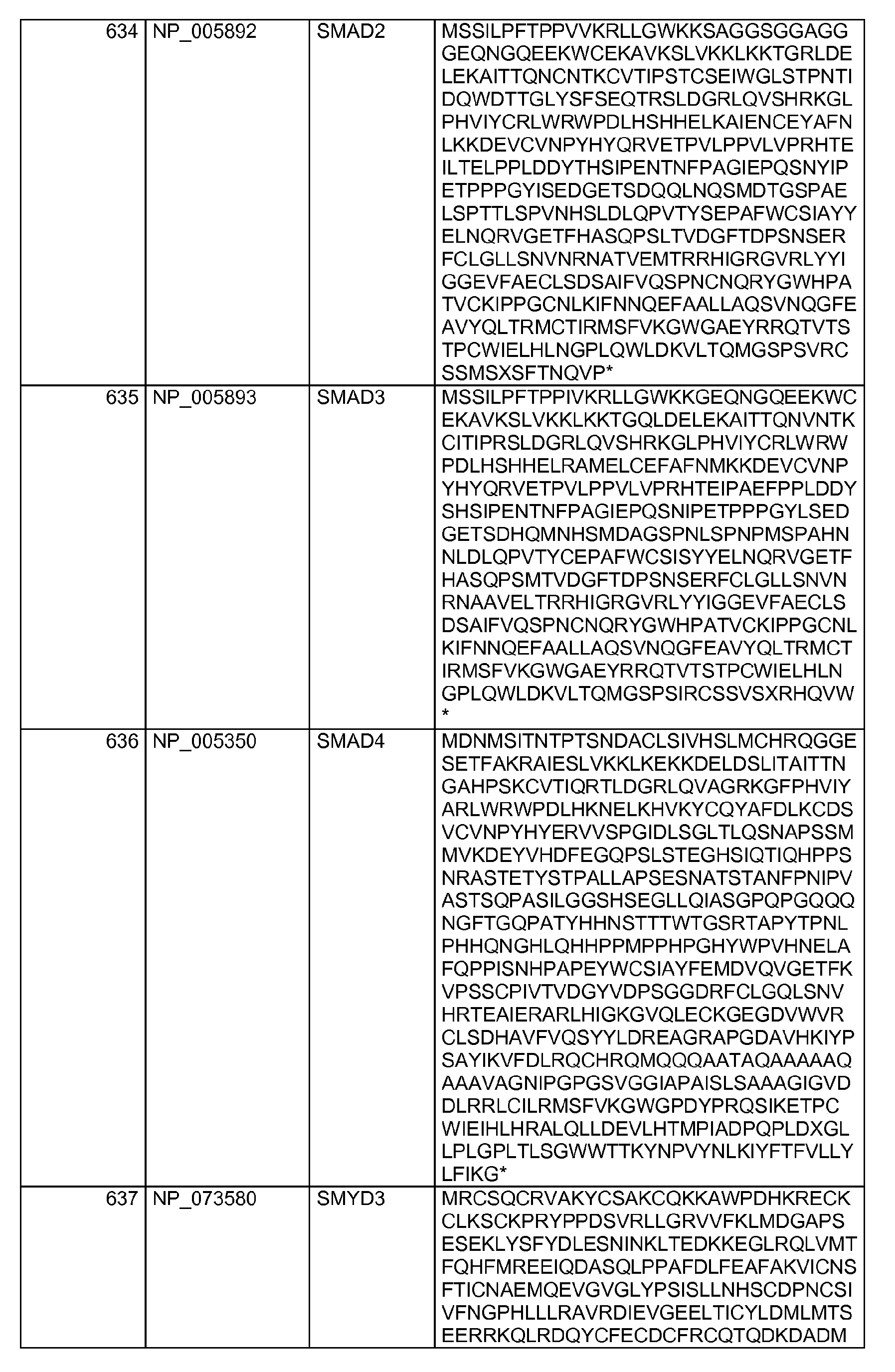

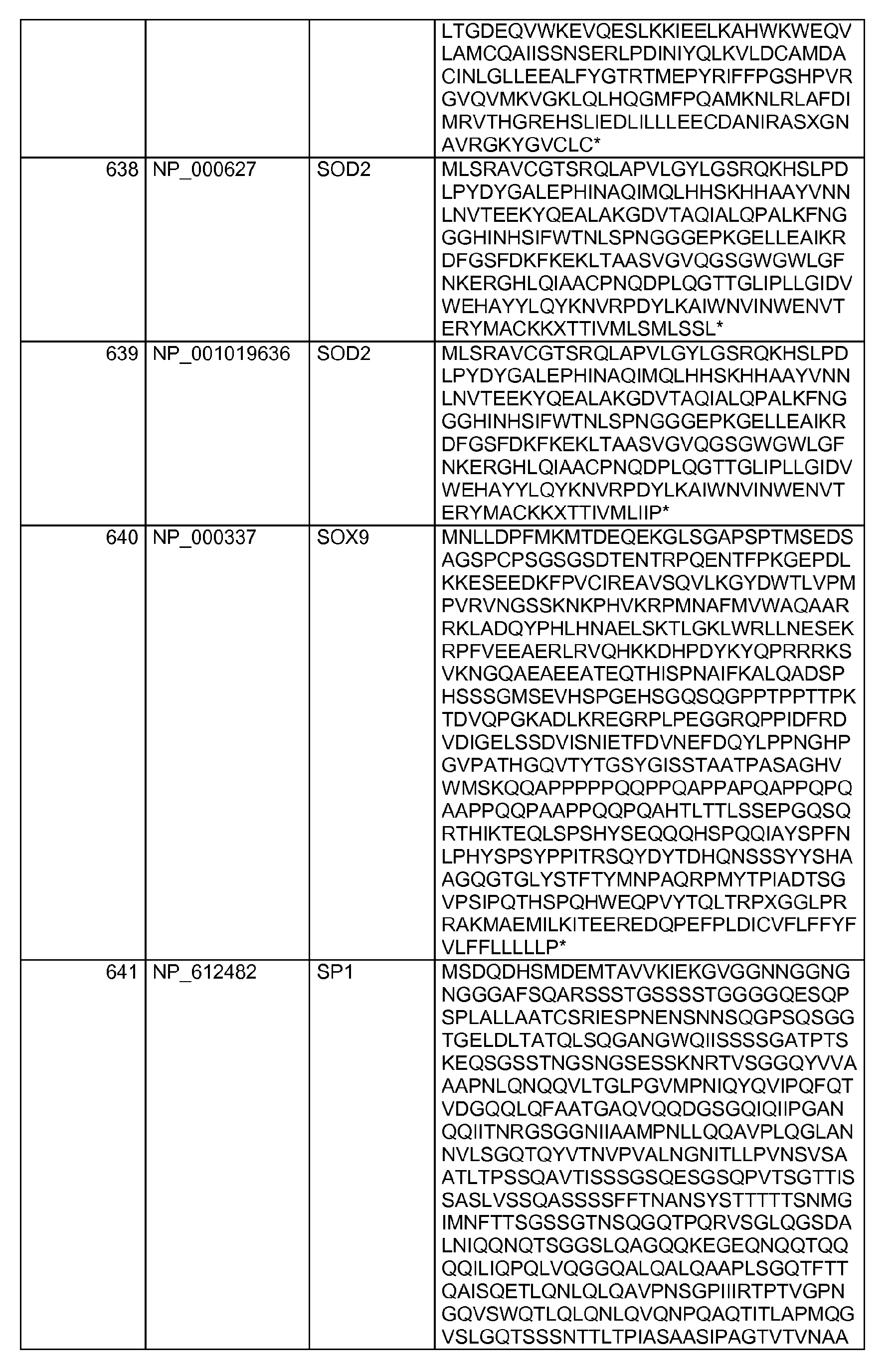

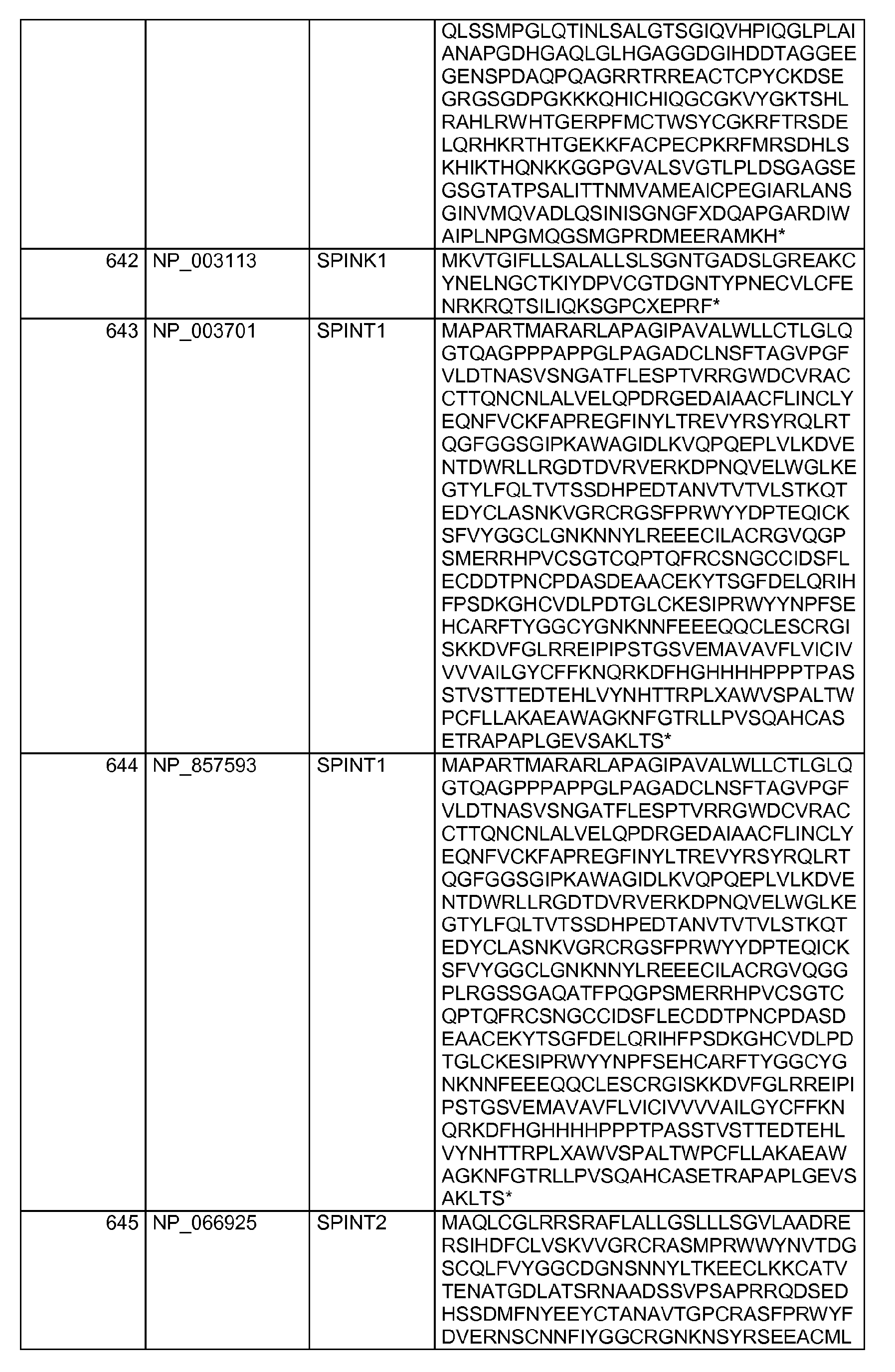

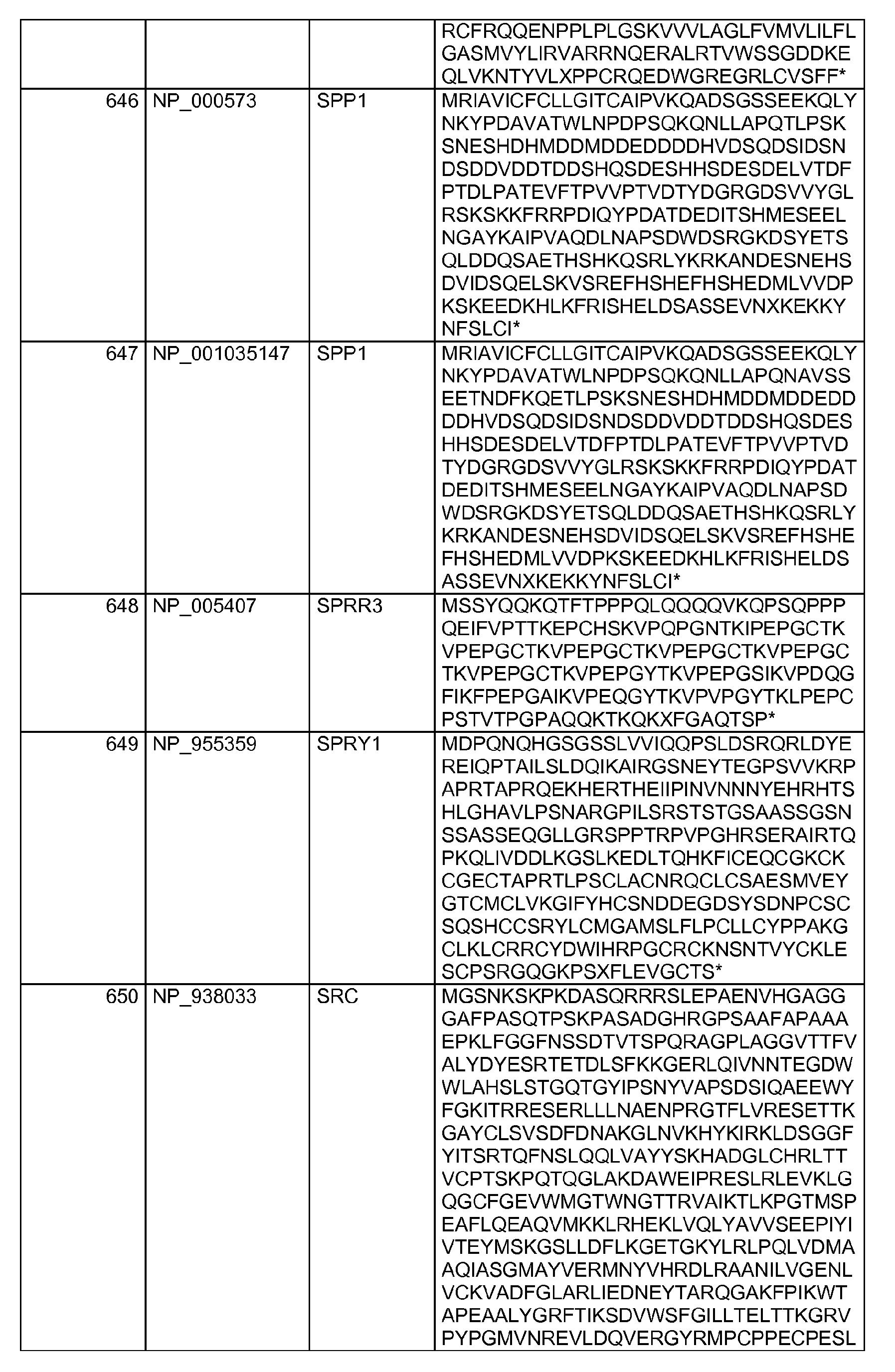

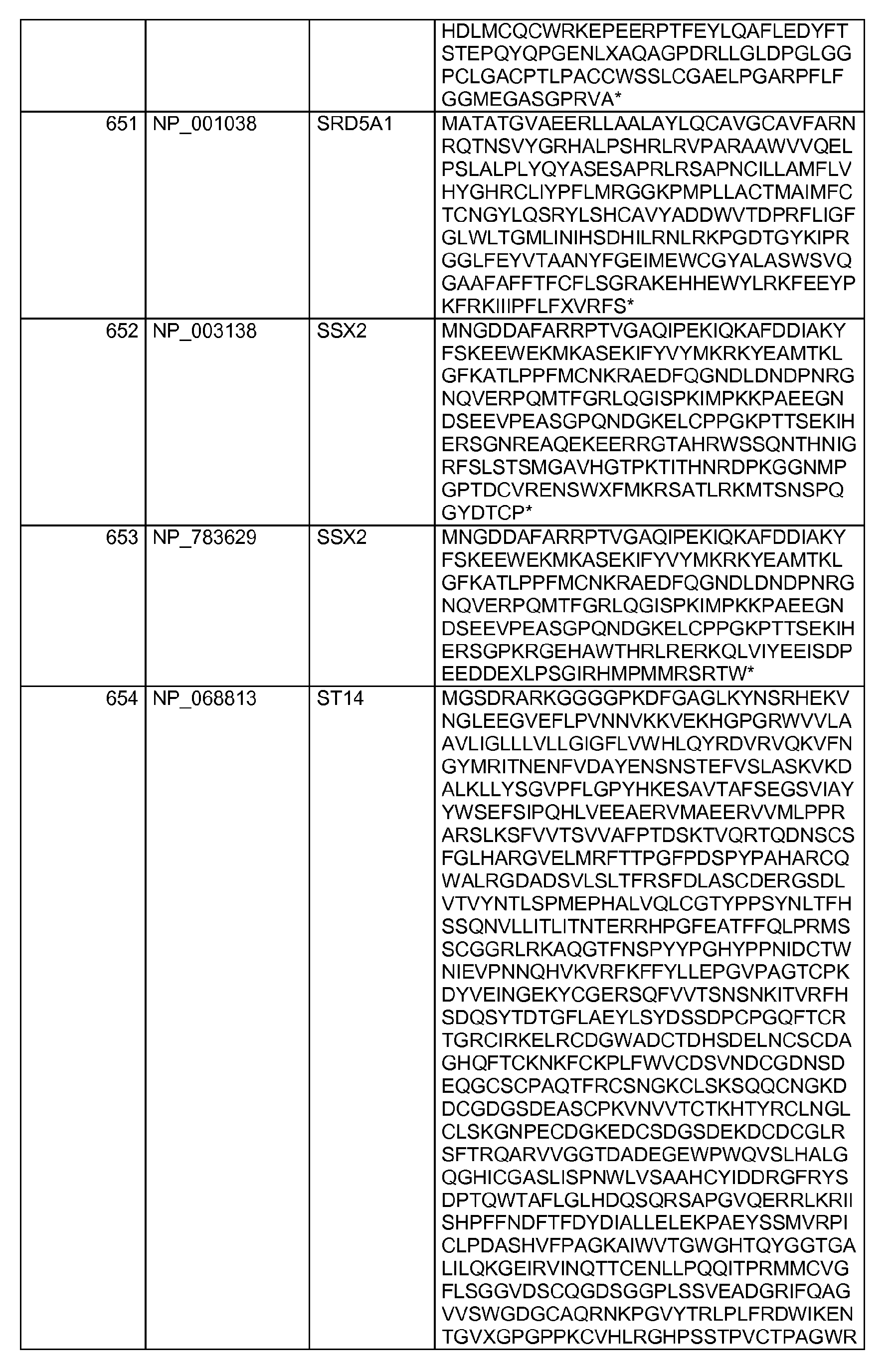

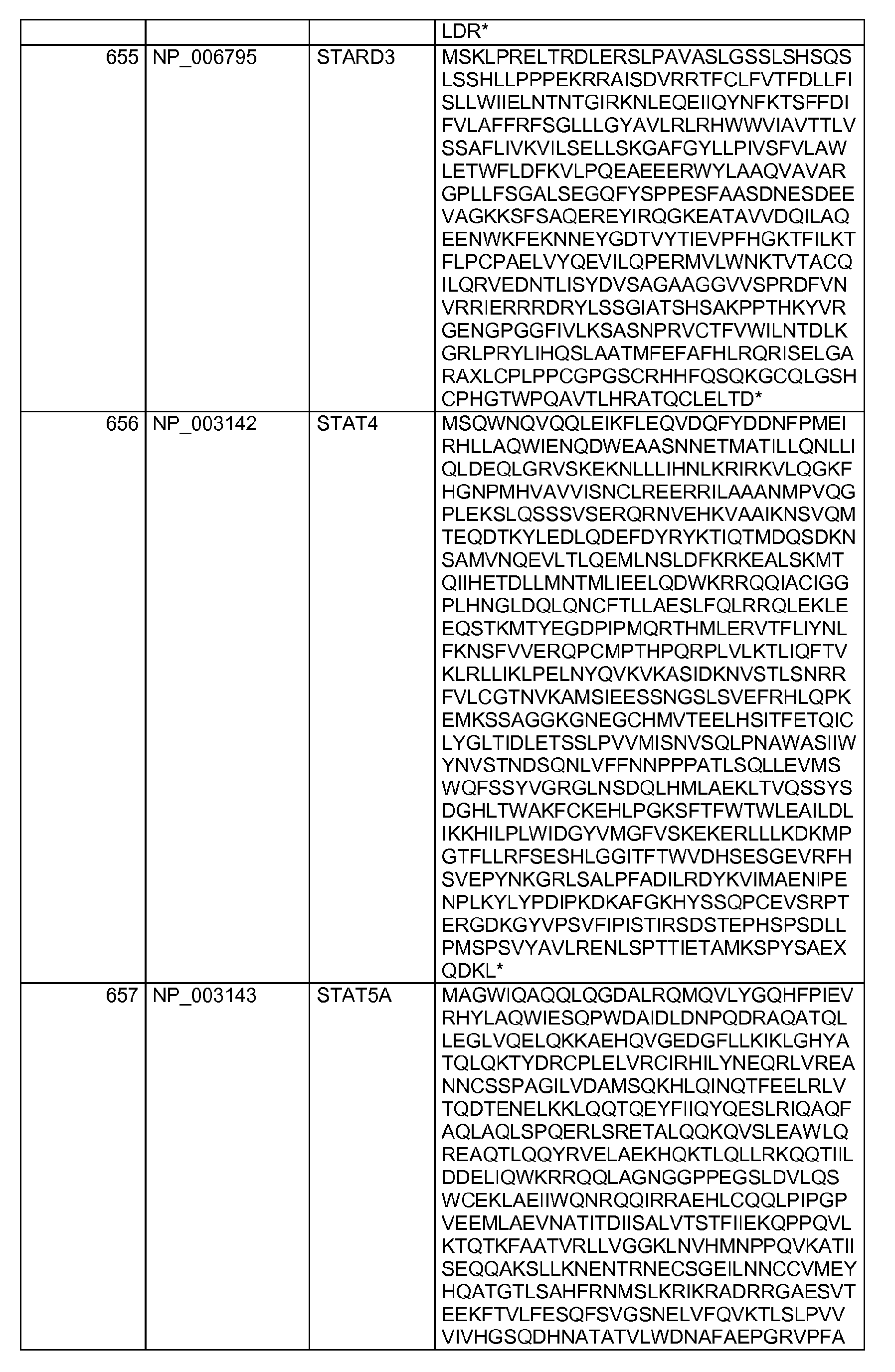

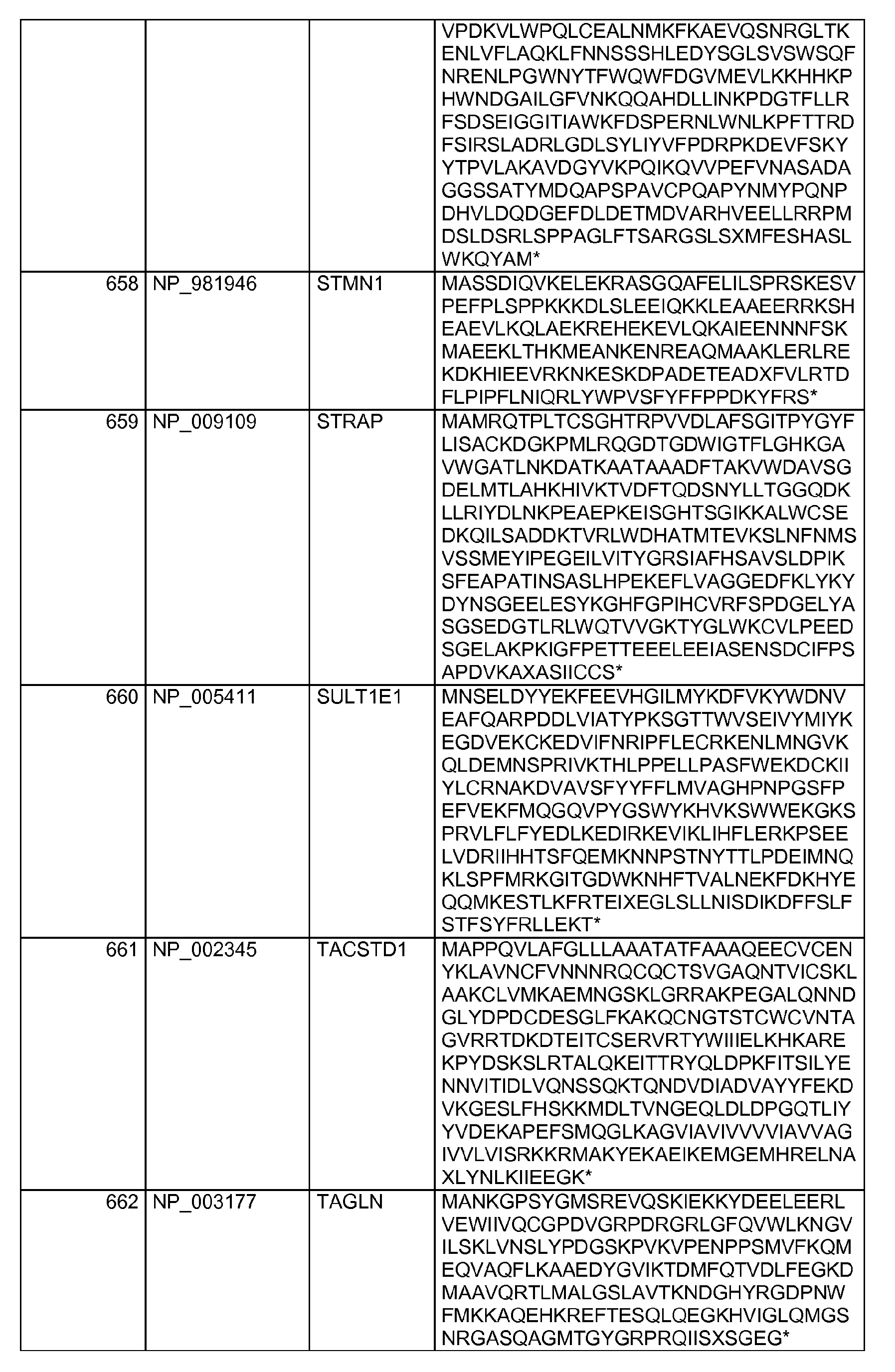

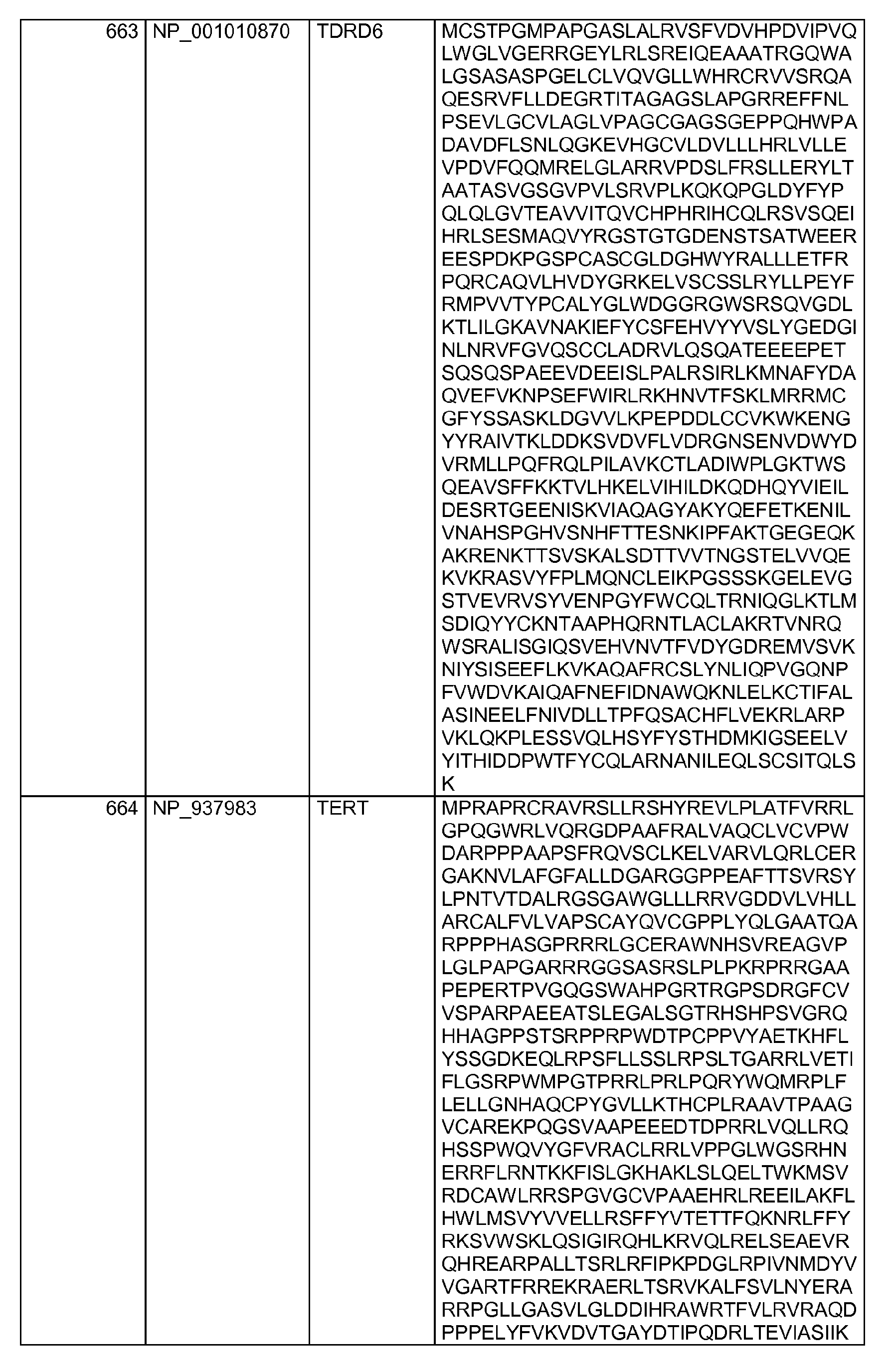

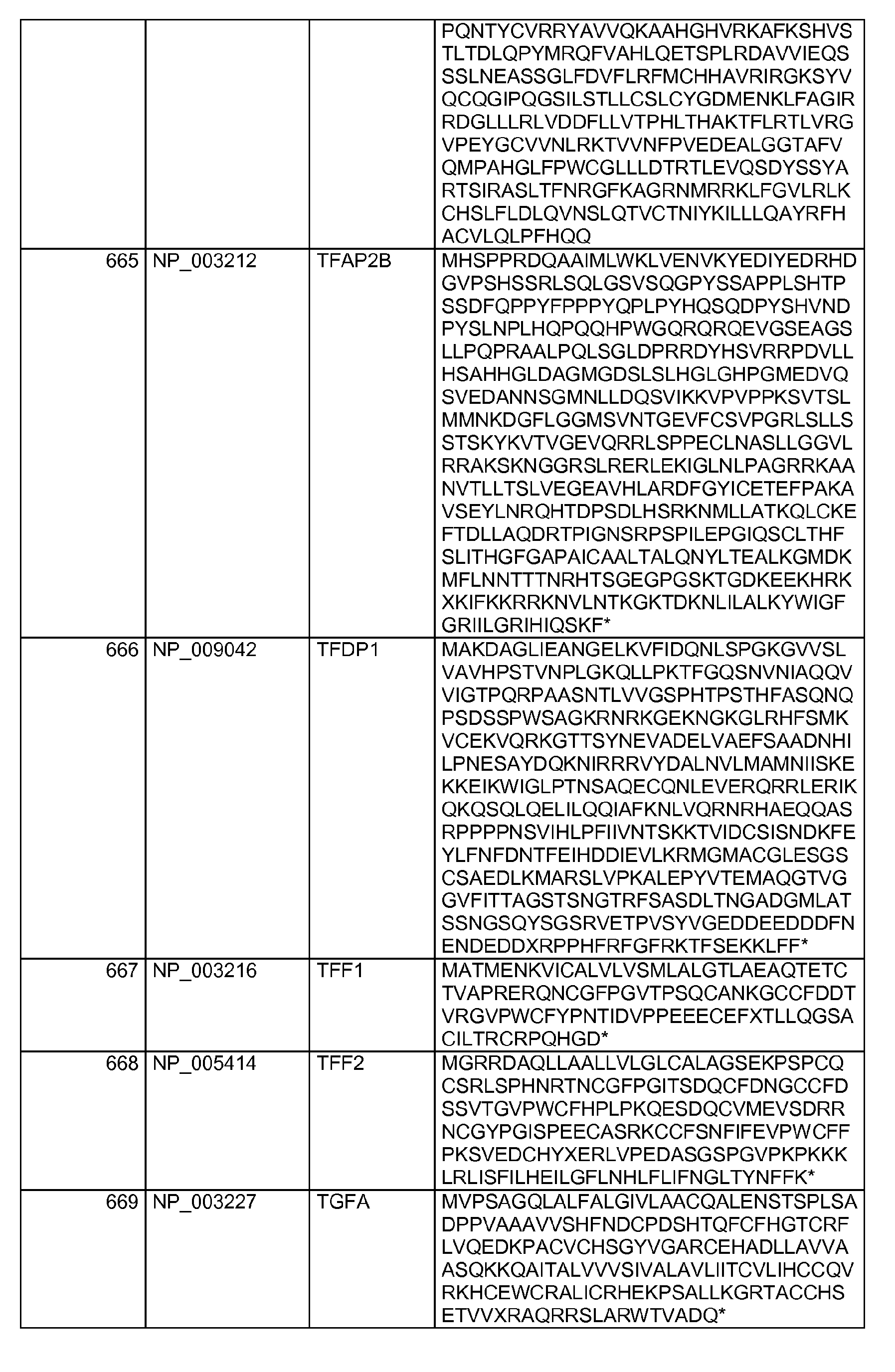

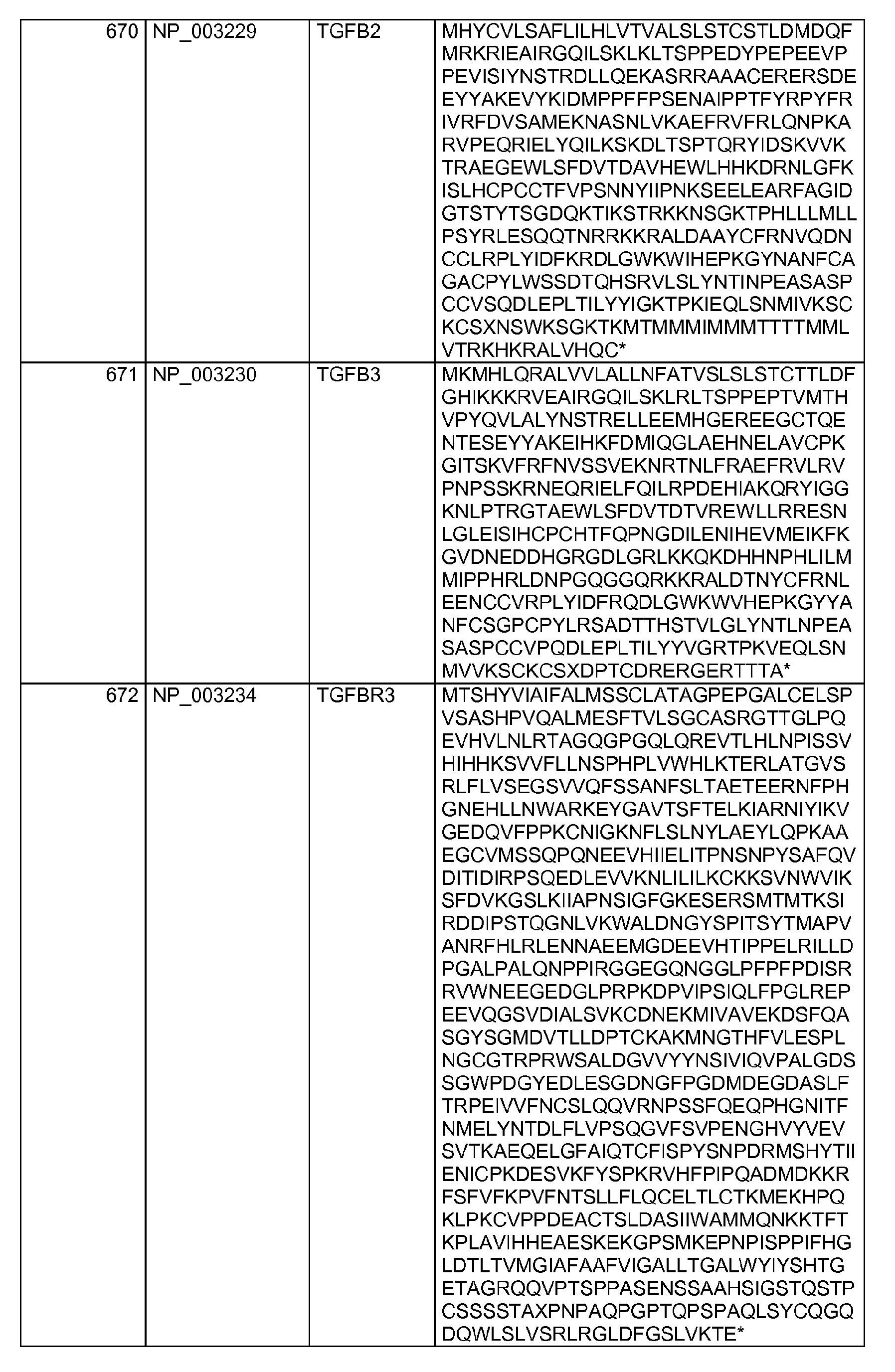

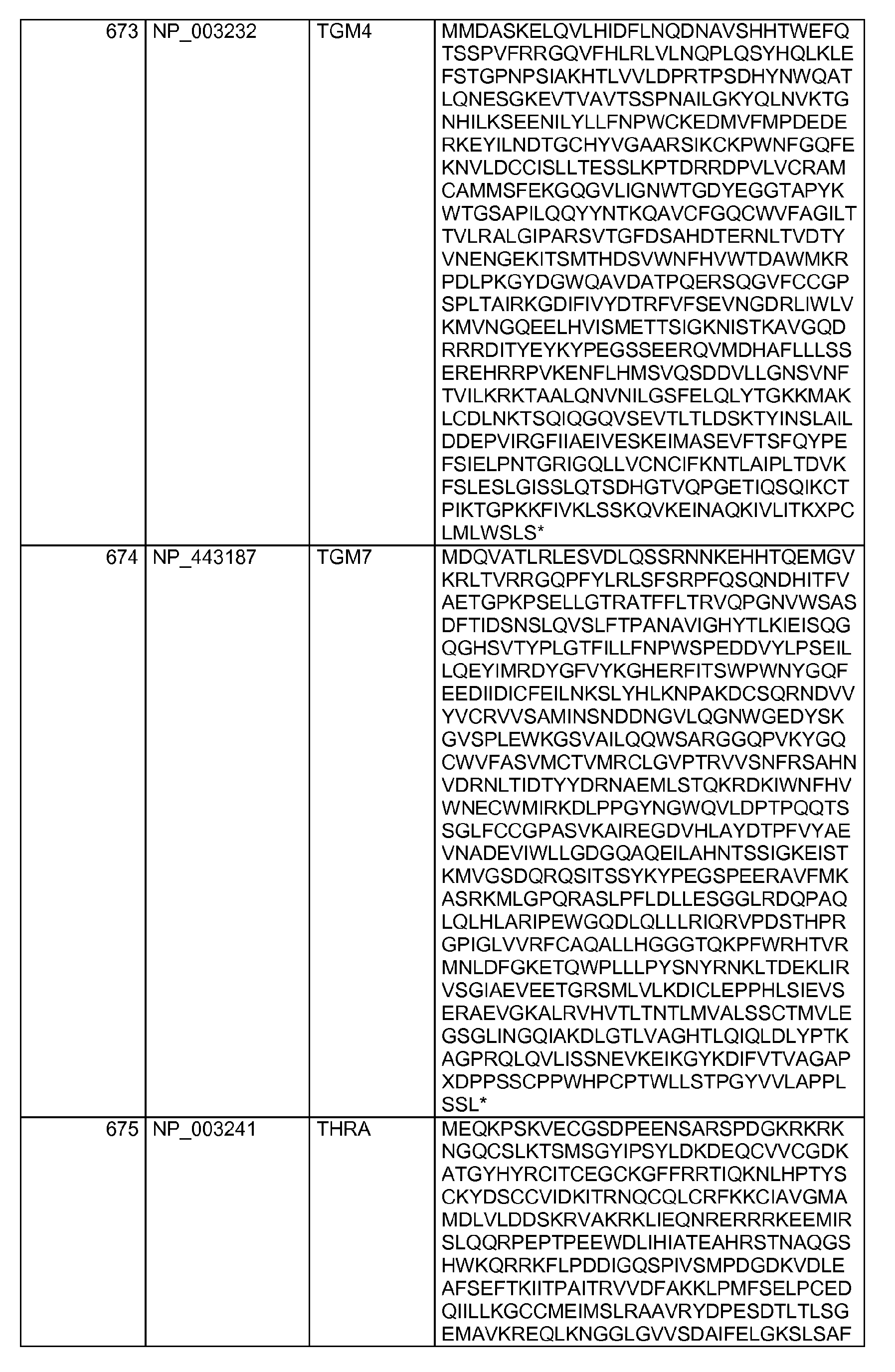

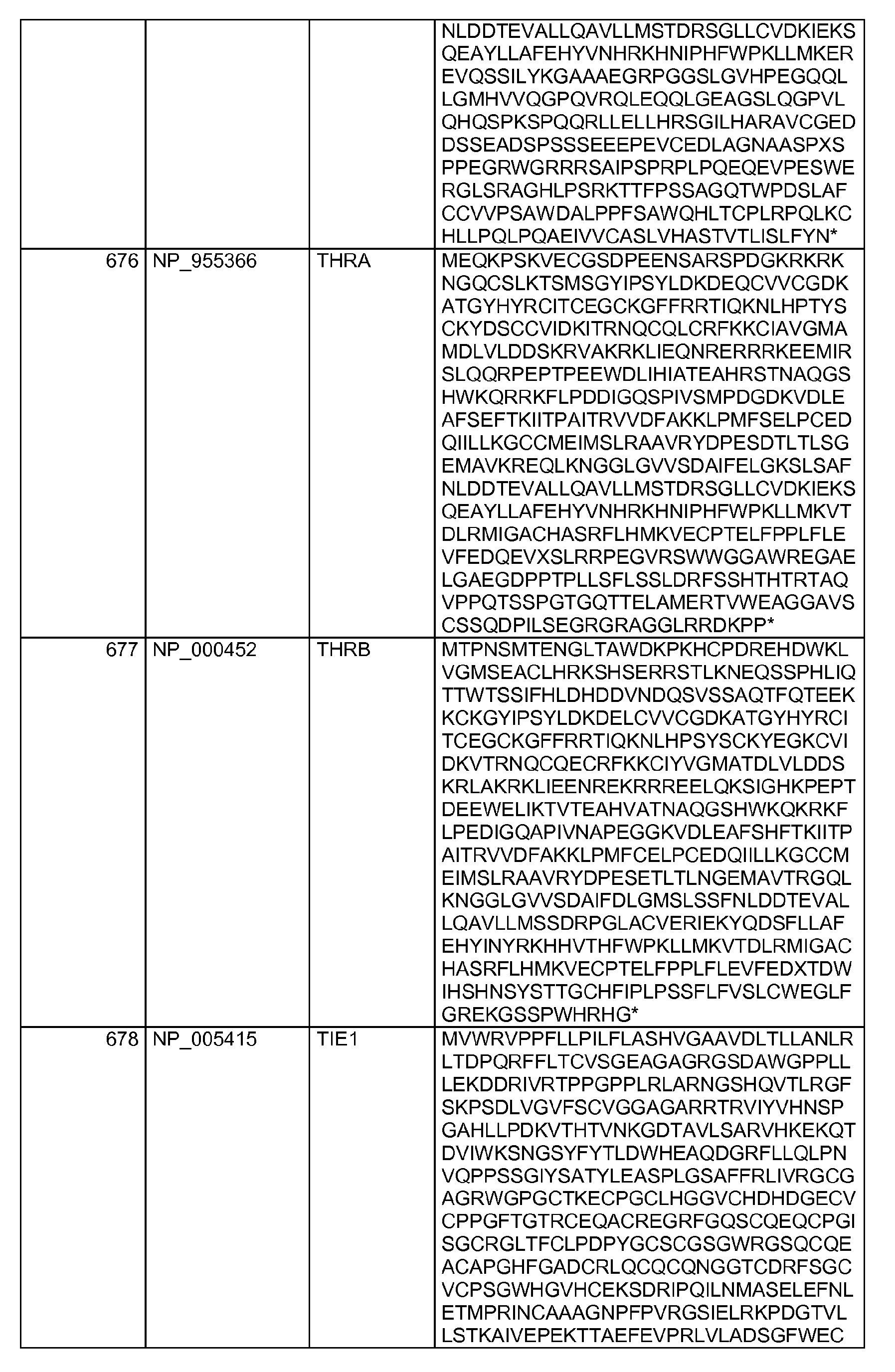

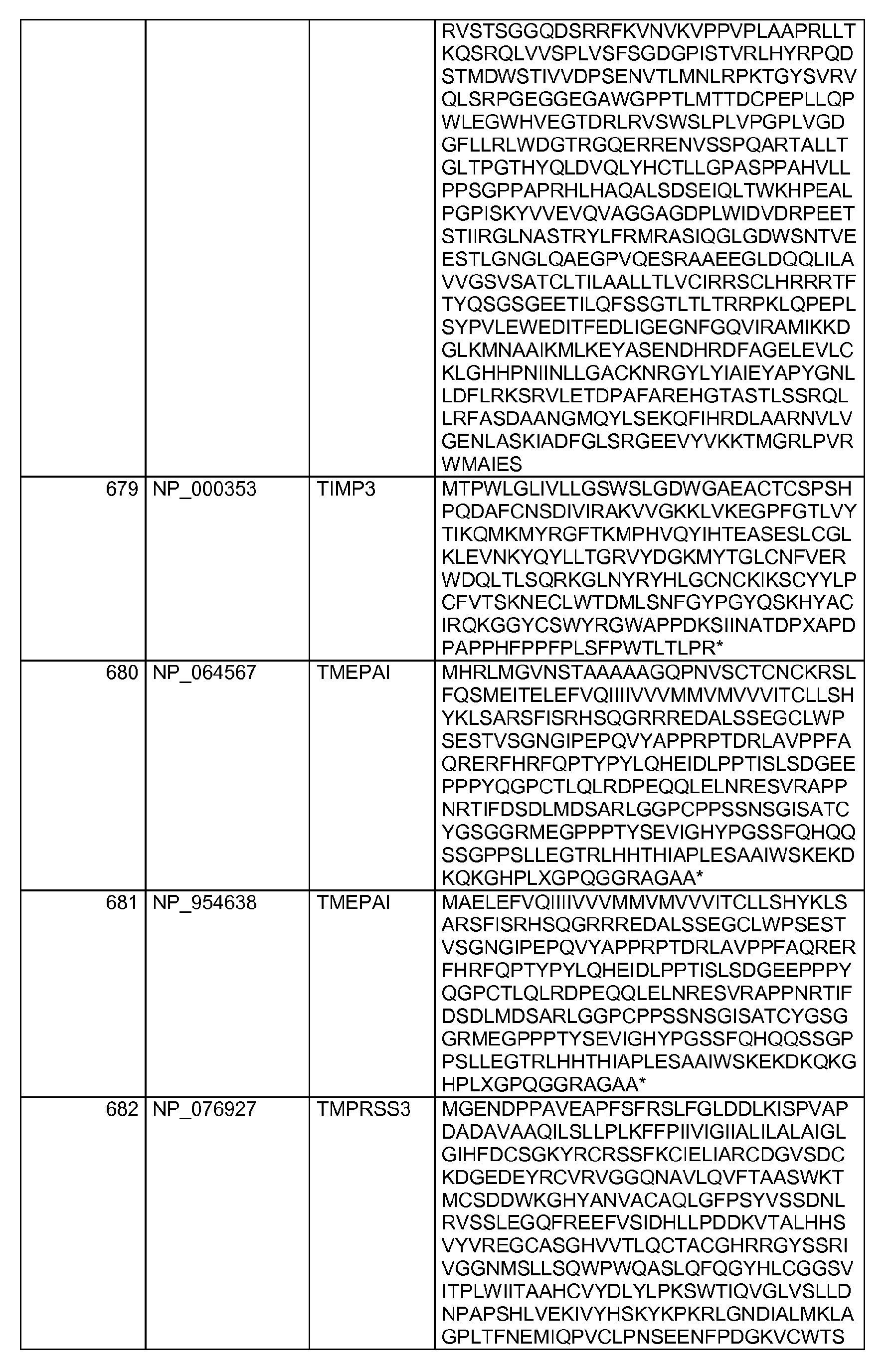

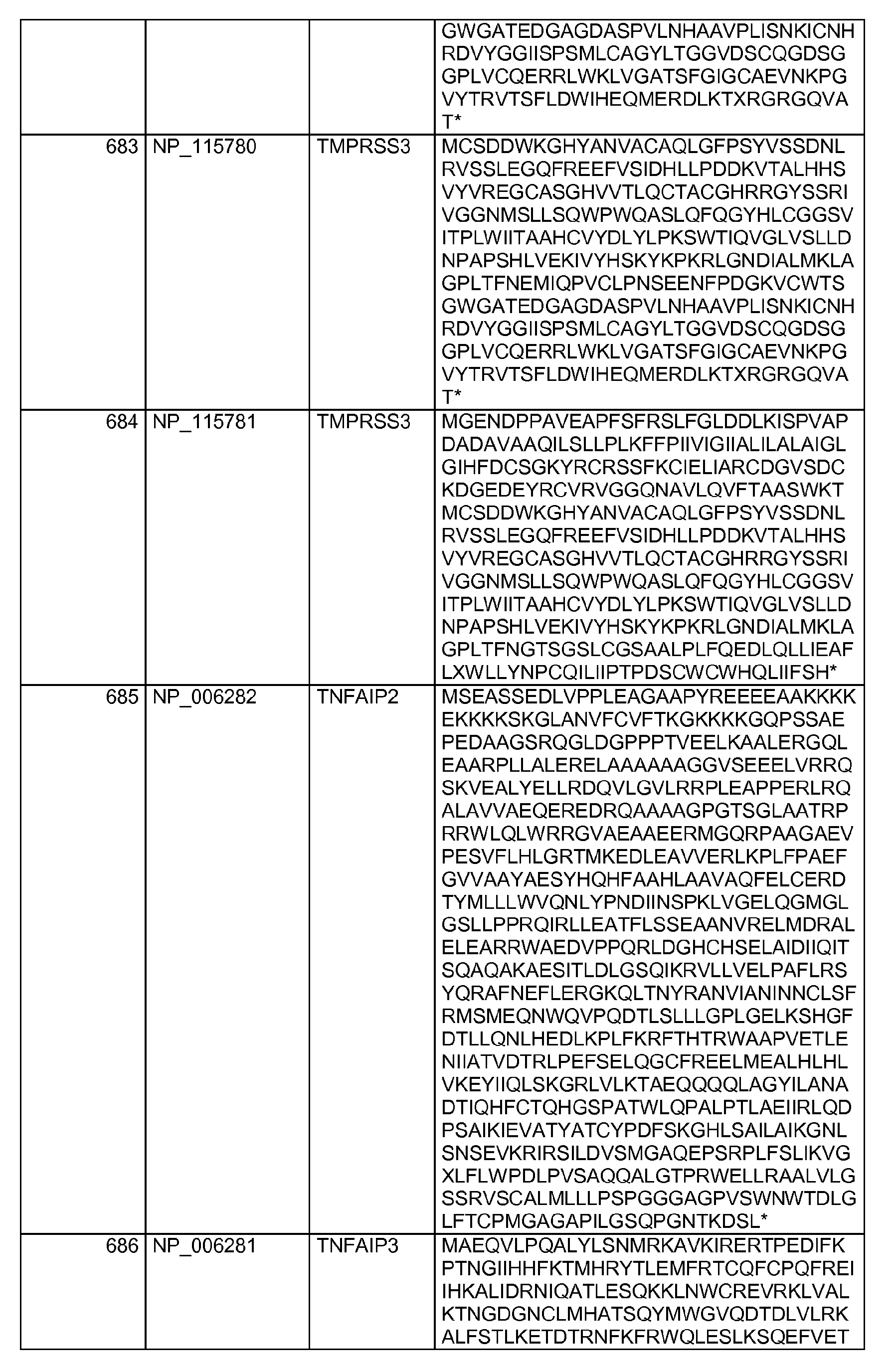

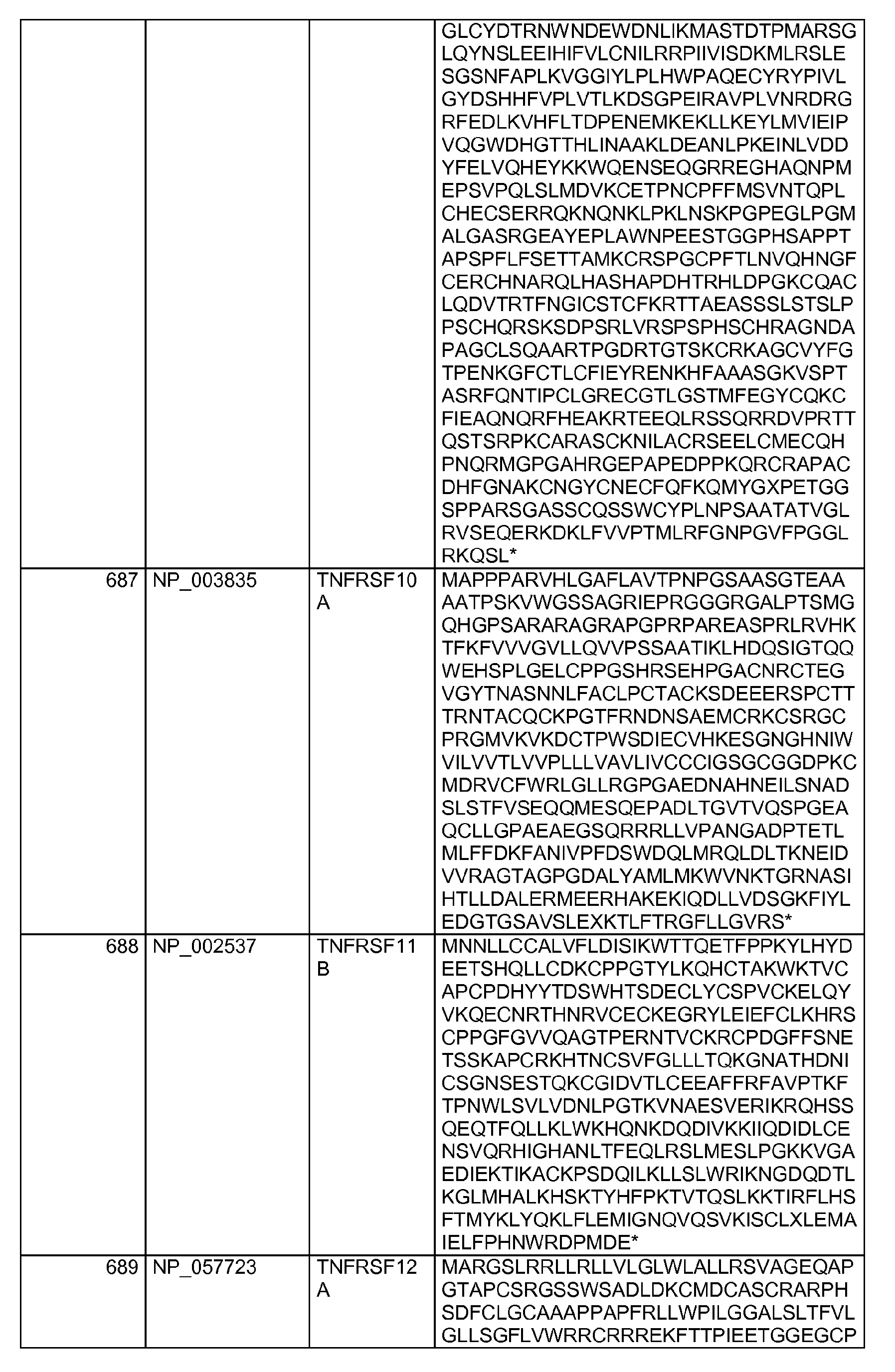

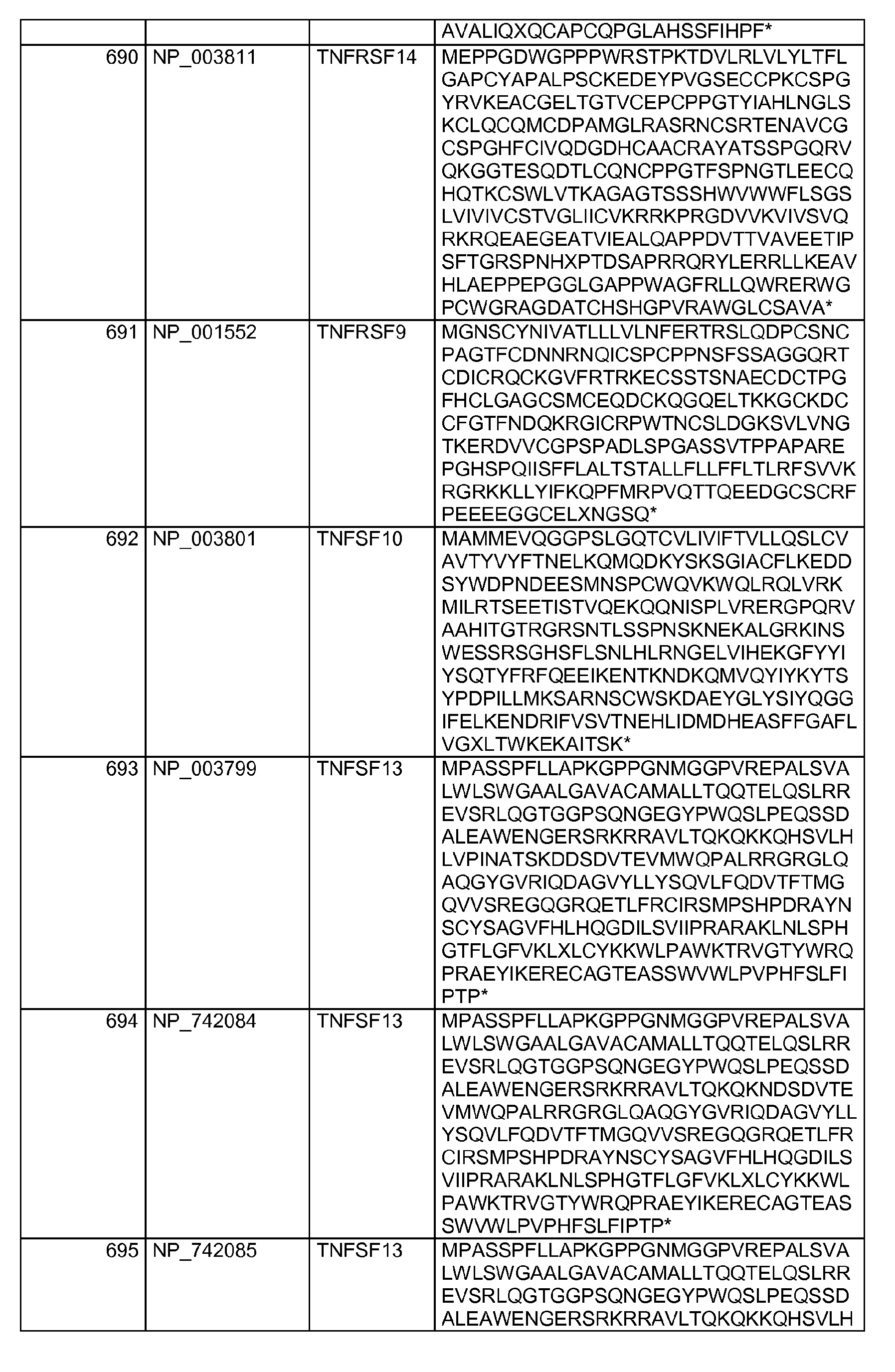

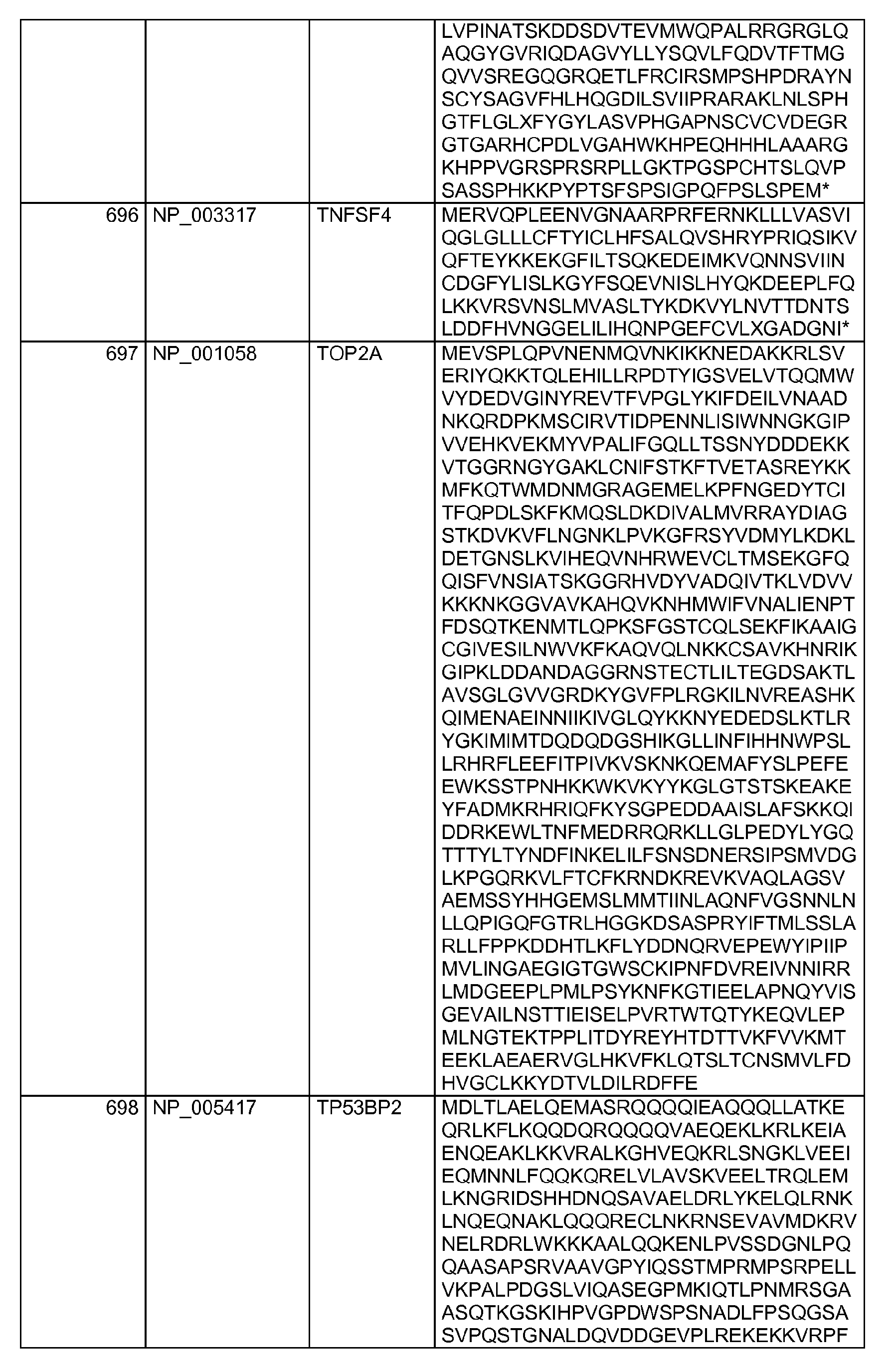

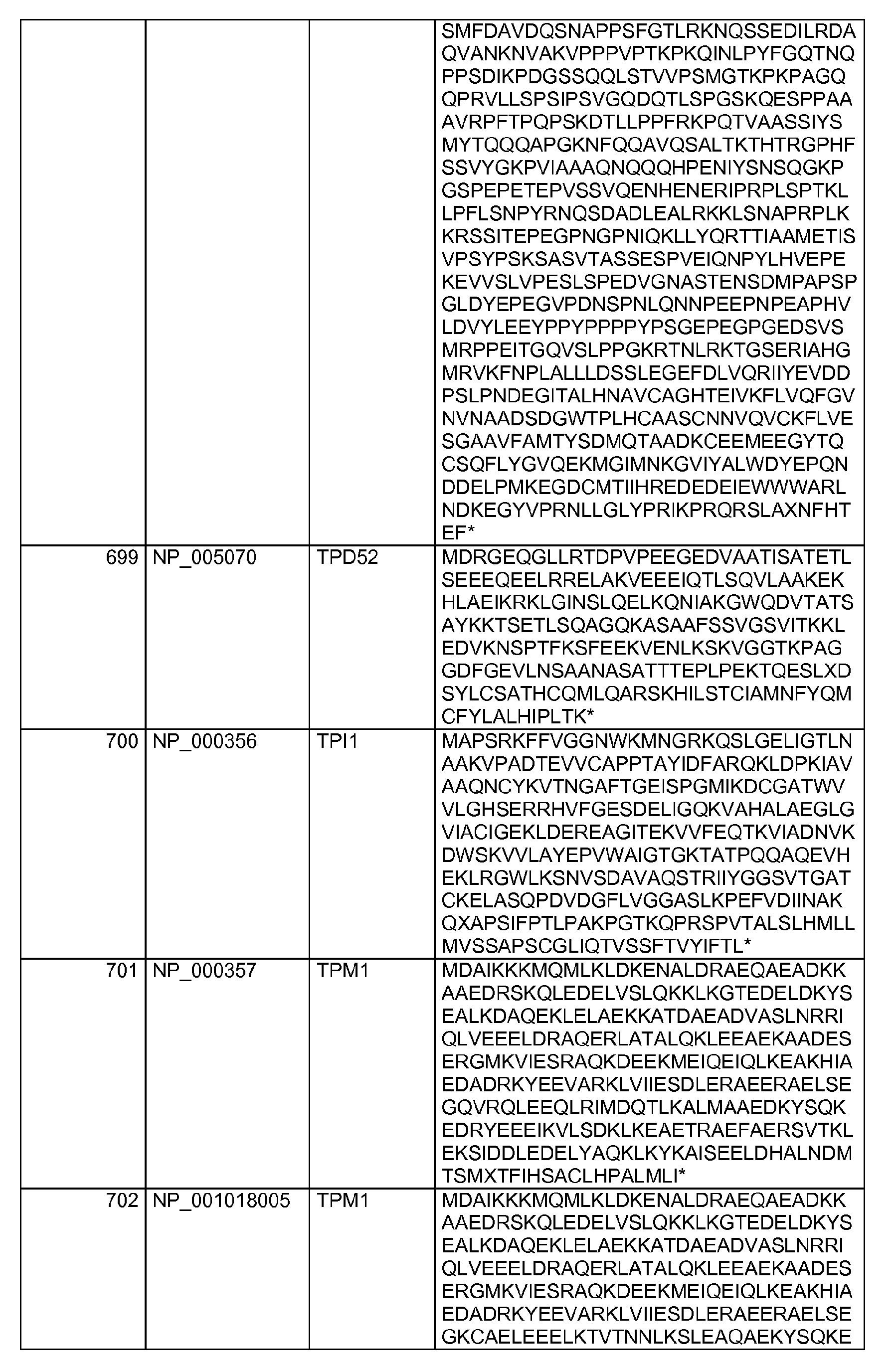

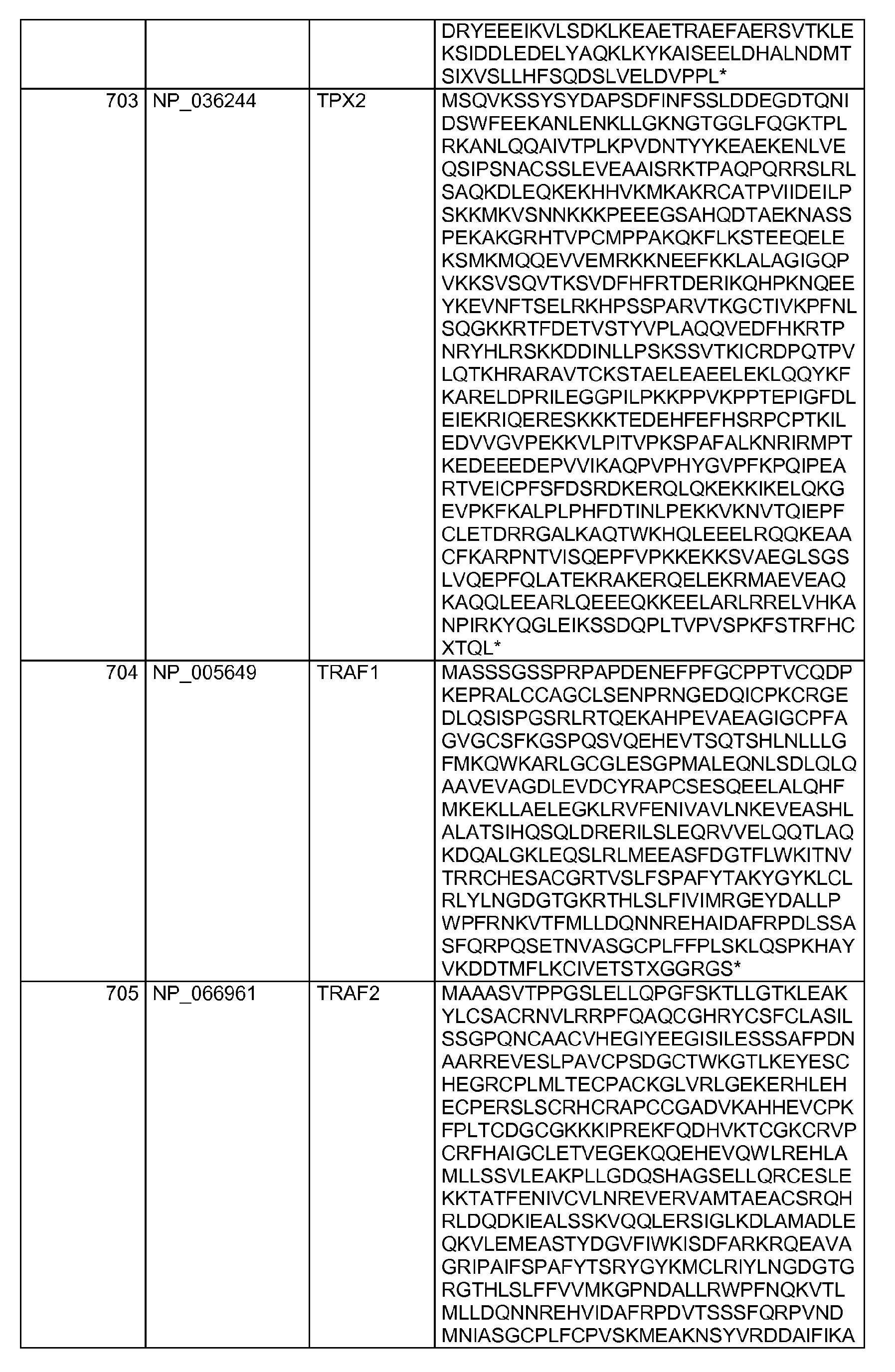

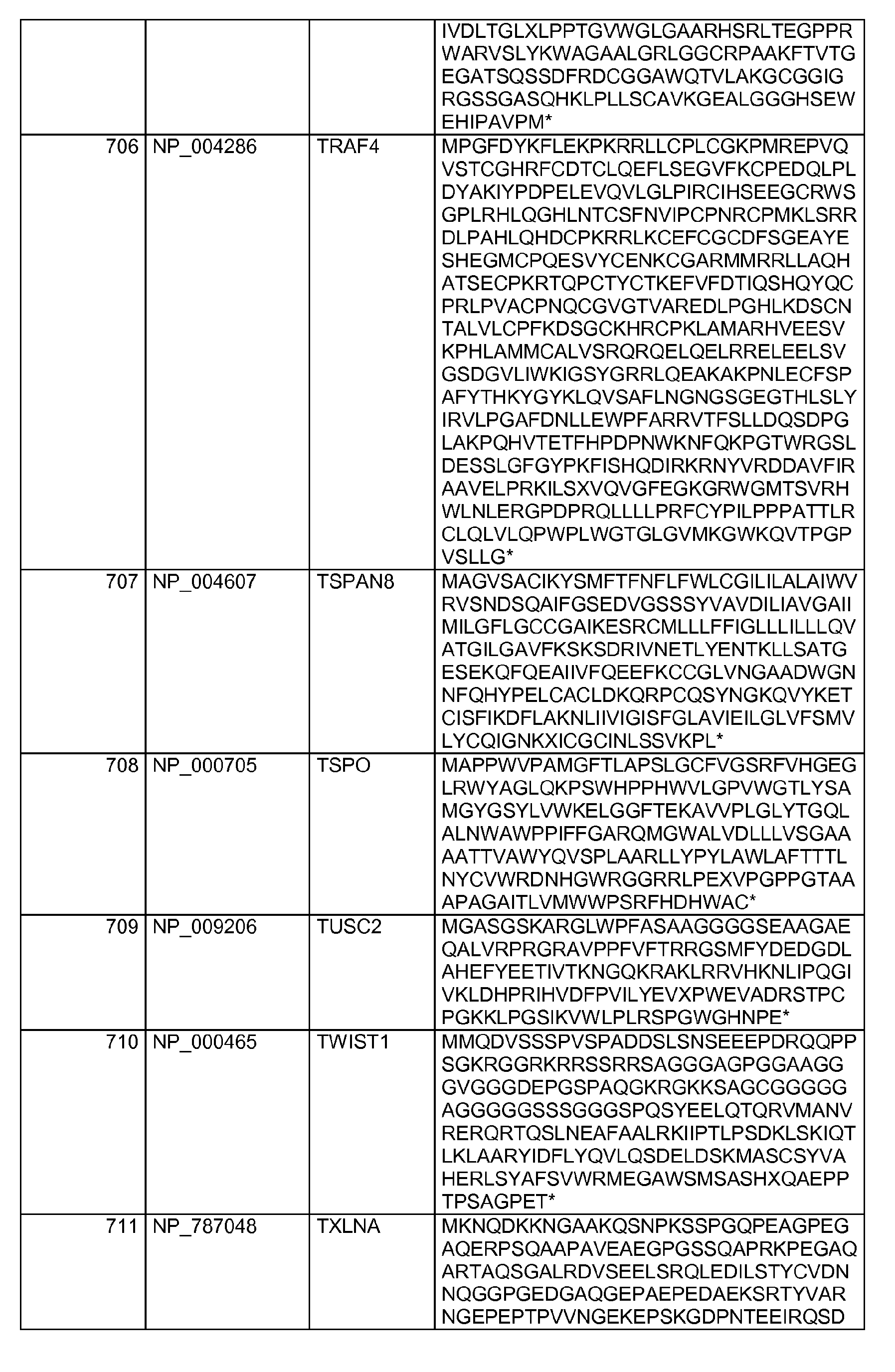

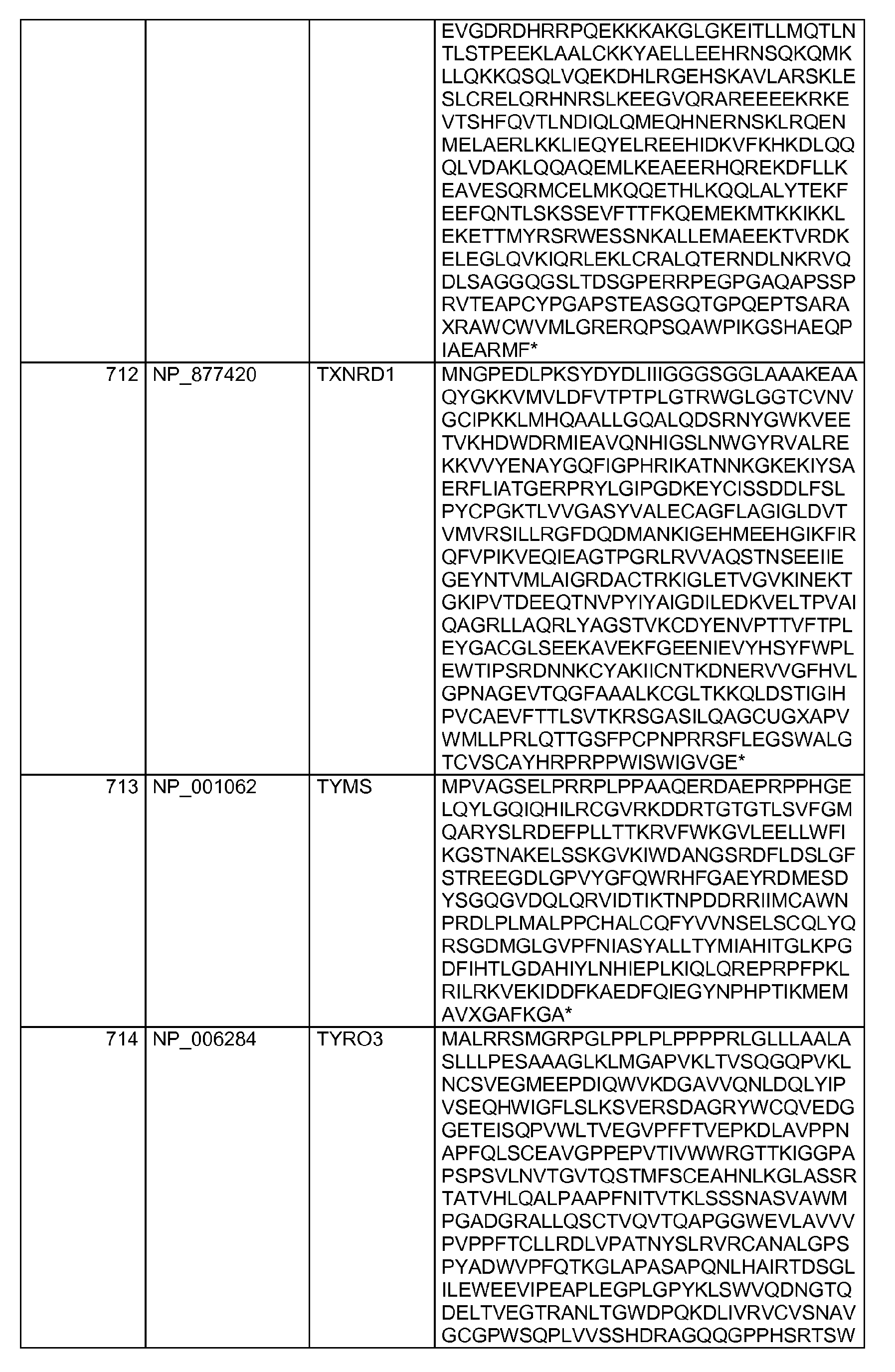

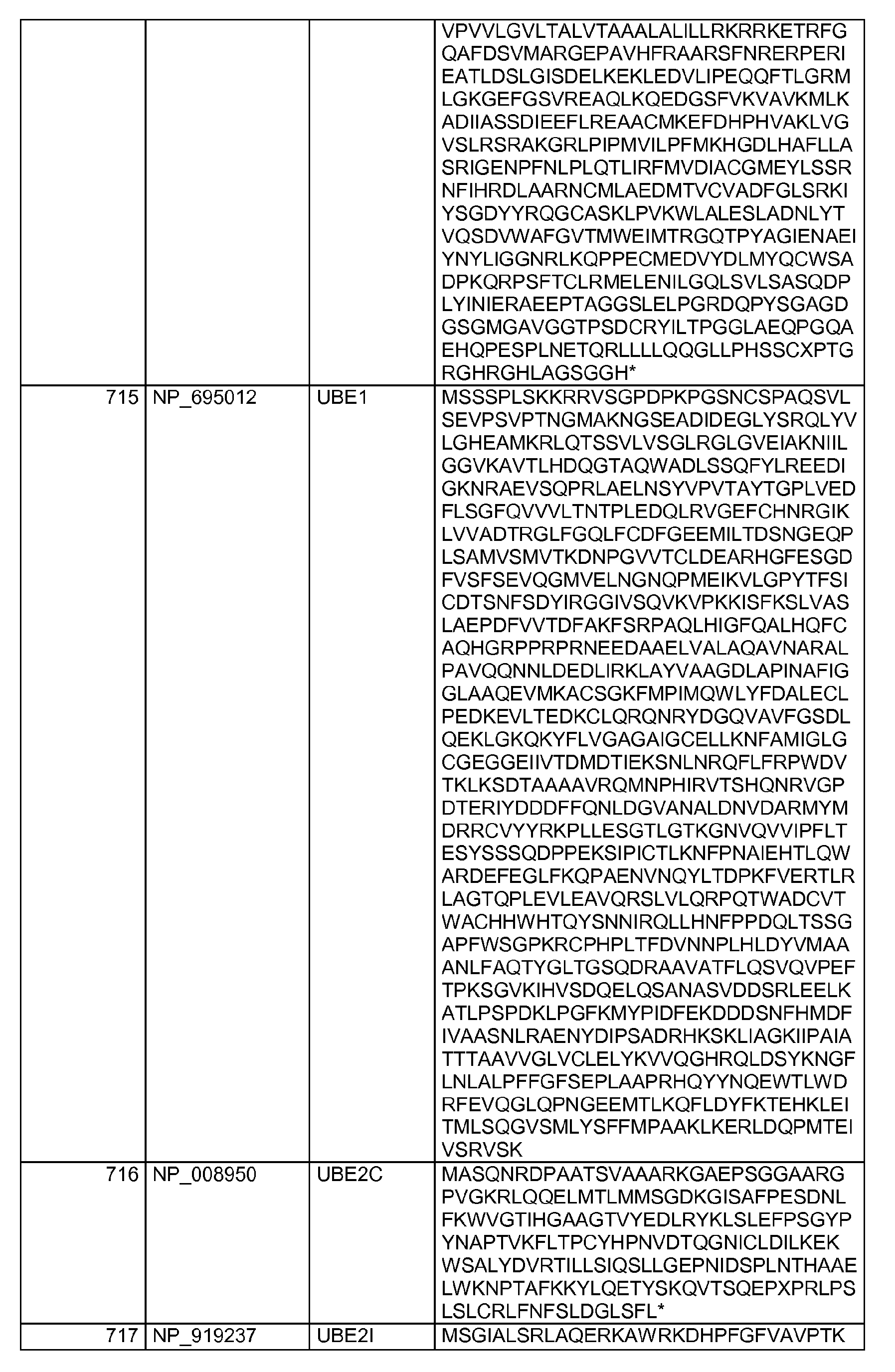

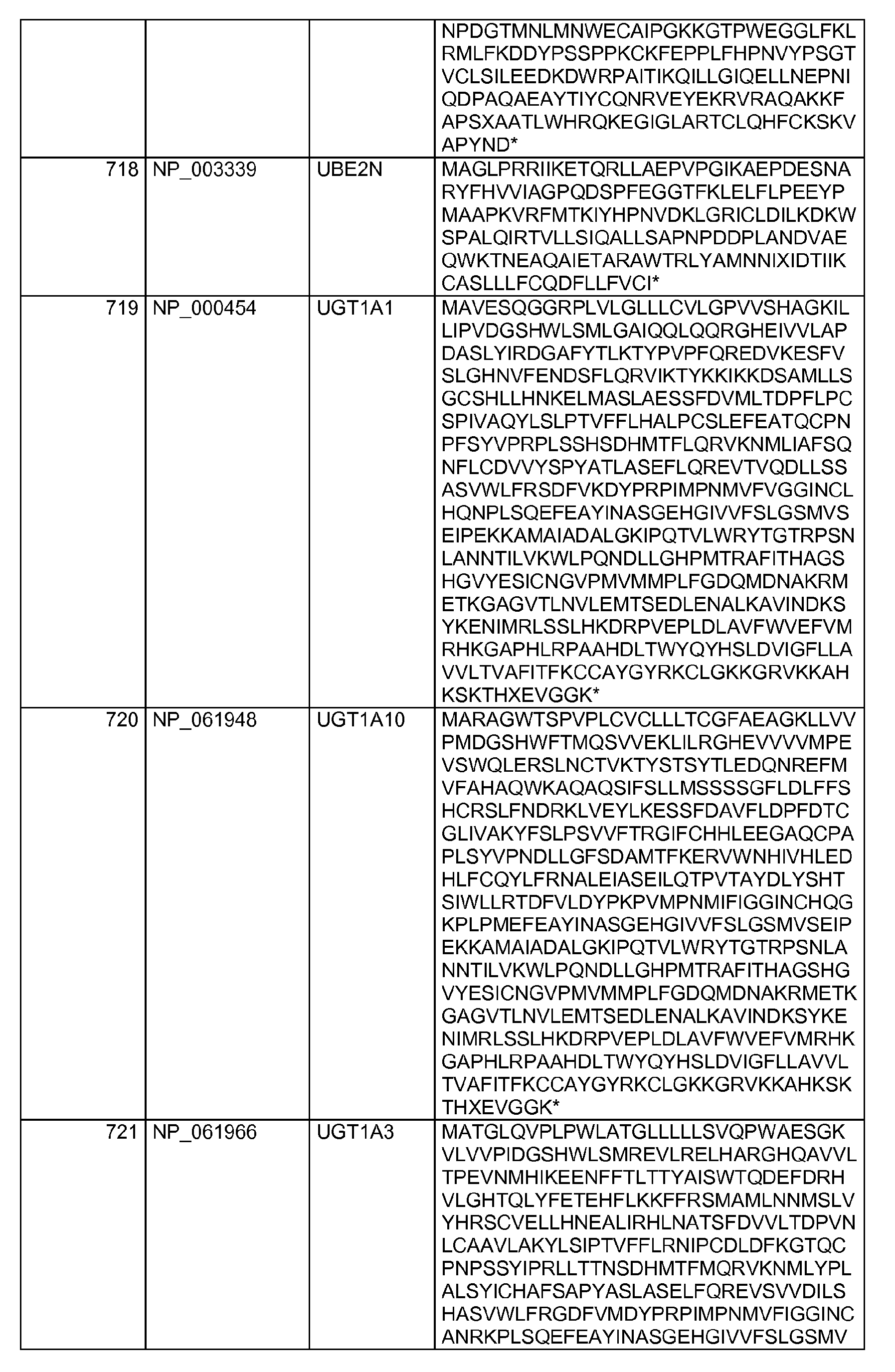

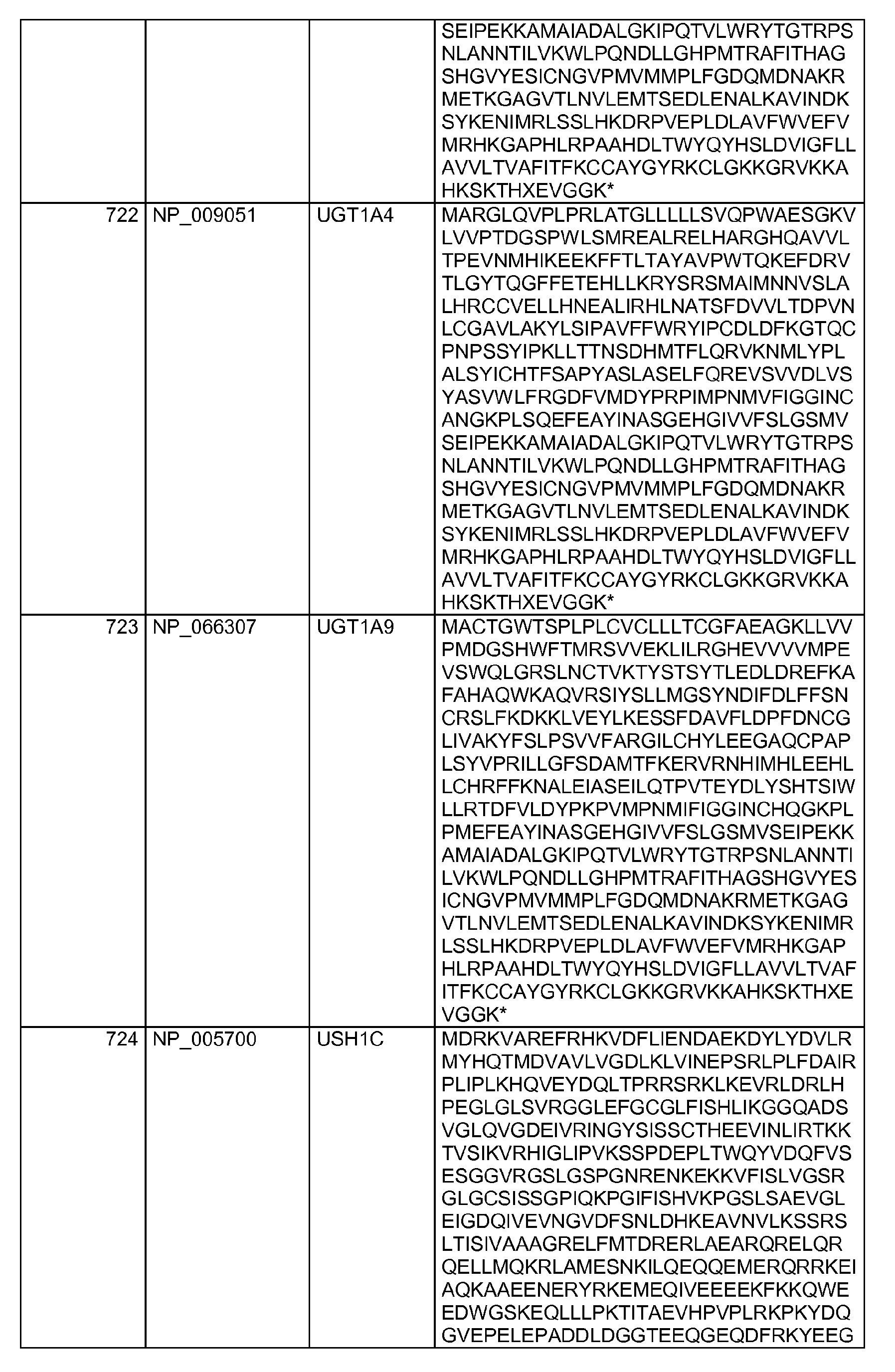

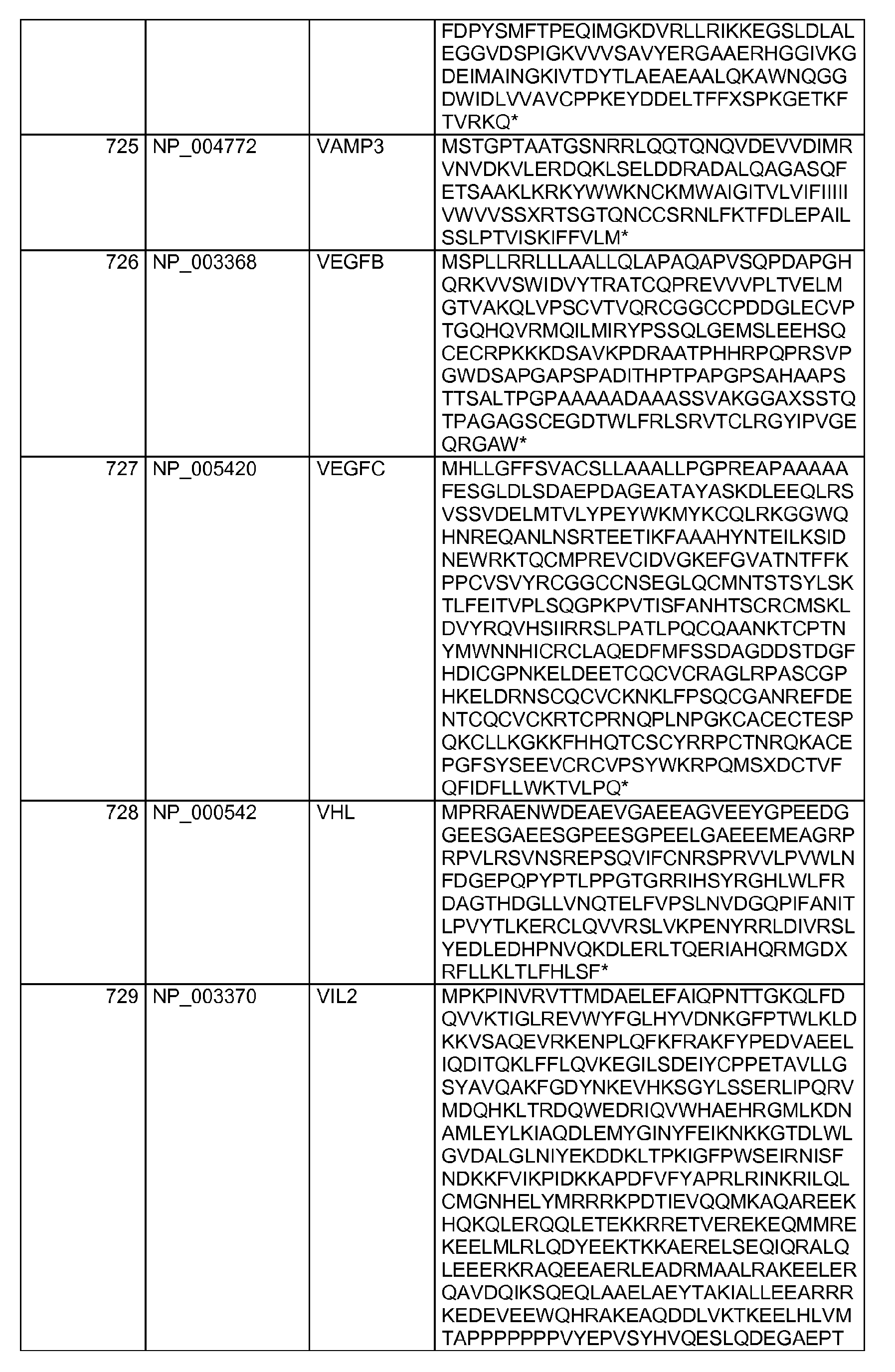

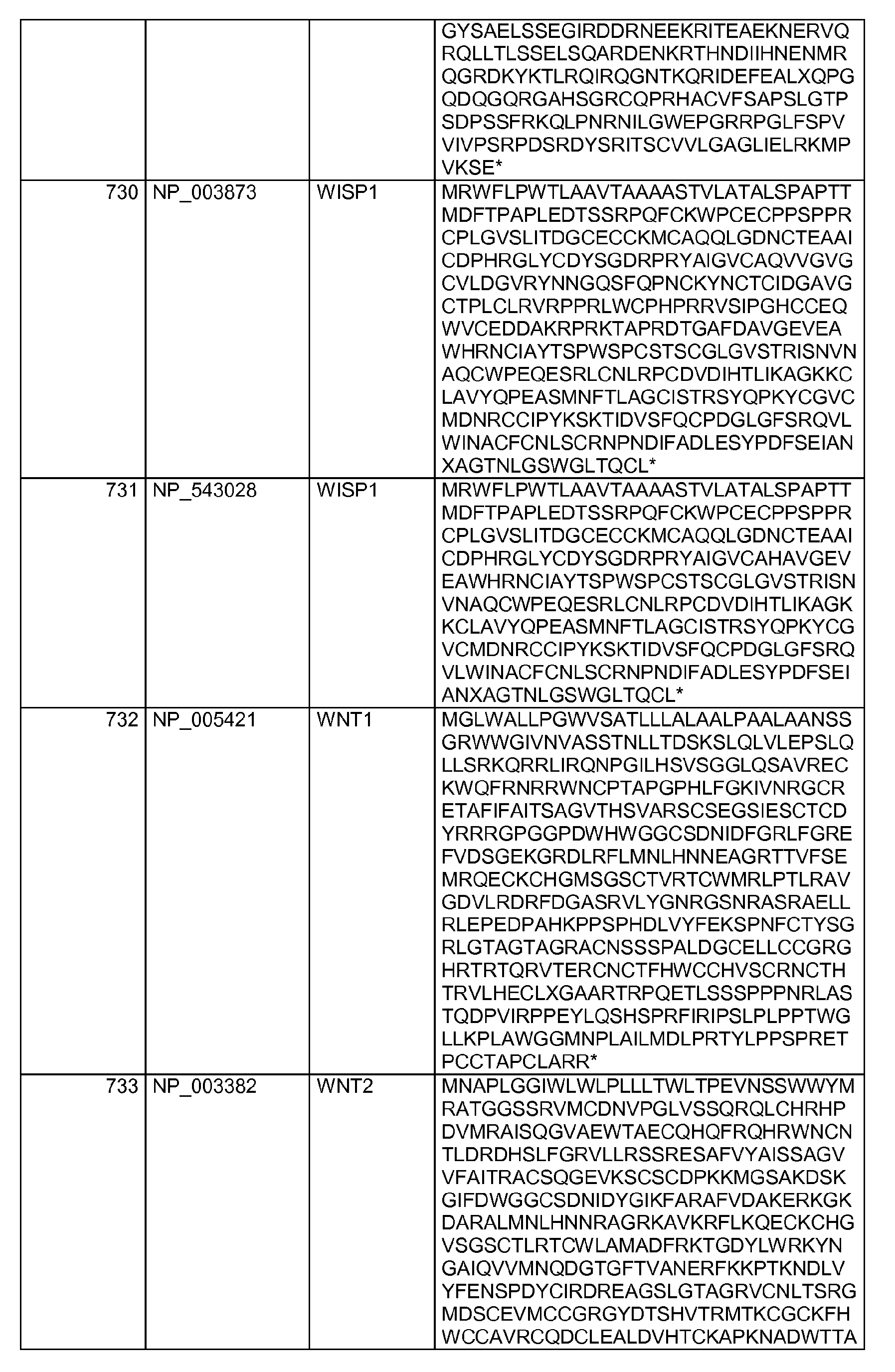

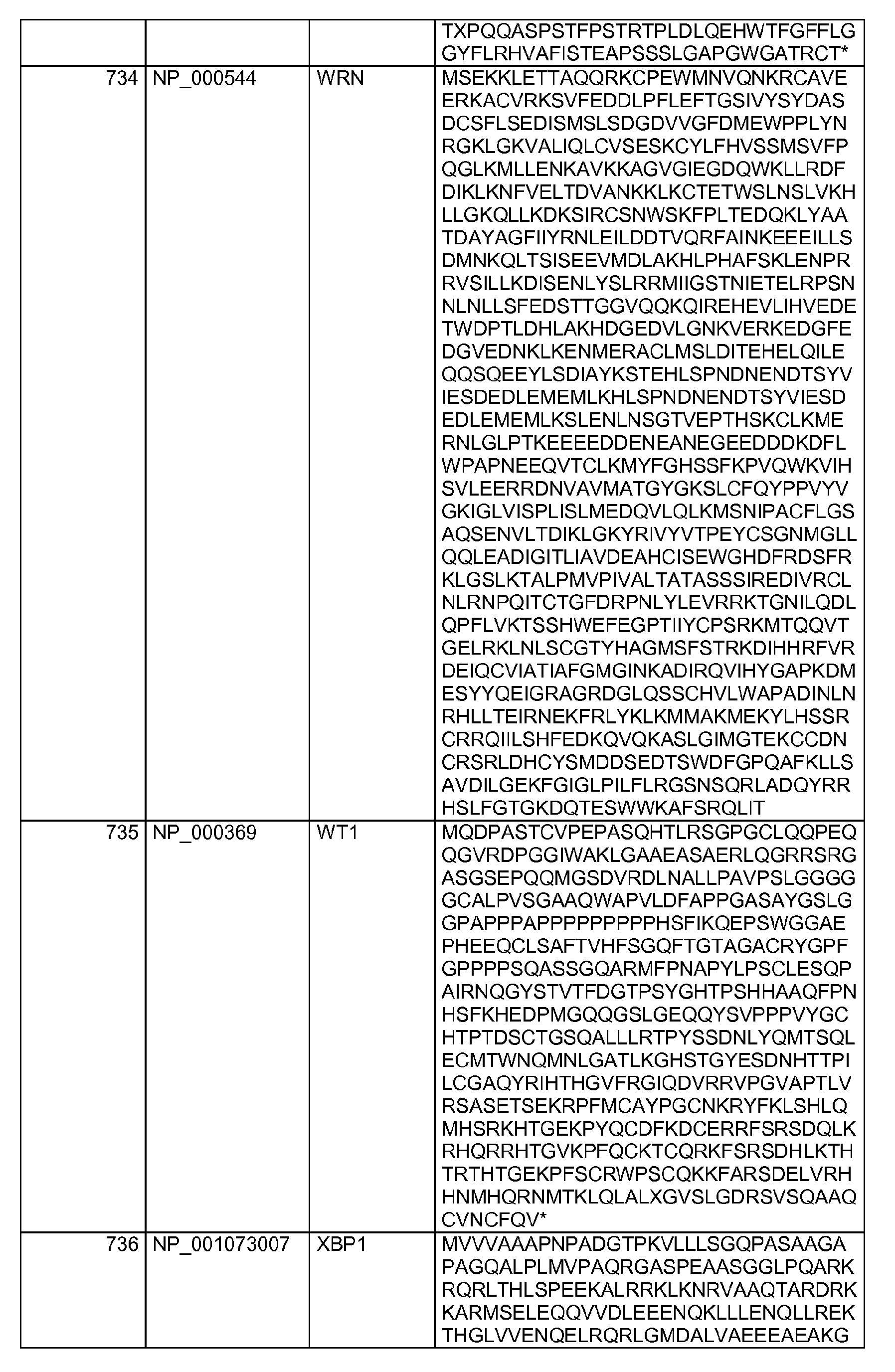

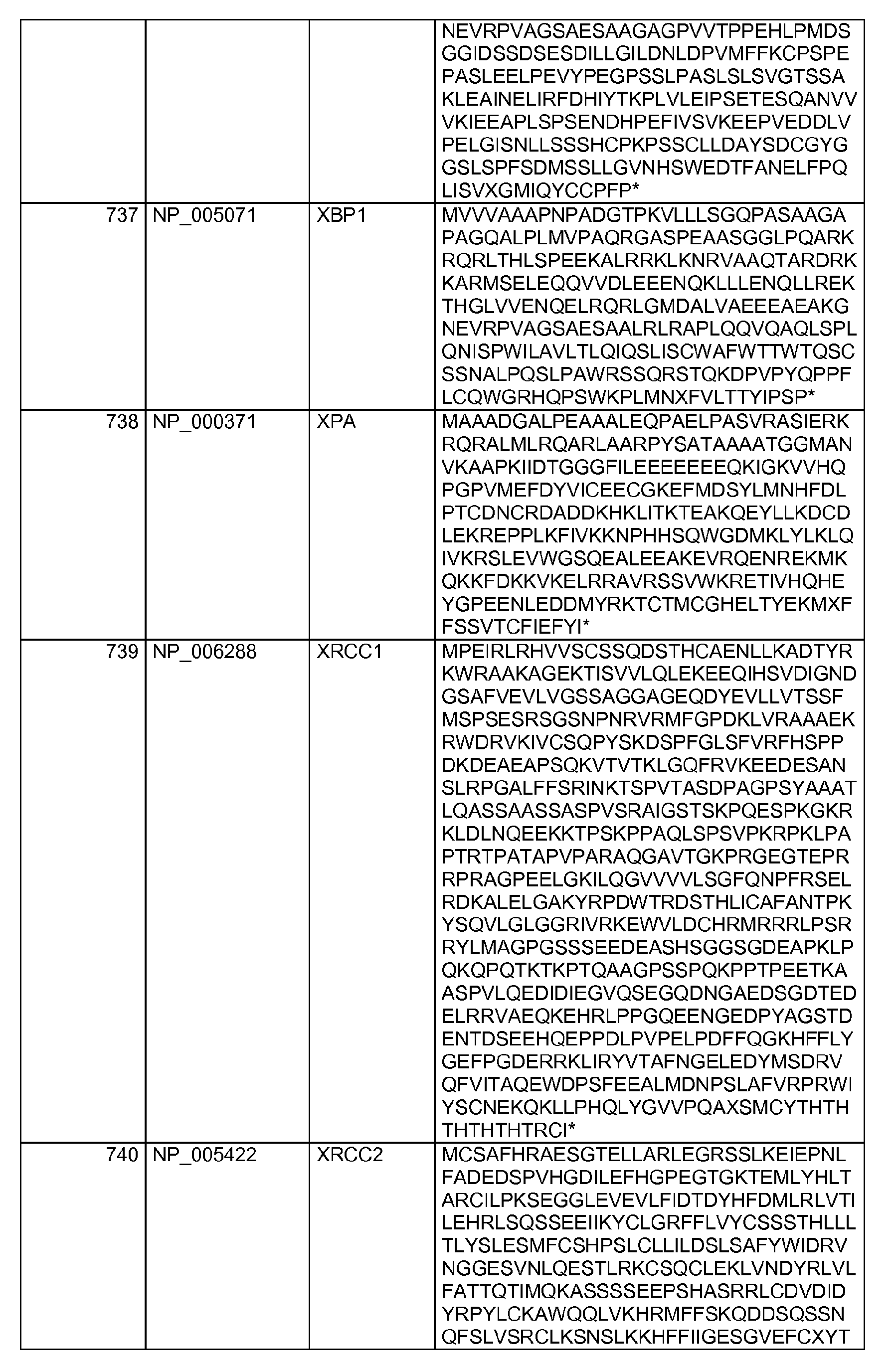

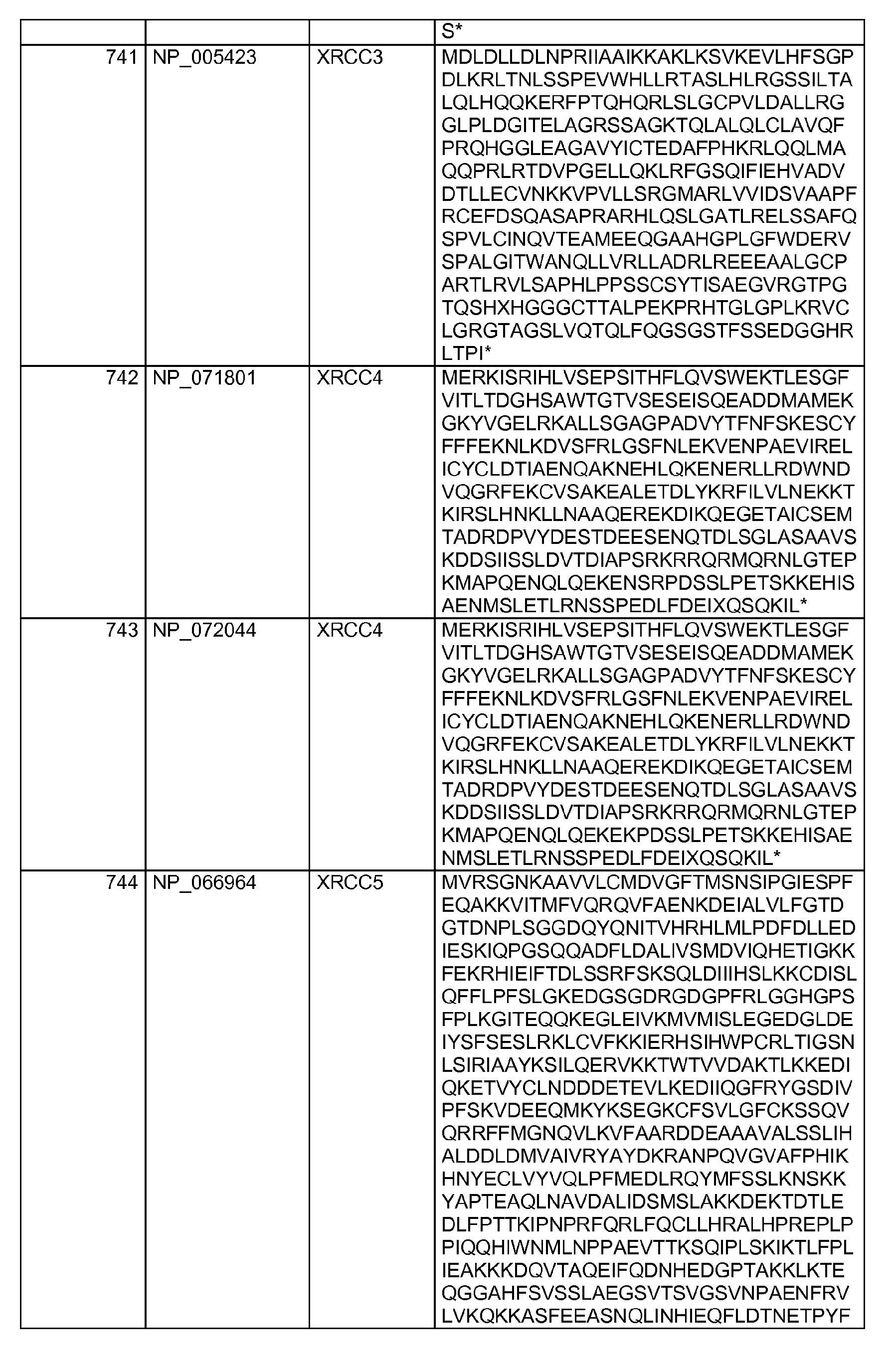

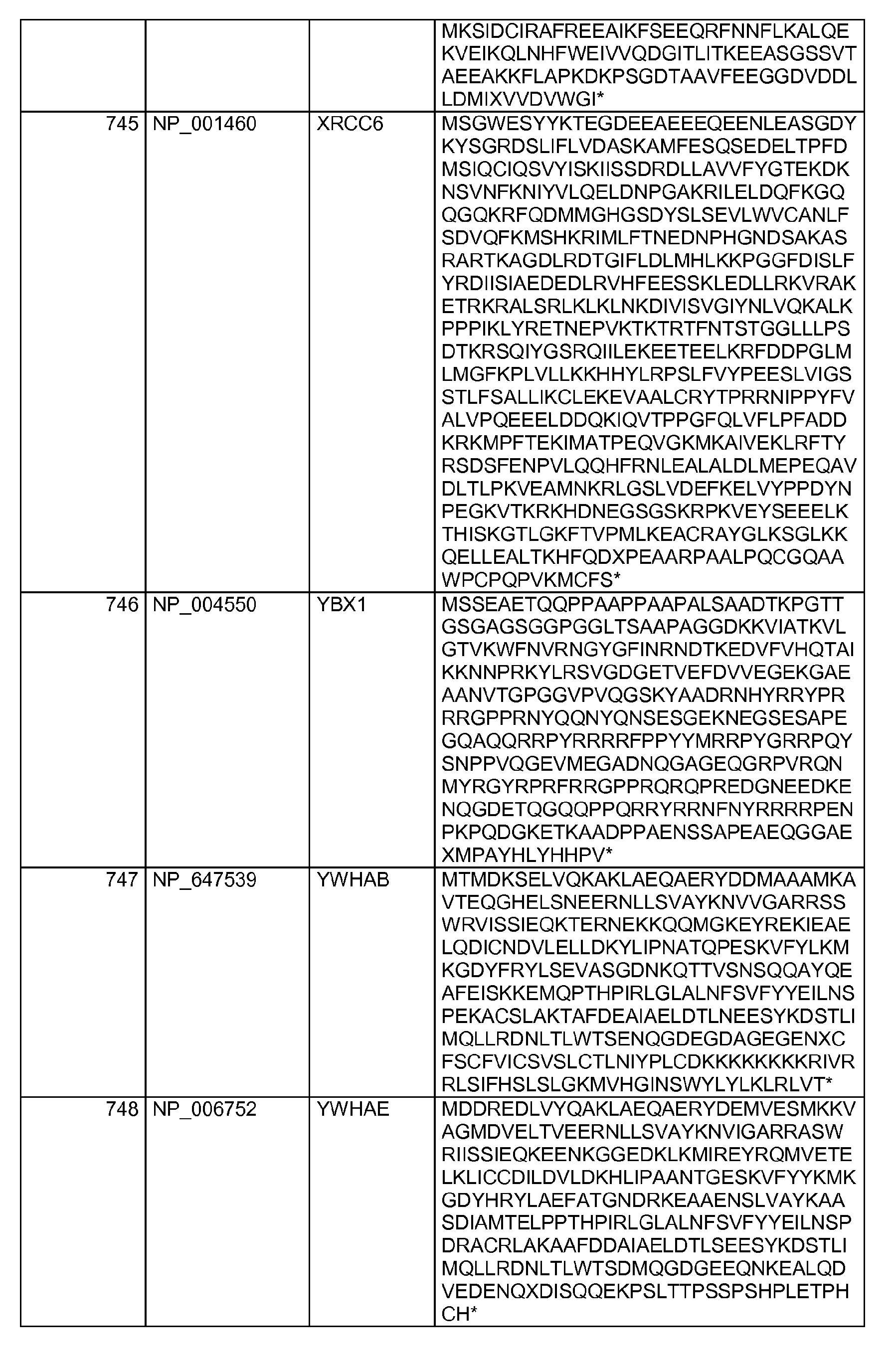

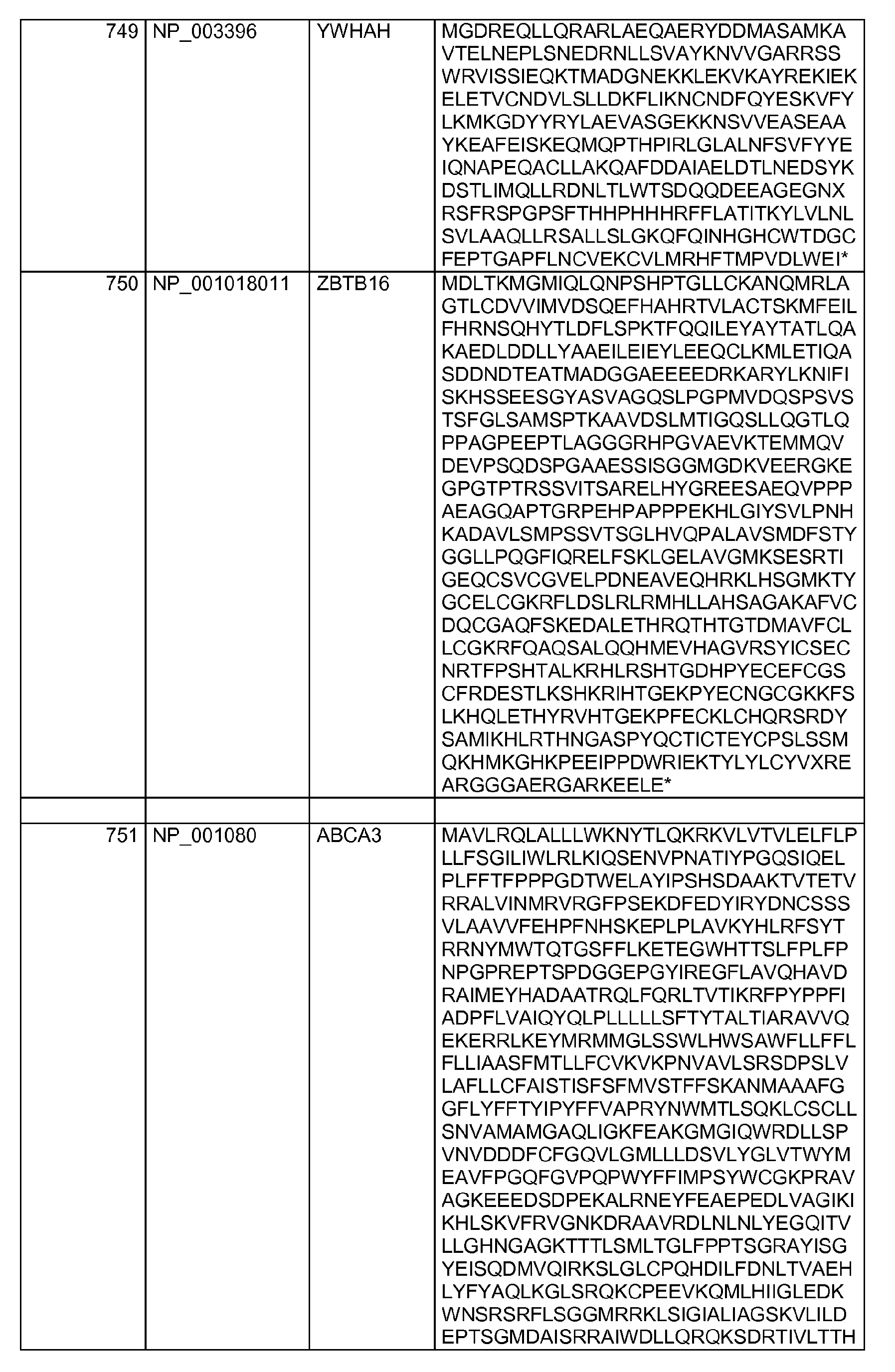

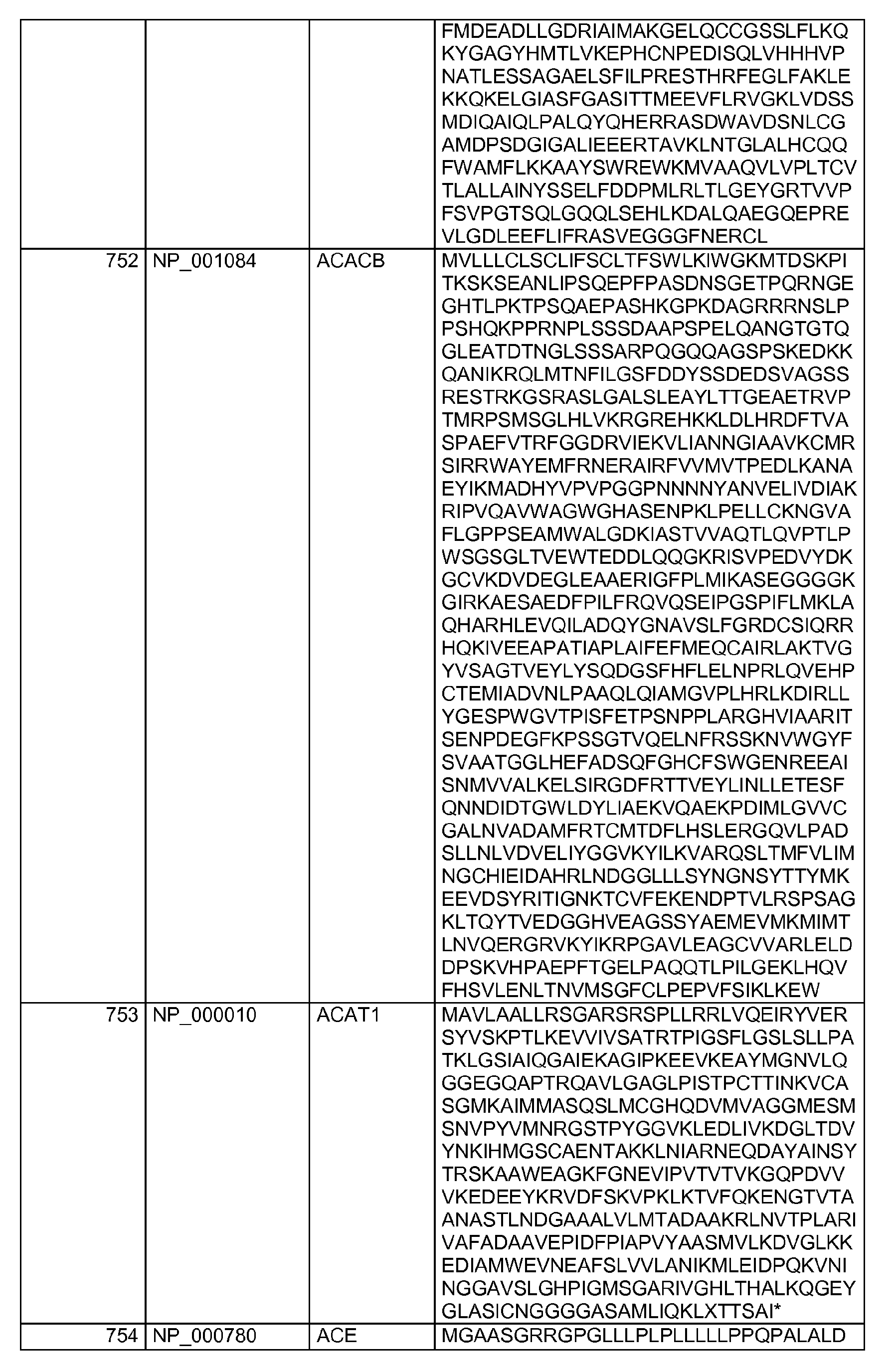

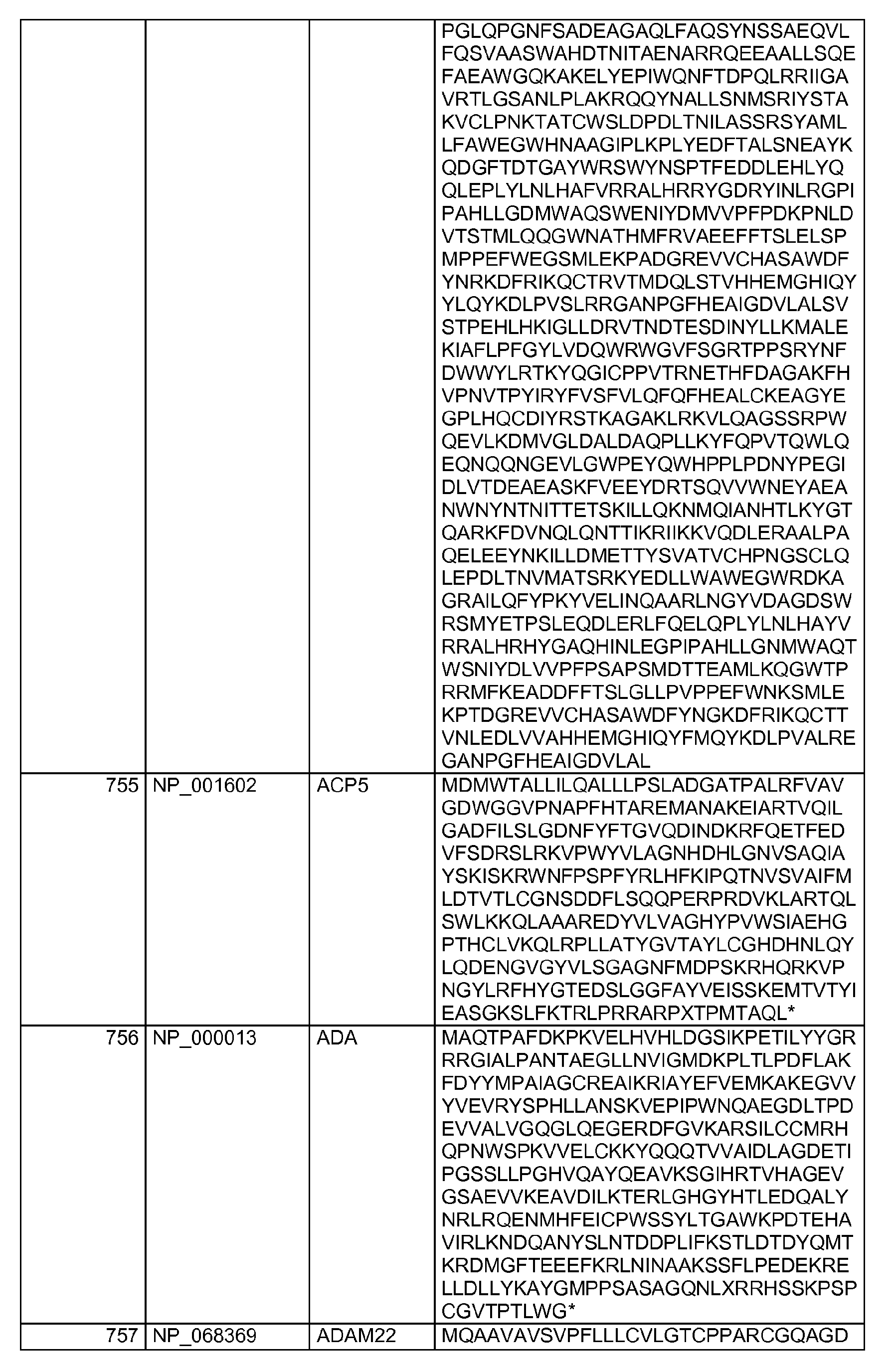

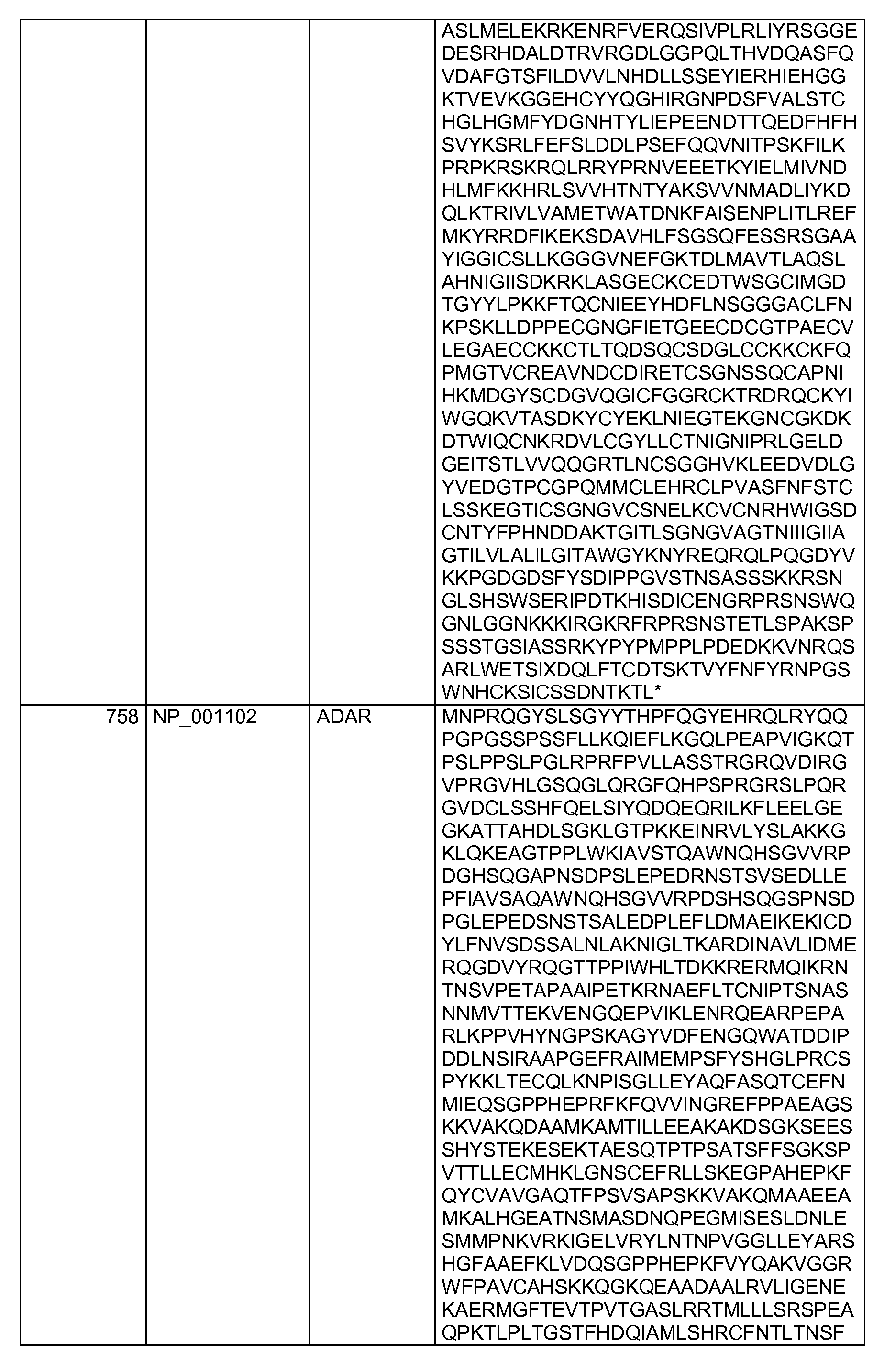

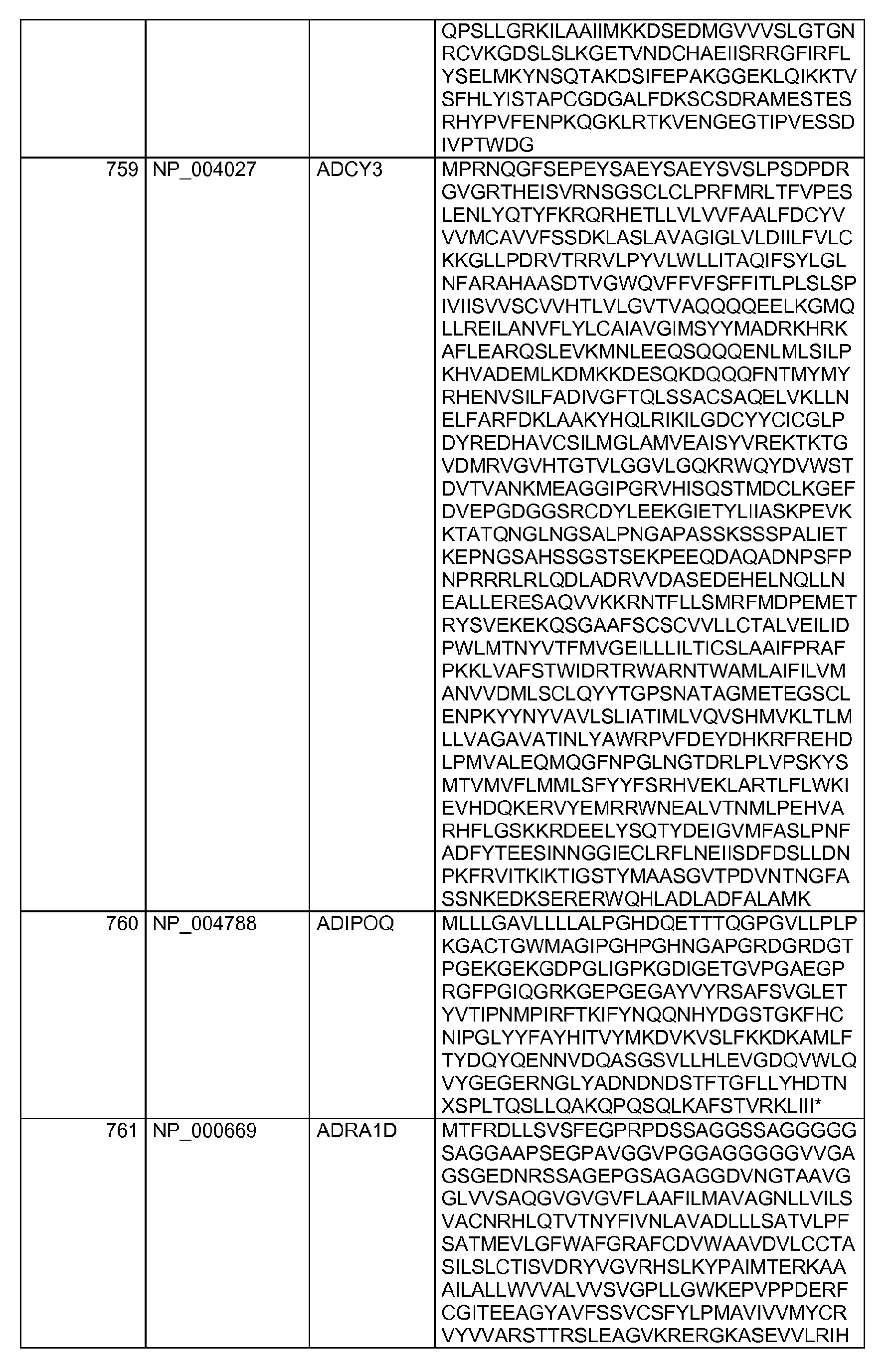

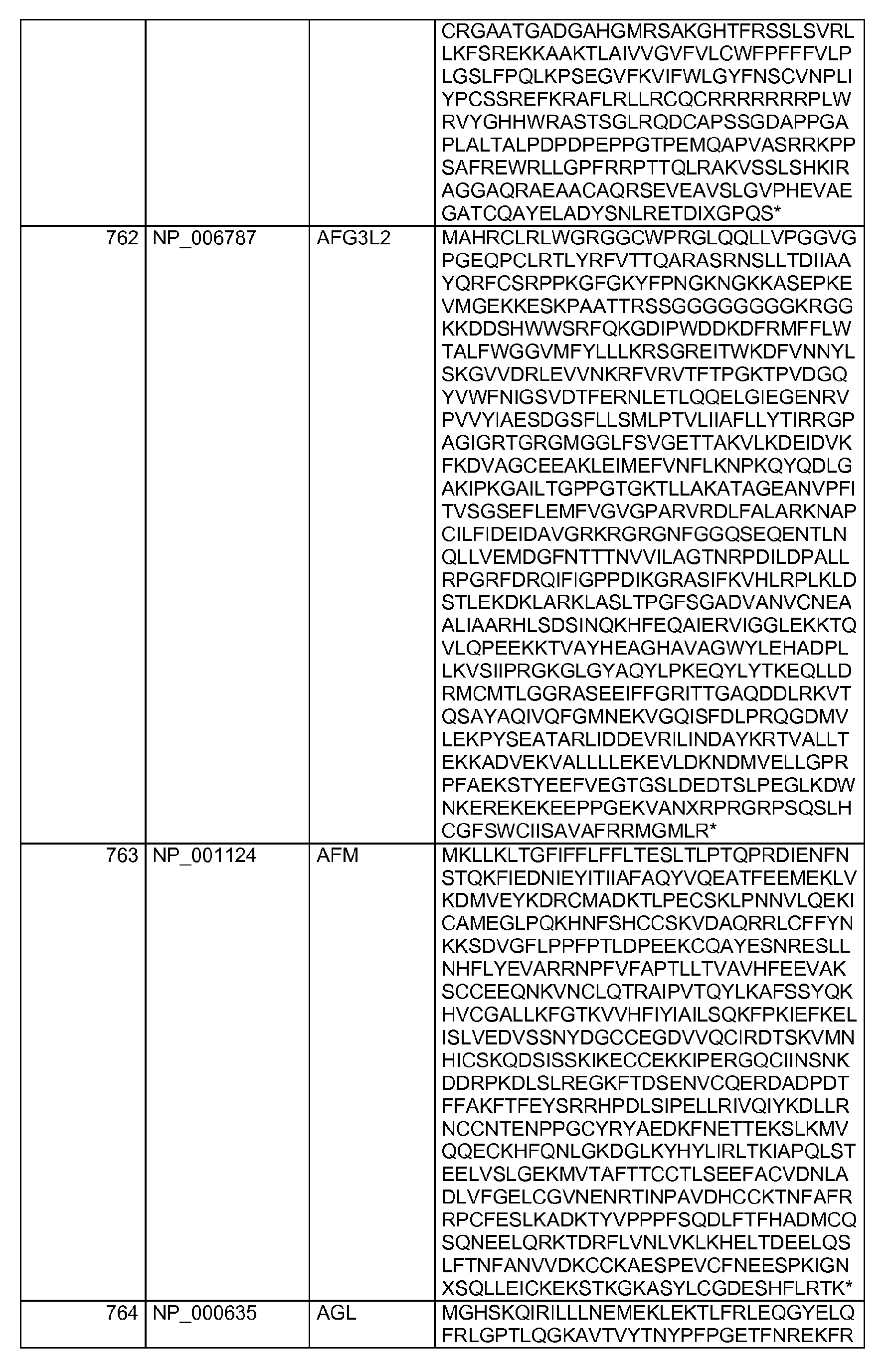

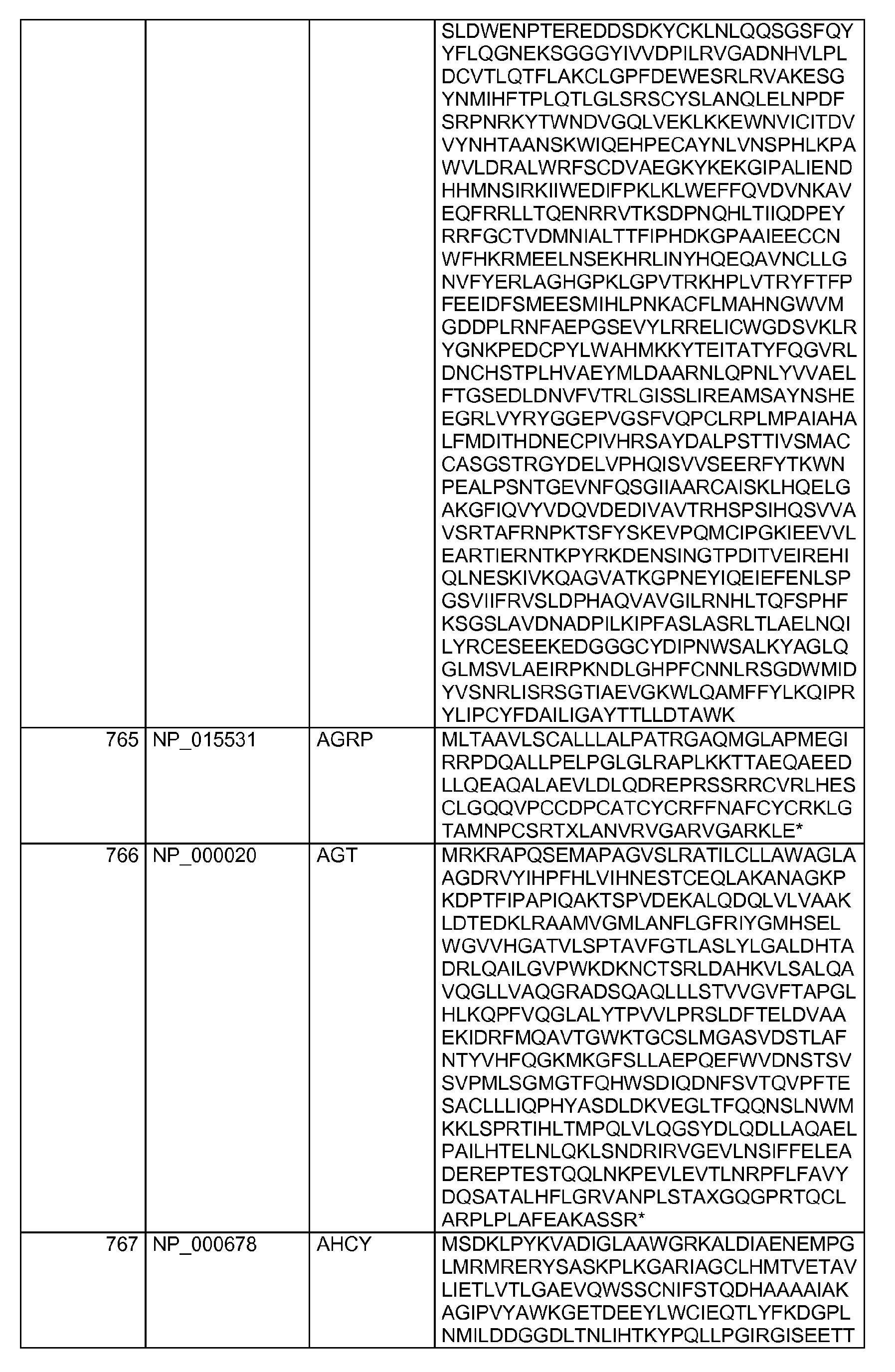

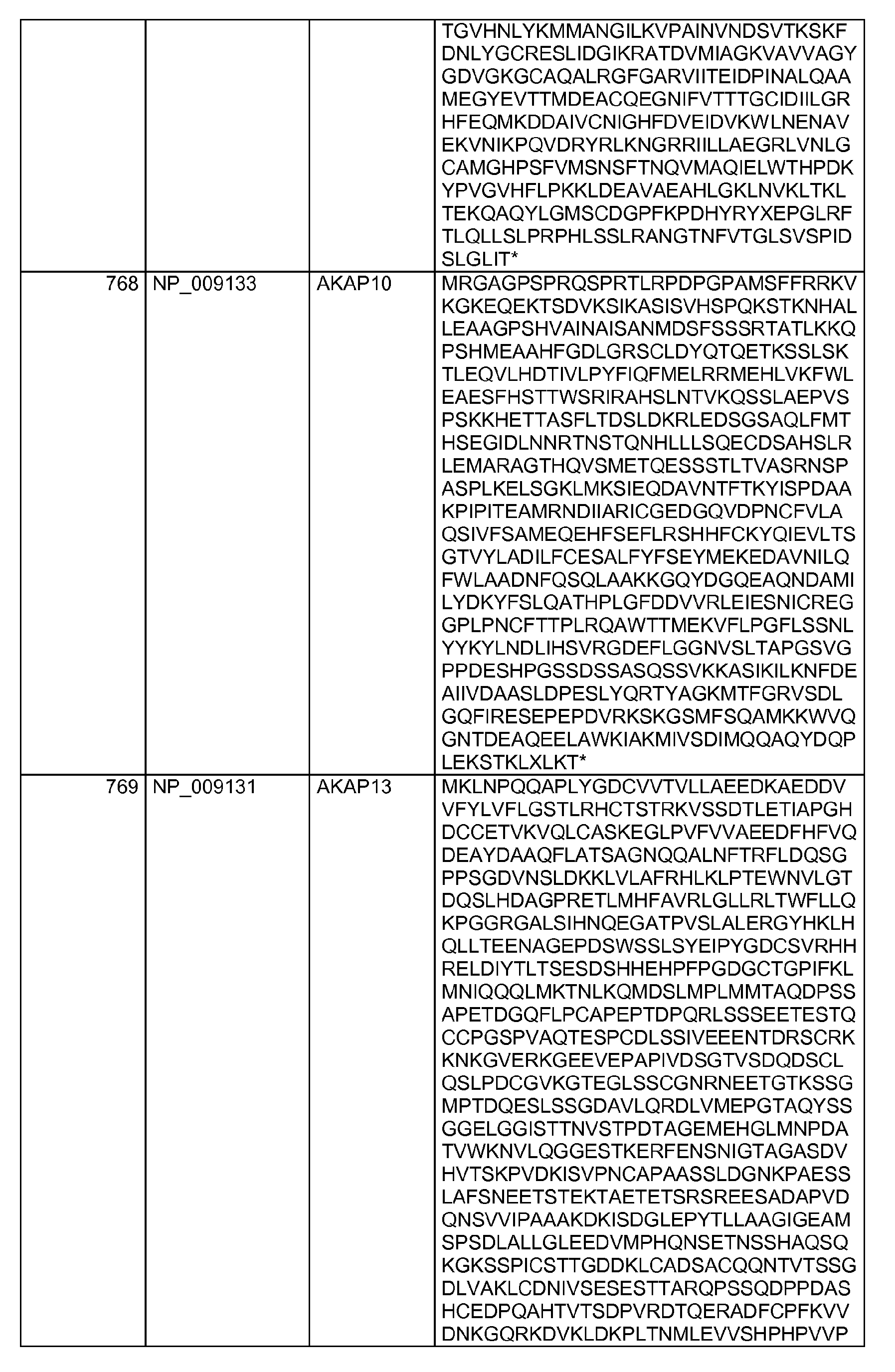

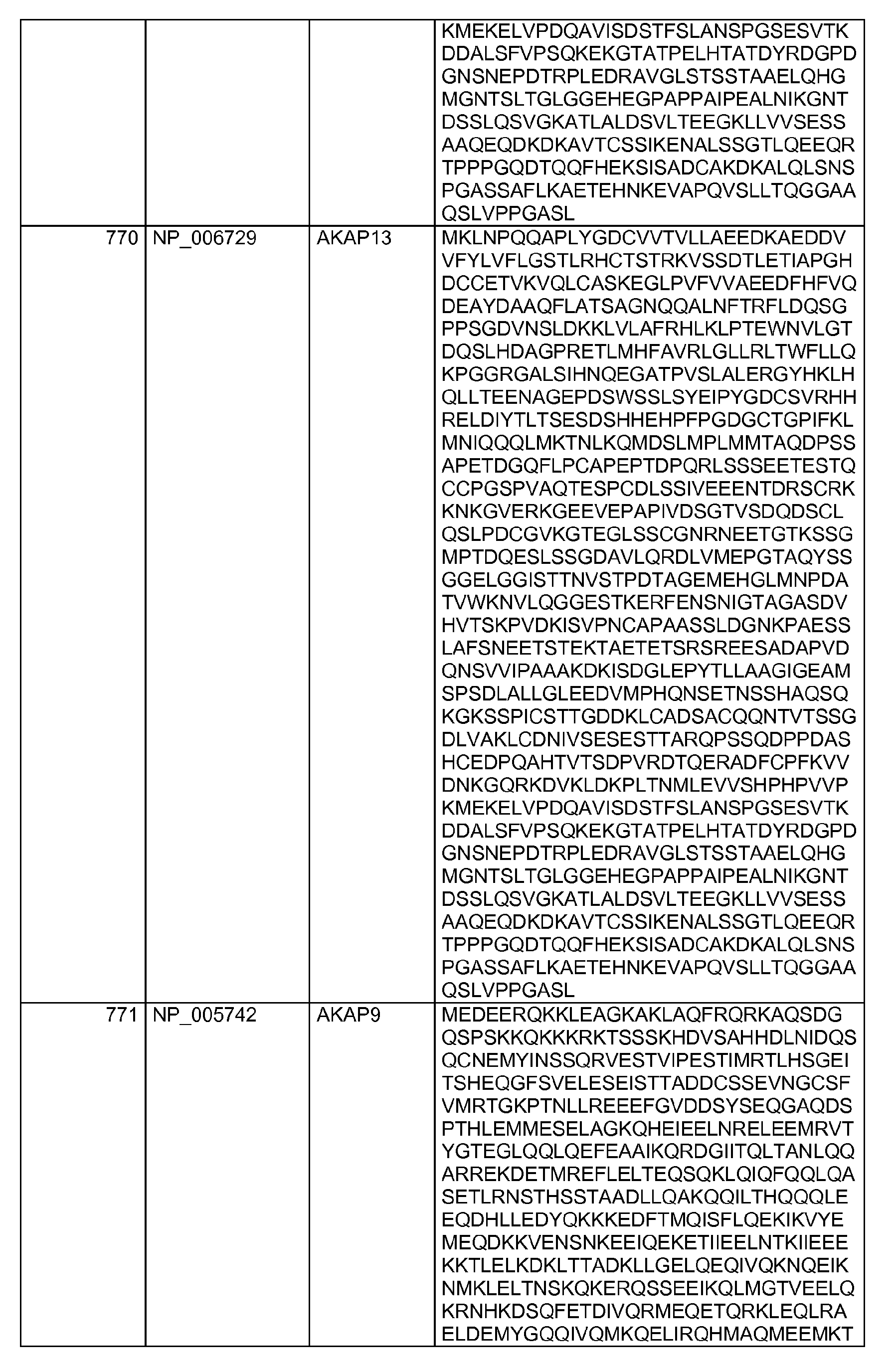

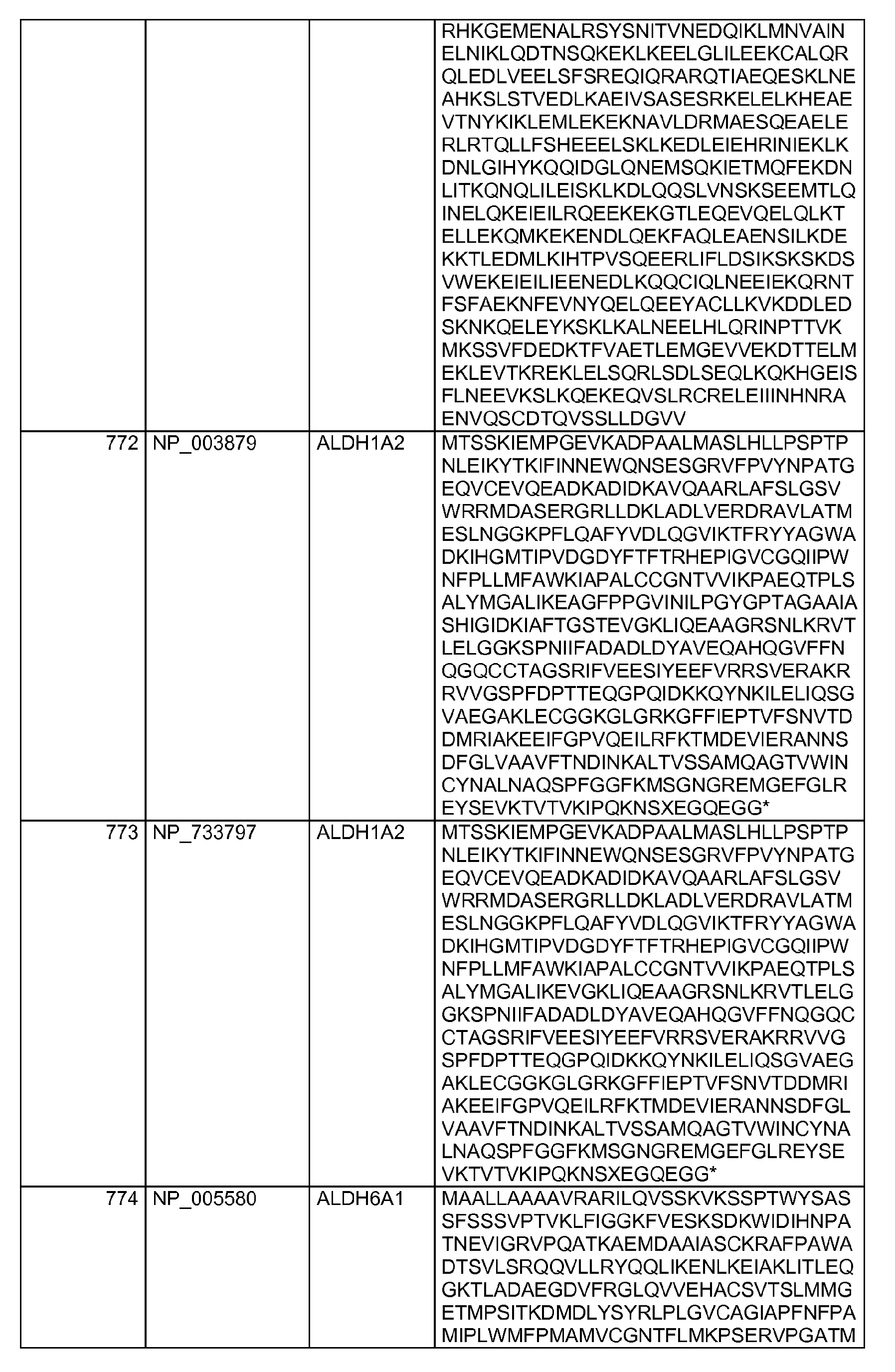

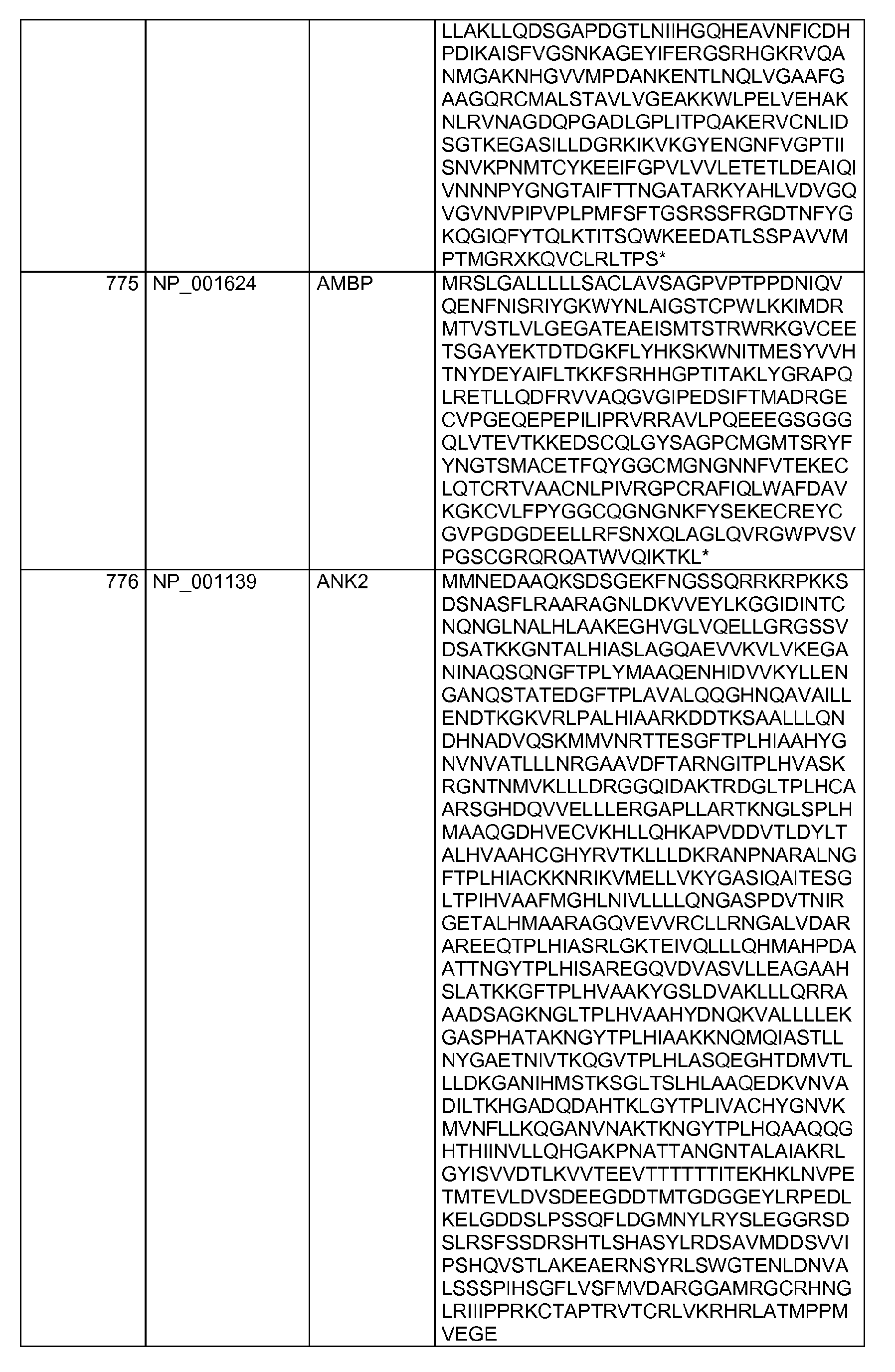

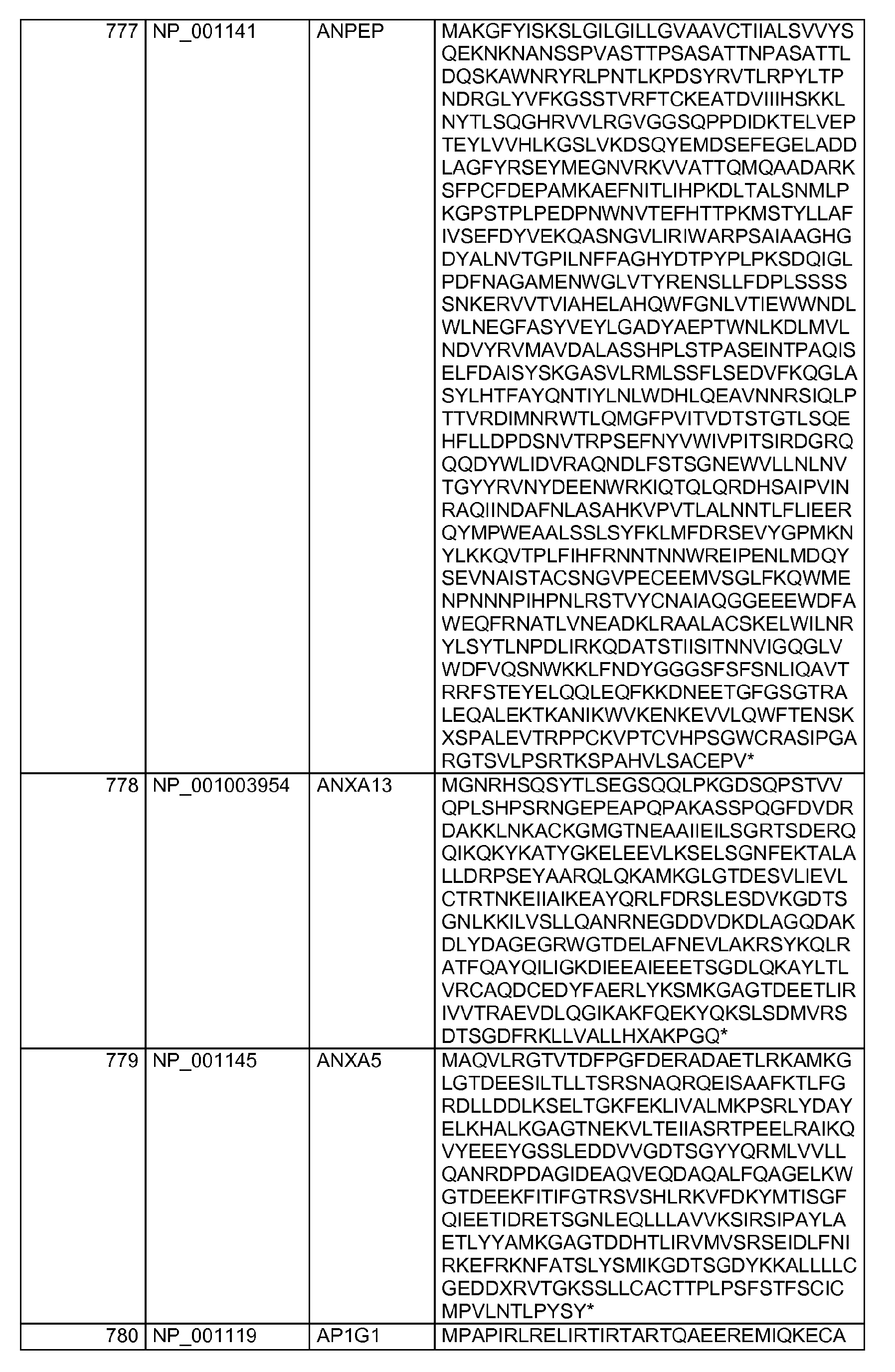

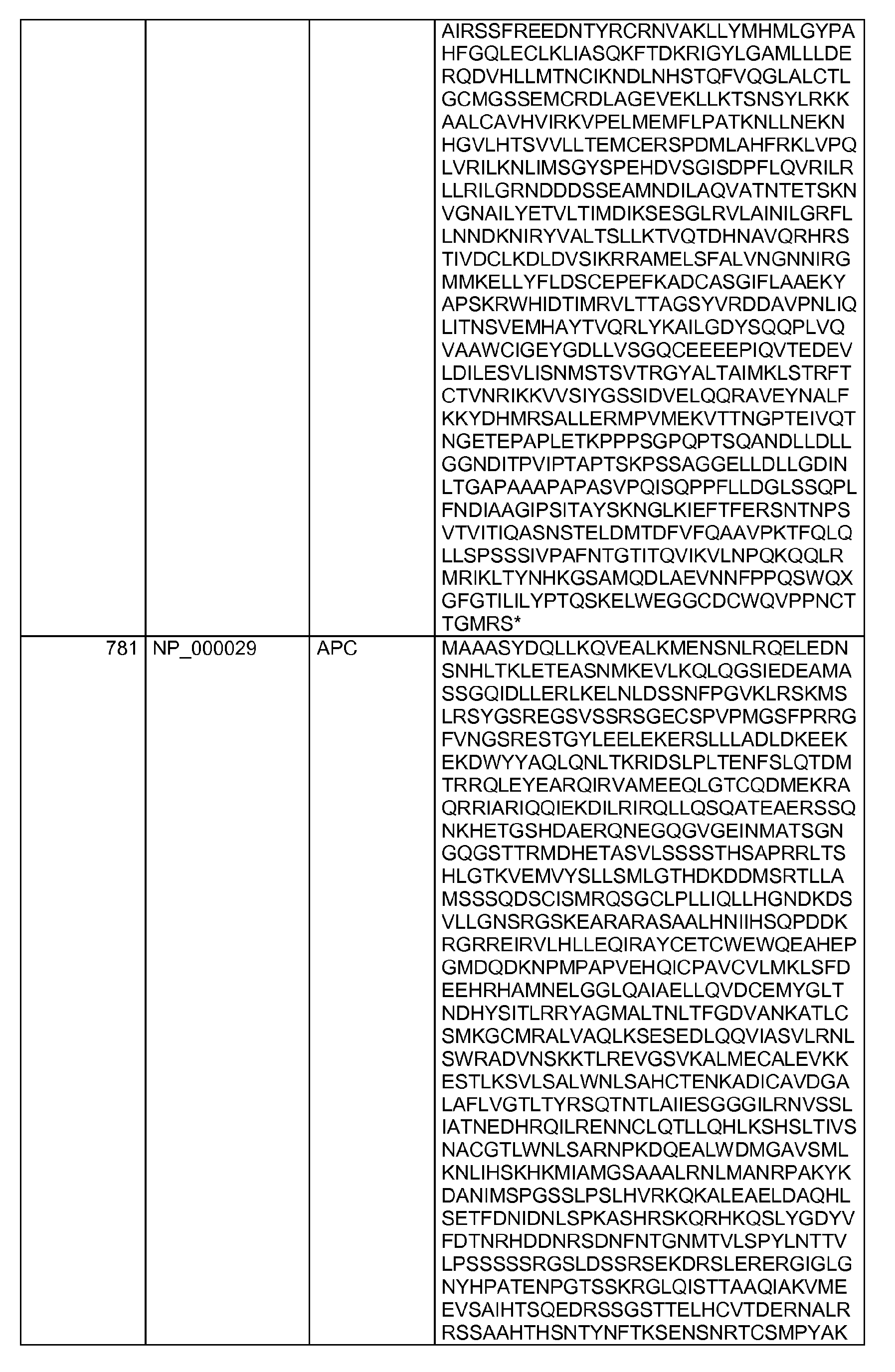

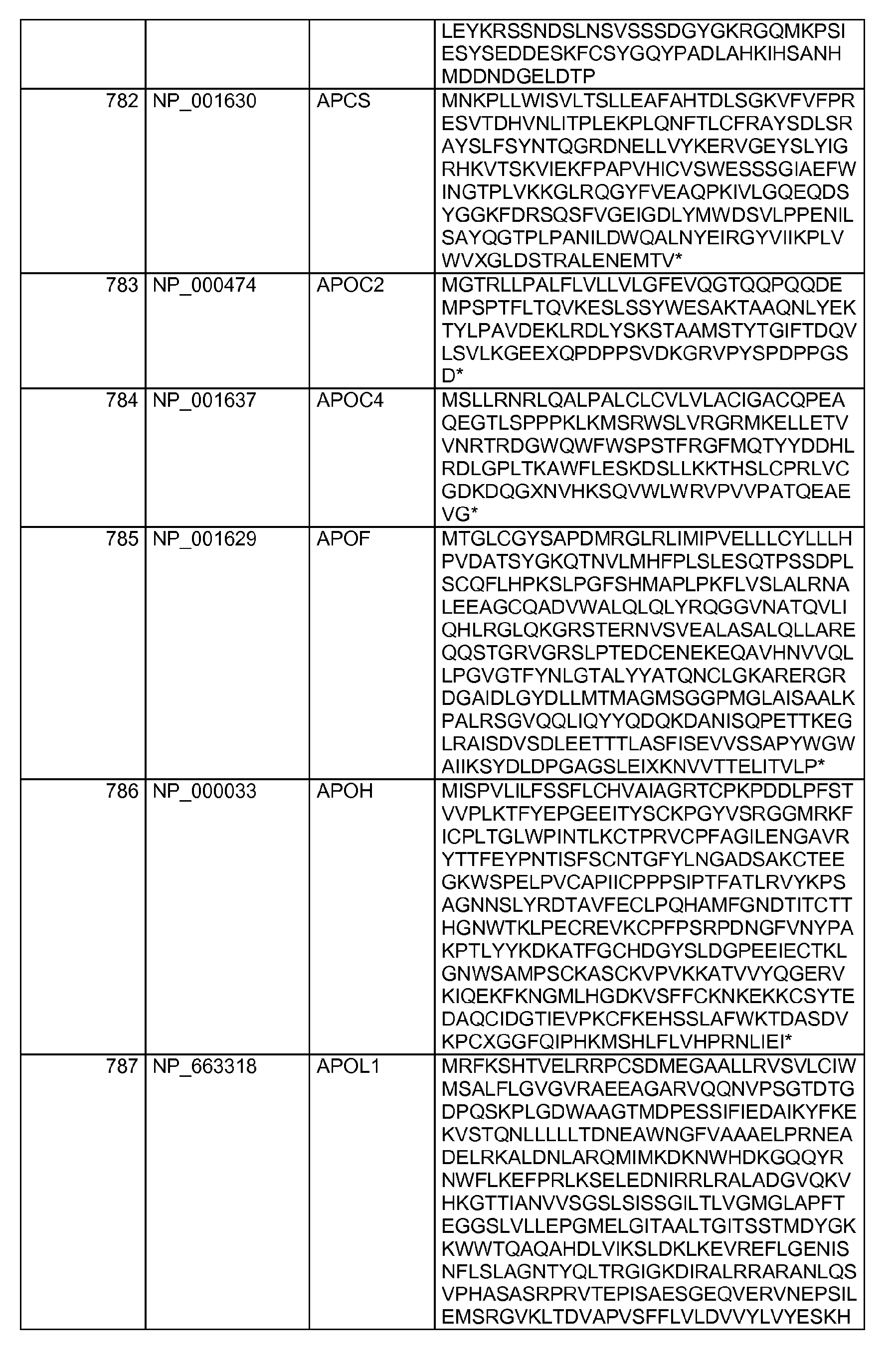

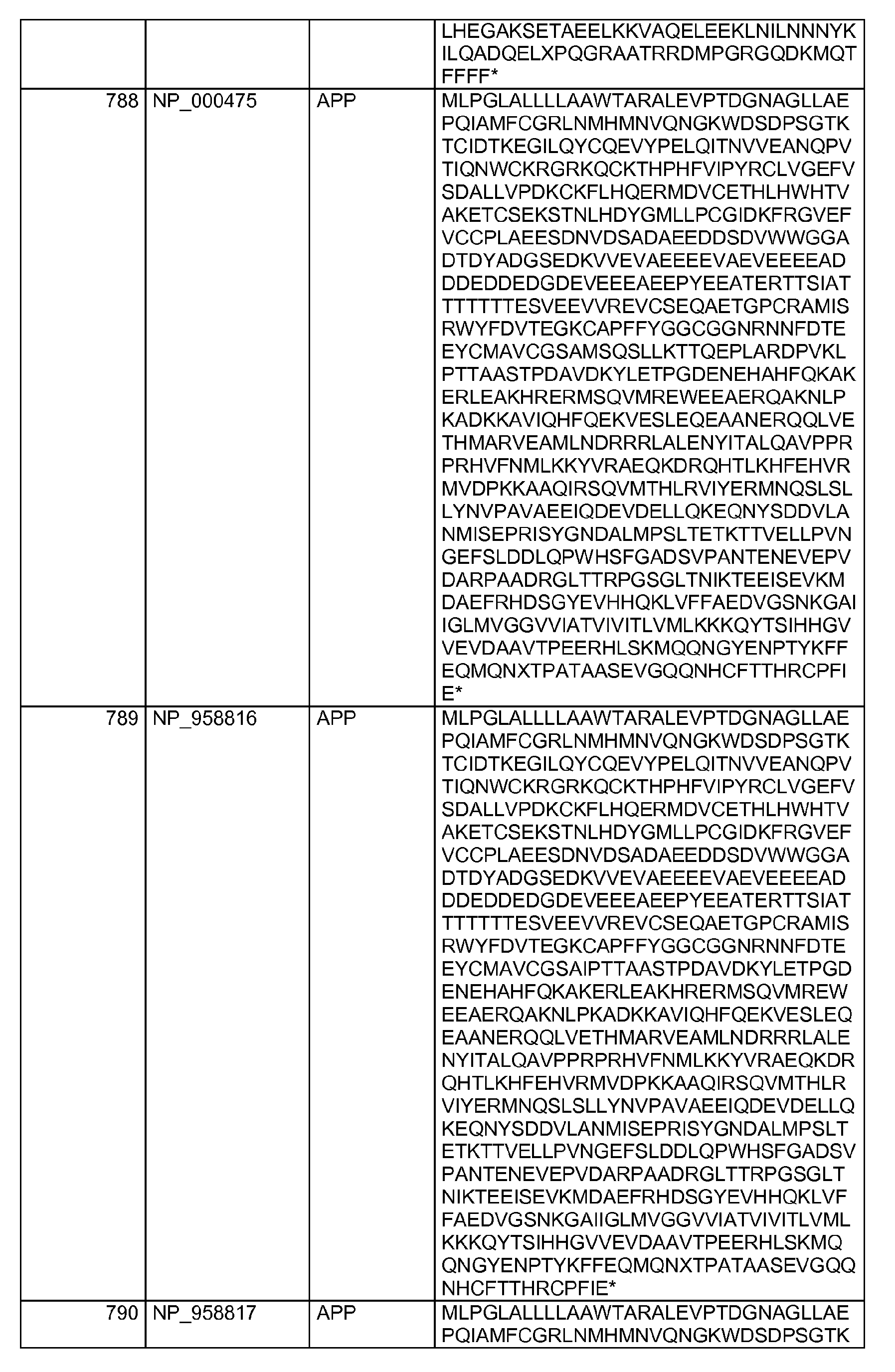

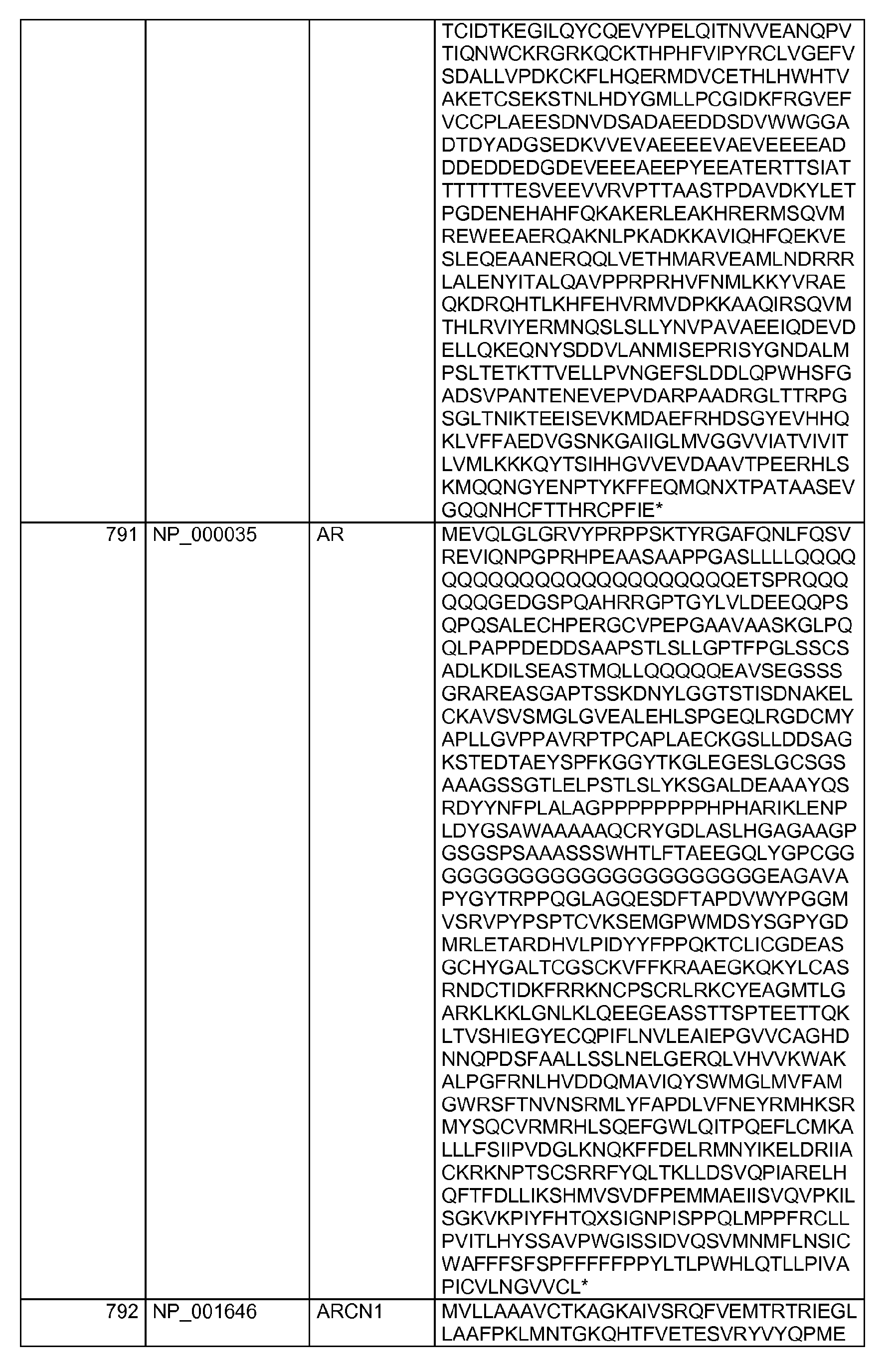

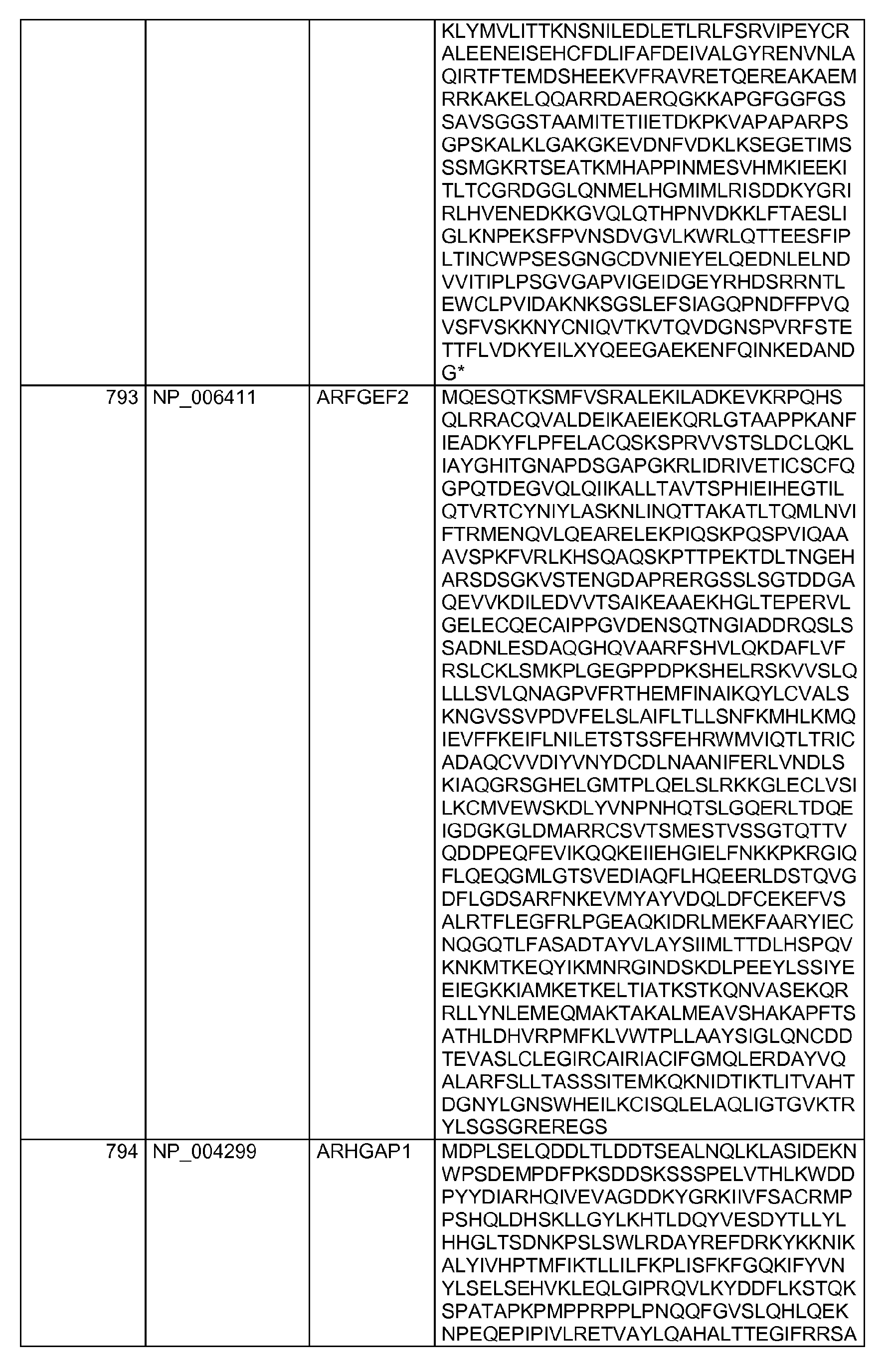

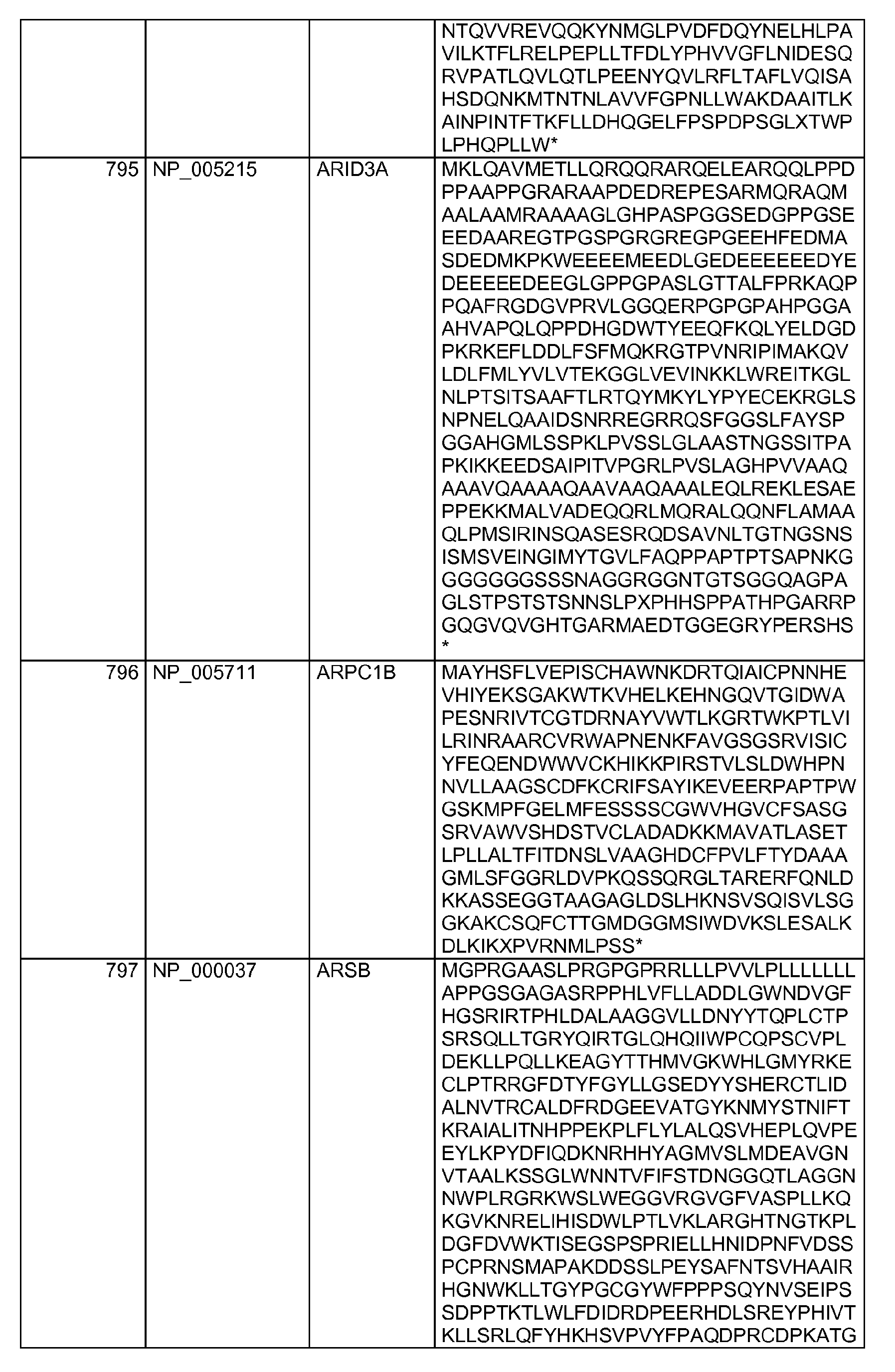

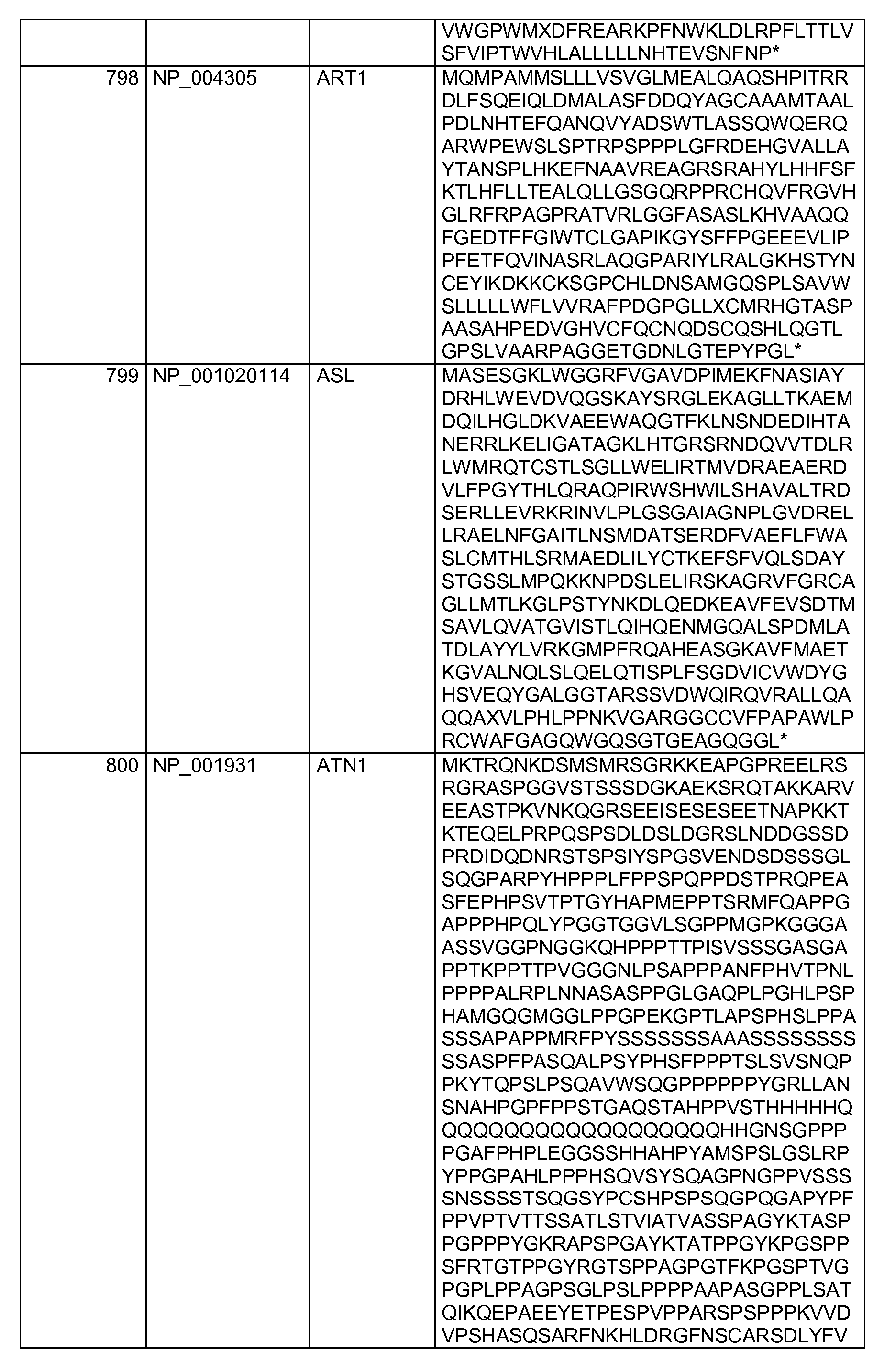

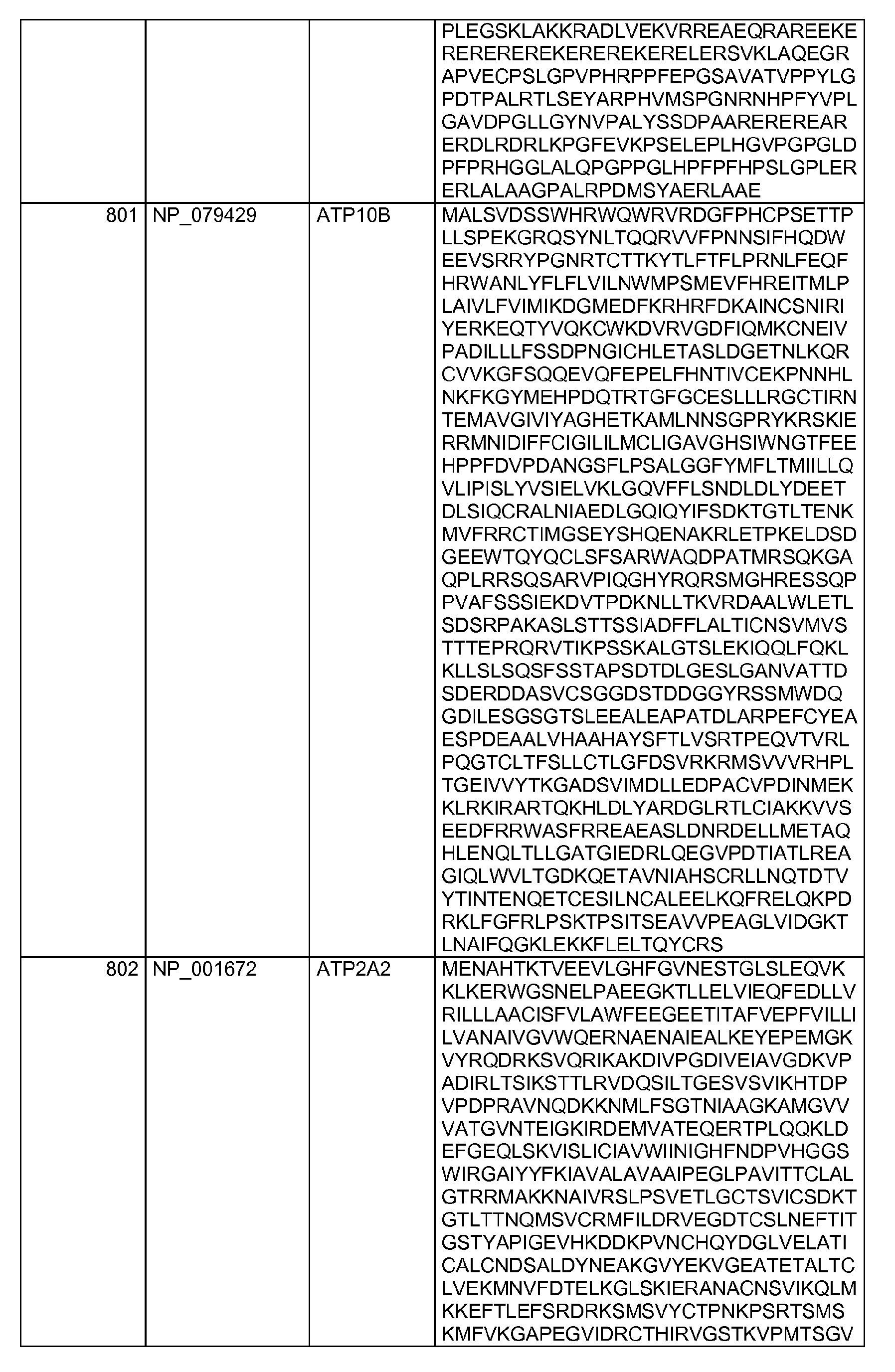

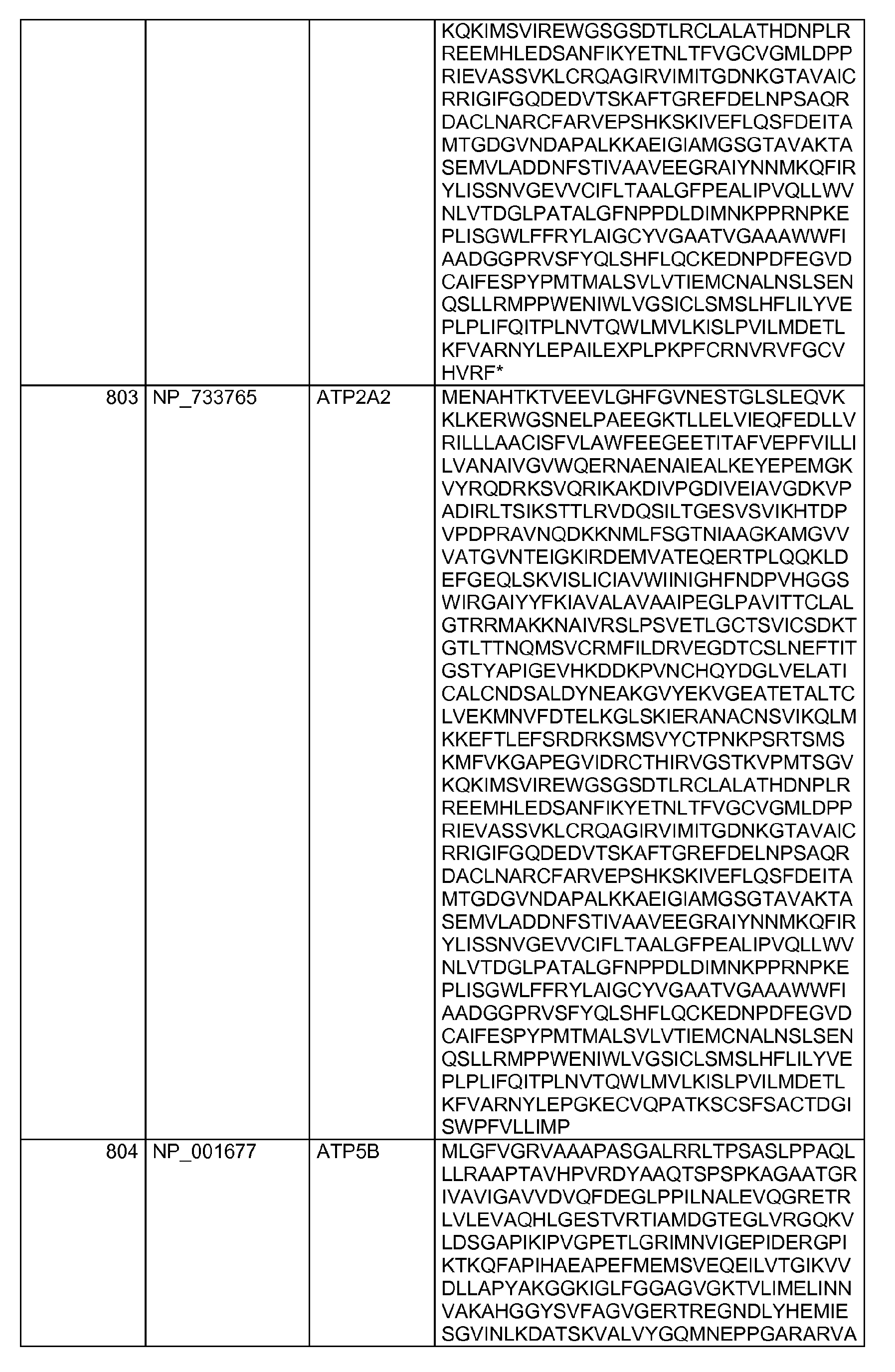

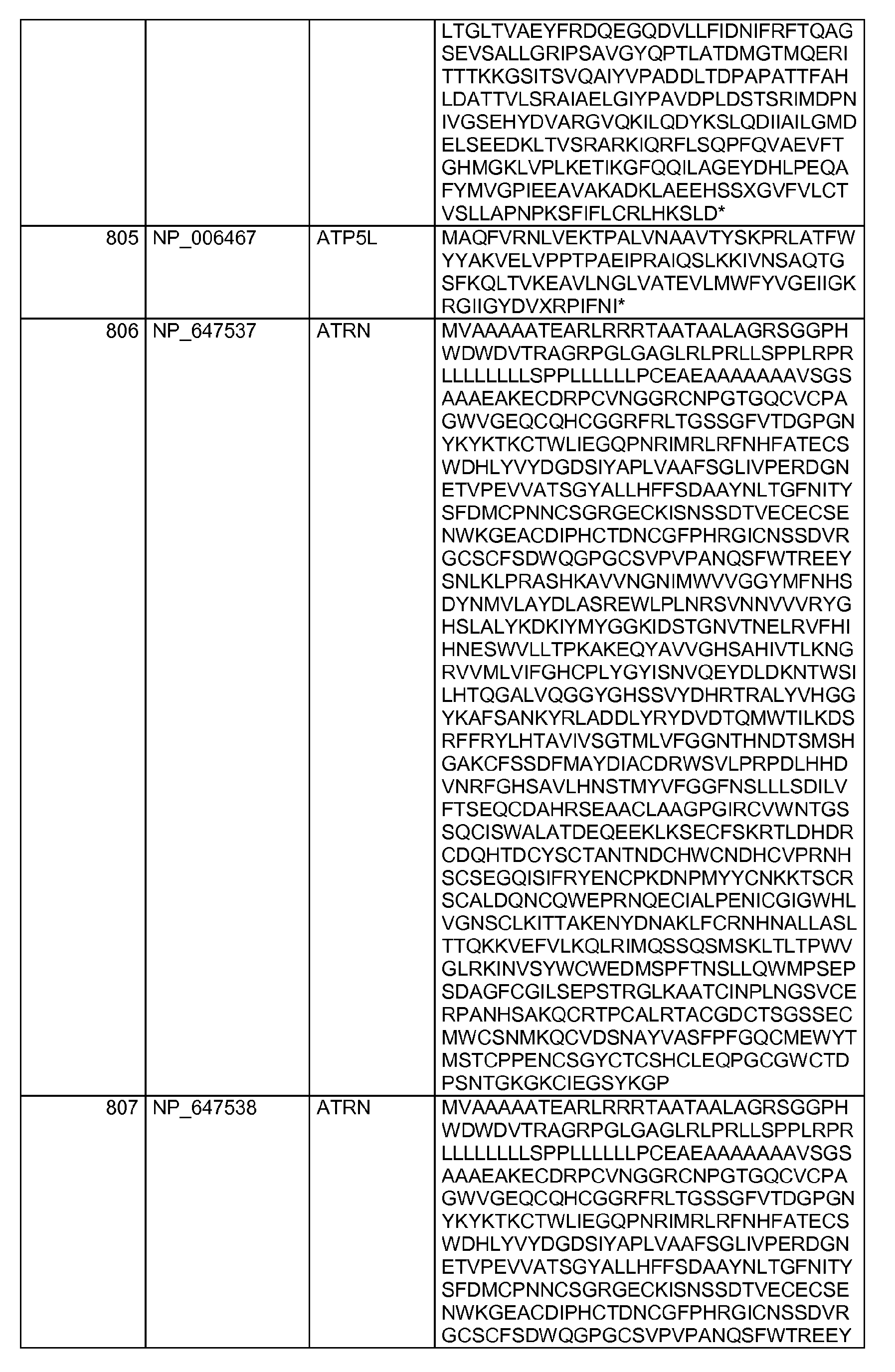

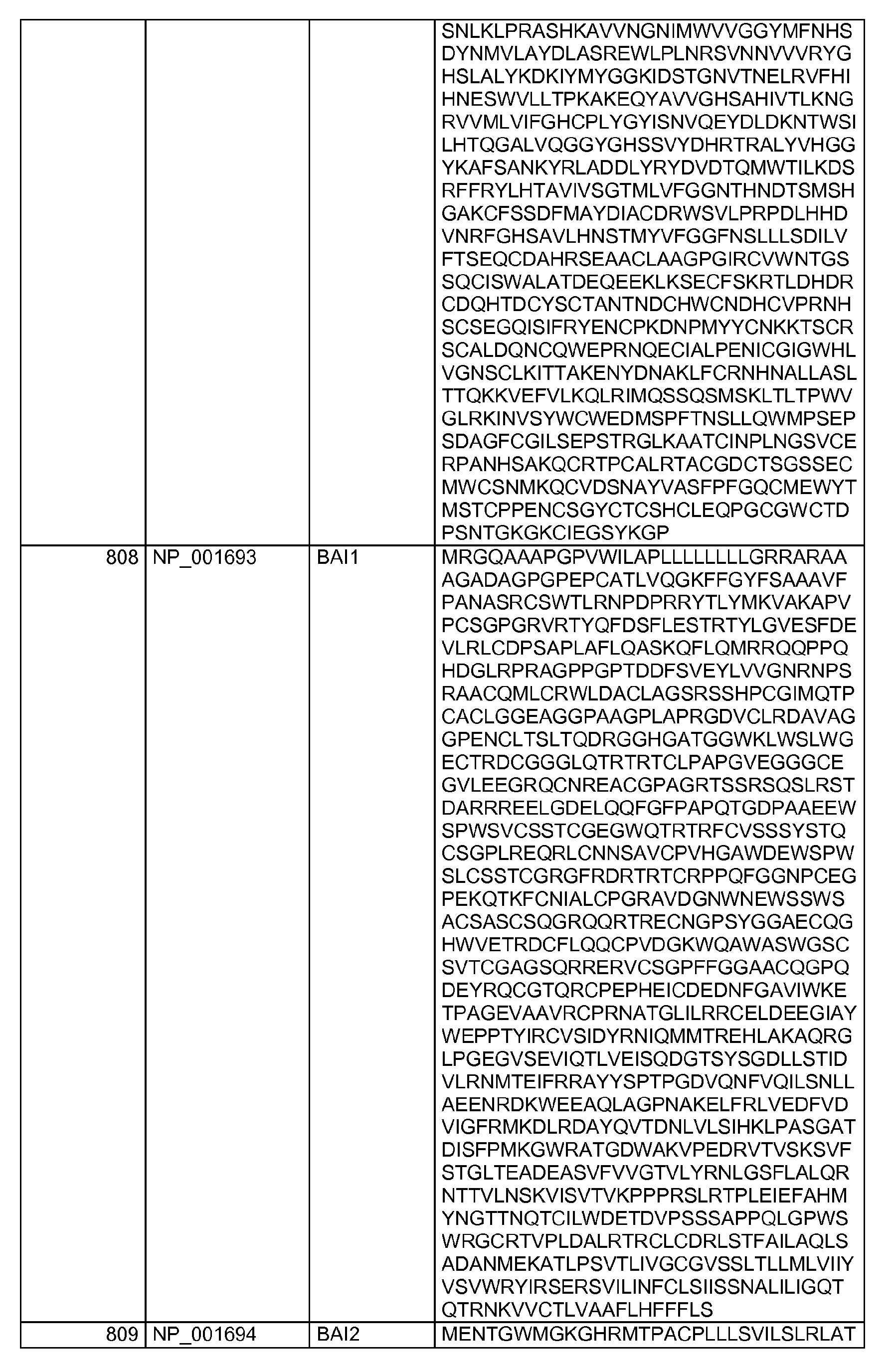

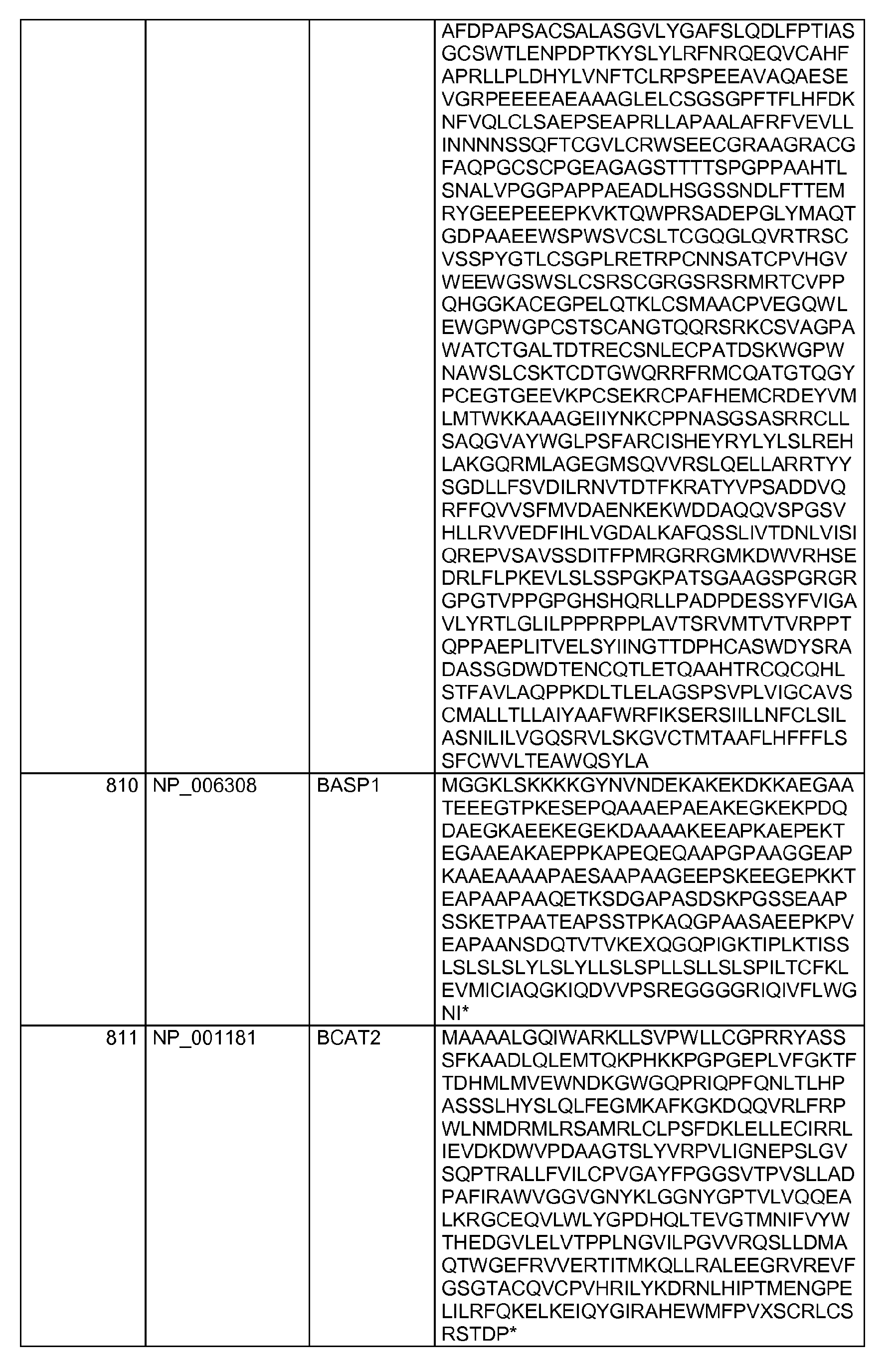

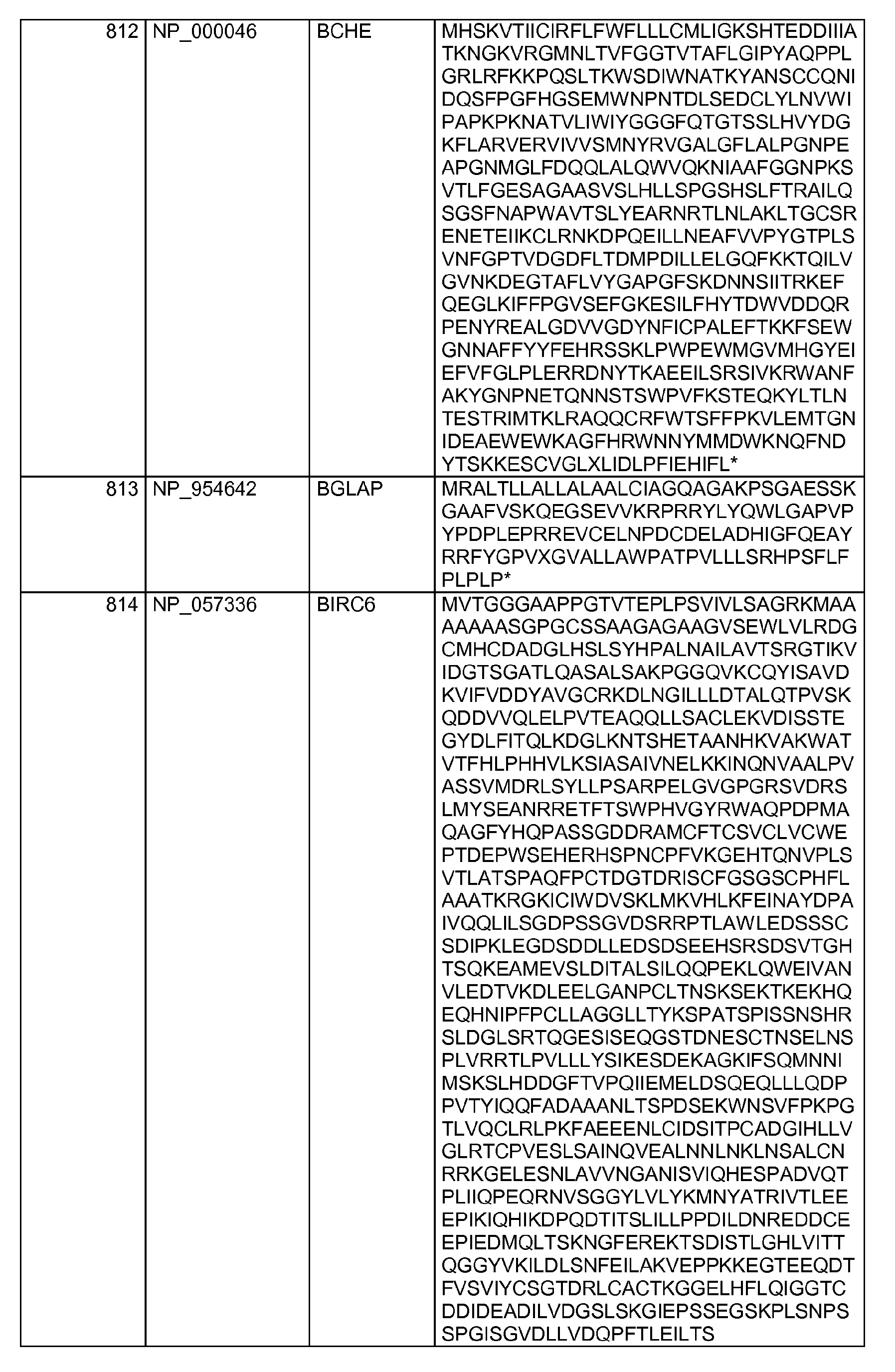

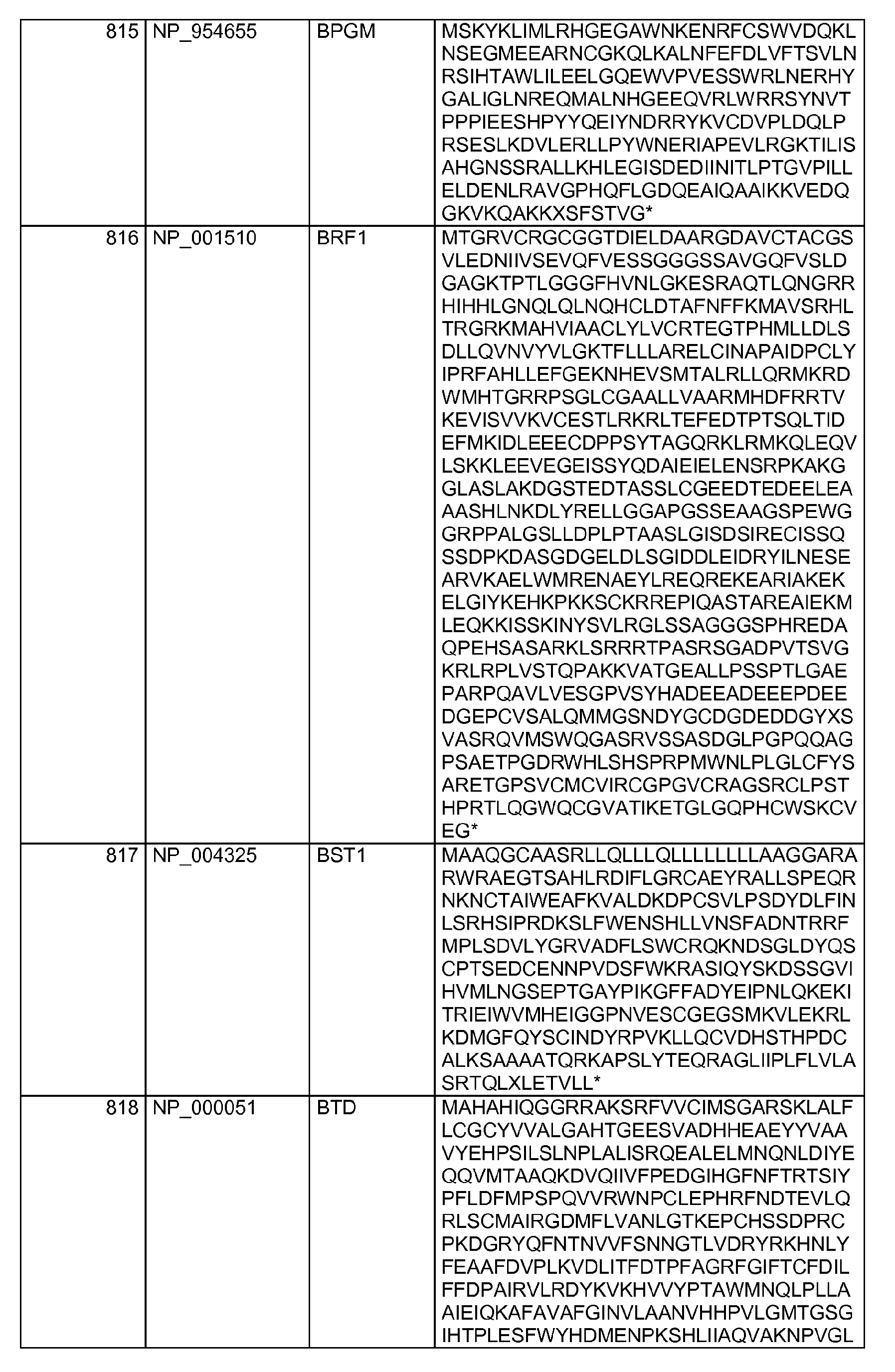

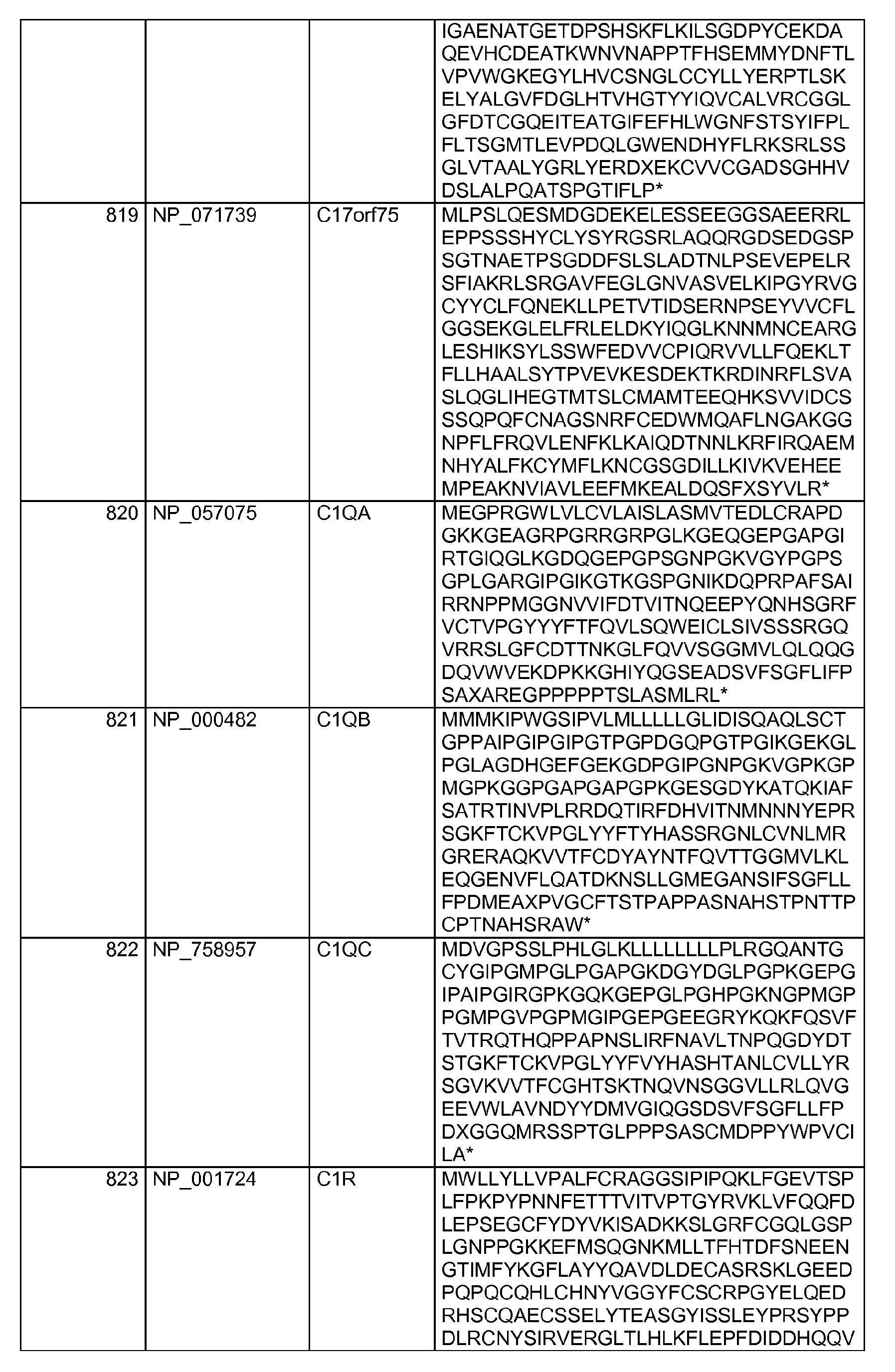

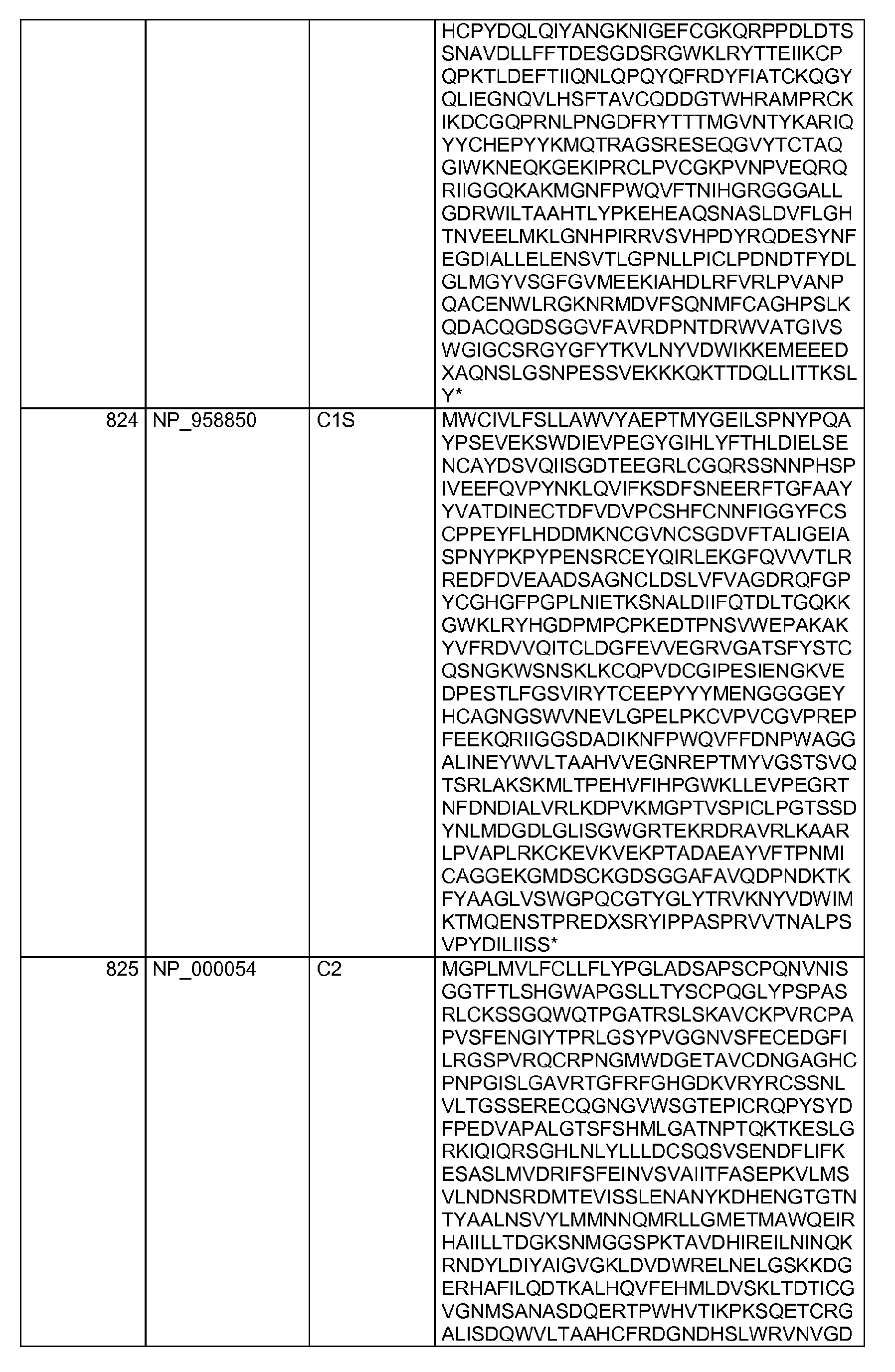

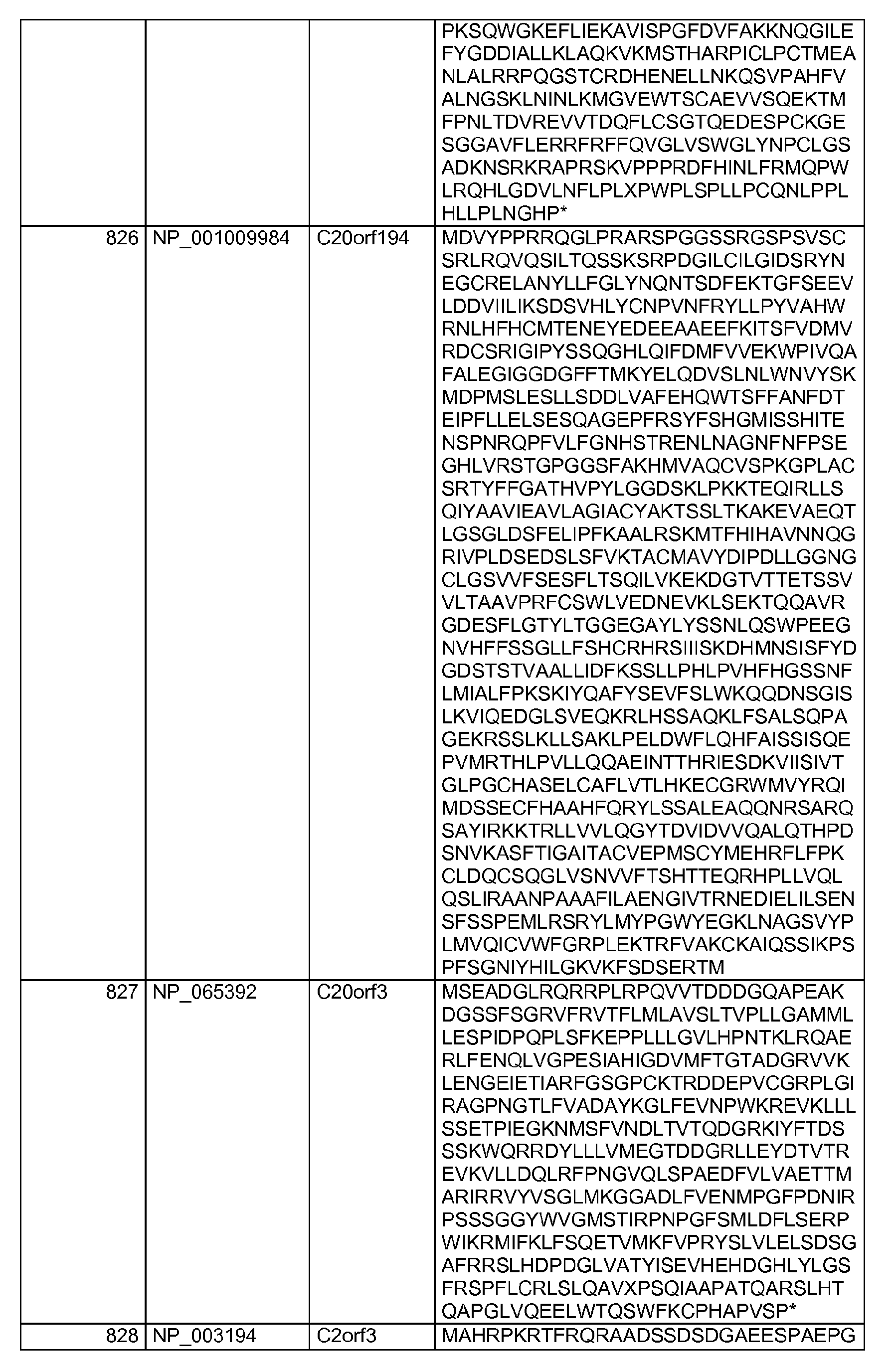

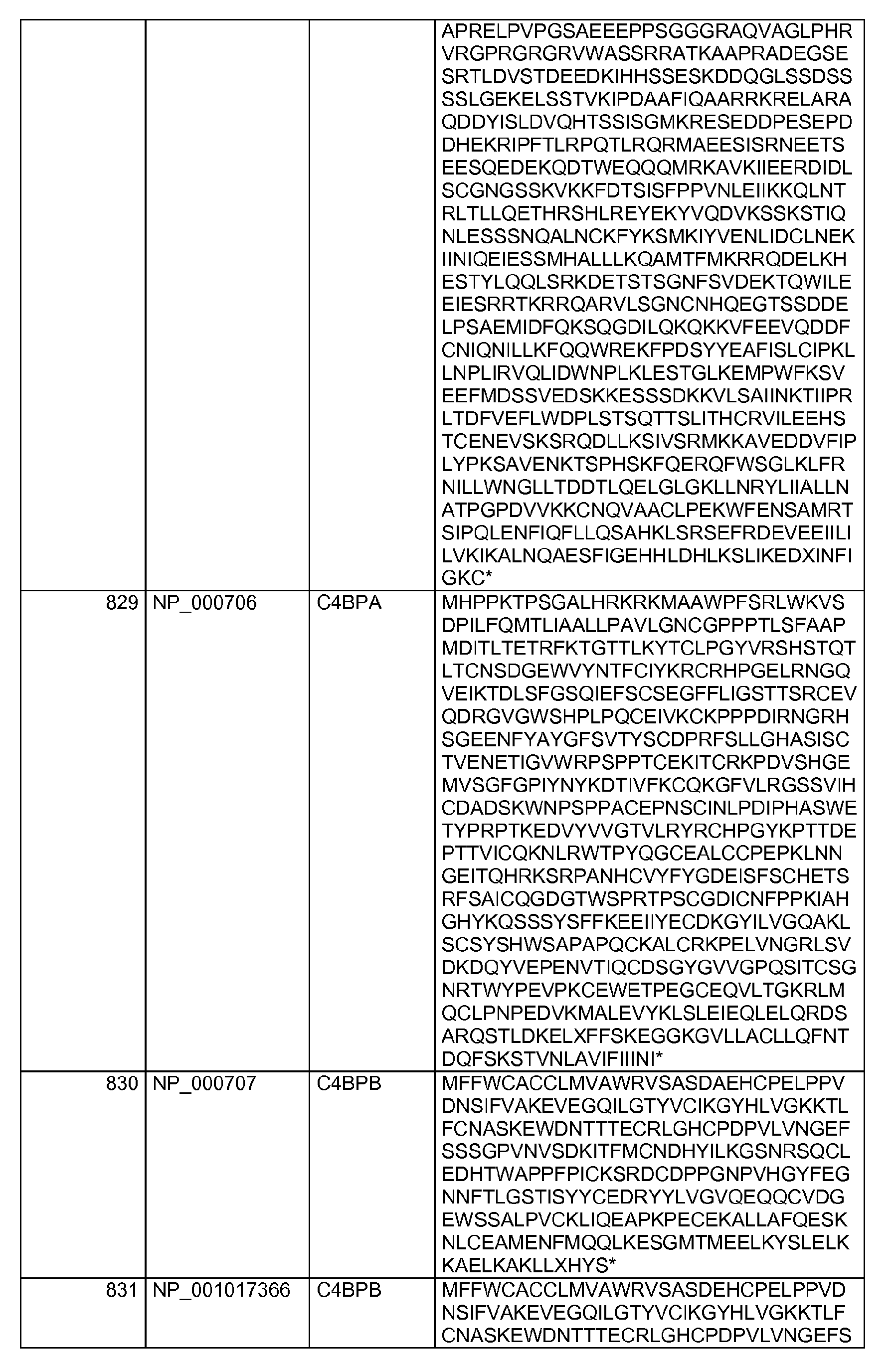

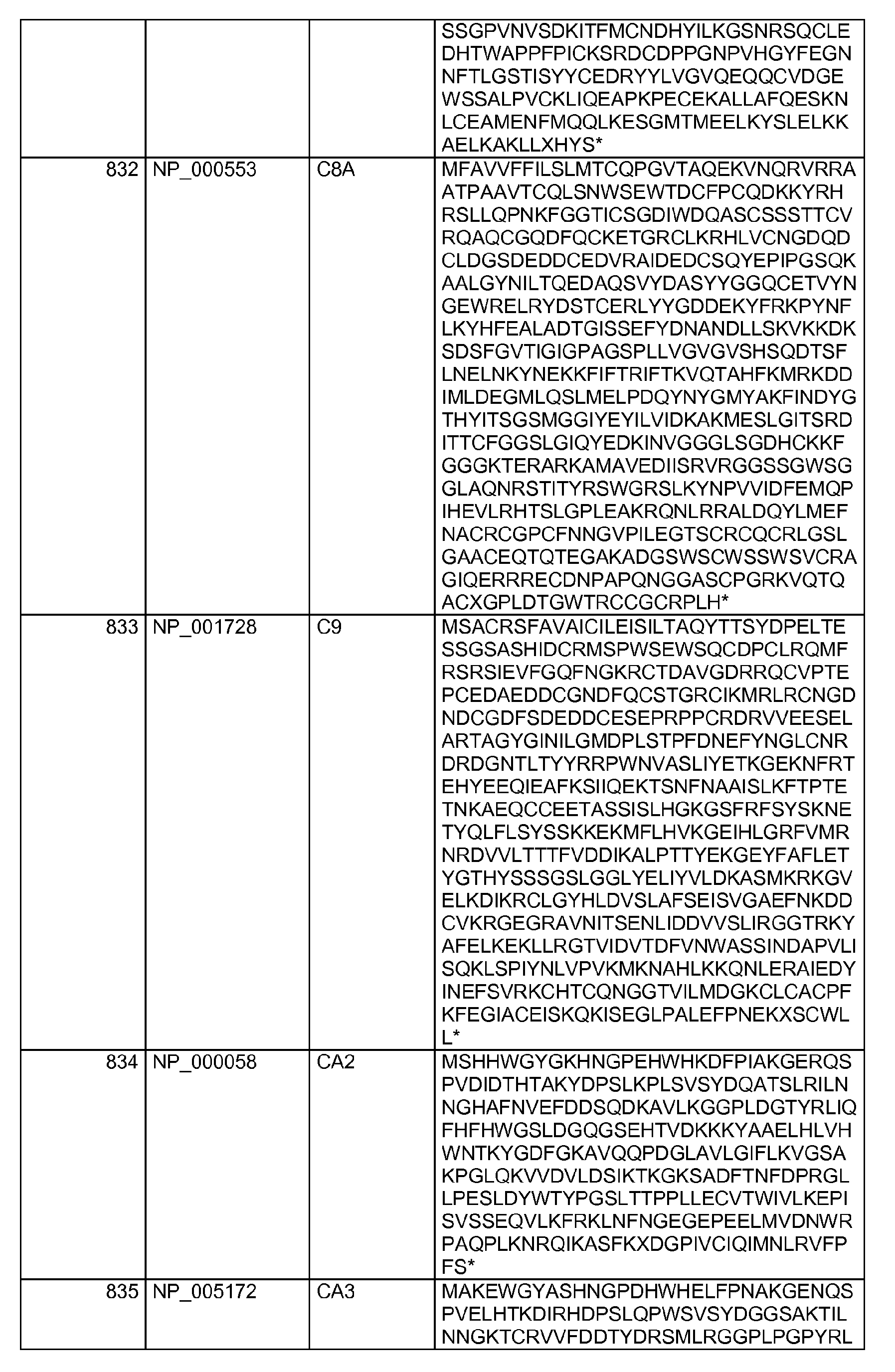

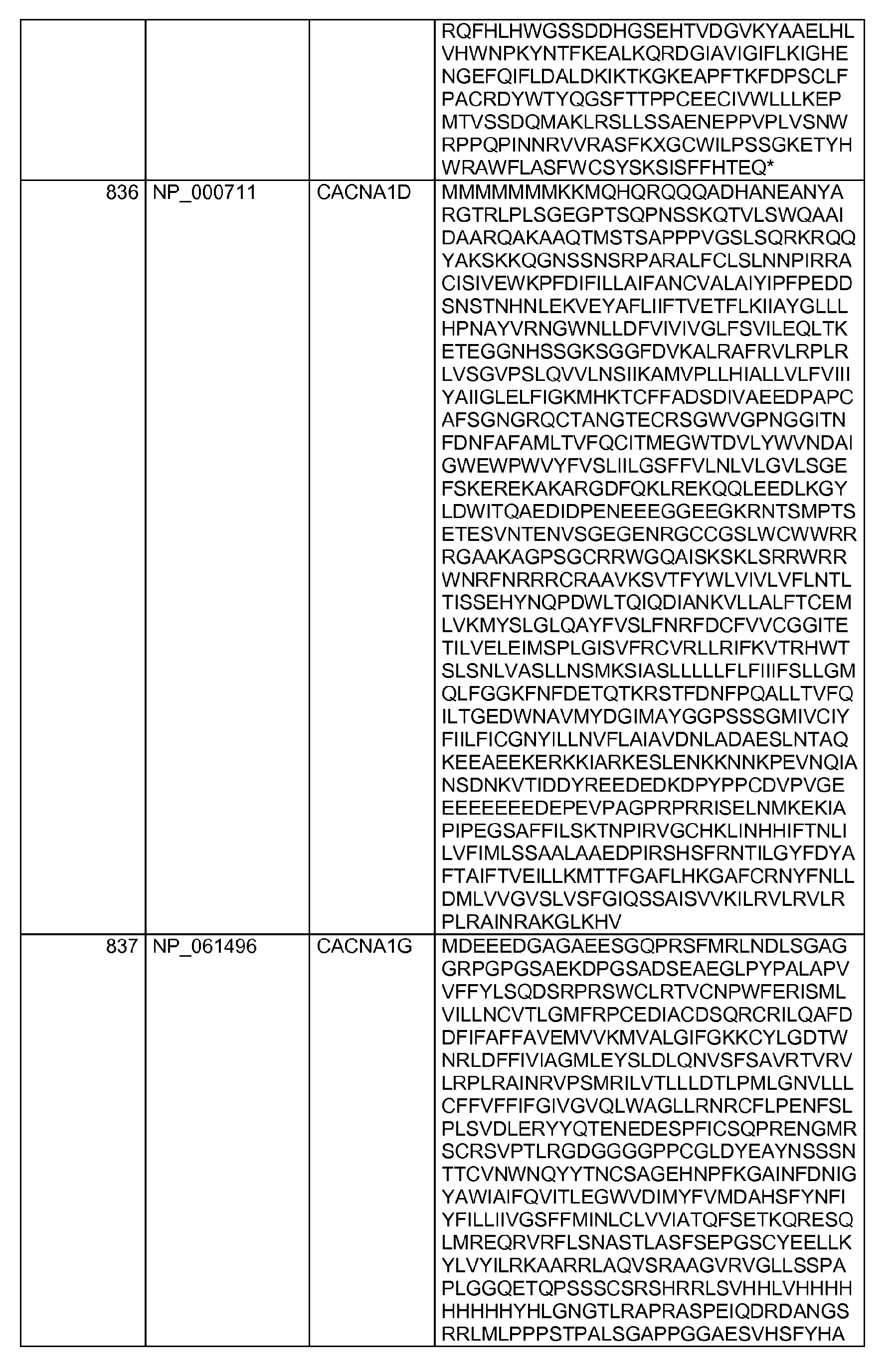

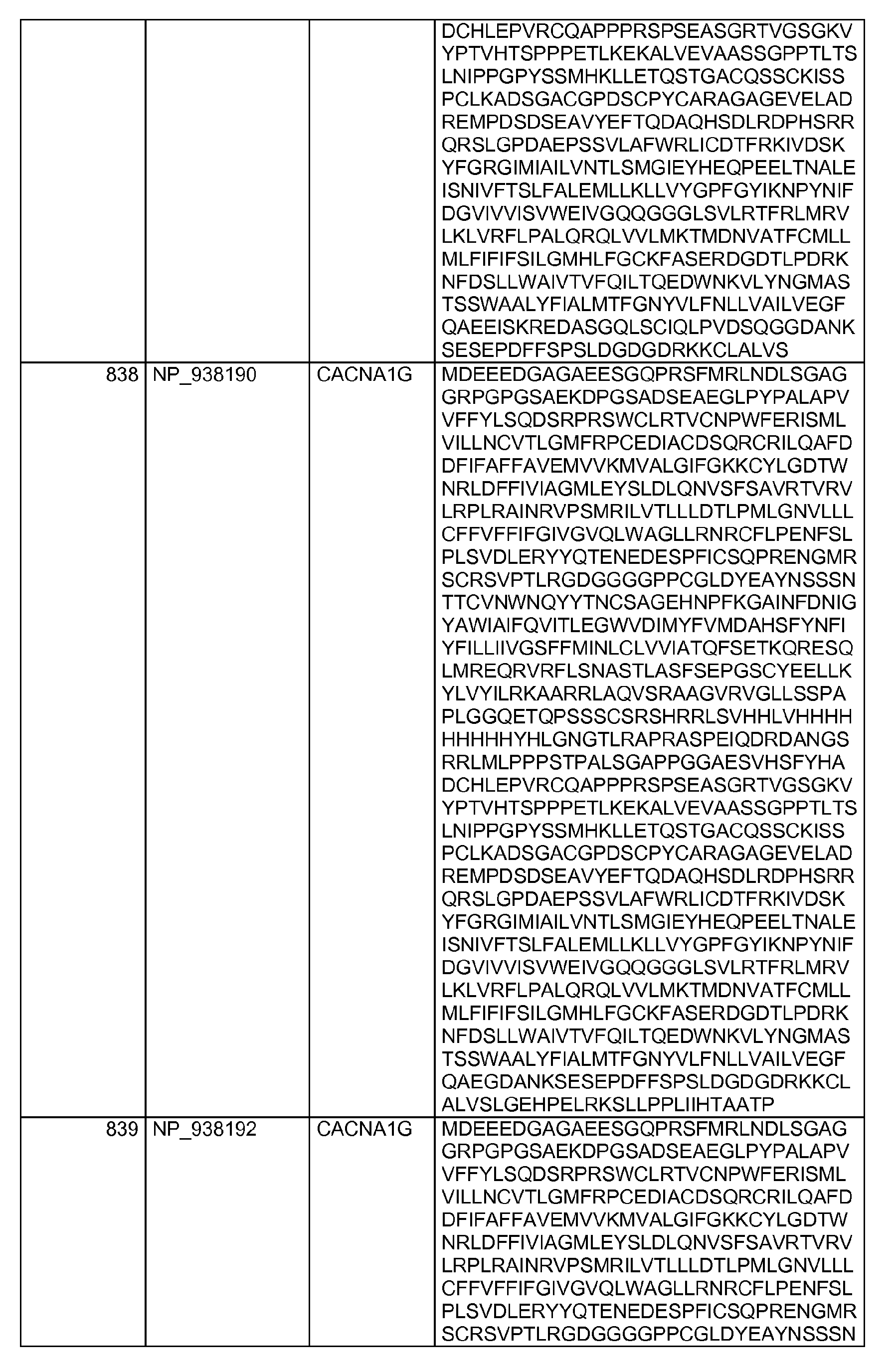

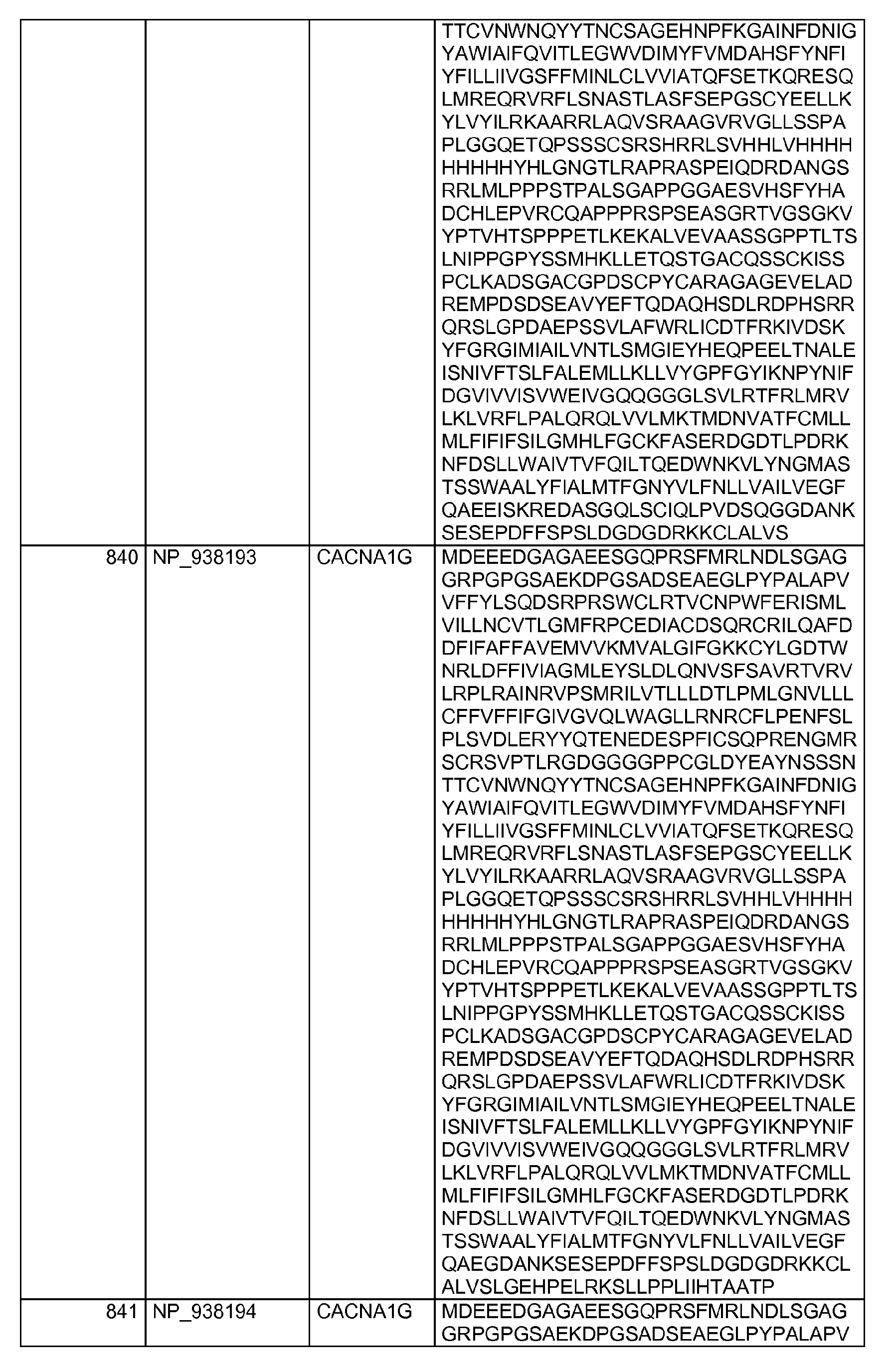

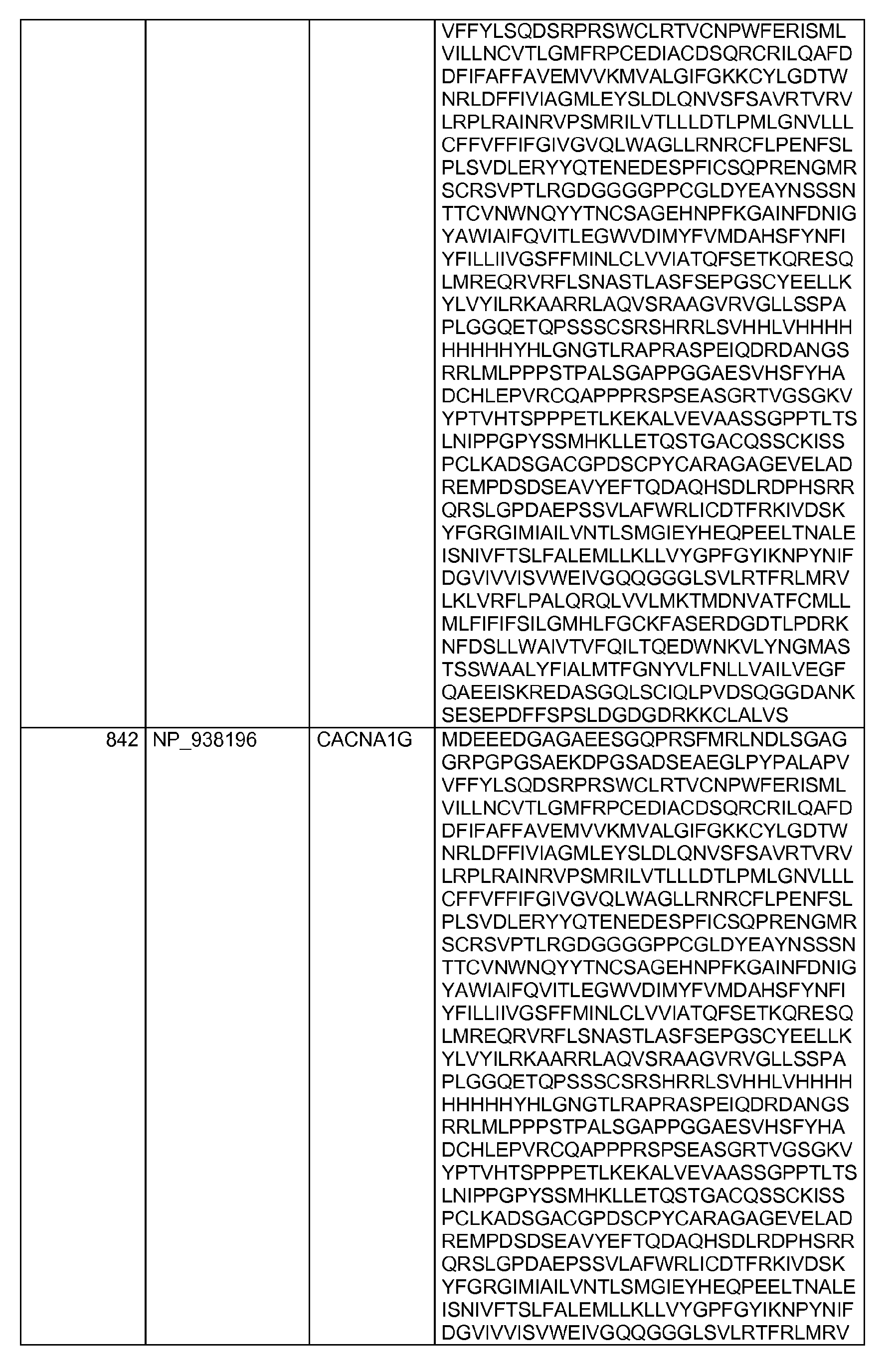

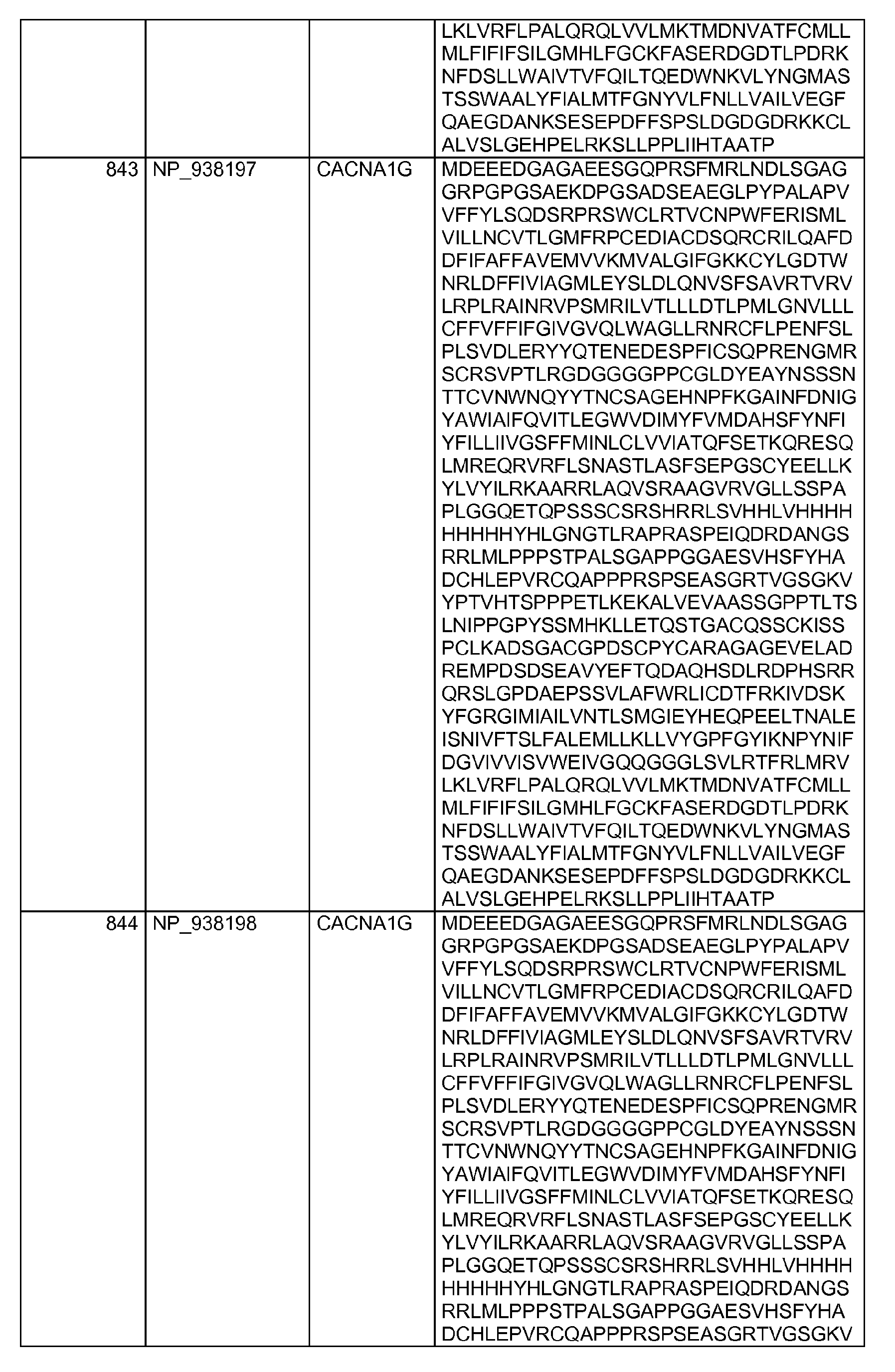

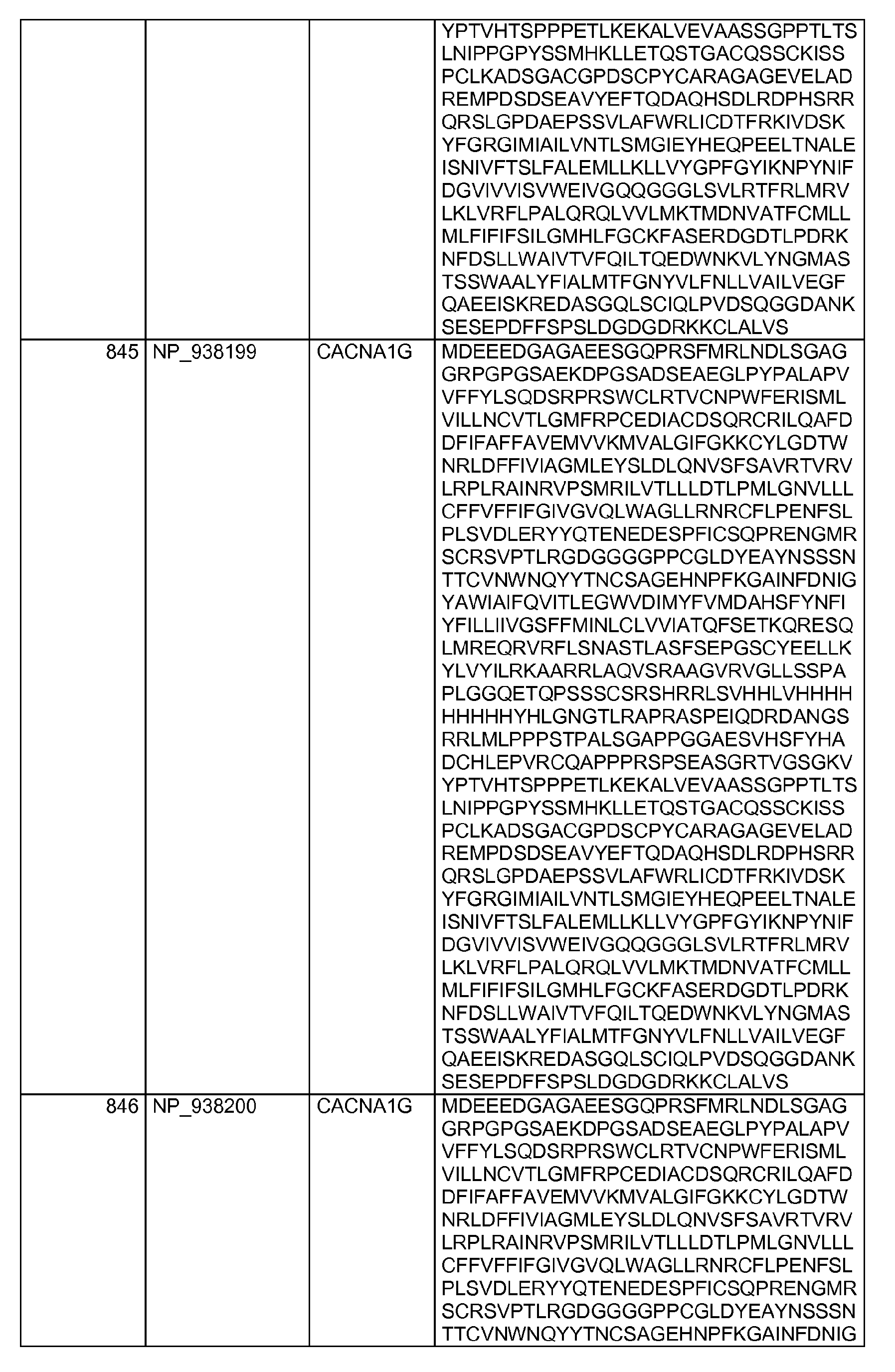

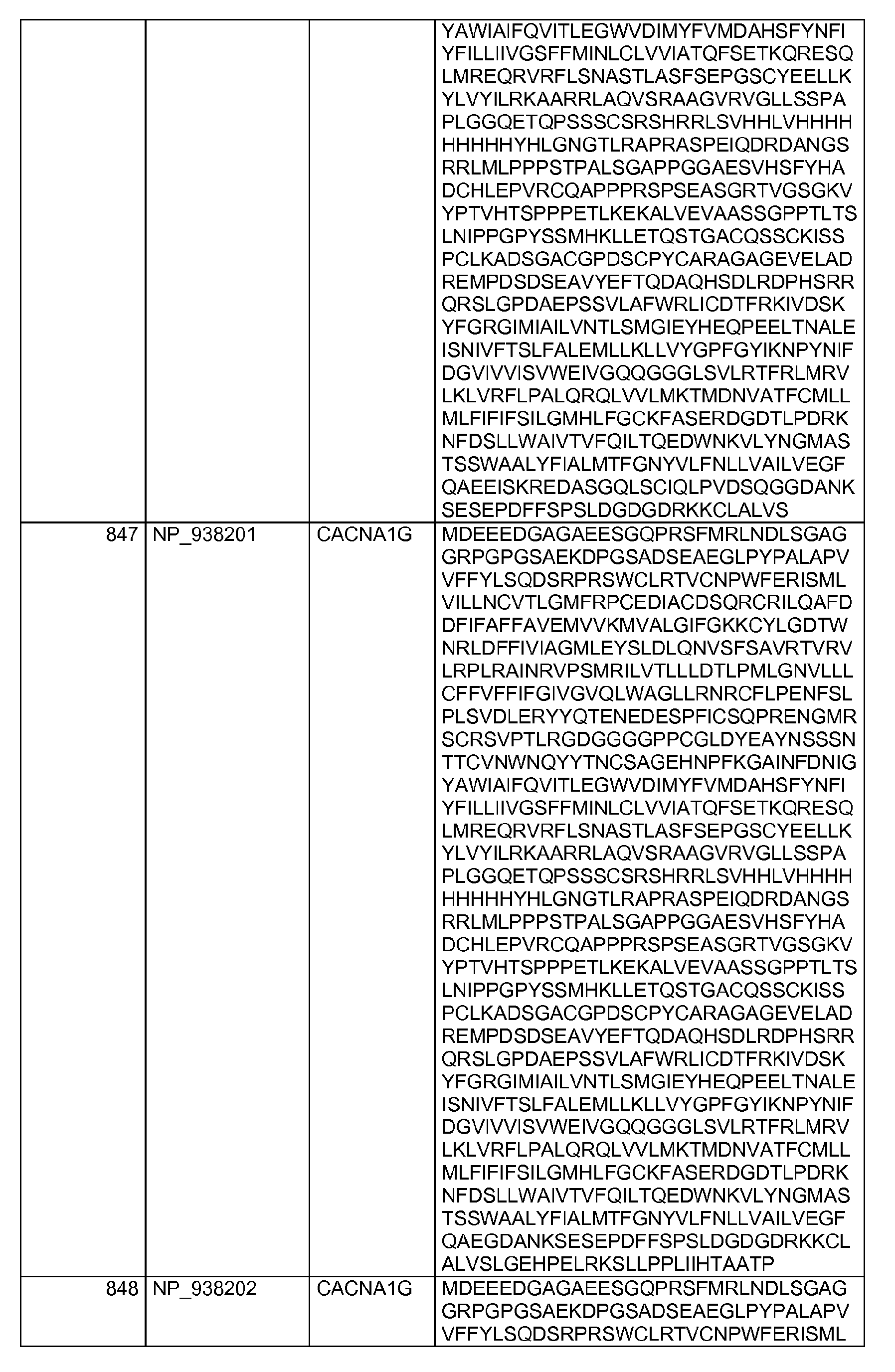

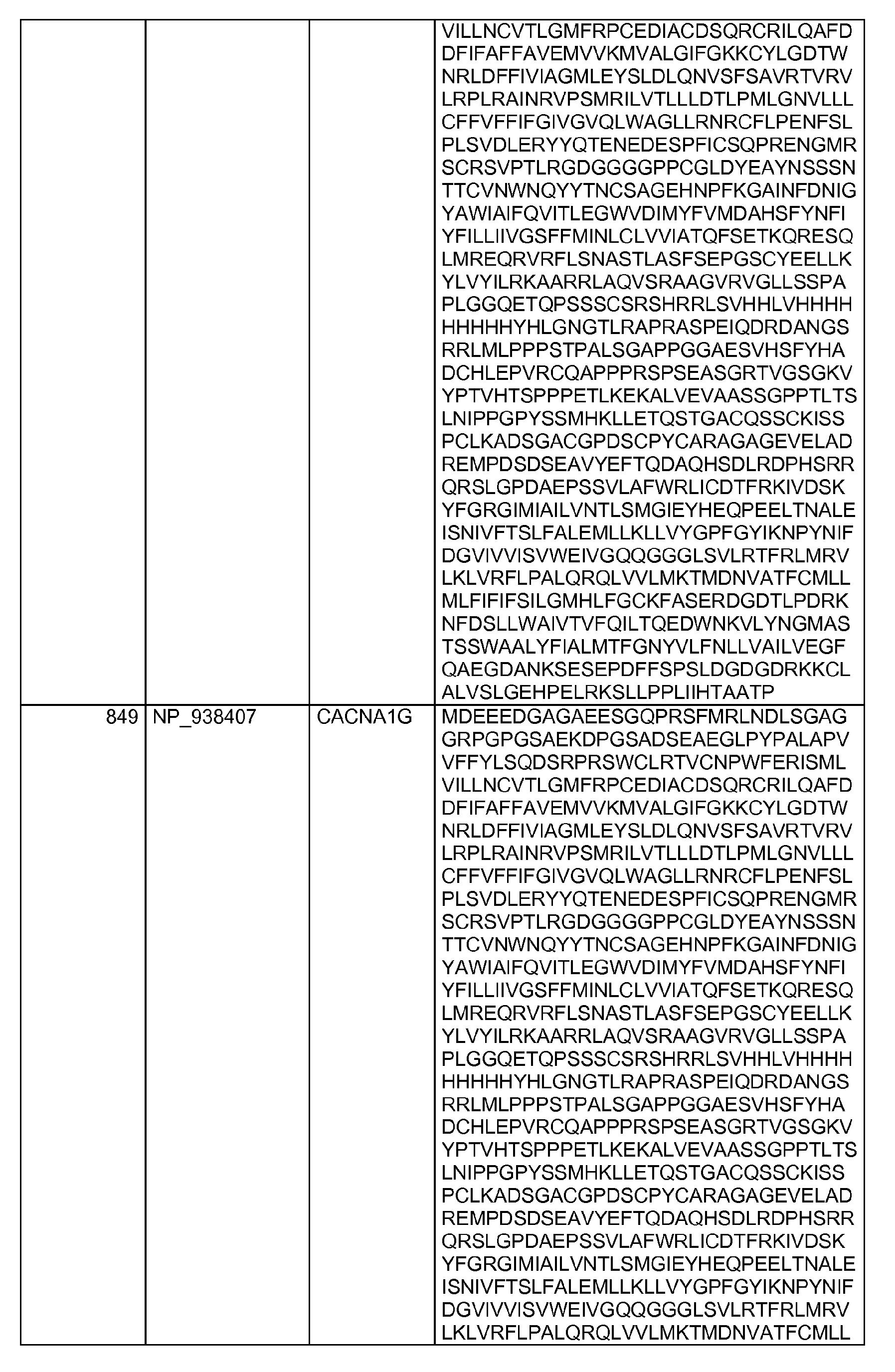

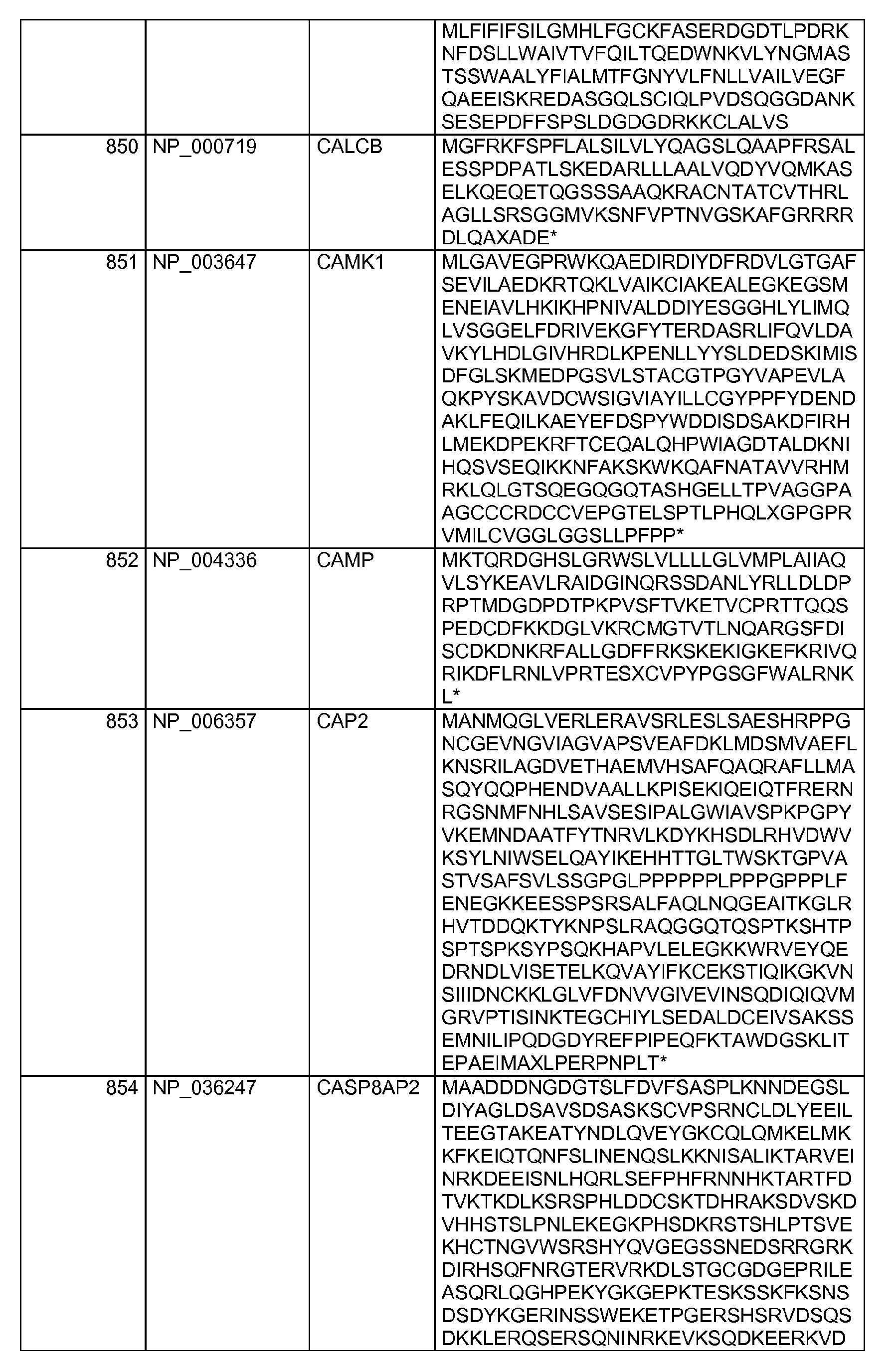

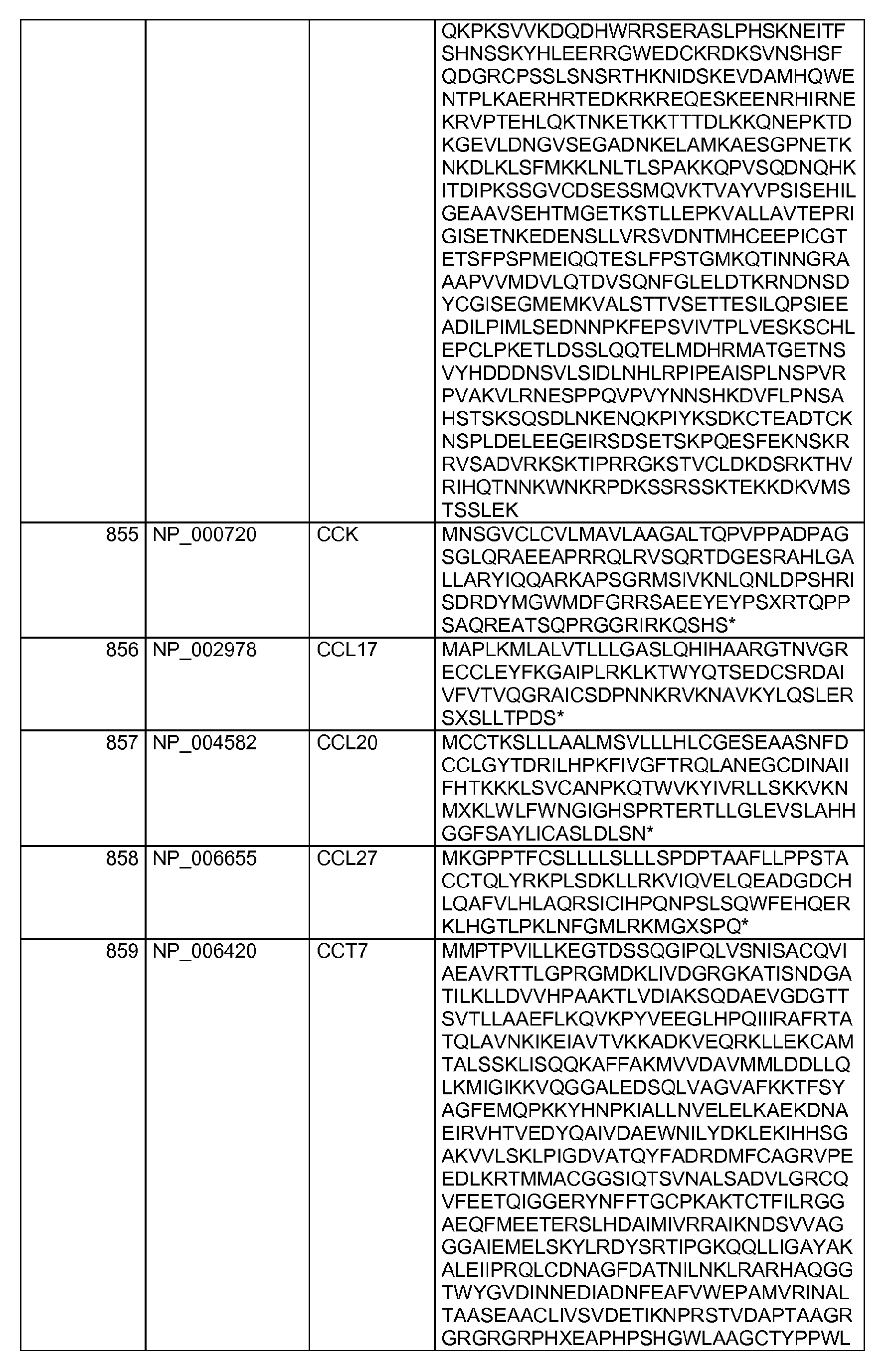

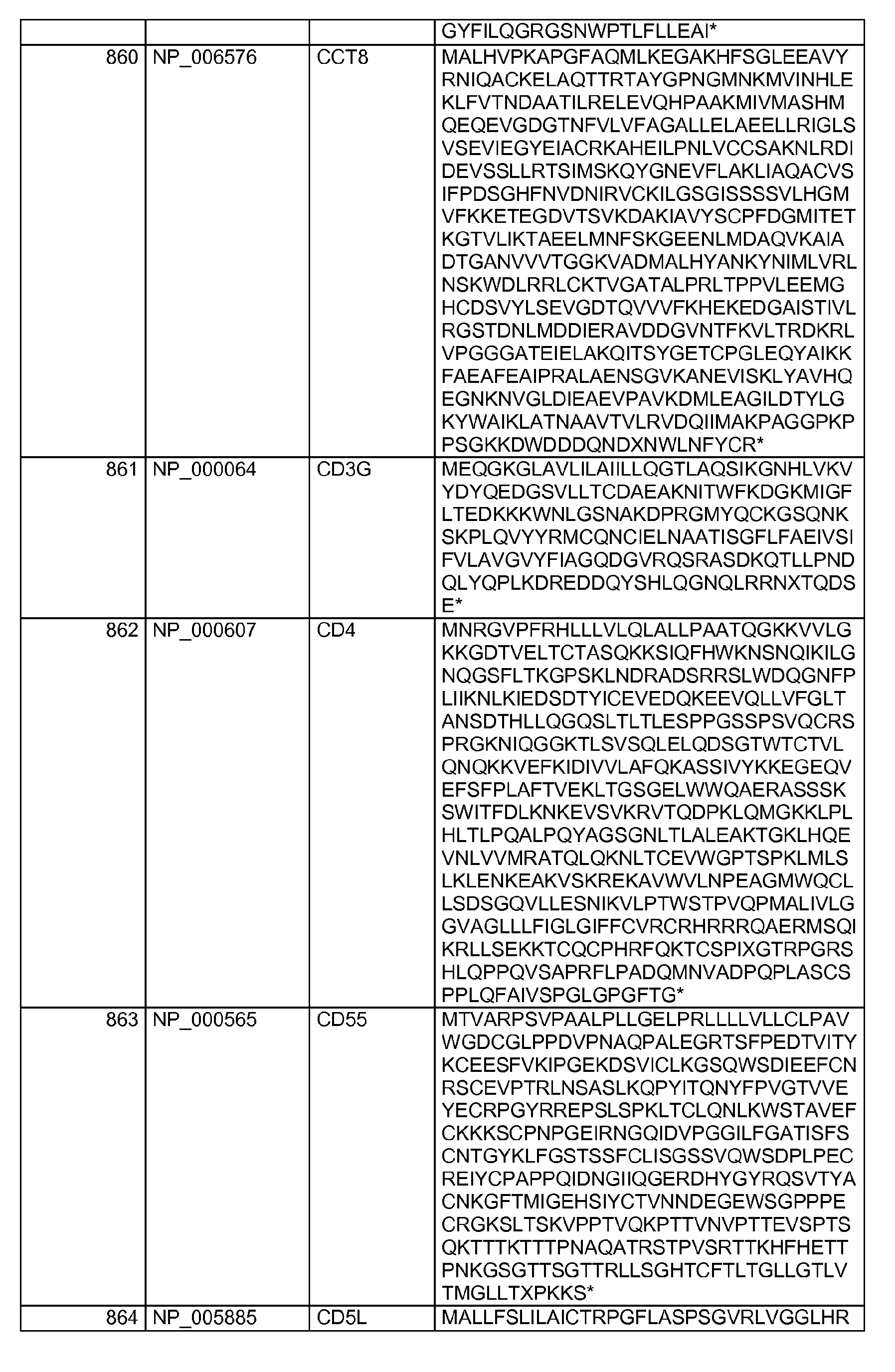

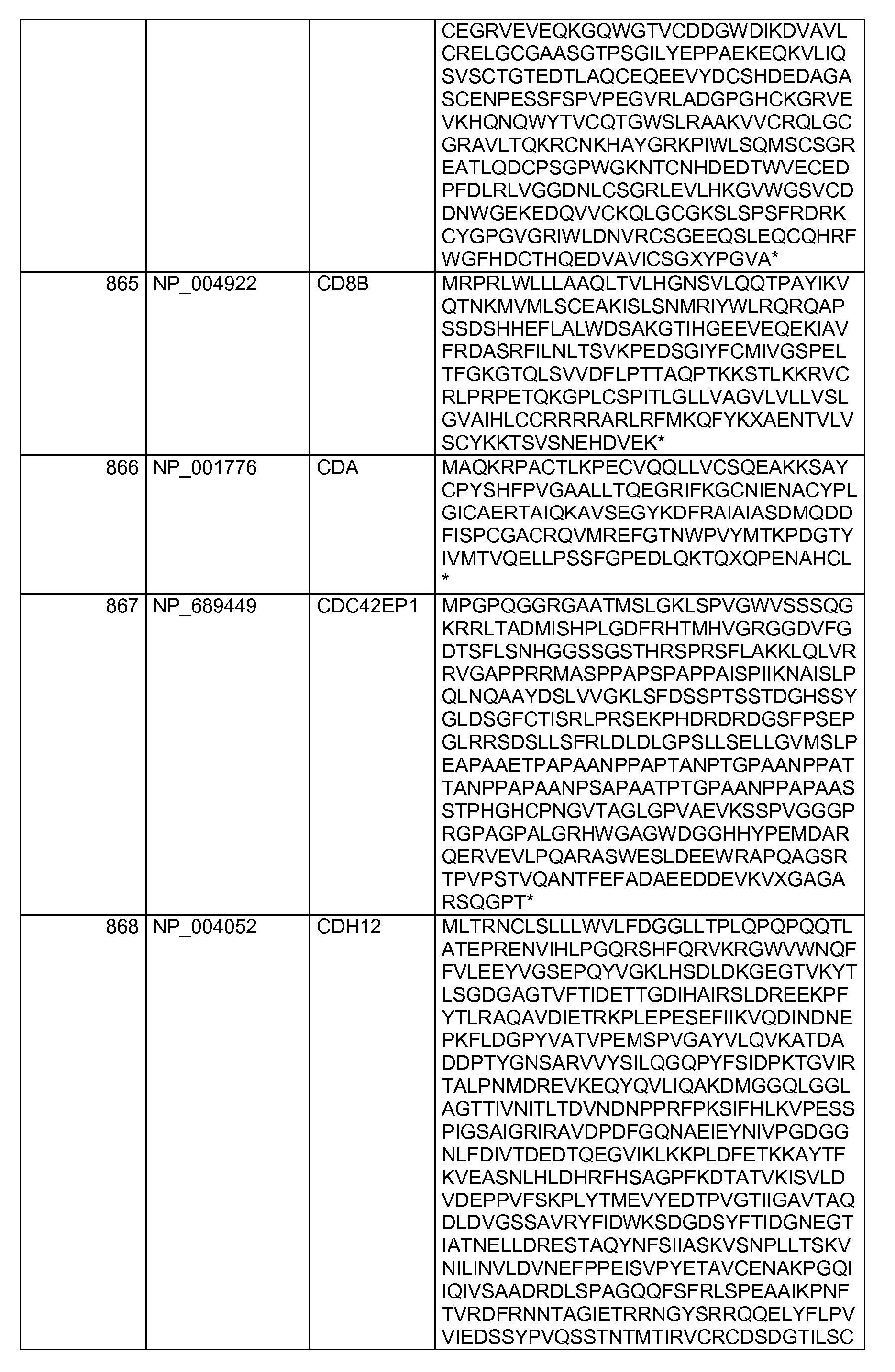

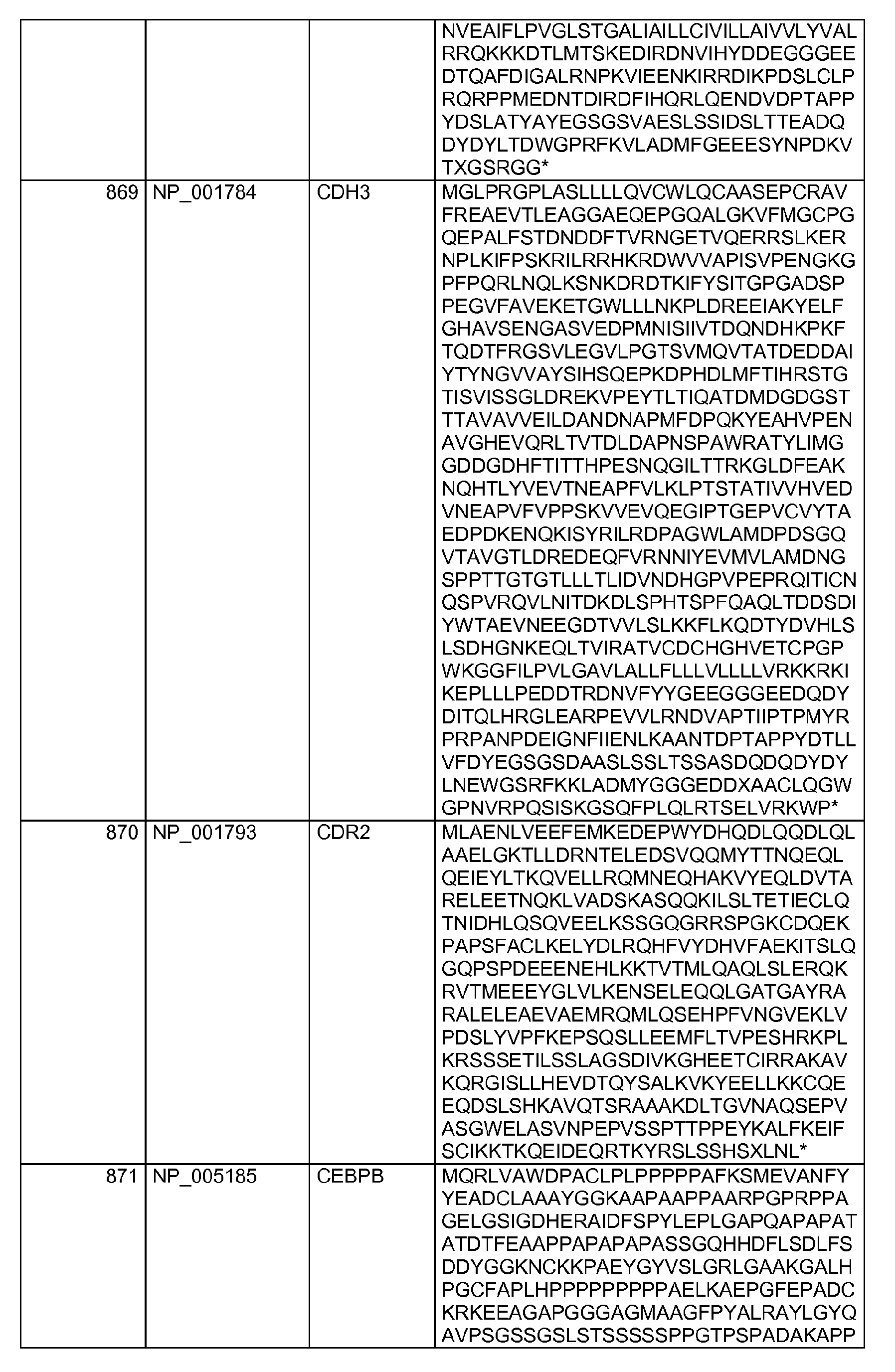

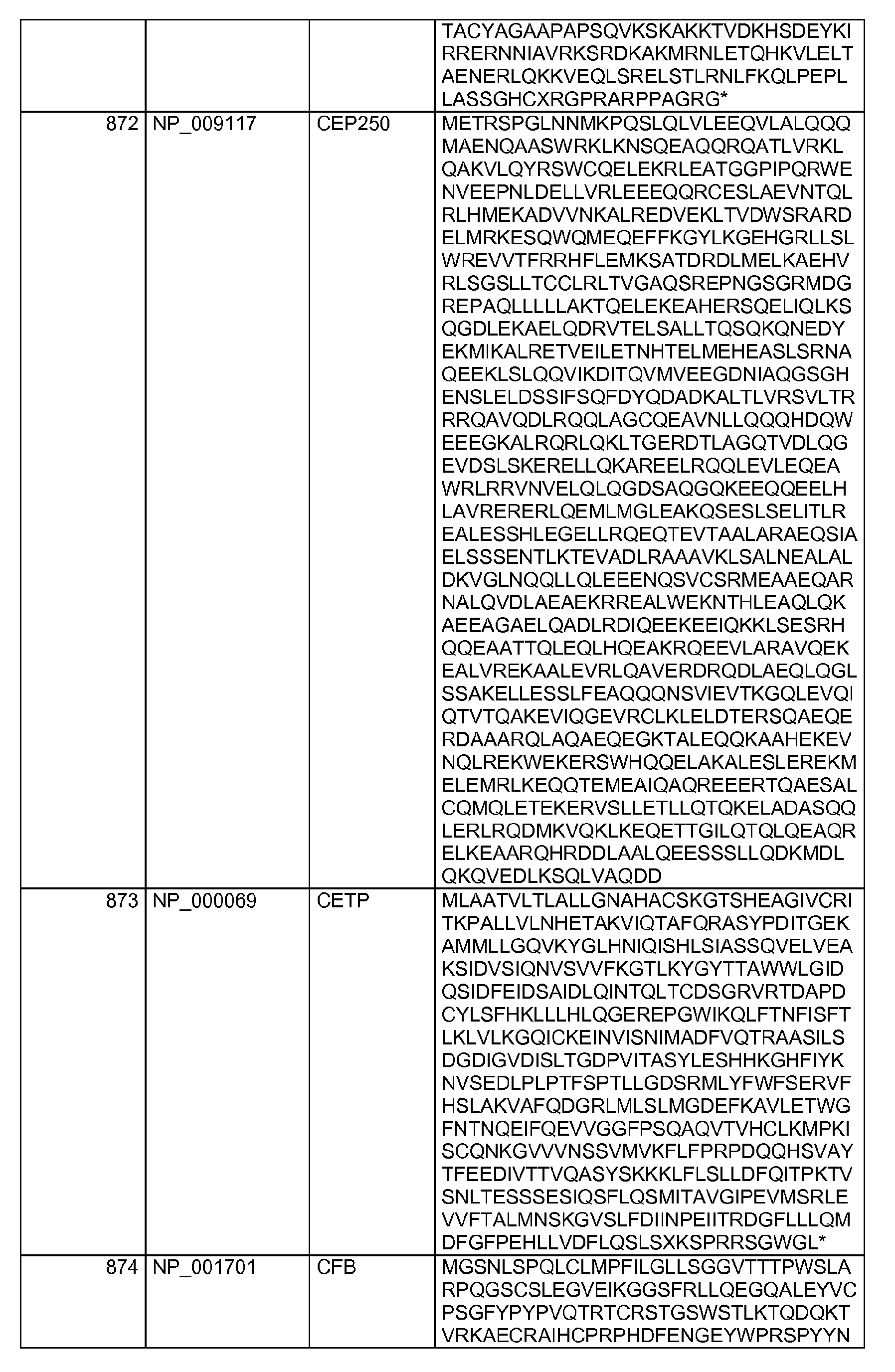

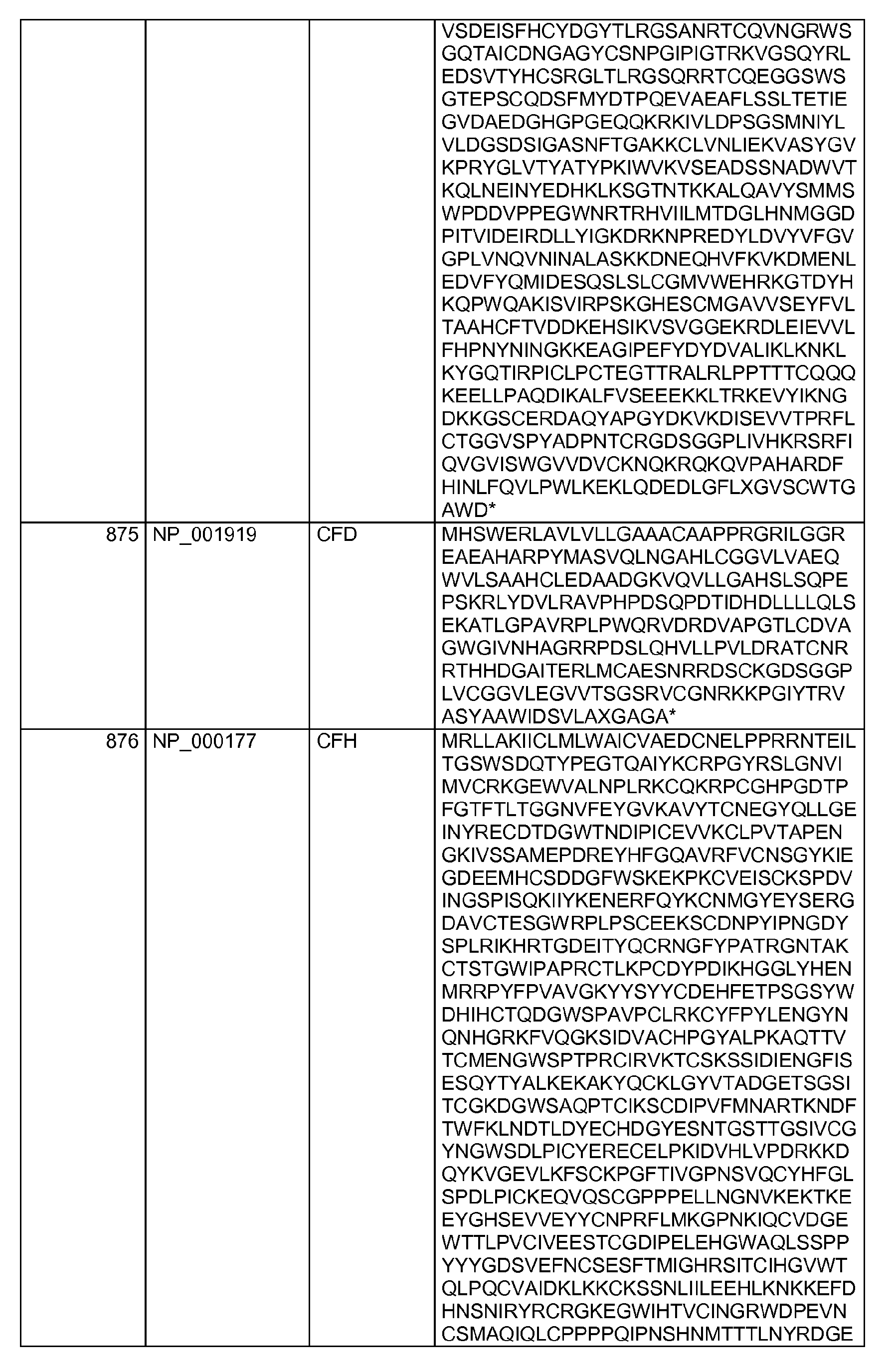

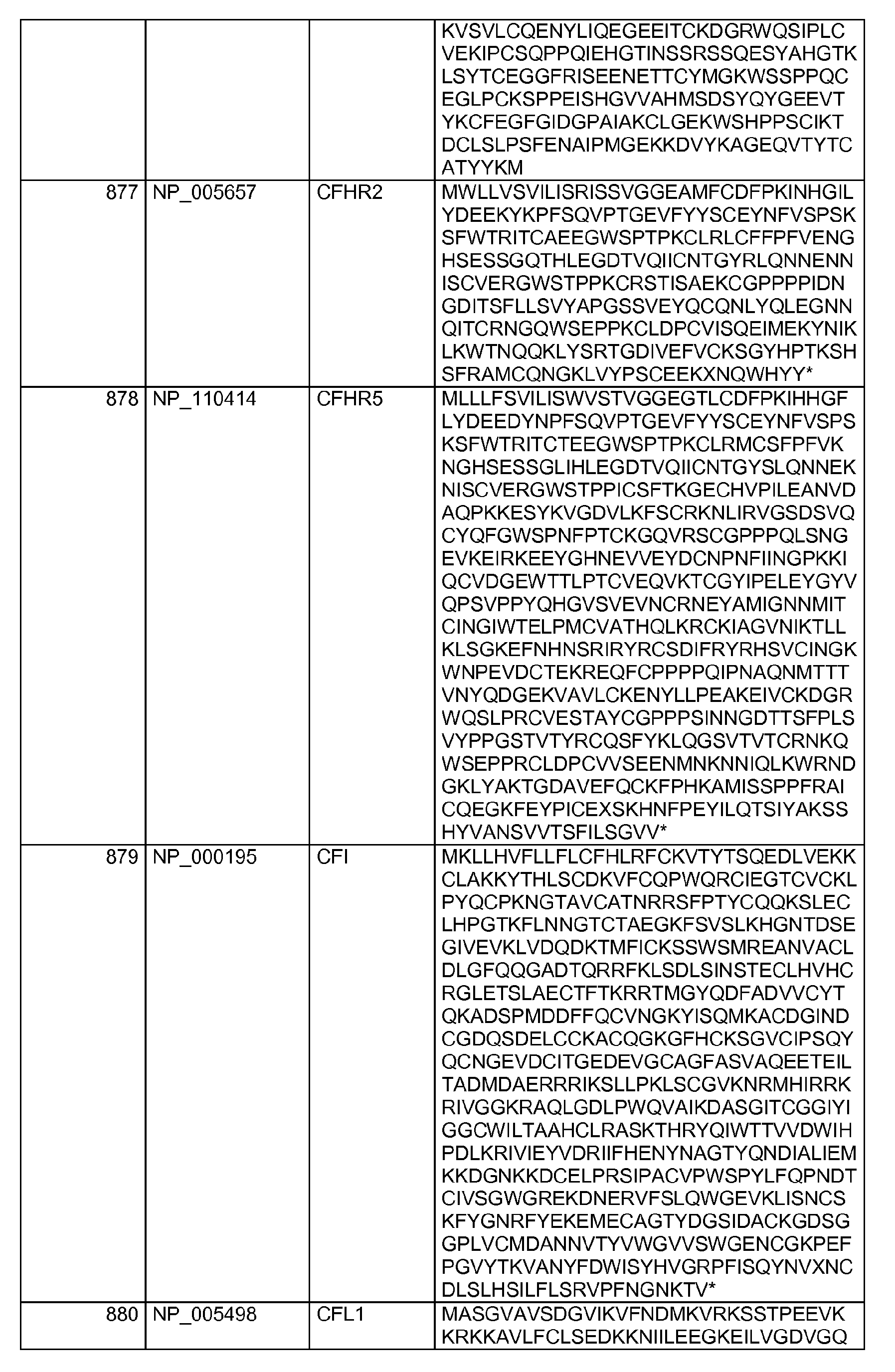

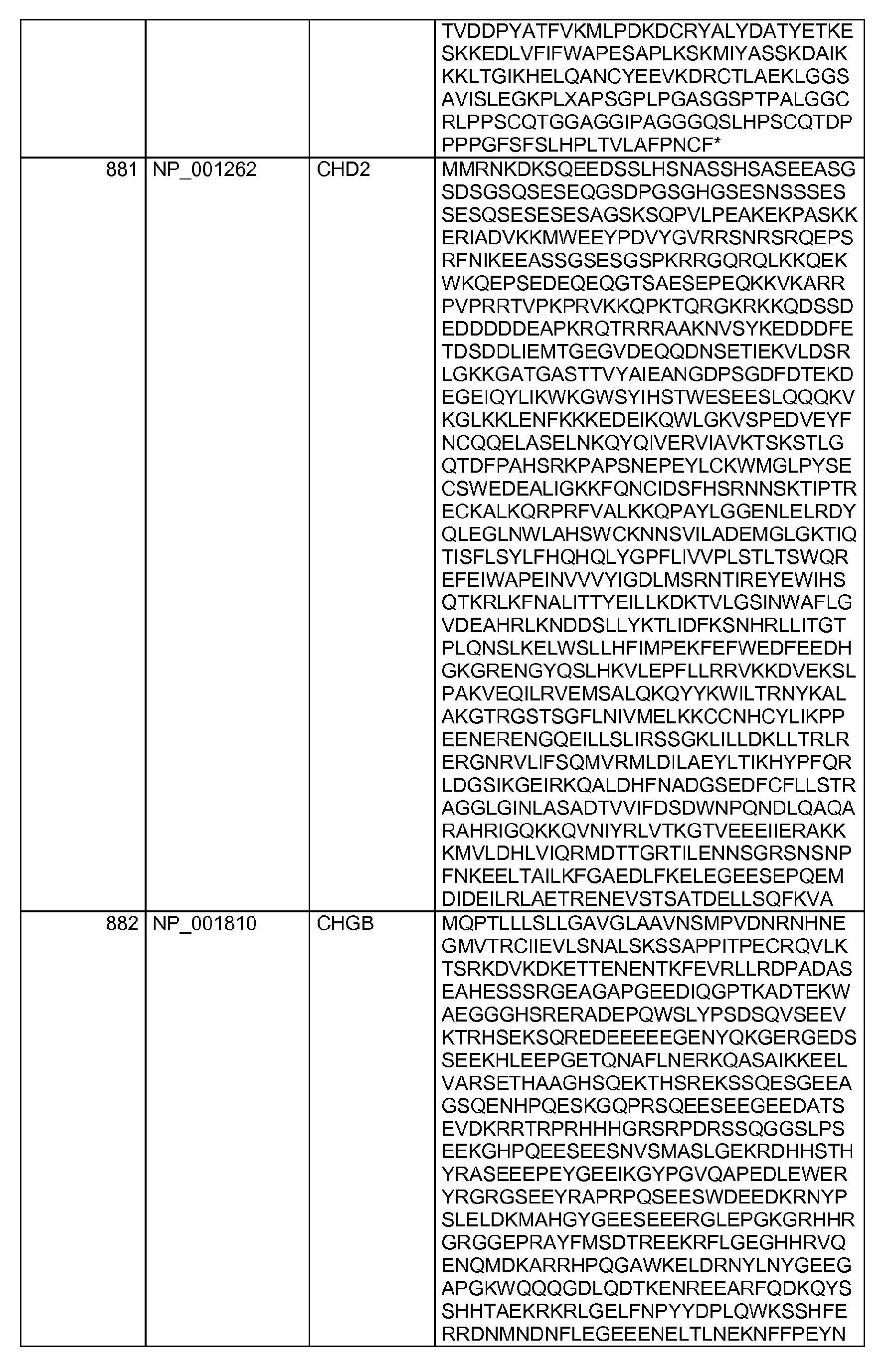

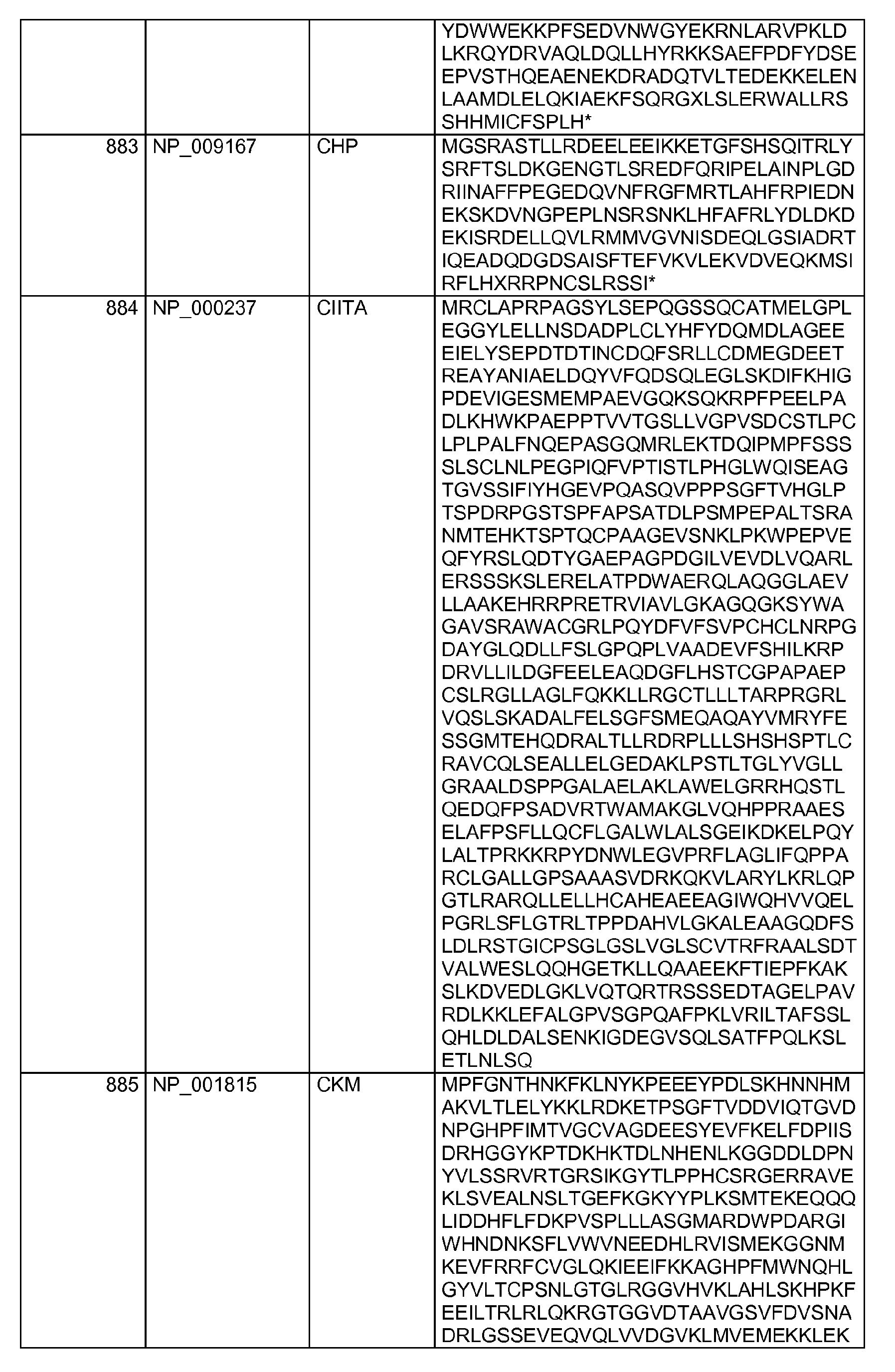

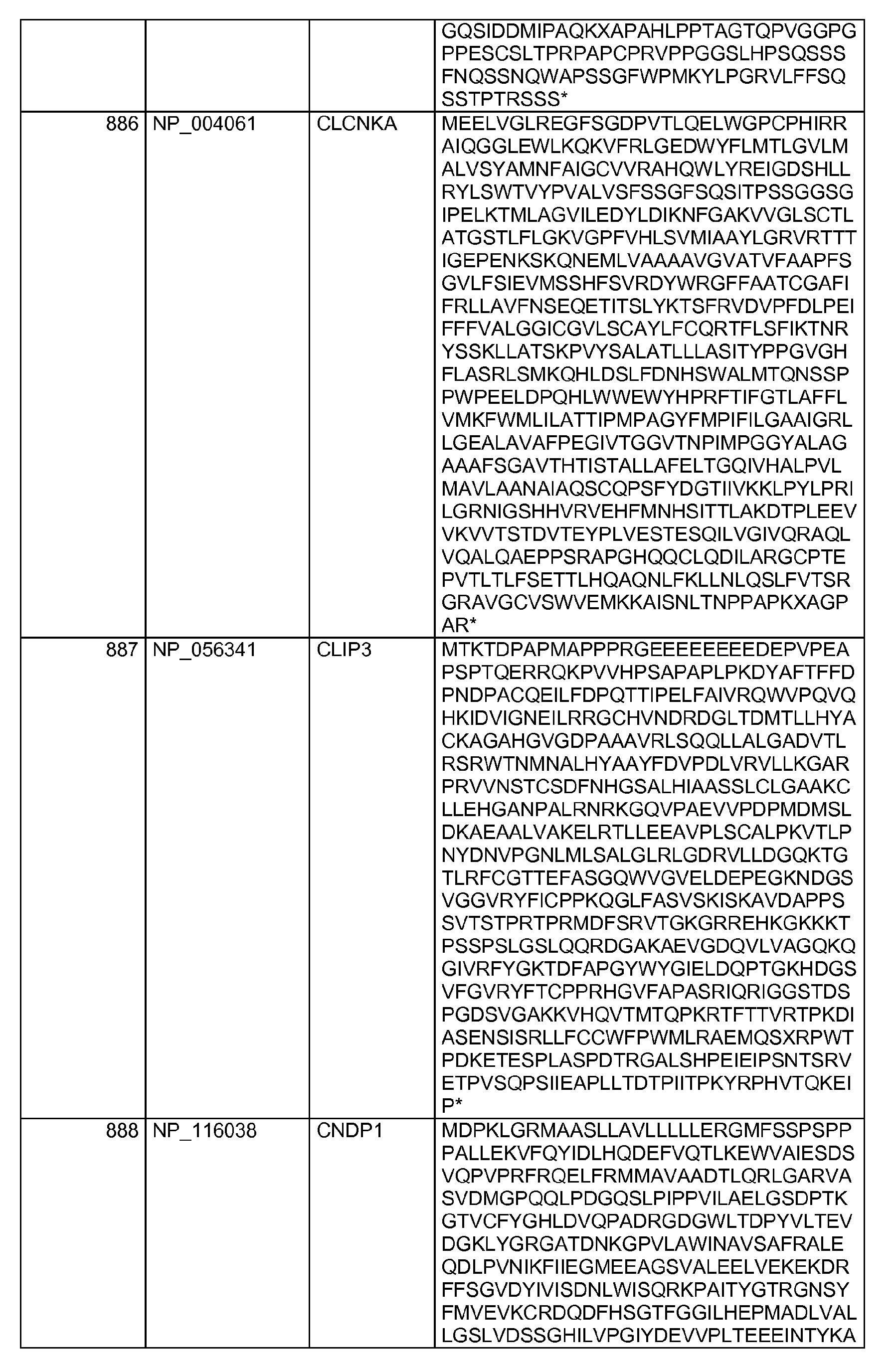

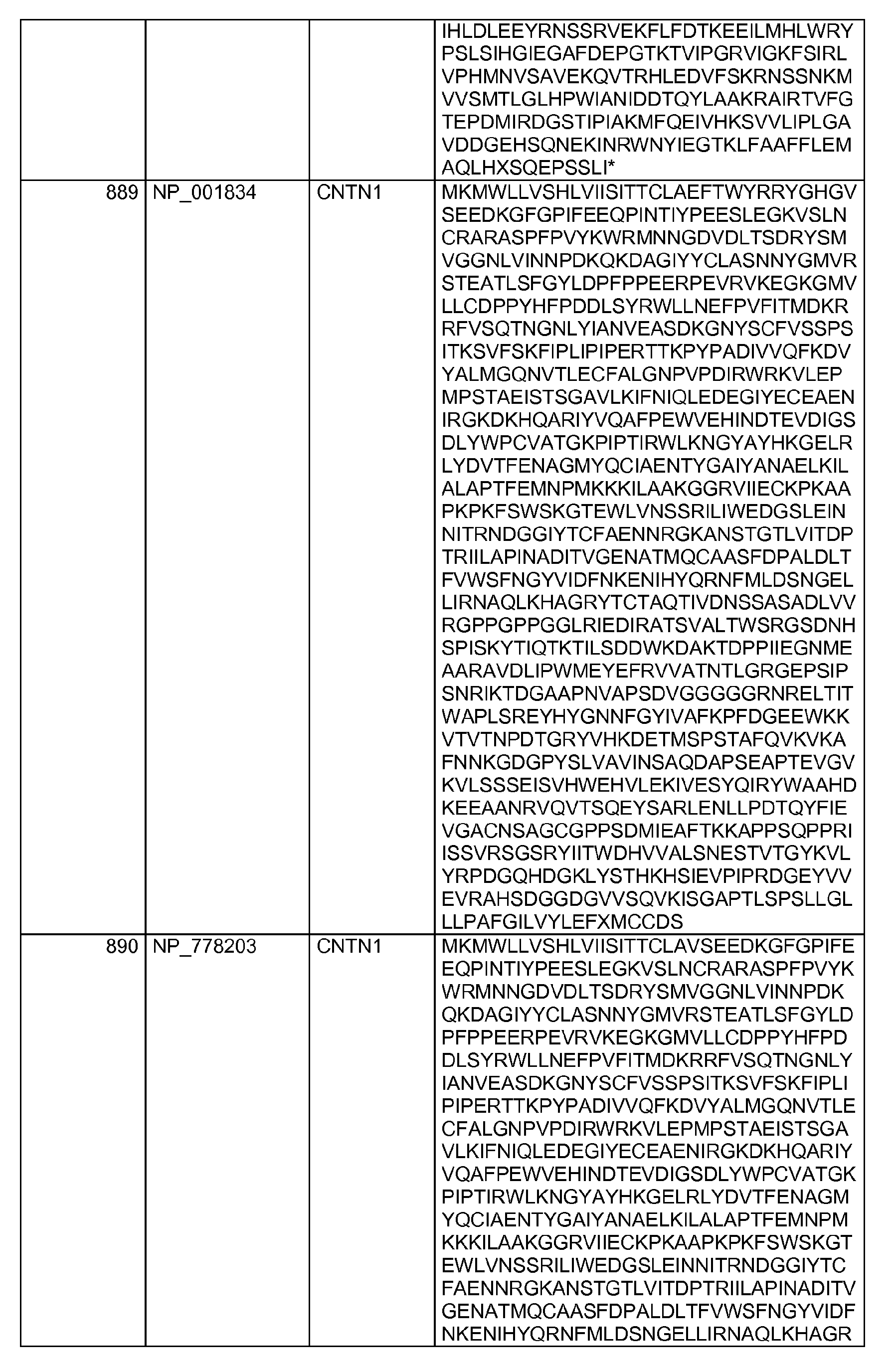

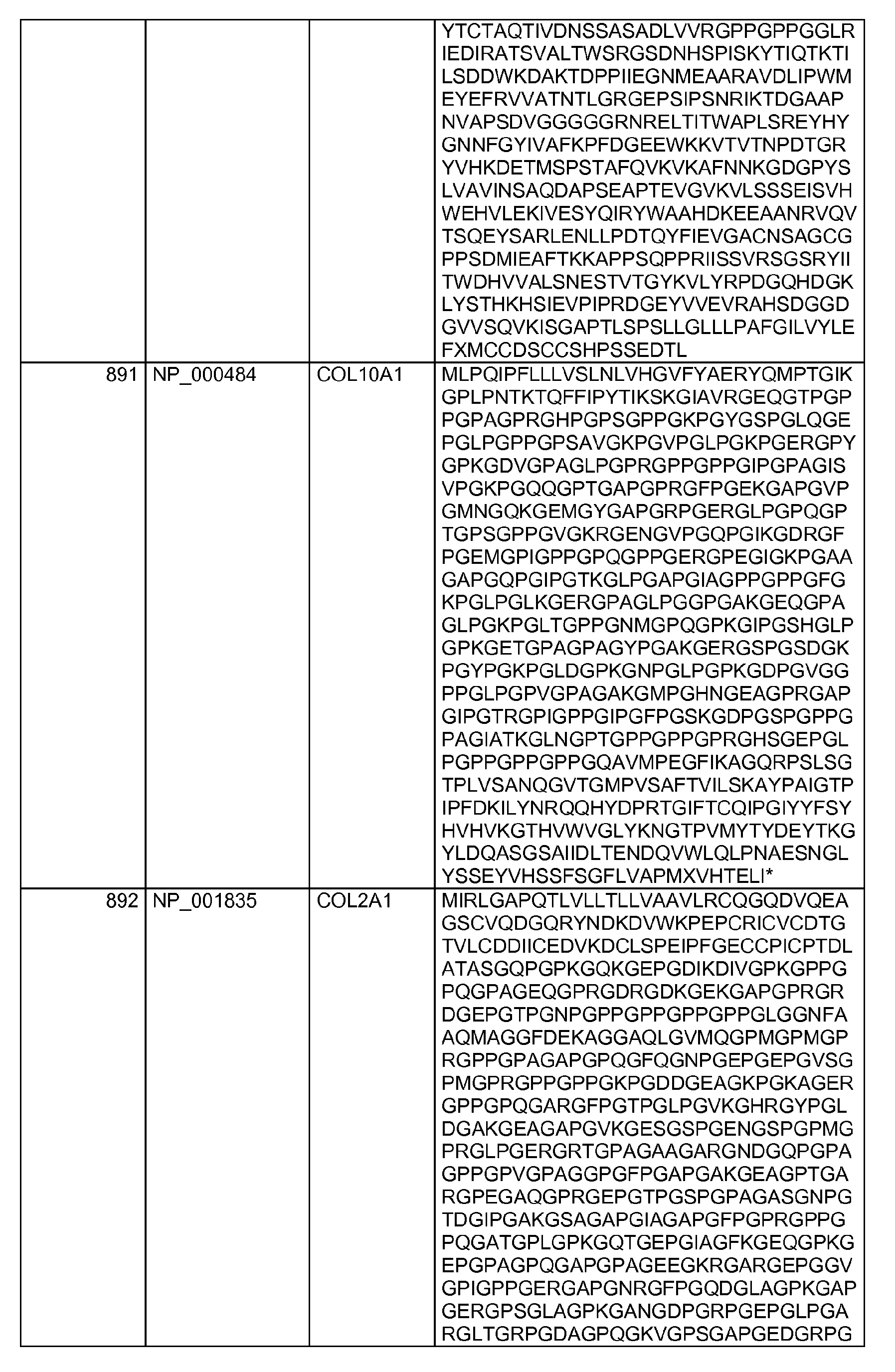

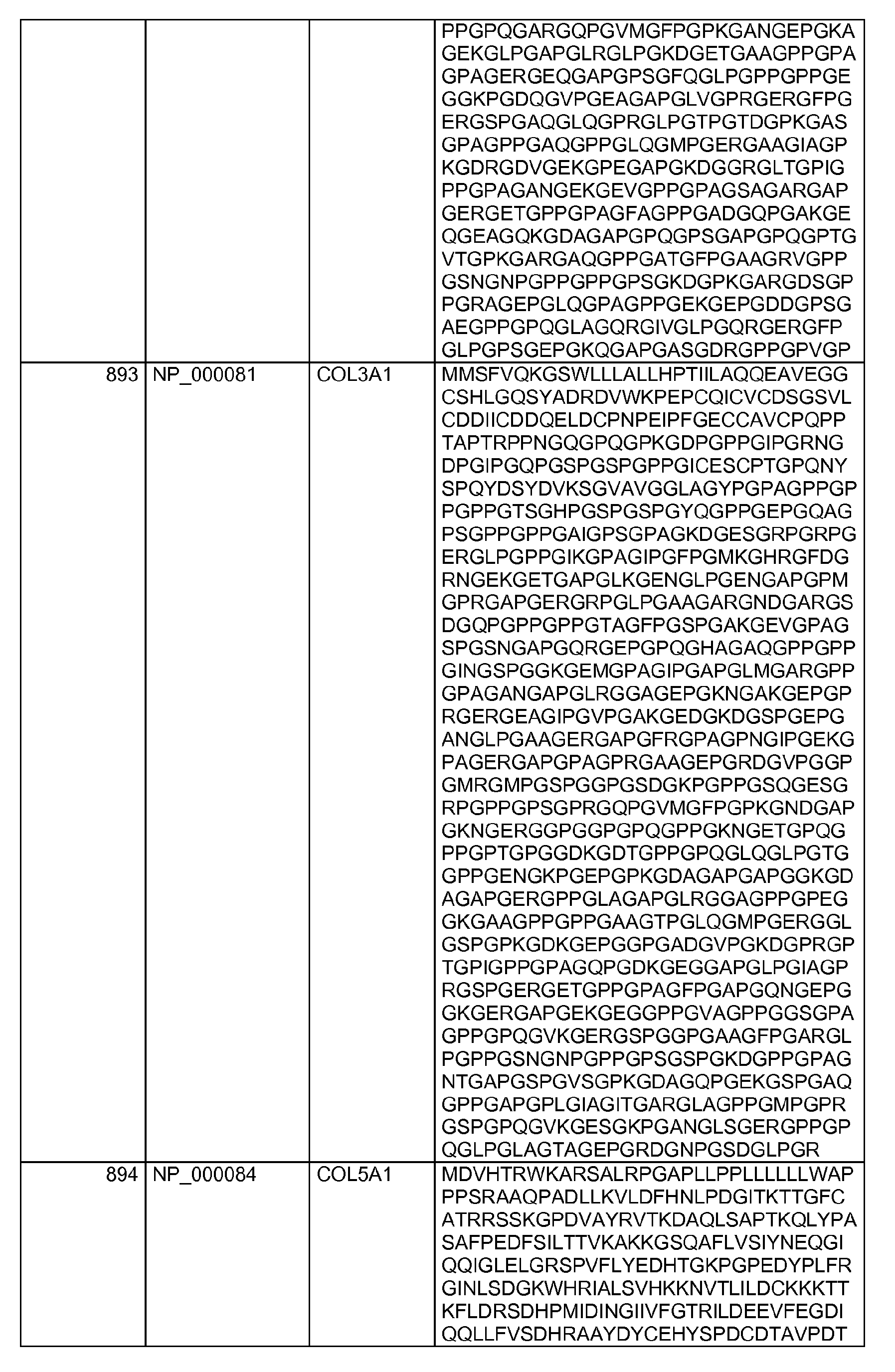

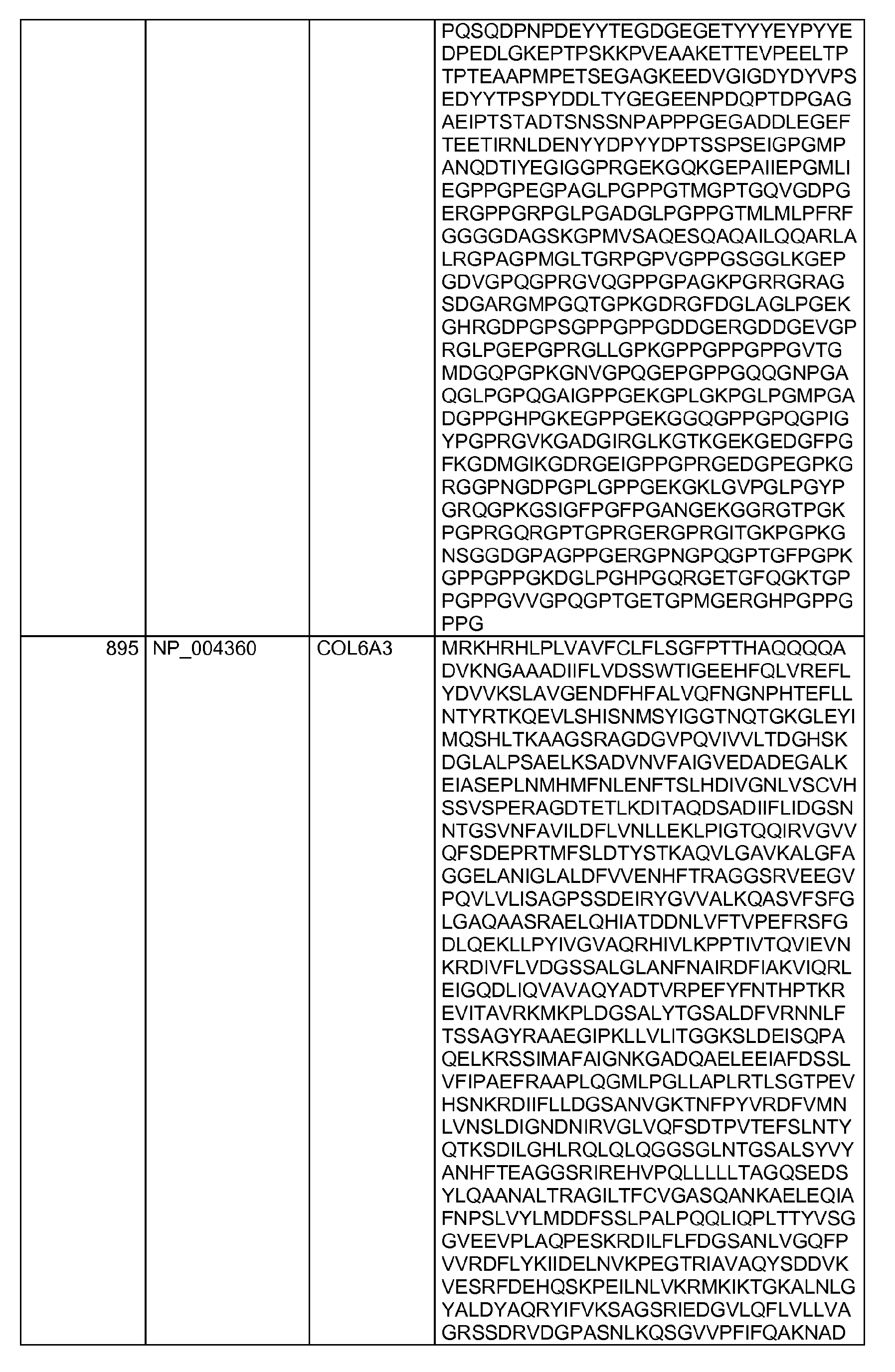

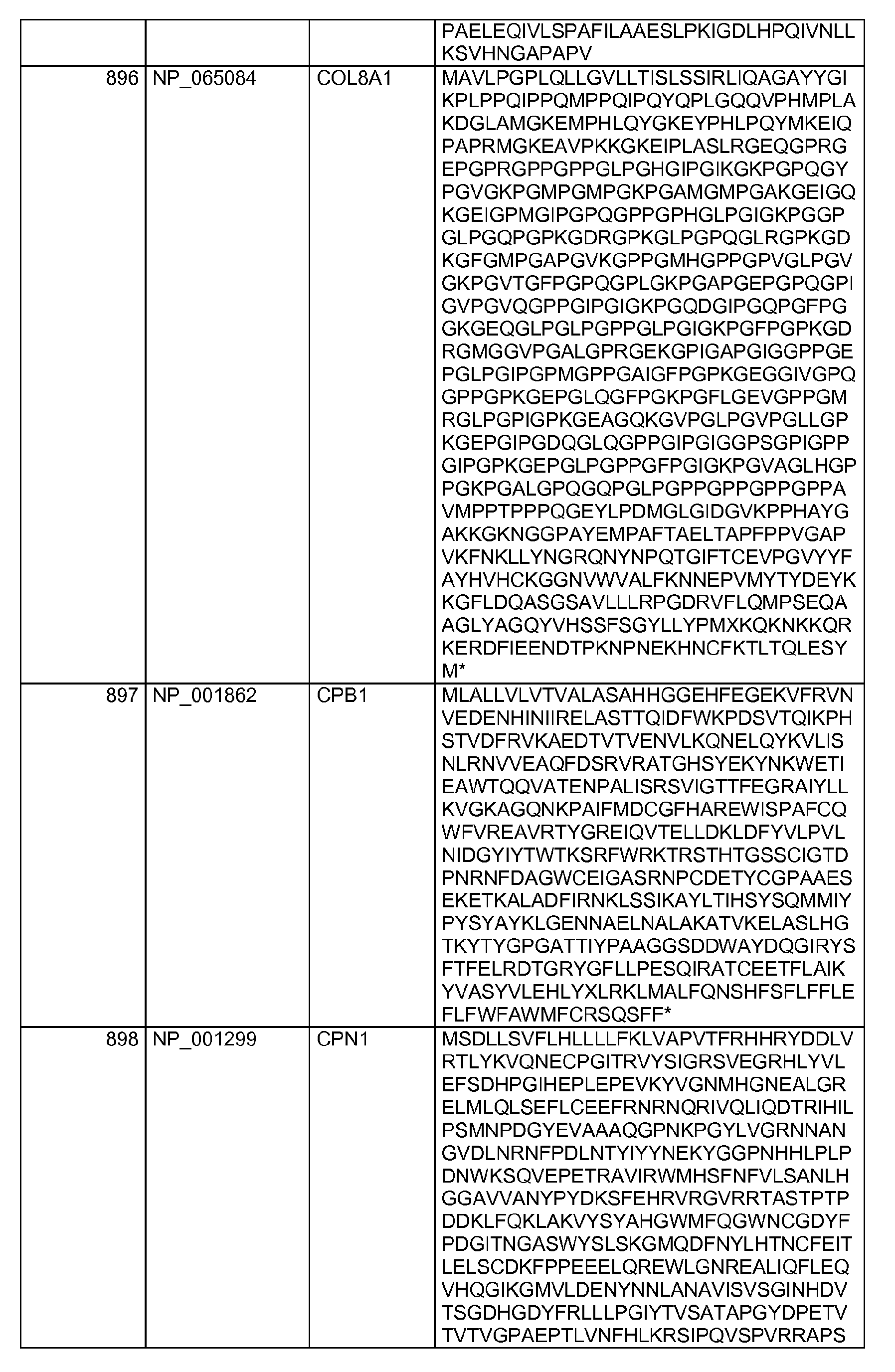

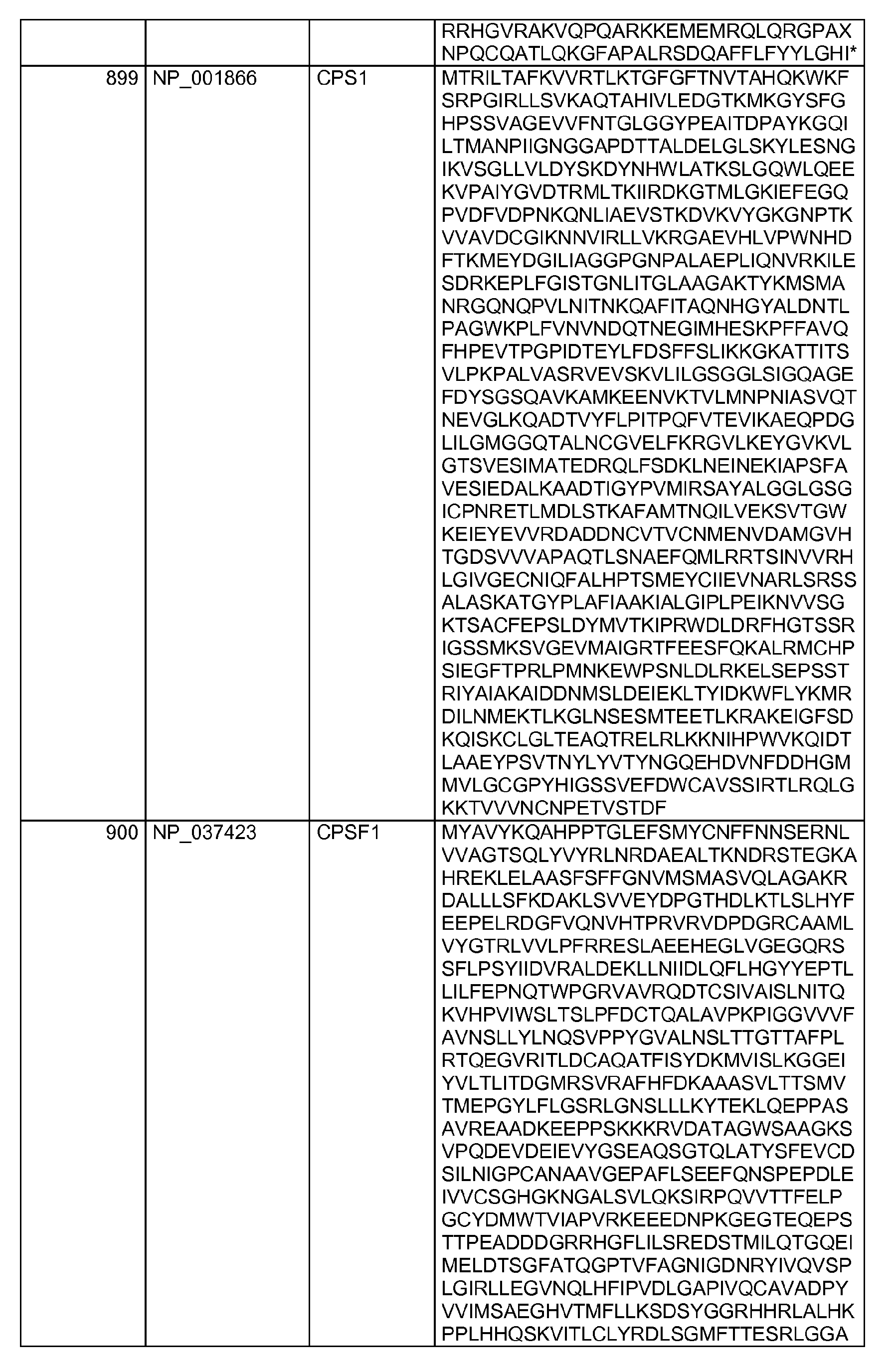

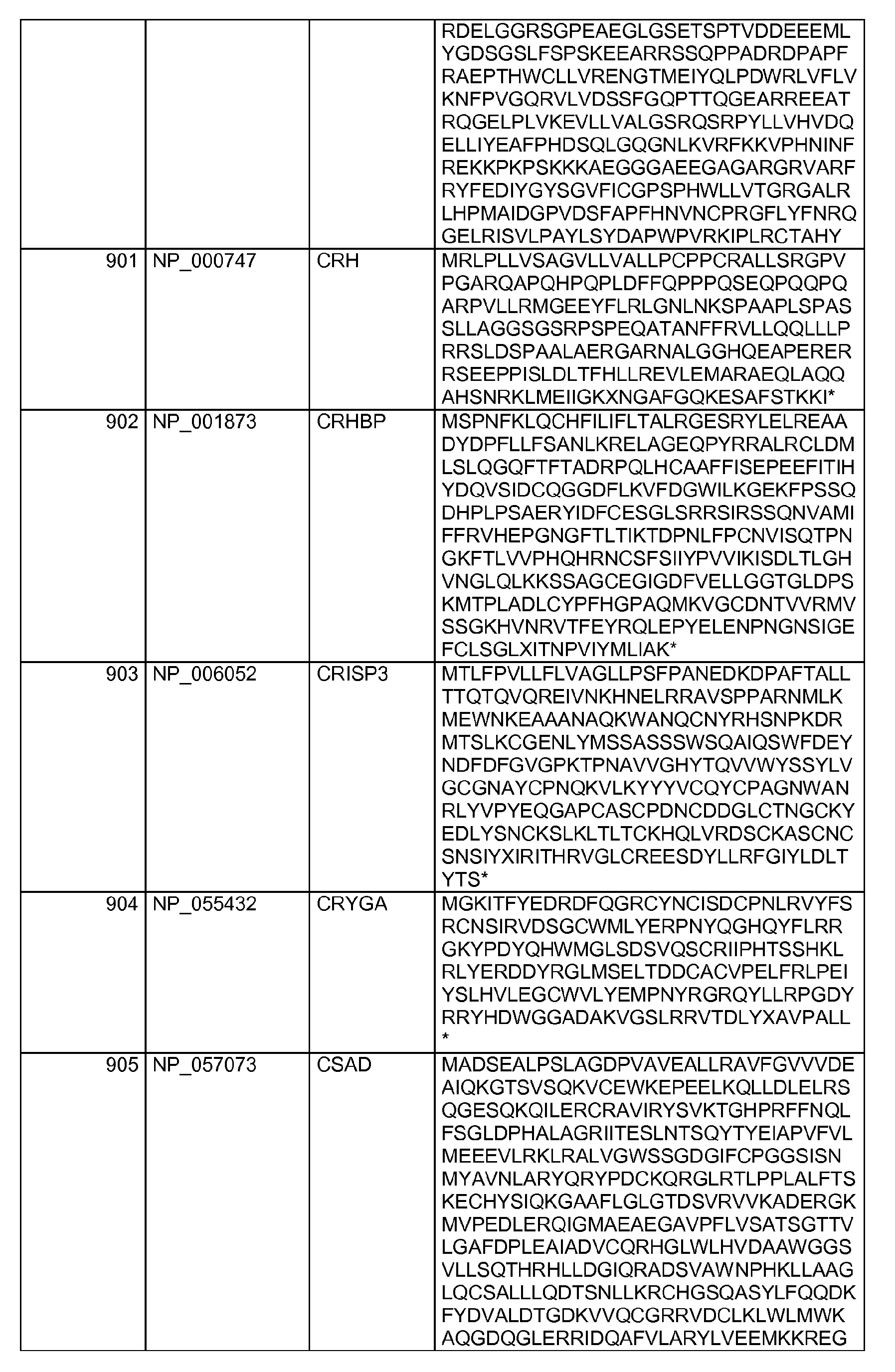

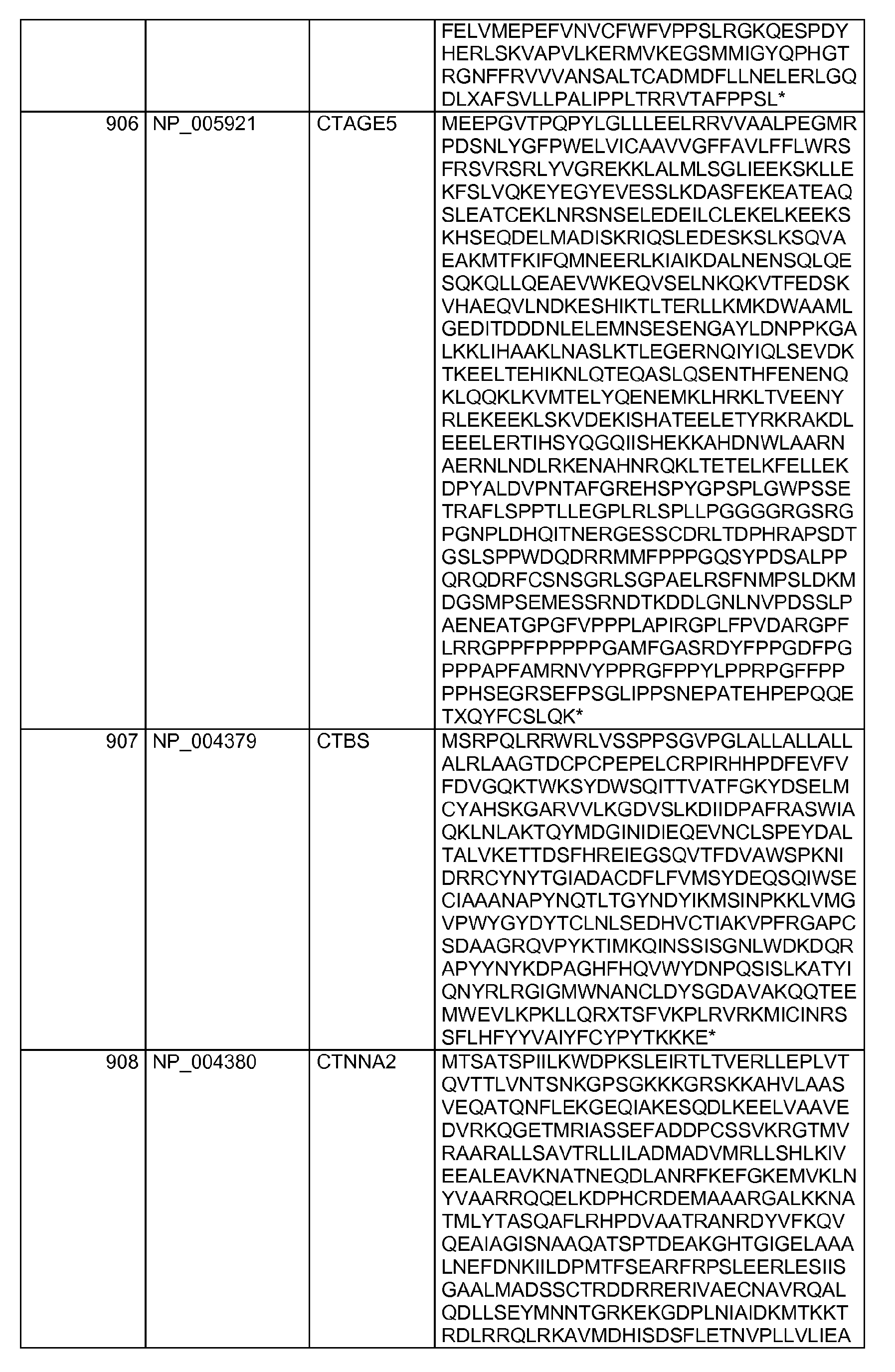

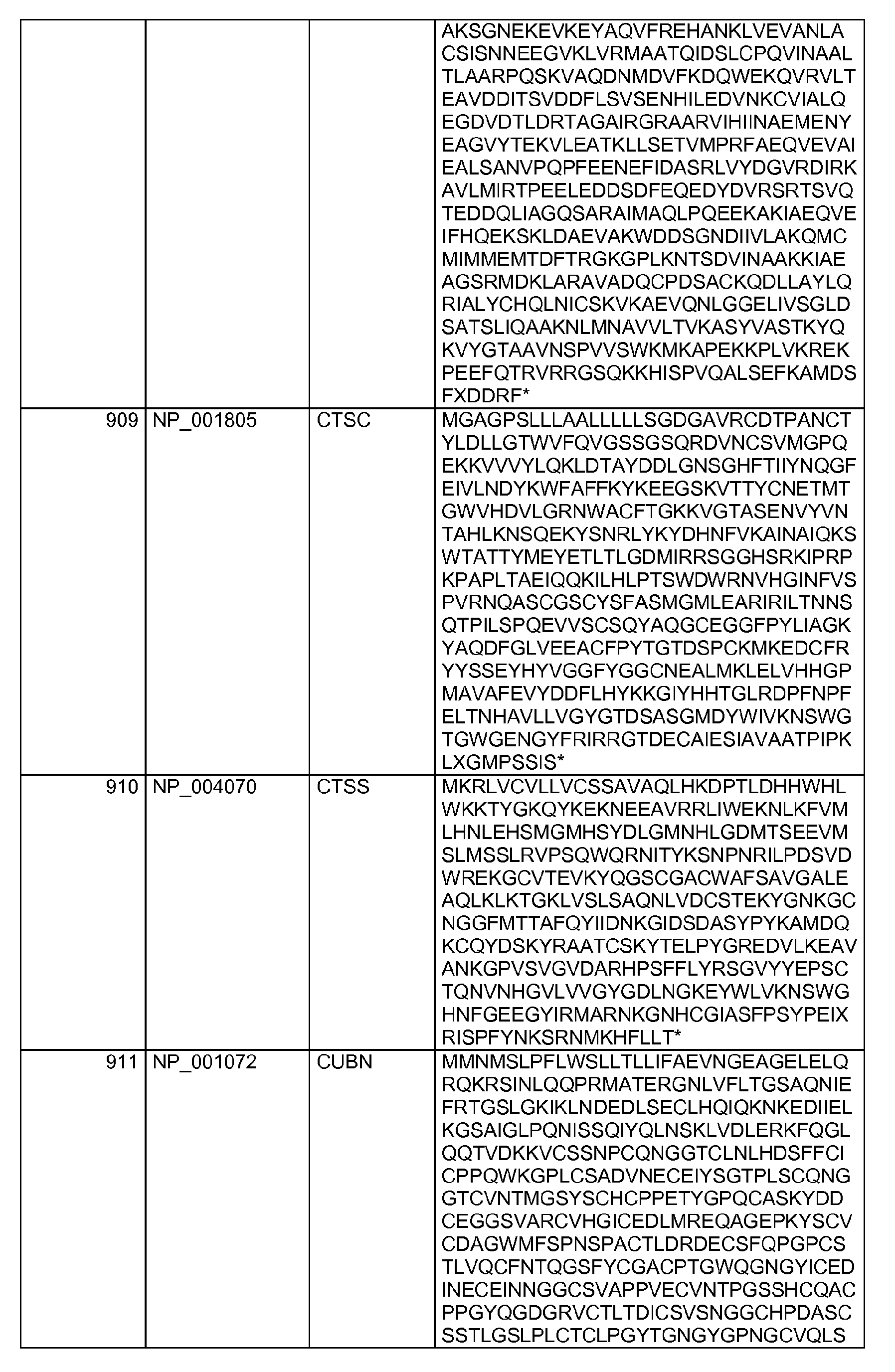

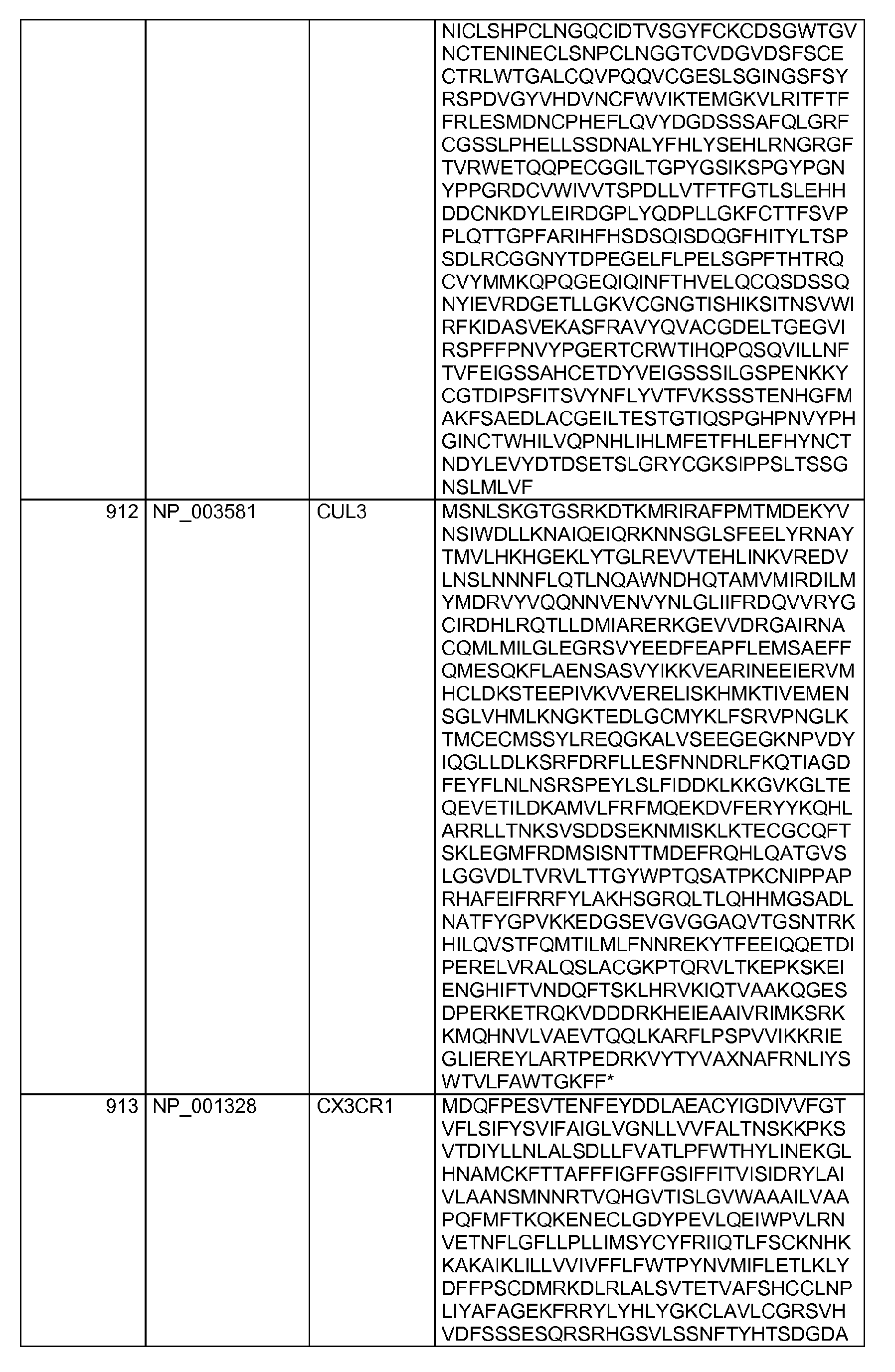

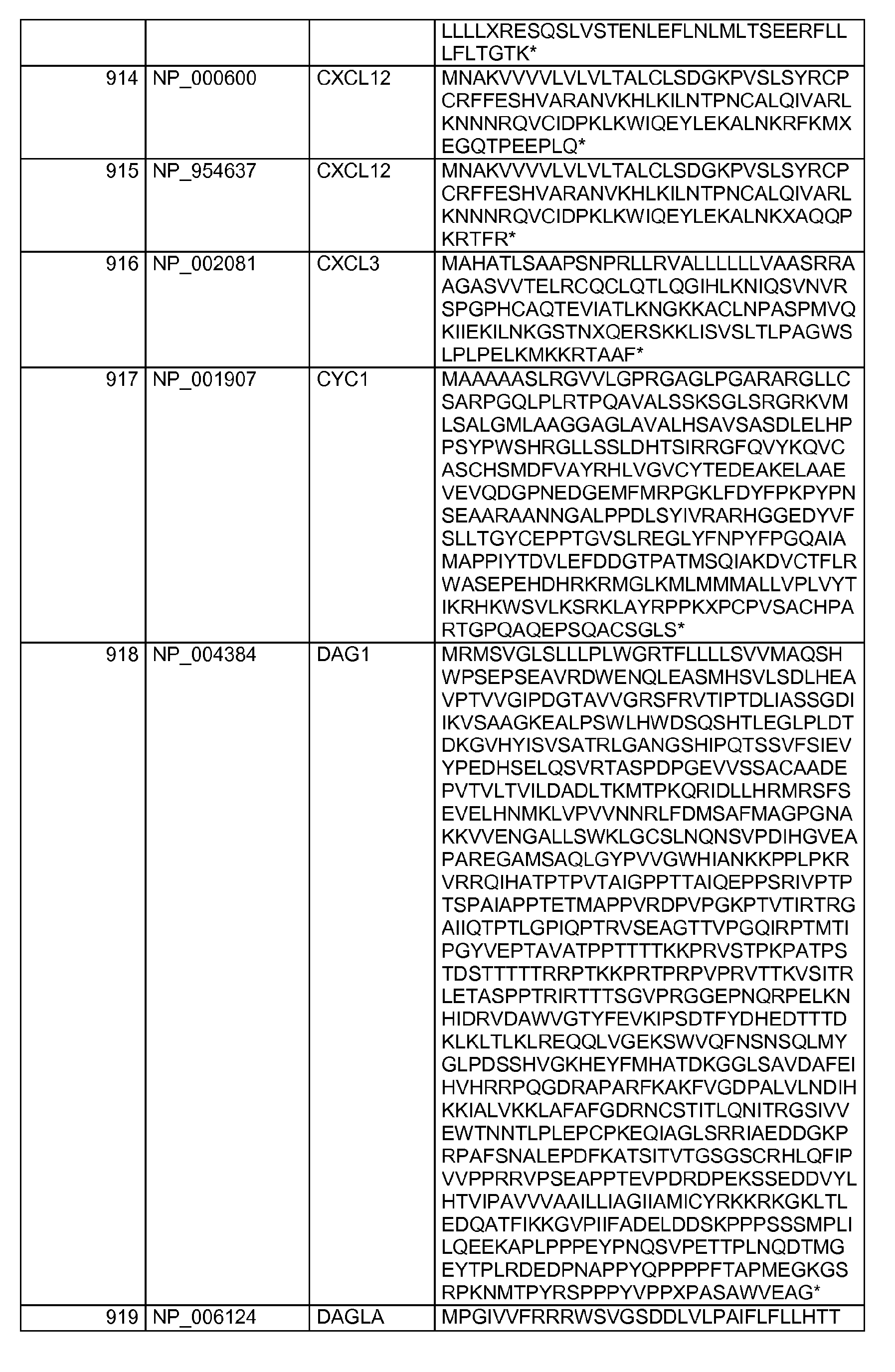

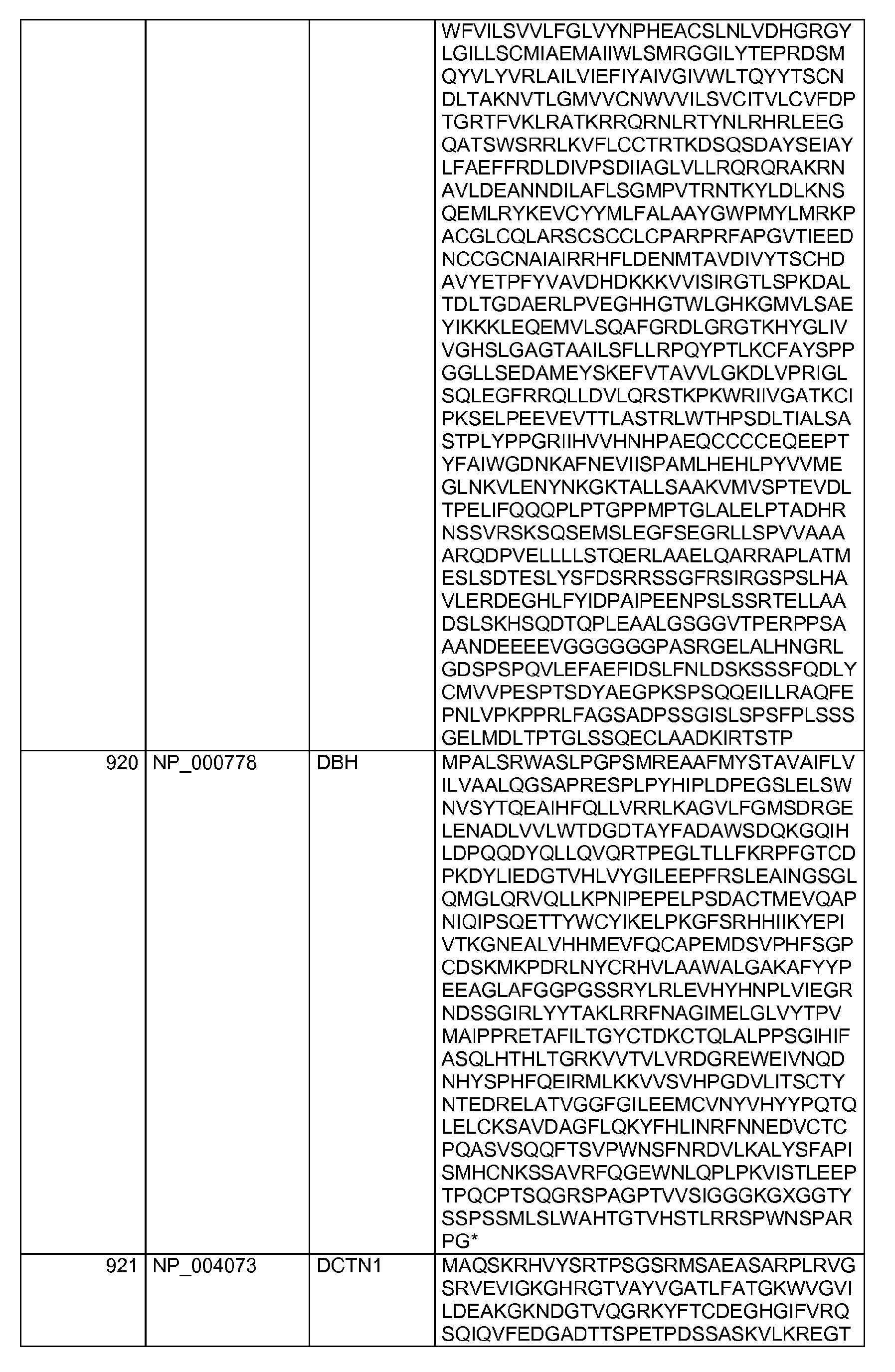

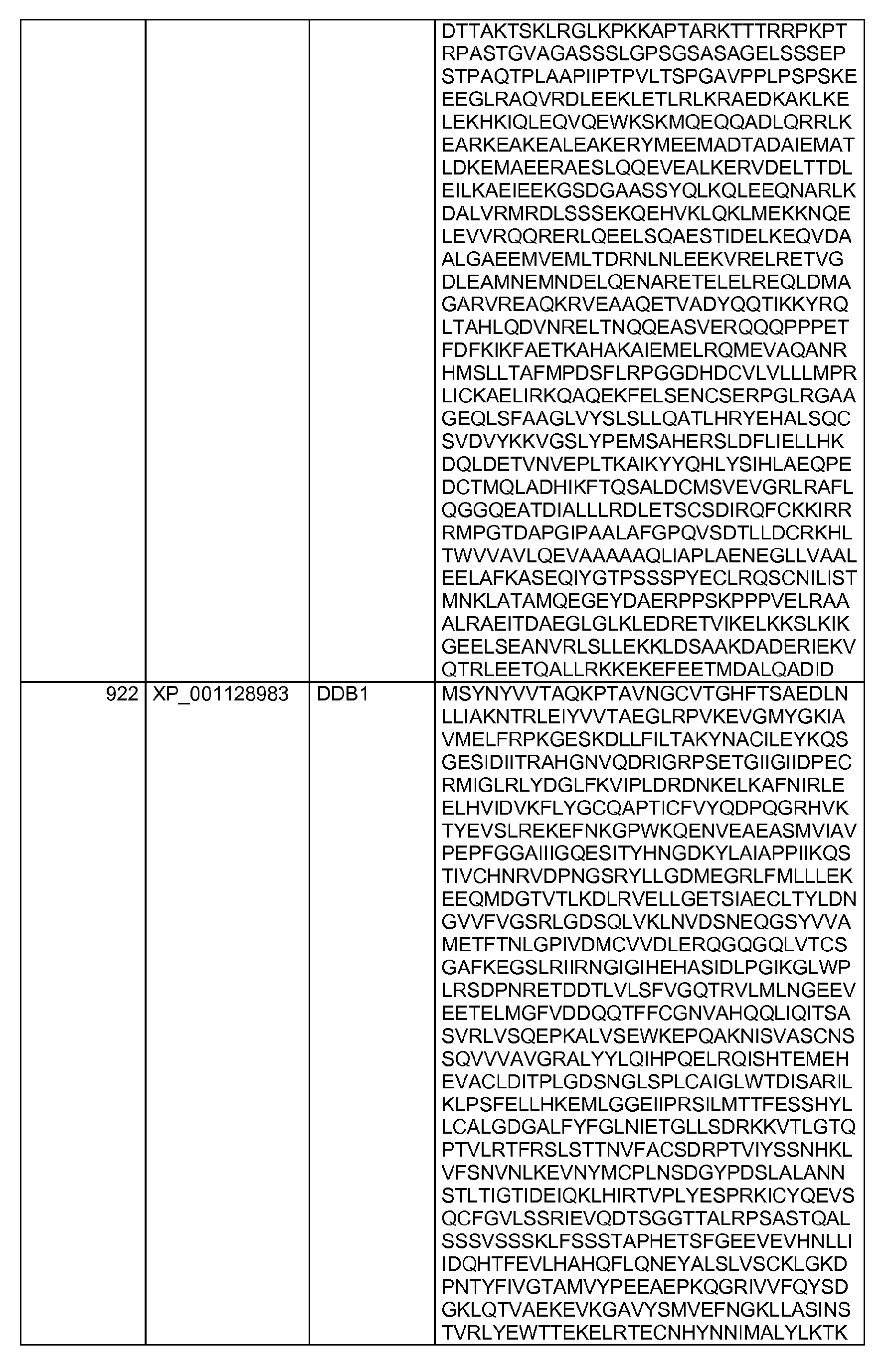

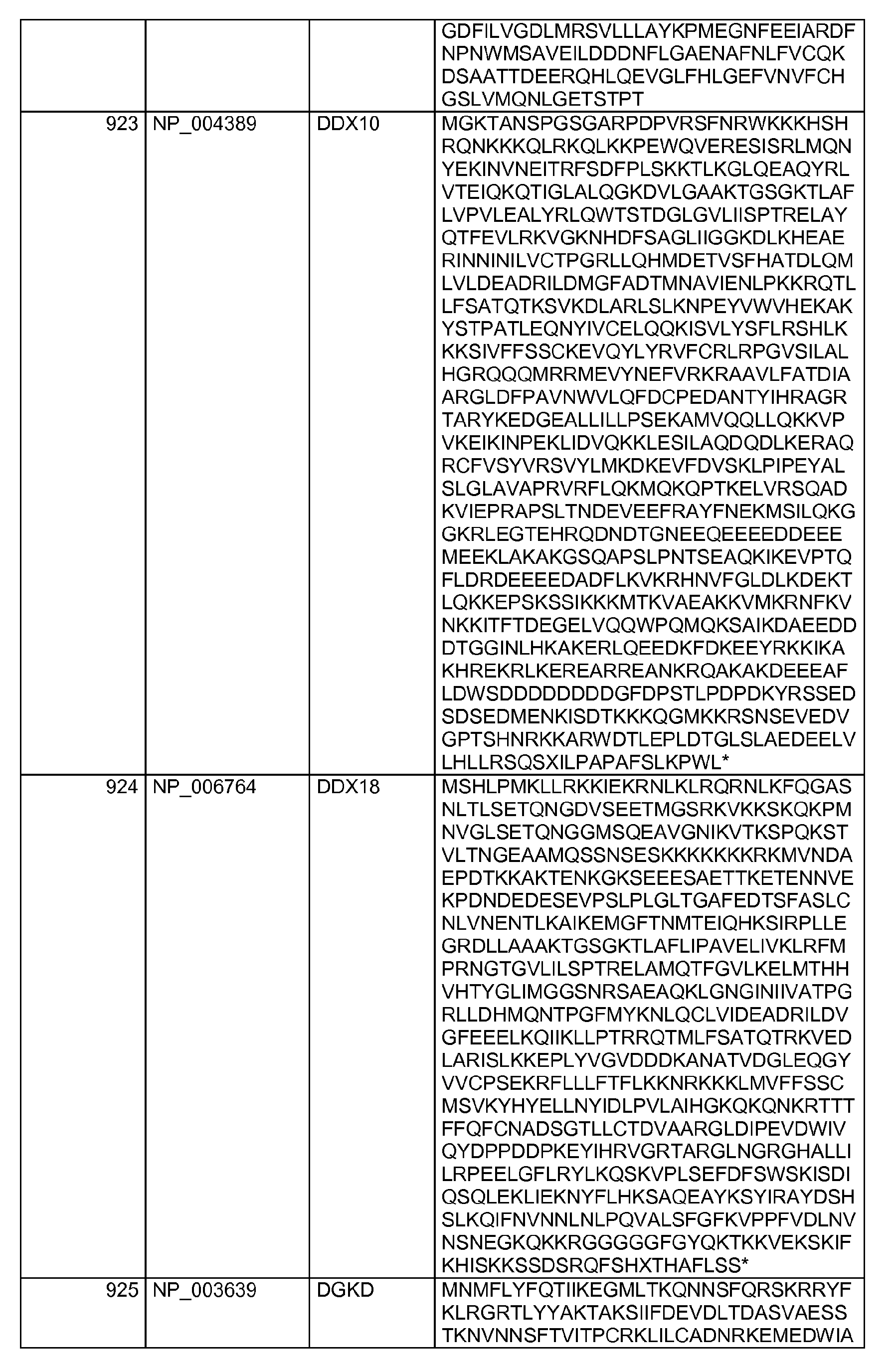

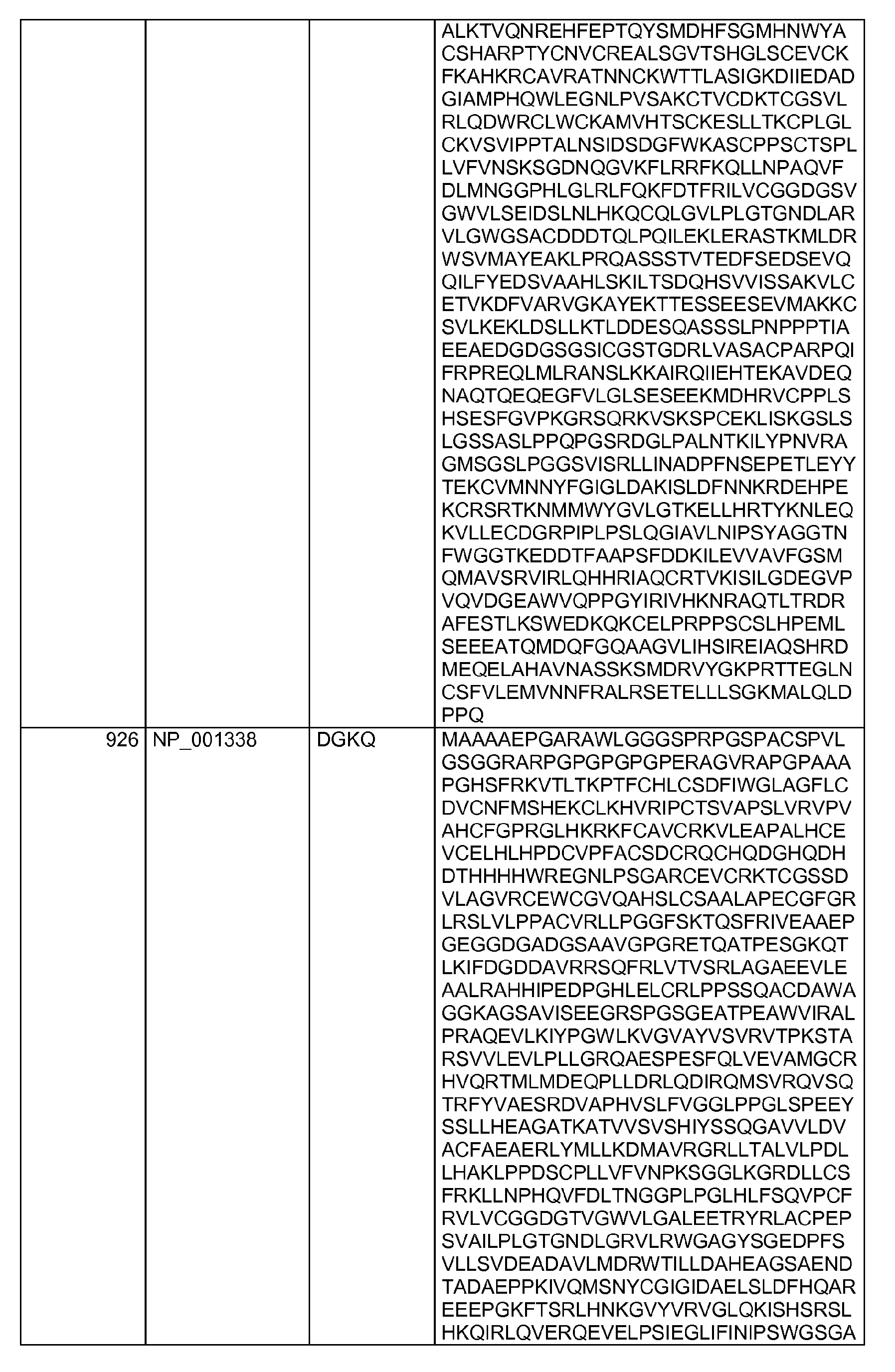

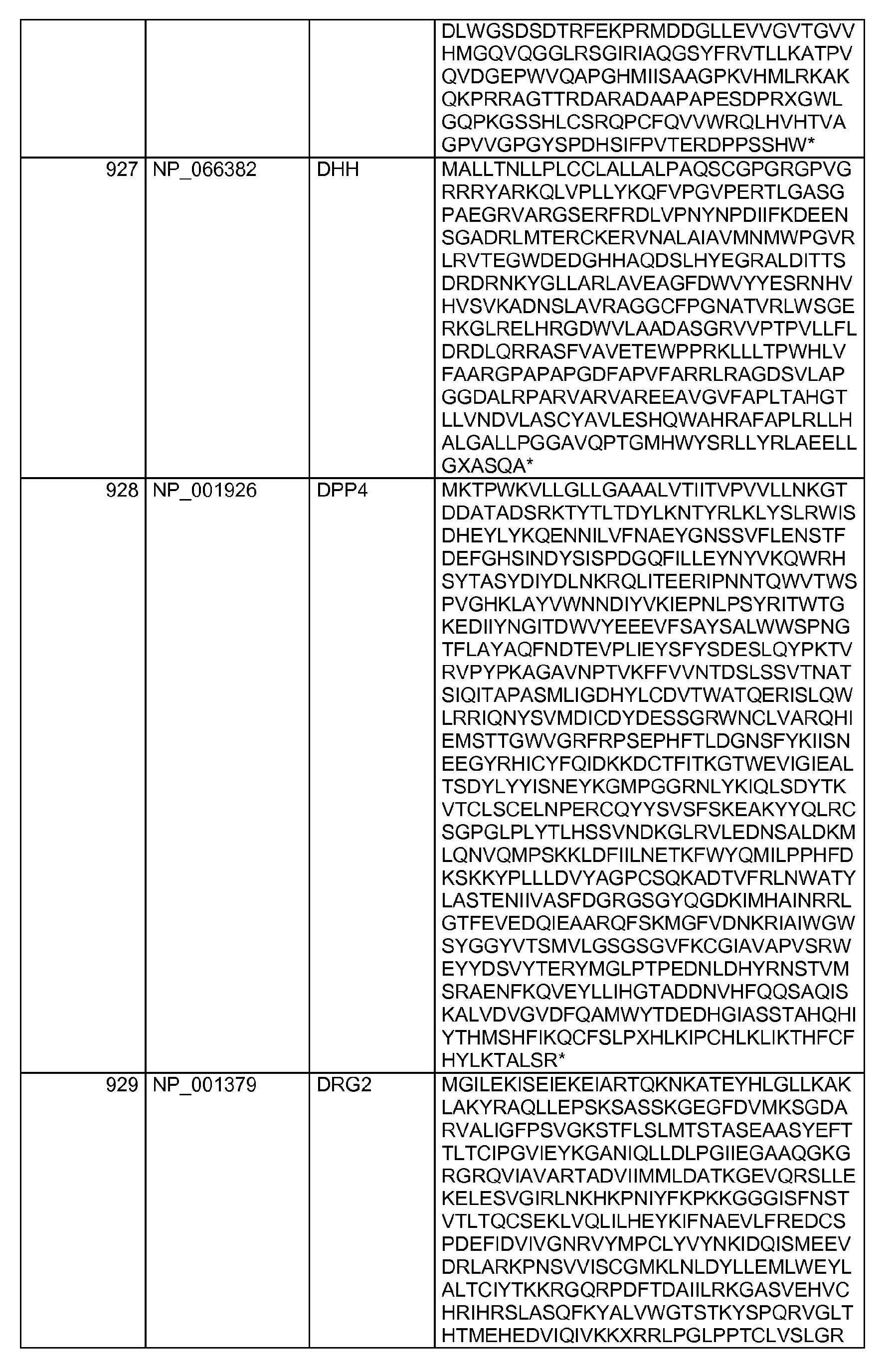

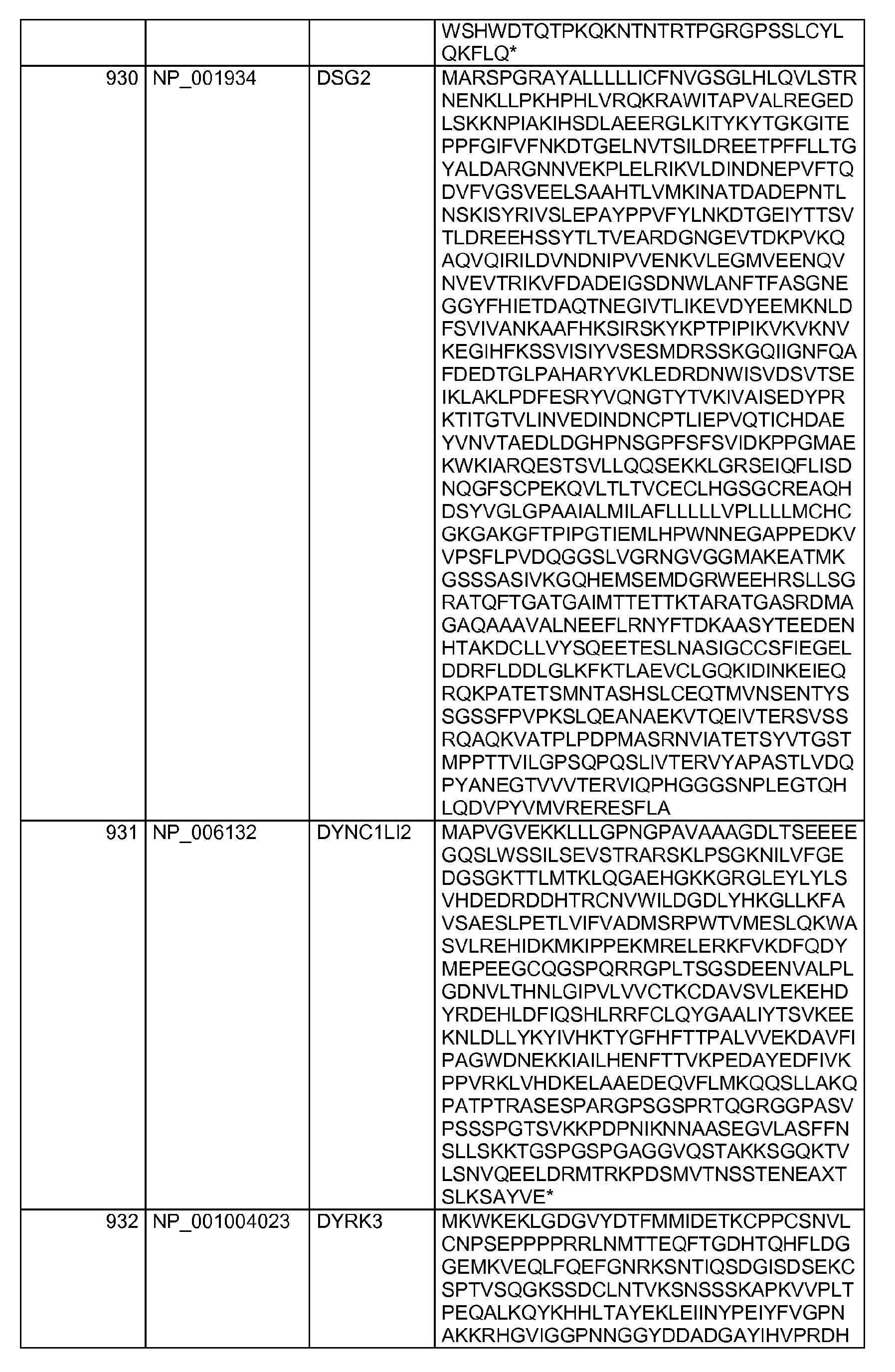

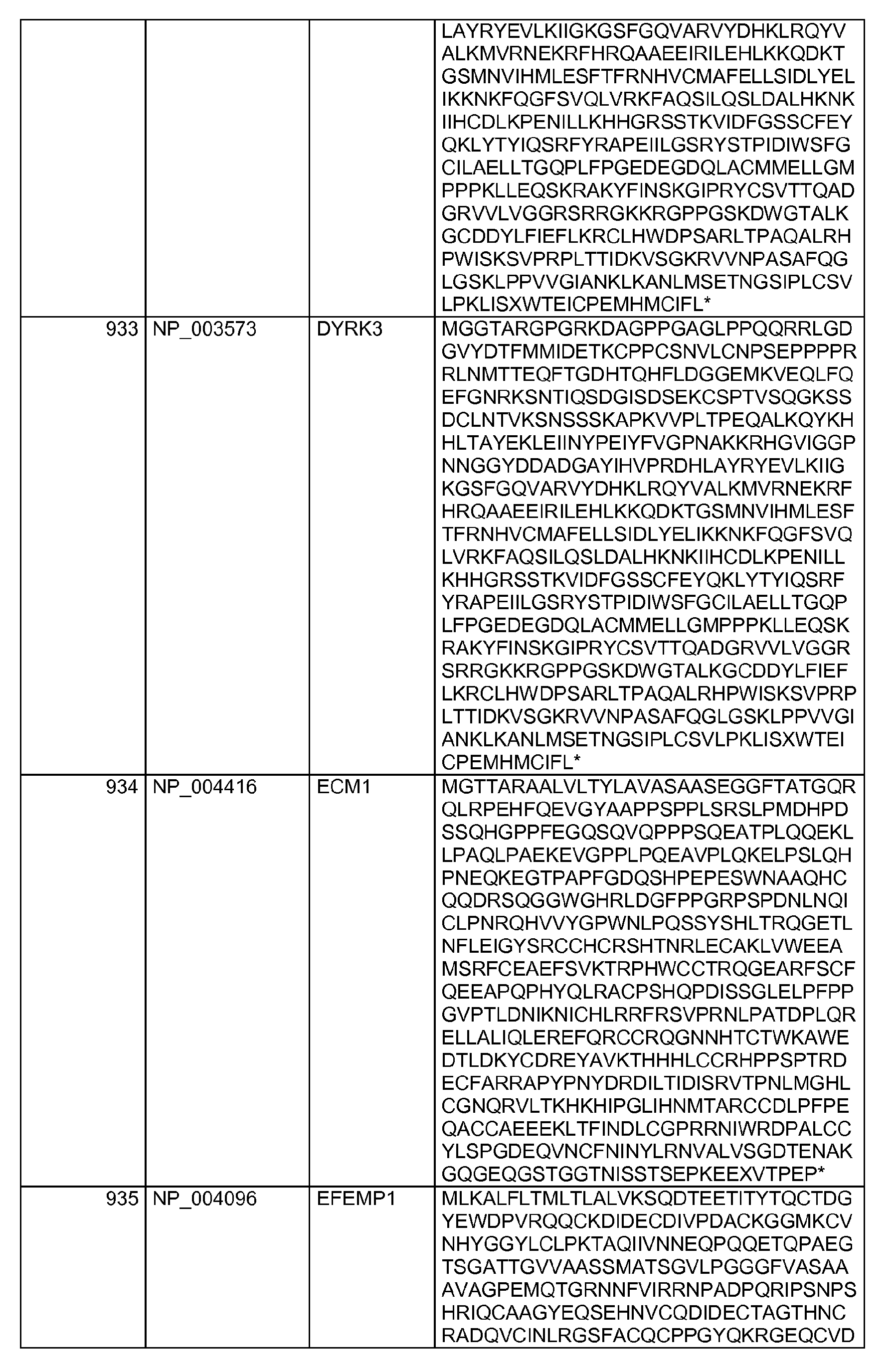

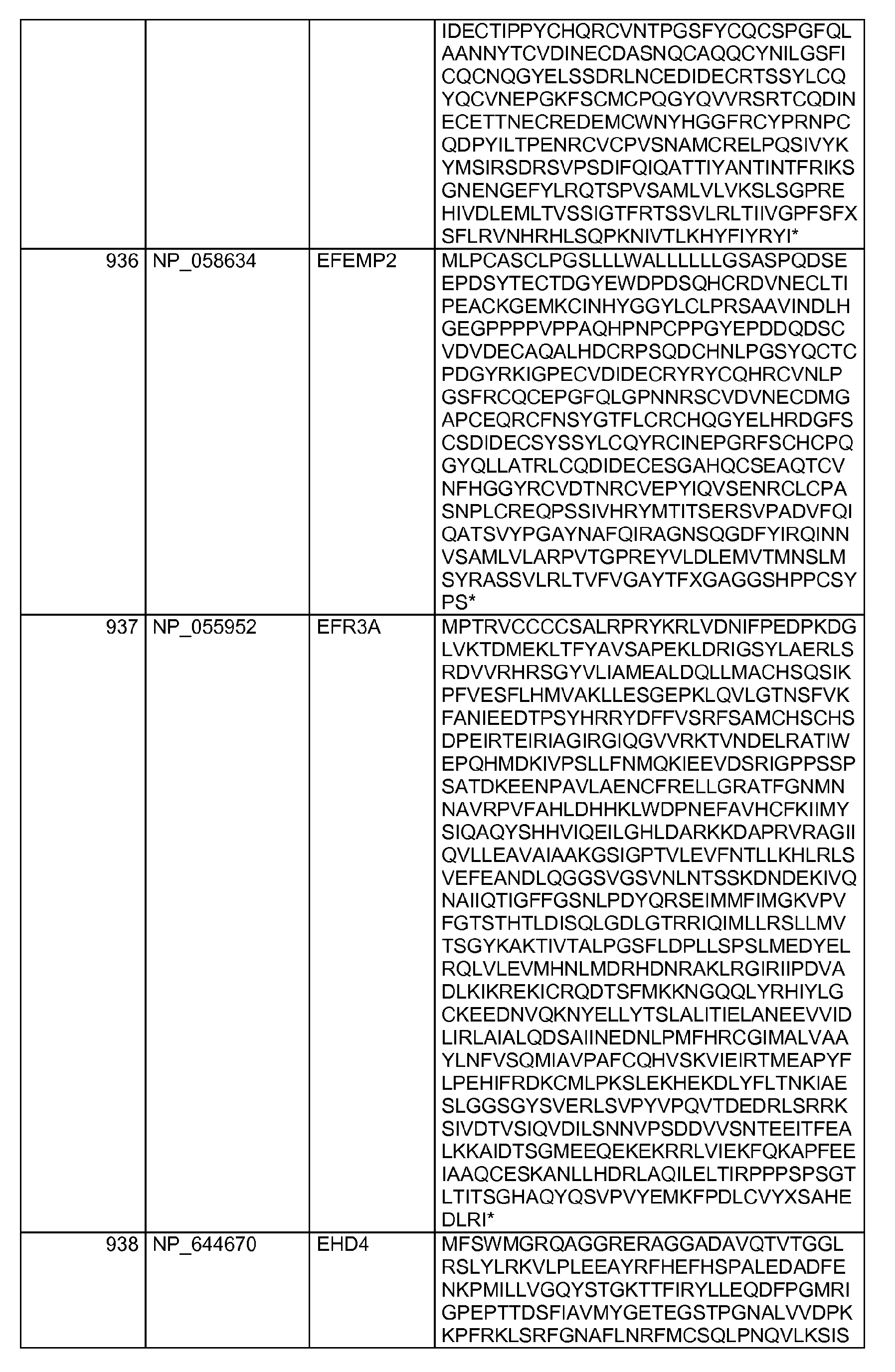

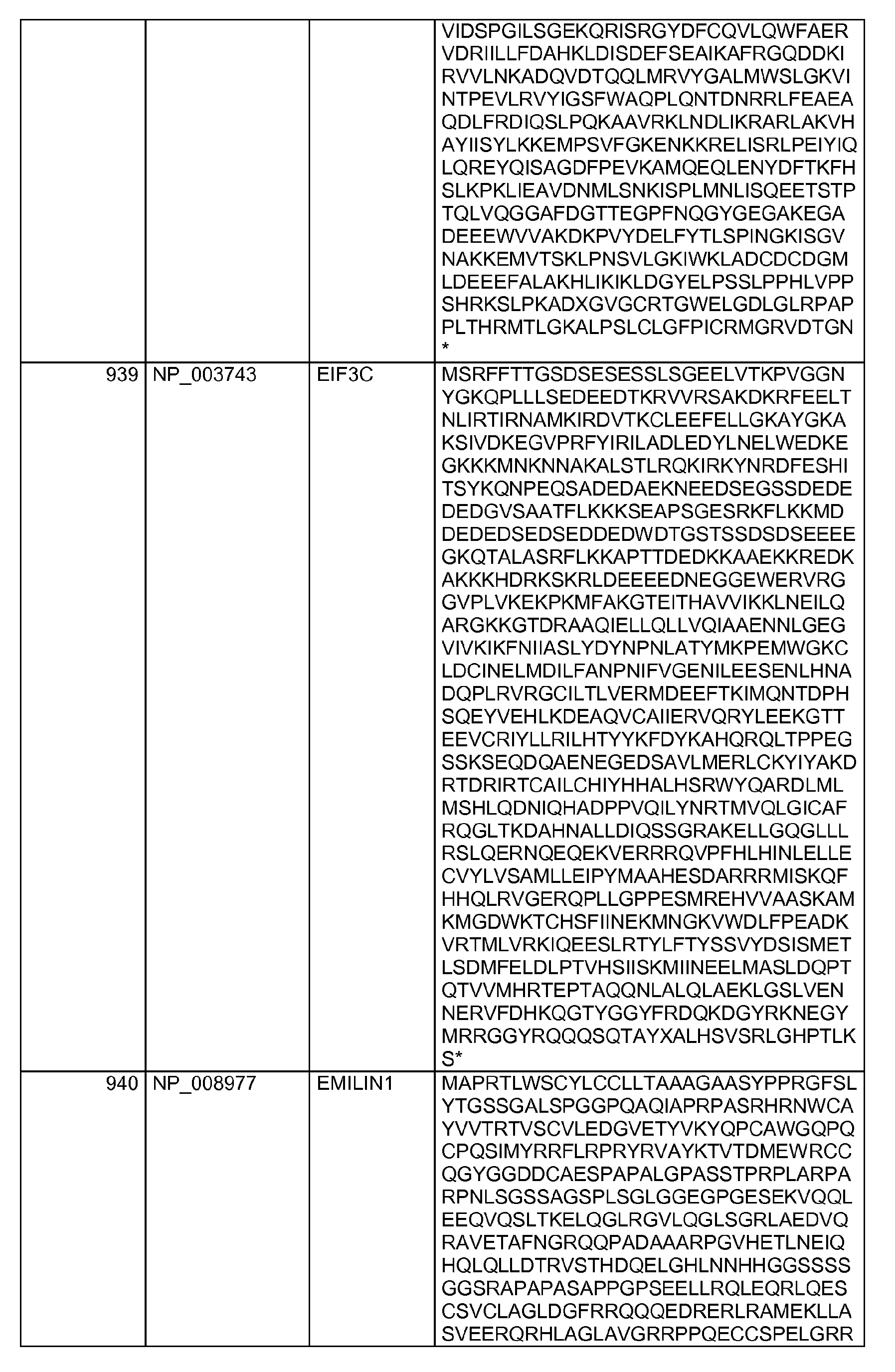

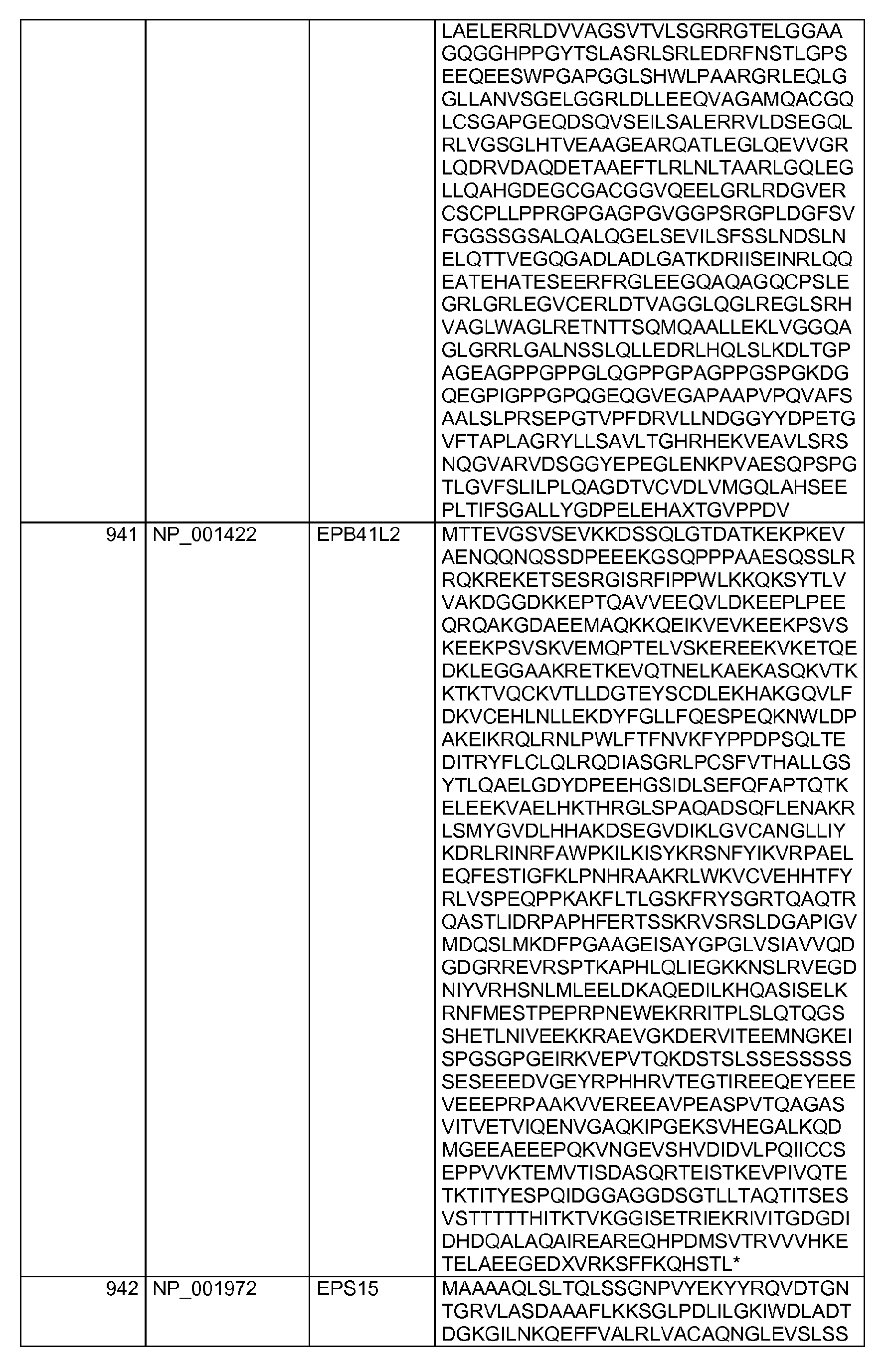

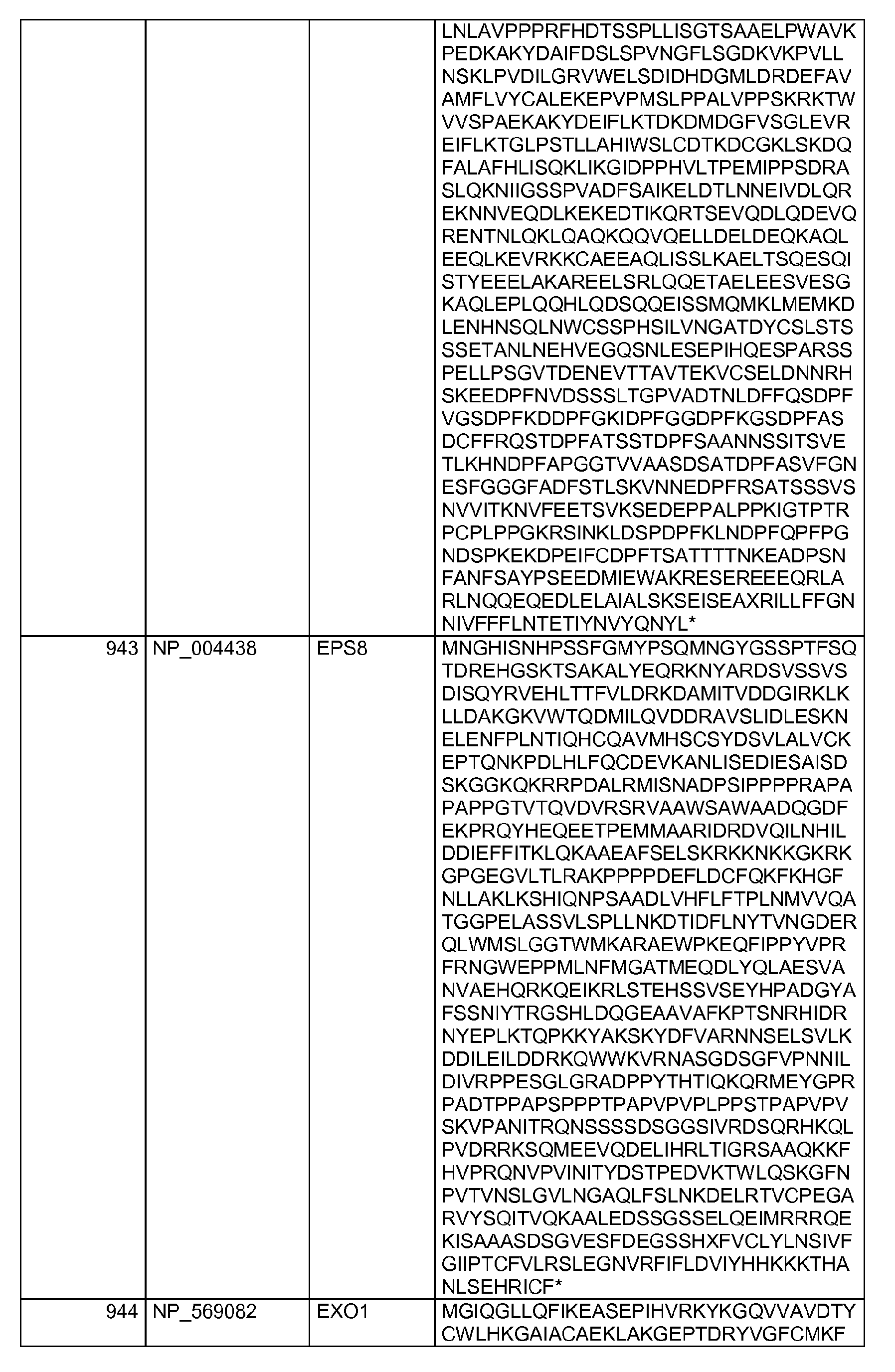

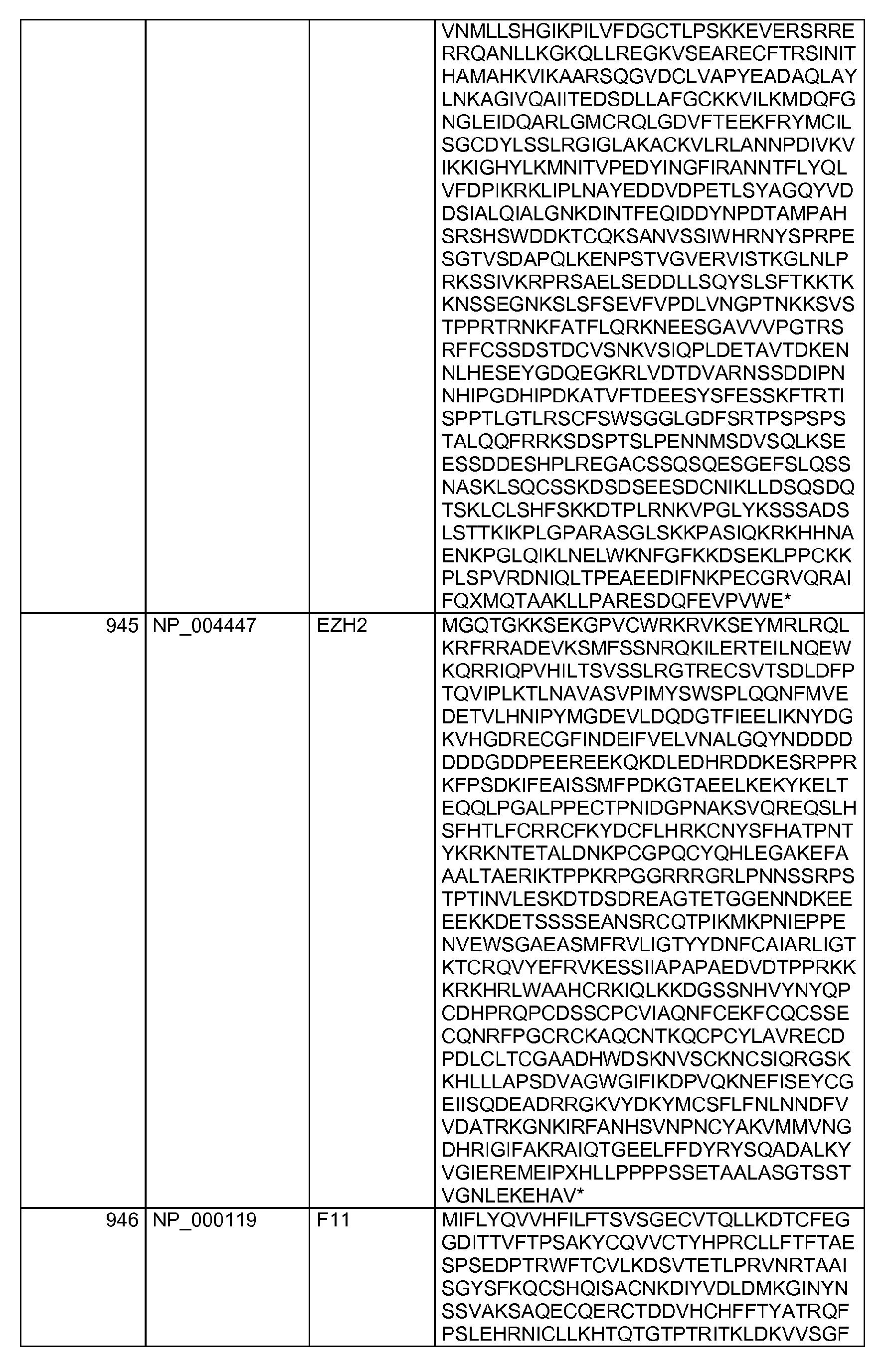

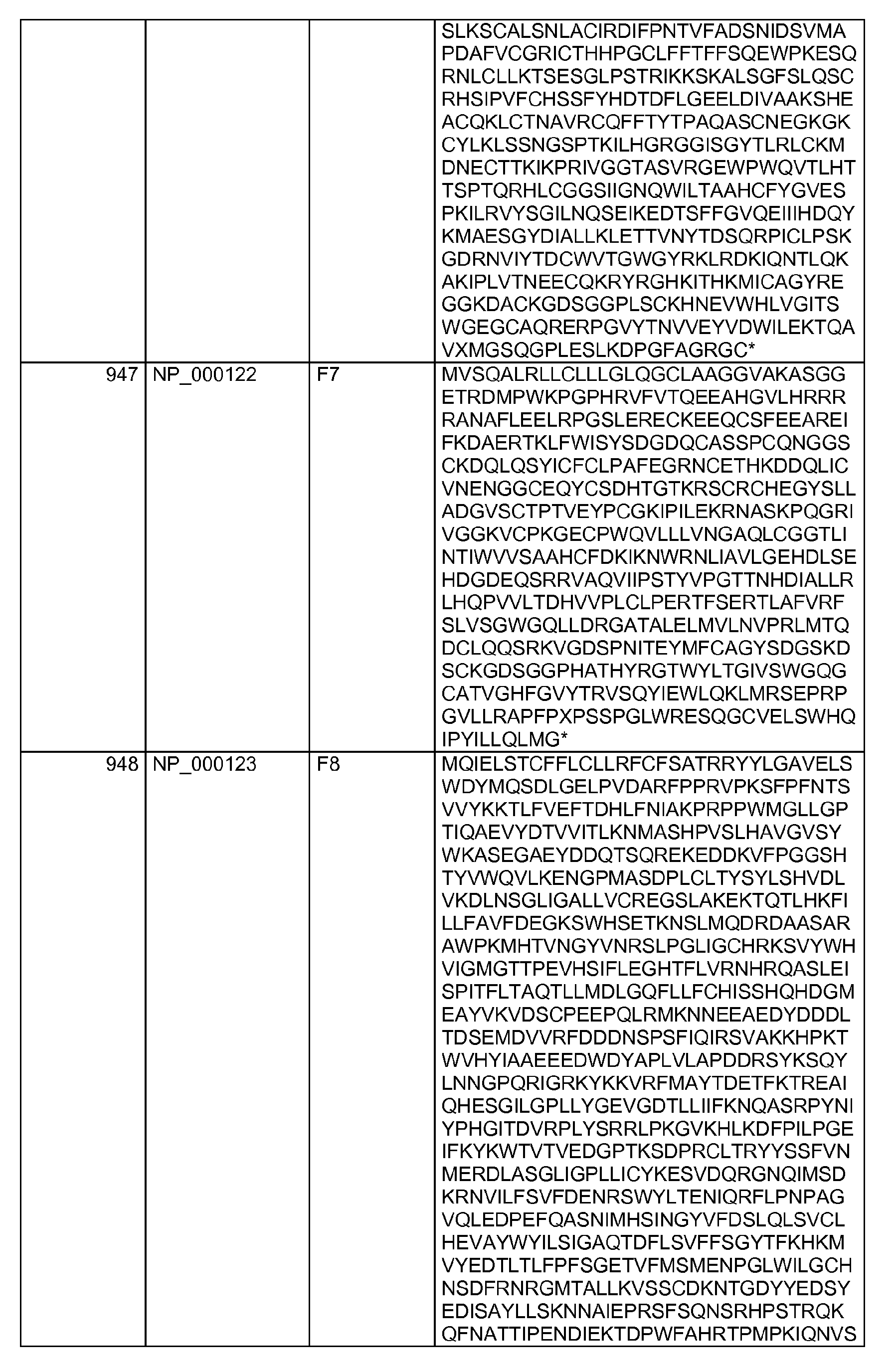

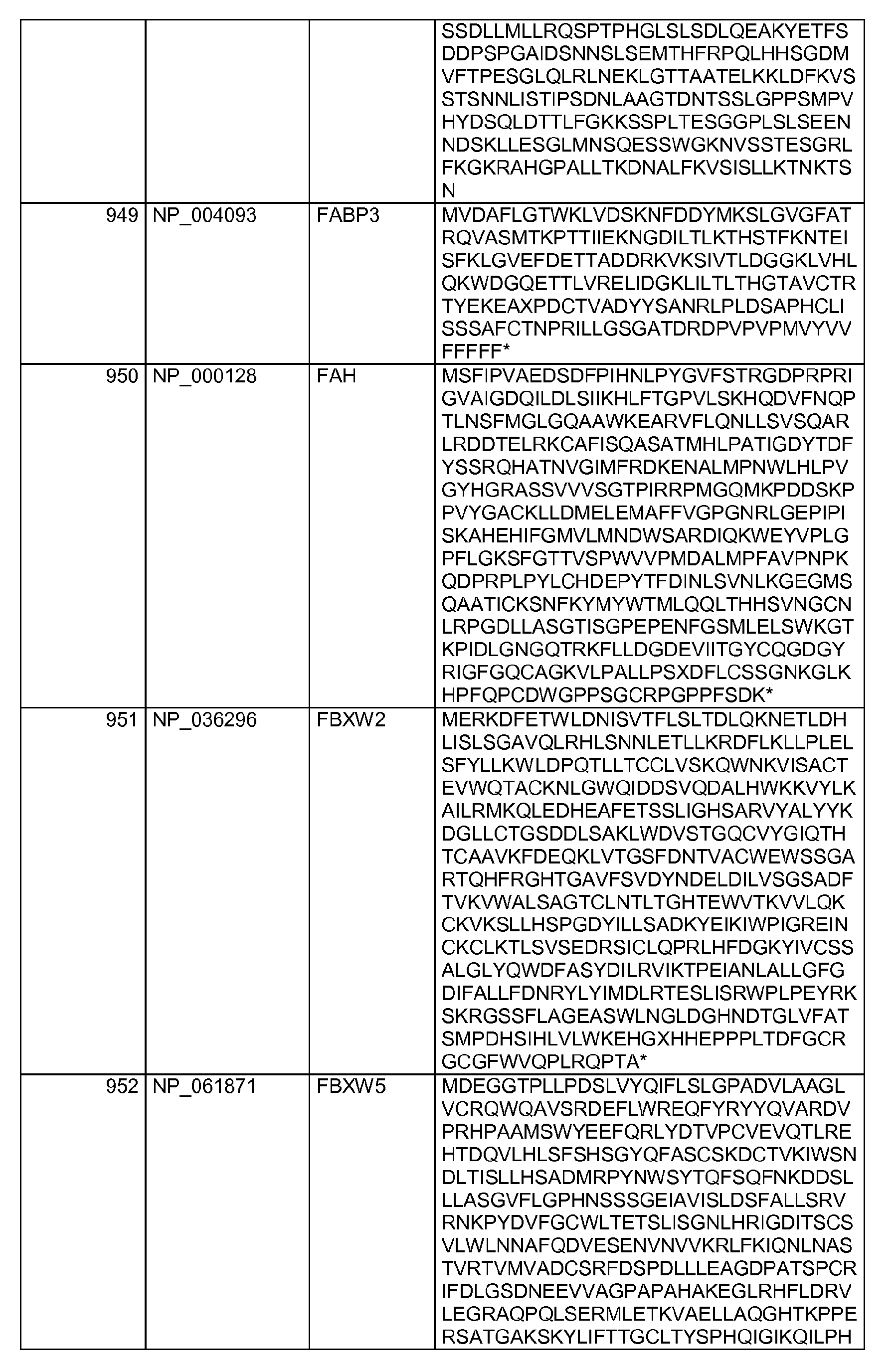

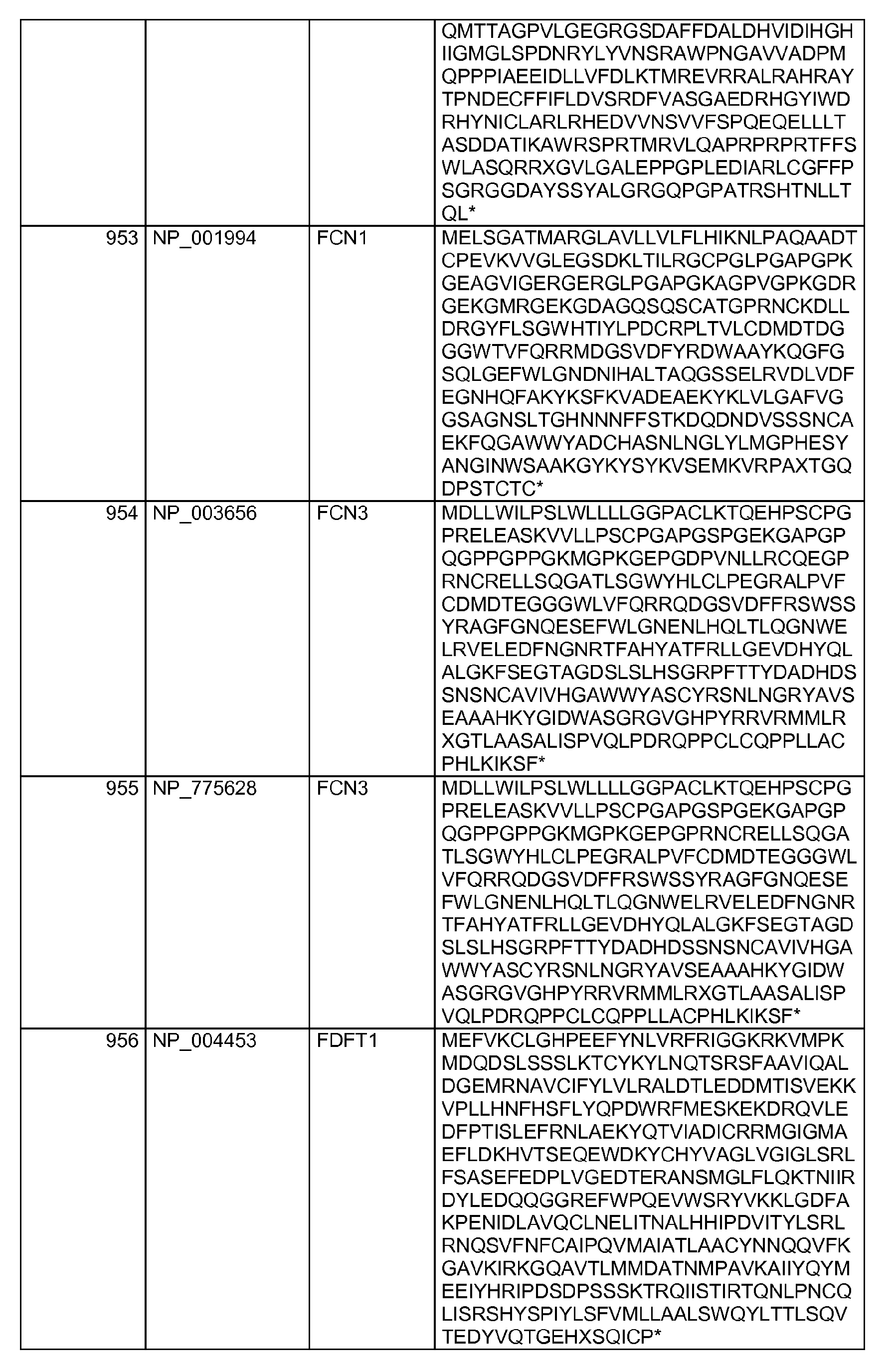

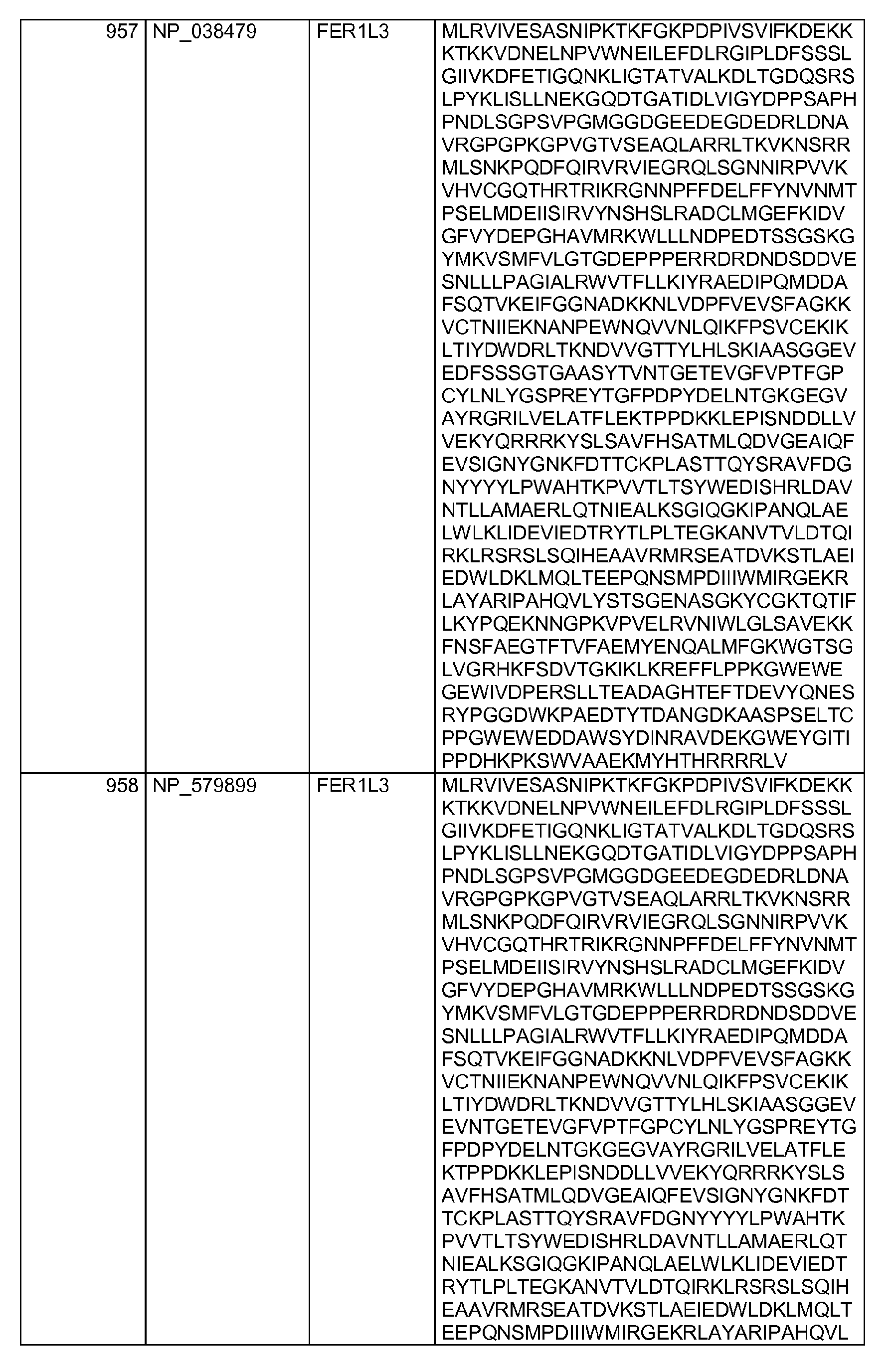

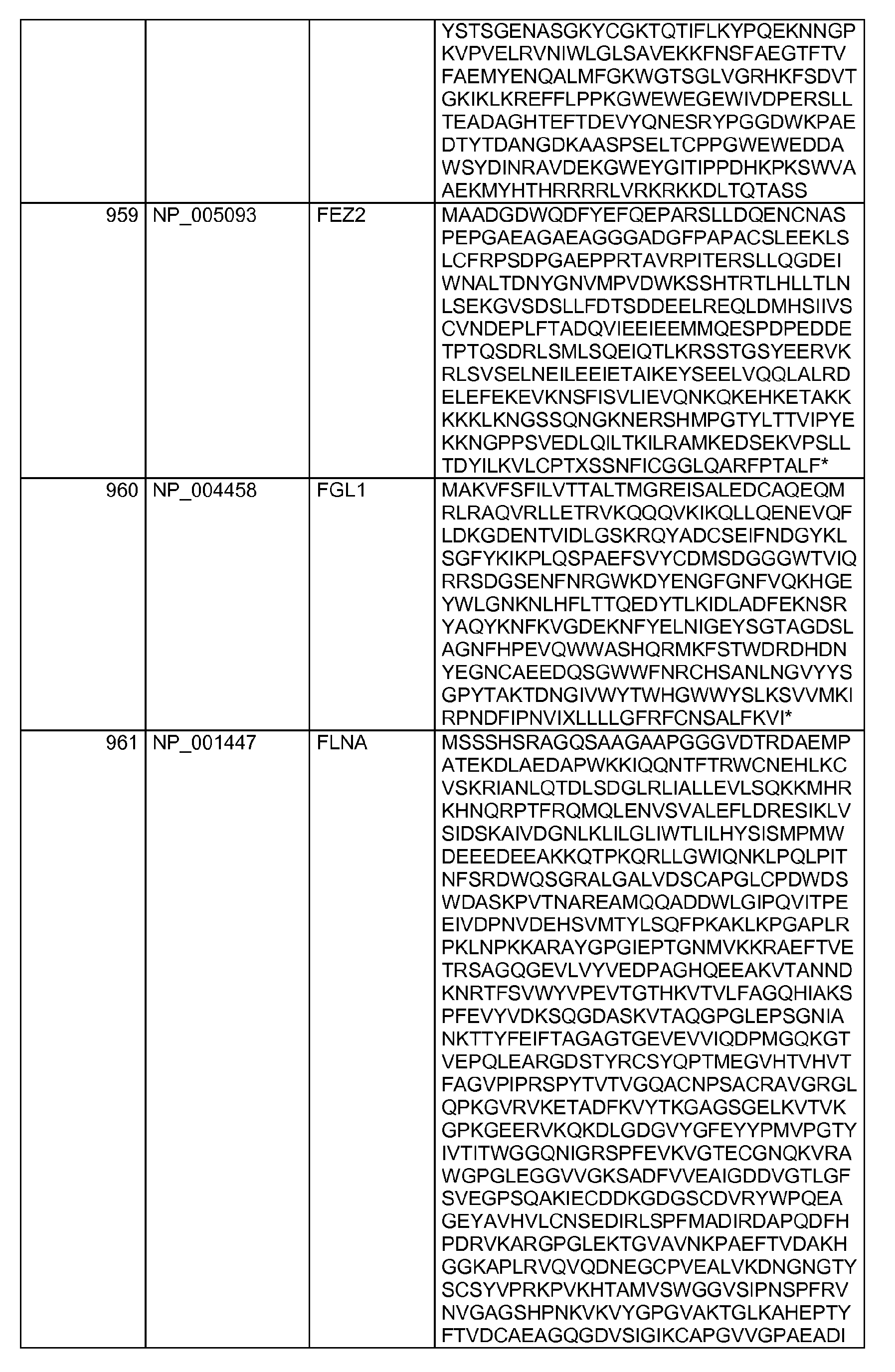

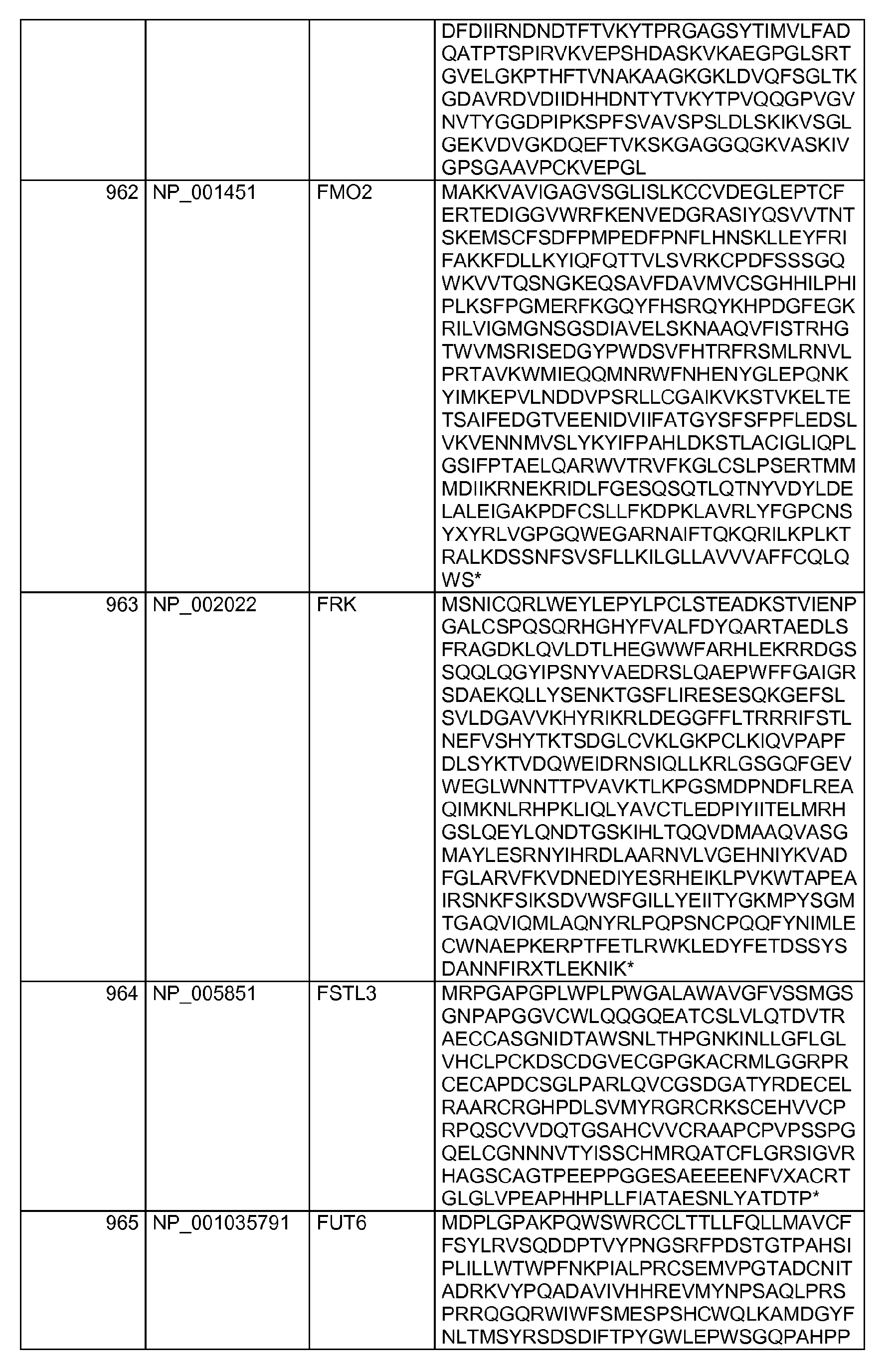

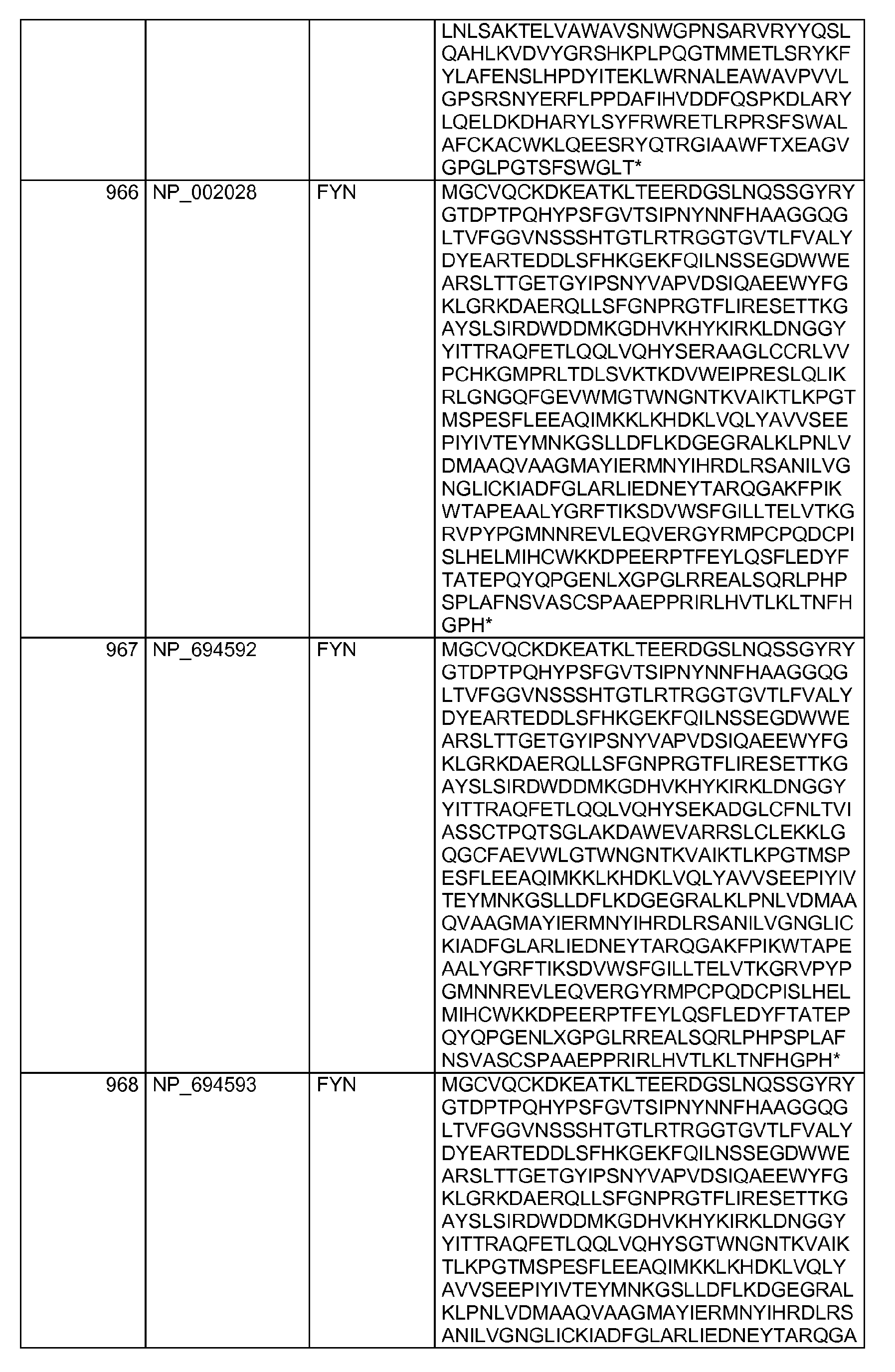

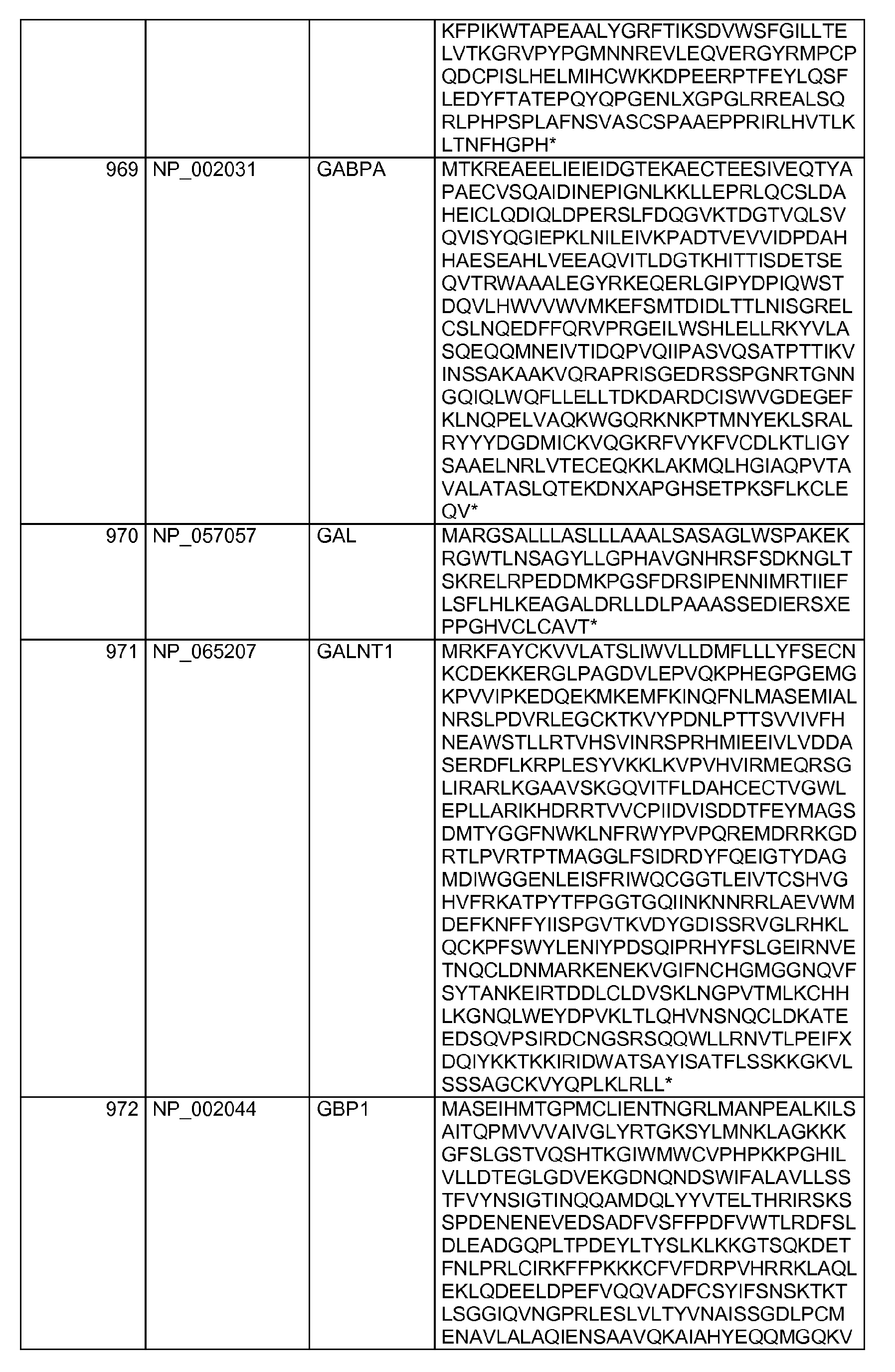

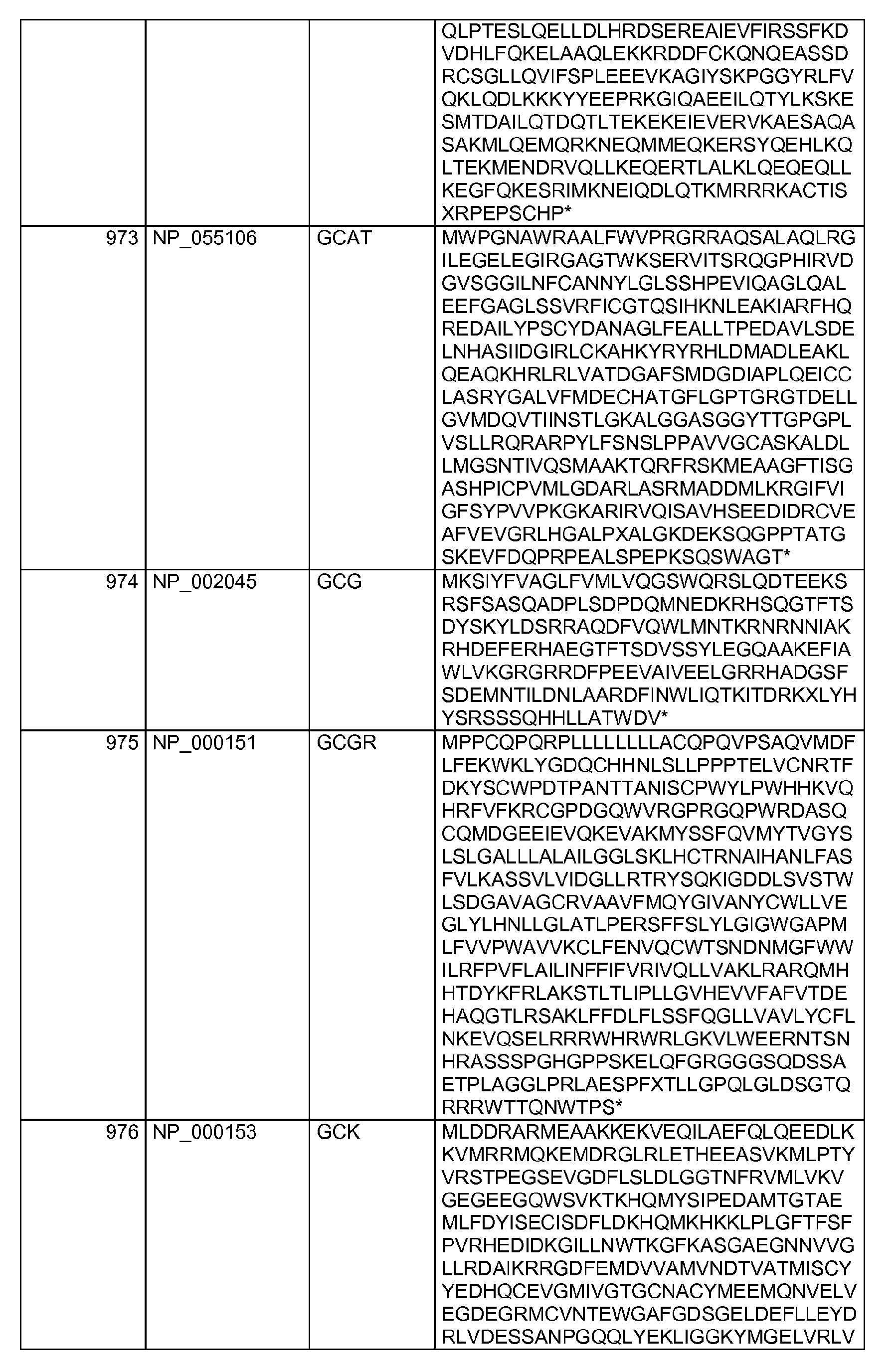

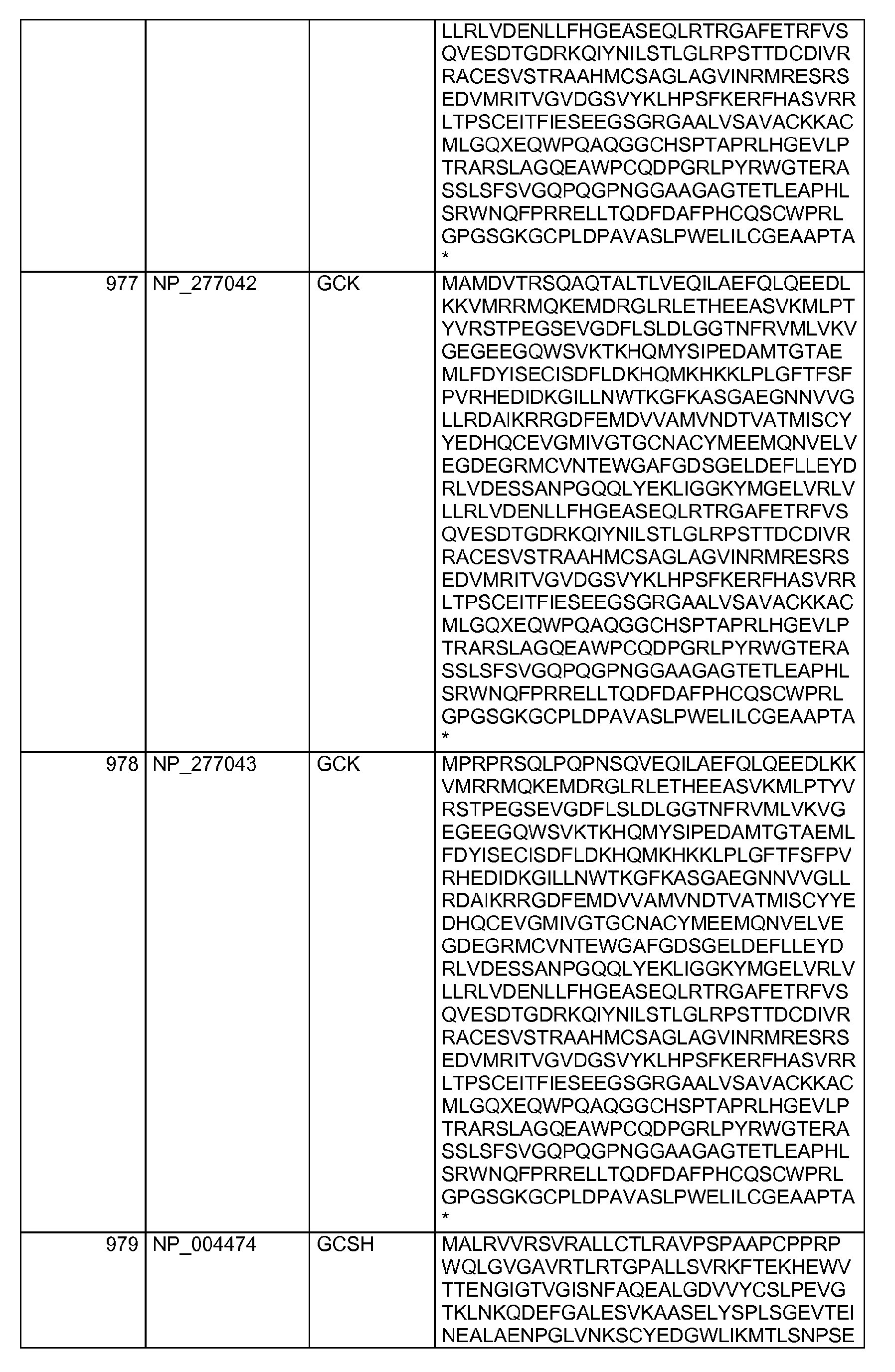

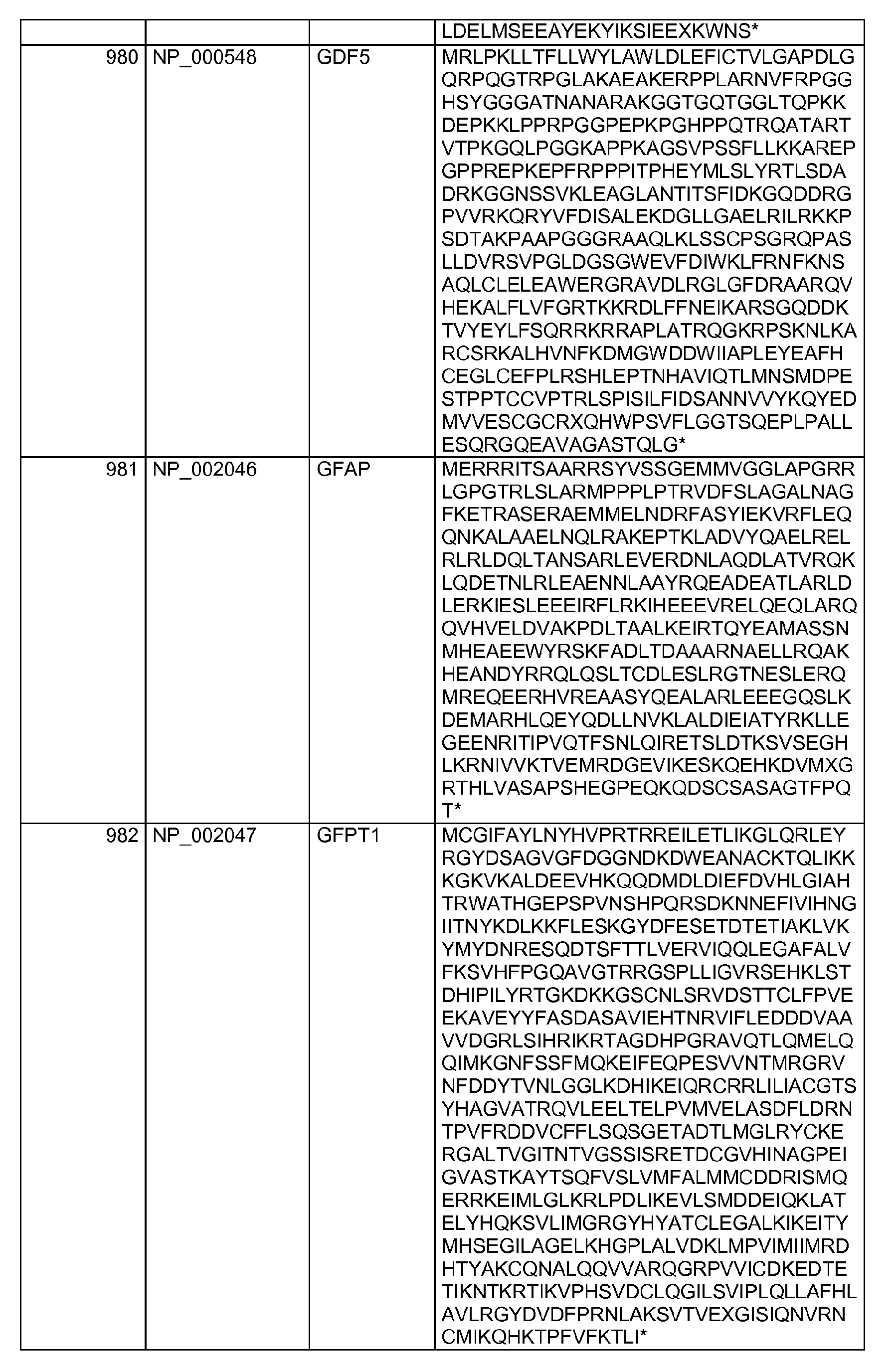

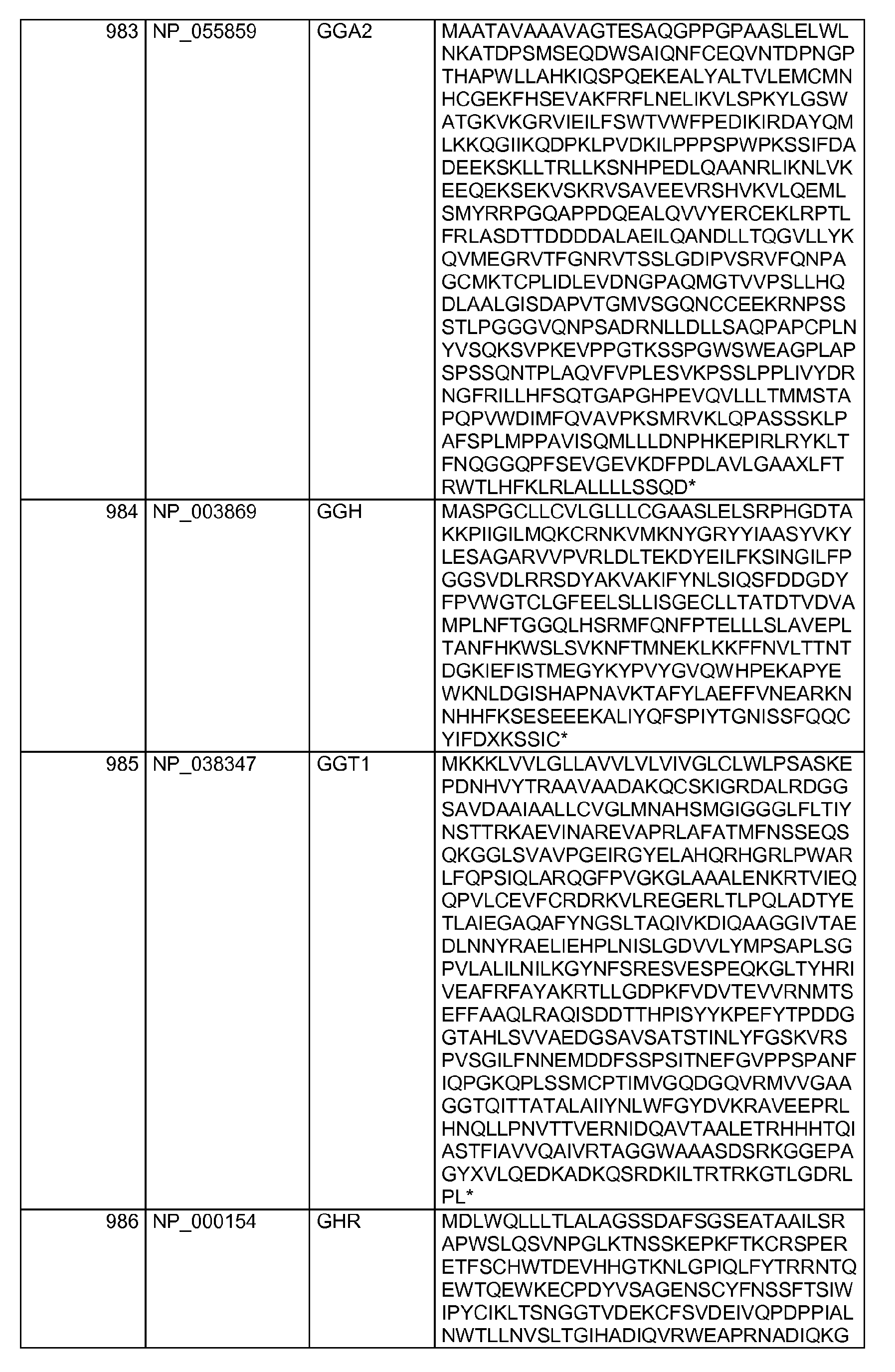

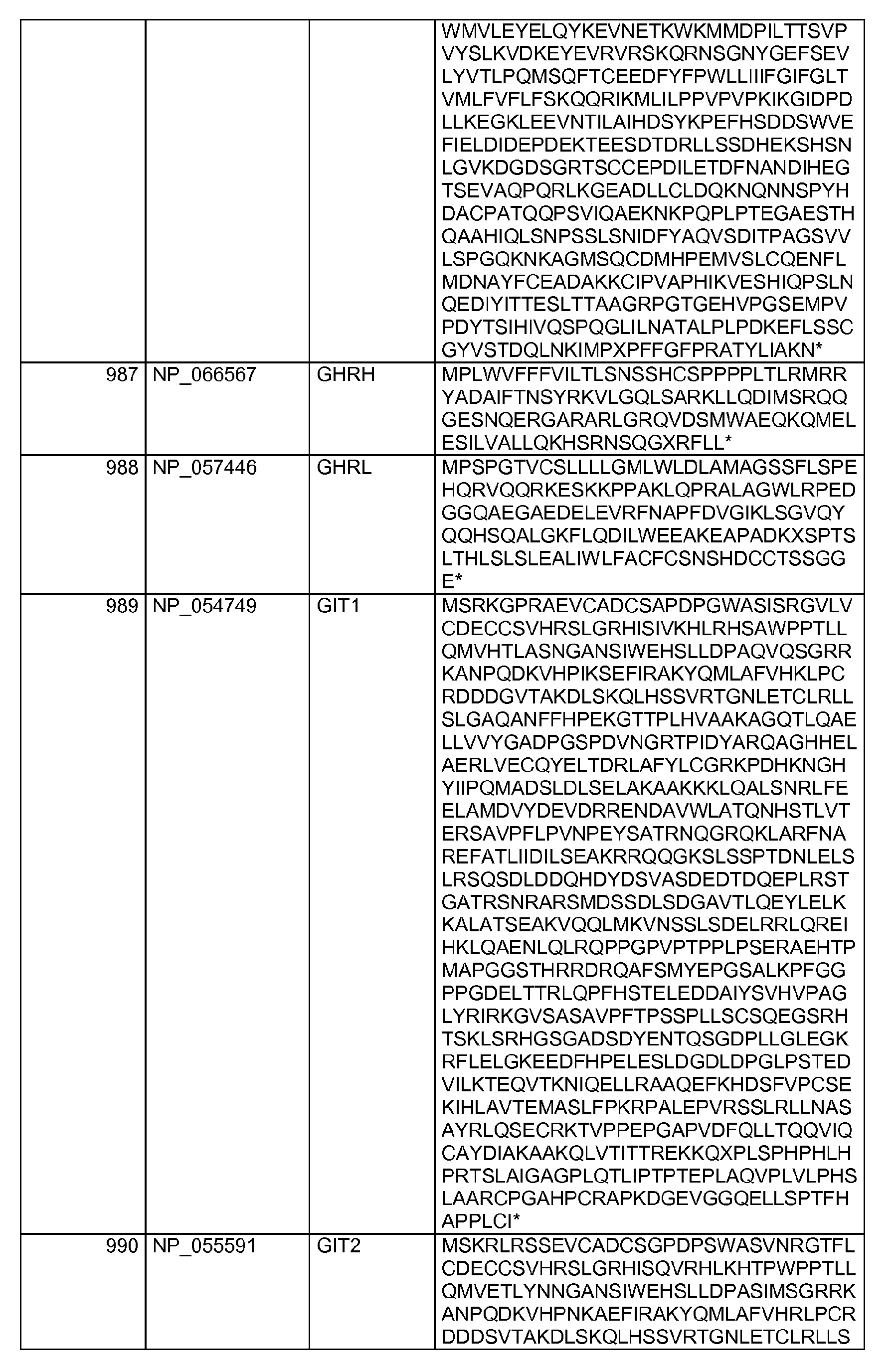

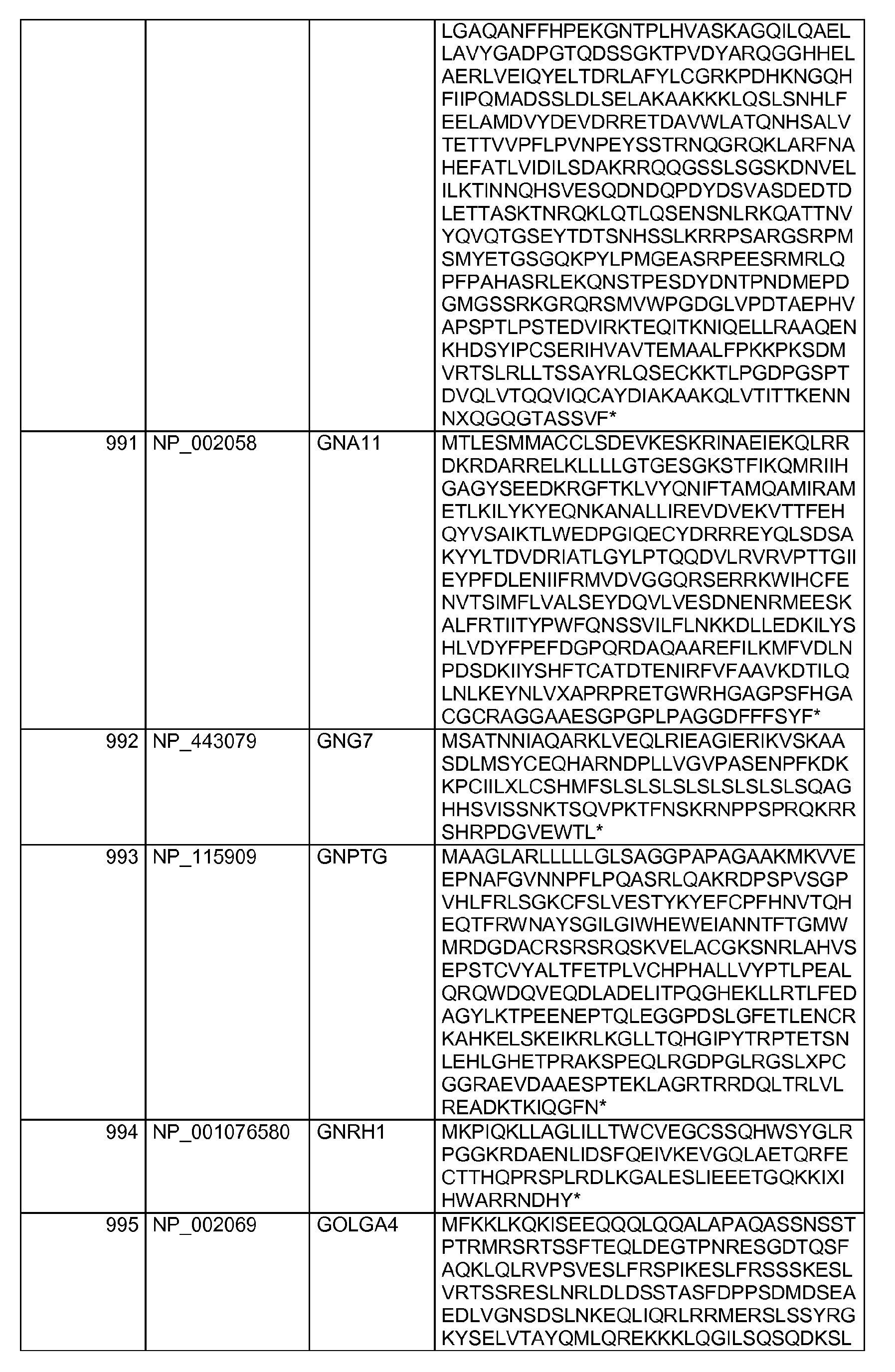

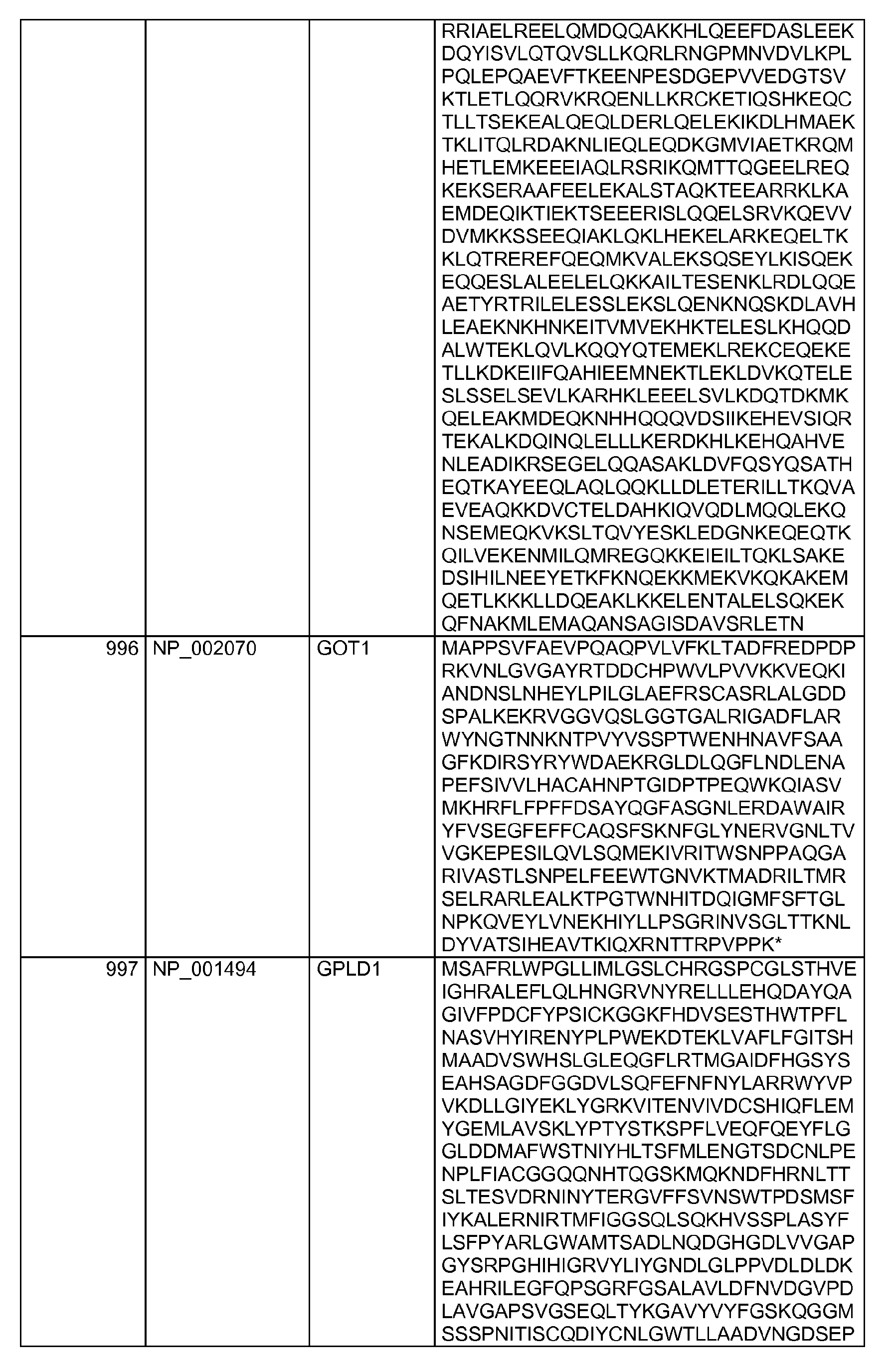

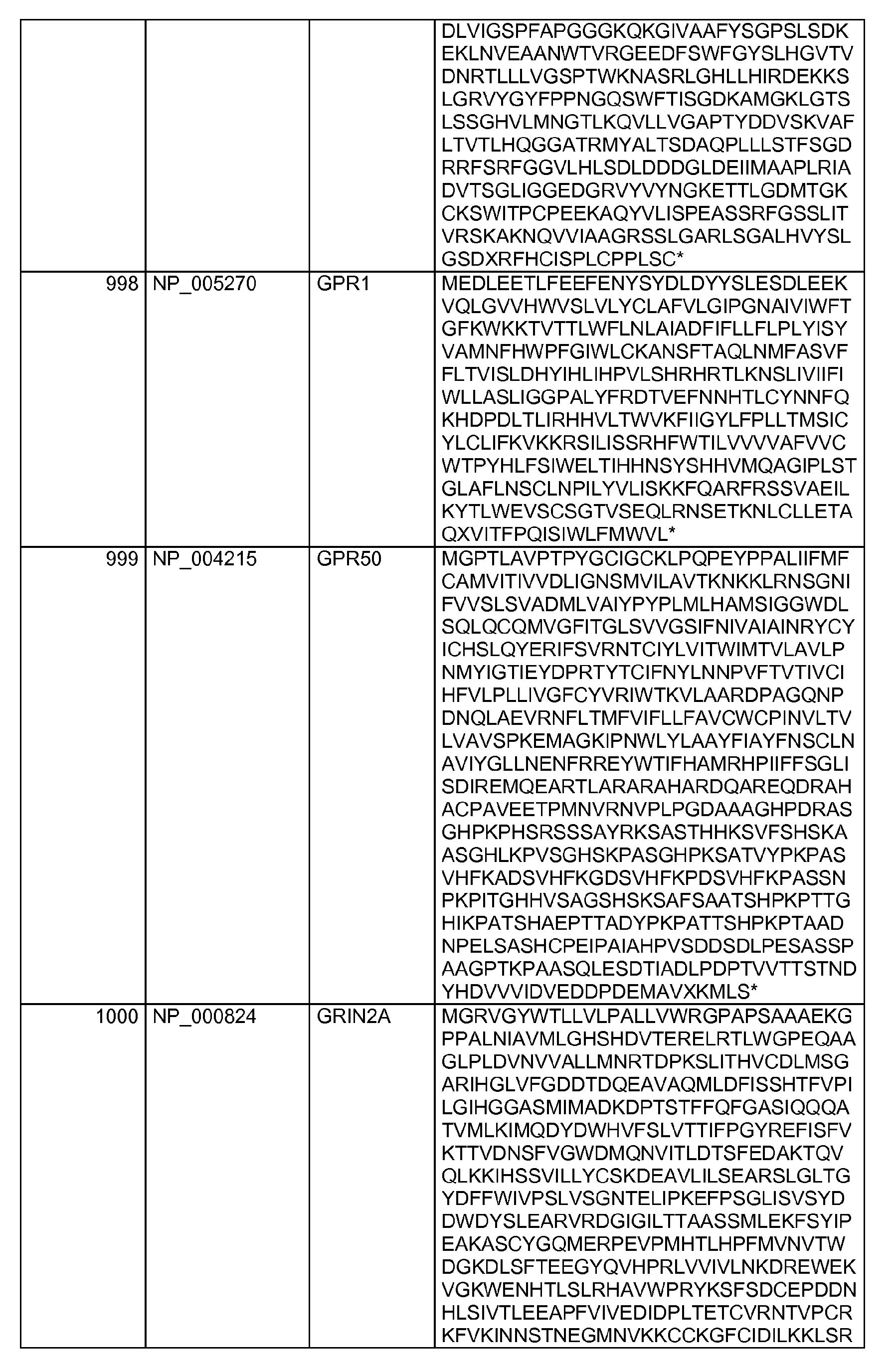

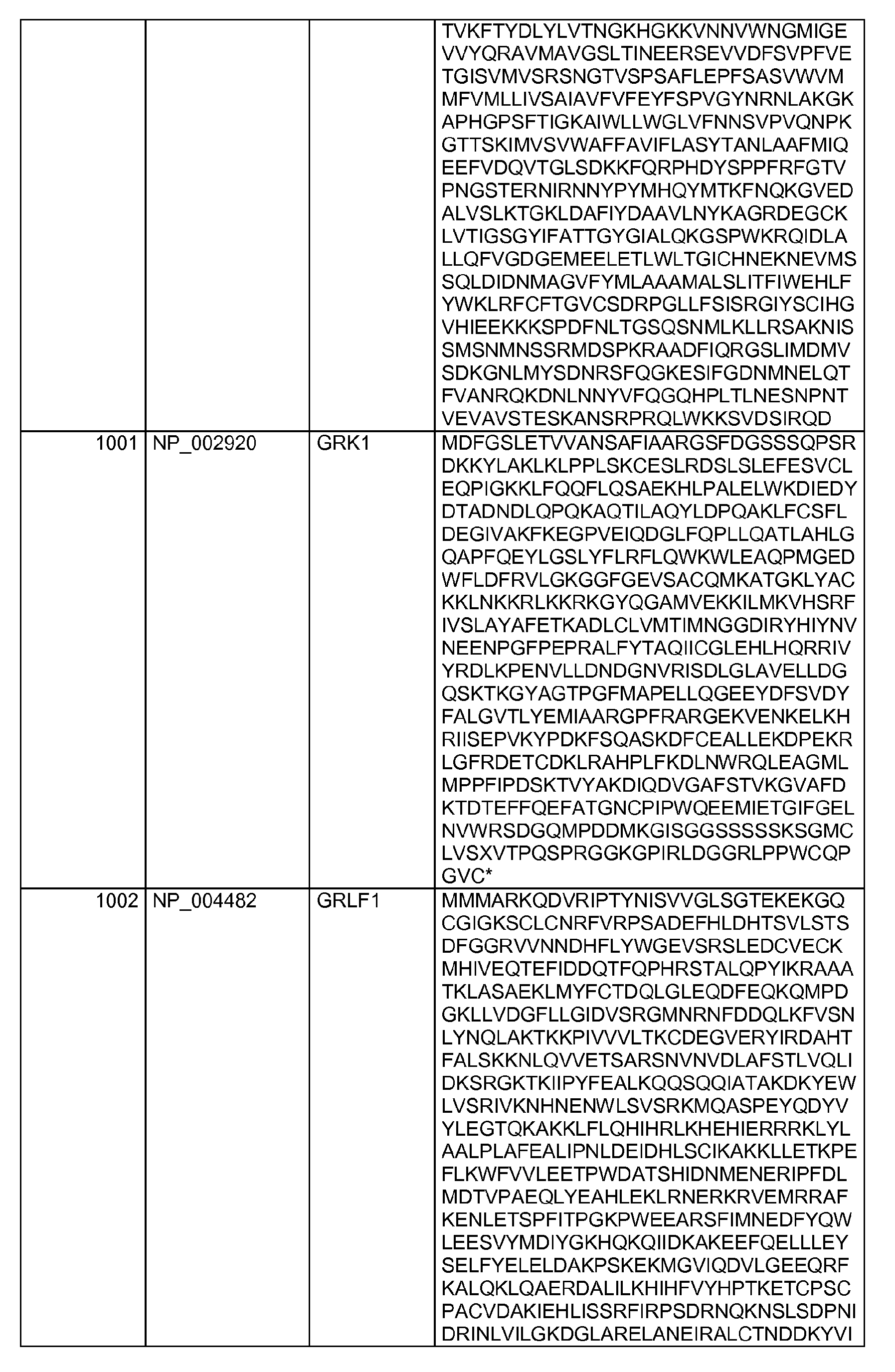

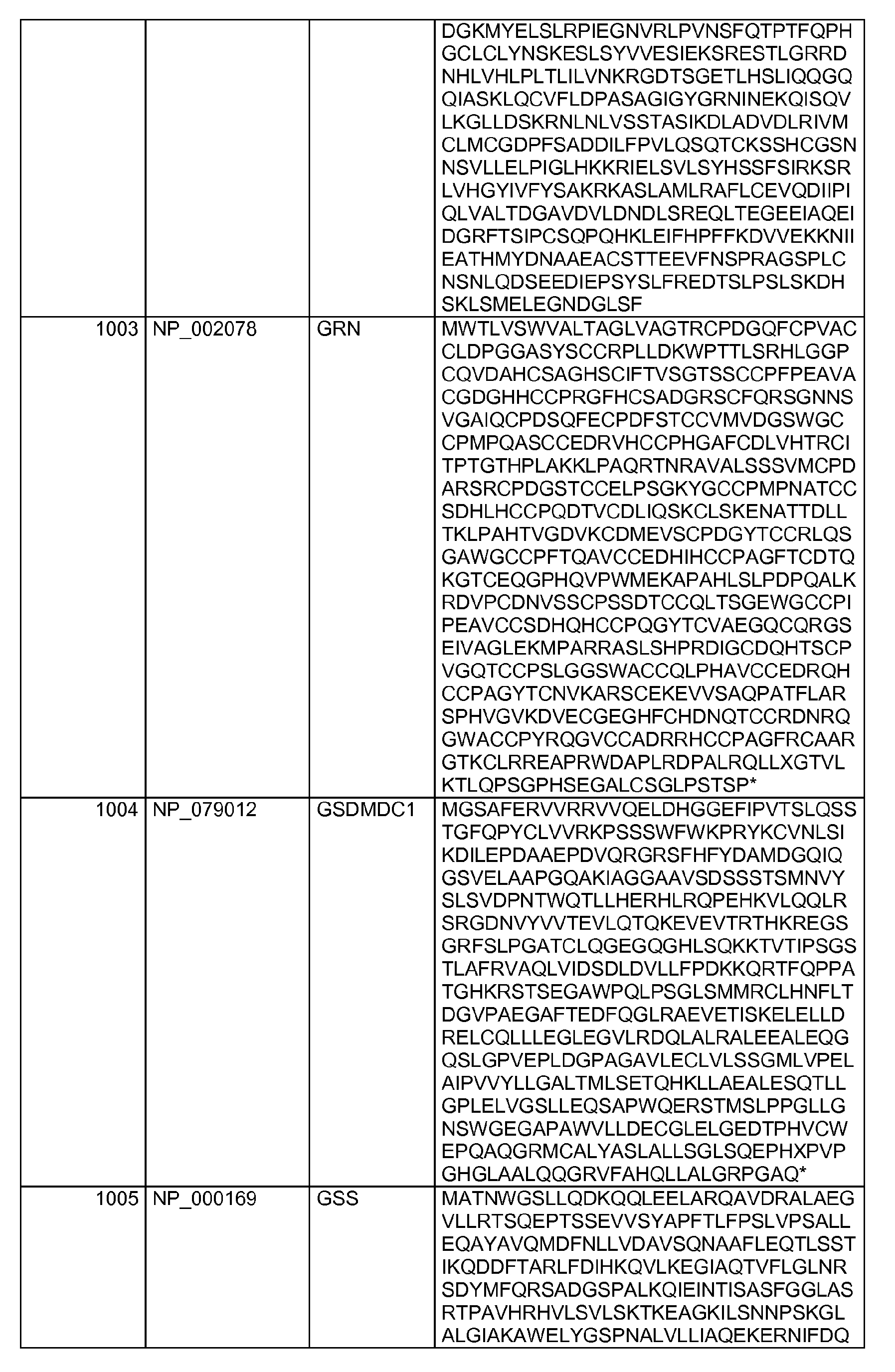

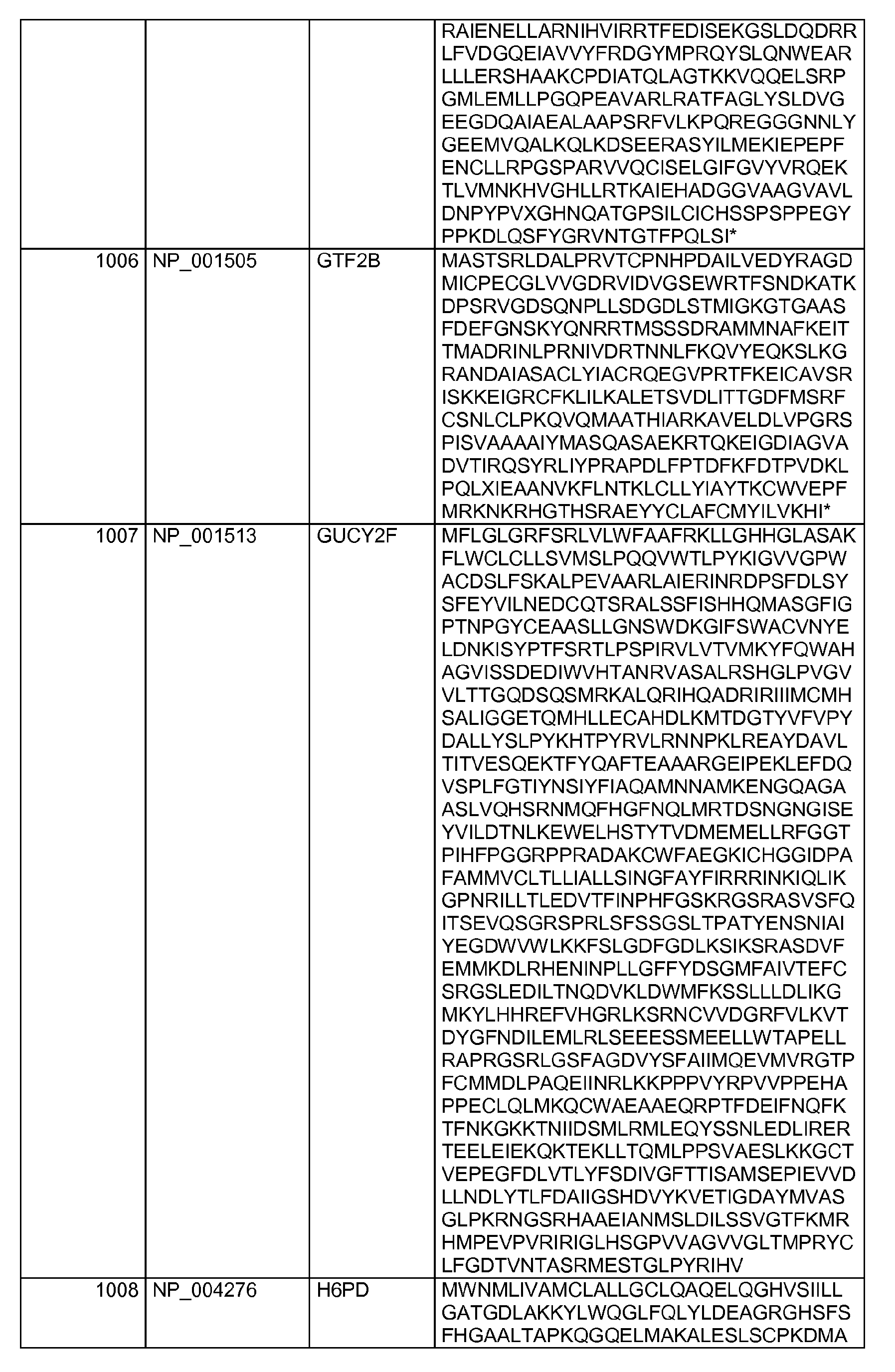

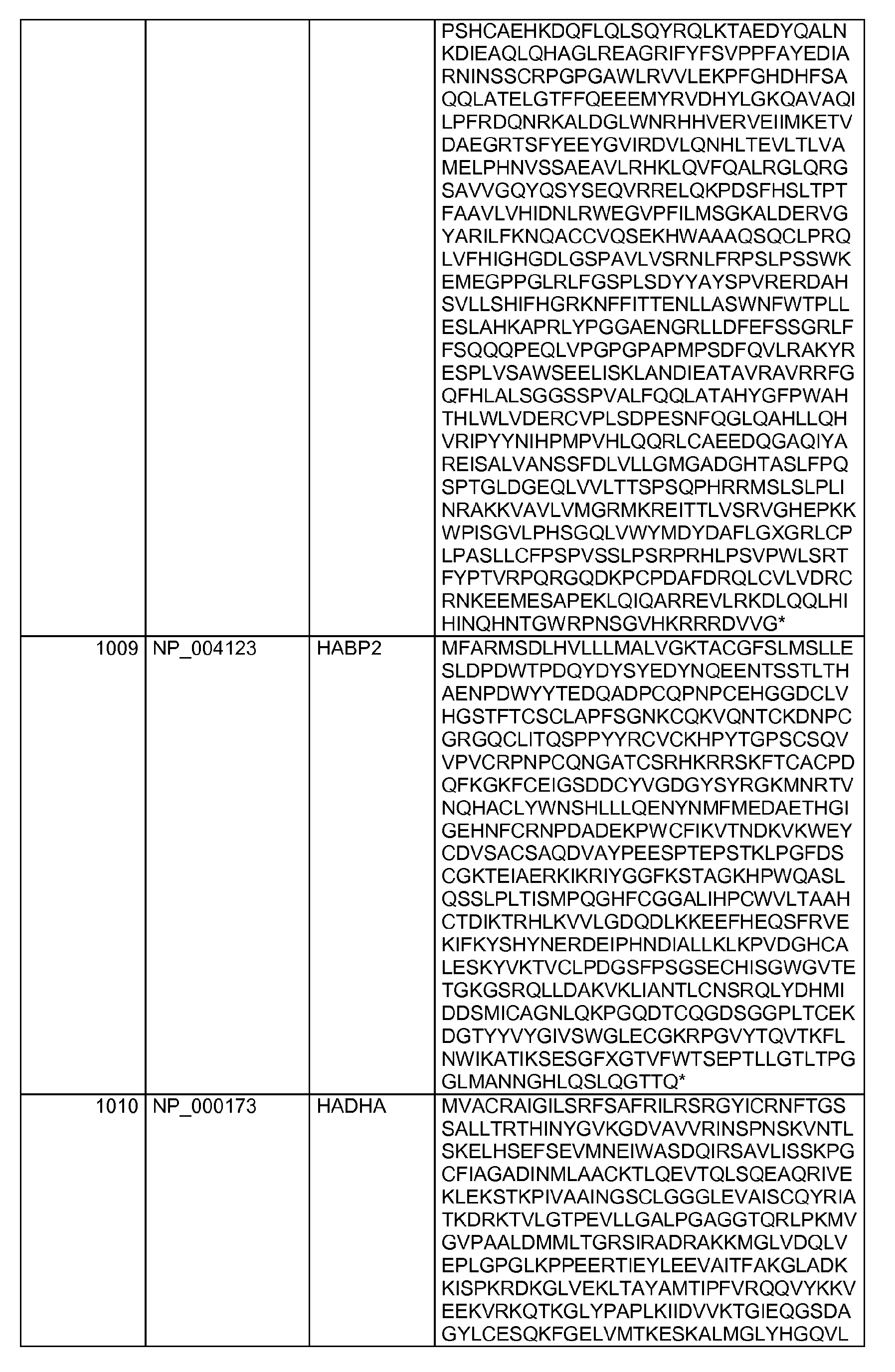

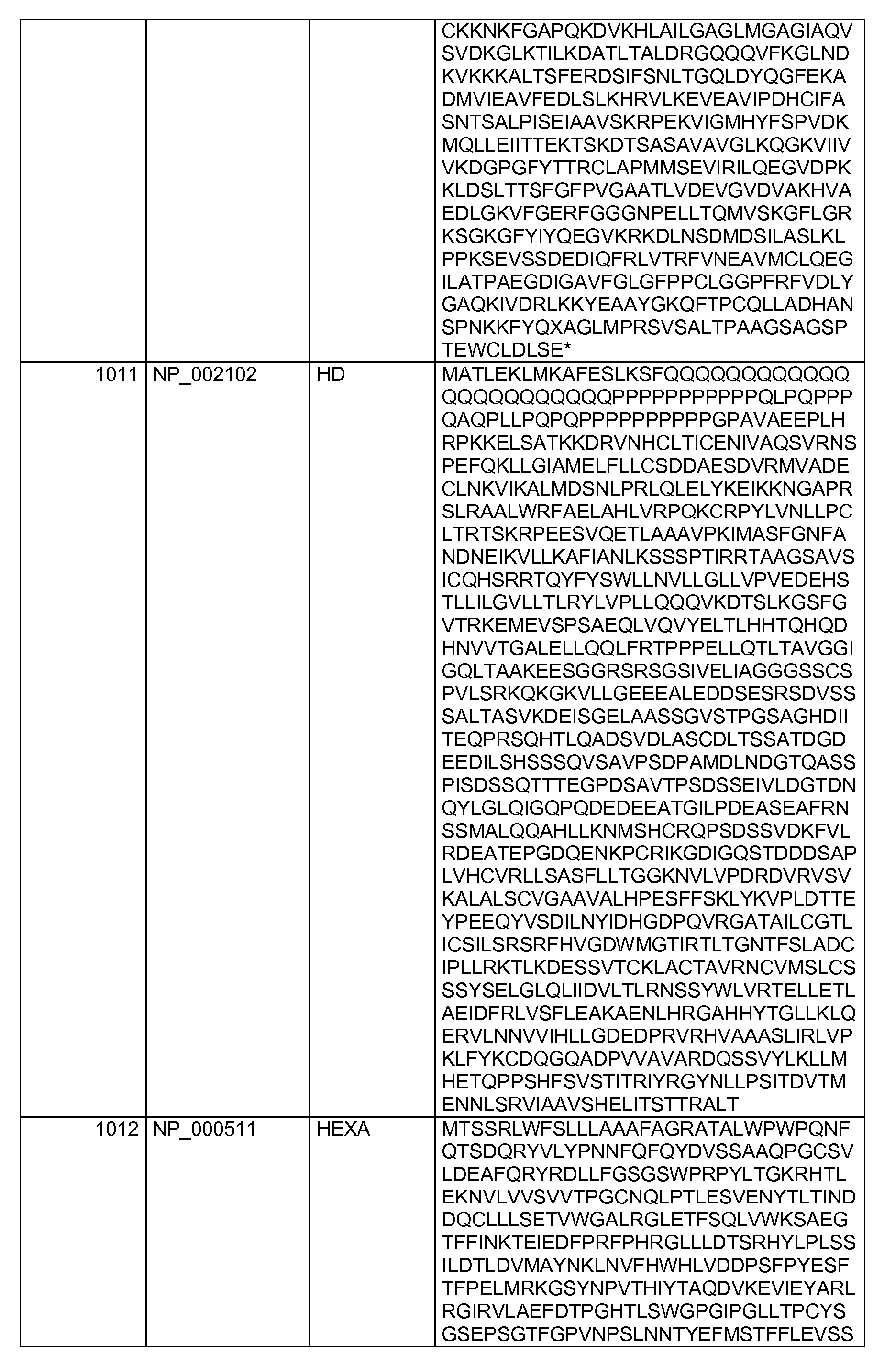

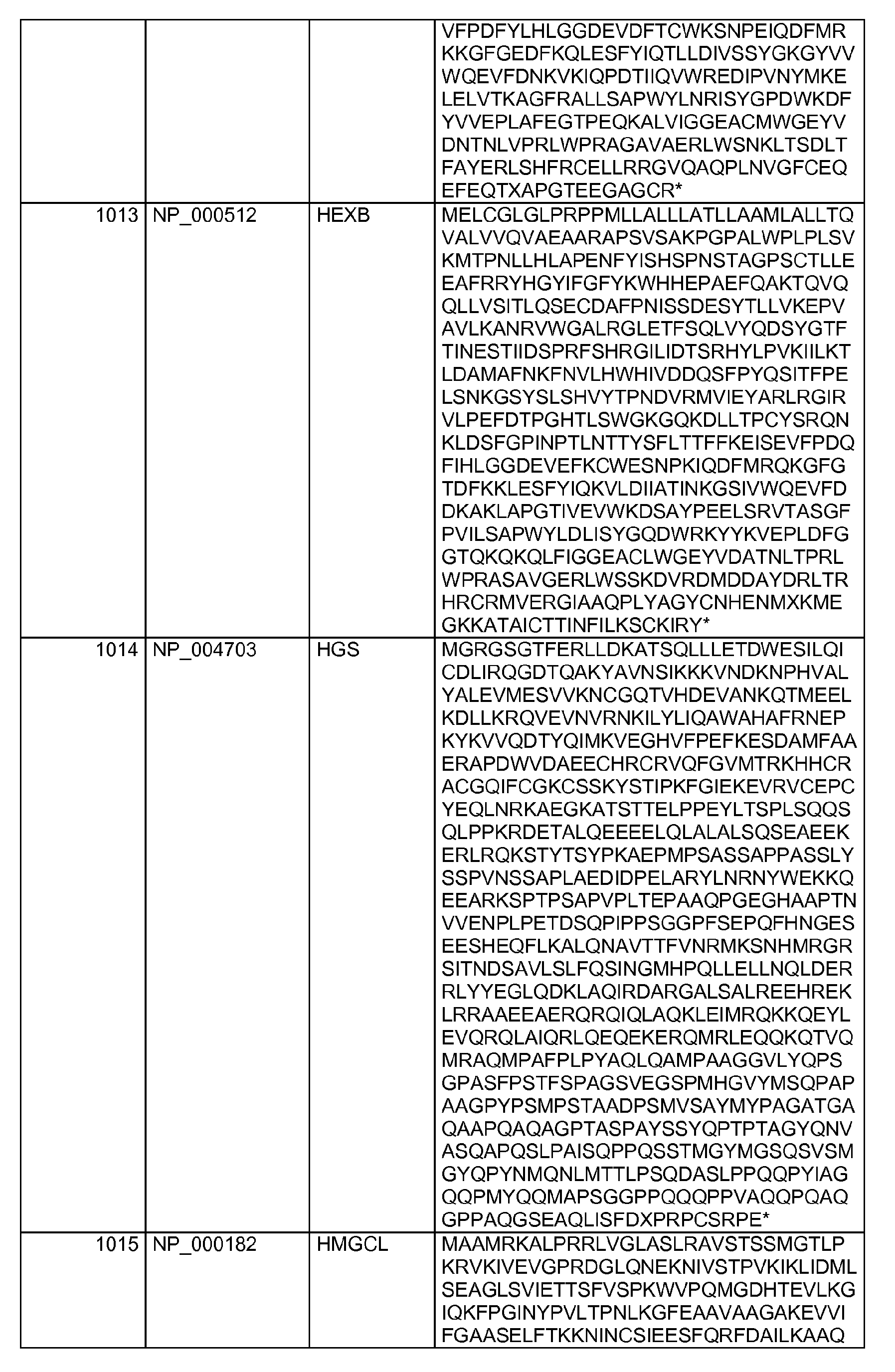

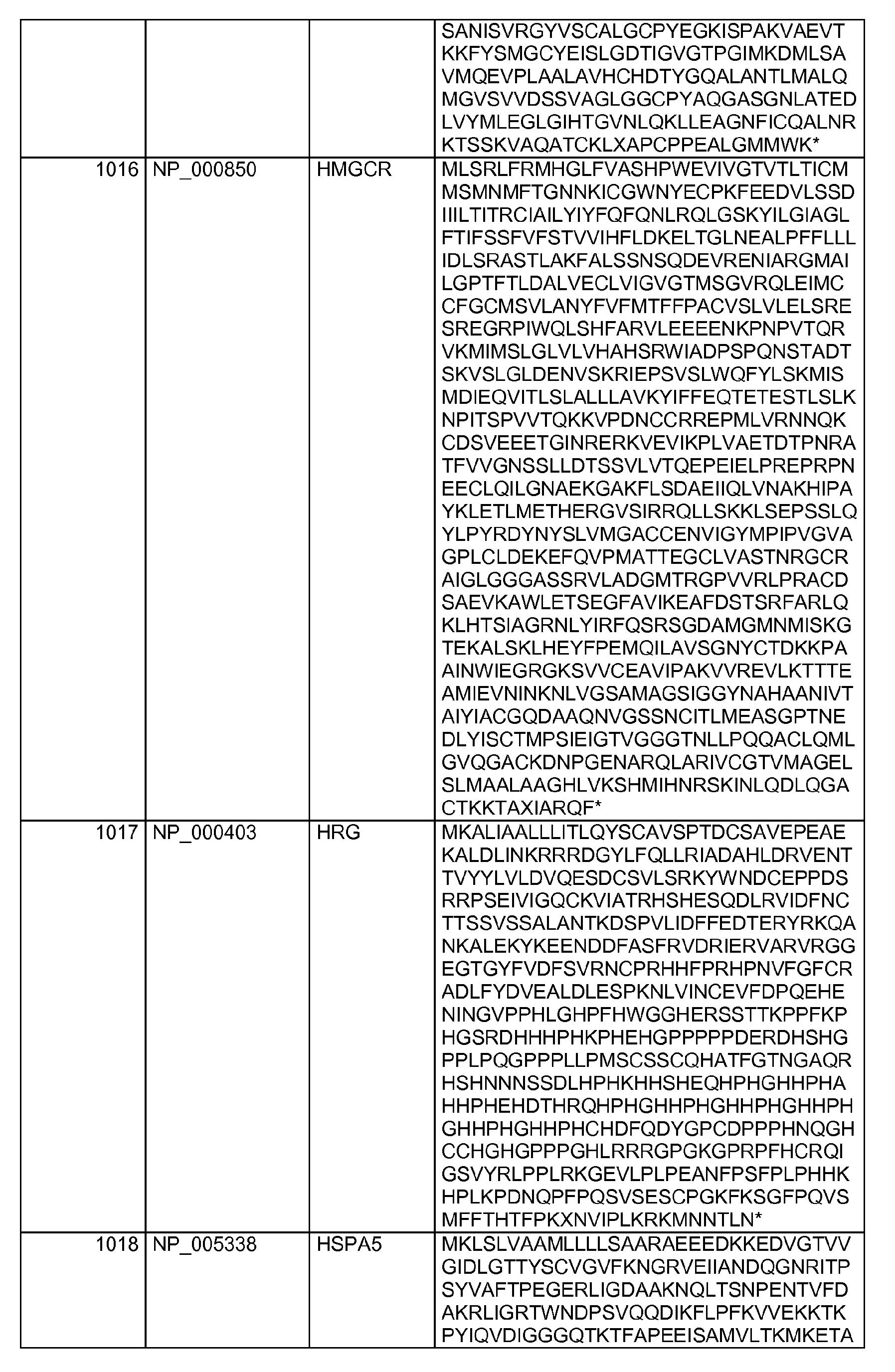

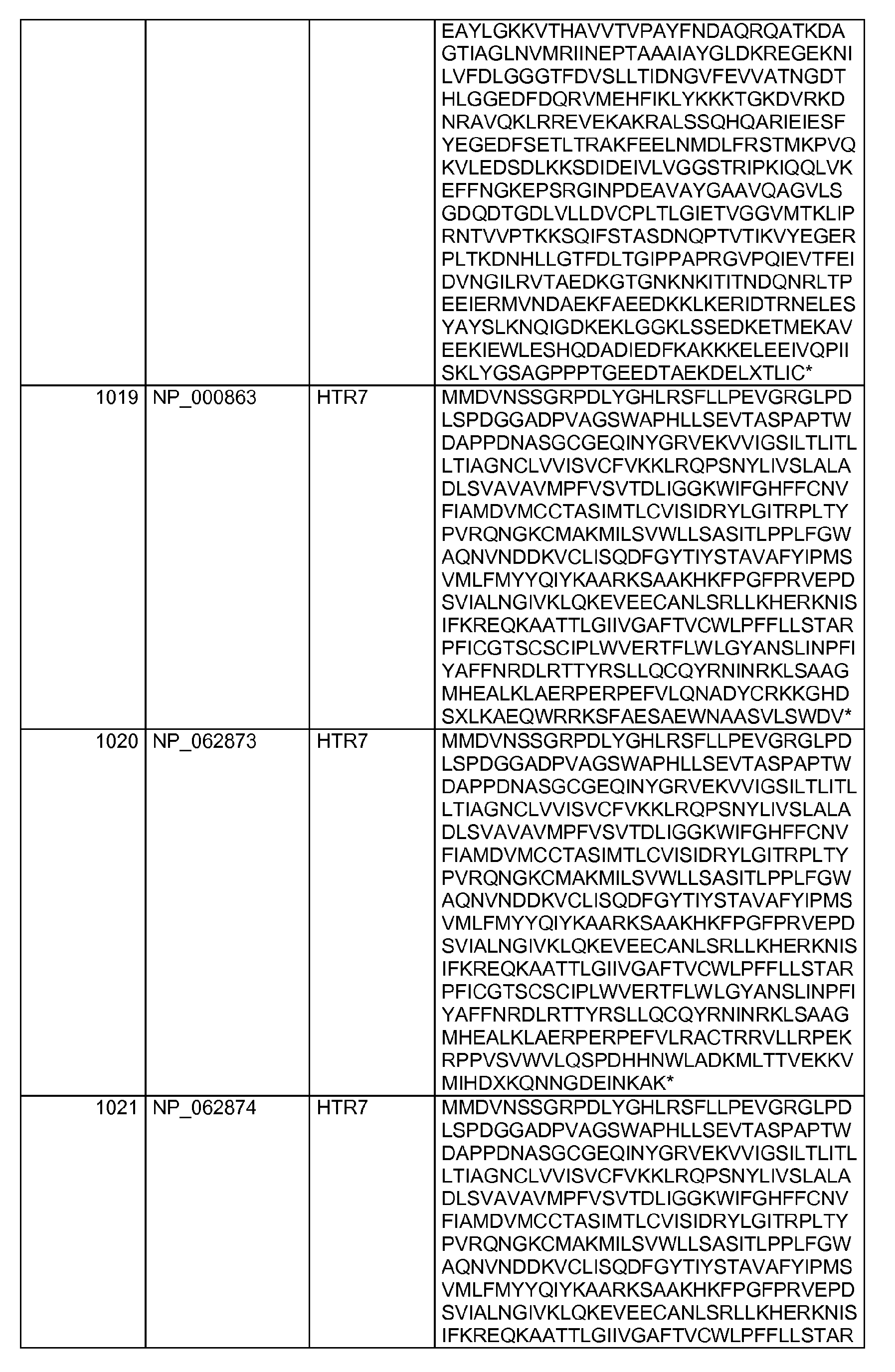

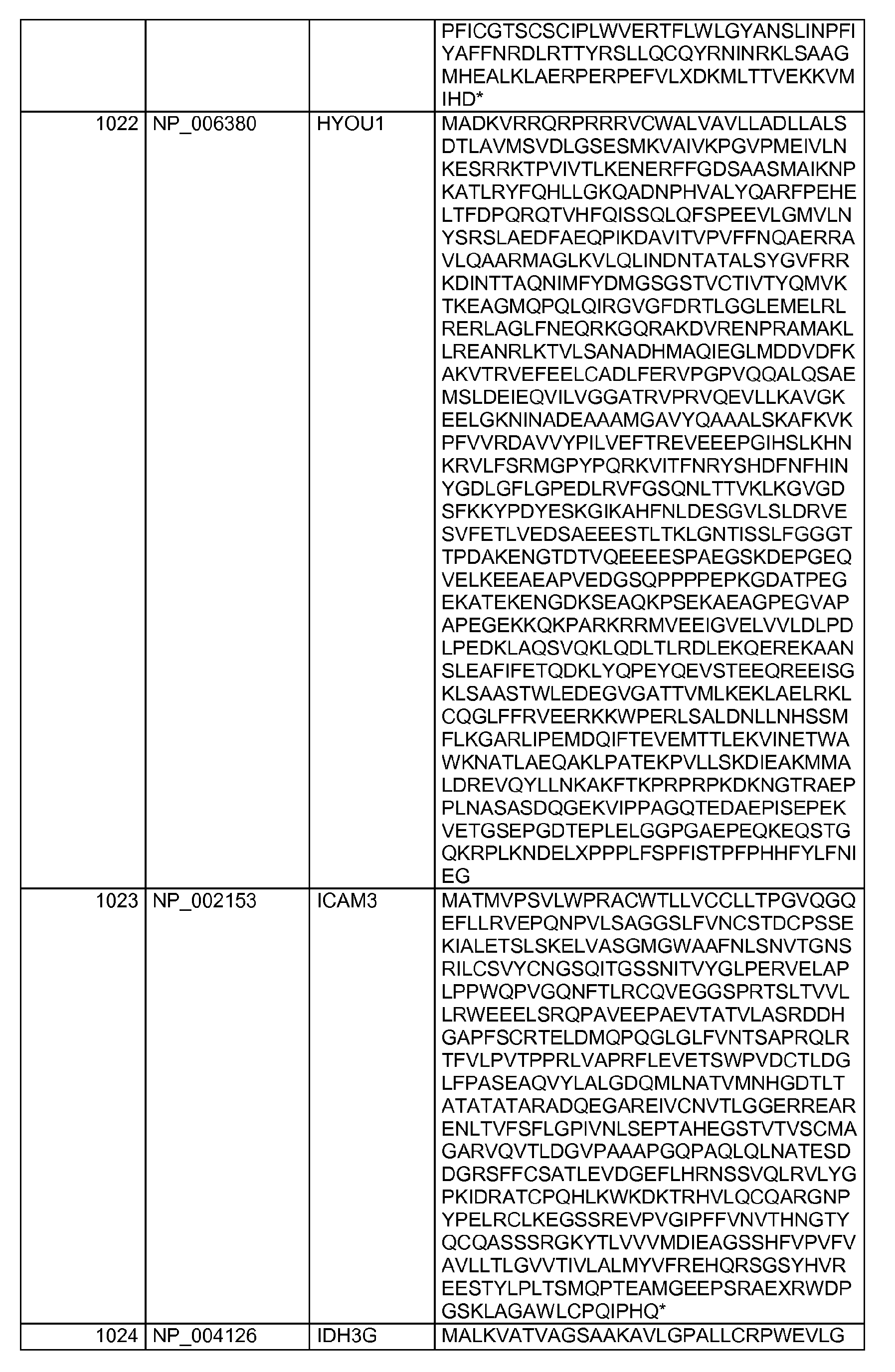

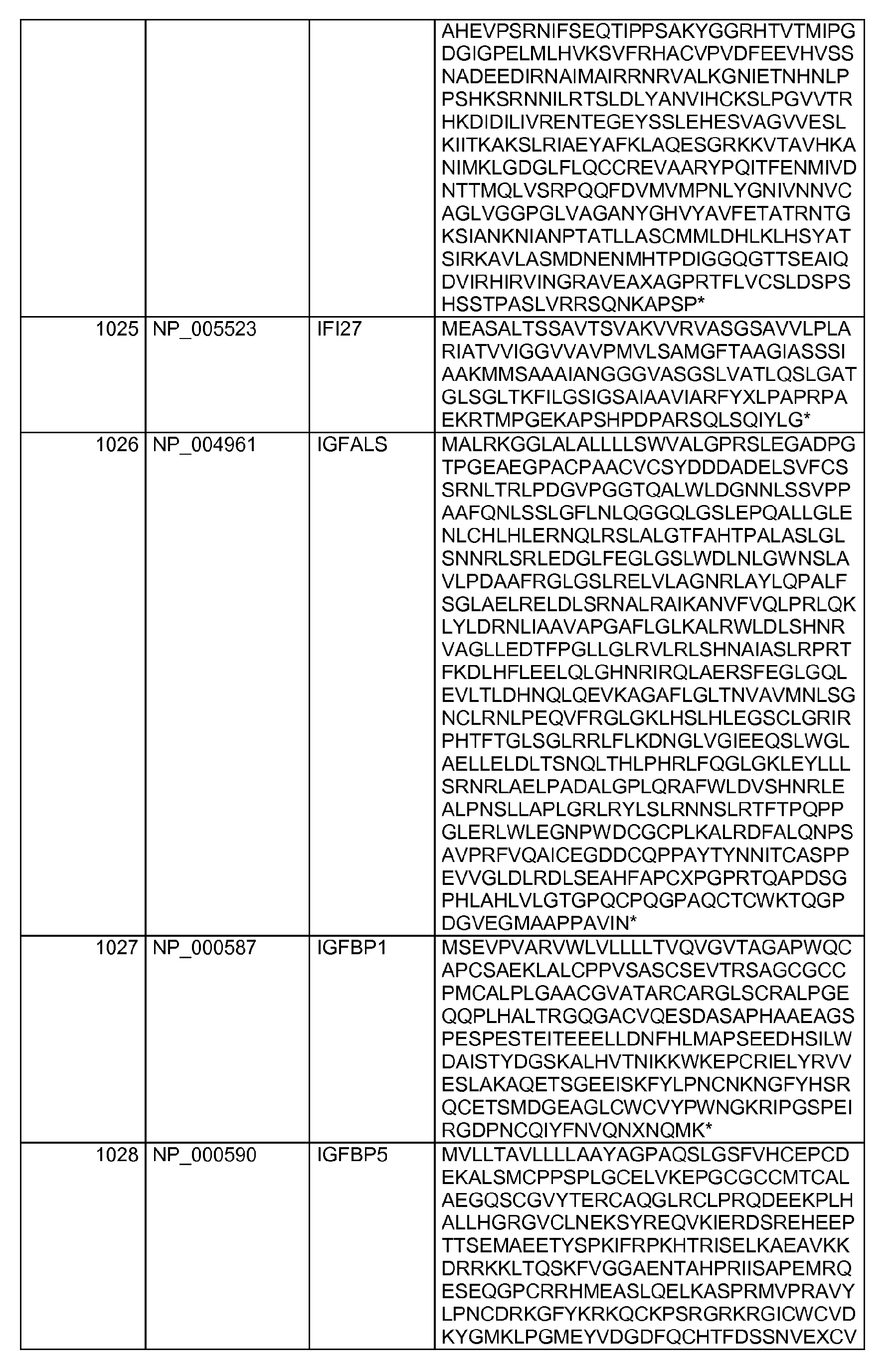

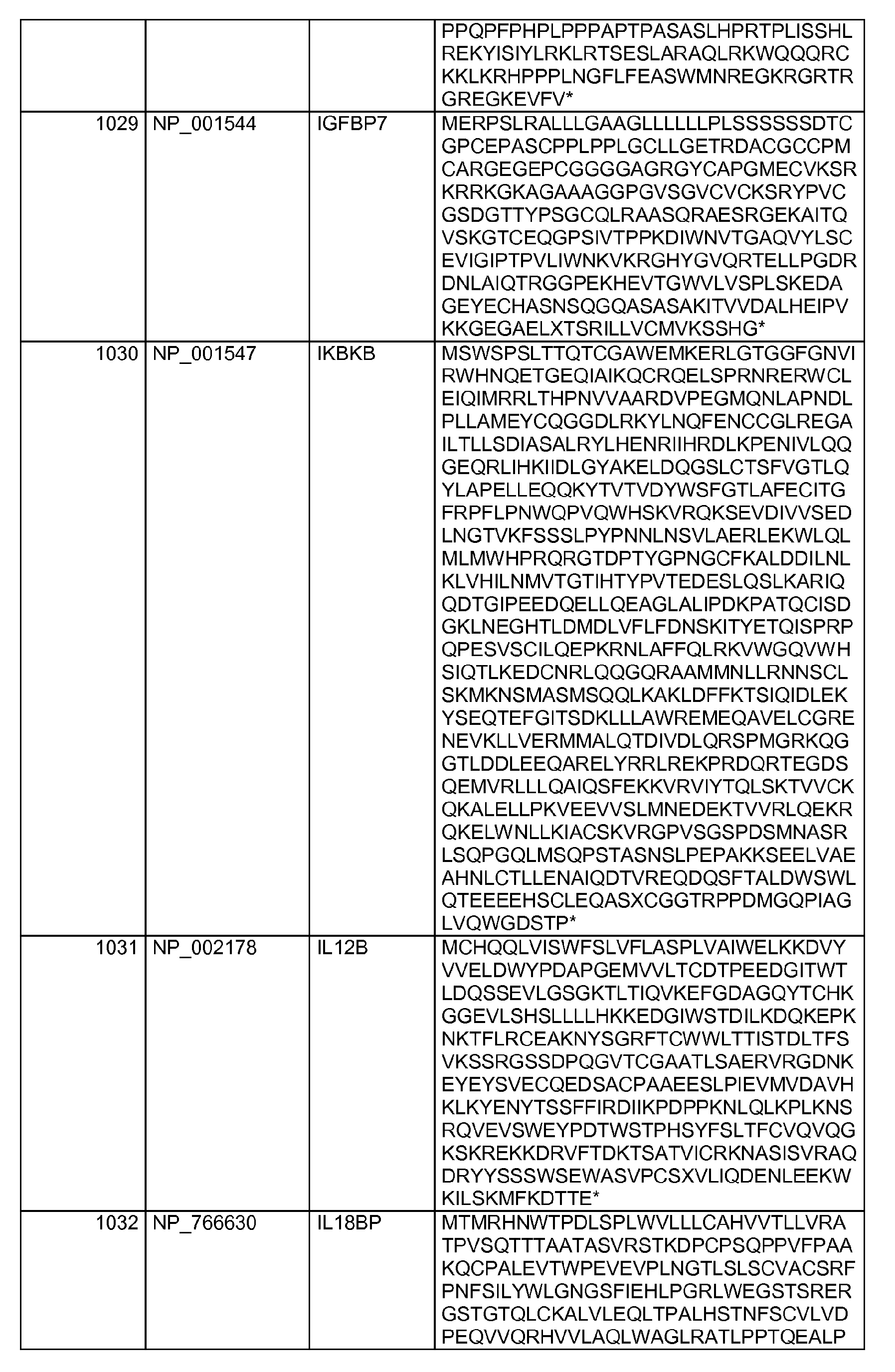

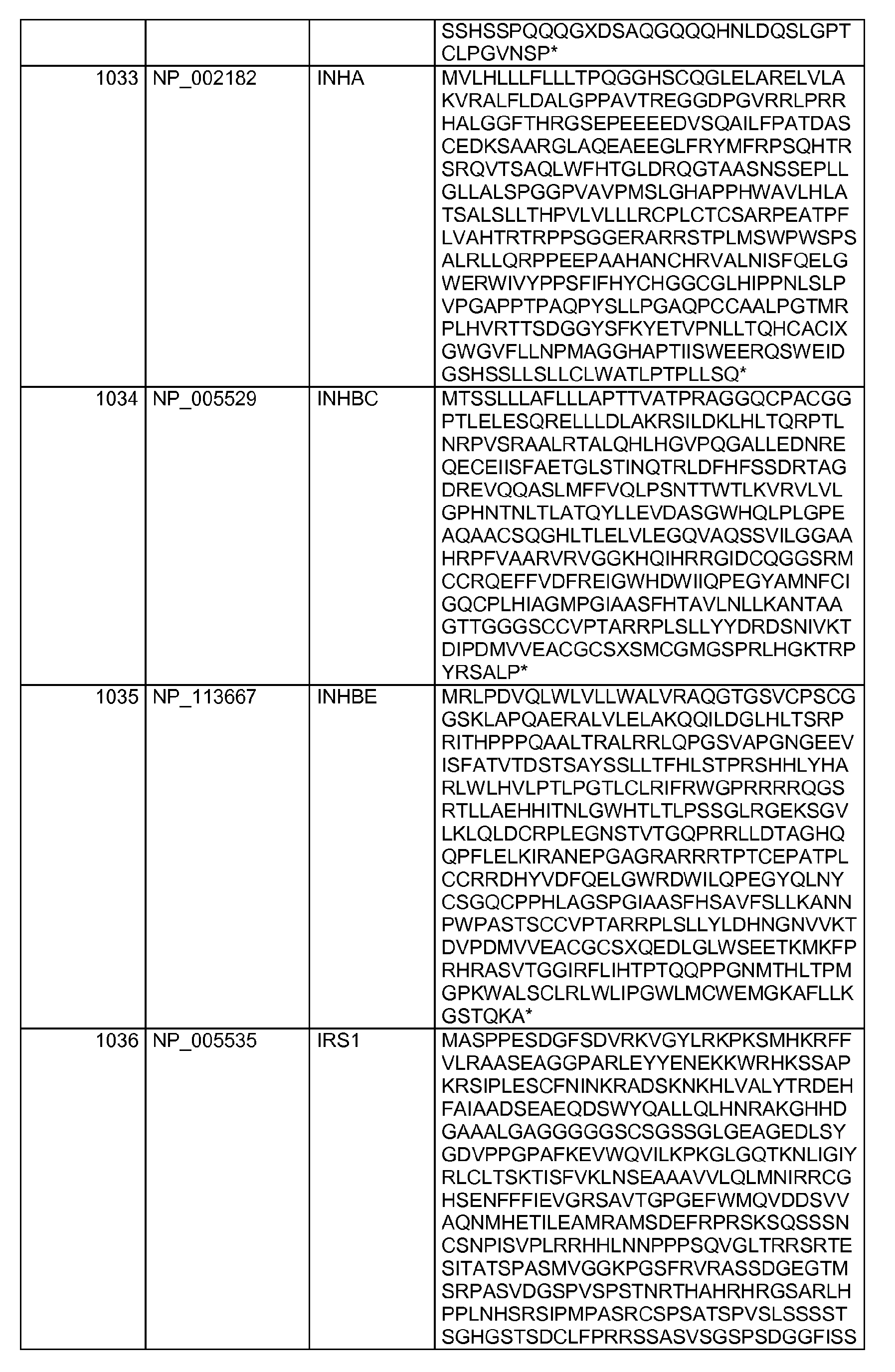

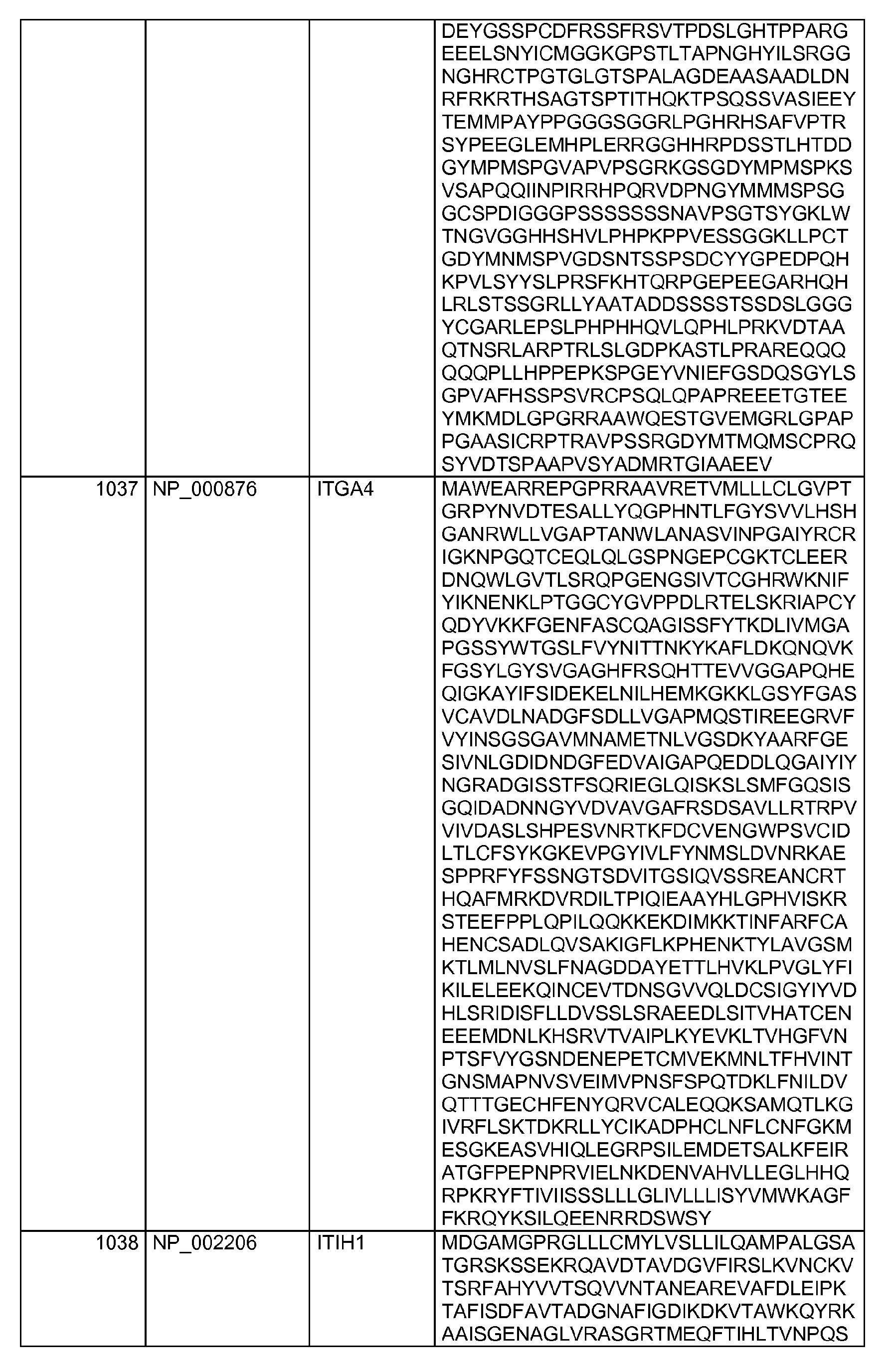

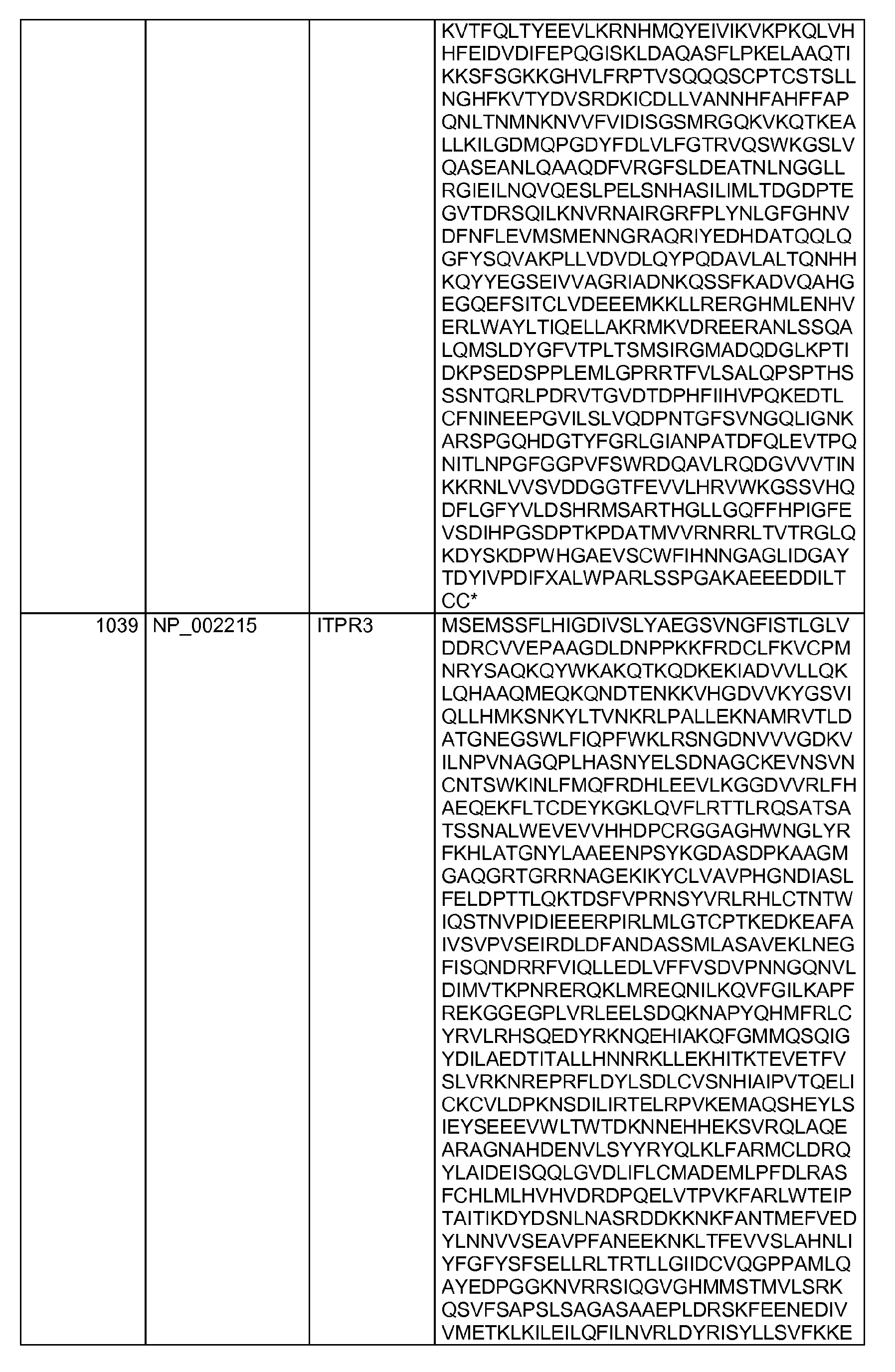

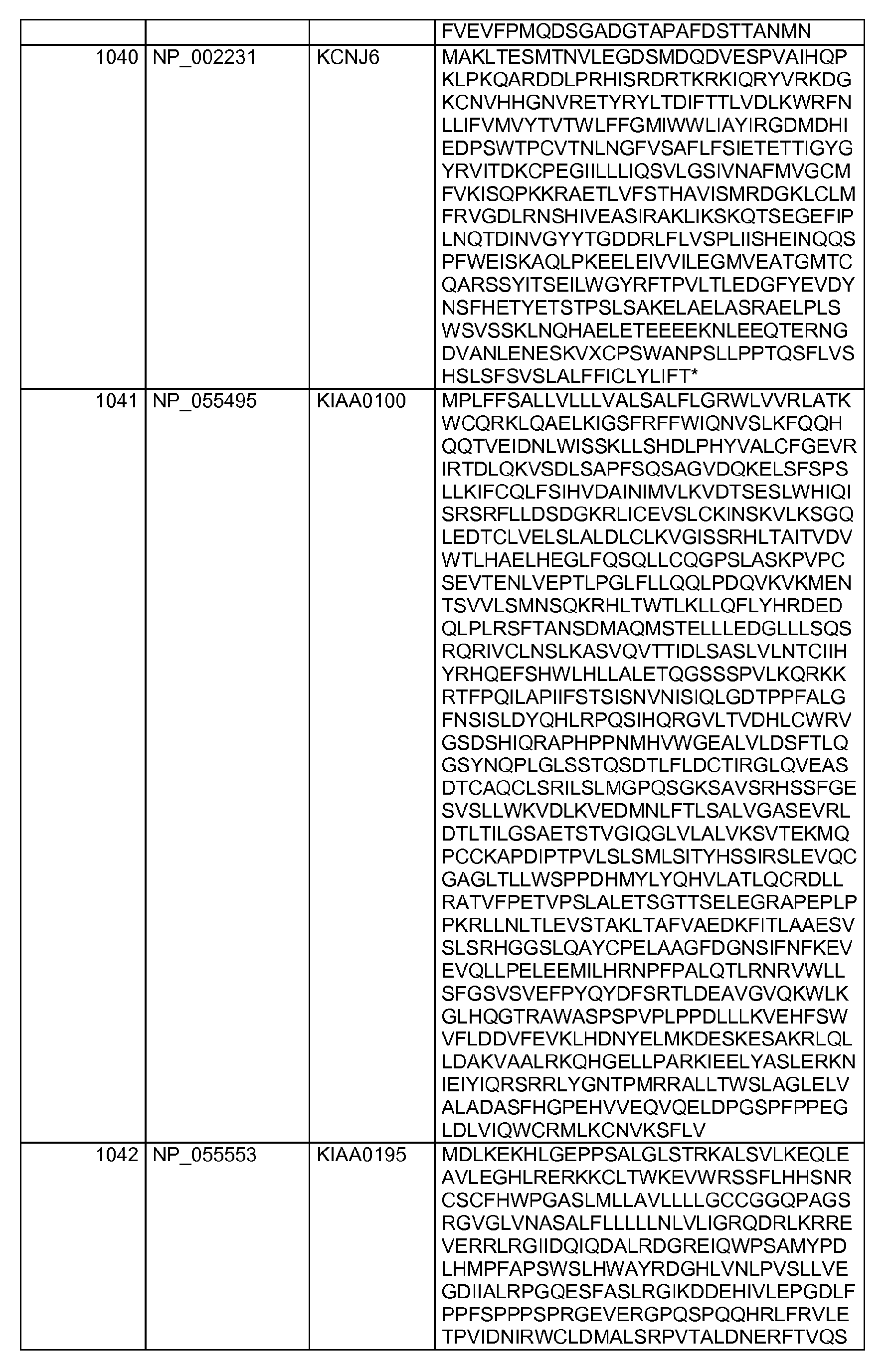

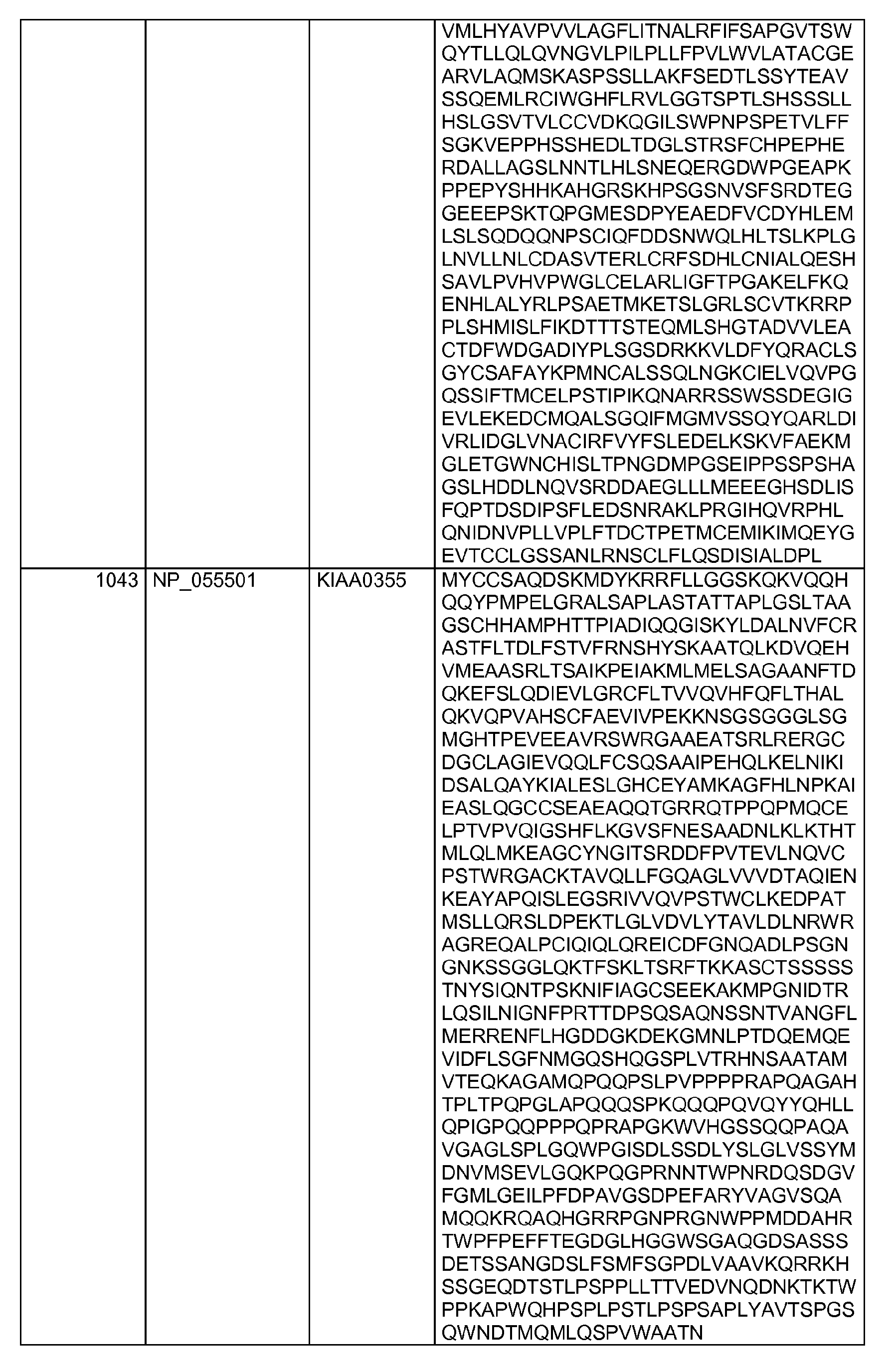

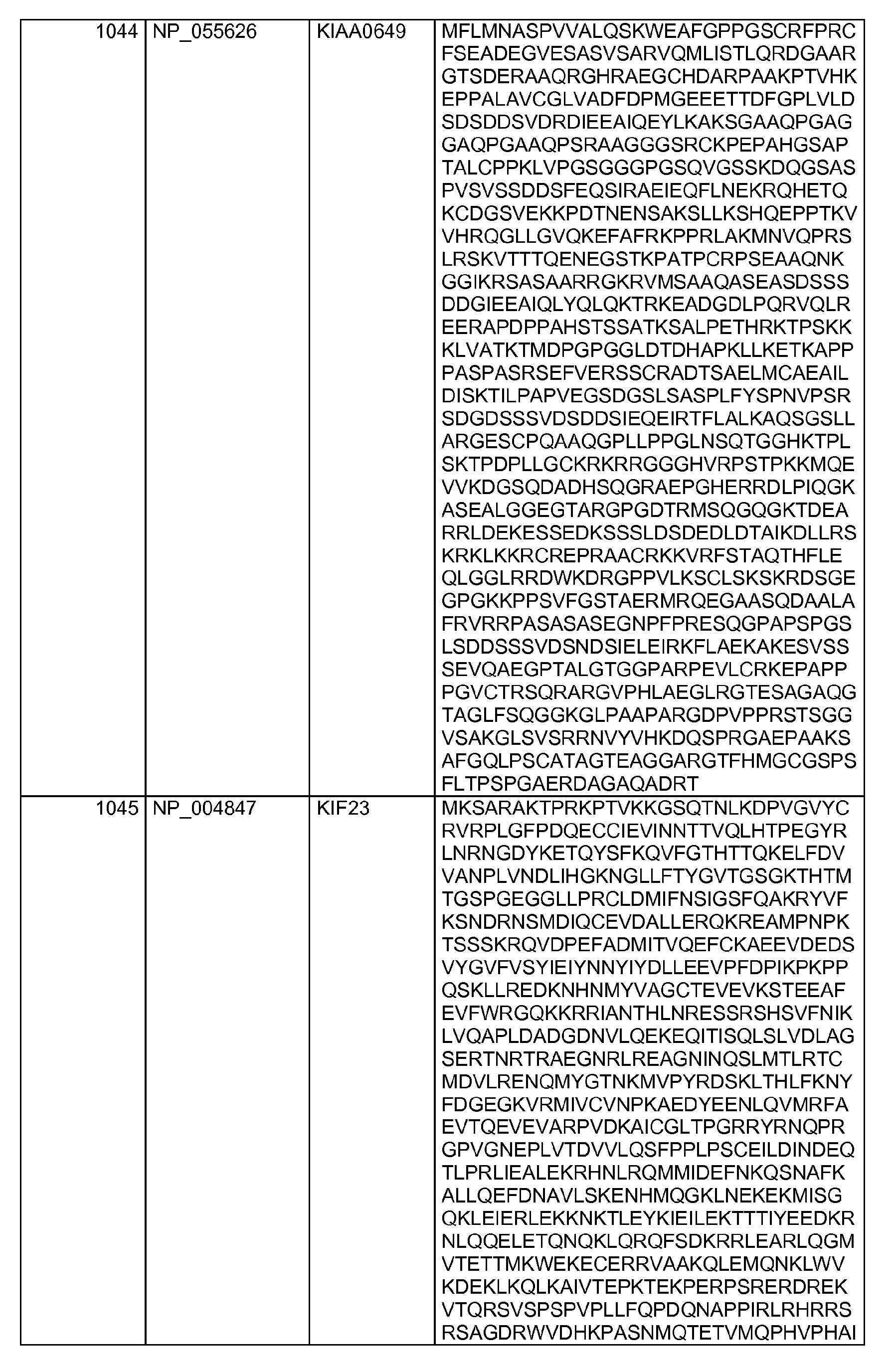

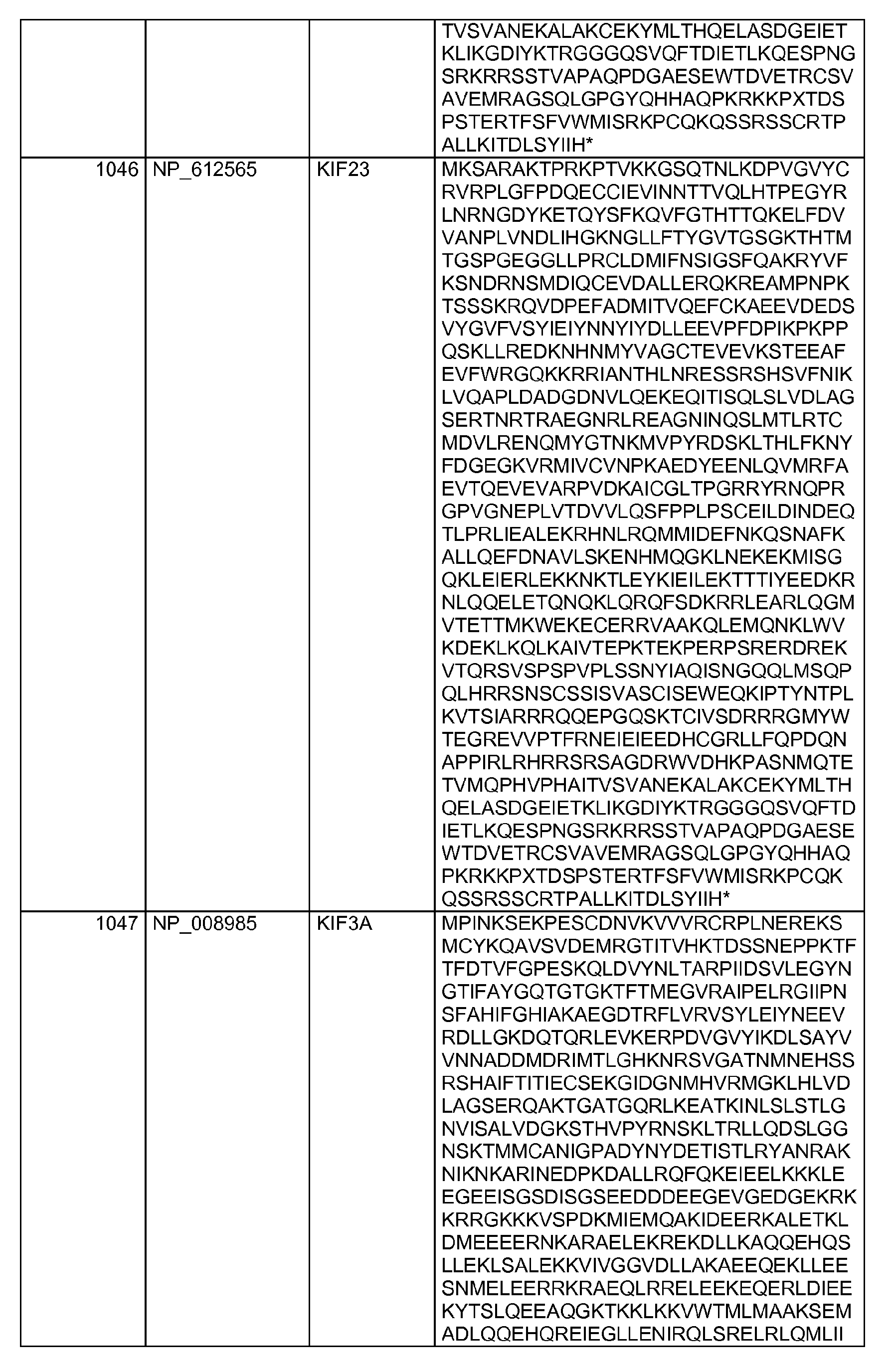

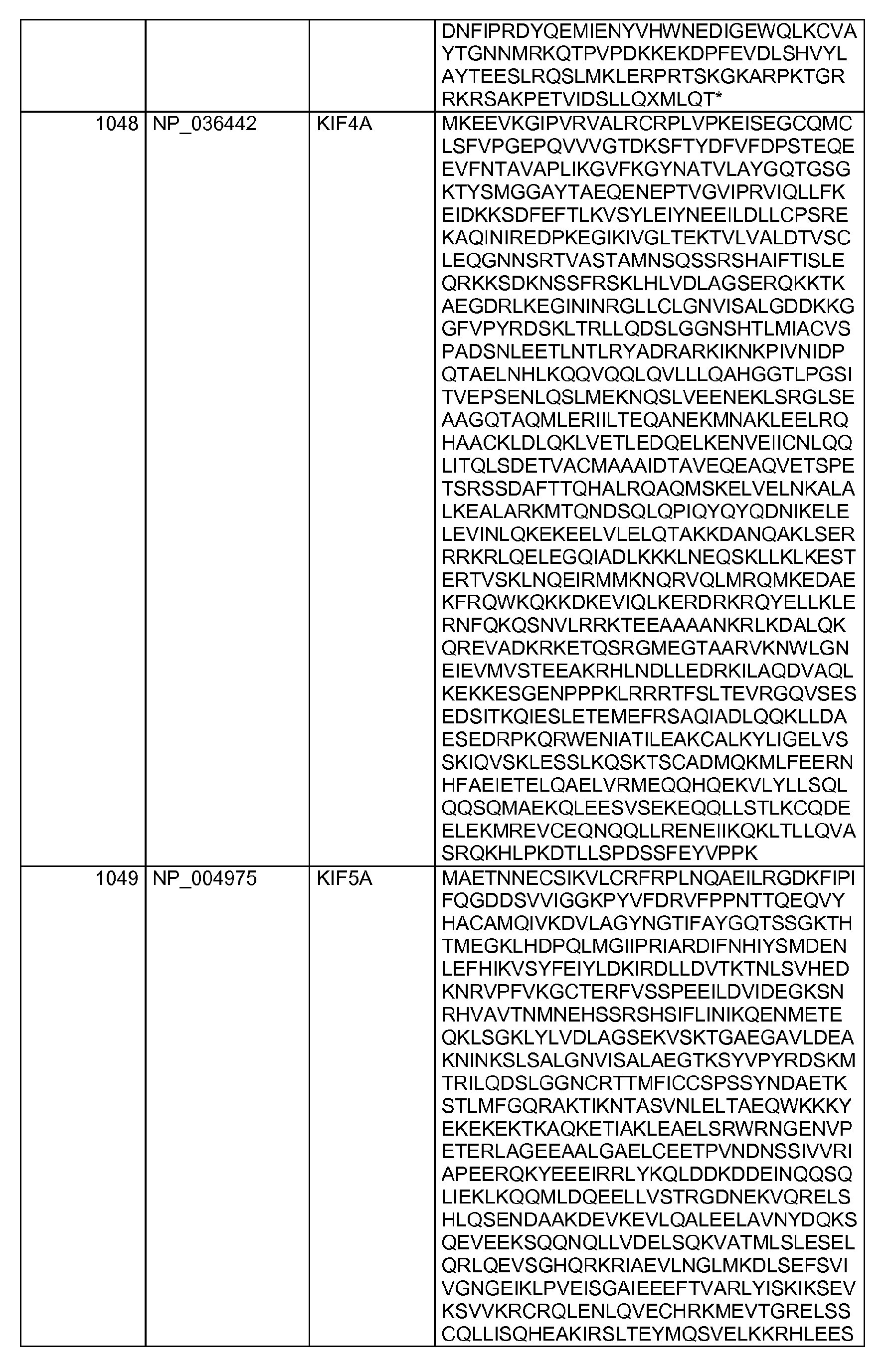

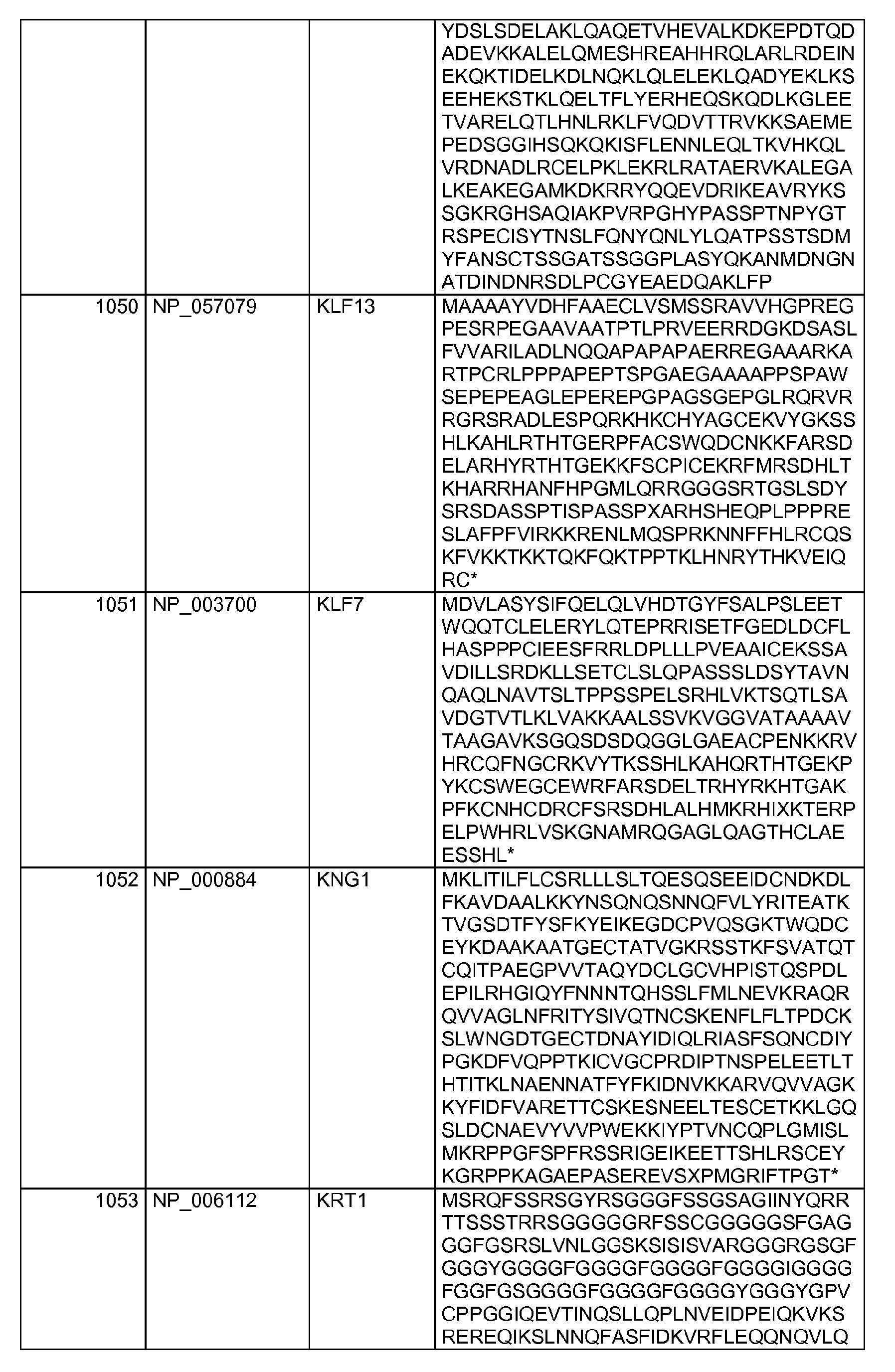

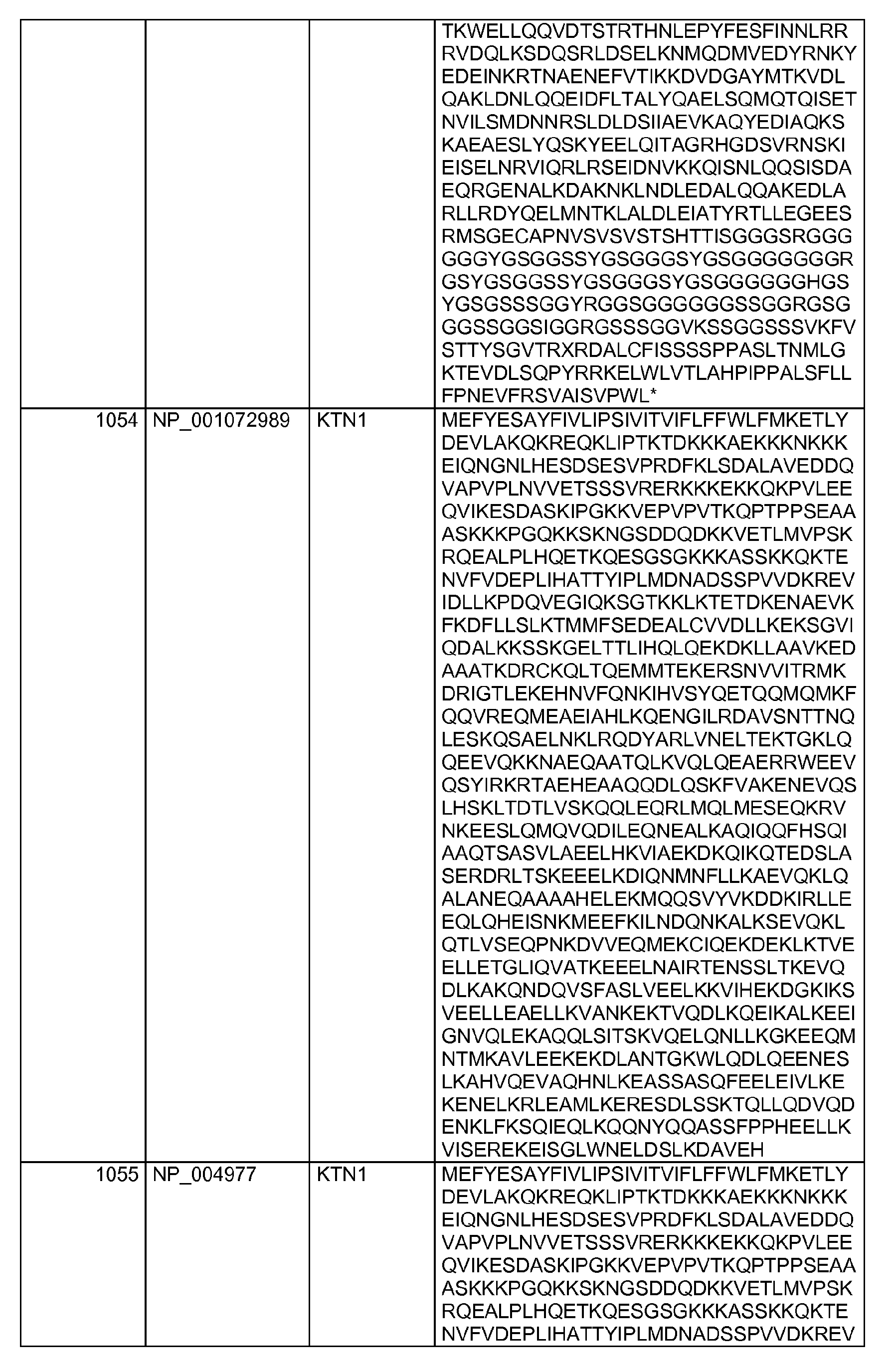

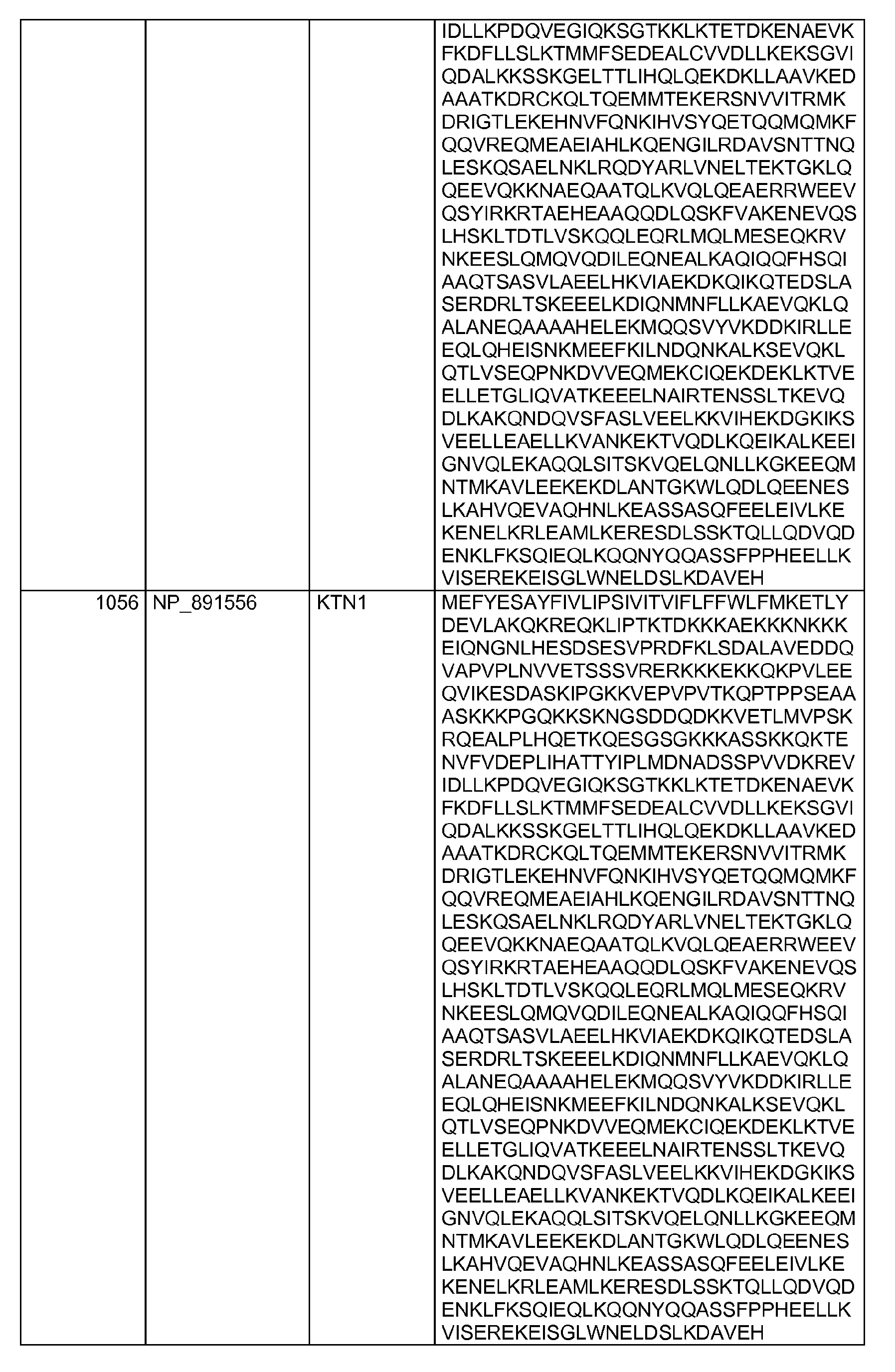

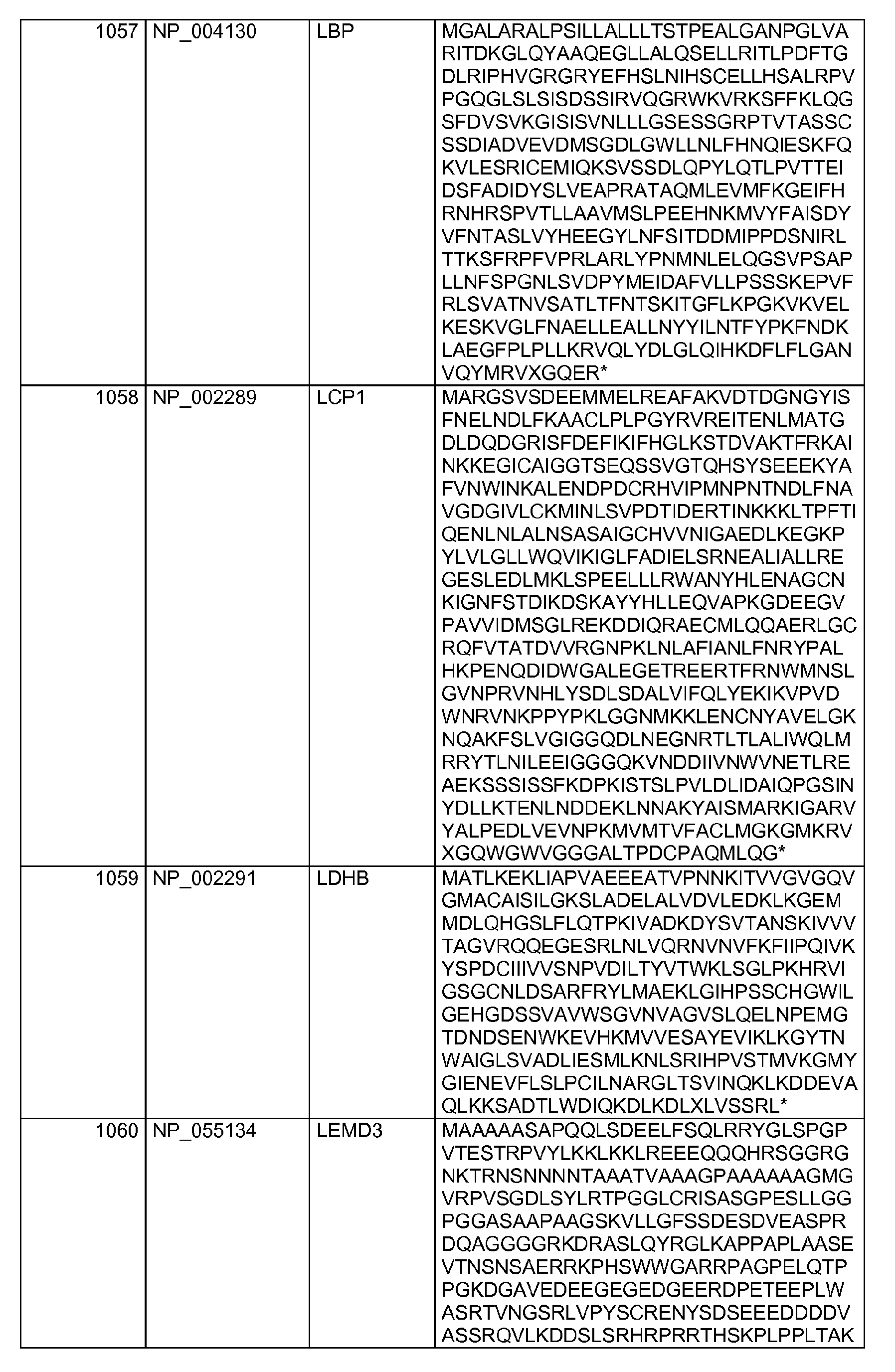

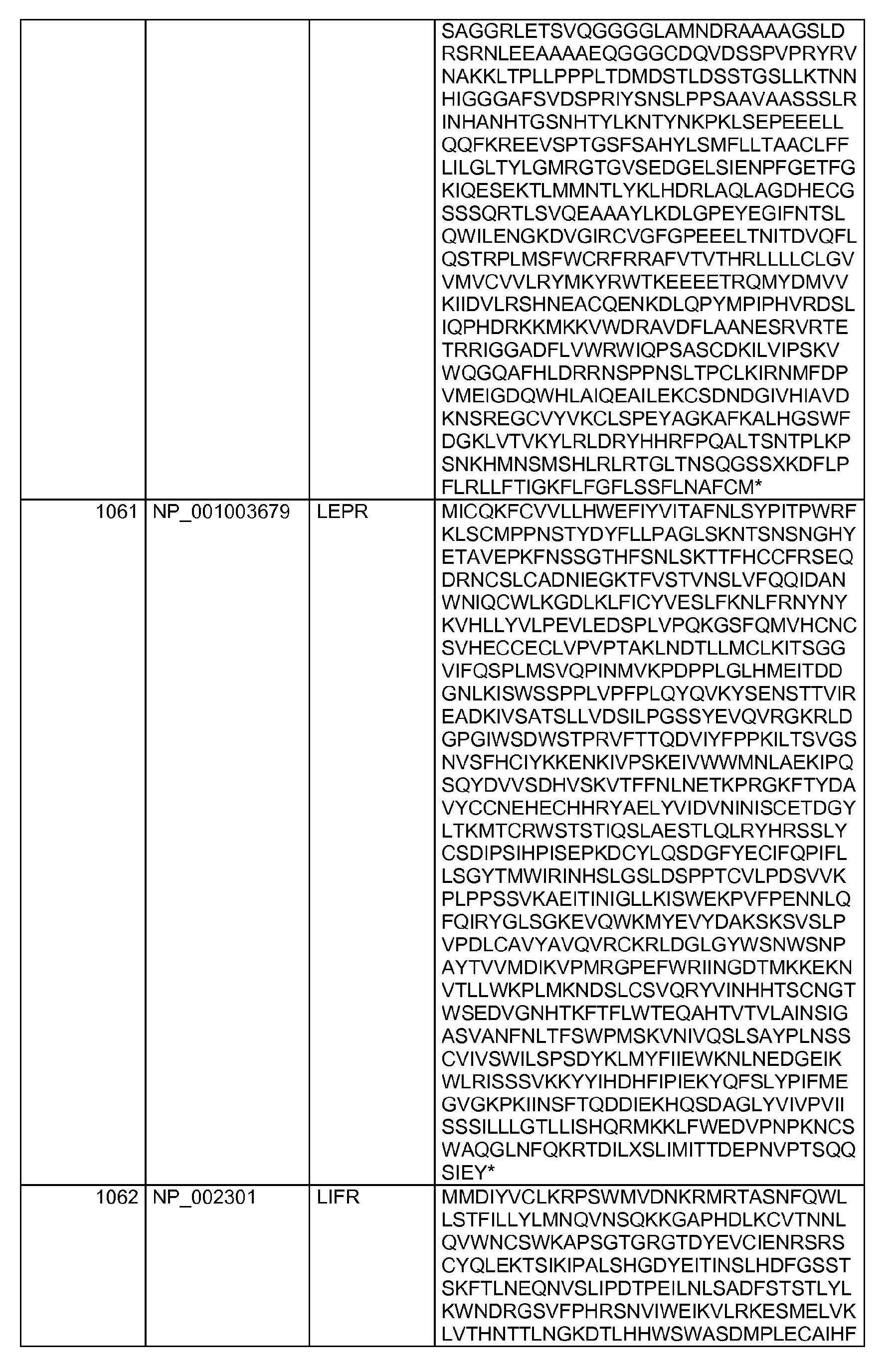

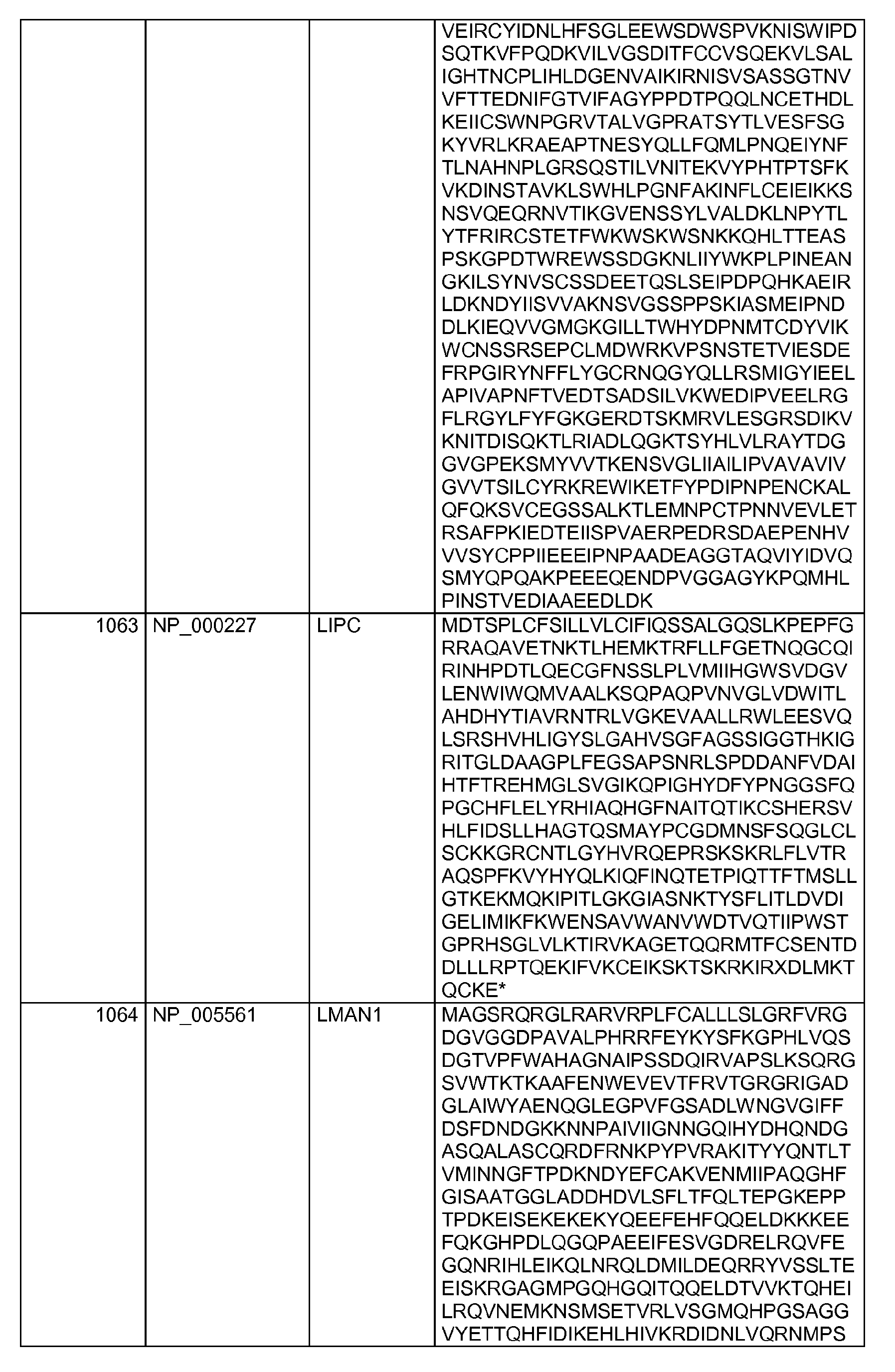

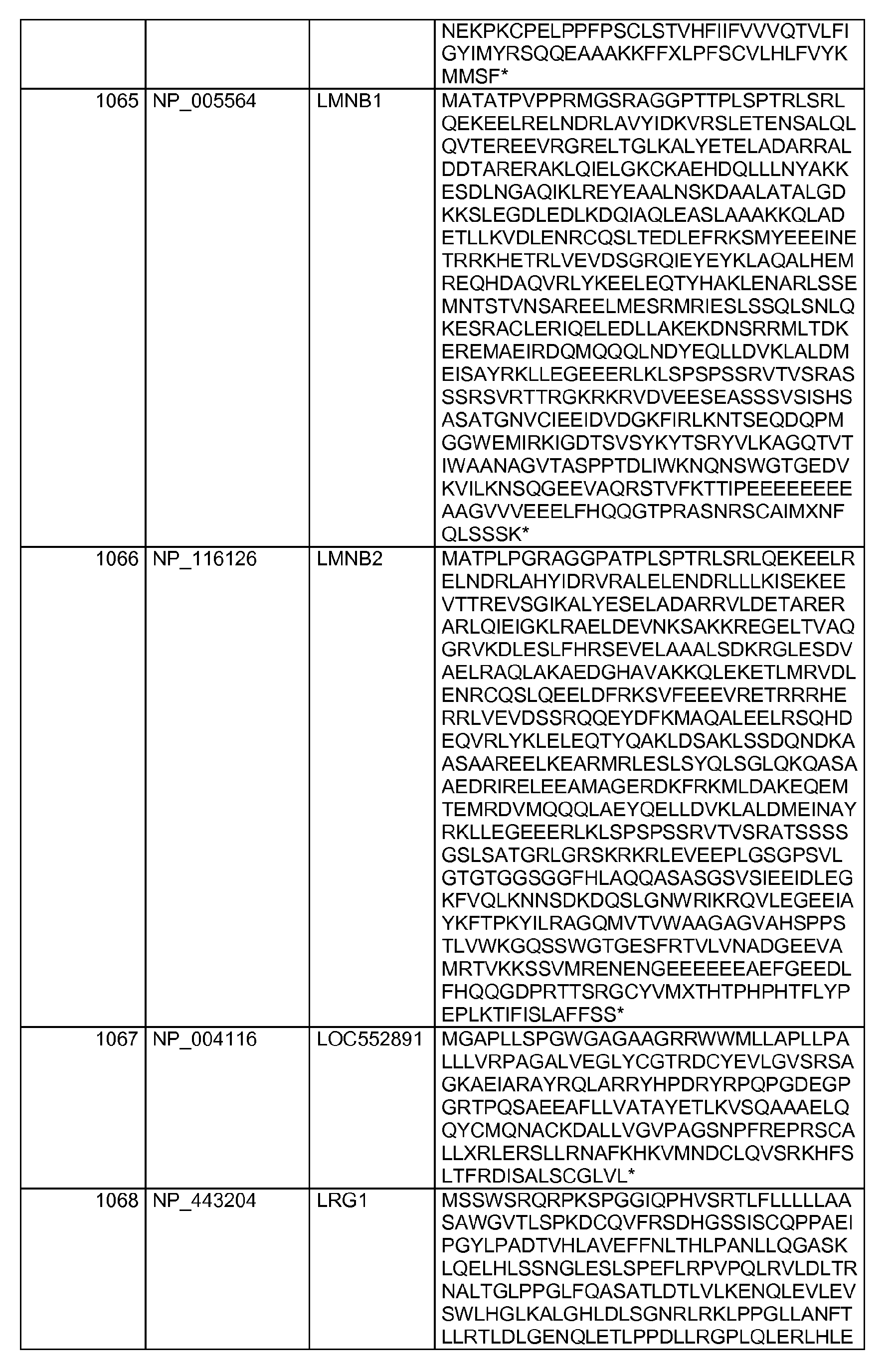

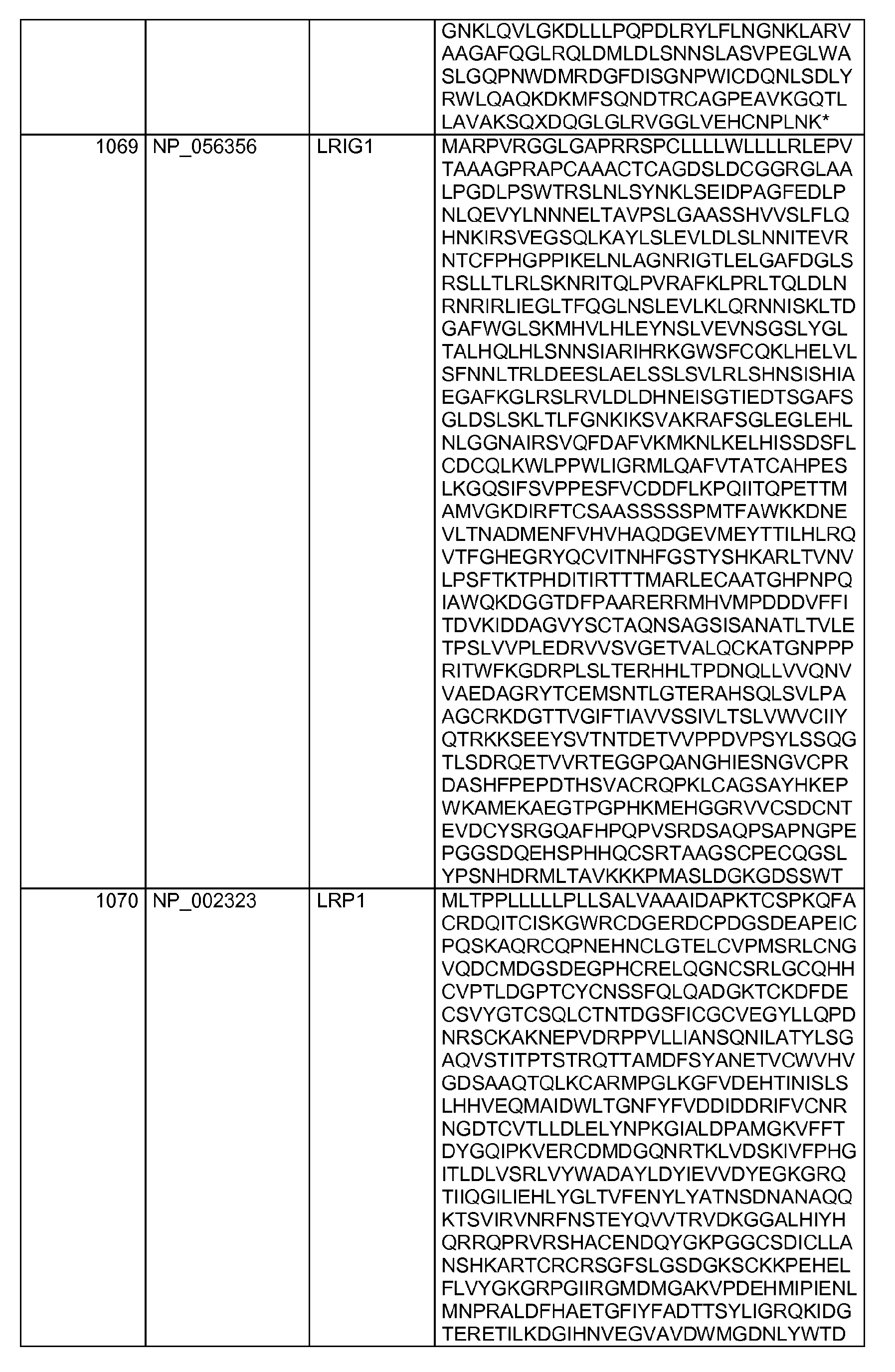

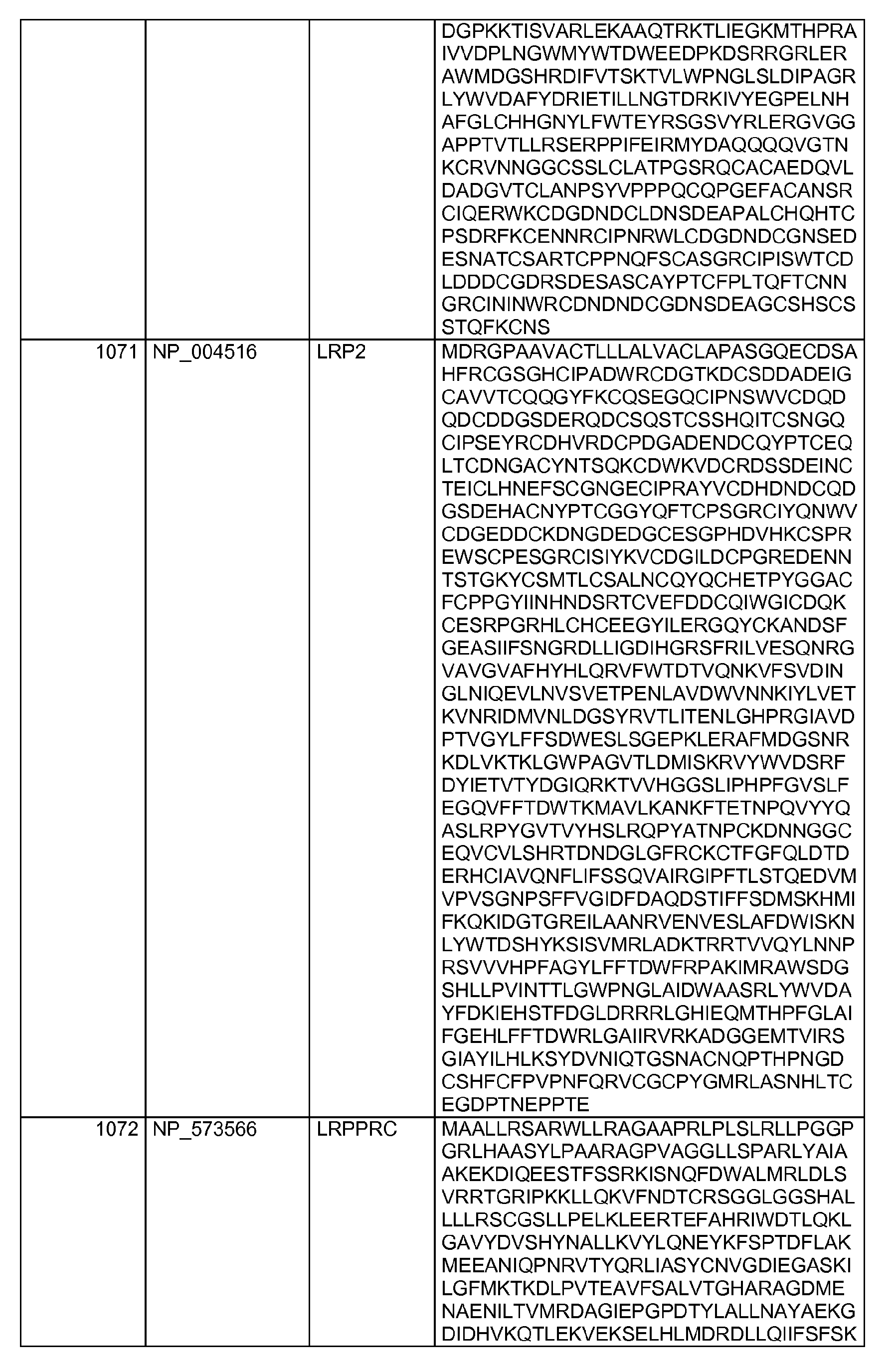

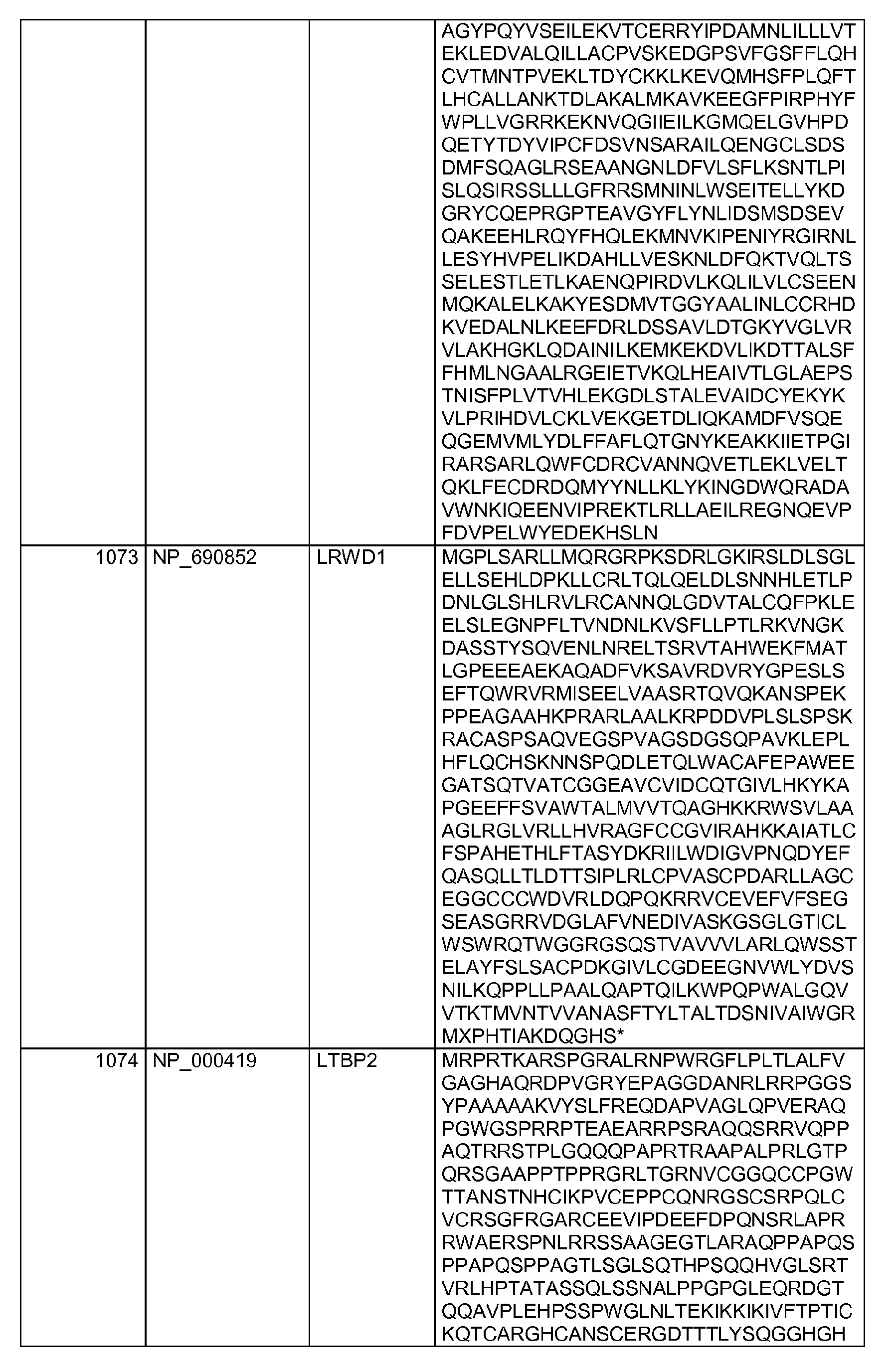

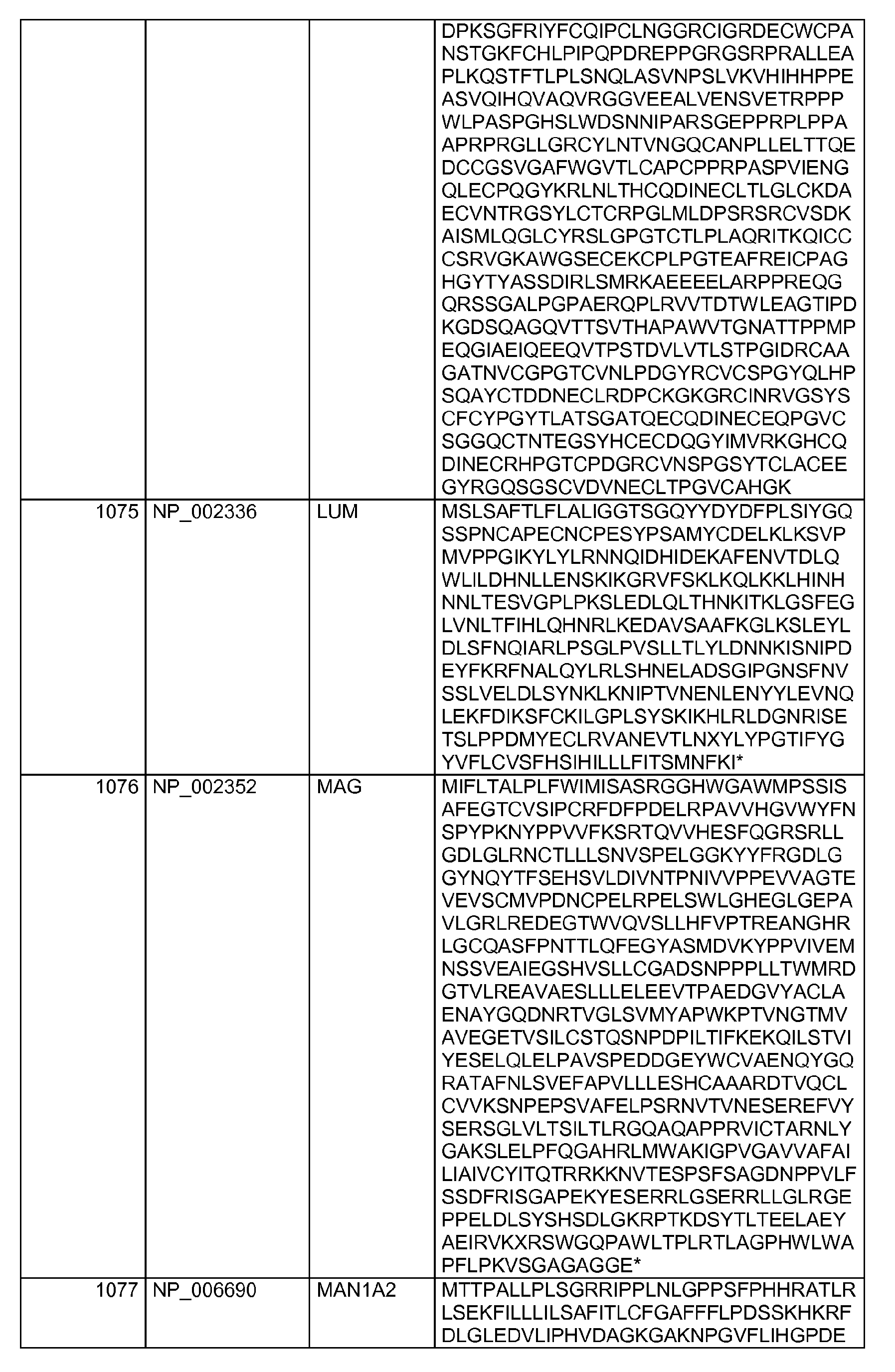

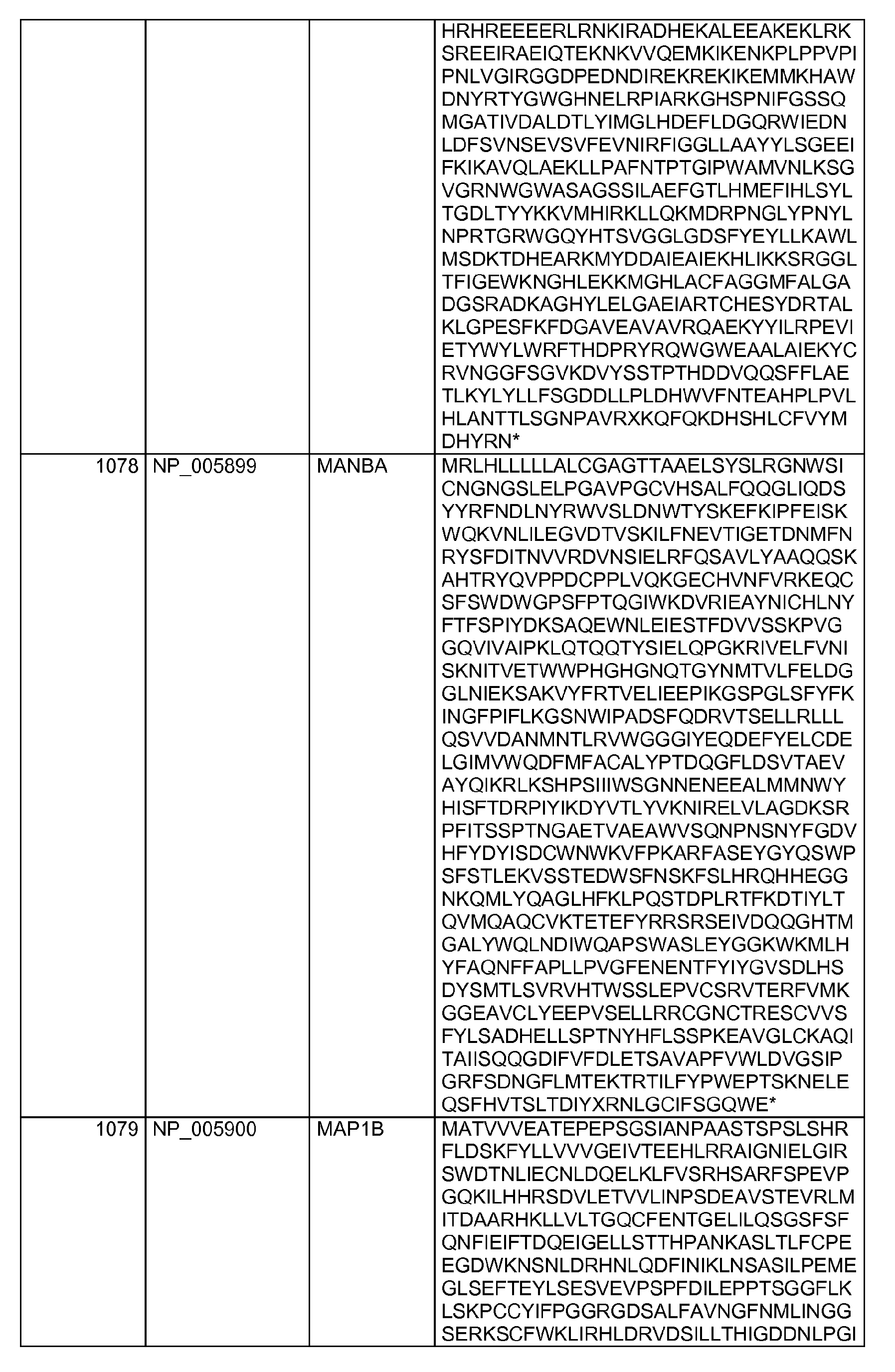

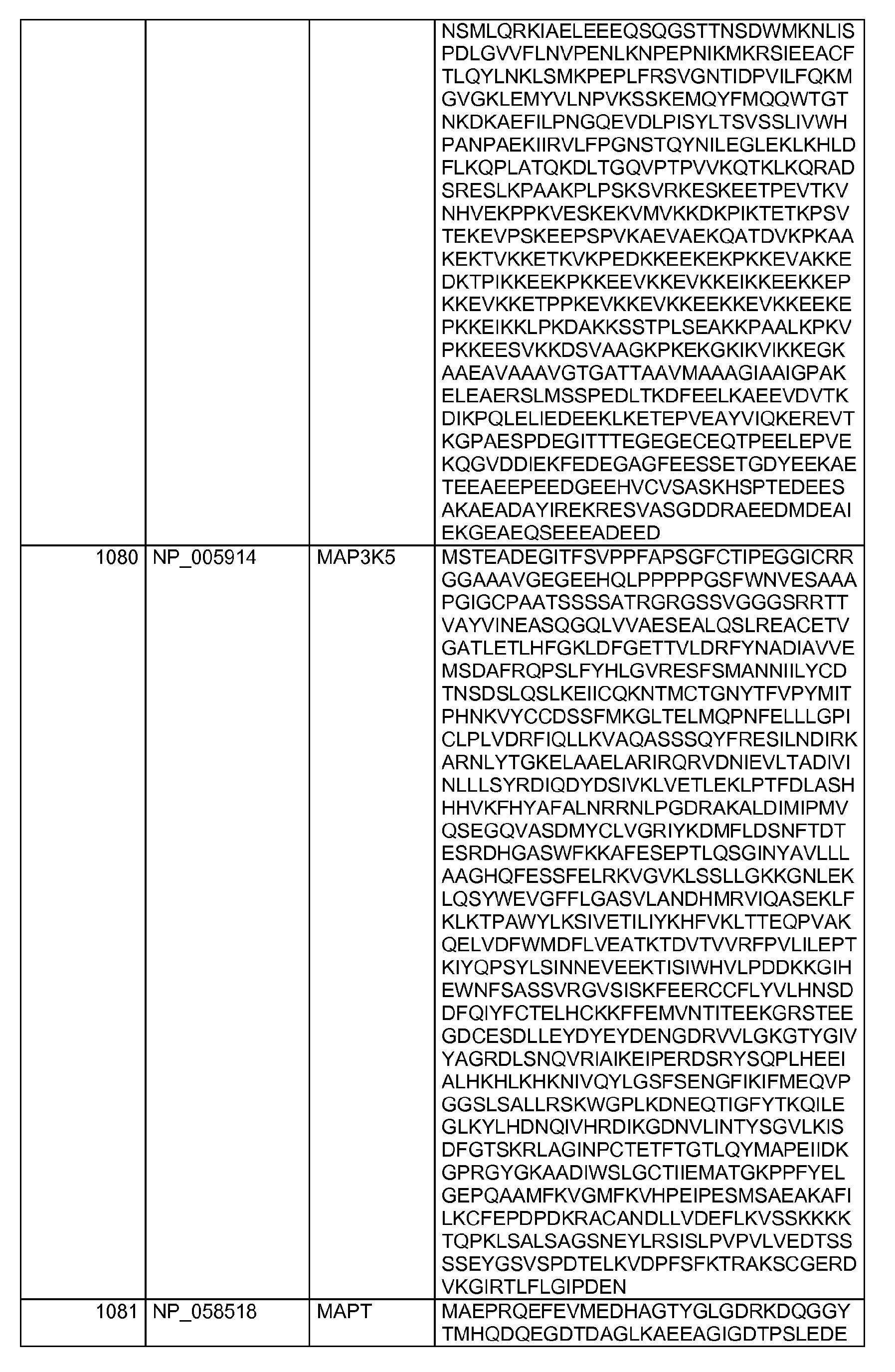

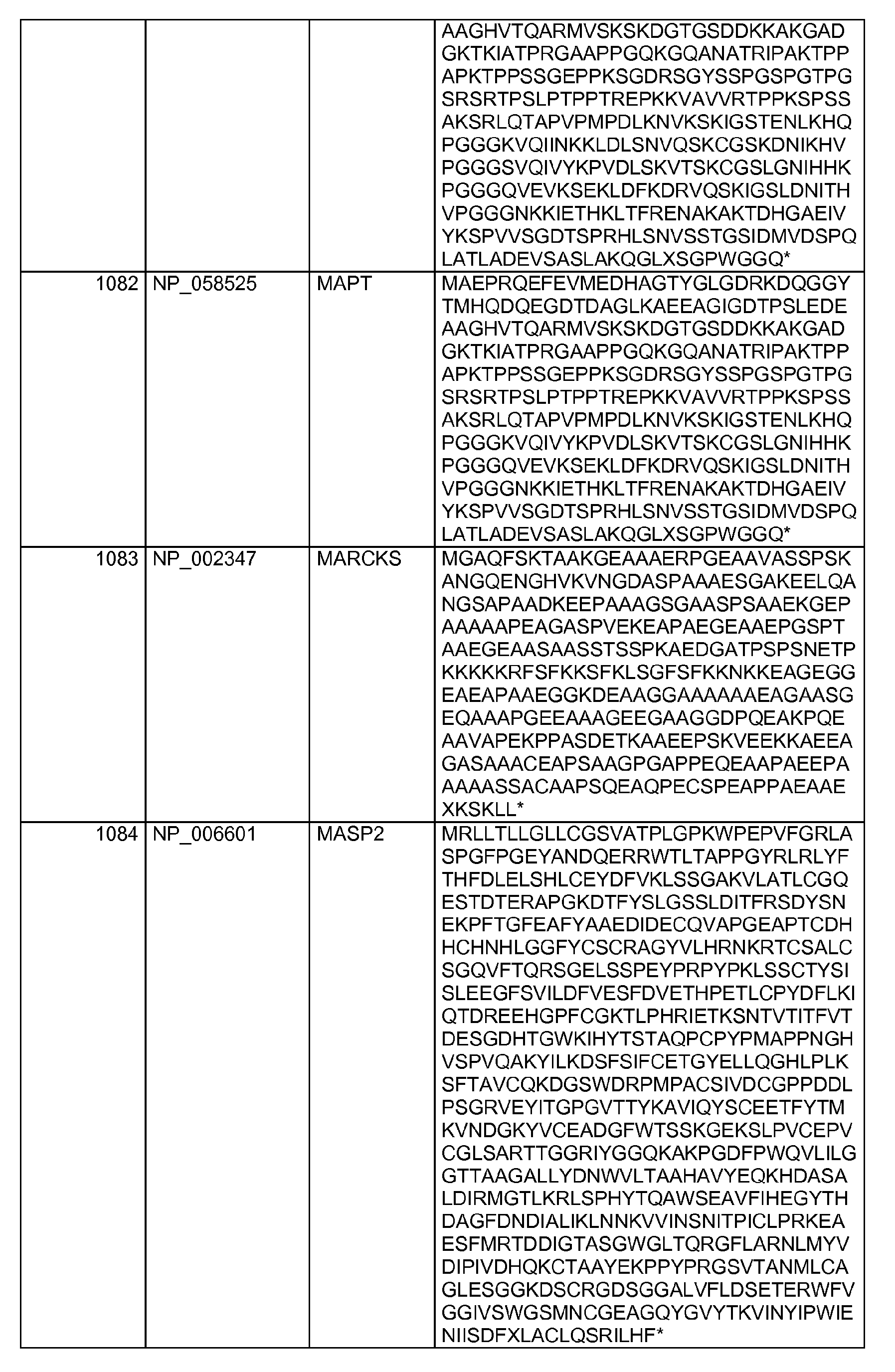

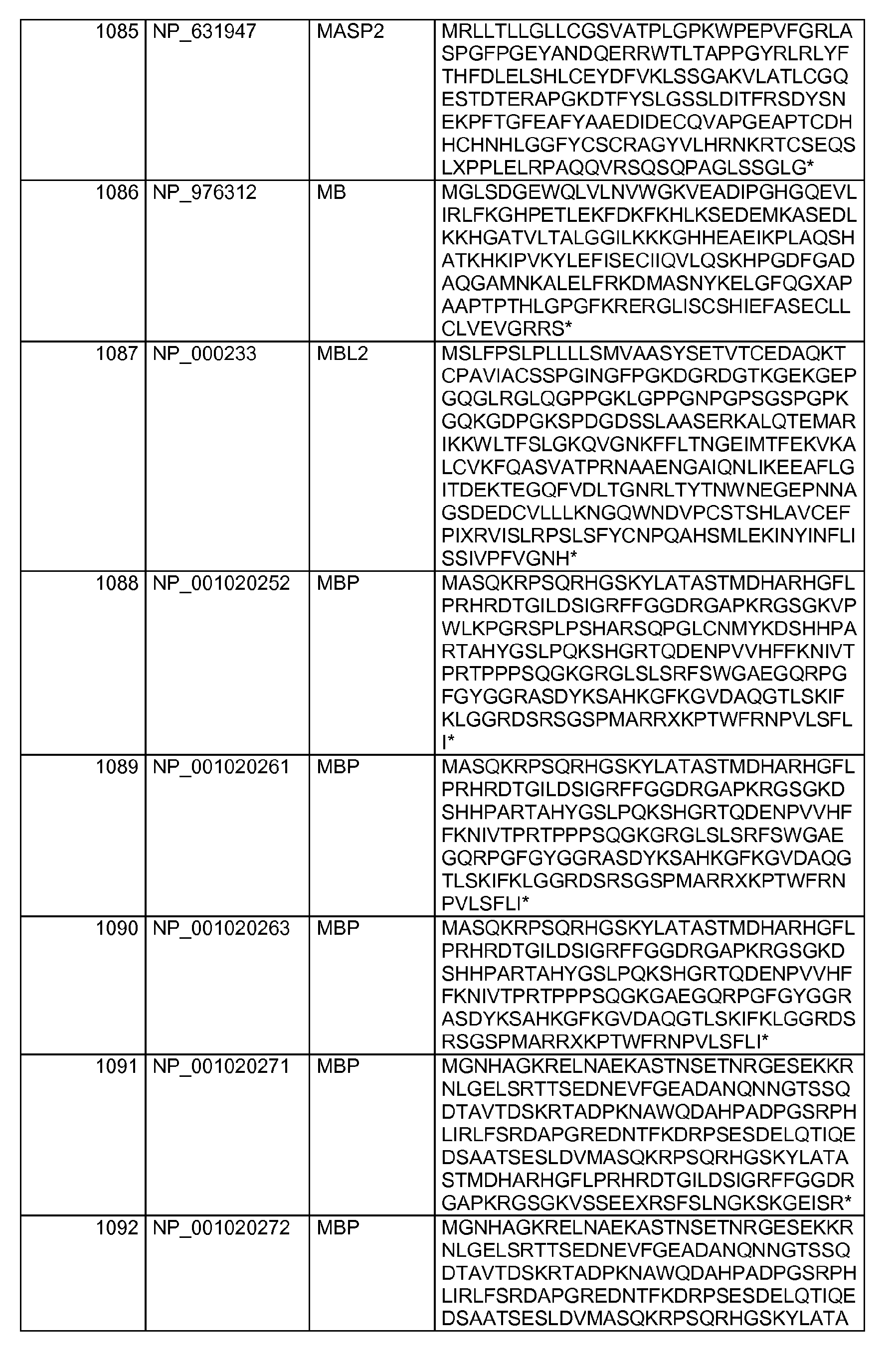

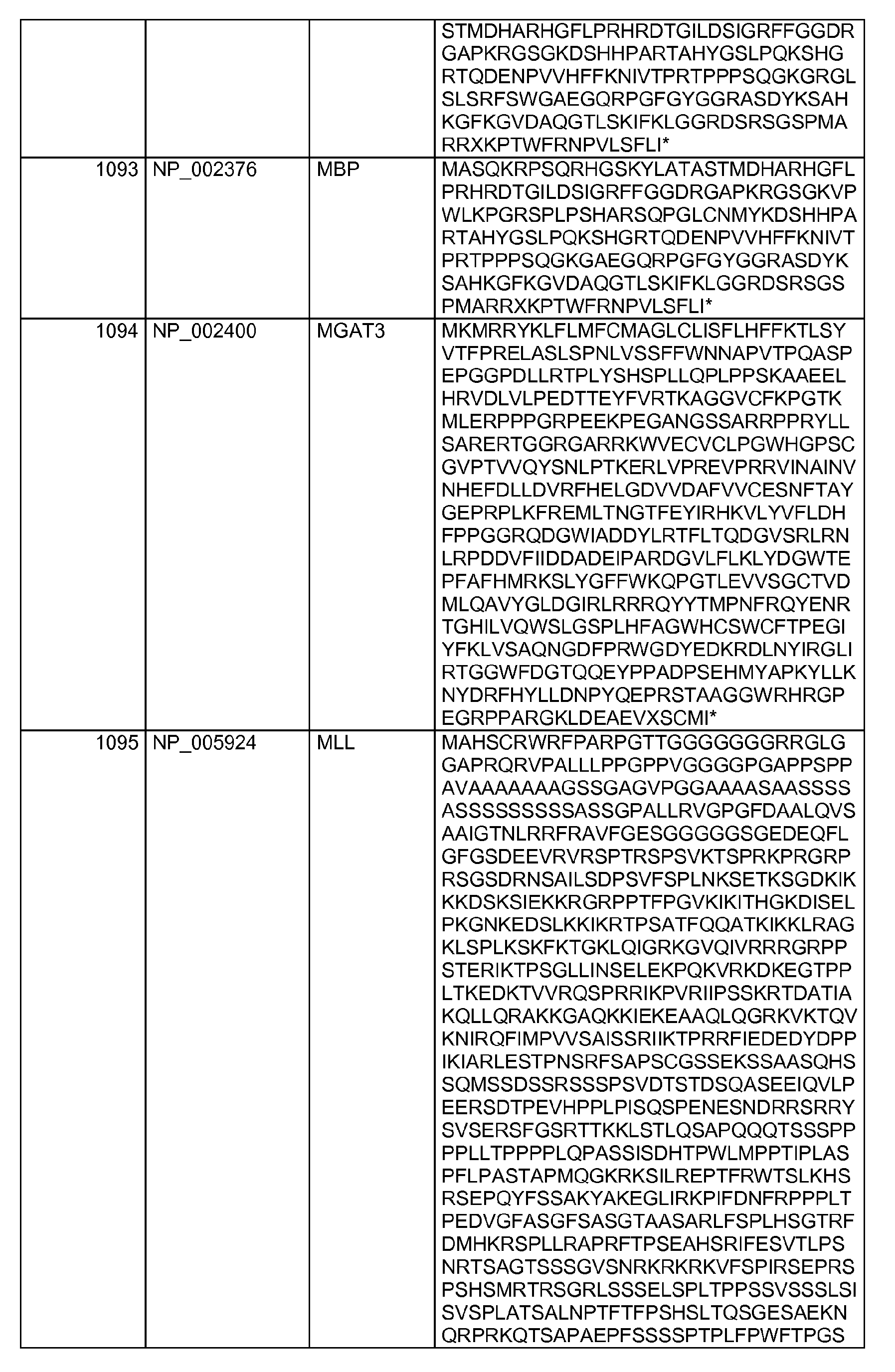

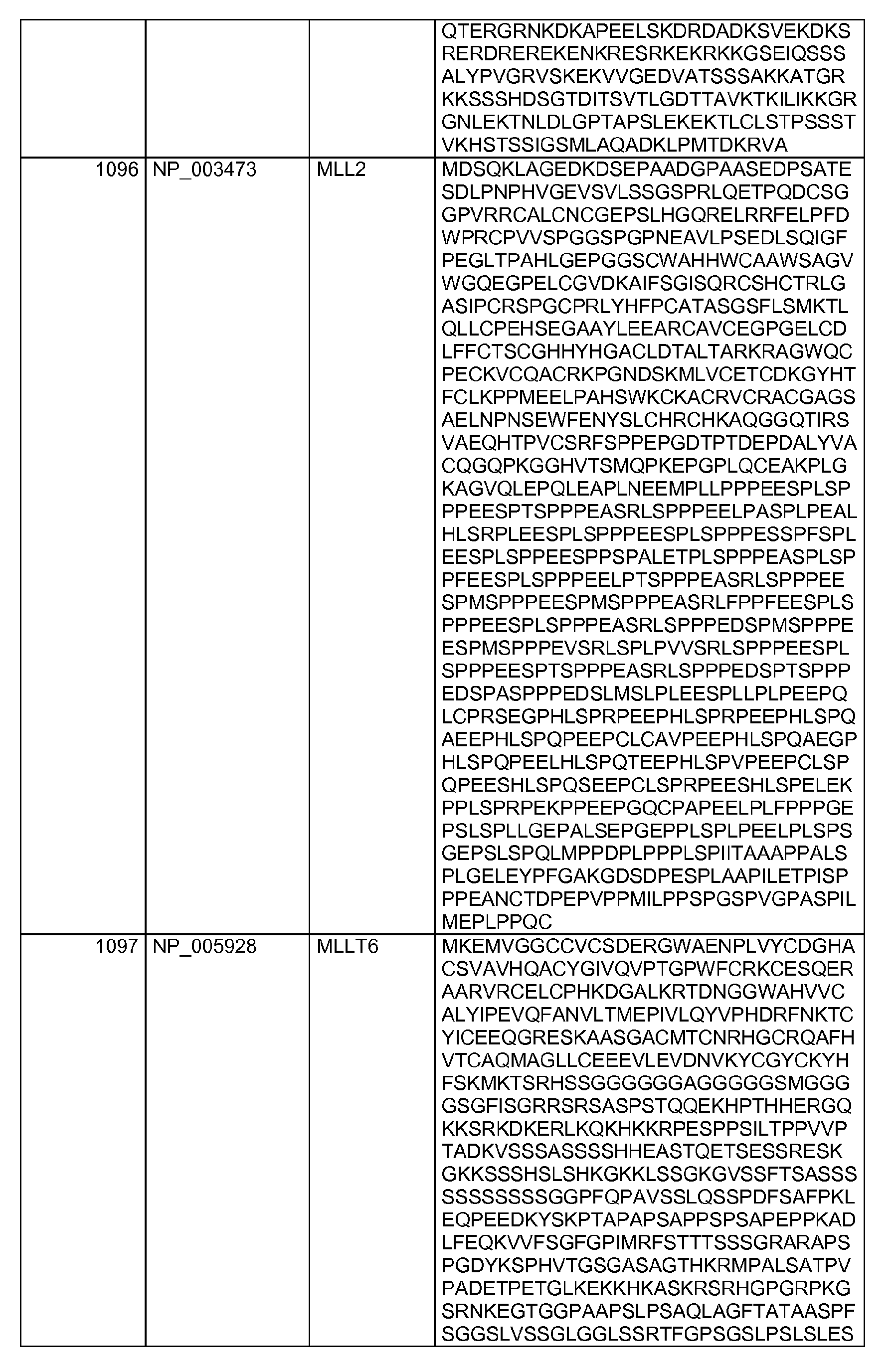

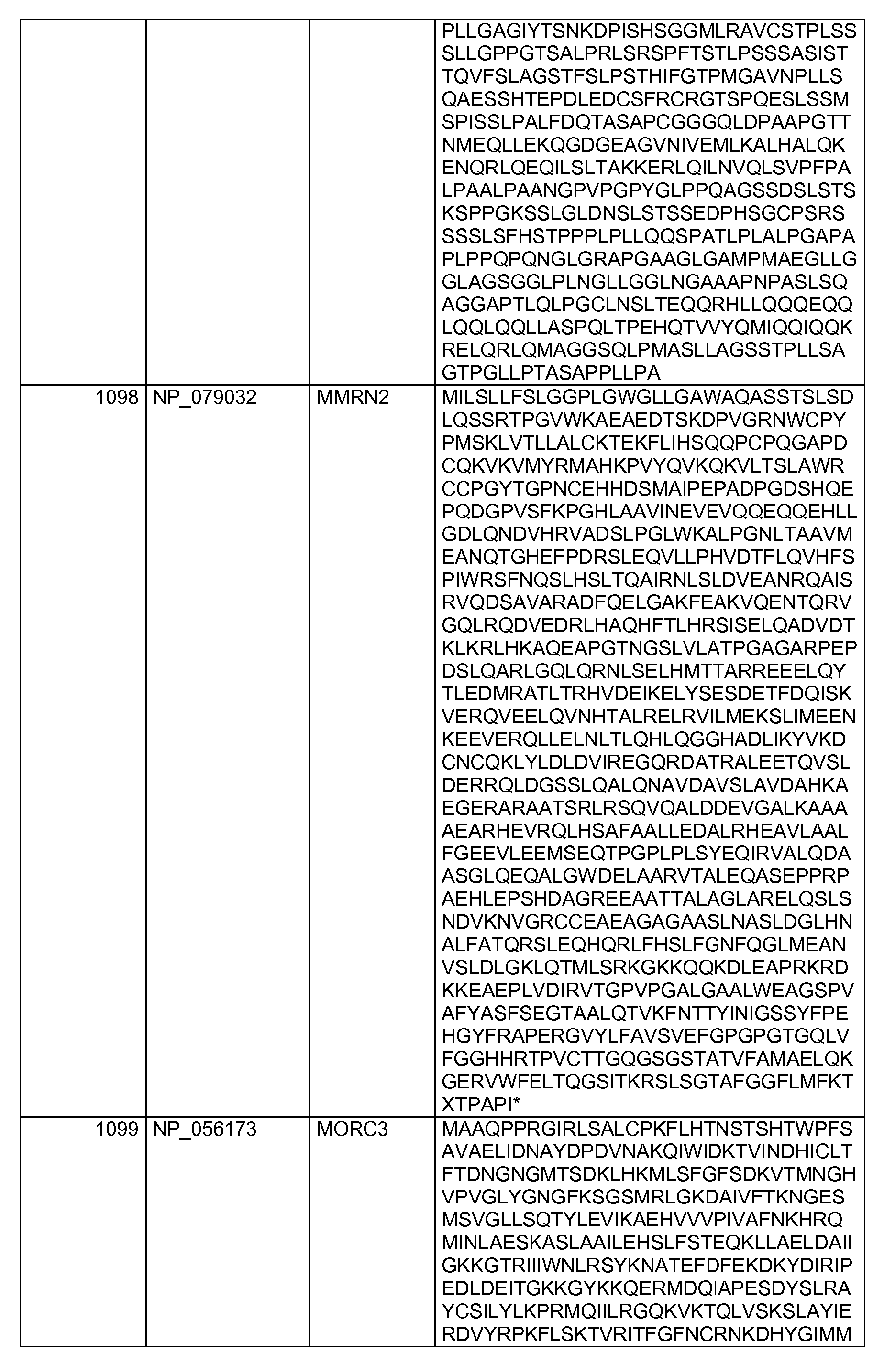

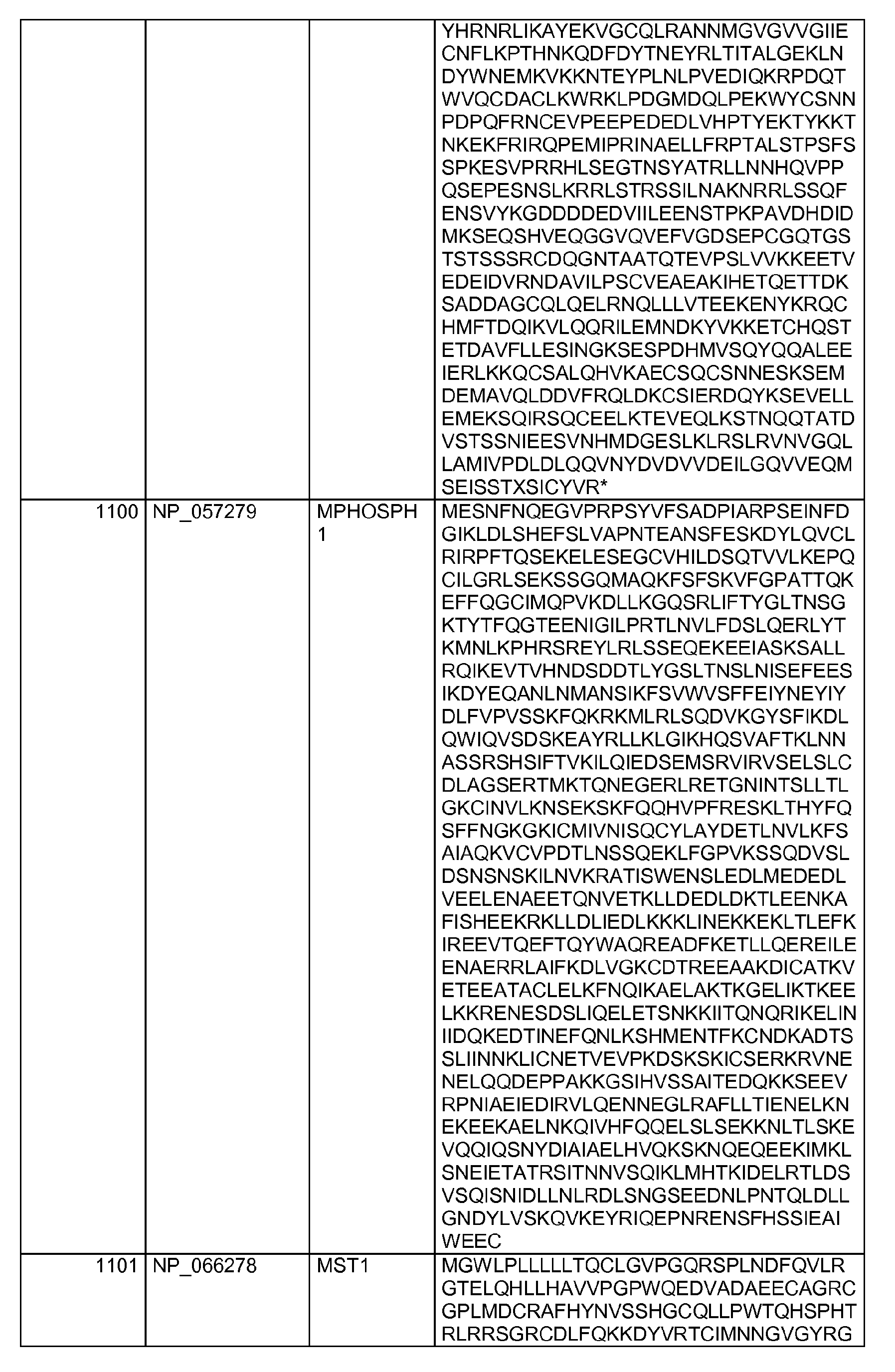

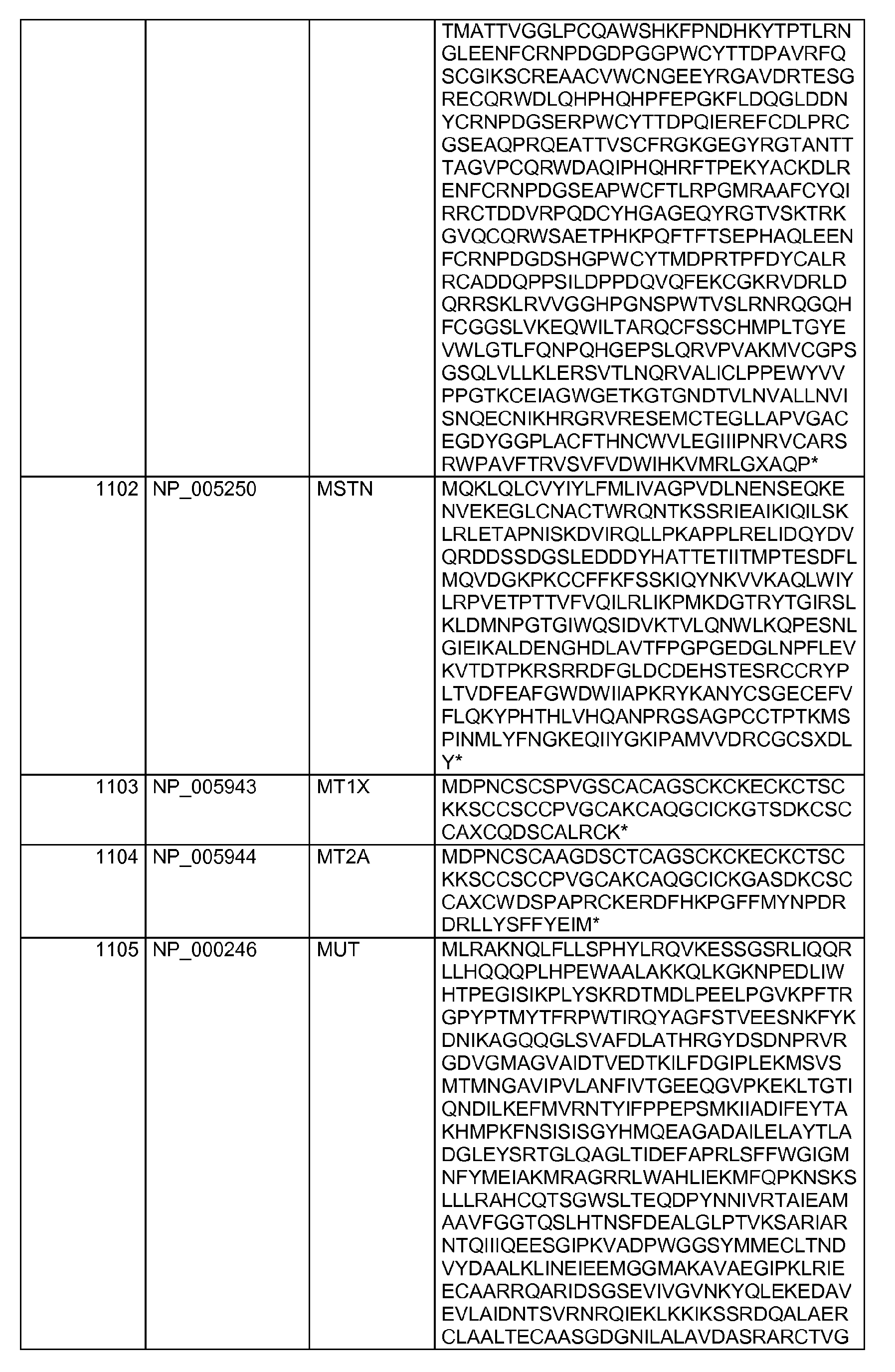

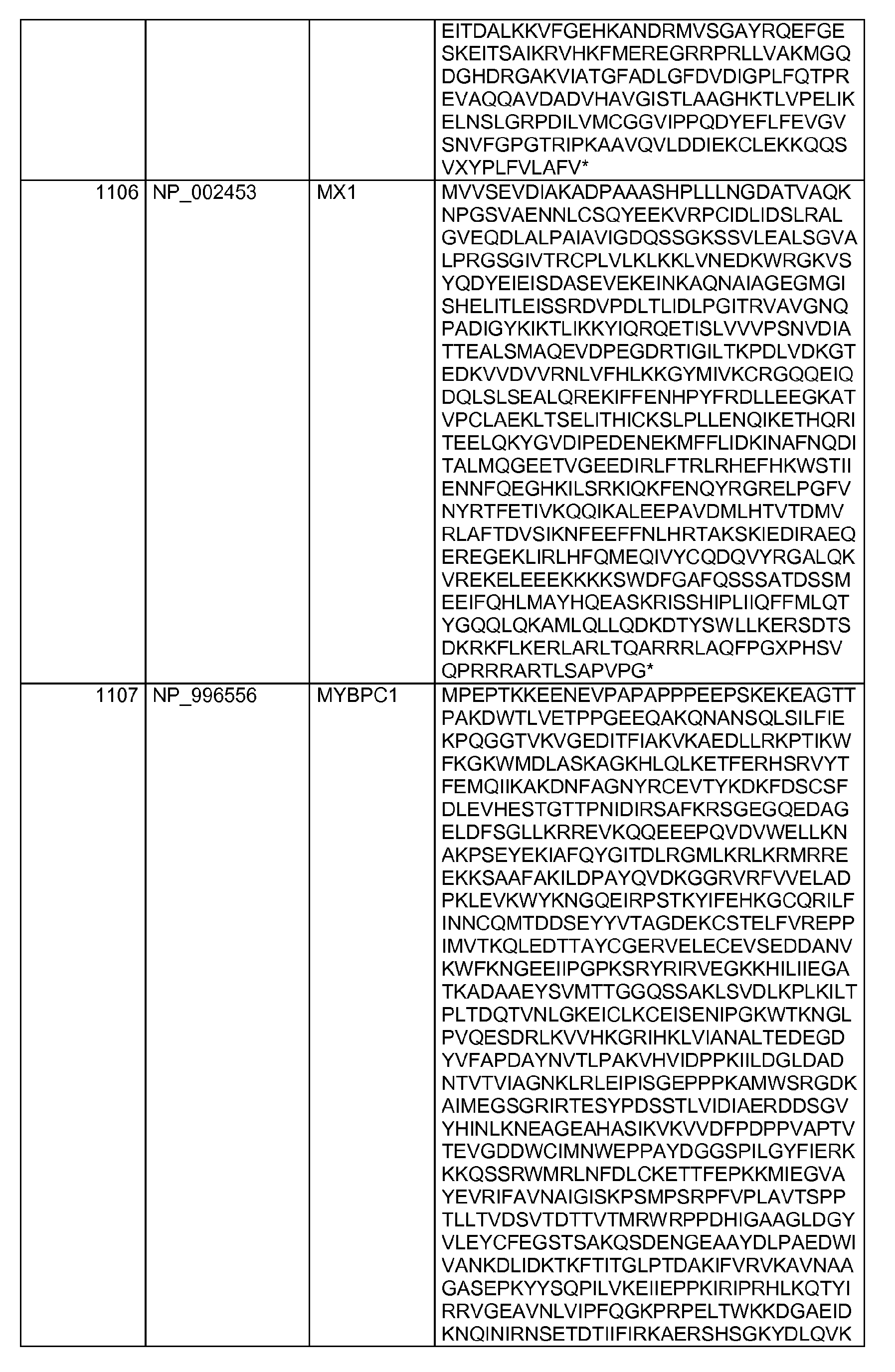

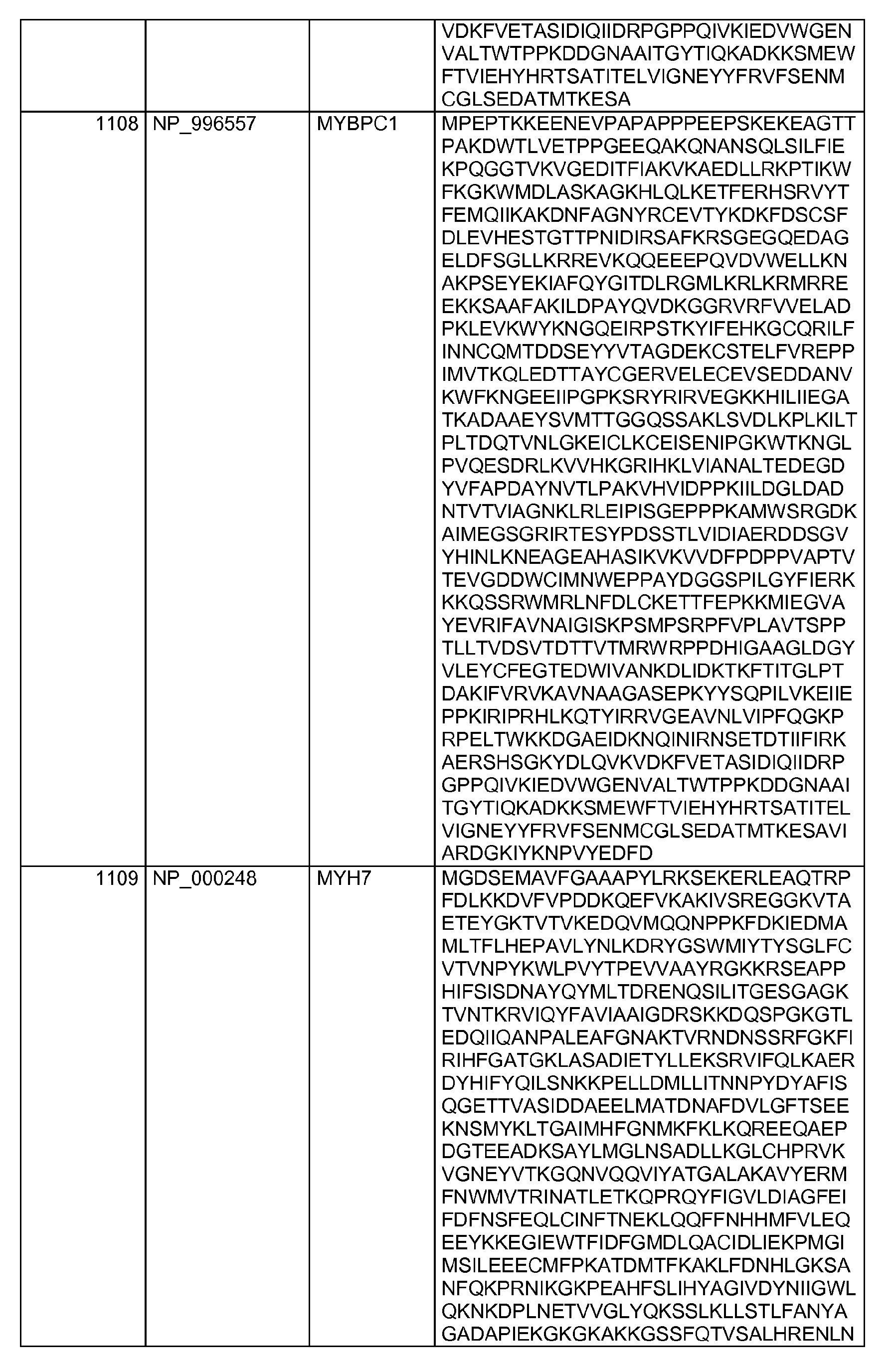

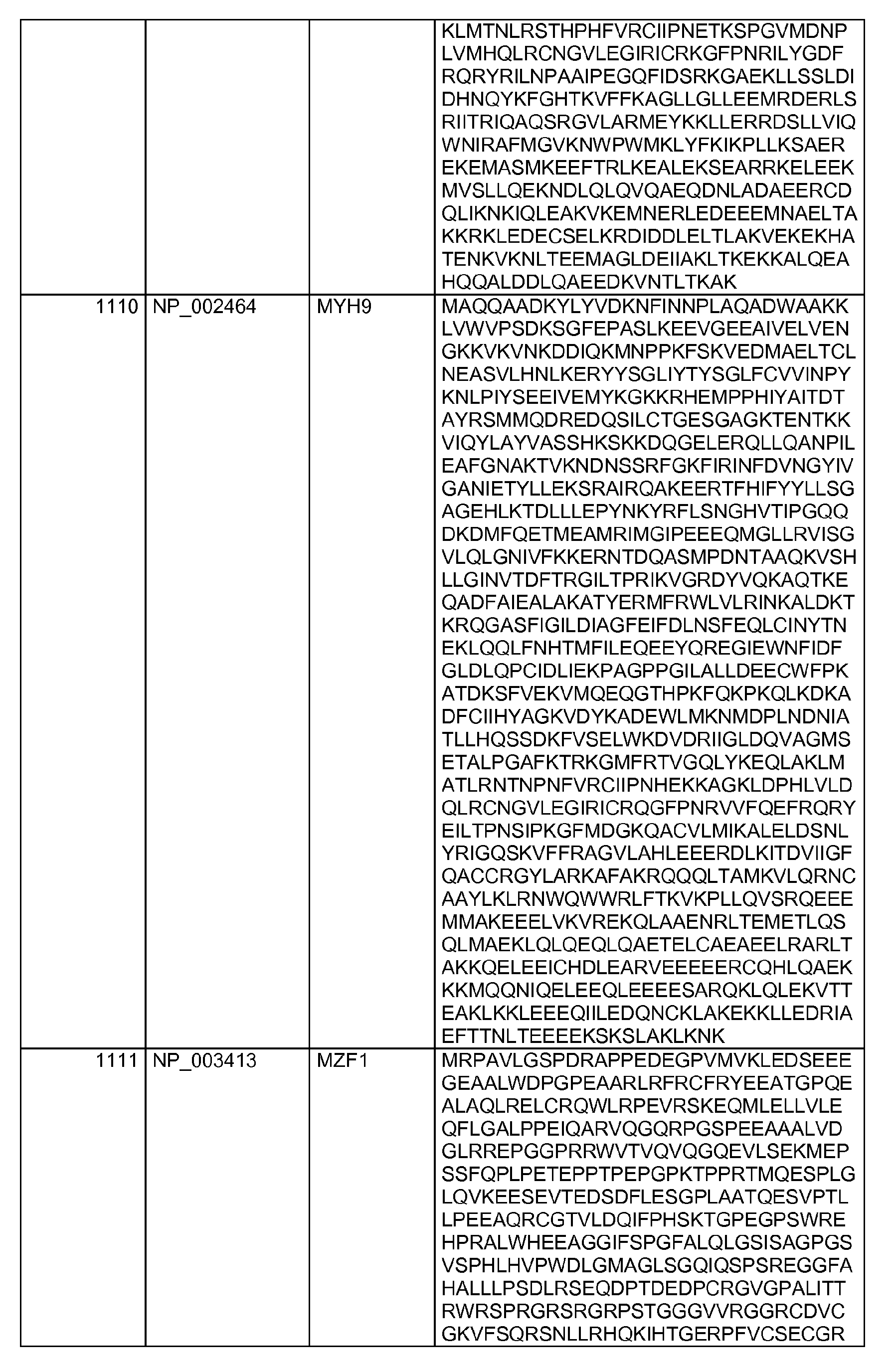

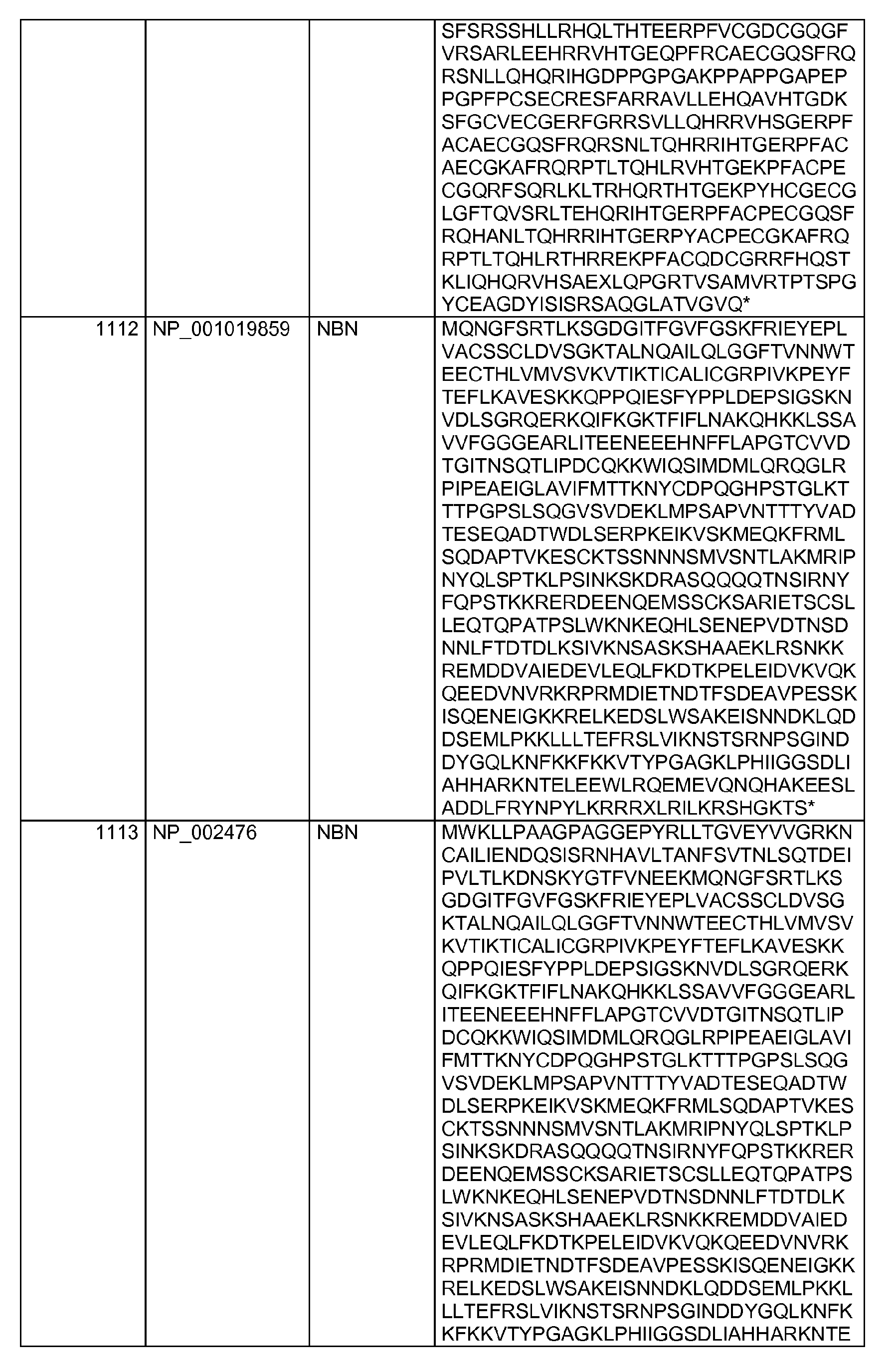

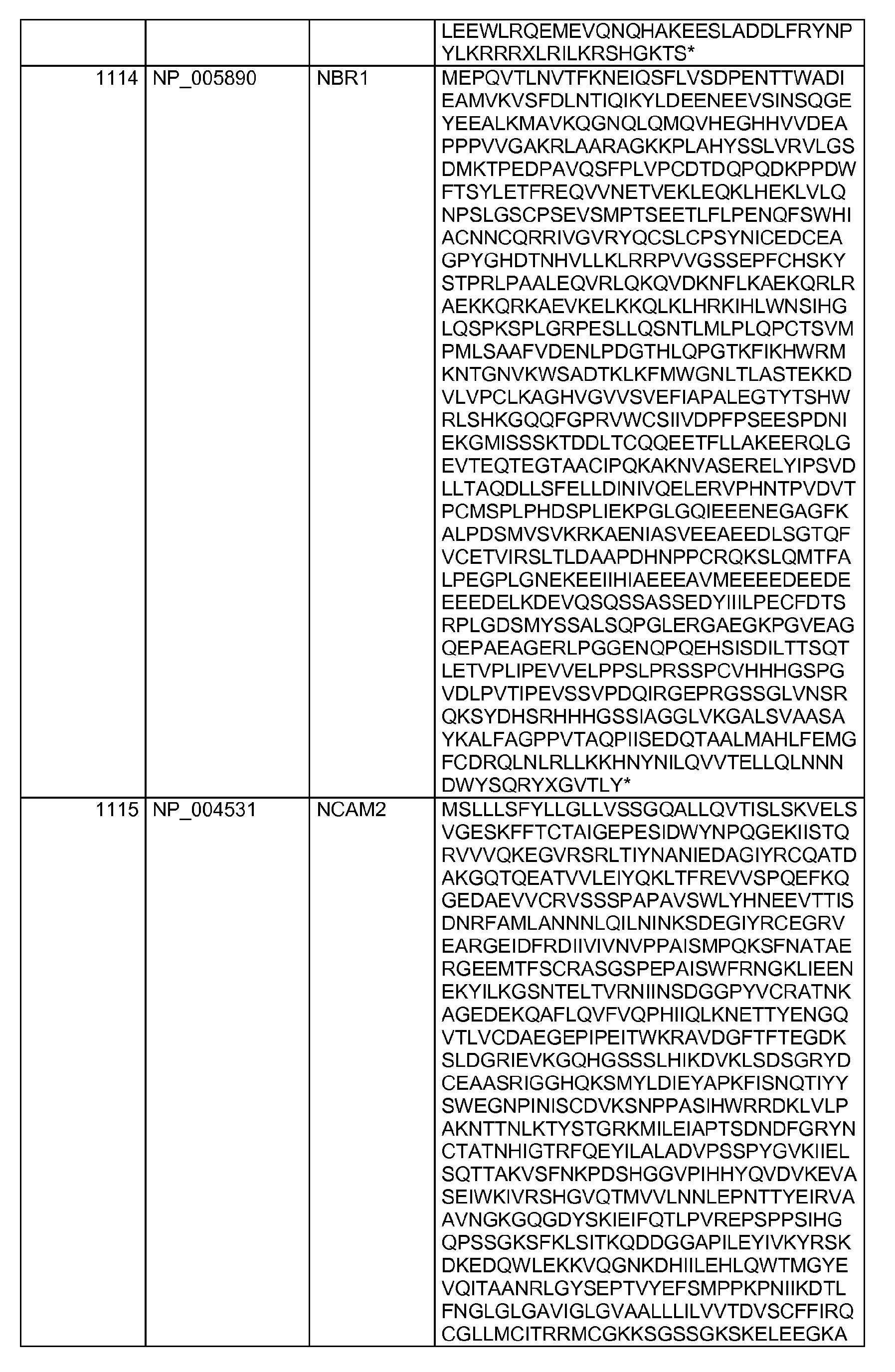

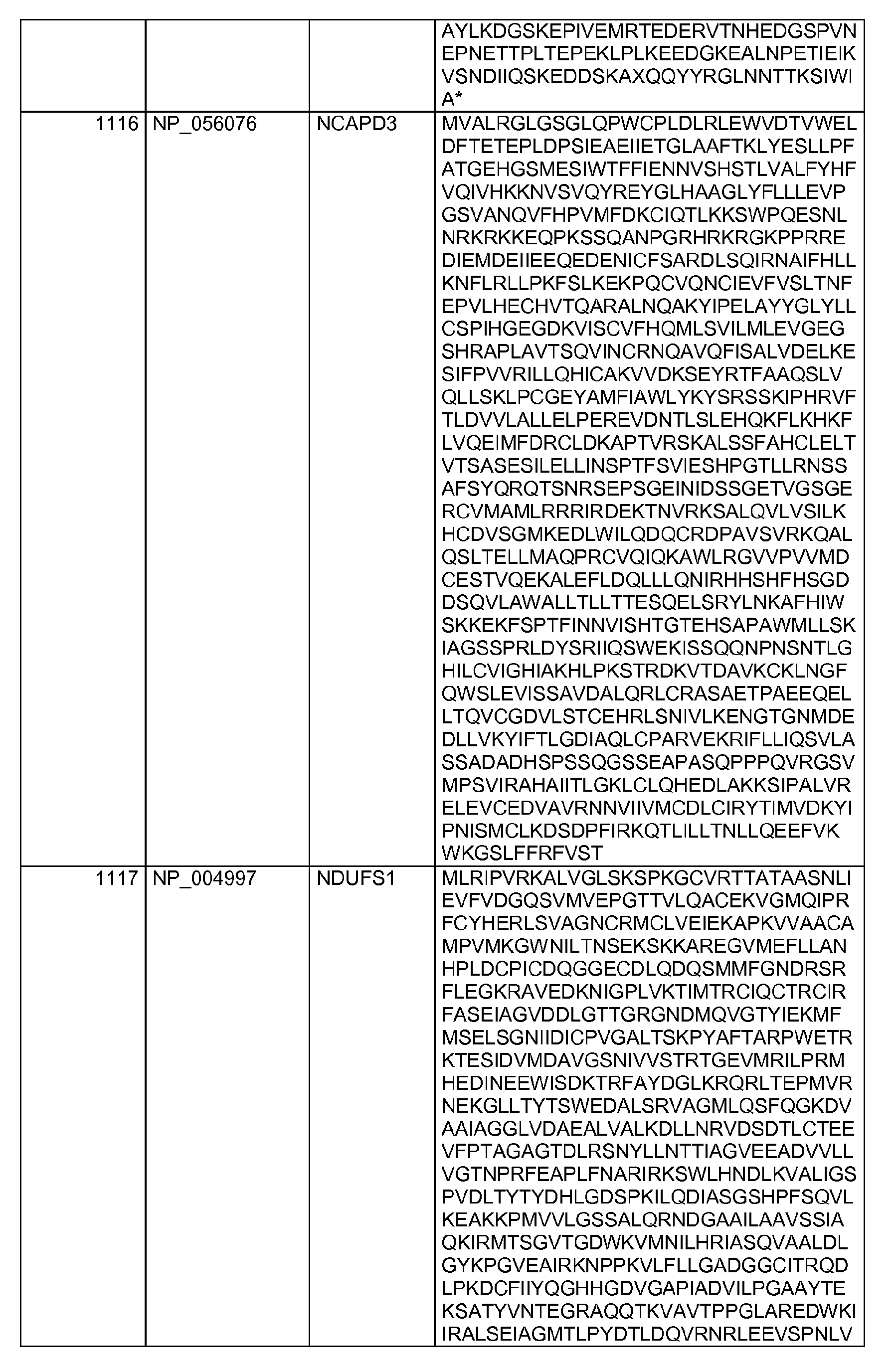

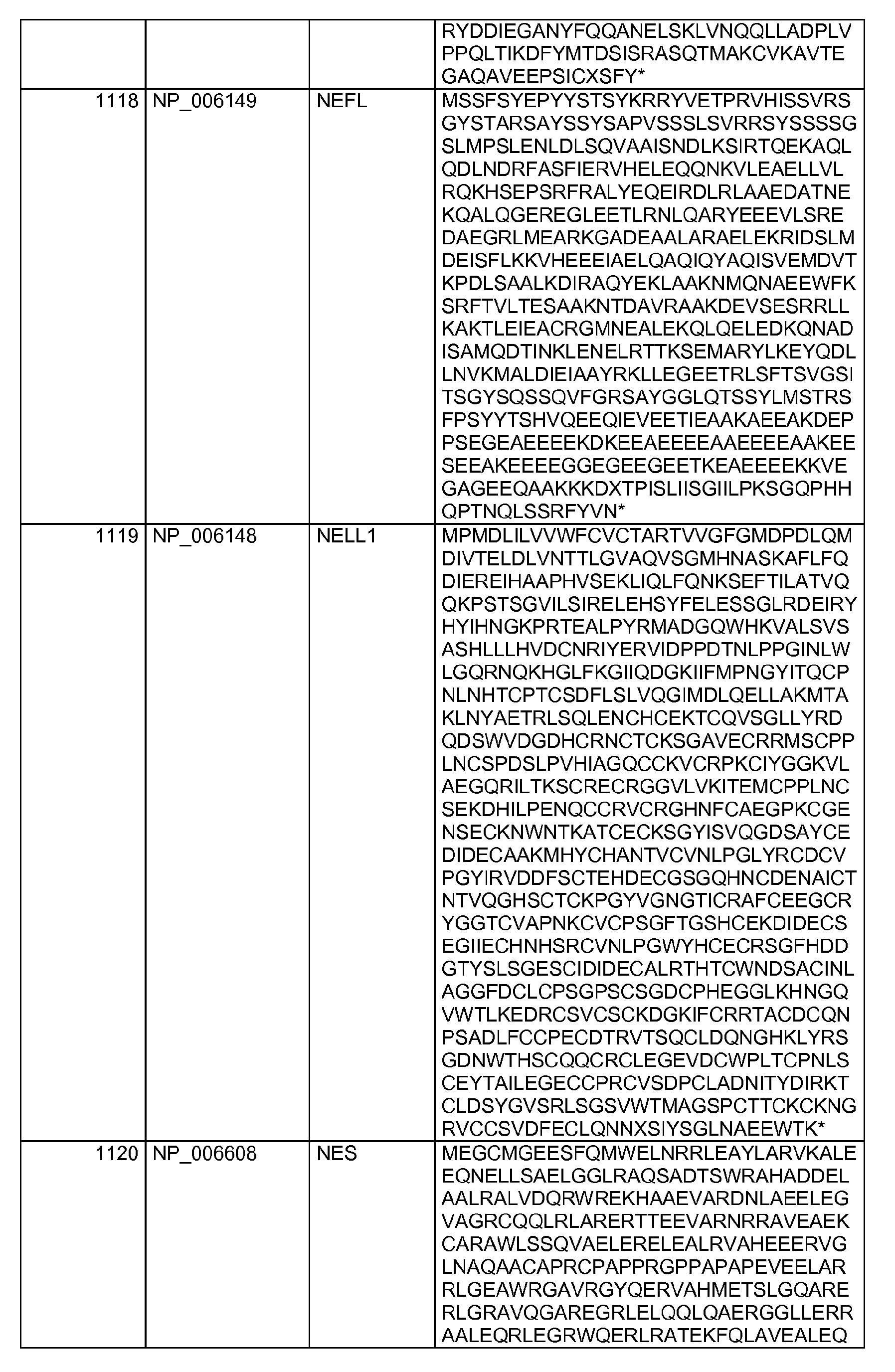

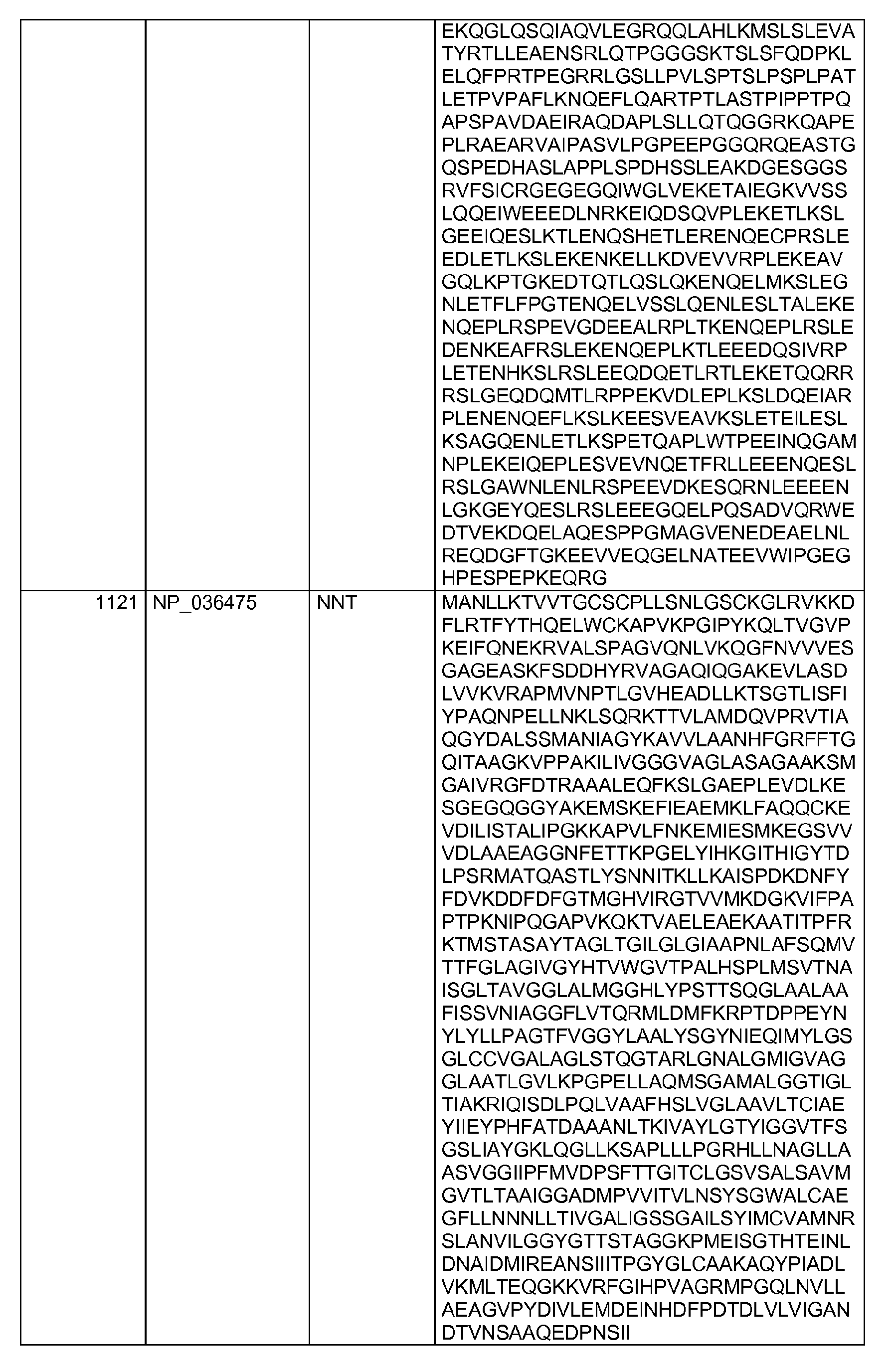

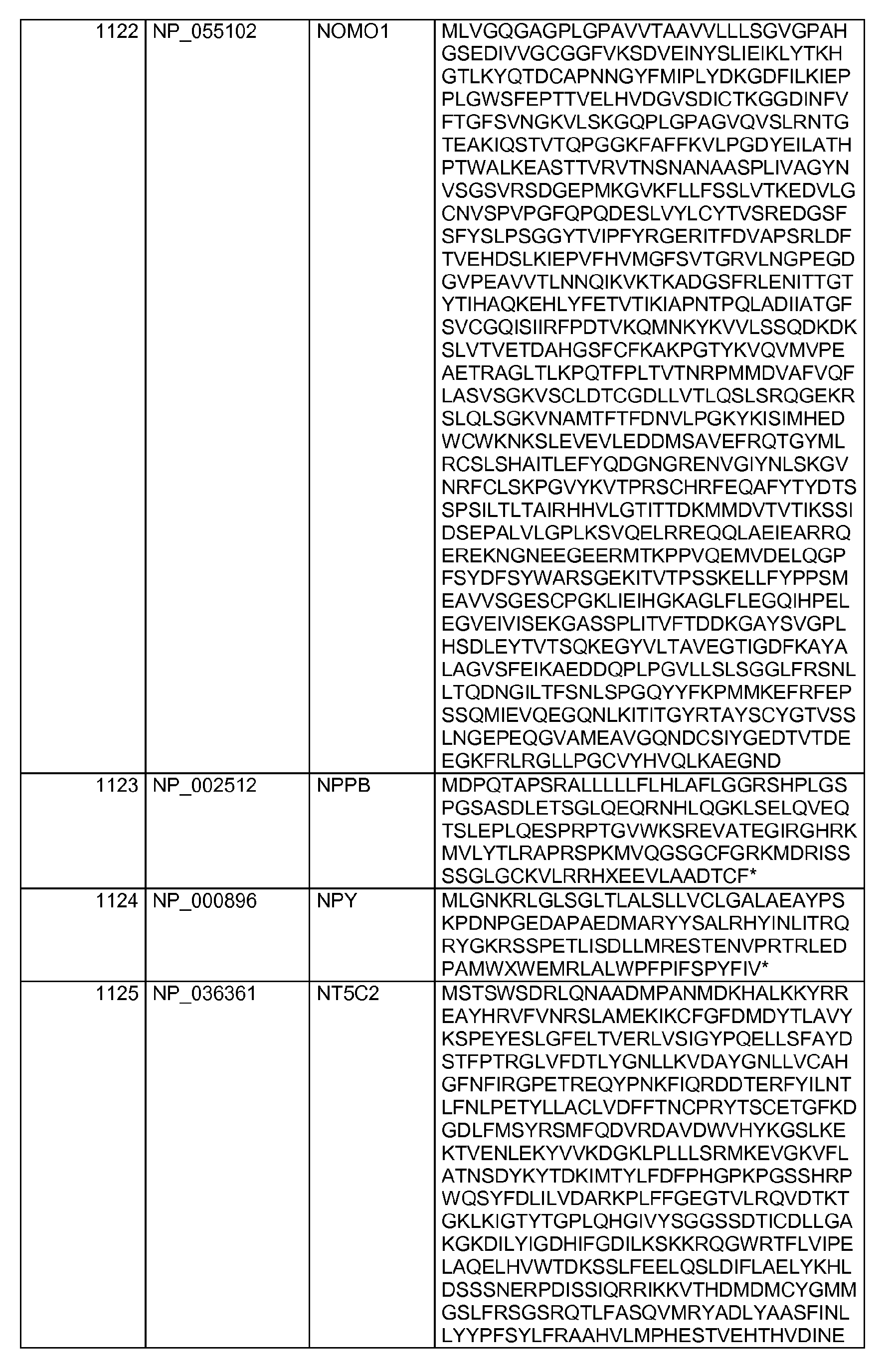

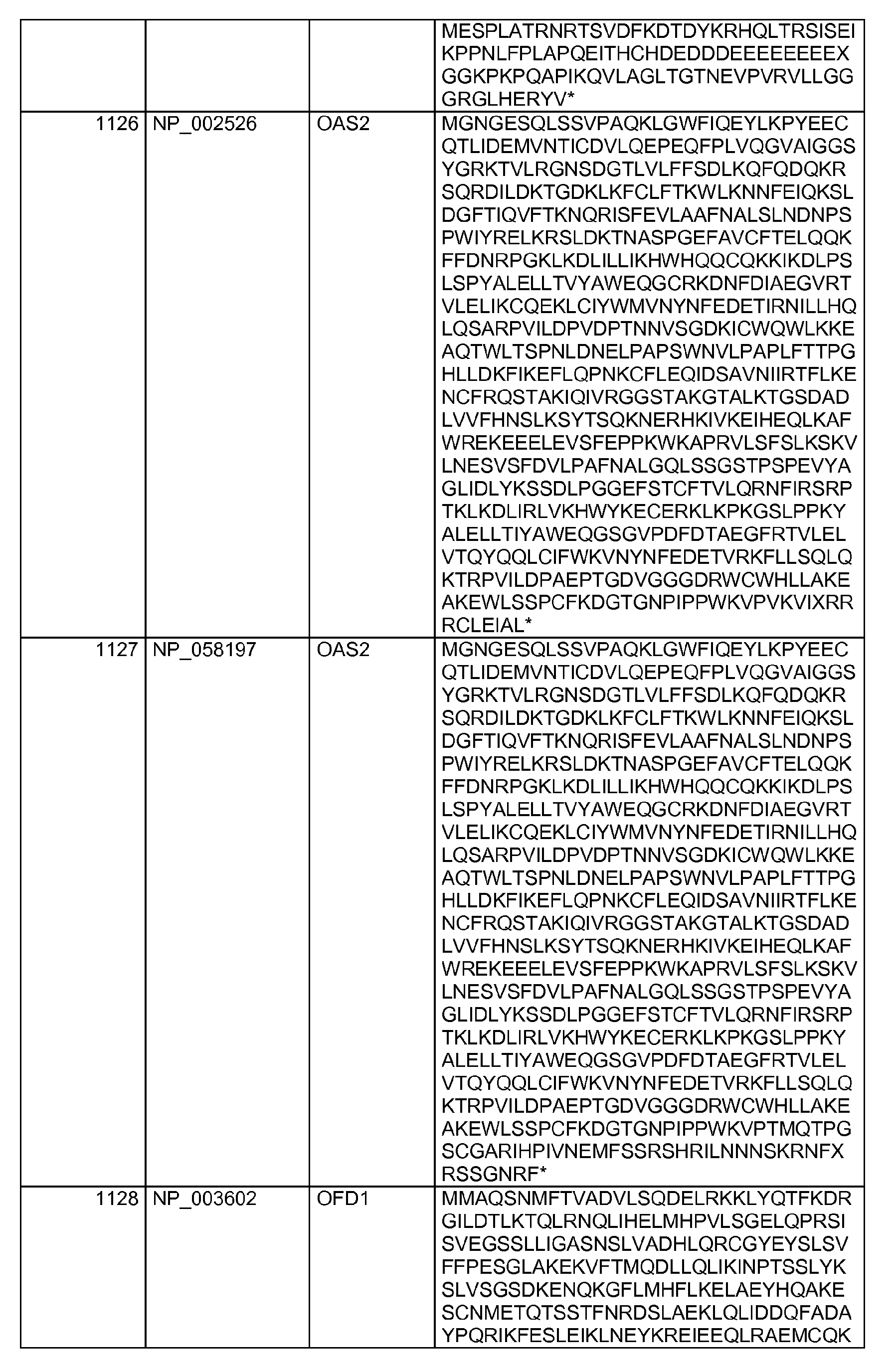

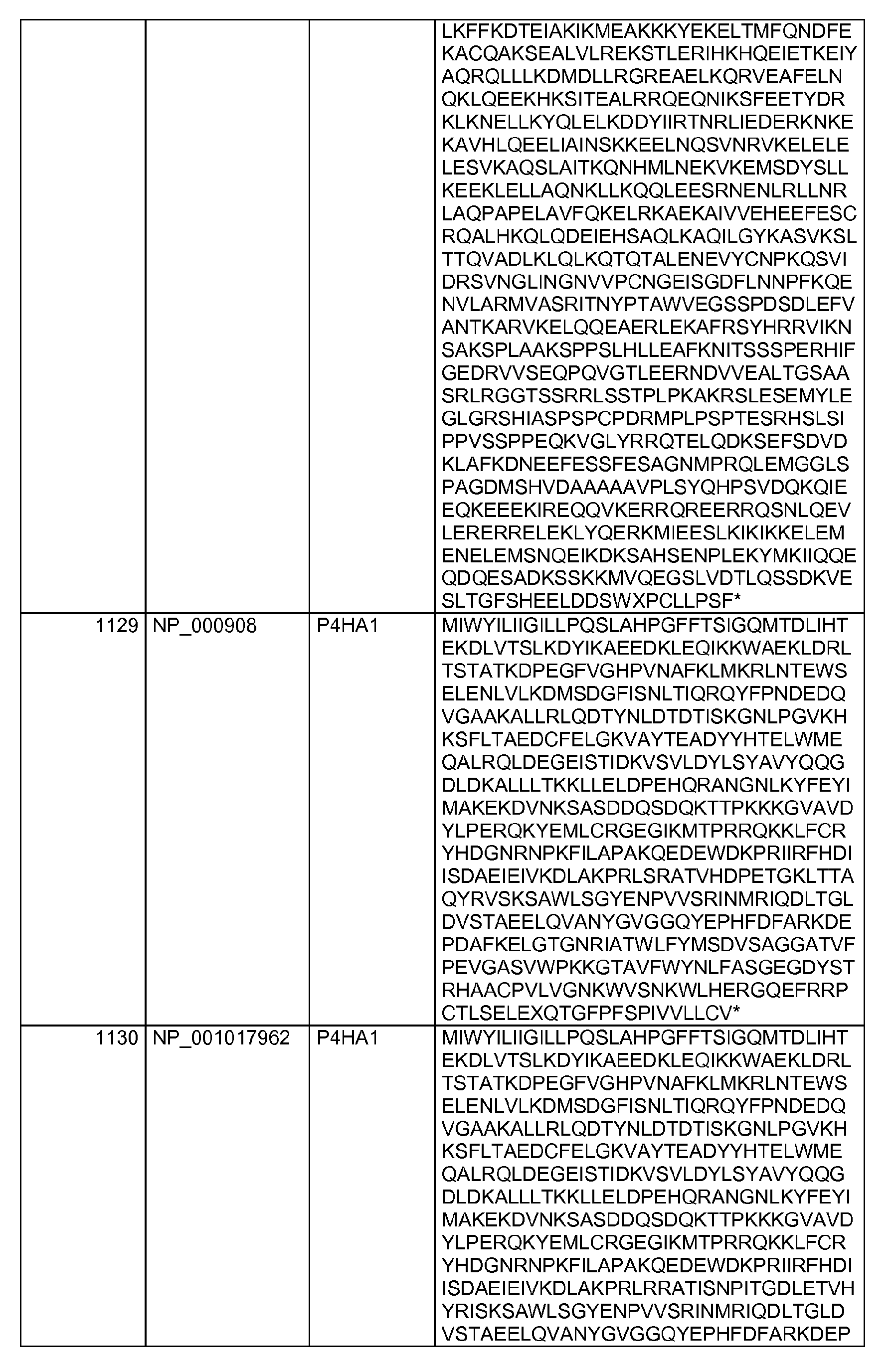

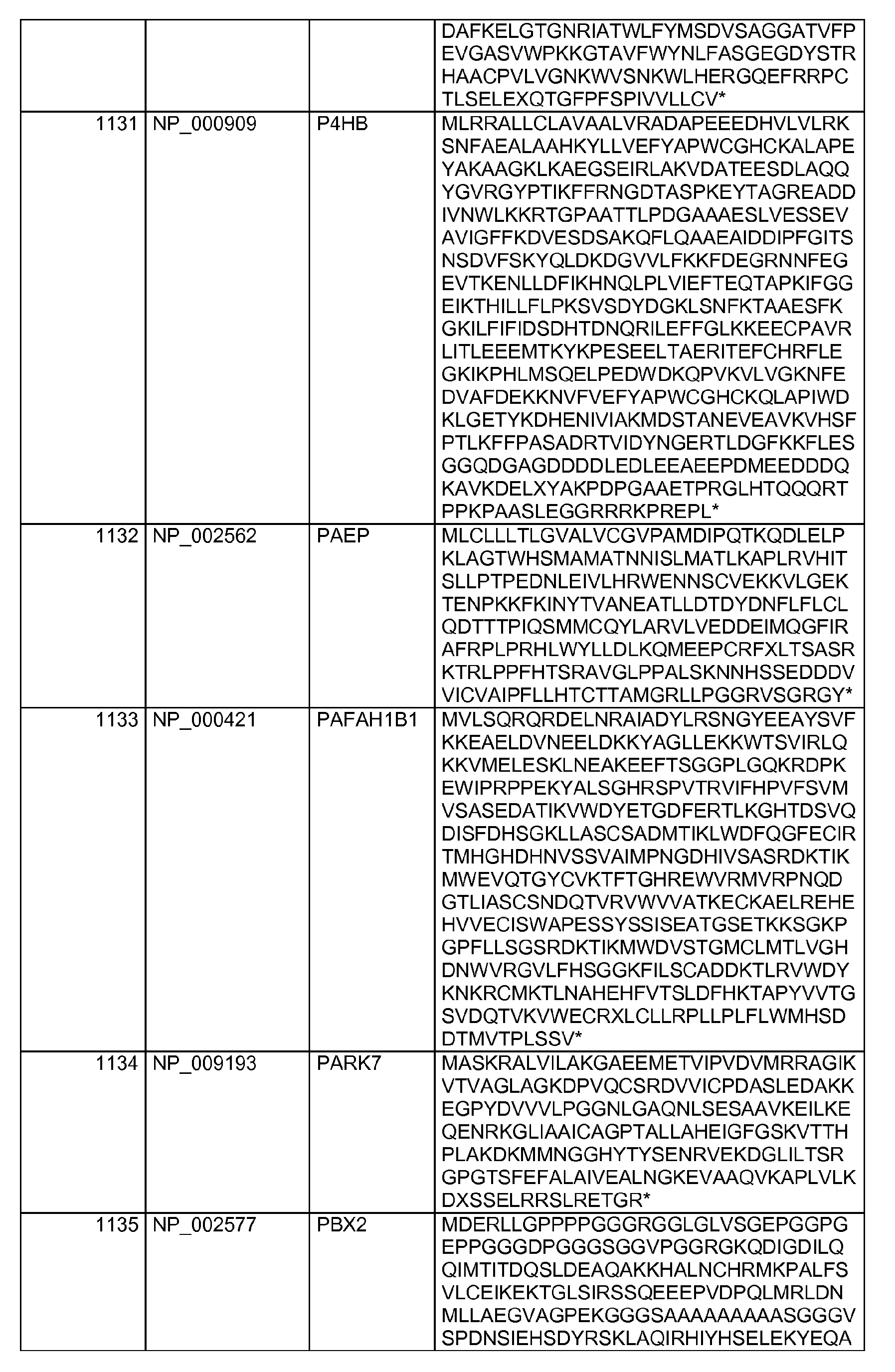

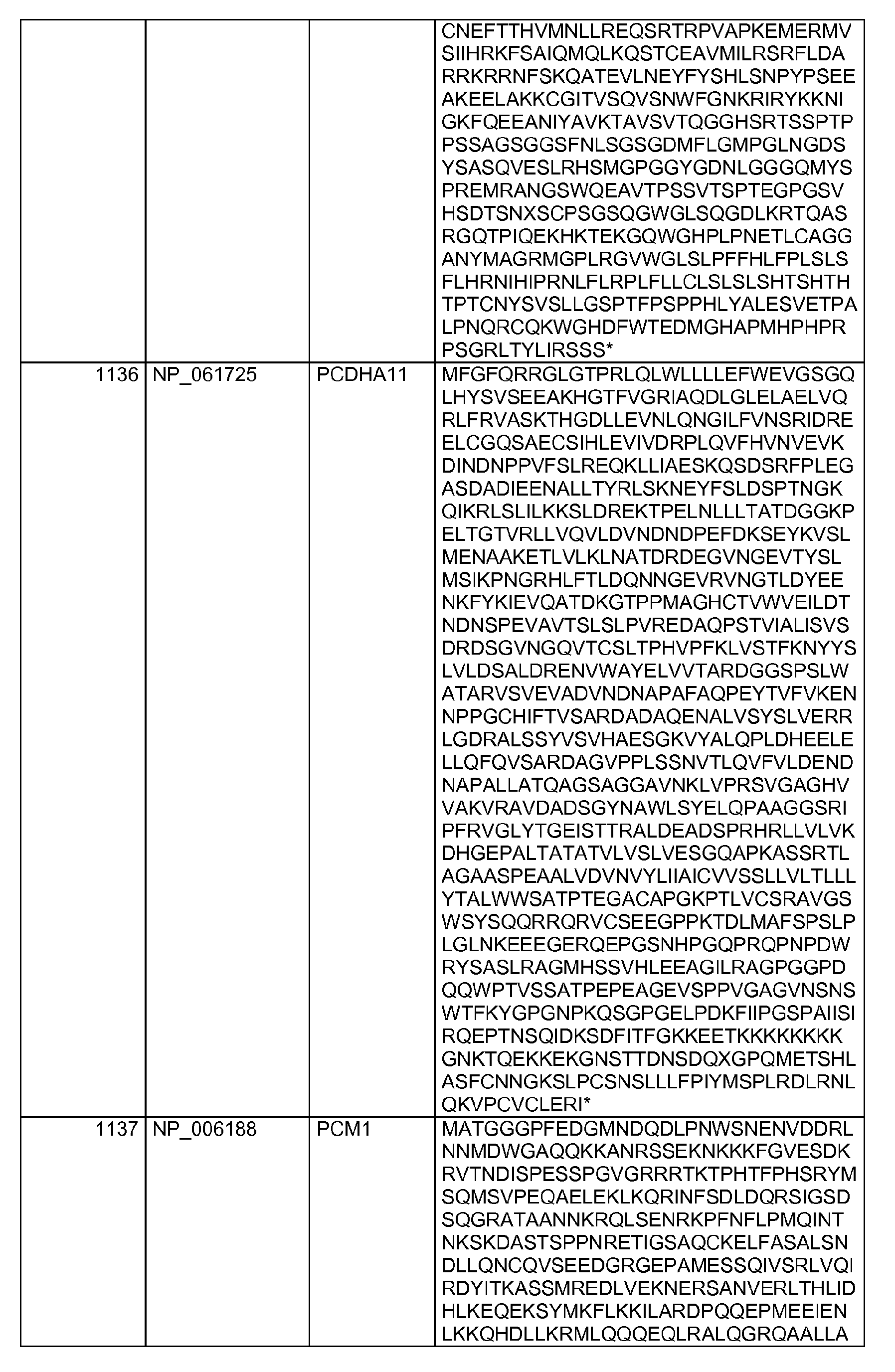

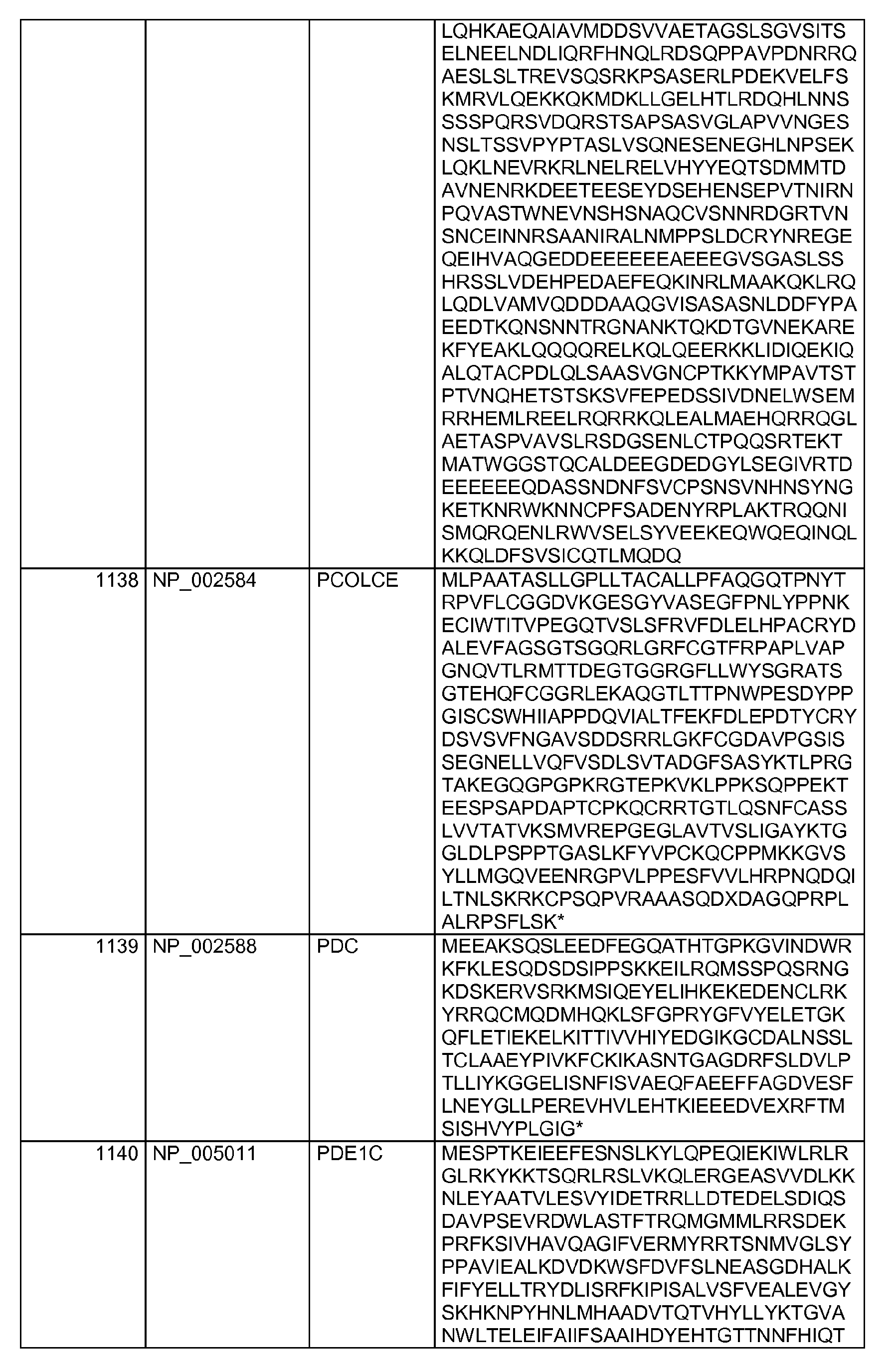

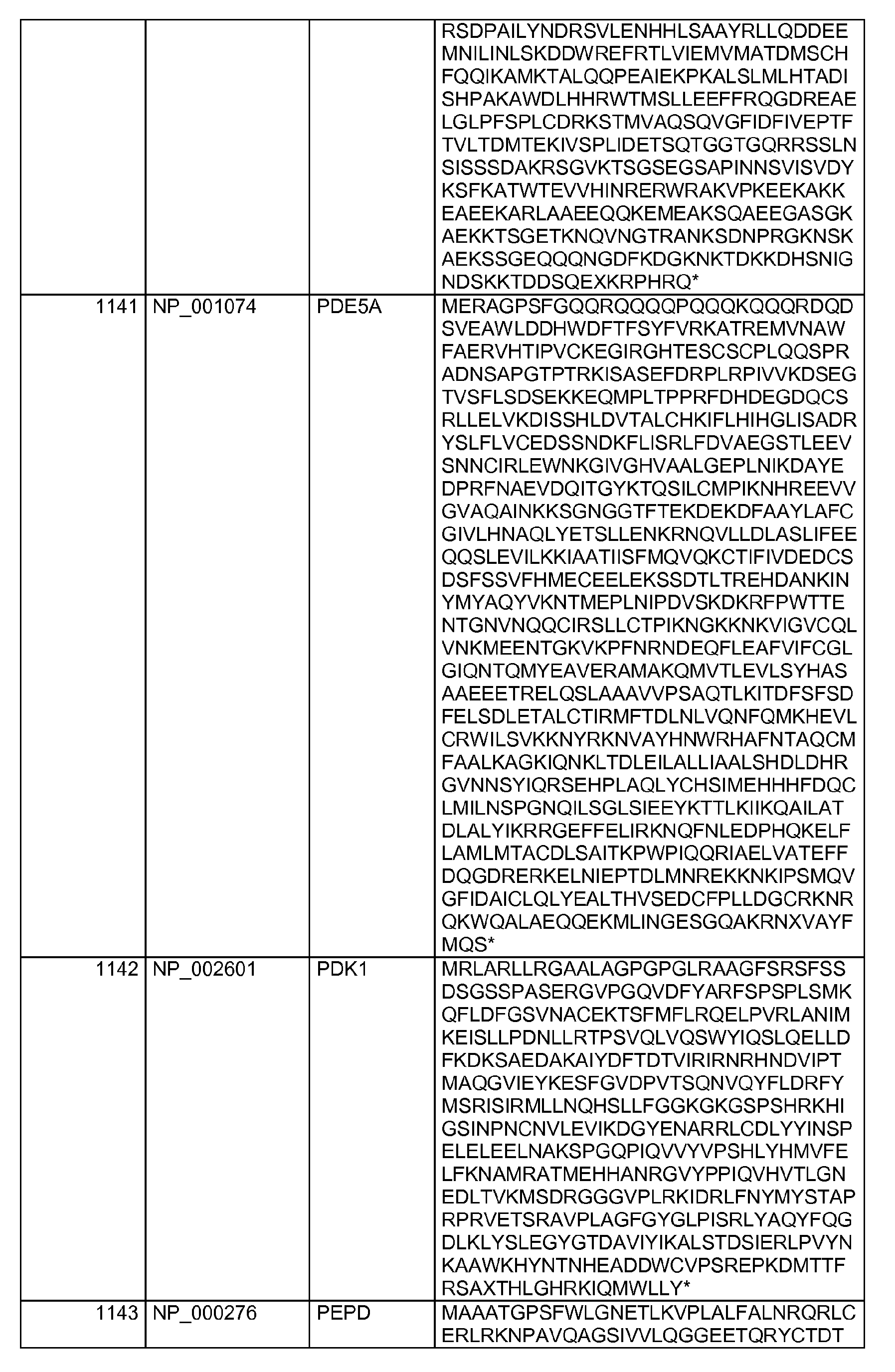

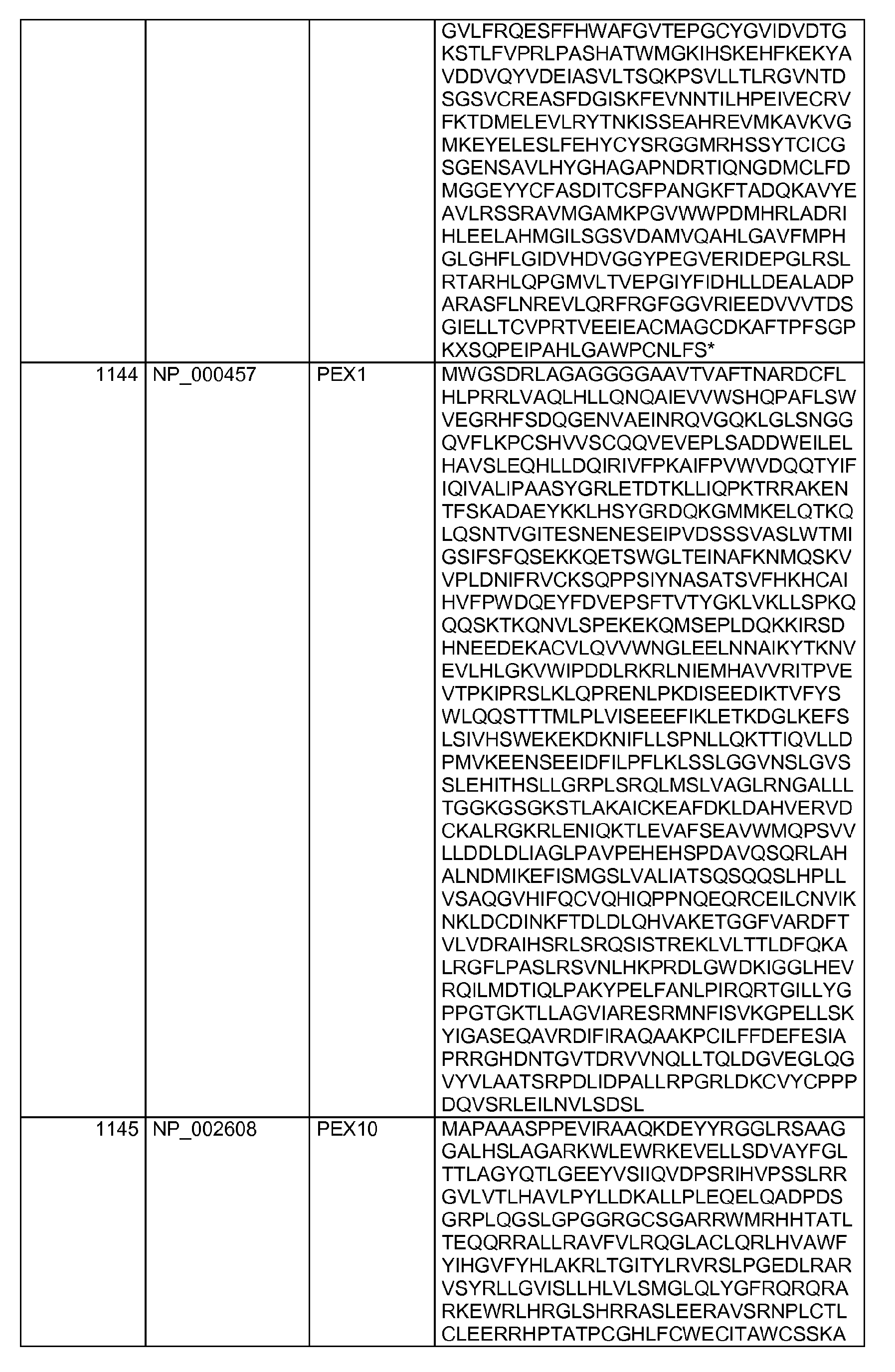

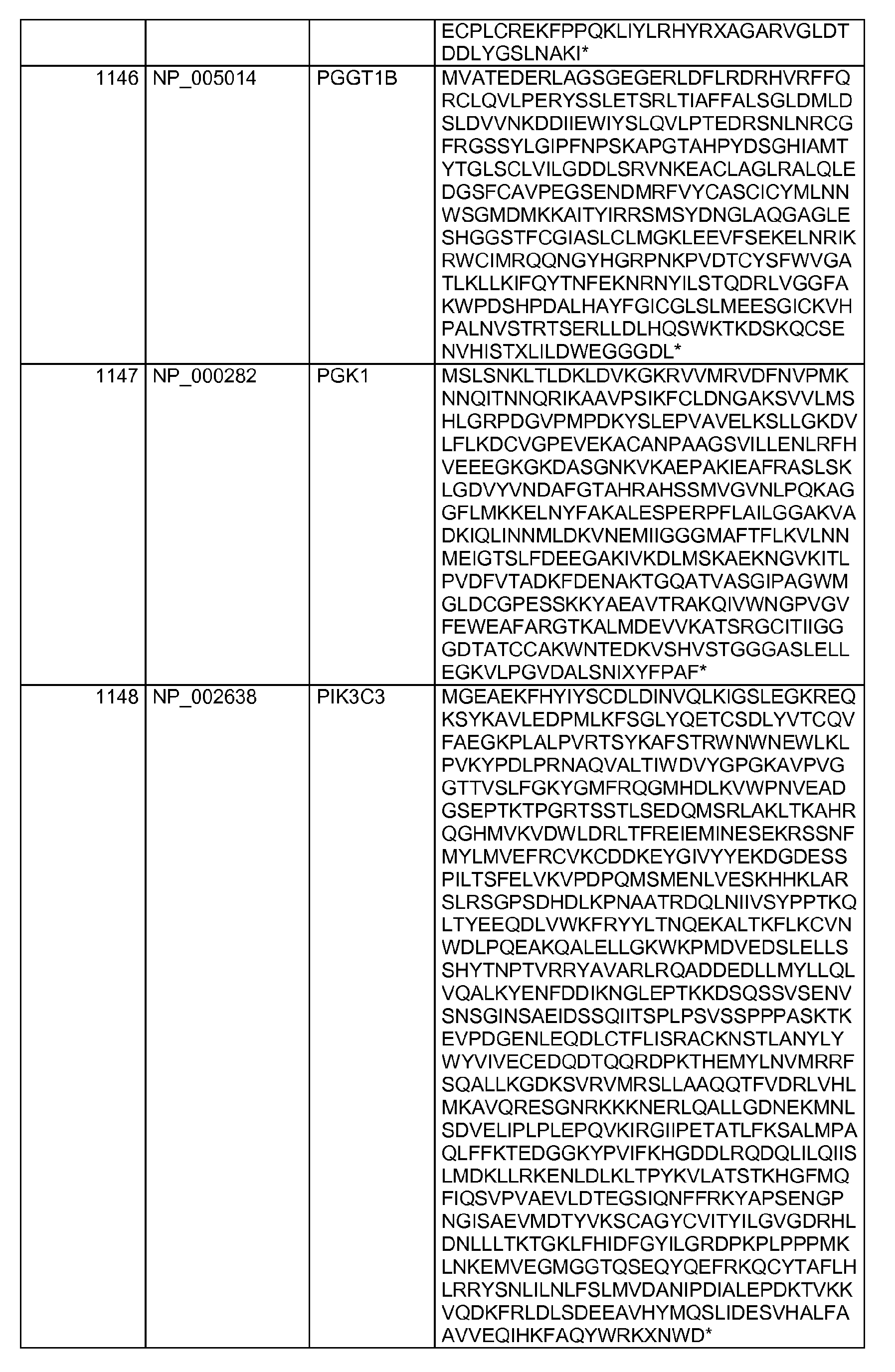

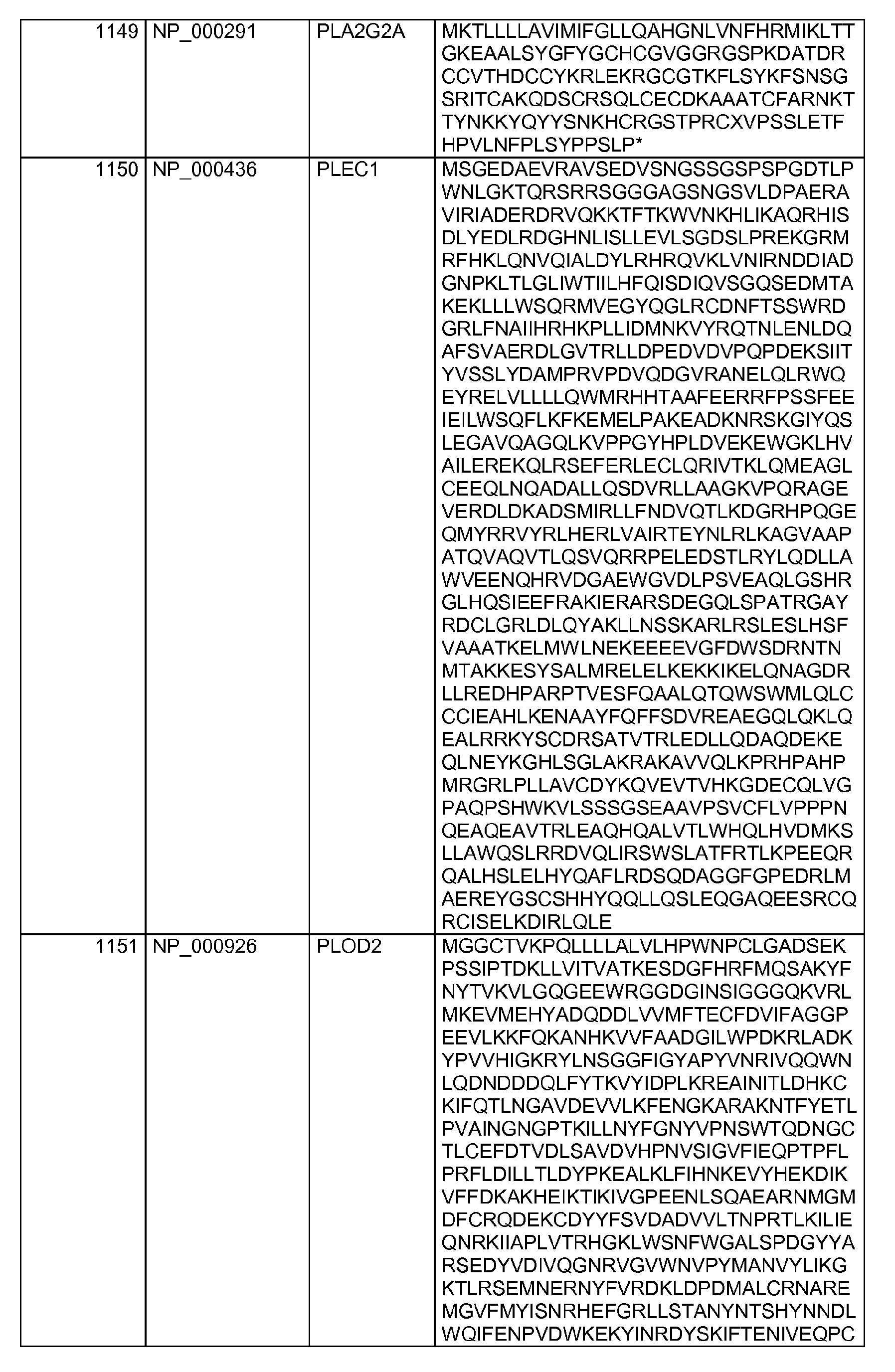

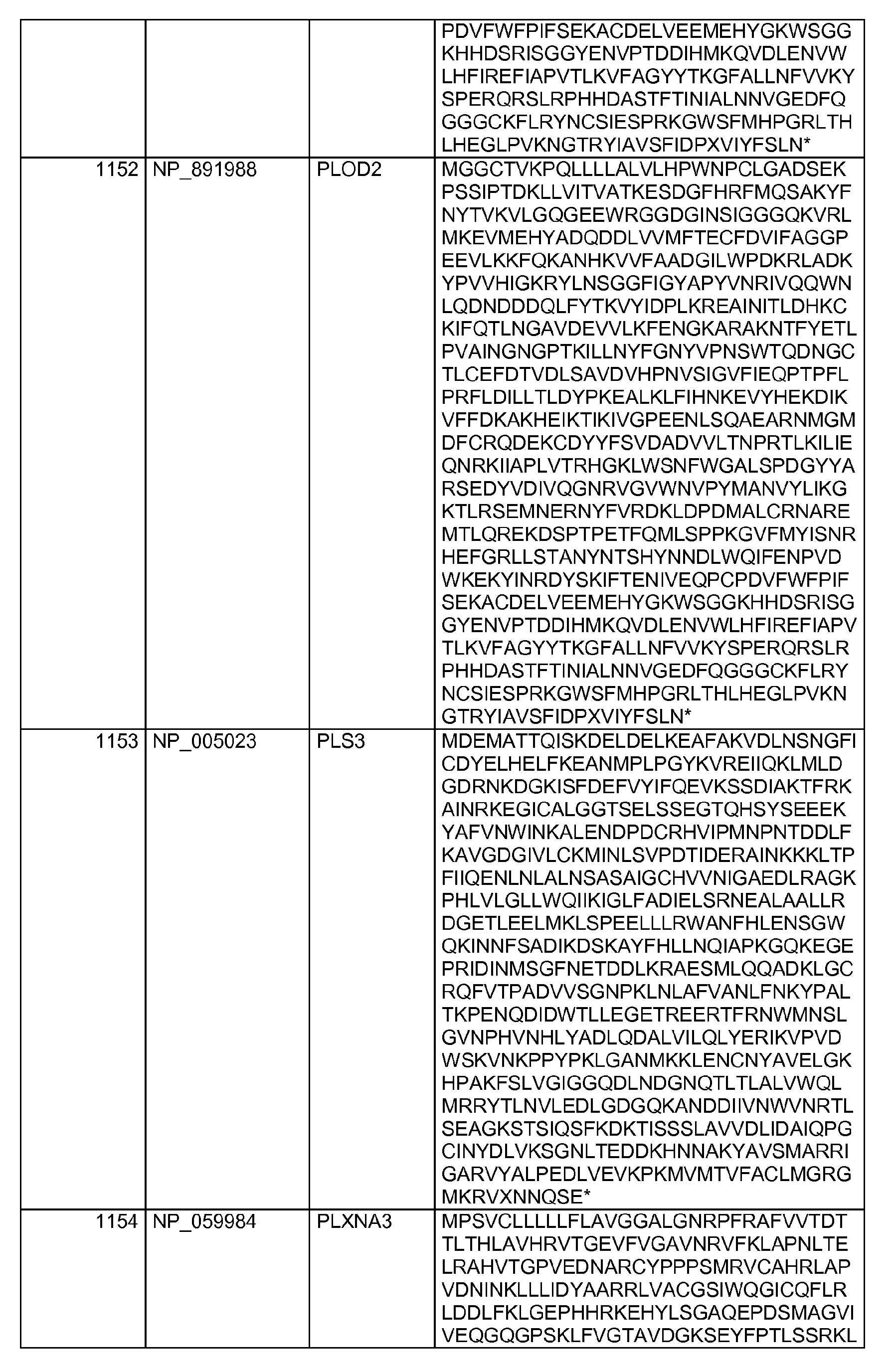

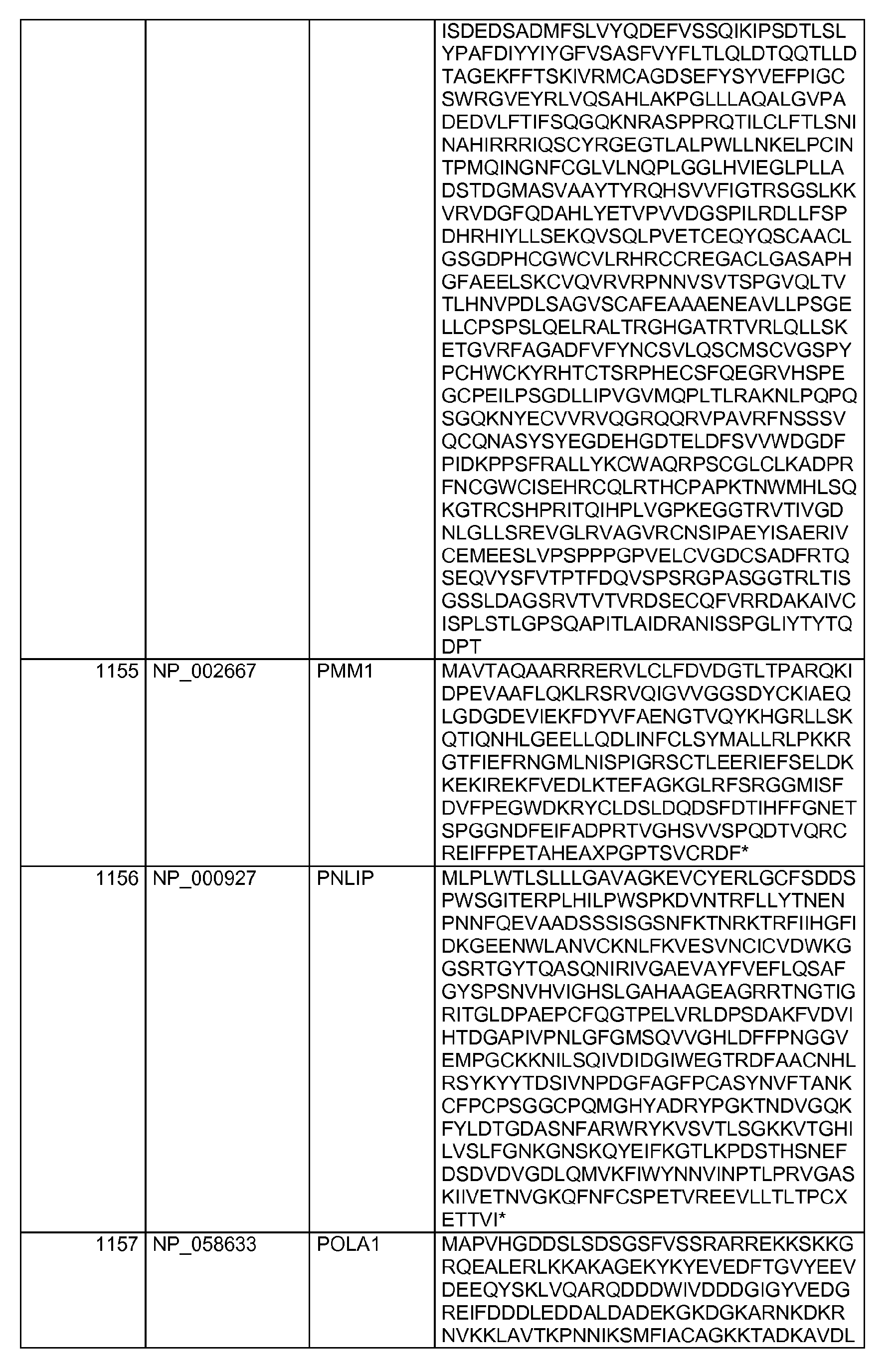

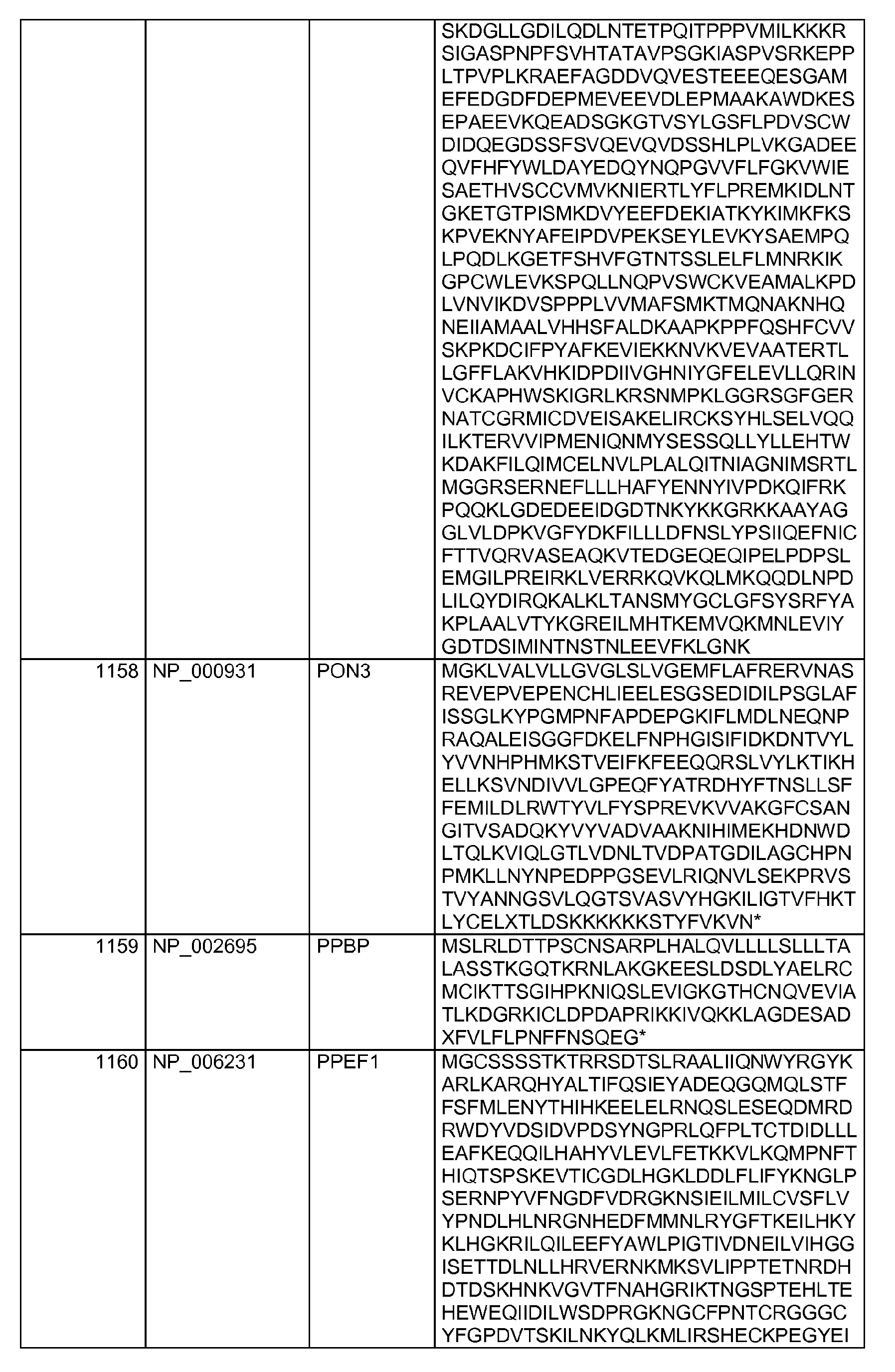

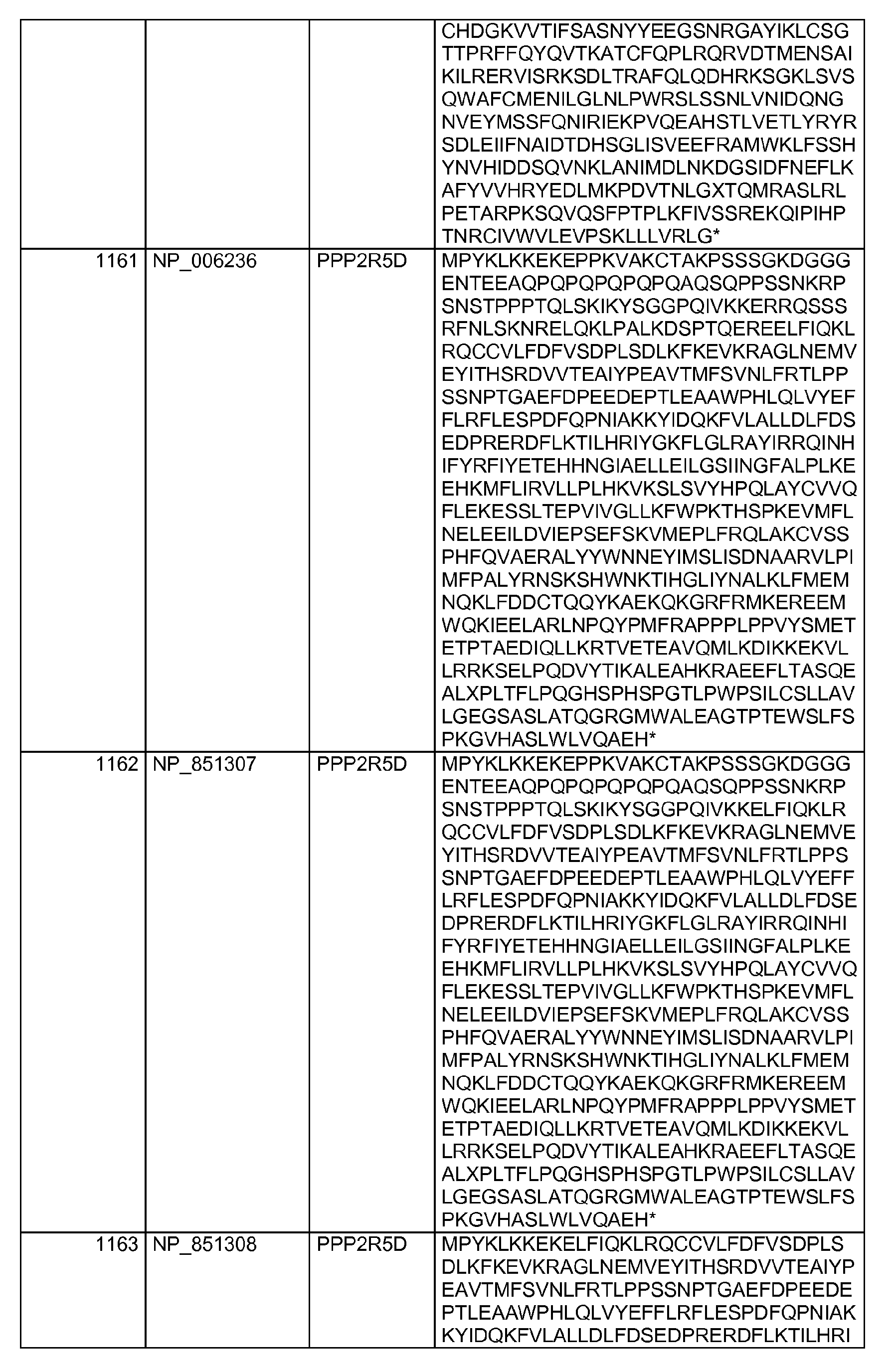

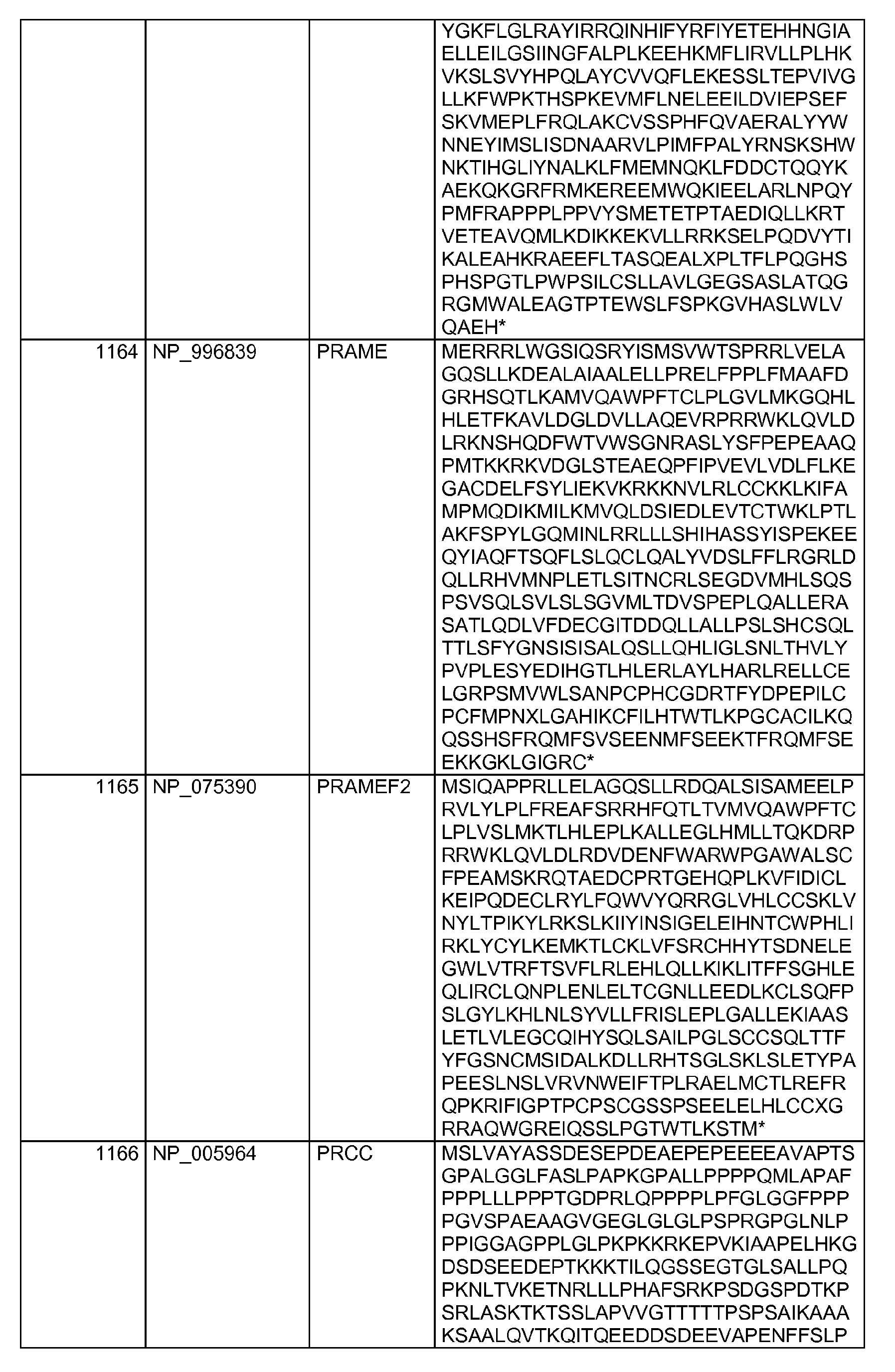

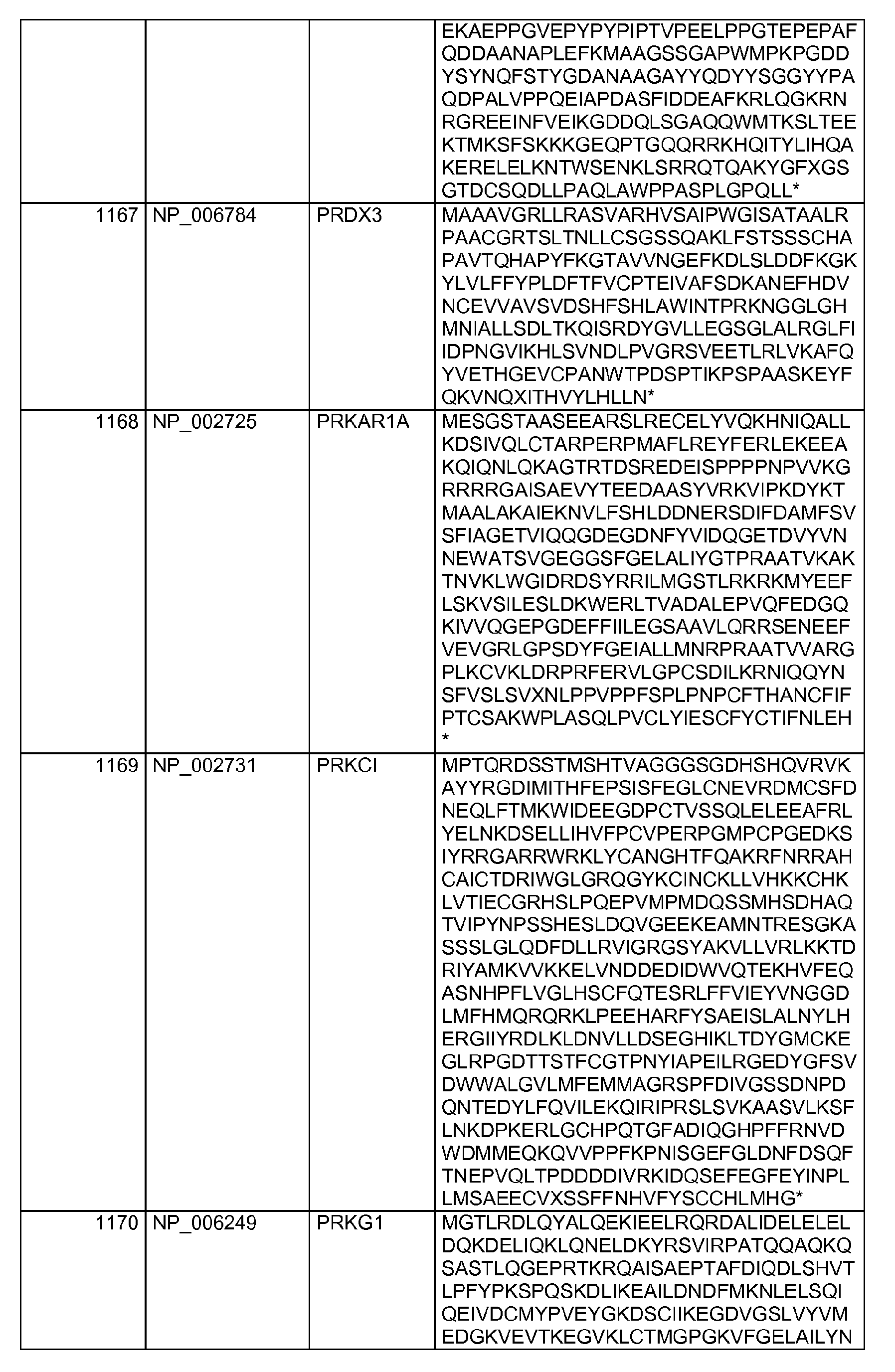

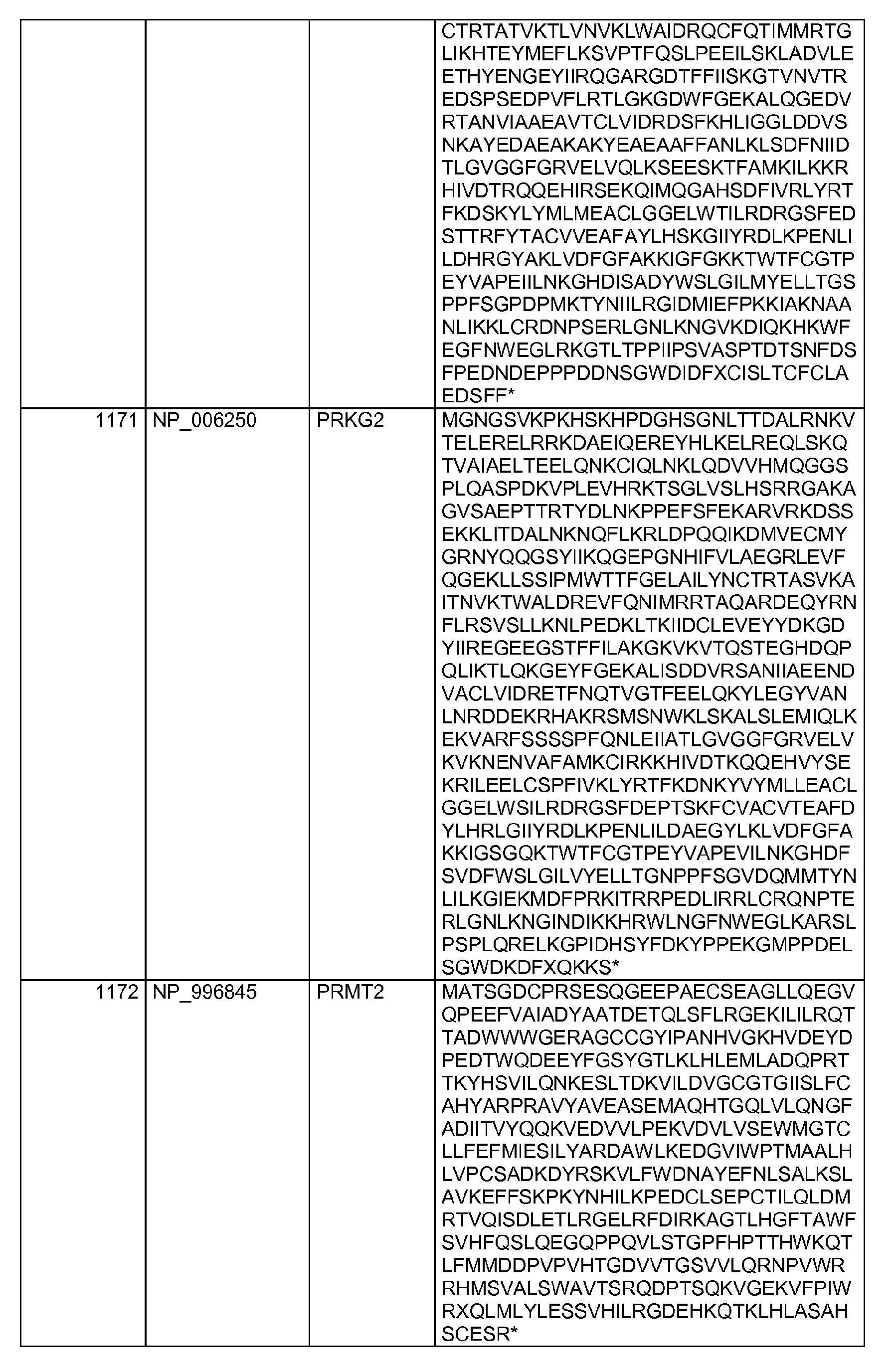

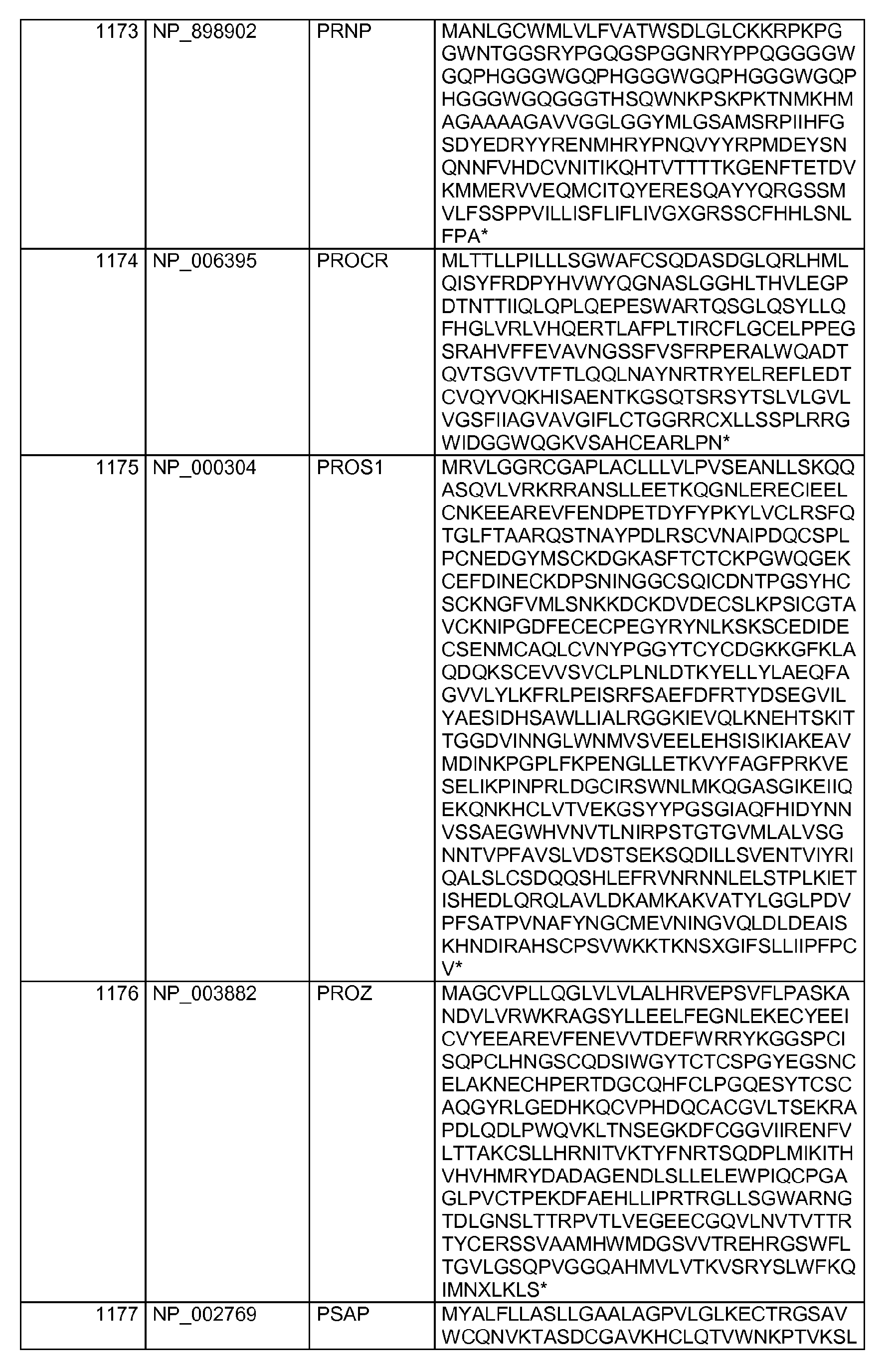

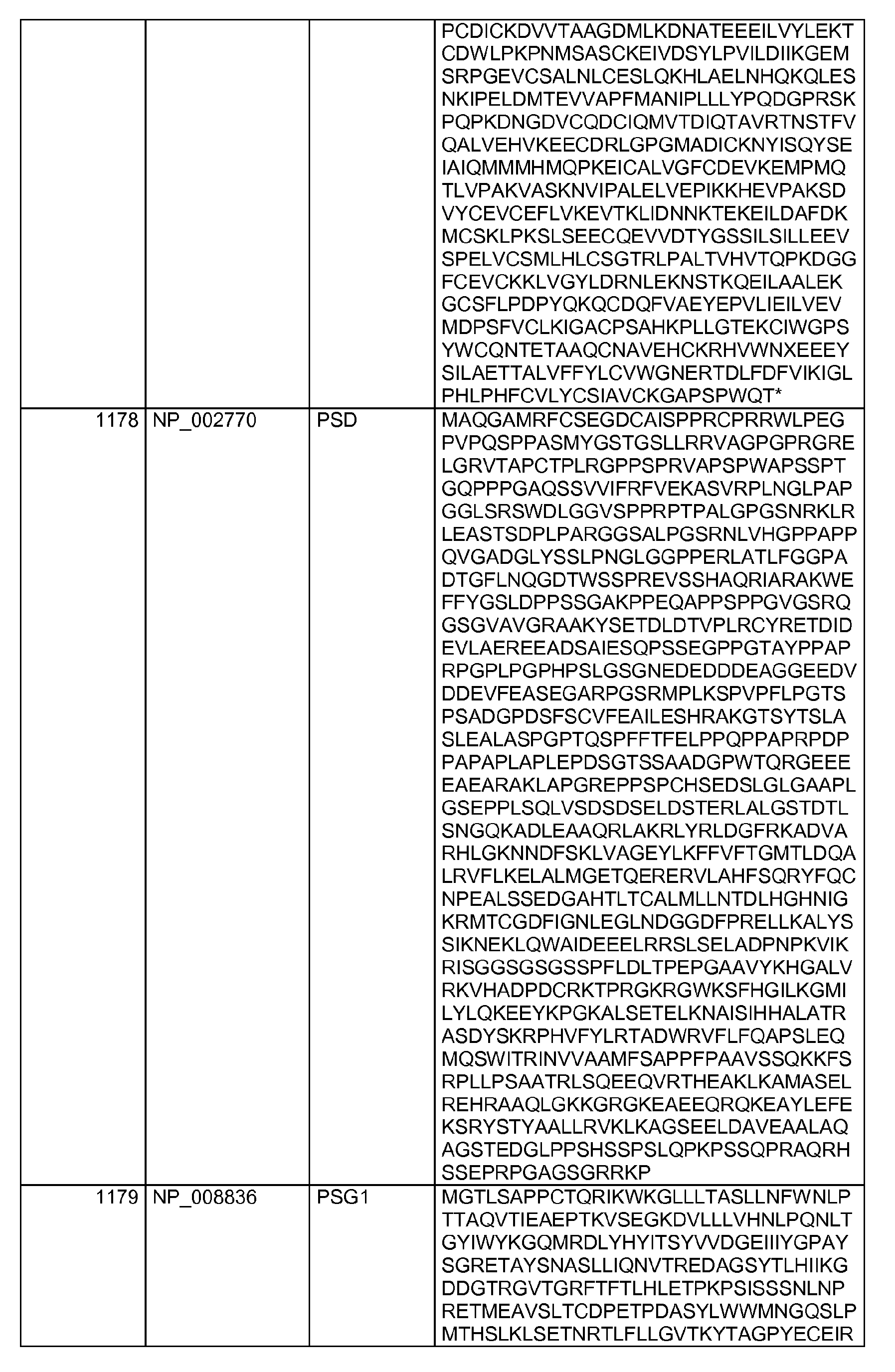

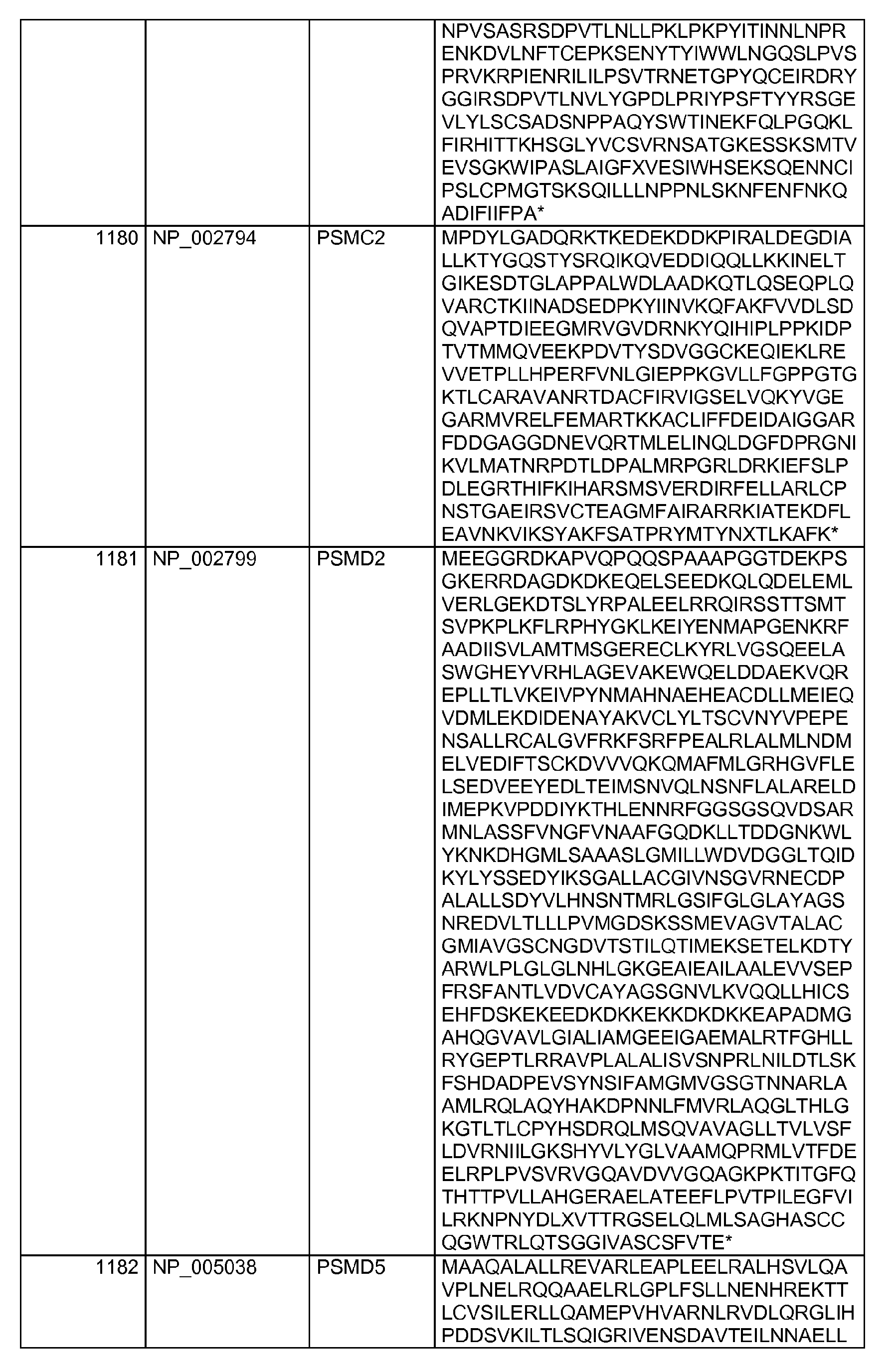

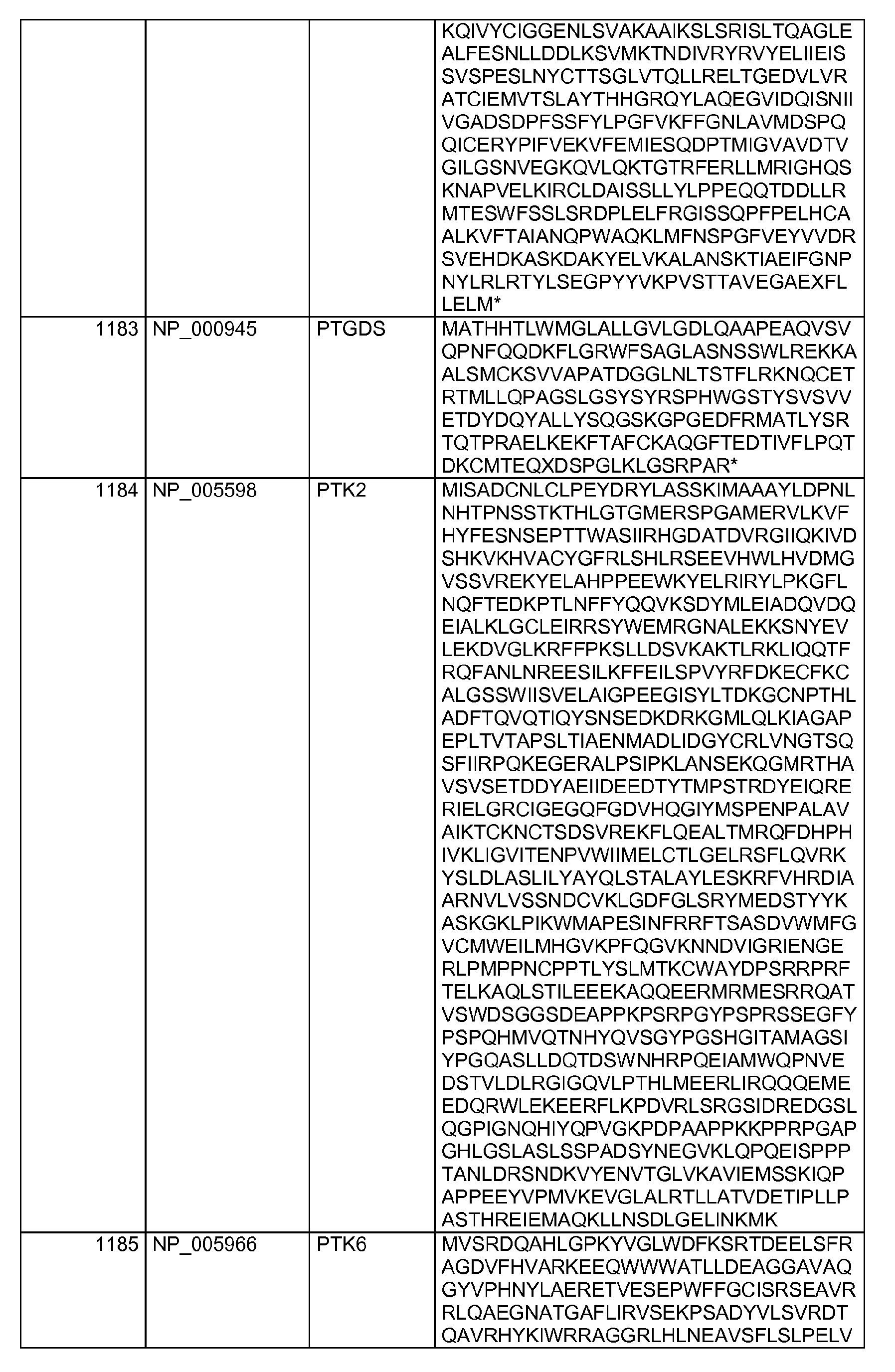

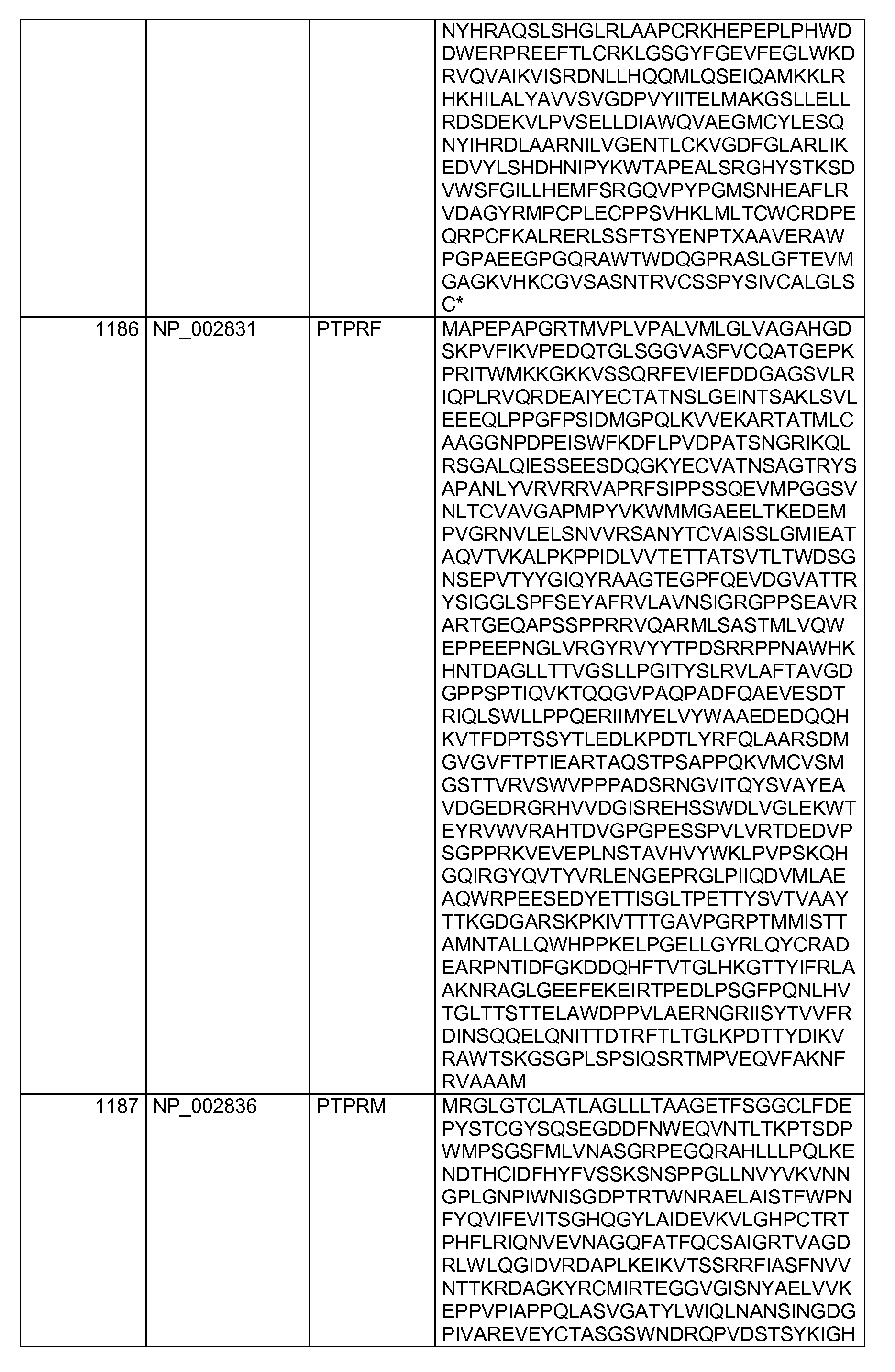

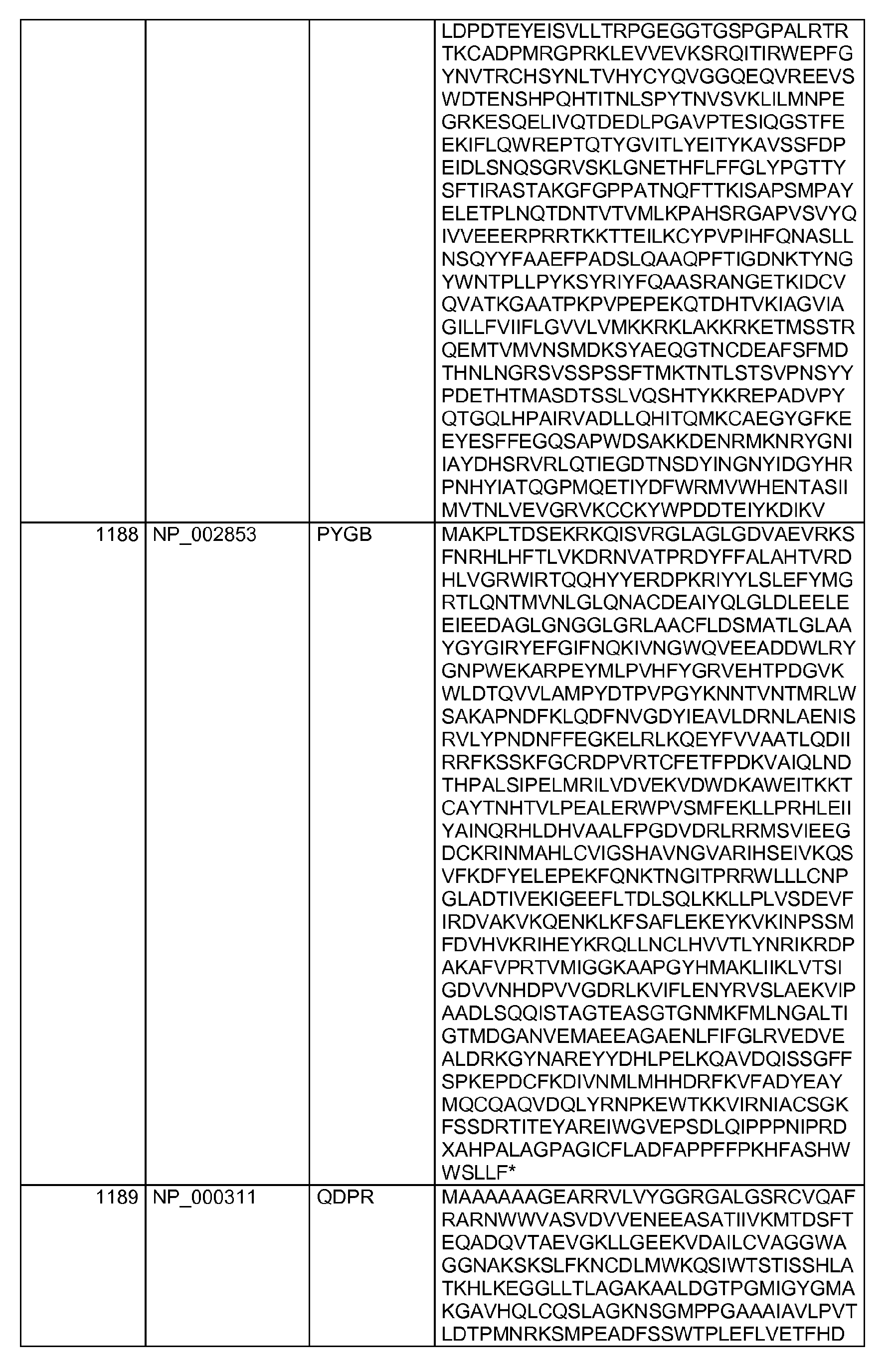

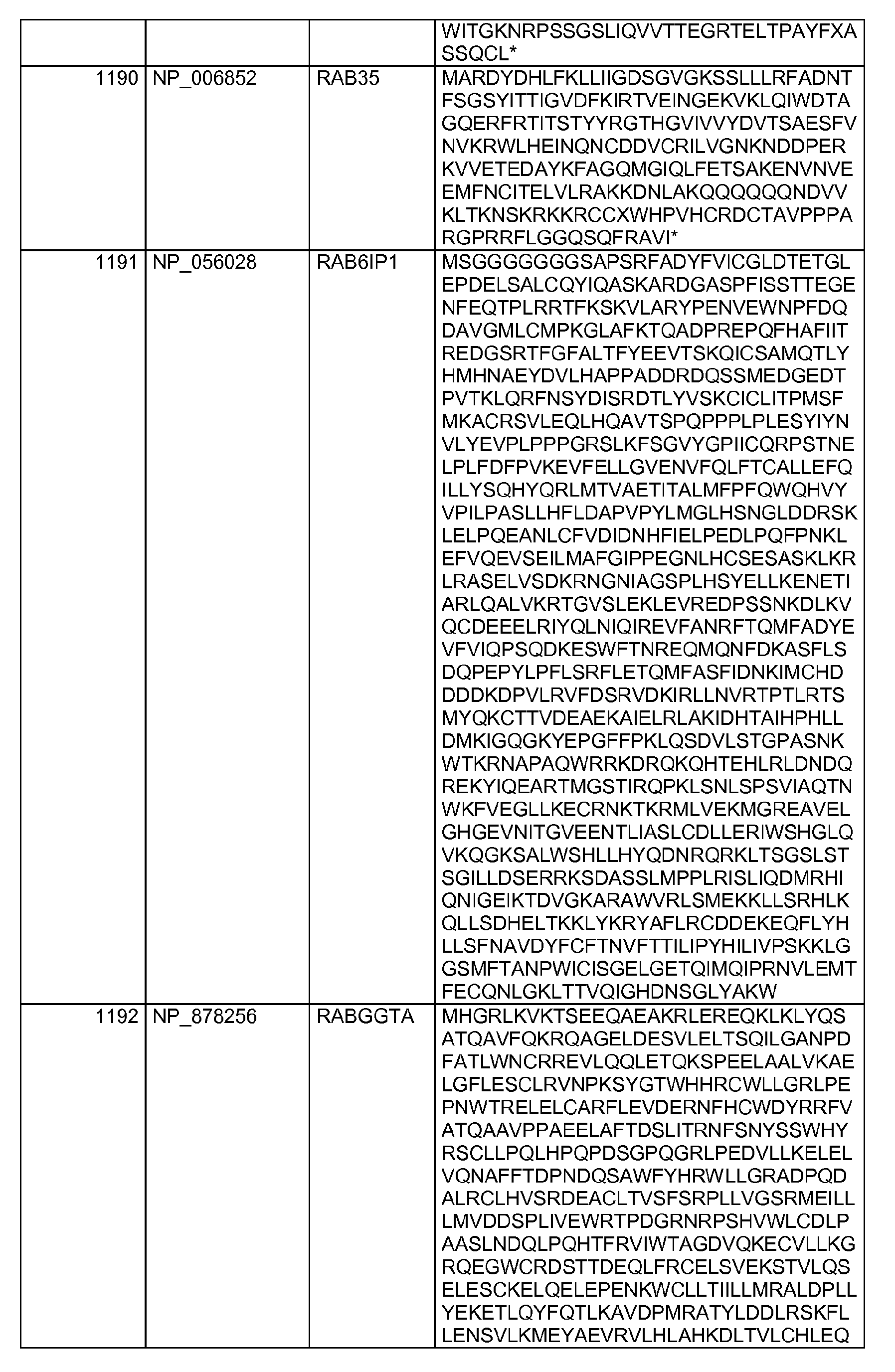

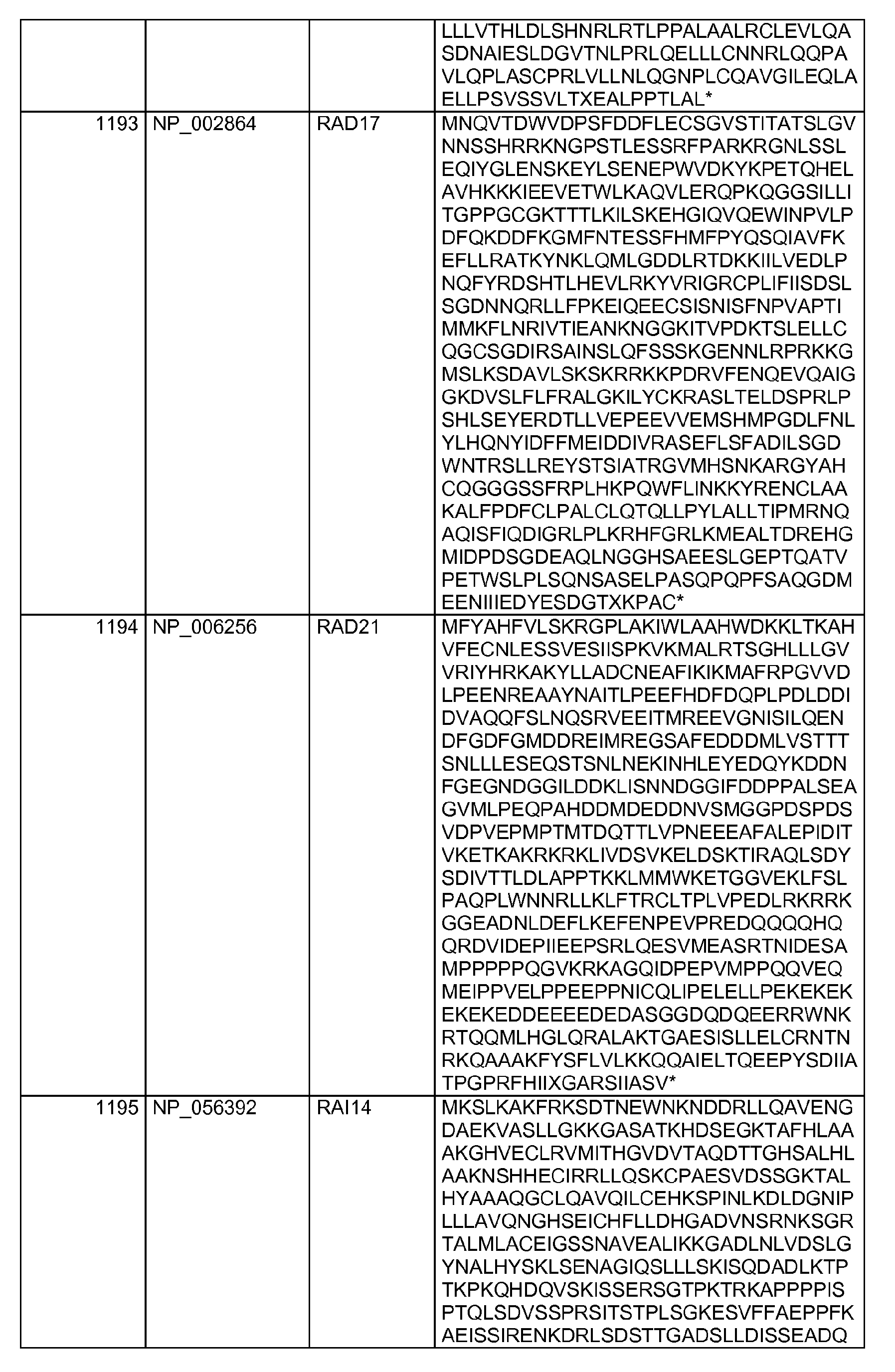

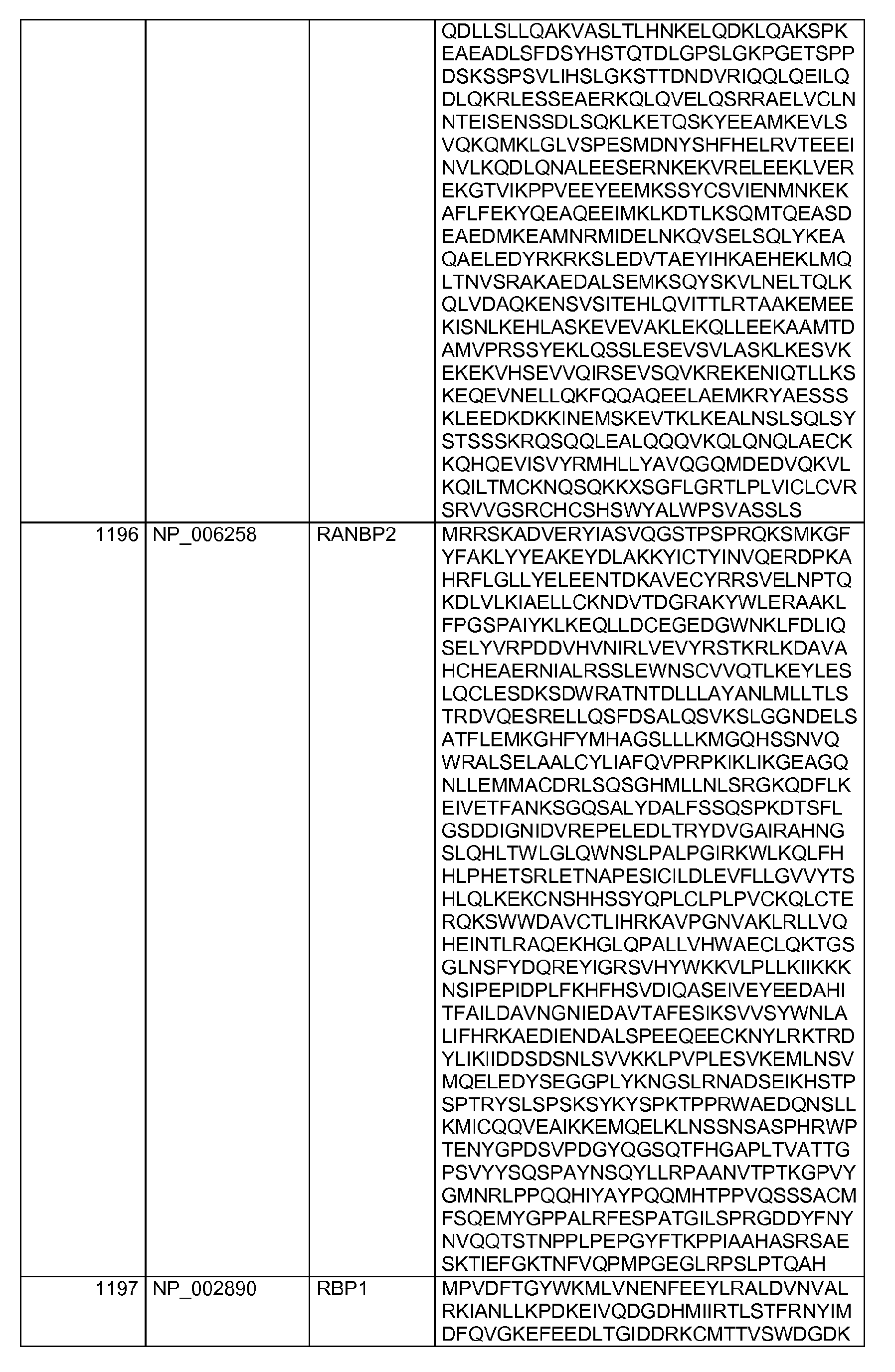

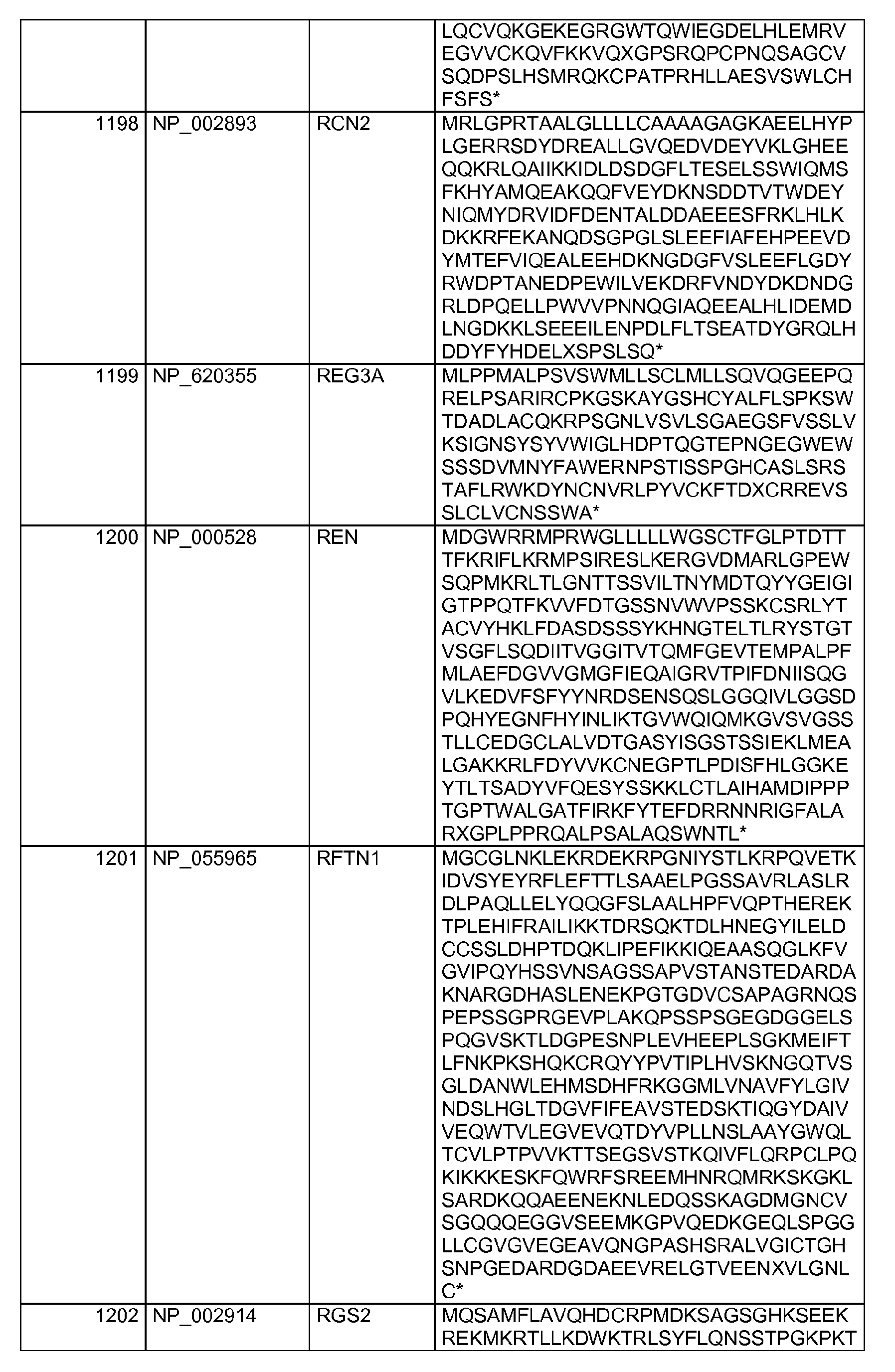

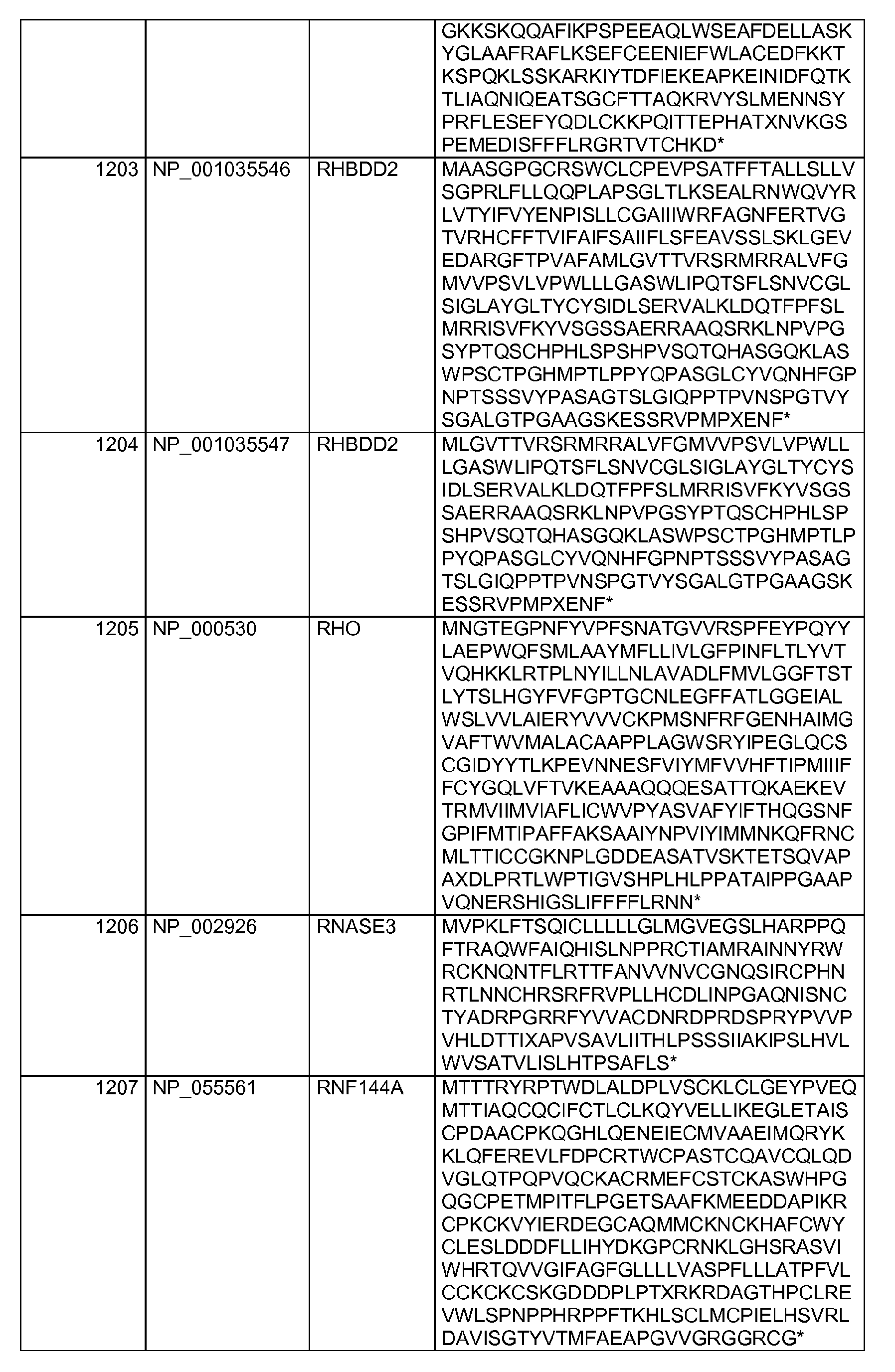

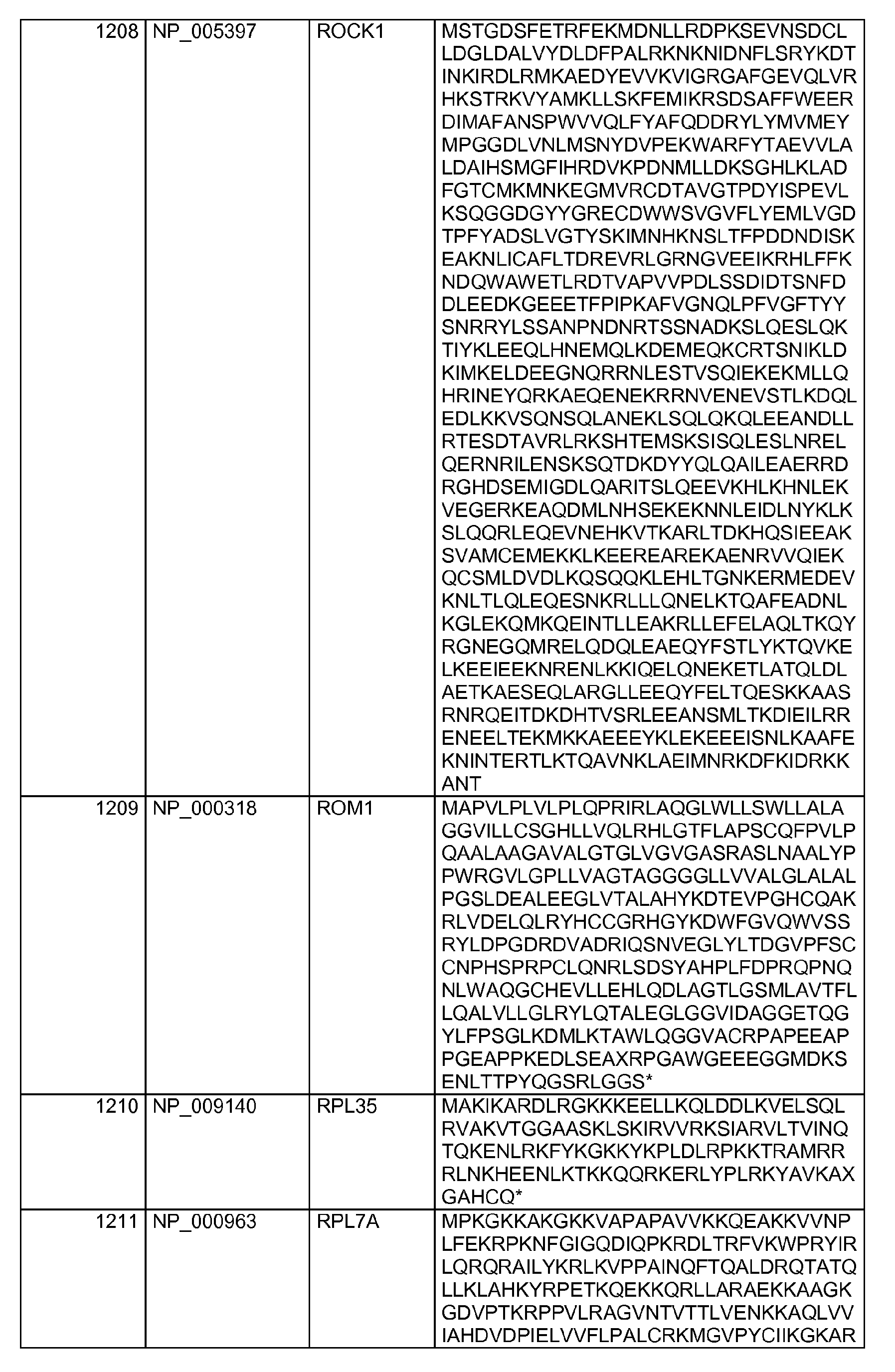

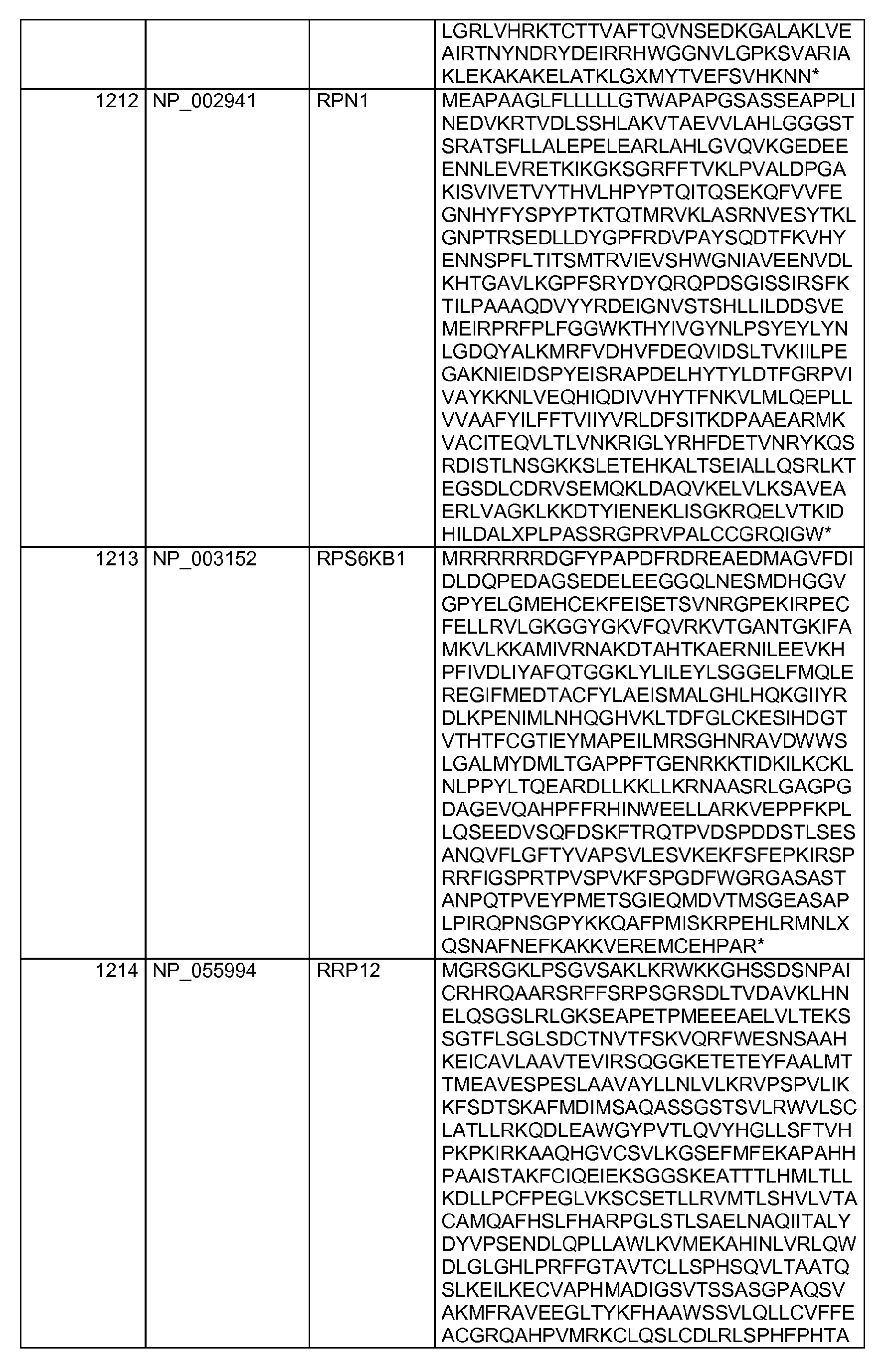

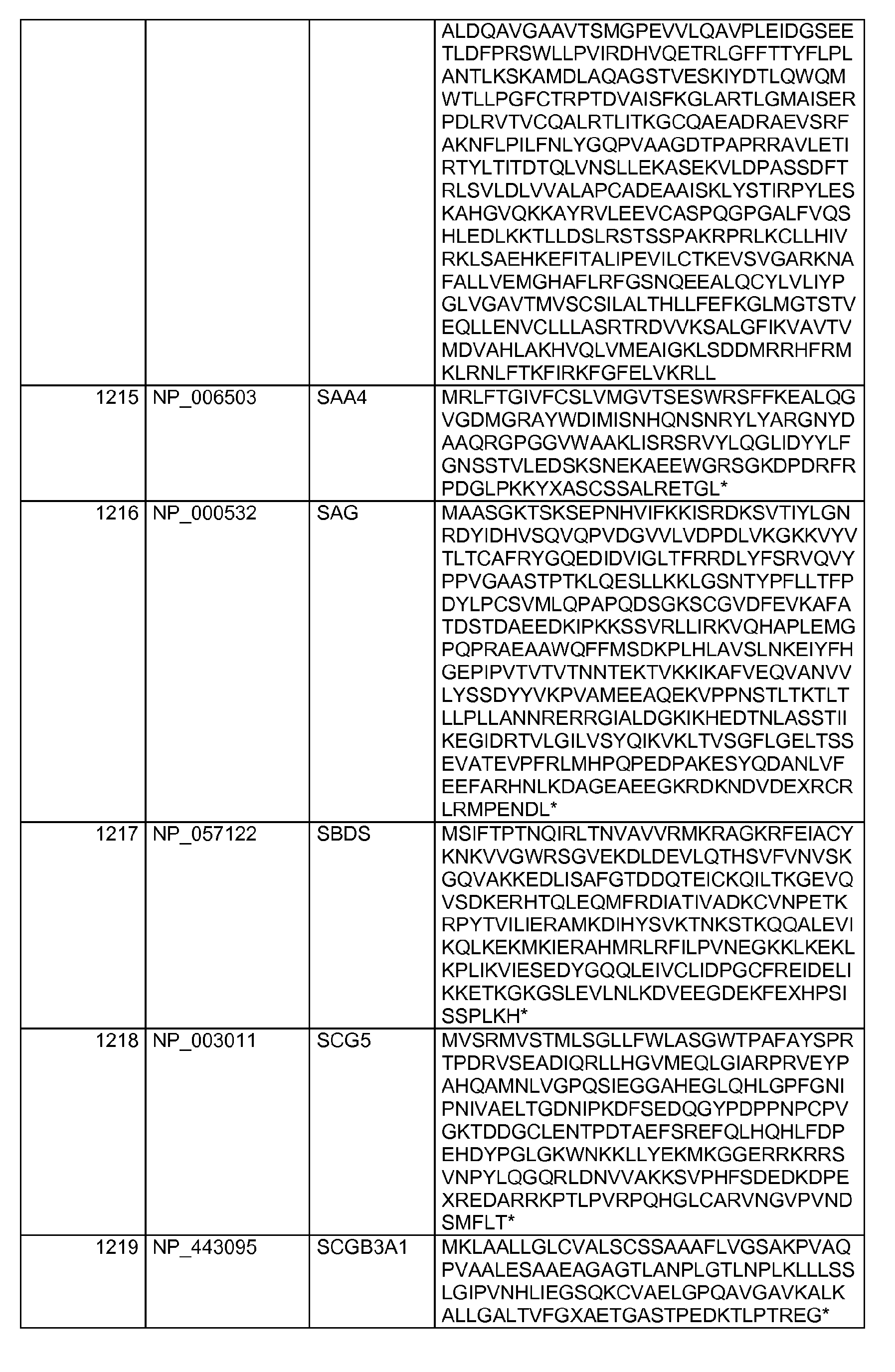

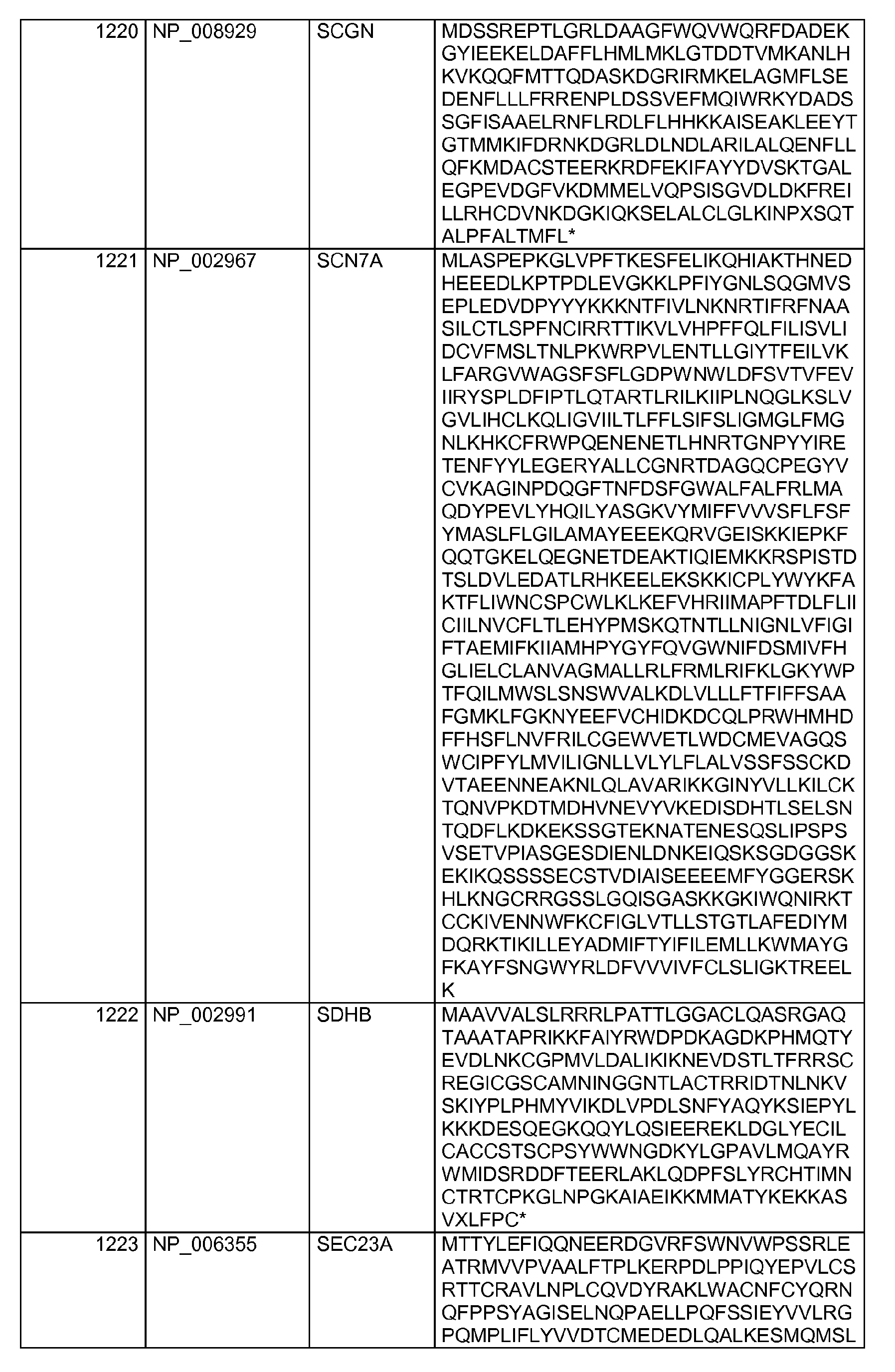

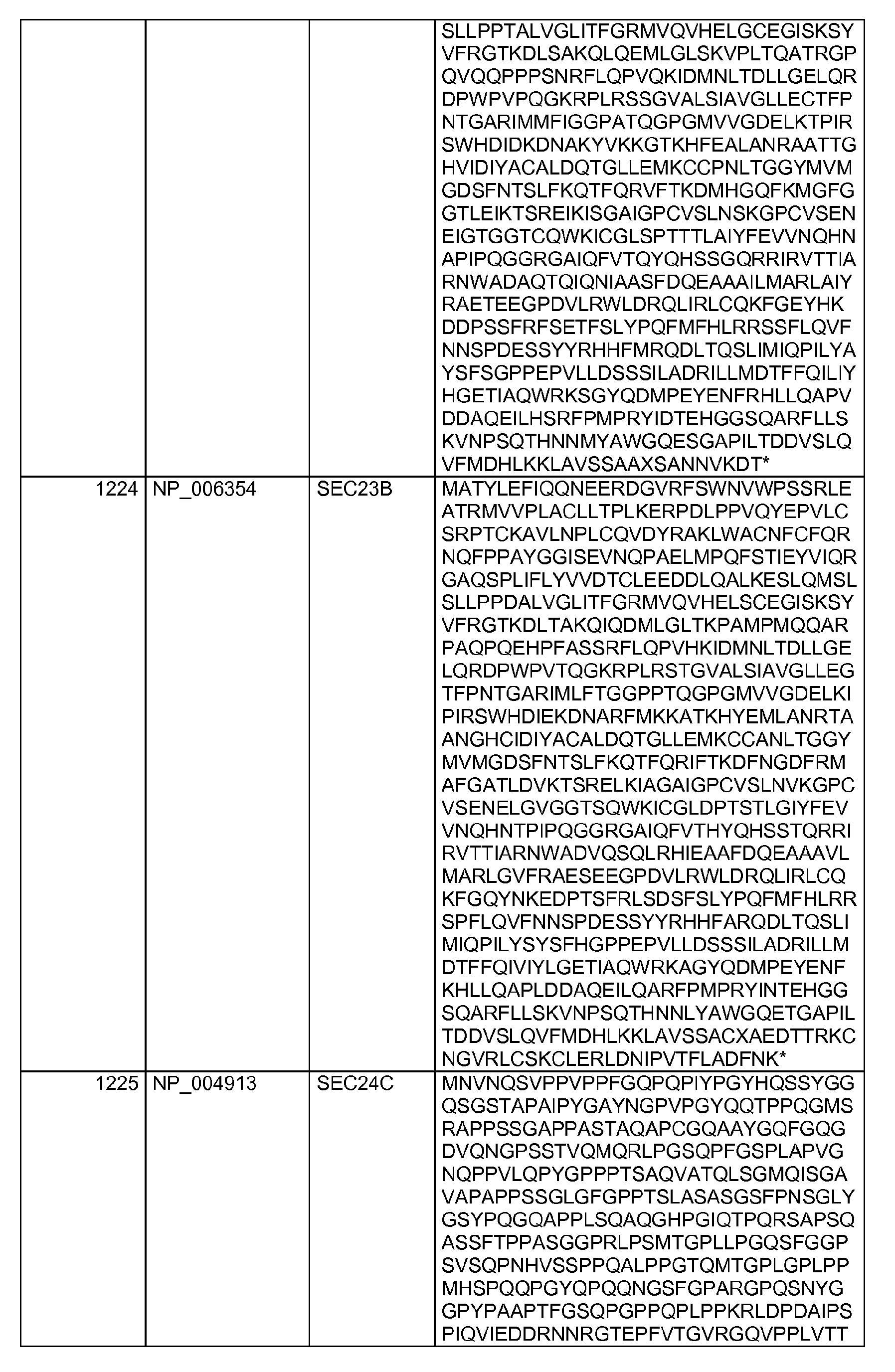

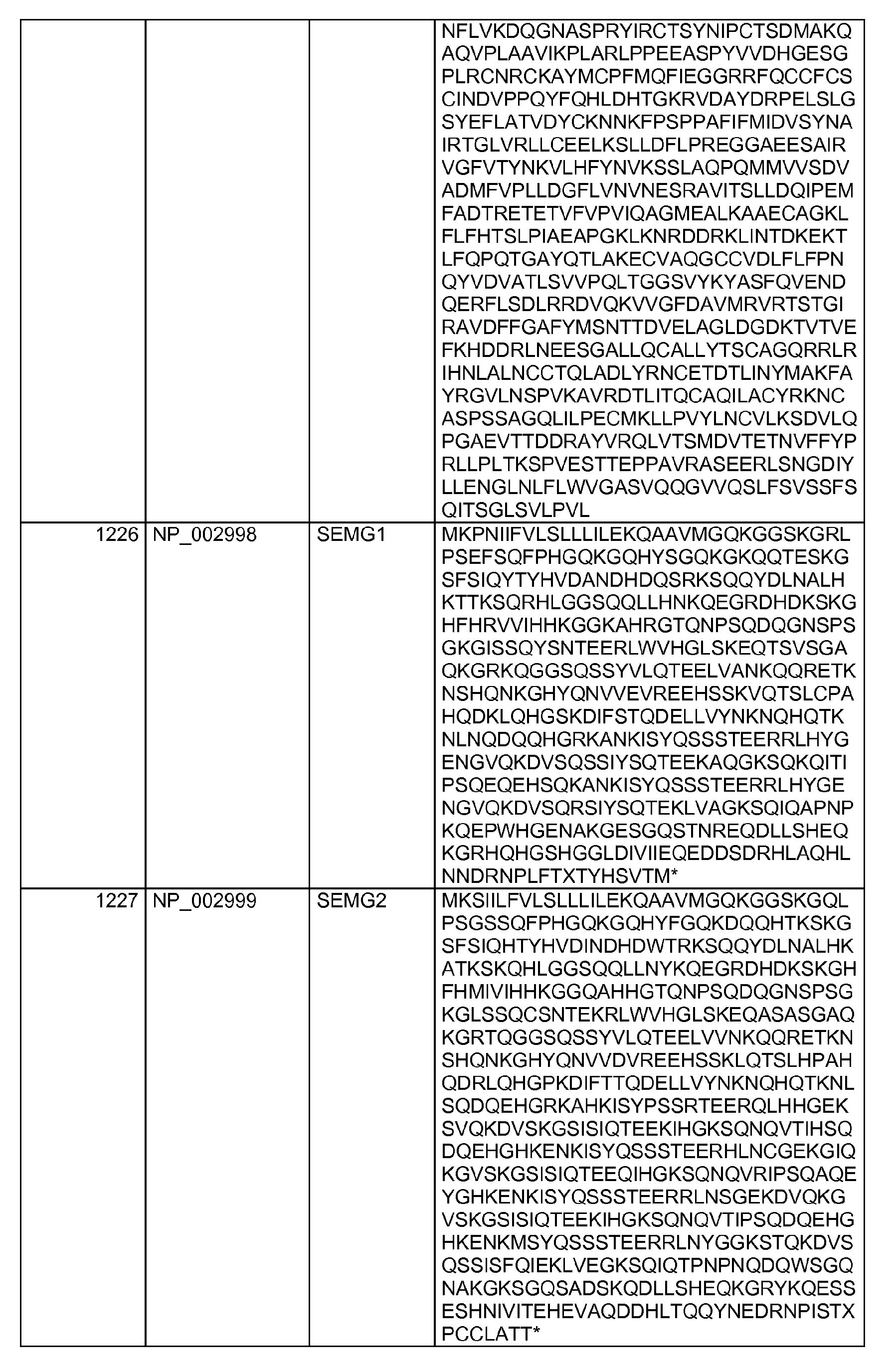

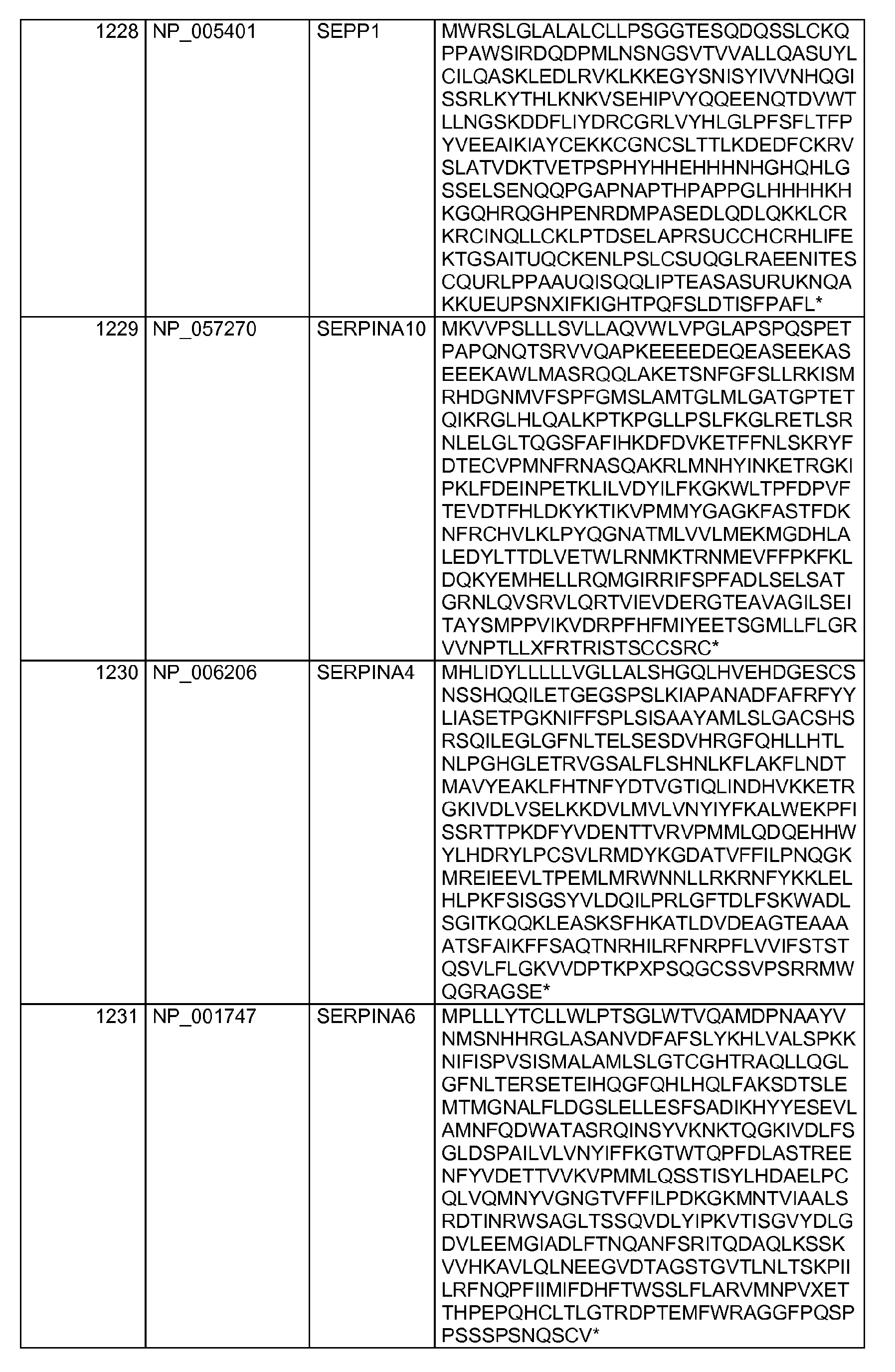

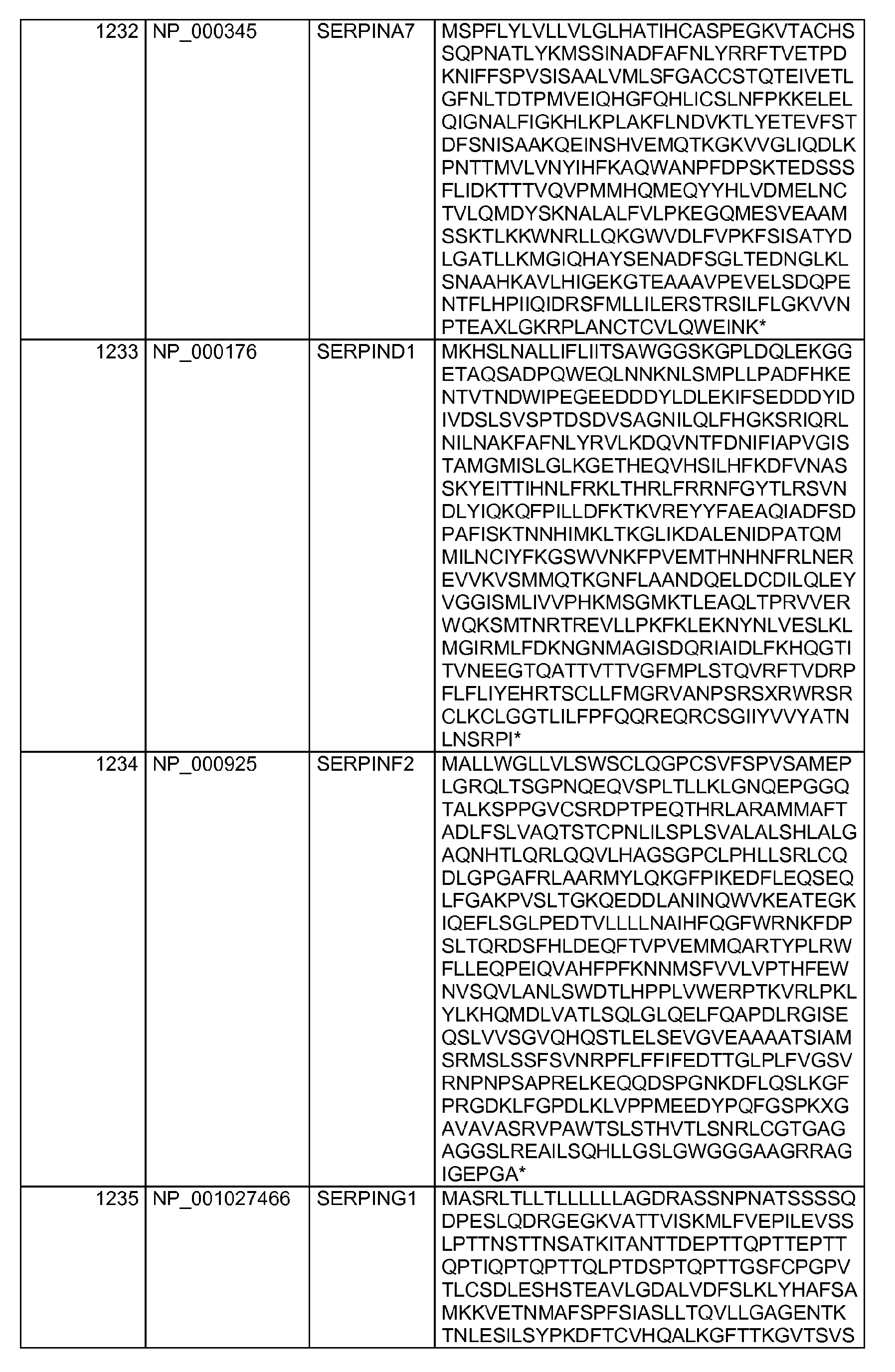

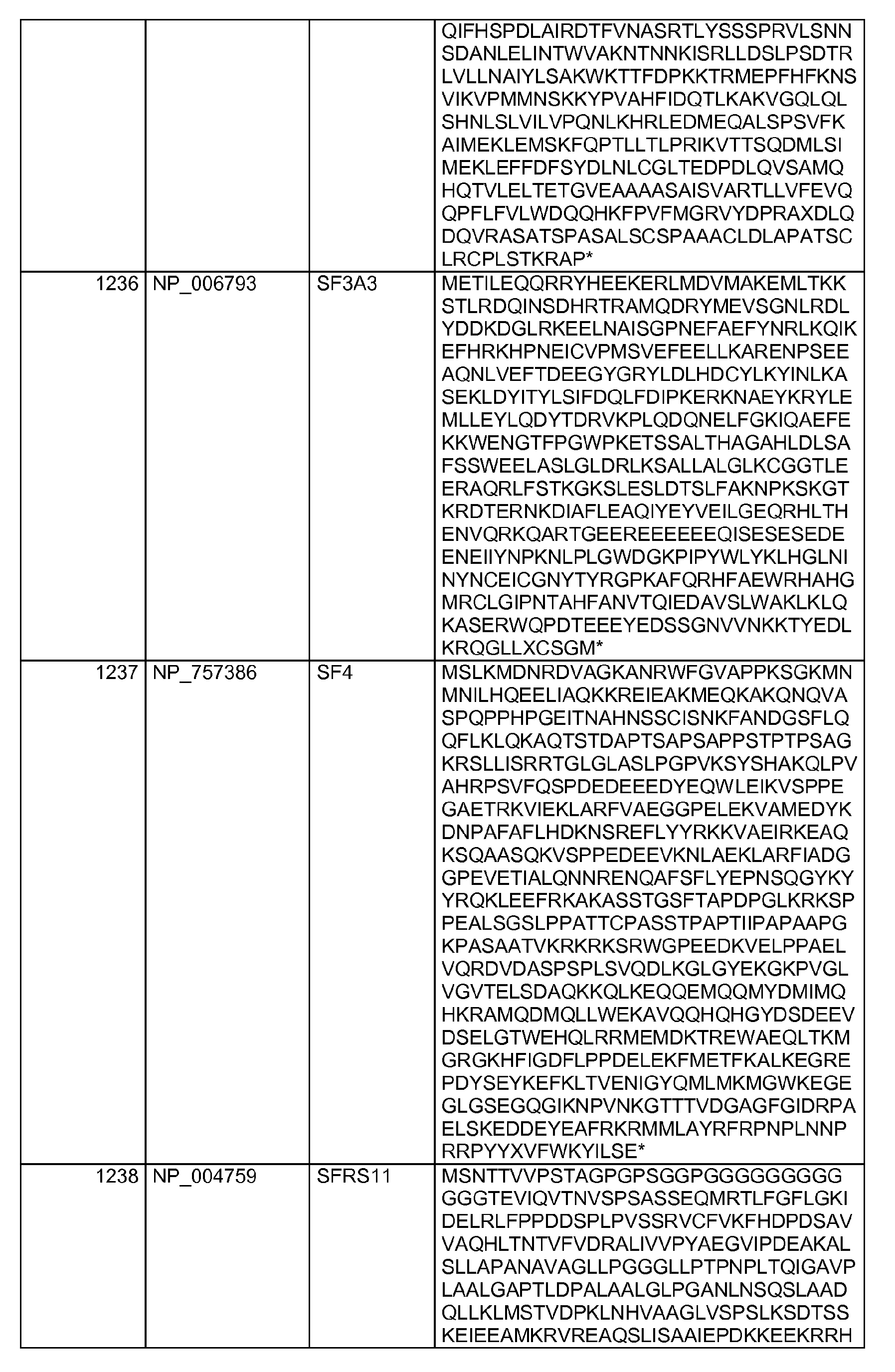

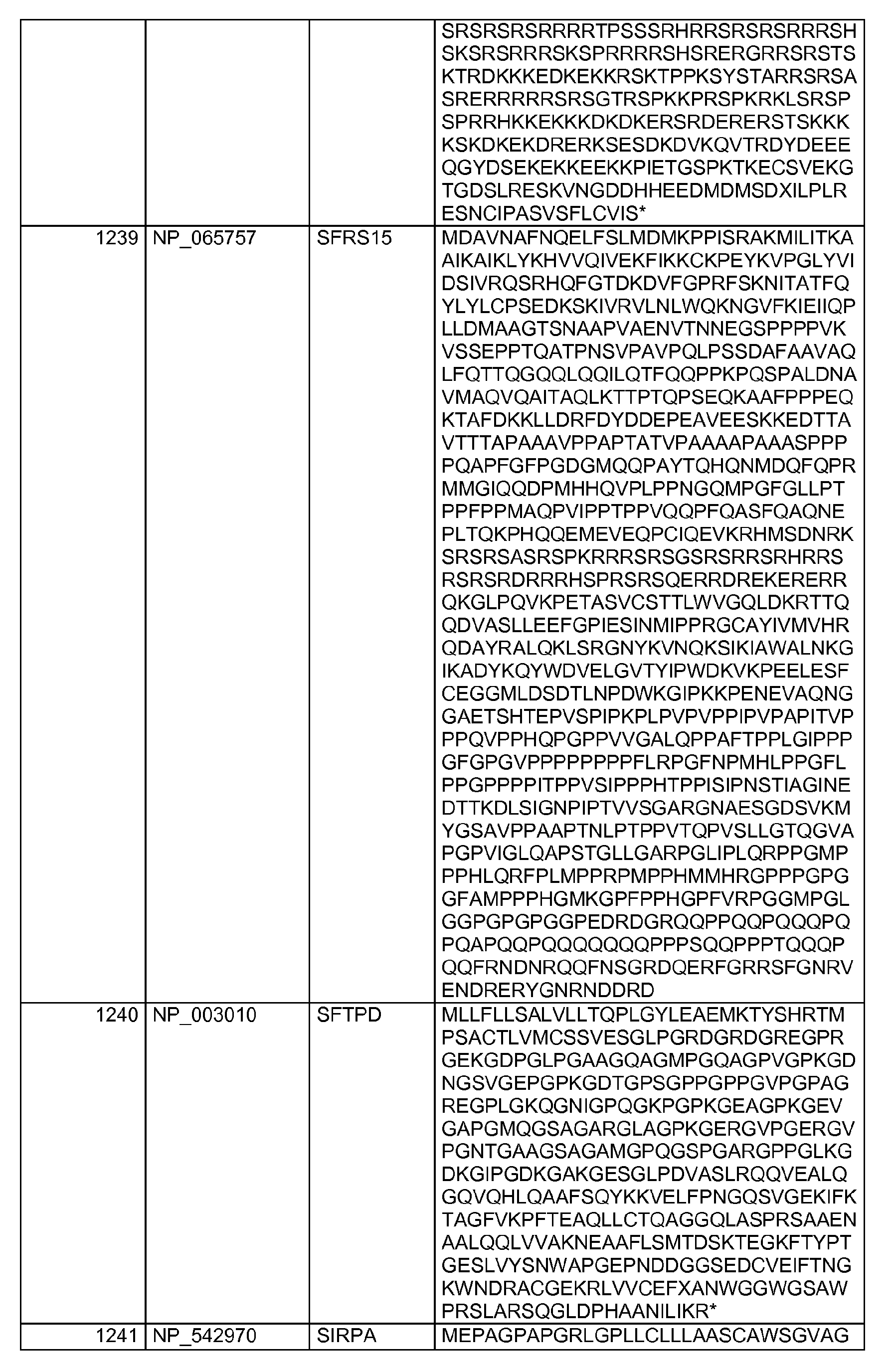

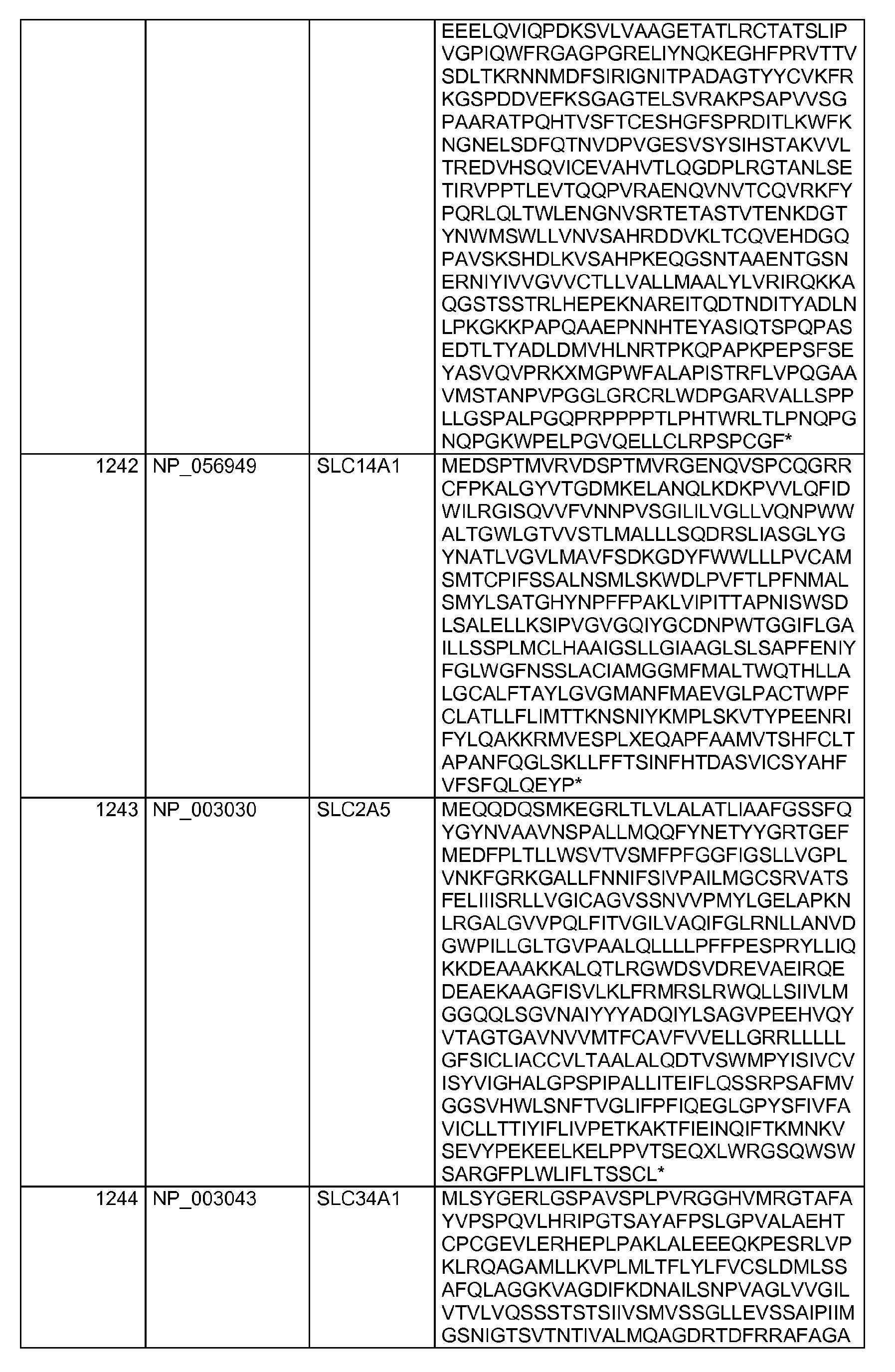

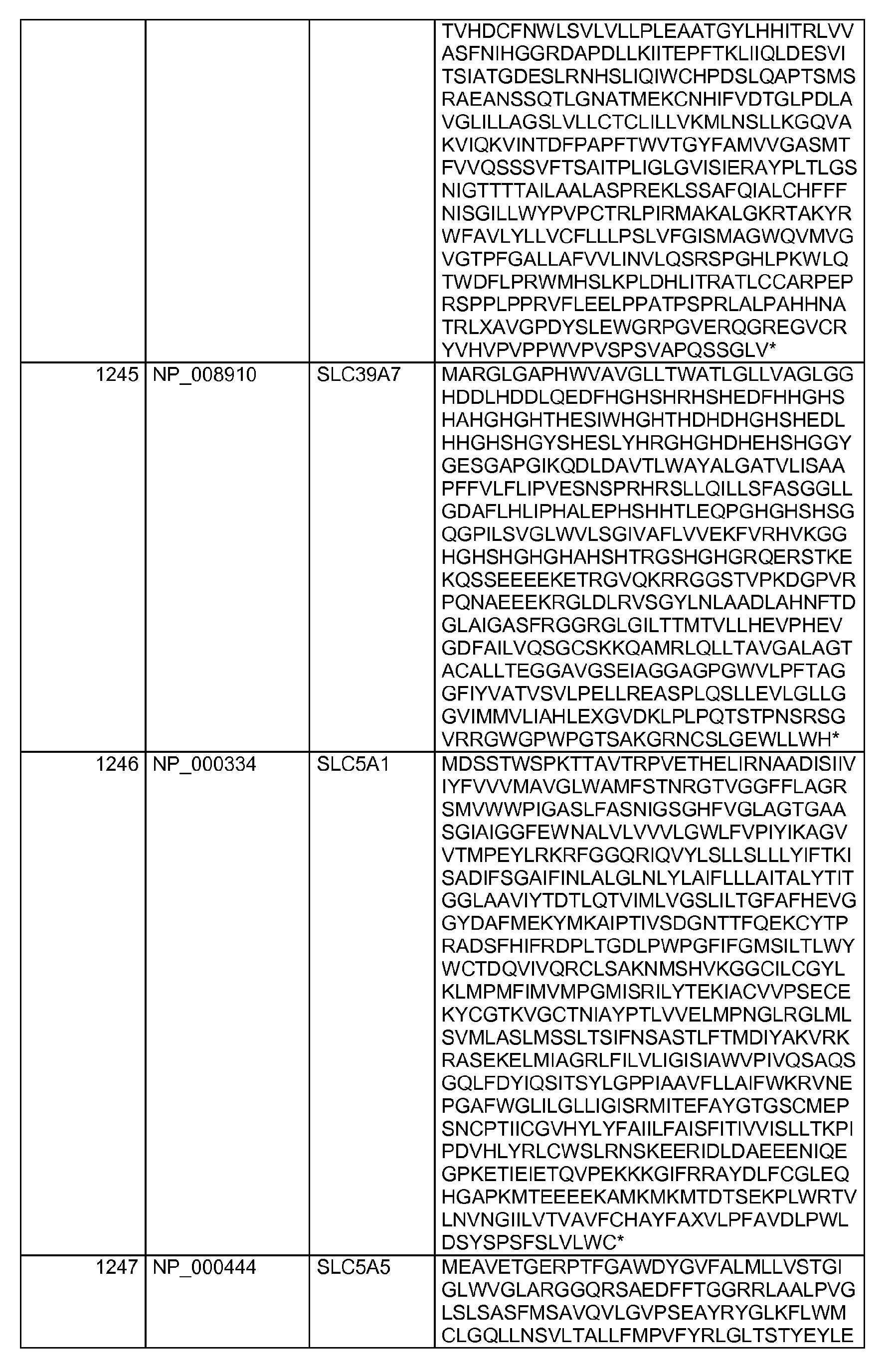

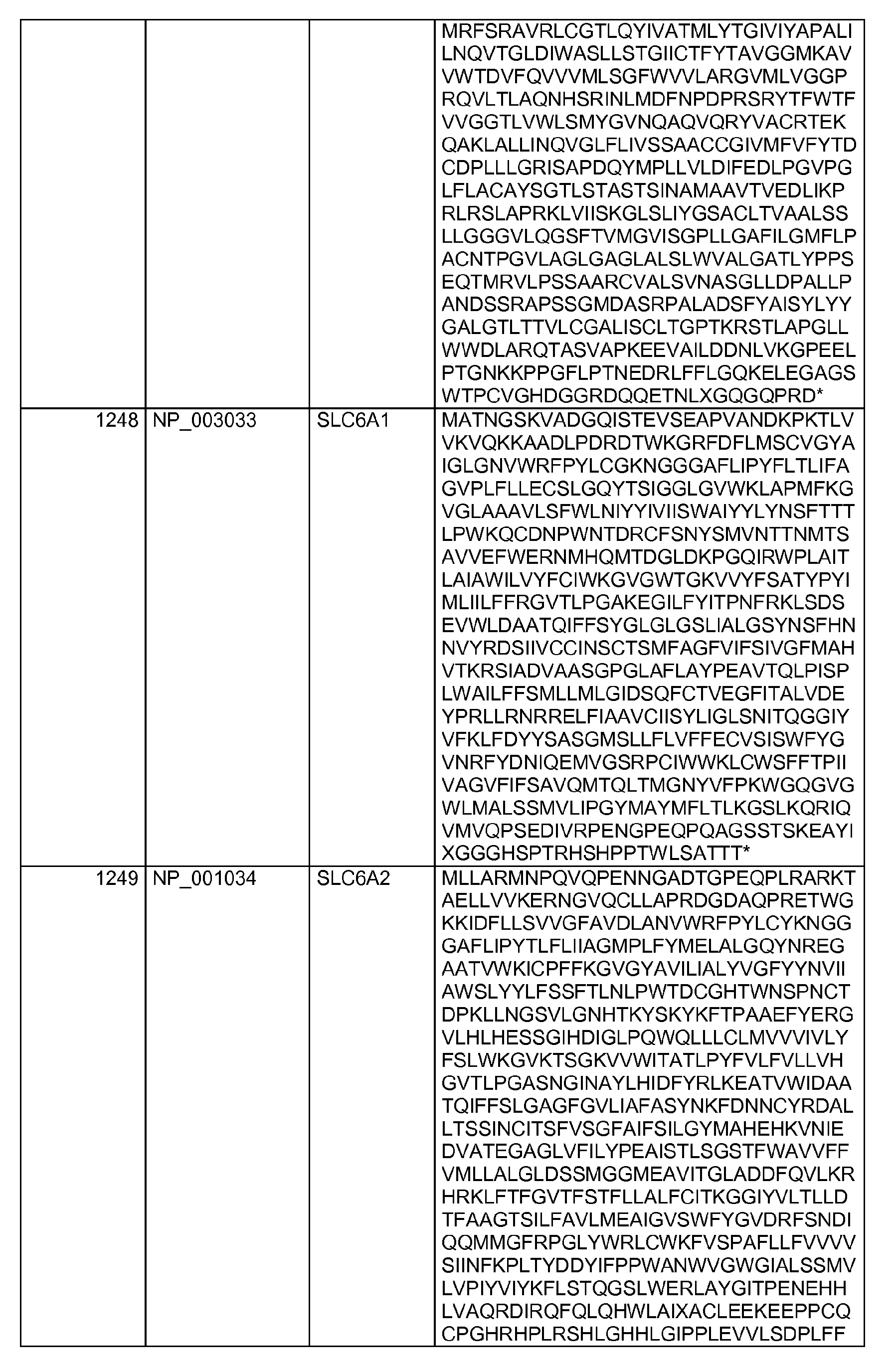

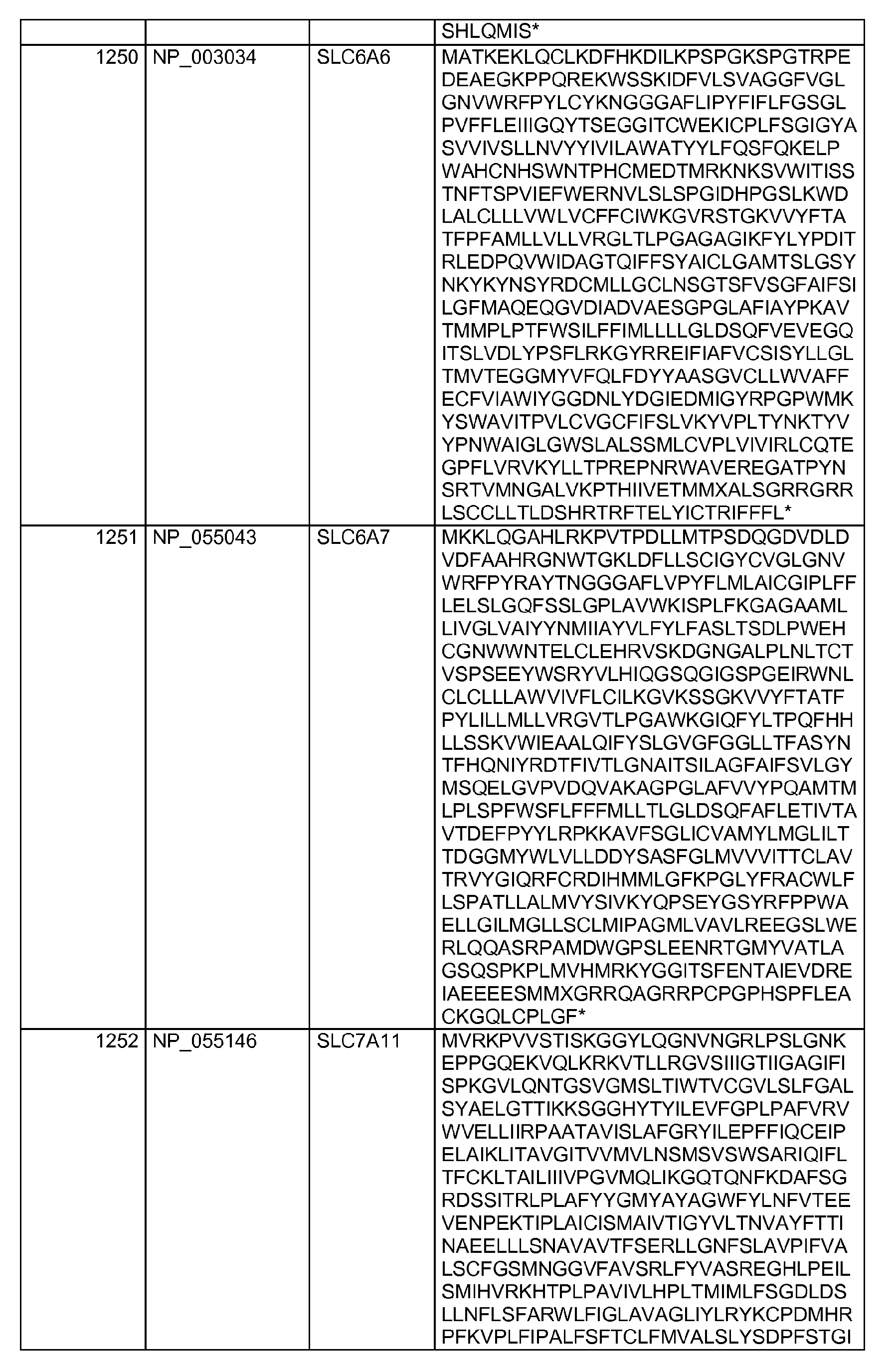

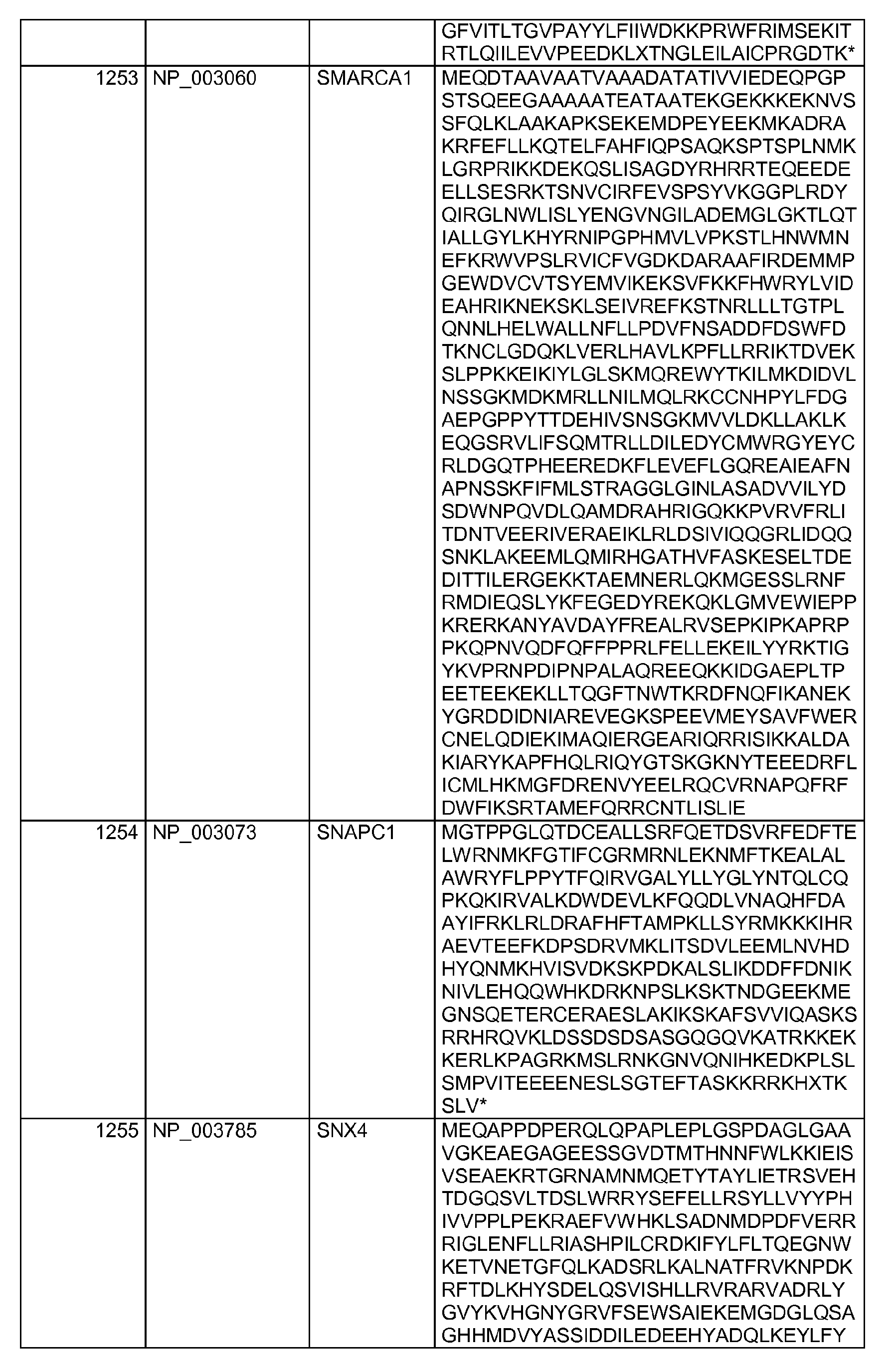

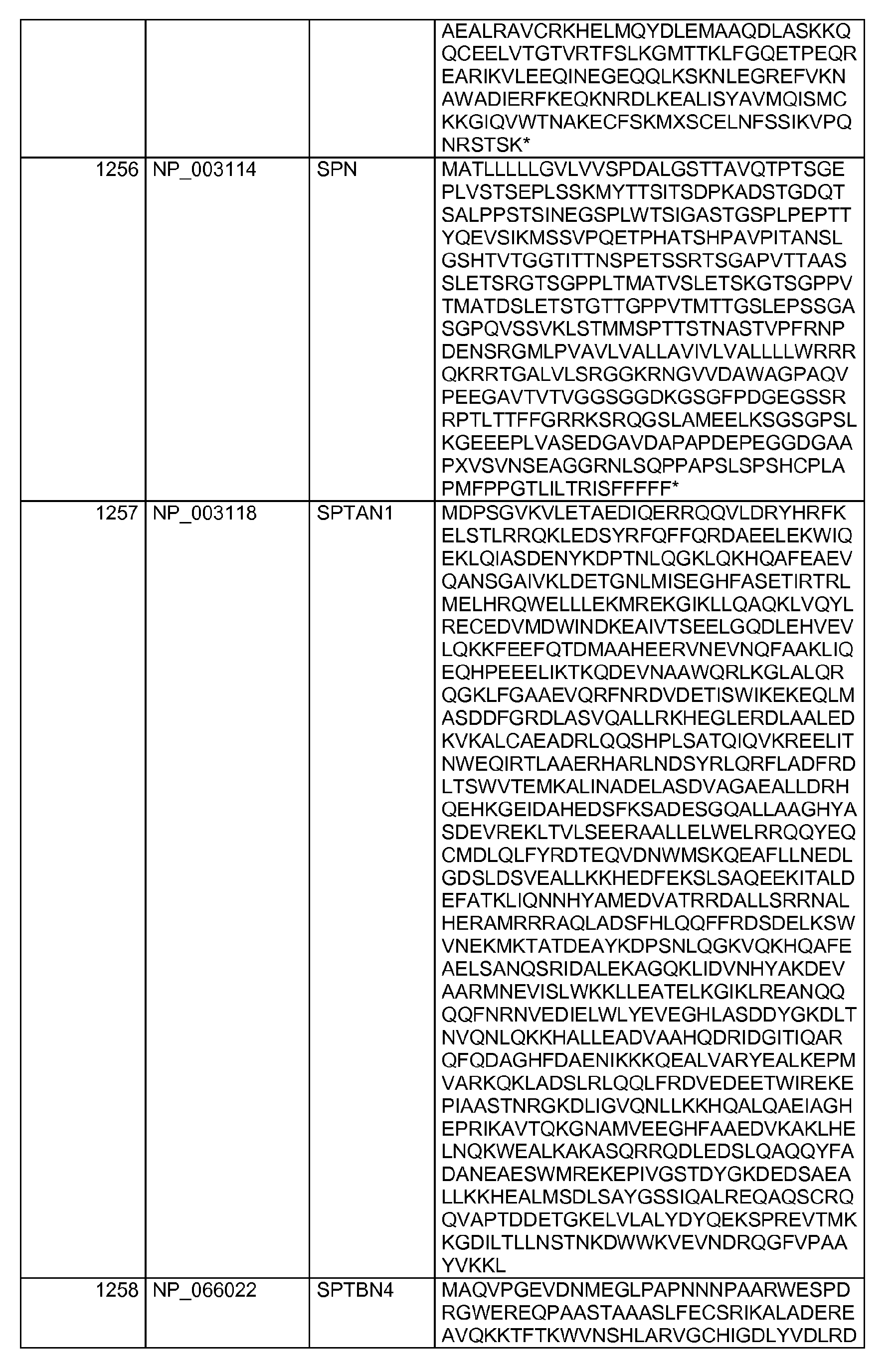

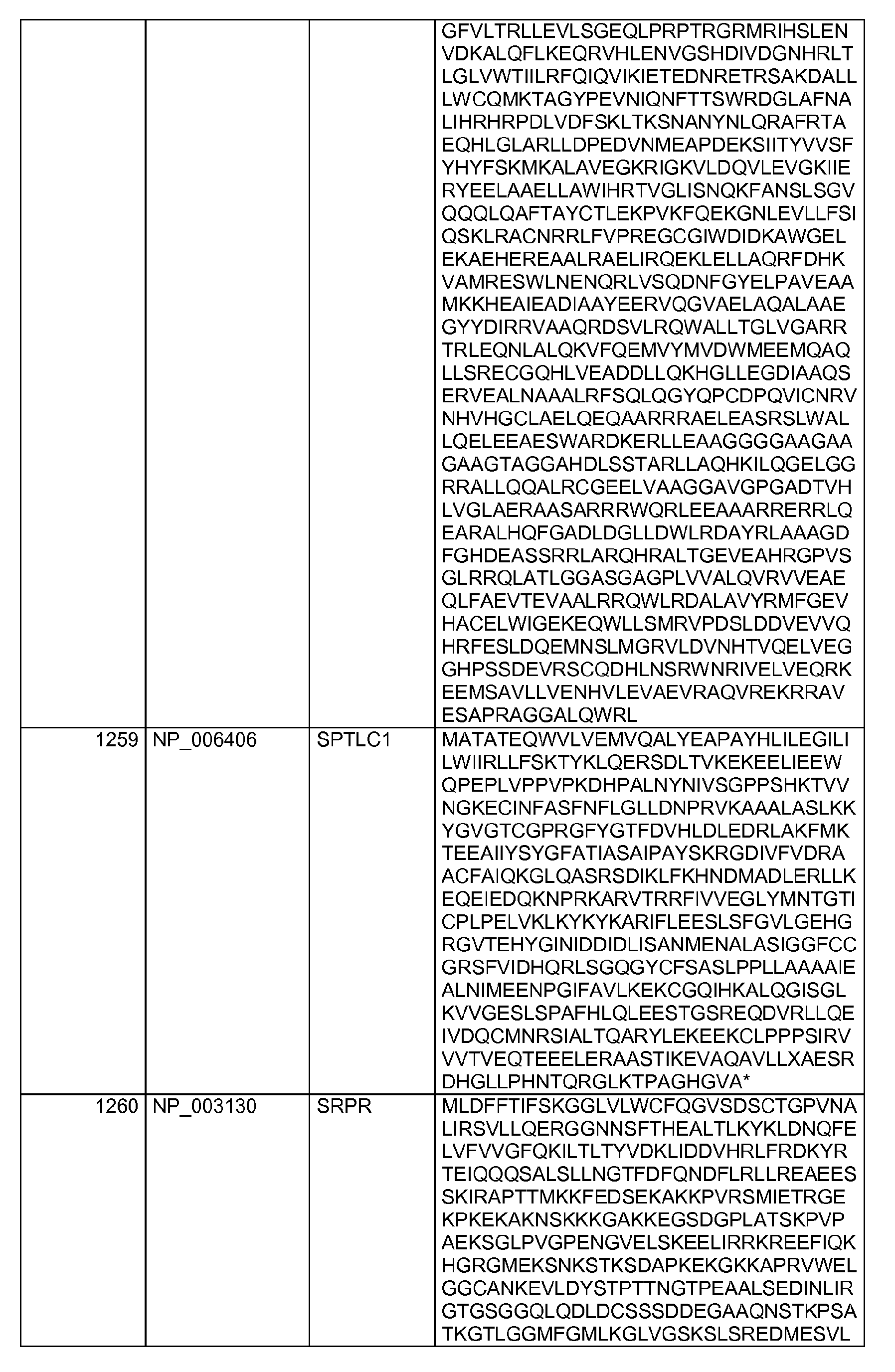

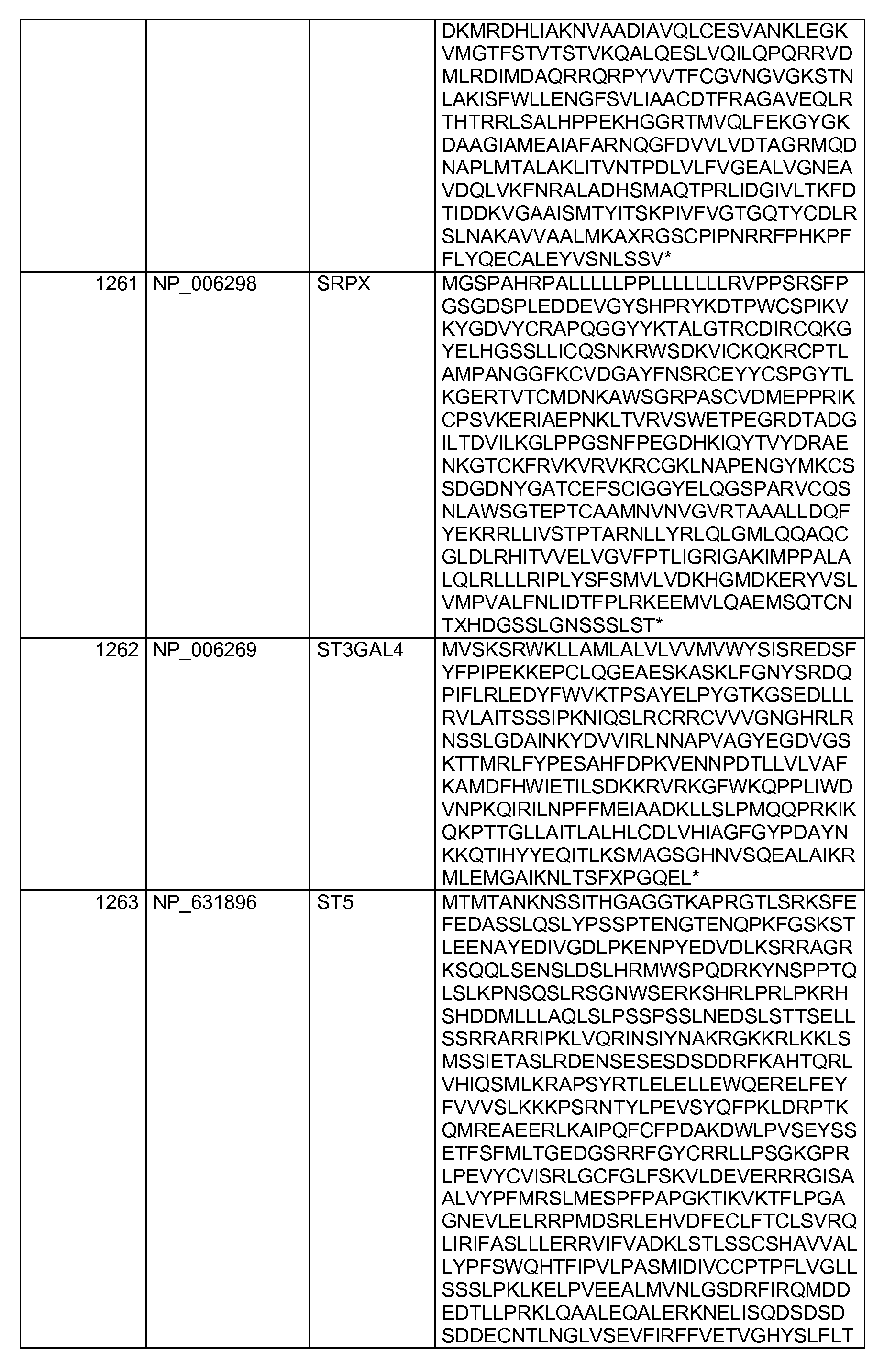

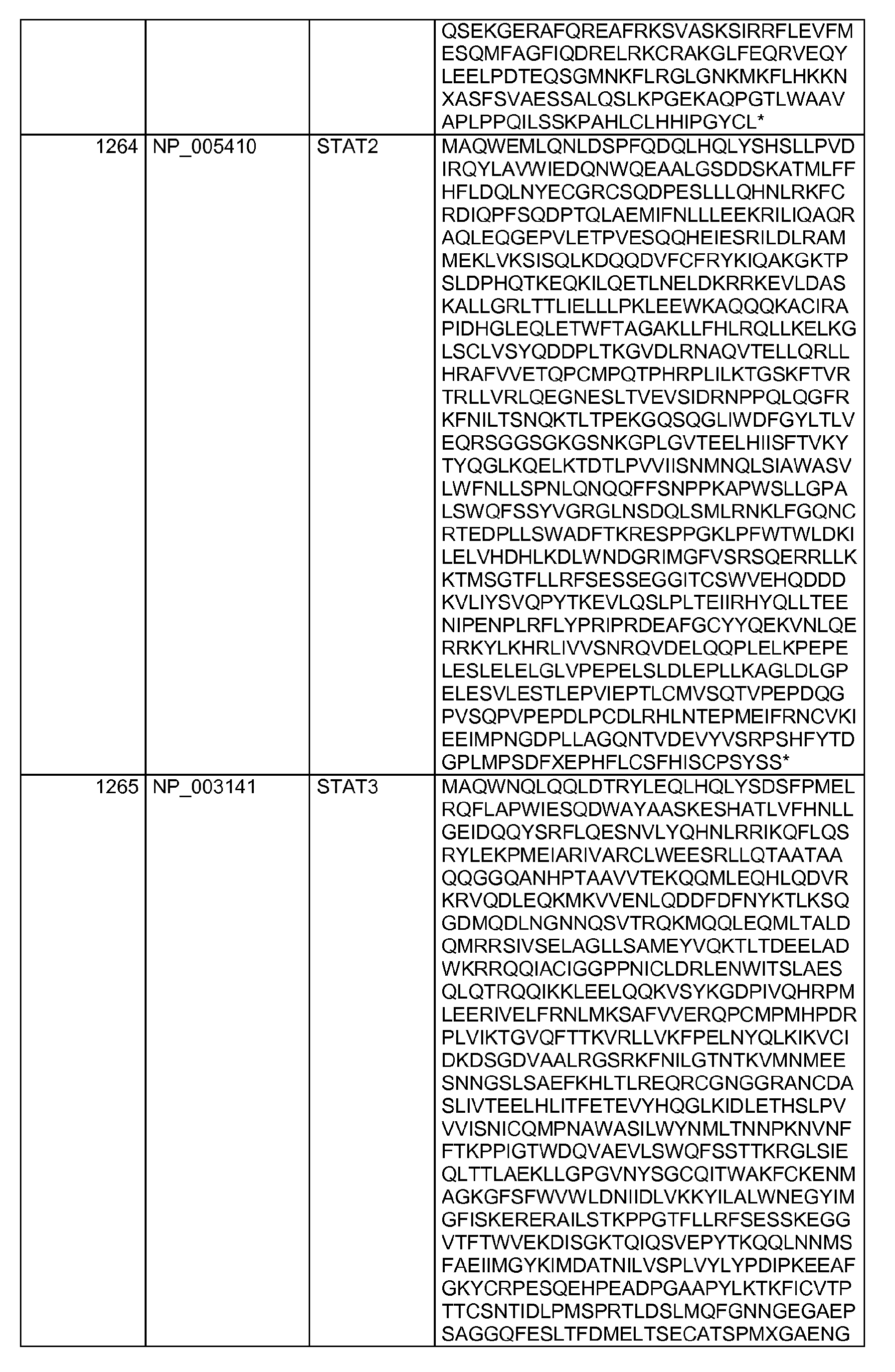

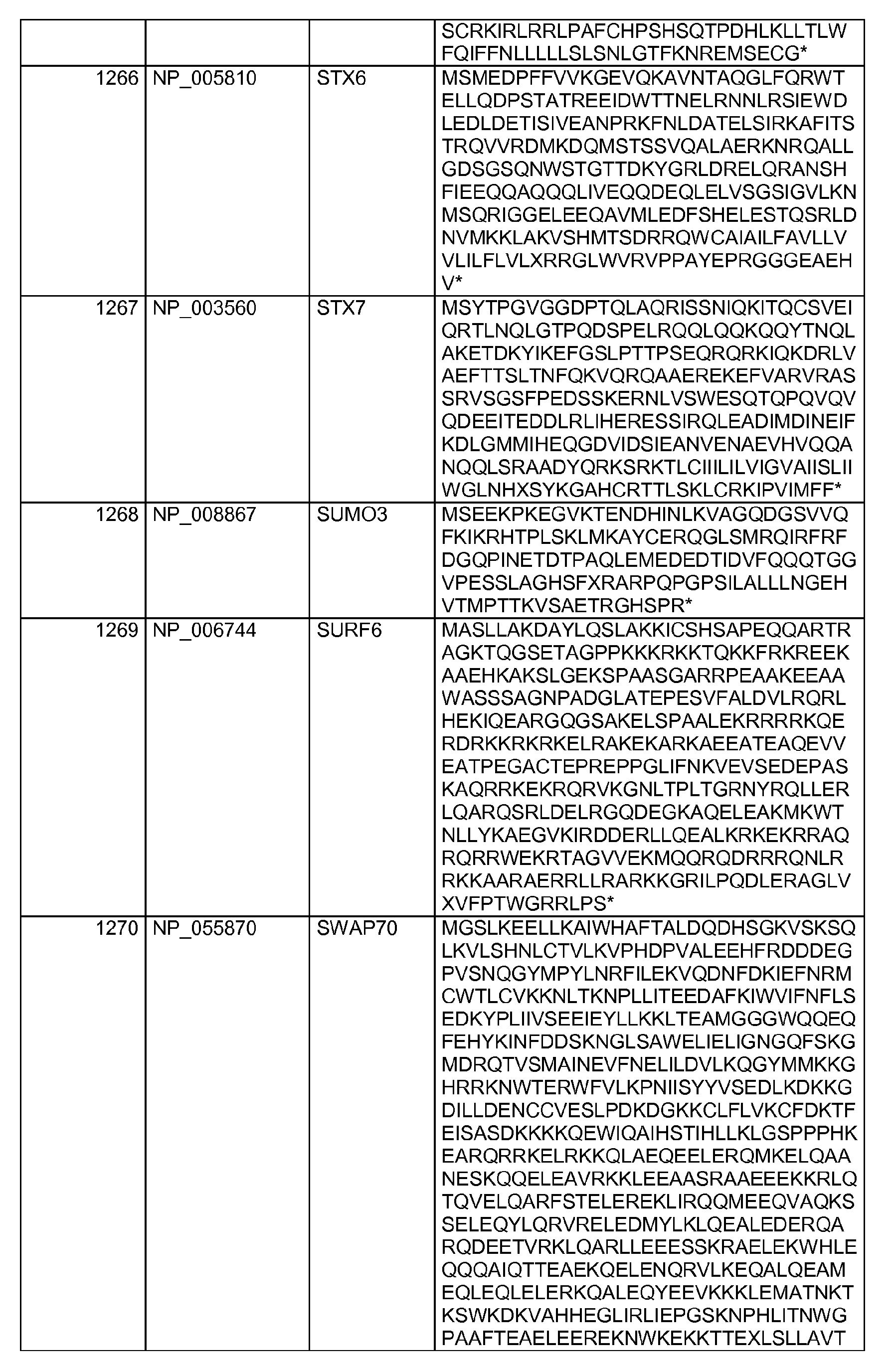

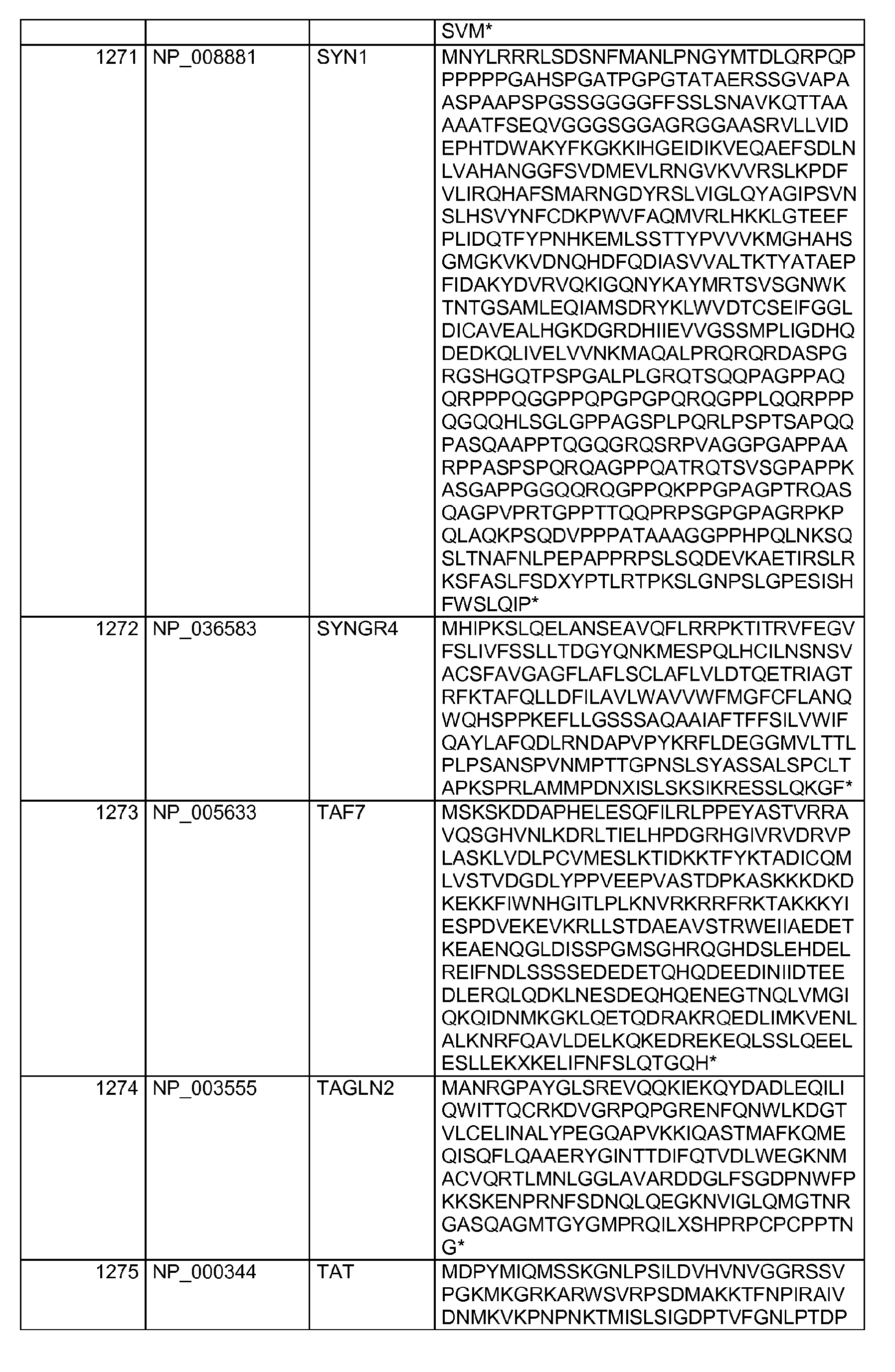

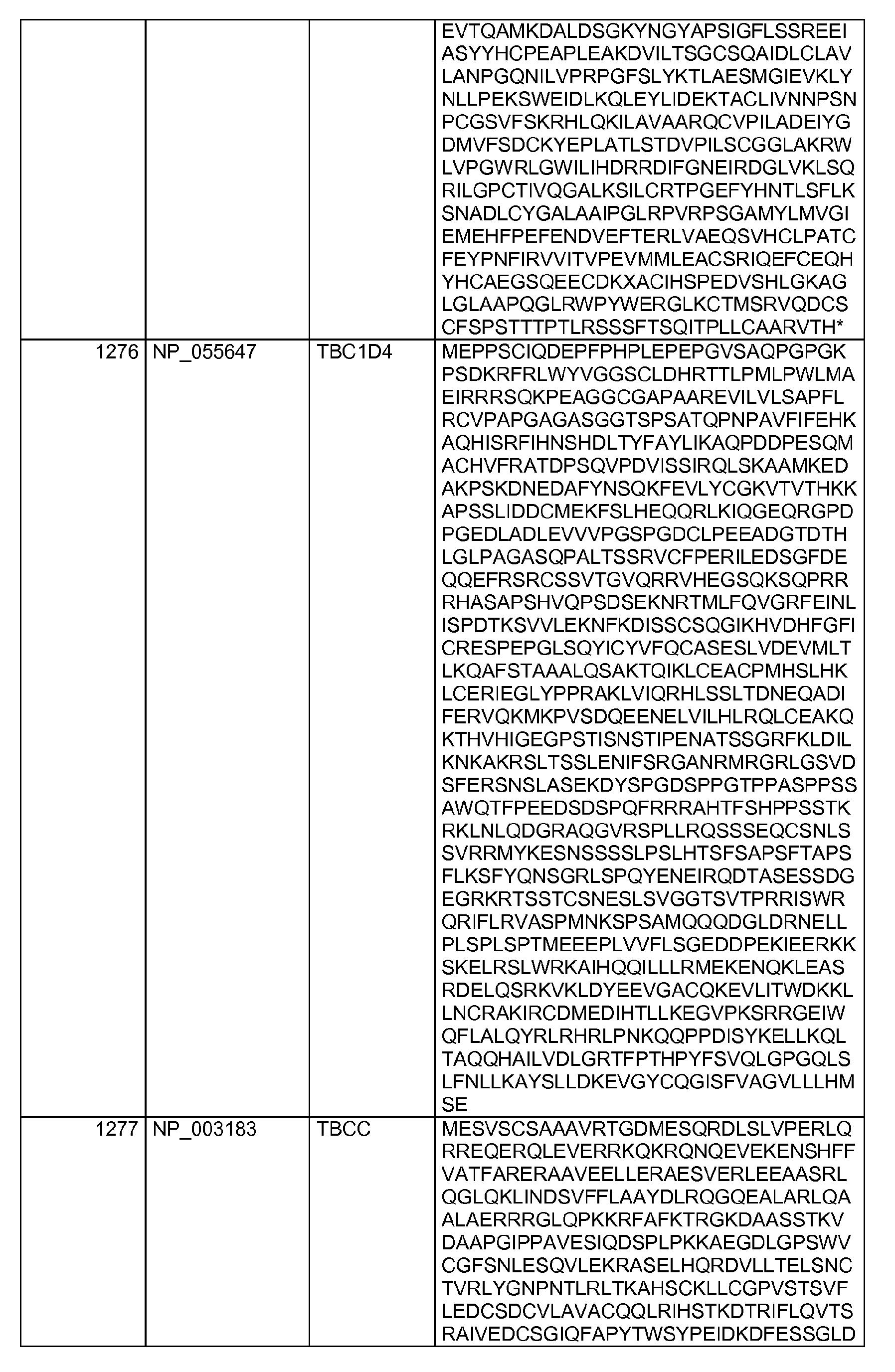

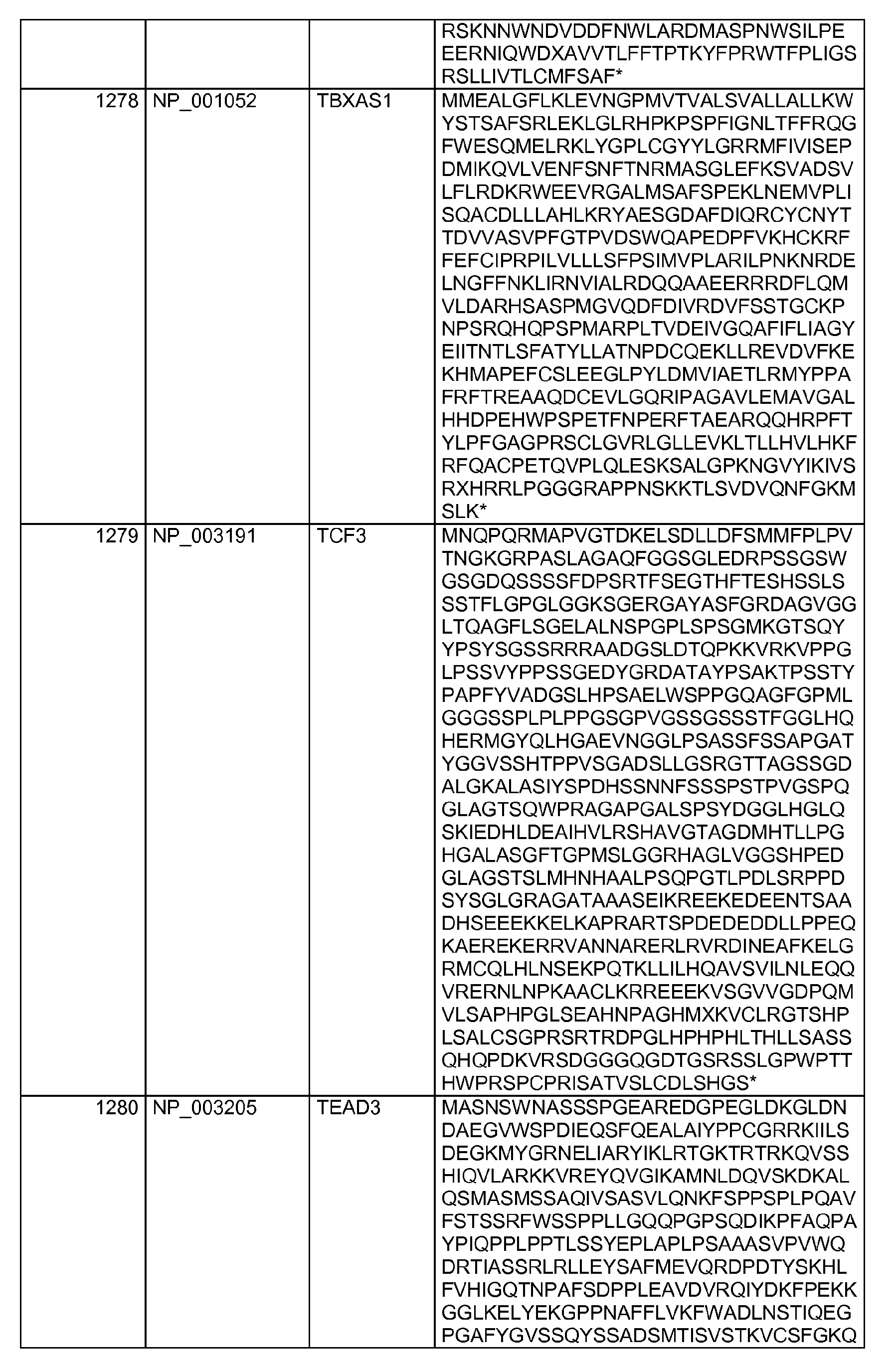

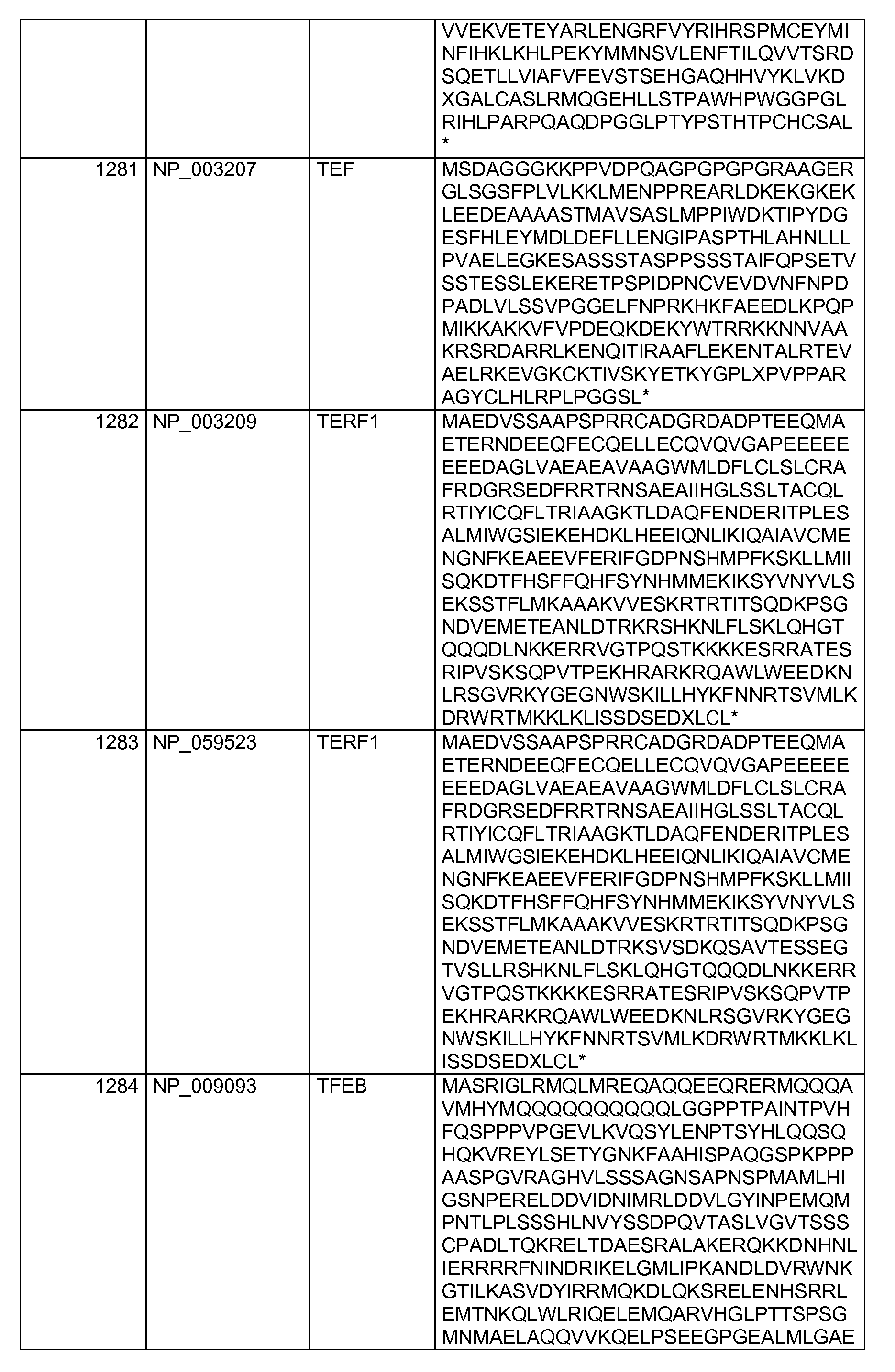

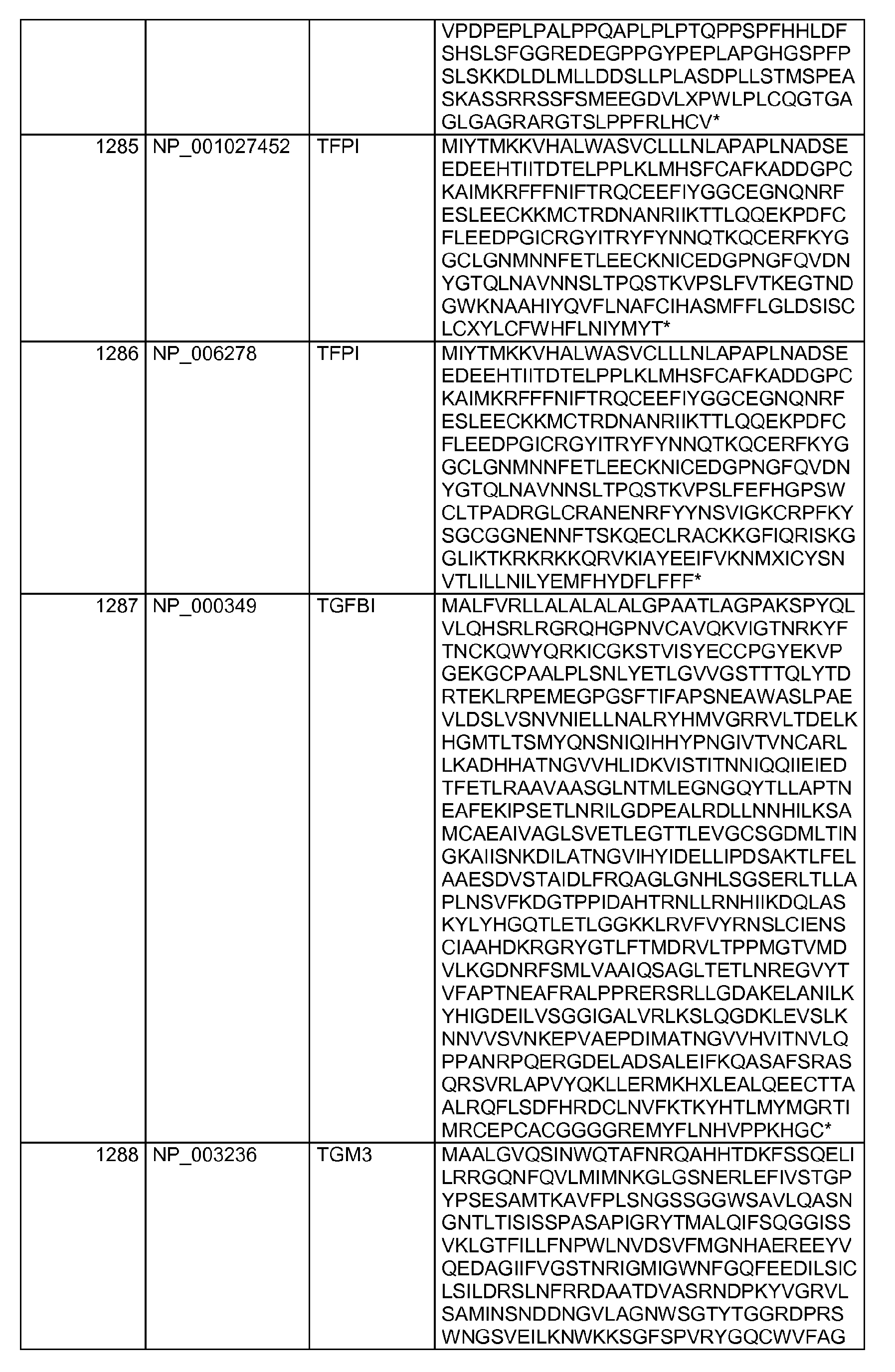

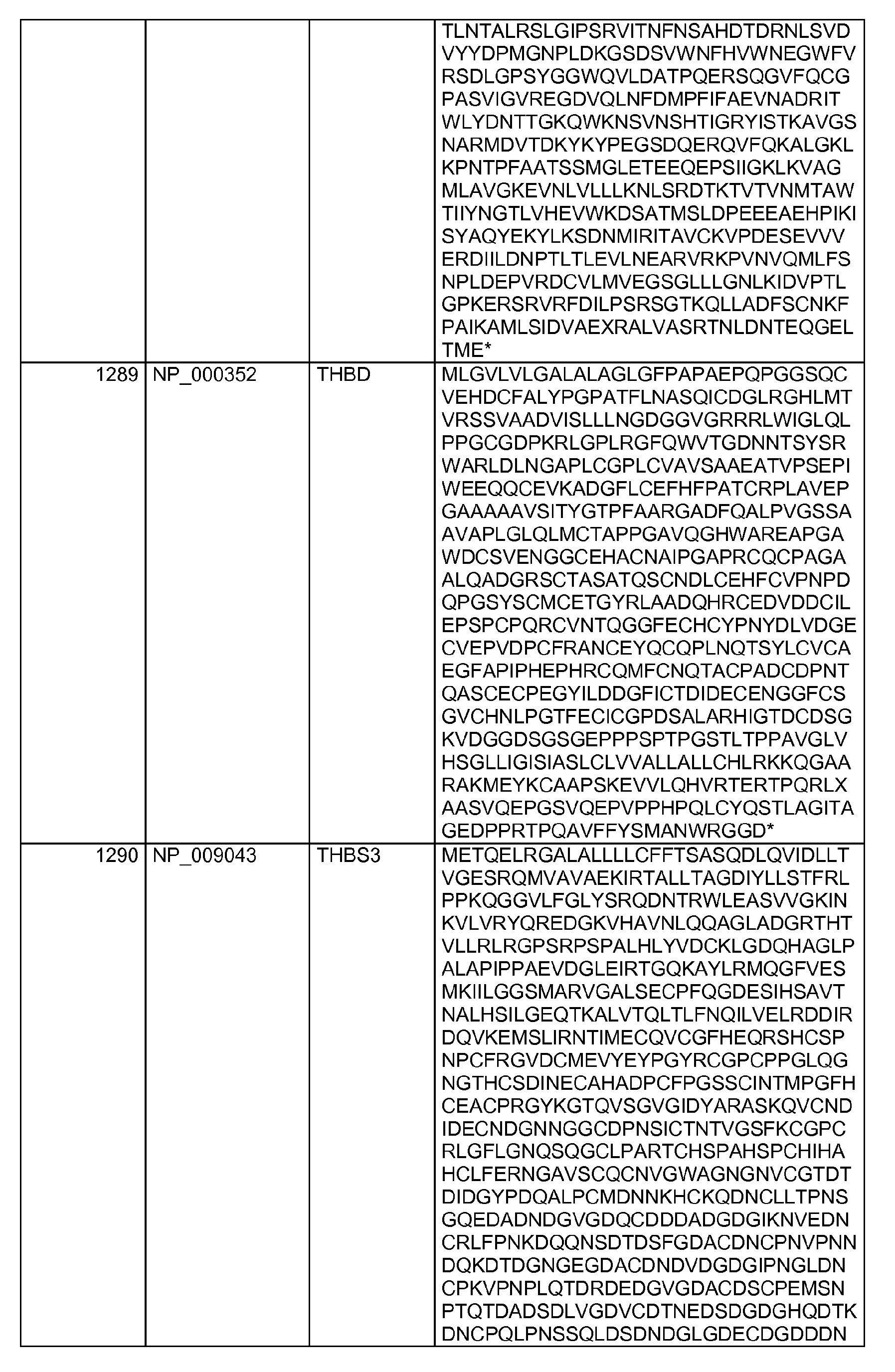

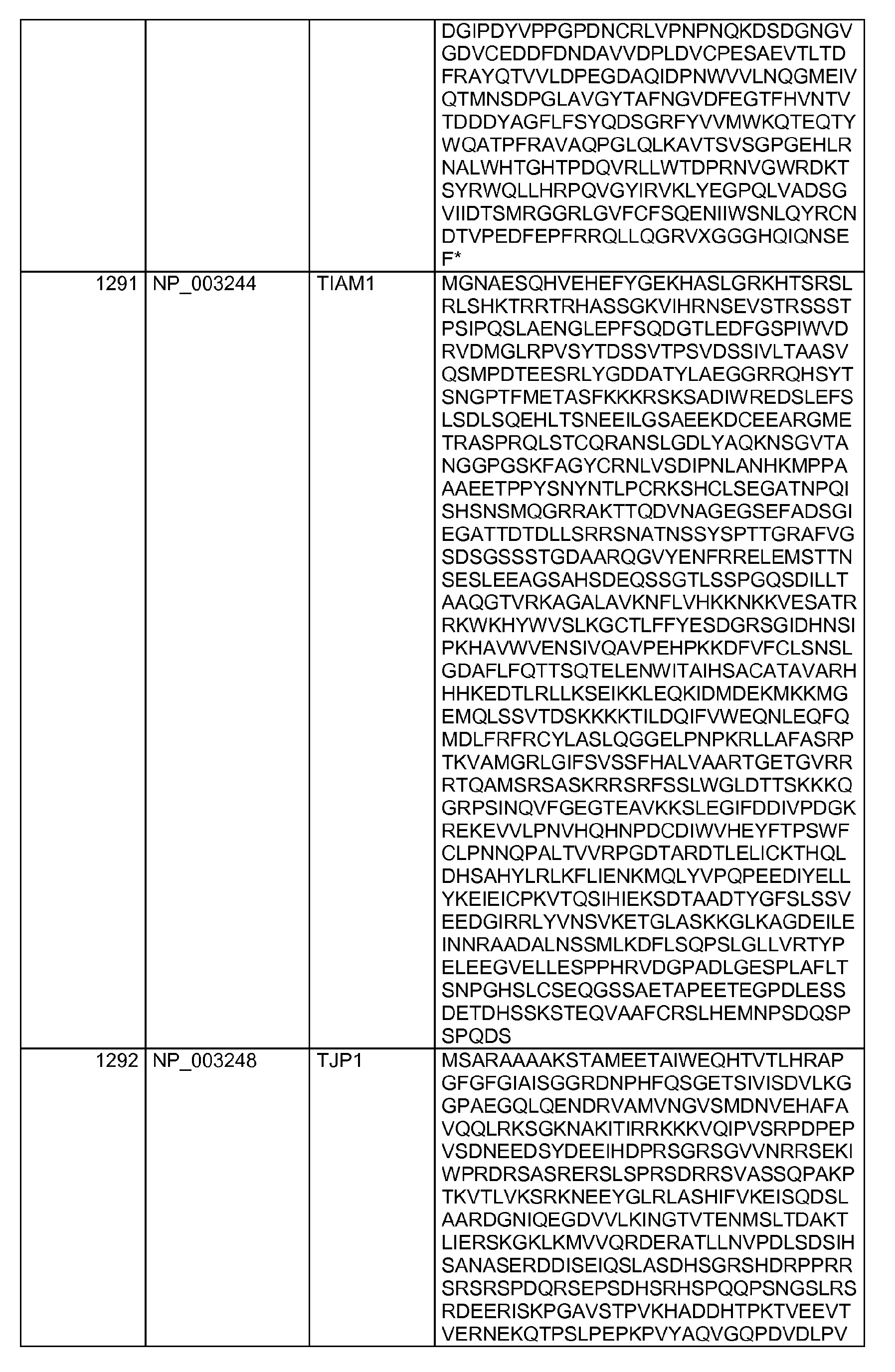

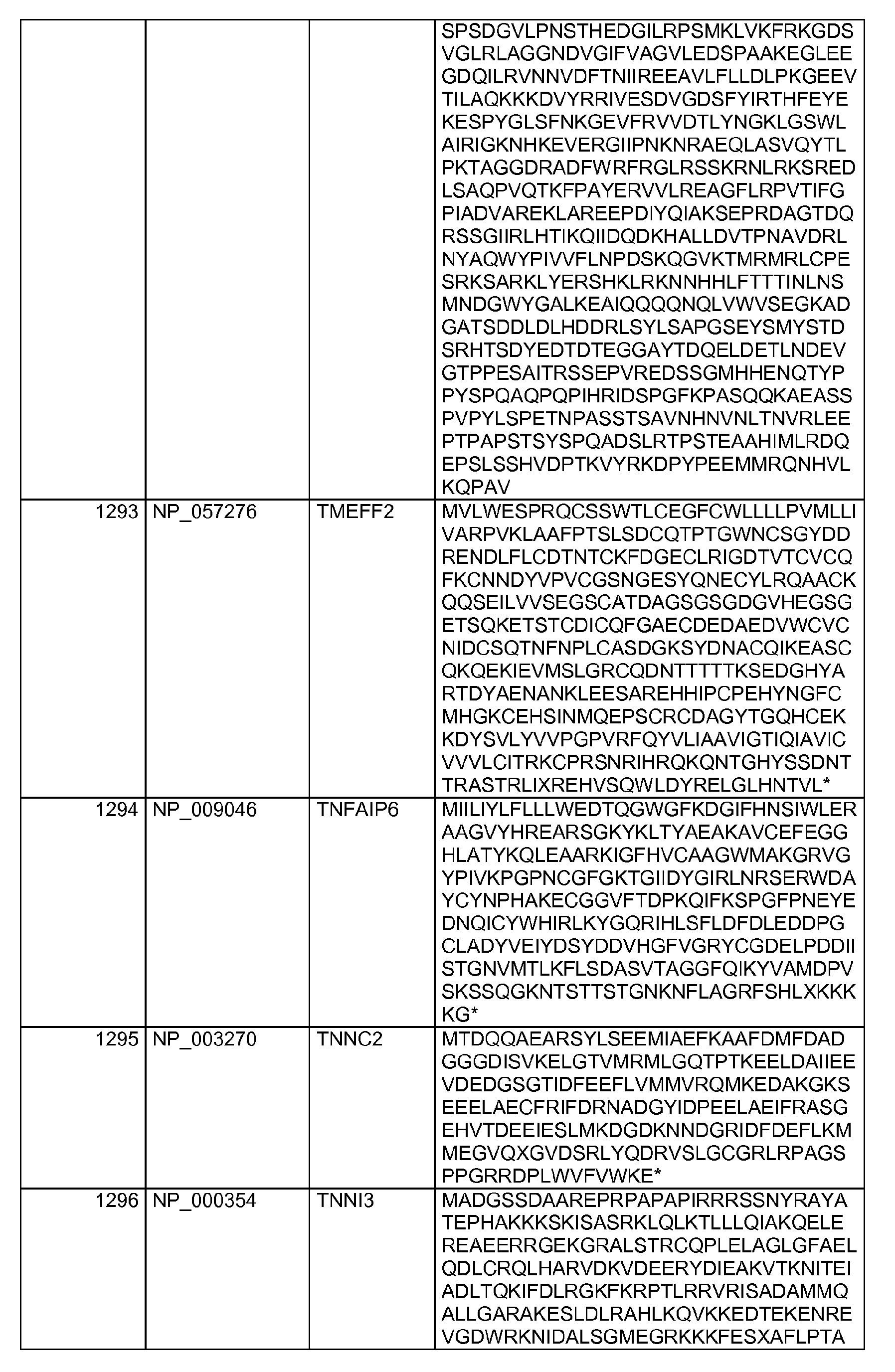

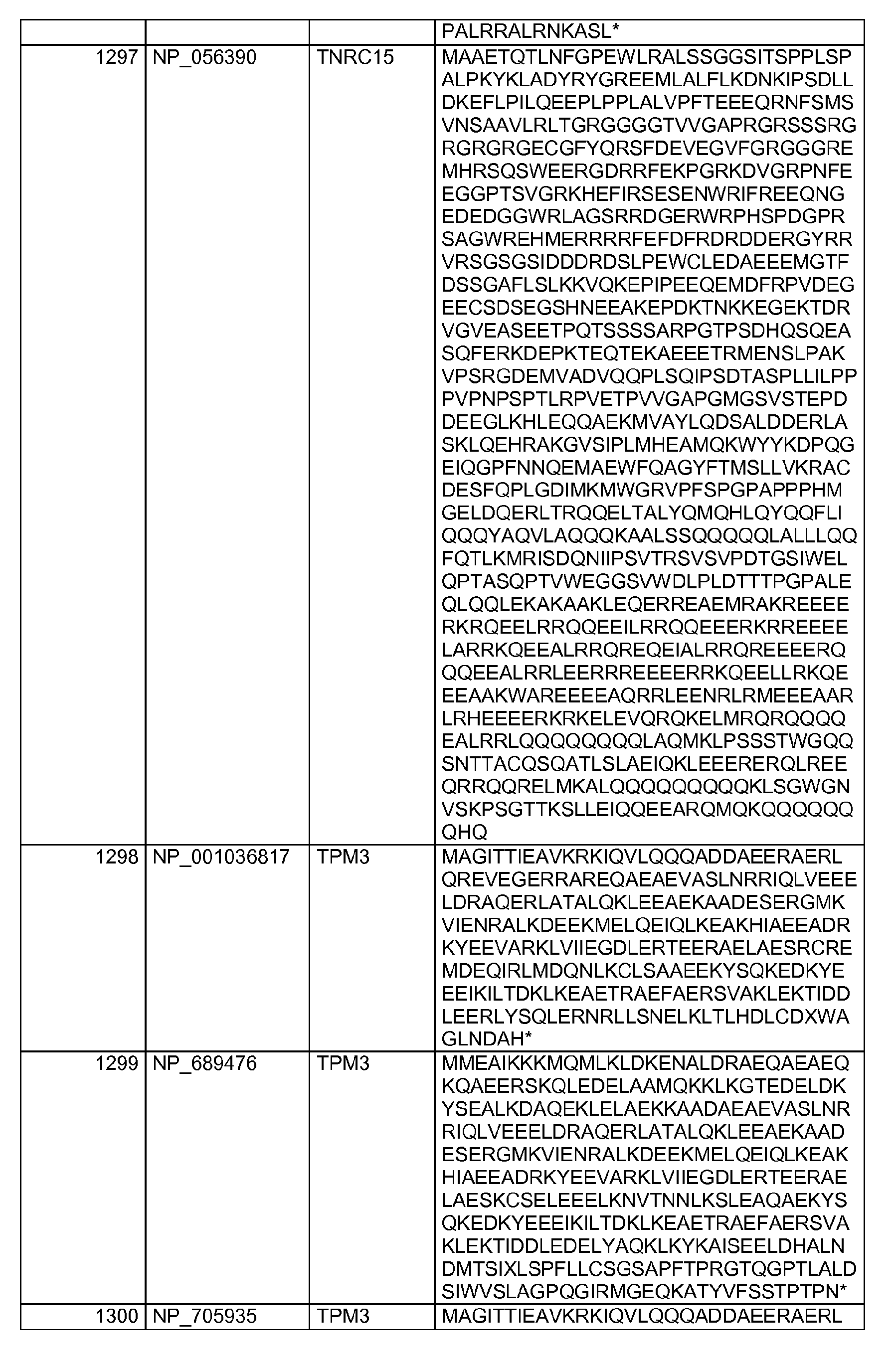

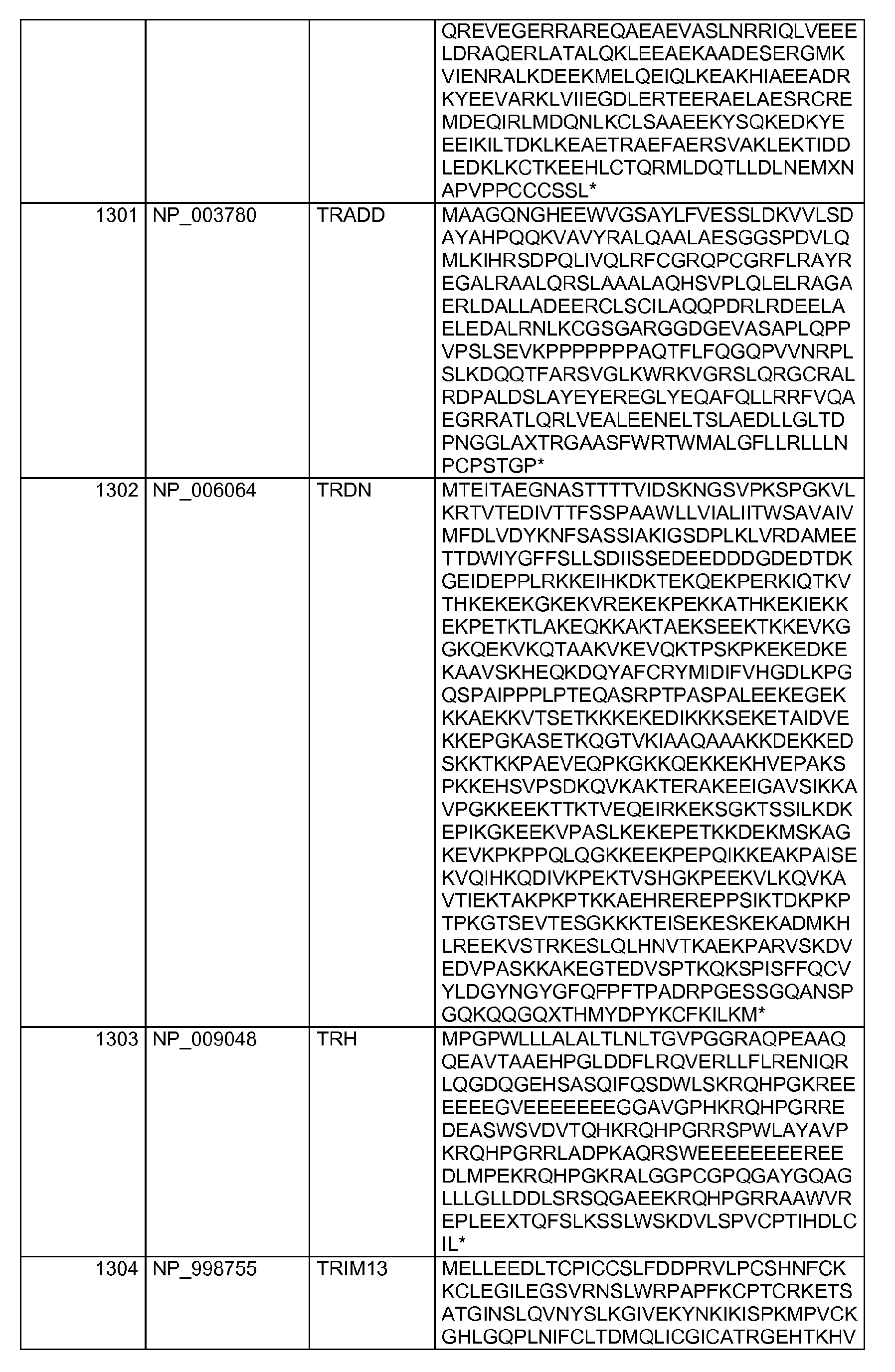

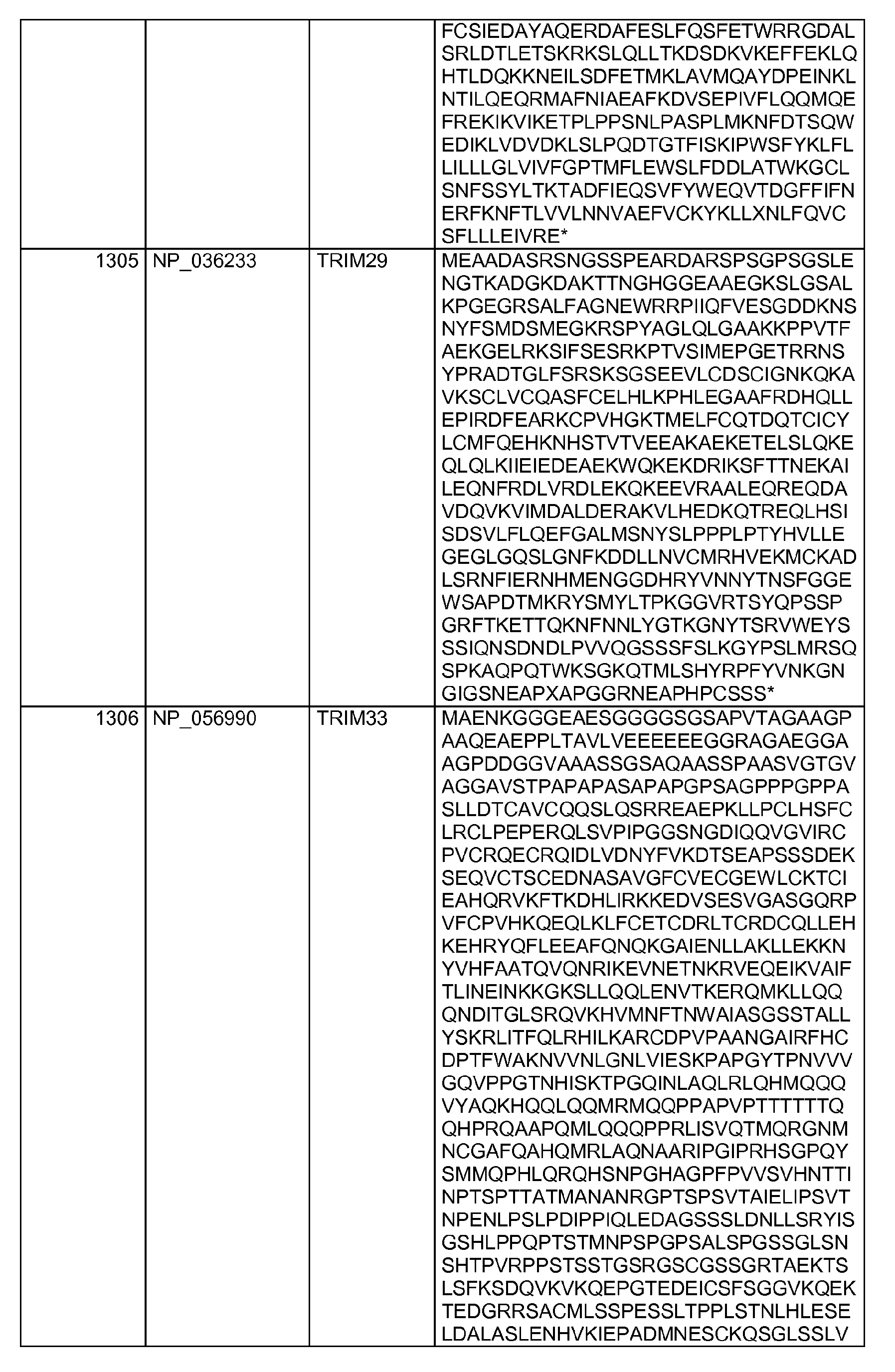

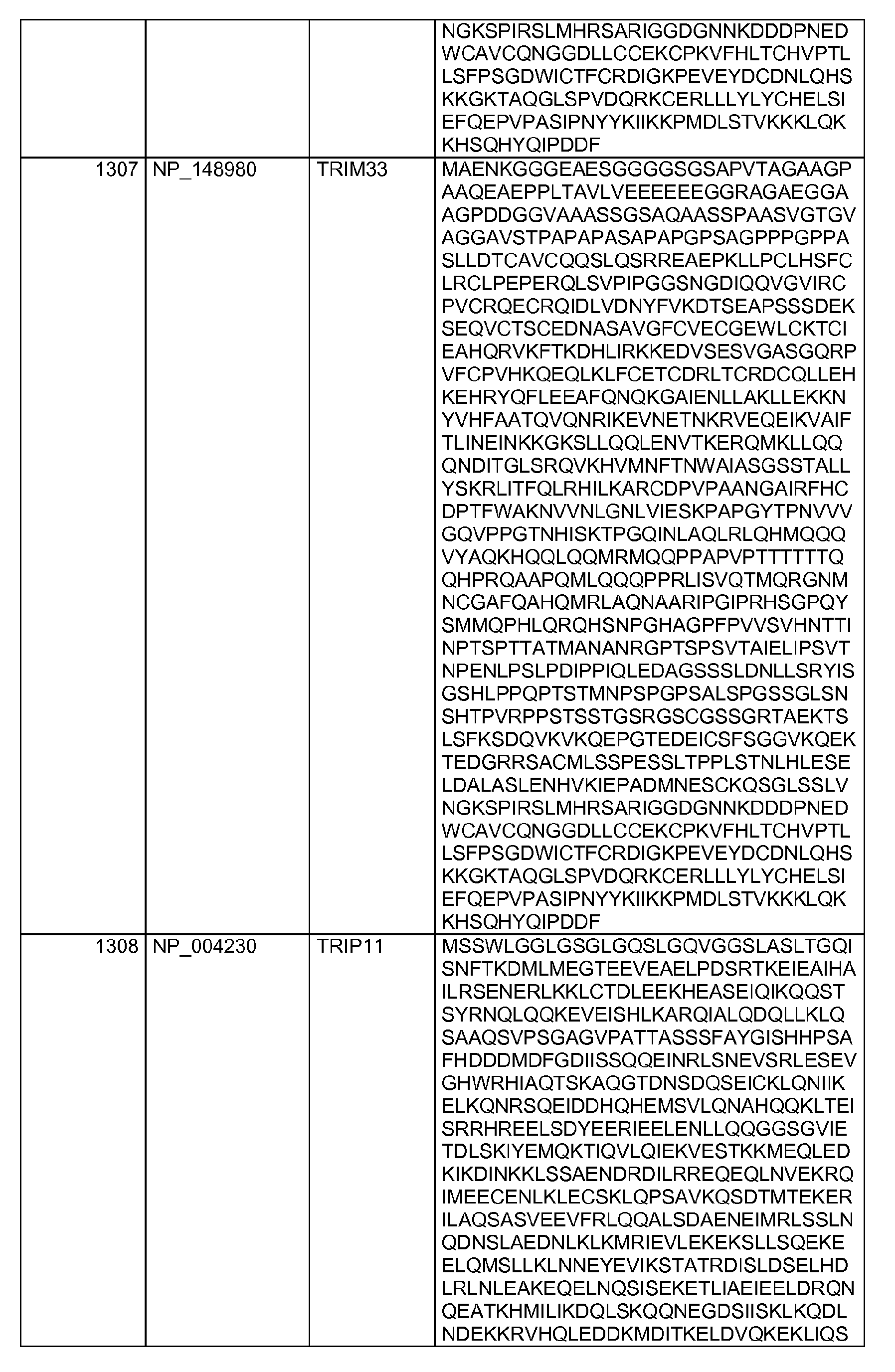

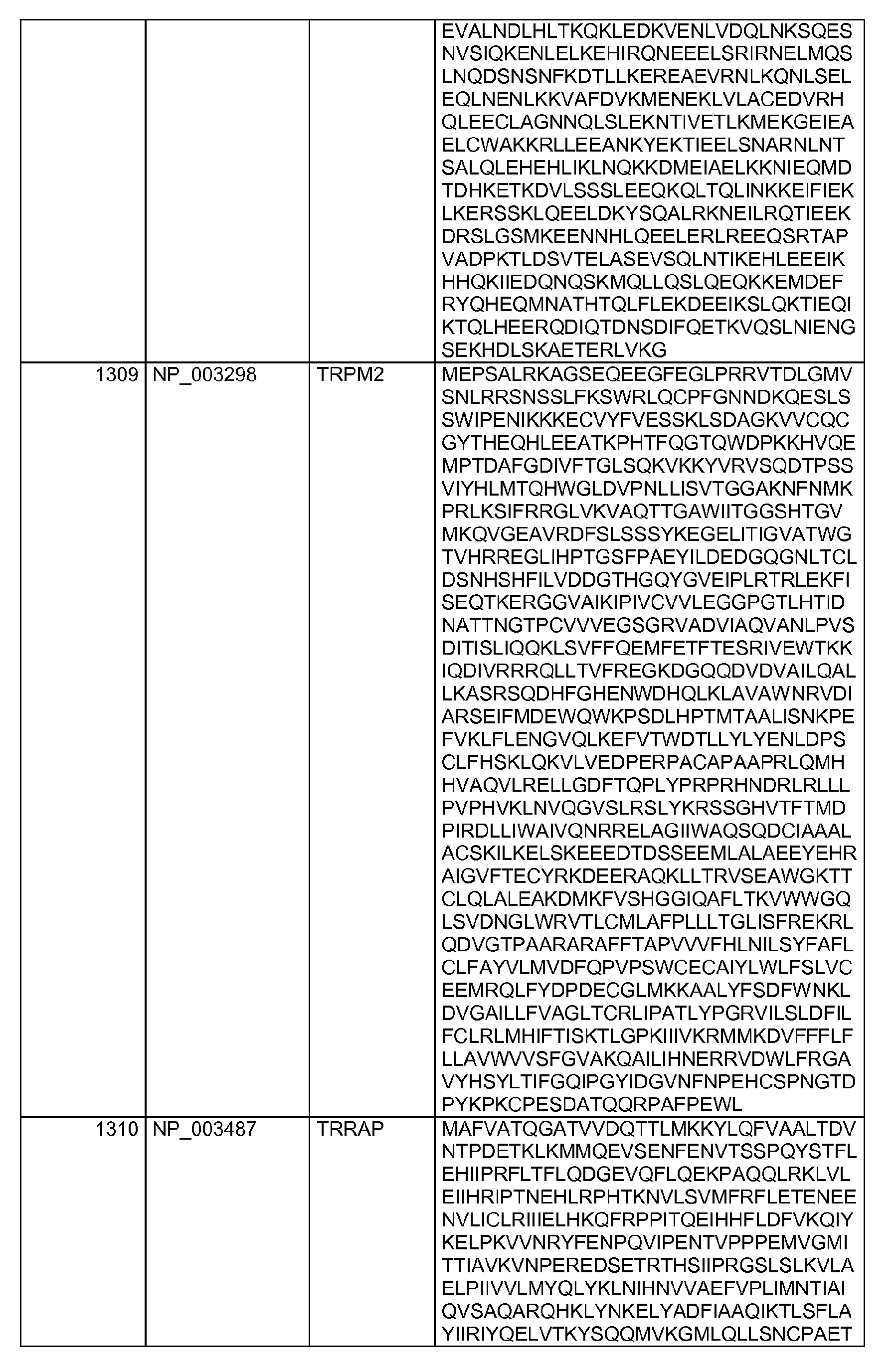

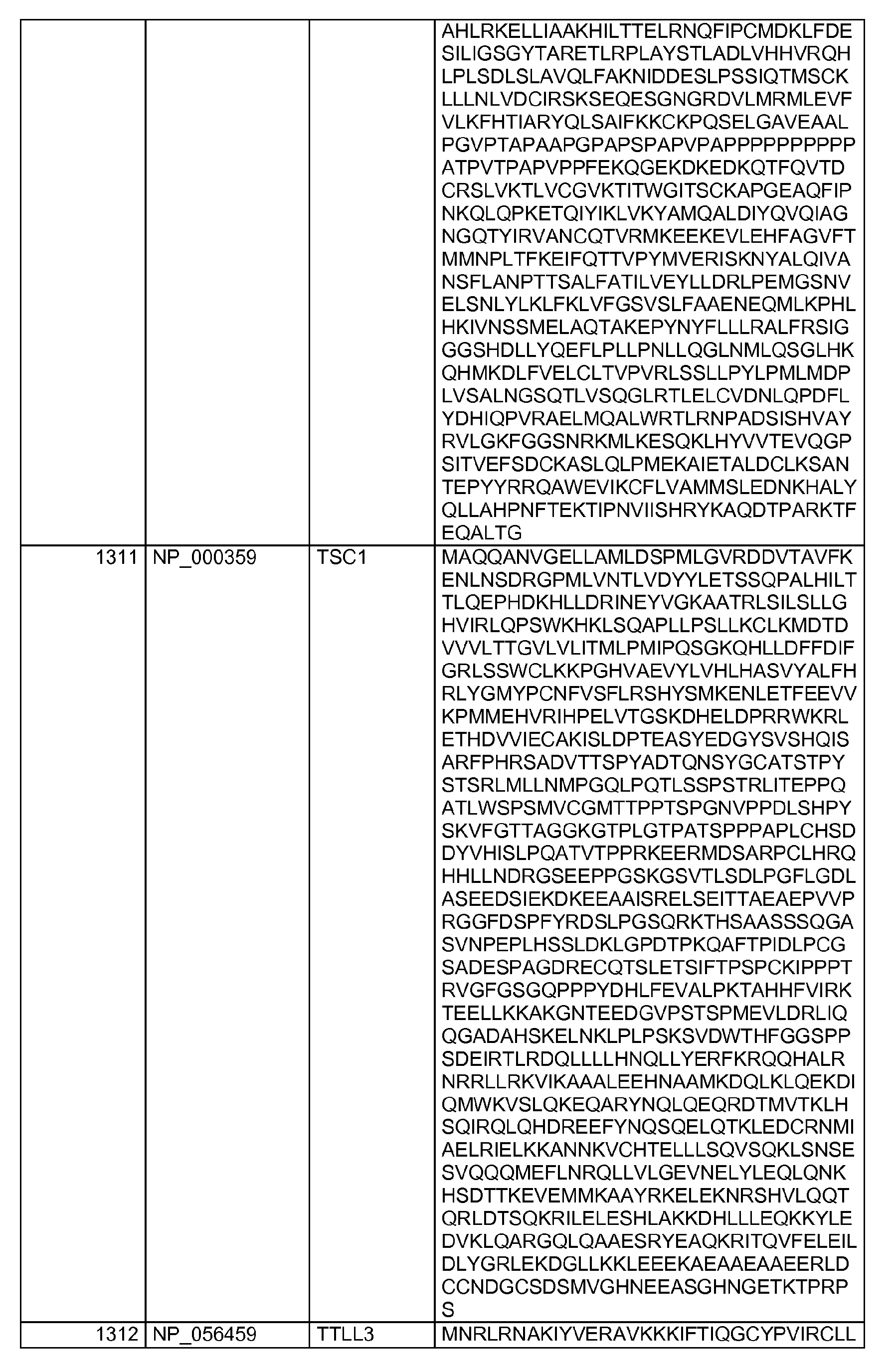

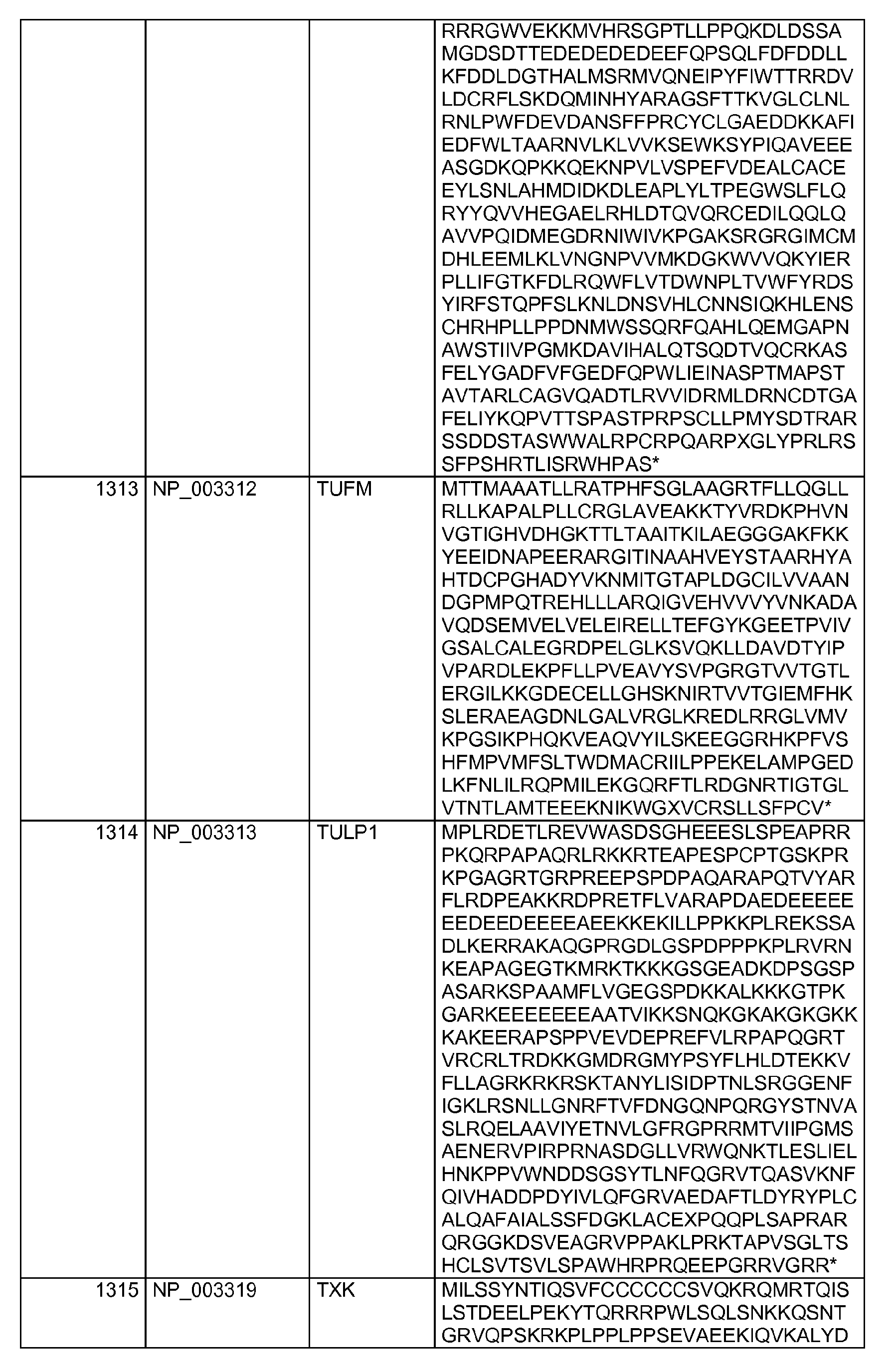

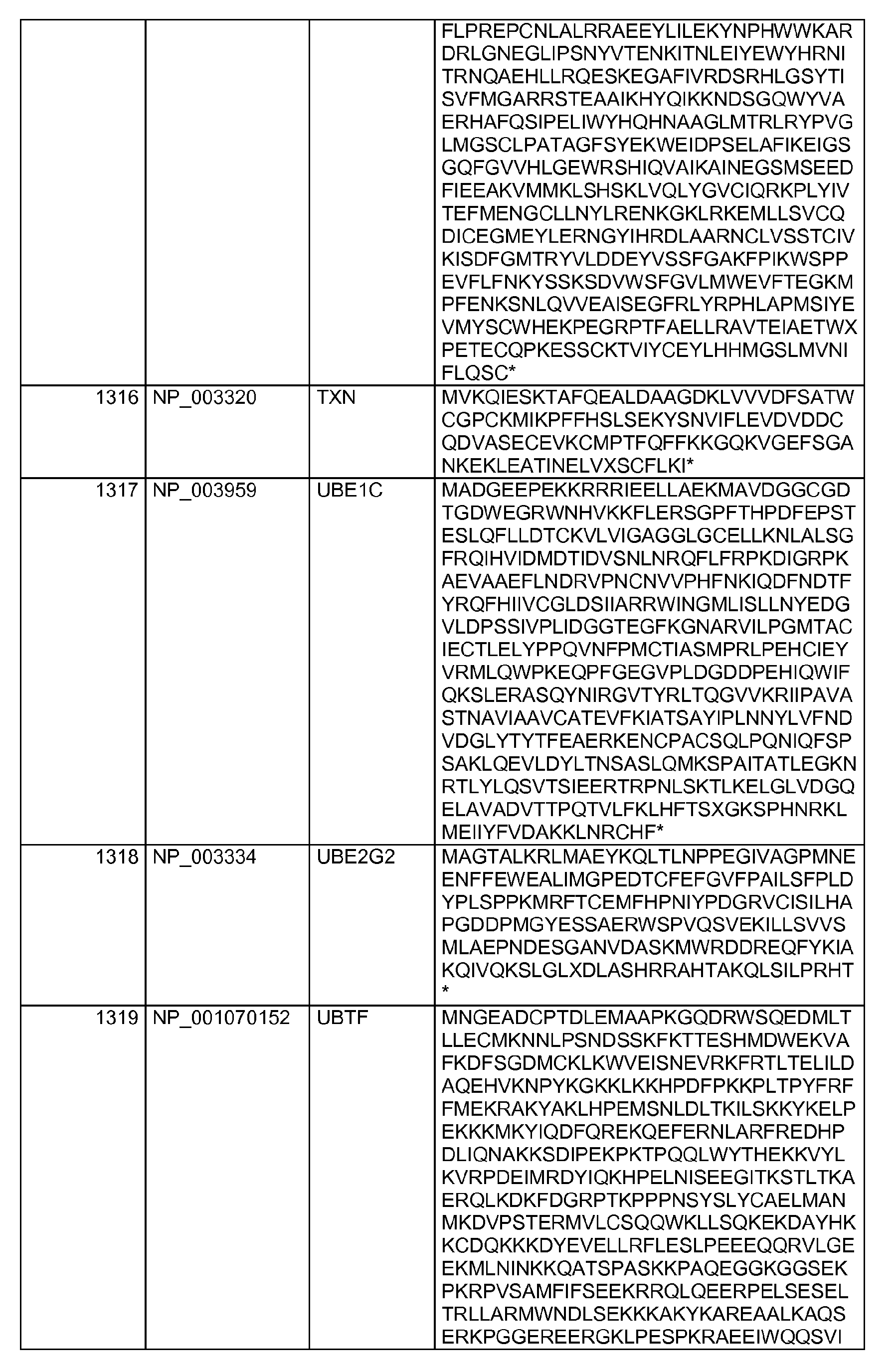

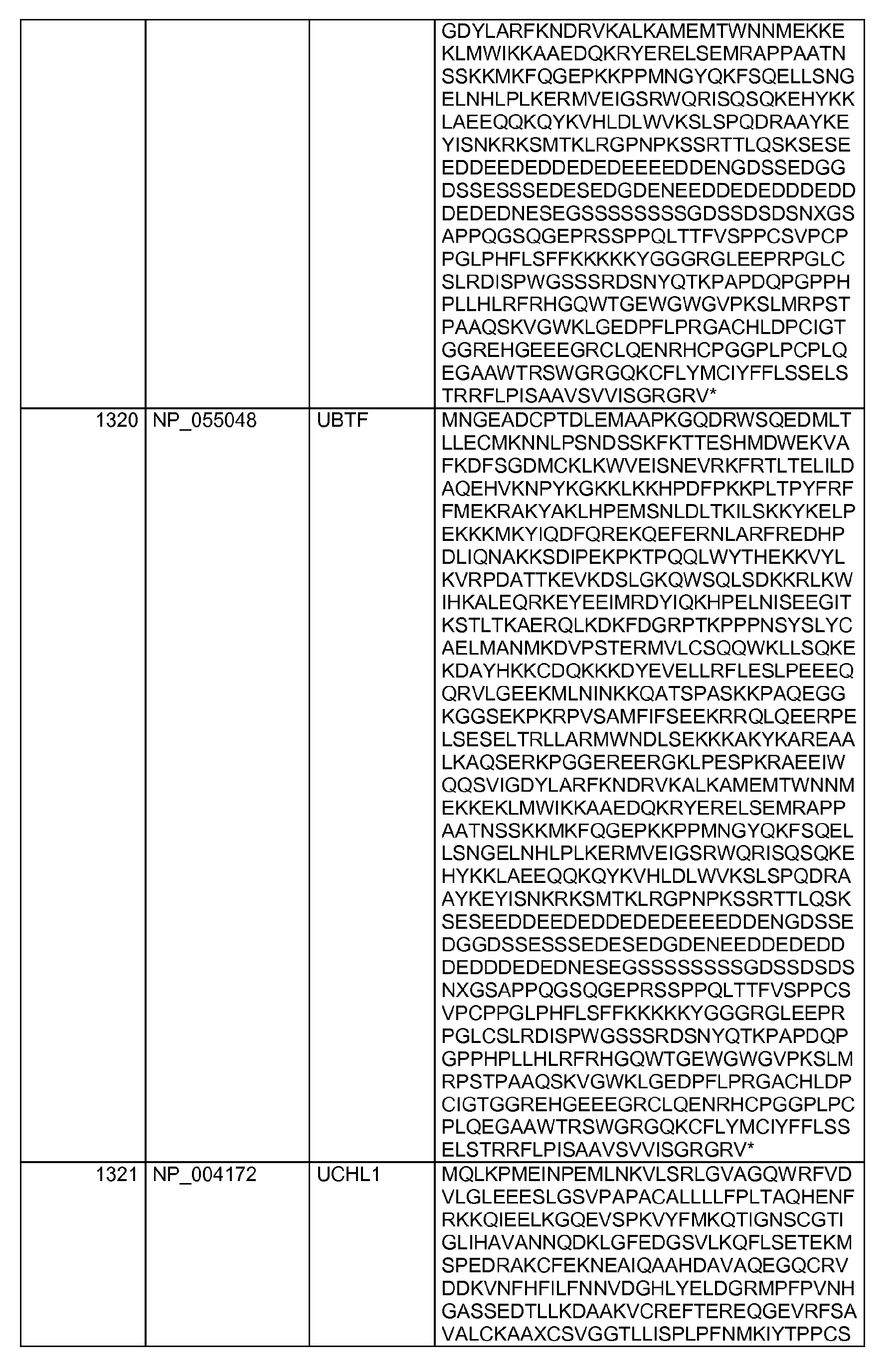

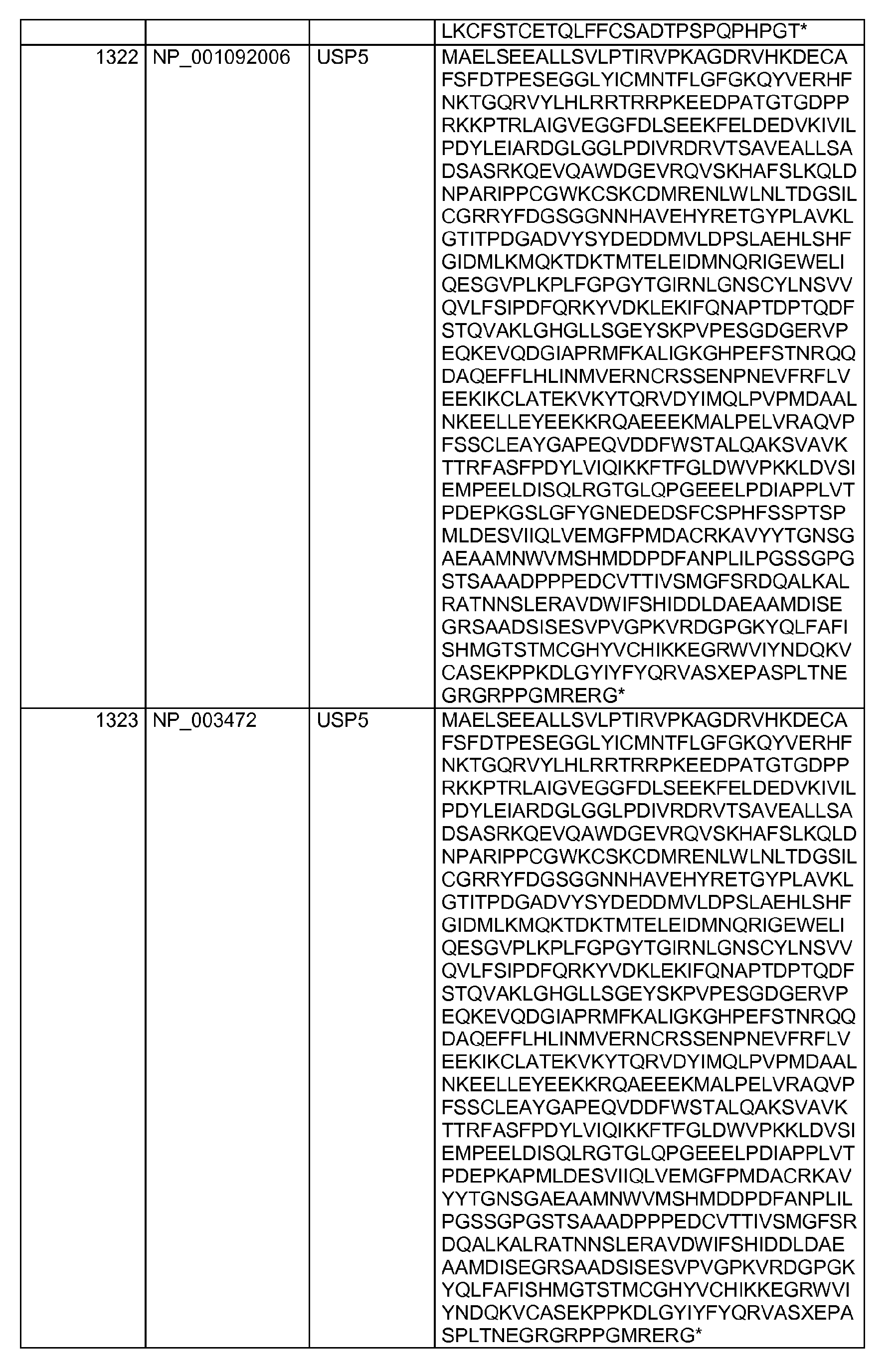

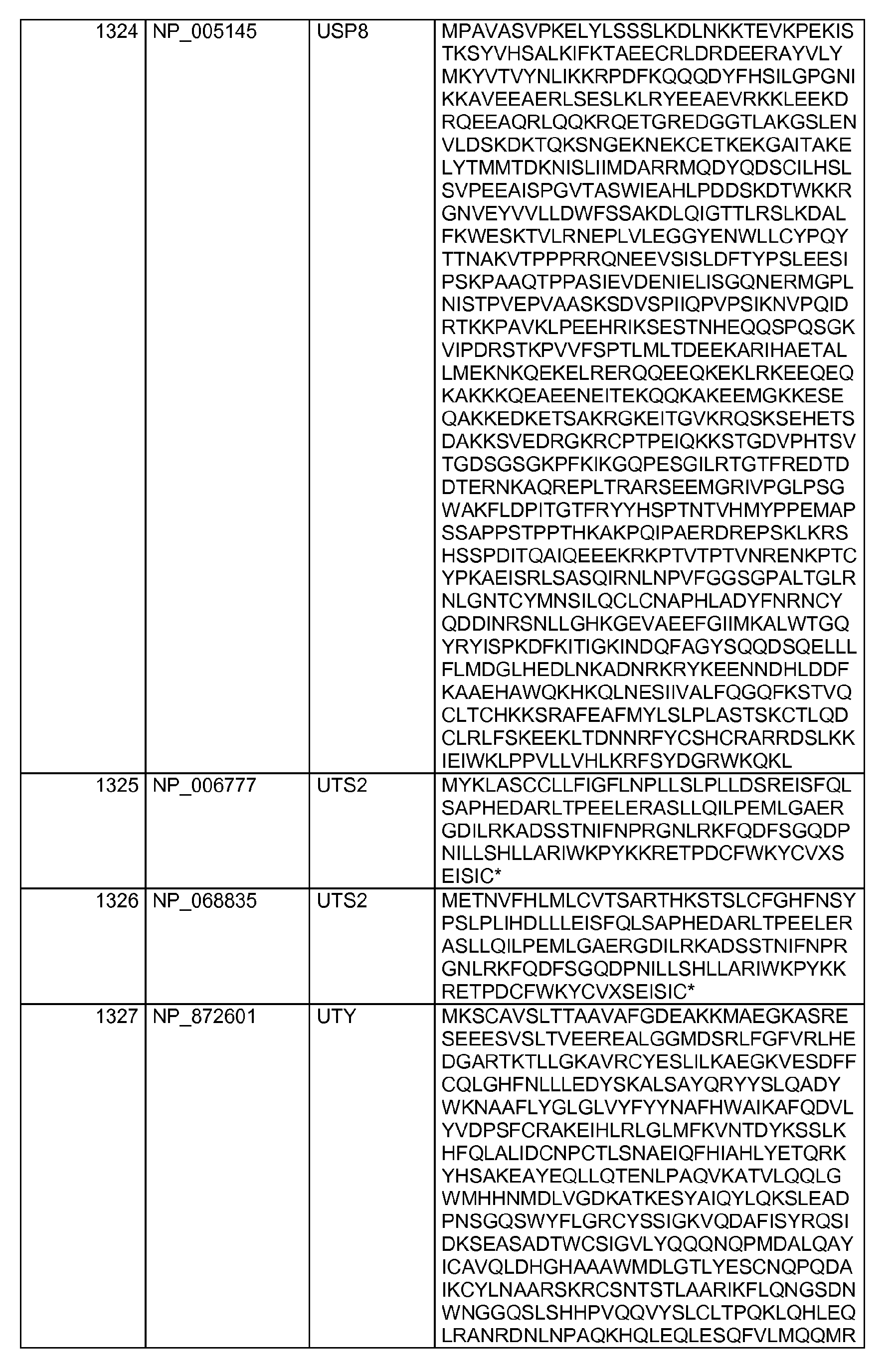

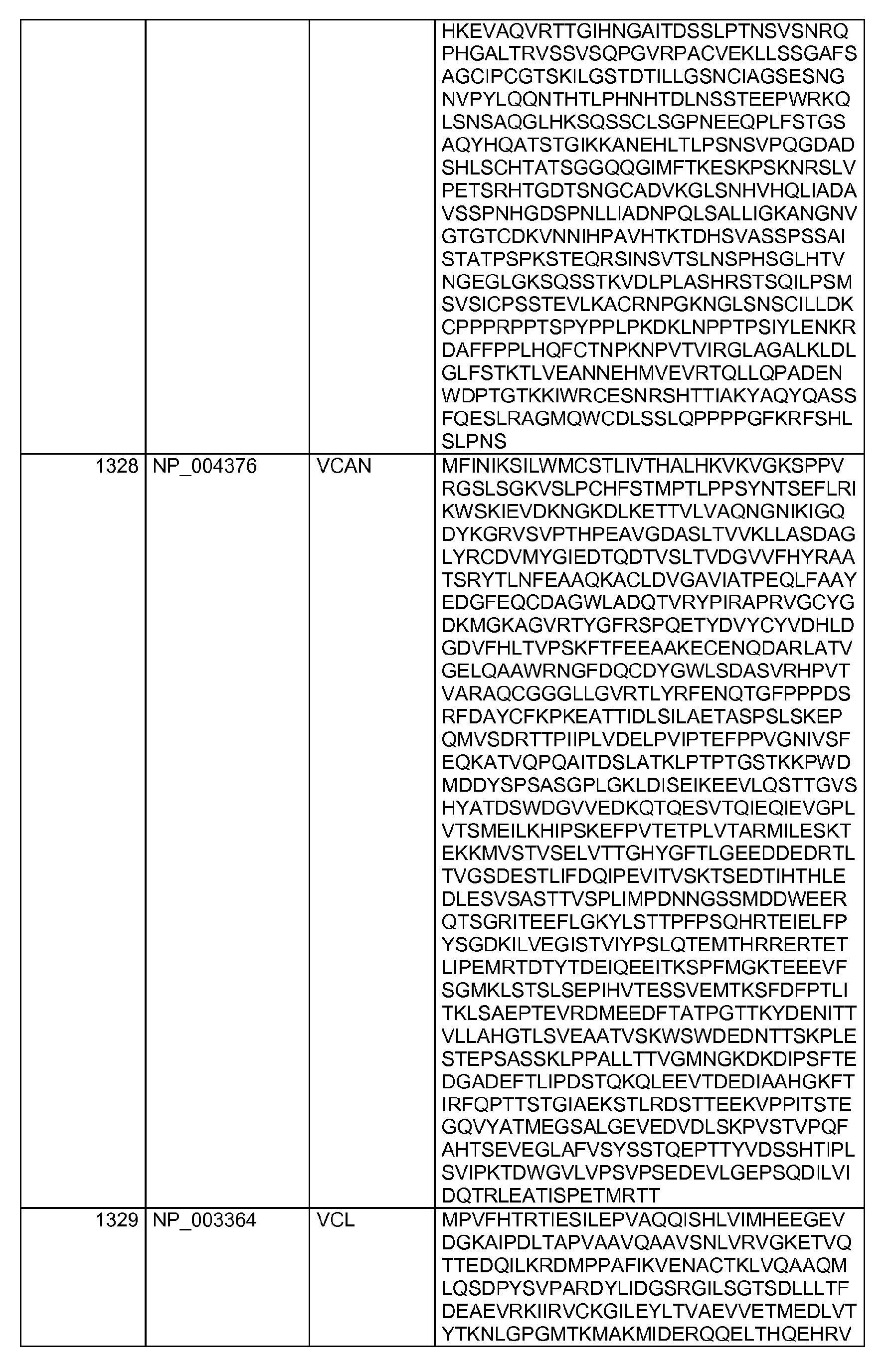

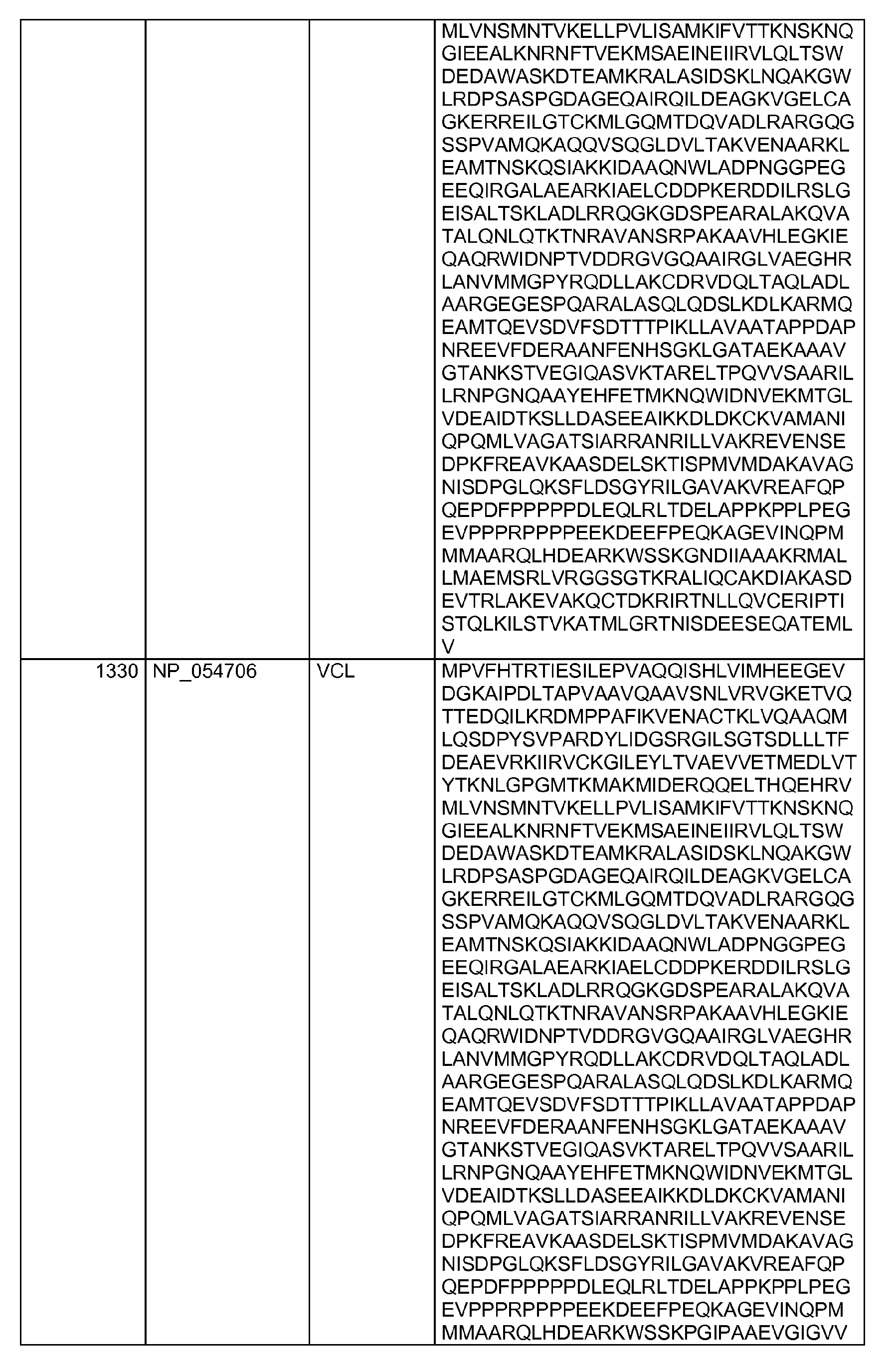

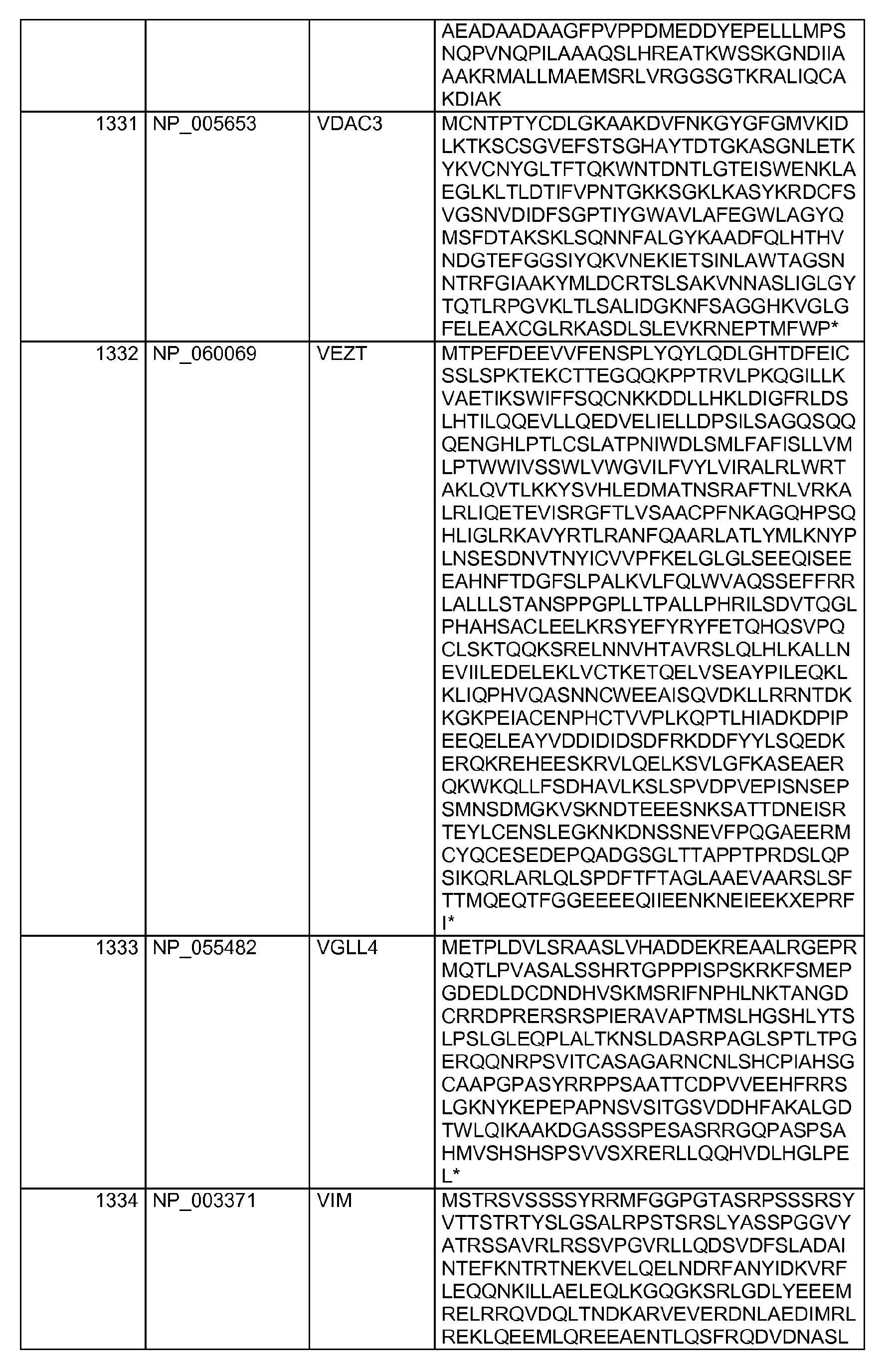

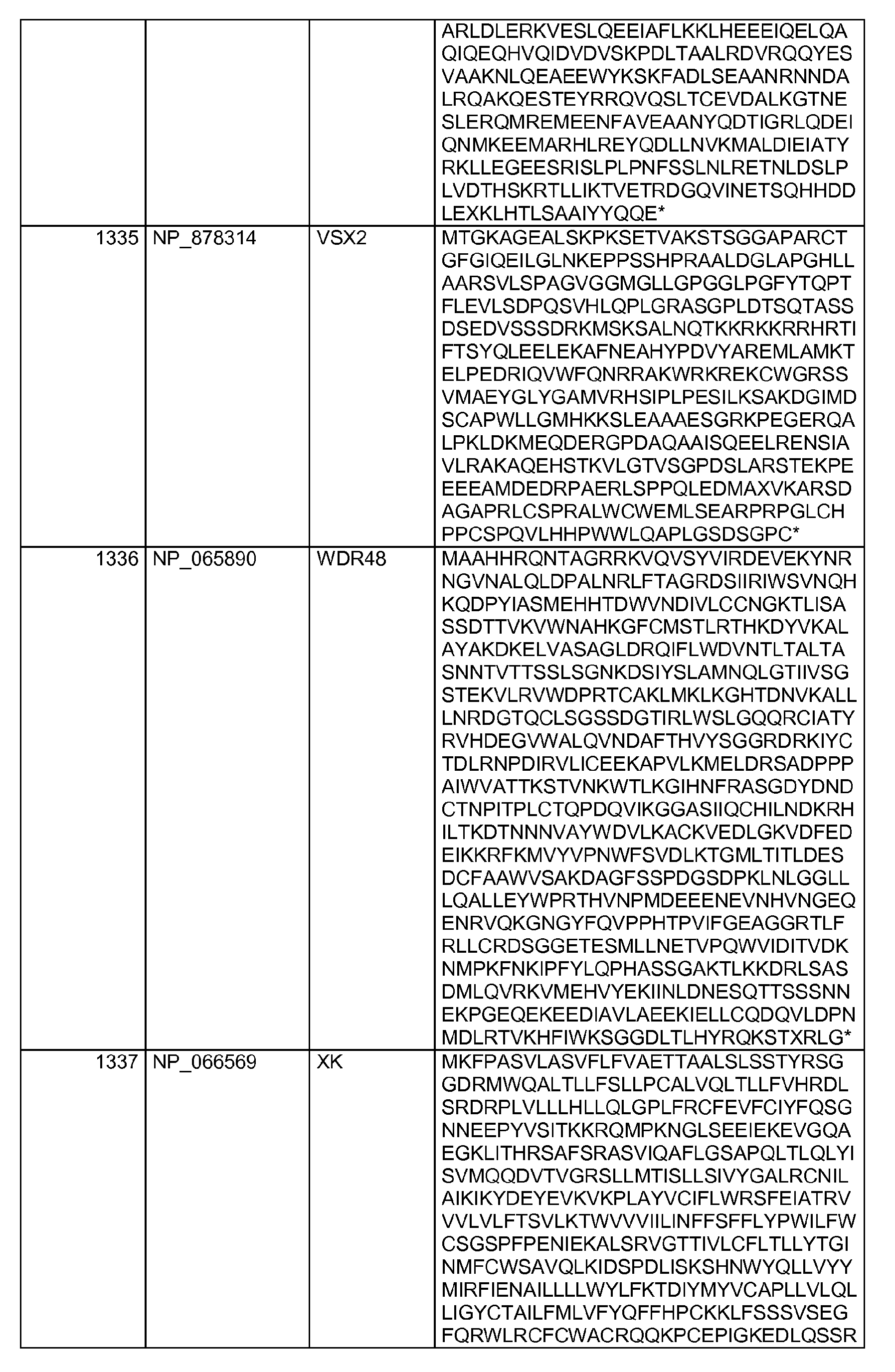

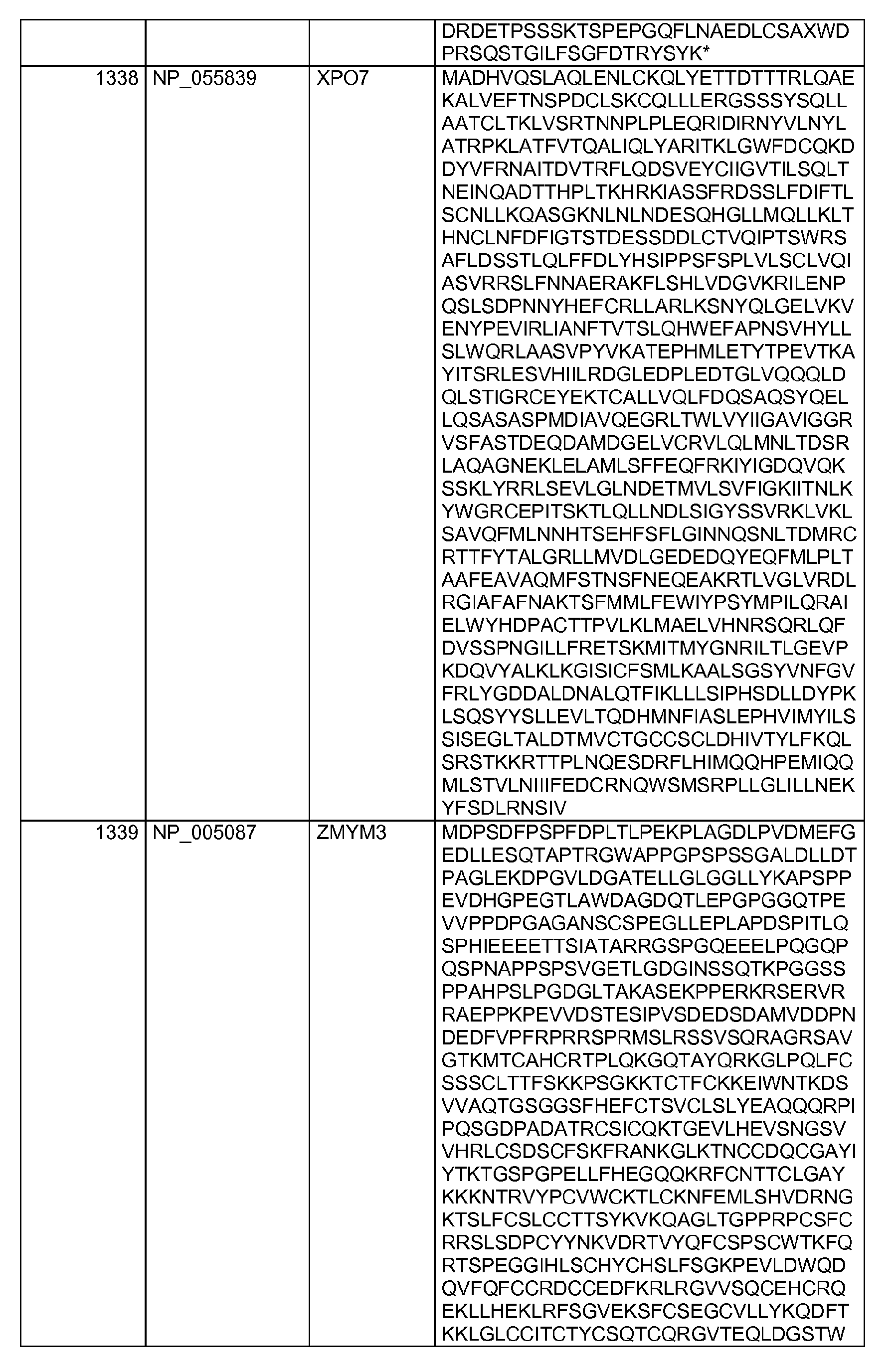

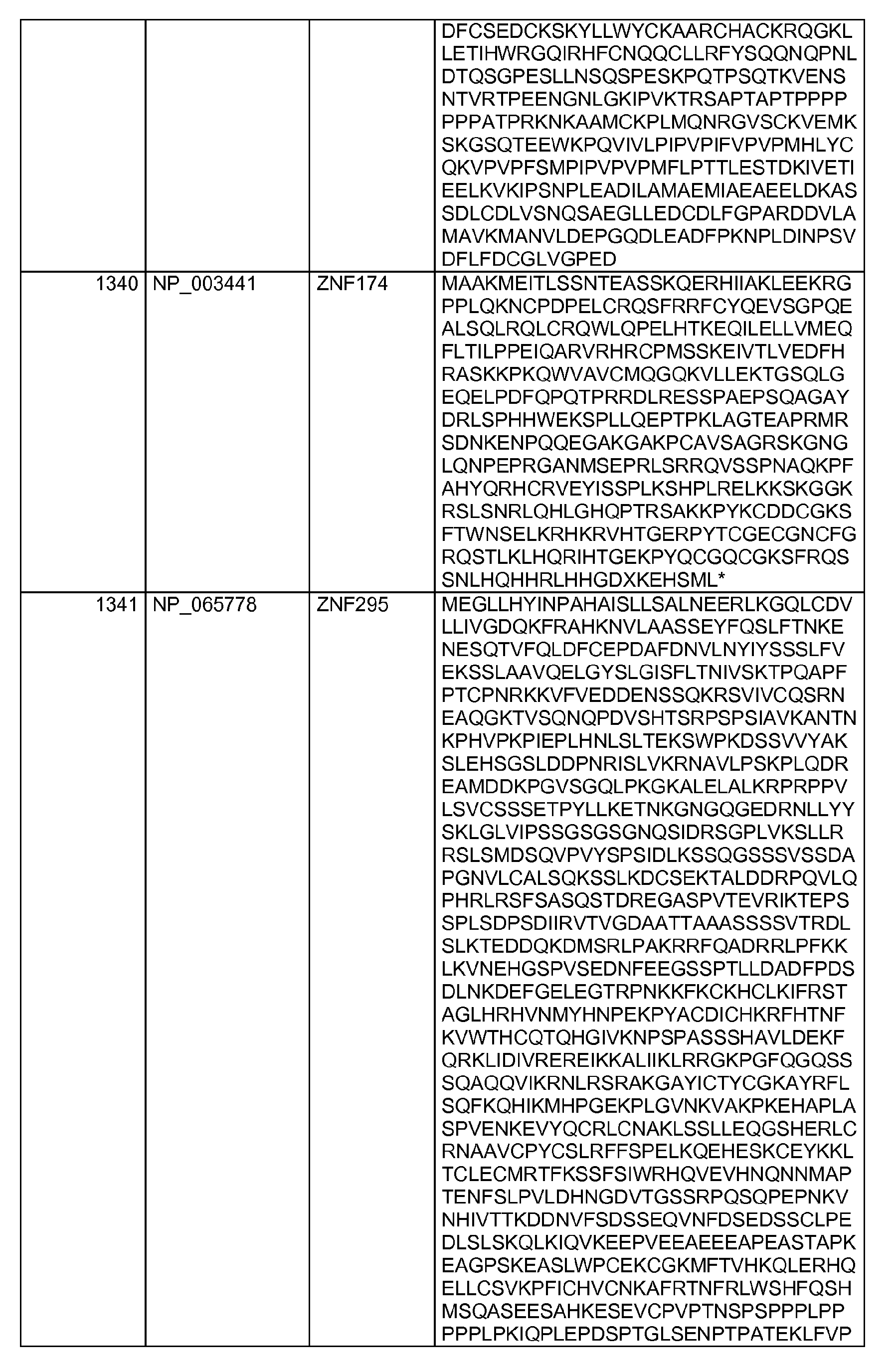

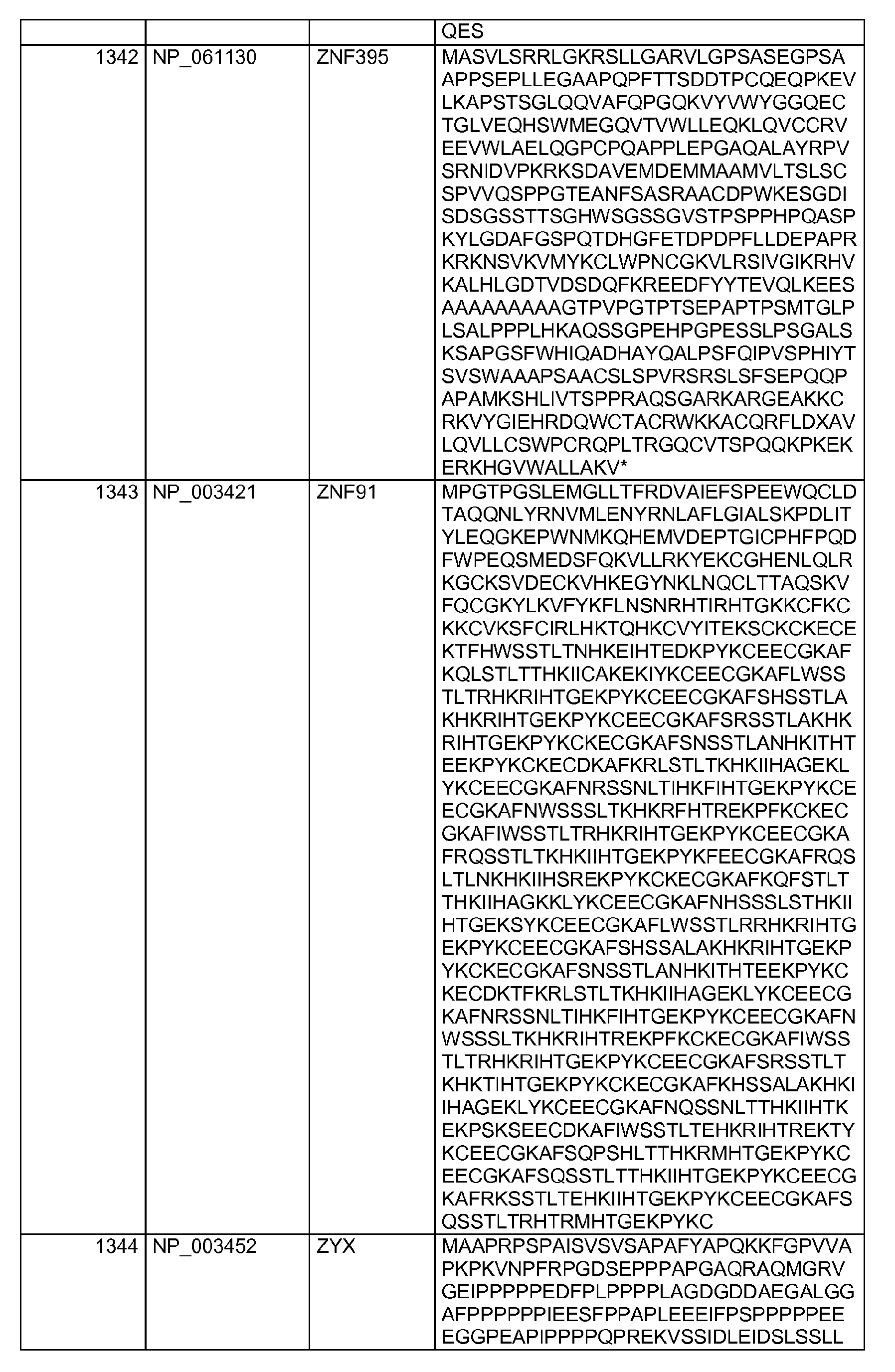

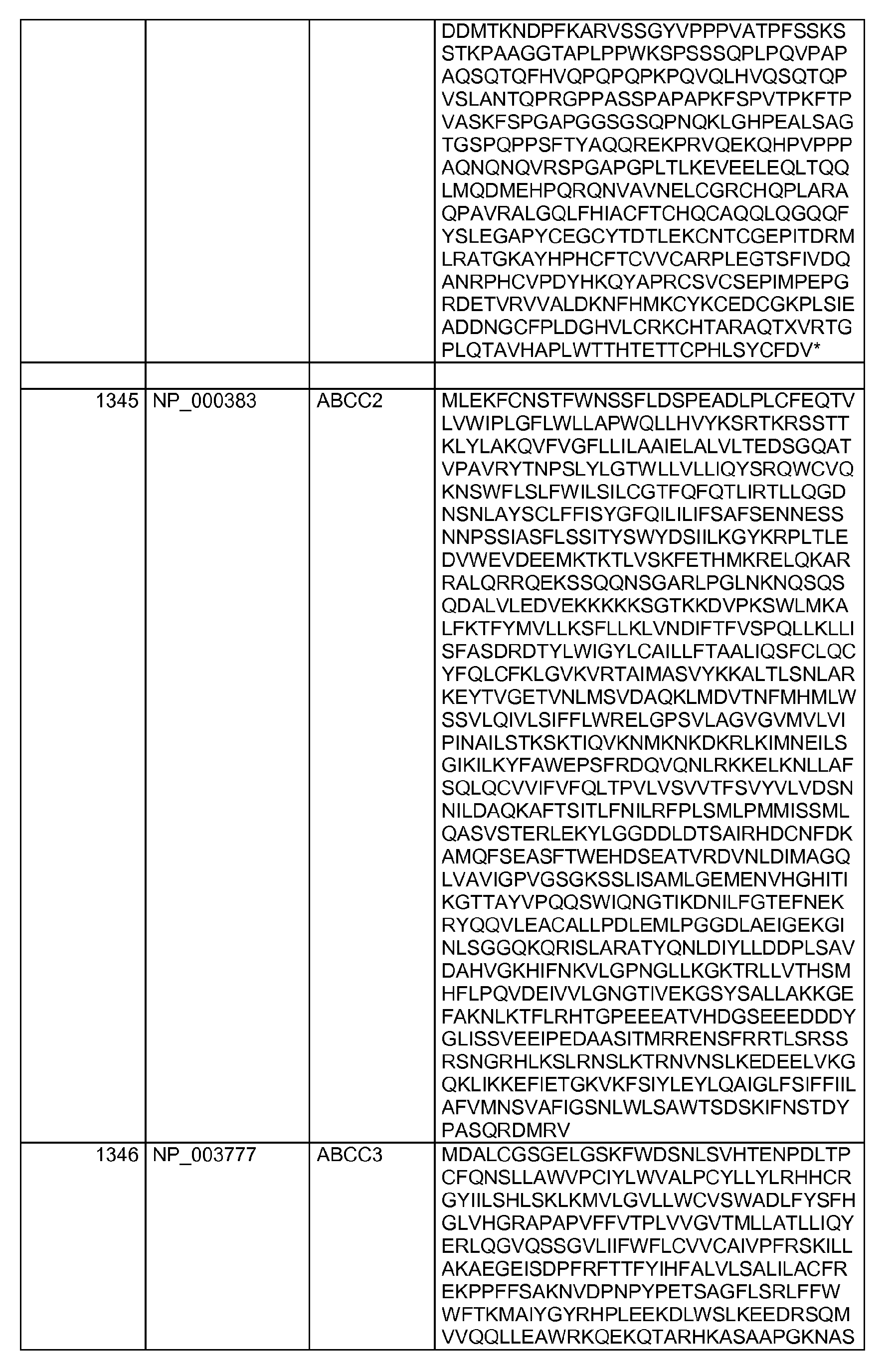

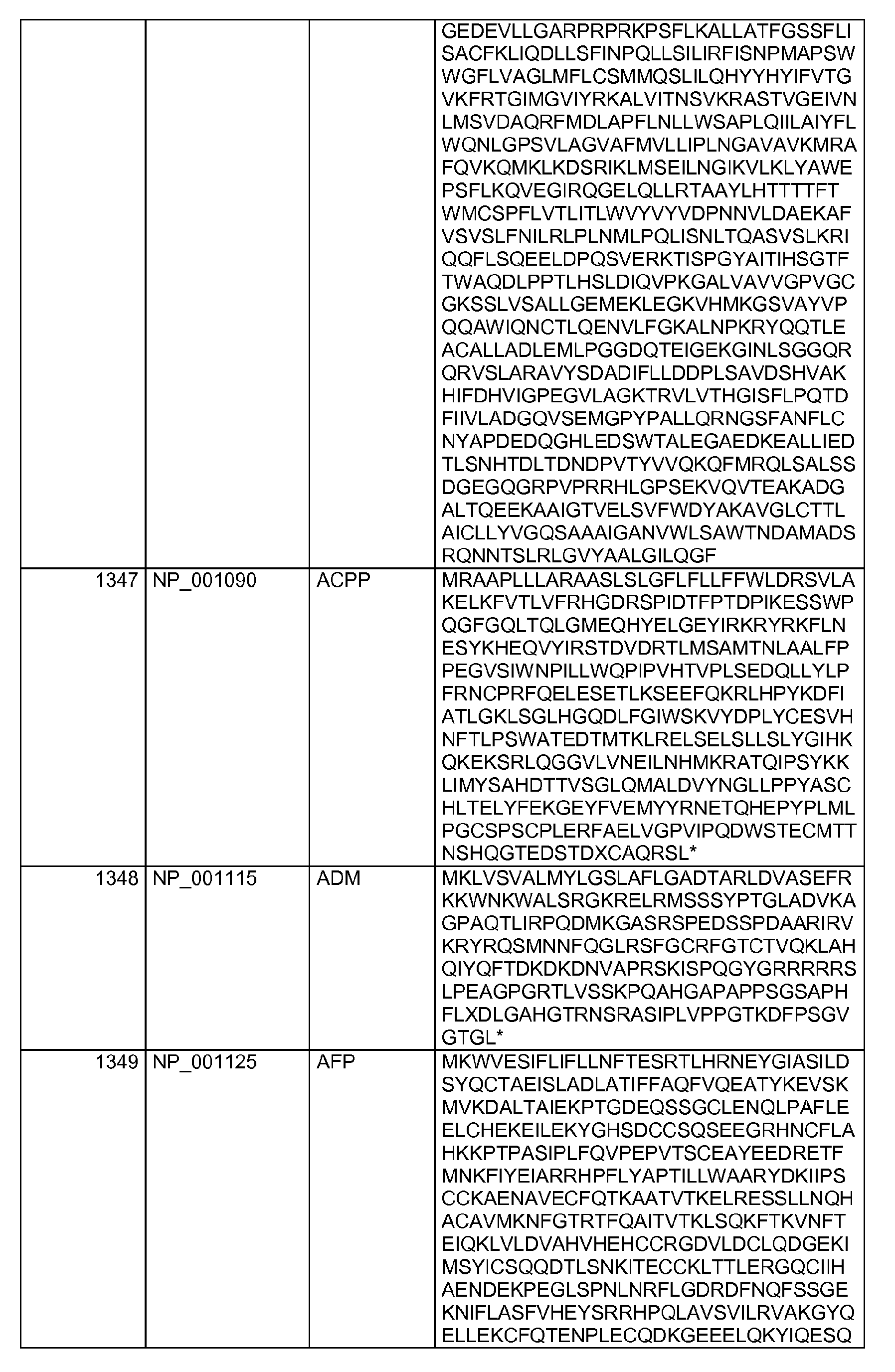

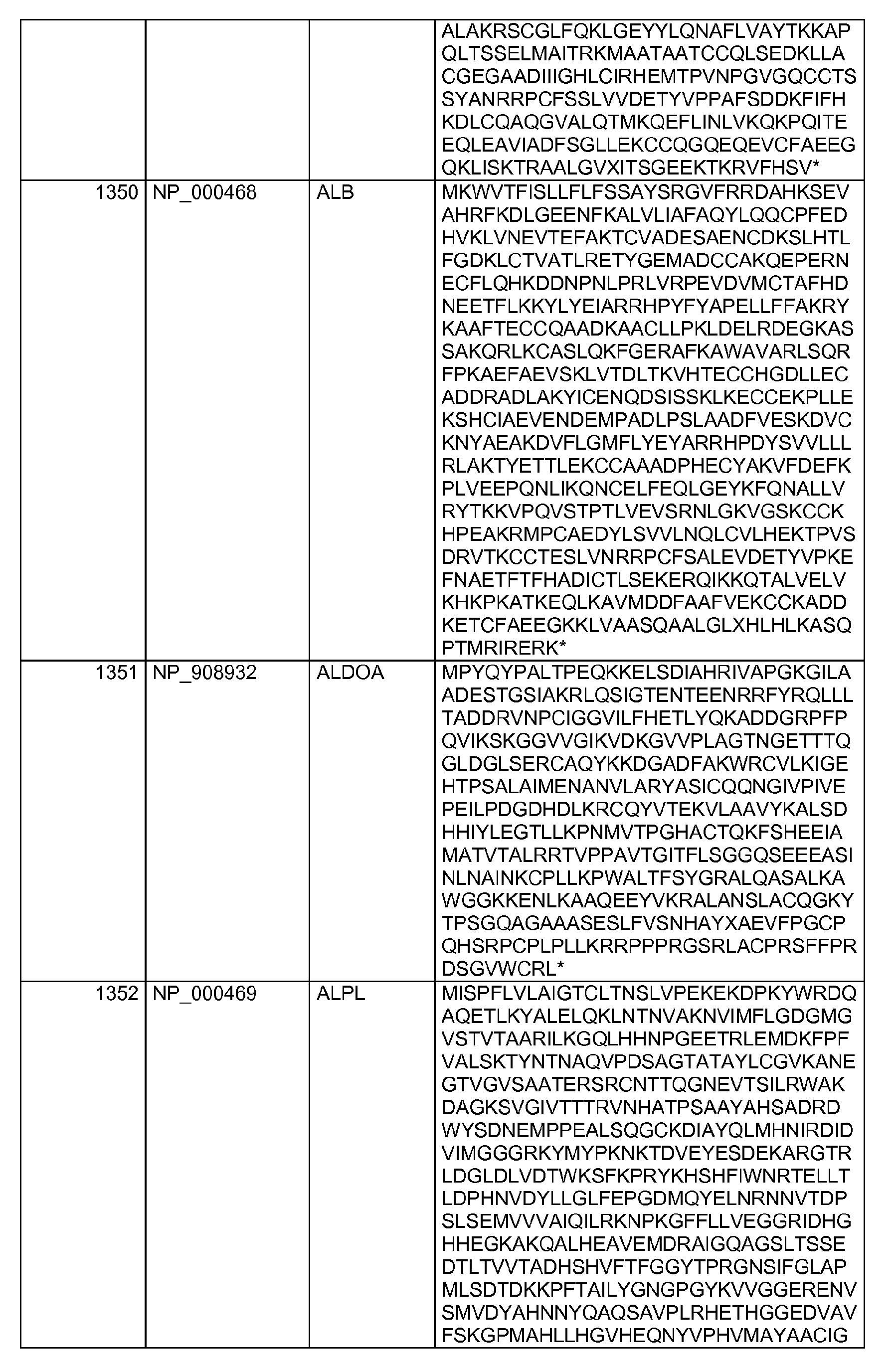

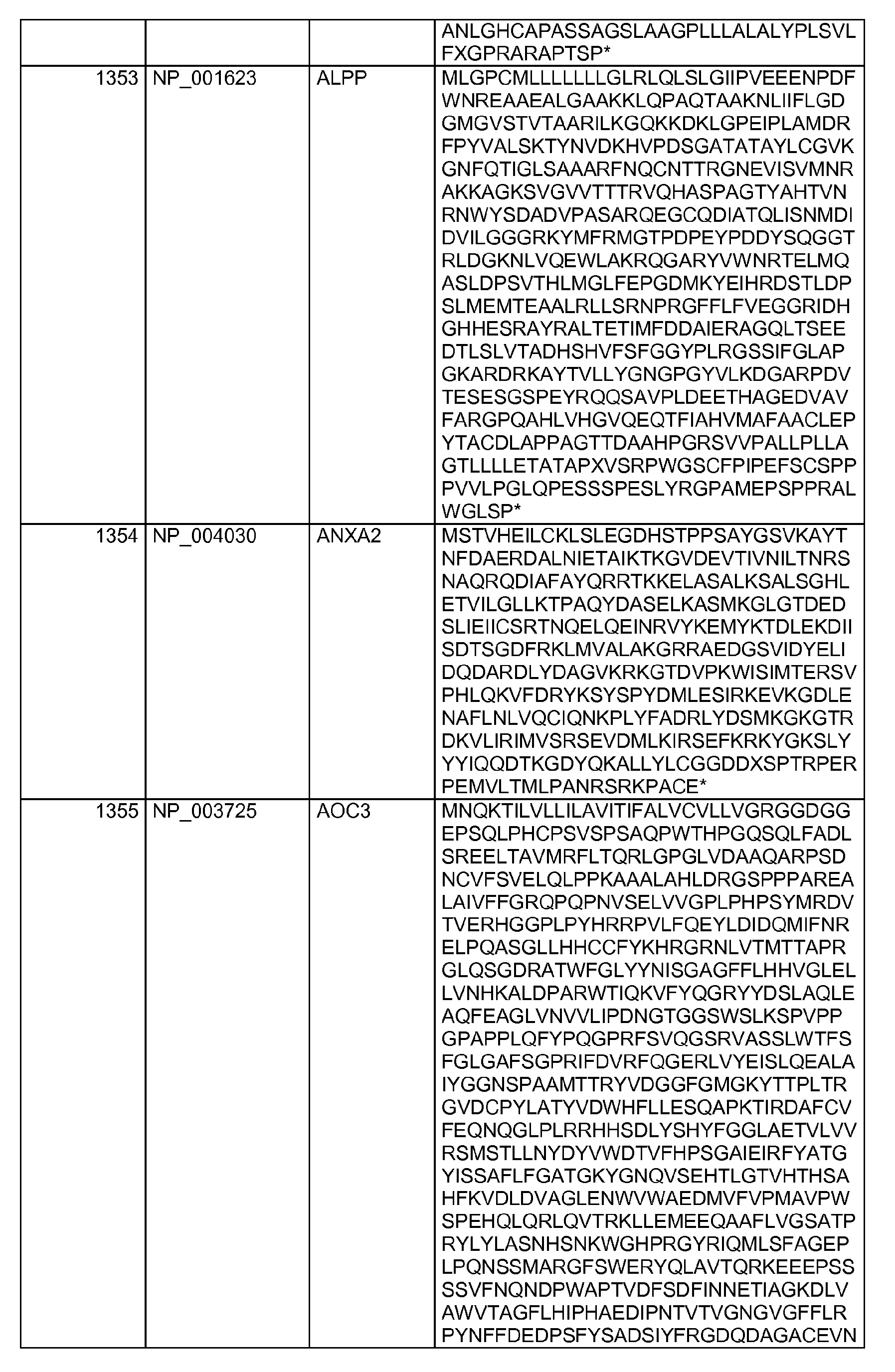

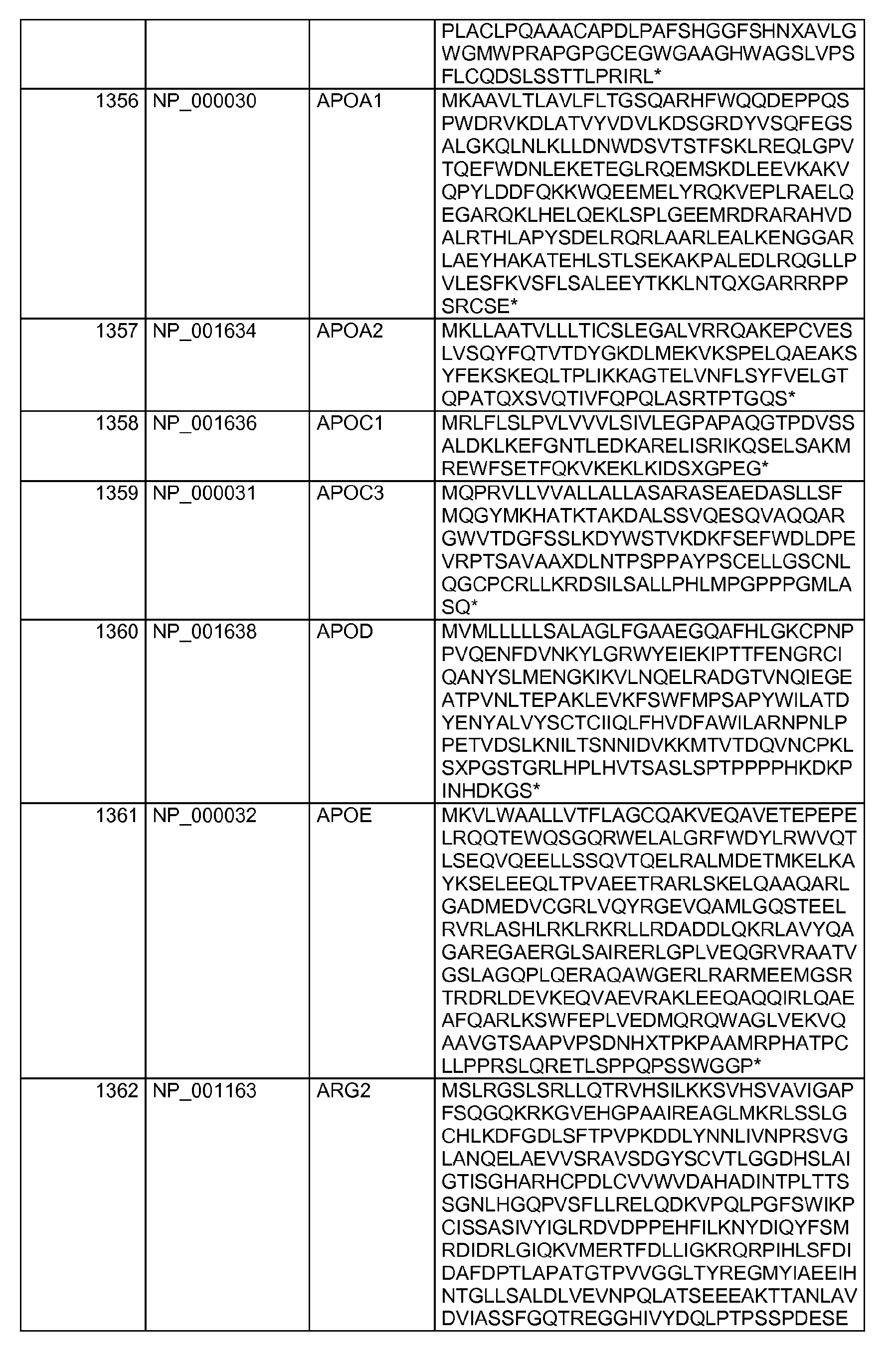

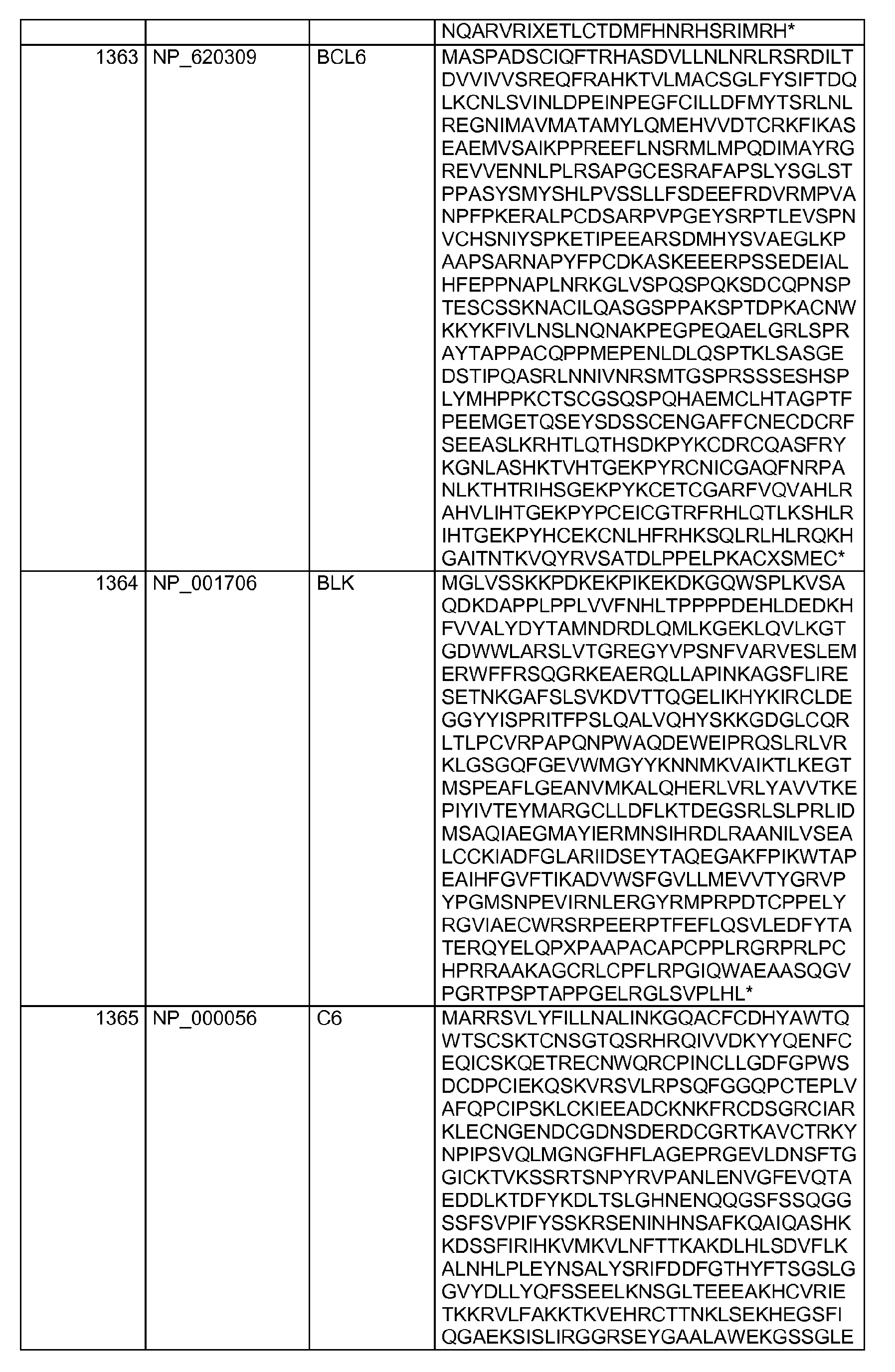

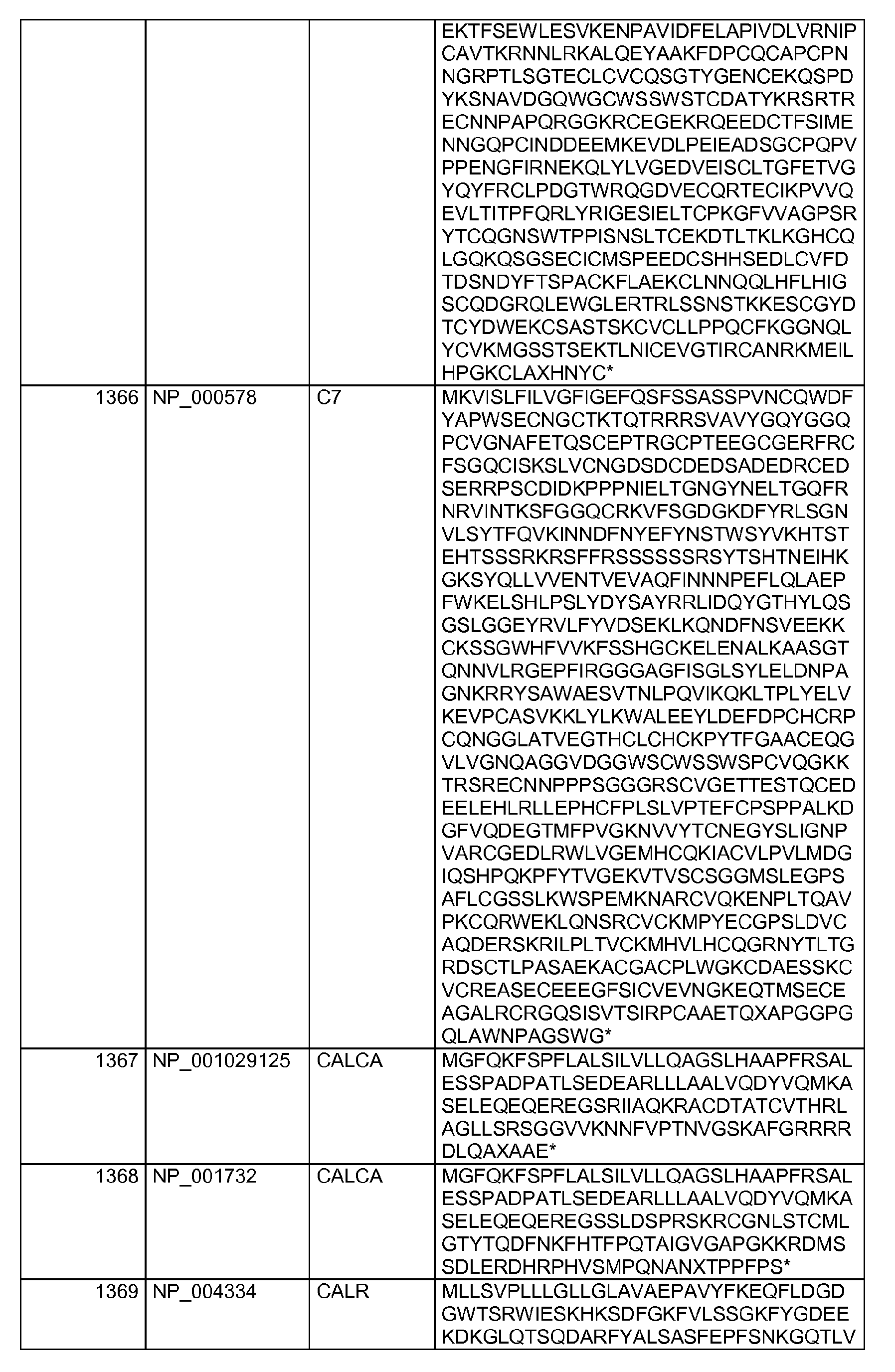

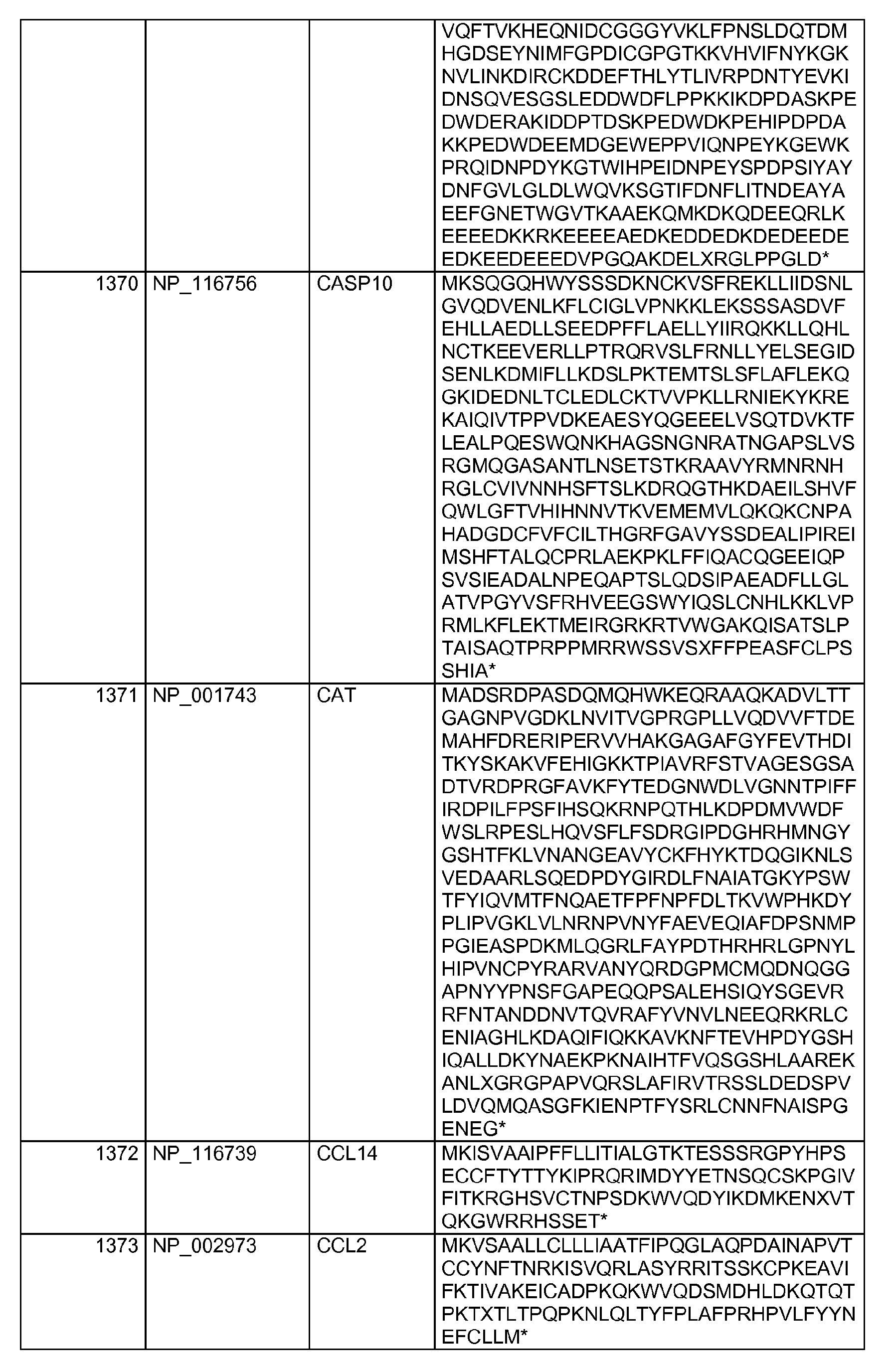

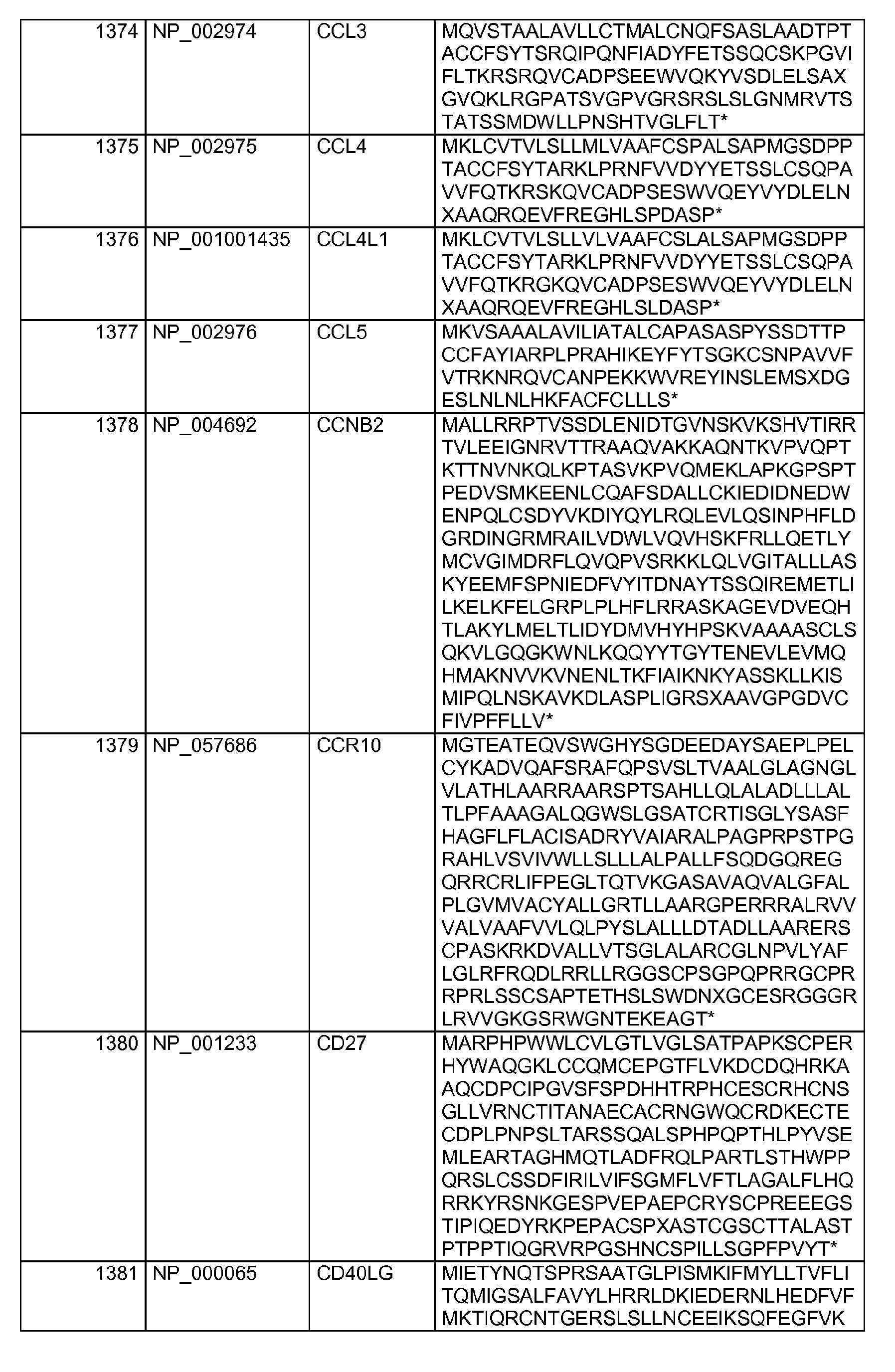

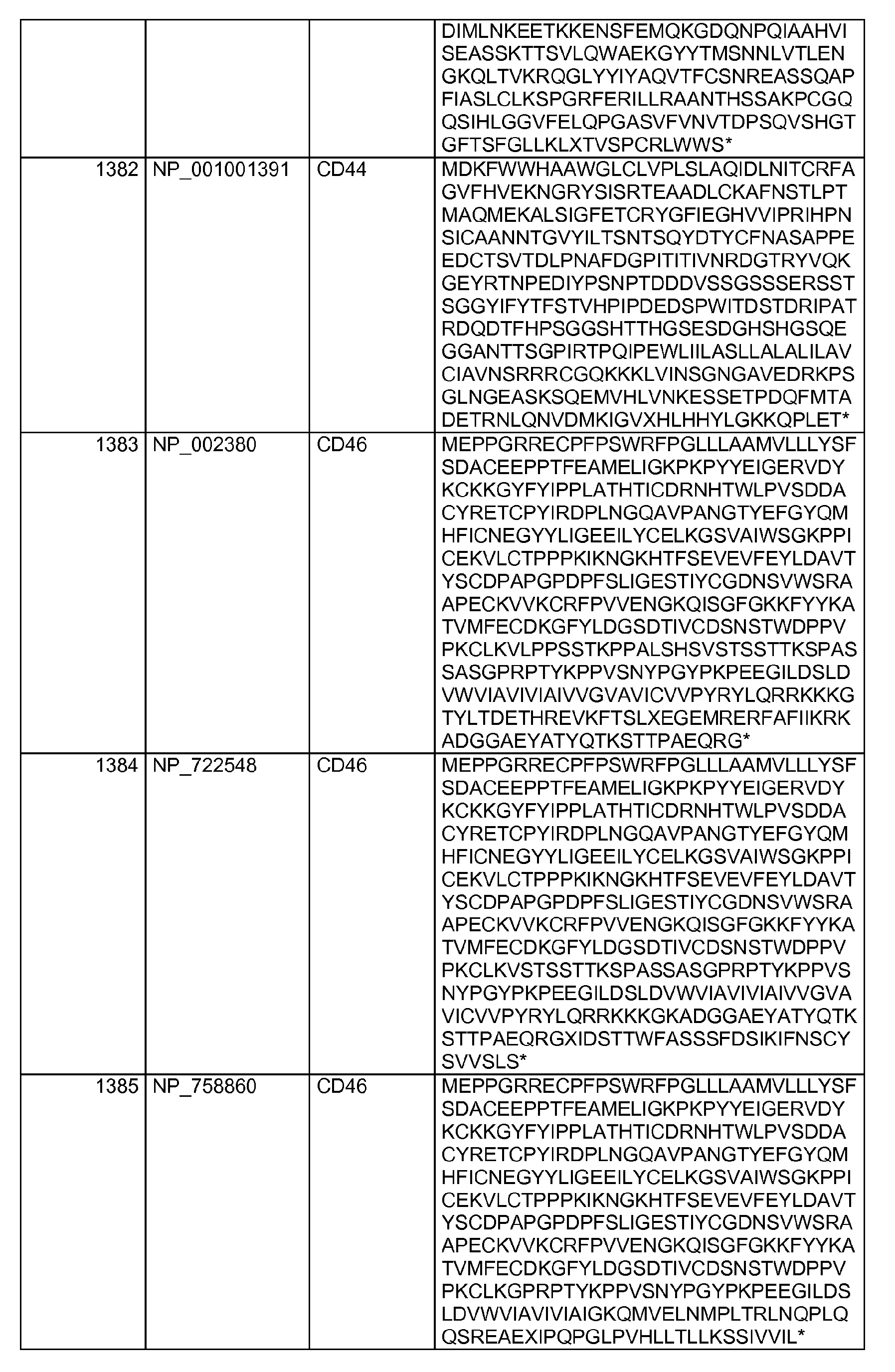

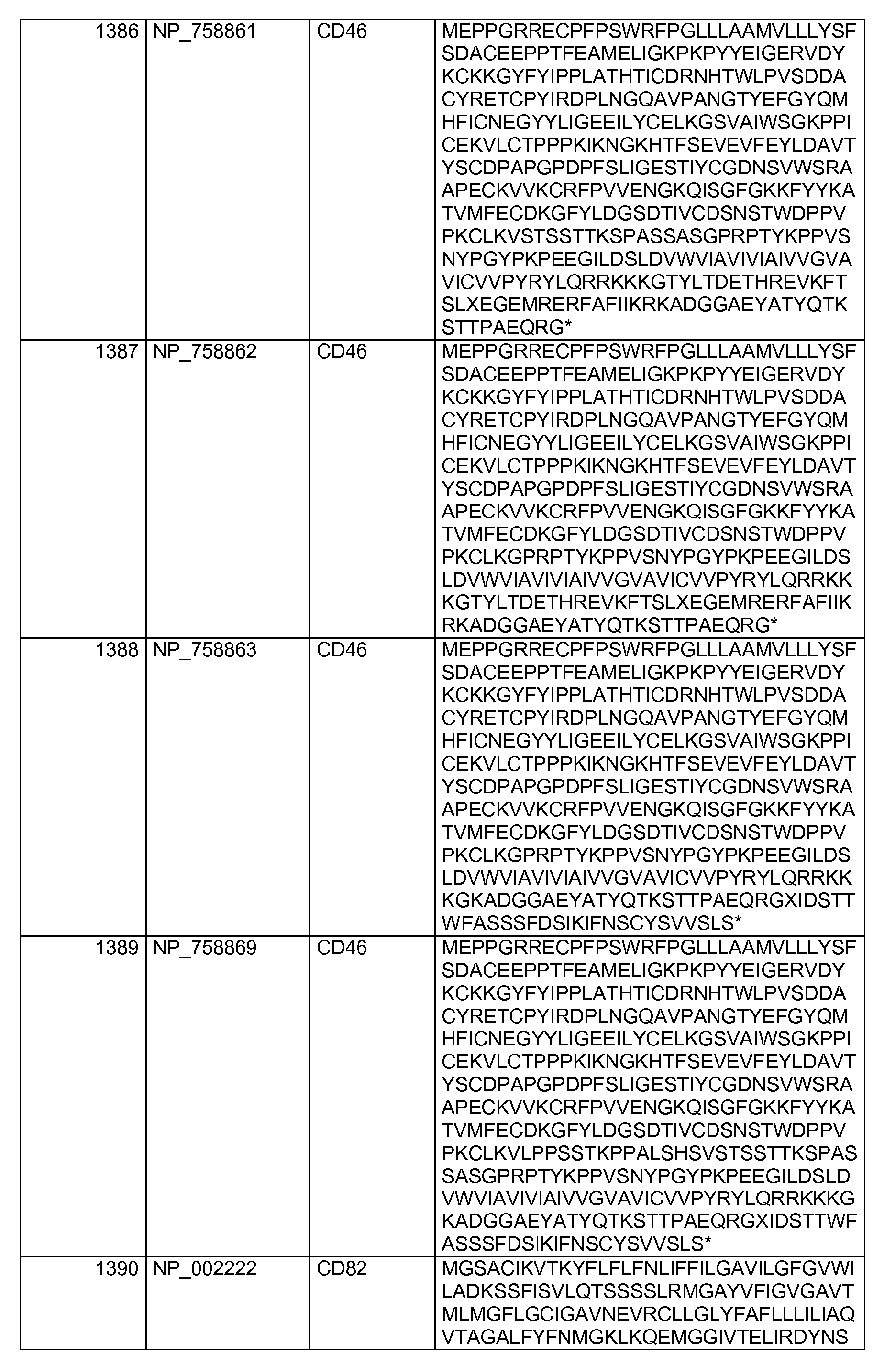

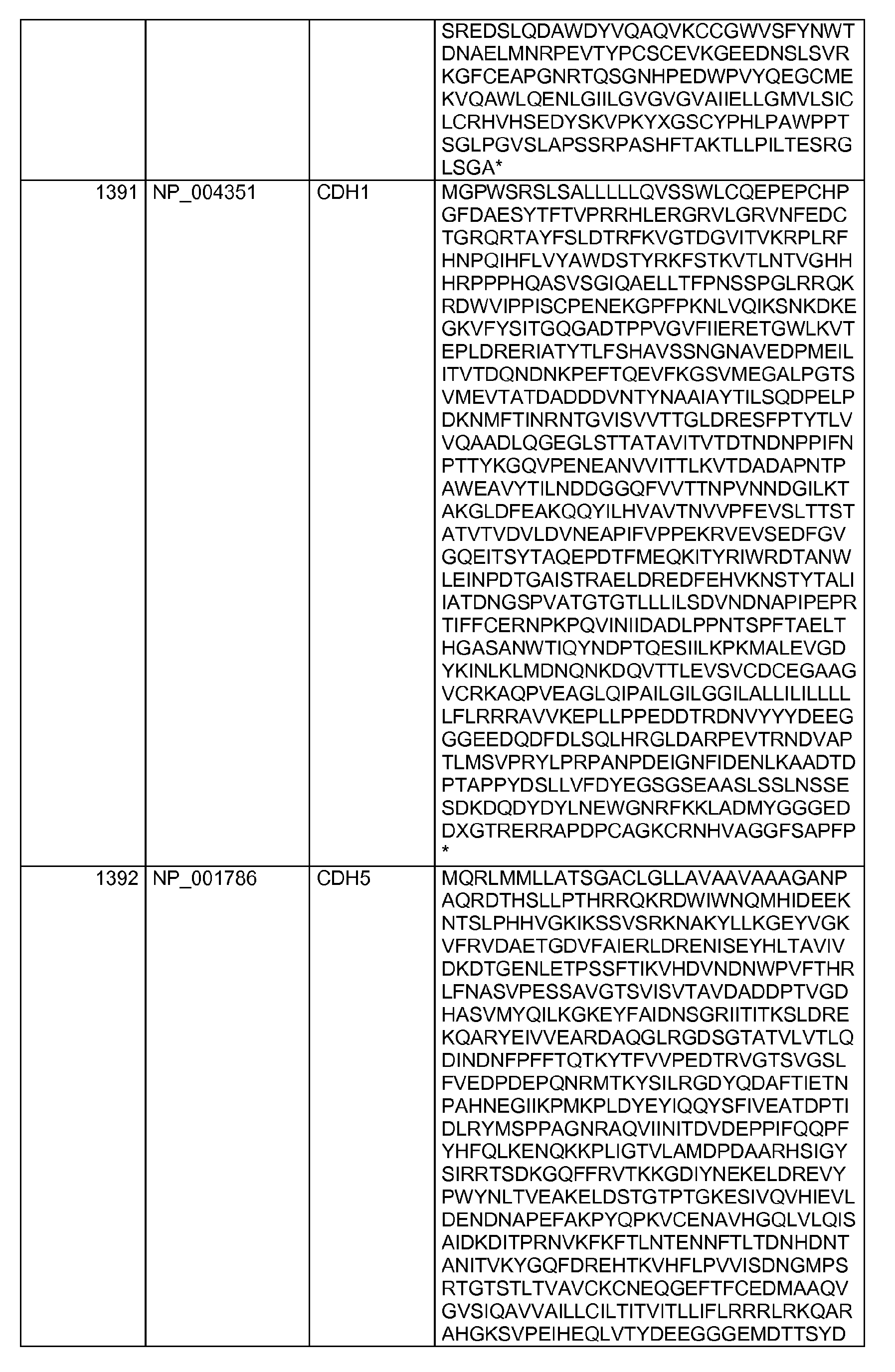

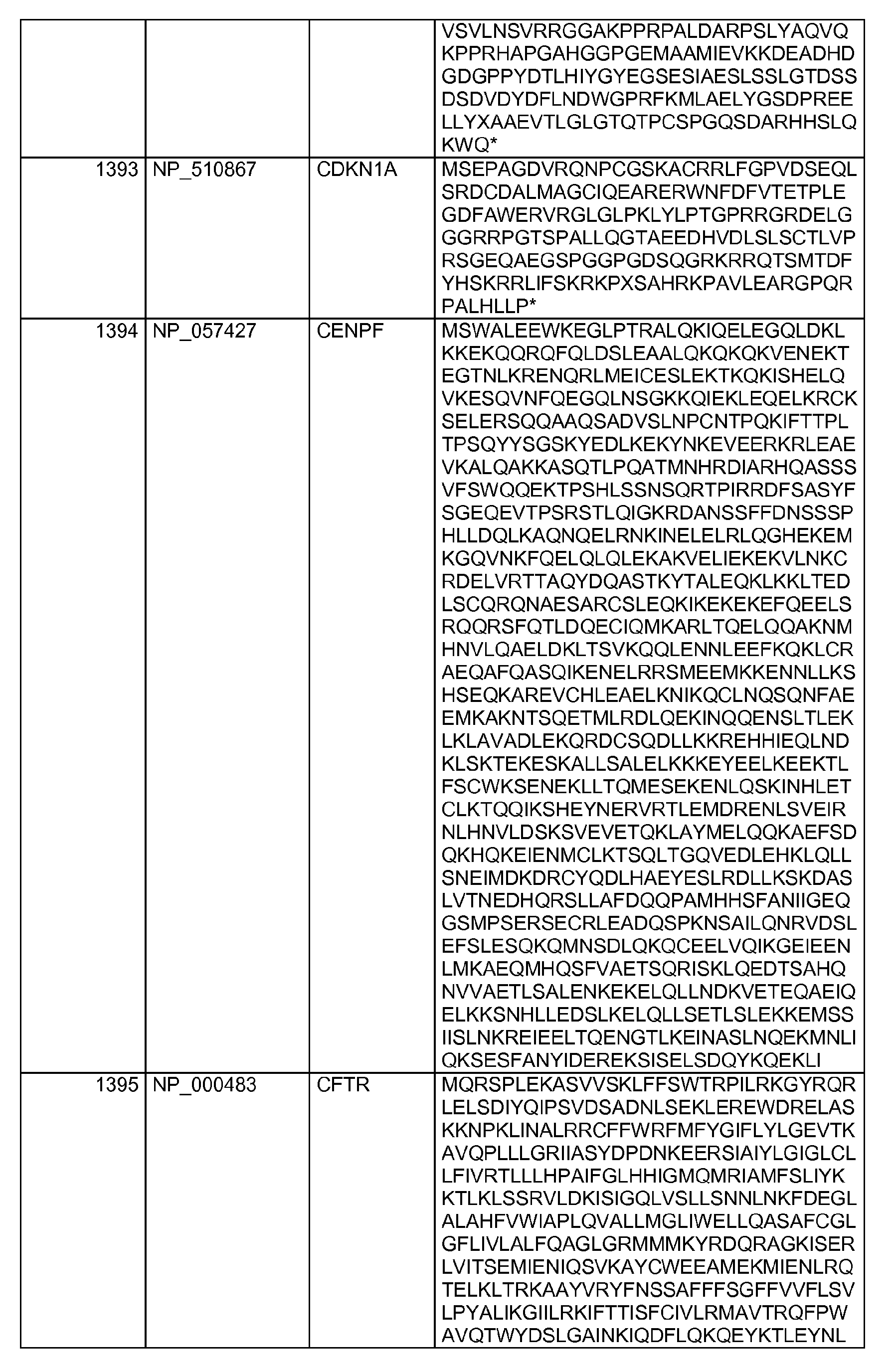

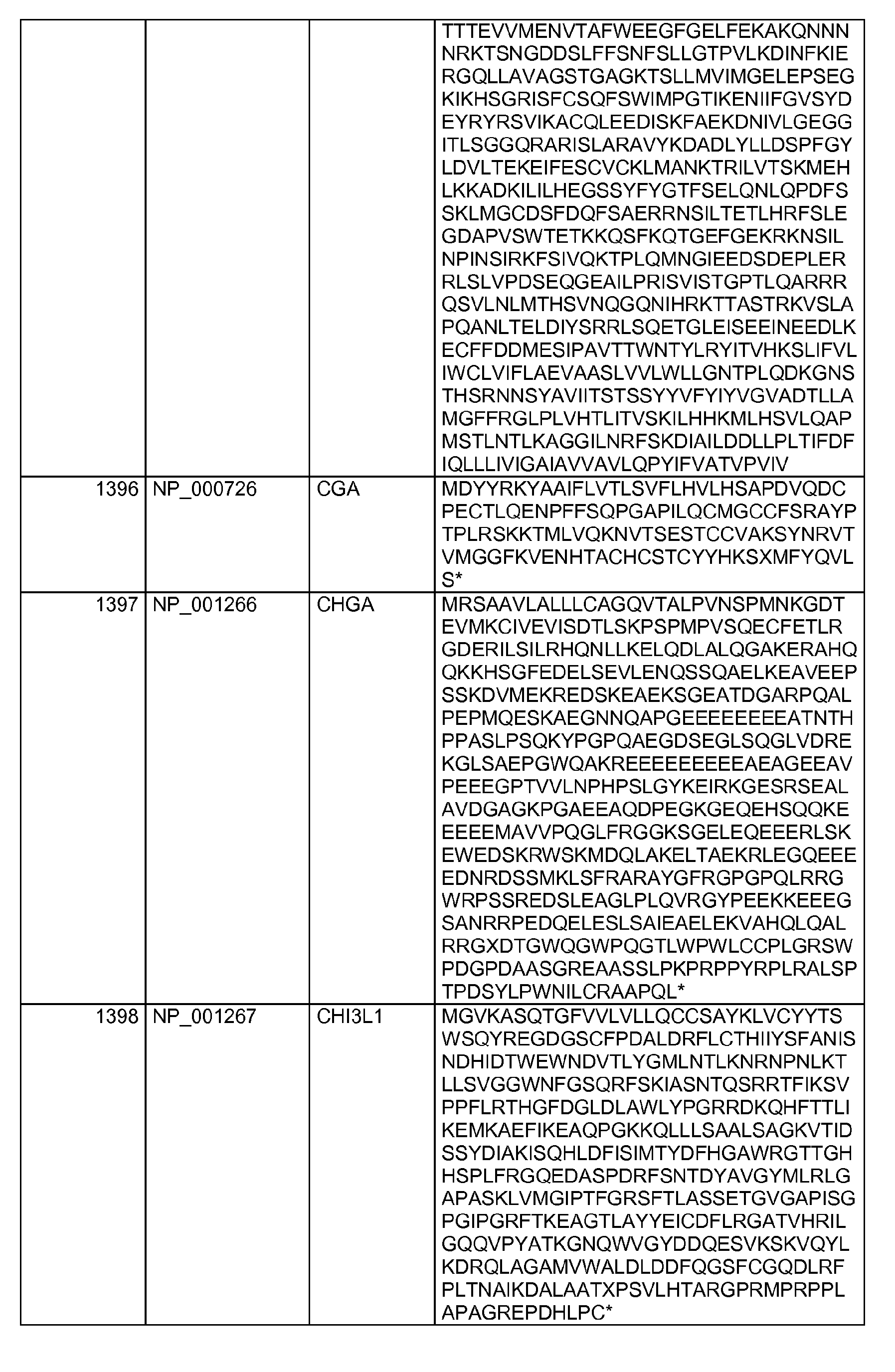

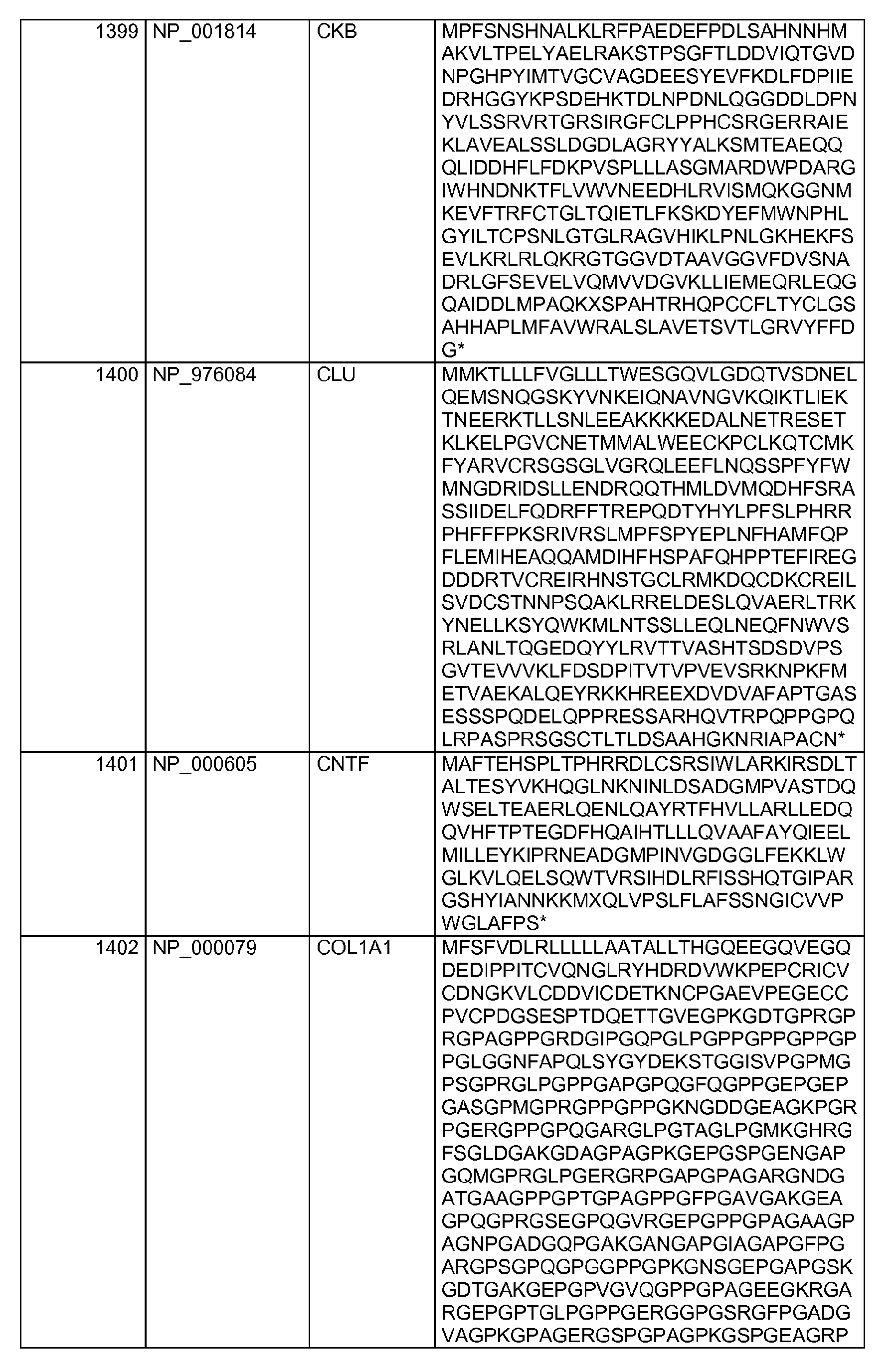

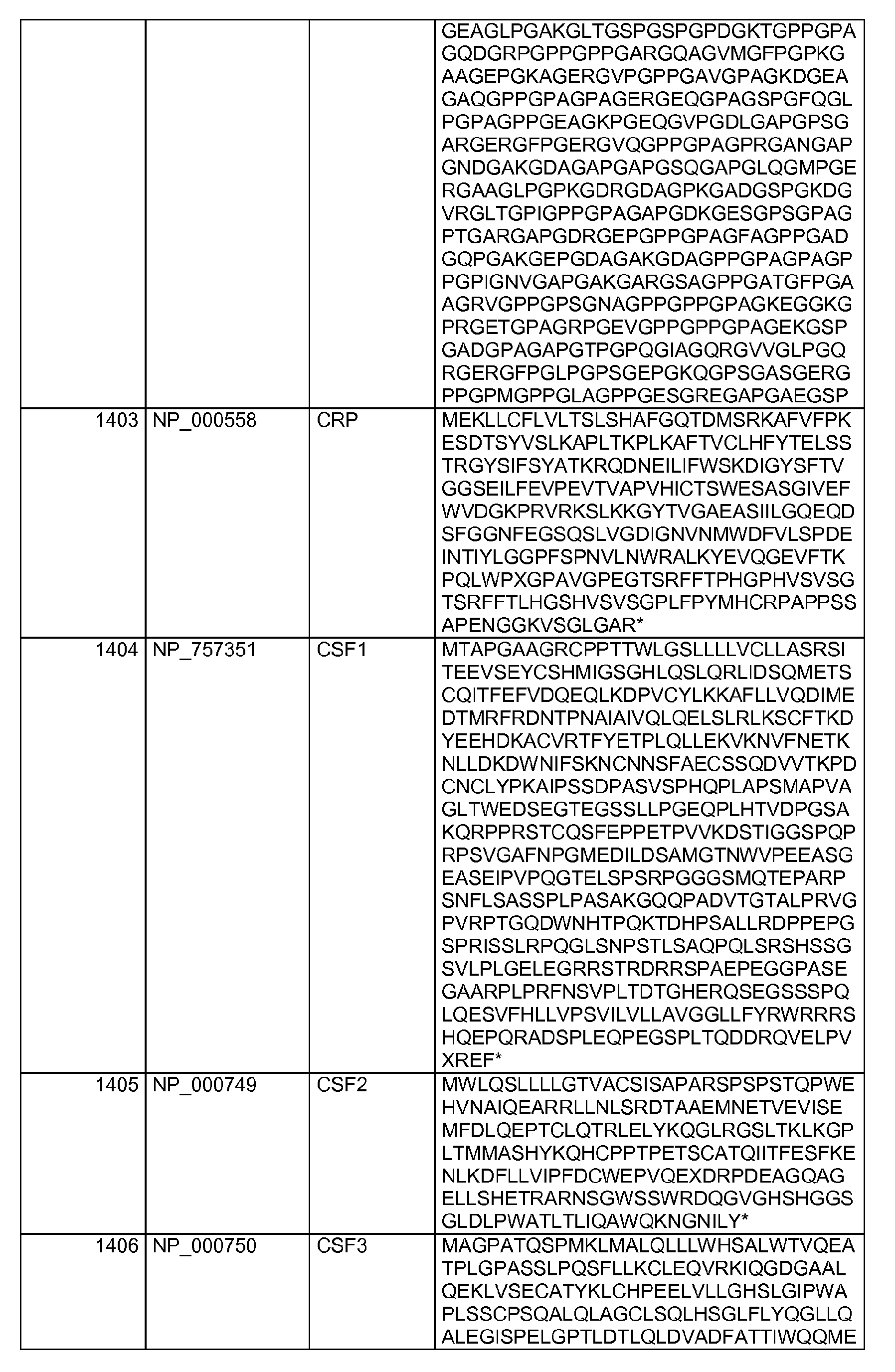

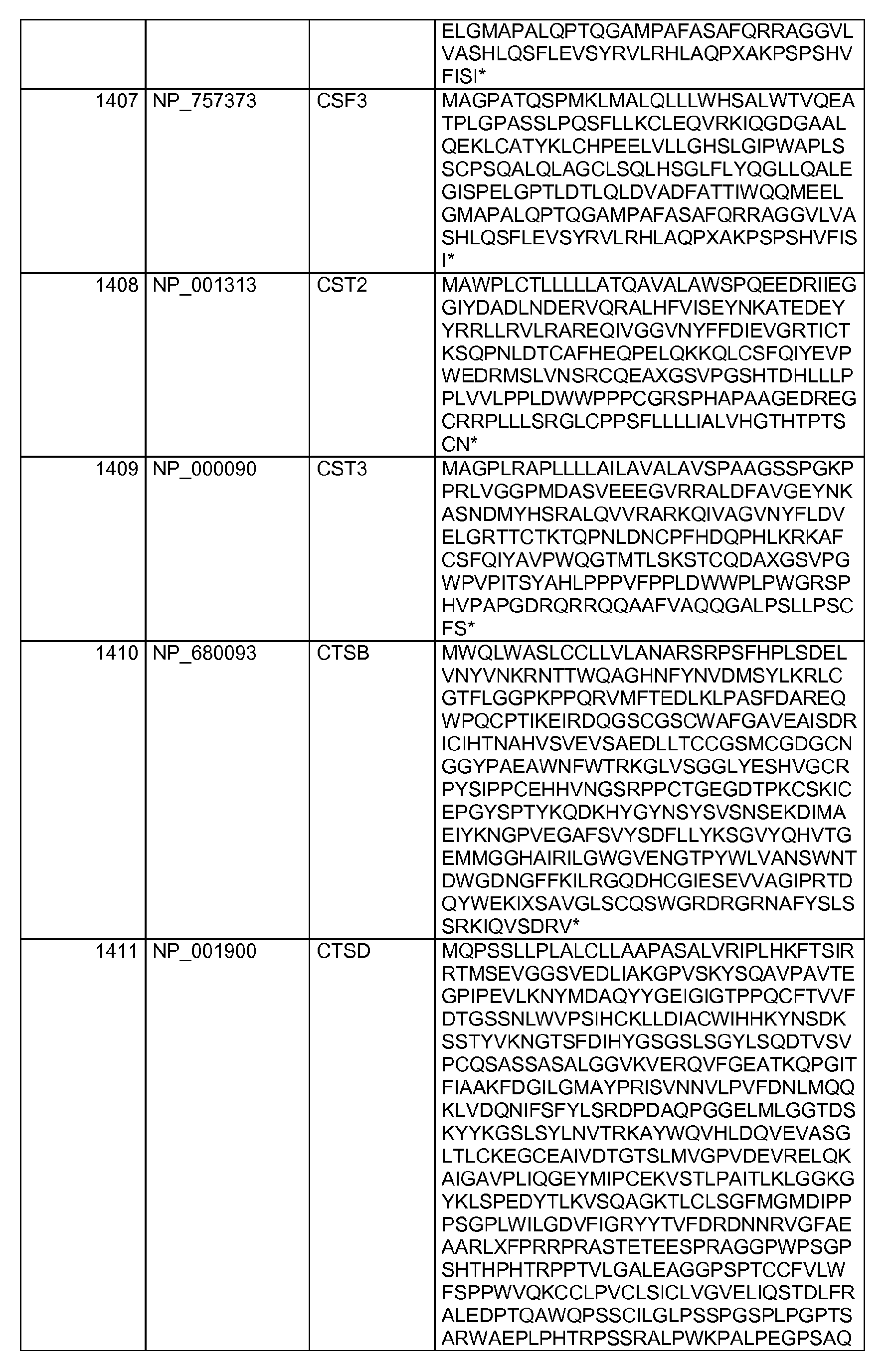

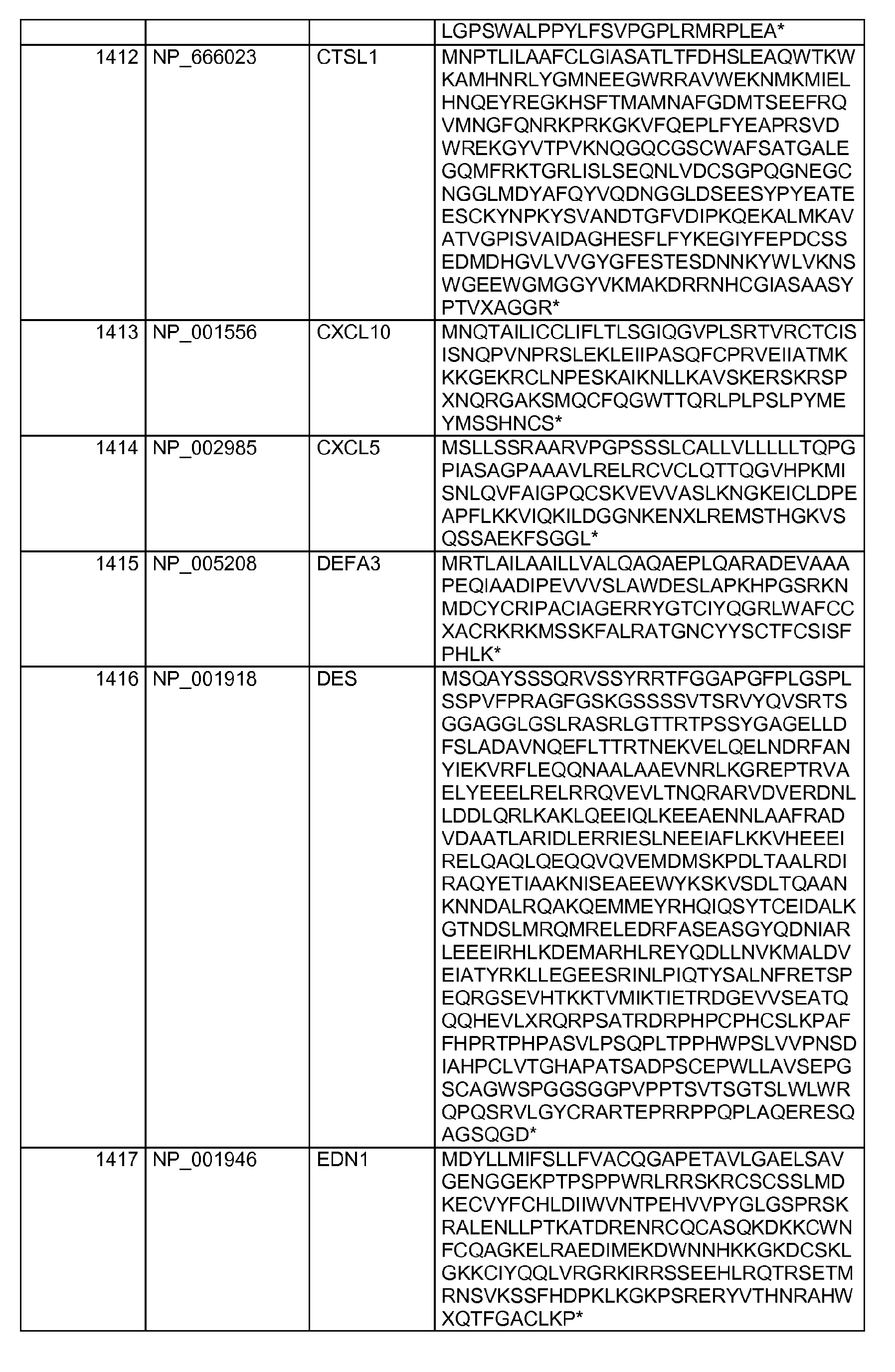

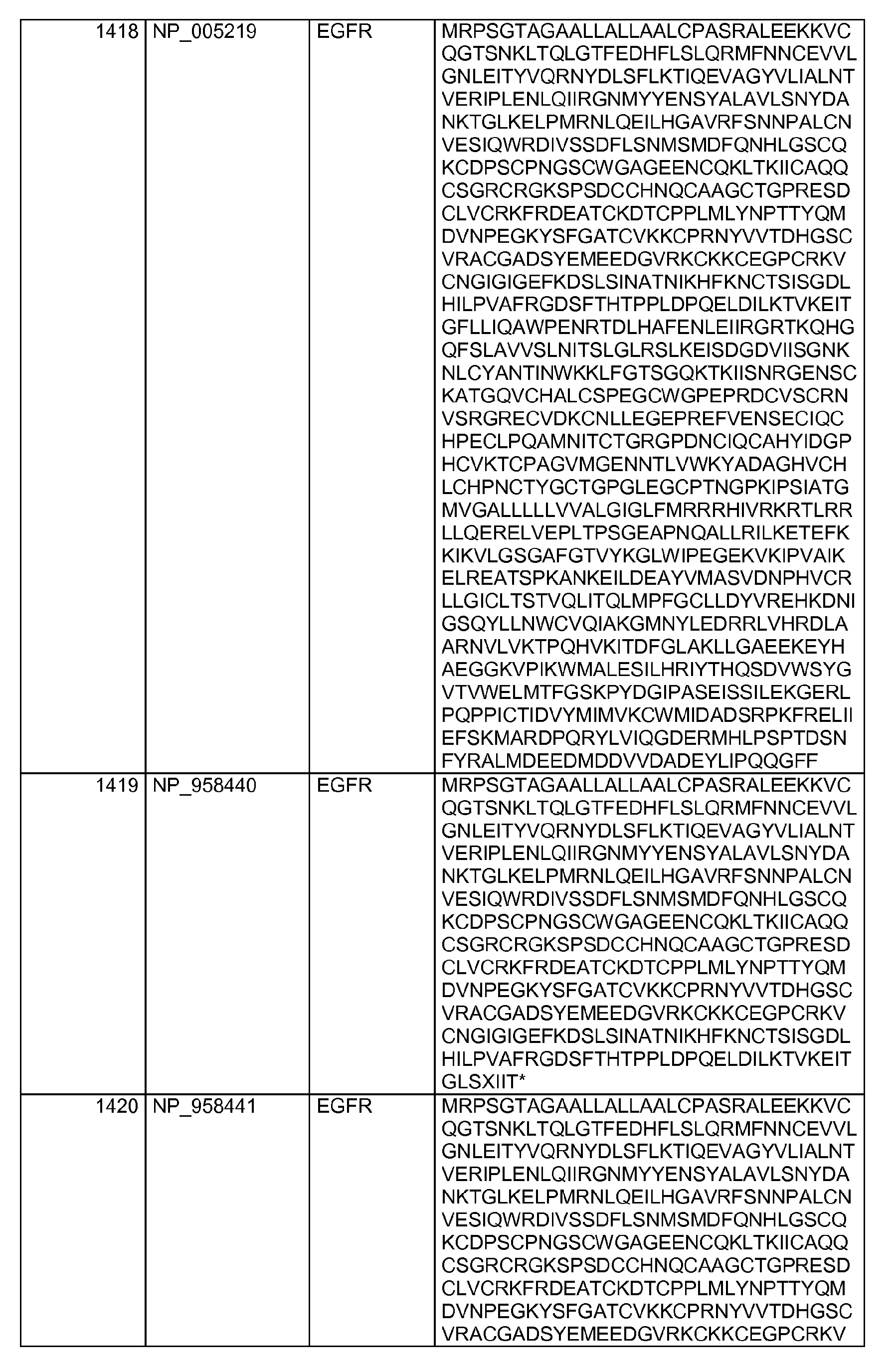

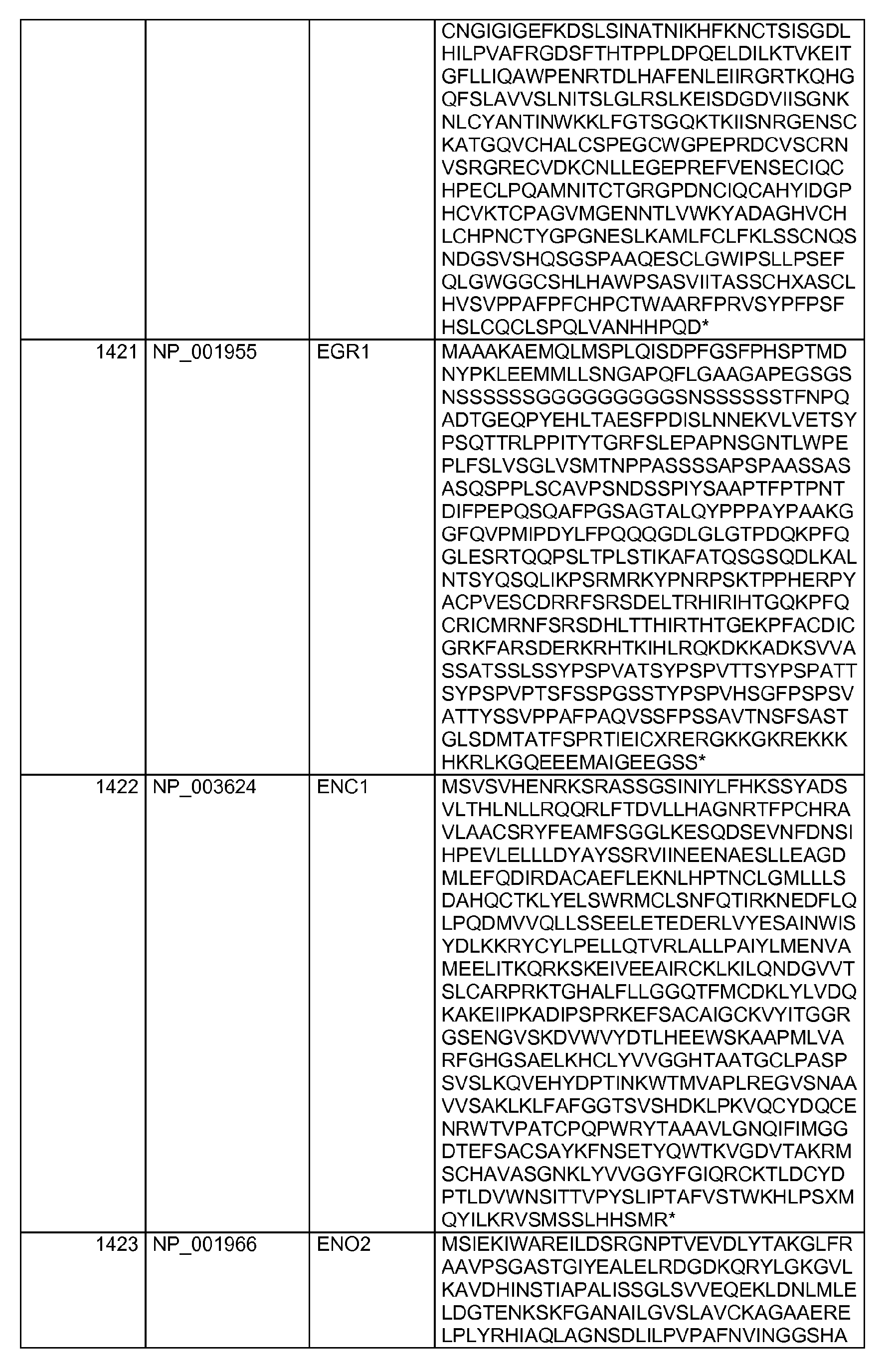

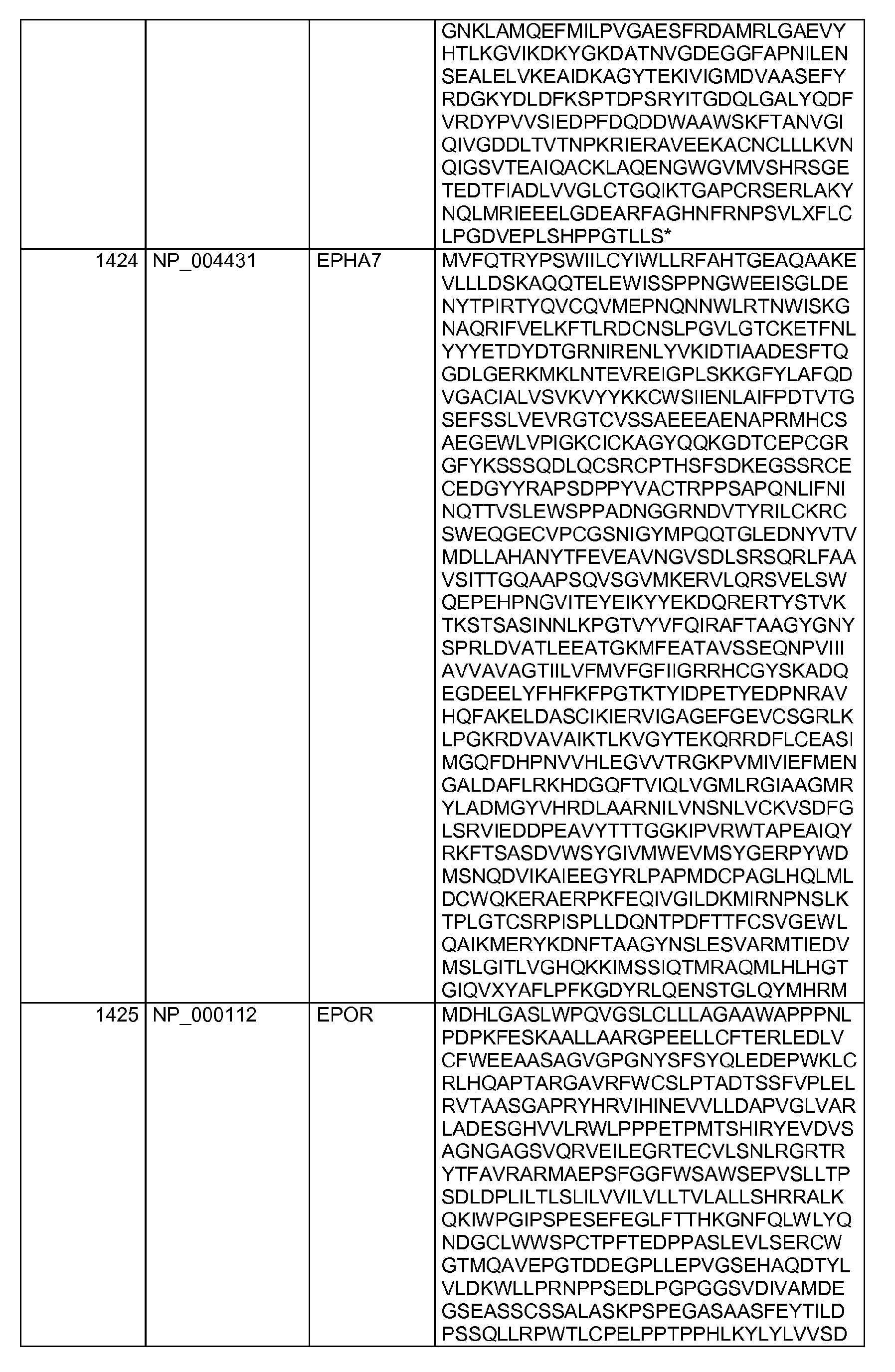

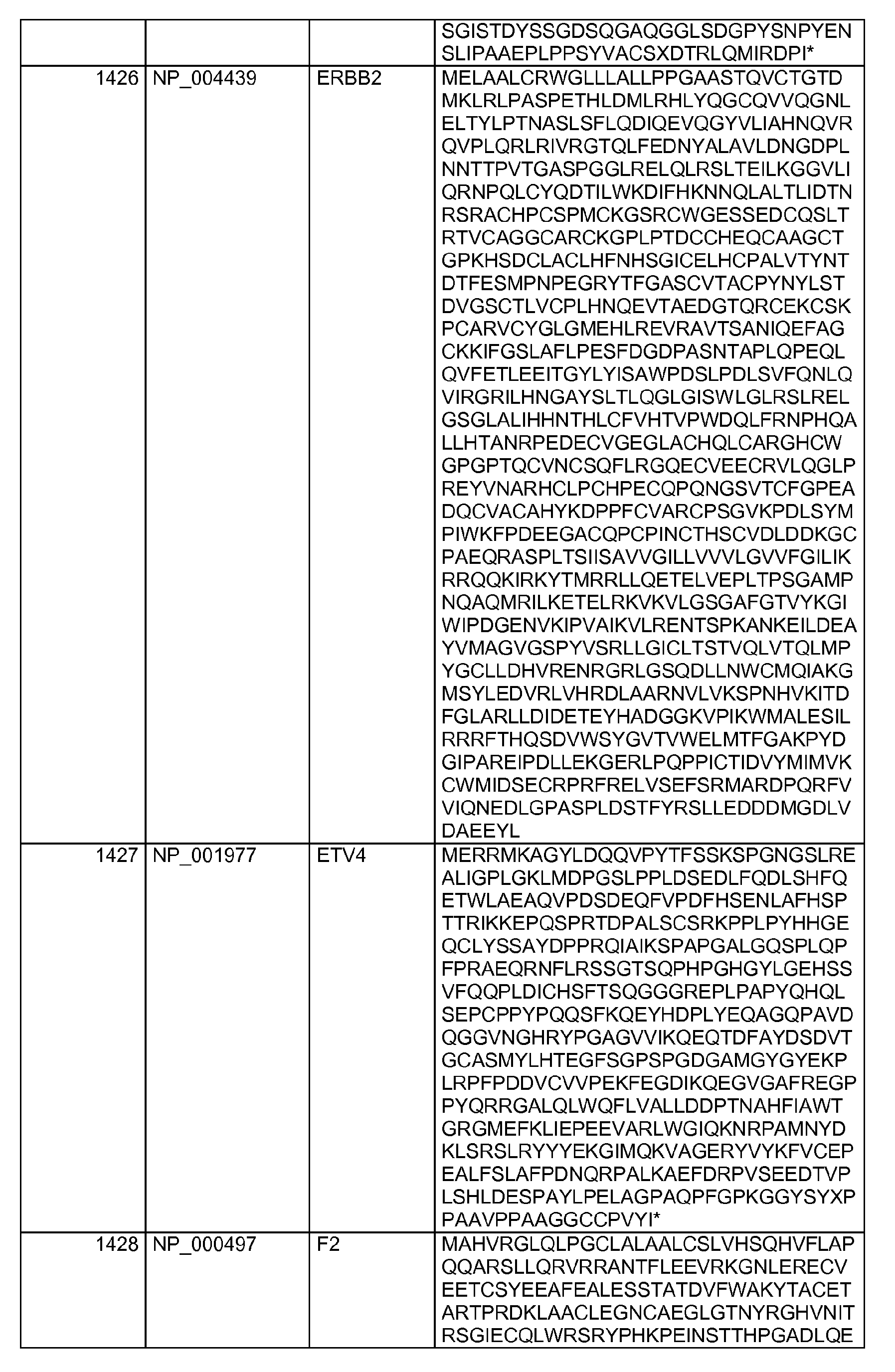

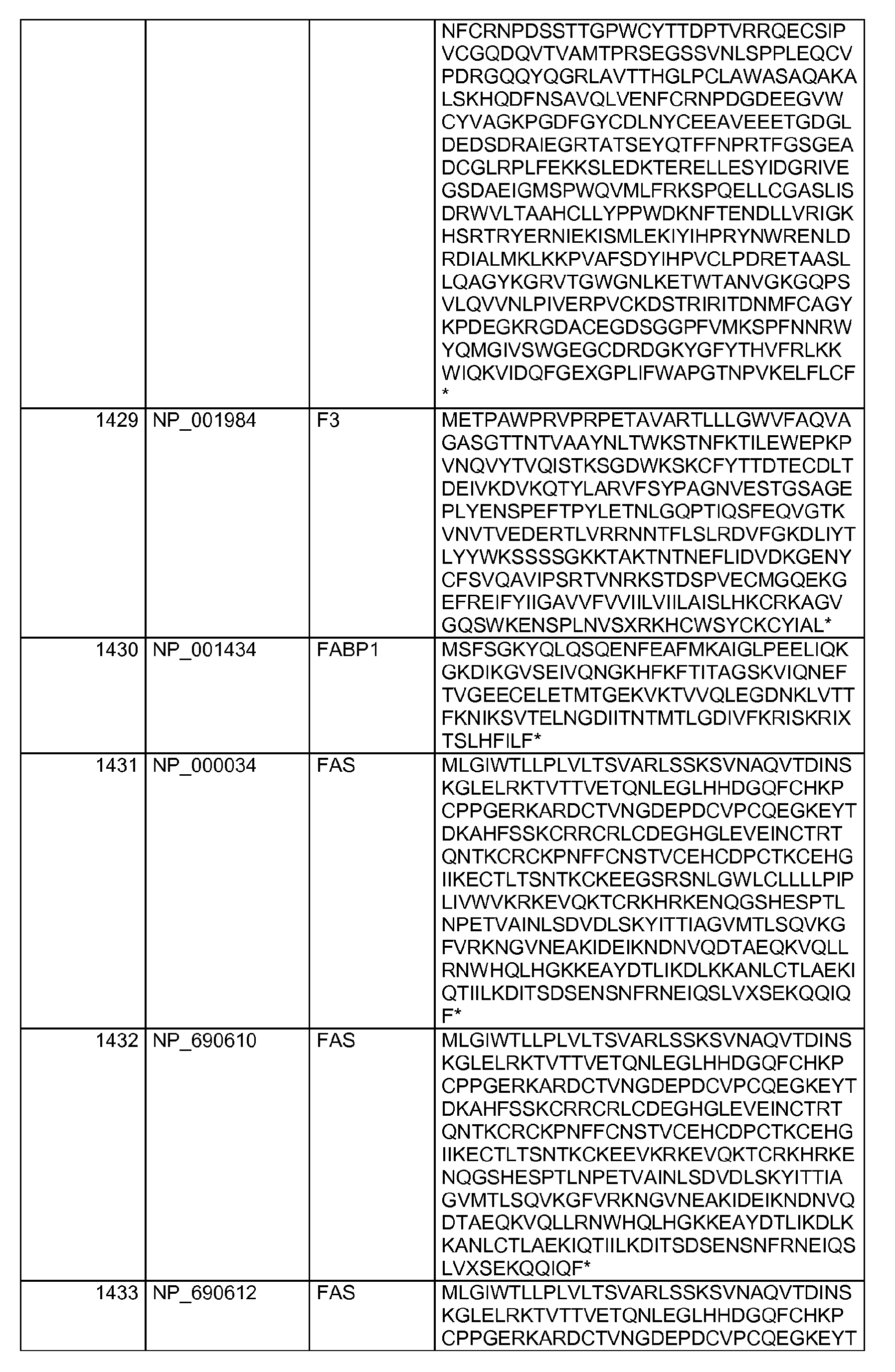

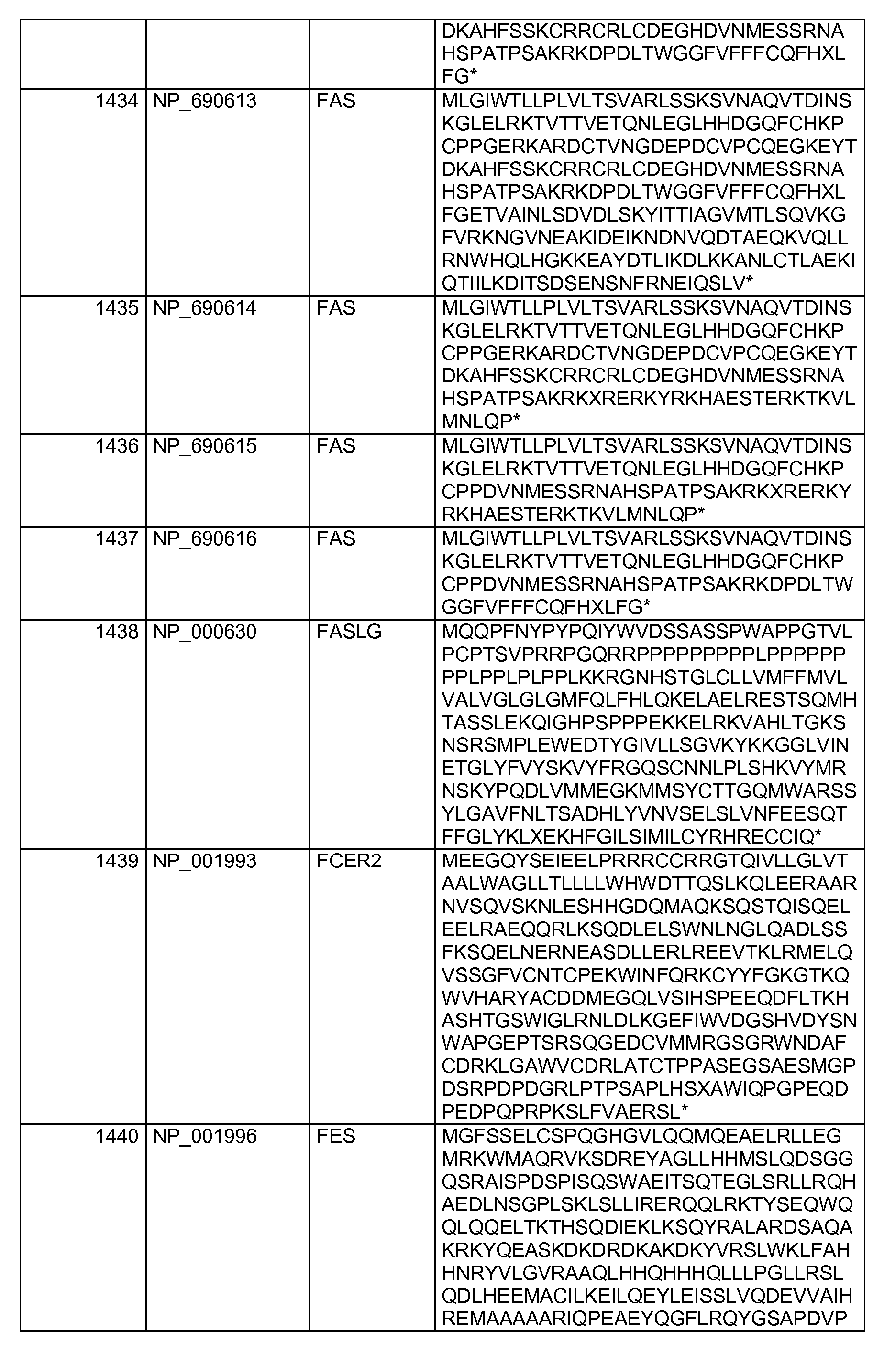

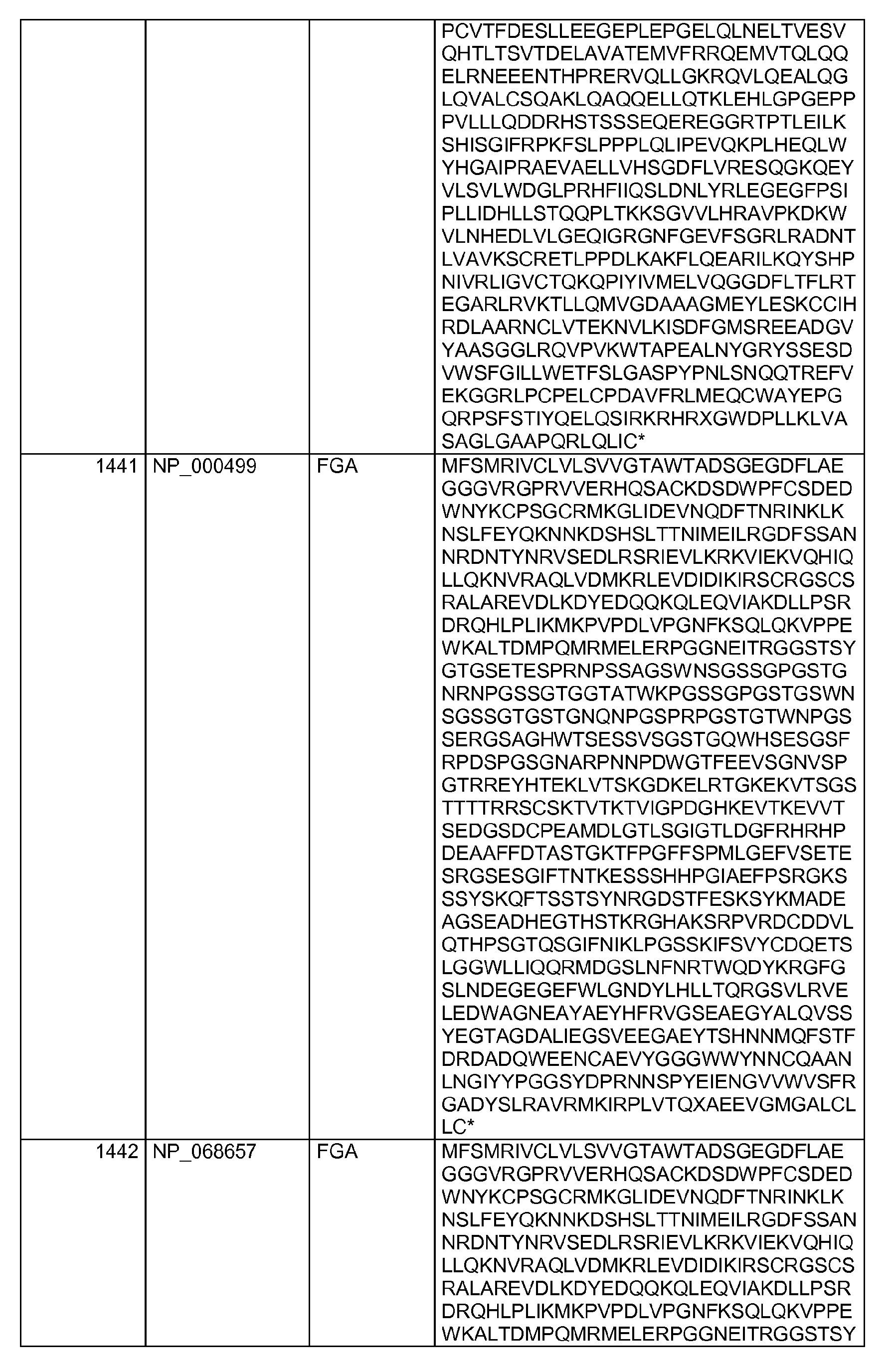

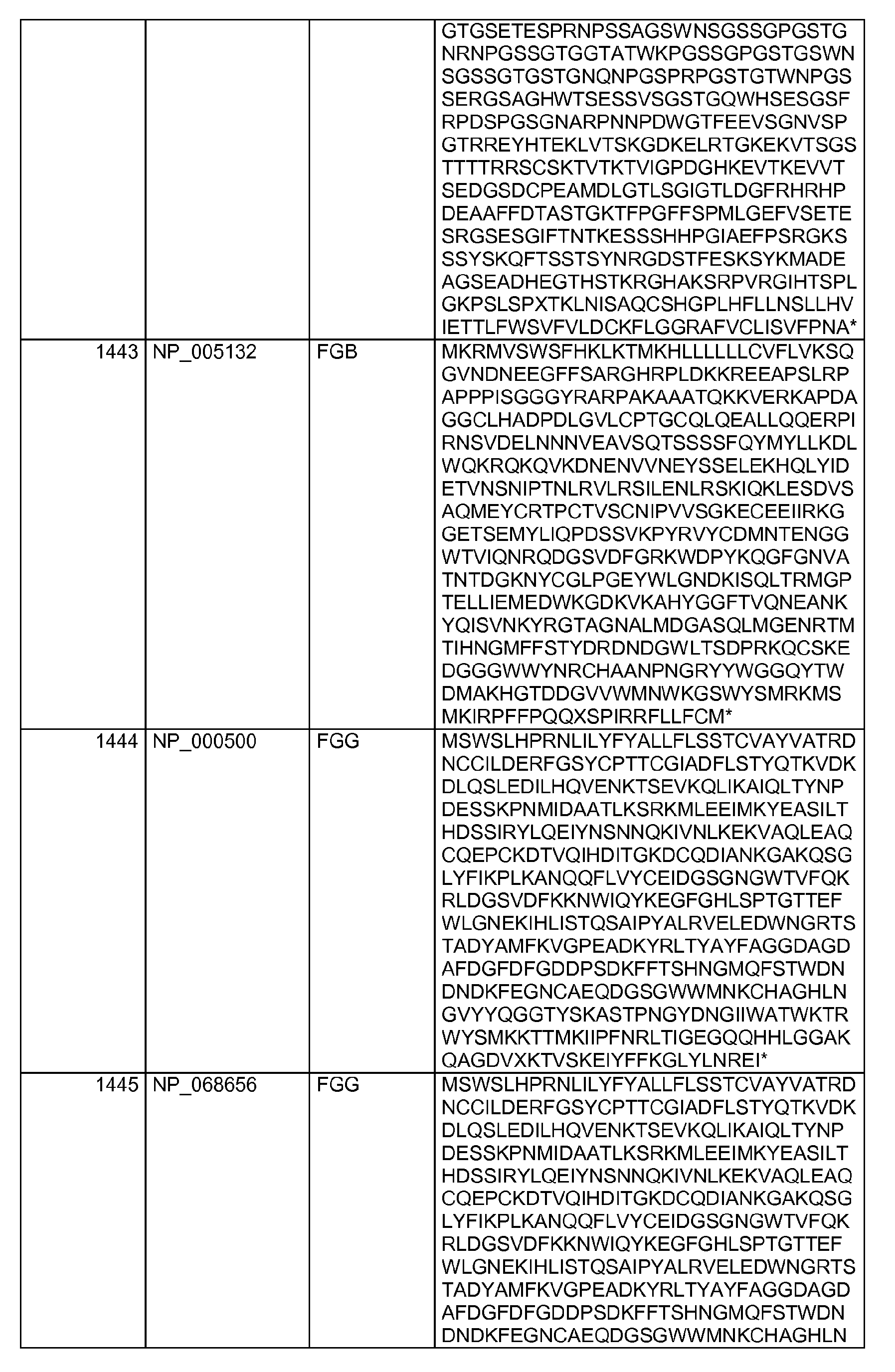

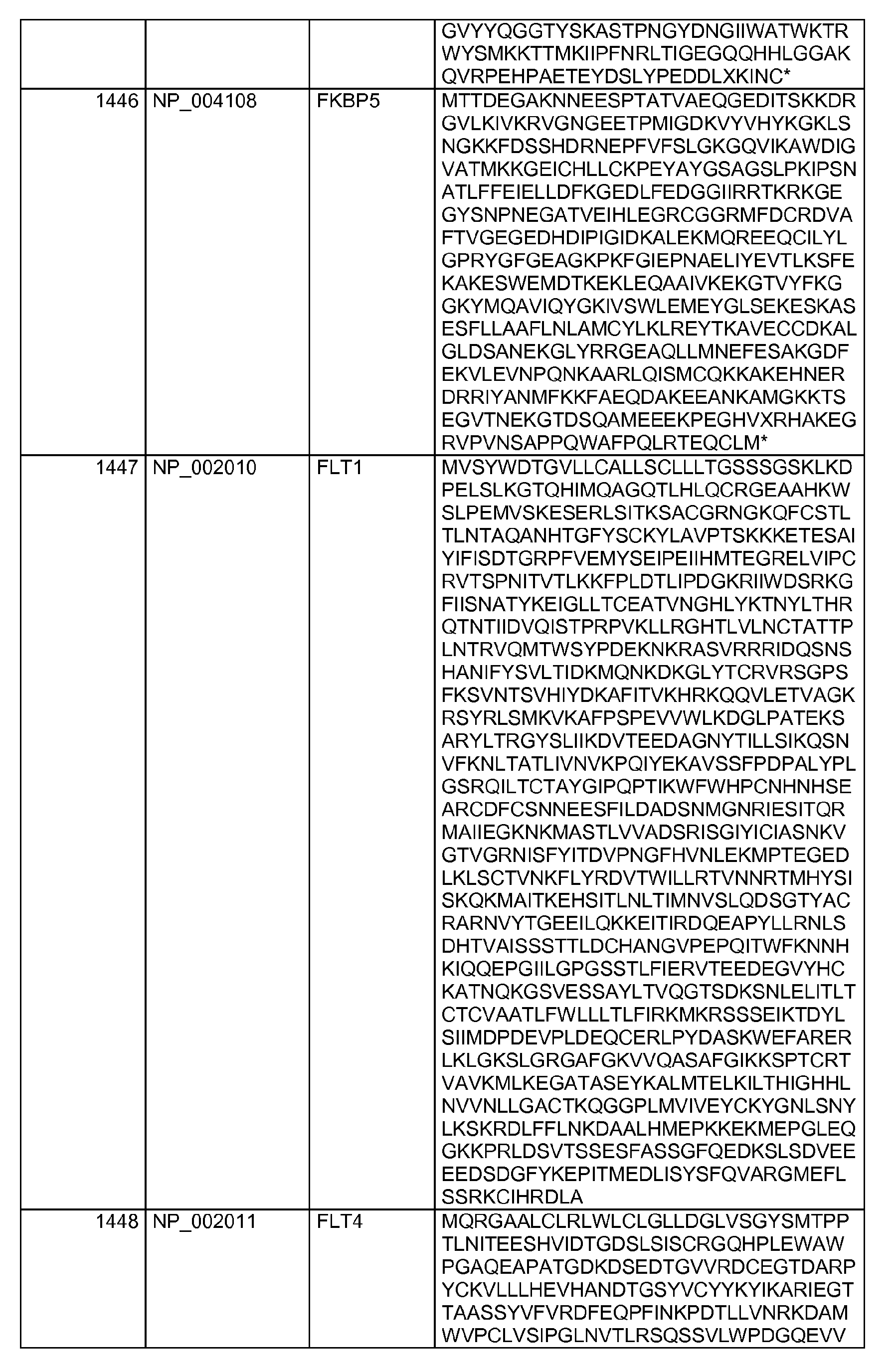

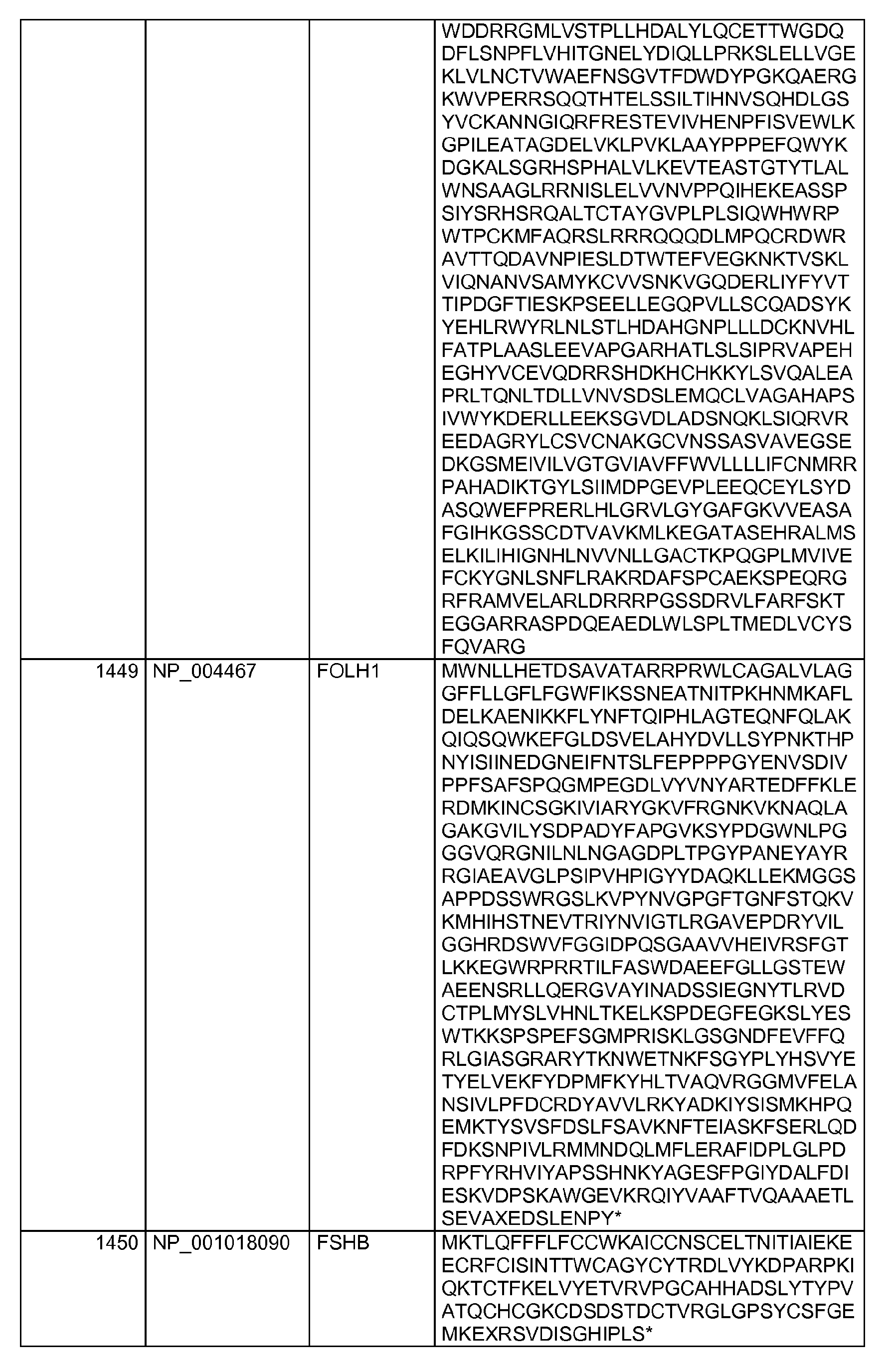

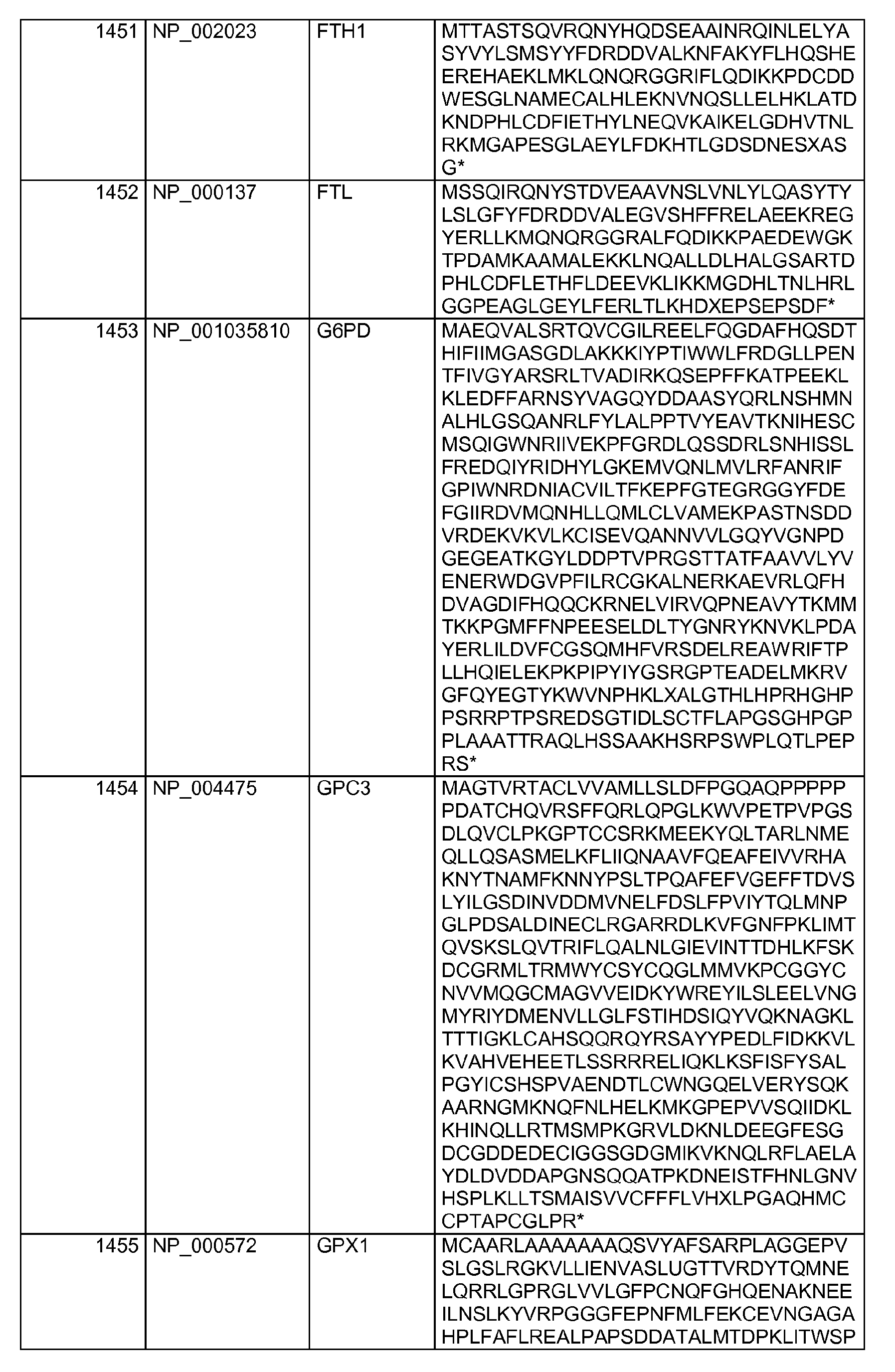

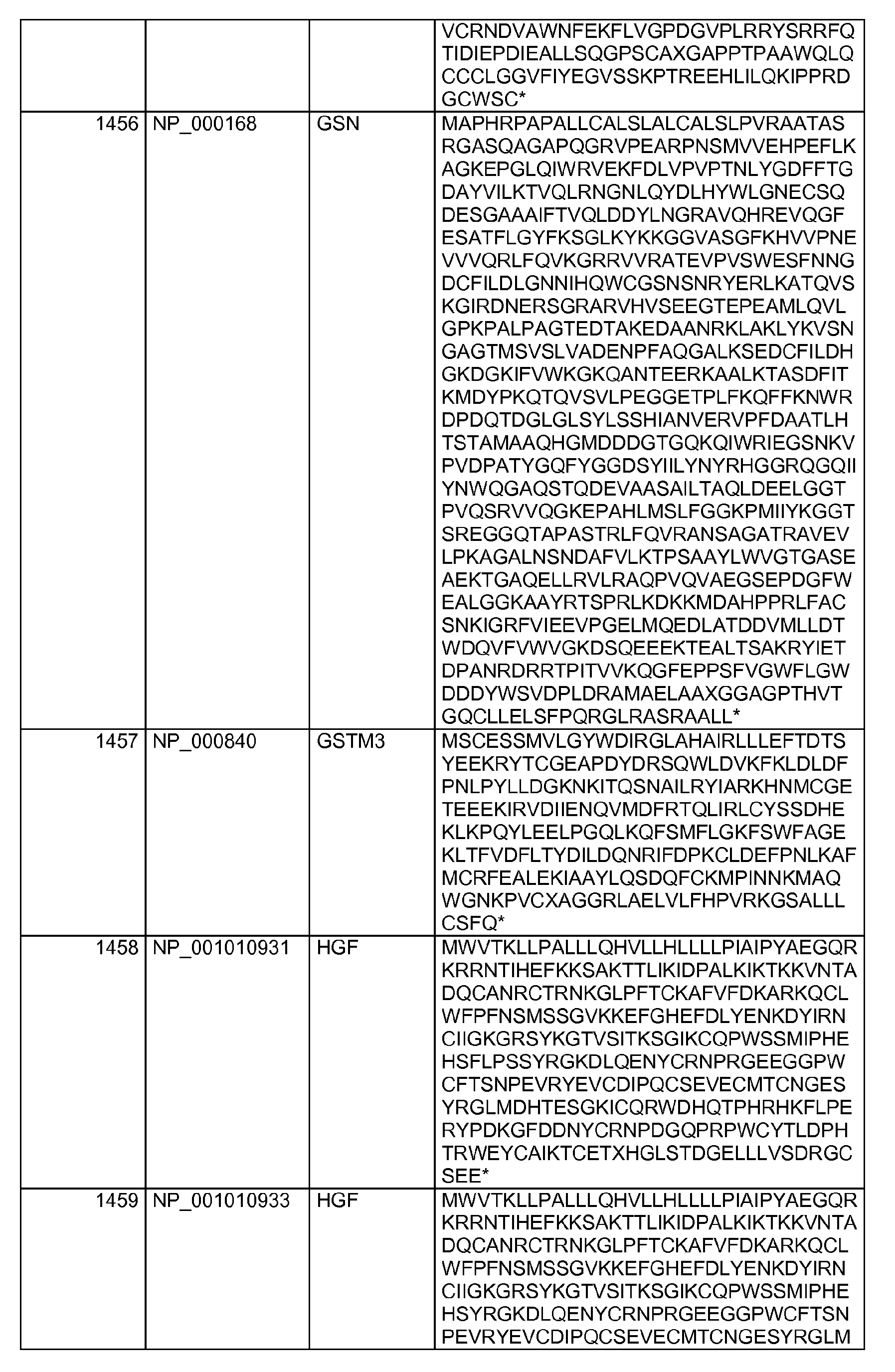

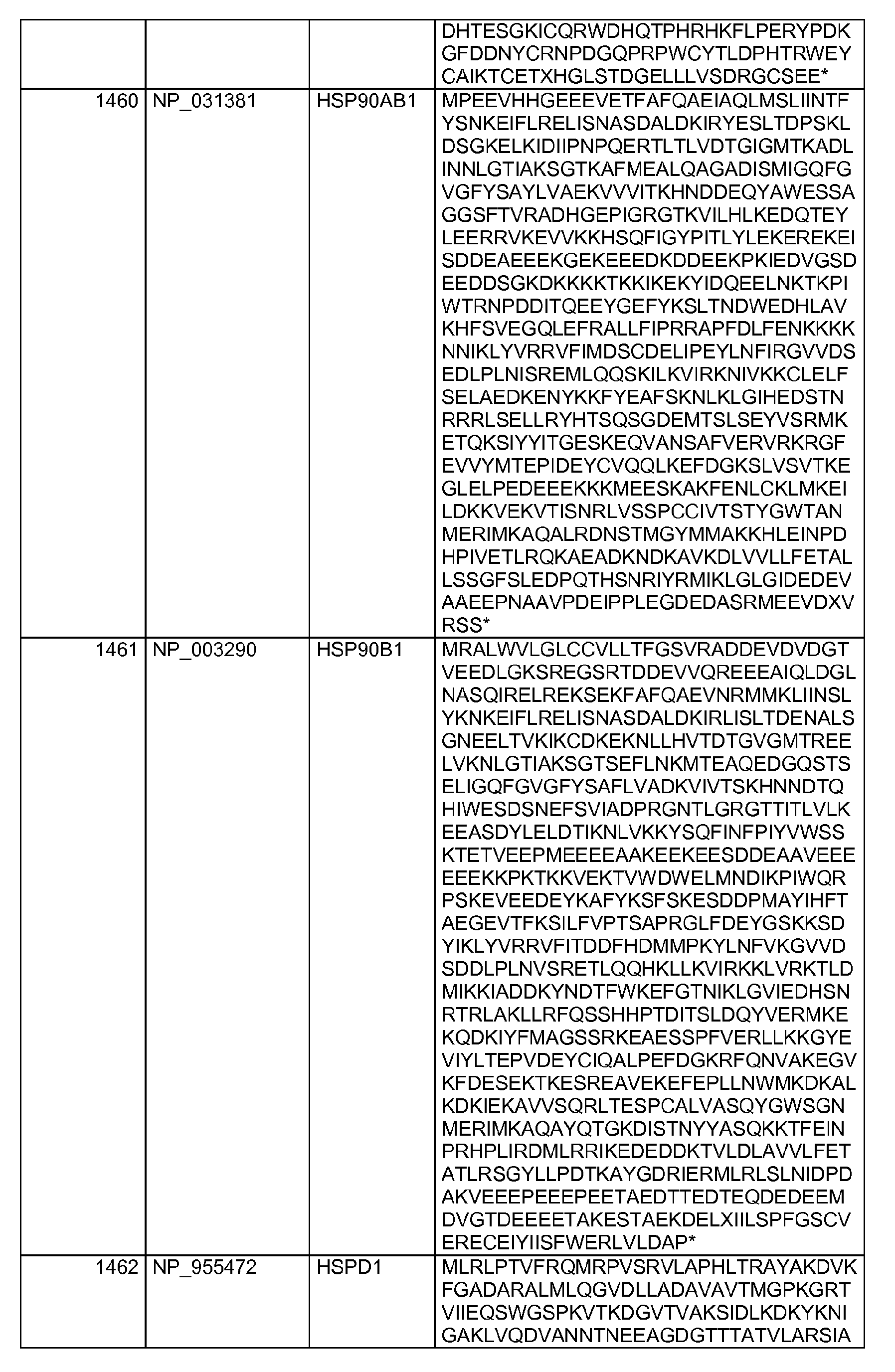

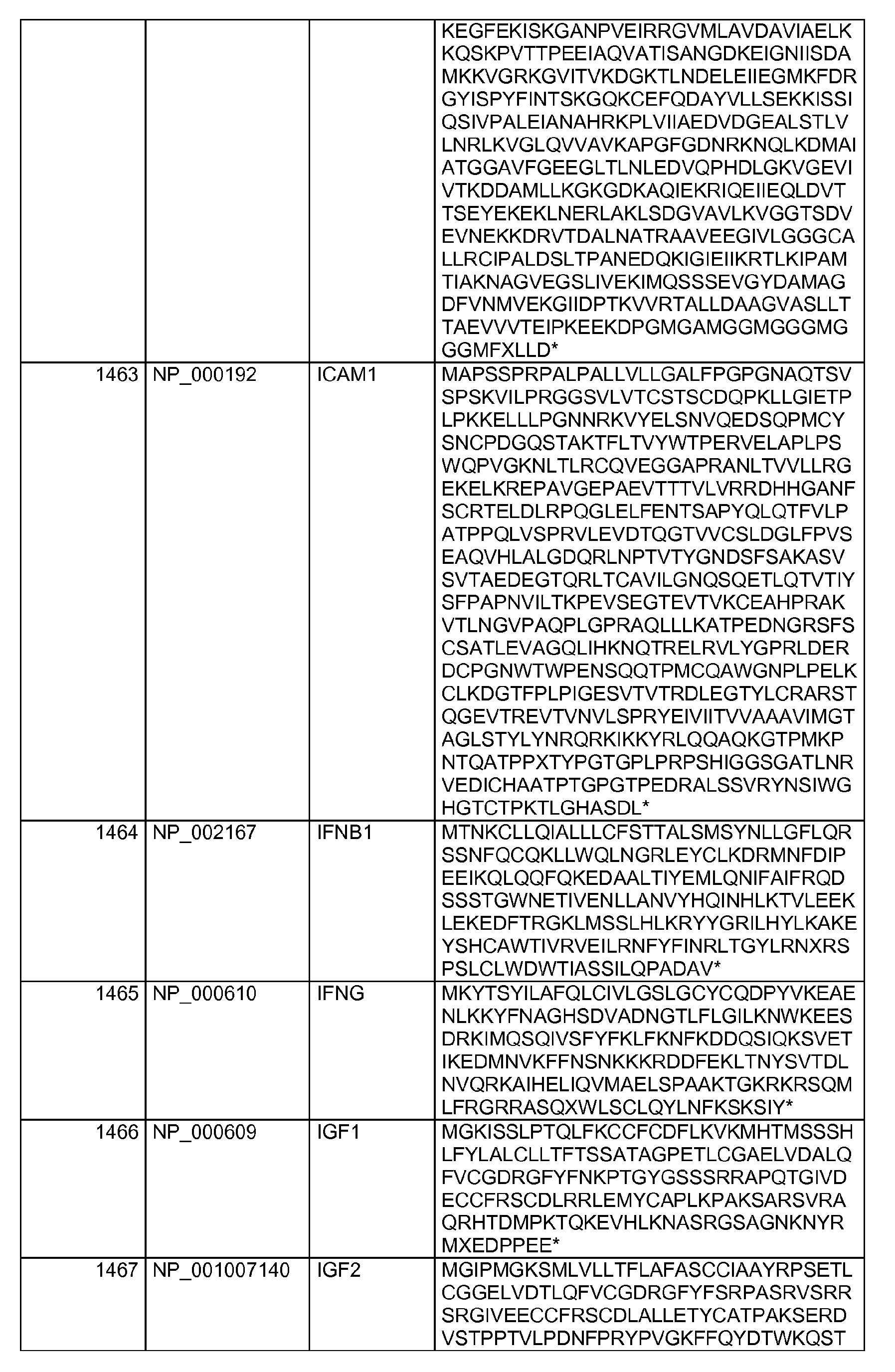

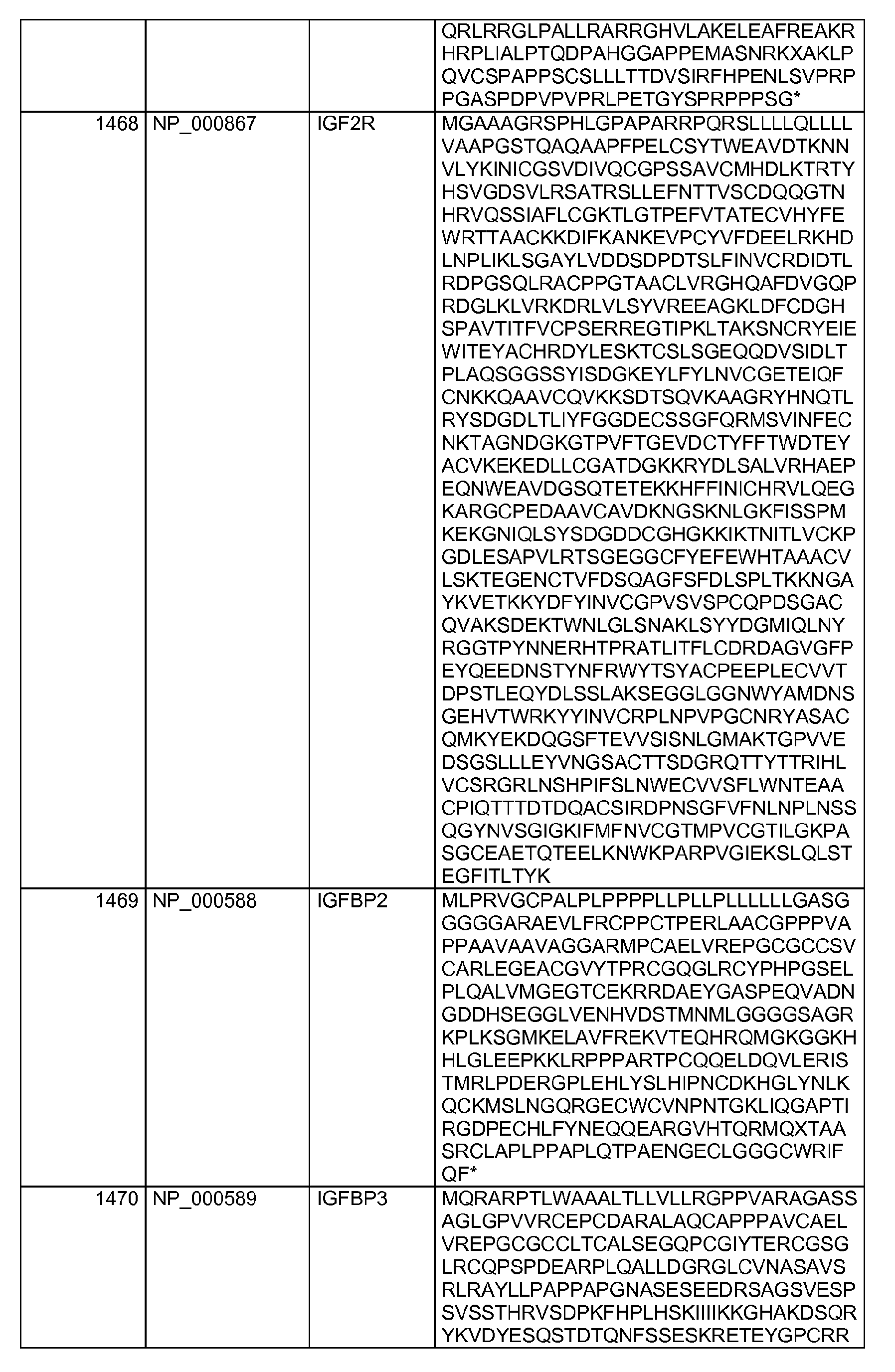

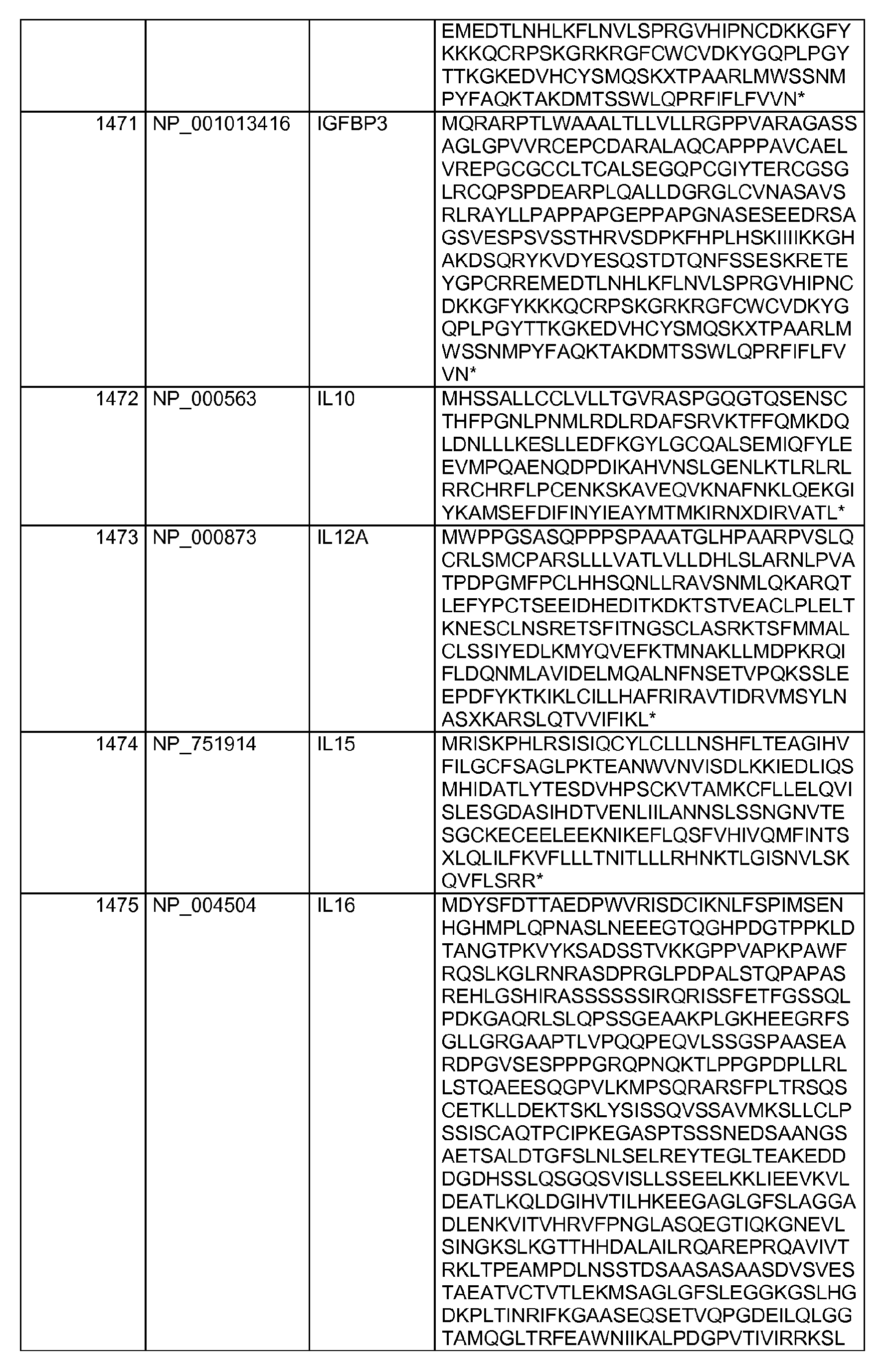

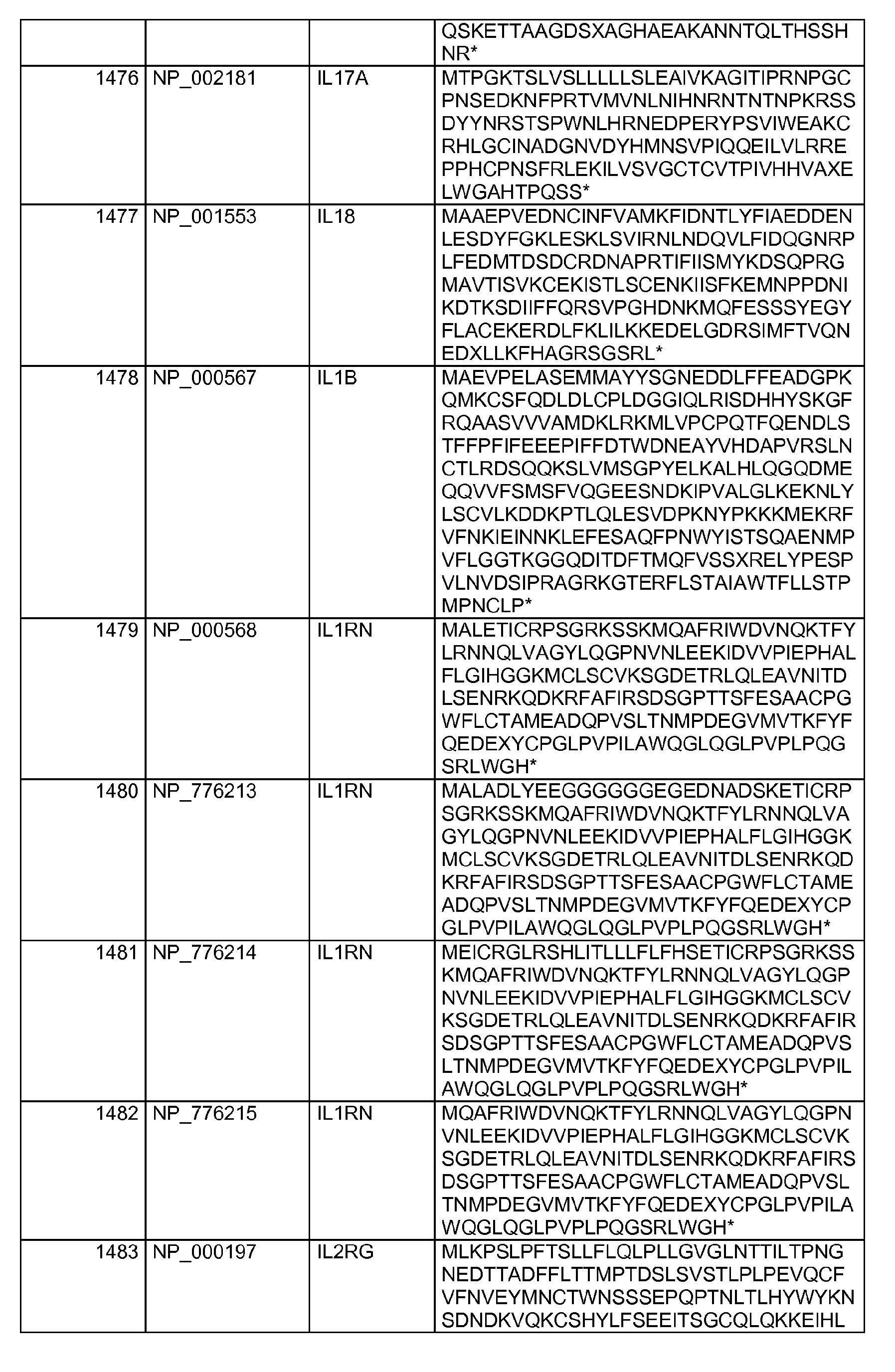

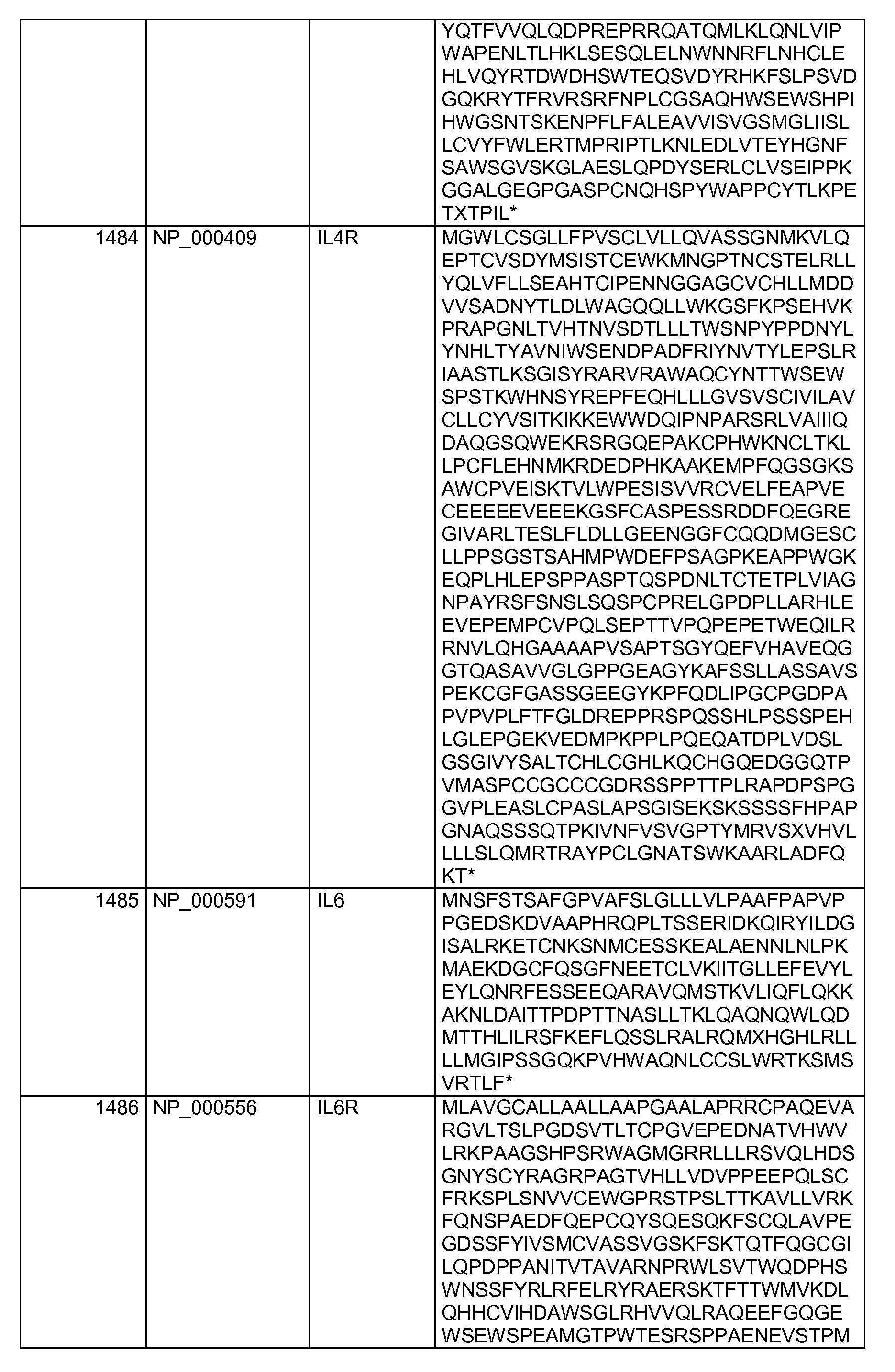

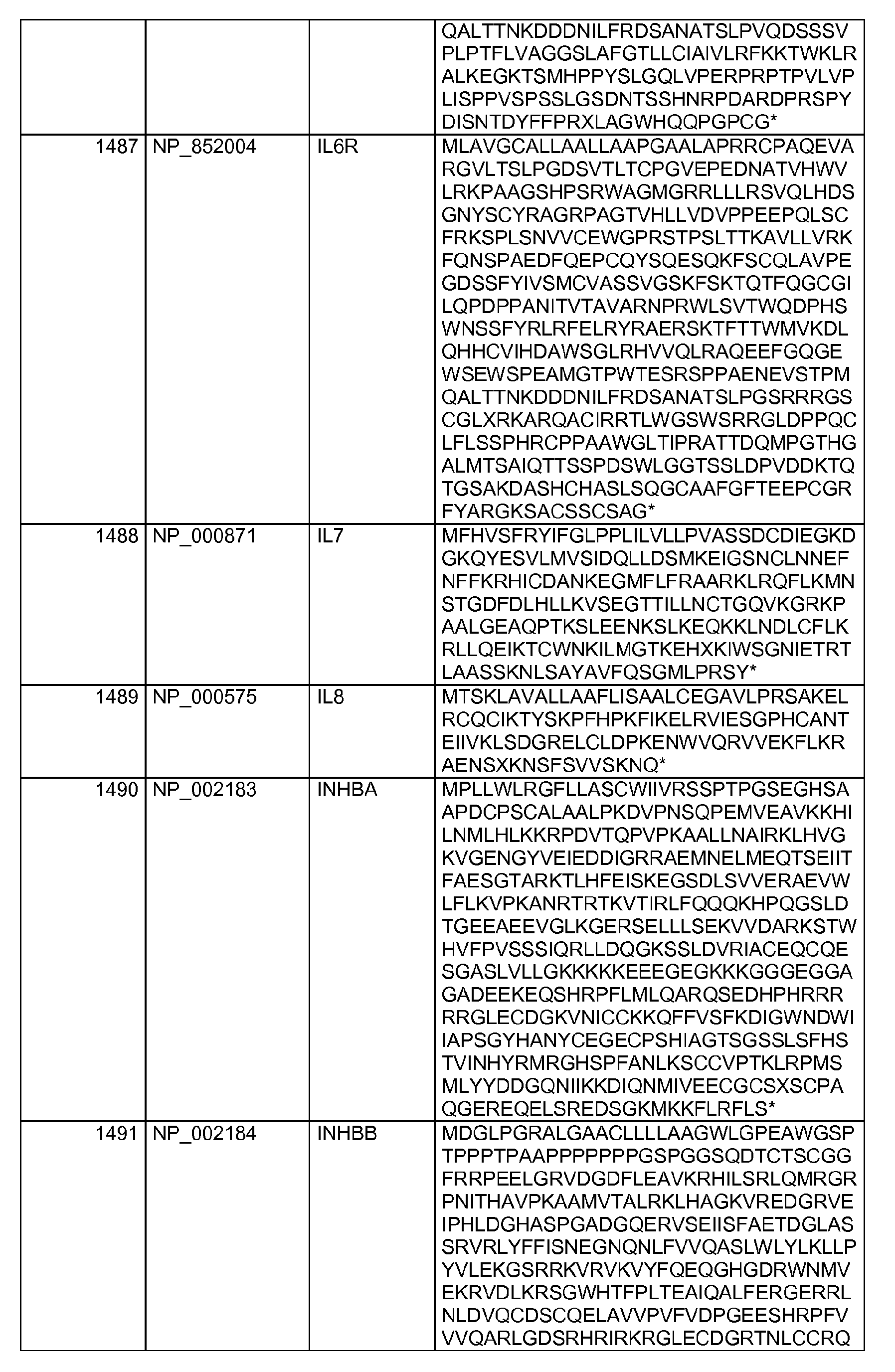

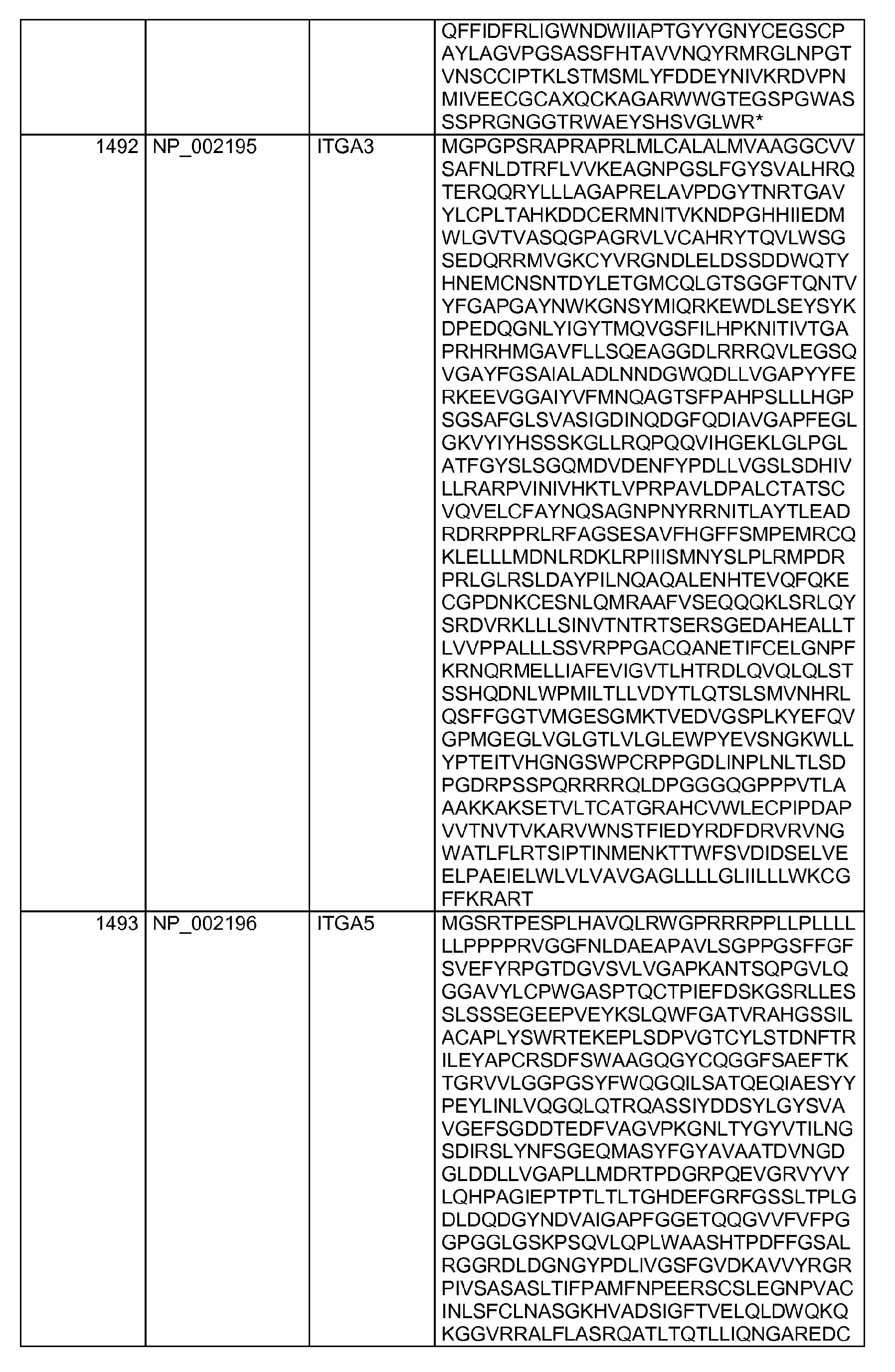

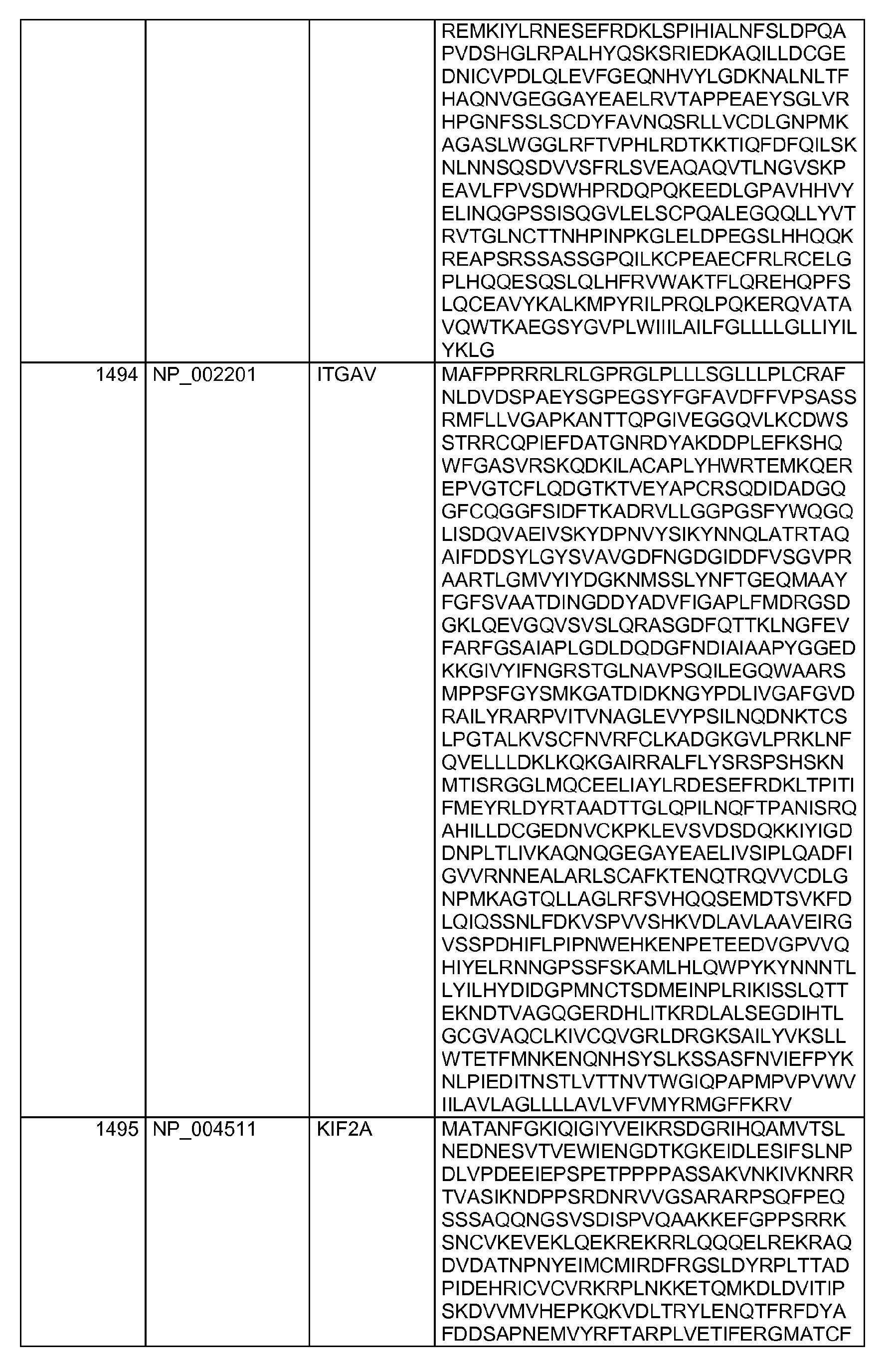

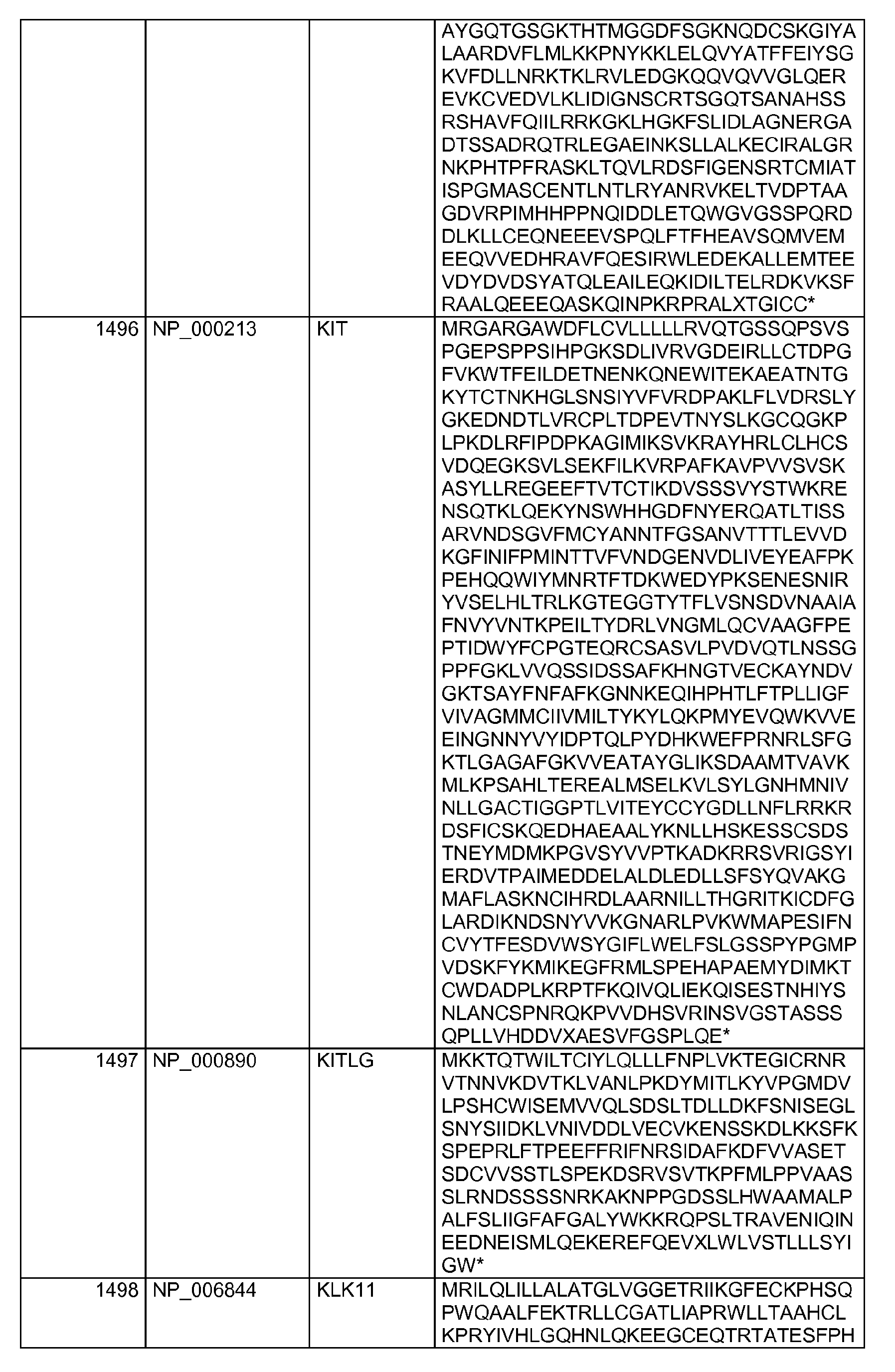

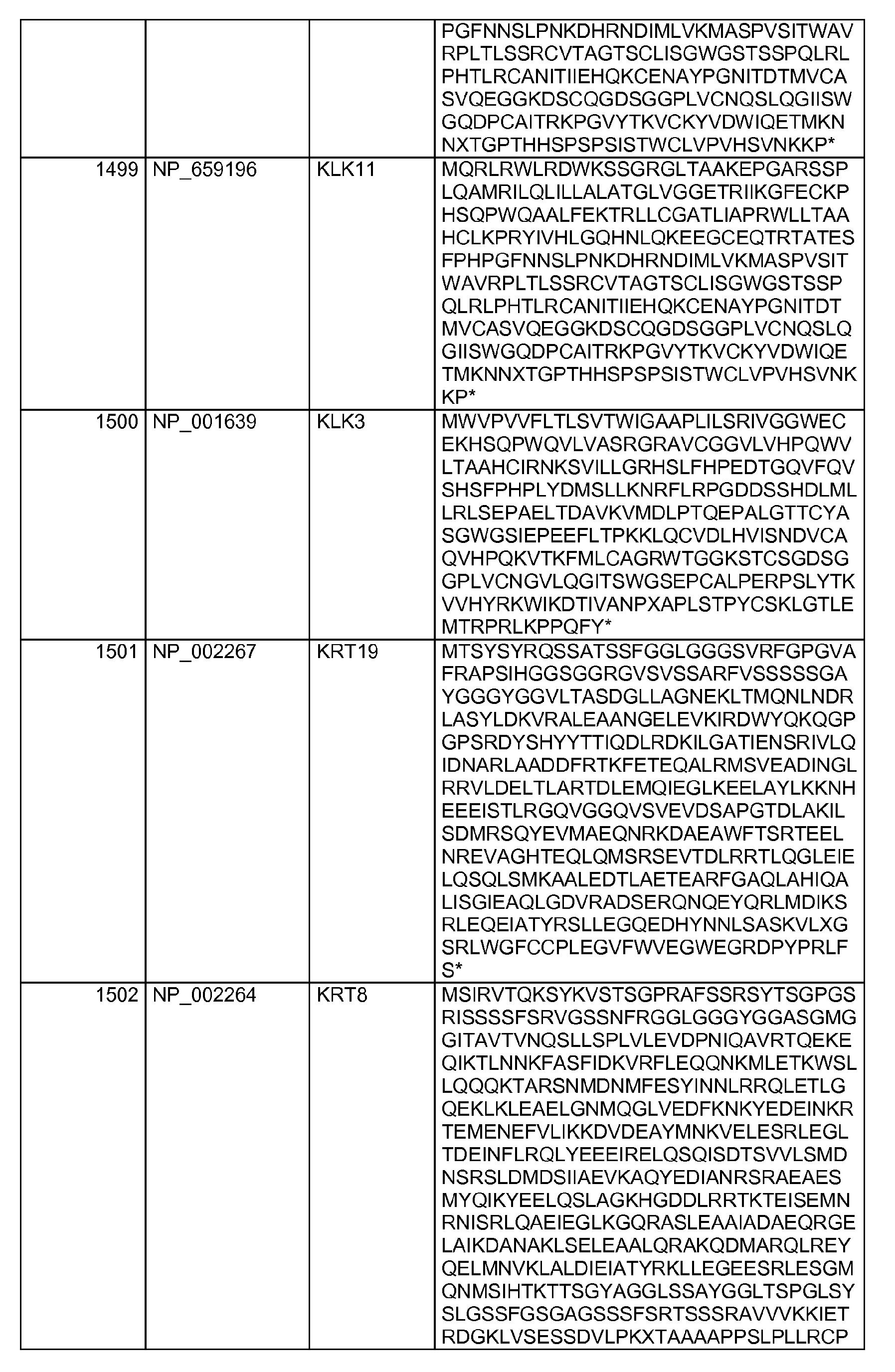

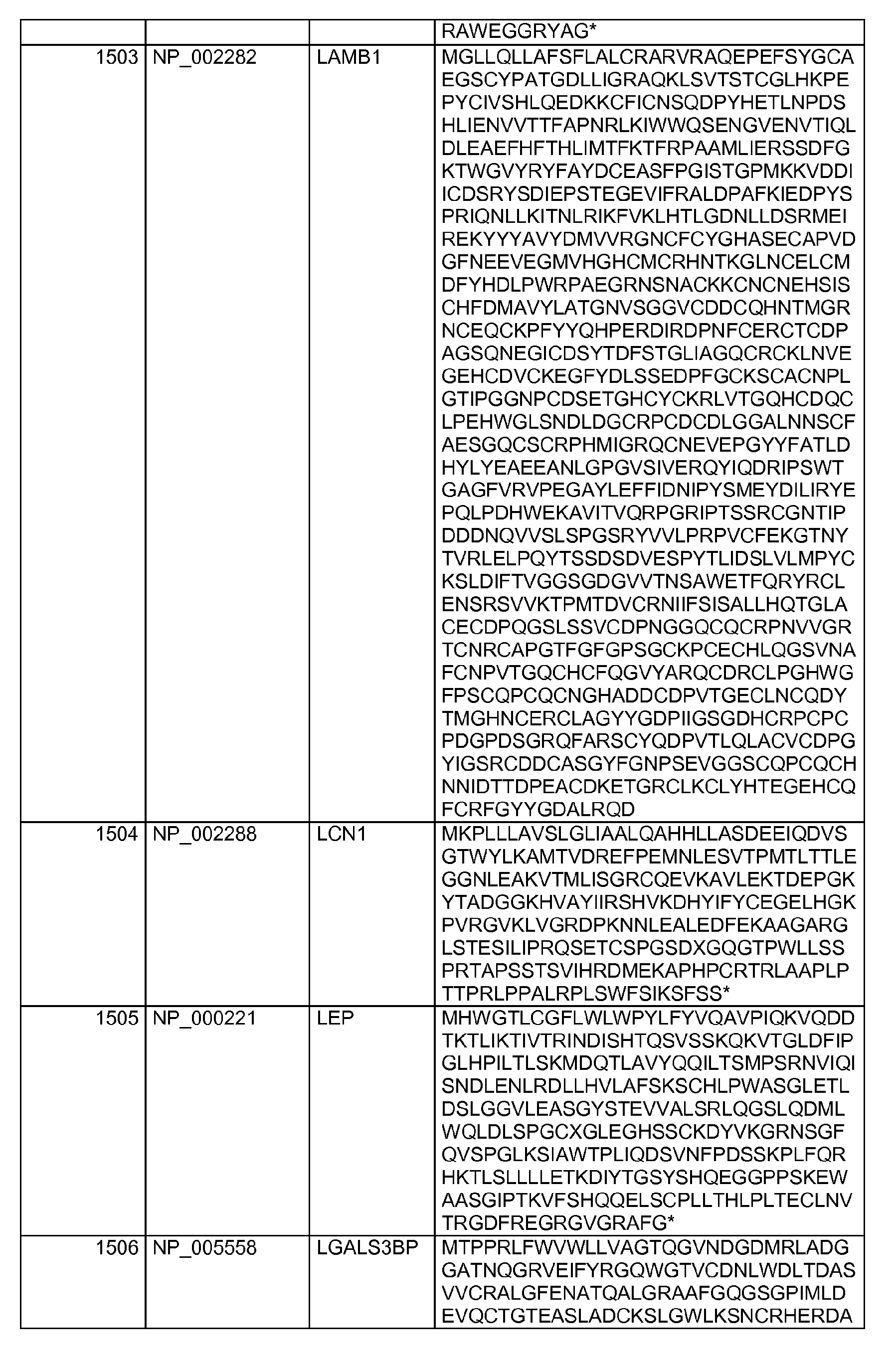

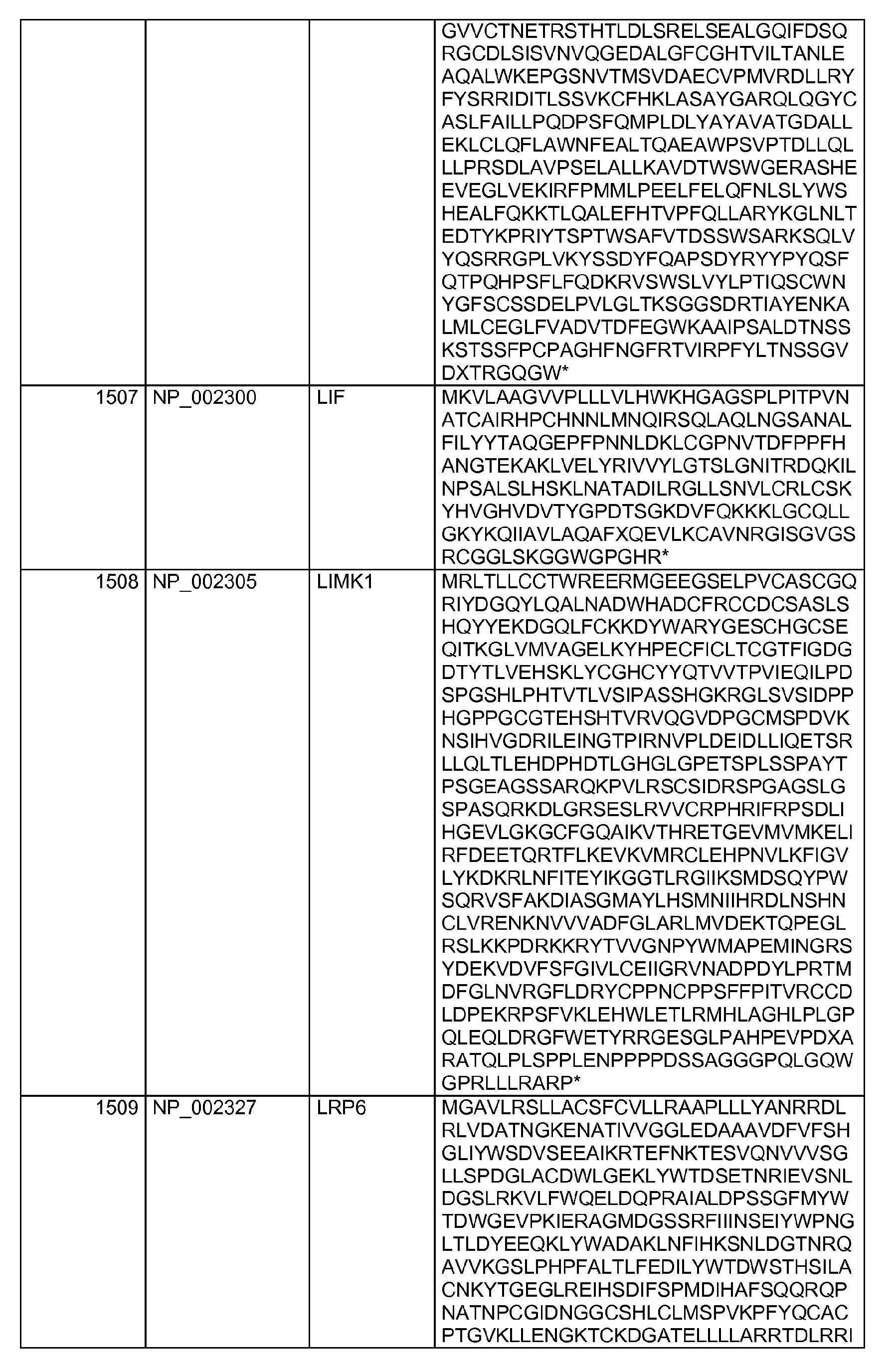

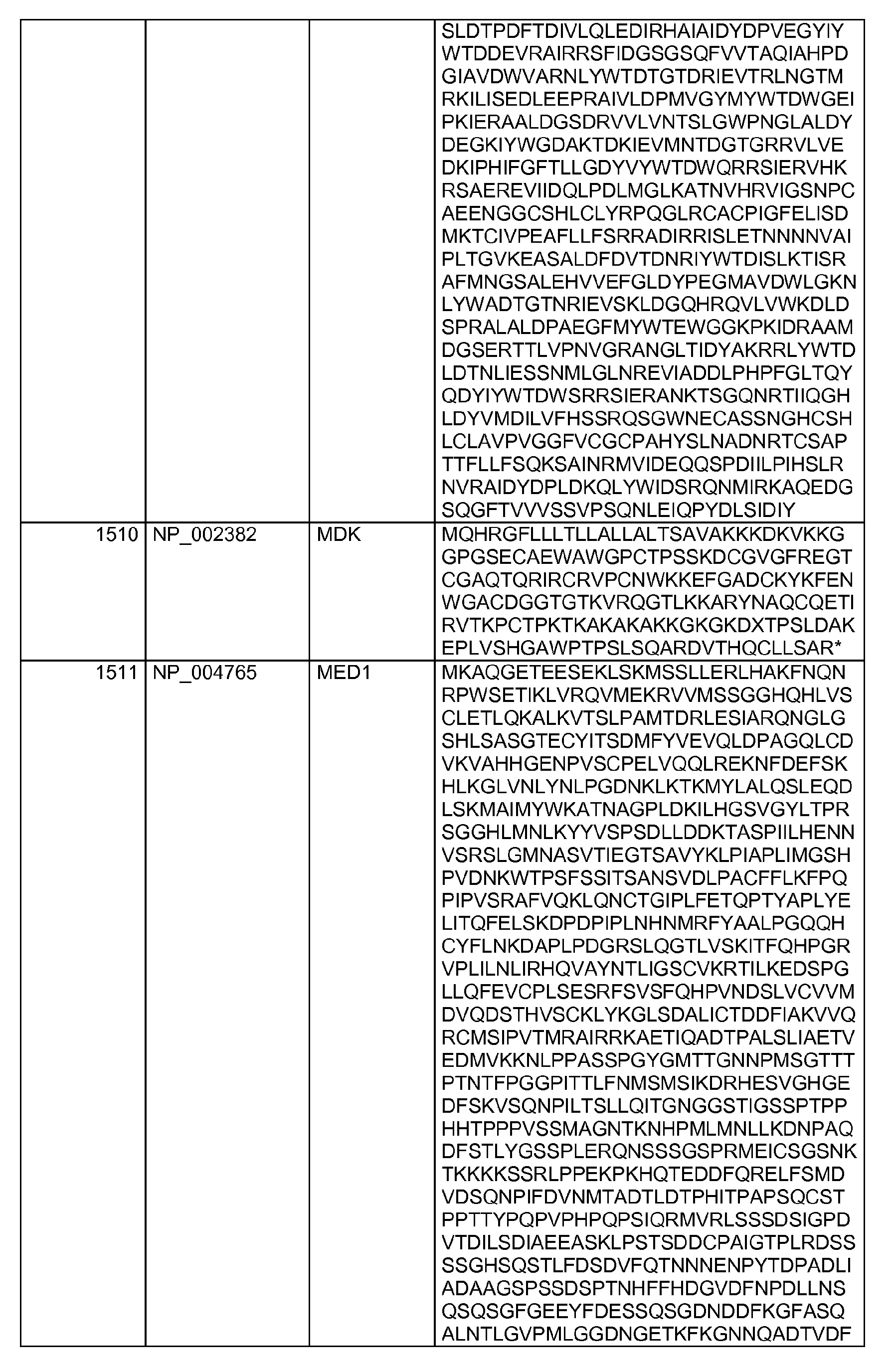

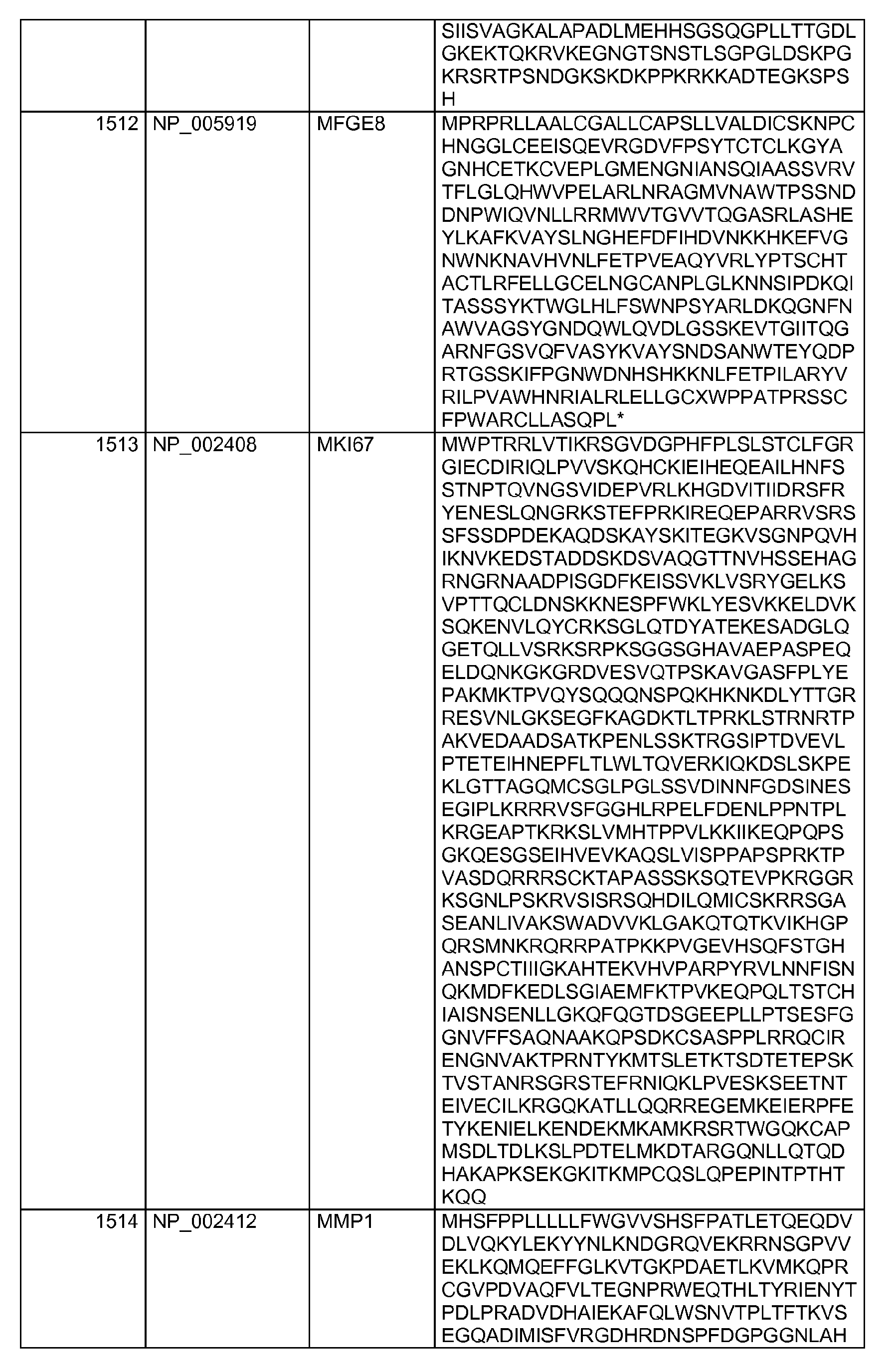

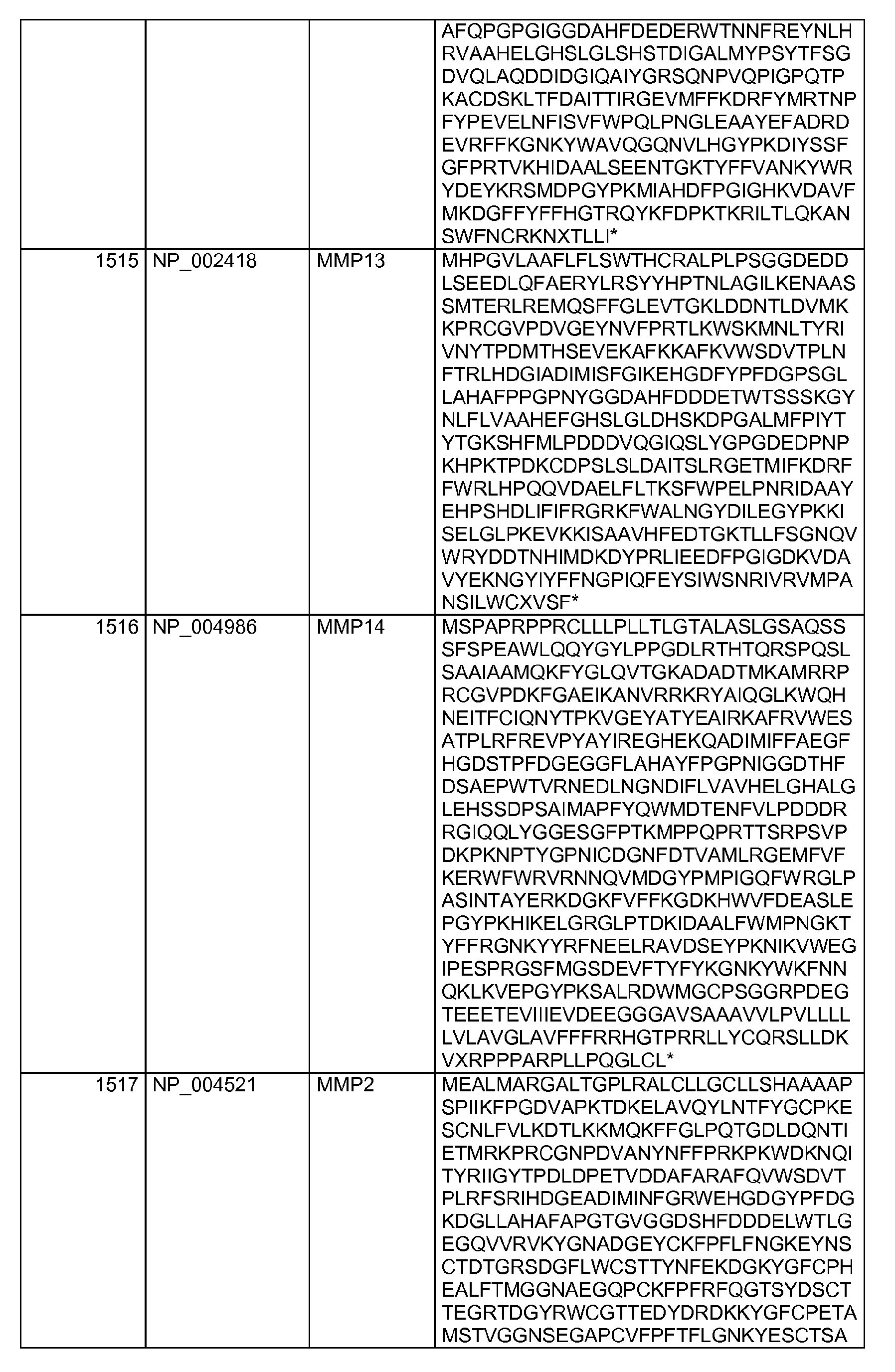

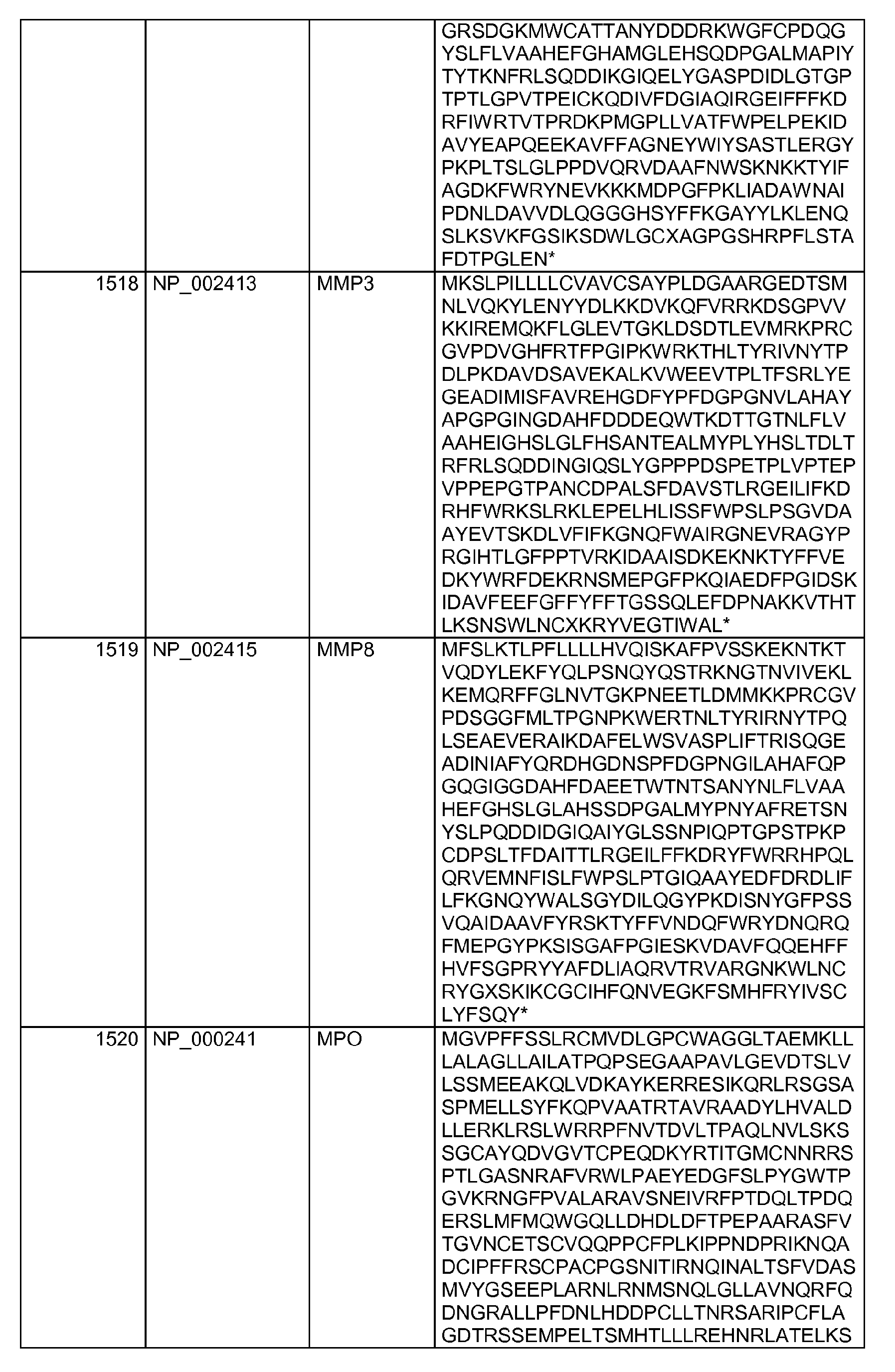

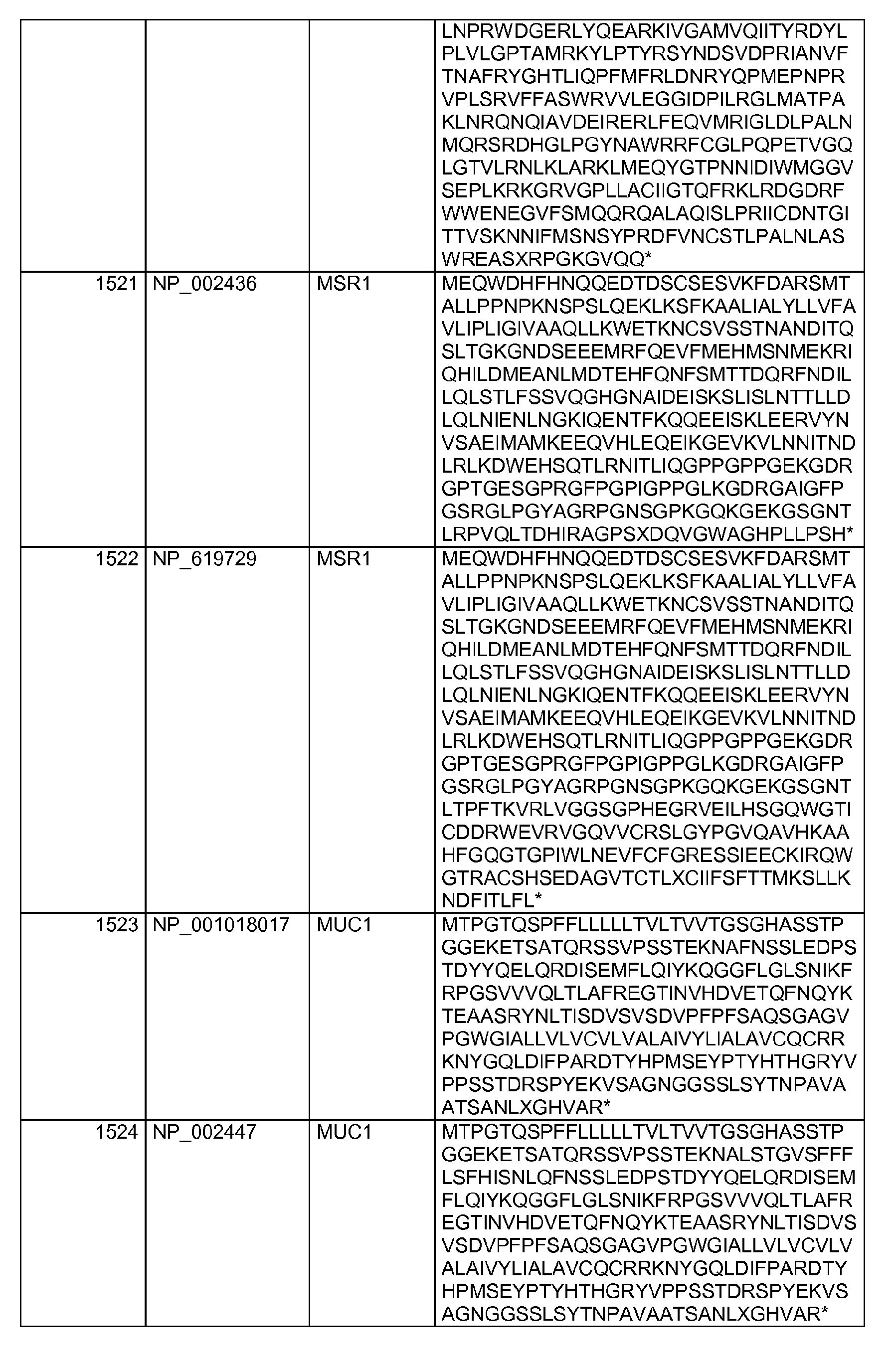

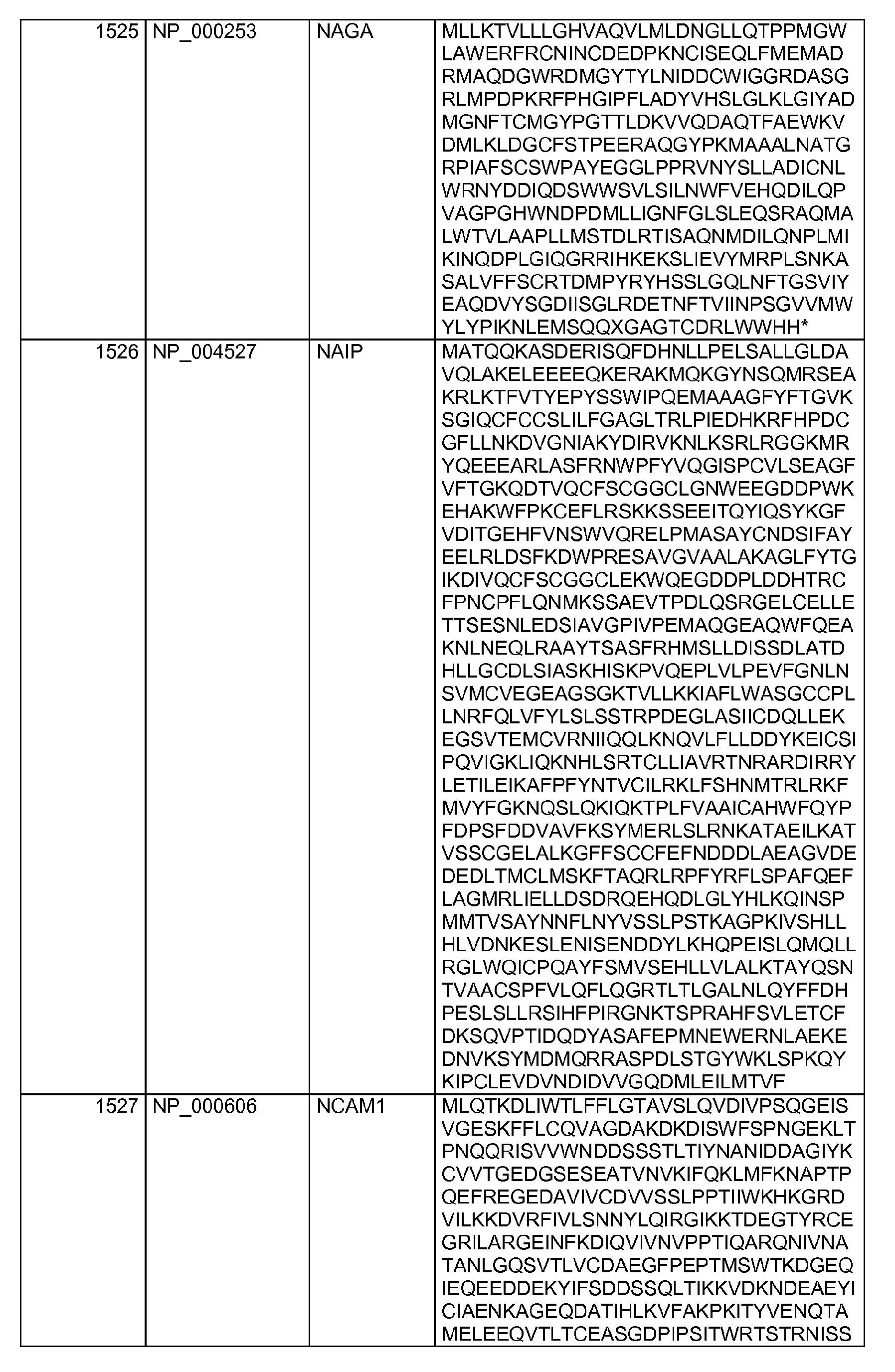

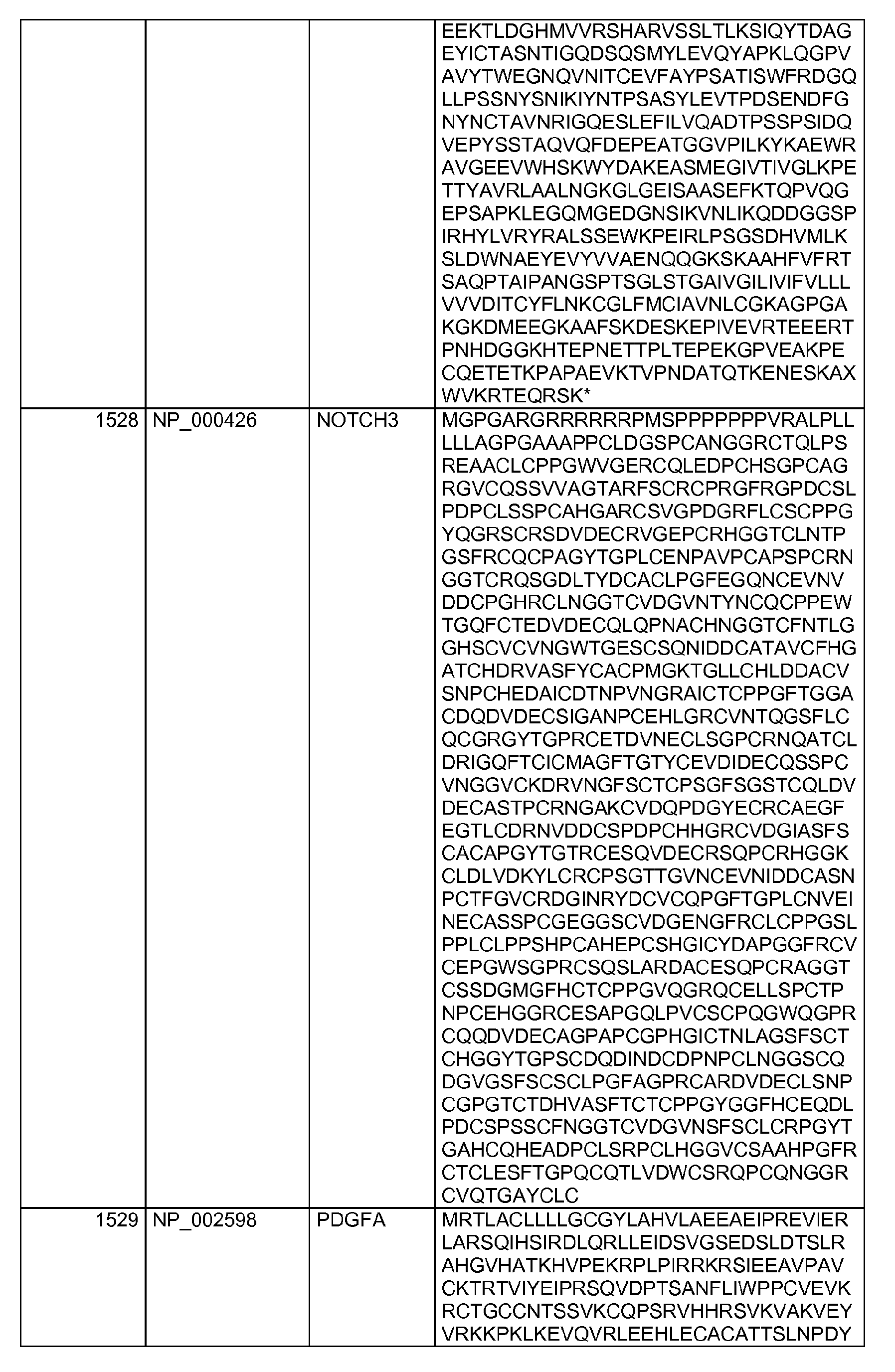

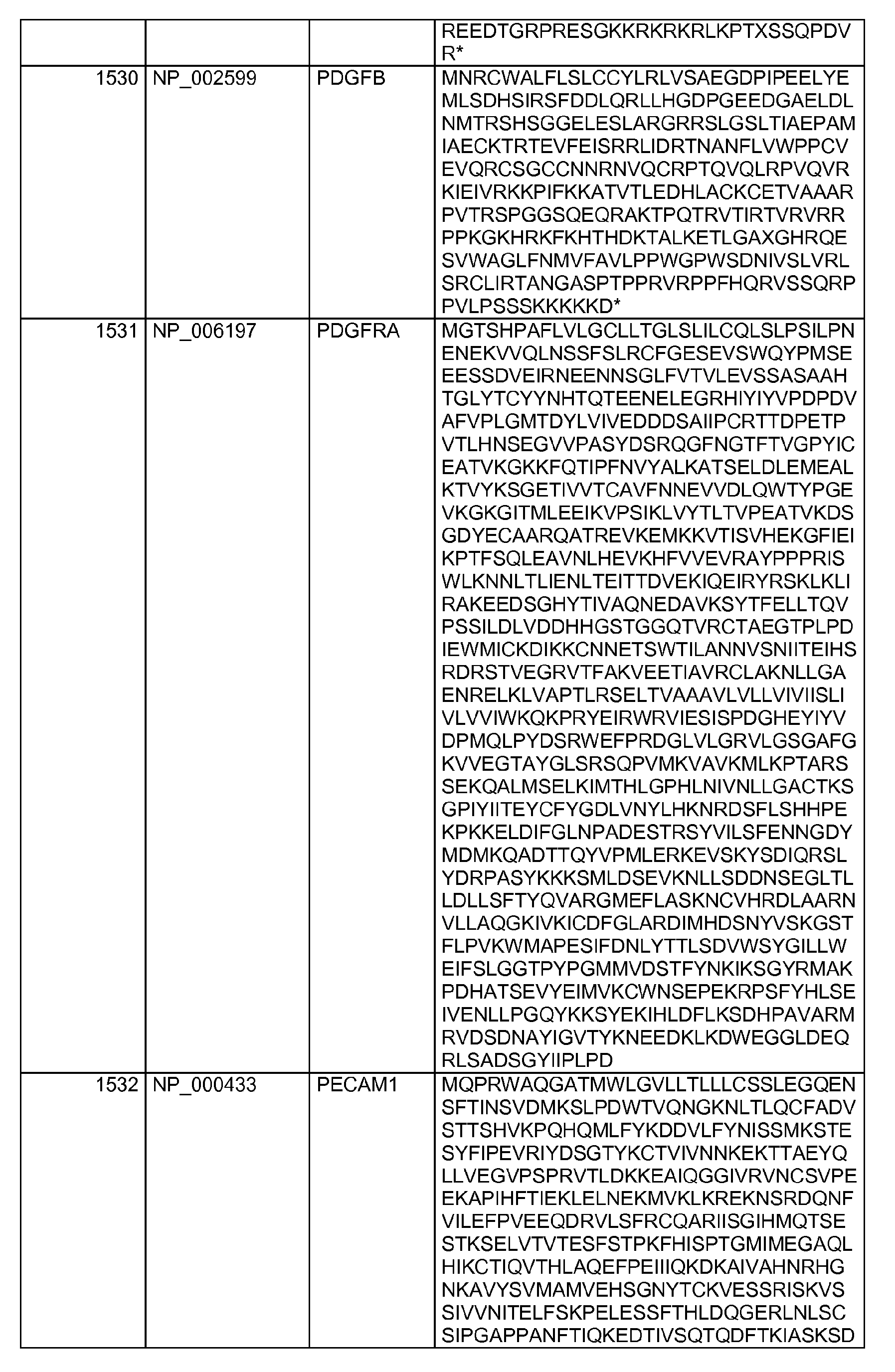

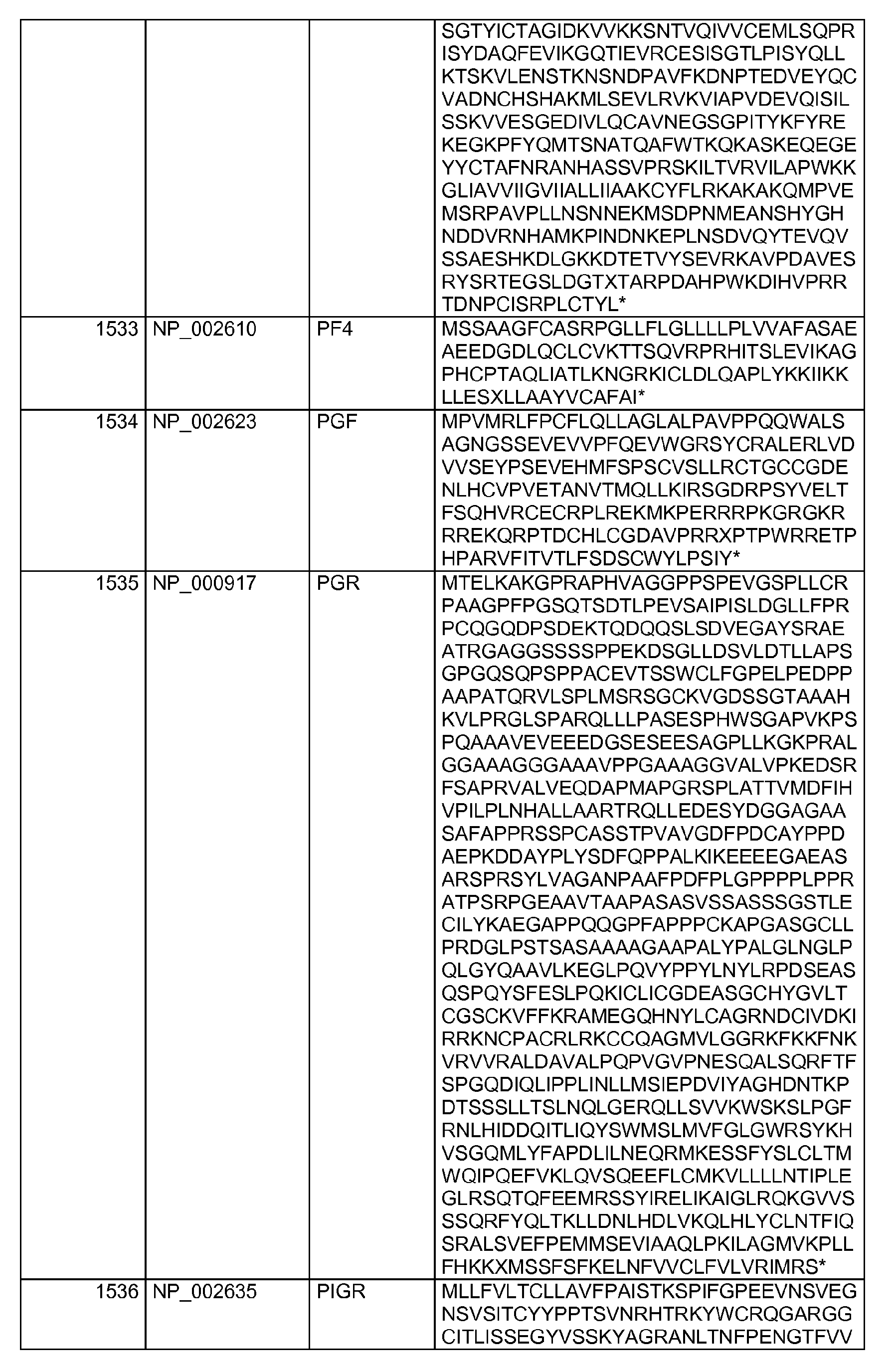

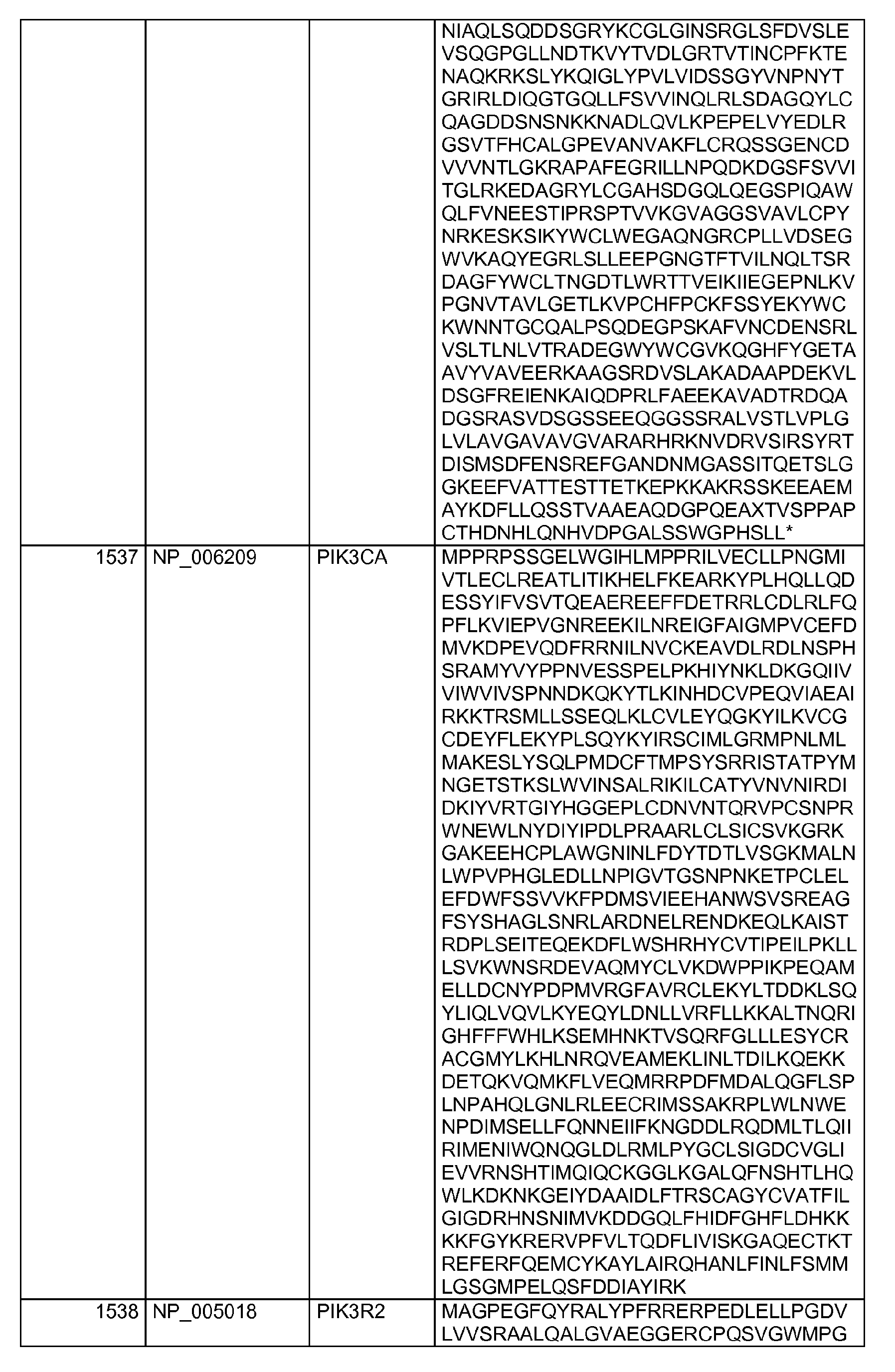

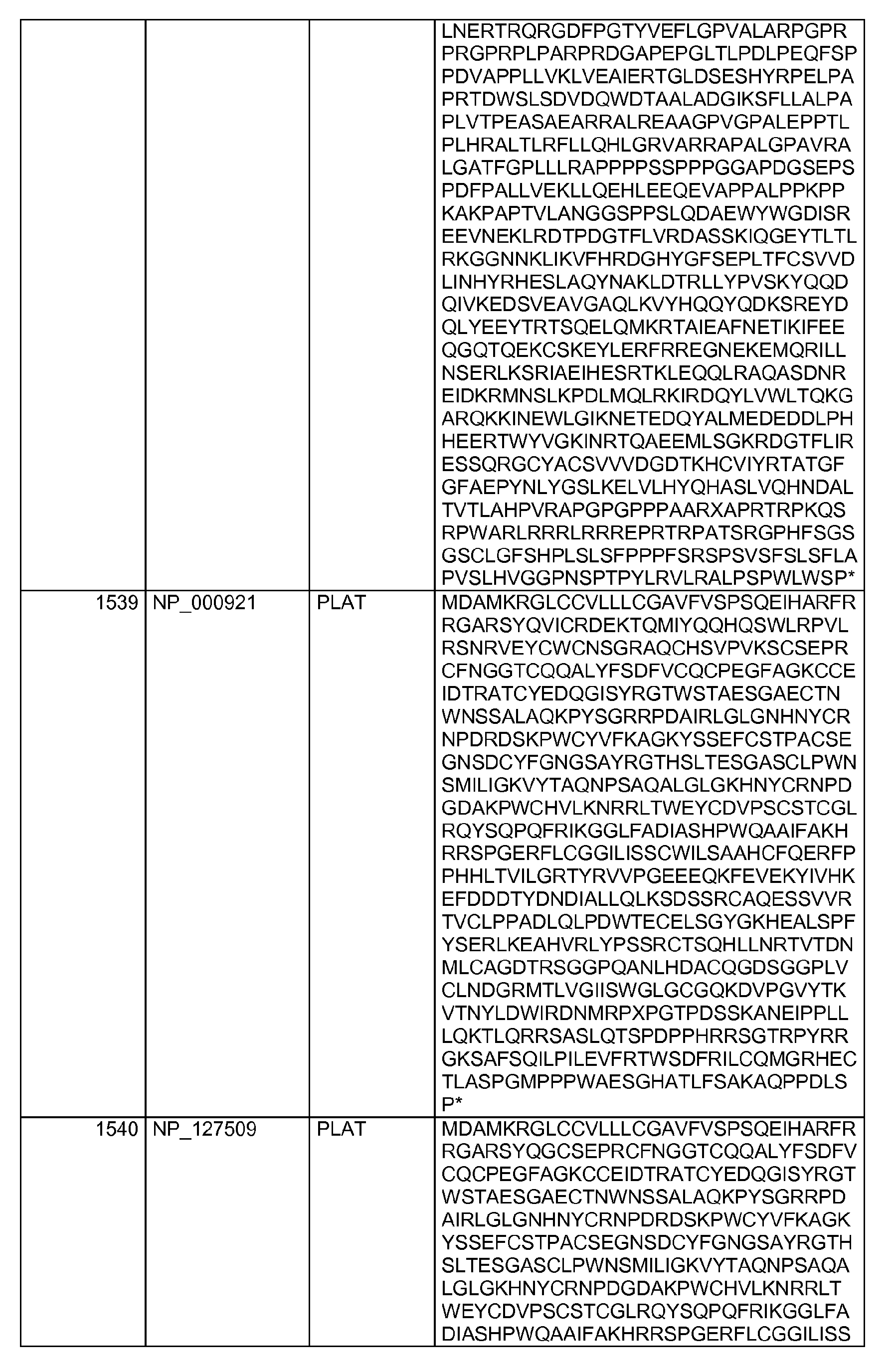

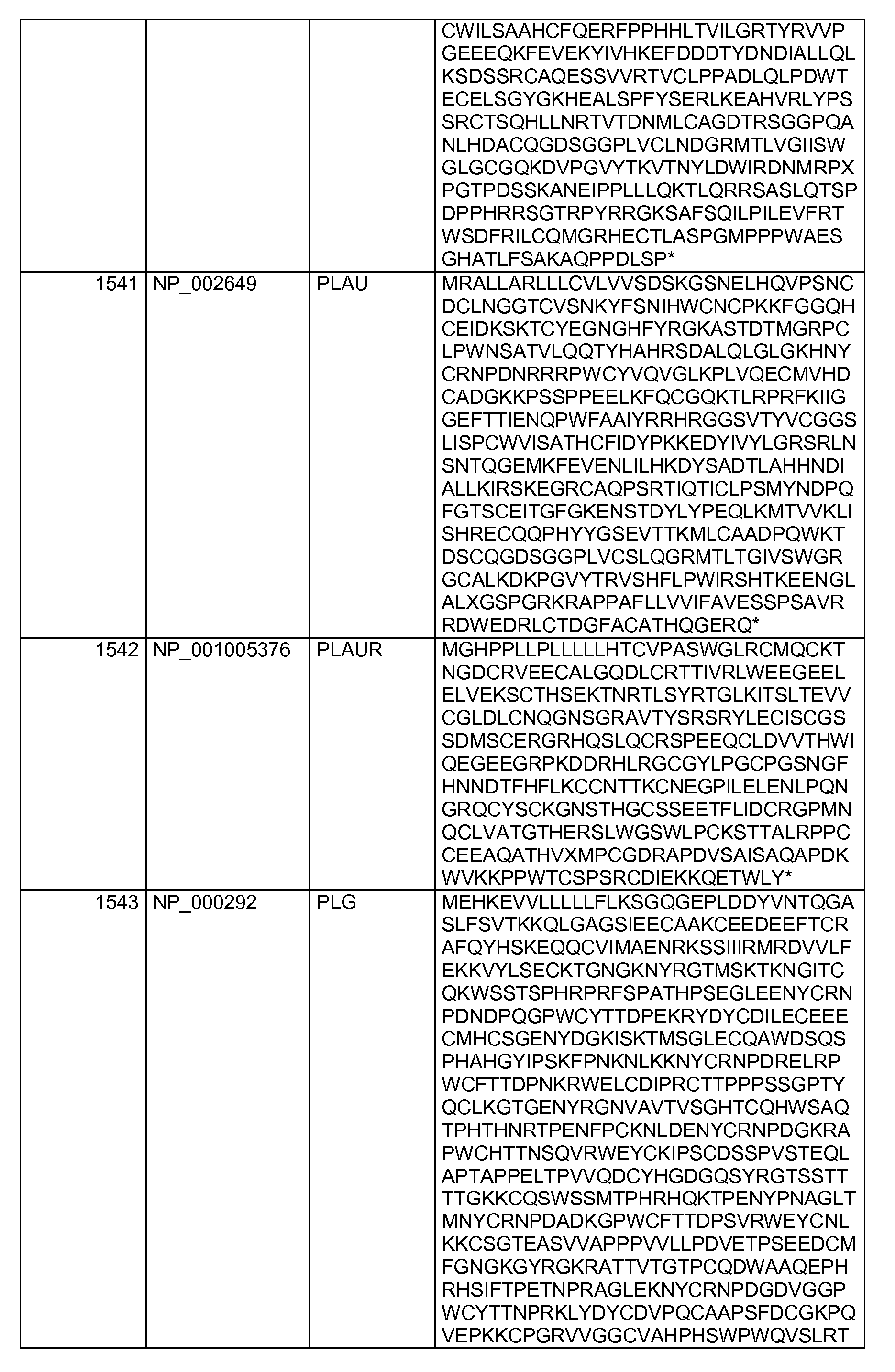

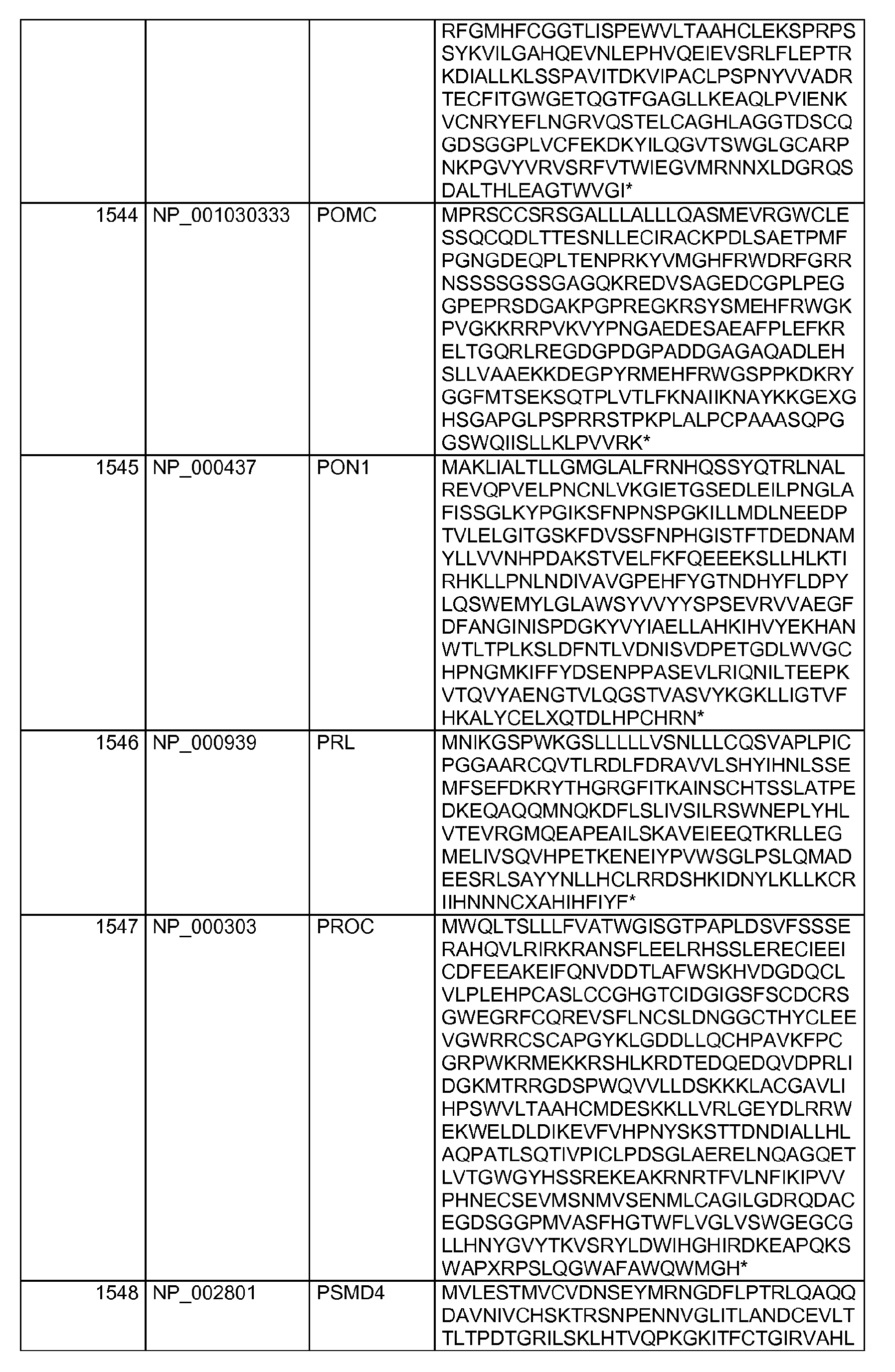

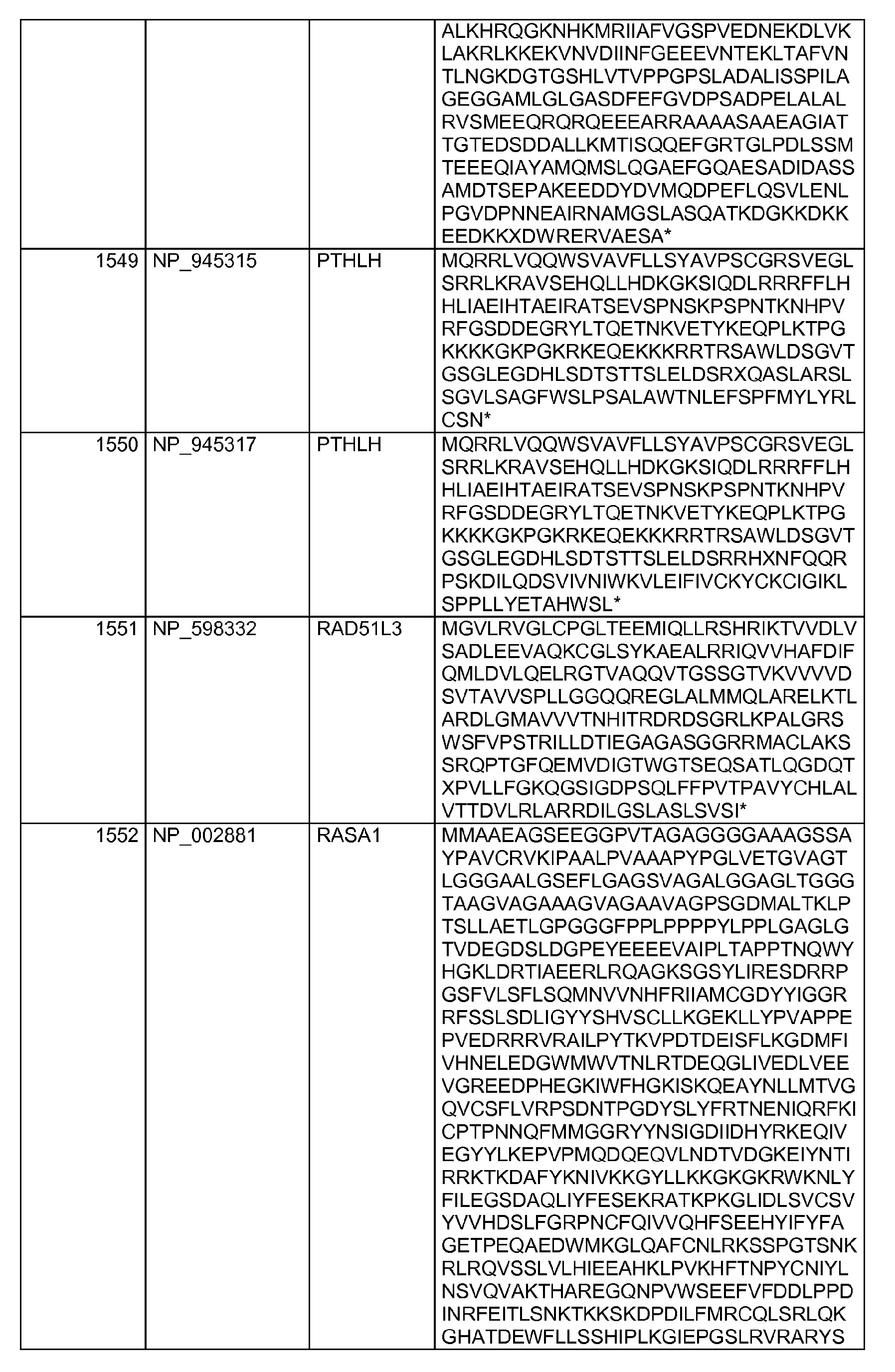

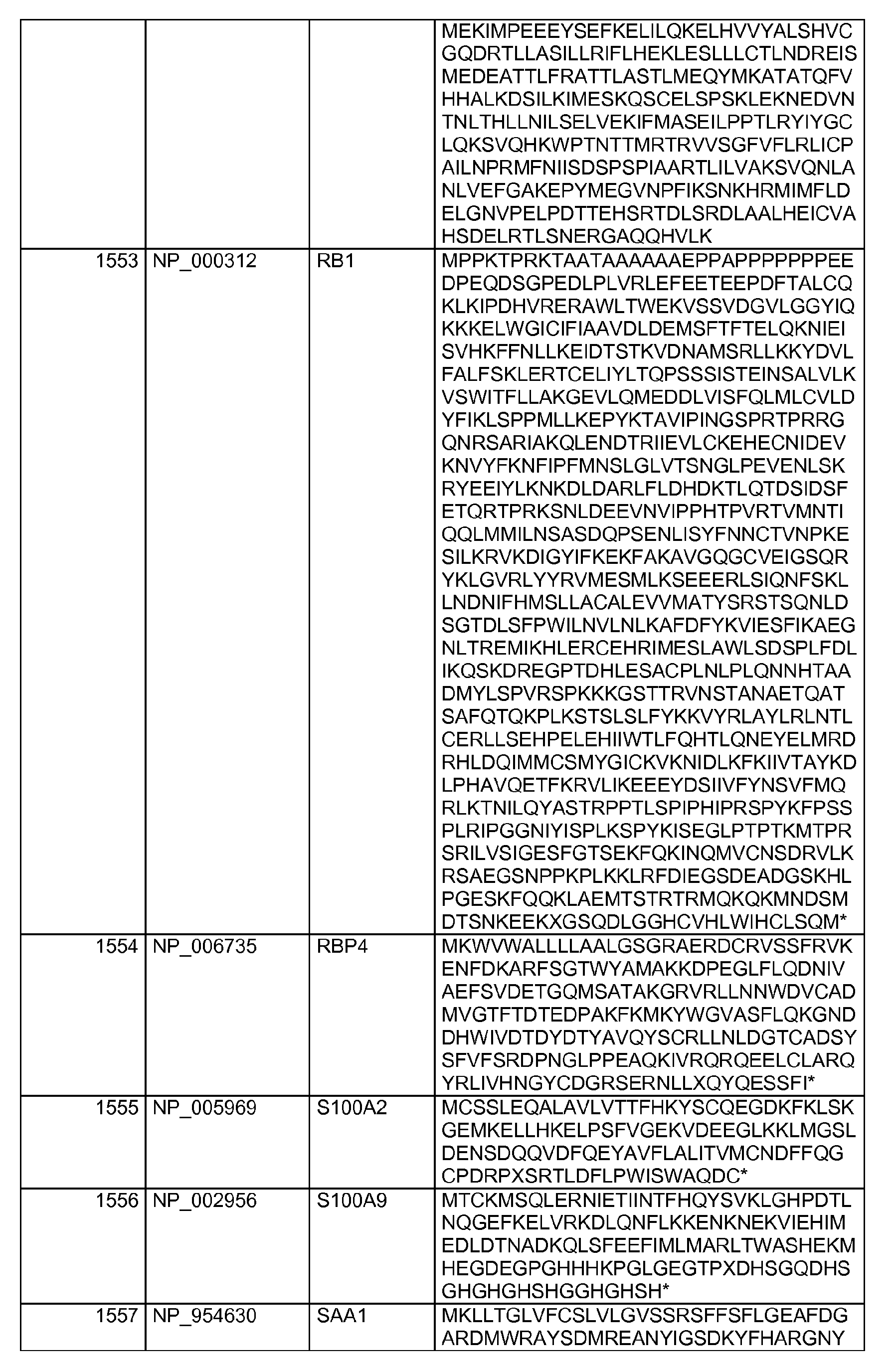

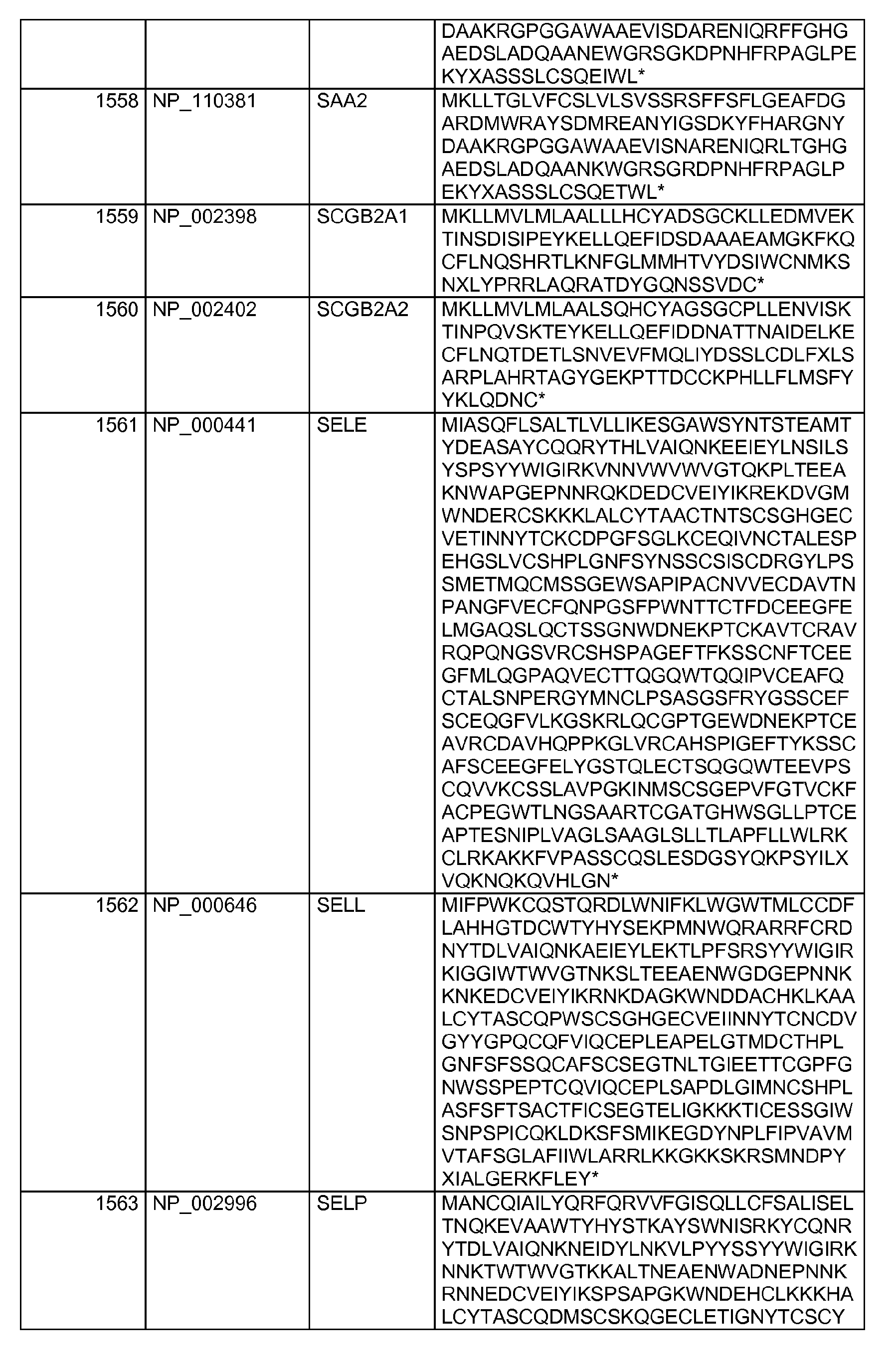

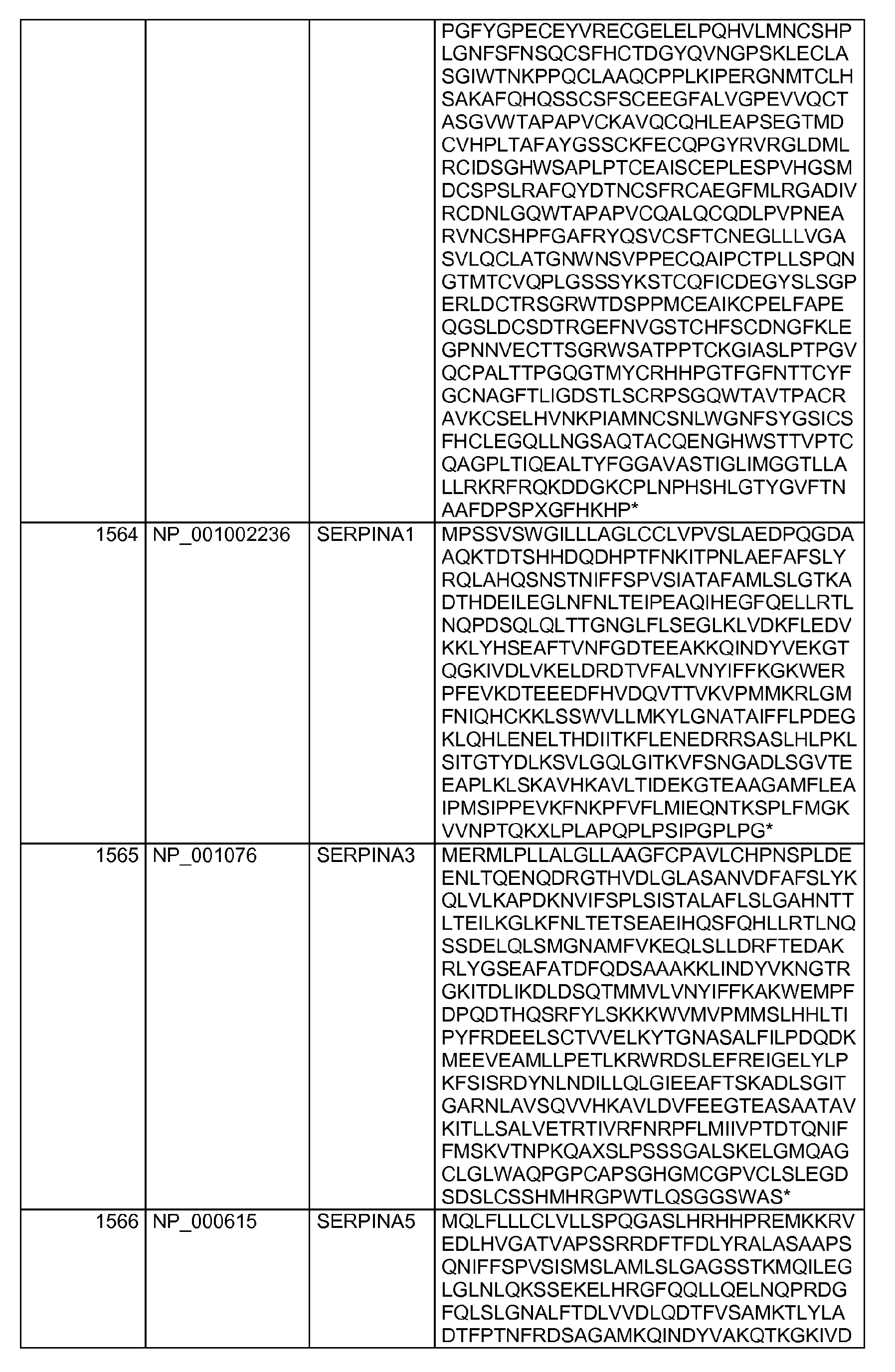

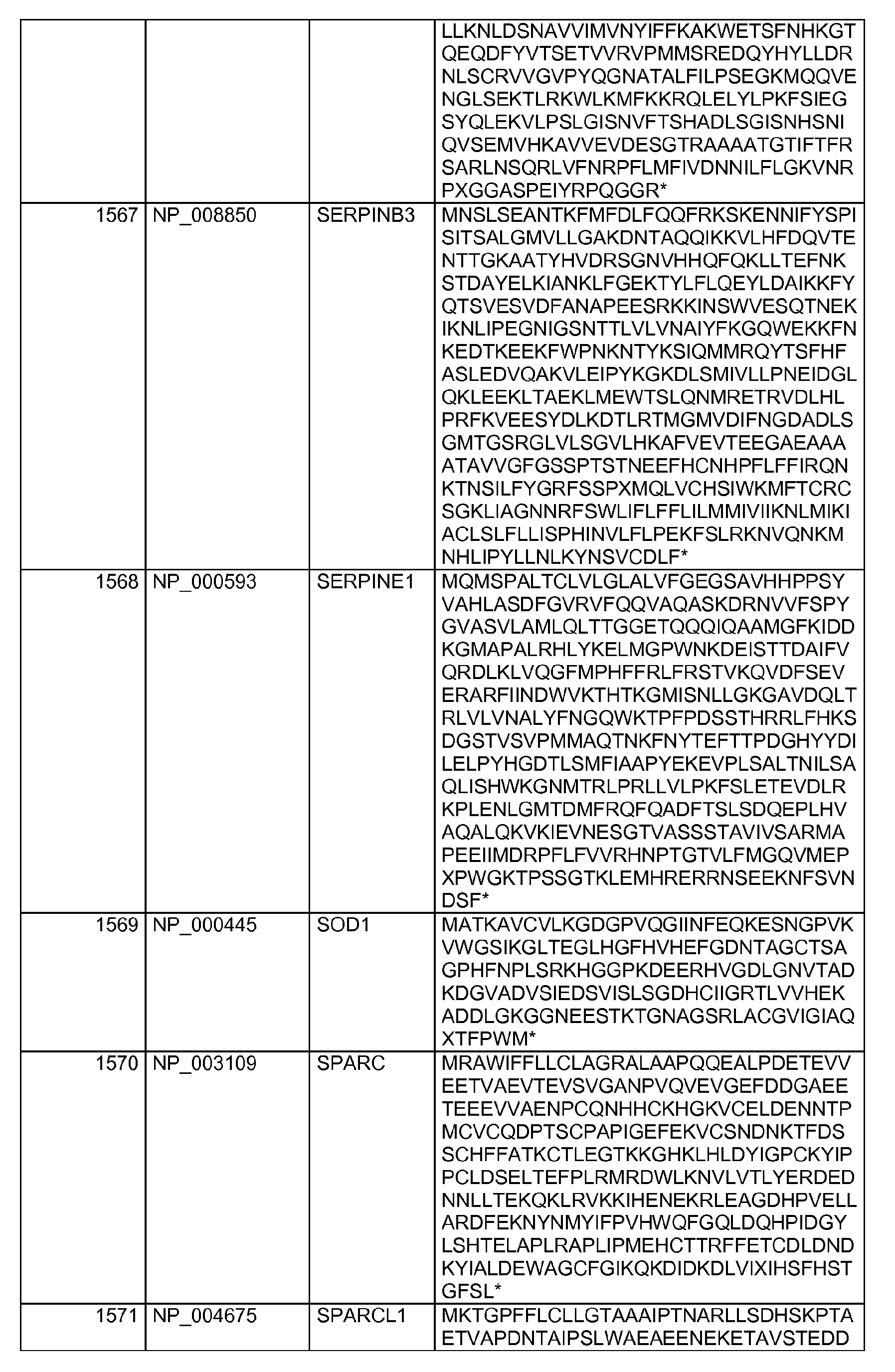

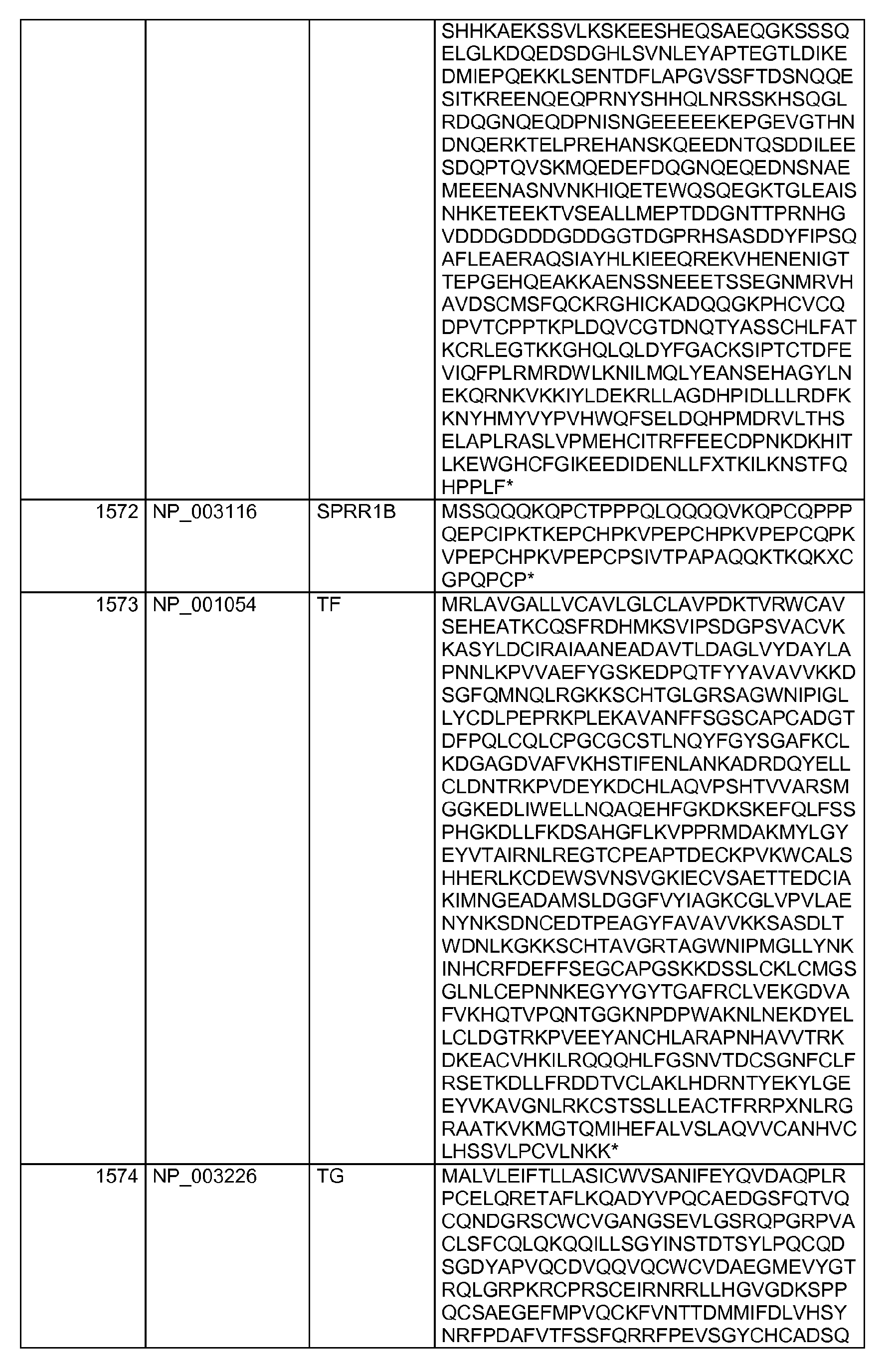

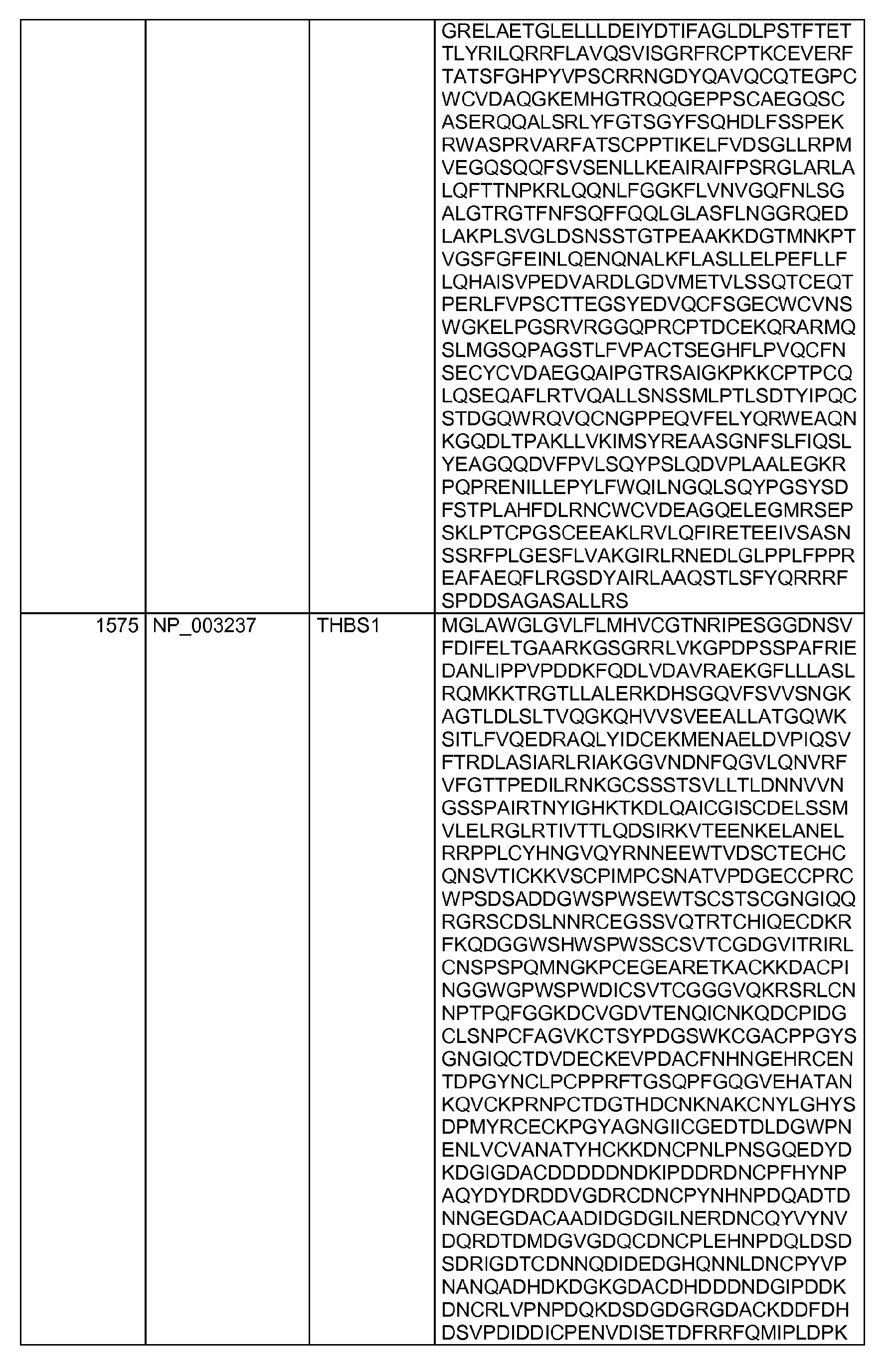

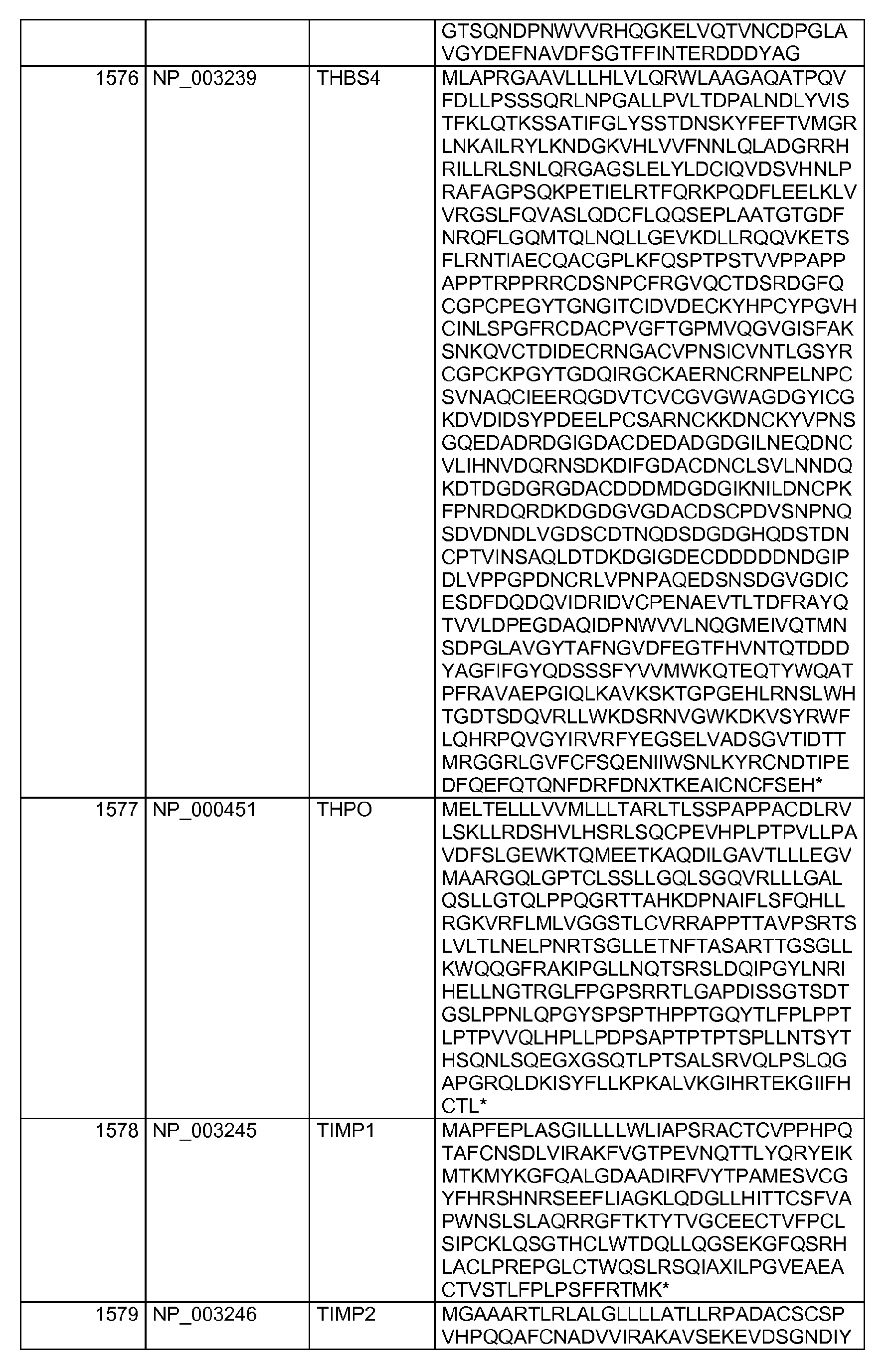

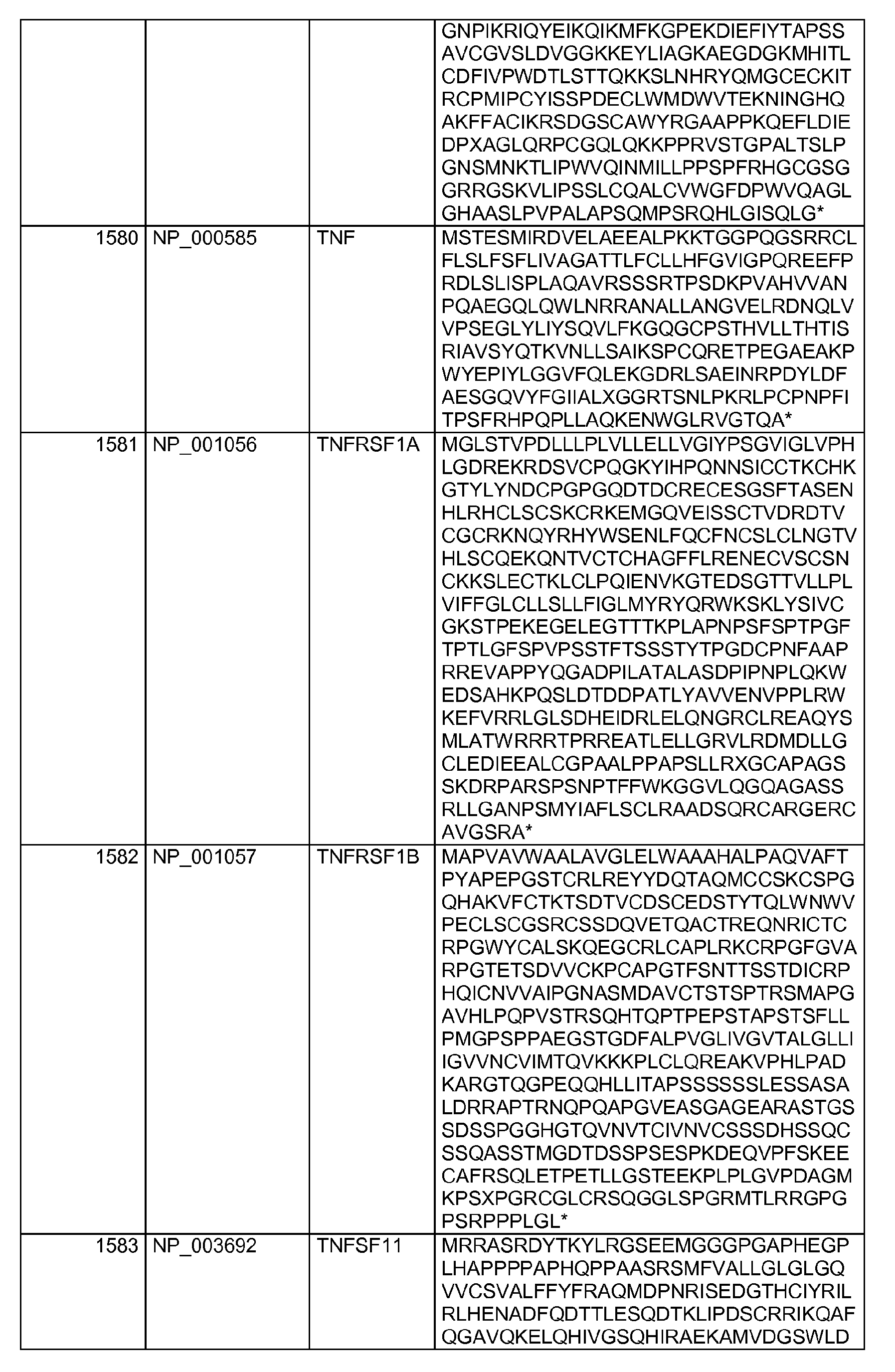

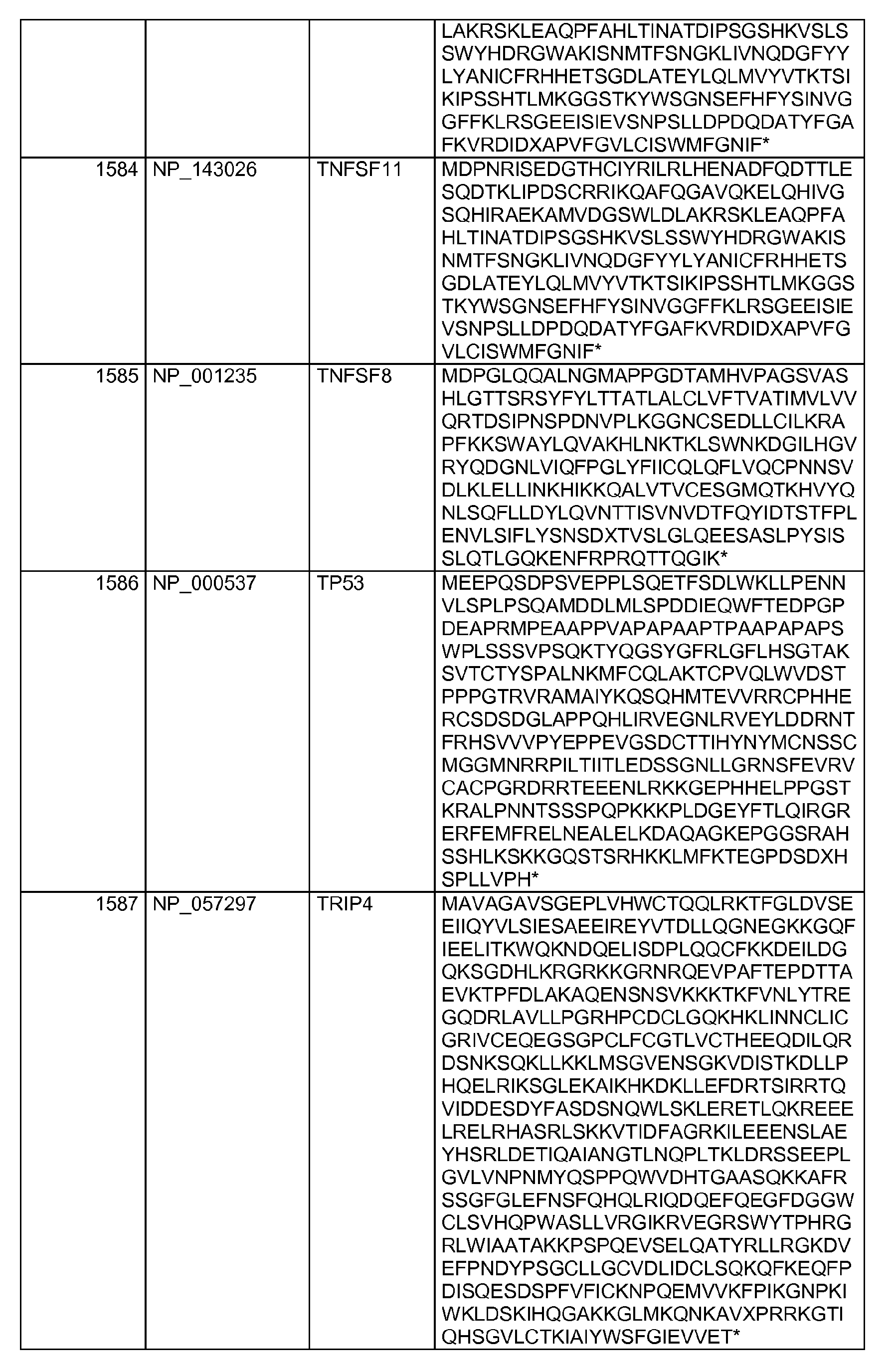

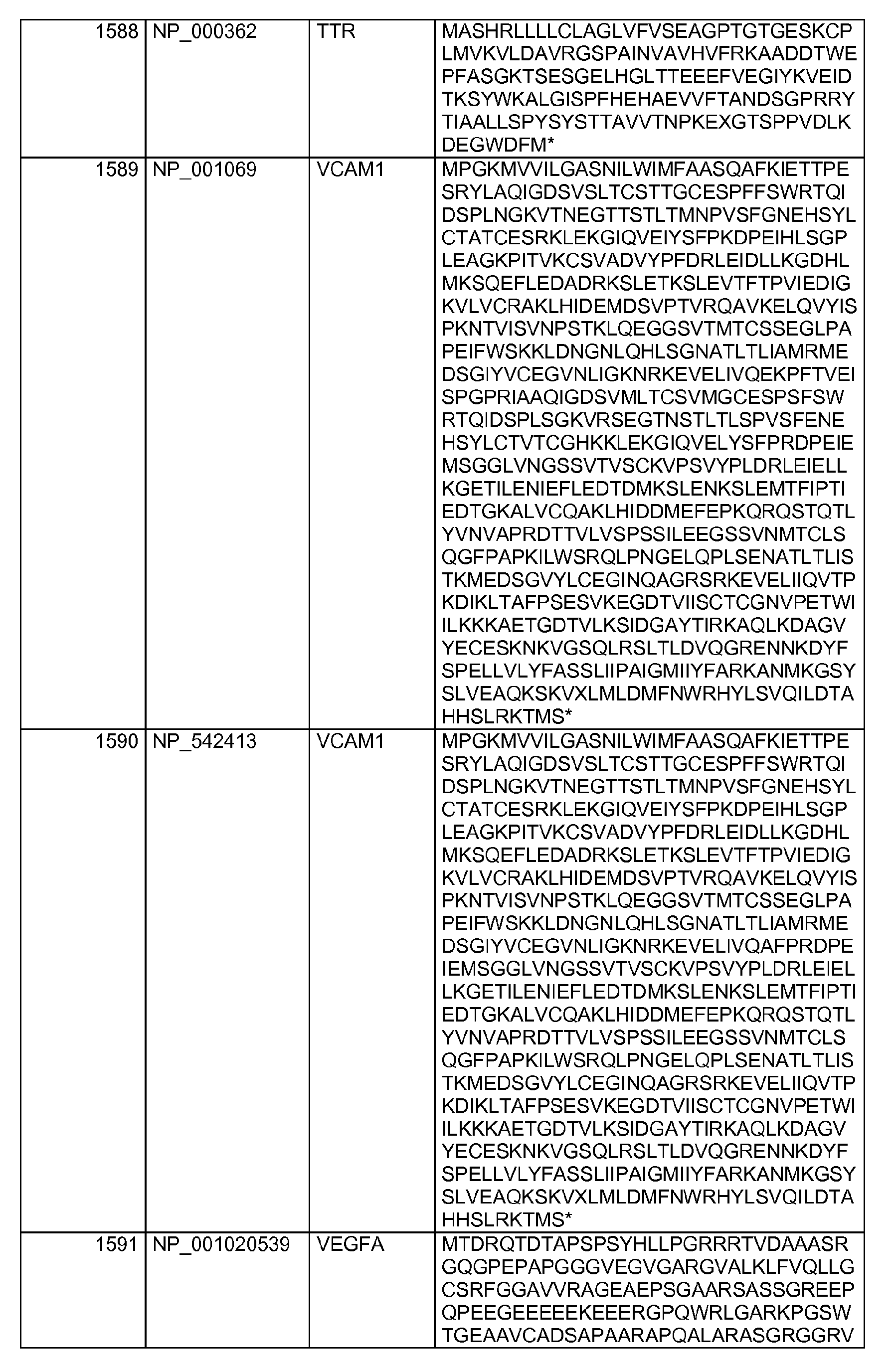

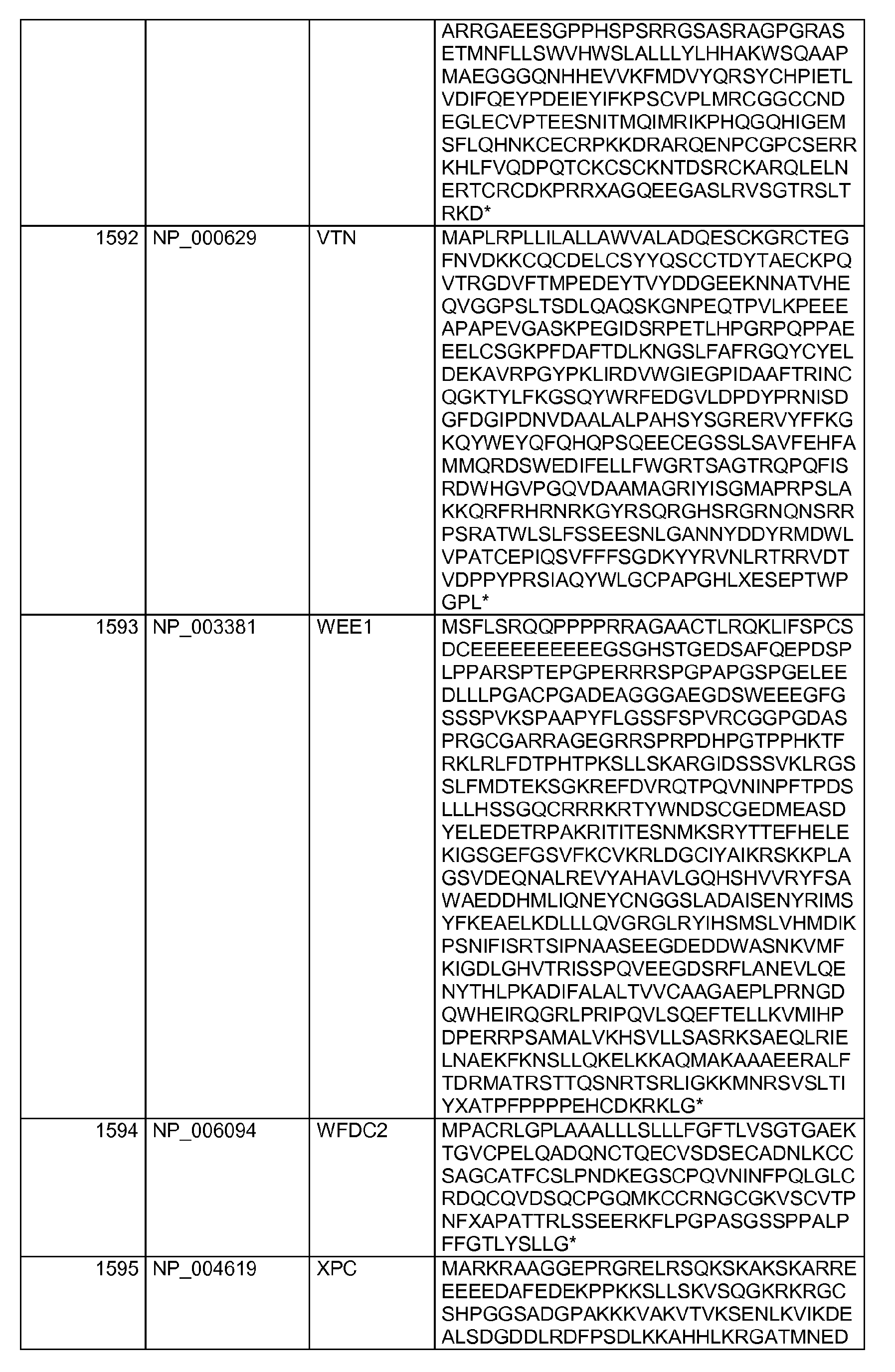

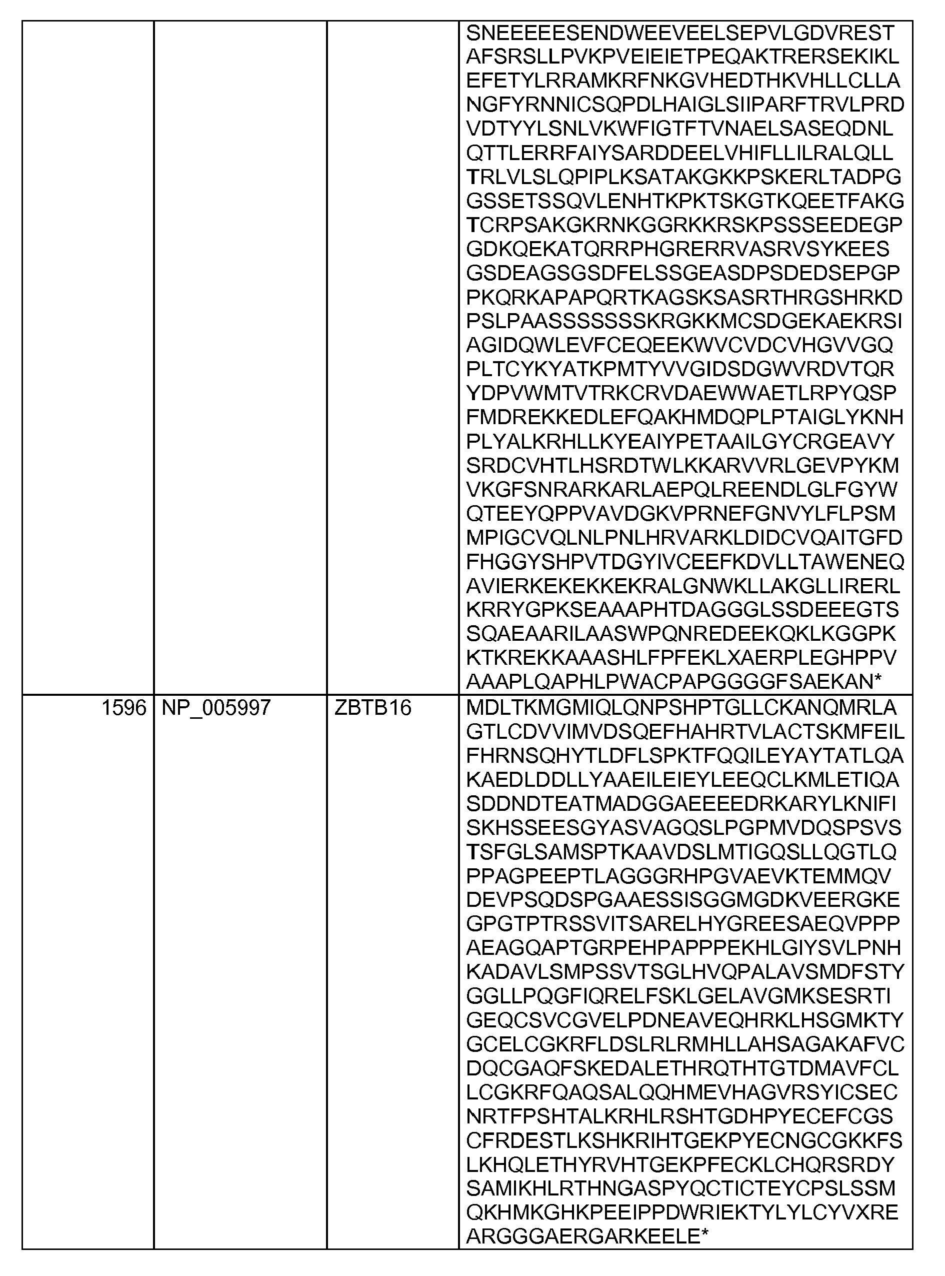

## Claims

1. A polypeptide comprising the sequence located on the C-terminal side of the X residue in any one of SEQ ID NOs: 1 to 1596, or of an epitope-containing fragment thereof.

2. The polypeptide of claim 1, having a length of from 3 to 100 amino acids.

3. A polynucleotide encoding a polypeptide of claim 1 or 2.

4. A polynucleotide comprising a first nucleotide sequence encoding a polypeptide comprising a PSP sequence as contained in any one of SEQ ID NOs: 1-1596 or a sequence complementary thereto and a second nucleotide sequence of 100 or less nucleotides in length, wherein said second nucleotide sequence is adjacent to said first nucleotide sequence in a naturally occurring nucleic acid.

5. An isolated antibody or portion of an antibody which specifically binds to a polypeptide comprising a PSP sequence as contained in any one of SEQ ID NOs: 1-1596.

6. An isolated cell which specifically binds to a polypeptide comprising a PSP sequence as contained in any one of SEQ ID NOs: 1-1596.

7. The isolated cell of Claim 5, wherein said cell is an immune cell comprising a TCR specific for a polypeptide comprising PSP sequence as contained in any one of SEQ ID NOs: 1-1596.

8. A solid support comprising an isolated nucleic acid which specifically binds to a polynucleotide encoding a polypeptide comprising a PSP sequence as contained in any one of SEQ ID NOs: 1-1596 or to a sequence complementary thereto.

9. A solid support comprising an antibody or portion of an antibody which specifically binds to a polypeptide comprising a PSP sequence as contained in any one of SEQ ID NOs: 1-1596.

10. A solid support comprising a polypeptide comprising a PSP sequence as contained in any one of SEQ ID NOs: 1-1596.

11. A method of determining whether an individual is making one or more polypeptides comprising a PSP sequence as contained in any one of SEQ ID NOs: 1-1596 comprising contacting a sample obtained from said individual with one or more agents indicative of the presence of said one or more polypeptides and determining whether said one or more agents bind to said sample.

12. The method of Claim 11, wherein said one or more agents are nucleic acids.

13. The method of Claim 11 wherein said nucleic acids are PCR primers which yield an amplification product only if said sample comprises nucleic acids encoding said one or more polypeptides or nucleic acids complementary to said nucleic acids encoding said one or more polypeptides.

14. The method of Claim 11, wherein said one or more agents are antibodies or portions thereof which specifically bind to a polypeptide comprising a PSP sequence as contained in any one of SEQ ID NOs: 1-1596.

15. The method of Claim 11, wherein said one or more agents are polypeptides which bind to antibodies in said sample which specifically bind to a polypeptide comprising a PSP sequence as contained in any one of SEQ ID NOs: 1-1596.

16. The method of Claim 11, wherein said one or more agents are cells.

17. The method of Claim 16, wherein said cells are immune cells comprising a TCR specific for a polypeptide comprising a PSP sequence as contained in any one of SEQ ID NOs: 1-1596.

18. A method of determining whether an individual is making one or more polypeptides comprising a PSP sequence as contained in any one of SEQ ID NOs: 1-1596 comprising determining whether a sample from said individual comprises nucleic acids encoding said one or more polypeptides or nucleic acids complementary thereto.

19. The method of Claim 18, wherein said determining step comprises contacting said sample with nucleic acid probes which specifically bind to said nucleic acids encoding said one or more polypeptides or to nucleic acids complementary thereto.

20. The method of Claim 19, wherein said probes are primers which yield an amplification product only if said sample comprises said nucleic acids encoding said one or more polypeptides or nucleic acids complementary thereto.

21. The method of Claim 18, wherein said determining step comprises contacting said sample with one or more antibodies or portions of antibodies which specifically bind to a polypeptide comprising a PSP sequence as contained in any one of SEQ ID NOs: 1-1596.

22. The method of Claim 18, wherein said determining step comprises contacting said sample with one or more cells which specifically bind to a polypeptide comprising a PSP sequence as contained in any one of SEQ ID NOs: 1-1596.

23. The method of Claim 22, wherein said cells are immune cell comprising a TCR specific for a polypeptide comprising a PSP sequence as contained in any one of SEQ ID NOs: 1-1596.

24. The method of Claim 18, said determining step comprises contacting said sample with one or more polypeptides comprising a PSP sequence as contained in any one of SEQ ID NOs: 1-1596 or a portion thereof and determining whether said sample comprises an antibody or cell which specifically binds to one or more polypeptides.

25. A method of determining the level of translation of post-stop peptides occurring in an individual comprising determining whether a sample from said individual comprises one or more post-stop peptides.

26. The method of Claim 25, wherein said one or more molecules comprise one or more nucleic acids encoding a post-stop peptide.

27. The method of Claim 25, wherein said one or more molecules comprise one or more post-stop peptides.

28. The method of Claim 25, wherein said one or more molecules comprise one or more antibodies which specifically bind to post-stop peptides.

29. The method of Claim 25, wherein said one or more molecules comprise TCR molecules expressed by an immune cell.

30. A method of determining whether a post-stop peptide is present in a sample comprising performing a mass spectrometry analysis on said sample and determining whether said sample contains a spectrum indicative of the presence of a post-stop peptide.

31. The method of Claim 30, wherein said post-stop peptide is selected from the group consisting of sequences located on the C-terminal side of the X residue in SEQ ID NOs: 1-1596.

32. The method of Claim 30, wherein said mass spectrometry analysis comprises a tandem mass spectrometry analysis.

33. A method for determining whether a post-stop peptide is differentially expressed in a first population of individuals relative to a second population of individuals comprising:
determining a first level of expression of said post-stop peptide in said first population of individuals;
determining a second level of said post-stop peptide in said second population of individuals; and
comparing said first level of expression and said second level of expression, whereby said post-stop peptide is differentially expressed in said first population of individuals relative to said second population of individuals if there is a statistically significant difference between said first level of expression and said second level of expression.

34. The method of Claim 33, wherein said first population of individuals suffers from a particular disease and said second population does not suffer from said disease.

35. The method of Claim 34, wherein said disease is cancer.

36. The method of Claim 33, wherein said post-stop peptide is selected from the group consisting of sequences located on the C-terminal side of the X residue in SEQ ID NOs: 1-1596.

37. A method of identifying differentially expressed nucleic acids encoding post-stop peptides comprising:
determining a first level of a variant nucleic acid in a first population of individuals, wherein said variant nucleic acid results from a base substitution which converts a stop codon into a codon encoding an amino acid, wherein said base substitution creates a new open reading frame encoding at least 3 amino acids beyond said converted stop codon and wherein said new open reading frame is in frame with an open reading frame preceding said stop codon;
determining a second level of said variant nucleic acid in a second population of individuals; and
comparing said first level of expression and said second level of expression, whereby said variant nucleic acid is differentially expressed in said first population of individuals relative to said second population of individuals if there is a statistically significant difference between said first level of expression and said second level of expression.

38. The method of Claim 37, wherein said first population of individuals suffers from a particular disease and said second population does not suffer from said disease.

39. The method of Claim 37, wherein said disease is cancer.

40. The method of Claim 37, wherein said variant nucleic acid encodes a post-stop peptide selected from the group consisting of sequences located on the C-terminal side of the X residue in SEQ ID NOs: 1-1596.

41. A method for identifying nucleic acids capable of encoding post-stop peptides comprising:
obtaining a plurality of nucleic acid sequences wherein each nucleic acid sequence comprises an open reading frame encoding a polypeptide, said open reading frame terminating with a stop codon; and
identifying those nucleic acids within said plurality of nucleic acid sequences which contain an open reading frame immediately after said stop codon which is in frame with the open reading frame encoding said polypeptide, wherein said open reading frame immediately after said stop codon encodes at least 3 amino acids.

42. The method of Claim 41, further comprising determining whether any of said identified nucleic acids are differentially expressed in a first population of individuals relative to a second population of individuals.

43. The method of Claim 42, wherein said first population of individuals suffers from a particular disease and said second population does not suffer from said disease.

44. The method of Claim 43, wherein said disease is cancer.

45. The method of Claim 41, wherein said plurality of nucleic acid sequences comprise nucleic acid sequences encoding secreted proteins.

46. The method of Claim 41, wherein said plurality of nucleic acid sequences comprise nucleic acid sequences encoding tumor markers.
